# EUROPEAN PATENT SPECIFICATION

(11) **EP 0 996 857 B1**
(45) Date of publication and mention of the grant of the patent: **09.09.2009**
(21) Application number: 98936860.0
(22) Date of filing: 15.07.1998
(51) Int. Cl.: G01N 33/574, C07K 14/00

(54) **CANCER ASSOCIATED NUCLEIC ACIDS AND POLYPEPTIDES**
KREBSASSOZIIERTE NUKLEINSÄURE UND PROTEINE
ACIDES NUCLEIQUES ET POLYPEPTIDES ASSOCIES AU CANCER

(30) Priority: 17.07.1997 US 896164; 10.10.1997 US 61599 P; 10.10.1997 US 61765 P; 10.10.1997 US 948705; 11.10.1997 GB 9721697; 22.06.1998 US 102322
(43) Date of publication of application: 03.05.2000
(73) Proprietor: LUDWIG INSTITUTE FOR CANCER RESEARCH, New York, New York 10158 (US); Sloan-Kettering Institute For Cancer Research, New York, New York 10021 (US); CORNELL RESEARCH FOUNDATION, INC., Ithaca, NY 14850 (US)
(72) Inventor: OLD, Lloyd, J., New York, NY 10158 (US); SCANLAN, Matthew, J., New York, NY 10021 (US); STOCKERT, Elisabeth, New York, NY 10021 (US); GURE, Ali, New York, NY 10021 (US); CHEN, Yao-Tseng, New York, NY 10021 (US); GOUT, Ivan, London WIP 8BT (GB); O'HARE, Michael, London W1P 8BT (GB); OBATA, Yuichi, Nagoya 464 (JP); PFREUNDSCHUH, Michael, D-66421 Homburg/Saar (DE); TURECI, Ozlem, D-66421 Homburg/Saar (DE); SAHIN, Ugur, D-66421 Homburg/Saar (DE)
(74) Representative: Gill, Siân Victoria
(86) International application number: PCT/US1998/014679
(87) International publication number: WO 1999/004265

(56) References cited:
- WO-A-92/20356
- WO-A-95/23874
- WO-A-96/10413
- WO-A-96/15149
- WO-A-96/29409
- WO-A-97/02362
- WO-A-97/17441
- WO-A-97/17470
- WO-A-97/21729
- WO-A-98/08866
- WO-A-98/32853
- WO-A-98/40483
- WO-A-98/48015
- GB-A- 2 273 099
- US-A- 5 858 723
- VAUGHAN, J.H. ET AL.: "Epstein-Barr virus-induced autoimmune responses." JOURNAL OF CLINICAL INVESTIGANTION, vol. 95, no. 3, March 1995, pages 1306-15, XP002103180 -& DATABASE EMBL - EMHUM1 Entry HSIGGAUA, Acc.no. L38696, 17 February 1995 VAUGHAN, J.H. ET AL.: "Homo sapiens autoantigen p542 mRNA, complete cds." XP002103198
- MASHIMO, J. ET AL.: "Decrease in the expression of a novel TGF beta1-inducible and ras-recision gene, TSC-36, in human cancer cells." CANCER LETTERS, vol. 113, March 1997, pages 213-9, XP002104545
- MACHIELS, B.M. ET AL.: "Nuclear lamin expression in normal testis and testicular germ cell tumours of adolescents and adults." JOURNAL OF PATHOLOGY, vol. 182, no. 2, June 1997, pages 197-204, XP002104546
- COATES, P.J. ET AL.: "Identification of the antigen recognized by the monoclonal antibody BU31 as lamins A and C" JOURNAL OF PATHOLOGY, vol. 178, no. 1, January 1996, pages 21-9, XP002104547
- ZWIJSEN, A. ET AL.: "Characterization of a rat C6 glioma-secreted follistatin-related protein (FRP); cloning and sequencing of the human homologue." EUROPEAN JOURNAL OF BIOCHEMISTRY, vol. 225, no. 3, November 1994, pages 937-46, XP002103181
- MINEGISHI, M. ET AL.: "Structure and function of Cas-L, a 105 kD Crk-associated substructure-related protein that is involved in beta-1 integrin-mediated signaling in lymphocytes." JOURNAL OF EXPERIMENTAL MEDICINE, vol. 184, no. 4, 1 October 1996, pages 1365-75, XP002103183
- JIN, Y-J. ET AL.: "The 25-kDa FK506-binding protein is localized in the nucleus and associated with casein kinase II and nucleolin." PROC.NAT'L.ACAD.SCI.USA, vol. 90, August 1993, pages 7769-73, XP002104548
- NOCE, T. ET AL.: "Expresson of a mouse zinc finger protein gene in both spermatocytes and oocytes during meiosis." DEVELOPMENTAL BIOLOGY, vol. 153, no. 2, October 1992, pages 356-67, XP002104549 -& DATABASE EMBL - EMROD Entry MMZFP51, Acc.no. D10630, 8 November 1992 NOCE, T. ET AL.: "Mouse mRNA for zinc finger protein, complete cds." XP002104555
- ONO M ET AL: "NUCLEOTIDE SEQUENCE OF HUMAN ENDOGENOUS RETROVIRUS GENOME RELATED TO THE MOUSE MAMMARY TUMOR VIRUS GENOME" JOURNAL OF VIROLOGY, vol. 60, no. 2, 1 November 1986, pages 589-598, XP000673638
- DATABASE EMBL - EMEST16 Entry HSC9958, Acc.no. C15995, 29 September 1996 FUJIWARA, T. ET AL.: "Human fetal brain cDNA 5'-end GEN-421G02." XP002103191
- DATABASE EMBL - EMEST13 Entry HS570350, Acc.no. W45570, 27 May 1996 HILLIER, L. ET AL.: "zc26f08.s1 Soares senescent fibroblasts NbHSF Homo sapiens cDNA clone 323463 3'" XP002103192
- DATABASE EMBL - EMEST15 Entry HSA07407, Acc.no. AA007407, 28 July 1996 HILLIER, L. ET AL.: "zh97b08.r1 Soares fetal liver spleen 1NFLS S1 Homo sapiens cDNA clone 429207 5'" XP002103193
- HUNG, D.T. ET AL.: "cDNA cloning of a human 25 kDa FK506 and rapamycin binding protein." BIOCHEMICAL AND BIOPHYSICAL RESEARCH COMMUNICATIONS, vol. 184, no. 2, 30 April 1992, pages 733-8, XP002103178
- JIN, Y-J. ET AL.: "Molecular cloning of a 25-kDa high affinity rapamycin binding protein, FKBP25." JOURNAL OF BIOLOGICAL CHEMISTRY, vol. 267, no. 16, 5 June 1992, pages 10942-5, XP002104550
- MACLEOD, A.R. ET AL.: "A muscle-type tropomycin in human fibroblasts: evidence for expression by an alternative RNA splicing mechanism." PROC.NAT'L.ACAD.SCI.USA, vol. 82, December 1985, pages 7835-9, XP002103179
- DATABASE EMBL - EMEST20 Entry/Acc.no. T09468, 8 August 1993 ADAMS, M.D. ET AL.: "EST07361 Homo sapiens cDNA clone HIBBU63 5' end." XP002103195 -& ADAMS, M.D. ET AL.: "Rapid DNA sequencing (expressed sequence tags) from a directionally cloned human infant brain cDNA library." NATURE GENETICS, vol. 4, 1993, pages 373-380, XP000574910
- DATABASE EMBL - EMEST17 Entry HSZZ32361, Acc.no. AA327309, 18 April 1997 ADAMS, M.D. ET AL.: "EST30621 Colon I Homo sapiens cDNA 5' end." XP002103199 -& ADAMS, M.D. ET AL.: "Initial assessment of human gene diversity and expression patterns based upon 83 million nucleotides of cDNA sequence." NATURE, vol. 377, 1995, pages 3-17, XP002042918
- DATABASE EMBL - EMEST15 Entry HSAA33416, Acc.no. AA133416, 6 December 1996 HILLIER, L. ET AL.: "zk96e08.r1 Soares pregnant uterus NbHPU Homo sapiens cDNA clone 490694 5'." XP002103196
- DATABASE EMBL - EMEST11 Entry HS1282878, Acc.no. AA487071, 28 June 1997 HILLIER, L. ET AL.: "ab18f11.s1 Stratagene lung (#937210) Homo sapiens cDNA clone 841197 3' similar to contains Alu repetitive element." XP002103197
- DATABASE EMBL - EMEST15 Entry HSAA21198, entry AA121198, 21 November 1996 HILLIER, L. ET AL.: "zl88g08.r1 Stratagene colon (#937204) Homo sapiens cDNA clone 511742 5'." XP002103200
- DATABASE EMBL - EMEST15 Entry HSAA21174, Acc.no. AA121174, 21 November 1996 HILLIER, L. ET AL.: "zl88g08.s1 Stratagene colon (#937204) Homo sapiens cDNA clone 511742 3'." XP002103202
- DATABASE EMBL - EMEST17 Entry HSW22160, Acc.no. W22160, 9 May 1996 NATHANS, J.: "63A6 Human retina cDNA Tsp509I-cleaved sublibrary Homo sapiens cDNA not directional." XP002103201
- DATABASE EMBL - EMEST15 Entry HSA29201, Acc.no. AA029201, 20 August 1996 HILLIER, L. ET AL.: "zk12f08.s1 Soares pregnant uterus NbHPU Homo sapiens cDNA clone 470343 3'." XP002103203
- DATABASE EMBL - EMEST17 Entry HSW29097, Acc.no. W29097, 14 May 1996 NATHANS, J.: "56d11 Human retina cDNA randomly primed sublibrary Homo sapiens cDNA." XP002103204
- MIKI Y ET AL: "A STRONG CANDIDATE FOR THE BREAST AND OVARIAN CANCER SUSCEPTIBILITY GENE BRCA1" SCIENCE, vol. 266, no. 12, 7 October 1994, pages 66-71, XP000202410 -& DATABASE EMBL - EMEST5 Entry/Acc.no. AF039241, 17 January 1998 MIKI, Y. ET AL.: "Homo sapiens clone 11-67js mRNA,partial sequence." XP002103205
- DATABASE EMBL - EMEST18 Entry MM1140465, Acc.no. AA221749, 15 February 1997 MARRA, M. ET AL.: "my28g01.r1 Barstead mouse pooled organs MPLRB4 Mus musculus cDNA clone 697200 5' similar to TR:E239664 E239664 CHROMOSOME XIV READING FRAME ORF YNL021W." XP002103206
- NAGASE T ET AL: "PREDICTION OF THE CODING SEQUENCES OF UNIDENTIFIED HUMAN GENES VI.THE CODING SEQUENCES OF 80 NEW GENES (KIAA0201-KIAA0280) DEDUCED BYANALYSIS OF CDNA CLONES FROM CELL LINE KG-1 AND BRAIN" DNA RESEARCH, vol. 3, no. 5, 1 January 1996, pages 321-329, XP002059454 -& DATABASE EMBL - EMHUM1 Entry HSD455, Acc.no. D87455, 9 November 1996 NOMURA, N.: "Human mRNA for KIAA0266 gene, complete cds." XP002103207
- DATABASE EMBL - EMEST16 Entry HSAA51187, Acc.no. AA151187, 15 December 1996 HILLIER, L. ET AL.: "zo03c11.r1 Stratagene colon (#937204) Homo sapiens cDNA clone 566612 5'." XP002103208
- DATABASE EMBL - EMHUM2 Entry HSU50839, Acc.no. U50839, 9 March 1997 LATIF, F. ET AL.: "Homo sapiens g16 protein (g16) mRNA, complete cds." XP002103209
- LI, H. ET AL.: "Isolation and sequence analysis of the human syntaxin-encoding gene." GENE, vol. 143, 1994, pages 303-4, XP002103182
- DATABASE EMBL - EMEST11 Entry HS1188646, Acc.no. AA285170, 5 April 1997 STRAUSBERG, R.: "zs48f04.s1 NCI_CGAP_GCB1 Homo sapiens cDNA clone IMAGE:700735 3'." XP002103210
- FISHER, D.Z. ET AL.: "cDNA sequencing of nuclear lamins A and C reveals primary and secondary structural homolgy to intermediate filament proteins." PROC.NAT'L.ACAD.SCI.USA, vol. 83, September 1986, pages 6450-4, XP002103184
- DATABASE EMBL - EMEST16 Entry HSAA54222, Acc.no. AA454222, 11 June 1997 HILLIER, L. ET AL.: "zx48g12.s1 Soares testis NHT Homo sapiens cDNA clone 795526 3' similar to gb:D42040 RING3 PROTEIN (HUMAN)" XP002103189
- DATABASE EMBL - EMEST11 EntryHS125289, Acc.no. AA454221, 11 June 1997 HILLIER, L. ET AL.: "zx48g12.r1 Soares testis NHT Homo sapiens cDNA clone 795526 5' similar to TR:E243068 E243068 KINASE." XP002103190
- DATABASE EMBL - EMEST20 Entry MMAA84412, Acc.no. AA184412, 19 February 1997 MARRA, M. ET AL.: "mt34f07.r1 Soares mouse 3NbMS Mus musculus cDNA clone 622981 5' similar to SW:OXYB_HUMAN P22059 OXYSTEROL-BINDING PROTEIN." XP002103194
- FRANZÉN, B. ET AL.: "Analysis of polypeptide expression in benign and malignant human breast lesions: down-regulation of cytokeratins." BRITISH JOURNAL OF CANCER, vol. 73, 1996, pages 1632-8, XP002104551
- BOON T ET AL: "Tumor antigens recognized by T cells" IMMUNOLOGY TODAY, vol. 18, no. 6, June 1997, page 267-268 XP004068293
- SAHIN, U. ET AL.: "Human neoplasms elicit multiple specific immune responses in the autologous host." PROC.NATL.ACAD.SCI.USA, vol. 92, December 1995, pages 11810-3, XP002091914 cited in the application
- DATABASE EMBL - EMHUM1 Entry/Acc.no. AC004022, 22 January 1998 HINDS, K. ET AL.: "Homo sapiens BAC clone GS155M11 from 7q21-q22, complete sequence." XP002091837
- ALAIYA, A.A. ET AL.: "Phenotypic analysis of ovarian carcinoma: polypeptide expression in benign, boderline and malignant tumors." JOURNAL OF CNACER, vol. 73, no. 5, 27 November 1997, pages 678-83, XP002104552
- GÜRE, A.O. ET AL.: "Human lung cancer antigens recognized by autologous antibodies: definition of a novel cDNA derived from the tumor suppressor gene locus on chromosome 3p21.3" CANCER RESEARCH, vol. 58, 1 March 1998, pages 1034-41, XP002103188
- SCANLAN, M.J. ET AL.: "Characterization of human colon cancer antigens recognized by autologous antibodies" INTERNATIONAL JOURNAL OF CANCER, 29 May 1998, pages 652-8, XP002103186
- NAGASE, T. ET AL.: "Prediction of the coding sequence of unidentified human genes. IX. The complete sequence of 100 new cDNA clones from barin which can code for large proteins in vivo." DNA RESEARCH, vol. 5, 28 February 1998, pages 31-39, XP002103187 -& DATABASE EMBL Entry/acc.no. AB011172, 10 April 1998 NAGASE, T. ET AL.: "Homo sapiens mRNA for KIAA0600 protein, partial cds." XP002104556
- JONES, M.H. ET AL.: "Identification and characterization of BRDT: a testis-specific gene related to the bromodomain genes RING3 and Drosophila fsh." GENOMICS, vol. 45, no. 3, 1 November 1997, pages 529-34, XP002103185
- ISHIKAWA K ET AL: "Prediction of the coding sequences of unidentified human genes. X The complete sequences of 100 new cDNA clones from brain which can code for large proteins in vitro" DNA RESEARCH, vol. 5, no. 321, 30 June 1998, pages 169-176, XP002089186

## Description

### Field of the Invention

The invention relates to nucleic acids and encoded polypeptides which are cancer associated antigens expressed in patients afflicted with breast cancer. The invention also relates to agents which bind the nucleic acids or polypeptides. The nucleic acid molecules, polypeptides coded for by such molecules and peptides derived therefrom, as well as related antibodies and cytolytic T lymphocytes, are useful, *inter alia*, in diagnostic and therapeutic contexts.

### Background of the Invention

The mechanism by which T cells recognize foreign materials has been implicated in cancer. A number of cytolytic T lymphocyte (CTL) clones directed against autologous melanoma antigens, testicular antigens, and melanocyte differentiation antigens have been described. In many instances, the antigens recognized by these clones have been characterized.

The use of autologous CTLs for identifying tumor antigens requires that the target cells which express the antigens can be cultured *in vitro* and that stable lines of autologous CTL clones which recognize the antigen-expressing cells can be isolated and propagated. While this approach has worked well for melanoma antigens, other tumor types, such as epithelial cancers including breast and colon cancer, have proved refractory to the approach.

More recently another approach to the problem has been described by Sahin et al. (Proc. Natl. Acad. Sci. USA 92:11810-11813, 1995). According to this approach, autologous antisera are used to identify immunogenic protein antigens expressed in cancer cells by screening expression libraries constructed from tumor cell cDNA. Antigen-encoding clones so identified have been found to have elicited an high-titer humoral immune response in the patients from which the antisera were obtained. Such a high-titer IgG response implies helper T cell recognition of the detected antigen. These tumor antigens can then be screened for the presence of MHC/HLA class I and class II motifs and reactivity with CTLs

The invention is elaborated upon in the disclosure which follows.

### Summary of the Invention

Autologous antibody screening has now been applied to cancer using antisera from cancer patients. Numerous cancer associated antigens have been identified. The invention provides, *inter alia*, isolated nucleic acid molecules, expression vectors containing those molecules and host cells transfected with those molecules. The invention also provides isolated proteins and peptides, antibodies to those proteins and peptides and CTLs which recognize the proteins and peptides. Fragments including functional fragments and variants of the foregoing also are provided. Kits containing the foregoing molecules additionally are provided. The foregoing can be used in the diagnosis, monitoring, research, or treatment of conditions characterized by the expression of one or more cancer associated antigens.

Prior to the present invention, only a handful of cancer associated genes had been identified in the past 20 years. The invention involves the surprising discovery of many genes, some previously known and many previously unknown, which are expressed in individuals who have cancer. These individuals all have serum antibodies against the proteins (or fragments thereof) encoded by these genes. Thus, abnormally expressed genes are recognized by the host's immune system and therefore can form a basis for diagnosis, monitoring and therapy.

The invention involves the use of a single material, a plurality of different materials and even large panels and combinations of materials. For example, a single gene, a single protein encoded by a gene, a single functional fragment thereof, a single antibody thereto, etc. can be used in methods and products of the invention. Likewise, pairs, groups and even panels of these materials can be used for diagnosis, monitoring and therapy. The pairs, groups or panels can involve 2, 3, 4, 5... to as many as 25, 50, 100 or more genes, gene products, fragments thereof or agents that recognize such materials. A plurality of such materials are not only useful in monitoring, typing, characterizing and diagnosing cells abnormally expressing such genes, but a plurality of such materials can be used therapeutically. An example of the use of a plurality of such materials for the prevention, delay of onset, amelioration, etc. of cancer cells, which express or will express such genes prophylactically or acutely. Any and all combinations of the genes, gene products, and materials which recognize the genes and gene products can be tested and identified for use according to the invention. It would be far too lengthy to recite all such combinations; those skilled in the art, particularly in view of the teaching contained herein, will readily be able to determine which combinations-are most appropriate for which circumstances.

As will be clear from the following discussion, the invention has *in vivo* and *in vitro* uses, including for therapeutic, diagnostic, monitoring and research purposes. One aspect of the invention is the ability to fingerprint a cell expressing a number of the genes identified according to the invention. Such fingerprints will be characteristic, for example, of the stage of the cancer, the type of the cancer, or even the effect in animal models of a therapy on a cancer. Cells also can be screened to determine whether such cells abnormally express the genes identified according to the invention.

The invention is defined by the claims, all of which are directed to SEQ ID No: 681. It should therefore be appreciated that references to other sequences described herein do not form part of the invention as such.

Hence, the invention provides, in a first aspect, an isolated nucleic acid molecule which is (I) a nucleic acid molecule which hybridizes at 65°C in hybridisation buffer (3.5 x SSC, 0.02% Ficoll, 0.02% polyvinyl pyrrolidone, 0.02% Bovine Serum Albumin, 2.5mM NaH₂PO₄ (pH7), 0.5% SDS, 2mM EDTA) to a molecule consisting of SEQ ID NO: 681 and which codes for a cancer associated antigen precursor; (II) a nucleic acid molecule that differs from the nucleic acid molecule of (I) in codon sequence due to the degeneracy of the genetic code; or (III) a complement of (I) or (II).

The invention, in one aspect, provides a method of diagnosing a cancer, the method comprising contacting a biological sample isolated from a subject with an agent that specifically brings to a nucleic acid molecule according to the first aspect of the invention, and determining the interaction between the agent and the nucleic acid molecule as a determination of a cancer; wherein the agent is a nucleic acid molecule which hybridizes at 65°C in hybridization buffer (3.5 x SSC, 0.02% Ficoll, 0.02% polyvinyl pyrrolidone, 0.02% Bovine Serum Albumin, 2.5mM NaH₈PO₄ (pH7), 0.5% SDS, 2mM EDTA) to a nucleic acid molecule according to the first aspect of the invention or fragment thereof.

In another aspect, there is provided a method of diagnosing a cancer, the method comprising contacting a biological sample isolated from a subject with an antibody or an antigen-binding fragment thereof that specifically binds to a polypeptide encoded by a nucleic acid molecule according to the first aspect of the invention and determining the interaction between the agent and the polypeptide as a determination of a cancer. The polypeptide may be complexed with an HLA molecule.

The methods involve the steps of contacting a biological sample isolated from a subject with an agent that specifically binds to the nucleic acid molecule, an expression product thereof, or a fragment of an expression product thereof complexed with an MHC, preferably an HLA, molecule, wherein the nucleic acid molecule is a NA Group 1 nucleic acid molecule, and determining the interaction between the agent and the nucleic acid molecule, the expression product or fragment of the expression product as a determination of the disorder.

In one embodiment the agent is selected from the group consisting of (a) a nucleic acid molecule comprising NA Group 1 nucleic acid molecules or a fragment thereof, (b) a nucleic acid molecule comprising NA Group 3 nucleic acid molecules or a fragment thereof, (c) a nucleic acid molecule comprising NA Group 17 nucleic acid molecules or a fragment thereof, (d) an antibody that binds to an expression product, or a fragment thereof, of NA group 1 nucleic acids, (e) an antibody that binds to an expression product, or a fragment thereof, of NA group 3 nucleic acids, (f) an antibody that binds to an expression product, or a fragment thereof, of NA group 17 nucleic acids, (g) and agent that binds to a complex of an MHC, preferably HLA, molecule and a fragment of an expression product of a NA Group 1 nucleic acid, (h) an agent that binds to a complex of an MHC, preferably HLA, molecule and a fragment of an expression product of a NA group 3 nucleic acid, and (I) an agent that binds to a complex of an MHC, preferably HLA, molecule and a fragment of an expression product of a NA Group 17 nucleic acid.

The disorder may be characterized by expression of a plurality of cancer associated antigen precursors and wherein the agent is a plurality of agents, each of which is specific for a different human cancer associated antigen precursor, and wherein said plurality of agents is at least 2, at least 3, at least 4, at least 5, at least 6, at least 7, at least 8, at least 9 or at least 10 such agents.

In each of the above embodiments the agent may be specific for a human cancer associated antigen precursor that is a breast, a gastric, a lung, a prostate, a renal or a colon cancer associated antigen precursor.

In another aspect the invention is a method for determining regression, progression or onset of a cancer characterized by expression of abnormal levels of a protein encoded by a nucleic acid molecule according to the first aspect of the invention, the method comprising: monitoring a sample from a patient who has or is suspected of having a cancer for a parameter selected from the group consisting of: (i) the protein, (ii) a peptide derived from the protein, (iii) an antibody which selectively binds the protein or peptide, and (iv) cytolytic T cells specific for a complex of the peptide derived from the protein and an MHC molecule, as a determination of regression, progression or onset of said cancer, wherein the sample is preferably a body fluid, a body effusion or a tissue.

The step of monitoring may comprise contacting the sample with a detectable agent selected from the group consisting of (a) an antibody or an antigen-binding fragment thereof which selectively binds the protein of (i), or the peptide of (ii); (b) a protein or peptide which binds the antibody of (iii); and (c) a cell which presents the complex of the peptide and MHC molecule of (iv).

The method may involve the steps of monitoring a sample, from a subject who has or is suspected of having the condition, for a parameter selected from the group consisting of (i) the protein, (ii) a peptide derived from the protein, (iii) an antibody which selectively binds the protein or peptide, and (iv) cytolytic T cells specific for a complex of the peptide derived from the protein and an MHC molecule, as a determination of regression, progression or onset of said condition. In one embodiment the sample is a body fluid, a body effusion or a tissue.

In another embodiment the step of monitoring comprises contacting the sample with a detectable agent selected from the group consisting of (a) an antibody which selectively binds the protein of (i), or the peptide of (ii), (b) a protein or peptide which binds the antibody of (iii), and (c) a cell which presents the complex of the peptide and MHC molecule of (iv). In a preferred embodiment the antibody, the protein, the peptide or the cell is labeled with a radioactive label or an enzyme. The sample in a preferred embodiment is assayed for the peptide.

According to another embodiment the nucleic acid molecule is one of the following: a NA Group 3 molecule, a NA Group 11 molecule, a NA Group 12 molecule, a NA Group 13 molecule, a NA Group 14 molecule, a NA Group 15 molecule, or a NA Group 16 molecule. In yet another embodiment the protein is a plurality of proteins, the parameter is a plurality of parameters, each of the plurality of parameters being specific for a different of the plurality of proteins.

The invention in another aspect is a pharmaceutical preparation for a human subject. The pharmaceutical preparation includes an agent which when administered to the subject enriches selectively the presence of complexes of an HLA molecule and a human cancer associated antigen, and a pharmaceutically acceptable carrier, wherein the human cancer associated antigen is a fragment of a human cancer associated antigen precursor encoded by a nucleic acid molecule which comprises a NA Group 1 molecule. In one embodiment the nucleic acid molecule is a NA Group 3 nucleic acid molecule.

The agent in one embodiment comprises a plurality of agents, each of which enriches selectively in the subject complexes of an HLA molecule and a different human cancer associated antigen. Preferably the plurality is at least two, at least three, at least four or at least 5 different such agents.

In another embodiment the agent is selected from the group consisting of (1) an isolated polypeptide comprising the human cancer associated antigen, or a functional variant thereof, (2) an isolated nucleic acid operably linked to a promoter for expressing the isolated polypeptide, or functional variant thereof, (3) a host cell expressing the isolated polypeptide, or functional variant thereof, and (4) isolated complexes of the polypeptide, or functional variant thereof, and an HLA molecule.

The agent may be a cell expressing an isolated polypeptide. In one embodiment the agent is a cell expressing an isolated polypeptide comprising the human cancer associated antigen or a functional variant thereof, and wherein the cell is nonproliferative. In another embodiment the agent is a cell expressing an isolated polypeptide comprising the human cancer associated antigen or a functional variant thereof, and wherein the cell expresses an HLA molecule that binds the polypeptide. The cell can express one or both of the polypeptide and HLA molecule recombinantly. In another preferred embodiment the cell is nonproliferative. In yet another embodiment the agent is at least two, at least three, at least four or at least five different polypeptides, each representing a different human cancer associated antigen or functional variant thereof.

The agent in one embodiment is a PP Group 2 polypeptide. In other embodiments the agent is a PP Group 3 polypeptide or a PP Group 4 polypeptide.

In an embodiment each of the pharmaceutical preparations described herein also includes an adjuvant.

According to another aspect the invention, a composition is provided of an isolated agent that binds selectively a PP Group 1 polypeptide. In separate embodiments the agent binds selectively to a polypeptide selected from the following: a PP Group 3 polypeptide, a PP Group 11 polypeptide, a PP Group 12 polypeptide, a PP Group 13 polypeptide, a PP Group 14 polypeptide, a PP Group 15 polypeptide, and a PP Group 16 polypeptide. In other embodiments, the agent is a plurality of different agents that bind selectively at least two, at least three, at least four, or at least five different such polypeptides. In each of the above described embodiments the agent may be an antibody.

In another aspect the invention is a composition of matter composed of a conjugate of the agent of the above-described compositions of the invention and a therapeutic or diagnostic agent. Preferably the conjugate is of the agent and a therapeutic or diagnostic that is an antineoplastic.

The invention in another aspect is a pharmaceutical composition of an isolated nucleic acid molecule selected from the group consisting of: (1) NA Group 1 molecules, and (2) NA Group 2 molecules, and a pharmaceutically acceptable carrier. In one embodiment the isolated nucleic acid molecule comprises a NA Group 3 or NA Group 4 molecule. In another embodiment the isolated nucleic acid molecule comprises at least two isolated nucleic acid molecules coding for two different polypeptides, each polypeptide comprising a different cancer associated antigen.

Preferably the pharmaceutical composition also includes an expression vector with a promoter operably linked to the isolated nucleic acid molecule. In another embodiment the pharmaceutical composition also includes a host cell recombinantly expressing the isolated nucleic acid molecule.

According to another aspect of the invention a pharmaceutical composition is provided. The pharmaceutical composition includes an isolated polypeptide comprising a PP Group 1 or a PP Group 2 polypeptide, and a pharmaceutically acceptable carrier. In one embodiment the isolated polypeptide comprises a PP Group 3 or a PP Group 4 polypeptide.

In another embodiment the isolated polypeptide comprises at least two different polypeptides, each comprising a different cancer associated antigen. In separate embodiments the isolated polypeptides are selected from the following: PP Group 11 polypeptides or HLA binding fragments thereof, PP Group 12 polypeptides or HLA binding fragments thereof, PP Group 13 polypeptides or HLA binding fragments thereof, PP Group 14 polypeptides or HLA binding fragments thereof, PP Group 15 polypeptides or HLA binding fragments thereof, or PP Group 16 polypeptides or HLA binding fragments thereof.

In an embodiment each of the pharmaceutical compositions described herein also includes an adjuvant.

Another aspect the invention is an isolated nucleic acid molecule comprising a NA Group 3 molecule. Another aspect the invention is an isolated nucleic acid molecule comprising a NA Group 4 molecule. In separate embodiments the isolated nucleic acid molecules are selected from the following: a Group 11 molecule or a functional fragment thereof, a Group 12 molecule or a functional fragment thereof, a Group 13 molecule or a functional fragment thereof, a Group 14 molecule or a functional fragment thereof, a Group.15 molecule or a functional fragment thereof, or a Group 16 molecule or a functional fragment thereof.

In another aspect, there is provided an isolated nucleic acid molecule selected from the group consisting of (a) a fragment of SEQ ID NO: 681 of sufficient length to represent a sequence unique within the human genome identifying a nucleic acid encoding a human cancer associated antigen precursor; and (b) complements of (a).

The isolated nucleic acid molecule may be selected from the group consisting of (a) a fragment of a nucleic acid selected from the group of nucleic acid molecules consisting of SEQ ID numbered below and comprising all nucleic acid sequences among SEQ ID NOs 1-816, of sufficient length to represent a sequence unique within the human genome, and identifying a nucleic acid encoding a human cancer associated antigen precursor, (b) complements of (a), provided that the fragment includes a sequence of contiguous nucleotides which is not identical to any sequence selected from the sequence group consisting of (1) sequences having the GenBank accession numbers of the sequence Group 1, (2) complements of (1), and (3) fragments of (1) and (2).

In one embodiment the sequence of contiguous nucleotides is selected from the group consisting of: (1) at least two contiguous nucleotides nonidentical to the sequence Group 1, (2) at least three contiguous nucleotides nonidentical to the sequence Group 1, (3) at least four contiguous nucleotides nonidentical to the sequence Group 1, (4) at least five contiguous nucleotides nonidentical to the sequence Group 1, (5) at least six contiguous nucleotides nonidentical to the sequence Group 1, or (6) at least seven contiguous nucleotides nonidentical to the sequence Group 1.

In another embodiment the fragment has a size selected from the group consisting of at least: 8 nucleotides, 10 nucleotides, 12 nucleotides, 14 nucleotides, 16 nucleotides, 18 nucleotides, 20, nucleotides, 22 nucleotides, 24 nucleotides, 26 nucleotides, 28 nucleotides, 30 nucleotides, 50 nucleotides, 75 nucleotides, 100 nucleotides, 200 nucleotides, 1000 nucleotides and every integer length therebetween.

In yet another embodiment the molecule encodes a polypeptide which, or a fragment of which, binds a human HLA receptor or a human antibody or antigen-binding fragment thereof.

In another aspect, there is provided an isolated nucleic acid molecule according to the first aspect of the invention consisting of a nucleic acid molecule having the sequence set out in SEQ ID NO: 681.

Another aspect of the invention is an expression vector comprising an isolated nucleic acid molecule of the invention described above operably linked to a promoter. The vector may comprise a nucleic acid coding for a polypeptide, or a protein thereof, which binds to an MHC molecule to form a complex recognized by an autologous antibody or lymphocyte.

According to one aspect the invention is an expression vector comprising a nucleic acid operably linked to a promoter, wherein the nucleic acid is a NA Group 2 molecule. In another aspect the invention is an expression vector comprising a NA Group 1 or Group 2 molecule and a nucleic acid encoding an MHC, preferably HLA, molecule.

In yet another aspect the invention is a host cell transformed or transfected with an expression vector of the invention described above.

In another aspect the invention is a host cell transformed or transfected with an expression vector comprising an isolated nucleic acid molecule of the invention described above operably linked to a promoter, or an expression vector comprising a nucleic acid operably linked to a promoter, wherein the nucleic acid is a NA Group 1 or 2 molecule and further comprising a nucleic acid encoding HLA.

According to another aspect of the invention an isolated polypeptide encoded by the isolated nucleic acid molecules the invention, described above, is provided. These include PP Group 1-17 polypeptides. The invention also includes a fragment of the polypeptide which is immunogenic. In one embodiment the fragment, or a portion of the fragment, binds HLA or a human antibody, or antigen-binding fragment thereof.

The invention includes in another aspect an isolated fragment of a human cancer associated antigen precursor which, or portion of which, binds HLA or a human antibody, wherein the precursor is encoded by a nucleic acid molecule that is a NA Group 1 molecule. In one embodiment the fragment is part of a complex with HLA. In another embodiment the fragment is between 8 and 12 amino acids in length. In another embodiment the invention includes an isolated polypeptide comprising a fragment of the polypeptide of sufficient length to represent a sequence unique within the human genome and identifying a polypeptide that is a human cancer associated antigen precursor.

According to another aspect of the invention there is provided a kit for detecting the presence of the expression of a human cancer associated antigen precursor comprising: a pair of isolated nucleic acid molecules each of which consists of a molecule selected from the group consisting of (a) a 12-32 nucleotide contiguous segment of the nucleotide sequence of any of the nucleic acid molecule in accordance with the first aspect of the invention; and (b) complements of (a), wherein the contiguous segments are nonoverlapping; and wherein the pair of isolated nucleic acid molecules selectively amplify an isolated nucleic acid molecule in accordance with the first aspect of the invention. The kit includes a pair of isolated nucleic acid molecules each of which consists essentially of a molecule selected from the group consisting of (a) a 12-32 nucleotide contiguous segment of the nucleotide sequence of any of the NA Group 1 molecules and (b) complements of ("a"), wherein the contiguous segments are nonoverlapping. In one embodiment the pair of isolated nucleic acid molecules is constructed and arranged to selectively amplify an isolated nucleic acid molecule that is a NA Group 3 molecule. Preferably, the pair amplifies a human NA Group 3 molecule.

According to another aspect of the invention a method for treating a subject with a disorder characterized by expression of a human cancer associated antigen precursor is provided. The method includes the step of administering to the subject an amount of an agent, which enriches selectively in the subject the presence of complexes of an HLA molecule and a human cancer associated antigen, effective to ameliorate the disorder, wherein the human cancer associated antigen is a fragment of a human cancer associated antigen precursor encoded by a nucleic acid molecule selected from the group consisting of (a) a nucleic acid molecule comprising NA group 1 nucleic acid molecules, (b) a nucleic acid molecule comprising NA group 3 nucleic acid molecules, (c) a nucleic acid molecule comprising NA group 17 nucleic acid molecules.

In one embodiment the disorder is characterized by expression of a plurality of human cancer associated antigen precursors and wherein the agent is a plurality of agents, each of which enriches selectively in the subject the presence of complexes of an HLA molecule and a different human cancer associated antigen. Preferably the plurality is at least 2, at least 3, at least 4, or at least 5 such agents.

In another embodiment the agent is an isolated polypeptide selected from the group consisting of PP Group 1, PP Group 2, PP Group 3, PP Group 4, PP Group 5, PP Group 6, PP Group 7, PP Group 8, PP Group 9, PP Group 10, PP Group 11, PP Group 12, PP Group 13, PP Group 14, PP Group 15, PP Group 16 and PP Group 17 polypeptides.

In yet another embodiment the disorder is cancer.

According to another aspect the invention is a method for treating a subject having a condition characterized by expression of a cancer associated antigen precursor in cells of the subject. The method includes the steps of (I) removing an immunoreactive cell containing sample from the subject, (ii) contacting the immunoreactive cell containing sample to the host cell under conditions favoring production of cytolytic T cells against a human cancer associated antigen which is a fragment of the precursor, (iii) introducing the cytolytic T cells to the subject in an amount effective to lyse cells which express the human cancer associated antigen, wherein the host cell is transformed or transfected with an expression vector comprising an isolated nucleic acid molecule operably linked to a promoter, the isolated nucleic acid molecule being selected from the group of nucleic acid molecules consisting of NA Group 1, NA Group 2, NA Group 3, NA Group 4, NA Group 5, NA Group 6, NA Group 7, NA Group 8, NA Group 9, NA Group 10, NA Group 11, NA Group 12, NA Group 13, NA Group 14, NA Group 15, NA Group 16, and NA Group 17.

In one embodiment the host cell recombinantly expresses an HLA molecule which binds the human cancer associated antigen. In another embodiment the host cell endogenously expresses an HLA molecule which binds the human cancer associated antigen.

The invention includes in another aspect a method for treating a subject having a condition characterized by expression of a cancer associated antigen precursor in cells of the subject. The method includes the steps of (I) identifying a nucleic acid molecule expressed by the cells associated with said condition, wherein said nucleic acid molecule is a NA Group 1 molecule (ii) transfecting a host cell with a nucleic acid selected from the group consisting of (a) the nucleic acid molecule identified, (b) a fragment of the nucleic acid identified which includes a segment coding for a cancer associated antigen, (c) deletions, substitutions or additions to (a) or (b), and (d) degenerates of (a), (b), or (c); (iii) culturing said transfected host cells to express the transfected nucleic acid molecule, and; (iv) introducing an amount of said host cells or an extract thereof to the subject effective to increase an immune response against the cells of the subject associated with the condition. Preferably, the antigen is a human antigen and the subject is a human.

In one embodiment the method also includes the step of (a) identifying an MHC molecule which presents a portion of an expression product of the nucleic acid molecule, wherein the host cell expresses the same MHC molecule as identified in (a) and wherein the host cell presents an MHC binding portion of the expression product of the nucleic acid molecule.

In another embodiment the method also includes the step of treating the host cells to render them non-proliferative.

In yet another embodiment the immune response comprises a B-cell response or a T cell response. Preferably the response is a T-cell response which comprises generation of cytolytic T-cells specific for the host cells presenting the portion of the expression product of the nucleic acid molecule or cells of the subject expressing the human cancer associated antigen.

In another embodiment the nucleic acid molecule is a NA Group 3 molecule.

Another aspect of the invention is a method for treating or diagnosing or monitoring a subject having a condition characterized by expression of an abnormal amount of a protein encoded by a nucleic acid molecule that is a NA Group 1 molecule. The method includes the step of administering to the subject an antibody which specifically binds to the protein or a peptide derived therefrom, the antibody being coupled to a therapeutically useful agent, in an amount effective to treat the condition.

In one embodiment the antibody is a monoclonal antibody. Preferably the monoclonal antibody is a chimeric antibody or a humanized antibody.

In another aspect the invention is a method for treating a condition characterized by expression in a subject of abnormal amounts of a protein encoded by a nucleic acid molecule that is a NA Group 1 nucleic acid molecule. The method involves the step of administering to a subject at least one of the pharmaceutical compositions of the invention described above in an amount effective to prevent, delay the onset of, or inhibit the condition in the subject. In one embodiment the condition is cancer. In another embodiment the method includes the step of first identifying that the subject expresses in a tissue abnormal amounts of the protein. The invention in another aspect is a method for treating a subject having a condition characterized by expression of abnormal amounts of a protein encoded by a nucleic acid molecule that is a NA Group 1 nucleic acid molecule. The method includes the steps of (I) identifying cells from the subject which express abnormal amounts of the protein; (ii) isolating a sample of the cells; (iii) cultivating the cells, and (iv) introducing the cells to the subject in an amount effective to provoke an immune response against the cells.

In one embodiment the cells express a protein selected from the group consisting of a PP Group 11 protein, a PP Group 12 protein, a PP Group 13 protein, PP Group 14 protein, a PP Group 15 protein and a PP Group 16 protein. In another embodiment the method includes the step of rendering the cells non-proliferative, prior to introducing them to the subject.

In another aspect the invention is a method for treating a pathological cell condition characterized by abnormal expression of a protein encoded by a nucleic acid molecule that is a NA Group 1 nucleic acid molecule. The method includes the step of administering to a subject in need thereof an effective amount of an agent which inhibits the expression or activity of the protein.

In one embodiment the agent is an inhibiting antibody which selectively binds to the protein and wherein the antibody is a monoclonal antibody, a chimeric antibody or a humanized antibody. In another embodiment the agent is an antisense nucleic acid molecule which selectively binds to the nucleic acid molecule which encodes the protein. In yet another important embodiment the nucleic acid molecule is a NA Group 3 nucleic acid molecule.

The invention includes in another aspect a composition of matter useful in stimulating an immune response to a plurality of a protein encoded by nucleic acid molecules that are NA Group 1 molecules. The composition is a plurality of peptides derived from the amino acid sequences of the proteins, wherein the peptides bind to one or more MHC molecules presented on the surface of the cells which express an abnormal amount of the protein.

In one embodiment at least a portion of the plurality of peptides bind to MHC molecules and elicit a cytolytic response thereto. In another embodiment the composition of matter includes an adjuvant. In another embodiment the adjuvant is a saponin, GM-CSF, or an interleukin.

According to another aspect the invention, there is provided an isolated antibody or an antigen-binding fragment thereof which selectively binds to a complex of (i) a peptide derived from a protein encoded by a nucleic acid molecule according to the first aspect of the invention, and (ii) an MHC molecule to which binds the peptide to form the complex, wherein the isolated antibody does not bind to (i) or (ii) alone, wherein the antibody is preferably a monoclonal antibody, a chimeric antibody or a humanized antibody.

In one embodiment the antibody is a monoclonal antibody, a chimeric antibody or a humanized antibody.

The invention also involves the use of the genes, gene products, fragments thereof, agents which bind thereto, and so on in the preparation of medicaments. A particular medicament is for treating cancer and a more particular medicament is for treating breast cancer, lung cancer, renal cancer, colon cancer, prostate cancer or gastric cancer.

### Detailed Description of the Invention

In the above summary and in the ensuing description, lists of sequences are provided. The lists are meant to embrace each single sequence separately, two or more sequences together where they form a part of the same gene, any combination of two or more sequences which relate to different genes, including and up to the total number on the list, as if each and every combination were separately and specifically enumerated. Likewise, when mentioning fragment size, it is intended that a range embrace the smallest fragment mentioned to the full-length of the sequence (-1 so that it is a fragment), each and every fragment length intended as if specifically enumerated. Thus, if a fragment could be between 10 and 15 in length, it is explicitly meant to mean 10, 11, 12, 13, 14, or 15 in length.

The summary and the claims mention antigen precursors and antigens. As used in the summary and in the claims, a precursor is substantially the full-length protein encoded by the coding region of the isolated DNA and the antigen is a peptide which complexes with MHC, preferably HLA, and which participates in the immune response as part of that complex. Such antigens are typically 9 amino acids long, although this may vary slightly.

As used herein, a subject is a human, non-human primate, cow, horse, pig, sheep, goat, dog, cat or rodent. In all embodiments human cancer antigens and human subjects are preferred.

The present invention in one aspect involves the cloning of cDNAs encoding human cancer associated antigen precursors using autologous antisera of subjects having cancer. The sequences of the clones representing genes identified according to the methods described herein are presented in the attached Sequence Listing, and the predicted amino acid sequences of some clones also are presented. Of the foregoing, it can be seen that some of the clones are considered completely novel as no nucleotide or amino acid homologies to coding regions were found in the databases searched. Other clones are novel but have some homology to sequences deposited in databases (mainly EST sequences). Nevertheless, the entire gene sequence was not previously known. In some cases no function was suspected and in other cases, even if a function was suspected, it was not know that the gene was associated with cancer. In all cases, it was not known or suspected that the gene encoded a cancer antigen which reacted with antibody from autologous sera. Analysis of the clone sequences by comparison to nucleic acid and protein databases determined that still other of the clones surprisingly are closely related to other previously-cloned genes. The sequences of these related genes is also presented in the Sequence Listing. The nature of the foregoing genes as encoding antigens recognized by the immune systems of cancer patients is, of course, unexpected.

The invention thus involves in one aspect cancer associated antigen polypeptides, genes encoding those polypeptides, functional modifications and variants of the foregoing, useful fragments of the foregoing, as well as diagnostics and therapeutics relating thereto.

Homologs and alleles of the cancer associated antigen nucleic acids of the invention can be identified by conventional techniques. Thus, an aspect of the invention is those nucleic acid sequences which code for cancer associated antigen precursors. Because this application contains so many sequences, the following chart is provided to identify the various groups of sequences discussed in the claims and in the summary:

### "Nucleic Acid Sequences"

NA Group 1.
   (a) nucleic acid molecules which hybridize under stringent conditions to a molecule consisting of a nucleic acid sequence selected from the group consisting of nucleic acid sequences among SEQ ID NOs 1-816 and which code for a cancer associated antigen precursor,
   (b) deletions, additions and substitutions which code for a respective cancer associated antigen precursor,
   (c) nucleic acid molecules that differ from the nucleic acid molecules of (a) or (b) in codon sequence due to the degeneracy of the genetic code, and
   (d) complements of (a), (b) or (c).
NA Group 2. Fragments of NA Group 1, which codes for a polypeptide which, or a portion of which, binds an MHC molecule to form a complex recognized by a an autologous antibody or lymphocyte.
NA Group 3. The subset of NA Group 1 where the nucleotide sequence is selected from the group consisting of:
   (a) previously unknown human nucleic acids coding for a human cancer associated antigen precursor,
   (b) deletions, additions and substitutions which code for a respective human cancer associated antigen precursor,
   (c) nucleic acid molecules that differ from the nucleic acid molecules of (a) or (b) in codon sequence due to the degeneracy of the genetic code, and
   (d) complements of (a), (b) or (c).
NA Group 4. Fragments ofNA Group 3, which code for a polypeptide which, or a portion of which, binds to an MHC molecule to form a complex recognized by an autologous antibody or lymphocyte.
NA Group 5. A subset of NA Group 1, wherein the nucleic acid molecule codes for a human breast cancer associated antigen precursor.
NA Group 6. A subset ofNA Group 1, wherein the nucleic acid molecule codes for a human colon cancer associated antigen precursor.
NA Group 7. A subset ofNA Group 1, wherein the nucleic acid molecule codes for a human gastric cancer associated antigen precursor.
NA Group 8. A subset of NA Group 1, wherein the nucleic acid molecule codes for a human lung cancer associated antigen precursor.
NA Group 9. A subset ofNA Group 1, wherein the nucleic acid molecule codes for a human renal cancer associated antigen precursor.
NA Group 10. A subset of NA Group 1, wherein the nucleic acid molecule codes for a human prostate cancer associated antigen precursor.
NA Group 11. A subset ofNA Group 3, wherein the nucleic acid molecule codes for a human breast cancer associated antigen precursor.
NA Group 12. A subset ofNA Group 3, wherein the nucleic acid molecule codes for a human colon cancer associated antigen precursor.
NA Group 13. A subset ofNA Group 3, wherein the nucleic acid molecule codes for a human gastric cancer associated antigen precursor.
NA Group 14. A subset of NA Group 3, wherein the nucleic acid molecule codes for a human lung cancer associated antigen precursor.
NA Group 15. A subset of NA Group 3, wherein the nucleic acid molecule codes for a human renal cancer associated antigen precursor.
NA Group 16. A subset of NA Group 3, wherein the nucleic acid molecule codes for a human prostate cancer associated antigen precursor.
NA Group 17. A subset of NA Group 1, comprising human cancer associated antigens that react with allogenic cancer antisera.

### Polypeptide Sequences

PP Group 1. Polypeptides encoded by NA Group 1.
PP Group 2. Polypeptides encoded by NA Group 2
PP Group 3. Polypeptides encoded by NA Group 3.
PP Group 4. Polypeptides encoded by NA Group 4.
PP Group 5. Polypeptides encoded by NA Group 5.
PP Group 6. Polypeptides encoded by NA Group 6.
PP Group 7. Polypeptides encoded by NA Group 7.
PP Group 8. Polypeptides encoded by NA Group 8.
PP Group 9. Polypeptides encoded by NA Group 9.
PP Group 10. Polypeptides encoded by NA Group 10.
PP Group 11. Polypeptides encoded by NA Group 11.
PP Group 12. Polypeptides encoded by NA Group 12.
PP Group 13. Polypeptides encoded by NA Group 13.
PP Group 14. Polypeptides encoded by NA Group 14.
PP Group 15. Polypeptides encoded by NA Group 15.
PP Group 16. Polypeptides encoded by NA Group 16.
PP Group 17. Polypeptides encoded by NA Group 17.

The term "stringent conditions" as used herein refers to parameters with which the art is familiar. Nucleic acid hybridization parameters may be found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. More specifically, stringent conditions, as used herein, refers, for example, to hybridization at 65°C in hybridization buffer (3.5 x SSC, 0.02% Ficoll, 0.02% polyvinyl pyrrolidone, 0.02% Bovine Serum Albumin, 2.5mM NaH₂PO₄(pH7), 0.5% SDS, 2mM EDTA). SSC is 0.15M sodium chloride/0.15M sodium citrate, pH7; SDS is sodium dodecyl sulphate; and EDTA is ethylenediaminetetracetic acid. After hybridization, the membrane upon which the DNA is transferred is washed, for example, in 2 x SSC at room temperature and then at 0.1 - 0.5 x SSC/0.1 x SDS at temperatures up to 68°C.

There are other conditions, reagents, and so forth which can be used, which result in a similar degree of stringency. The skilled artisan will be familiar with such conditions, and thus they are not given here. It will be understood, however, that the skilled artisan will be able to manipulate the conditions in a manner to permit the clear identification of homologs and alleles of cancer associated antigen nucleic acids of the invention (e.g., by using lower stringency conditions). The skilled artisan also is familiar with the methodology for screening cells and libraries for expression of such molecules which then are routinely isolated, followed by isolation of the pertinent nucleic acid molecule and sequencing.

In general homologs and alleles typically will share at least 40% nucleotide identity and/or at least 50% amino acid identity to the sequences of breast cancer associated antigen nucleic acid and polypeptides, respectively, in some instances will share at least 50% nucleotide identity and/or at least 65% amino acid identity and in still other instances will share at least 60% nucleotide identity and/or at least 75% amino acid identity. The homology can be calculated using various, publicly available software tools developed by NCBI (Bethesda, Maryland) that can be obtained through the internet (ftp:/ncbi.nlm.nih.gov/pub/). Exemplary tools include the BLAST system available at http://wwww.ncbi.nlm.nih.gov. Pairwise and ClustalW alignments (BLOSUM30 matrix setting) as well as Kyte-Doolittle hydropathic analysis can be obtained using the MacVetor sequence analysis software (Oxford Molecular Group). Watson-Crick complements of the foregoing nucleic acids also are embraced by the invention.

In screening for cancer associated antigen genes, a Southern blot may be performed using the foregoing conditions, together with a radioactive probe. After washing the membrane to which the DNA is finally transferred, the membrane can be placed against X-ray film to detect the radioactive signal. In screening for the expression of cancer associated antigen nucleic acids, Northern blot hybridizations using the foregoing conditions (see also the Examples) can be performed on samples taken from breast cancer patients or subjects suspected of having a condition characterized by expression of breast cancer associated antigen genes. Amplification protocols such as polymerase chain reaction using primers which hybridize to the sequences presented also can be used for detection of the cancer associated antigen genes or expression thereof.

The breast cancer associated genes correspond to SEQ ID NOs. 1-40 and 66. The preferred breast cancer associated antigens for the methods of diagnosis disclosed herein are those set forth in SEQ ID NOs:[31, 33 and 34], which were found to react with allogeneic breast cancer antisera. Encoded polypeptides (e.g., proteins), peptides and antisera thereto are also preferred for diagnosis.

The colon cancer associated genes correspond to SEQ ID Nos. 544-586, even numbers only. The preferred colon cancer associated antigens for the methods of diagnosis disclosed herein are those, which were found to react with allogeneic colon cancer antisera. Encoded polypeptides (e.g., proteins), peptides and antisera thereto are also preferred for diagnosis.

The gastric cancer associated genes correspond to SEQ ID NOs 176-436 and 588-674. The preferred gastric cancer associated antigens for the methods of diagnosis disclosed herein are those, which were found to react with allogeneic gastric cancer antisera. Encoded polypeptides (e.g., proteins), peptides and antisera thereto are also preferred for diagnosis.

The renal cancer associated genes correspond to SEQ ID Nos. 89-169, odd numbers only, and 170, 172, and 174. The preferred renal cancer associated antigens for the methods of diagnosis disclosed herein are those, which were found to react with allogeneic renal cancer antisera. Encoded polypeptides (e.g., proteins), peptides and antisera thereto are also preferred for diagnosis.

The lung cancer associated genes correspond to SEQ ID Nos. 689, 691, 692, 694, 696-707, 709, 711, and 712. The preferred lung cancer associated antigens for the methods of diagnosis disclosed herein are those, which were found to react with allogeneic lung cancer antisera. Encoded polypeptides (e.g., proteins), peptides and antisera thereto are also preferred for diagnosis.

The prostate cancer associated genes correspond to SEQ ID NOs 437-543. The preferred prostate cancer associated antigens for the methods of diagnosis disclosed herein are those, which were found to react with allogeneic prostate cancer antisera. Encoded polypeptides (e.g., proteins), peptides and antisera thereto are also preferred for diagnosis.

The invention also includes degenerate nucleic acids which include alternative codons to those present in the native materials. For example, serine residues are encoded by the codons TCA, AGT, TCC, TCG, TCT and AGC. Each of the six codons is equivalent for the purposes of encoding a serine residue. Thus, it will be apparent to one of ordinary skill in the art that any of the serine-encoding nucleotide triplets may be employed to direct the protein synthesis apparatus, *in vitro* or *in vivo,* to incorporate a serine residue into an elongating breast cancer associated antigen polypeptide. Similarly, nucleotide sequence triplets which encode other amino acid residues include, but are not limited to: CCA, CCC, CCG and CCT (proline codons); CGA, CGC, CGG, CGT, AGA and AGG (arginine codons); ACA, ACC, ACG and ACT (threonine codons); AAC and AAT (asparagine codons); and ATA, ATC and ATT (isoleucine codons). Other amino acid residues may be encoded similarly by multiple nucleotide sequences. Thus, the invention embraces degenerate nucleic acids that differ from the biologically isolated nucleic acids in codon sequence due to the degeneracy of the genetic code.

The invention also provides isolated unique fragments of cancer associated antigen nucleic acid sequences or complements thereof. A unique fragment is one that is a 'signature' for the larger nucleic acid. It, for example, is long enough to assure that its precise sequence is not found in molecules within the human genome outside of the cancer associated antigen nucleic acids defined above (and human alleles). Those of ordinary skill in the art may apply no more than routine procedures to determine if a fragment is unique within the human genome. Unique fragments, however, exclude fragments completely composed of the nucleotide sequences of any of GenBank accession numbers listed in Table 1 or other previously published sequences as of the filing date of the priority documents for sequences listed in a respective priority document or the filing date of this application for sequences listed for the first time in this application which overlap the sequences of the invention.

A fragment which is completely composed of the sequence described in the foregoing GenBank deposits is one which does not include any of the nucleotides unique to the sequences of the invention. Thus, a unique fragment must contain a nucleotide sequence other than the exact sequence of those in GenBank or fragments thereof. The difference may be an addition, deletion or substitution with respect to the GenBank sequence or it may be a sequence wholly separate from the GenBank sequence.

Unique fragments can be used as probes in Southern and Northern blot assays to identify such nucleic acids, or can be used in amplification assays such as those employing PCR. As known to those skilled in the art, large probes such as 200, 250, 300 or more nucleotides are preferred for certain uses such as Southern and Northern blots, while smaller fragments will be preferred for uses such as PCR. Unique fragments also can be used to produce fusion proteins for generating antibodies or determining binding of the polypeptide fragments, or for generating immunoassay components. Likewise, unique fragments can be employed to produce nonfused fragments of the cancer associated antigen polypeptides, useful, for example, in the preparation of antibodies, and in immunoassays. Unique fragments further can be used as antisense molecules to inhibit the expression of cancer associated antigen nucleic acids and polypeptides, particularly for therapeutic purposes as described in greater detail below.

As will be recognized by those skilled in the art, the size of the unique fragment will depend upon its conservancy in the genetic code. Thus, some regions of cancer associated antigen sequences and complements thereof will require longer segments to be unique while others will require only short segments, typically between 12 and 32 nucleotides (e.g. 12, 13, 14, 15, 16, 17, 18, 19, 20, 21, 22, 23, 24, 25, 26, 27, 28, 29, 30, 31 and 32 or more bases long, up to the entire length of the disclosed sequence. As mentioned above, this disclosure intends to embrace each and every fragment of each sequence, beginning at the first nucleotide, the second nucleotide and so on, up to 8 nucleotides short of the end, and ending anywhere from nucleotide number 8, 9, 10 and so on for each sequence, up to the very last nucleotide, (provided the sequence is unique as described above).

Virtually any segment of the polypeptide coding region of novel cancer associated antigen nucleic acids, or complements thereof, that is 18 or more nucleotides in length will be unique. Those skilled in the art are well versed in methods for selecting such sequences, typically on the basis of the ability of the unique fragment to selectively distinguish the sequence of interest from other sequences in the human genome of the fragment to those on known databases typically is all that is necessary, although *in vitro* confirmatory hybridization and sequencing analysis may be performed. Especially preferred include nucleic acids encoding a series of epitopes, known as "polytopes". The epitopes can be arranged in sequential or overlapping fashion (*see, e*.*g*., Thomson et al., Proc. Natl. Acad. Sci. USA 92:5845-5849, 1995; Gilbert et al., Nature Biotechnol. 15:1280-1284, 1997), with or without the natural flanking sequences, and can be separated by unrelated linker sequences if desired. The polytope is processed to generated individual epitopes which are recognized by the immune system for generation of immune responses.

Thus, for example, peptides derived from a polypeptide having an amino acid sequence encoded by one of the nucleic acid disclosed herein, and which are presented by MHC molecules and recognized by CTL or T helper lymphocytes, can be combined with peptides from one or more other cancer associated antigens (e.g. by preparation of hybrid nucleic acids or polypeptides) to form "polytopes". The two or more peptides (or nucleic acids encoding the peptides) can be selected from those described herein, or they can include one or more peptides of previously known cancer associated antigens. Exemplary cancer associated peptide antigens that can be administered to induce or enhance an immune response are derived from tumor associated genes and encoded proteins including MAGE-1, MAGE-2, MAGE-3, MAGE-4, MAGE-5, MAGE-6, MAGE-7, MAGE-8, MAGE-9, MAGE-10, MAGE-11, GAGE-1, GAGE-2, GAGE-3, GAGE-4, GAGE-5, GAGE-6, BAGE-1, RAGE-1, LB33/MUM-1, PRAME, NAG, MAGE-Xp2, MAGE-Xp3, MAGE-Xp4, tyrosinase, brain glycogen phosphorylase, Melan-A, and MAGE-C 1. See, for example, PCT application publication no. WO96/10577. Other examples will be known to one of ordinary skill in the art (for example, see Coulie, Stem Cells 13:393-403, 1995), and can be used in the invention in a like manner as those disclosed herein. One of ordinary skill in the art can prepare polypeptides comprising one or more peptides and one or more of the foregoing cancer associated peptides, or nucleic acids encoding such polypeptides, according to standard procedures of molecular biology.

Thus polytopes are groups of two or more potentially immunogenic or immune response stimulating peptides which can be joined together in various arrangements (e.g. concatenated, overlapping). The polytope (or nucleic acid encoding the polytope) can be administered in a standard immunization protocol, e.g. to animals, to test the effectiveness of the polytope in stimulating, enhancing and/or provoking an immune response.

The peptides can be joined together directly or via the use of flanking sequences to form polytopes, and the use of polytopes as vaccines is well known in the art (see, e.g., Thomson et al., Proc. Acad. Natl. Acad. Sci USA 92(13):5845-5849, 1995; Gilbert et al., Nature Biotechnol. 15(12):1280-1284, 1997; Thomson et al., J. Immunol. 157(2):822-826, 1996; Tam et al., J. Exp. Med. 171(1):299-306, 1990).for example, Tam showed that polytopes consisting of both MHC class I and class II binding epitopes successfully generated antibody and protective immunity in a mouse model. Tam also demonstrated that polytopes comprising "strings" of epitopes are processed to yield individual epitopes which are presented by MHC molecules and recognized by CTLs. Thus polytopes containing various numbers and combinations of epitopes can be prepared and tested for recognition by CTLs and for efficacy in increasing an immune response.

It is known that tumors express a set of tumor antigens, of which only certain subsets may be expressed in the tumor of any given patient (for examples of this, see the Examples below). Polytopes can be prepared which correspond to the different combination of epitopes representing the subset of tumor rejection antigens expressed in a particular patient. Polytopes also can be prepared to reflect a broader spectrum of tumor rejection antigens known to be expressed by a tumor type. Polytopes can be introduced to a patient in need of such treatment as polypeptide structures, or via the use of nucleic acid delivery systems known in the art (see, e.g., Allsopp et al., Eur. J. Immunol. 26(8):1951-1959, 1996). Adenovirus, pox virus, Ty-virus like particles, adeno-associated virus, plasmids, bacteria, etc. can be used in such delivery. One can test the polytope delivery systems in mouse models to determine efficacy of the delivery system. The systems also can be tested in human clinical trials.

In instances in which a human HLA class I molecule presents tumor rejection antigens derived from cancer associated nucleic acids, the expression vector may also include a nucleic acid sequence coding for the HLA molecule that presents any particular tumor rejection antigen derived from these nucleic acids and polypeptides. Alternatively, the nucleic acid sequence coding for such a HLA molecule can be contained within a separate expression vector. In a situation where the vector contains both coding sequences, the single vector can be used to transfect a cell which does not normally express either one. Where the coding sequences for a cancer associated antigen precursor and the HLA molecule which presents it are contained on separate expression vectors, the expression vectors can be cotransfected. The cancer associated antigen precursor coding sequence may be used alone, when, e.g. the host cell already expresses a HLA molecule which presents a cancer associated antigen derived from precursor molecules. Of course, there is no limit on the particular host cell which can be used. As the vectors which contain the two coding sequences may be used in any antigen-presenting cells if desired, and the gene for cancer associated antigen precursor can be used in host cells which do not express a HLA molecule which presents a cancer associated antigen. Further, cell-free transcription systems may be used in lieu of cells.

As mentioned above, the invention embraces antisense oligonucleotides that selectively bind to a nucleic acid molecule encoding a cancer associated antigen polypeptide, to reduce the expression of cancer associated antigens. This is desirable in virtually any medical condition wherein a reduction of expression of cancer associated antigens is desirable, e.g., in the treatment of cancer. This is also useful for *in vitro* or *in vivo* testing of the effects of a reduction of expression of one or more cancer associated antigens.

As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA. The antisense molecules are designed so as to interfere with transcription or translation of a target gene upon hybridization with the target gene or transcript. Those skilled in the art will recognize that the exact length of the antisense oligonucleotide and its degree of complementarity with its target will depend upon the specific target selected, including the sequence of the target and the particular bases which comprise that sequence. It is preferred that the antisense oligonucleotide be constructed and arranged so as to bind selectively with the target under physiological conditions, i.e., to hybridize substantially more to the target sequence than to any other sequence in the target cell under physiological conditions. Based upon the sequences of nucleic acids encoding breast cancer associated antigen, or upon allelic or homologous genomic and/or cDNA sequences, one of skill in the art can easily choose and synthesize any of a number of appropriate antisense molecules for use in accordance with the present invention. In order to be sufficiently selective and potent for inhibition, such antisense oligonucleotides should comprise at least 10 and, more preferably, at least 15 consecutive bases which are complementary to the target, although in certain cases modified oligonucleotides as short as 7 bases in length have been used successfully as antisense oligonucleotides (Wagner et al., Nature Biotechnol. 14:840-844, 1996). Most preferably, the antisense oligonucleotides comprise a complementary sequence of 20-30 bases. Although oligonucleotides may be chosen which are antisense to any region of the gene or mRNA transcripts, in preferred embodiments the antisense oligonucleotides correspond to N-terminal or 5' upstream sites such as translation initiation, transcription initiation or promoter sites. In addition, 3'-untranslated regions may be targeted. Targeting to mRNA splicing sites has also been used in the art but may be less preferred if alternative mRNA splicing occurs. In addition, the antisense is targeted, preferably, to sites in which mRNA secondary structure is not expected (see, e.g., Sainio et al., Cell Mol. Neurobiol. 14(5):439-457, 1994) and at which proteins are not expected to bind. Finally, although the listed sequences are cDNA sequences, one of ordinary skill in the art may easily derive the genomic DNA corresponding to the cDNA of a cancer associated antigen. Thus, the present invention also provides for antisense oligonucleotides which are complementary to the genomic DNA corresponding to nucleic acids encoding breast cancer associated antigens. Similarly, antisense to allelic or homologous cDNAs and genomic DNAs are enabled without undue experimentation.

In one set of embodiments, the antisense oligonucleotides of the invention may be composed of "natural" deoxyribonucleotides, ribonucleotides, or any combination thereof. That is, the 5' end of one native nucleotide and the 3' end of another native nucleotide may be covalently linked, as in natural systems, via a phosphodiester internucleoside linkage. These oligonucleotides may be prepared by art recognized methods which may be carried out manually or by an automated synthesizer. They also may be produced recombinantly by vectors.

In preferred embodiments, however, the antisense oligonucleotides of the invention also may include "modified" oligonucleotides. That is, the oligonucleotides may be modified in a number of ways which do not prevent them from hybridizing to their target but which enhance their stability or targeting or which otherwise enhance their therapeutic effectiveness.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, carboxymethyl esters and peptides.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus modified oligonucleotides may include a 2'-O-alkylated ribose group. In addition, modified oligonucleotides may include sugars such as arabinose instead of ribose. The present invention, thus, contemplates pharmaceutical preparations containing modified antisense molecules that are complementary to and hybridizable with, under physiological conditions, nucleic acids encoding breast cancer associated antigen polypeptides, together with pharmaceutically acceptable carriers.

Antisense oligonucleotides may be administered as part of a pharmaceutical composition. Such a pharmaceutical composition may include the antisense oligonucleotides in combination with any standard physiologically and/or pharmaceutically acceptable carriers which are known in the art. The compositions should be sterile and contain a therapeutically effective amount of the antisense oligonucleotides in a unit of weight or volume suitable for administration to a patient. The term "pharmaceutically acceptable" means a non-toxic material that does not interfere with the effectiveness of the biological activity of the active ingredients. The term "physiologically acceptable" refers to a non-toxic material that is compatible with a biological system such as a cell, cell culture, tissue, or organism. The characteristics of the carrier will depend on the route of administration. Physiologically and pharmaceutically acceptable carriers include diluents, fillers, salts, buffers, stabilizers, solubilizers, and other materials which are well known in the art, as further described below.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., β-galactosidase or alkaline phosphatase), and genes which visibly affect the phenotype of transformed or transfected cells, hosts, colonies or plaques (e.g., green fluorescent protein). Preferred vectors are those capable of autonomous replication and expression of the structural gene products present in the DNA segments to which they are operably joined.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. Especially, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g., Sambrook et al., Molecular Cloning: A Laboratory Manual, Second Edition, Cold Spring Harbor Laboratory Press, 1989. Cells are genetically engineered by the introduction into the cells of heterologous DNA (RNA) encoding a breast cancer associated antigen polypeptide or fragment or variant thereof. That heterologous DNA (RNA) is placed under operable control of transcriptional elements to permit the expression of the heterologous DNA in the host cell.

Preferred systems for mRNA expression in mammalian cells are those such as pRc/CMV (available from Invitrogen, Carlsbad, CA) that contain a selectable marker such as a gene that confers G418 resistance (which facilitates the selection of stably transfected cell lines) and the human cytomegalovirus (CMV) enhancer-promoter sequences. Additionally, suitable for expression in primate or canine cell lines is the pCEP4 vector (Invitrogen), which contains an Epstein Barr Virus (EBV) origin of replication, facilitating the maintenance of plasmid as a multicopy extrachromosomal element. Another expression vector is the pEF-BOS plasmid containing the promoter of polypeptide Elongation Factor 1α, which stimulates efficiently transcription *in vitro.* The plasmid is described by Mishizuma and Nagata (Nuc. Acids Res. 18:5322, 1990), and its use in transfection experiments is disclosed by, for example, Demoulin (Mol. Cell. Biol. 16:4710-4716, 1996). Still another preferred expression vector is an adenovirus, described by Stratford-Perricaudet, which is defective for E1 and E3 proteins (J. Clin. Invest. 90:626-630, 1992). The use of the adenovirus as an Adeno.P1A recombinant for the expression of an antigen is disclosed by Warnier et al., in intradermal injection in mice for immunization against P1A (Int. J. Cancer, 67:303-310, 1996). Additional vectors for delivery of nucleic acid are provided below.

The invention also embraces so-called expression kits, which allow the artisan to prepare a desired expression vector or vectors. Such expression kits include at least separate portions of a vector and one or more of the previously discussed breast cancer associated antigen nucleic acid molecules. Other components may be added, as desired, as long as the previously mentioned nucleic acid molecules, which are required, are included. The invention also includes kits for amplification of a breast cancer associated antigen nucleic acid, including at least one pair of amplification primers which hybridize to a breast cancer associated antigen nucleic acid. The primers preferably are 12-32 nucleotides in length and are non-overlapping to prevent formation of "primer-dimers". One of the primers will hybridize to one strand of the breast cancer associated antigen nucleic acid and the second primer will hybridize to the complementary strand of the breast cancer associated antigen nucleic acid, in an arrangement which permits amplification of the breast cancer associated antigen nucleic acid. Selection of appropriate primer pairs is standard in the art. For example, the selection can be made with assistance of a computer program designed for such a purpose, optionally followed by testing the primers for amplification specificity and efficiency.

The invention also permits the construction of cancer associated antigen gene "knock-outs" in cells and in animals, providing materials for studying certain aspects of cancer and immune system responses to cancer.

The invention also provides isolated polypeptides (including whole proteins and partial proteins) encoded by the foregoing cancer associated antigen nucleic acids. Such polypeptides are useful, for example, alone or as fusion proteins to generate antibodies, as components of an immunoassay or diagnostic assay or as therapeutics. Cancer associated antigen polypeptides can be isolated from biological samples including tissue or cell homogenates, and can also be expressed recombinantly in a variety of prokaryotic and eukaryotic expression systems by constructing an expression vector appropriate to the expression system, introducing the expression vector into the expression system, and isolating the recombinantly expressed protein. Short polypeptides, including antigenic peptides (such as are presented by MHC molecules on the surface of a cell for immune recognition) also can be synthesized chemically using well-established methods of peptide synthesis.

A unique fragment of a cancer associated antigen polypeptide, in general, has the features and characteristics of unique fragments as discussed above in connection with nucleic acids. As will be recognized by those skilled in the art, the size of the unique fragment will depend upon factors such as whether the fragment constitutes a portion of a conserved protein domain. Thus, some regions of breast cancer associated antigens will require longer segments to be unique while others will require only short segments, typically between 5 and 12 amino acids (e.g. 5, 6, 7, 8, 9, 10, 11 or 12 or more, including each integer up to the full length, amino acids long).

Unique fragments of a polypeptide preferably are those fragments which retain a distinct functional capability of the polypeptide. Functional capabilities which can be retained in a unique fragment of a polypeptide include interaction with antibodies, interaction with other polypeptides or fragments thereof, selective binding of nucleic acids or proteins, and enzymatic activity. One important activity is the ability to act as a signature for identifying the polypeptide. Another is the ability to complex with HLA and to provoke in a human an immune response. Those skilled in the art are well versed in methods for selecting unique amino acid sequences, typically on the basis of the ability of the unique fragment to selectively distinguish the sequence of interest from non-family members. A comparison of the sequence of the fragment to those on known databases typically is all that is necessary.

The invention embraces variants of the cancer associated antigen polypeptides described above. As used herein, a "variant" of a cancer associated antigen polypeptide is a polypeptide which contains one or more modifications to the primary amino acid sequence of a cancer associated antigen polypeptide. Modifications which create a cancer associated antigen variant can be made to a cancer associated antigen polypeptide 1) to reduce or eliminate an activity of a cancer associated antigen polypeptide; 2) to enhance a property of a cancer associated antigen polypeptide, such as protein stability in an expression system or the stability of protein-protein binding; 3) to provide a novel activity or property to a cancer associated antigen polypeptide, such as addition of an antigenic epitope or addition of a detectable moiety; or 4) to provide equivalent or better binding to an HLA molecule. Modifications to a cancer associated antigen polypeptide are typically made to the nucleic acid which encodes the cancer associated antigen polypeptide, and can include deletions, point mutations, truncations, amino acid substitutions and additions of amino acids or non-amino acid moieties. Alternatively, modifications can be made directly to the polypeptide, such as by cleavage, addition of a linker molecule, addition of a detectable moiety, such as biotin, addition of a fatty acid, and the like. Modifications also embrace fusion proteins comprising all or part of the cancer associated antigen amino acid sequence. One of skill in the art will be familiar with methods for predicting the effect on protein conformation of a change in protein sequence, and can thus "design" a variant cancer associated antigen polypeptide according to known methods. One example of such a method is described by Dahiyat and Mayo in Science 278:82-87, 1997, whereby proteins can be designed *de novo*. The method can be applied to a known protein to vary a only a portion of the polypeptide sequence. By applying the computational methods of Dahiyat and Mayo, specific variants of a cancer associated antigen polypeptide can be proposed and tested to determine whether the variant retains a desired conformation.

In general, variants include cancer associated antigen polypeptides which are modified specifically to alter a feature of the polypeptide unrelated to its desired physiological activity. For example, cysteine residues can be substituted or deleted to prevent unwanted disulfide linkages. Similarly, certain amino acids can be changed to enhance expression of a breast cancer associated antigen polypeptide by eliminating proteolysis by proteases in an expression system (e.g., dibasic amino acid residues in yeast expression systems in which KEX2 protease activity is present).

Mutations of a nucleic acid which encode a cancer associated antigen polypeptide preferably preserve the amino acid reading frame of the coding sequence, and preferably do not create regions in the nucleic acid which are likely to hybridize to form secondary structures, such a hairpins or loops, which can be deleterious to expression of the variant polypeptide.

Mutations can be made by selecting an amino acid substitution, or by random mutagenesis of a selected site in a nucleic acid which encodes the polypeptide. Variant polypeptides are then expressed and tested for one or more activities to determine which mutation provides a variant polypeptide with the desired properties. Further mutations can be made to variants (or to non-variant cancer associated antigen polypeptides) which are silent as to the amino acid sequence of the polypeptide, but which provide preferred codons for translation in a particular host. The preferred codons for translation of a nucleic acid in, e.g., *E. coli*, are well known to those of ordinary skill in the art. Still other mutations can be made to the noncoding sequences of a cancer associated antigen gene or cDNA clone to enhance expression of the polypeptide. The activity of variants of cancer associated antigen polypeptides can be tested by cloning the gene encoding the variant cancer associated antigen polypeptide into a bacterial or mammalian expression vector, introducing the vector into an appropriate host cell, expressing the variant cancer associated antigen polypeptide, and testing for a functional capability of the cancer associated antigen polypeptides as disclosed herein. For example, the variant cancer associated antigen polypeptide can be tested for reaction with autologous or allogeneic sera as disclosed in the Examples. Preparation of other variant polypeptides may favor testing of other activities, as will be known to one of ordinary skill in the art.

The skilled artisan will also realize that conservative amino acid substitutions may be made in cancer associated antigen polypeptides to provide functionally equivalent variants of the foregoing polypeptides, i.e, the variants retain the functional capabilities of the cancer associated antigen polypeptides. As used herein, a "conservative amino acid substitution" refers to an amino acid substitution which does not alter the relative charge or size characteristics of the protein in which the amino acid substitution is made. Variants can be prepared according to methods for altering polypeptide sequence known to one of ordinary skill in the art such as are found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Exemplary functionally equivalent variants of the cancer associated antigen polypeptides include conservative amino acid substitutions of in the amino acid sequences of SEQ ID proteins disclosed herein. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

For example, upon determining that a peptide derived from a cancer associated antigen polypeptide is presented by an MHC molecule and recognized by CTLs (e.g., as described in the Examples), one can make conservative amino acid substitutions to the amino acid sequence of the peptide, particularly at residues which are thought not to be direct contact points with the MHC molecule. For example, methods for identifying functional variants of HLA class II binding peptides are provided in a published PCT application of Strominger and Wucherpfennig ( WO 96/27387 ). Peptides bearing one or more amino acid substitutions also can be tested for concordance with known HLA/MHC motifs prior to synthesis using, e.g. the computer program described by D'Amaro and Drijfhout (D'Amaro et al., Human Immunol. 43:13-18, 1995; Drijfhout et al., Human Immunol. 43:1-12, 1995). The substituted peptides can then be tested for binding to the MHC molecule and recognition by CTLs when bound to MHC. These variants can be tested for improved stability and are useful, *inter alia*, in vaccine compositions.

Conservative amino-acid substitutions in the amino acid sequence of cancer associated antigen polypeptides to produce functionally equivalent variants of cancer associated antigen polypeptides typically are made by alteration of a nucleic acid encoding a cancer associated antigen polypeptide. Such substitutions can be made by a variety of methods known to one of ordinary skill in the art. For example, amino acid substitutions may be made by PCR-directed mutation, site-directed mutagenesis according to the method of Kunkel (Kunkel, Proc. Nat. Acad. Sci. U.S.A. 82: 488-492, 1985), or by chemical synthesis of a gene encoding a cancer associated antigen polypeptide. Where amino acid substitutions are made to a small unique fragment of a cancer associated antigen polypeptide, such as an antigenic epitope recognized by autologous or allogeneic sera or cytolytic T lymphocytes, the substitutions can be made by directly synthesizing the peptide. The activity of functionally equivalent fragments of cancer associated antigen polypeptides can be tested by cloning the gene encoding the altered cancer associated antigen polypeptide into a bacterial or mammalian expression vector, introducing the vector into an appropriate host cell, expressing the altered cancer associated antigen polypeptide, and testing for a functional capability of the cancer associated antigen polypeptides as disclosed herein. Peptides which are chemically synthesized can be tested directly for function, e.g., for binding to antisera recognizing associated antigens.

The invention as described herein has a number of uses, some of which are described elsewhere herein. First, the invention permits isolation of the cancer associated antigen protein molecules. A variety of methodologies well-known to the skilled practitioner can be utilized to obtain isolated cancer associated antigen molecules. The polypeptide may be purified from cells which naturally produce the polypeptide by chromatographic means or immunological recognition. Alternatively, an expression vector may be introduced into cells to cause production of the polypeptide. In another method, mRNA transcripts may be microinjected or otherwise introduced into cells to cause production of the encoded polypeptide. Translation of mRNA in cell-free extracts such as the reticulocyte lysate system also may be used to produce polypeptide. Those skilled in the art also can readily follow known methods for isolating cancer associated antigen polypeptides. These include, but are not limited to, immunochromatography, HPLC, size-exclusion chromatography, ion-exchange chromatography and immune-affinity chromatography.

The isolation and identification of cancer associated antigen genes also makes it possible for the artisan to diagnose a disorder characterized by expression of cancer associated antigens. These methods involve determining expression of one or more cancer associated antigen nucleic acids, and/or encoded cancer associated antigen polypeptides and/or peptides derived therefrom. In the former situation, such determinations can be carried out via any standard nucleic acid determination assay, including the polymerase chain reaction, or assaying with labeled hybridization probes. In the latter situation, such determinations can be carried out by screening patient antisera for recognition of the polypeptide.

The invention also makes it possible isolate proteins which bind to cancer associated antigens as disclosed herein, including antibodies and cellular binding partners of the cancer associated antigens. Additional uses are described further herein.

The invention also provides, in certain embodiments, "dominant negative" polypeptides derived from cancer associated antigen polypeptides. A dominant negative polypeptide is an inactive variant of a protein, which, by interacting with the cellular machinery, displaces an active protein from its interaction with the cellular machinery or competes with the active protein, thereby reducing the effect of the active protein. For example, a dominant negative receptor which binds a ligand but does not transmit a signal in response to binding of the ligand can reduce the biological effect of expression of the ligand. Likewise, a dominant negative catalytically-inactive kinase which interacts normally with target proteins but does not phosphorylate the target proteins can reduce phosphorylation of the target proteins in response to a cellular signal. Similarly, a dominant negative transcription factor which binds to a promoter site in the control region of a gene but does not increase gene transcription can reduce the effect of a normal transcription factor by occupying promoter binding sites without increasing transcription.

The end result of the expression of a dominant negative polypeptide in a cell is a reduction in function of active proteins. One of ordinary skill in the art can assess the potential for a dominant negative variant of a protein, and using standard mutagenesis techniques to create one or more dominant negative variant polypeptides. For example, given the teachings contained herein of cancer associated antigens, especially those which are similar to known proteins which have known activities, one of ordinary skill in the art can modify the sequence of the cancer associated antigens by site-specific mutagenesis, scanning mutagenesis, partial gene deletion or truncation, and the like. See, e.g., U.S. Patent No. 5,580,723 and Sambrook et al., Molecular Cloning: A Laboratory Manual, Second Edition, Cold Spring Harbor Laboratory Press, 1989. The skilled artisan then can test the population of mutagenized polypeptides for diminution in a selected and/or for retention of such an activity. Other similar methods for creating and testing dominant negative variants of a protein will be apparent to one of ordinary skill in the art.

The invention also involves agents such as polypeptides which bind to cancer associated antigen polypeptides. Such binding agents can be used, for example, in screening assays to detect the presence or absence of cancer associated antigen polypeptides and complexes of cancer associated antigen polypeptides and their binding partners and in purification protocols to isolated cancer associated antigen polypeptides and complexes of cancer associated antigen polypeptides and their binding partners. Such agents also can be used to inhibit the native activity of the cancer associated antigen polypeptides, for example, by binding to such polypeptides.

The invention, therefore, embraces peptide binding agents which, for example, can be antibodies or fragments of antibodies having the ability to selectively bind to cancer associated antigen polypeptides. Antibodies include polyclonal and monoclonal antibodies, prepared according to conventional methodology.

Significantly, as is well-known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. Thus, for example, PCT International Publication Number WO 92/04381 teaches the production and use of humanized murine RSV antibodies in which at least a portion of the murine FR regions have been replaced by FR regions of human origin. Such antibodies, including fragments of intact antibodies with antigen-binding ability, are often referred to as "chimeric" antibodies.

Thus, as will be apparent to one of ordinary skill in the art, the present invention also provides for F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

Thus, the invention involves polypeptides of numerous size and type that bind specifically to cancer associated antigen polypeptides, and complexes of both cancer associated antigen polypeptides and their binding partners. These polypeptides may be derived also from sources other than antibody technology. For example, such polypeptide binding agents can be provided by degenerate peptide libraries which can be readily prepared in solution, in immobilized form or as phage display libraries. Combinatorial libraries also can be synthesized of peptides containing one or more amino acids. Libraries further can be synthesized of peptoids and non-peptide synthetic moieties.

Phage display can be particularly effective in identifying binding peptides useful according to the invention. Briefly, one prepares a phage library (using e.g. m13, fd, or lambda phage), displaying inserts from 4 to about 80 amino acid residues using conventional procedures. The inserts may represent, for example, a completely degenerate or biased array. One then can select phage-bearing inserts which bind to the cancer associated antigen polypeptide. This process can be repeated through several cycles of reselection of phage that bind to the cancer associated antigen polypeptide. Repeated rounds lead to enrichment of phage bearing particular sequences. DNA sequence analysis can be conducted to identify the sequences of the expressed polypeptides. The minimal linear portion of the sequence that binds to the cancer associated antigen polypeptide can be determined. One can repeat the procedure using a biased library containing inserts containing part or all of the minimal linear portion plus one or more additional degenerate residues upstream or downstream thereof. Yeast two-hybrid screening methods also may be used to identify polypeptides that bind to the cancer associated antigen polypeptides. Thus, the cancer associated antigen polypeptides of the invention, or a fragment thereof, can be used to screen peptide libraries, including phage display libraries, to identify and select peptide binding partners of the cancer associated antigen polypeptides of the invention. Such molecules can be used, as described, for screening assays, for purification protocols, for interfering directly with the functioning of cancer associated antigen and for other purposes that will be apparent to those of ordinary skill in the art.

As detailed herein, the foregoing antibodies and other binding molecules may be used for example to identify tissues expressing protein or to purify protein. Antibodies also may be-coupled to specific diagnostic labeling agents for imaging of cells and tissues that express cancer associated antigens or to therapeutically useful agents according to standard coupling procedures. Diagnostic agents include, but are not limited to, barium sulfate, iocetamic acid, iopanoic acid, ipodate calcium, diatrizoate sodium, diatrizoate meglumine, metrizamide, tyropanoate sodium and radiodiagnostics including positron emitters such as fluorine-18 and carbon-11, gamma emitters such as iodine-123, technitium-99m, iodine-131 and indium-111, nuclides for nuclear magnetic resonance such as fluorine and gadolinium. Other diagnostic agents useful in the invention will be apparent to one of ordinary skill in the art. As used herein, "therapeutically useful agents" include any therapeutic molecule which desirably is targeted selectively to a cell expressing one of the cancer antigens disclosed herein, including antineoplastic agents, radioiodinated compounds, toxins, other cytostatic or cytolytic drugs, and so forth. Antineoplastic therapeutics are well known and include: aminoglutethimide, azathioprine, bleomycin sulfate, busulfan, carmustine, chlorambucil, cisplatin, cyclophosphamide, cyclosporine, cytarabidine, dacarbazine, dactinomycin, daunorubicin, doxorubicin, taxol, etoposide, fluorouracil, interferon-α, lomustine, mercaptopurine, methotrexate, mitotane, procarbazine HCl, thioguanine, vinblastine sulfate and vincristine sulfate. Additional antineoplastic agents include those disclosed in Chapter 52, Antineoplastic Agents (Paul Calabresi and Bruce A. Chabner), and the introduction thereto, 1202-1263, of Goodman and Gilman's "The Pharmacological Basis of Therapeutics", Eighth Edition, 1990, McGraw-Hill, Inc. (Health Professions Division). Toxins can be proteins such as, for example, pokeweed anti-viral protein, cholera toxin, pertussis toxin, ricin, gelonin, abrin, diphtheria exotoxin, or *Pseudomonas* exotoxin. Toxin moieties can also be high energy-emitting radionuclides such as cobalt-60.

In the foregoing methods, antibodies prepared according to the invention also preferably are specific for the cancer associated antigen/MHC complexes described herein.

When "disorder" is used herein, it refers to any pathological condition where the cancer associated antigens are expressed. An example of such a disorder is cancer, breast, colon, gastric, renal, prostate and lung cancers as particular examples.

Samples of tissue and/or cells for use in the various methods described herein can be obtained through standard methods such as tissue biopsy, including punch biopsy and cell scraping, and collection of blood or other bodily fluids by aspiration or other methods.

In certain embodiments of the invention, an immunoreactive cell sample is removed from a subject. By "immunoreactive cell" is meant a cell which can mature into an immune cell (such as a B cell, a helper T cell, or a cytolytic T cell) upon appropriate stimulation. Thus immunoreactive cells include CD34⁺ hematopoietic stem cells, immature T cells and immature B cells. When it is desired to produce cytolytic T cells which recognize a cancer associated antigen, the immunoreactive cell is contacted with a cell which expresses a cancer associated antigen under conditions favoring production, differentiation and/or selection of cytolytic T cells; the differentiation of the T cell precursor into a cytolytic T cell upon exposure to antigen is similar to clonal selection of the immune system.

Some therapeutic approaches based upon the disclosure are premised on a response by a subject's immune system, leading to lysis of antigen presenting cells, such as breast cancer cells which present one or more cancer associated antigens. One such approach is the administration of autologous CTLs specific to a cancer associated antigen/MHC complex to a subject with abnormal cells of the phenotype at issue. It is within the ability of one of ordinary skill in the art to develop such CTLs *in vitro.* An example of a method for T cell differentiation is presented in International Application number WO 96/33265 . Generally, a sample of cells taken from a subject, such as blood cells, are contacted with a cell presenting the complex and capable of provoking CTLs to proliferate. The target cell can be a transfectant, such as a COS cell of the type described herein. These transfectants present the desired complex of their surface and, when combined with a CTL of interest, stimulate its proliferation. COS cells, such as those used herein are widely available, as are other suitable host cells. Specific production of a CTL clone is described herein, and is well known in the art. The clonally expanded autologous CTLs then are administered to the subject.

Another method for selecting antigen-specific CTL clones has recently been described (Altman et al., Science 274:94-96, 1996; Dunbar et al., Curr. Biol. 8:413-416, 1998), in which fluorogenic tetramers ofMHC class I molecule/peptide complexes are used to detect specific CTL clones. Briefly, soluble MHC class I molecules are folded *in vitro* in the presence of β₂-microglobulin and a peptide antigen which binds the class I molecule. After purification, the MHC/peptide complex is purified and labeled with biotin. Tetramers are formed by mixing the biotinylated peptide-MHC complex with labeled avidin (e.g. phycoerythrin) at a molar ratio or 4:1. Tetramers are then contacted with a source of CTLs such as peripheral blood or lymph node. The tetramers bind CTLs which recognize the peptide antigen/MHC class I complex. Cells bound by the tetramers can be sorted by fluorescence activated cell sorting to isolate the reactive CTLs. The isolated CTLs then can be expanded *in vitro* for use as described herein.

To detail a therapeutic methodology, referred to as adoptive transfer (Greenberg, J. Immunol. 136(5): 1917, 1986; Riddel et al., Science 257: 238, 1992; Lynch et al, Eur. J. Immunol. 21: 1403-1410,1991; Kast et al., Cell 59: 603-614, 1989), cells presenting the desired complex are combined with CTLs leading to proliferation of the CTLs specific thereto. The proliferated CTLs are then administered to a subject with a cellular abnormality which is characterized by certain of the abnormal cells presenting the particular complex. The CTLs then lyse the abnormal cells, thereby achieving the desired therapeutic goal.

The foregoing therapy assumes that at least some of the subject's abnormal cells present the relevant HLA cancer associated antigen complex. This can be determined very easily, as the art is very familiar with methods for identifying cells which present a particular HLA molecule, as well as how to identify cells expressing DNA of the pertinent sequences, in this case a cancer associated antigen sequence. Once cells presenting the relevant complex are identified via the foregoing screening methodology, they can be combined with a sample from a patient, where the sample contains CTLs. If the complex presenting cells are lysed by the mixed CTL sample, then it can be assumed that a cancer associated antigen is being presented, and the subject is an appropriate candidate for the therapeutic approaches-set forth *supra.*

Adoptive transfer is not the only form of therapy that is available in accordance with the invention. CTLs can also be provoked *in vivo*, using a number of approaches. One approach is the use of non-proliferative cells expressing the complex. The cells used in this approach may be those that normally express the complex, such as irradiated tumor cells or cells transfected with one or both of the genes necessary for presentation of the complex (i.e. the antigenic peptide and the presenting HLA molecule). Chen et al. (Proc. Natl. Acad. Sci. USA 88: 110-114,1991) exemplifies this approach, showing the use of transfected cells expressing HPVE7 peptides in a therapeutic regime. Various cell types may be used. Similarly, vectors carrying one or both of the genes of interest may be used. Viral or bacterial vectors are especially preferred. For example, nucleic acids which encode a breast cancer associated antigen polypeptide or peptide may be operably linked to promoter and enhancer sequences which direct expression of the cancer associated antigen polypeptide or peptide in certain tissues or cell types. The nucleic acid may be incorporated into an expression vector. Expression vectors may be unmodified extrachromosomal nucleic acids, plasmids or viral genomes constructed or modified to enable insertion of exogenous nucleic acids, such as those encoding cancer associated antigen, as described elsewhere herein. Nucleic acids encoding a cancer associated antigen also may be inserted into a retroviral genome, thereby facilitating integration of the nucleic acid into the genome of the target tissue or cell type. In these systems, the gene of interest is carried by a microorganism, e.g., a Vaccinia virus, retrovirus or adenovirus, and the materials de facto "infect" host cells. The cells which result present the complex of interest, and are recognized by autologous CTLs, which then proliferate.

A similar effect can be achieved by combining the cancer associated antigen or a stimulatory fragment thereof with an adjuvant to facilitate incorporation into antigen presenting cells *in vivo.* The breast cancer associated antigen polypeptide is processed to yield the peptide partner of the HLA molecule while a cancer associated antigen peptide may be presented without the need for further processing. Generally, subjects can receive an intradermal injection of an effective amount of the cancer associated antigen. Initial doses can be followed by booster doses, following immunization protocols standard in the art. Preferred cancer associated antigens include those found to react with allogeneic cancer antisera, such as the nucleic acids (and encoded polypeptides and peptides) of SEQ ID NO:31,33 and 34 and others, for example, shown in the examples below.

The invention involves the use of various materials disclosed herein to "immunize" subjects or as "vaccines". As used herein, "immunization" or "vaccination" means increasing or activating an immune response against an antigen. It does not require elimination or eradication of a condition but rather contemplates the clinically favorable enhancement of an immune response toward an antigen. Generally accepted animal models can be used for testing of immunization against breast cancer using a cancer associated antigen nucleic acid. For example, cancer cells can be introduced into a mouse to create a tumor, and one or more cancer associated antigen nucleic acids can be delivered by the methods described herein. The effect on the cancer cells (e.g., reduction of tumor size) can be assessed as a measure of the effectiveness of the cancer associated antigen nucleic acid immunization. Of course, testing of the foregoing animal model using more conventional methods for immunization include the administration of one or more cancer associated antigen polypeptides or peptides derived therefrom, optionally combined with one or more adjuvants and/or cytokines to boost the immune response. Methods for immunization, including formulation of a vaccine composition and selection of doses, route of administration and the schedule of administration (e.g. primary and one or more booster doses), are well known in the art. The tests also can be performed in humans, where the end point is to test for the presence of enhanced levels of circulating CTLs against cells bearing the antigen, to test for levels of circulating antibodies against the antigen, to test for the presence of cells expressing the antigen and so forth.

As part of the immunization compositions, one or more cancer associated antigens or stimulatory fragments thereof are administered with one or more adjuvants to induce an immune response or to increase an immune response. An adjuvant is a substance incorporated into or administered with antigen which potentiates the immune response. Adjuvants may enhance the immunological response by providing a reservoir of antigen (extracellularly or within macrophages), activating macrophages and stimulating specific sets of lymphocytes. Adjuvants of many kinds are well known in the art. Specific examples of adjuvants include monophosphoryl lipid A (MPL, SmithKline Beecham), a congener obtained after purification and acid hydrolysis of *Salmonella minnesota* Re 595 lipopolysaccharide; saponins including QS21 (SmithKline Beecham), a pure QA-21 saponin purified from *Quillja saponaria* extract; DQS21, described in PCT application WO96/33739 (SmithKline Beecham); QS-7, QS-17, QS-18, and QS-L1 (So et al., Mol. Cells 7:178-186, 1997); incomplete Freund's adjuvant; complete Freund's adjuvant; montanide; and various water-in-oil emulsions prepared from biodegradable oils such as squalene and/or tocopherol. Preferably, the peptides are administered mixed with a combination of DQS21/MPL. The ratio of DQS21 to MPL typically will be about 1:10 to 10:1, preferably about 1:5 to 5:1 and more preferably about 1:1. Typically for human administration, DQS21 and MPL will be present in a vaccine formulation in the range of about 1 µg to about 100 µg. Other adjuvants are known in the art and can be used in the invention (*see, e*.*g*. Goding, Monoclonal Antibodies: Principles and Practice, 2nd Ed., 1986). Methods for the preparation of mixtures or emulsions of peptide and adjuvant are well known to those of skill in the art of vaccination.

Other agents which stimulate the immune response of the subject can also be administered to the subject. For example, other cytokines are also useful in vaccination protocols as a result of their lymphocyte regulatory properties. Many other cytokines useful for such purposes will be known to one of ordinary skill in the art, including interleukin-12 (IL-12) which has been shown to enhance the protective effects of vaccines (*see, e*.*g*., Science 268: 1432-1434, 1995), GM-CSF and IL-18. Thus cytokines can be administered in conjunction with antigens and adjuvants to increase the immune response to the antigens.

There are a number of immune response potentiating compounds that can be used in vaccination protocols. These include costimulatory molecules provided in either protein or nucleic acid form. Such costimulatory molecules include the B7-1 and B7-2 (CD80 and CD86 respectively) molecules which are expressed on dendritic cells (DC) and interact with the CD28 molecule expressed on the T cell. This interaction provides costimulation (signal 2) to an antigen/MHC/TCR stimulated (signal 1) T cell, increasing T cell proliferation and effector function. B7 also interacts with CTLA4 (CD152) on T cells and studies involving CTLA4 and B7 ligands indicate that the B7-CTLA4 interaction can enhance antitumor immunity and CTL proliferation, Zheng P., et al. PNAS 95 (11) 6284-6289 (1998).

B7 typically is not expressed on tumor cells so they are not efficient antigen presenting cells (APCs) for T cells. Induction of B7 expression would enable the tumor cells to stimulate more efficiently CTL proliferation and effector function. A combination of B7/IL-6/IL-12 costimulation has been shown to induce IFN-gamma and a Th1 cytokine profile in the T cell population leading to further enhanced T cell activity, Gajewski et al., J. Immunol, 154:5637-5648 (1995). Tumor cell transfection with B7 has ben discussed in relation to *in vitro* CTL expansion for adoptive transfer immunotherapy by Wang et al., J Immunol, 19:1-8 (1986). Other delivery mechanisms for the B7 molecule would include nucleic acid (naked DNA) immunization Kim J., et al. Nat Biotechnol., 15:7:641-646 (1997) and recombinant viruses such as adeno and pox (Wendtner et al., Gene Ther, 4:7:726-735 (1997)). These systems are all amenable to the construction and use of expression cassettes for the coexpression of B7 with other molecules of choice such as the antigens or fragment(s) of antigens discussed herein (including polytopes) or cytokines. These delivery systems can be used for induction of the appropriate molecules *in vitro* and for *in vivo* vaccination situations. The use of anti-CD28 antibodies to directly stimulate T cells *in vitro* and *in vivo* could also be considered.

Lymphocyte function associated antigen-3 (LFA-3) is expressed on APCs and some tumor cells and interacts with CD2 expressed on T cells. This interaction induces T cell IL-2 and IFN-gamma production and can thus complement but not substitute, the B7/CD28 costimulatory interaction, Parra et al., J. Immunol., 158:637-642 (1997), Fenton et al., J. Immunother, 21:2:95-108 (1989).

Lymphocyte function associated antigen-1 (LFA-1) is expressed on leukocytes and interacts with ICAM-1 expressed on APCs and some tumor cells. This interaction induces T cell IL-2 and IFN-gamma production and can thus complement but not substitute, the B7/CD28 costimulatory interaction, Fenton et al., J. Immunothera, 21:2:95-108 (1998). LFA-1 is thus a further example of a costimulatory molecule that could be provided in a vaccination protocol in the various ways discussed above for B7.

Complete CTL activation and effector function requires Th cell help through the interaction between the Th cell CD40L (CD40 ligand) molecule and the CD40 molecule expressed by DCS, Ridge et al., Nature, 393:474 (1998), Bennett et al., Nature, 393:478 (1998), Schoenberger et al., Nature, 393:480 (1998). This mechanism of this costimulatory signal is likely to involve upregulation of B7 and associated IL-6/IL-12 production by the DC (APC). The CD40-CD40L interaction thus complements the signal 1 (antigen/MHC-TCR) and signal 2 (B7-CD28) interactions.

The use of anti-CD40 antibodies to stimulate DC cells directly, would be expected to enhance a response to tumor antigens which are normally encountered outside of a inflammatory context or are presented by non-professional APCs (tumor cells). In these situations Th help and B7 costimulation signals are not provided. This mechanism might be used in the context of antigen pulsed DC based therapies or in situations where Th epitopes have not been defined within known TRA precursors.

A cancer associated antigen polypeptide, or a fragment thereof, also can be used to isolate their native binding partners. Isolation of such binding partners may be performed according to well-known methods. For example, isolated cancer associated antigen polypeptides can be attached to a substrate (e.g., chromatographic media, such as polystyrene beads, or a filter), and then a solution suspected of containing the binding partner may be applied to the substrate. If a binding partner which can interact with cancer associated antigen polypeptides is present in the solution, then it will bind to the substrate-bound cancer associated antigen polypeptide. The binding partner then may be isolated.

It will also be recognized that the invention embraces the use of the cancer associated antigen cDNA sequences in expression vectors, as well as to transfect host cells and cell lines, be these prokaryotic (e.g., *E. coli*), or eukaryotic (e.g., dendritic cells, B cells, CHO cells, COS cells, yeast expression systems and recombinant baculovirus expression in insect cells). Especially useful are mammalian cells such as human, mouse, hamster, pig, goat, primate, etc. They may be of a wide variety of tissue types, and include primary cells and cell lines. Specific examples include keratinocytes, peripheral blood leukocytes, bone marrow stem cells and embryonic stem cells. The expression vectors require that the pertinent sequence, i.e., those nucleic acids described *supra*, be operably linked to a promoter.

The invention also contemplates delivery of nucleic acids, polypeptides or peptides for vaccination. Delivery of polypeptides and peptides can be accomplished according to standard vaccination protocols which are well known in the art. In another embodiment, the delivery of nucleic acid is accomplished by *ex vivo* methods, i.e. by removing a cell from a subject, genetically engineering the cell to include a breast cancer associated antigen, and reintroducing the engineered cell into the subject. One example of such a procedure is outlined in U.S. Patent 5,399,346 and in exhibits submitted in the file history of that patent, all of which are publicly available documents. In general, it involves introduction *in vitro* of a functional copy of a gene into a cell(s) of a subject, and returning the genetically engineered cell(s) to the subject. The functional copy of the gene is under operable control of regulatory elements which permit expression of the gene in the genetically engineered cell(s). Numerous transfection and transduction techniques as well as appropriate expression vectors are well known to those of ordinary skill in the art, some of which are described in PCT application WO95/00654. *In vivo* nucleic acid delivery using vectors such as viruses and targeted liposomes also is contemplated according to the invention.

In preferred embodiments, a virus vector for delivering a nucleic acid encoding a cancer associated antigen is selected from the group consisting of adenoviruses, adeno-associated viruses, poxviruses including vaccinia viruses and attenuated poxviruses, Semliki Forest virus, Venezuelan equine encephalitis virus, retroviruses, Sindbis virus, and Ty virus-like particle. Examples of viruses and virus-like particles which have been used to deliver exogenous nucleic acids include: replication-defective adenoviruses (e.g., Xiang et al., Virology 219:220-227, 1996; Eloit et al., J. Virol 7:5375-5381, 1997; Chengalvala et al., Vaccine 15:335-339, 1997), a modified retrovirus (Townsend et al., J. Virol. 71:3365-3374, 1997), a nonreplicating retrovirus (Irwin et al., J. Virol. 68:5036-5044, 1994), a replication defective Semliki Forest virus (Zhao et al., Proc. Natl. Acad. Sci. USA 92:3009-3013, 1995), canarypox virus and highly attenuated vaccinia virus derivative (Paoletti, Proc. Natl. Acad. Sci. USA 93:11349-11353, 1996), non-replicative vaccinia virus (Moss, Proc. Natl. Acad Sci. USA 93:11341-11348, 1996), replicative vaccinia virus (Moss, Dev. Biol. Stand. 82:55-63, 1994), Venzuelan equine encephalitis virus (Davis et al., J. Virol. 70:3781-3787, 1996), Sindbis virus (Pugachev et al., Virology 212:587-594, 1995), and Ty virus-like particle (Allsopp et al., Eur J. Immunol 26:1951-1959, 1996). In preferred embodiments, the virus vector is an adenovirus.

Another preferred virus for certain applications is the adeno-associated virus, a doublestranded DNA virus. The adeno-associated virus is capable of-infecting a wide range of cell types and species and can be engineered to be replication-deficient. It further has advantages, such as heat and lipid solvent stability, high transduction frequencies in cells of diverse lineages, including hematopoietic cells, and lack of superinfection inhibition thus allowing multiple series of transductions. The adeno-associated virus can integrate into human cellular DNA in a site-specific manner, thereby minimizing the possibility of insertional mutagenesis and variability of inserted gene expression. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

In general, other preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Adenoviruses and retroviruses have been approved for human gene therapy trials. In general, the retroviruses are replication-deficient (i.e., capable of directing synthesis of the desired proteins, but incapable of manufacturing an infectious particle). Such genetically altered retroviral expression vectors have general utility for the high-efficiency transduction of genes *in vivo.* Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, M., "Gene Transfer and Expression, A Laboratory Manual," W.H. Freeman C.O., New York (1990) and Murry, E.J. Ed. "Methods in Molecular Biology," vol. 7, Humana Press, Inc., Cliffton, New Jersey (1991).

Preferably the foregoing nucleic acid delivery vectors: (1) contain exogenous genetic material that can be transcribed and translated in a mammalian cell and that can induce an immune response in a host, and (2) contain on a surface a ligand that selectively binds to a receptor on the surface of a target cell, such as a mammalian cell, and thereby gains entry to the target cell.

Various techniques may be employed for introducing nucleic acids of the invention into cells, depending on whether the nucleic acids are introduced *in vitro* or *in vivo* in a host. Such techniques include transfection of nucleic acid-CaPO₄ precipitates, transfection of nucleic acids associated with DEAE, transfection or infection with the foregoing viruses including the nucleic acid of interest, liposome mediated transfection, and the like. For certain uses, it is preferred to target the nucleic acid to particular cells. In such instances, a vehicle used for delivering a nucleic acid of the invention into a cell (e.g., a retrovirus, or other virus; a liposome) can have a targeting molecule attached thereto. For example, a molecule such as an antibody specific for a surface membrane protein on the target cell or a ligand for a receptor on the target cell can be bound to or incorporated within the nucleic acid delivery vehicle. Preferred antibodies include antibodies which selectively bind a cancer associated antigen, alone or as a complex with a MHC molecule. Especially preferred are monoclonal antibodies. Where liposomes are employed to deliver the nucleic acids of the invention, proteins which bind to a surface membrane protein associated with endocytosis may be incorporated into the liposome formulation for targeting and/or to facilitate uptake. Such proteins include capsid proteins or fragments thereof tropic for a particular cell type, antibodies for proteins which undergo internalization in cycling, proteins that target intracellular localization and enhance intracellular half life, and the like. Polymeric delivery systems also have been used successfully to deliver nucleic acids into cells, as is known by those skilled in the art. Such systems even permit oral delivery of nucleic acids. When administered, the therapeutic compositions of the present invention can be administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents.

The therapeutics of the invention can be administered by any conventional route, including injection or by gradual infusion over time. The administration may, for example, be oral, intravenous, intraperitoneal, intramuscular, intracavity, subcutaneous, or transdermal. When antibodies are used therapeutically, a preferred route of administration is by pulmonary aerosol. Techniques for preparing aerosol delivery systems containing antibodies are well known to those of skill in the art. Generally, such systems should utilize components which will not significantly impair the biological properties of the antibodies, such as the paratope binding capacity (see, for example, Sciarra and Cutie, "Aemsols," in Remington's Pharmaceutical Sciences, 18th edition, 1990, pp 1694-1712). Those of skill in the art can readily determine the various parameters and conditions for producing antibody aerosols without resort to undue experimentation. When using antisense preparations of the invention, slow intravenous administration is preferred.

The compositions of the invention are administered in effective amounts. An "effective amount" is that amount of a cancer associated antigen composition that alone, or together with further doses, produces the desired response, e.g. increases an immune response to the cancer associated antigen. In the case of treating a particular disease or condition characterized by expression of one or more cancer associated antigens, such as cancer, the desired response is inhibiting the progression of the disease. This may involve only slowing the progression of the disease temporarily, although more preferably, it involves halting the progression of the disease permanently. This can be monitored by routine methods or can be monitored according to diagnostic methods of the invention discussed herein. The desired response to treatment of the disease or condition also can be delaying the onset or even preventing the onset of the disease or condition.

Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of breast cancer associated antigen or nucleic acid encoding cancer associated antigen for producing the desired response in a unit of weight or volume suitable for administration to a patient. The response can, for example, be measured by determining the immune response following administration of the cancer associated antigen composition via a reporter system as described herein, by measuring downstream effects such as gene expression, or by measuring the physiological effects of the breast cancer associated antigen composition, such as regression of a tumor or decrease of disease symptoms. Other assays will be known to one of ordinary skill in the art and can be employed for measuring the level of the response.

The doses of cancer associated antigen compositions (e.g., polypeptide, peptide, antibody, cell or nucleic acid) administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

In general, for treatments for eliciting or increasing an immune response, doses of cancer associated antigen are formulated and administered in doses between 1 ng and 1 mg, and preferably between 10 ng and 100 µg, according to any standard procedure in the art. Where nucleic acids encoding cancer associated antigen of variants thereof are employed, doses of between 1 ng and 0.1 mg generally will be formulated and administered according to standard procedures. Other protocols for the administration of cancer associated antigen compositions will be known to one of ordinary skill in the art, in which the dose amount, schedule of injections, sites of injections, mode of administration (e.g., intra-tumoral) and the like vary from the foregoing. Administration of cancer associated antigen compositions to-mammals other than humans, e.g. for testing purposes or veterinary therapeutic purposes, is carried out under substantially the same conditions as described above.

As part of the immunization compositions, the peptide antigens are administered with one or more adjuvants to induce an immune response or to increase an immune response. An adjuvant is a substance incorporated into or administered with antigen which potentiates the immune response. Adjuvants may enhance the immunological response by providing a reservoir of antigen (extracellularly or within macrophages), activating macrophages and stimulating specific sets of lymphocytes. Adjuvants of many kinds are well known in the art. Specific examples of adjuvants include monophosphoryl lipid A (MPL, SmithKline Beecham), a congener obtained after purification and acid hydrolysis of *Salmonella minnesota* Re 595 lipopolysaccharide; saponins including QS21 (SmithKline Beecham), a pure QA-21 saponin purified from *Quillja saponaria* extract; DQS21, described in PCT application WO96/33739 (SmithKline Beecham); QS-7, QS-17, QS-18, and QS-L1 (So et al., Mol. Cells 7:178-186, 1997); incomplete Freund's adjuvant; complete Freund's adjuvant; montanide; and various water-in-oil emulsions prepared from biodegradable oils such as squalene and/or tocopherol. Other adjuvants are known in the art and can be used in the invention (*see*, *e.g.* Goding, Monoclonal Antibodies: Principles and Practice, 2nd Ed., 1986). Methods for the preparation of mixtures or emulsions of peptide and adjuvant are well known to those of skill in the art of vaccination.

Where cancer associated antigen peptides are used for vaccination, modes of administration which effectively deliver the cancer associated antigen and adjuvant, such that an immune response to the antigen is increased, can be used. For administration of a cancer associated antigen peptide in adjuvant, preferred methods include intradermal, intravenous, intramuscular and subcutaneous administration. Although these are preferred embodiments, the invention is not limited by the particular modes of administration disclosed herein. Standard references in the art (e.g., Remington's Pharmaceutical Sciences, 18th edition, 1990) provide modes of administration and formulations for delivery of immunogens with adjuvant or in a non-adjuvant carrier.

When administered, the pharmaceutical preparations of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

A breast cancer associated antigen composition may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction which would substantially impair the desired pharmaceutical efficacy.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration conveniently comprise a sterile aqueous or non-aqueous preparation of breast cancer associated antigen polypeptides or nucleic acids, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in *Remington's Pharmaceutical Sciences,* Mack Publishing Co., Easton, PA.

### Examples

### Example 1: Preparation of breast cancer cDNA expression libraries

Step 1: Purification of total RNA from tumors.
   Total RNA was isolated from tumor samples using the guanidium thiocyanate-phenol-chloroform extraction protocol described by Chomczynski and Sacci (Anal. Biochem. 162:156-159, 1987).
Step 2: Purification of mRNA.
   A Dynabeads mRNA isolation kit (Dynal, Cat.No. 610.01) was used to isolate mRNA from the pool of total RNA isolated in step 1 above according to the manufacturer's instructions.
Step 3: cDNA synthesis.
   cDNA synthesis was performed using a ZAP-cDNA synthesis Kit (Stratagene, La Jolla CA; Cat. No. 200400) according to the manufacturer's protocol. A specific linker-primer which contains a XbaI cloning site was designed and used in this protocol, to facilitate subcloning into TriplEx vector. The sequence of the primer was:
   GAGAGAGAGAGAGAGAGAGAAGTCGACTCTAGATTTTTTTTTTTTTTTTT-Xba 1 site
Step 4: Ligation into the TriplEx vector arms.
   The cDNAs generated in step 3 above were ligated into TriplEx vector arms (Clontech, Palo Alto, CA; Cat. No. 6162-1); the arms were predigested with EcoR I/Xba I.
Step 5: Packaging into phages with Gigapack III kit.
   The ligation mix (TriplEx/cDNA) from step 4 was packed into phages using the Gigapack III Gold Cloning Kit (Stratagene, Cat. N.200450) according to the protocol supplied with the kit.
Step 6: Titering and amplification of generated libraries was performed accoding to the Stratagene protocols.

The foregoing protocol was used to prepare several libraries from tumor sample of different patients. Some libraries were prepared using the UNI-ZAP XR vector system (Stratagene) according to the manufacturer's protocol, and some using the TriplEx system as described above.

**Table 2**

| UNI-ZAP Libraries | | |
|---|---|---|
| Code for tumors | Titer of the library | Histopathological diagnosis |
| HBR173 | 1.8 x 10⁶ pfu | Ductal Carcinoma, Grade III |
| HBR184 | 3.5 x 10⁶ pfu | Invasive Ductal Carcinoma,Grade II |

| TriplEx libraries | | |
|---|---|---|
| Code for tumors | Titer of the library | Histopoathological diagnosis |
| HBR173 | 2.3 x 10⁶ pfu | Ductal Carcinoma, Grade III |
| HBR184 | 1.1 x 10⁶ pfu | Ivasive Ductal Carcinoma, Grade II |
| HBR257 | 2.5 x 10⁶ pfu | Invasive Ductal Carcinoma, Grade II |
| HBR297 | 4.0 x 10⁶ pfu | Ductal Carcinoma, Grade II |
| HBR248 | 1.0 x 10⁶ pfu | Invasive Ductal Carcinoma with Vascular Permeation,Grade III |
| HBR271 | 2.5 x 10⁶ pfu | Medullary Carcimoma |
| HBR263 | 10.0 x 10⁶ pfu | Inv. Pleiomorphic Lobular Carcinoma, Grade II |

All libraries were screened with the exception of HBR173 (no autologous serum). No serum-positive clones were found by screening HBR271 library.

### Example 2: Immunoscreening

Sera was obtained from donors undergoing routine diagnostic and therapeutic procedures. It was stored at - 70°C prior to absorption. Sera, at a dilution of 1:10 in Tris buffered saline (TBS, pH 7.5), was sequentially passed through Sepharose 4B columns which had been coupled to lysates from *E. coli* Y1090 and bacteriophage infected *E. coli* BNN97 (5 Prime 3 Prime, Inc. Boulder, Co.). Final serum dilutions were prepared in 0.2% non-fat dried milk/TBS (NFDM) and stored at 4°C. Library screening was performed as described by Sahin et al.(Proc. Natl. Acad. Sci. USA 92:11810-11813, 1995) with following modifications. Recombinant phage at a concentration of 4 x 10³ per 15 cm plate were amplified for 6 hours and transferred to nitrocellulose membranes for an additional 15 hours at 37°C. Membranes were then blocked with 5% NFDM. As an alternative to generation of IgG subtracted libraries, membranes were pre-screened in a 1:2000 dilution of peroxidase conjugated, Fc fragment specific, goat anti-human IgG (Jackson Immunoresearch Laboratories Inc., West Grove, PA) for 1 hour at room temperature. Color was developed with 3,3' diaminobenzidine tetrahydrochloride and IgG encoding clones were scored. Membranes were then incubated in a 1:100 dilution of absorbed autologous sera for 15 hours at room temperature. Following serum exposure, filters were incubated in a 1:3000 dilution of alkaline phosphatase conjugated, Fc fragment specific, goat anti-human IgG (Jackson Immunoresearch Laboratories Inc.) for 1 hour at room temperature and processed for 4-nitro blue tetrazolium chloride/5-bromo-4-chloro- 3-indolyl-phosphate color development. Serum positive clones were subcloned and retested for serum reactivity as above except nitrocellulose transfer was decreased to 3 hours. For the determination of allogeneic serum reactivity, plates containing an equal number of serum positive clones and negative control plaques were similarly processed less the IgG prescreening steps. A minimum of 5 x 10⁵ recombinants were screened per cDNA library, a number which approximates a point at which the likelihood of repeat isolations of previously identified clones outweigh the prospect of identifying new clones.

### Example 3: DNA Sequencing

Phage cDNA clones were converted to pBKCMV phagemid forms by in vivo excision. Plasmid DNA was purified on Qiaprep spin columns (Qiagen Inc. Chatsworth, CA) and subjected to EcoRI/XbaI restriction enzyme digestion. Clones representing different cDNA inserts were sequenced at Cornell University DNA services (Ithaca, NY) using an ABI Prism (Perkin Elmer) automated DNA sequencer. The sequences of the clones were compared with sequences in GenBank and HGI databases to detect homologous nucleic acid and/or protein sequences. The following table lists exemplary related sequences.

**Table 3: Sequences Related to Breast Cancer Associated Antigen Clones**

| Clone | Nucleotide Homology | Clone | Nucleotide Homology | Clone | Nucleotide Homology |
|---|---|---|---|---|---|
| LONY-Br-1 | L34543 | LONY-Br-23 | AA262134, U74628 | LONY-Br-44 | D15057 |
| LONY-Br-2 | S75417 | LONY-Br-24 | AA282633 | LONY-Br-45 | AB000815 |
| LONY-Br-3 | J05211 | LONY-Br-25 | M62324 | LONY-Br-46 | L04733 |
| LONY-Br-4 | X15187 | LONY-Br-26 | M99389 | LONY-Br-47 | X88791 |
| LONY-Br-5 | X62083 | LONY-Br-27 | X79389 | LONY-Br-48 | AF000430 |
| LONY-Br-6 | J04965 | LONY-Br-28 | D44466 | LONY-Br-49 | none |
| LONY-Br-7 | D63784 | LONY-Br-29 | M33197 | LONY-Br-50 | AA226732 |
| LONY-Br-8 | U11292 | LONY-Br-30 | M17886 | LONY-Br-51 | AA046574 |
| LONY-Br-9 | HSB06D102 | LONY-Br-31 | L38941 | LONY-Br-52 | none |
| LONY-Br-10 | none | LONY-Br-32 | X17644 | LONY-Br-53 | AB002307 |
| LONY-Br-11 | none | LONY-Br-33 | X75342 | 92 | AA127328 |
| LONY-Br-12 | AA430998 | LONY-Br-33 | X75342 | 101 | AA167314 |
| LONY-Br-13 | D83032 | LONY-Br-34 | U43368 | 102 | AA508139 |
| LONY-Br-14 | AA034417 | LONY-Br-35 | X15882 | 107 | none |
| LONY-Br-15 | AA167070 | LONY-Br-37 | AA121558 | 109 | AA220229 |
| LONY-Br-16 | none | LONY-Br-38 | AA211771 | 110 | W67775 |
| LONY-Br-17 | AA161103 | LONY-Br-39 | AA367417 | 111 | AA280070 |
| LONY-Br-19 | R13835 | LONY-Br-40 | AA188052 | 112 | AF004292 |
| LONY-Br-20 | HUMORF003 | LONY-Br-41 | THC83518 | 131 | none |
| LONY-Br-21 | S74572 | LONY-Br-42 | none | 143 | AA481578 |
| LONY-Br-22 | AA070233 | LONY-Br-43 | HU35246 | 162 | AA481578 |

### Example 4: Reverse transcriptase (RT) PCR and Rapid Amplification of cDNA Ends (RACE)

The mRNA expression pattern of selected cDNA clones was determined by RT- PCR using a panel of normal tissue RNA. This test panel consisted of lung, testis, small intestine, colon, breast, liver, and placenta, and was purchased from Clontech Laboratories Inc. (Palo Alto, CA). Colon tumor RNA was also included in this panel and was prepared as described above. As a control for genomic DNA contamination, all cDNA synthesis reactions were set up in duplicate with the additional sample lacking reverse transcriptase. Gene specific PCR primers were designed to amplify 5' fragments of 300-400 bp and were purchased commercially (Gibco BRL, Grand Island, NY). PCR reactions were undertaken at an annealing-temperature of 68°C using a Perkin Elmer thermal cycler. In certain cases, RT-PCR products were subcloned into the pCR2.1 plasmid vector (Invitrogen) and multiple clones were subjected to DNA sequencing as described. 5' and 3' RACE reactions were undertaken using gene specific and adapter primers in conjunction with Marathon Ready normal colon cDNA and KlenTaq polymerase (Clontech) as per manufacturers protocol. Products were then subcloned into the pCR2.1 plasmid vector (Invitrogen) and screened by PCR with internal primers for presence of the desired insert. Multiple RACE clones were subjected to DNA sequencing as described.

### Example 5: Northern blot analysis

Northern blots containing the transfer yields of 2 µg poly A⁺ RNA from a panel of normal tissues were obtained commercially (Clontech). Random primed ³²P labeled probes consisting of 300-600 bp PCR products from 5 prime coding sequences of serum positive cDNA clones were hybridized for 1.5 hours in Expresshyb (Clontech) at 68°C and washed at high stringency (2 times, 30 min. each, 0.1X SSCP/0.1% SDS at 68°C). Resultant blots were used to expose Biomax MS autoradiography film (Eastman Kodak Co., Rochester, NY).

**Table 4: Breast Cancer Associated Antigen Clone mRNA sizes**

| Clone | Size(kb) | Clone | Size (kb) | Clone | Size (kb) |
|---|---|---|---|---|---|
| LONY-Br-1 | 1.8 | LONY-Br-17 | 1.0 | LONY-Br-33 | 2.6 |
| LONY-Br-2 | 2.9 | LONY-Br-19 | 1.5 | LONY-Br-34 | 2.1 |
| LONY-Br-3 | 4.8 | LONY-Br-20 | 2.4 | LONY-Br-35 | 1.9 |
| LONY-Br-4 | 1.2 | L ONY-Br-21 | 2.4 | LONY-Br-36 | 0.8 |
| LONY-Br-5 | 0.9 | LONY-Br-22 | 1.6 | LONY-Br-37 | 1.0 |
| LONY-Br-6 | 1.4 | LONY-Br-23 | 1.3 | LONY-Br-38 | 2.2 |
| LONY-Br-7 | 1.3 | LONY-Br-24 | 3.9 | LONY-Br-39 | 1.9 |
| LONY-Br-8 | 0.9 | LONY-Br-25 | 1.9 | LONY-Br-40 | 3.4 |
| LONY-Br-9 | 6.0 | LONY-Br-26 | 1.5 | LONY-Br-41 | 3.9 |
| LONY-Br-10 | 3.6 | LONY-Br-27 | 1.2 | LONY-Br-42 | 0.6 |
| LONY-Br-11 | 4.6 | LONY-Br-28 | 0.5 | LONY-Br-43 | 1.4 |
| LONY-Br-12 | 2.2 | LONY-Br-29 | 0.6 | LONY-Br-44 | 0.7 |
| LONY-Br-13 | 1.2 | LONY-Br-30 | 0.8 | LONY Br-45 | 3.0 |
| LONY-Br-14 | 0.8 | LONY-Br-31 | 0.4 | LONY-Br-46 | 3.7 |
| LONY-Br-15 | 0.9 | LONY-Br-32 | 22 | LONY-Br-47 | 0.5 |
| LONY-Br=16 | 2.5 | LONY-Br-33 | 2.6 | LONY-Br-48 | 1.6 |

### Example 6: Isolation of gastric and prostate clones

A stomach cancer cDNA library was established, using standard techniques, then the library, was screened, using the SEREX methodology described supra, and set forth by Sahin et al., Proc. Natl. Acad Sci. USA 92: 11810 (1995), and by Chen et al., Proc. Natl. Acad Sci. USA 94: 1914 (1997).

To be specific, total RNA was isolated by homogenizing tumor samples in 4M guanidium thiocyanate/0.5% sodium N-lauryl sarcosine/ and 25 mM EDTA followed by centrifugation in 5.7 M CsC1/25 mM sodium acetate/10 uM EDTA at 320,000 rpm. Total mRNA was removed by passing the sample over an oligo-dT cellulose column. The cDNA libraries were then constructed by taking 5 ug of mRNA, using standard methodologies to reverse transcribe the material.

Libraries were prepared from four different stomach cancer patients, referred to as "SM", "CK" and "SS" and "KM" respectively. A total of 2.5x10⁶, 1.1x10⁶, and 1.7x10⁶ cDNA clones were obtained from the "SM", "CK" and "SS" individuals. Additional libraries were prepared from prostate cancer patient "OT".

The cDNA was used to construct a lambda phage library, and 500 phages were plated onto XL1-Blue MRF E. coli, and incubated for eight hours at 37°C. A nitrocellulose membrane was then placed on the plate, followed by overnight incubation. The membrane was then washed, four times, without TBS which contained 0.05% Tween, and was then immersed in TBS containing 5% non-fat dried milk. After one hour, the membrane was incubated with conjugates of peroxidase-goat anti human IgG specific for Fc portions of huma antibody (1:2000, diluted in TBS with 1% BSA. The incubation was carried out for one hour, at room temperature, and the membrane was then washed three times with TBS. Those clones which produced antibodies were visualized with 0.06%, 3,3'diamino benzidine tetrachloride, and 0.015% H₂O₂, in 50 mM Tris (pH 7.5). Any clones which produced immunoglobulin were marked, and then the membrane was washed, two further times, with TBS that contained 0.05% Tween, and then twice with "neat" TBS.

The membranes were then incubated in 1:100 diluted patient serum, overnight, at 4°C. The patient serum had been pretreated. Specifically, 5 ml samples were diluted to 10 ml with TBS containing 1% bovine serum albumin, and 0.02% Na₃N. The serum had been treated to remove antibodies to bacteriophage, by passing it through a 5 ml Sepharose column, to which a lysate ofE. coli Y1090 had been attached, followed by passage over a second column which had E. coli lysate and lysate of E. coli infected with lambda bacteriophage. The screening was carried out five time. The samples were then diluted to 50 ml, and kept at -80°C, until used as described herein.

Following the overnight incubation with the membrane, the membrane was washed twice with TBS/0.05% Tween 20, and then once with TBS. A further incubation was carried out, using the protocols discussed supra, for the POD labelled antibodies.

The positive clones were then sequenced, using standard techniques. Following comparison of the sequences to information available in data banks, a total of 36 clones were resolved into known and unknown genes. In the table that follows, the "+" and "-" signs are essentially used to compare signals to each other. All were positive.

Previously identified human proteins and the nucleotide sequences, set forth in SEQ ID NOS:588-626 are known. The four molecules which follow in SEQ ID NOs:627-634 (gelsolin, zinc finger protein family, variant zinc finger motif protein goliath and homeodomain proteins), have not been identified in humans previously, although there are related molecules found in other species. Finally, the last four moieties, i.e., prepro-α collagen, heterogeneous ribonucleoprotein D, nucleosome assembly protein 2, and NY-ESO-2/Ulsn NRP/V 1 small nuclear ribonucleoprotein, are also known. Nucleotide sequences are set forth at SEQ ID NOS:635-642. The nucleic acid molecules having the nucleotide sequences set forth at SEQ ID NOS:643-670 represent molecules for which no related sequences were found. SEQ ID NO:671 combines the sequences of SEQ ID NOS:627-630, inclusive. SEQ ID NO:672 combines SEQ ID NOS:643-656, SEQ ID NO:673 combines SEQ ID NOS:657, 659 and 662, while SEQ ID NO:674 combines SEQ ID NOS: 658, 660, 661 and 663.

SEREX analysis of clones from libraries derived from patients "CK," "SS", "KM" (all gastric cancer) and patient "OT" (prostate cancer) was carried out as described above. The nucleotide sequences of clones derived from gastric cancer patients are presented as SEQ ID NOs: 176-436. The nucleotide sequences of clones derived from prostate cancer patient "OT" are presented as SEQ ID Nos:437-543.

### Example 7:Isolation and analysis of colon clones

Colon tumor samples were obtained as surgical samples, and were frozen at -80°C until ready for use.

Total RNA was then isolated from the samples, using the guanidium thiocyanate method of Chirgwin, et al., Biochemistry 18: 5294-5299 (1979). The total RNA thus obtained was then purified to isolate all poly A⁺ RNA, using commercially available products designed for this purpose.

The poly A⁺ RNA was then converted into cDNA, and ligated into λZAP, a commercially available expression vector, according to the manufacturer's suggested protocol.

Three cDNA libraries were constructed in this way, using colorectal carcinoma samples.

A fourth library, also from colorectal carcinoma, was prepared, albeit in a different way. The fourth library was an IgG subtraction library, prepared by using a subtraction partner, generated by PCR amplification of a cDNA clone which encoded an IgG molecule. *See*, *e.g.,* Ace et al, Endocrinology 134: 1305-1309 (1994). IgG subtraction is done to eliminate any false, positive signals resulting from interaction of cDNA clones which encode IgG, with the IgG then interacting with the anti-human IgG used in the SEREX assay, as described herein. PCR products were biotinylated, and hybridized with denatured second strand cDNA, at 68 °C for 18 hours. Biotinylated hybrid molecules were coupled to streptavidin, and then removed by phenol chloroform extraction. Any remaining cDNA was also ligated into λZAP. All libraries were amplified, prior to immunoscreening.

Immunoscreening was carried out using sera obtained from patients undergoing routine diagnostic and therapeutic procedures. The sera were stored at -70 °C prior to use. Upon thawing, the sera were diluted at 1:10 in Tris buffered saline (pH 7.5), and were then passed through Sepharose 4B columns. First, the sera were passed through columns which had E. coli Y1090 lysates coupled thereto, and then lysates from bacteriophage infected E. coli BNN97 lysates. Final serum dilutions were then prepared in 0.2% non-fat dried milk/Tris buffered saline.

The method of Sahin et al., Proc. Natl. Acad. Sci USA 92:11810-11813 (1995), and U.S. Patent No. 5,698,396, was used, with some modifications. Specifically, recombinant phages at a concentration of 4x10³ phages per 15 cm plate (pfus), were amplified for six hours, after which they were transferred to nitrocellulose membranes for 15 hours. The membranes then were blocked with 5% nonfat dried milk.

As an alternative to the IgG subtraction procedure discussed above, membranes were prescreened in a 1:2000 dilution of peroxidase conjugated, Fc fragment specific goat anti-human IgG, for one hour, at room temperature. Color was developed using 3,3'-diaminobenzidine tetrahydrochloride, which permitted scoring of IgG encoding clones.

Membranes were then incubated in 1:100 dilutions of autologous sera, which had been pretreated with the Sepharose 4B columns, as described supra. The filters were then incubated, in a 1:3000 dilution of alkaline phosphatase conjugated Fc fragment specific, goat anti-human IgG, for one hour, at room temperature. The indicator system 4-nitroblue tetrazolium chloride/5-bromo-4-chloro-3-indolyl-phosphate was then added, and color development assessed. Any positive clones were subcloned, and retested, except the time on the nitrocellulose membrane was reduced to three hours.

Positive clones were isolated and sequenced according to standard procedures. The nucleotide sequences of the clones are set forth in the even numbered sequences from SEQ ID Nows:544-586. The odd numbered sequences from SEQ ID Nos:545-587 represent the translated amino acid sequences of the colon nucleic acid clones. Analysis of probes for SEQ ID NOS:544 and 546 confirmed their universal expression.

The foregoing results reflect SEREX isolation of colon cancer clones using autologous serum. The positive clones were then rescreened, using allogeneic serum, following the same method discussed supra, in example 2, except IgG prescreening was omitted. The allogeneic sera was obtained from sixteen normal blood donors, and twenty nine patients who had been diagnosed with colorectal cancer.

The analysis with the two types of serum revealed that fourteen reacted with a subset of sera from normal and cancer patients, twenty-eight only with autologous sera, and six with both allogeneic and autologous sera. Over 60% of the allogeneic serum samples tested reacted with at least one of these positive clones. About 20% reacted with two or more.

In view of the results described above, further experiments were carried out using serum samples from patients with other forms of cancer, i.e., renal cancer (13 samples), lung cancer (23 samples), and breast cancer (10 samples). The results are set forth in Table 6 which follows:

**Table 6: Allogeneic serotyping using colon cancer clones**

| Clone Number | Normal Sera | Colon Cancer | Renal Cancer | Lung Cancer | Breast Cancer |
|---|---|---|---|---|---|
| NY-Co-8 | 0/16 | 8/29 | 1/13 | 0/23 | 0/10 |
| NY-Co-9 | 0/16 | 5/29 | 1/13 | 1/23 | 0/10 |
| NY-Co-13 | 0/16 | 5/29 | 0/13 | 0/23 | 0/10 |
| NY-Co-16 | 0/16 | 3/29 | 0/13 | 0/23 | 0/10 |
| NY-Co-20 | 0/16 | 4/29 | 0/13 | 0/23 | 0/10 |
| NY-Co-38 | 0/16 | 4/29 | 3/13 | 0/23 | 1/10 |

Of the six clones which were identified as being reactive with autologous and allogeneic cancer serum, and not with normal serum, two were found to be identical to previously identified molecules (NY-Co-. Four others were found to have little or no homology to known sequences and thus are preferred allogeneic-reactive colon cancer clones. These nucleic acids and their polypeptide translations are presented as SEQ ID NOS: 544-551: SEQ ID NO: 544/545 (NY-CO-8), SEQ ID NO: 546/547 (NY-CO-9), SEQ ID NO: 548/549 (NY-CO-16) and SEQ ID NO: 550/551 (NY-CO-38).. Of twenty seven allogeneic colon cancer serum samples tested, 67% reacted with at least one of these antigens.

The expression pattern of mRNA corresponding to SEQ ID NOS:544, 546 and 550, as well as other sequences identified via the preceding examples was determined. To do this, RT-PCR was carried out on a panel of RNA samples, taken from normal tissue. The panel contained RNA of lung, testis, small intestine, colon, breast, liver and placenta tissues. The RNA was purchased from a commercial source. RNA from a colon tumor sample was also included. All samples were set up for duplicate runs, so that genomic DNA contamination could be accounted for. In the controls, no reverse transcriptase was used.

Primers were designed which were specific for the cDNA, which would amplify 5'-fragments, from 300-400 base pairs in length. The PCR reactions were undertaken at an annealing temperature of 68°C. Where appropriate, 5' and 3'-RACE reactions were undertaken, using gene specific primers, and adapter primers, together with commercially available reagents. Specifically, SEQ ID NOS: 546 and 550 were tested using RACE. The resulting products were subcloned into vector pCR 2.1, screened via PCR using internal primers, and then sequenced.

SEQ ID NOS:544 and 546 were found to be amplified in all tissues tested. SEQ ID NO:550 was found in colon tumor, colon metastasis, gastric cancer, renal cancer and colon cancer cell lines Colo 204 and HT29, as well as in normal colon, small intestine, brain, stomach, testis, pancreas, liver, lung, heart, fetal brain, mammary gland, bladder, adrenal gland tissues. It is was not found in normal uterine, skeletal muscle, peripheral blood lymphocytes, placental, spleen thymus, or esophagus tissue, nor in lung cancer.

The analysis also identified differential expression of a splice variant of SEQ ID NO:550, i.e., SEQ ID N0:552. When the two sequences were compared, it was found that SEQ ID NO:550 encodes a putative protein of 652 amino acids (SEQ ID NO:551), and molecular weight of 73,337 daltons. SEQ ID NO:552, in contrast, lacks an internal 74 base pairs, corresponding to nucleotides 1307-1380 of SEQ ID NO:550. The deletion results in formation of a stop codon at the splice function, and a putative protein of 403 amino acids (SEQ ID NO:553), and molecular weight 45,839. The missing segment results in the putative protein lacking a PEST protein degradation sequence, thereby suggesting a longer half life for this protein.

In additional experiments, primers designed not to differentiate between SEQ ID NOS: 550 and 552 resulted in almost universal amplification (placenta being the only exception). In contrast, when primers specific for SEQ ID NO:552 were used differences were seen in normal pancreatic, liver, lung, heart, fetal brain, mammary gland, bladder, and adrenal gland tissue, where there was no expression of SEQ ID NO:552 found.

Northern blotting was also carried out for SEQ ID NOS: 544, 546, 550 and 552. These experiments employed the same commercially available RNA libraries discussed above were used.

Samples (2 ug) of polyA⁺ RNA were analyzed from these samples, using random, ³²P labelled probes 300-360 nucleotides in length, obtained from PCR products. These probes were hybridized to the RNA, for 1.5 hours, at 68°C, followed by two washes at 0.1xSSC, 0.1 % SDS, 68°C, for 30 minutes each time.

SEQ ID NOs:544 and 546 were again found to be universally expressed.

Further screening identified additional isoforms of SEQ ID NOS:544 and 550. These are set forth as SEQ ID NOS: 554, 556, 558 and 560. The isoform represented by SEQ ID NO:554 (translated as SEQ ID N0:555) is a naturally occurring splice variant of SEQ ID NO:544, found in normal colon. SEQ ID N0:556 (translated as SEQ ID N0:557), which is an isoform of SEQ ID NO:550 (translated as SEQ ID N0:551), was found in brain tissue, primarily spinal chord and medulla. SEQ ID NO:558 (translated as SEQ ID NO:559), was found in normal kidney and in colon tumors, metastasized colon cancer, renal cancer, gastric cancer, and in colon cancer cell line Colo 205. It was not found in any normal tissue other than kidney.

The nucleic acid molecule whose nucleotide sequence set forth as SEQ ID N0:560 (translated as SEQ ID NO:561), is a further isoform of SEQ ID NO:552. It is similar to SEQ ID NO:558, except it contains a long nucleotide insert encoding a longer COOH terminus. It was expressed in normal bladder and kidney cells, and renal cancer cells. It was not expressed in colon cancer cells.

It is reported above that fourteen clones reacted with subsets of serum from both normal and cancer patients, while twenty eight reacted with autologous sera only. These clones were sequenced, in accordance with standard, art recognized methods. Of the clones which reacted only with autologous sera, nine appear to be previously unidentified sequences. These are set forth as SEQ ID NOS: 562, 564, 566, 568, 570, 572, 574, 576 and 578. SEQ ID NO:562 (translated as SEQ ID NO:563) is 1445 nucleotides long, and shows some similarity to known sequences for myosin and tropomyosin. SEQ ID N0:564 (translated as SEQ ID NO:565), which is 1226 nucleotides long, contains a TPR motif. The sequence set forth in SEQ ID NO:566 (translated as SEQ ID NO:567) is 1857 nucleotides long, and shows similarity to cyclophillins. The nucleotide sequence set forth in SEQ ID NO:568 (translated as SEQ ID N0:569) is 1537 nucleotides long, and shows similarity to murine gene 22A3, which has unknown function, but resembles an unconventional form of myosin, as well as an EST for heat shock inducible mRNA. As for the molecule set forth in SEQ ID NO:570 (translated as SEQ ID N0:571), it appears to resemble a nucleic targeting signal protein. SEQ ID NO: 572 (translated as SEQ ID NO:573) is 604 nucleotides long, and may encode a lysosymal protein. The molecule set forth in SEQ ID NO:574 (translated as SEQ ID N0:575) is 742 nucleotides long, and encodes a protein with an SH3 domain and which shows some similarity to GRB2 and human neutrophil oxidase factor. The molecule set forth in SEQ ID NO:576 (translated as SEQ ID NO:577) is 1087 nucleotides long, and encodes a protein which contains coiled core domains. The molecule set forth in SEQ ID NO:578 (translated as SEQ ID NO:579) is 2569 nucleotides long, shows some similarity with Drosophila homeotic material tudor protein, and has a DY(F)GN repeat.

Additional sequences were identified which were expressed in both normal sera and cancer cells. The sequence set forth in SEQ ID NO:580 (translated as SEQ ID N0:581), e.g., is 2077 nucleotides long, and was expressed by both colorectal cancer and normal cells. Analysis of the sequence showed that it possesses a nuclear targeting sequence. The molecule set forth in SEQ ID N0:582 (translated as SEQ ID N0:583) is 3309 nucleotides long, was expressed by colorectal cancer and normal cells, and is similar to heat shock protein 110 family members. The molecule presented in SEQ ID N0:584 (translated as SEQ ID NO:585) was expressed in a colon to lung metastasis, as well as by normal tissue. It is 2918 nucleotides in length. Analysis shows that it contains 2 zinc finger domains. The nucleotide sequence of SEQ ID NO:586 (translated as SEQ ID NO:587) was also expressed in a colon to lung metastasis, is 1898 nucleotides long, and is also expressed by normal tissue. Specifically, the reactivity of the molecules was as follows:

**Table 7**

| SEQ ID NO: | Normal Sera Reactivity | Tumor Sera Reactivity |
|---|---|---|
| 580 | 2/16 | 2/16 |
| 582 | 2/16 | 3/16 |
| 584 | 2/16 | 2/16 |
| 586 | 2/8 | 1/16 |

A more extensive set of RT-PCR experiments were carried out to study the expression pattern of SEQ ID NOS: 550, 552, 558 and 560. The results follow.

**Table 8: RT-PCR analysis of colon SEREX clones**

| normal tissue | SEQ ID NO.:550 | SEQ ID NO.:552 | SEQ ID NO.:558 | SEQ ID NO.:560 |
|---|---|---|---|---|
| kidney | + | Negative | Negative | Negative |
| colon | + | Negative | Negative | Negative |
| small | | Negative | Negative | Negative |
| intest. | + | Negative | Negative | Negative |
| brain | + | Negative | Negative | Negative |
| stomach | + | Negative | Negative | Negative |
| testis | + | Negative | Negative | Negative |
| pancreas | + | Negative | Negative | Negative |
| lung | + | Negative | Negative | Negative |
| liver | + | Negative | Negative | Negative |
| heart | + | Negative | Negative | Negative |
| fetal | | Negative | Negative | Negative |
| brain | + | Negative | Negative | Negative |
| mammary | | Negative | Negative | Negative |
| gland | + | Negative | Negative | Negative |
| bladder | + | Negative | Negative | Negative |
| adrenal | | Negative | Negative | Negative |
| gland | + | Negative | Negative | Negative |
| uterus | Negative | Negative | Negative | Negative |
| skeletal | | Negative | Negative | Negative |
| muscle | Negative | Negative | Negative | Negative |
| PBL | Negative | Negative | Negative | Negative |
| placenta | Negative | Negative | Negative | Negative |
| spleen | Negative | Negative | Negative | Negative |
| thymus | Negative | Negative | Negative | Negative |
| esophagus | Negative | Negative | Negative | Negative |

| Tumor Tissue | | | | |
|---|---|---|---|---|
| renal | | | | |
| cancer (4) | + (2/4) | + (2/4) | + (2/4) | + (2/4) |
| colon | | | | |
| primary | | | | |
| tumors (10) | + (10/10) | + (10/10) | +(10/10) | Negative |
| colon | | | - | |
| mets (4) | + (4/4) | + (4/4) | + (4/4) | Negative |
| breast | | | | |
| cancer (6) | + (3/6) | Negative | Negative | Negative |
| lung | | | | |
| cancer (6) | + (6/6) | Negative | Negative | Negative |
| gastric cancer (1) | + | + | + | Not tested |

| colon cancer cell lines | | | | |
|---|---|---|---|---|
| colo 205 | + | + | + | Negative |
| HT29 | + | + | Negative | Negative |
| HCT15 | Negative | Negative | Negative | Negative |

### Example 8:Isoiation and analysis of additional clones

For the establishment of a cDNA library from human tissue total RNA was obtained from 0.5 g of a renal clear cell carcinoma and established according to the method of Chomzynski as described above The mRNA was extracted from total RNA with oligo-dT-cellulose. The synthesis of the first strand cDNA was accomplished by the method described by Gubler and Hoffmann, Gene 25: 263 (1983) using RNase H and DNA polymerase I. For adaptation of the cDNA Klenow enzyme, adaptors with EcoRI restriction enzyme sites were ligated to the cDNA ends using T4 DNA ligase (Ferretti L and Sgamerella V, Nucl. Acids Res. 9: 3695 (1981)). Following restriction enzymatic digestion with the enzyme Xhol, cDNA molecules of different length were separated using Sephacryl 400 and transfected into λZAPII phage vectors (Short JM et al., Nucleic Acids Res. 16: 7583 (1988)). The recombinant phage DNA was packaged into phages after ligation with packaging extracts and used for the transfection of *E. coli* bacteria. The titration of the library resulted in 1.8 x 10⁶ recombinant primary clones. The total cDNA library was transfected in *E. coli* and amplified. The titer of the cDNA library after amplification was 10¹¹ plaque forming units per ml (pfu/ml). These transfected cells were used in experiments which follow.

In accordance with the invention as described above, identification of immunogenic material was achieved by using human sera which has been completely depleted of antibodies directed against antigens derived from native and lytic λ phage-transfected *E. coli* bacteria. To this end, the serum was absorbed, as follows.

*E. coli* bacteria of the strain XL1-blue were cultured in 50 ml LB medium overnight. After achieving an optical density of OD₆₀₀ = 1.0, the bacteria were pelleted by centrifugation, resuspended in 5 ml phosphate buffered saline (PBS), and lysed by sonication. The bacterial lysate was bound onto a matrix of activated Sepharose, which was then put into a column and used for the absorption of the human serum. The serum was run over this column 10 times.

A culture of *E. coli* XL1 blue bacteria in the exponential growth phase was pelleted by centrifugation, transfected in 0.01 M magnesium sulfate with 10⁶ λZAPII phages without a recombinant insert and incubated in 5 ml LB medium for four hours. The lysate of the transfected bacteria was used in the same manner as the untransfected bacteria, with the human serum described supra being passed through the column an addition ten times.

To complete the depletion of the serum, interfering antibodies from lytically transfected *E*. *col*i bacteria were cultured on agar plates and their proteins were blotted onto nitrocellulose membranes after 10 hours of culture at 37°C. Following this, the serum which had been preabsorbed according to the above steps was transferred to the blotted nitrocellulose membrane, and the absorption procedure was repeated five times. The serum, which was processed in accordance with the invention, was totally depleted of antibodies directed against antigens derived from *E. coli* and phages.

In this, a renal cancer-specific antigen was identified via the following steps. Bacteria of the strain XL1 blue were transfected with recombinant phages derived from the described cDNA library and plated at a density of 4-5x10³ plaque forming units (pfu) per plate in LB-medium with isopropylthiogalactopyranoside ("IPTG"). After 12 hours of incubation at 37°C, nitrocellulose membranes were put on top of the cultures and culture plates were incubated for another four hours. This was followed by incubation of the nitrocellulose membrane for one hour in Tris-buffered saline (PBS) with 5% milk powder. After washing the nitrocellulose membranes three times in TBS, the stripped human serum secured following Example 2 was diluted 1:1000 in TBS/0.5% (w/v) milk power and incubated overnight with gentle shaking. After the incubation with the nitrocellulose membrane the serum was removed and kept for additional testing. Following incubation with serum, the nitrocellulose membranes were washed three times in TBS, and incubated with a polyclonal alkaline phosphatase-conjugated goat anti-human IgG serum for one hour. Following this, the nitrocellulose membranes were washed repeatedly with TBS/0.01% (v/v Tween 20). The reaction was developed using nitroblue tetrazolium chloride and bromochloro-indoyl-phosphate in TBS. The binding of human antibodies to the expressed protein became visible by a blue ringformed color deposit on the nitro-cellulose membrane. The efficient preabsorption of the serum made in possible to develop the membrane at 37°C over several hours without compromising the quality of the test because of background reactivity caused by antibodies against *E. coli* and phage antigens.

Positive clones were localized on the agar plates, transferred into transfection buffer, and used for a second round of transfection and subcloning. A total of 1.8x10⁶ recombinant clones were subjected to screening and five different positive-reacting clones were identified.

Positive clones, i.e., those which had bound antibodies derived from the processed human serum, were subcloned to monoclonality by repeated rounds of transfection and testing of reactivity with the processed human serum. P-bluescript phagemids with the respective cDNA inserts were cloned by in vivo excision (Hay B and Short JM, Strategies 5: 16-19, 1992) from the λZAPII phage vectors and used for the transfection of E. coli SOLR bacteria. Plasmids were isolated from the bacteria after alkaline lysis with NaOH in a modification of the method of Birnboim HC and Doly J. J. Nucl. Acids Res. 7: 1513 (1979). The recombinant plasmid DNA was sequenced according to standard methods using M13-forward and M13-reverse oligonucleotides. The DNA sequence obtained and the resulting amino acid sequence were compared with nucleic acid and protein data banks (Gene Bank, EMBL, Swiss Prot). The sequencing of the cDNA inserts was continued using internal oligonucleotides. Analysis showed no homology with any sequences deposited in the data banks. The full length cDNA clone, referred to as SK313, was cloned with the RACE method (Frohman MA, Dush MK, Martin GR, Proc. Natl. Acad Sci. USA 85: 8998 (1988)), and had a. carbonic anhydrase domain at the 5' end.

As a continuation of these experiments, RNA was isolated from a spectrum of malignant and normal human tissues and Northern blots were performed with labeled SK313 (also referred to as clone HOM-RCC-313). The Norther blot analysis demonstrated that the mRNA of clone HOM-RCC-313 was overexpressed in 4 out of 19 renal cell carcinomas compared to normal kidneys. Very weak expression was found only in colonic mucosal tissue and in normal kidney. Expression in other tissues was not observed.

To determine the incidence of antibodies against antigens which are identified above, allogeneic sera from healthy individuals and tumor patients were analyzed. To this end, the sera were processed as described above and depleted from antibodies against antigens derived from *E. coli* and phages. For the detection of antigen-specific antibodies, phages derived from reactive clones were mixed with non-reactive phages derived from the same cDNA library at a ratio of 1:10 and tested as described above for reactivity with antibodies in the human test serum. The serum which had been used for the identification of the antigen was used as a positive control. The non-reactive phages served as a negative control. A serum sample was positive for antigen reactive antibodies, if the expected percentage of the phage plaques showed a positive reaction. In the case of the renal cell carcinoma antigen represented by clone HOM-RCC-313, the analysis of a spectrum of human sera showed that only sera from renal cell carcinoma patients contained reactive antibodies. Sera from healthy controls and patients with other tumors did not contain such antibodies.

The cDNA for clone HOM-RCC-313 was excised from the plasmid DNA by digestion with the restriction enzyme EcoR1, was separated by agarose gel electrophoresis, followed by extraction from the gel. This was then used to create a vector which expresses a fusion protein with the bacterial protein anthranilate synthetase. A relevant fragment in the exact open reading frame was cloned into pATH plasmid vectors (Koerner et al., Meth. Enzymol. 194: 477 (1991)). Induction of protein expression was obtained after transformation of the plasmids into E. coli of strain BL21 as described (Spindleret al., J. Virol. 49: 132 (1984)). Expressed fusion proteins were separated by SDS gel electrophoresis, excised from the gel, eluted and freeze dried. Rabbits were immunized by subcutaneous injection with 100 µg of the lyophilisate combined with Freund's adjuvant according to standard procedures. Immunization was repeated three times at two-week intervals using incomplete Freund's adjuvant. The rabbit was bled and antiserum was obtained. The obtained antiserum was depleted from antibodies reactive with E. coli and phages as described above and tested for reactivity against the renal carcinoma antigen as described for the human serum. Reactivity was detected at dilutions of 1: > 100,000.

Additional clones were identified from pancreatic cancer tumor specimen using the SEREX method of Sahin et al., (1995). A cDNA library was prepared and reacted with high titer IgG in sera of pancreatic carcinoma patients. A total of 8x10⁵ clones were screened with autologous serum, and 4.Sx10³ clones were screened with three different allogeneic sera. Twenty three clones, representing seven different transcripts were found. Four were previously unknown, unisolated genes. Of the remaining three, glycolytic enzyme aldolase A was found (SEQ ID Nos:799 and 800). Another molecule was "known" in that it was homologous to the rat eIF-5 gene (SEQ ID Nos:801 and 802), which is a eukaryotic translation initiation factor. The human eIF-5 gene was not previously known.

When hepatocelullar carcinoma libraries were studied in the same way, a total of 1.5x10⁶ clones were screened, and 98 positives were found. A total of 59 of these were sequenced, and corresponded to at least 20 different transcripts. Nine of these were assayed with allogeneic sera from hepatocellular cancer (HCC) patients and normal patients. High titered antibody was restricted to HCC patients. The majority of isolated sequences did not correspond to known molecules. Three which did were human albumin (SEQ ID Nos:803 and 804), senescence marker protein SMP30 (SEQ ID NOs:805 and 806), and C3VS (SEQ ID NOs:807 and 808). The latter was overexpressed in 2 of 4 hepatocarcinoma tissues, as compared to normal. Expression of SMP30 was found to vary highly.

The methodology was combined with subtractive cDNA techniques when assaying leukemia cells (T-ALL). An antigen was found which was identical to a broadly expressed, DNA repair enzyme.

Further assays identified the known molecule galectin-9 (SEQ ID NOs:809 and 810), as being highly expressed on human macrophages and dendritic cells. Expression is upregulated during differentiation ofmonocytes to macrophages. Highest levels were found on monocyte derived, dendritic cells.

Fusion proteins "LD1-mFc" and "LD2-mFc" were constructed to help analyze galectin-9. These consist of murine IgG heavy chain fragments, and a lectin domain (LD1, or LD2), as the N-terminus. Analysis indicated that the C-terminal lectin domain binds to the surface ligands, while the cell surface ligands recognized by the C-terminal lectin domain of galactin-9 was expressed only in a small, subpopulation of dendritic cells.

Further analysis of ovarian cancer cells (500,000 clones, using the SEREX method described above), identified previously known antigens MAGE-4 (SEQ ID Nos:811 and 812) and restin (SEQ ID Nos:813 and 814), and six other newly identified molecules.

Further experiments were carried out which involved restin. A variation of restin is known, i.e., "CLIP170", which was reported to mediate binding of endosomes to microlubules. It was found that both resin and CLIP 170 are highly expressed in dendritic cells, and are involved in the formation and transport of macropinosomes, a feature of professional antigen presenting cells. Expression of restin was induced after 48 hours of culture of monocytes in GM-CSF/IL-4 supplemented medium. Highest levels were found in immature dendritic cells. When microlubile systems, which are essential for the activity of restin/CLIP-170 were disrupted, macropinocytosis was lost completely.

Further work with the methodology disclosed herein on glioma identified a clone encoding nm23-H2 protein (SEQ ID Nos:815 and 816). This clone corresponds to subunit B of nucleoside diphosphate kinase, which is implicated in tumor metastasis control. It is also known as PuF, a transcriptional factor, for c-myc proto-oncogenes. Antibodies against the protein were found in 1 of 18 sera of brain malignancy patients, 3 of 20 melanoma patients, and 2 of 20 sera from healthy patients. When expression studies were carried out using RT-PCR, 25 of 28 brain tumor, and 4 or 5 mengioma tumor samples were found to express the gene.

### Example 9:Isolation and analysis of lung cancer clones

A cDNA library was constructed from a case of moderately differentiated adenocarcinoma of the lung, obtained from the Department of Pathology at The New York Hospital. The library was constructed in a λZAP Express vector using a cDNA library kit (Stratagene, La Jolla, CA).

The cDNA library was screened with autologous patient's serum as described previously [Sahin, U. et al., Proc Natl Acad Sci USA 92:11810-3 (1995); Chen, Y.T. et al. Proc Natl Acad Sci USA. 94:1914-8 (1997)]. Briefly, the serum was diluted 1:10, pre-absorbed with transfected *E. coli* lysate, and a 1:10 dilution of the absorbed serum (final dilution of serum 1:100) was incubated overnight at room temperature with the nitrocellulose membranes containing the phage plaques. After washing, the filters were incubated with alkaline phosphatase-conjugated goat anti-human Fc γ secondary antibodies and the reactive phage plaques were visualized by incubating with 5-bromo-4-chloro-3-indolyl-phosphate and nitroblue tetrazolium. Phagemid clones encoding human immunoglobulin sequences were subsequently eliminated during the secondary screening.

The reactive clones were subcloned, purified, and *in vitro* excised to pBK-CMV plasmid forms (Stratagene). Plasmid DNA was prepared using Wizard Miniprep DNA Purification System (Promega, Madison, WI). The inserted DNA was evaluated by EcoRI-XbaI restriction mapping, and clones representing different cDNA inserts were sequenced. The sequencing reactions were performed by DNA Services at Cornell University (Ithaca, NY) using ABI PRISM (Perkin Elmer) automated sequencers.

To evaluate the mRNA expression pattern of the cloned cDNA in normal and malignant tissues, gene-specific oligonucleotide primers for PCR were designed to amplify cDNA segments of 300-400bp in length, with the estimated primer melting temperature in the range of 65-70°C. All primers were commercially synthesized (Operon Technologies, Alameda, CA). RT-PCR were performed using 35 amplification cycles in a thermal cycler (Perkin Elmer) at an annealing temperature of 60°C.

Genomic DNA were extracted from cell lines and frozen tumor tissue. Following restriction enzyme digestion, the DNA was separated on a 0.7% agarose gel, blotted onto nitrocellulose filters, and hybridized to an a ³²P-labeled DNA probe at high stringency (65°C, aqueous buffer). Washing of the blot was also under high stringency conditions, with a final wash in 0.2XSSC with 0.2% SDS at 65°C.

To identify the 5'end of the mRNA transcripts, RACE (rapid amplification of cDNA ends) methodology was utilized using the Marathon cDNA amplification kit (Clontech) and adaptor-ligated testicular cDNA as the substrate. The PCR products, after separation by agarose gel electrophoresis, were cloned into the direct PCR cloning vector pGEM-T (Promega).

Single-strand conformation polymorphism (SSCP) analysis was performed to analyze cDNA from various tissues, using previously described protocols [Dracopoli, C.D. et al., New York: John Wiley and Sons, Inc. (1997)]. Briefly, PCR was performed with 5 µl RT product in a final volume of 25 µl, with 2µCi of α³²p-dCTP (~3000 Ci/mmole, New England Nuclear) per reaction. The PCR conditions was as described for RT-PCR above. After the PCR, 1 µl of the mixture was diluted with 5 µl of denaturing buffer (95% formamide, 20 mM EDTA, 0.05% bromophenol blue, 0.05% xylene cyanol), heat-denatured at 98°C for 2 min, and electrophoresed through an 8% polyacrylamide gel with 10% glycerol. As controls, aliquots of the same samples were diluted with a standard non-denaturing DNA loading dye and electrophoresed in parallel. The electrophoresis was performed at room temperature at a constant power of 10-12 watts. The gel was then dried and autoradiography performed for 15-24 hours with an intensifying screen.

### Identification of Immunoreactive cDNA clones

A cDNA expression library of 1.42x10⁷ primary clones was prepared from Lu15, a specimen of moderately differentiated adenocarcinoma of the lung and 8x10⁵ phage plaques were immunoscreened with absorbed autologous patient serum at 1:100 dilution. Excluding false-positive clones encoding immunoglobulin gene fragments, 20 positive clones were identified. These clones were purified and sequence analyzed. Comparisons of the sequences showed that these clones represented cDNAs from 12 distinct genes, designated NY-LU-1 through NY-LU-12 (Table 9). A homology search through the GenBank/EMBO databases revealed that 4 of the 12 genes corresponded to previously known molecules, and 8 others were unknown genes, with sequence identity limited only to short segments of known genes or to expressed sequence tags (ESTs).

**Table 9: NY-LU clones**

| **Gene Designation** | **Gene/Sequence Identity [Accession Number]** | **cDNA** | **Comments** |
|---|---|---|---|
| **NY-LU-1** | Aldolase A (N and H type) [X06352] | Lu-15/24, 72, 83, 158, 219, 241 | Human fructose, 1,6 diphosphate aldolase A. Expressed in muscle (M type), but also in most other tissues (N and H types). Levels increased in most lung cancers; released into blood upon trauma and in several cancers. |
| **NY-LU-2** | hASNA-1 [U60276] | Lu-15/26, 66 | Human homolog of the ATP-biding ars A component of the bacterial arsenite transporter. Previously cloned by SEREX from a testicular library (Chen et al., unpolished). Ubiquitously expressed. |
| **NY-LU-3** | Annexin IX [L19605] | LU-15/64 | Homosapiens 56K autoantigen. Antibodies to Annexin 1X are found in multiple autoimmune diseases. ubiquitously expressed. |
| **NY-LU-4** | Rip-1 [U55766] | Lu-15/65 | Human HIV Rev-interacting protein. Expressed in B cells, monocytes and rhabdomyoma cells. |
| **NY-LU-5** | Unknown [W61291, W92962, etc.] | Lu-15/80 | Expressed ubiquitously (by RT-PCR). |
| **NY-LU-6** | Unknown [none] | Lu-15/85 | Sequence contains no ORF, expressed ubiquitously (by RT-PCR). |
| **NY-LU-7** | Unknown [W23466, AA 167732, etc.] | Lu-15/135,217 | Expressed in neuron, pregnant uterus, lung ca., parathyroid tumors, etc. |
| **NY-LU-8** | Unknown [Z78323, N39225, etc.] | Lu-15/139 | Expressed in fetal heart, retin, multiple sclerosis, etc. |
| **NY-LU-9** | Unknown [W26569, AA036884, etc.] | Lu-15/145 | Expressed in retina, pregnant uterus, fetal liver-spleen, etc. |
| **NY-LU-10** | Unknown [M29204, etc.] | Lu-15/154 | Expressed in colon, pancreas, pregnant uterus, fibroblasts, etc. |
| **NY-LU-11** | Unknown [W23466, AA057400, etc.] | Lu-15/270 | Expressed in retina, pregnant uterus, fetal heart, fetal liver-spleen, parathyroid tumors, etc. |
| **NY-LU-12** | g16 | Lu-15/251 | Located at the 3p21 TSG locus (see text) |

Of the 4 known genes, aldolase A (NY-LU-1; SEQ ID NOs:689 and 690) was most frequently isolated, representing 6 of 20 primary positive clones in the entire screening. NY-LU-2 (SEQ ID NO:691), represented by two isolates, was the human homolog of the ATP-binding arsA component of the bacterial arsenite transporter, a gene which has been shown to be ubiquitously expressed in various tissues [Kurdi-Haidar, B. et al., Genomics 36:486-91 (1996)]. NY-LU-3 (SEQ ID Nos:692 and 693) encodes annexin XI, which is a 56KD ubiquitously expressed antigen to which autoantibodies have been described in sera from patients with various autoimmune diseases [Misaki, Y. et al., J Biol Chem 269:4240-6 (1994); Misaki, Y. et al., J Rheumatol. 22:97-102 (1995)]. The last gene in this group, NY-LU-4 (SEQ ID NOs:694 and 695), codes for the human HIV Rev interacting protein Rip-1, which has been shown to be expressed in the monocyte cell line U937, the rhabdomyoma cell line RD, as well as in adherent monocytes and primary lymphocytes [Refaeli, Y. et al., Proc Natl Acad Sci USA 92:3621-5 (1995)].

Of the eight unknown genes, 6 (NY-LU-5, 7, 8, 9, 10, 11; SEQ ID Nos:696, 698, 699, 700, 701 and 702/703, respectively) shared sequence identify with reported expressed sequence tags (EST), likely representing cDNA products derived from the same genes. These ESTs were derived from various somatic tissues unrelated to lung, e.g., neuron, pregnant uterus, colon, endothelial cells, etc., suggesting that these genes are widely expressed in human tissues (Table 9), making them unlikely candidates for vaccine-based tumor immunotherapy. These clones were not further investigated. The only novel gene in this group, NY-LU-6 (SEQ ID NO:697), showed no sequence identity to deposited sequences in the public databases. The tissue expression pattern of this gene was evaluated by RT-PCR analysis using gene-specific primers and a normal tissue RNA panel consisting of lung, colon, kidney, liver, brain and testis. Results showed universal expression in these tissues, and this clone was not further analyzed.

### NY-LU-12 is on TSG locus of chromosome 3p21.

The last gene in the unknown gene group, NY-LU-12, was represented by the immunoreactive clone Lulls-251. This clone, 1081bp in length, contained an uninterrupted open reading frame (ORF) of 952 bp, followed by a 129bp 3'untranslated region. No translation initiation codon was identified, indicating that this was a partial cDNA clone.

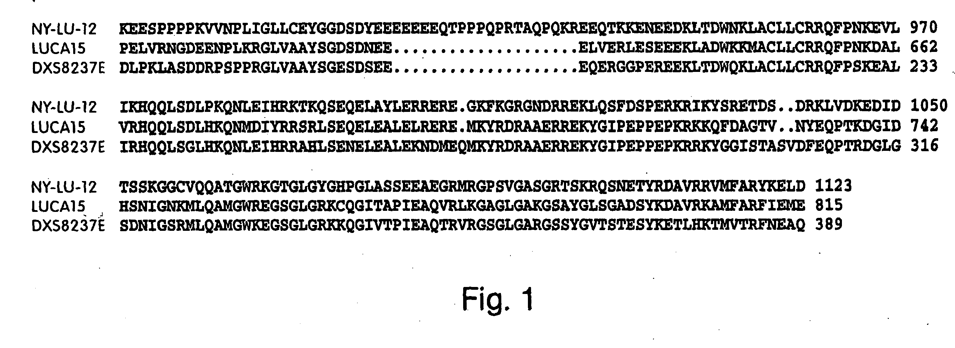

A sequence homology search revealed that this gene shared up to 30% homology with two different human proteins at its C-terminus (Fig. 1), LUCA15 and DXS8237E (GenBank accession numbers U23946, and P98175) and also shared homology to S1-1, the rat counterpart of DXS8237E [Inoue, A. et al., Nucleic Acids Res. 24:2990-7 (1996)]. LUCA15 was subsequently proven to be a gene immediately centromeric to NY-LU-12 on the *TSG* locus on chromosome 3p21 (see below and [Wei, M.H. et al., Cancer Res. 56: 2487-92 (1996))]. Our analysis of LUCA15 revealed the presence of a nuclear localization signal in the putative LUCA15 protein. DXS8237E, was located on chromosome Xp11.23 [Coleman, M.P. et al., Genomics 31:135-8 (1996)] and its rat homolog, S1-1, has been shown to be an RNA-binding protein [Inoue, A. et al., Nucleic Acids Res. 24:2990-7 (1996)].

Of particular interest, however, was that a short segment (92bp) at the 5' end of NY-LU-12 was identical to a previously identified gene, g16 (GenBank accession number U50839), which was mapped to chromosome 3p21.3 and was interrupted in the small cell lung cancer line NCI-H740.

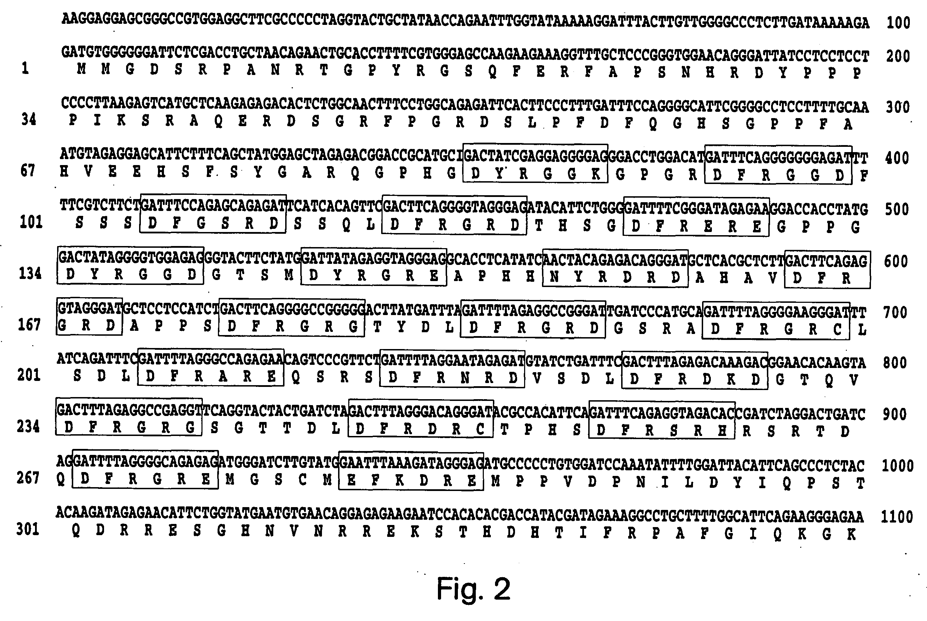

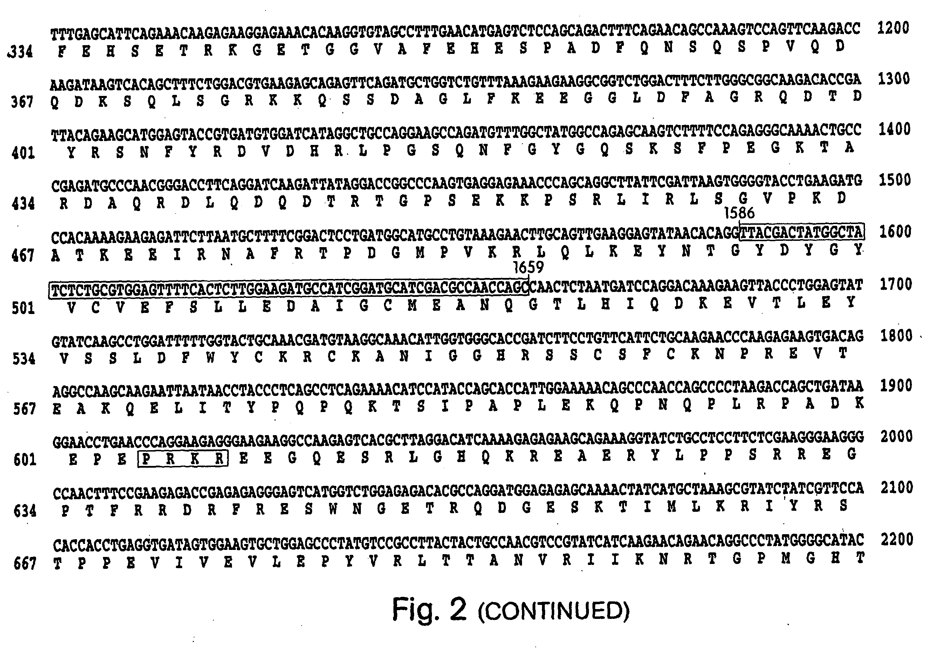

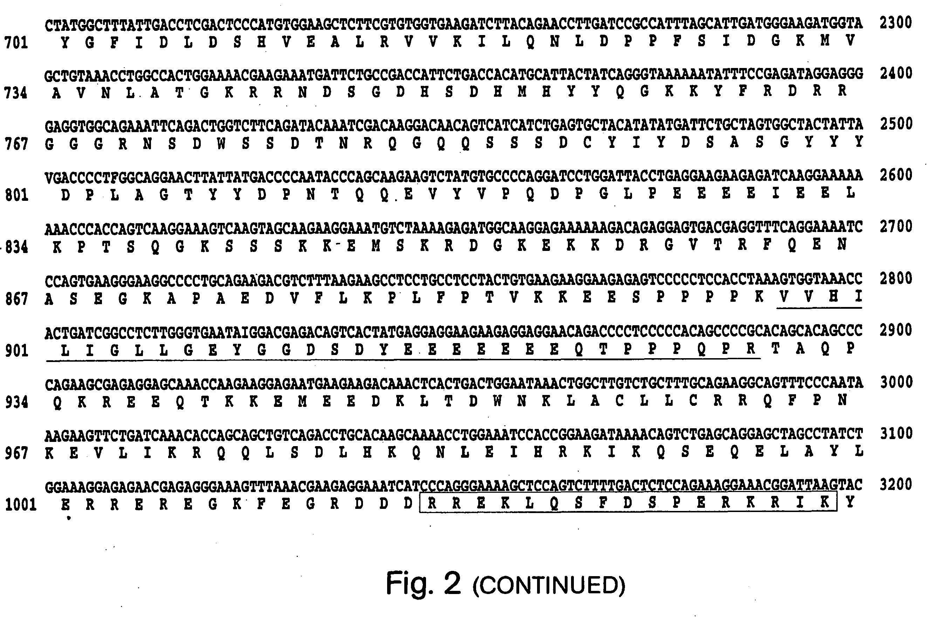

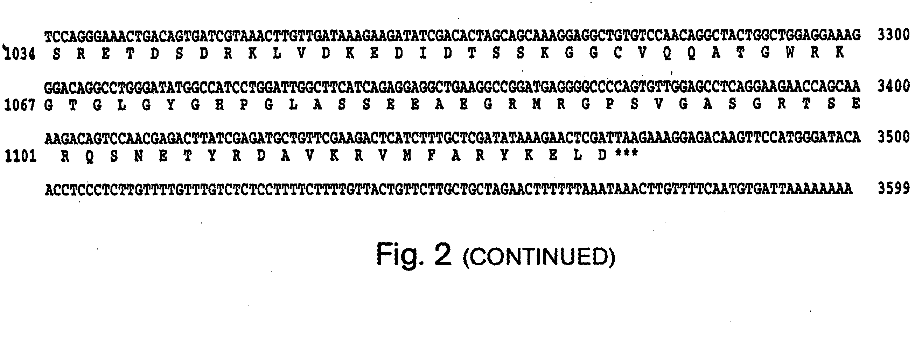

To compare NY-LU-12 with g16, the full-length NY-LU-12 cDNA sequence was obtained from normal testicular mRNA through a combination of 5'RACE and direct PCR cloning strategies. The predominant cDNA form (SEQ ID No:707), excluding the poly A tail, is of 3591bp in length. An open-reading-frame of 1123 amino acid residues (SEQ ID No:708) was identified (nt. 102-3470), with 101bp of5' untranslated and 129bp of the 3' untranslated region. The nucleotide and amino acid sequences are shown in Fig. 2.

Comparison with the g16 sequence verified that these two are identical genes and mapped NY-LU-12 to *TSG* locus on 3p21. However, the reported g16 sequence, 2433 bp in length, lacks the 5' end 110 bases which include the translational initiation codon at nucleotide 102, and also the 3' end 980 nucleotides of NY-LU-12. In addition, 74bp DNA segment (nt. 1587-1659 of NY-LU-12) was absent in the reported g16 sequence. Oligonucleotide primers flanking this 74 bp region were designed and used to amplify RNA from 1 normal lung, 5 lung cancer cell lines, and 6 lung cancer specimens. Two RT-PCR products were seen in every specimen, corresponding to the sizes of the two cDNA variants. It was thus concluded that this variation represents an alternate splicing event which occurs in both normal and cancerous lung tissues. Of interest, however, was the difference in the putative translational products resulting from this additional 74bp exon. In the absence of this exon, the open-reading-frame of NY-LU-12 would end in the termination codon at nt.1736, as reported for g16, with a total length of 520 amino acid residues (in contrast to 1123 residues in the longer transcript). Moreover, this shorter form would not encode the C-terminal portion of the NY-LU-12 protein, the segment responsible for the immunoreactivity of Lu15-251 to the autologous patient serum.

### Additional cDNA variants of NY-LU-12

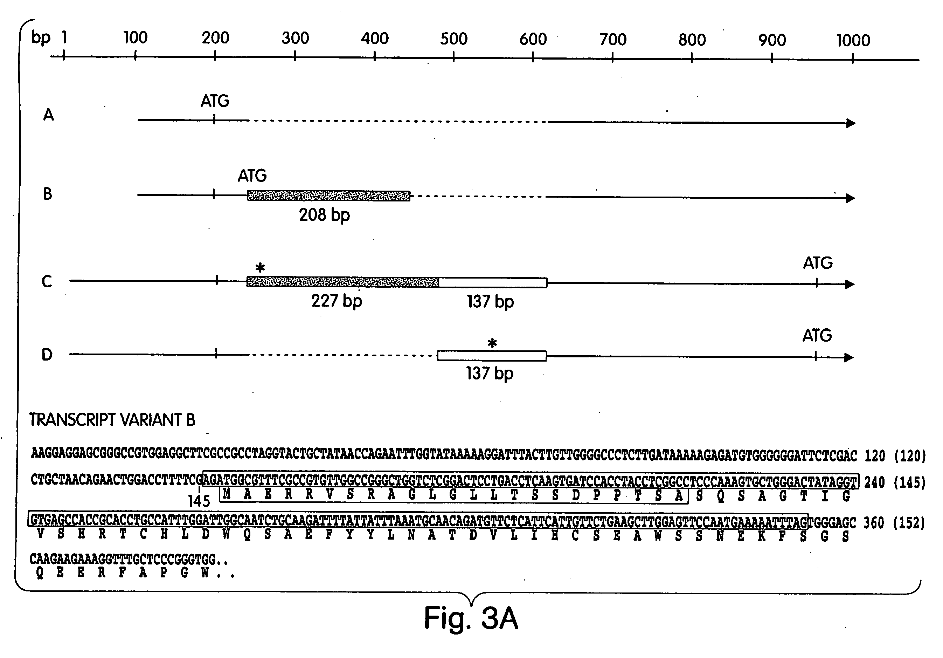

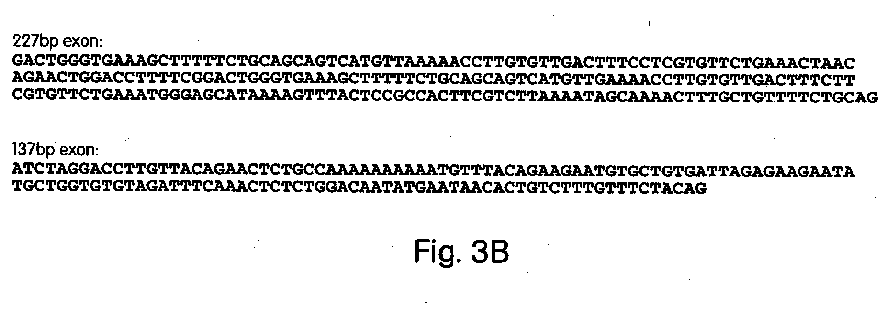

In the process of 5'RACE cloning of the full-length NY-LU-12, three minor forms of cDNA products were identified which varied in their transcriptional initiation site and in their exon usage in the 5' segment of this gene. These variants will be described as transcripts B, C, and D (SEQ ID Nos:709, 711 and 712). Fig. 3 shows the comparison of these transcripts to the predominant cDNA form (transcript A, see Fig. 2).

Transcript B (Fig. 3A, bottom) contains an additional exon of 208 base pairs, inserted at nucleotide 145 of the NY-LU-12 sequence. The original ORF of NY-LU-12 is disrupted due to this inserted sequence, and the AUG initiation codon used by transcript A is thus unlikely to be used by this transcript. A new potential translational initiation site, however, is found within this new exon and would continue the translation into the ORF of transcript A. The final product would be a protein of 1177 amino acids (SEQ ID N0:710), with the 69 residues at the N-terminus different from transcript A. Interestingly, this new exon encodes for a signal peptide not present in the transcript A (Fig. 3A, bottom), and it is possible that these two products are localized to different subcellular compartments.

Similar to transcript B, transcripts C and D both contained additional exon(s) not present in transcript A. Transcript C contained two extra exons in tandem and a length of 364bp, only one of which (137bp) was present in transcript D, Figure 3B. These extra exon(s), inserted at the same alternate splicing site as transcript B, disrupted the original ORF, and the only long ORF would initiate at nucleotide position 498 of NY-LU-12-(959 of transcript C, 635 of transcript D). Considering the long untranslated region at the 5' end, it is doubtful whether transcripts C and D are indeed translated *in vivo*.

Correlating with this variation of NY-LU-12 mRNA, Northern blot analysis showed several RNA species in normal tissues, ranging approximately from 3 to 4.4 Kb. The intensity of individual bands also appear to vary among different tissues, suggesting post-transcriptional tissue specific regulation of NY-LU-12 mRNA.

### Feature of NY-LU-12 and its putative gene product

Analysis of the NY-LU-12 amino acid sequence showed 20 inexact 6 amino acid repeats with a consensus sequence of D(F/Y)RGR(D/E) close to the N-terminus (Fig. 2). These repeats were separated by 4 to 6 amino acid intervals, which showed no apparent sequence homology among each other. This feature in primary sequence is distinctive among known proteins. Hydrophilicity plot revealed that this region, although hydrophilic in general, has regular hydrophobic turns, and these cycles of hydrophilicity changes correspond to the hexapeptide repeats. Although the significance of this characteristic is unclear at present, this segment of sequence is highly rich in arginine and aspartic acid, a feature shared by RNA binding proteins. Similar motifs, rich in arginine and aspartic acid residues, were found in other RNA-binding proteins [Witte, M.M. et al., Proc Natl Acad Sci USA 94: 1212-7 (1997); Wilson, R. et al., Nature 368:32-8 (1994); Seraphin, B. et al., Nature 337:84-7 (1989); Takagaki, Y. et al., Proc Natl Acad Sci USA 89:1403-7 (1992)], e.g., RNA [Seraphin, B. et al., Nature 337:84-7 (1989)] hnRNA 3'end cleavage stimulation factor [Takagaki, Y. et al., Proc Natl Acad Sci USA 89:1403-7 (1992)], etc., indicating that NY-LU-12 is likely to be an RNA-binding protein. Consistent with this, PROSITE analysis of the putative NY-LU-12 protein identified a bipartite nuclear localization signal between amino acids 1016-1032 and a 4-residue nuclear localization pattern (PRKR) at amino acid 604-607 (Fig. 2), suggesting that NY-LU-12 is a nuclear protein. Analysis for post-translational modification sites showed potential sites for tyrosine sulfation, amidation, as well as phosphorylation sites for protein kinase A, C, casein kinase II, and tyrosine kinase. A PEST region, peptide sequences consistently found among unstable proteins with short half lives, was identified at amino acids 897-928 (Fig. 2), implying NY-LU-12 as an unstable protein.

### Southern blot analysis of NY-LU-12 in normal and tumor tissues

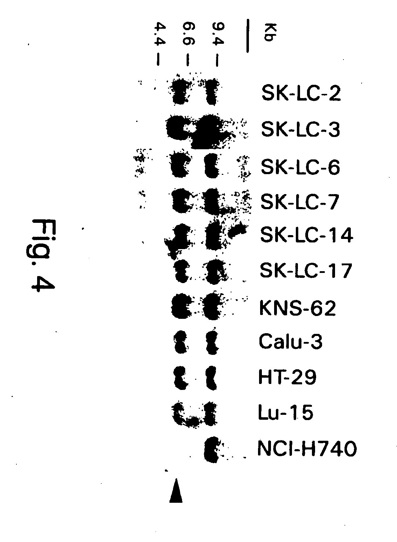

To investigate the status of NY-LU-12 in normal and tumor cells, Southern blot analysis was performed on 9 lung cancer cell lines (3 adenocarcinoma, 2 squamous, and 3 large cell anaplastic), Lu15 tumor DNA, and a colon cancer cell line HT29 (Fig. 4). (HT29 was included due to the finding of an EST identified in the GenBank, accession number AA079461, which appeared to be a fusion sequence between semaphorin IV gene and NY-LU-12.) Using a 1.1Kb cDNA probe (nucleotide 1095-2140) and HindIII digested DNA, the results showed that one of the two hybridizing bands was absent in NCI-H740, confirming that NY-LU-12 was partially deleted in this cell line. The breakpoint of this deletion, by using primers from different regions, was further defined to be between nucleotides 1433 and 1777 of NY-LU-12, with the 3' sequences homozygously deleted. Besides NCI-H740, however, no evidence of homozygous deletion was seen in any other tumor cell line sample or in LU15. The similar band intensities and identical sizes of the DNA signals in all specimens also argued against the possibility of a heterozygous deletion or translocation of this gene, at least in the region analyzed. No change was found in HT29, suggesting that the semaphorin IV/NY-LU-12 fusion sequence in the GenBank probably represents a cloning artifact.

### SSCP and sequence analysis of NY-LU-12 in Lu15 tumor DNA.

The mapping ofNY-LU-12 to the lung cancer *TSG* locus raised the possibility that an altered protein product due to mutational event may be the basis for the autologous immune recognition. This possibility was explored using DNA sequencing and single-strand confirmational polymorphism (SSCP) analysis.

The DNA sequence contained in the immunoreactive clone Lu15-251 (nucleotide 2518-3599 of NY-LU-12) was obtained from the normal counterpart by RT-PCR cloning using autologous normal lung tissue, and no mutations were found when compared to Lulls-251.

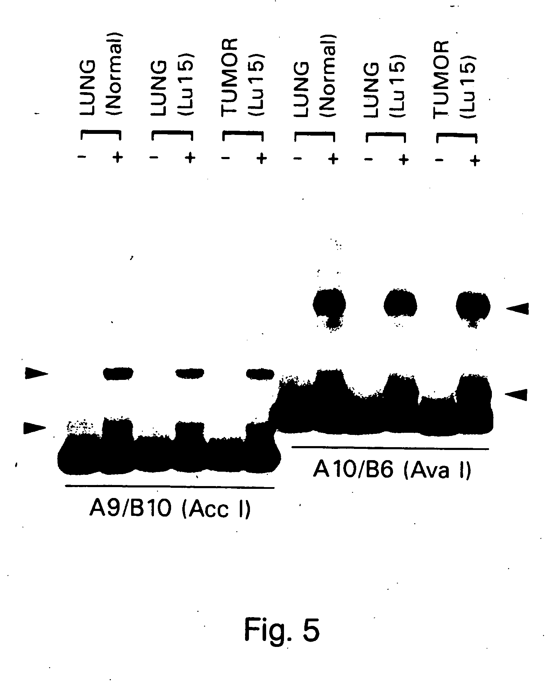

RT-PCR SSCP was then used to analyze the entire NY-LU-12 gene, comparing Lulls tumor tissue and autologous normal lung tissue. To encompass the whole sequence, 10 sets of primer pairs were designed, each amplifying a range of 205 to 603 bps. For products >400bps, a restriction enzyme digestion step was added prior to the electrophoresis step to further reduce the fragment sizes and increase the assay sensitivity. Results showed no reproducible changes between normal and tumor tissues, and thus no evidence of mutation in Lu15 tumor cDNA. A representative set of SSCP analysis is shown in Fig. 5.

### Serological response to NY-LU-12 in lung cancer patient

The frequency of anti-NY-LU-12 response was examined among normal adult and patient sera using the phage plaque assay identical to the original immunoscreening procedure. Of 21 absorbed sera from allogeneic lung cancer patients, one (Lu22) reacted strongly with the-Lul 5-251 plaque at 1:1000 dilution, and another (Lu7) also reacted at 1:1000, but only weakly. Nineteen other lung cancer patient sera were non-reactive, nor were the sera from 16 healthy donors, 15 colon cancer, 5 breast cancer, 1 renal cancer, 1 prostate cancer, 1 esophageal cancer, and 1 melanoma patients.

### Example 10: Expression analysis of additional cancer associated nucleic acids

The clone RING 3 was isolated from breast SEREX analysis as LONY-Br-5 (see above). The gene was identified as homologous to the "bromodomain testis" gene (BRDT; GenBank accession number AF019085). Analysis of related genes identified BRDT as a gene expressed only in testis, which was then investigated by RT-PCR analysis as described above.

The primers used to perform RT-PCR had the following sequences:
BRDT F1: CAAGAAAGGCACTCAACAG (bp 543-563 of BRDT)
BRDT R1: TTCACTACTTGCTTTAACTGC (bp 776-797 of BRDT)

The meiotic protein H1T (Histone 1 Testis; GenBank accession number M60094) was identified through a literature search for meiotic proteins (testis specific expression). -

The primers used to perform RT-PCR had the following sequences:
H1F1: TGCCGAACCTCTCTGTGTC (bp 116-135 of H1T)
H1R1: GCTTCGTGTAGATTTAGGAATC (bp 344-366 of H1T)

**Table 10: RT-PCR analysis**

| Normal Tissue | BRDT | H1T |
|---|---|---|
| mammary gland | - | - |
| liver | - | - |
| small intestine | - | - |
| brain | - | +/- (very weak) |
| lung | - | - |
| fetal brain | - | - |
| placenta | + | + |
| kidney | - | - |
| skeletal muscle | - | - |
| pancreas | - | - |
| adrenal gland | - | - |
| heart | - | - |
| thymus | - | - |
| uterus | - | - |
| prostate | - | +/- (very weak) |
| spleen | - | - |
| Testis | + | + |

| Tumor Tissue | BRDT | H1T |
|---|---|---|
| Colon | 0/6 | 0/6 |
| Breast | 0/6 | 6/6+ |
| Melanoma | 0/12 | 3/12+ |
| Lung | 8/26+ | 4/26+ |
| Renal | 0/2 | 0/2 |
| Ovary | 0/2 | 0/2 |
| Esophageal | 0/1 _ | 0/1 |
| Gastric | 0/1 | 0/1 |
| Bladder | 0/2 | 0/2 |

Lung cancer specific expression of BRDT was observed (see table above). BRDT was expressed only in normal testis and possibly in placenta. The expression analysis of H1T revealed that all breast tumor samples (6 of 6) and ∼30% lung cancers and melanoma tissue samples expressed H1T. H1T was expressed in normal testis and possibly in placenta and brain.

### Example 11: allogeneic serotyping

To confirm the cancer associated expression of SEREX clones, allogenic sera screening of gastric cancer patients' sera was conducted. Sera from normal patients (gastritis) was used as a control for expression of the clones in non-gastric cancer. The screening procedure used was as described above for the SEREX screening, except for the absorption of anti-bacterial and anti-bacteriophage antibodies. The modifications were as follows.

Serum from a stomach cancer patient or a normal individual was diluted to 1:10 in TBS (Tris buffered saline; final volume 5 ml) and passed through a column (BIO-RAD Poly-Prep Chromatography Column, Hercules. CA, USA) containing 0.5 ml Sepharose-4B cross linked to E. coli Y1090 lysate and 0.5 ml Sepharose-4B cross linked to E. coli BNN97 (5 Prime 3 Prime, Inc, Boulder, CO, USA). After repeating the column chromatography 10 times, serum was then diluted to 1:100 in TBS containing 1% BSA and 0.02% sodium azide. To remove antibodies to bacteria and baceteriophages further, 10 ml absorbed serum was incubated overnight with a 82 mm nitrocellulose membrane on which XL-1 Blue MRF' bacteria and lambda ZAP Express phages (Stratagene, La Jolla, CA USA) were immobilized. The serum was stored at - 80°C until use. For allogeneic typing, an equal numbers of positive phage and negative phage were mixed and plated and processed by the standard SEREX screening procedure.

The results of the allogenic screening experiments follow:

**Table 11: Allogenic Sera Screening of SEREX Sequences from Gastric Patients**

| **Sequence** | | **Isolated in Serex Patients** | **Allogenic Serotyping Gastric Cancer Sera** | **Allogenic Serotyping Normal Sera** |
|---|---|---|---|---|
| **Gene/Clone** | **Number** | | | |
| RPB-J H-2K binding factor | | SM 1 | 6/12 | 6/16 |
| Telomeric repeat binding protein | | SM1 | 1/12 | 0/16 |
| Ser/Thr protein kinase | | SM1 | 1/12 | 0/16 |
| SRY interacting protein-1 | | SM1 | 2/12 | 1/16 |
| Sterol carrier protein X | | SM1 | 2/12 | 0/16 |
| Archain | | SM1 | 1/12 | 1/16 |
| HEM-1 | | SM1 | 2/12 | 1/16 |
| Id-1 helix-loop-helix protein | | SM1 | 1/12 | 0/16 |
| helix-loop-helix transcription factor | | SM1 | 1/12 | 0/16 |
| Follistatin related precursor protein | | SM1,CK, KM | 6/12 | 0/16 |
| Translation initiation factor eIF-4gamma | | SM1,SS1, KM | 5/12 | 2/16 |
| M phase phophoprotein I | | SM1,SS1 | 8/12 | 5/16 |
| Lysal tRNA synthase | | SM1 | 1/12 | 0/16 |
| Gelsolin | | SM1 | 4/12 | 0/16 |
| Zinc finger protein | | SM1 | 1/12 | 1/16 |
| Goliath | | SM1 | 2/12 | 1/16 |
| zhx-1 | | SM1 | 1/12 | 1/16 |
| SG24 | | SM1,SS1, KM | 5/12 | 0/16 |
| SG132 | | SM1 | 3/12 | 0/16 |
| S553 | | SM1 | 7/12 | 7/16 |
| S134 | | SM1 | 3/12 | 0/16 |
| S328 | | SM1 | 2/12 | 1/16 |
| S365 | | SM1, KM | 2/12 | 0/16 |
| FKBP25 | | KM, SS1 | 5/12 | 0/16 |
| Pros-27 | | KM, CK | 3/12 | 1/16 |
| BS4 | | KM | 1/12 | 1/16 |
| GnRH-II | | KM | 1/12 | 0/16 |
| CTBP | | KM | 1/12 | 0/16 |
| ETF | | KM | 3/12 | 1/16 |
| KIAA0438 | | KM | 1/12 | 5/16 |
| KIAA0367 | | KM | 4/12 | 3/16 |
| APK1 | | KM | 2/12 | 0/16 |
| IPP | | KM | 1/12 | 0/16 |
| Tropomyosin | | KM | 1/12 | 0/16 |
| p63 | | KM | 1/12 | 0/16 |
| KIAA0181 | | KM | 1/12 | 0/16 |
| KIAA0349 | | KM | 1/12 | 0/16 |
| RPB1 | | KM | 5/12 | 9/15 |
| PPIM | | KM | 1/12 | - |
| EB virus | | KM | 3/12 | - |
| G.KM073 | | KM | 6/12 | - |
| G.KM403 | | KM | 1/12 | - |
| KM192 | | KM | 1/12 | - |
| KM294 | | KM | 1/12 | - |
| KM362 | | KM | 1/12 | - |
| KM031 | | KM | 1/12 | - |
| KM081 | | KM | 3/12 | - |
| KM201 | | KM | 1/12 | - |
| KM1496 | | KM | 1/12 | - |
| KM334 | | KM | 1/12 | - |
| KM313 | | KM | 1/12 | |
| E-cad/Y | | CK | 1/12 | 0/16 |
| IPBP | | SS1 | 1/4 | - |
| OS-9 | | SS1 | 1/4 | - |
| Kinesin light chain | | SS1 | 1/4 | - |

The screening results shown above confirm the association of the SEREX clones with cancer. There is a higher correlation of cancer and the expression of certain clones, in particular, follistatin related precursor protein, the translation initiation factor eIF-4gamma, the unknown sequence SG24, the FK506-binding protein 25, and the unknown sequence G.KM073. These clones are well suited to serve as diagnostic indicators of disease and as targets for therapeutics (e.g., vaccine compositions) development.

### Example 12: Preparation of recombinant cancer associated antigens

To facilitate screening of patients' sera for antibodies reactive with cancer associated antigens, for example by ELISA, recombinant proteins are prepared according to standard procedures. In one method, the clones encoding cancer associated antigens are subcloned into a baculovirus expression vector, and the recombinant expression vectors are introduced into appropriate insect cells. Baculovirus/insect cloning systems are preferred because post-translational modifications are carried out in the insect cells. Another preferred eukaryotic system is the *Drosophila* Expression System from Invitrogen. Clones which express high amounts of the recombinant protein are selected and used to produce the recombinant proteins. The recombinant proteins are tested for antibody recognition using serum from the patient which was used to isolated the particular clone, or in the case of cancer associated antigens recognized by allogeneic sera, e.g. certain breast cancer and gastric cancer associated antigens, by the sera from any of the patients used to isolate the clones or sera which recognize the clones' gene products.

Alternatively, the cancer associated antigen clones are inserted into a prokaryotic expression vector for production of recombinant proteins in bacteria. Other systems, including yeast expression systems and mammalian cell culture systems also can be used.

### Example 13: Preparation of antibodies to cancer associated antigens

The recombinant cancer associated antigens produced as in Example 12 above are used to generate polyclonal antisera and monoclonal antibodies according to standard procedures. The antisera and antibodies so produced are tested for correct recognition of the cancer associated antigens by using the antisera/antibodies in assays of cell extracts of patients known to express the particular cancer associated antigen (e.g. an ELISA assay). These antibodies can be used for experimental purposes (e.g. localization of the cancer associated antigens, immunoprecipitations, Western blots, etc.) as well as diagnostic purposes (e.g., testing extracts of tissue biopsies, testing for the presence of cancer associated antigens).

### Example 14: Expression of cancer associated antigens in cancers of similar and different origin.

The expression of one or more of the cancer associated antigens is tested in a range of tumor samples to determine which, if any, other malignancies should be diagnosed and/or treated by the methods described herein. Tumor cell lines and tumor samples are tested for cancer associated antigen expression, preferably by RT-PCR according to standard procedures. Northern blots also are used to test the expression of the cancer associated antigens. Antibody based assays, such as ELISA and western blot, also can be used to determine protein expression. A preferred method of testing expression of cancer associated antigens (in other cancers and in additional same type cancer patients) is allogeneic serotyping using a modified SEREX protocol (as described above for gastric clones). -

In all of the foregoing, extracts from the tumors of patients who provided sera for the initial isolation of the cancer associated antigens are used as positive controls. The cells containing recombinant expression vectors described in the Examples above also can be used as positive controls.

The results generated from the foregoing experiments provide panels of multiple cancer associated nucleic acids and/or polypeptides for use in diagnostic (e.g. determining the existence of cancer, determining the prognosis of a patient undergoing therapy, etc.) and therapeutic methods (e.g., vaccine composition, etc.).

### Example 15: HLA typing of patients positive for cancer associated antigen

To determine which HLA molecules present peptides derived from the cancer associated antigens, cells of the patients which express the cancer associated antigens are HLA typed. Peripheral blood lymphocytes are taken from the patient and typed for HLA class I or class II, as well as for the particular subtype of class I or class II. Tumor biopsy samples also can be used for typing. HLA typing can be carried out by any of the standard methods in the art of clinical immunology, such as by recognition by specific monoclonal antibodies, or by HLA allele-specific PCR (e.g. as described in WO97/31126).

### Example 16: Characterization of breast cancer associated antigen peptides presented by MHC class I and class II molecules.

Antigens which provoke an antibody response in a subject may also provoke a cell-mediated immune response. Cells process proteins into peptides for presentation on MHC class I or class II molecules on the cell surface for immune surveillance. Peptides presented by certain MHC/HLA molecules generally conform to motifs. These motifs are known in some cases, and can be used to screen the breast cancer associated antigens for the presence of potential class I and/or class II peptides. Summaries of class I and class II motifs have been published (e.g., Rammensee et al., Immunogenetics 41:178-228, 1995). Based on the results of experiments such as those described in Example 15, the HLA types which present the individual breast cancer associated antigens are known. Motifs of peptides presented by these HLA molecules thus are preferentially searched.

One also can search for class I and class II motifs using computer algorithms. For example, computer programs for predicting potential CTL epitopes based on known class I motifs has been described (*see, e*.*g*., Parker et al, J. Immunol. 152:163, 1994; D'Amaro et al., Human Immunol. 43:1.3-18,1995; Drijthout et al., Human Immunol. 43:1-12,1995). HLA binding predictions can conveniently be made using an algorithm available via the Internet on the National Institutes of Health World Wide Web site at URL http://bimas.dcrt.nih.gov. Methods for determining HLA class II peptides and making substitutions thereto are also known (e.g. Strominger and Wucherpfennig (WO 96/27387)).

The lung cancer SEREX clone polypeptides NY-LU-12 and NY-LU-12B (variant B), SEQ ID NOs: 708 and 710, were subjected to the HLA binding peptide analysis described above, using the NIH website, to identify HLA binding peptides for several common HLA molecules (HLA-A1, A2, A3, A24, B7, B44, and B52). The results are listed below in Table 12.

**Table 12: Identification of HLA binding peptides in lung SEREX clones**

| | | amino acids of | |
|---|---|---|---|
| HLA | peptide | NY-LU-12 protein | SEQ ID NO |
| A1 | NVEE-HSFSY | 67 - 75 | 713 |
| | PVDP-NILDY | 287 - 295 | 714 |
| | DTDY-RSMEY | 398 - 406 | 715 |
| | | | |
| A2 | SLLE-DAIGC | 506 - 514 | 716 |
| | TLMI-QDKEV | 521 - 529 | 717 |
| | YVSSLDFWYC | 533 - 542 | 718 |
| | VIVEVLEPYV | 671 - 680 | 719 |
| | KLTD-WNKLA | 948 - 956 | 720 |
| | QLSDLHKQNL | 975 - 984 | 721 |
| | KQSEQELAYL | 991 - 1000 | 722 |
| | KLVDKEDIDT | 1042 - 1051 | 723 |
| | VMFA-RYKEL | 1114 - 1122 | 724 |
| | | | |
| A3 | QMFG-YGQSK | 417 - 425 | 725 |
| | GMPVKNLQLK | 481 - 490 | 726 |
| | GLPE-EEEIK | 823 - 831 | 727 |
| | LLCRRQFPNK | 958 - 967 | 728 |
| | | | |
| A24 | EYRD-VDHRL | 405 - 413 | 729 |
| | GYVC-VEFSL | 499 - 507 | 730 |
| | DYGY-VCVEF | 497 - 505 | 731 |
| | WYCKRCKANI | 540 - 549 | 732 |
| | TYPQPQKTSI | 574 - 583 | 733 |
| | IYRSTPPEVI | 663 - 672 | 734 |
| | HYYQ-GKKYF | 754 - 762 | 735 |
| | VYVP-QDPGL | 816 - 824 | 736 |
| | | | |
| B7 | WNRDYPPPPL | 26 - 35 | 737 |
| | MPPV-DPNIL | 285 - 293 | 738 |
| | TARD-AQRDL | 432 - 440 | 739 |
| | GPSEEKPSRL | 448 - 457 | 740 |
| | TPPEVIVEVL | 667 - 676 | 741 |
| | RVMFARYKEL | 1113 - 1122 | 742 |
| | | | |
| B44 | REMG-SCMEF | 272 - 280 | 743 |
| | EEQSSDAGLF | 376 - 385 | 744 |
| | KEYN-TGYDY | 490 - 498 | 745 |
| | TEAKQELITY | 566 - 575 | 746 |
| | VEALRVVKIL | 710 - 719 | 747 |
| | GEYG-GDSDY | 906 - 914 | 748 |
| | LERREREGKF | 1000 - 1009 | 749 |
| | | | |
| B52 | RQDGESKTIM | 650 - 659 | 750 |
| | TPPEVIVEVL | 667 - 676 | 751 |
| | YGFIDLDSHV | 701 - 710 | 752 |
| | RQFP-NKEVL | 962 - 970 | - 753 |
| | | | |

| **NY-LU-12B (variant B)** | | | |
|---|---|---|---|
| A1 | NVEE-HSFSY | 121 - 129 | 754 |
| | PVDP-NILDY | 341 - 349 | 755 |
| | DTDY-RSMEY | 452 - 460 | 756 |
| | | | |
| A2 | WQSA-RFYYL | 41 - 49 | 757 |
| | SLLE-DAIGC | 560 - 568 | 758 |
| | TLMI-QDKEV | 575 - 583 | 759 |
| | YVSSLDFWYC | 587 - 596 | 760 |
| | VIVEVLEPYV | 725 - 734 | 761 |
| | KLTD-WNKLA | 1002 - 1010 | 762 |
| | QLSDLHKQNL | 1029 - 1038 | 763 |
| | KQSEQELAYL | 1045 - 1054 | 764 |
| | KLVDKEDIDT | 1096 - 1105 | 765 |
| | VMFA-RYKEL | 1168 - 1176 | 766 |
| | | | |
| A3 | QMFG-YGQSK | 471 - 479 | 767 |
| | GMPVKNLQLK | 535 - 544 | 768 |
| | GLPE-EEEIK | 877 - 885 | 769 |
| | LLCRRQFPNK | 1012 - 1021 | 770 |
| | | | |
| A24 | YYLN-ATDVL | 47 - 55 | 771 |
| | FYYLNATDVL | 46 - 55 | 772 |
| | EYRD-VDHRL | 459 - 467 | 773 |
| | GYVC-VEFSL | 553 - 561 | 774 |
| | DYGY-VCVEF | 551 - 559 | 775 |
| | WYCKRCKANI | 594 - 603 | 776 |
| | TYPQPQKTSI | 628 - 637 | 777 |
| | IYRSTPPEVI | 717 - 726 | 778 |
| | HYYQ-GKKYF | 808 - 816 | 779 |
| | VYVP-QDPGL | 870 - 878 | 780 |
| | | | |
| B7 | WNRDYPPPPL | 80 - 89 | 781 |
| | MPPV-DPNIL | 339 - 347 | 782 |
| | TARD-AQRDL | 486 - 494 | 783 |
| | GPSEEKPSRL | 502 - 511 | 784 |
| | TPPEVIVEVL | 721 - 730 | 785 |
| | RVMFARYKEL | 1167 - 1176 | 786 |
| | | | |
| B44 | SEAWSSNEKF | 59 - 68 | 787 |
| | REMG-SCMEF | 326 - 334 | 788 |
| | EEQSSDAGLF | 430 - 439 | 789 |
| | KEYN-TGYDY | 544 - 552 | 790 |
| | TEAKQELITY | 620 - 629 | 791 |
| | VEALRWKIL | 764 - 773 | 792 |
| | GEYG-GDSDY | 960 - 968 | 793 |
| | LERREREGKF | 1054 - 1063 | 794 |
| | | | |
| B52 | RQDGESKTIM | 704 - 713 | 795 |
| | TPPEVIVEVL | 721 - 730 | 796 |
| | YGFIDLDSHV | 755 - 764 | 797 |
| | RQFP-NKEVL | 1016 - 1024 | 798 |

Likewise, other clones identified herein can be analyzed for the presence of candidate HLA binding peptides using no more than routine experimentation.

### Example 17: Identification of the portion of a cancer associated polypeptide encoding an antigen

To determine if the cancer associated antigens isolated as described above can provoke a cytolytic T lymphocyte response, the following method is performed. CTL clones are generated by stimulating the peripheral blood lymphocytes (PBLs) of a patient with autologous normal cells transfected with one of the clones encoding a cancer associated antigen polypeptide or with irradiated PBLs loaded with synthetic peptides corresponding to the putative protein and matching the consensus for the appropriate HLA class I molecule (as described above) to localize an antigenic peptide within the cancer associated antigen clone (*see*, e.g., Knuth et al., Proc. Natl. Acad. Sci. USA 81:3511-3515, 1984; van der Bruggen et al., Eur. J Immunol.24:3038-3043, 1994). These CTL clones are screened for specificity against COS cells transfected with the cancer associated antigen clone and autologous HLA alleles as described by Brichard et al. (Eur. J Immunol. 26:224-230, 1996). CTL recognition of a cancer associated antigen is determined by measuring release of TNF from the cytolytic T lymphocyte or by ⁵¹Cr release assay (Herin et al., Int. J. Cancer 39:390-396, 1987). If a CTL clone specifically recognizes a transfected COS cell, then shorter fragments of the cancer associated antigen clone transfected in that COS cell are tested to identify the region of the gene that encodes the peptide. Fragments of the cancer associated antigen clone are prepared by exonuclease III digestion or other standard molecular biology methods. Synthetic peptides are prepared to confirm the exact sequence of the antigen.

Optionally, shorter fragments of cancer associated antigen cDNAs are generated by PCR. Shorter fragments are used to provoke TNF release or ⁵¹Cr release as above.

Synthetic peptides corresponding to portions of the shortest fragment of the cancer associated antigen clone which provokes TNF release are prepared. Progressively shorter peptides are synthesized to determine the optimal cancer associated antigen tumor rejection antigen peptides for a given HLA molecule.

A similar method is performed to determine if the cancer associated antigen contains one or more HLA class II peptides recognized by CTLs. One can search the sequence of the cancer associated antigen polypeptides for HLA class II motifs as described above. In contrast to class I peptides, class II peptides are presented by a limited number of cell types. Thus for these experiments, dendritic cells or B cell clones which express HLA class II molecules preferably are used.

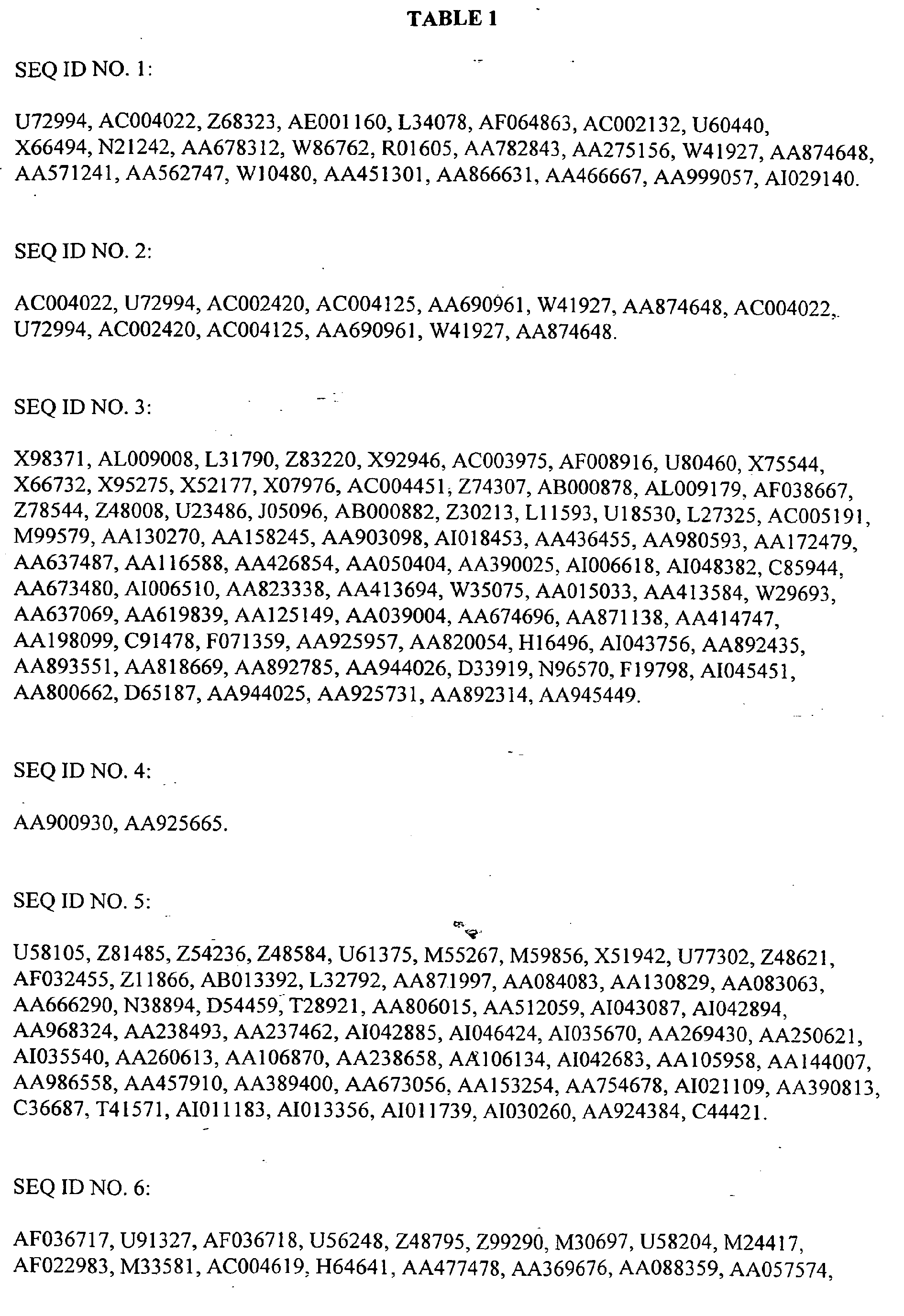

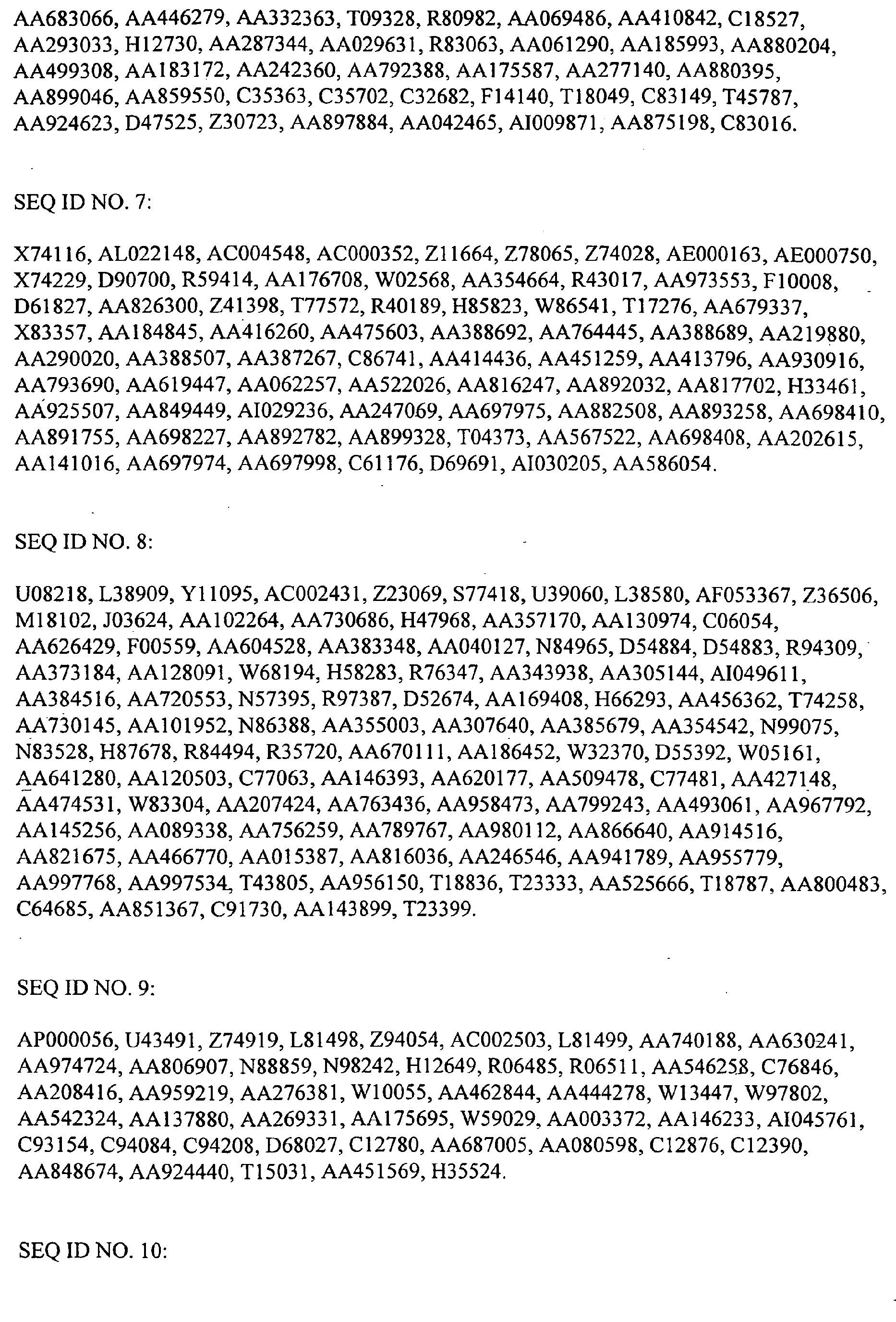

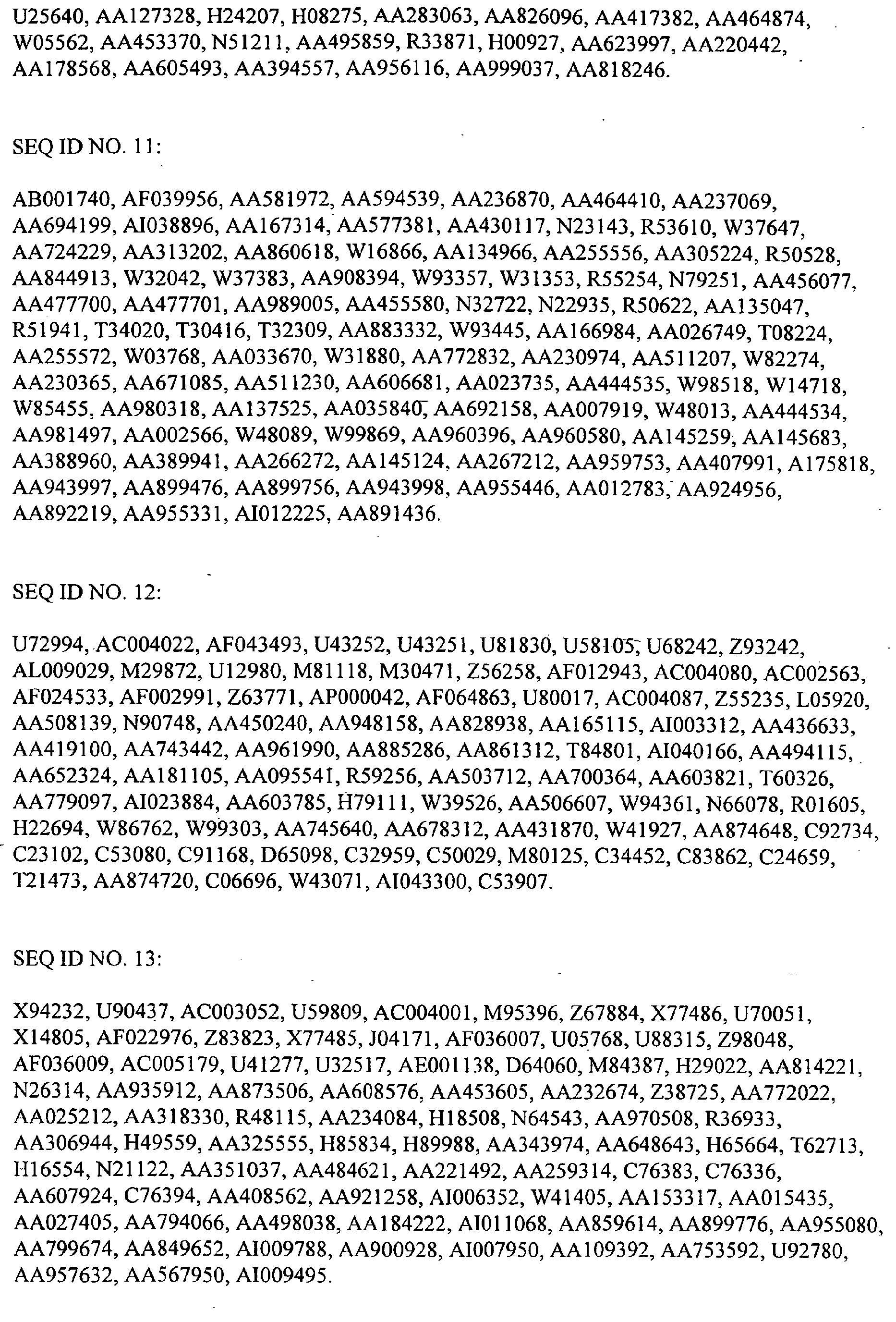

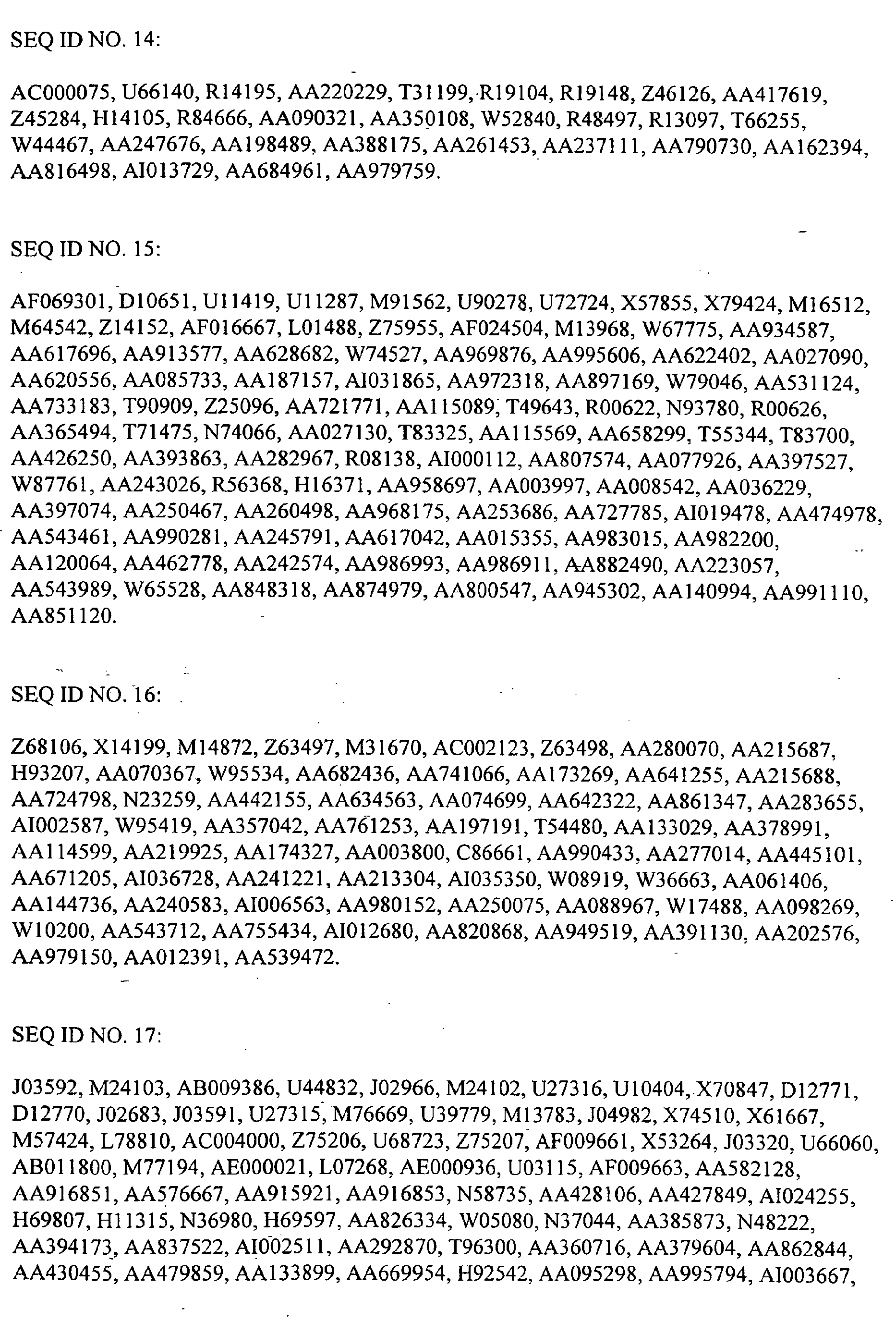

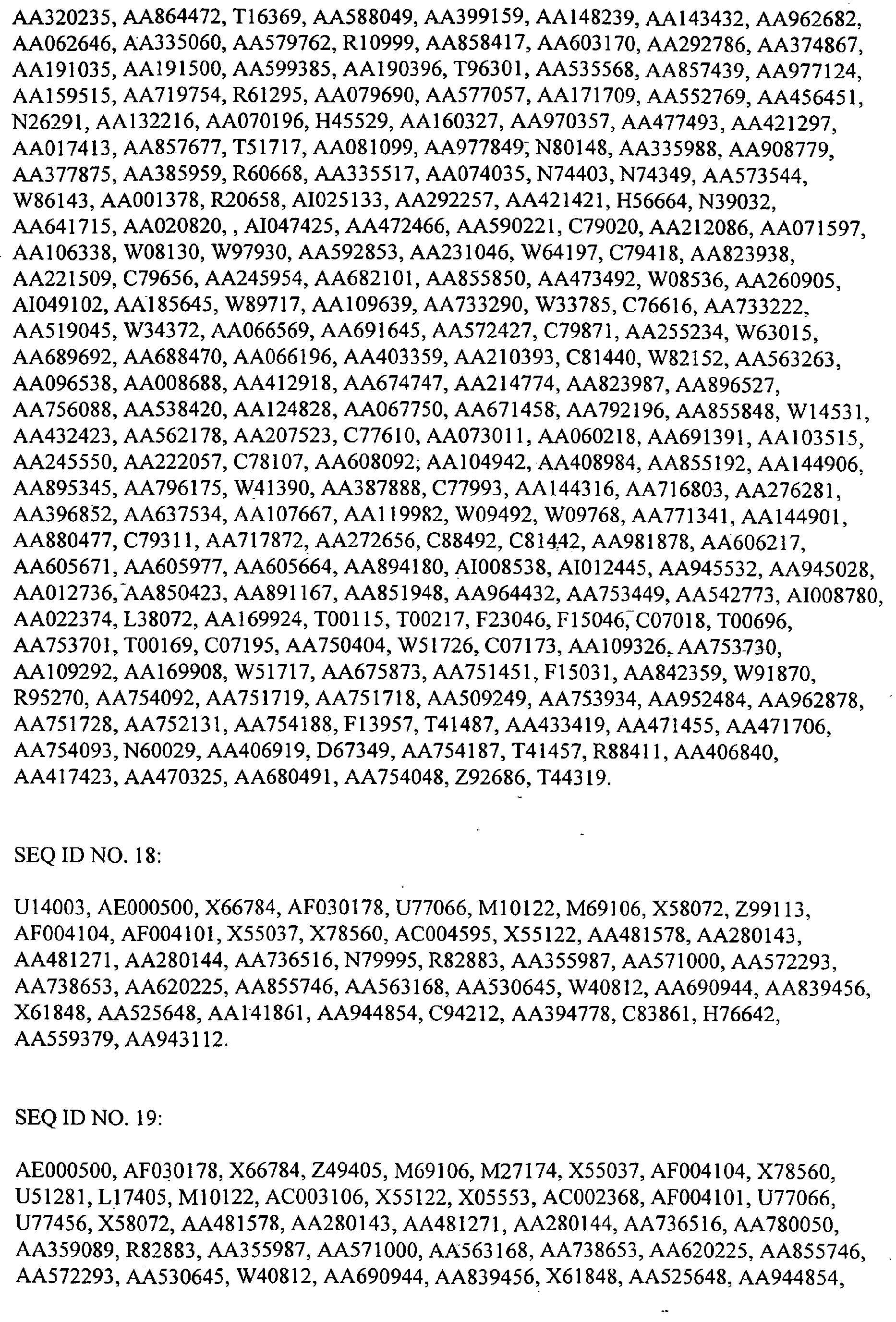

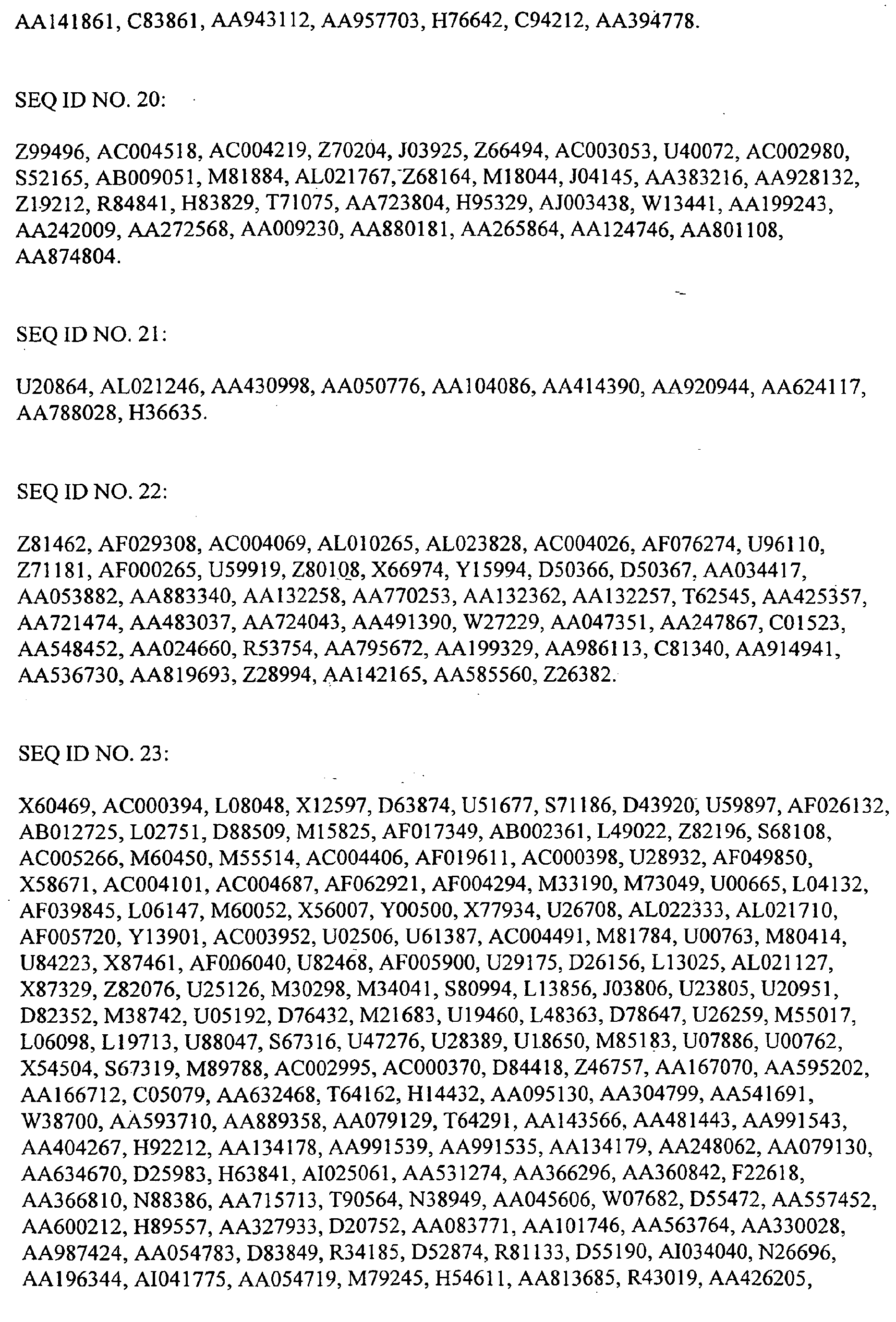

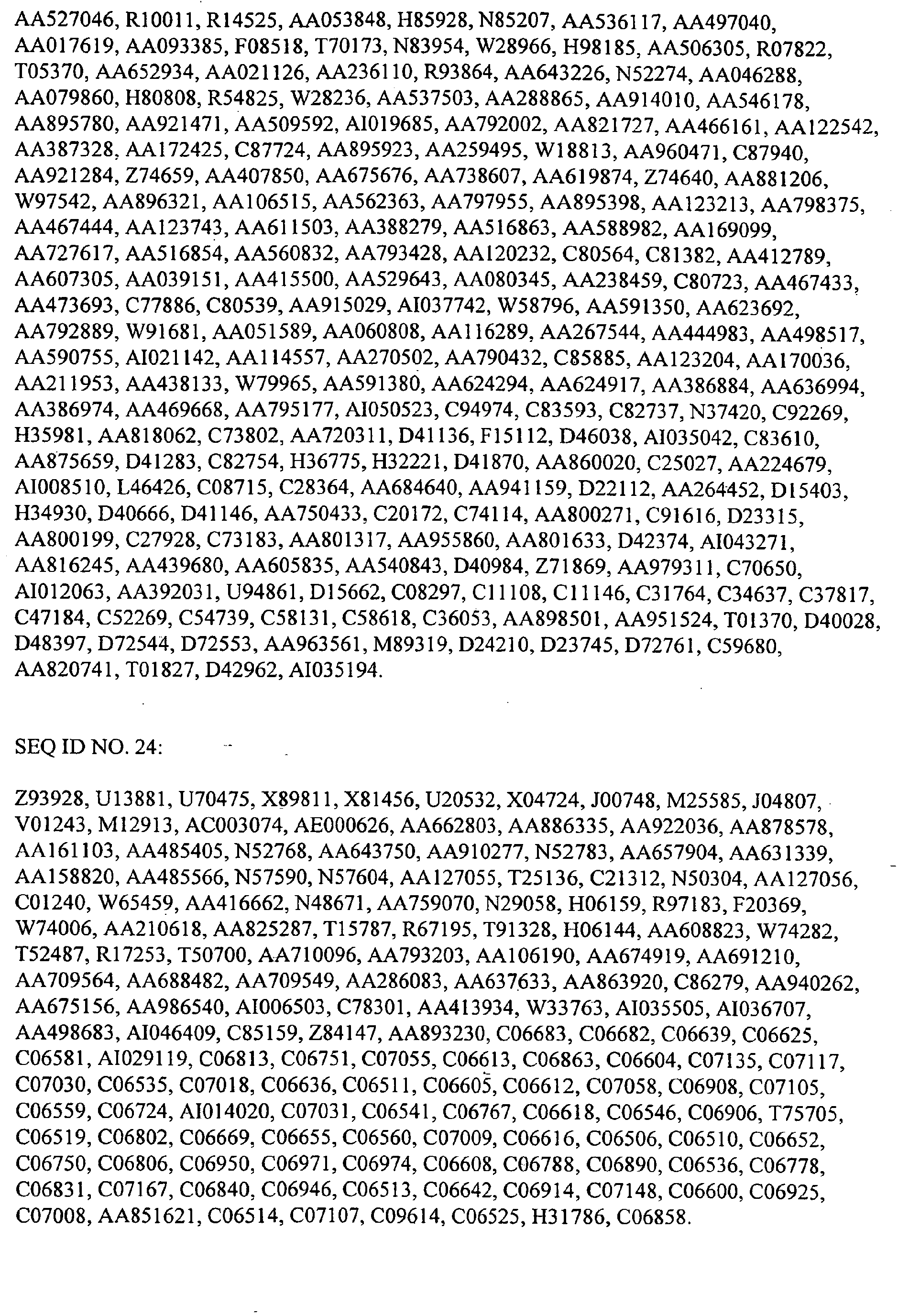

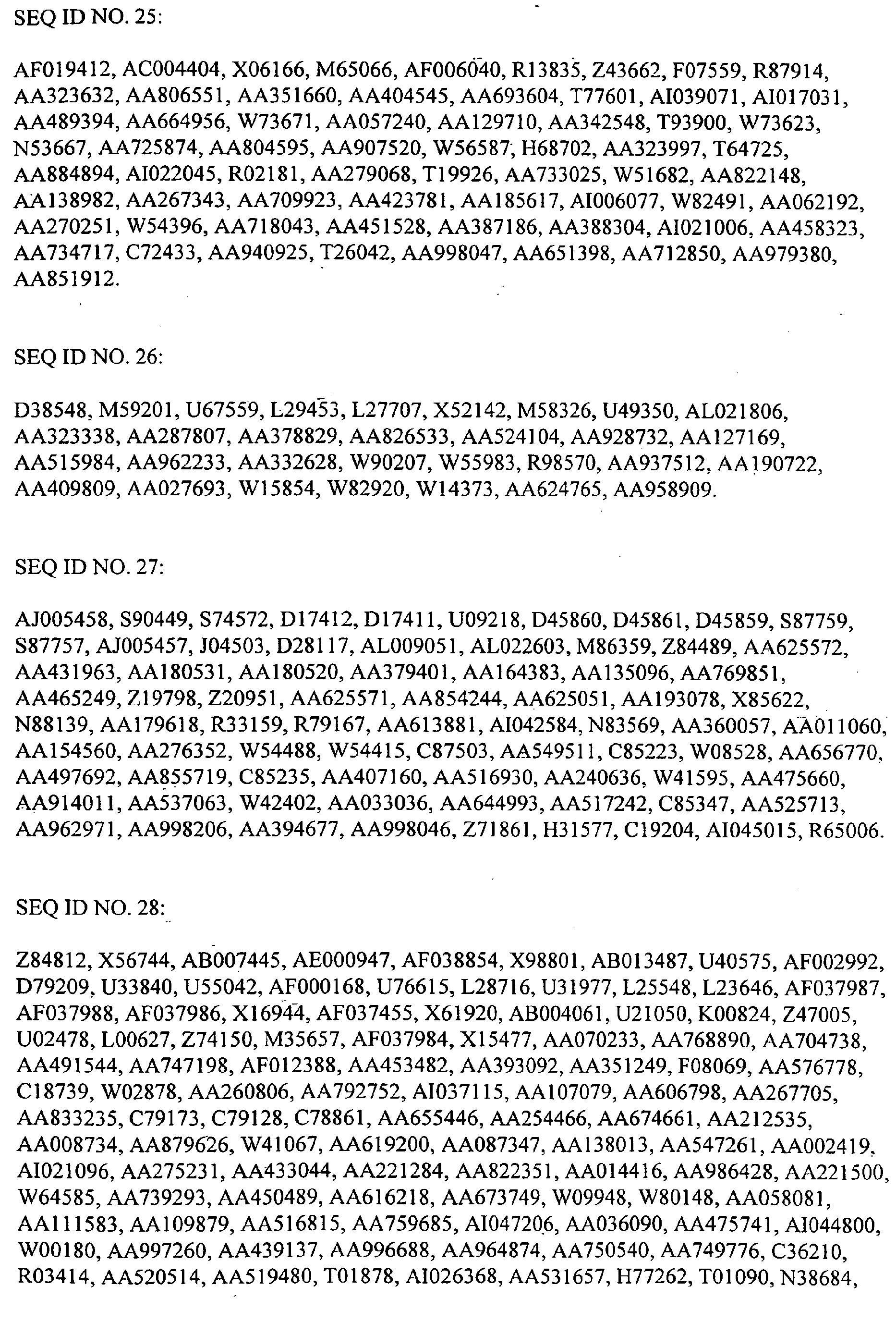

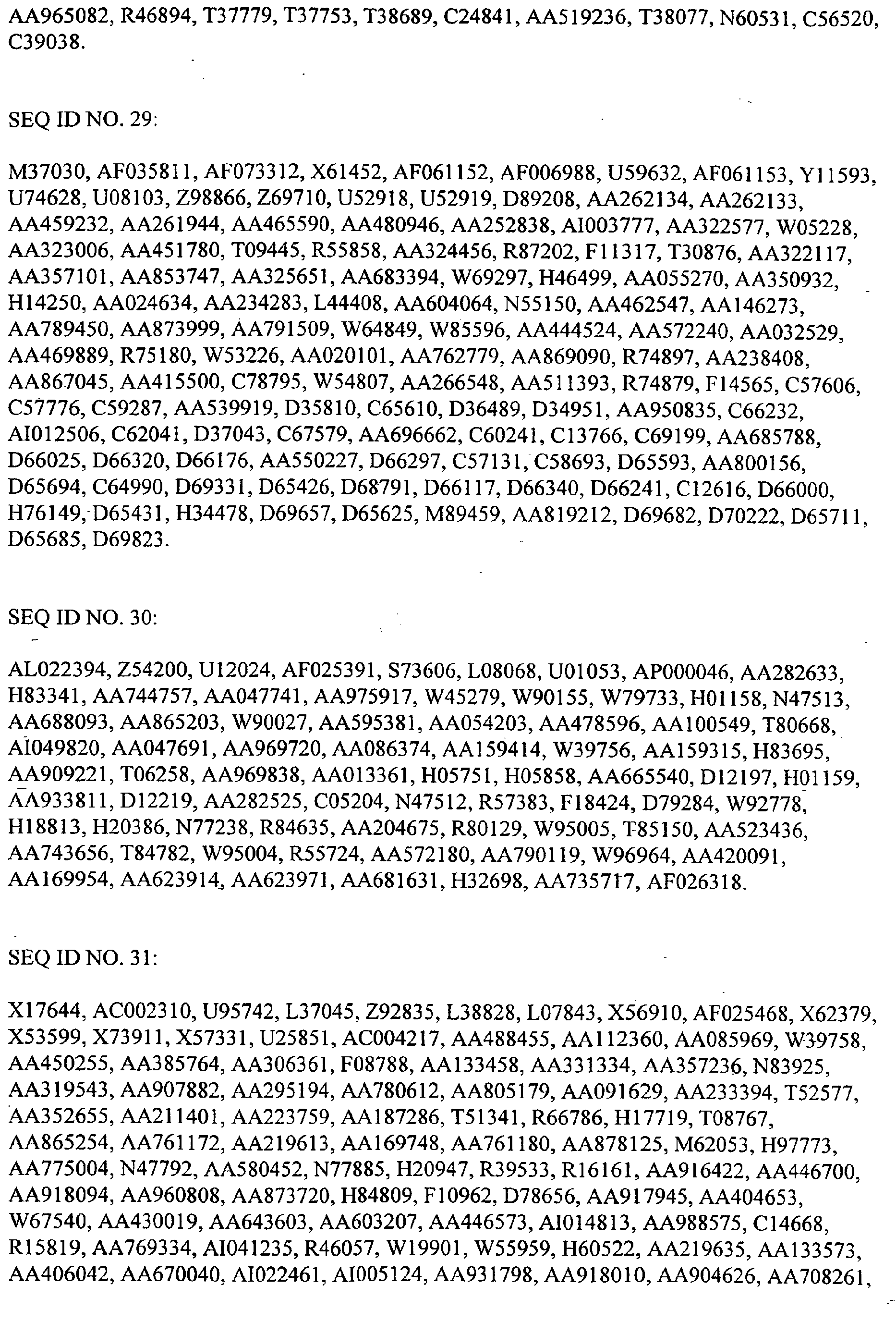

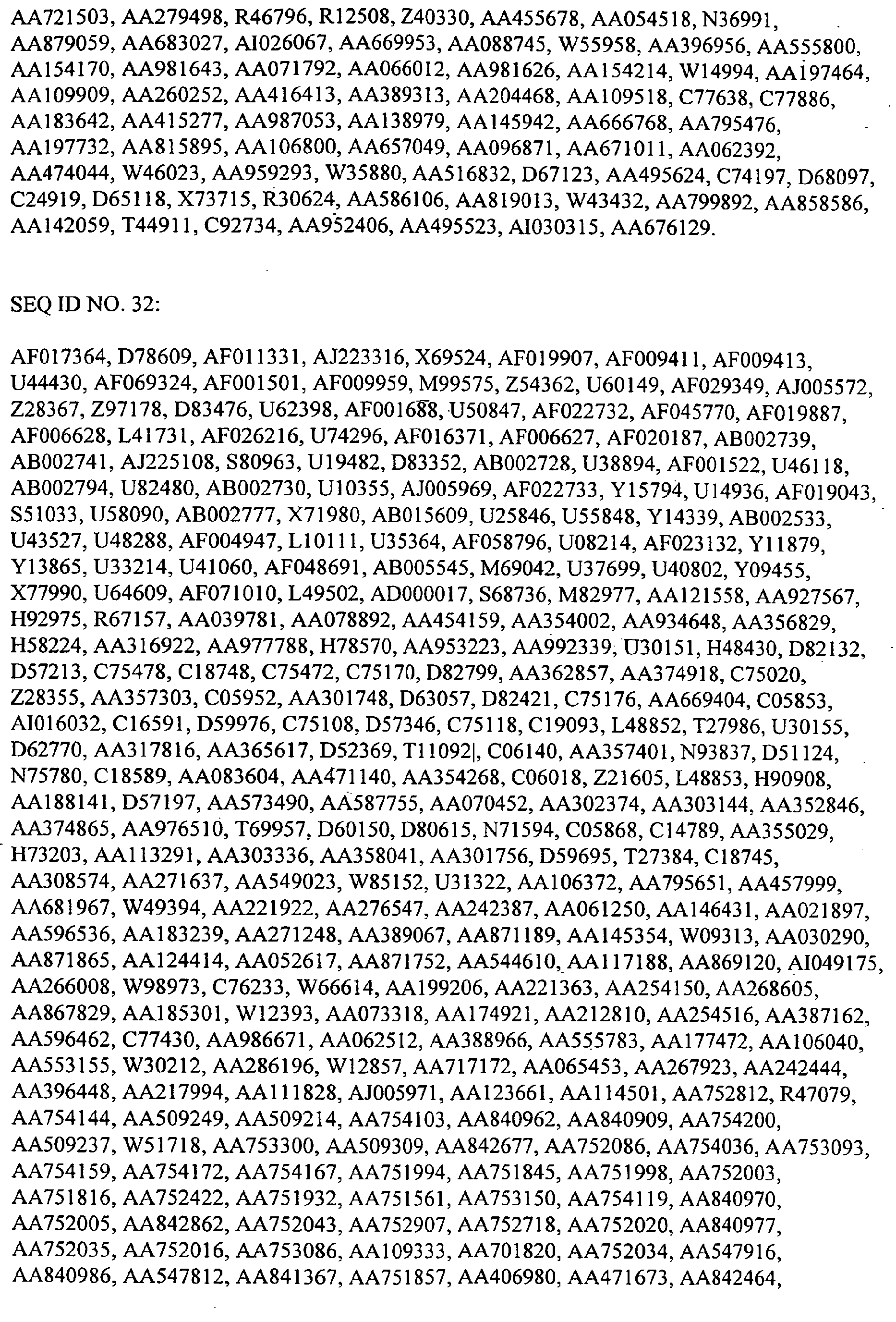

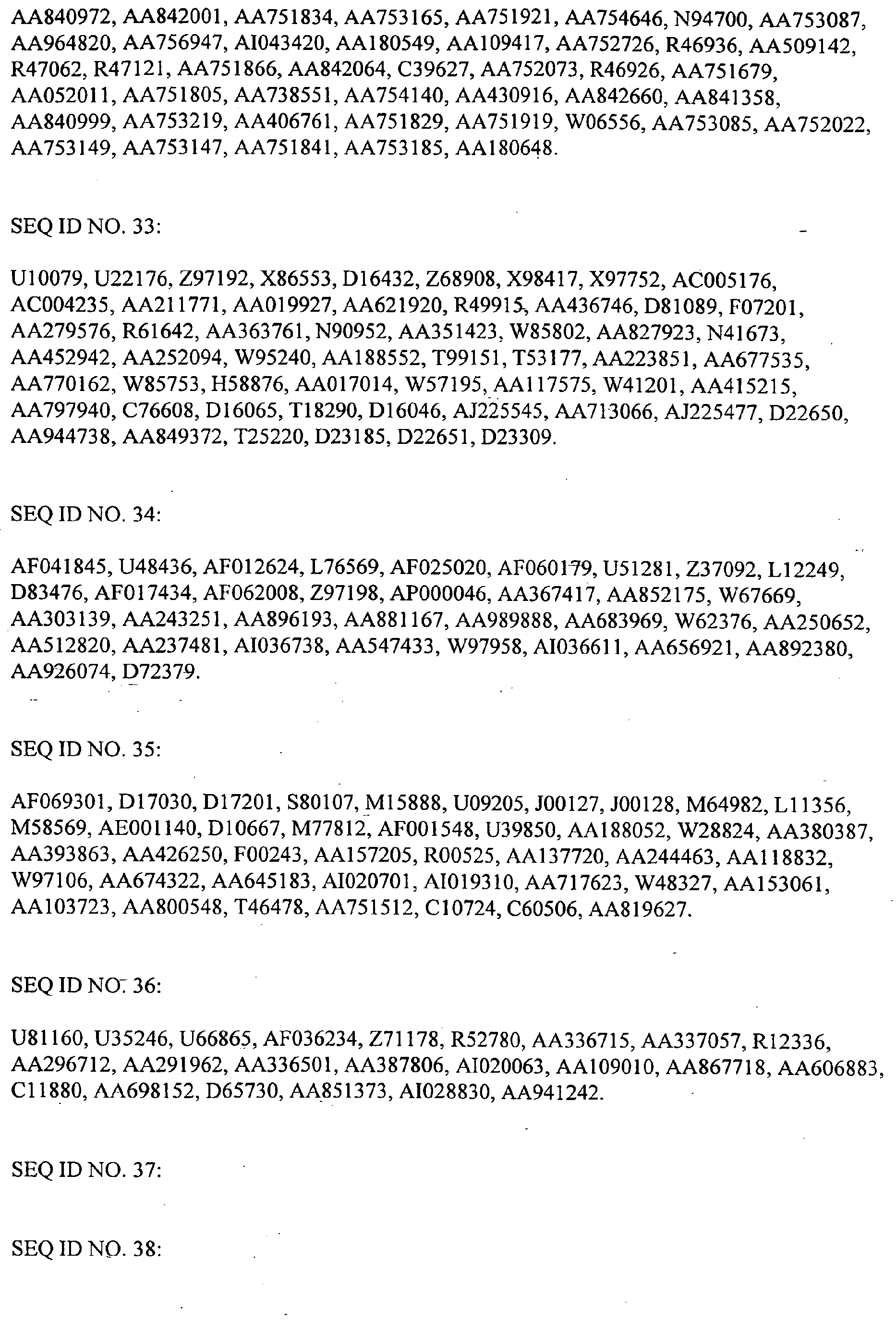

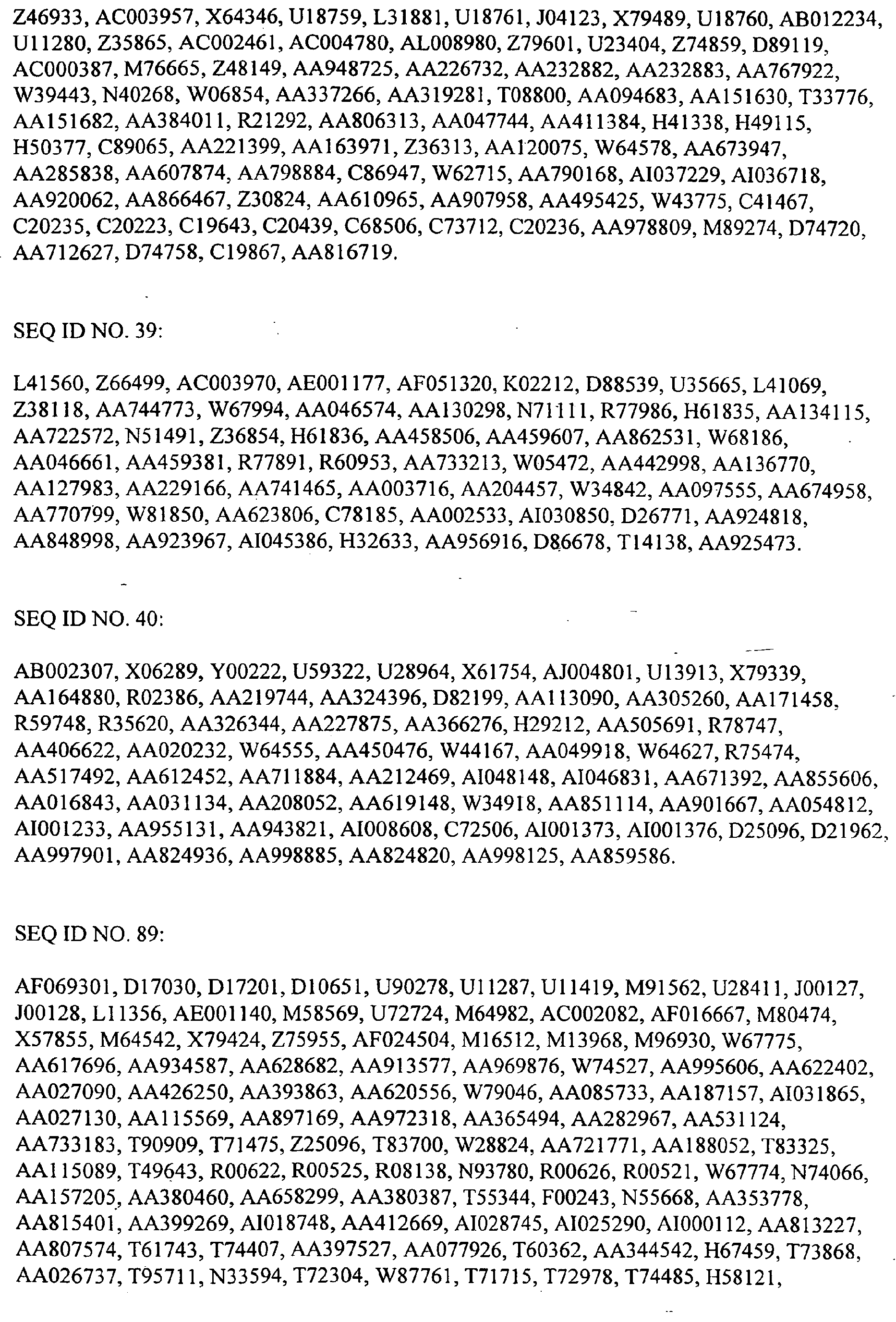

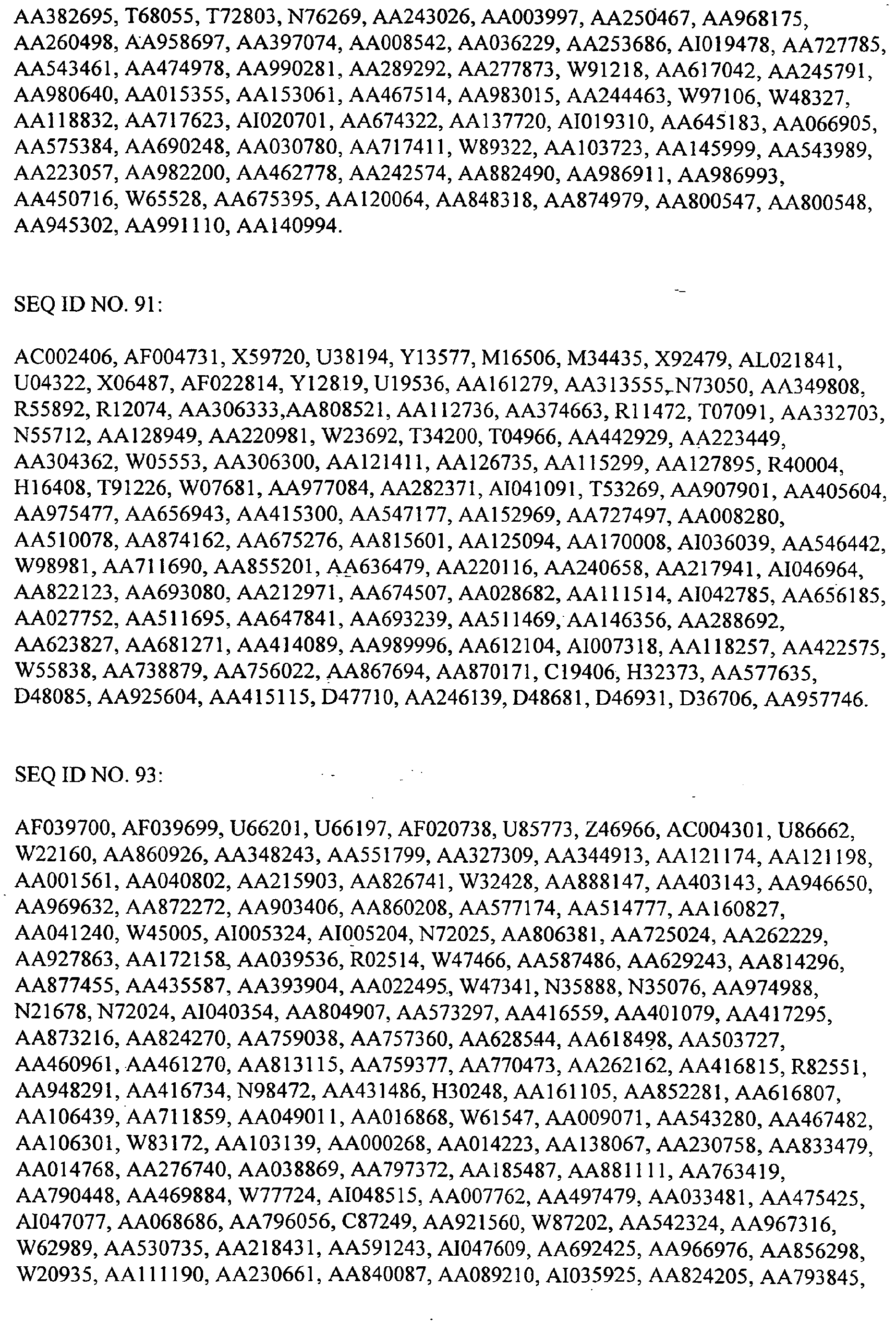

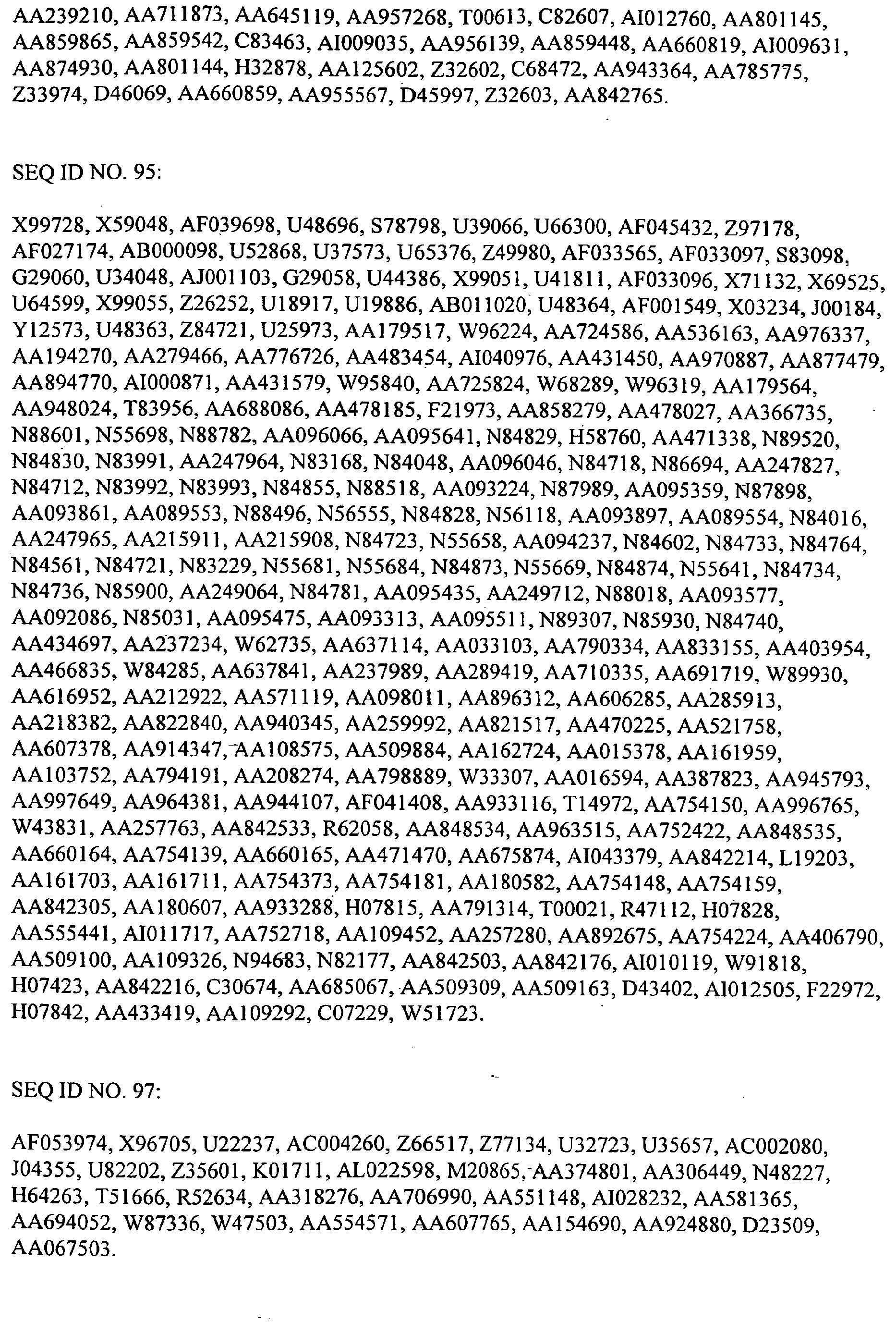

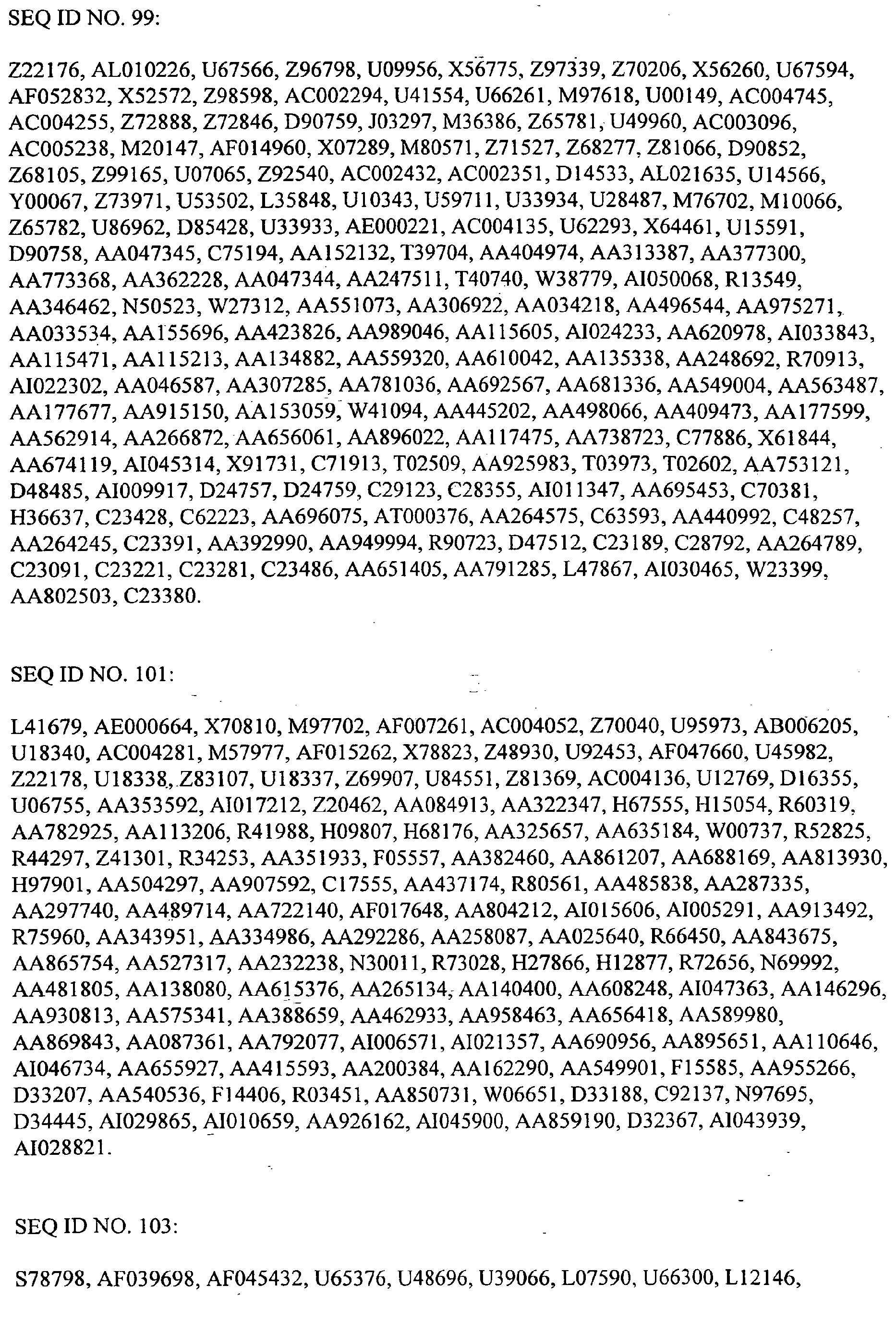

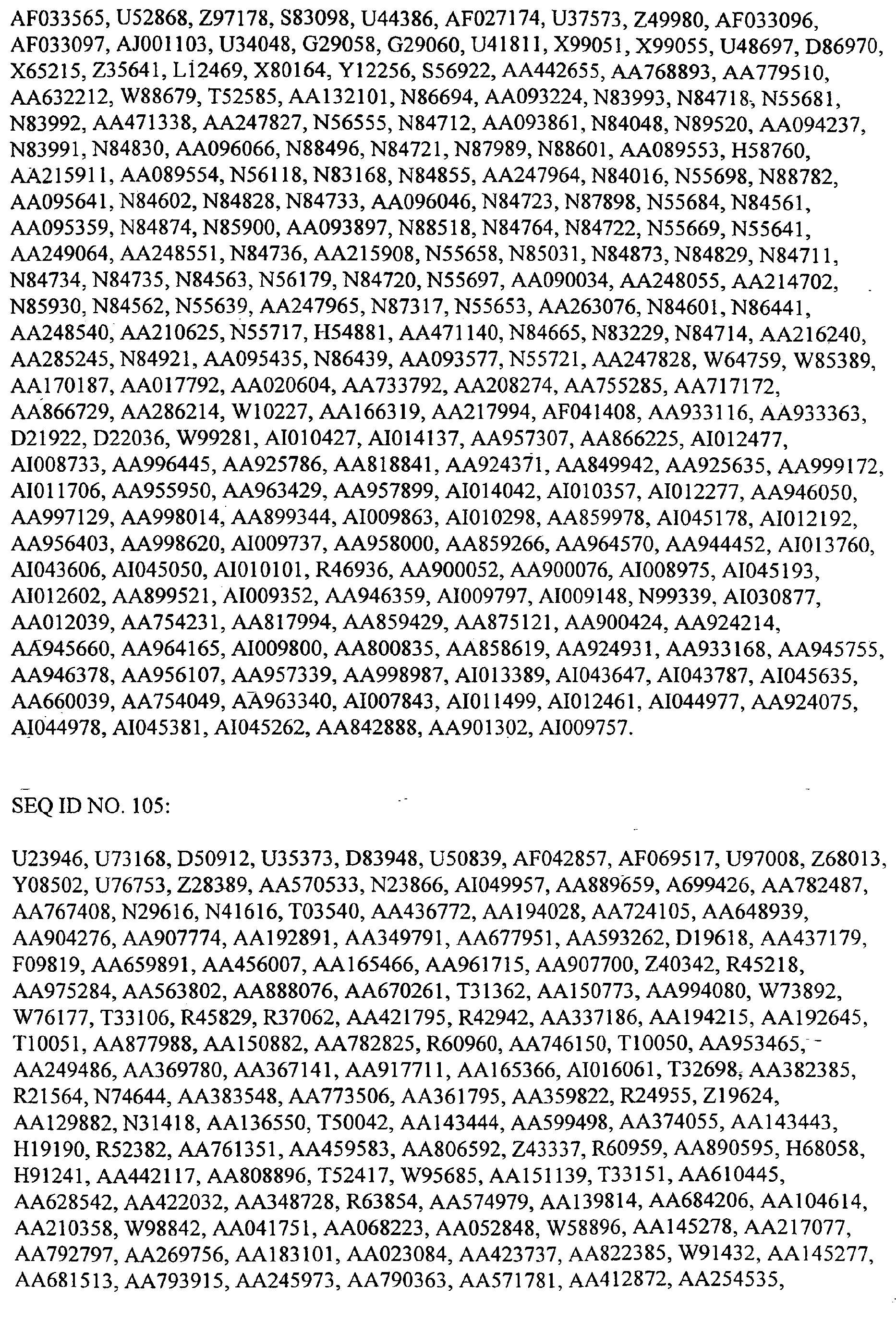

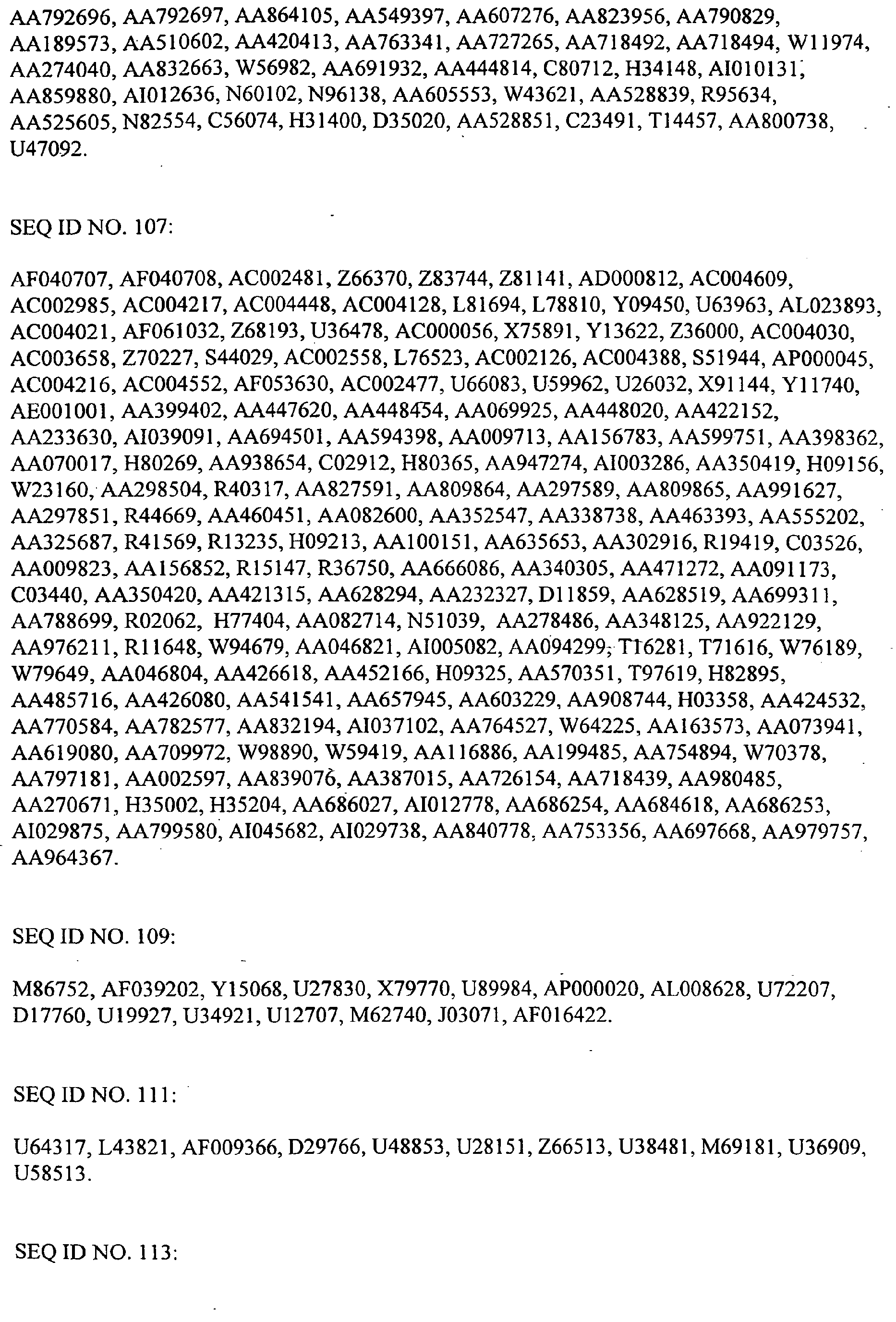

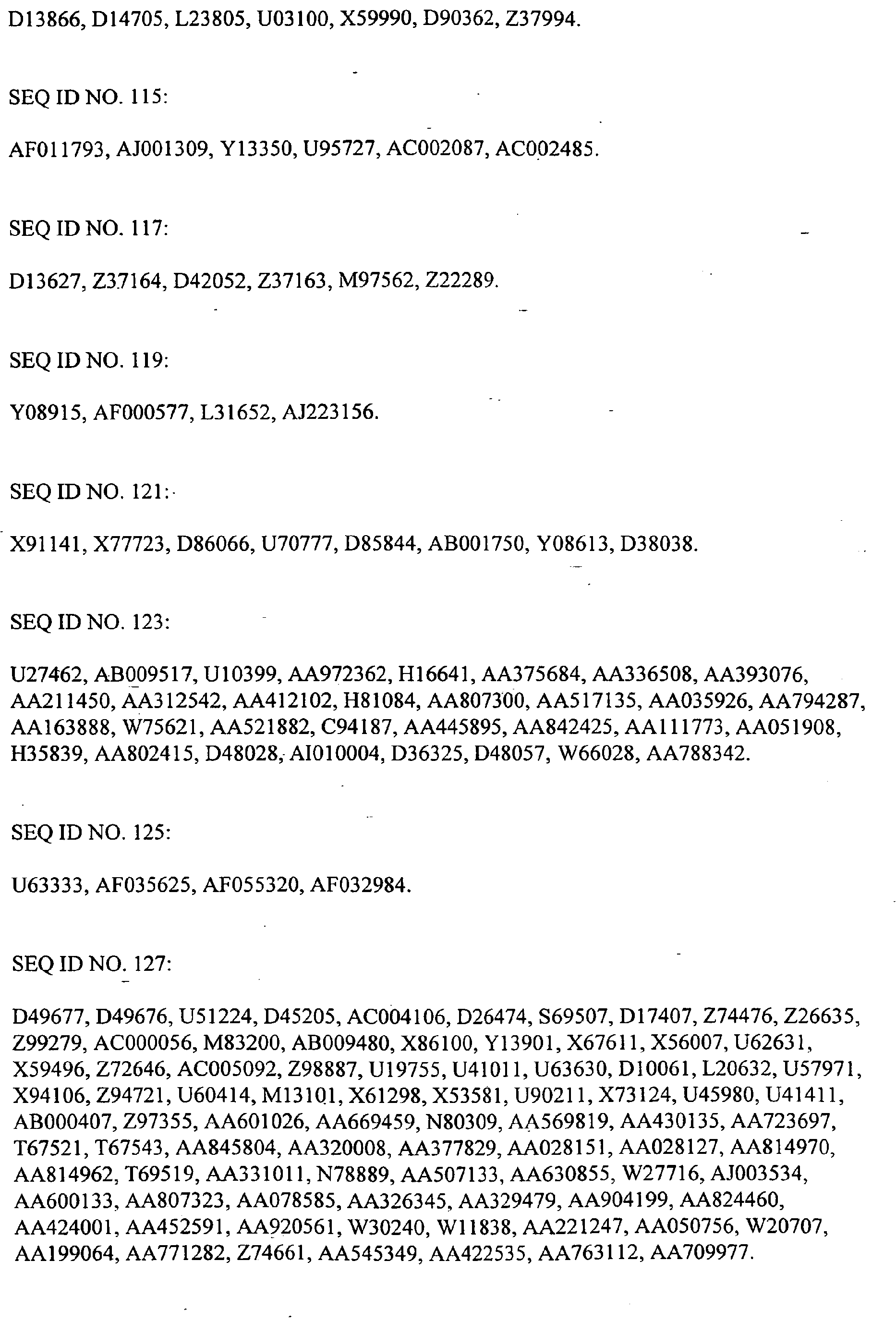

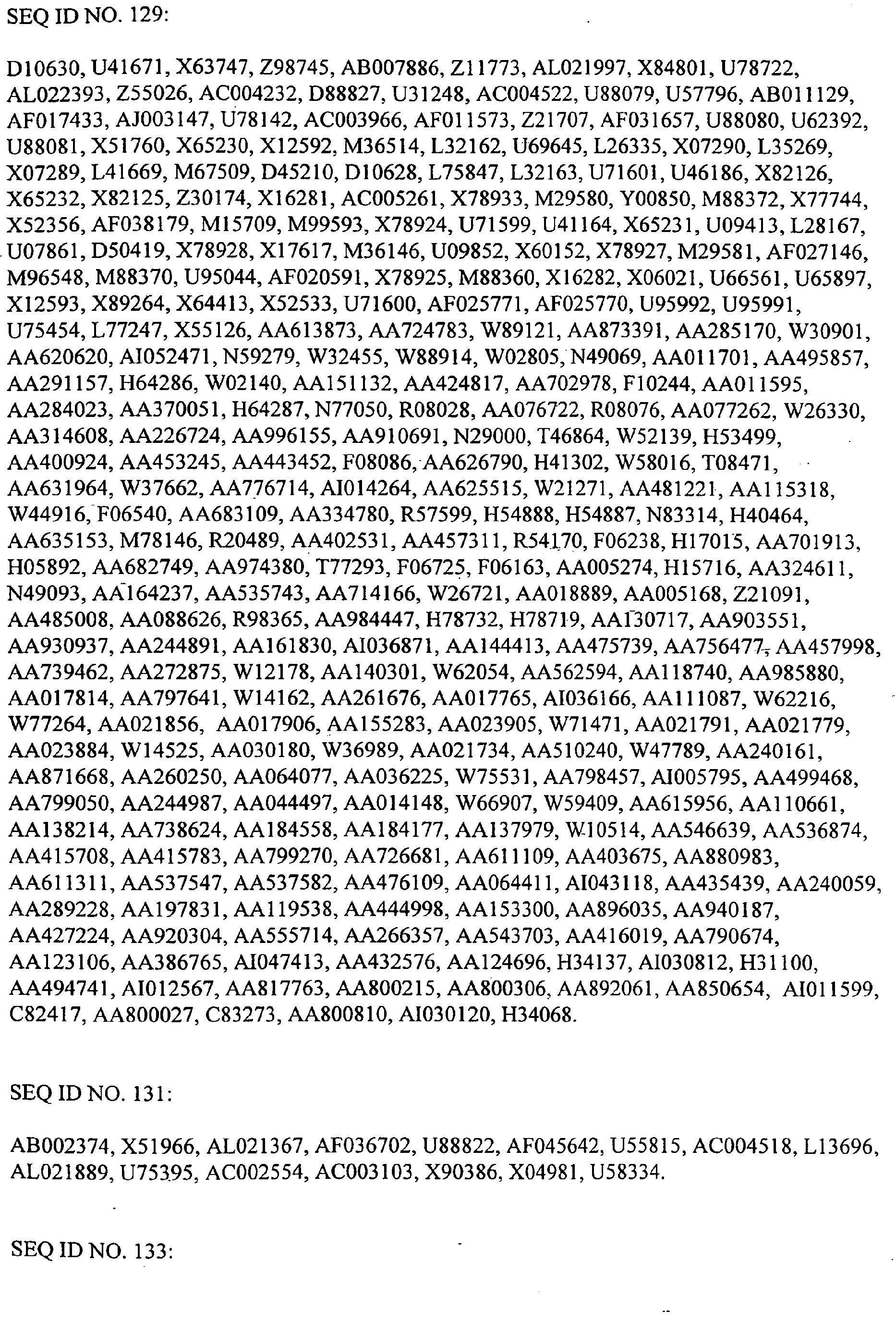

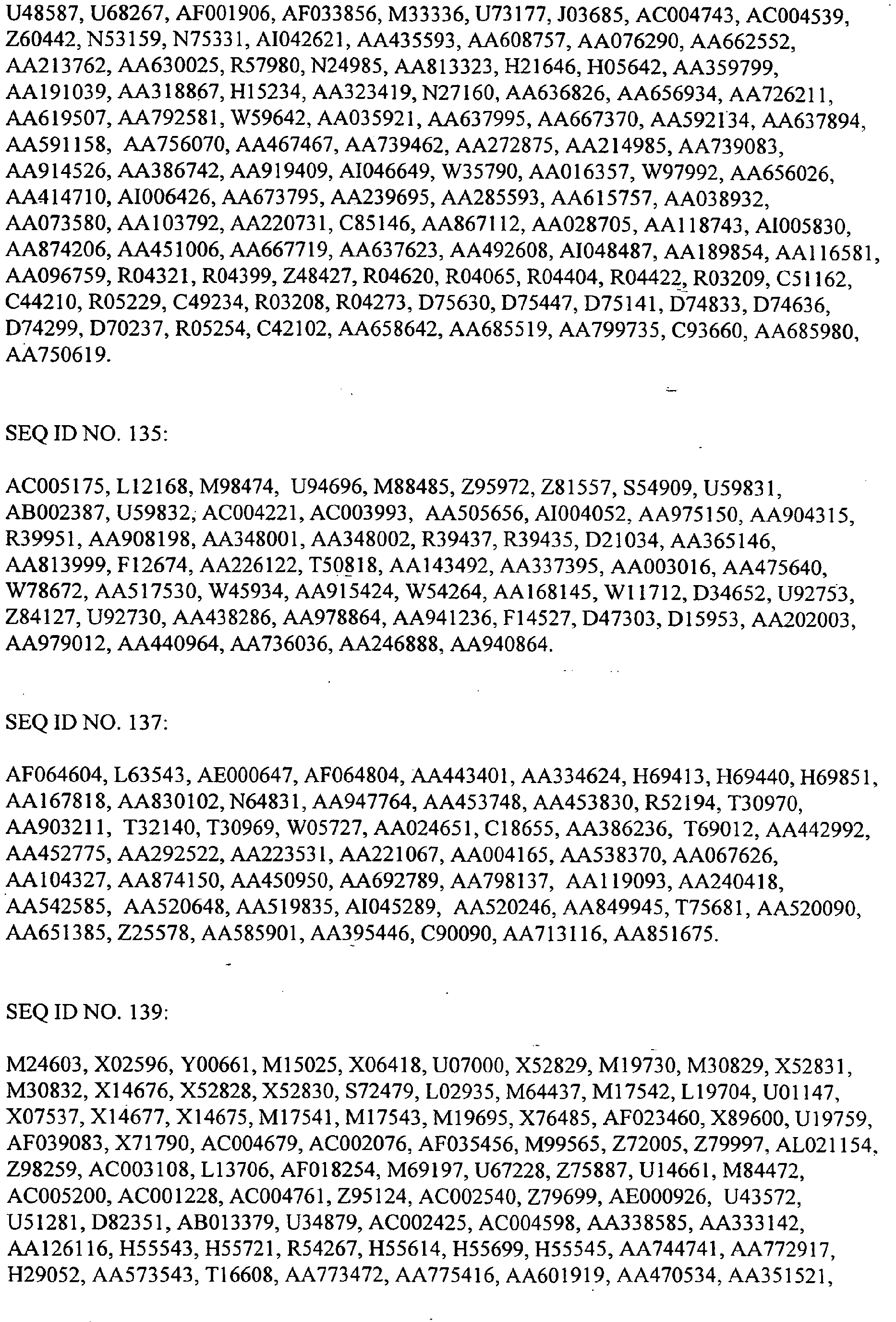

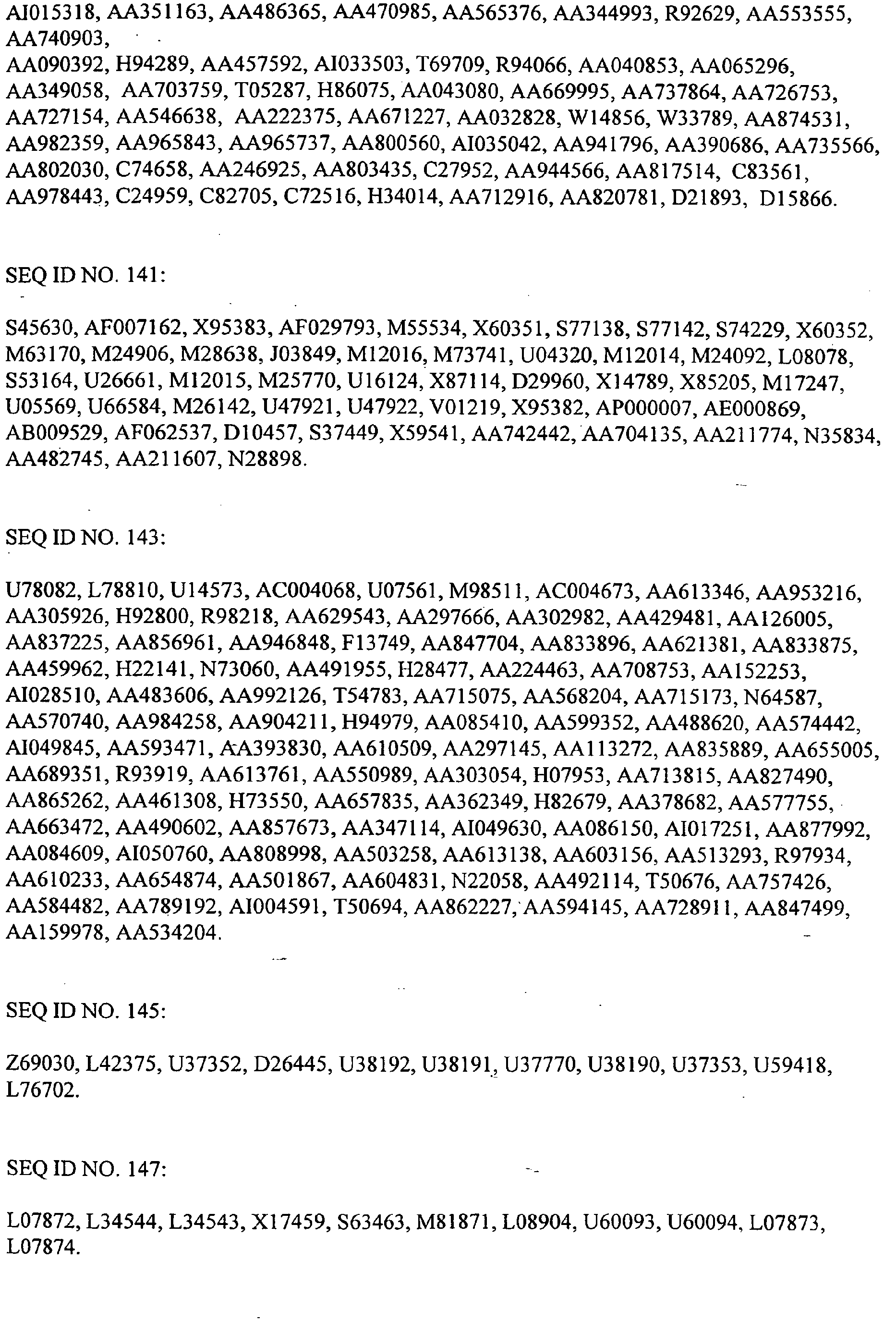

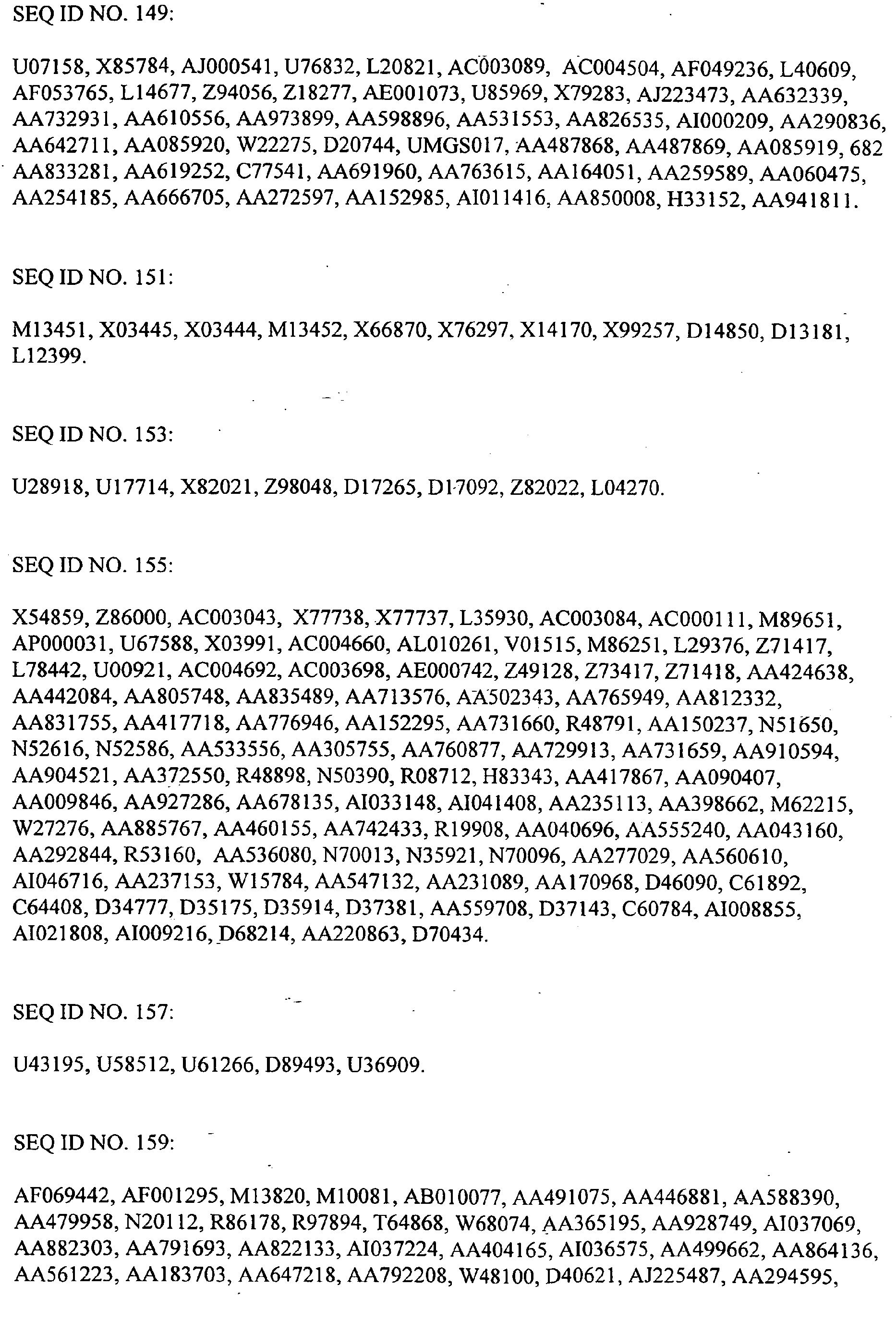

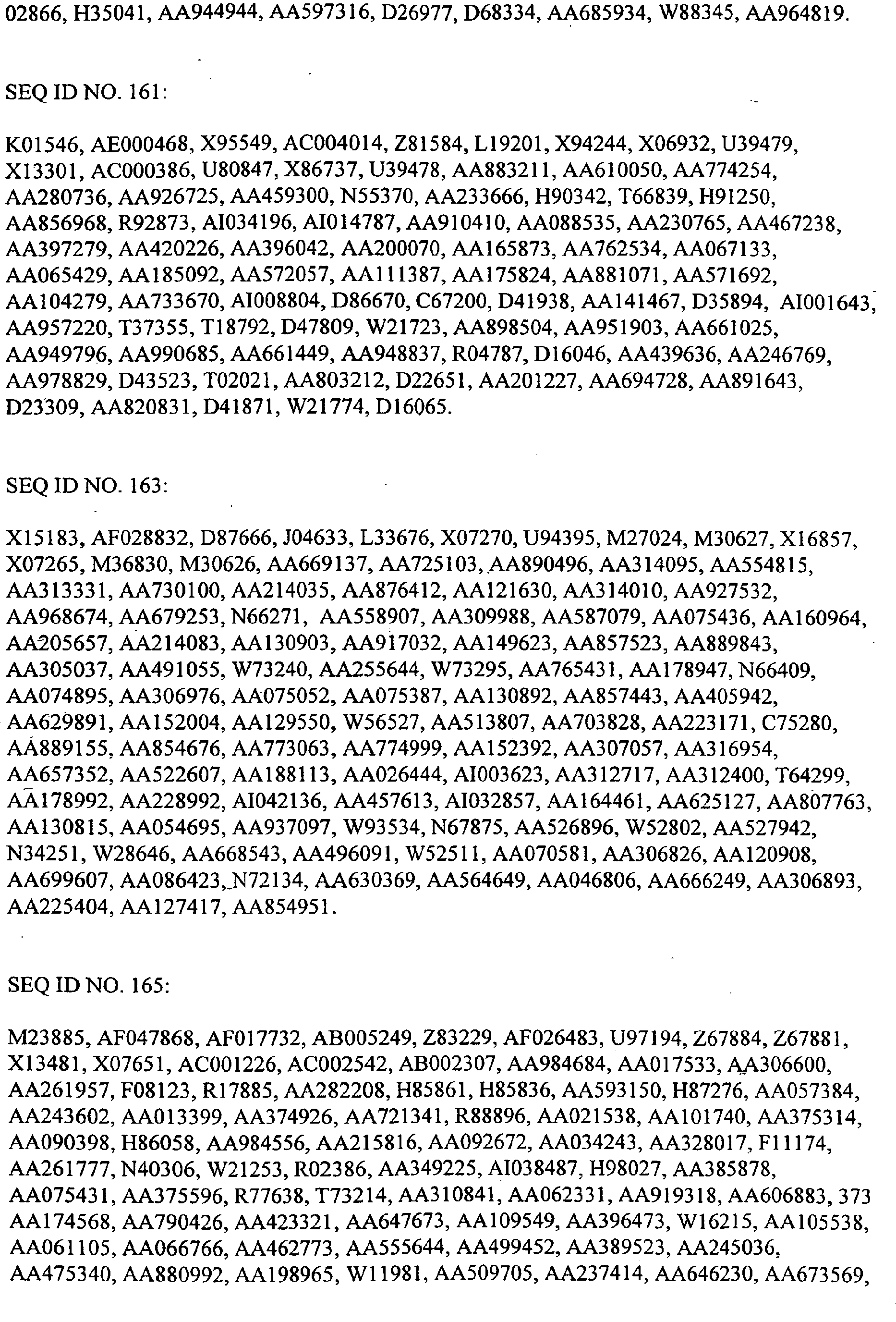

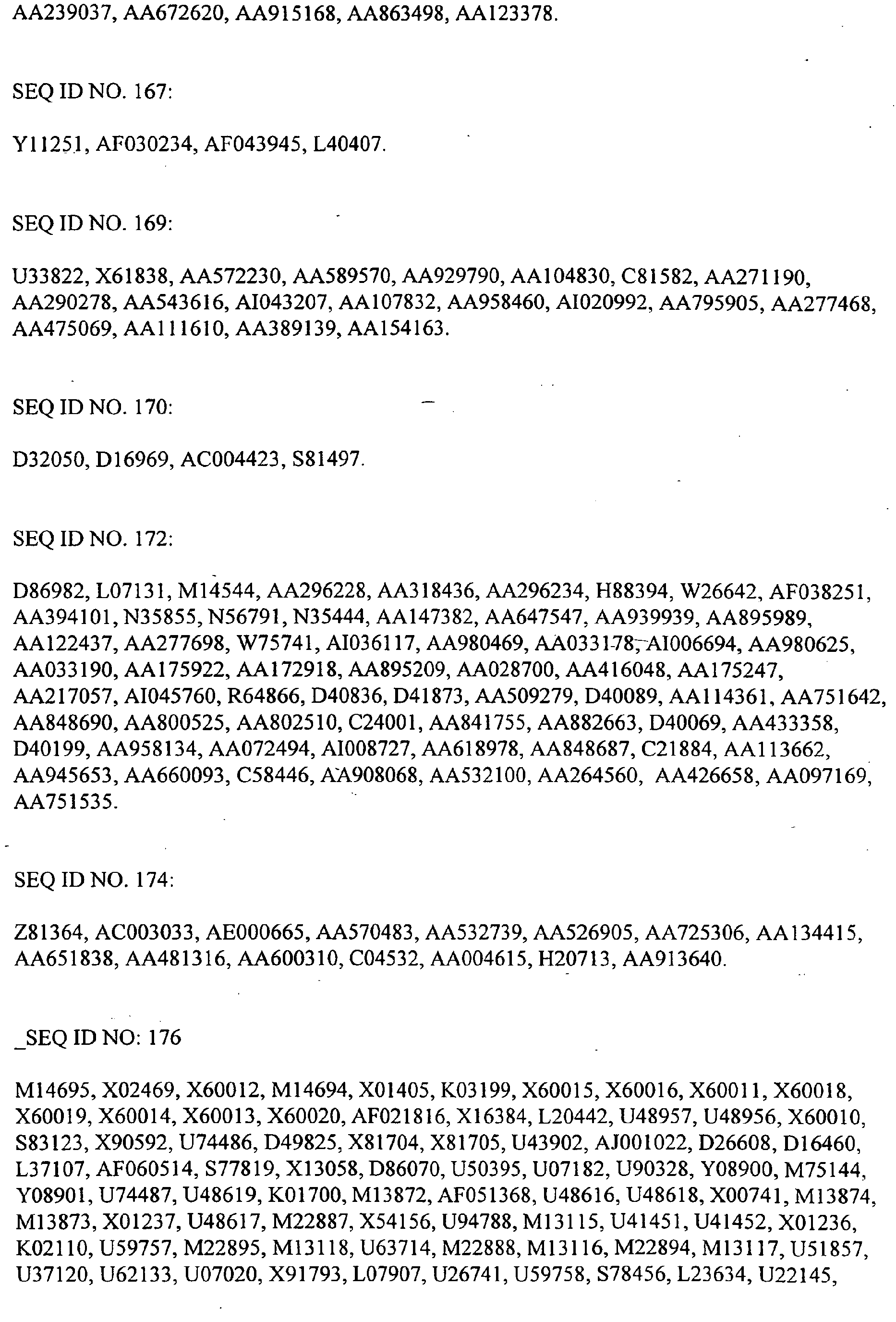

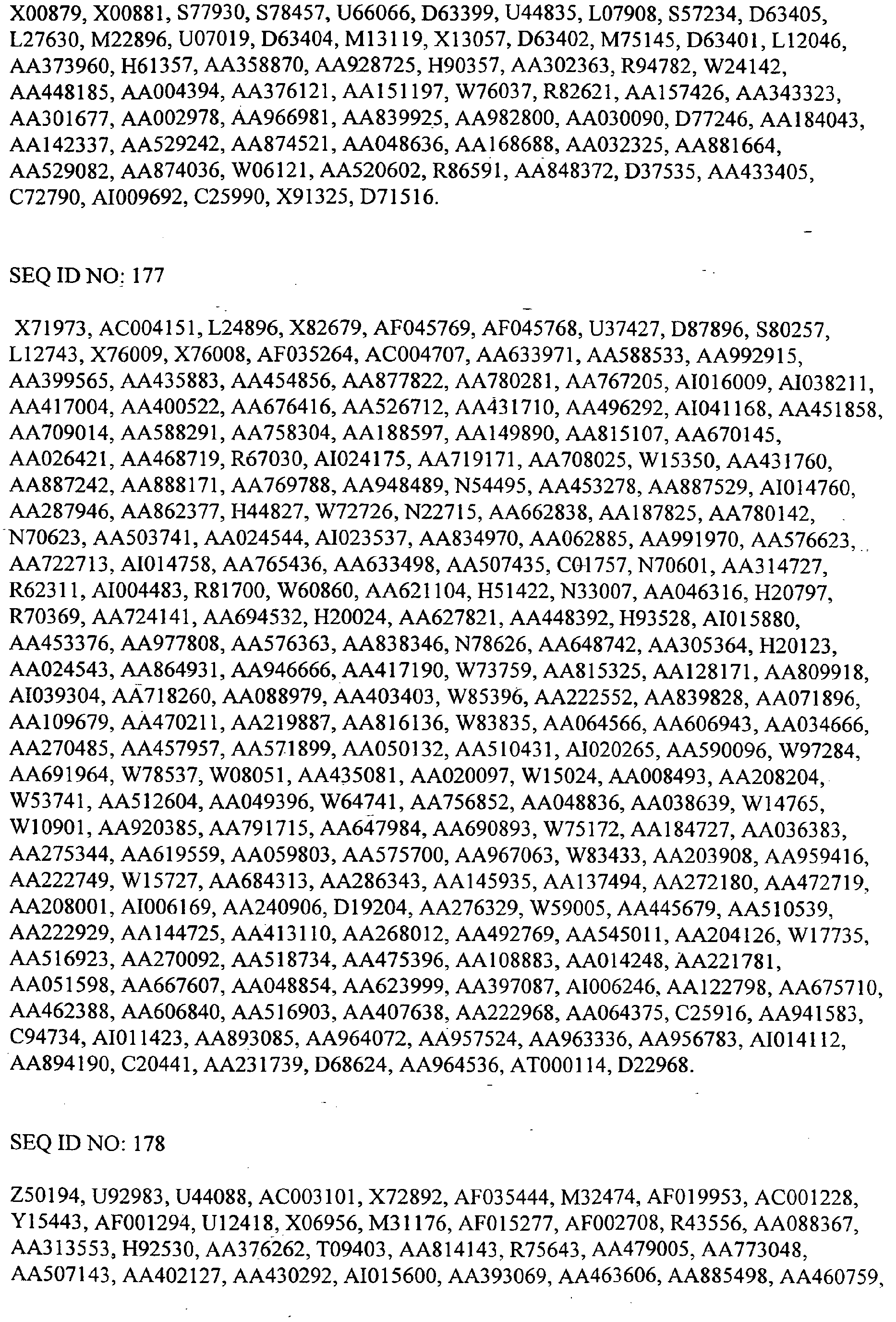

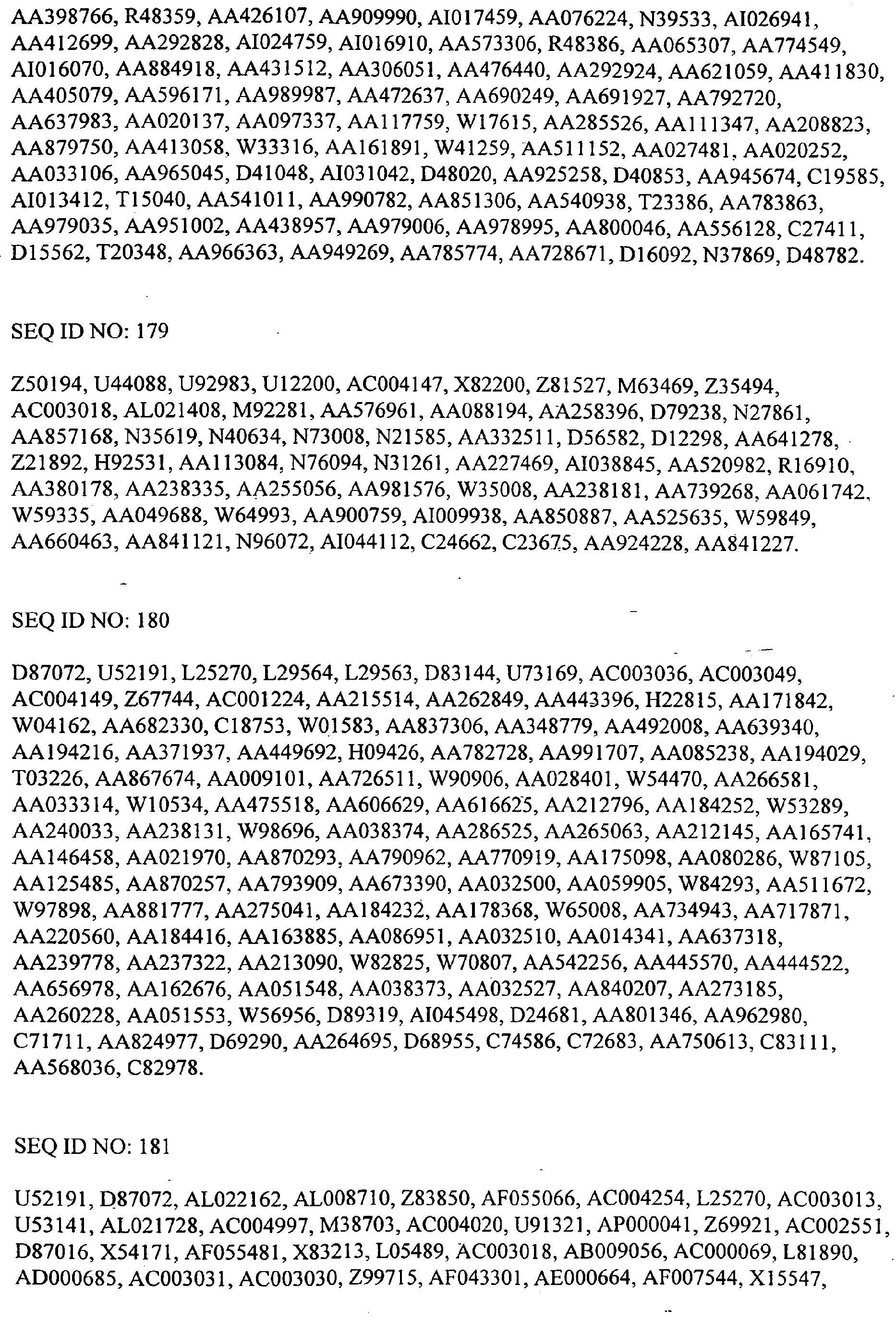

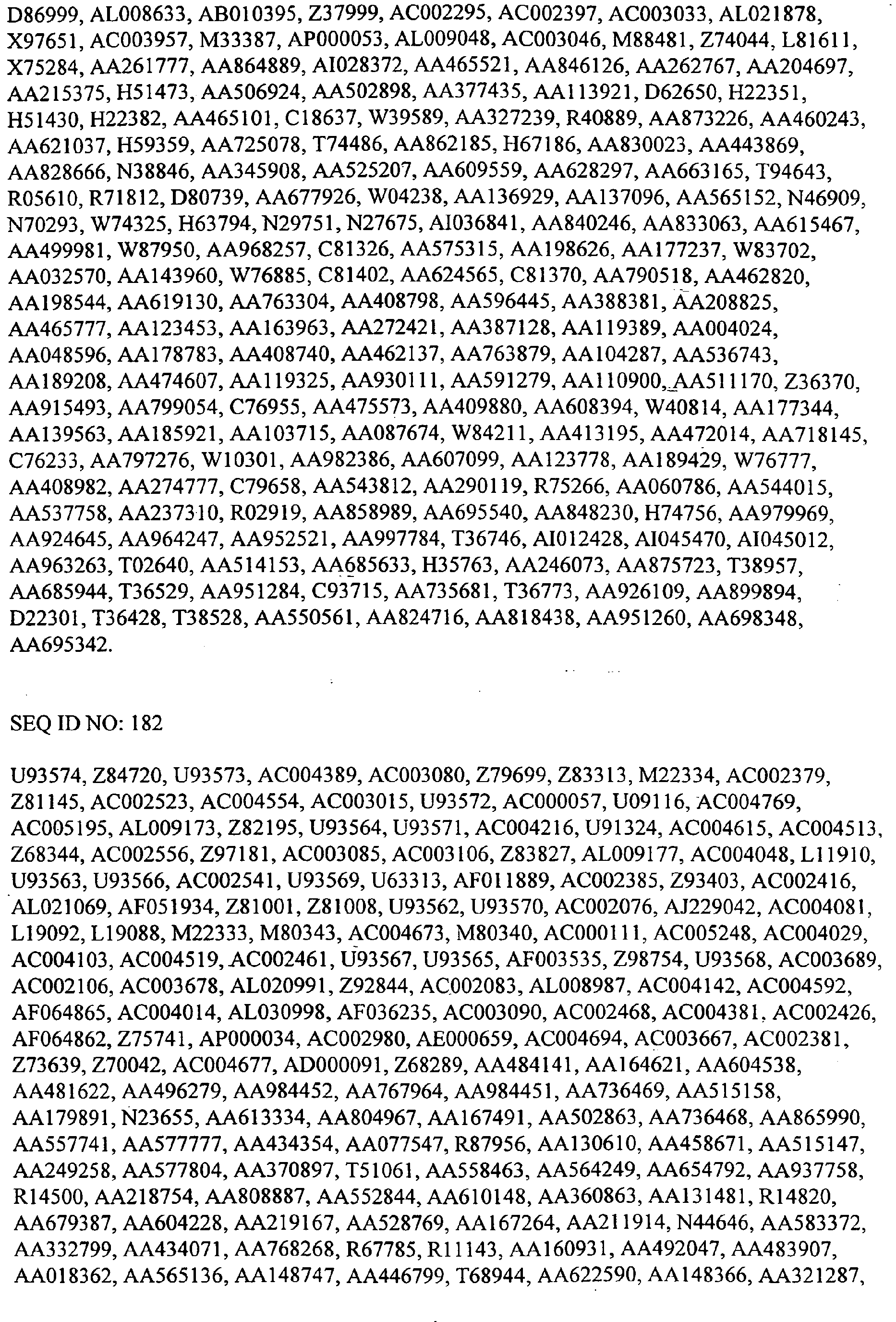

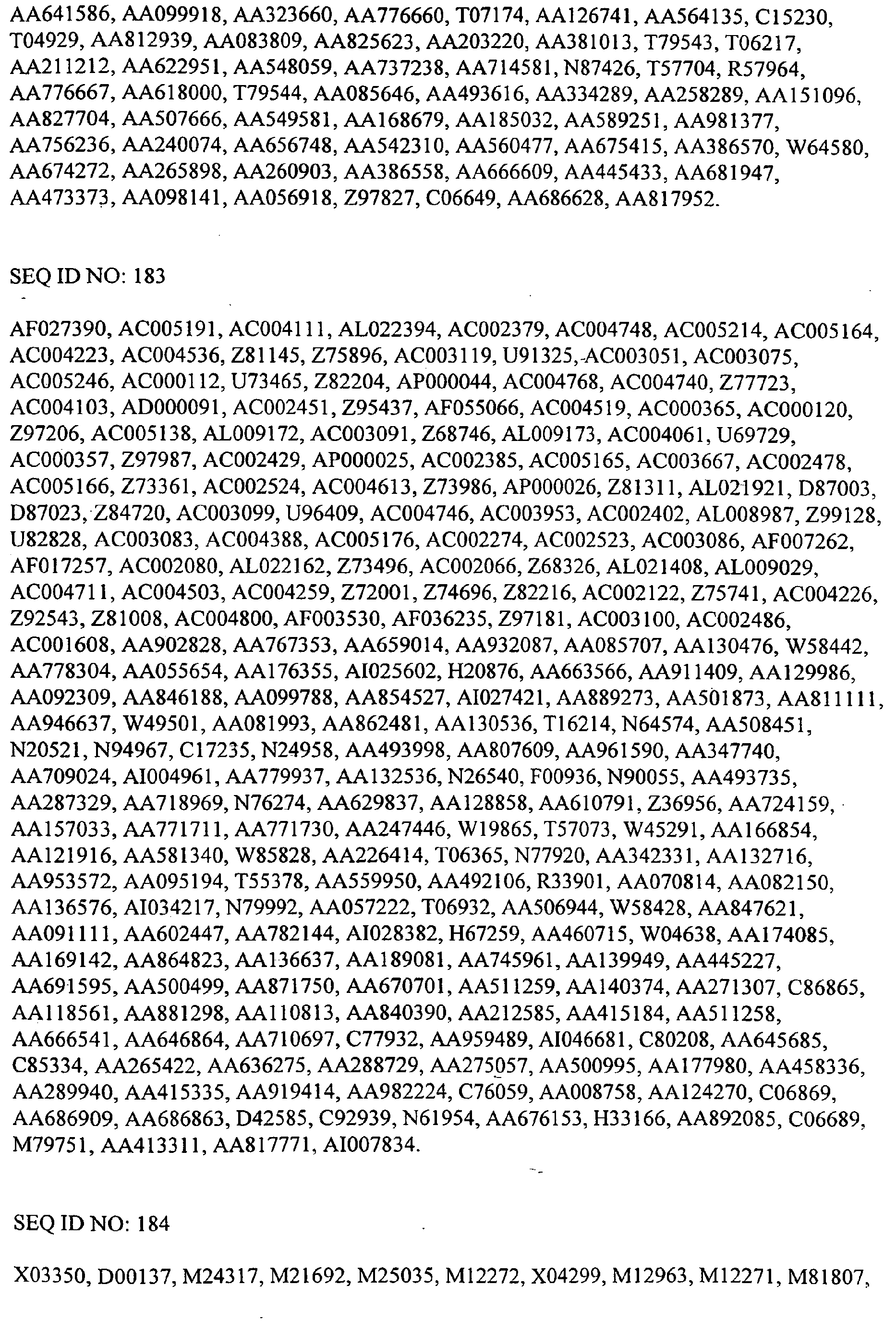

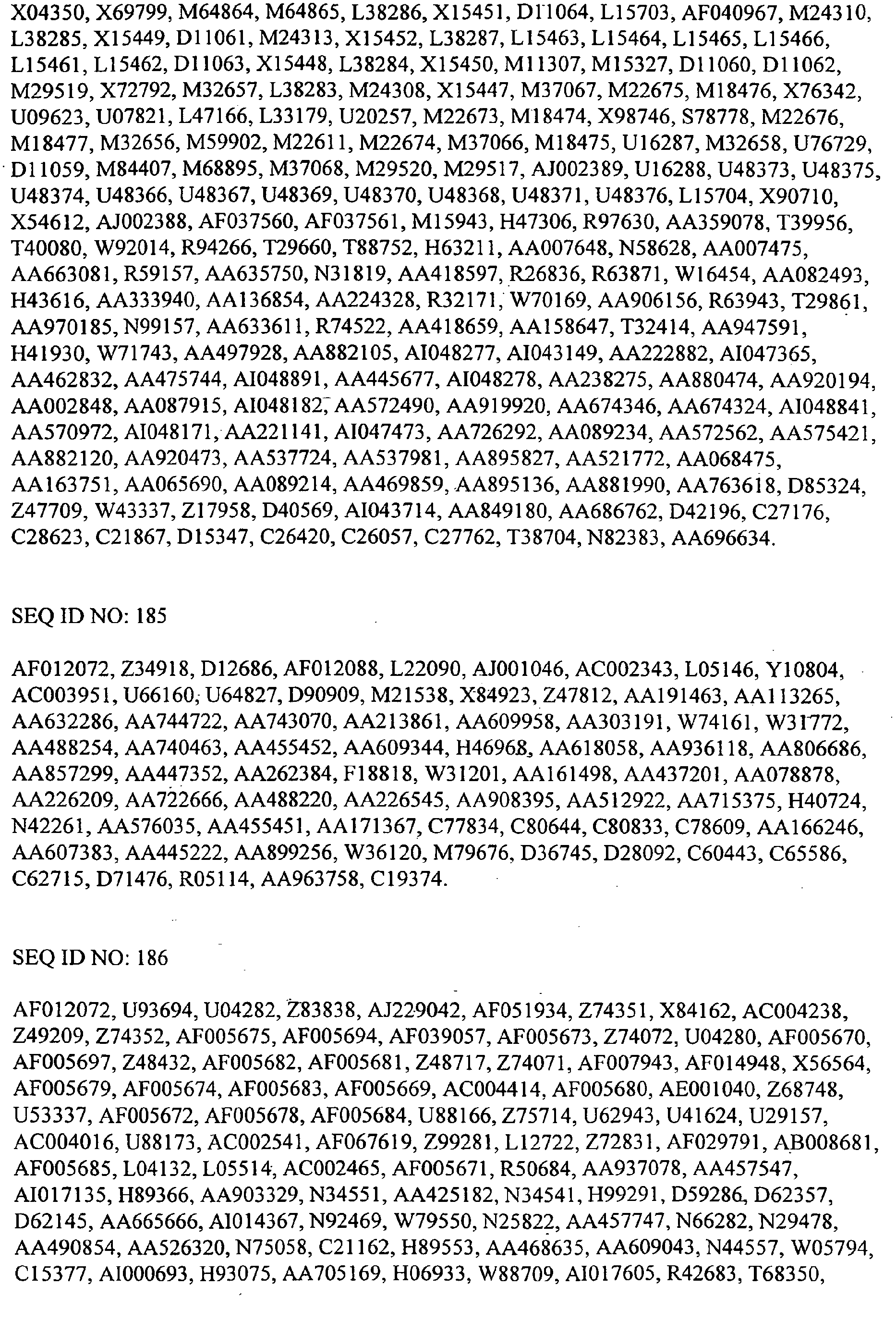

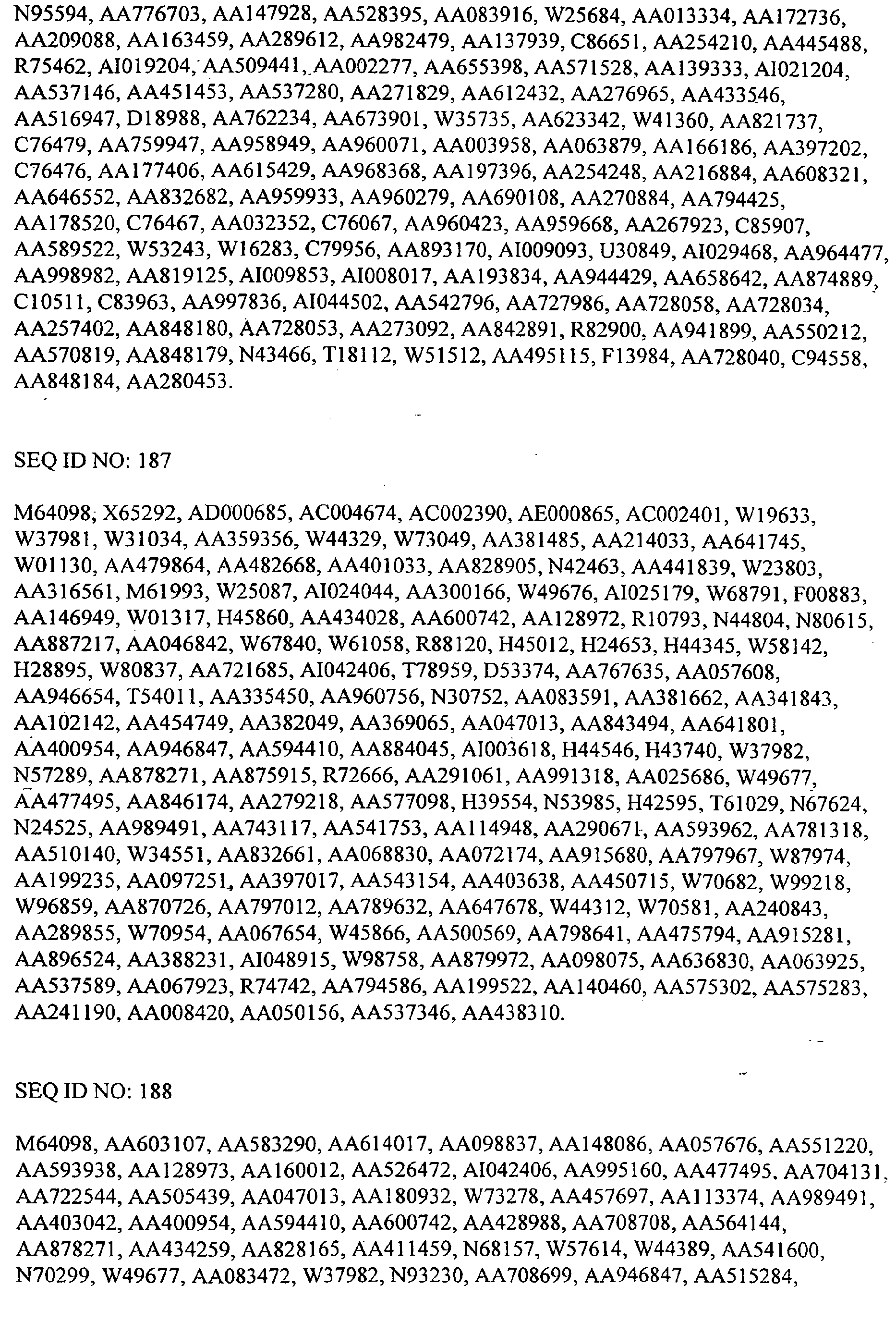

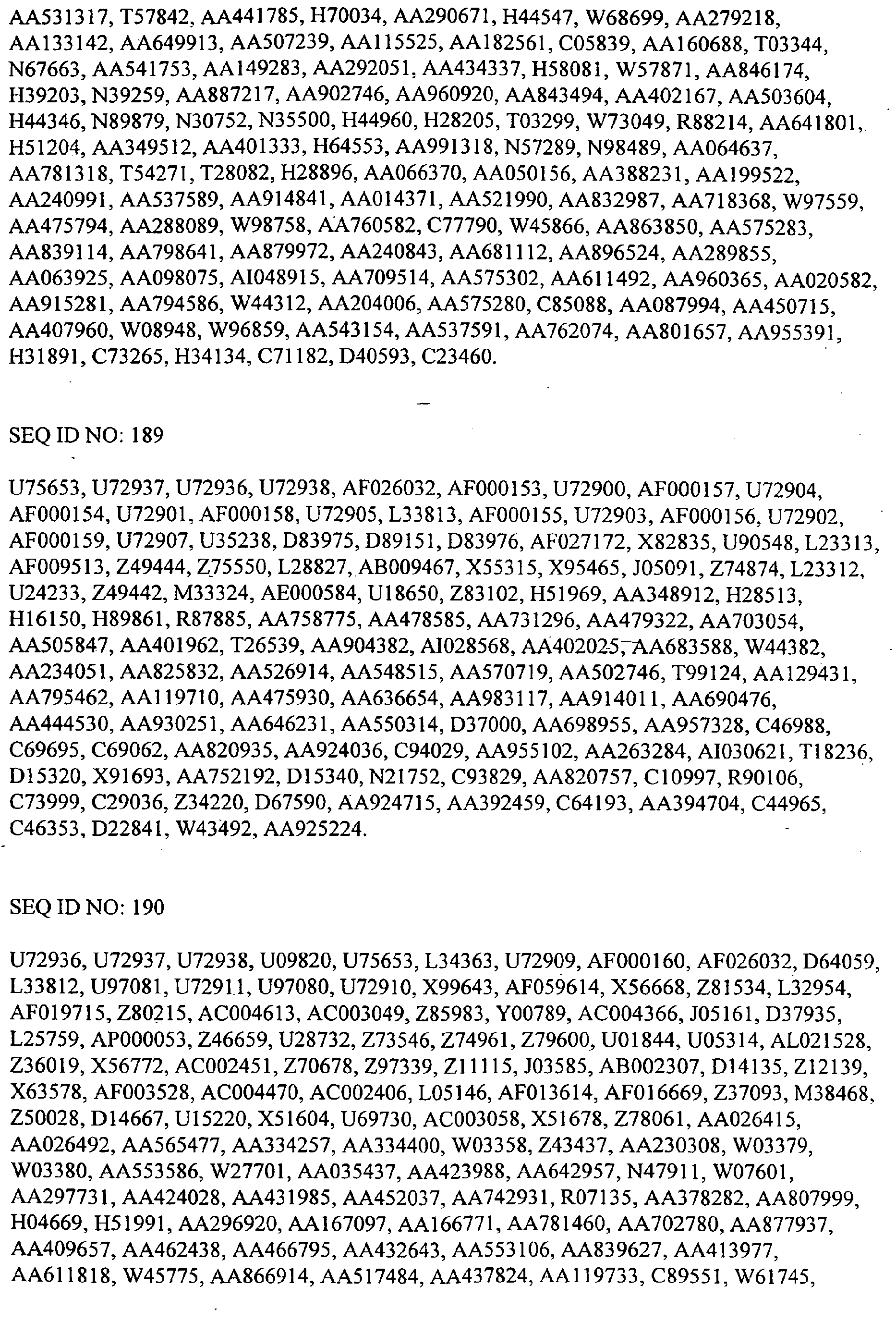

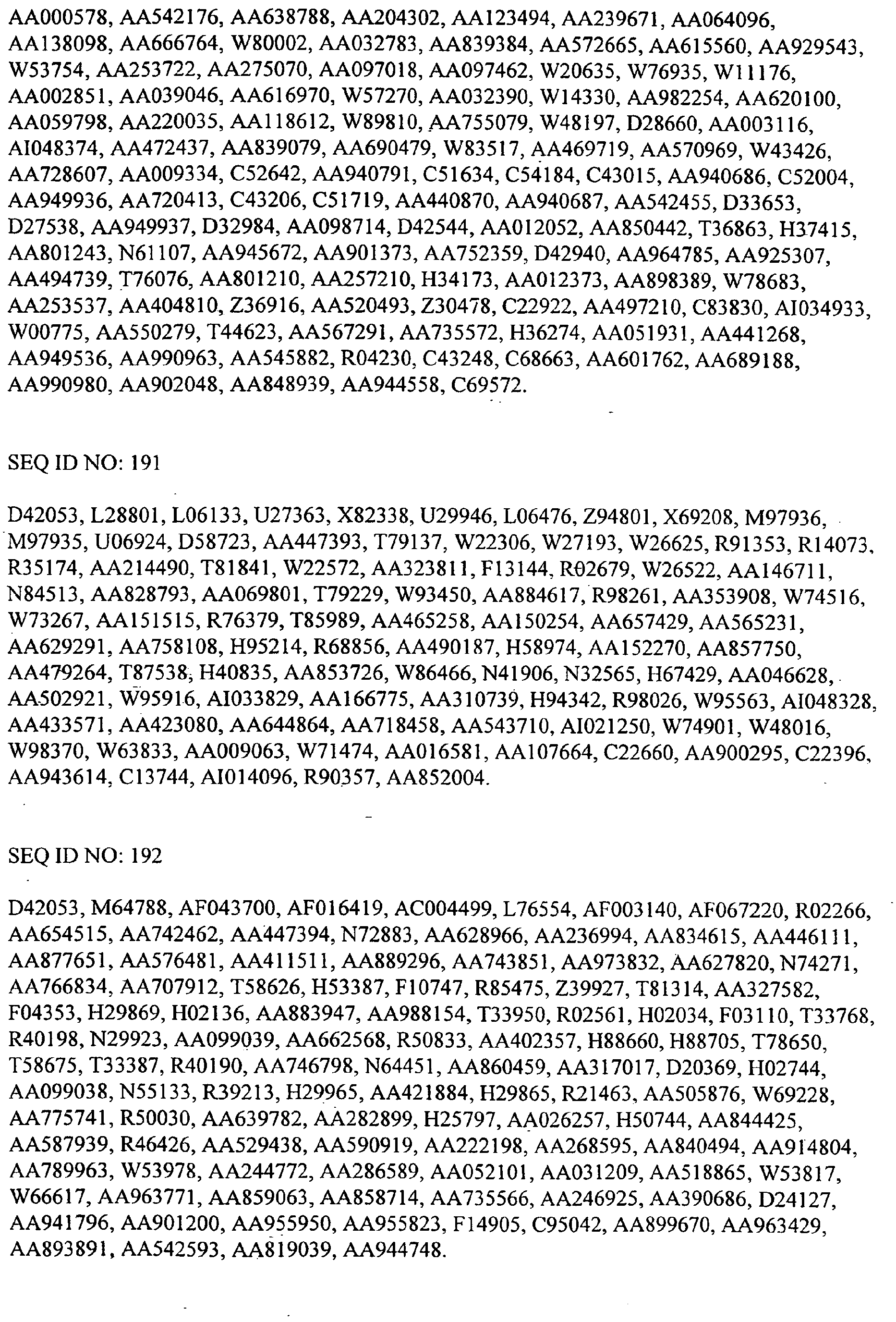

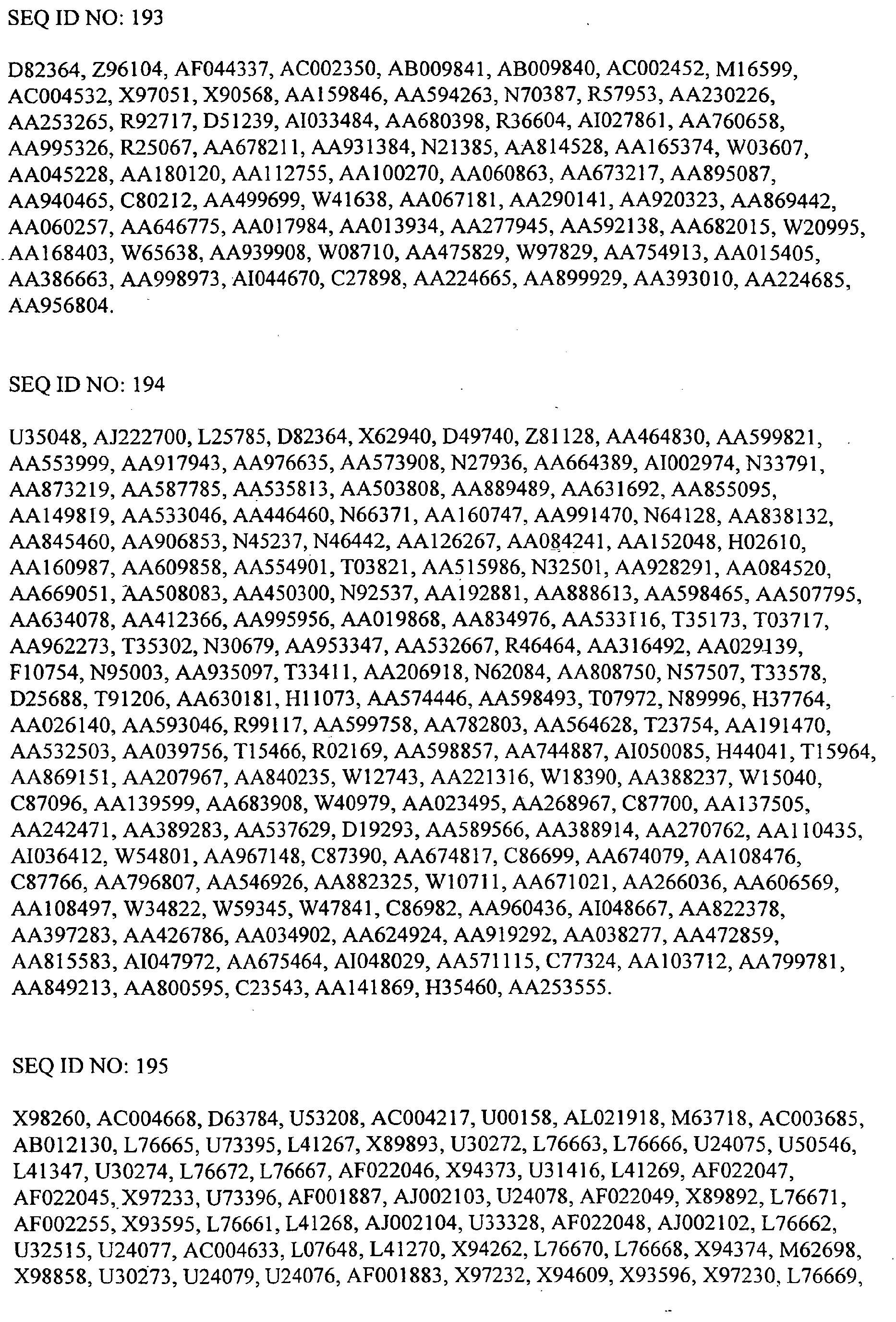

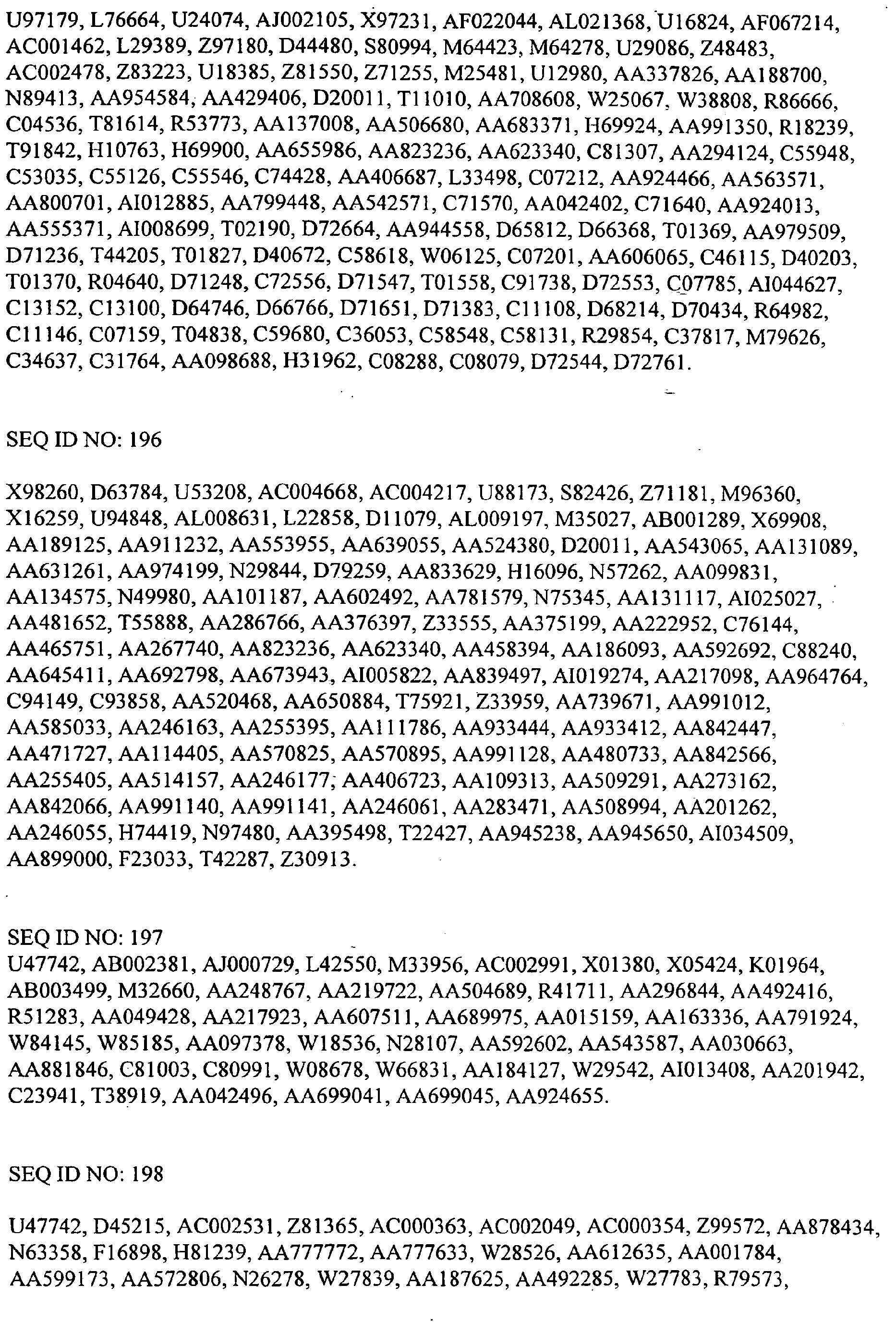

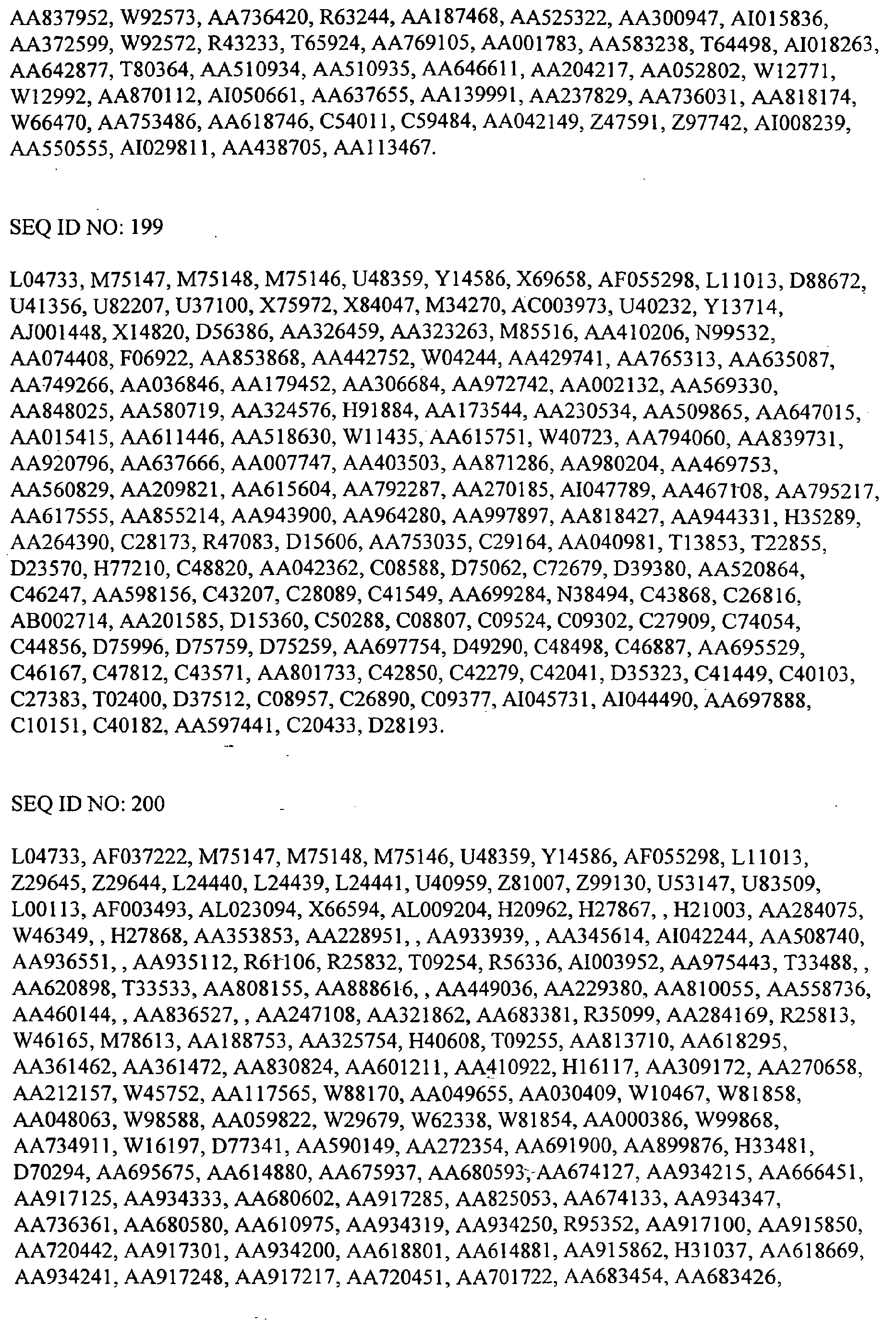

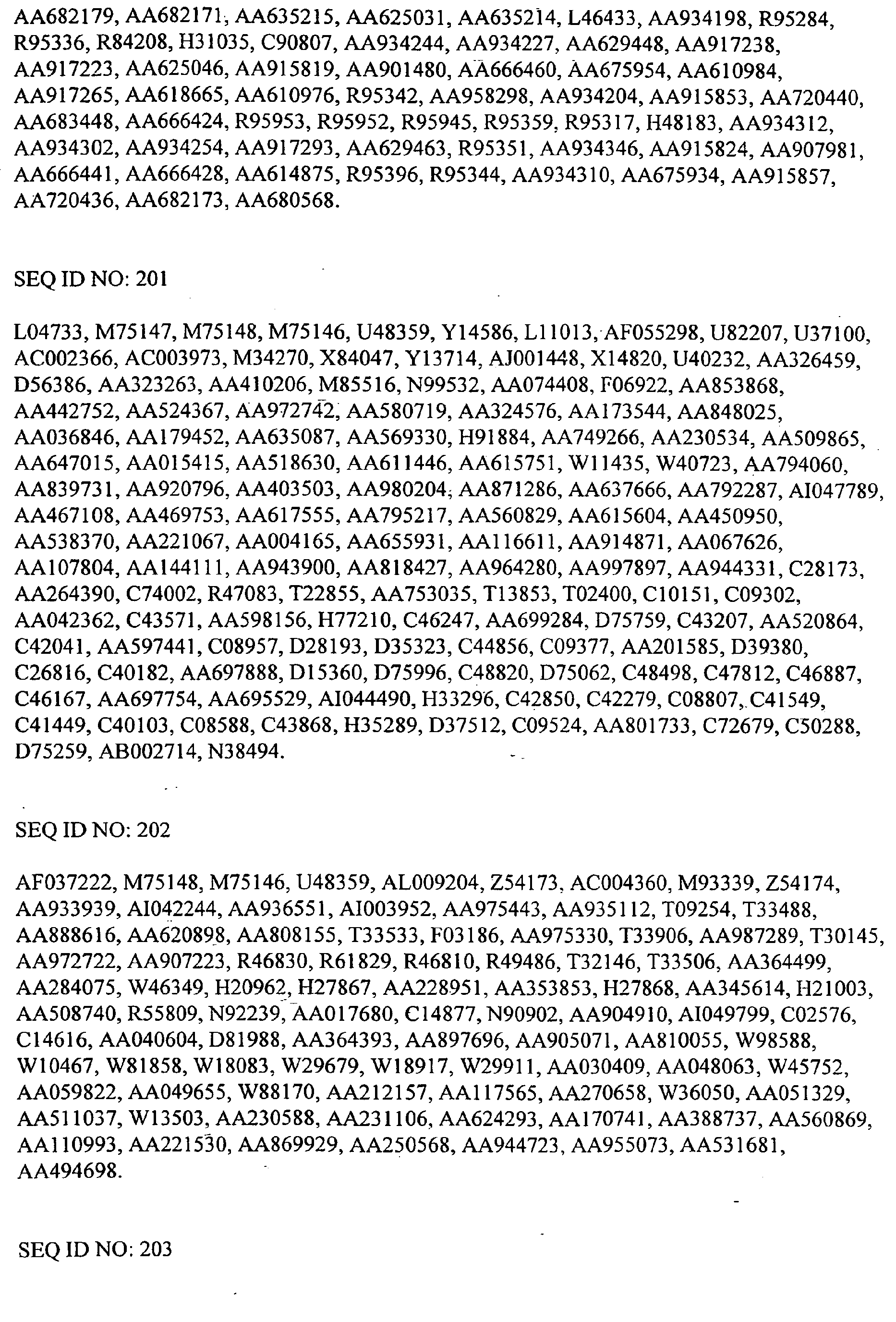

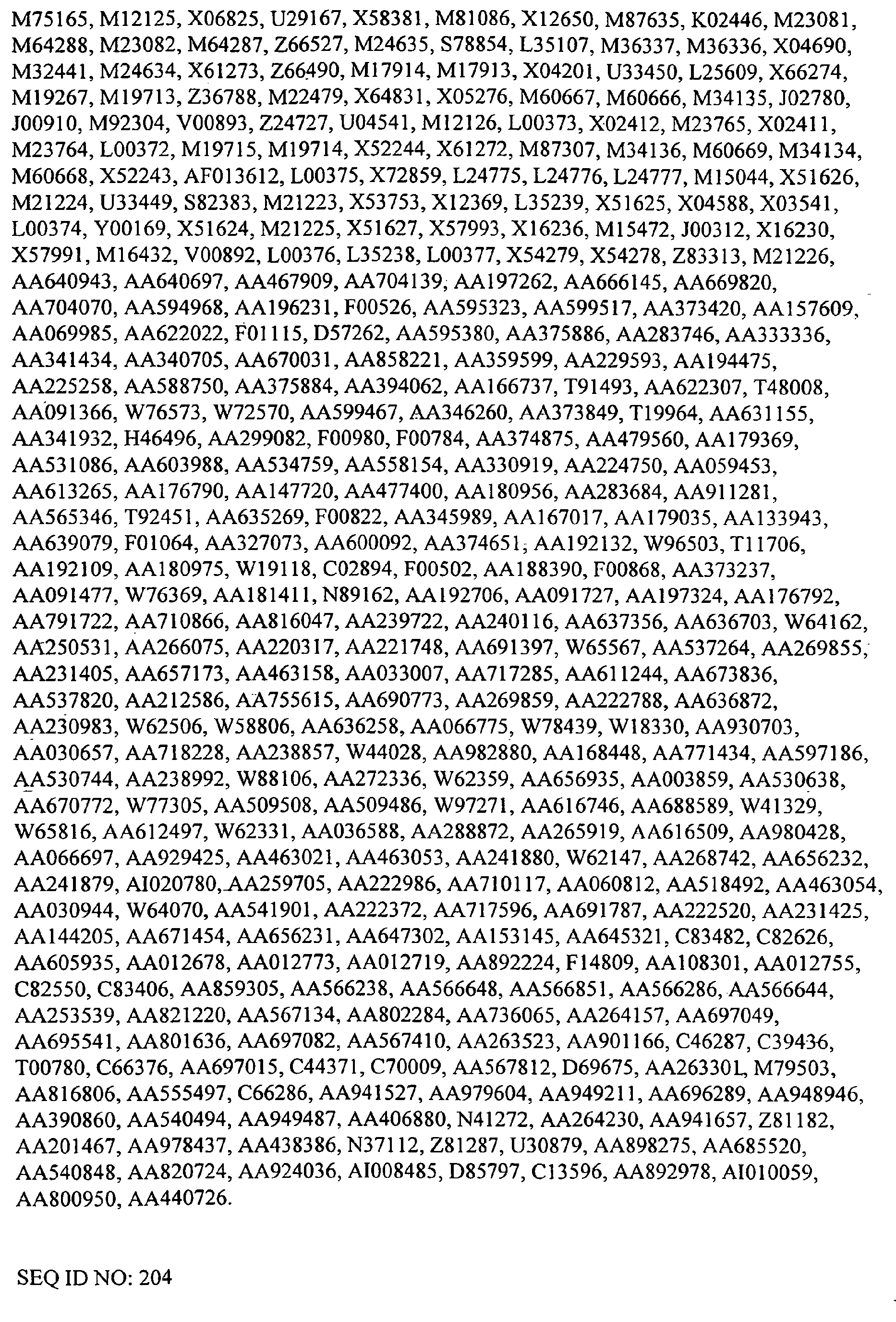

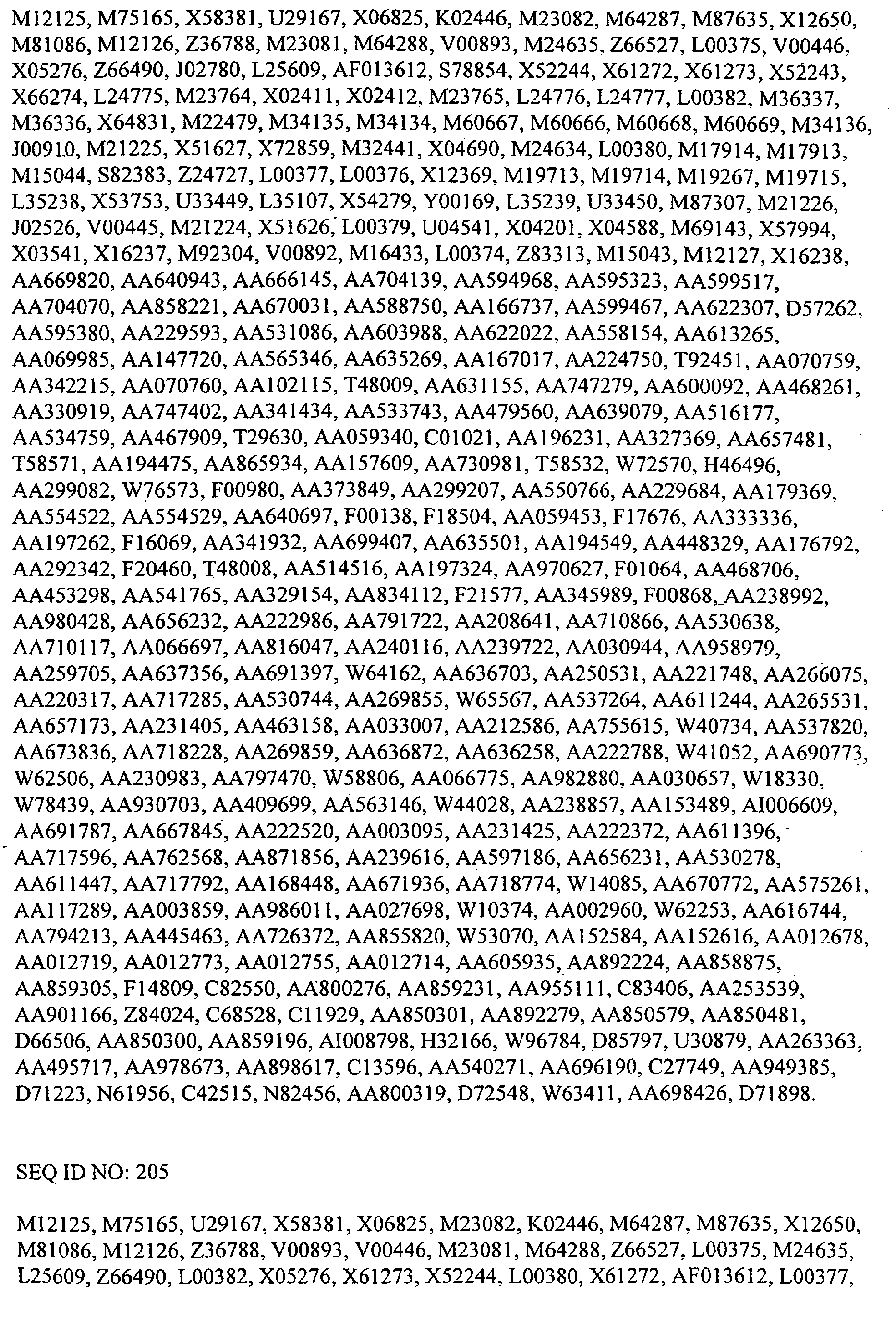

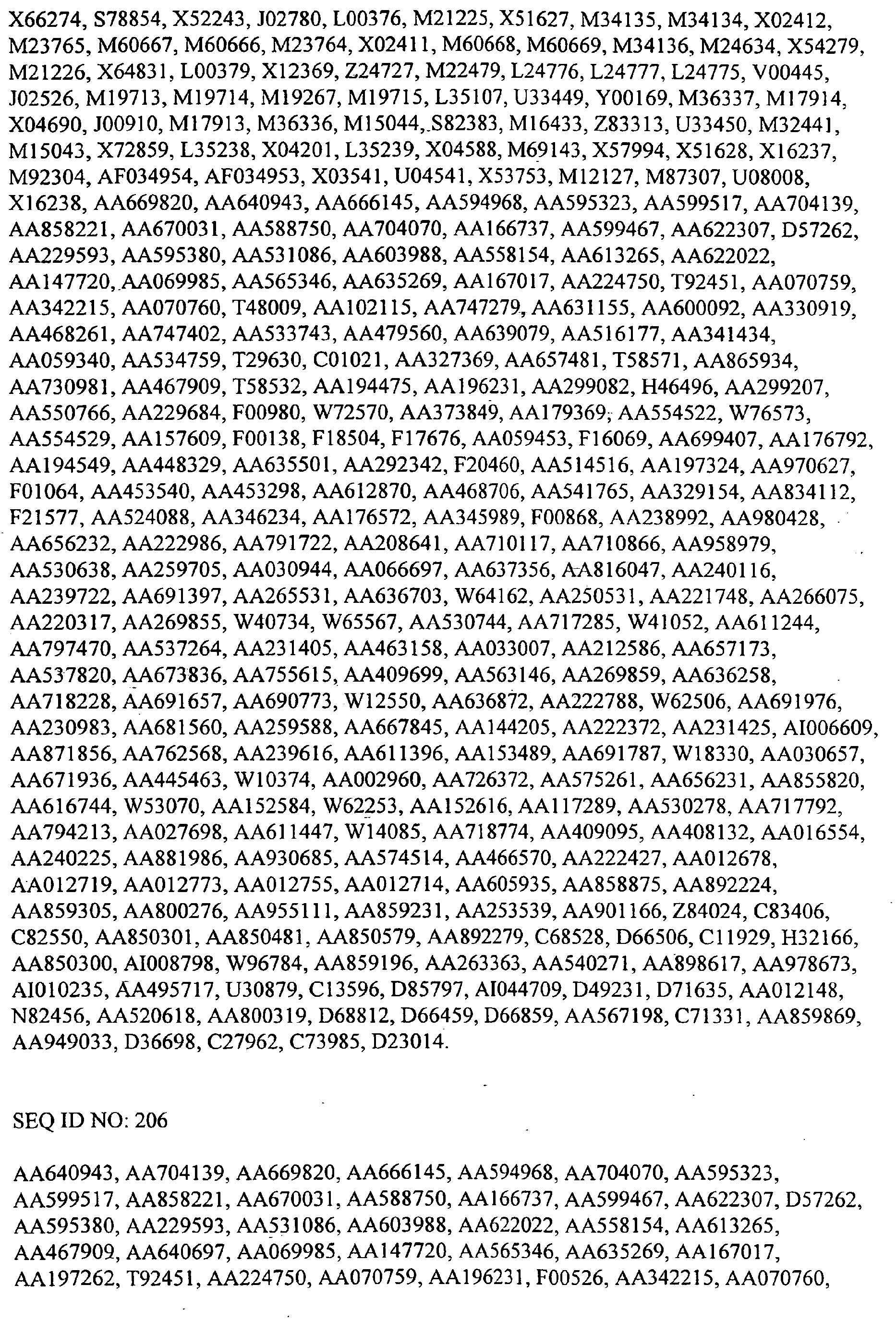

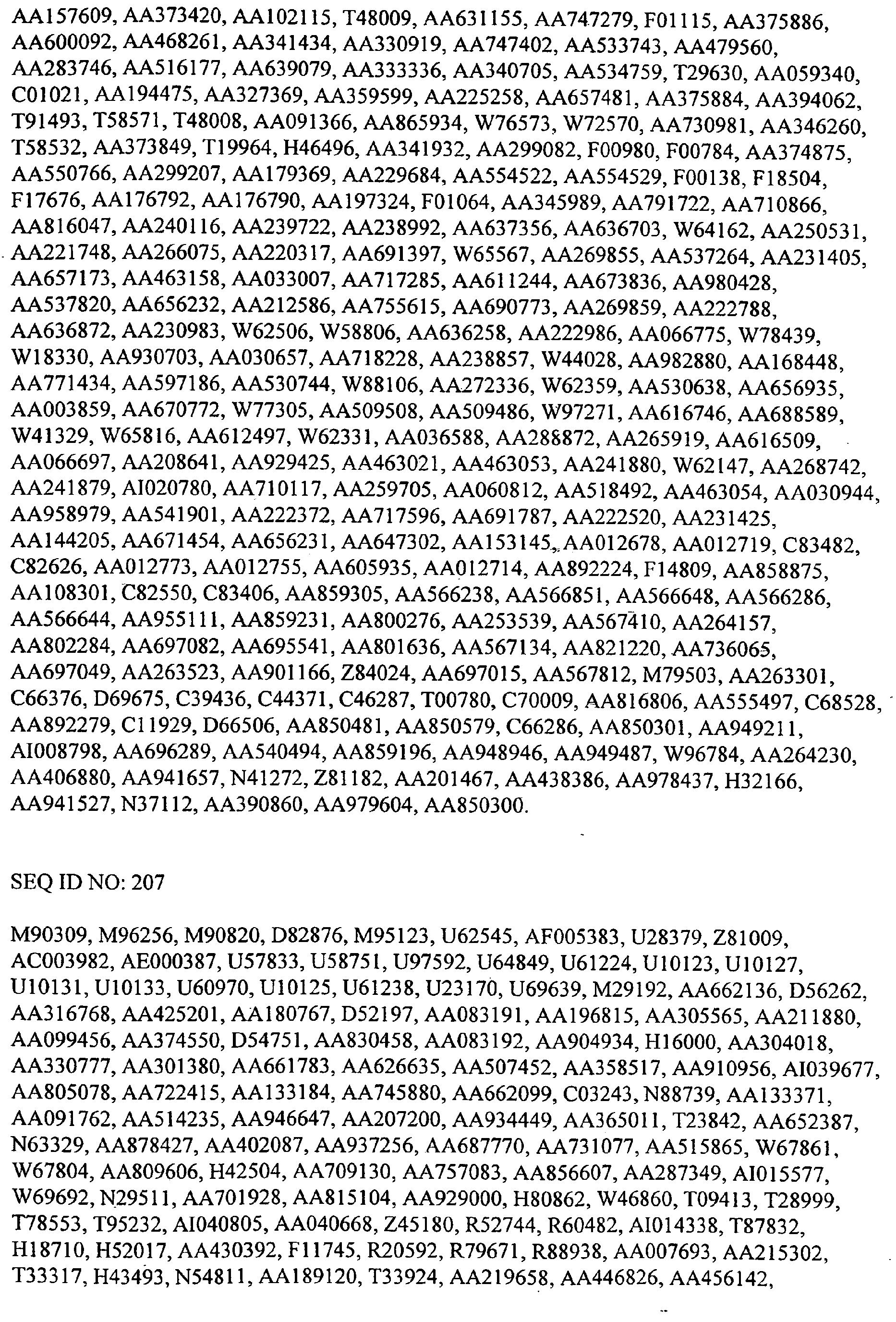

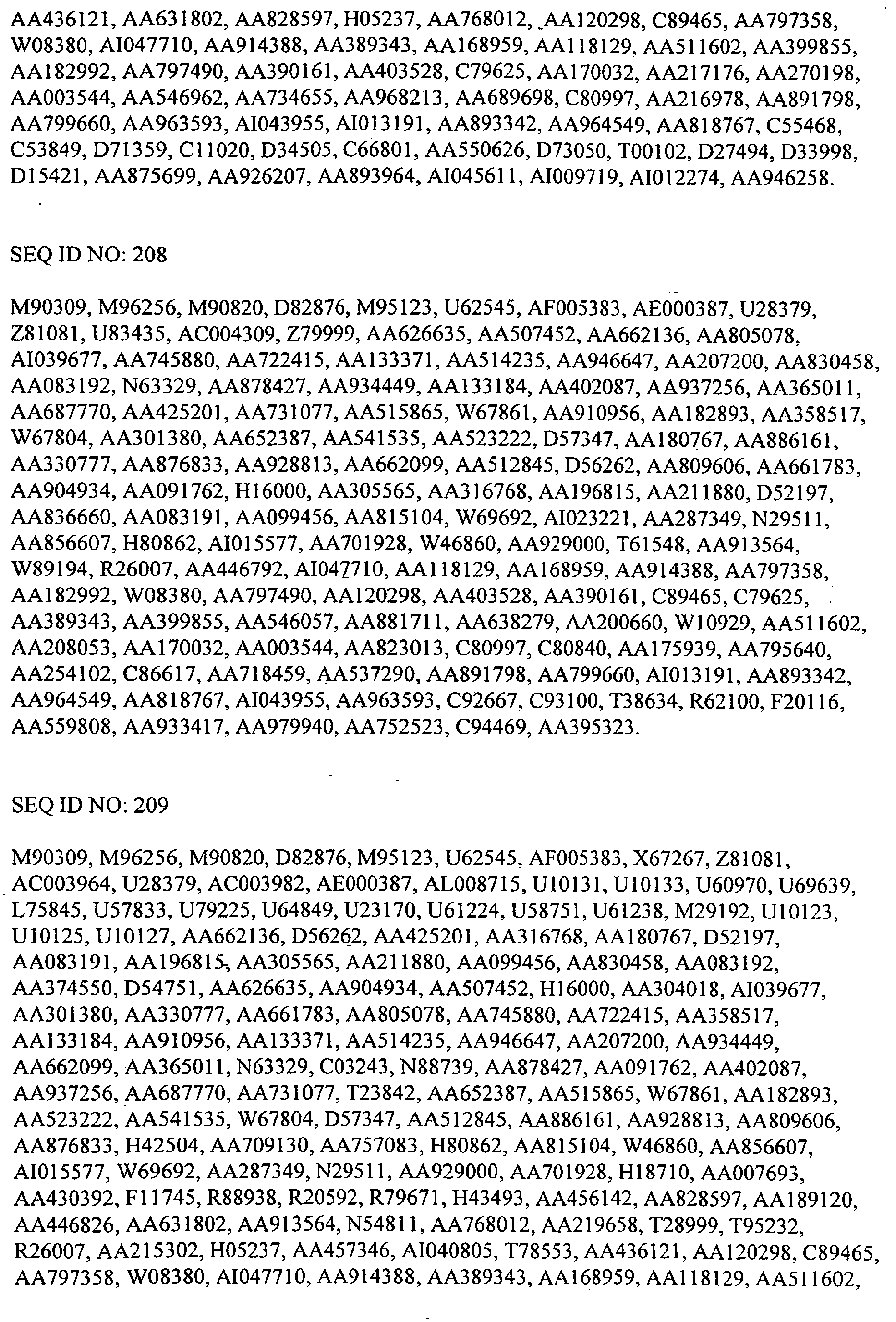

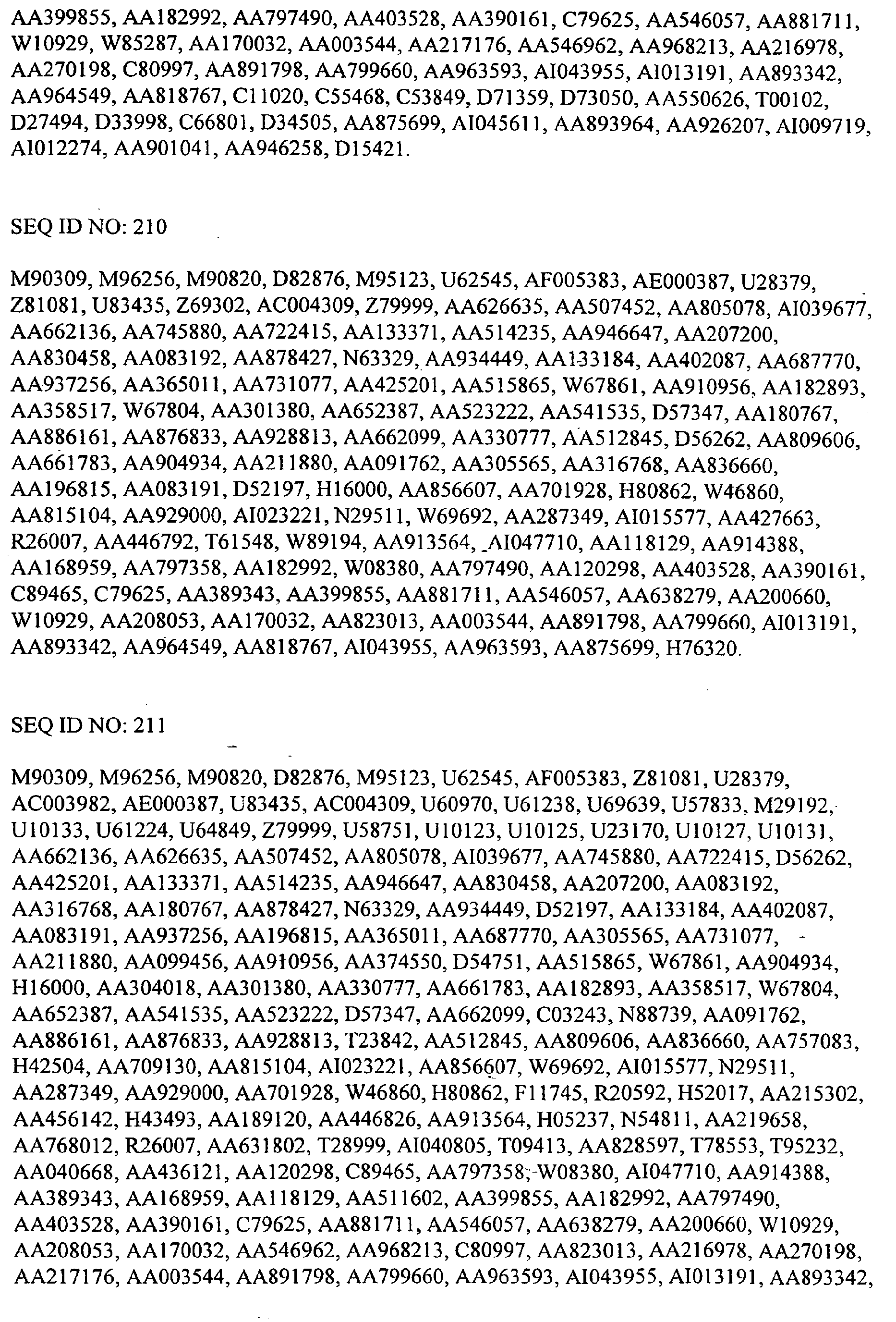

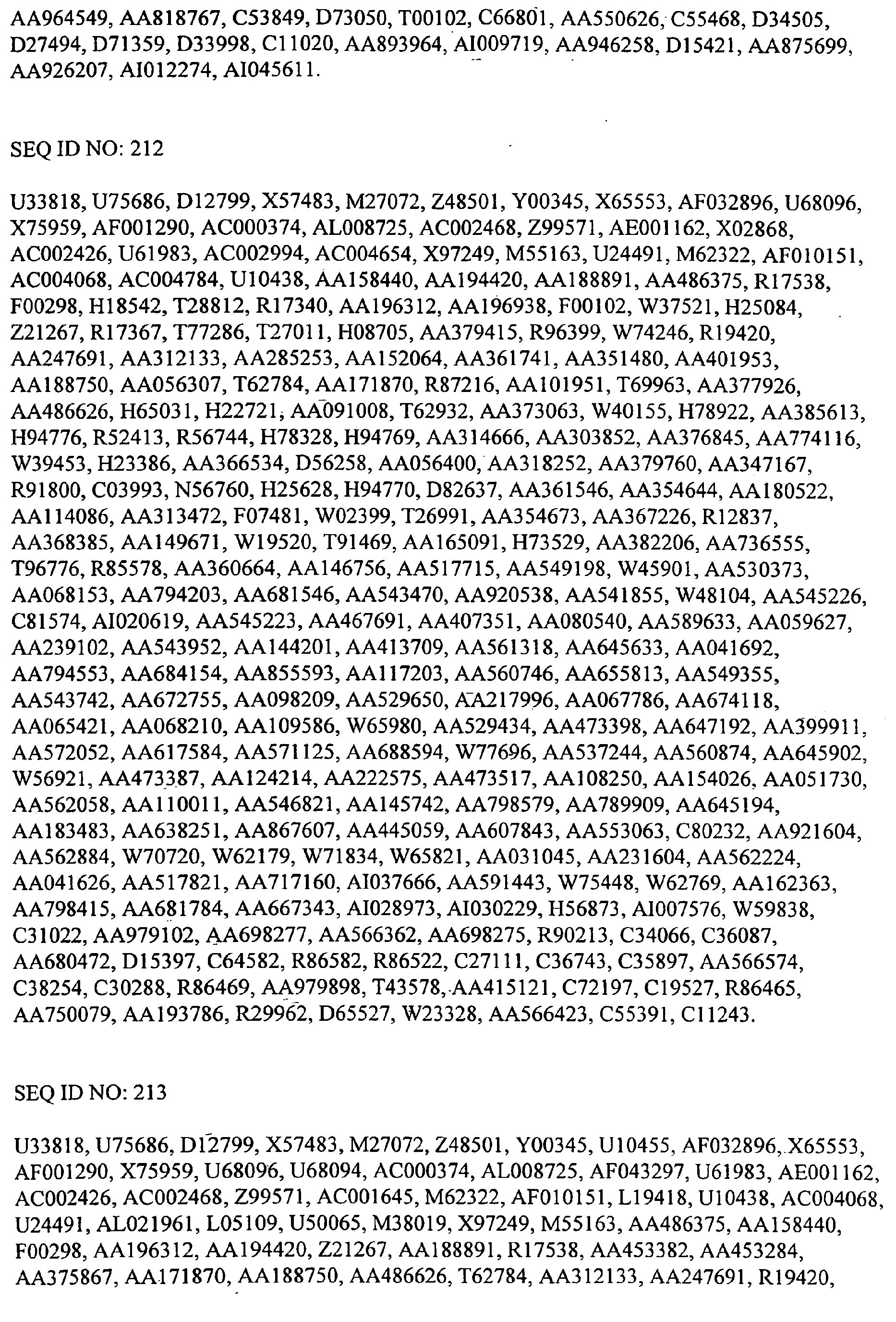

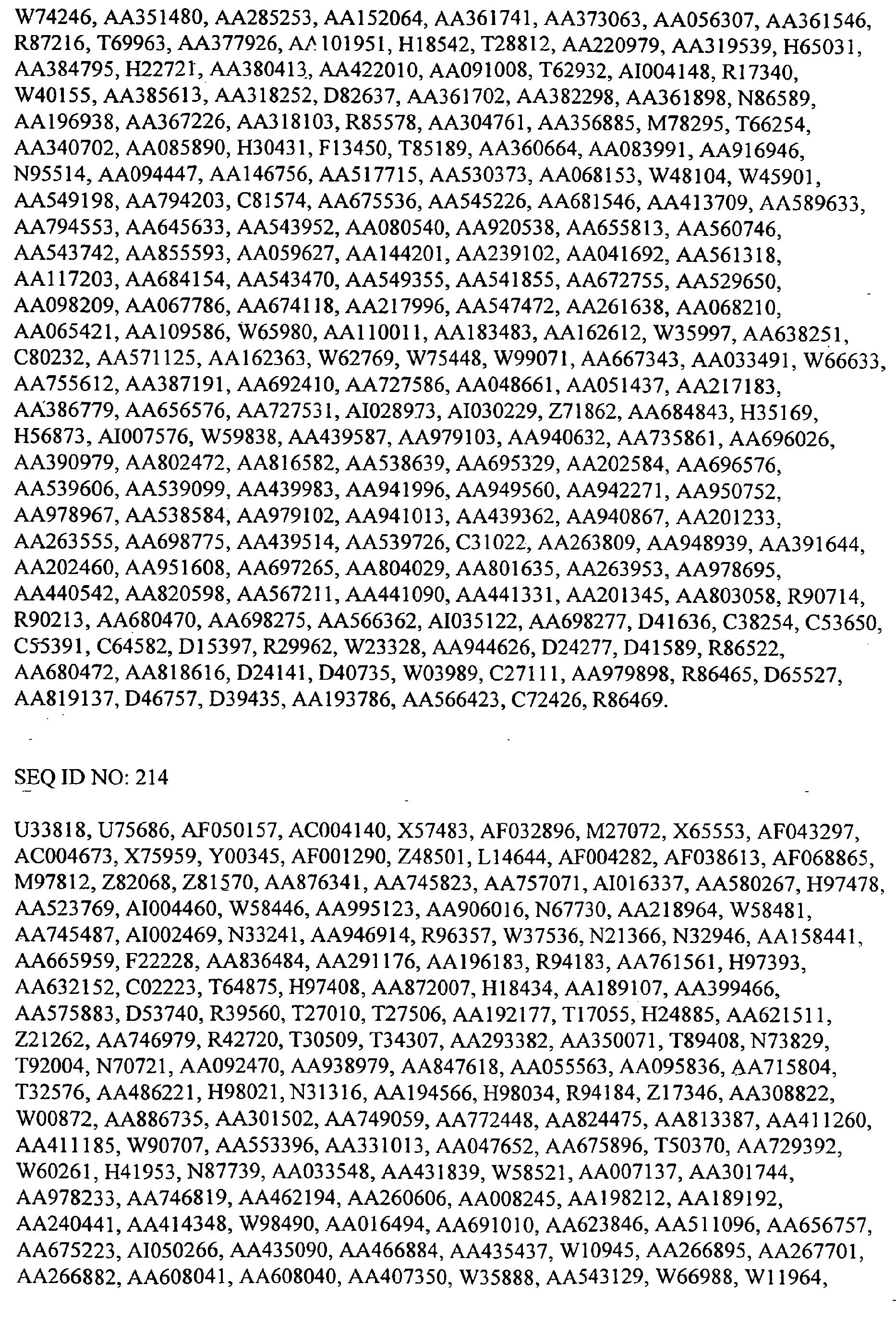

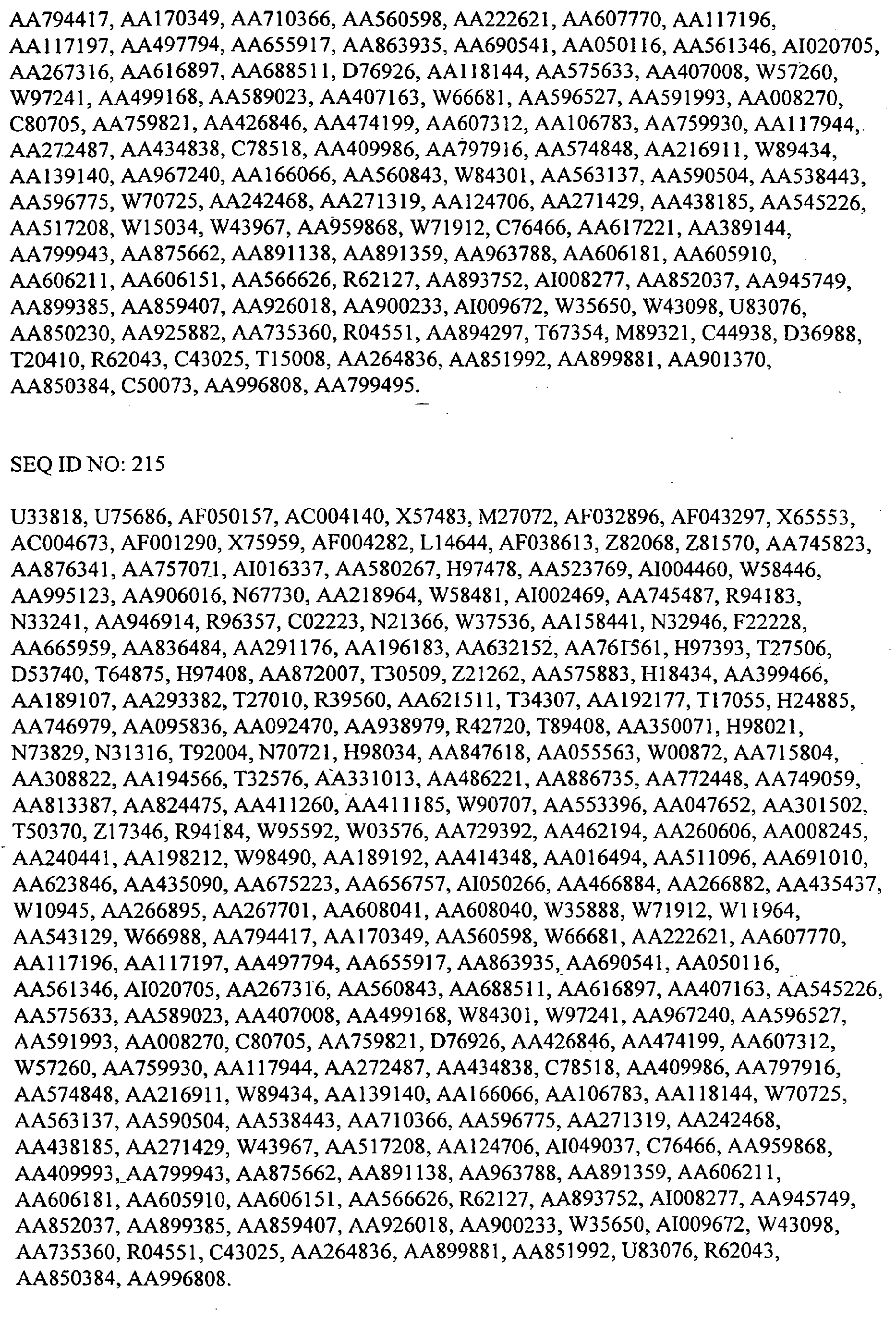

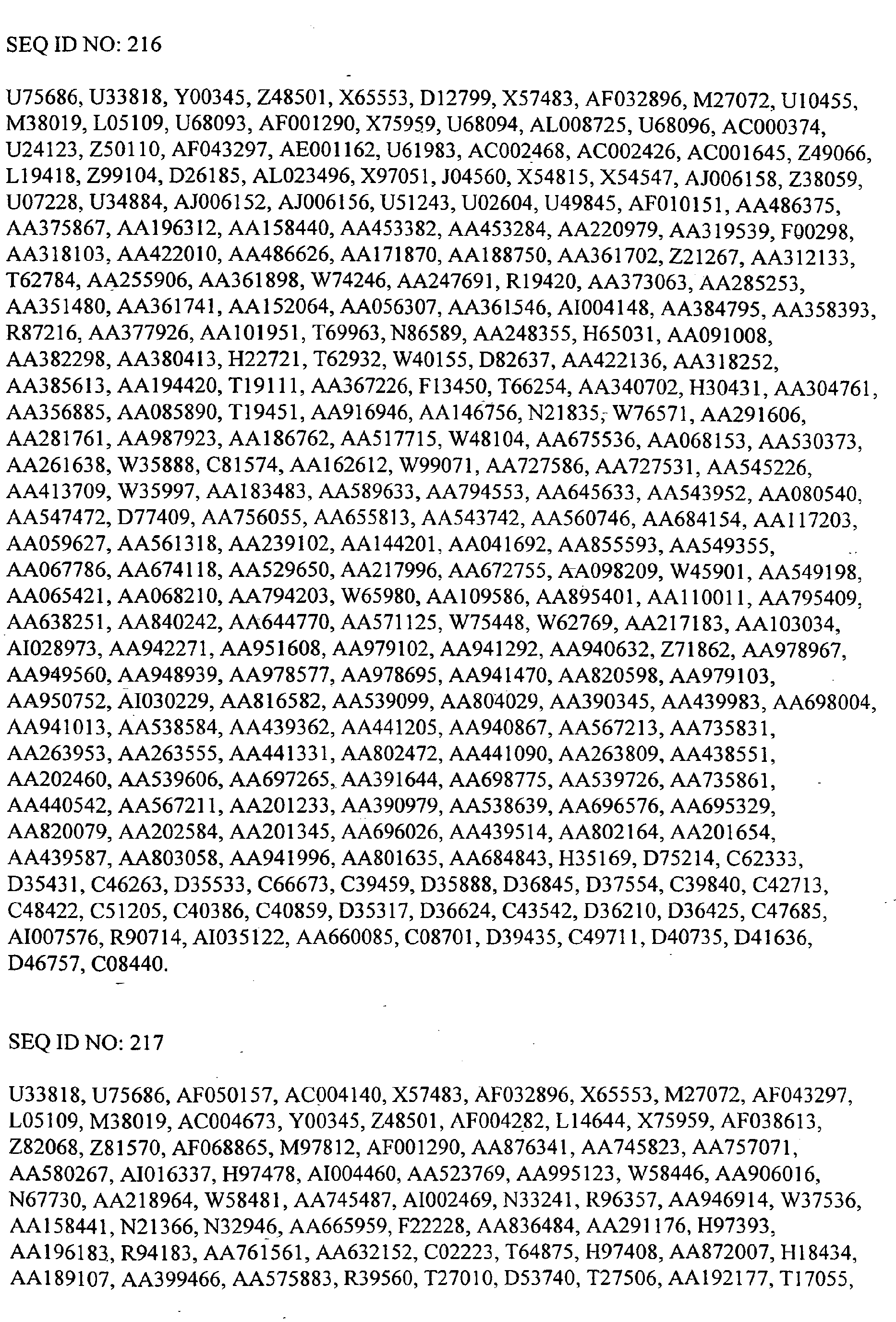

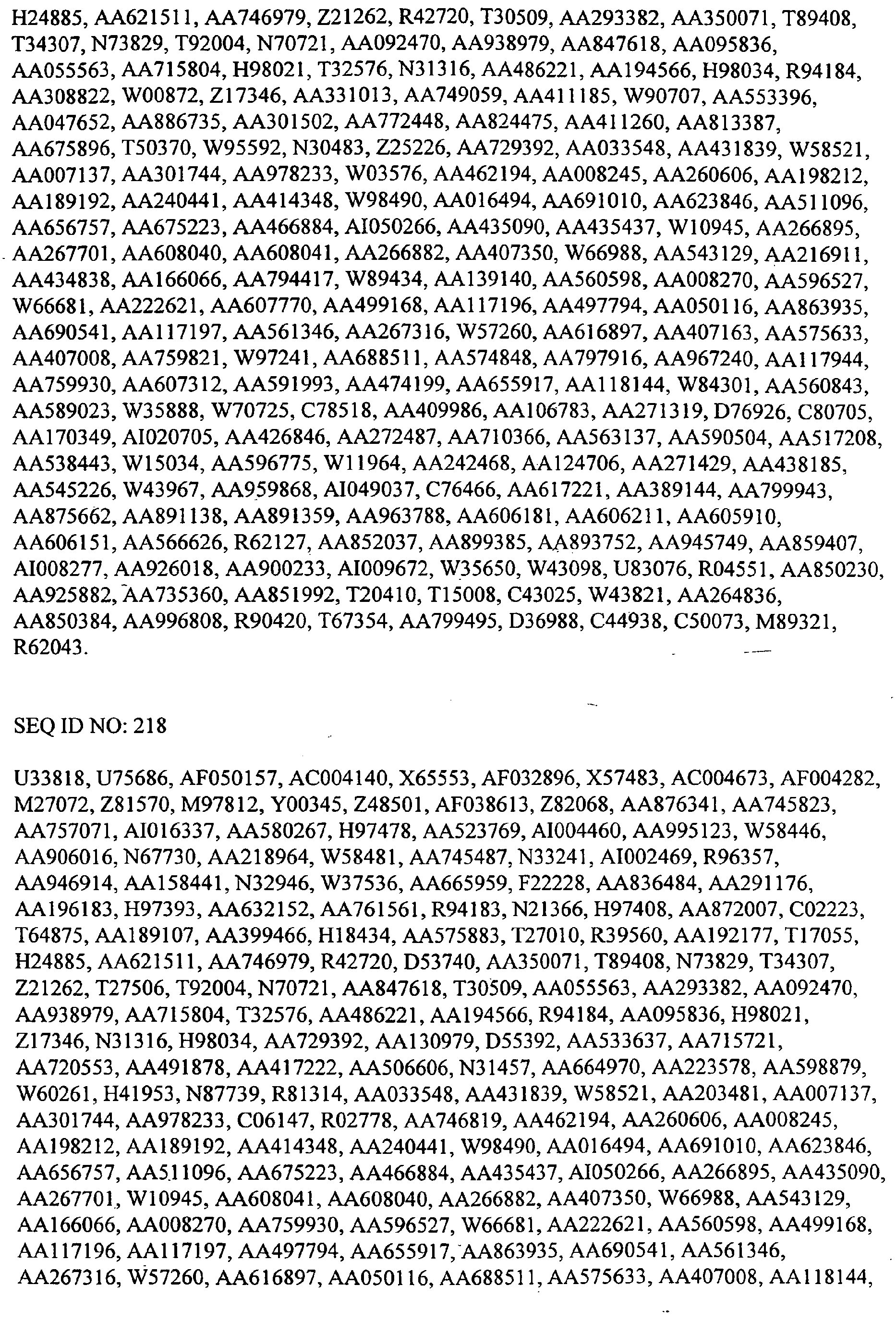

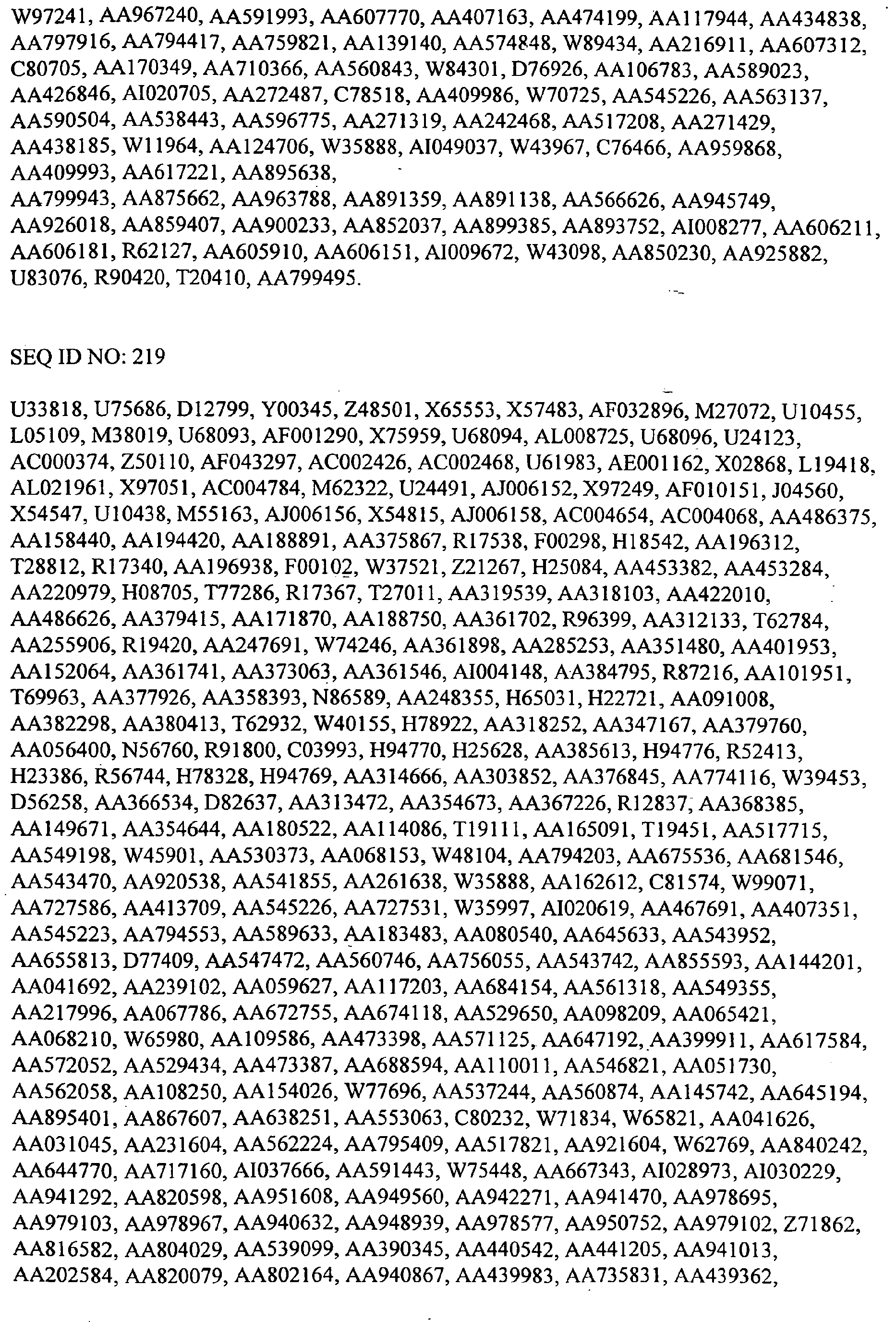

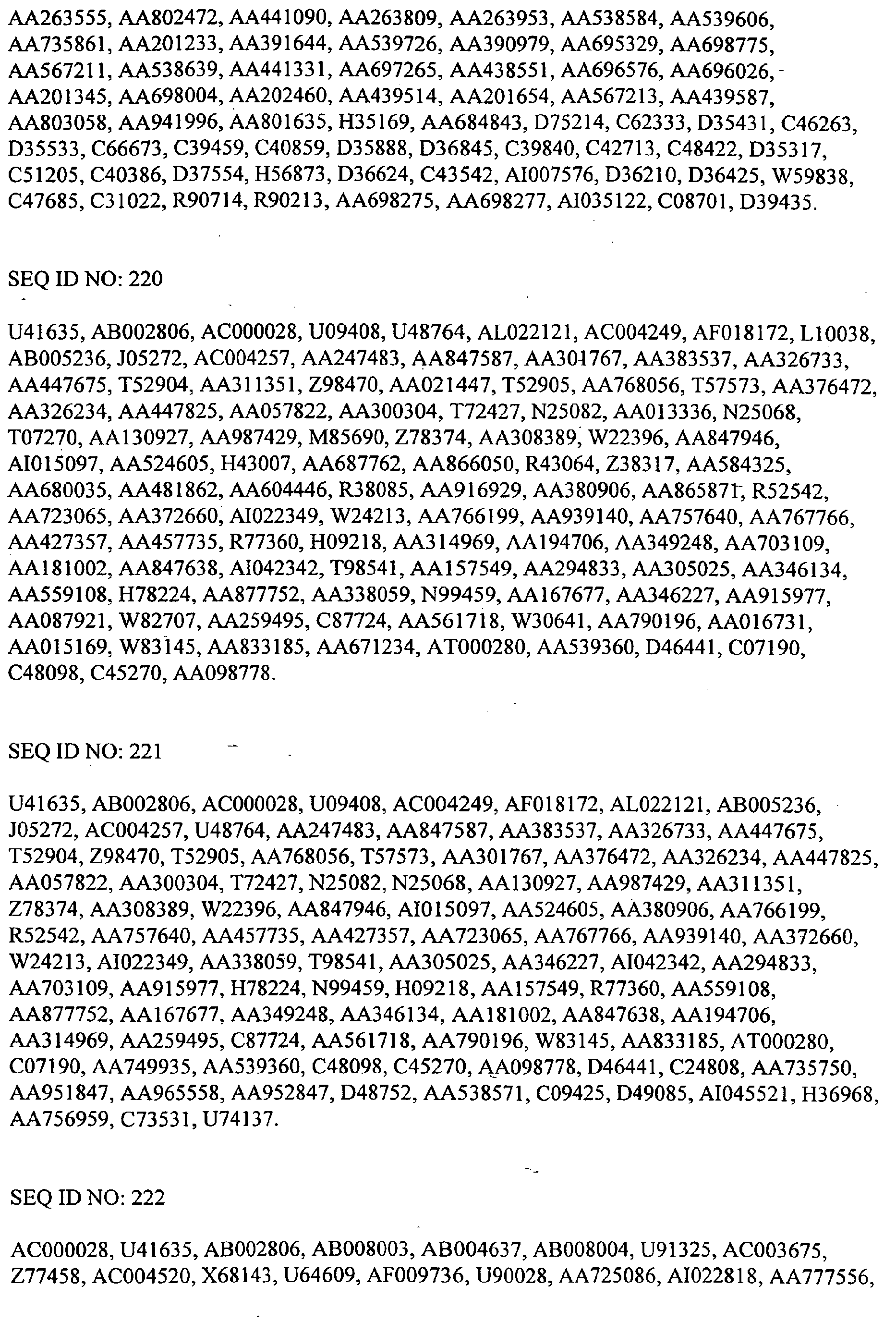

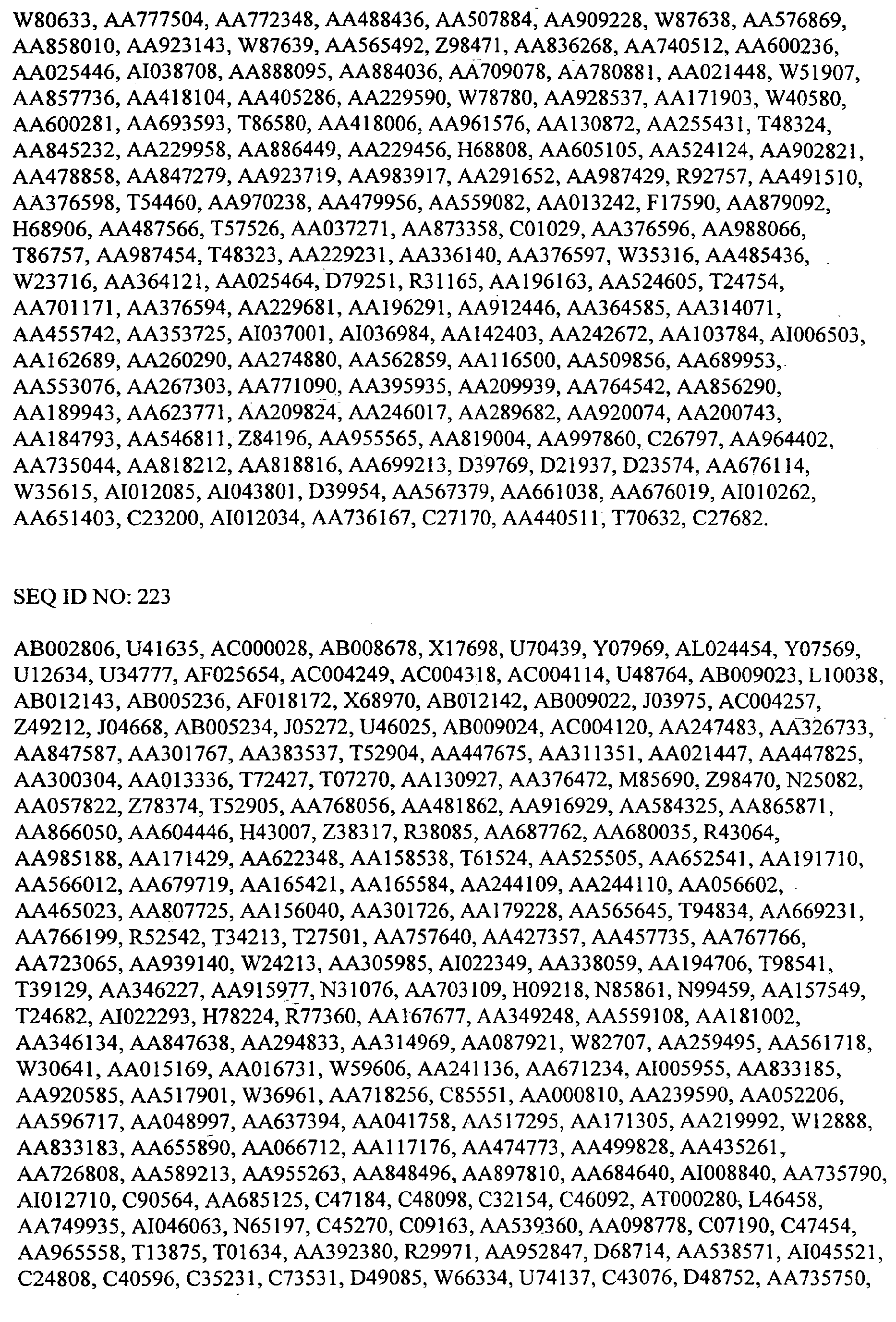

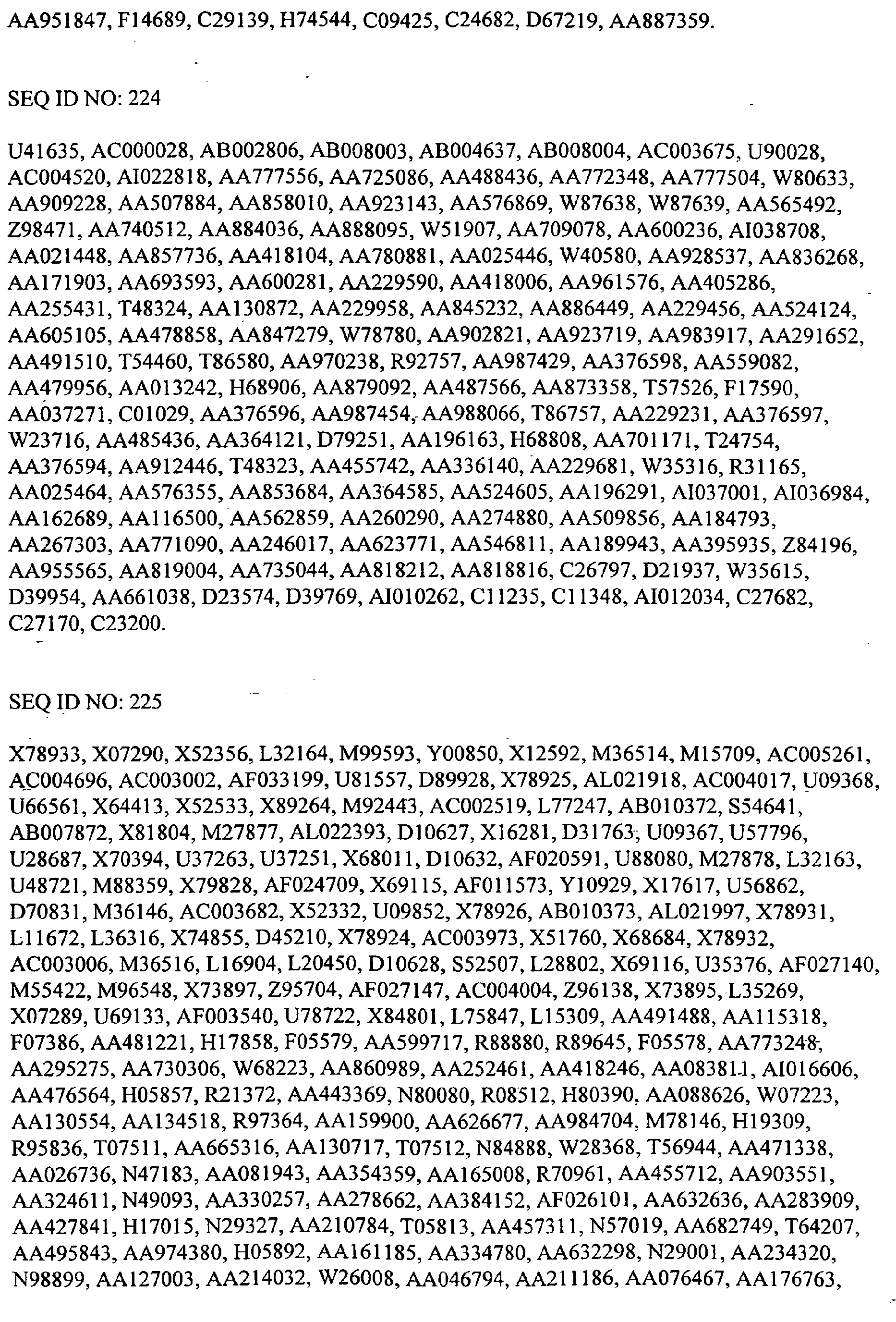

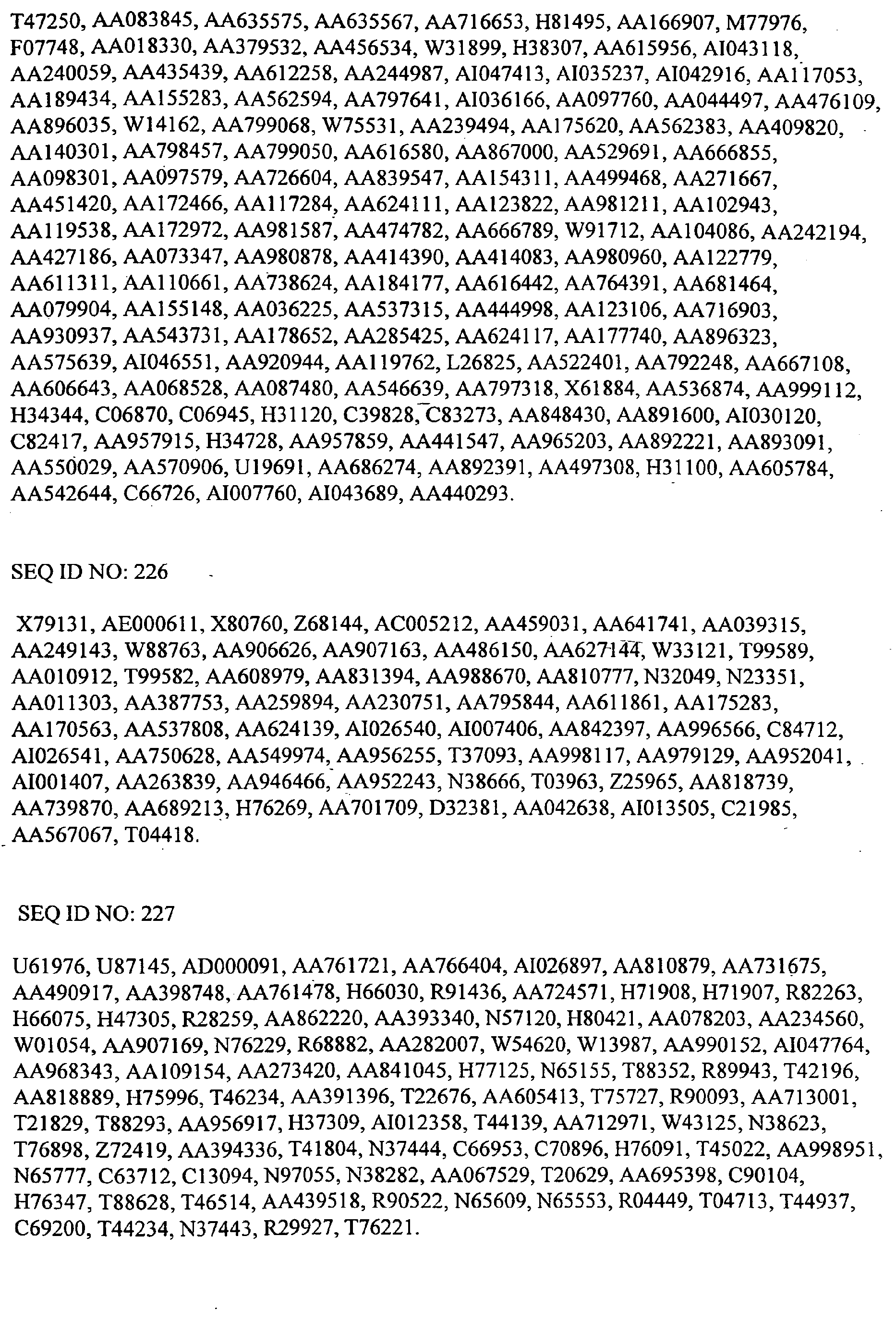

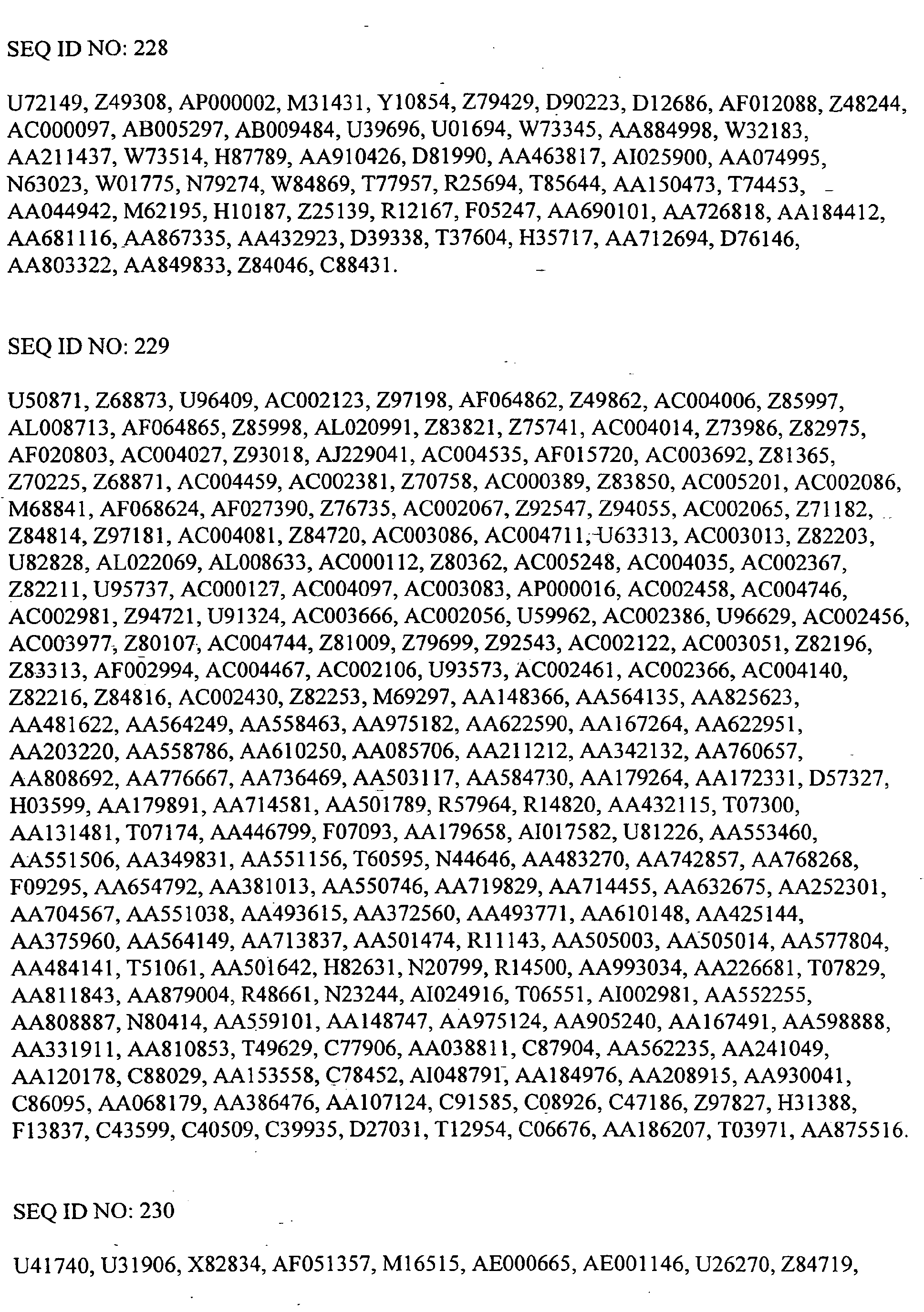

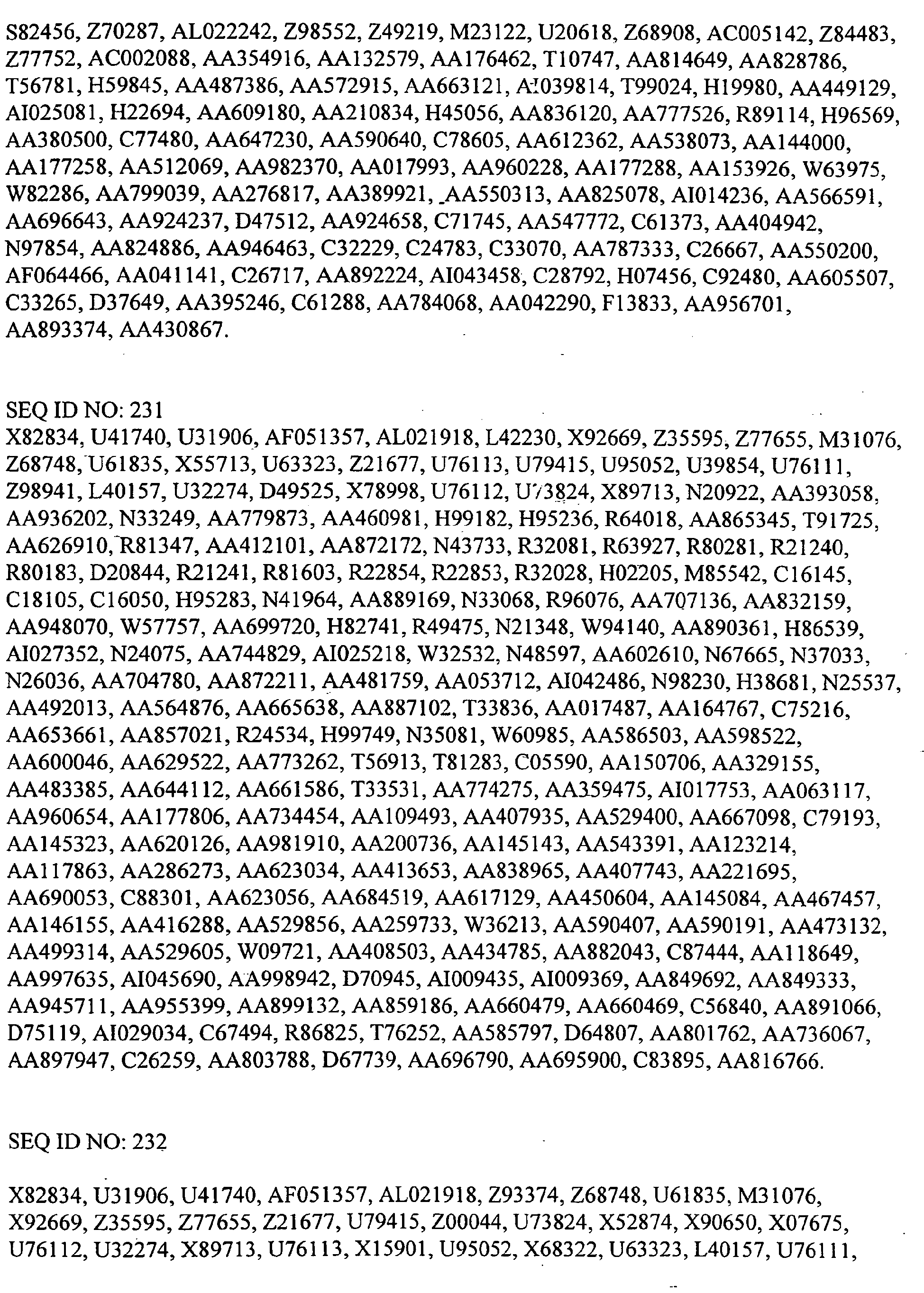

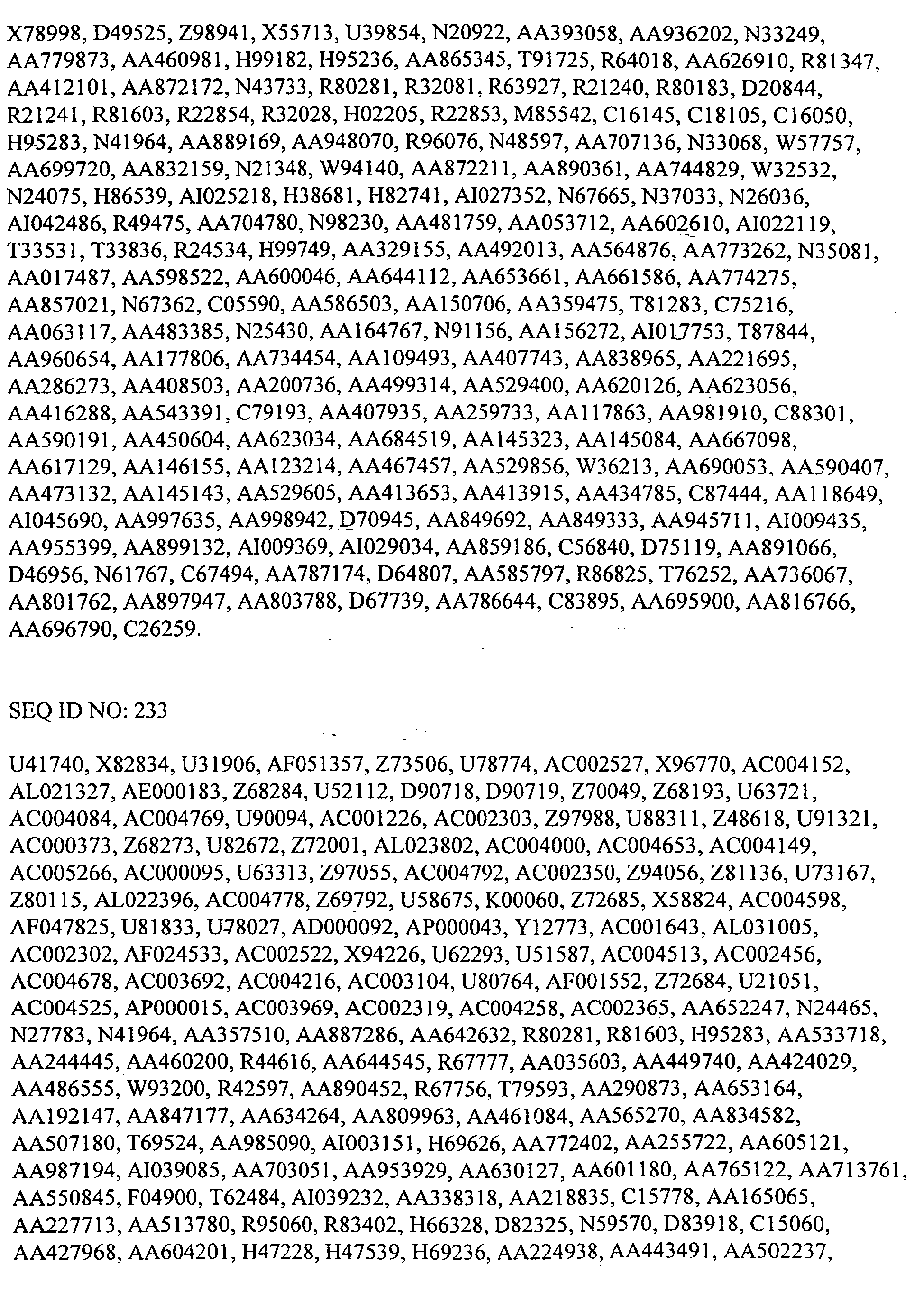

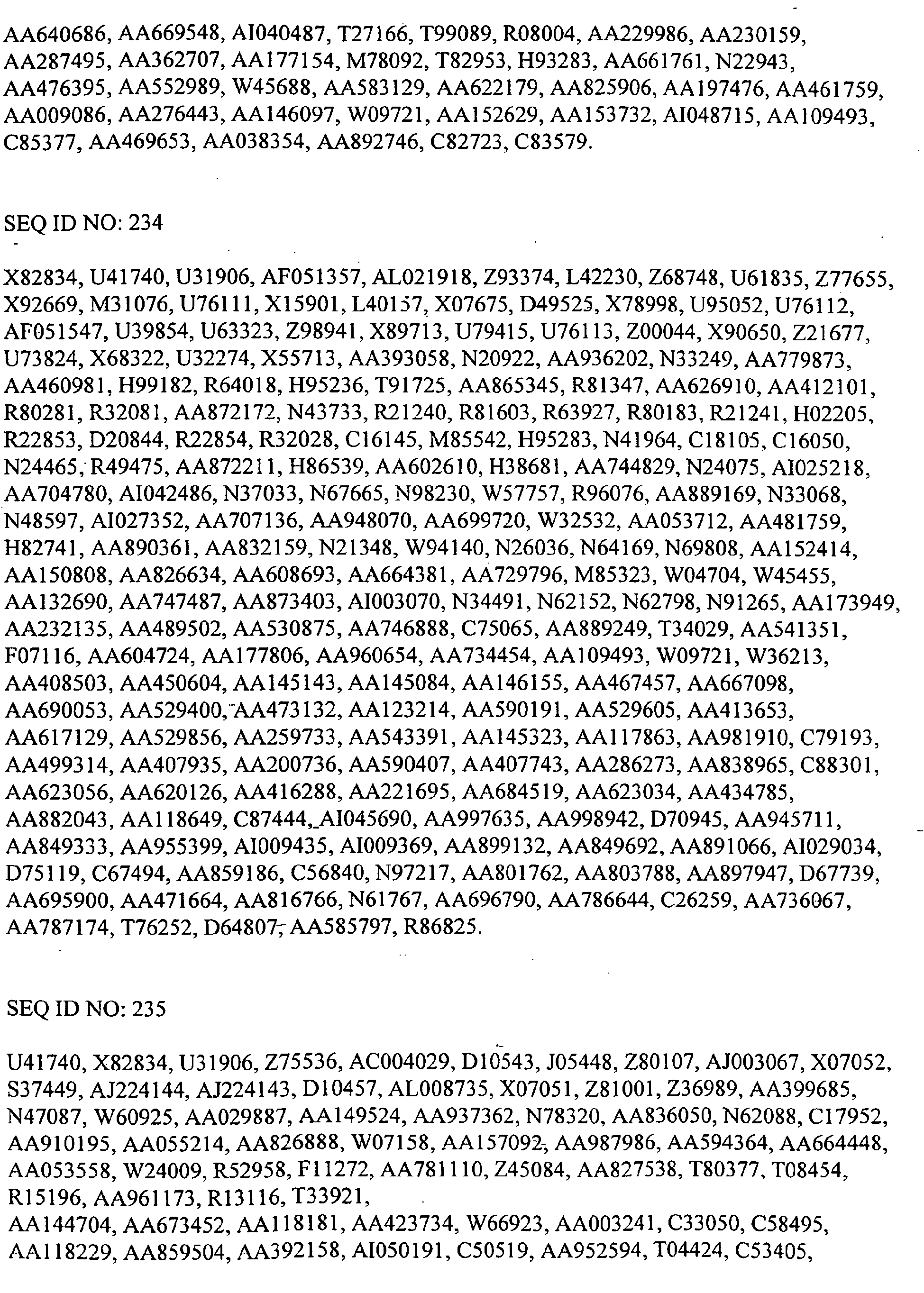

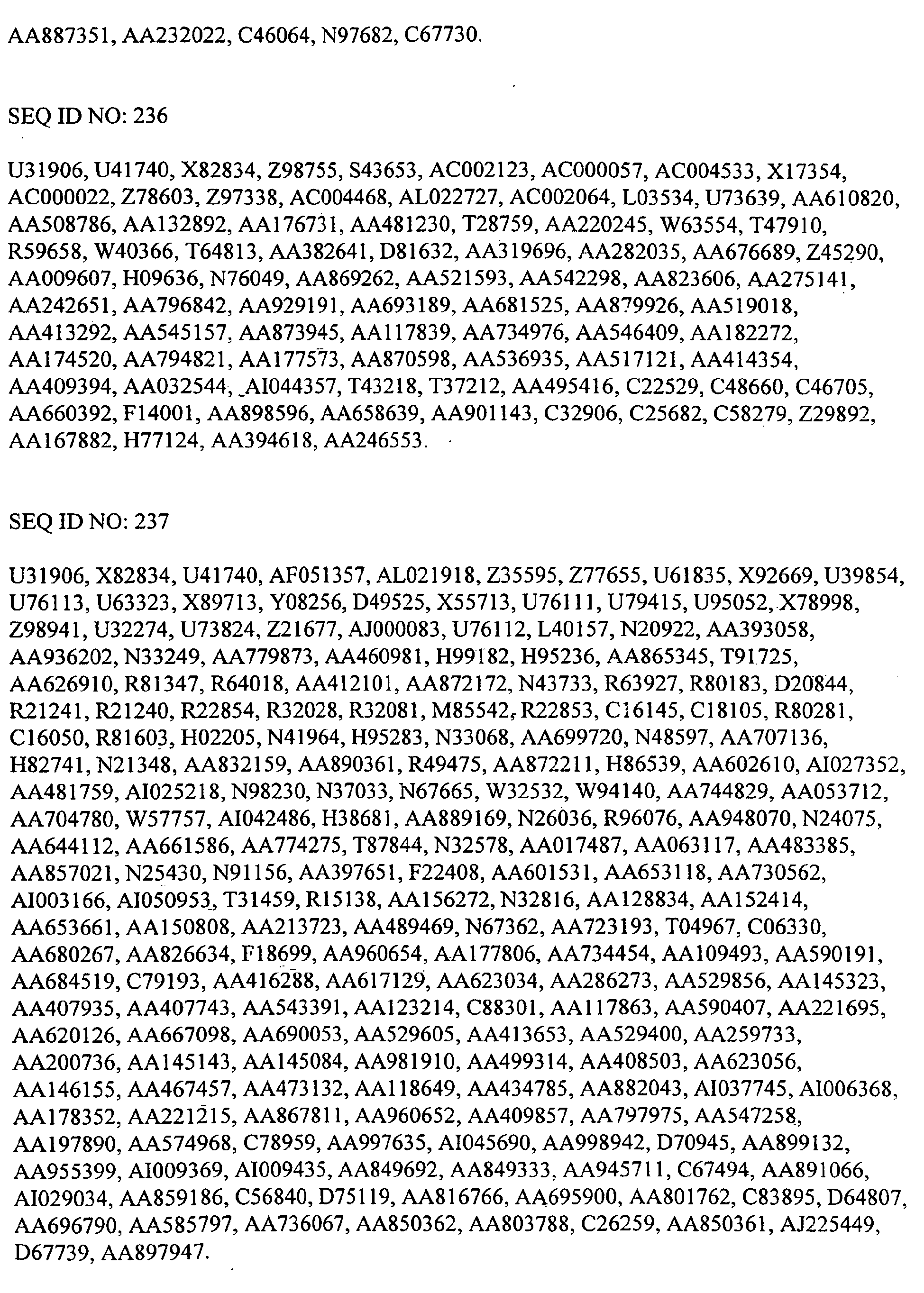

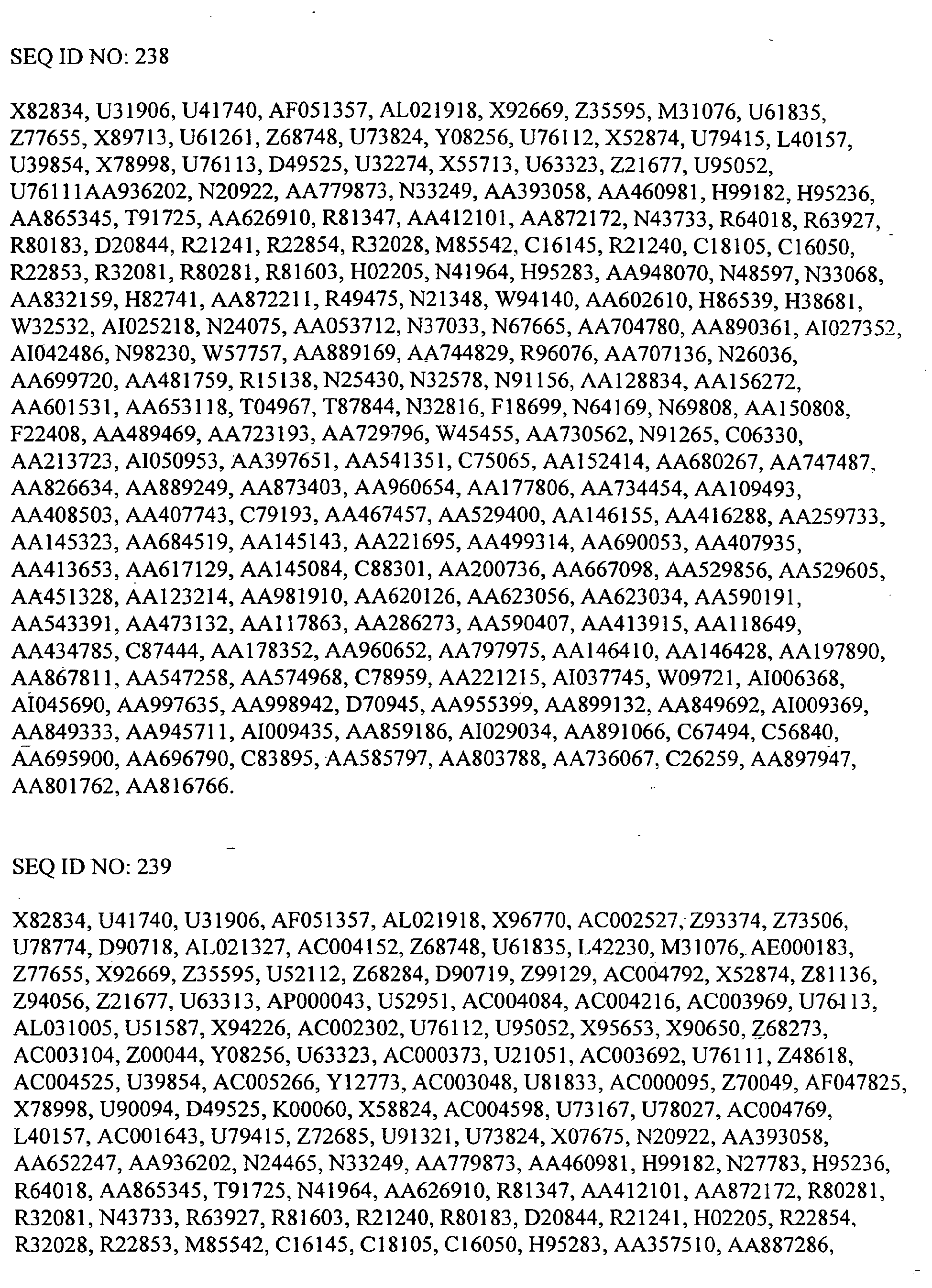

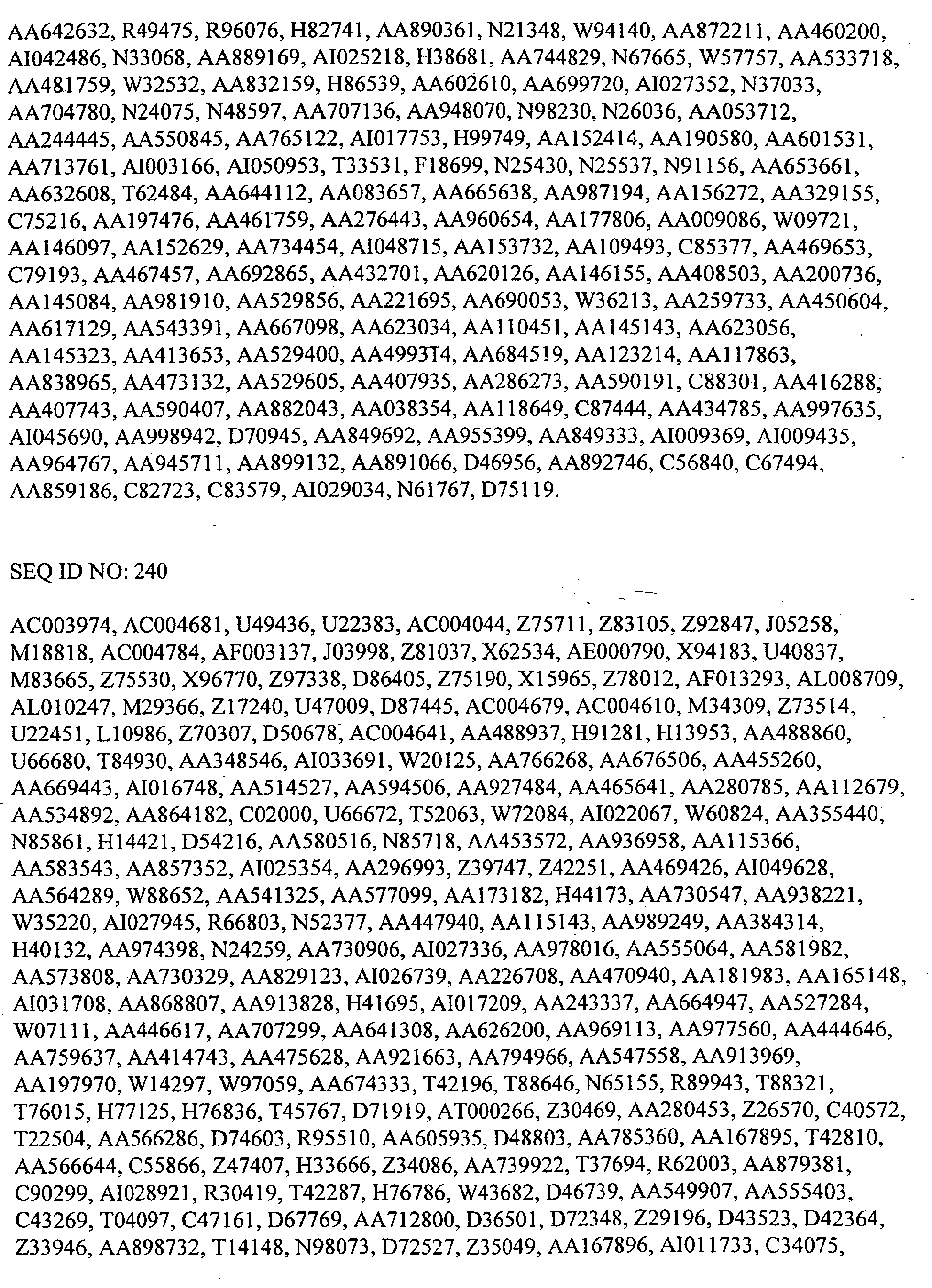

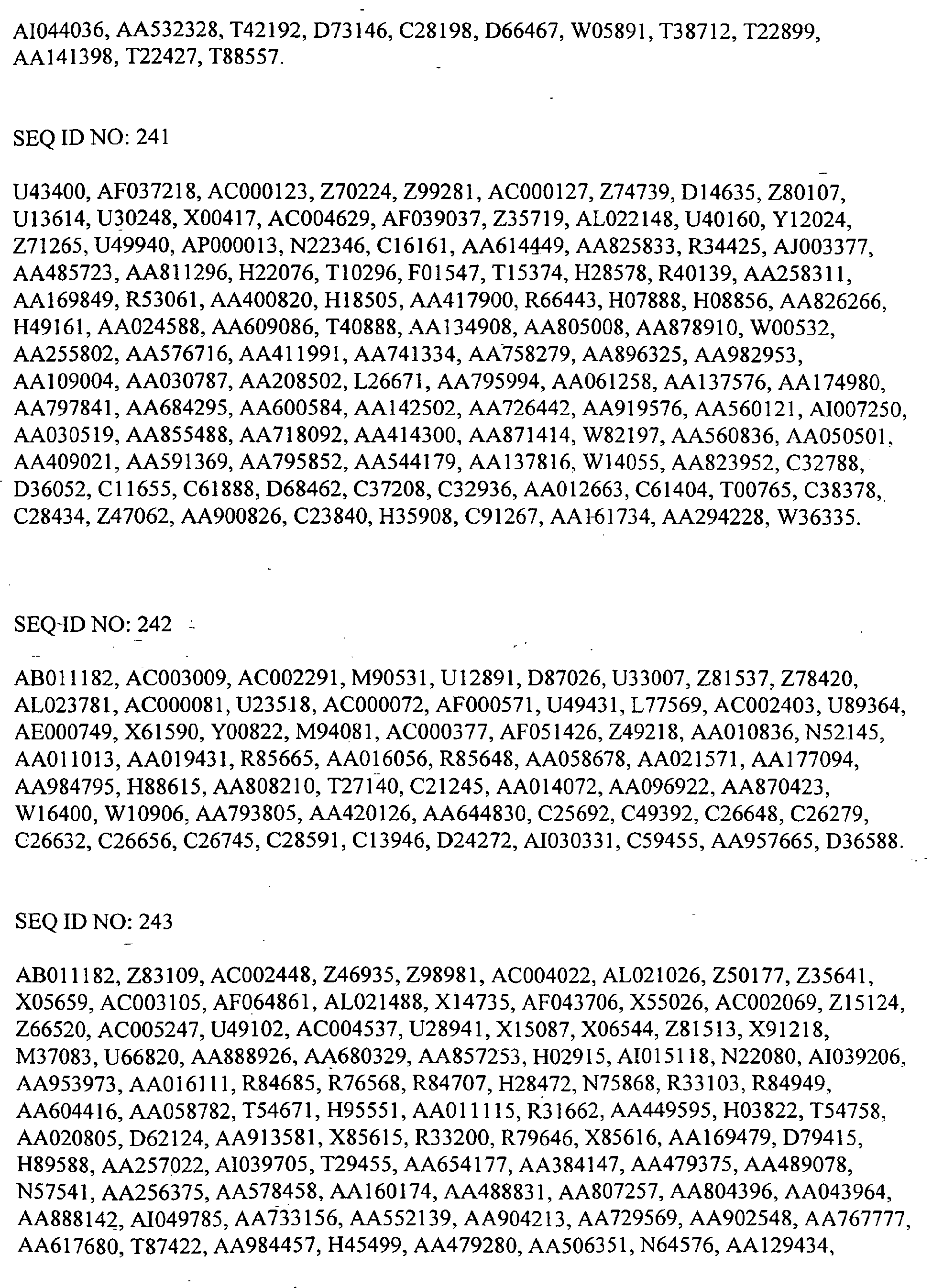

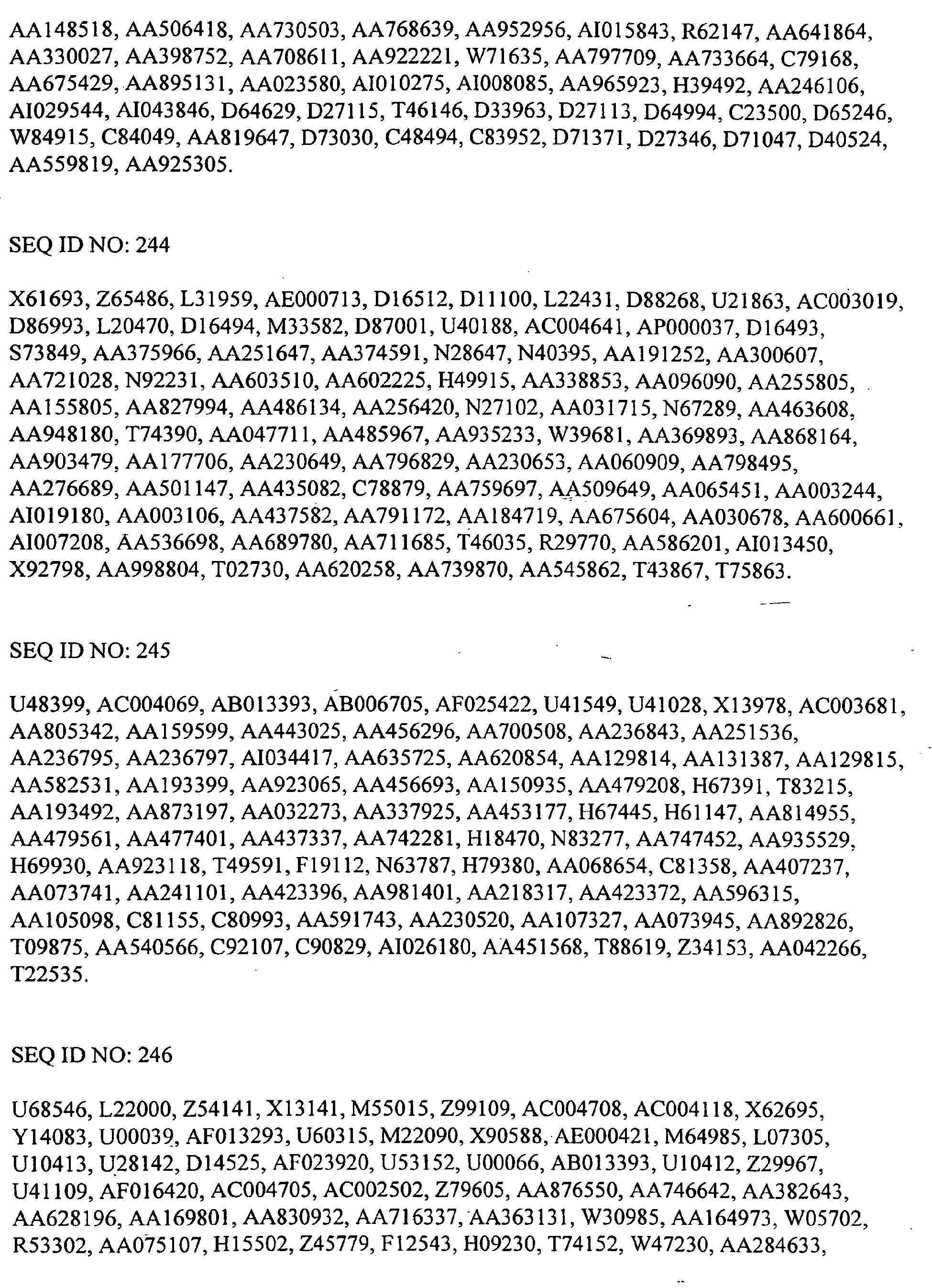

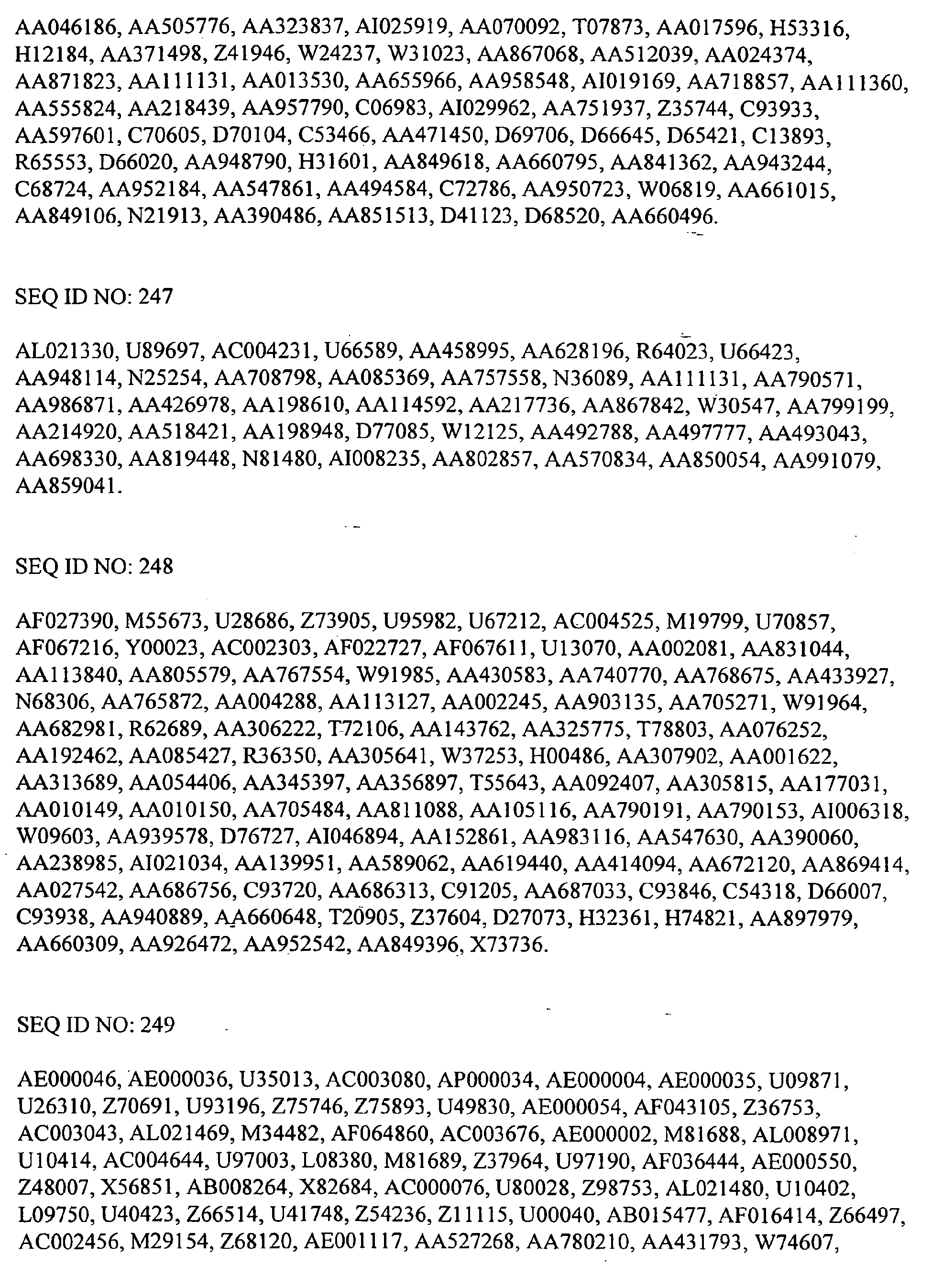

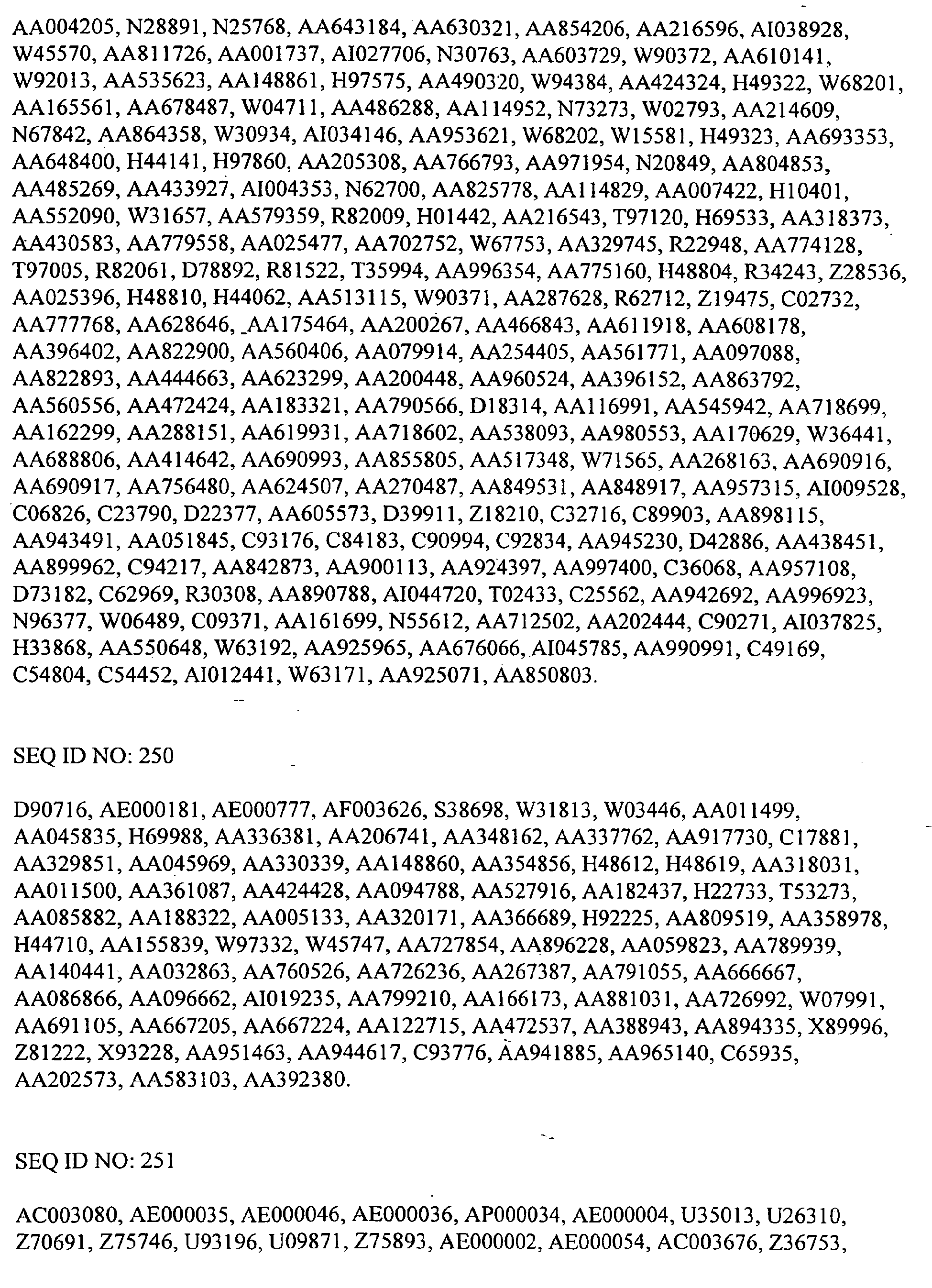

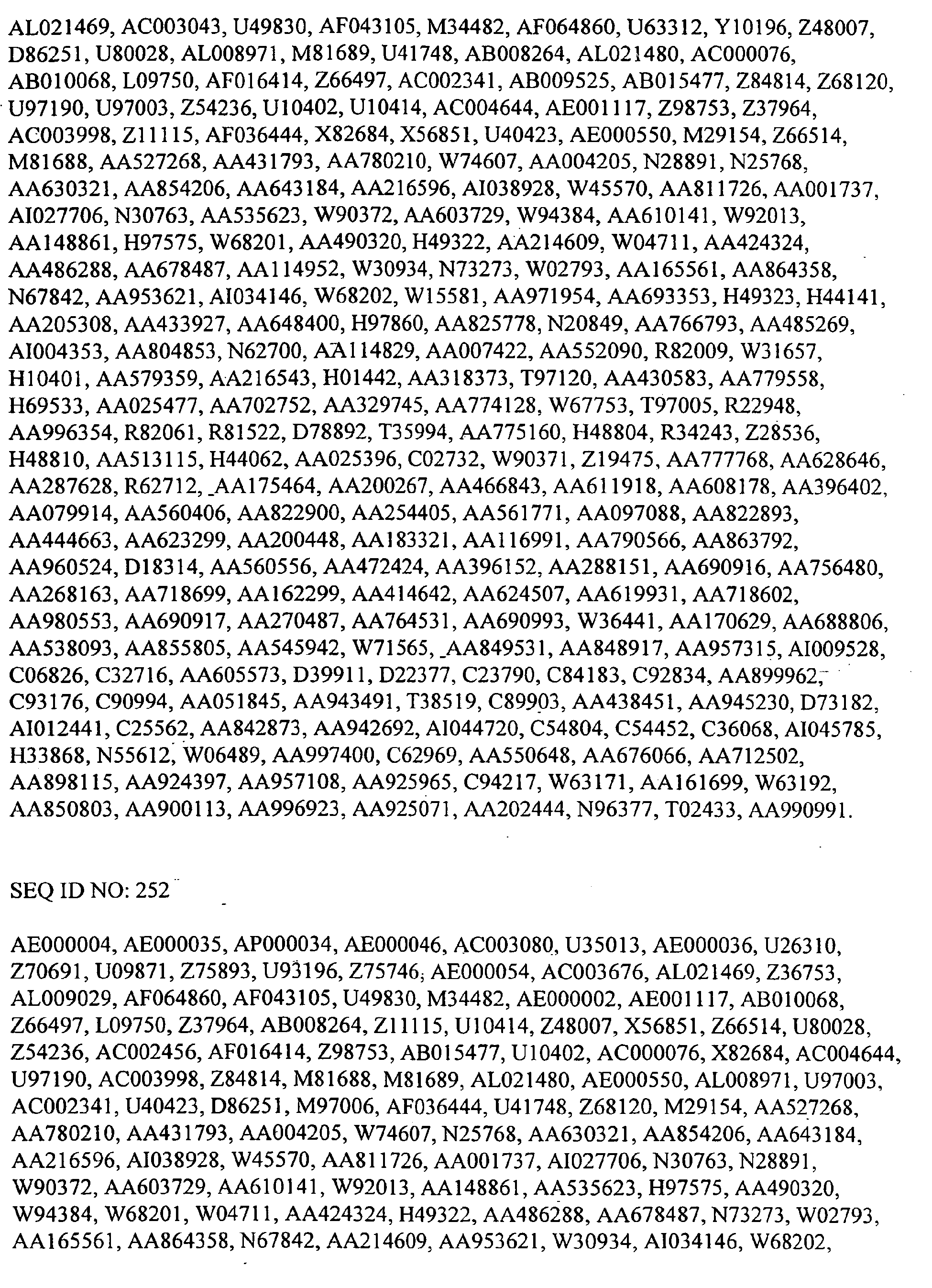

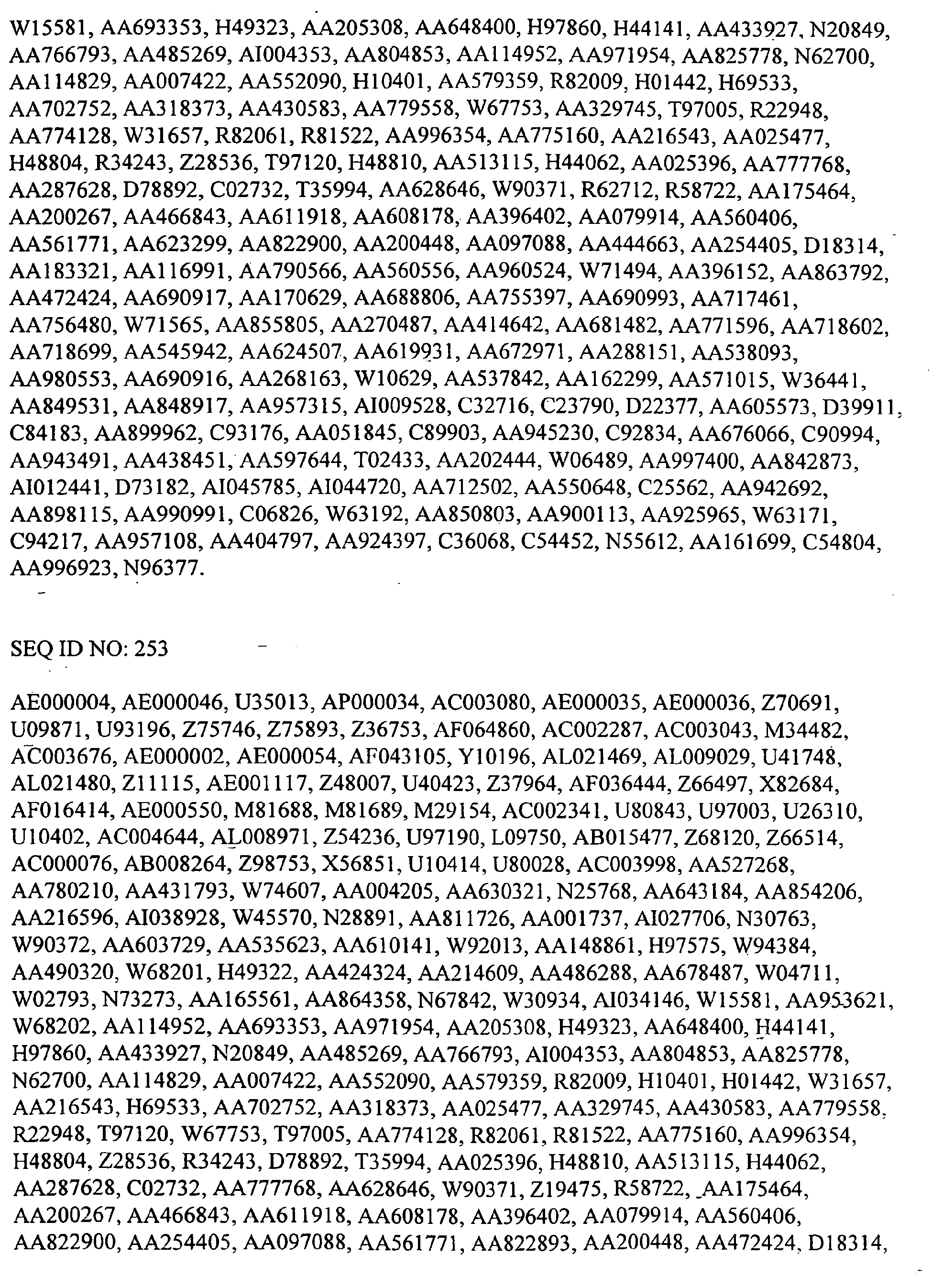

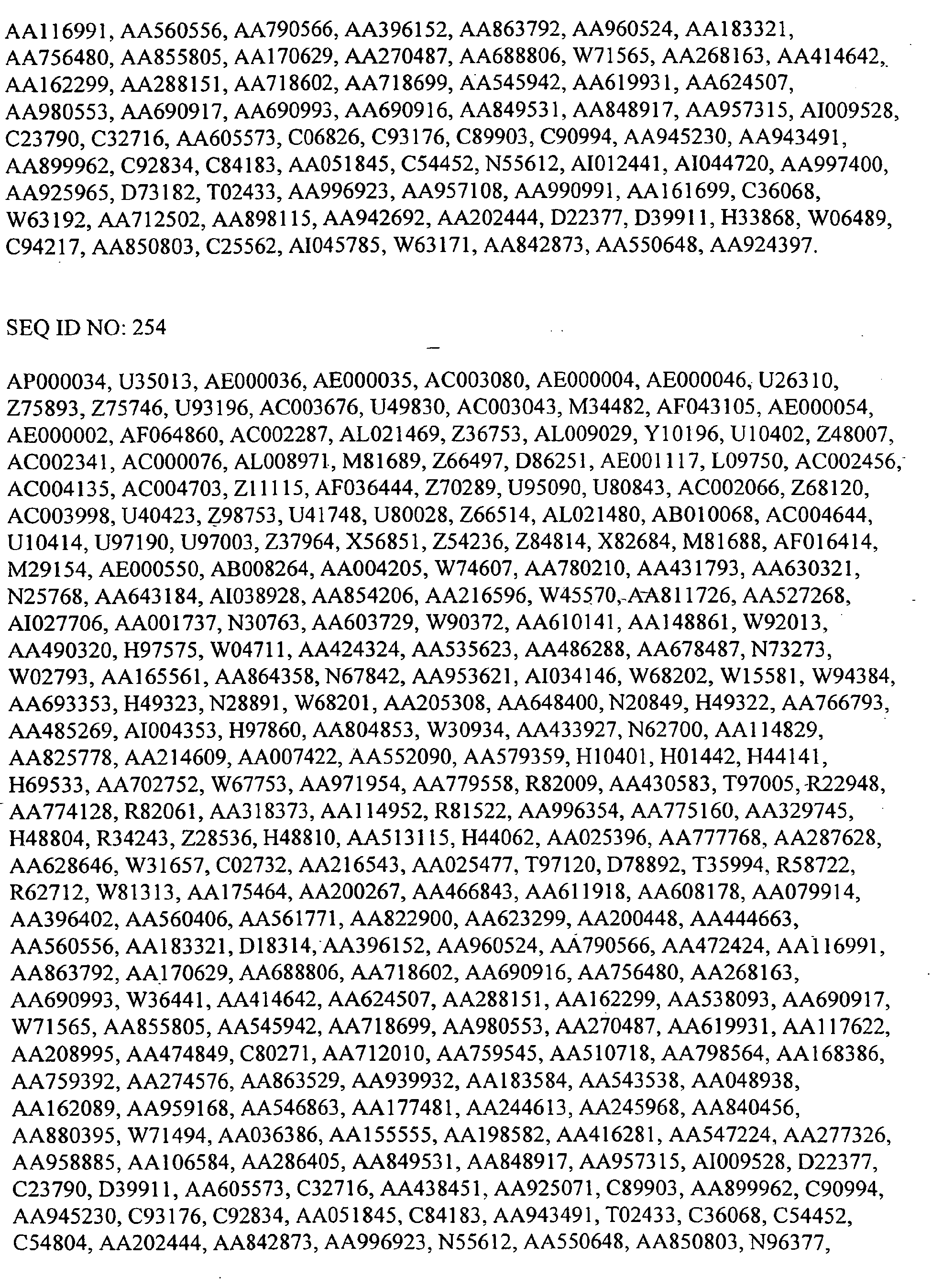

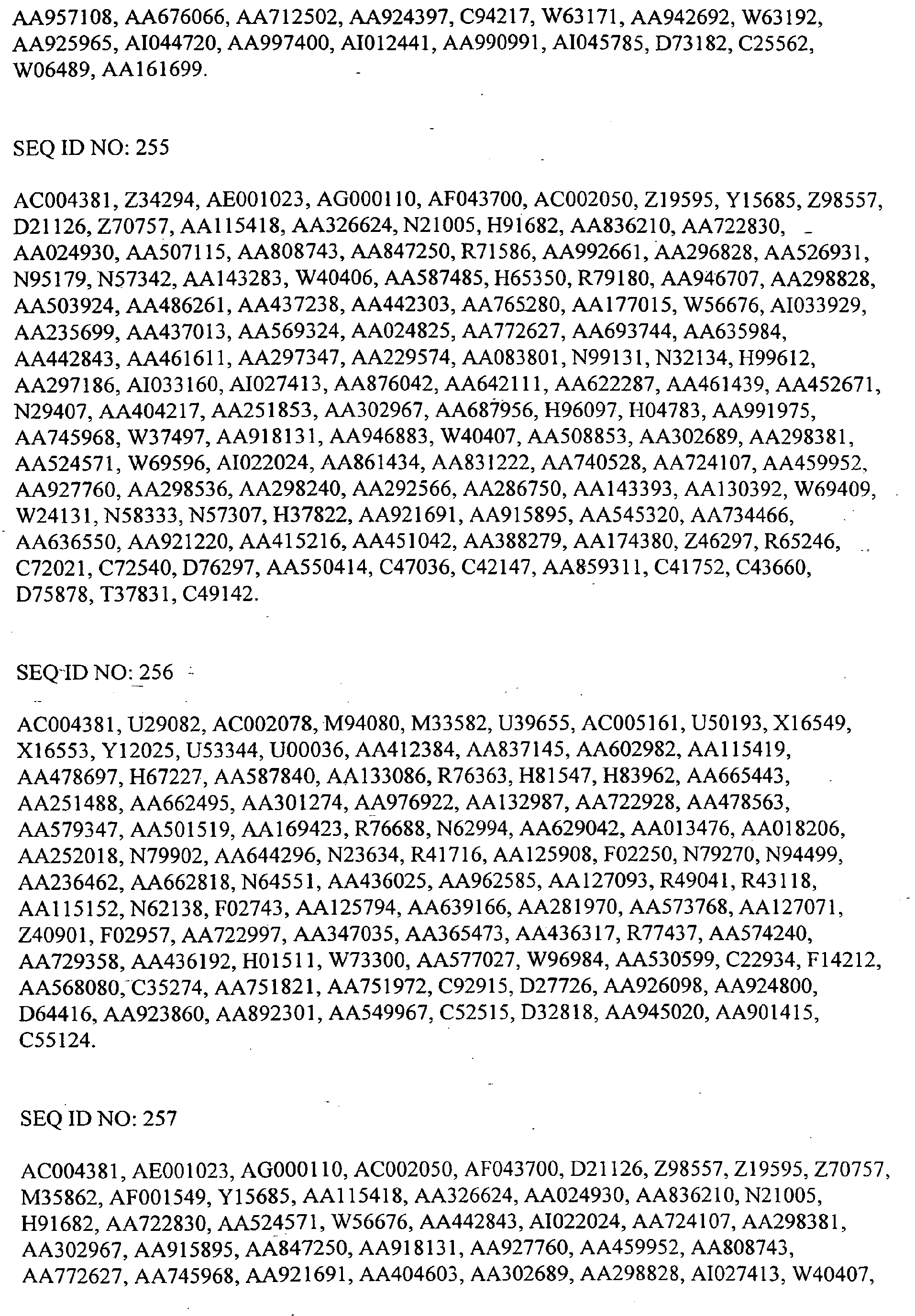

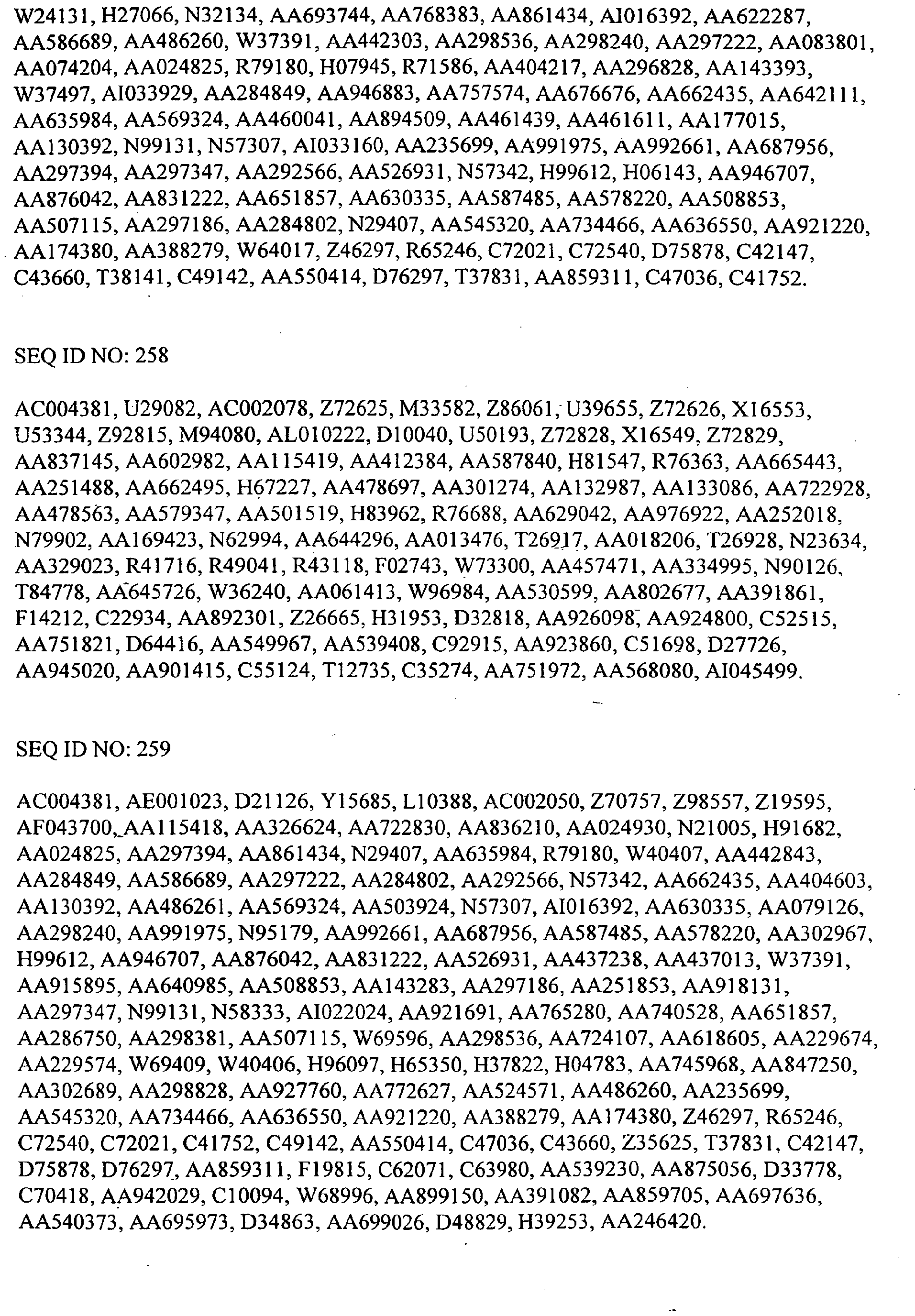

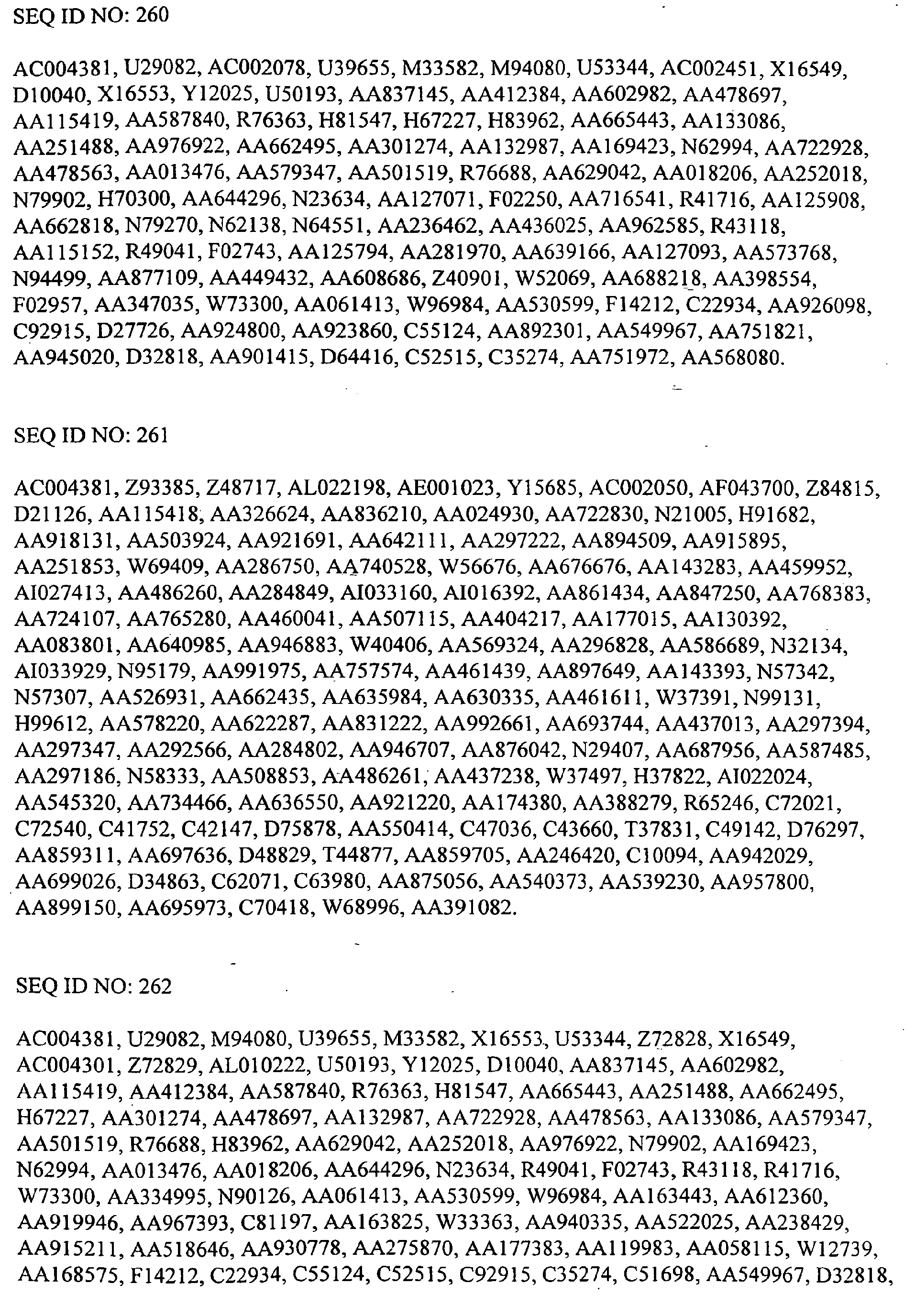

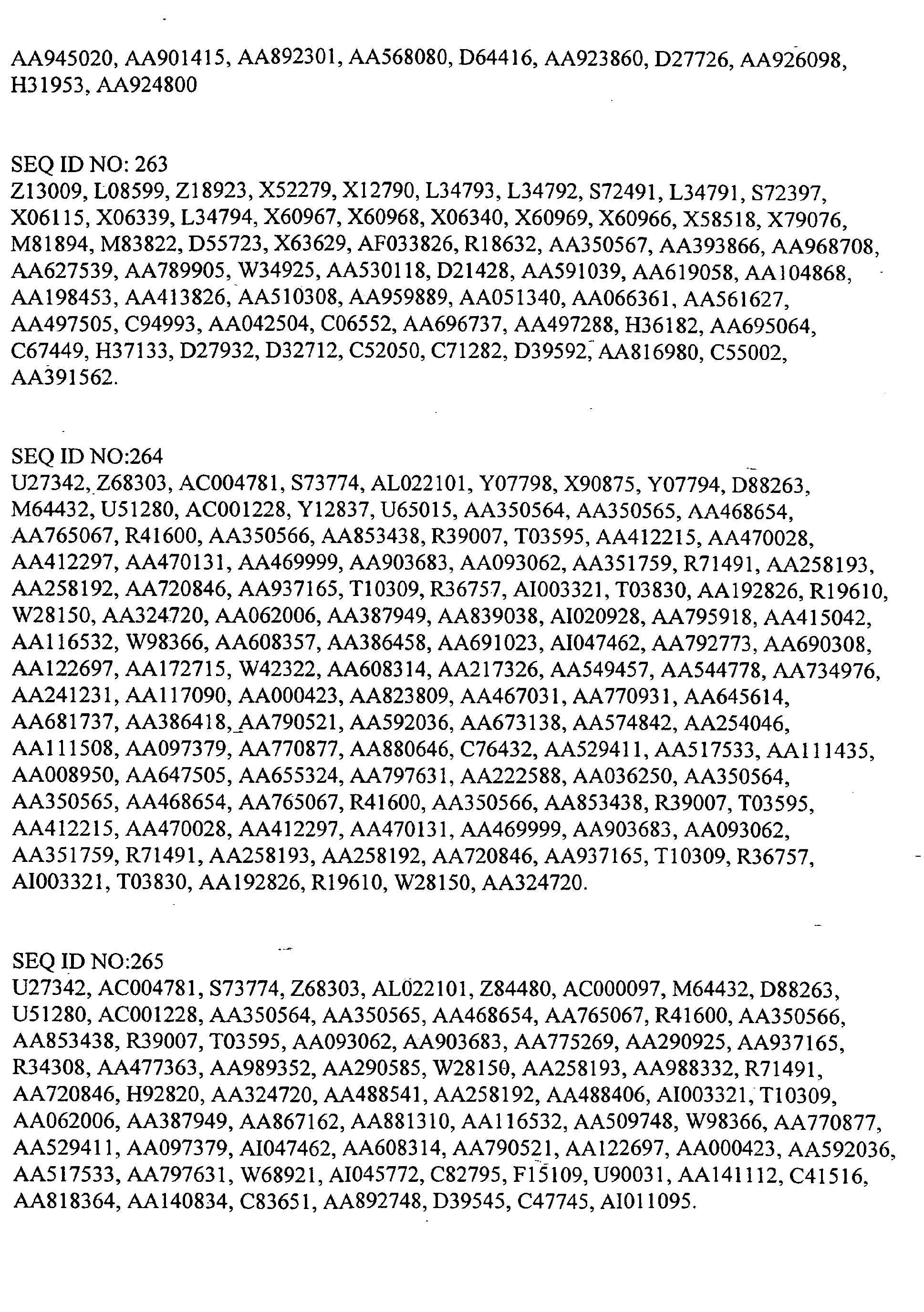

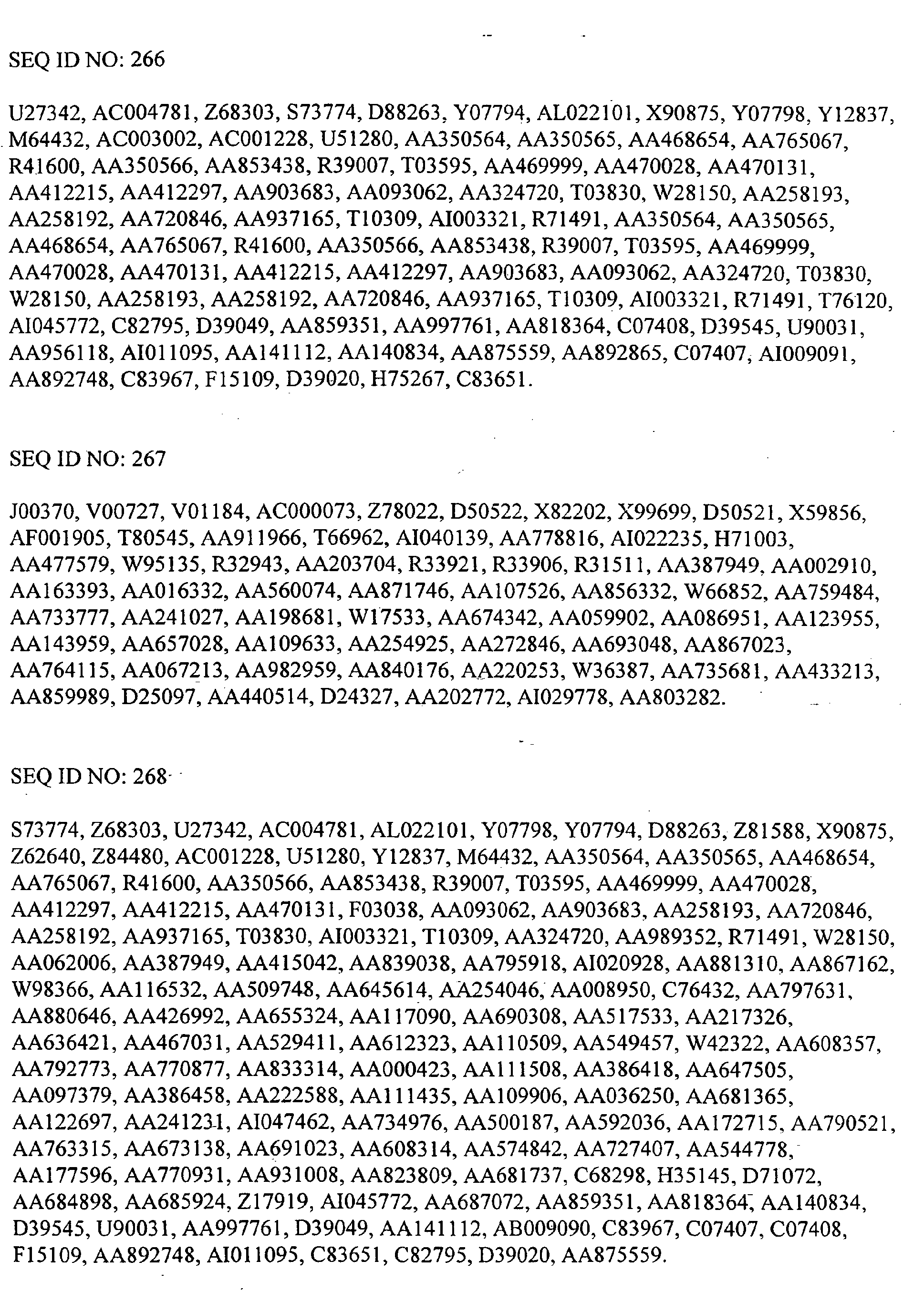

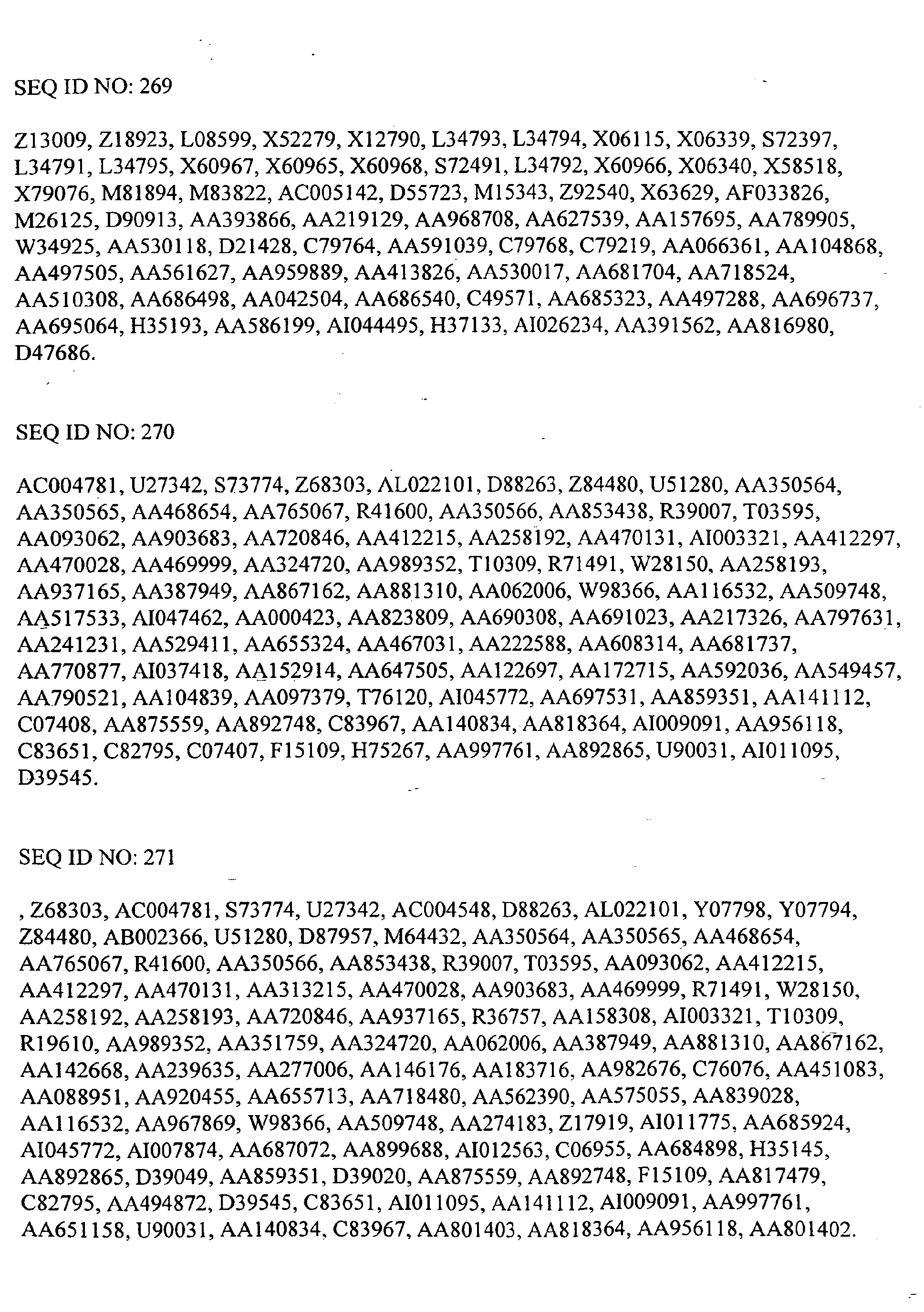

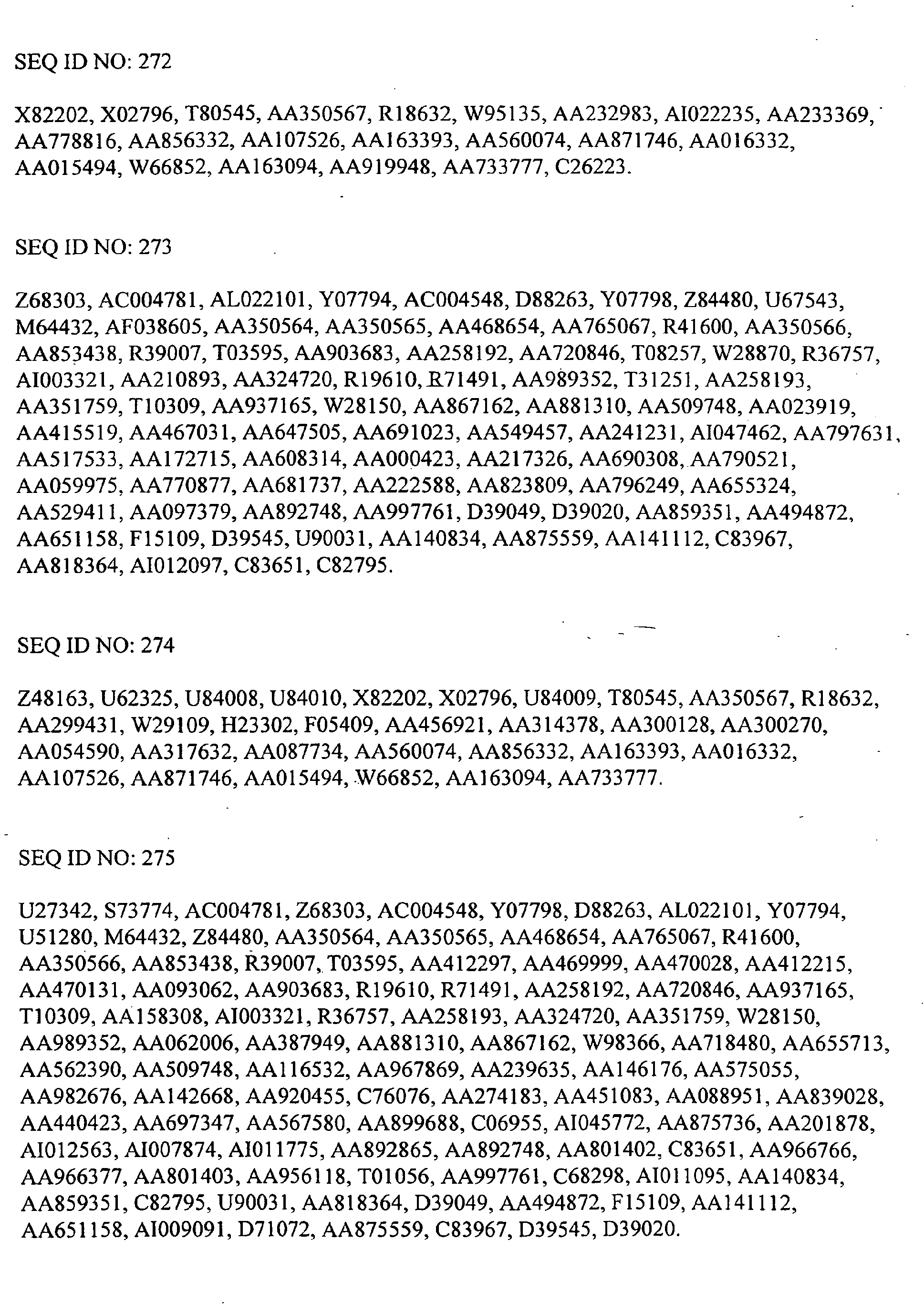

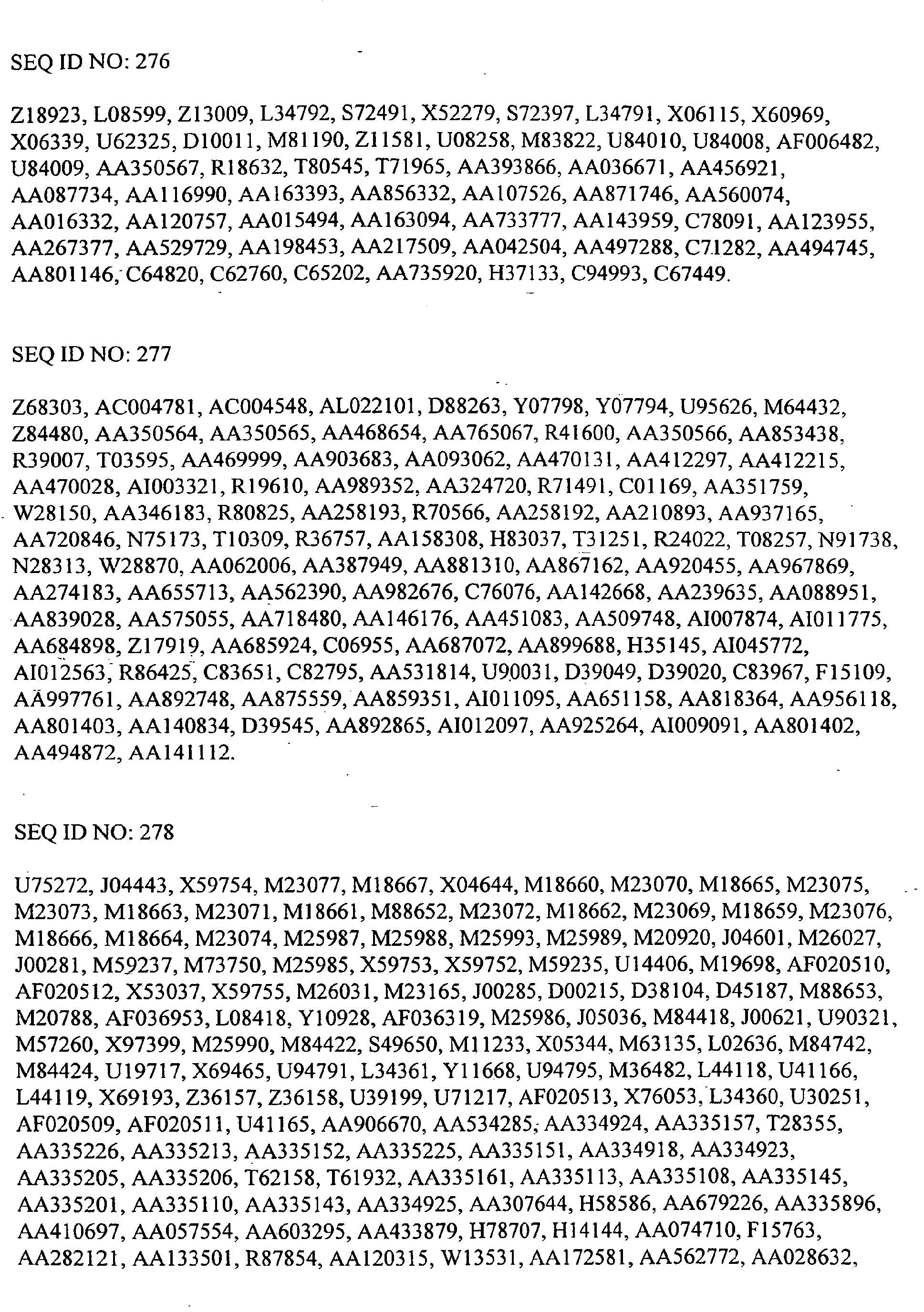

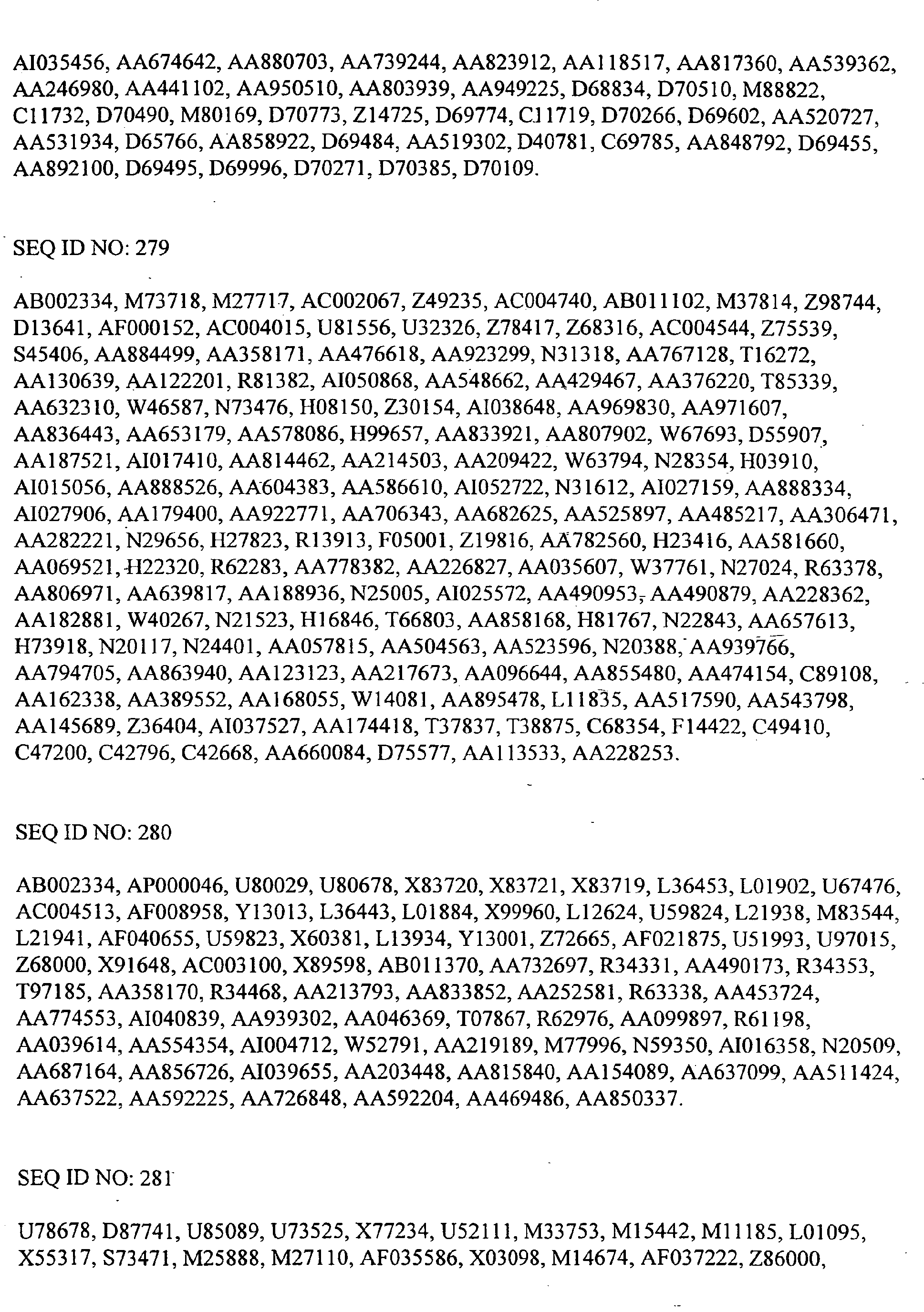

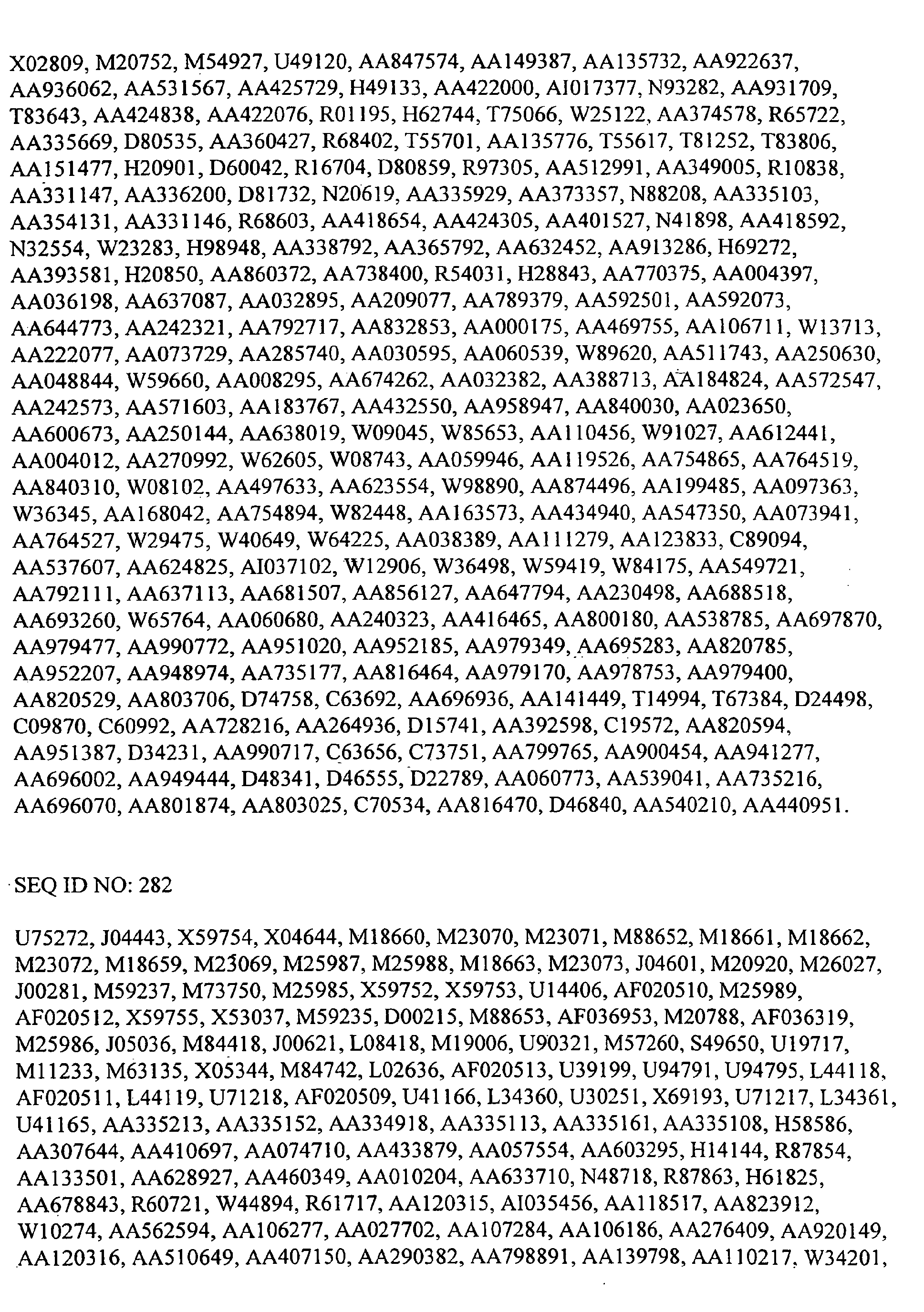

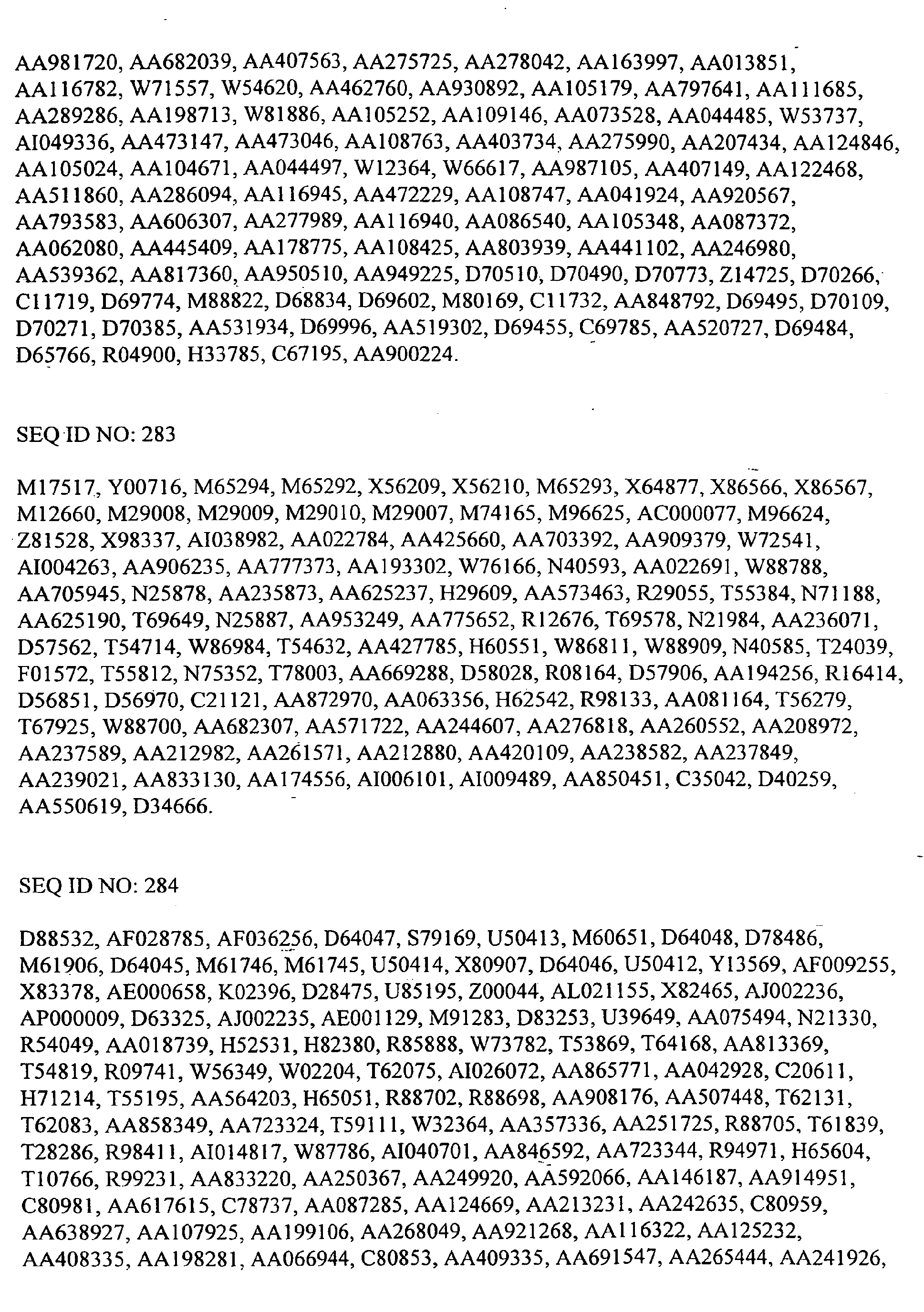

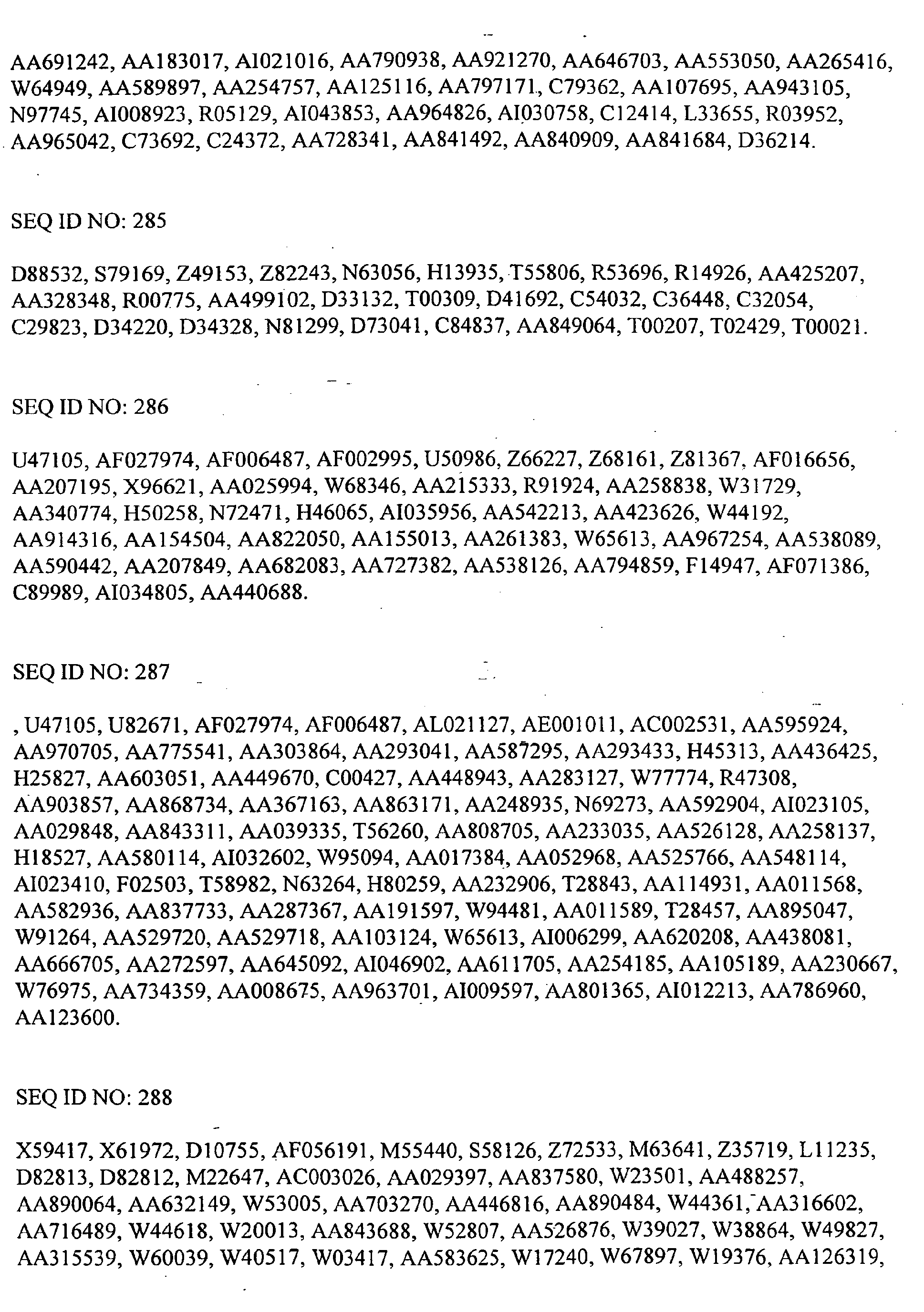

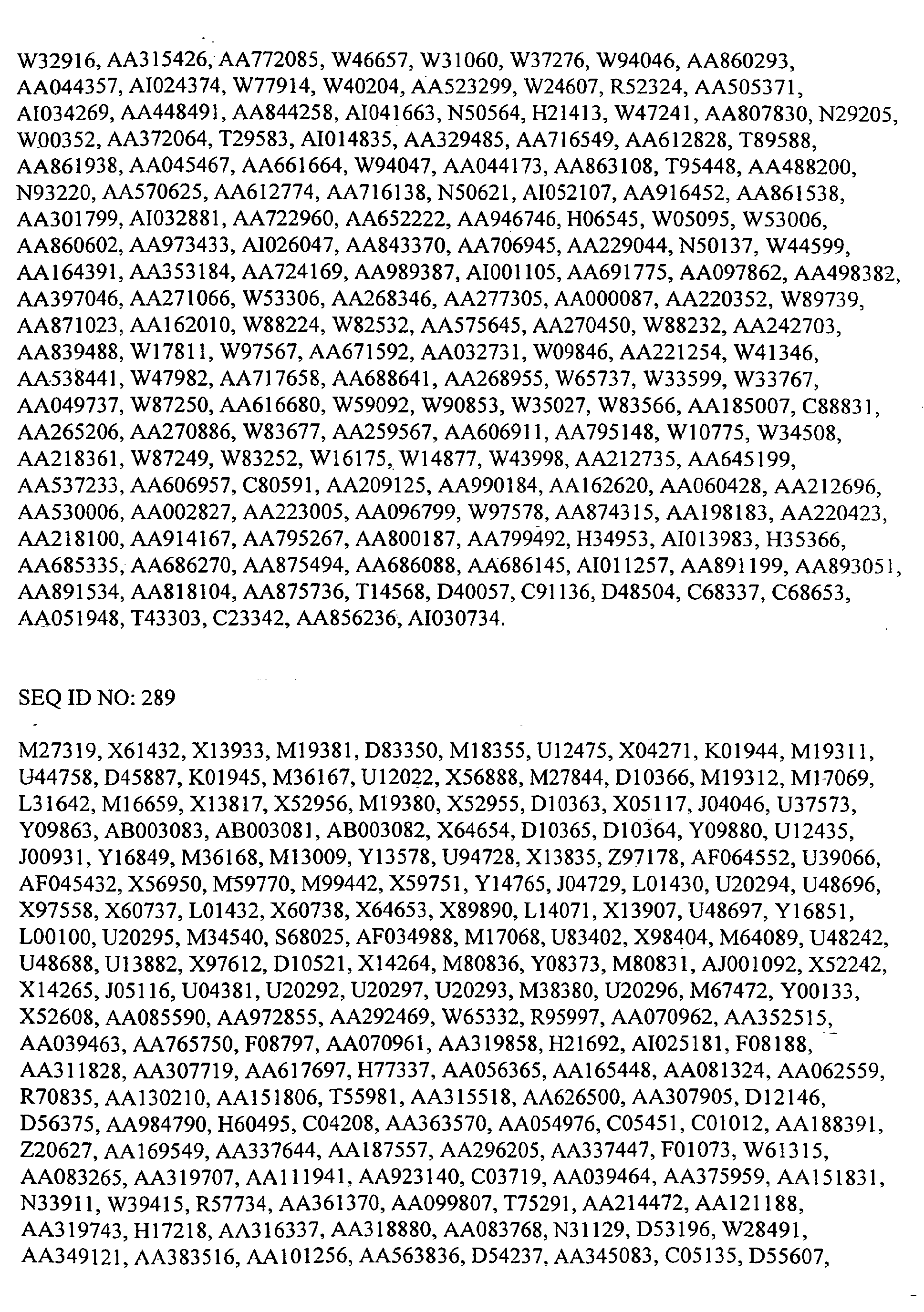

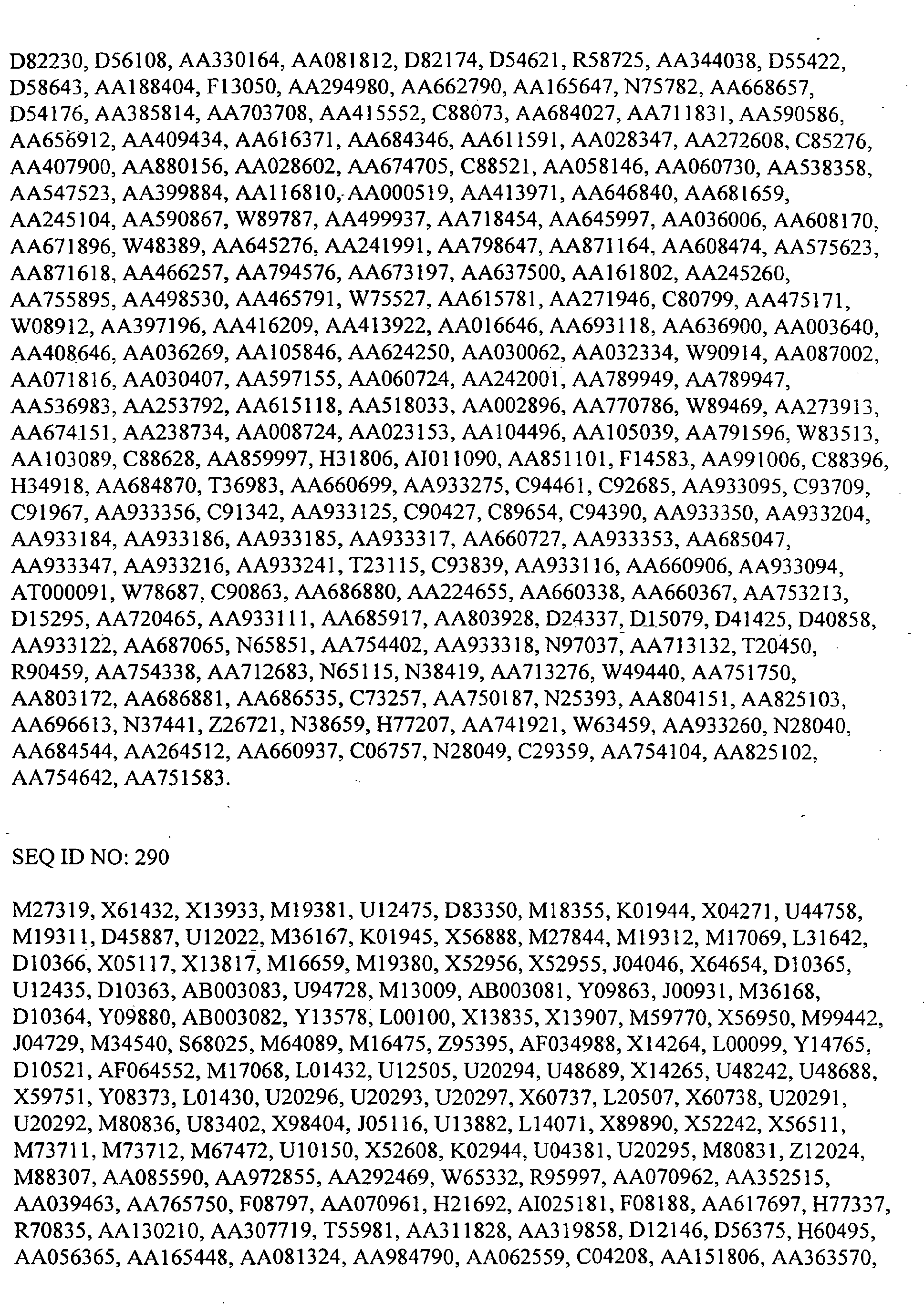

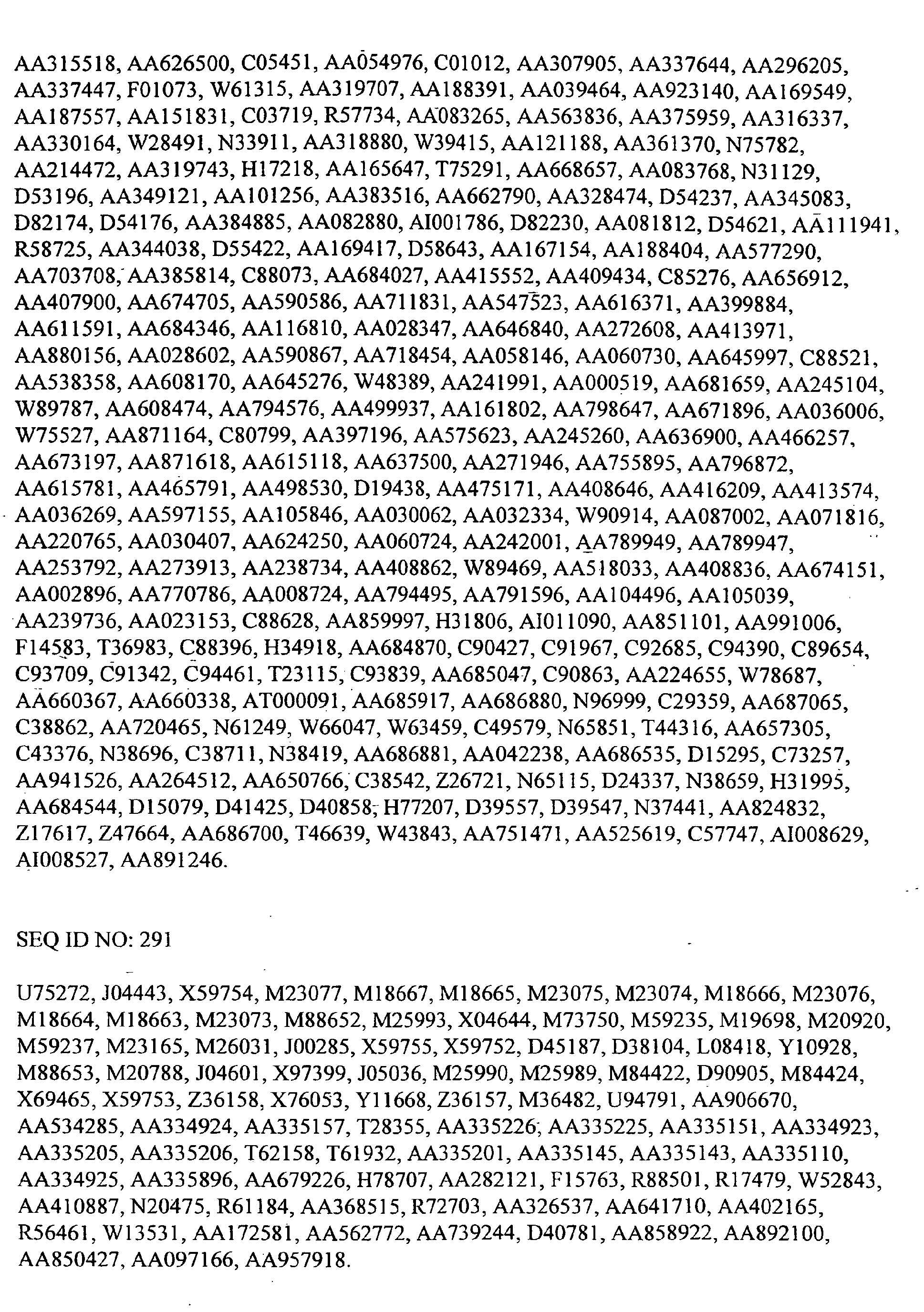

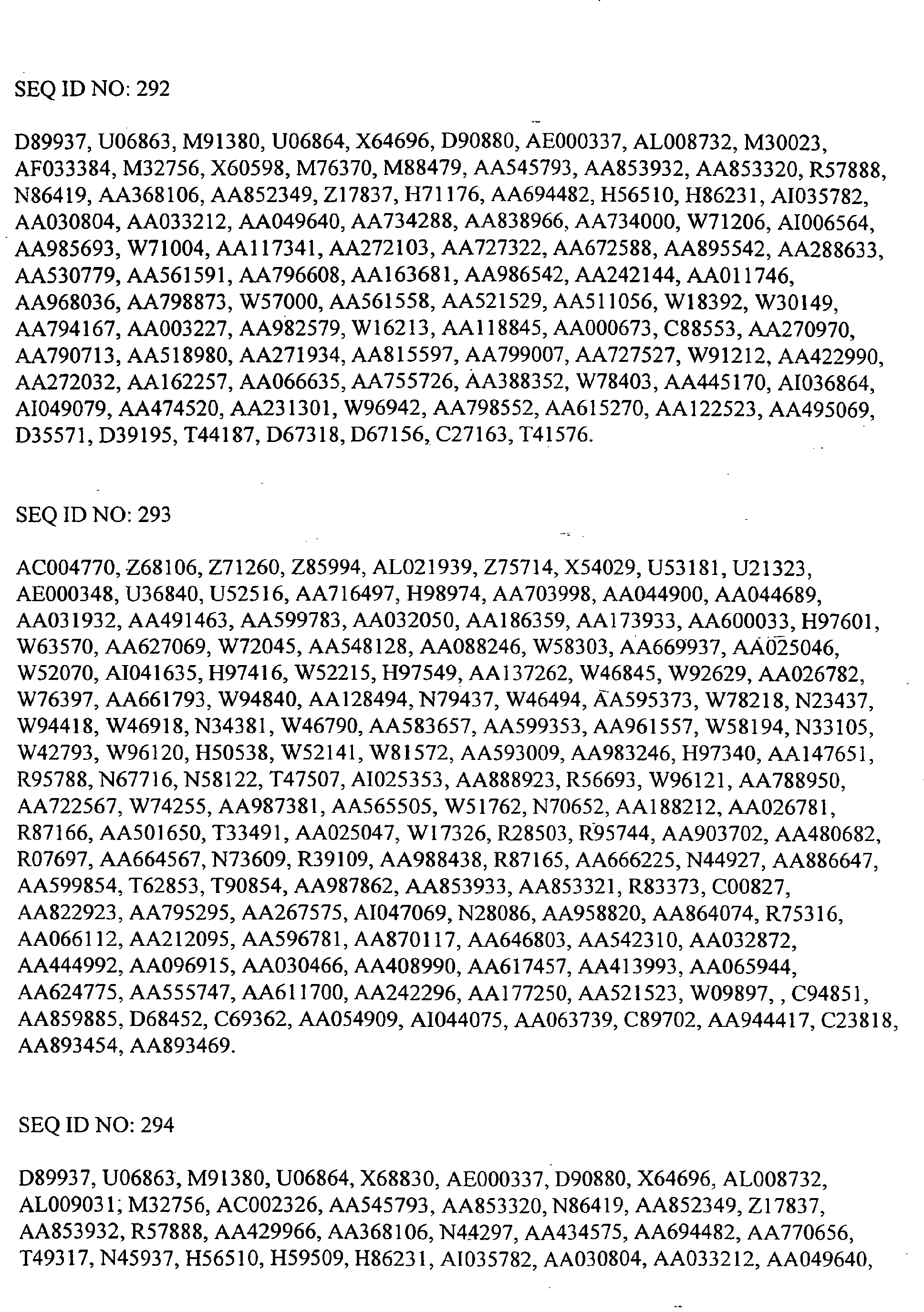

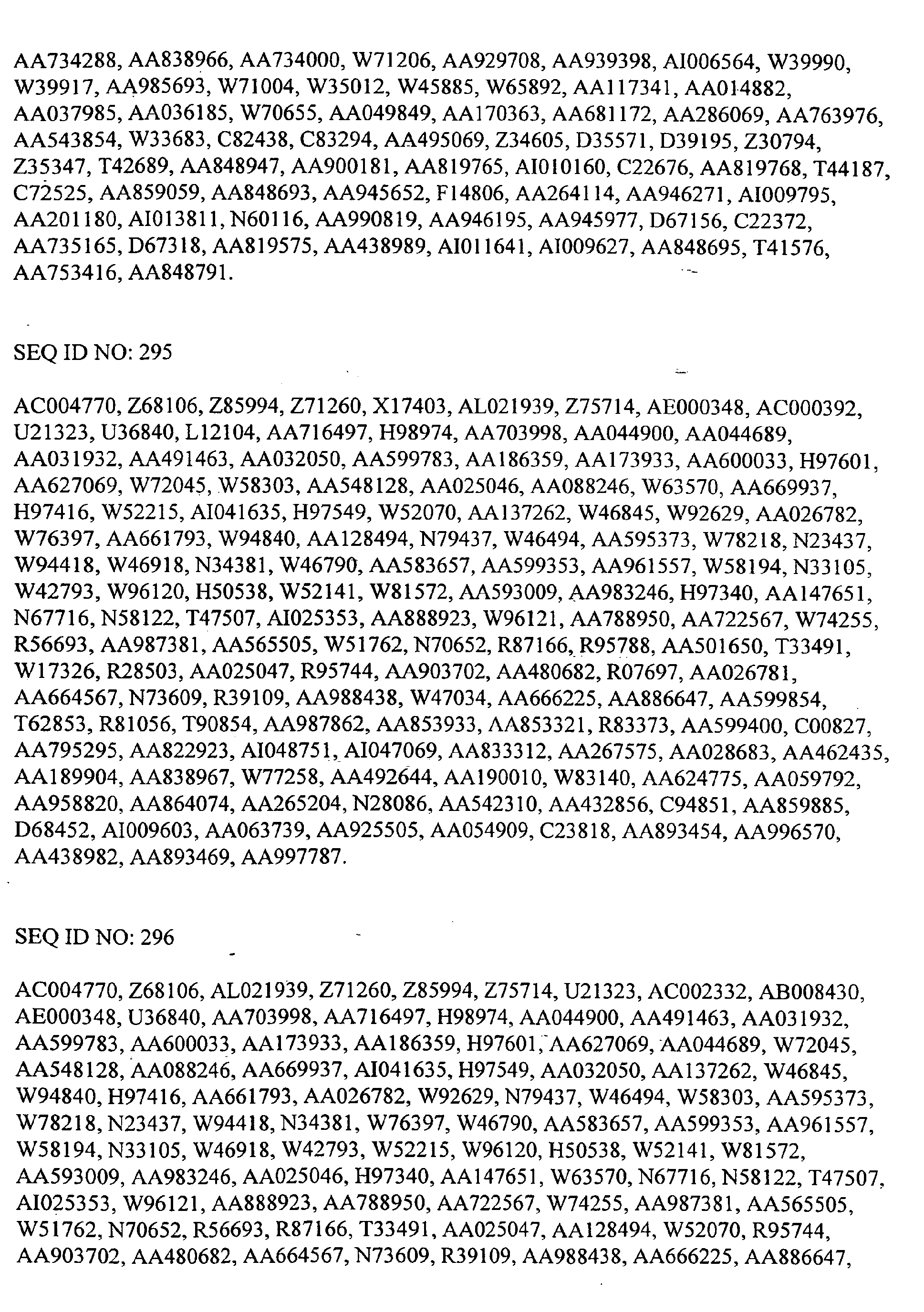

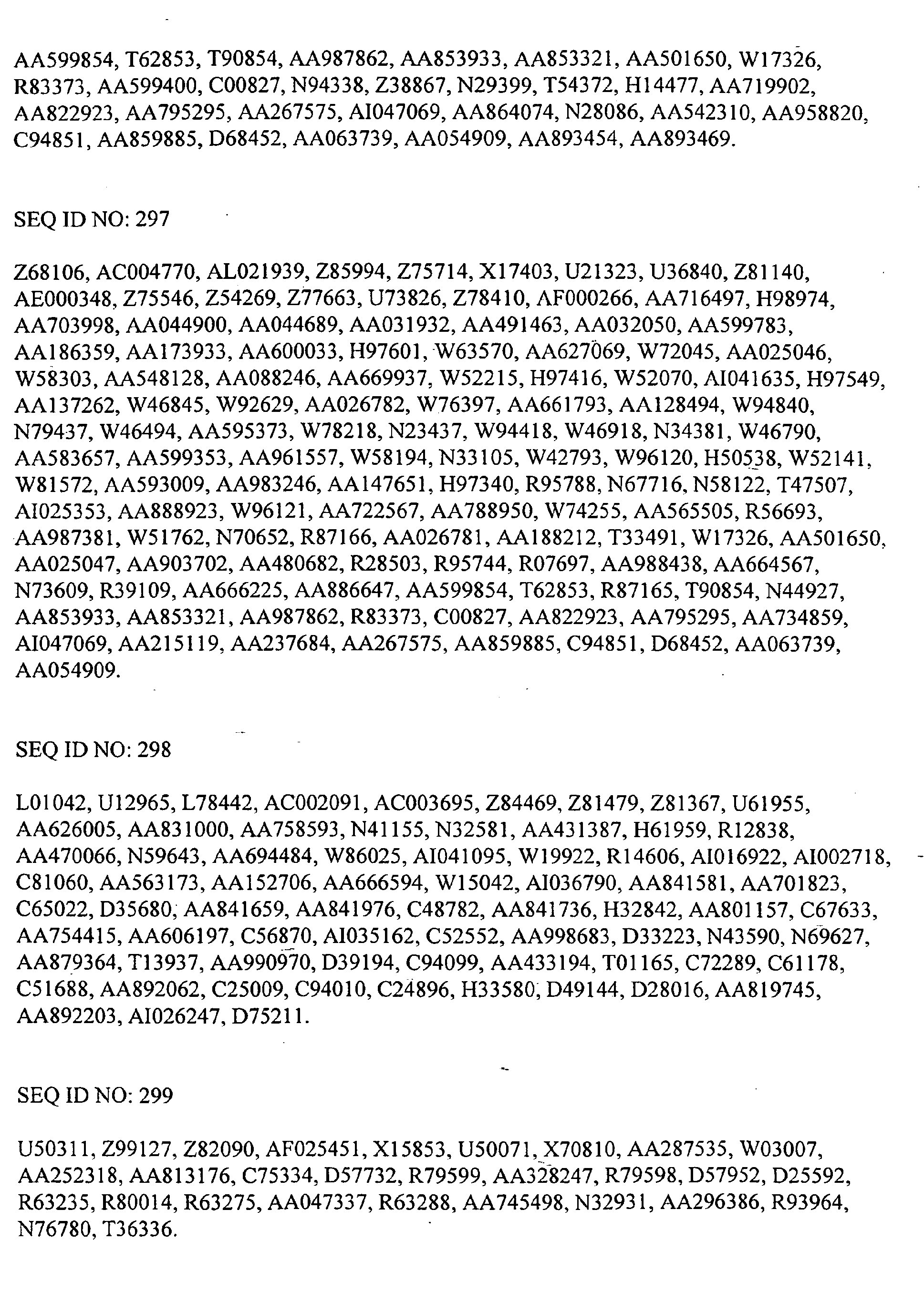

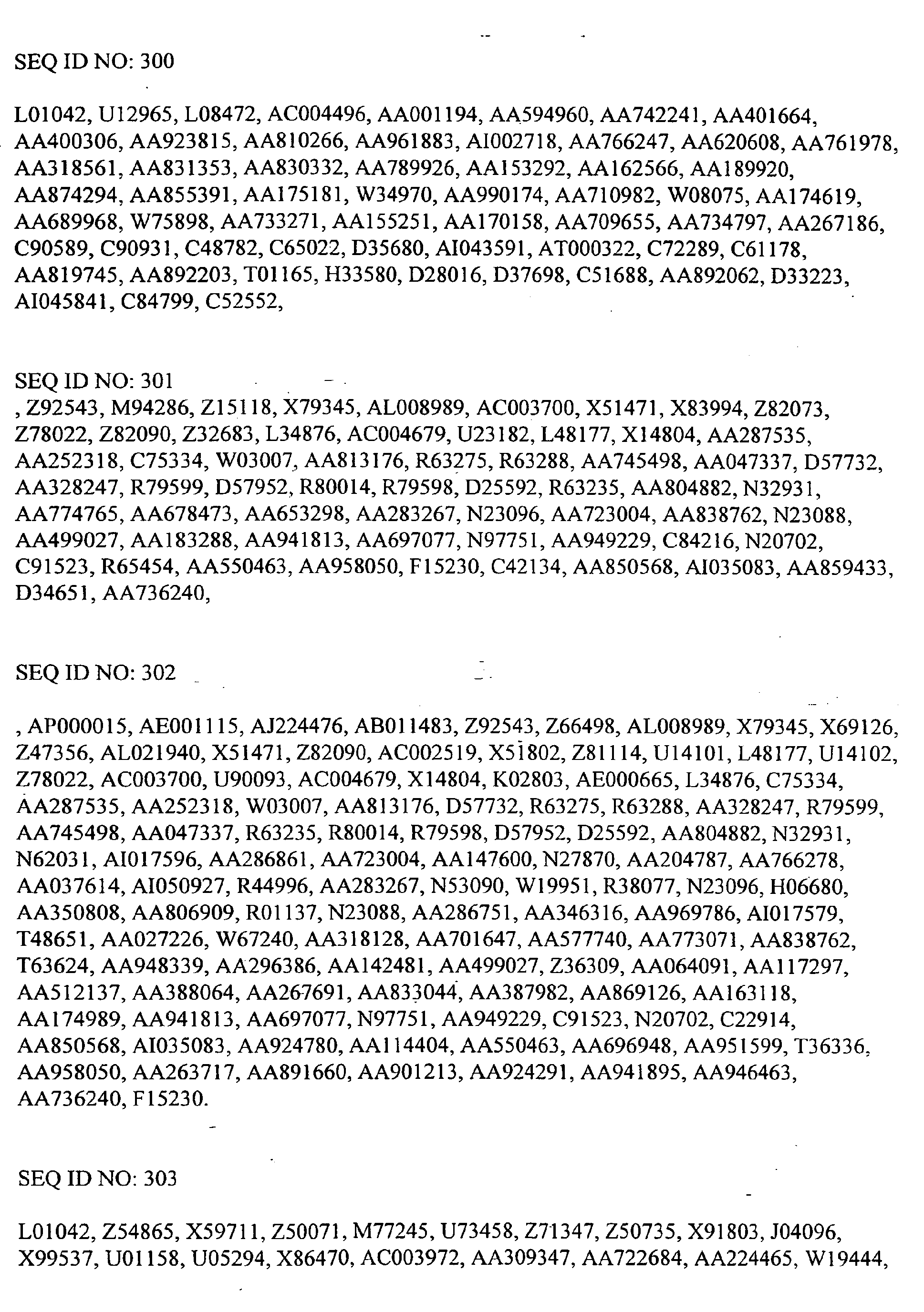

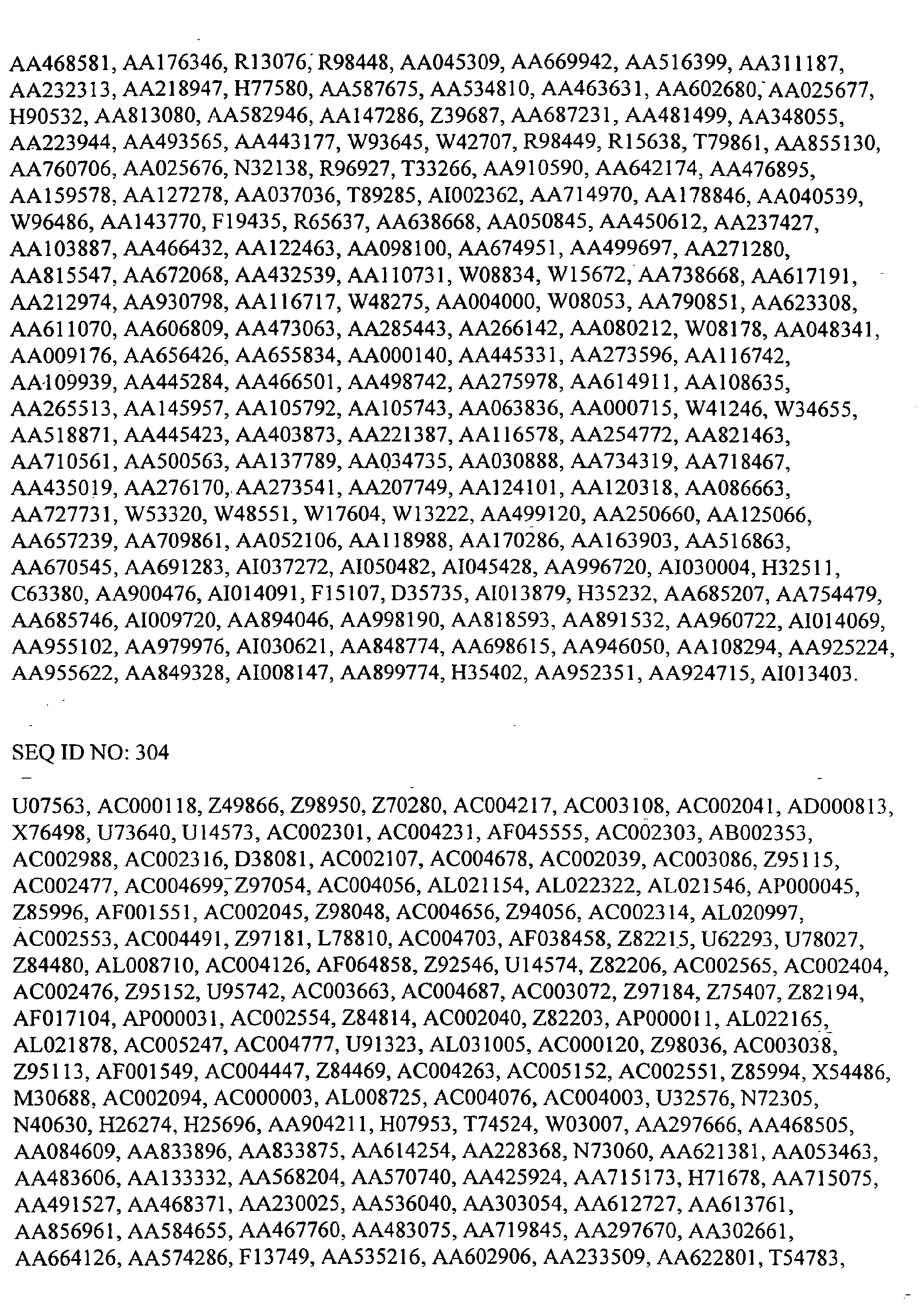

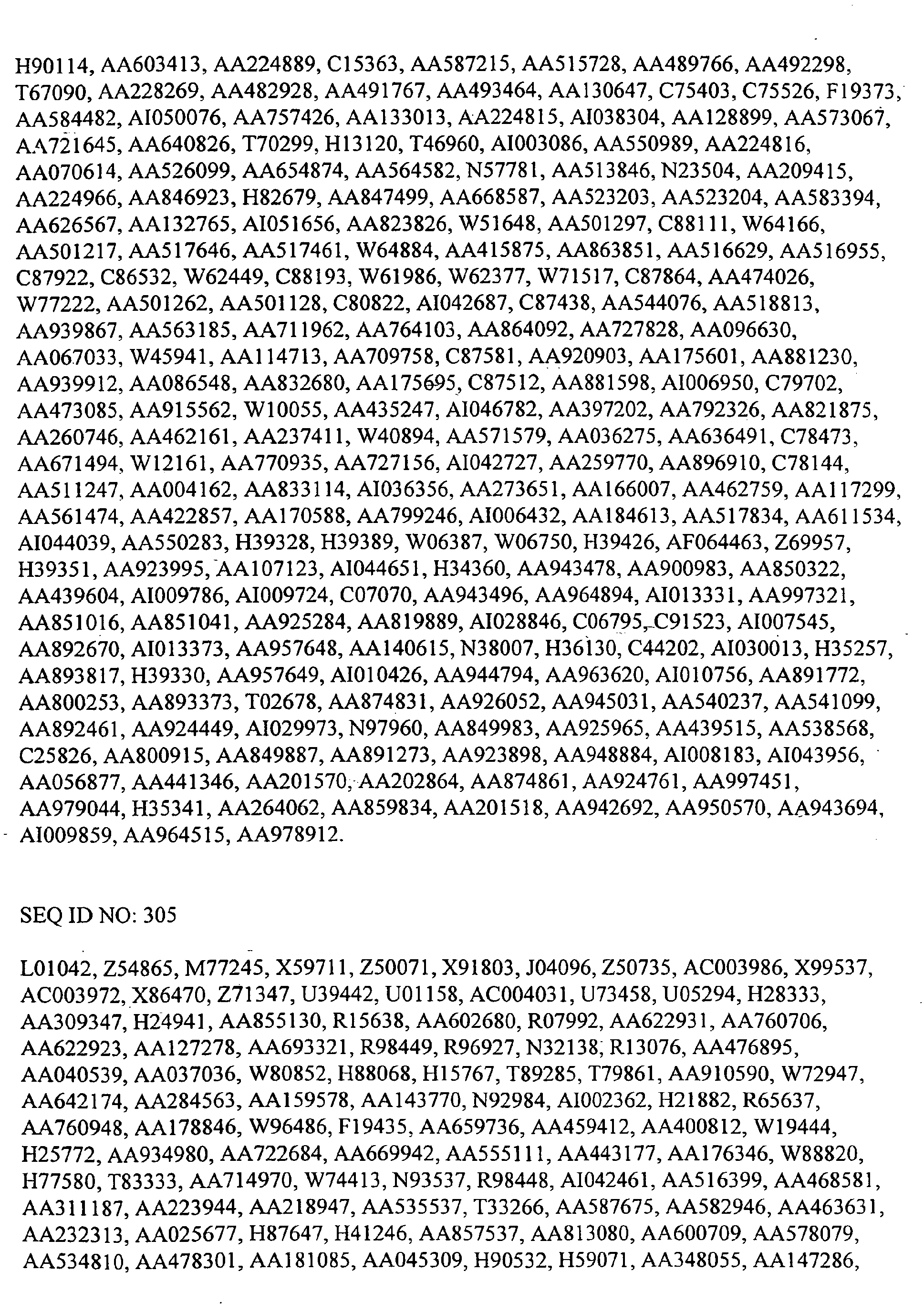

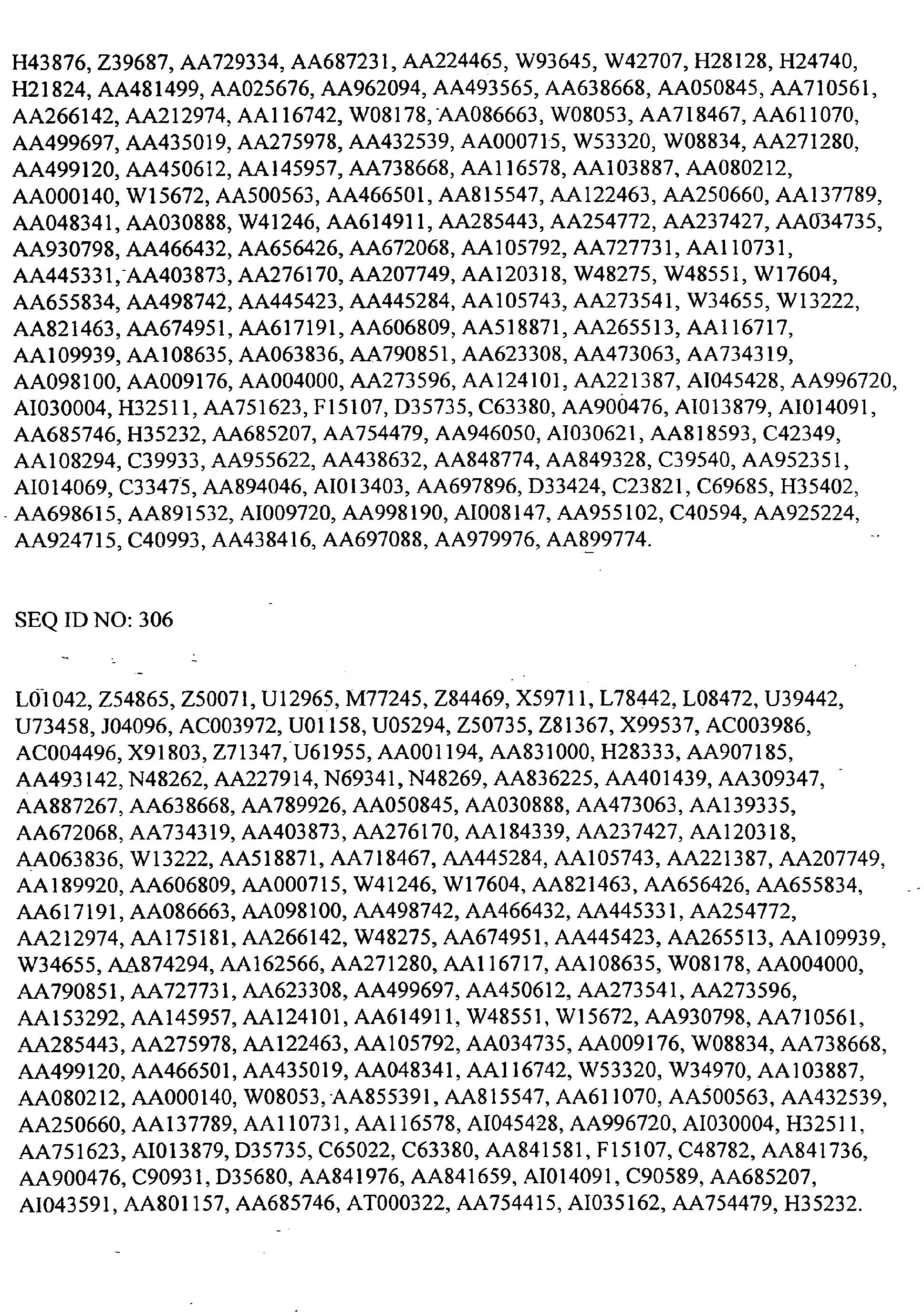

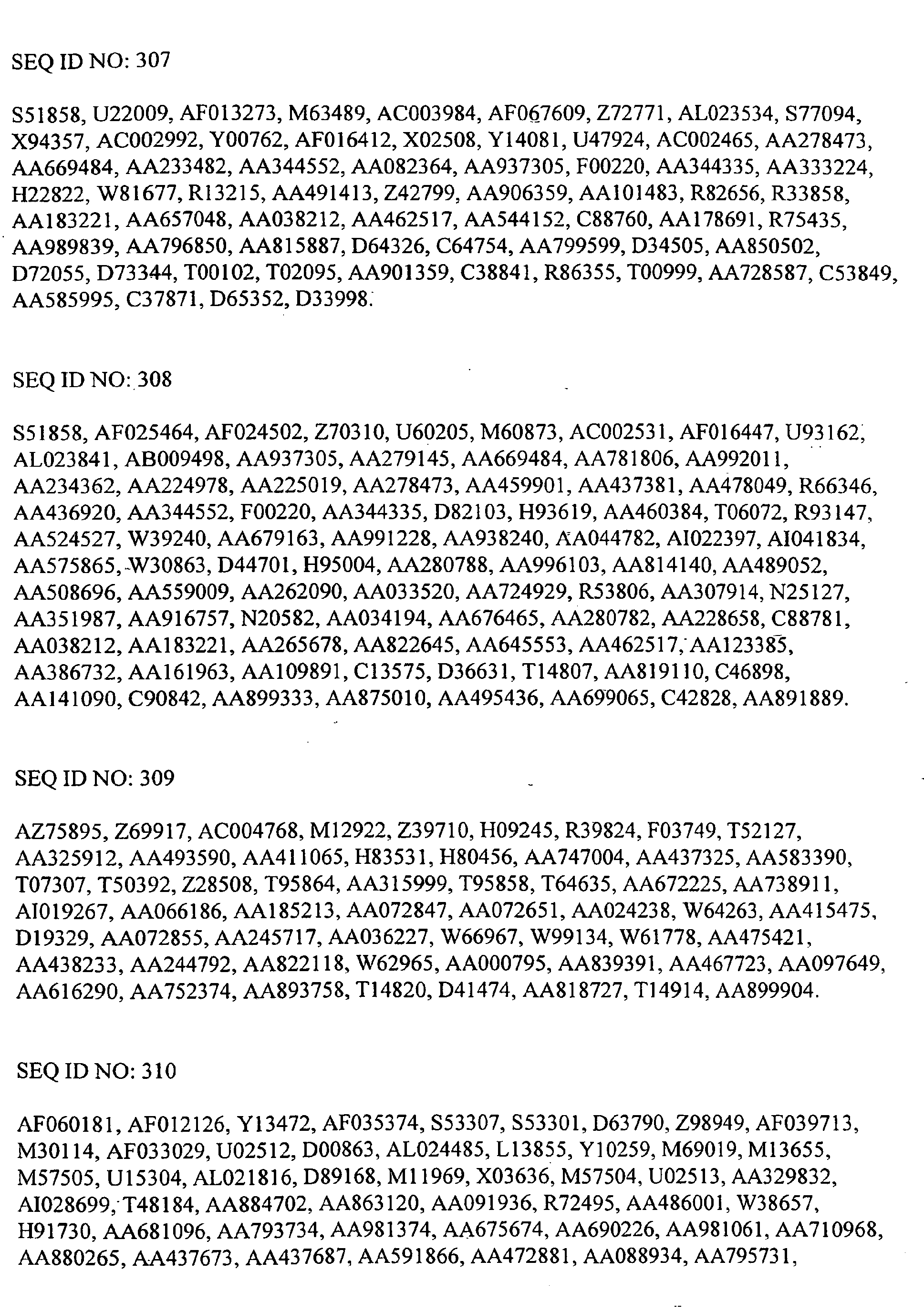

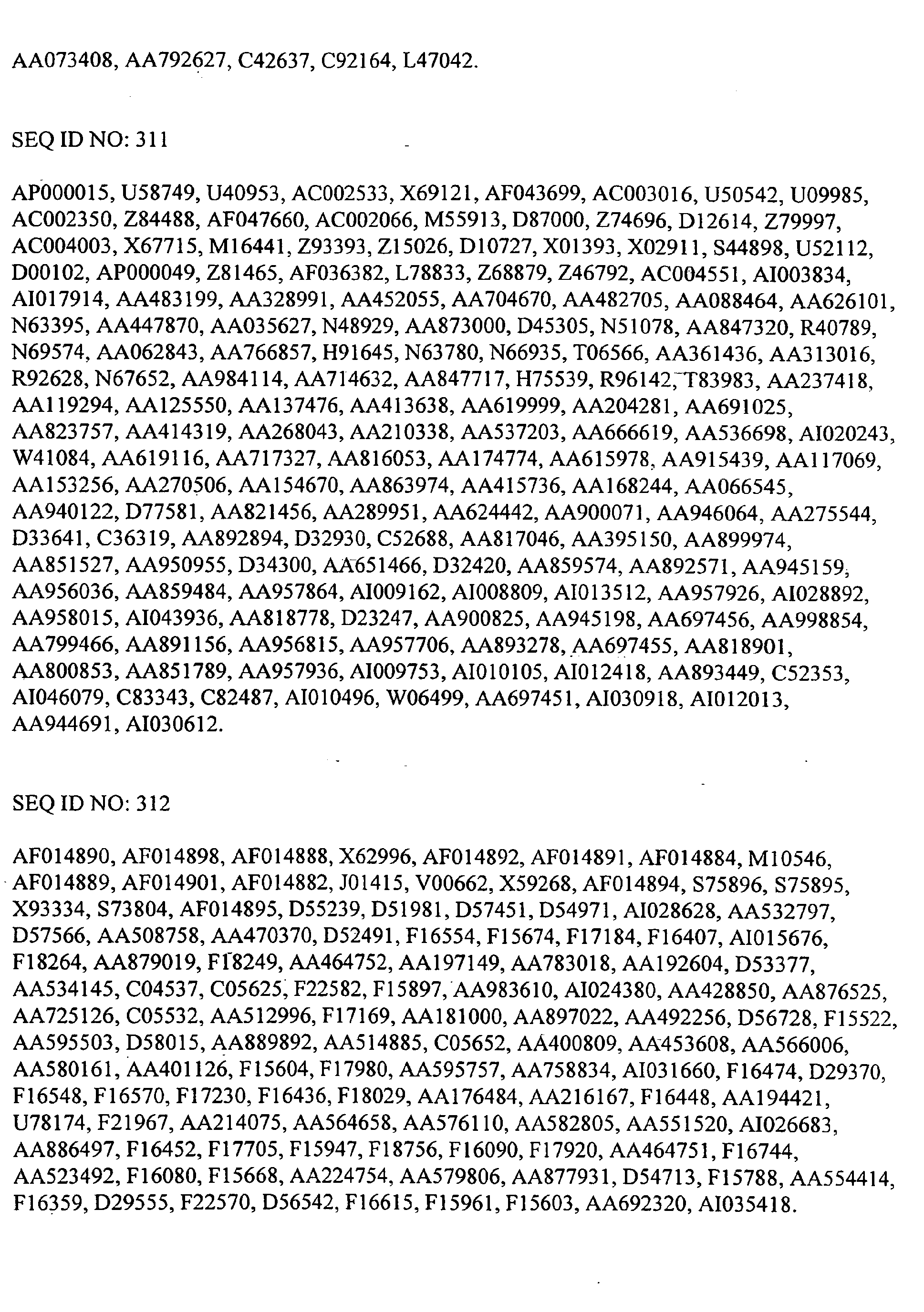

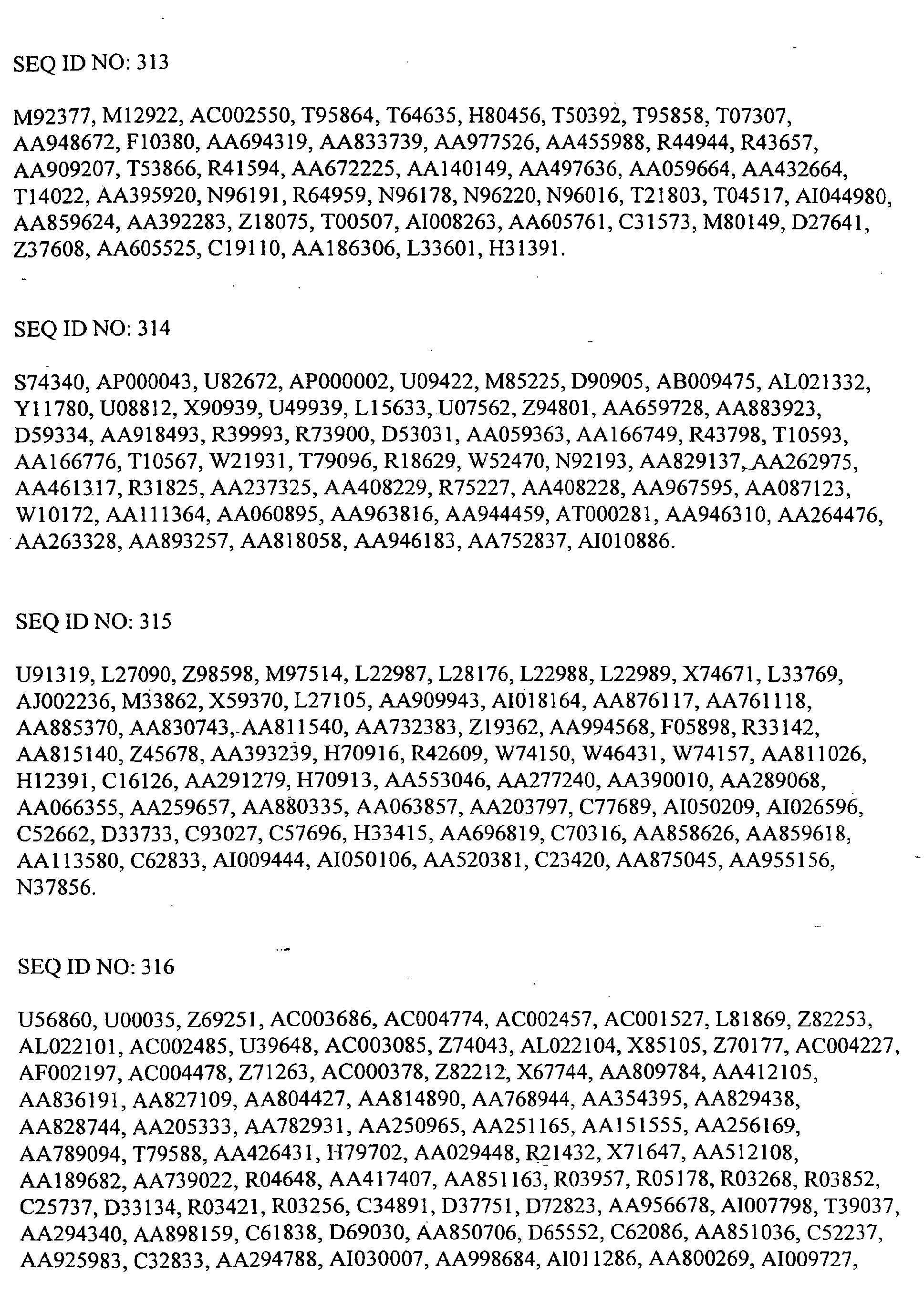

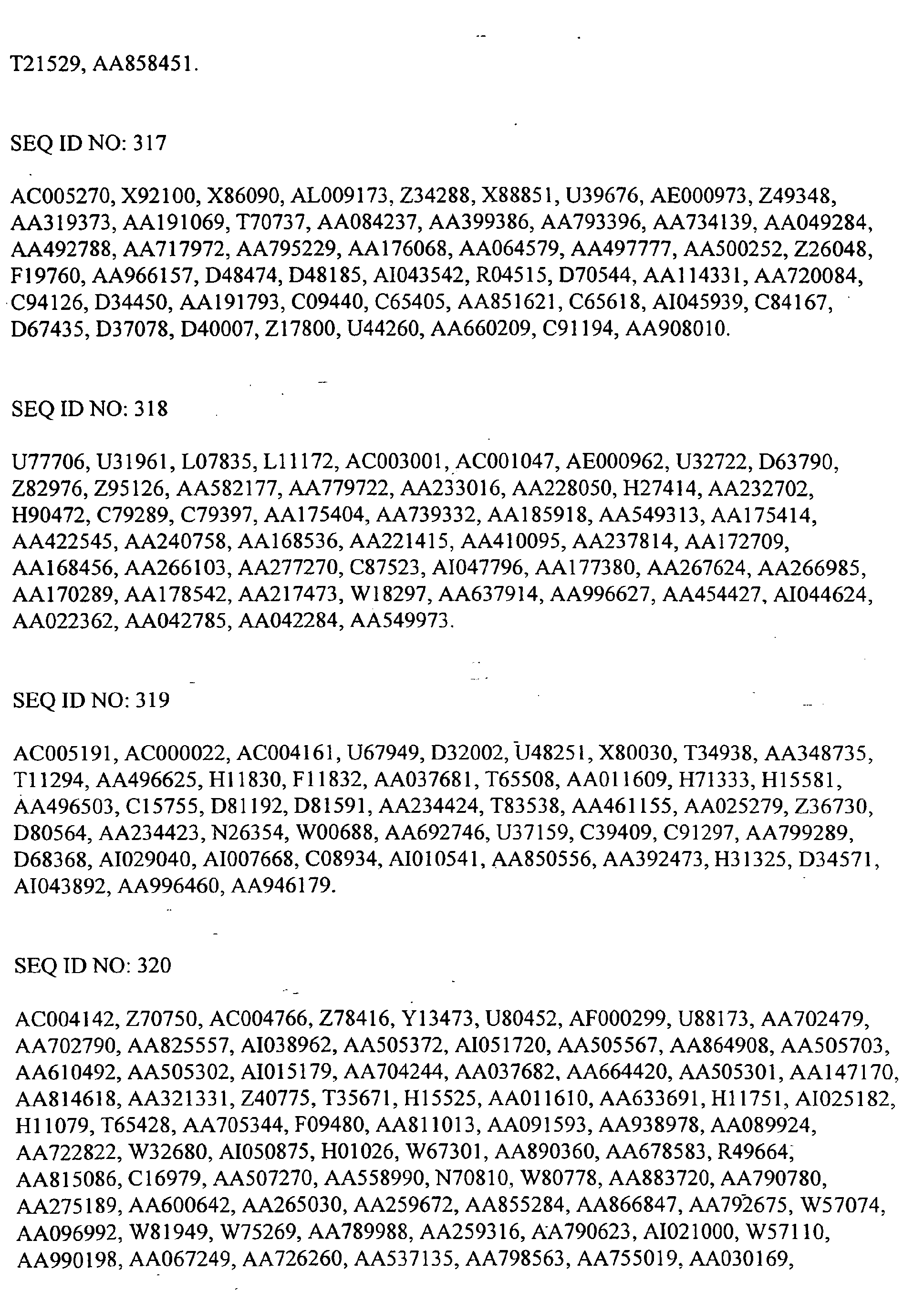

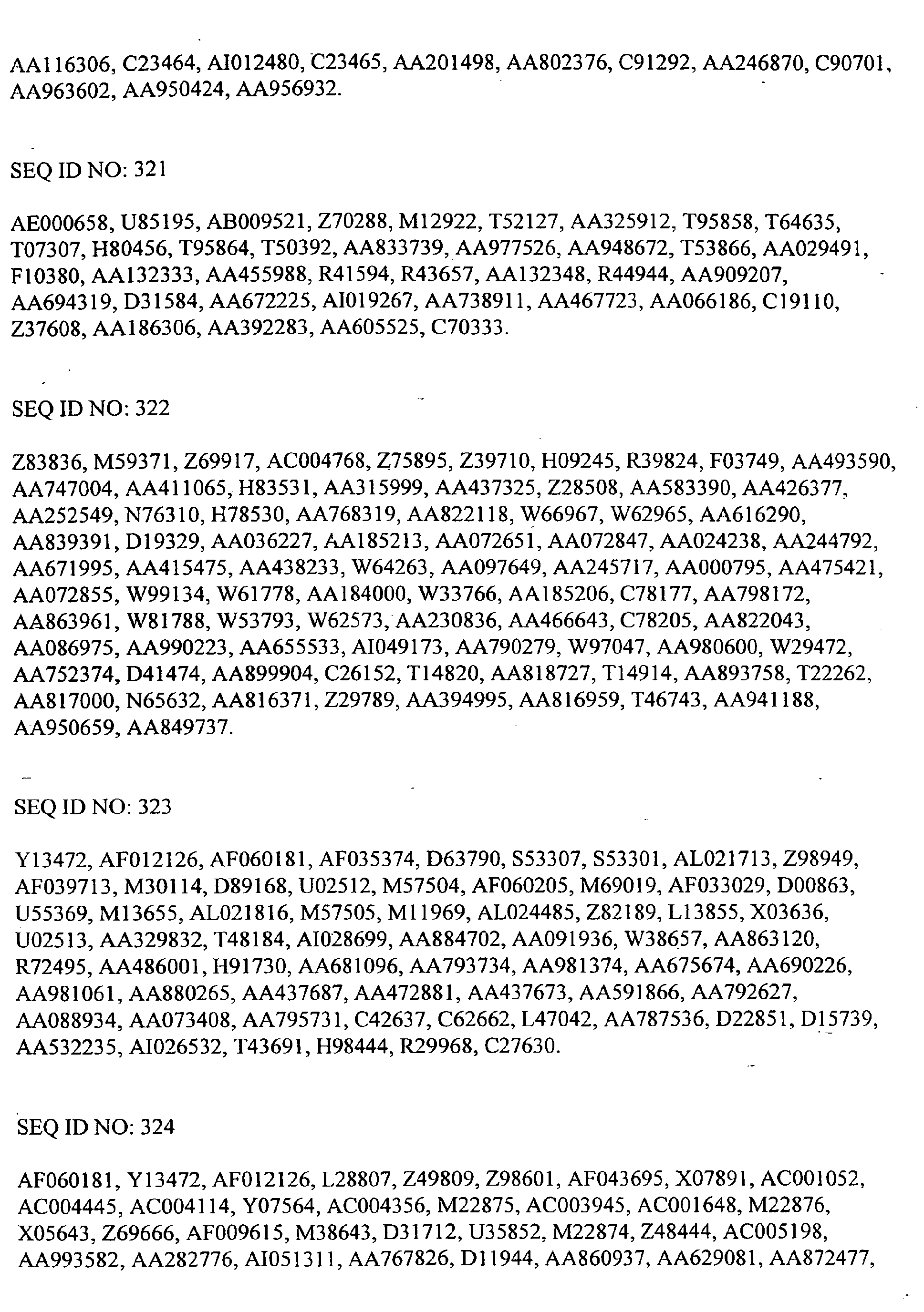

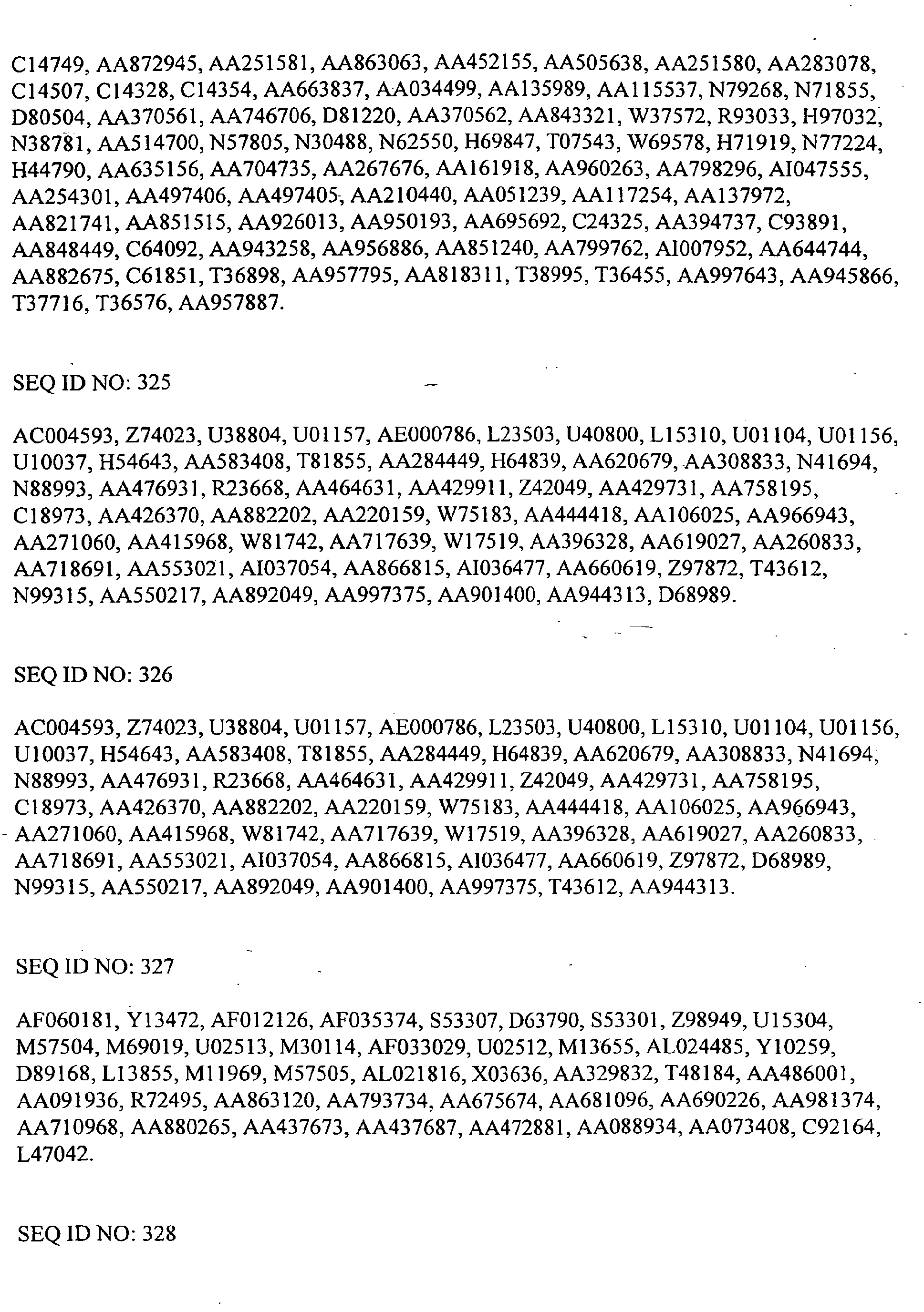

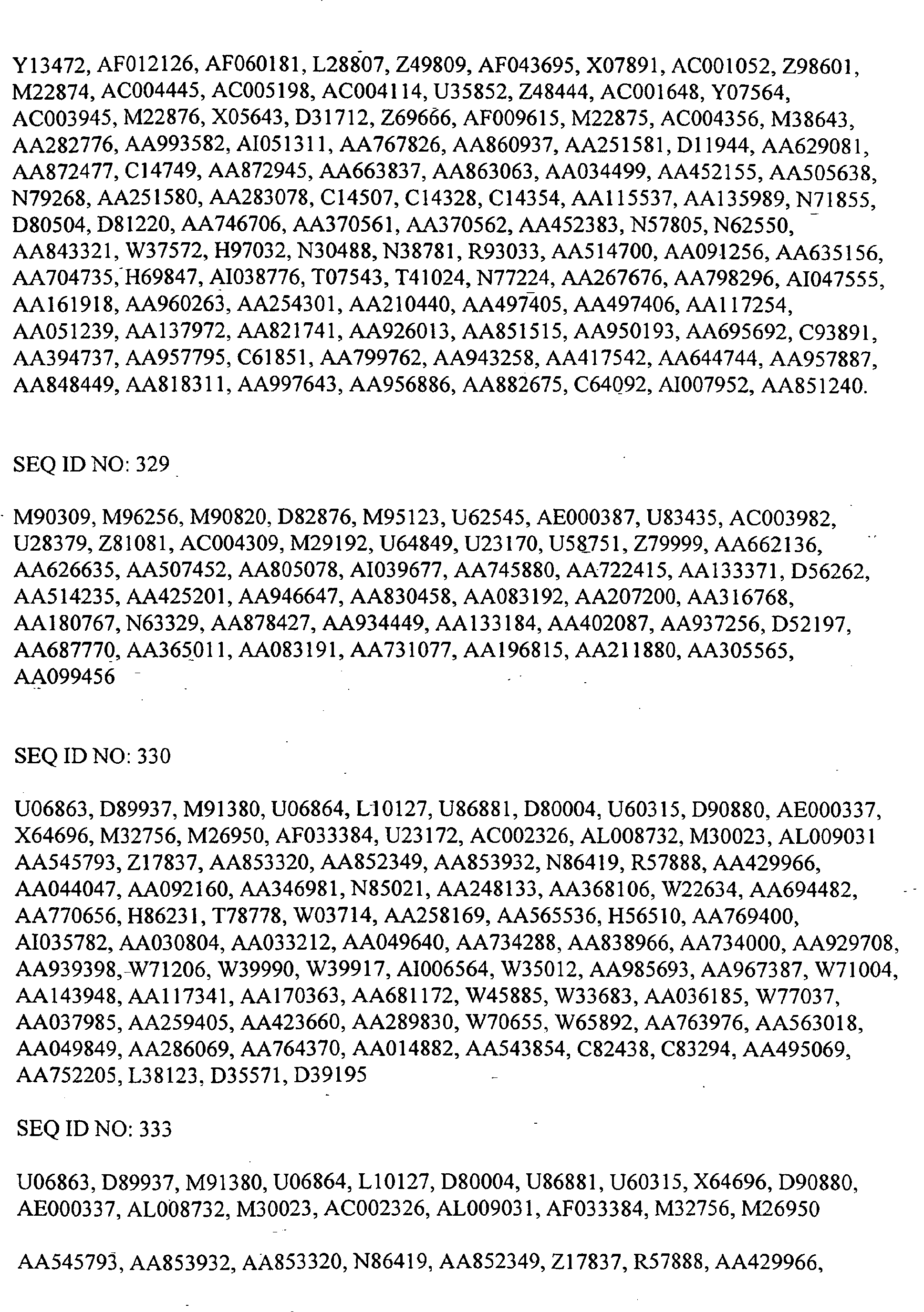

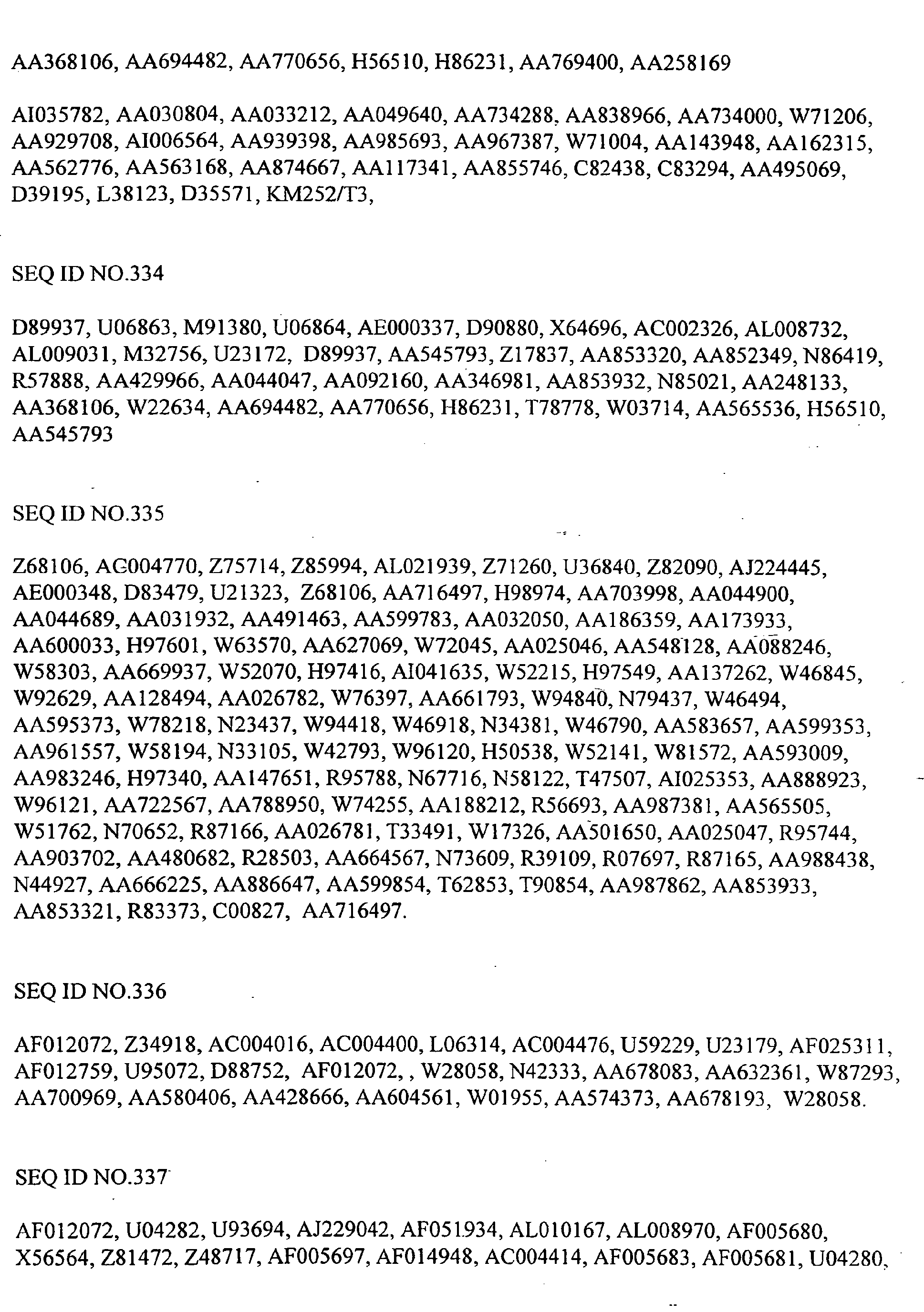

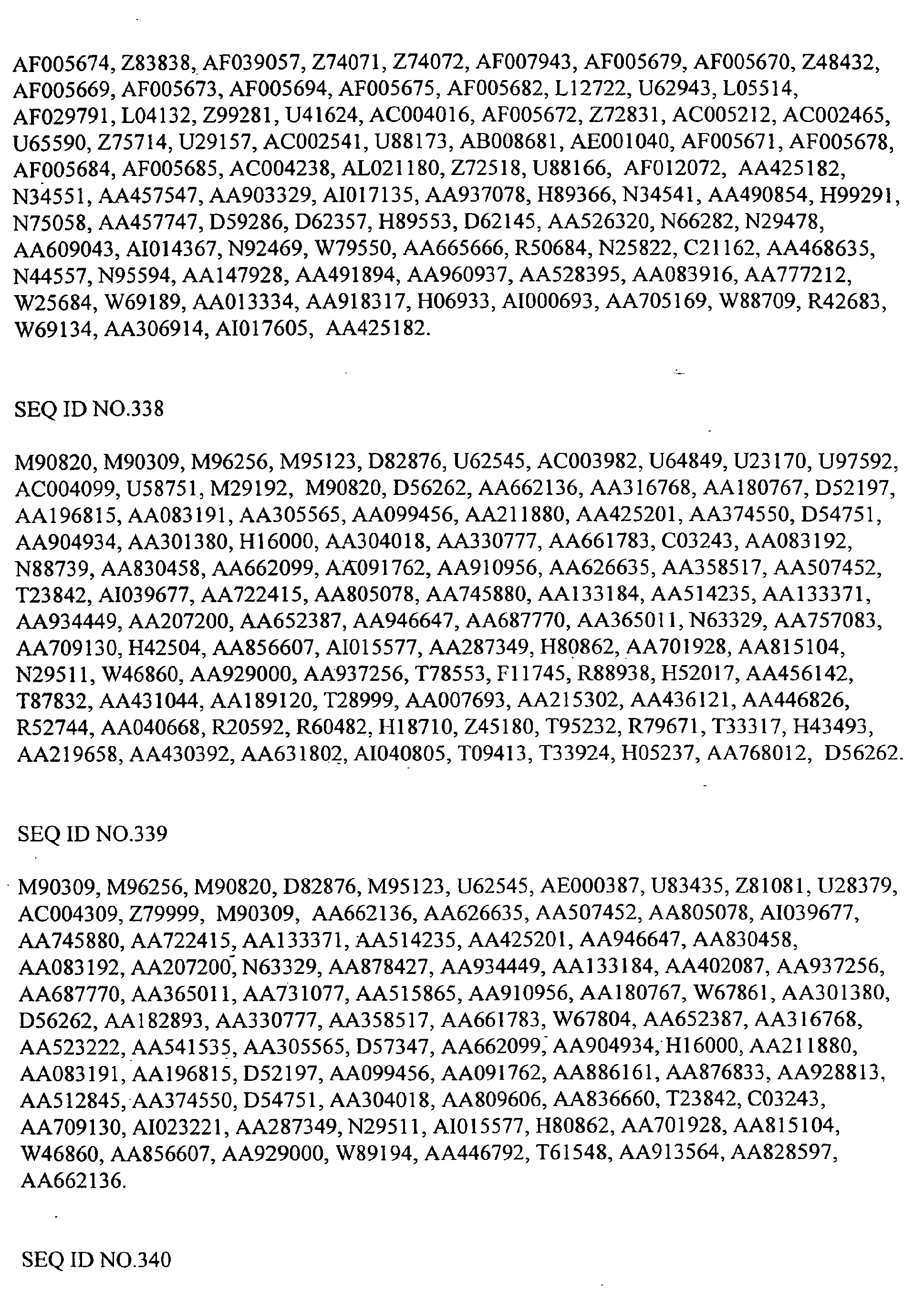

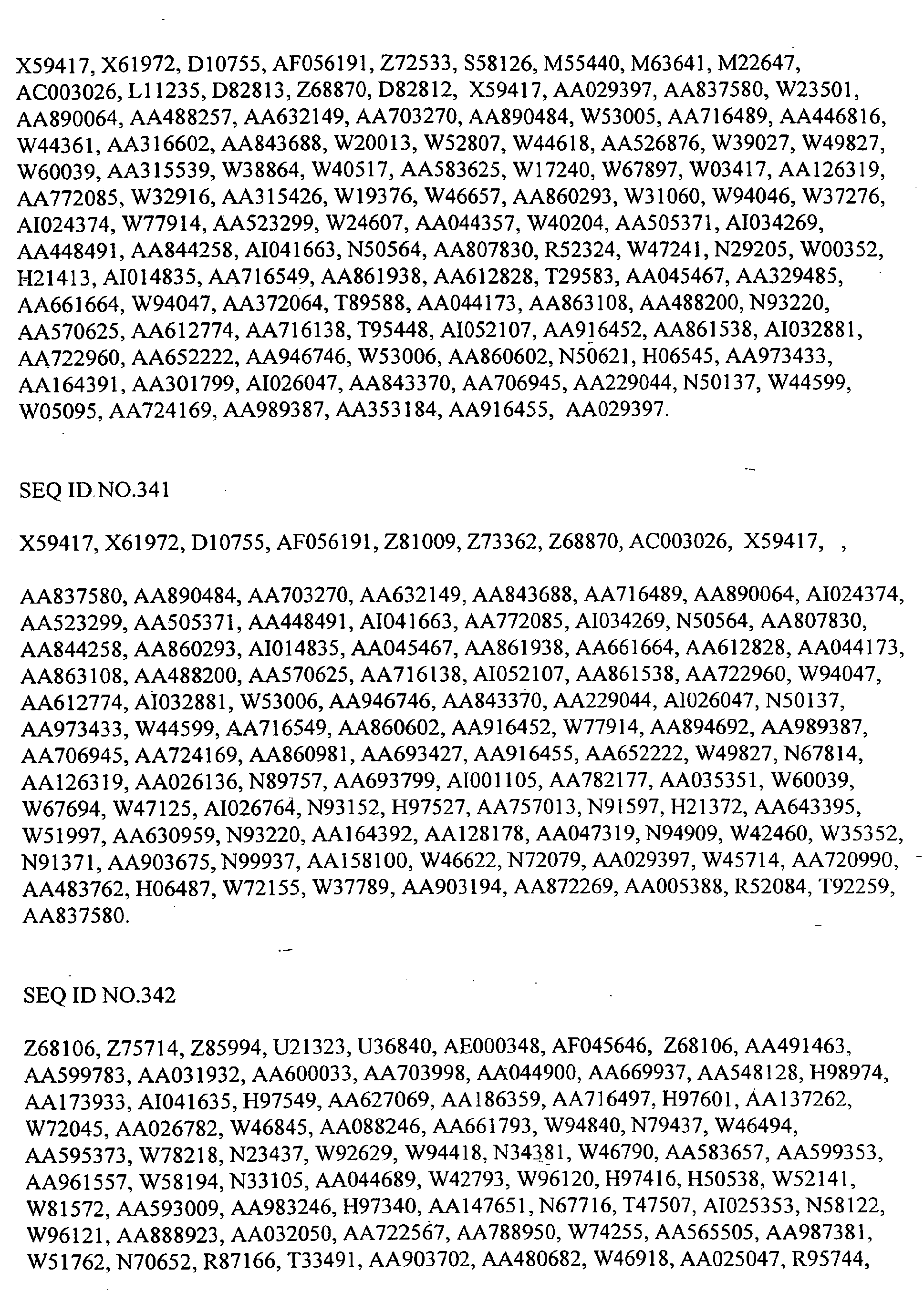

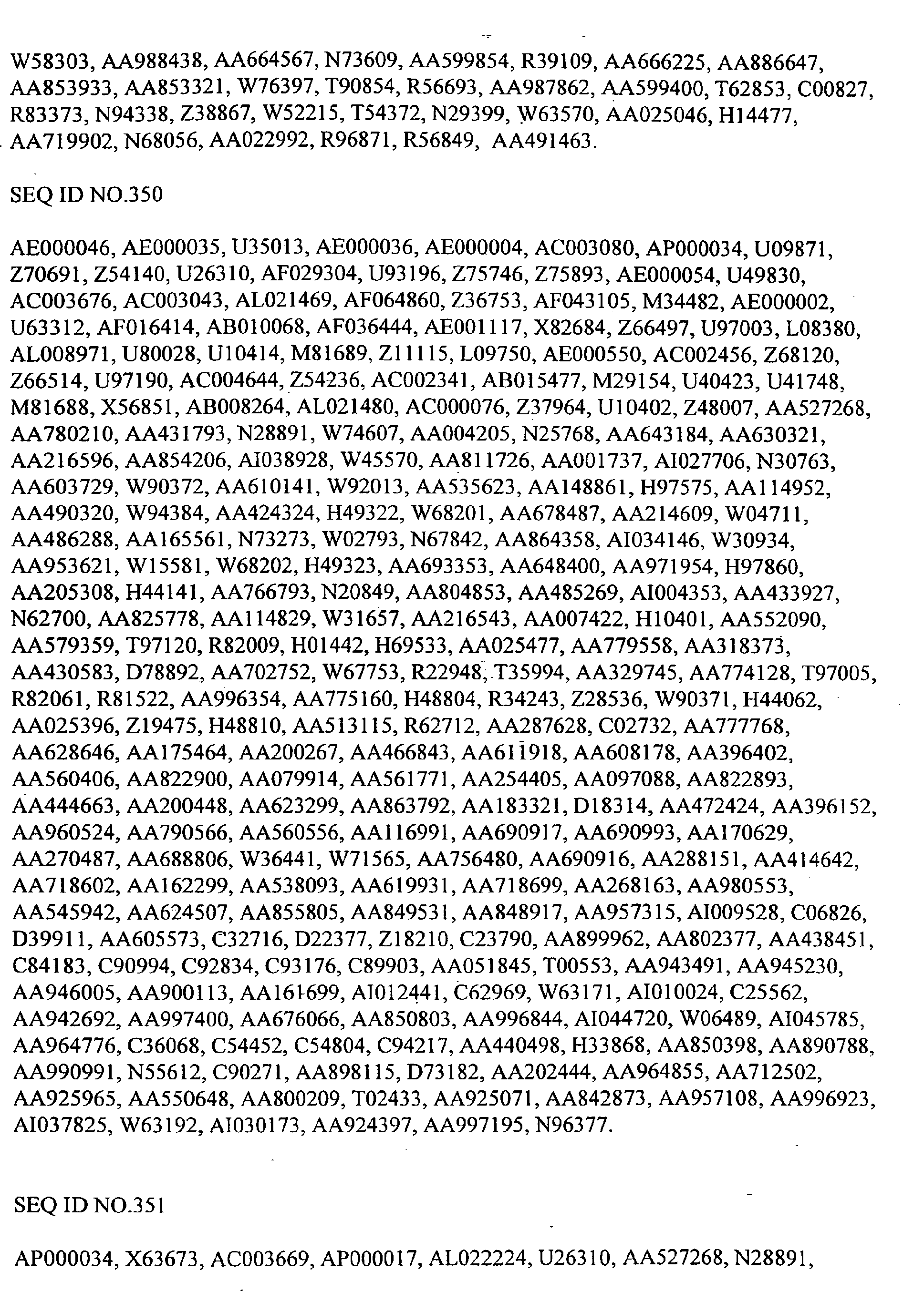

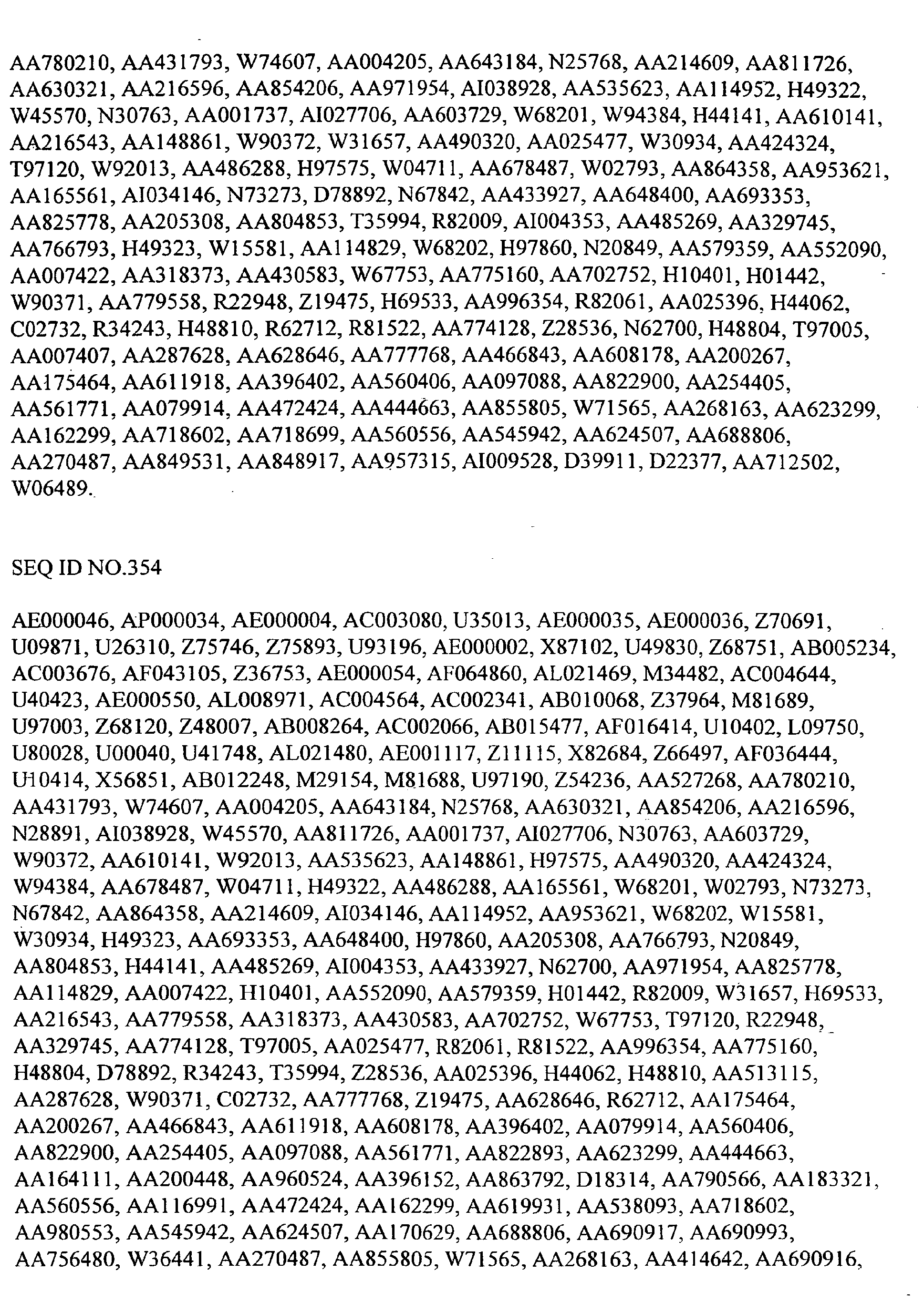

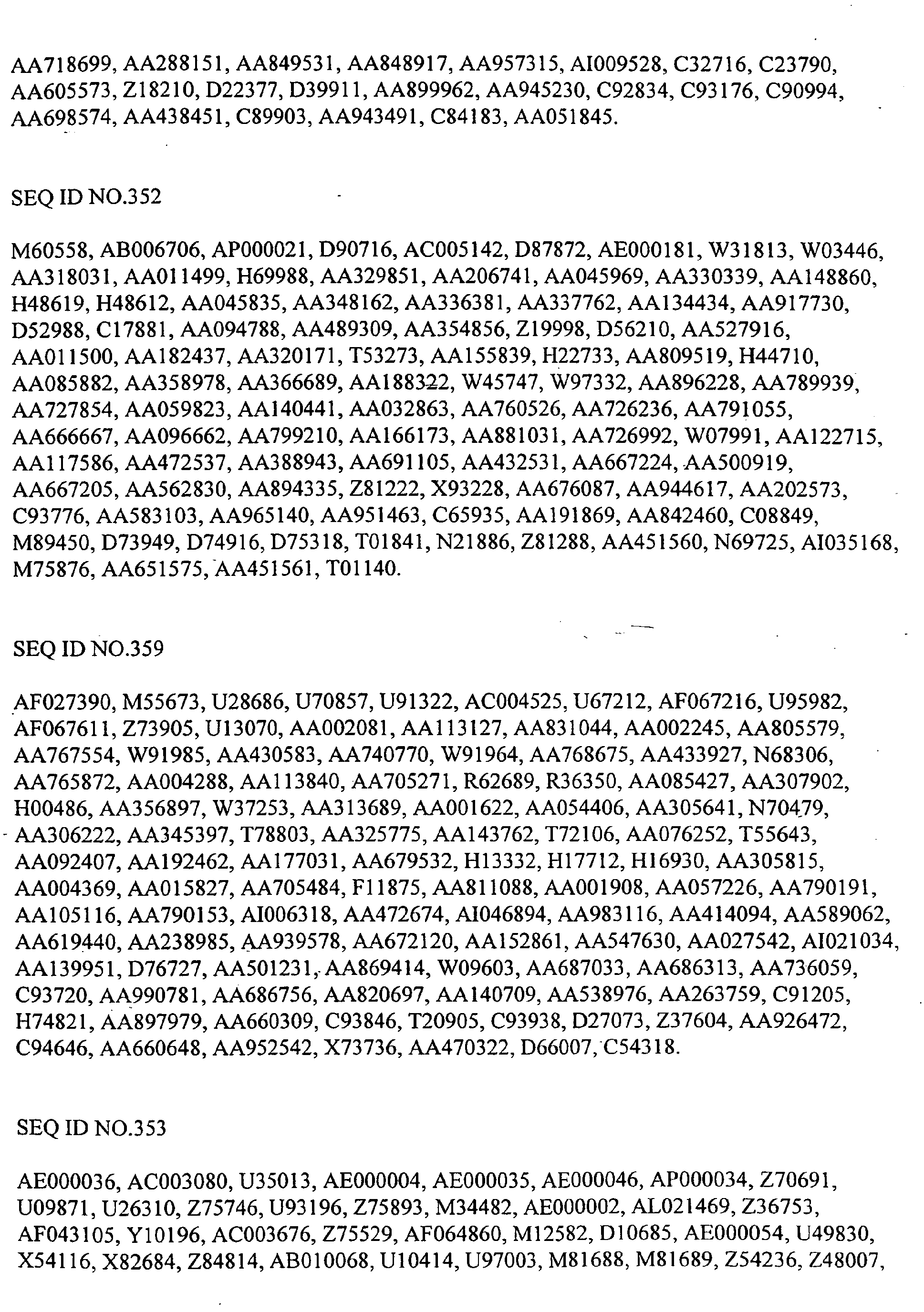

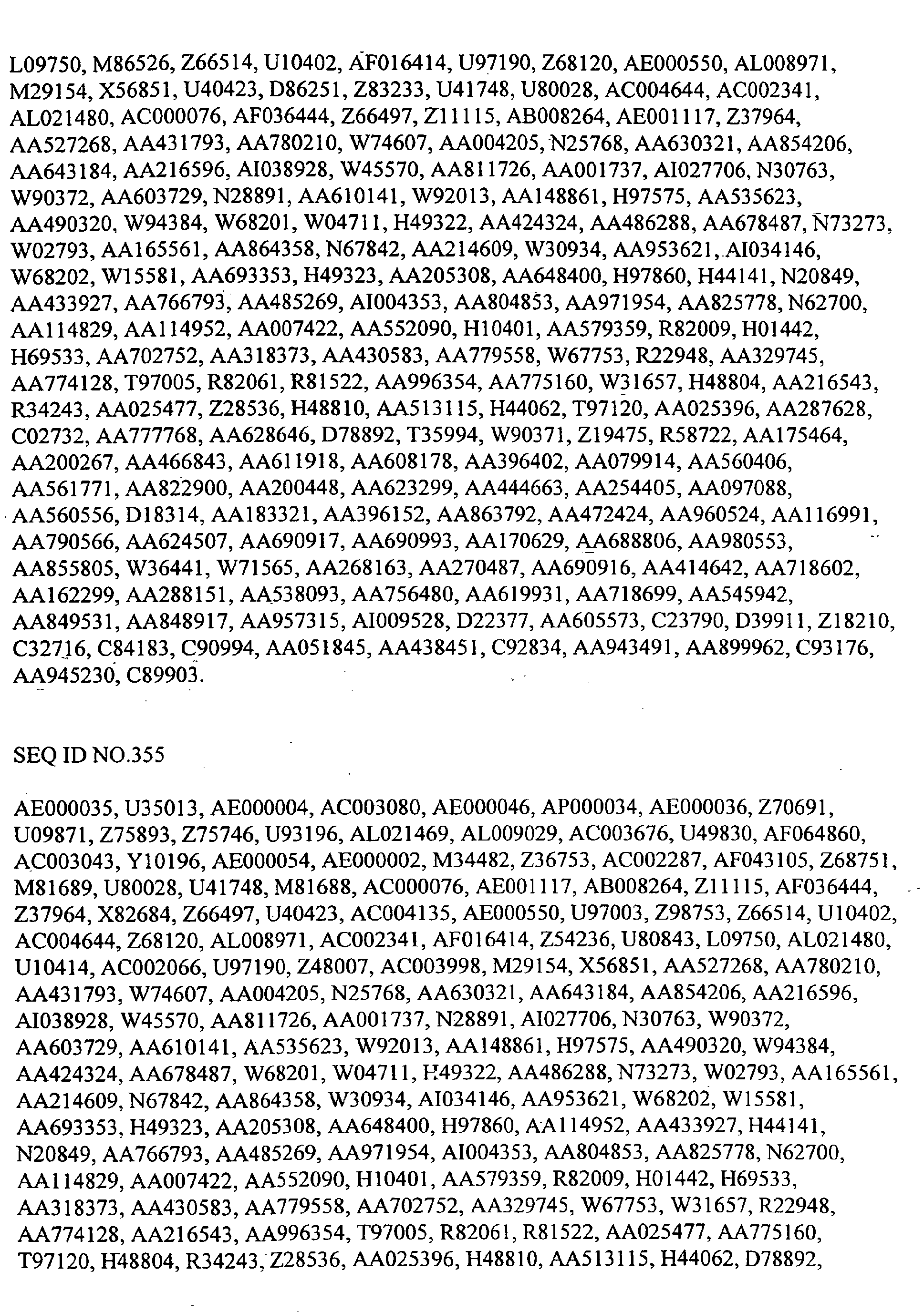

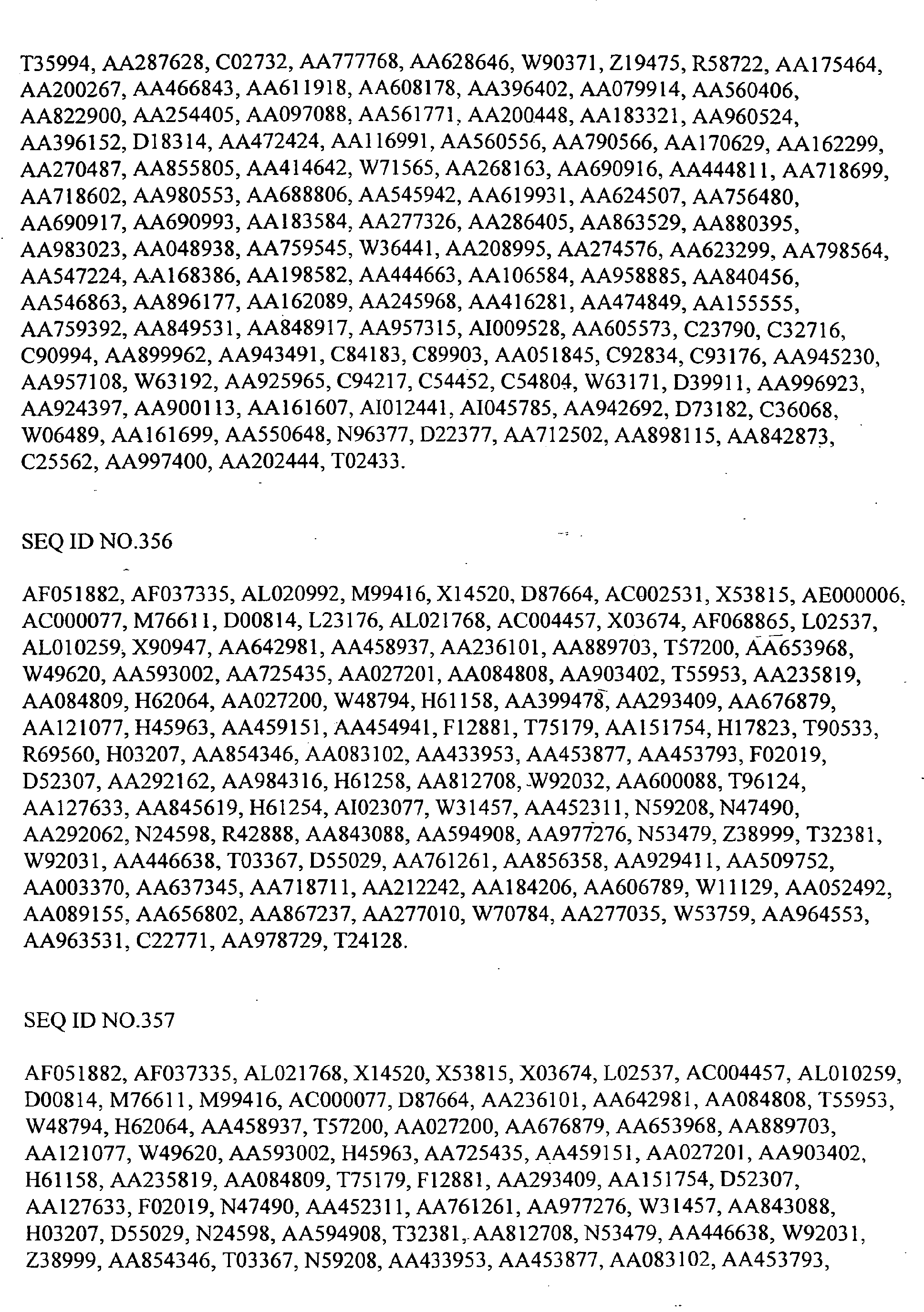

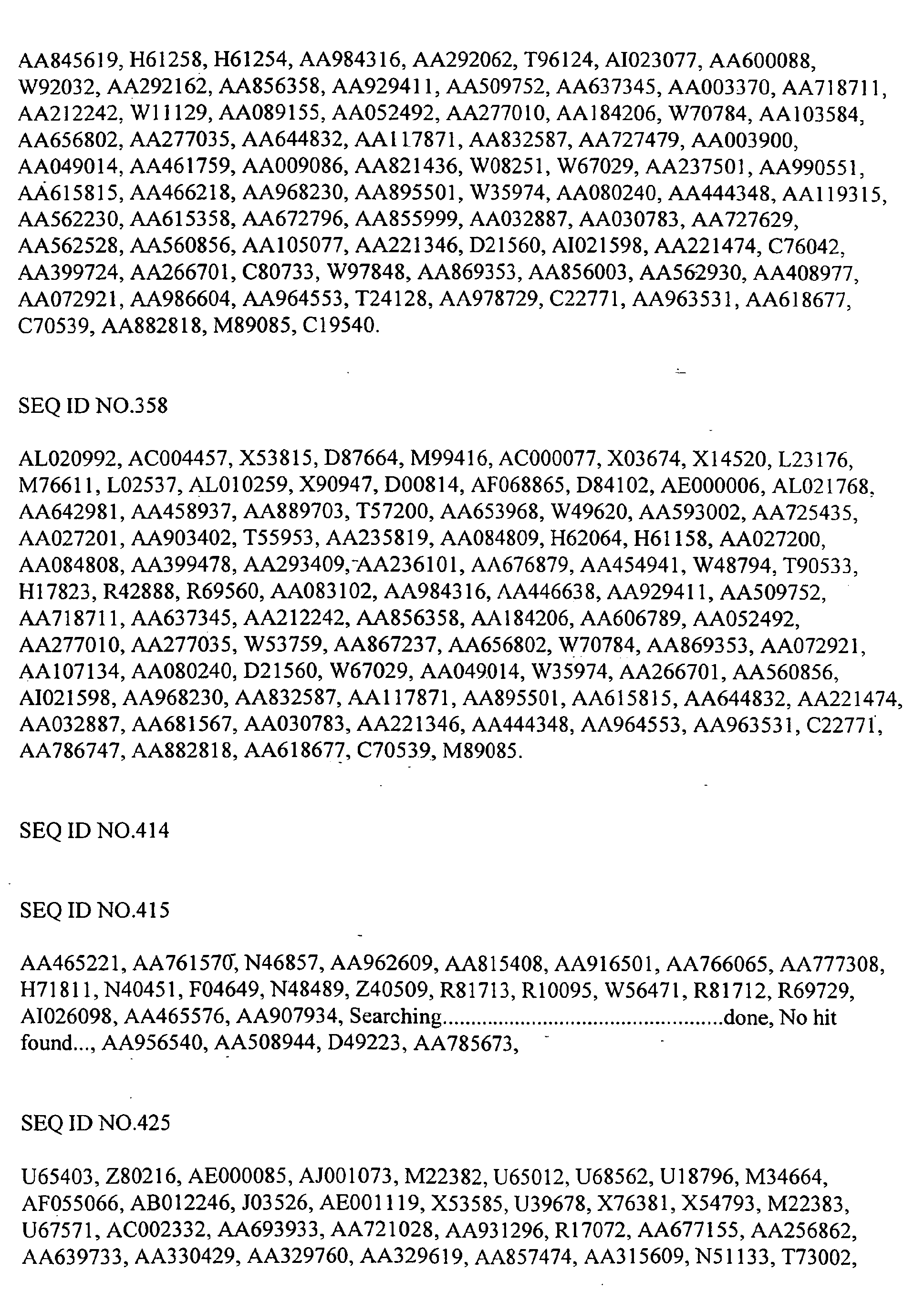

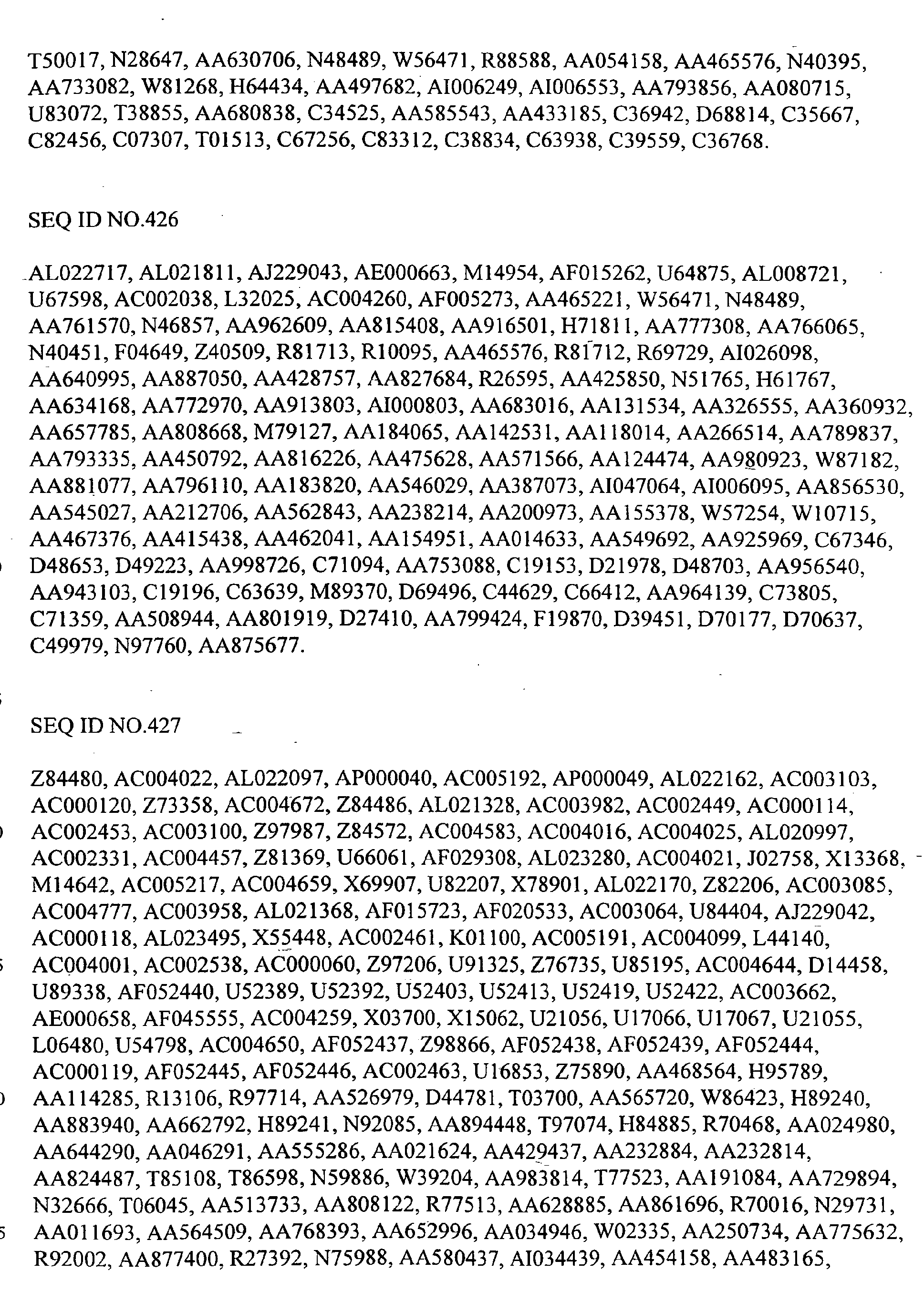

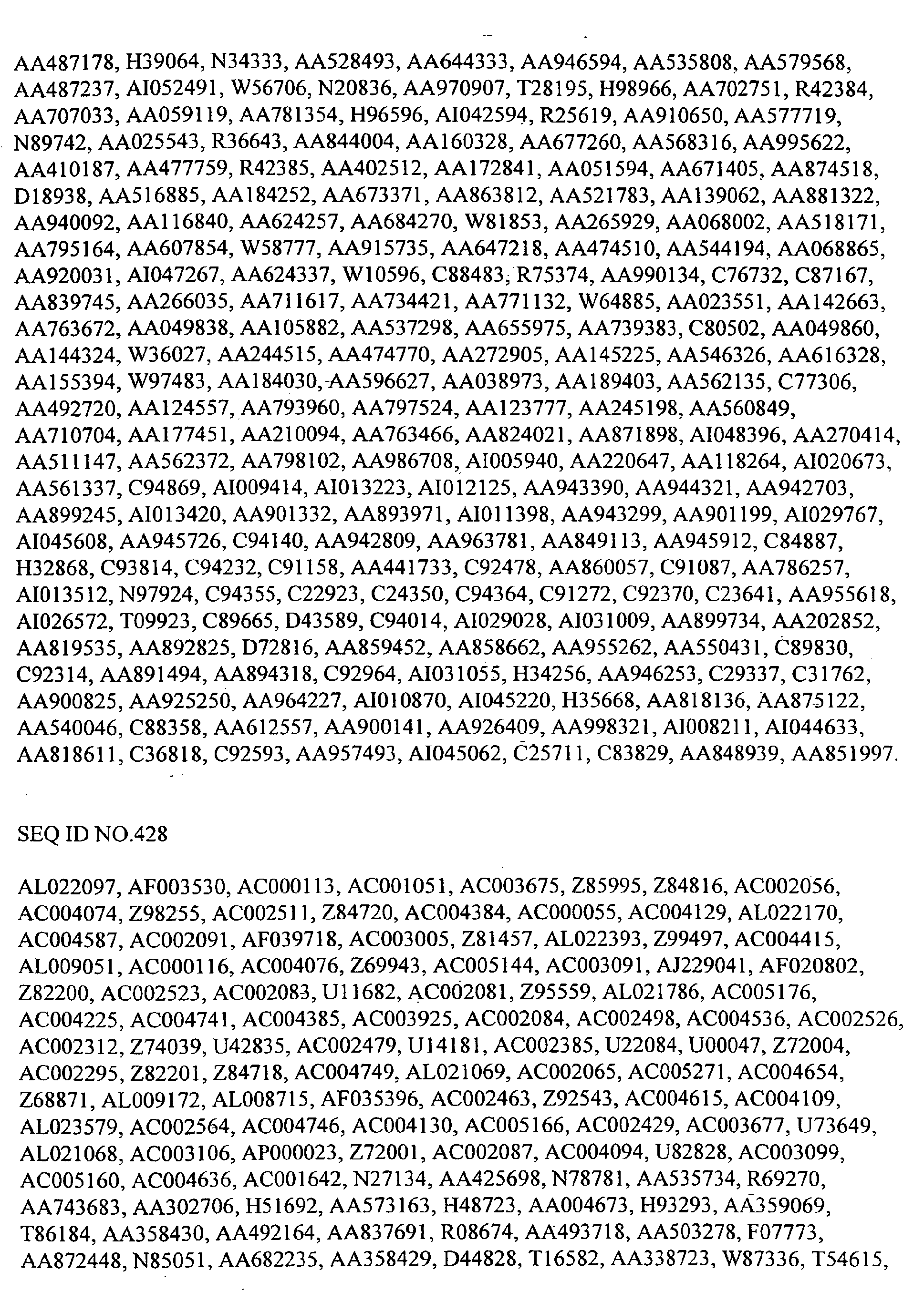

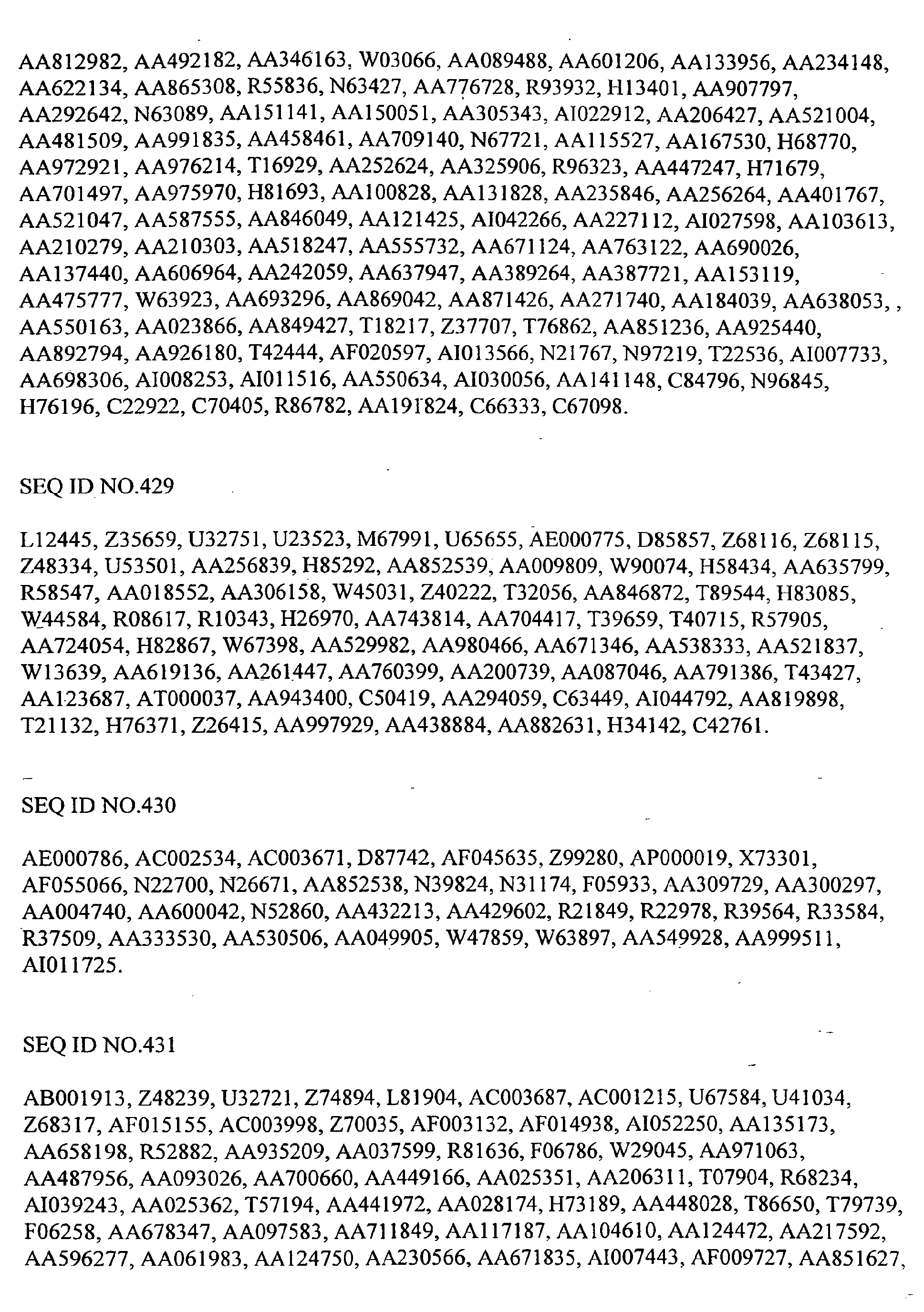

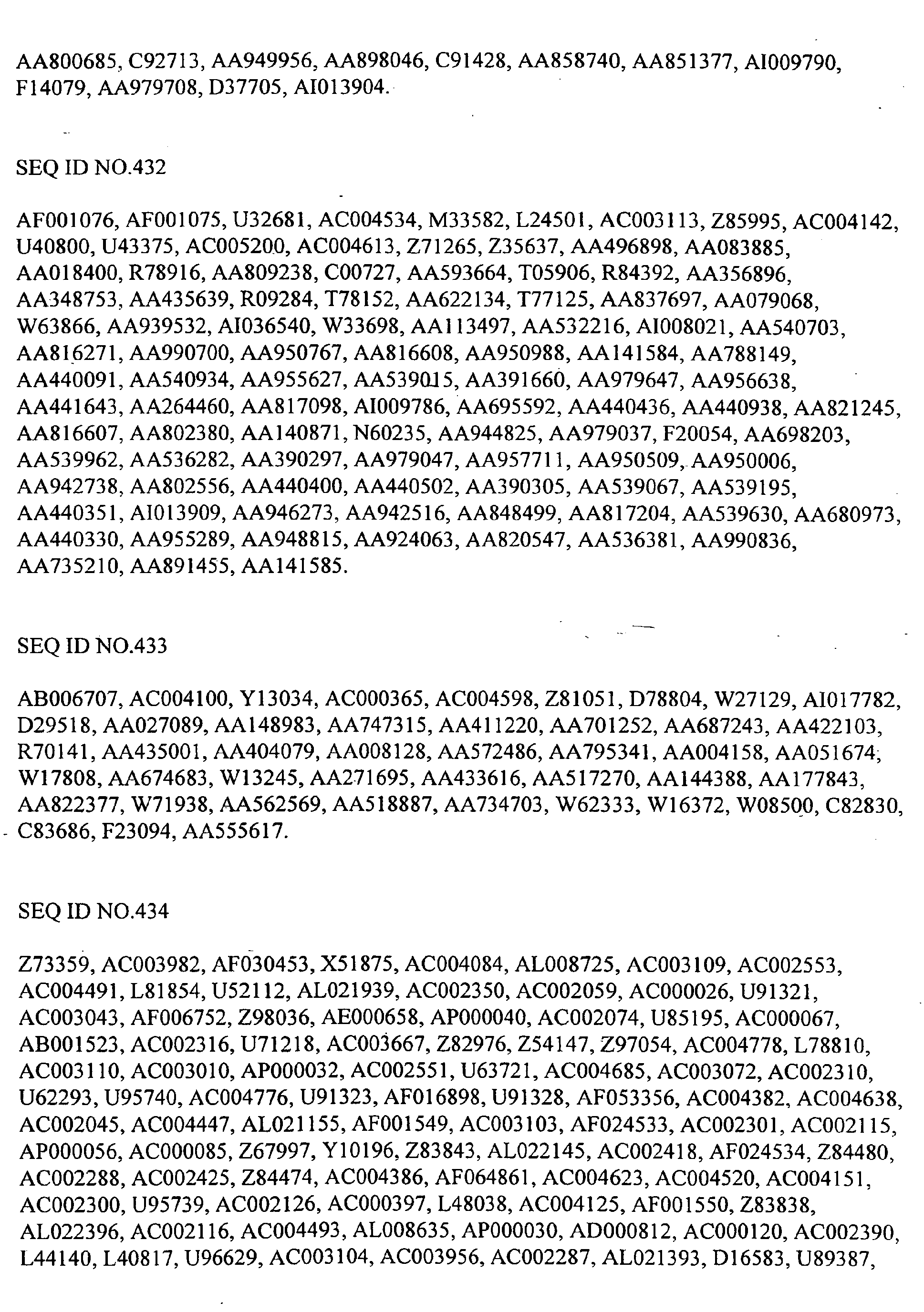

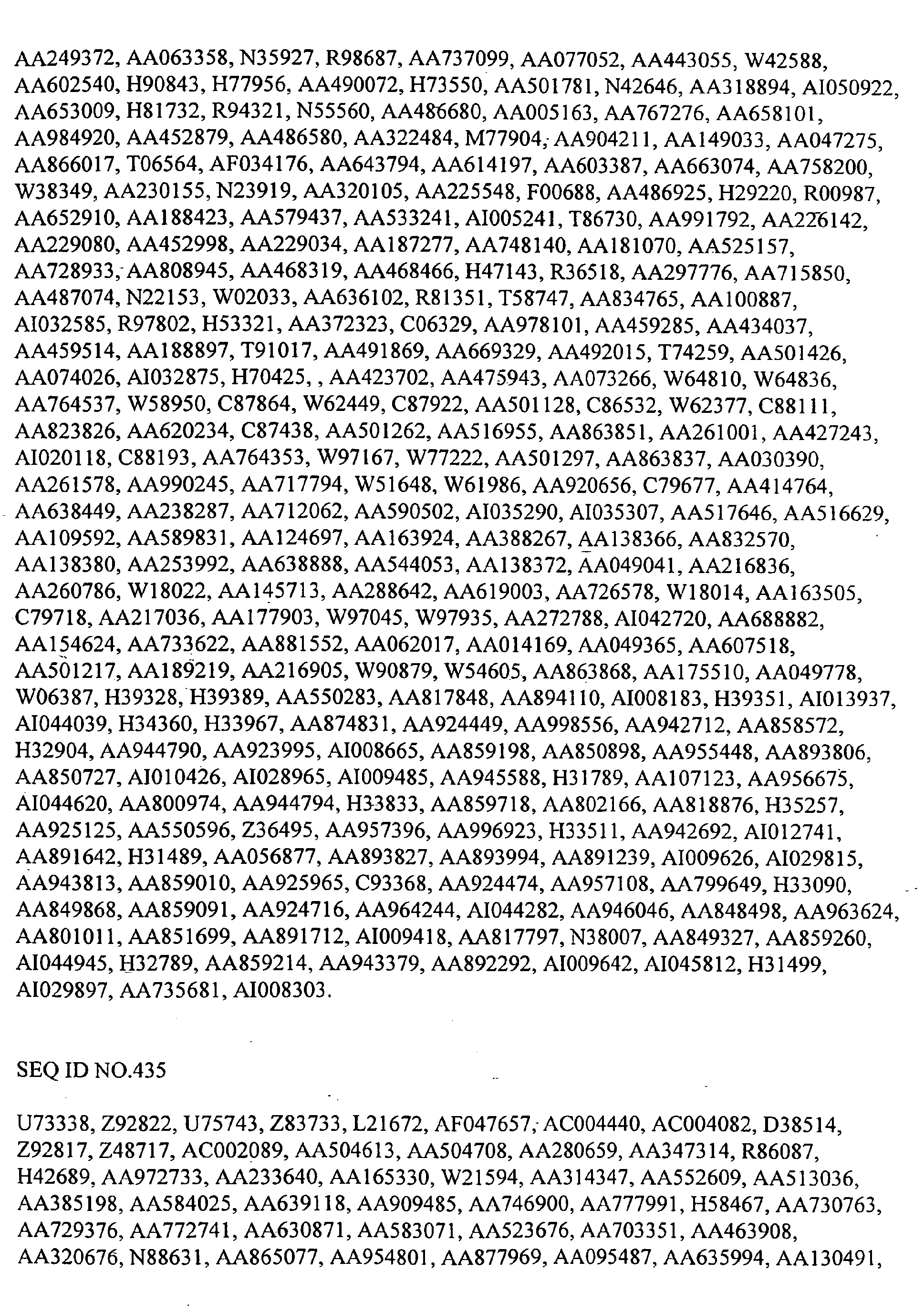

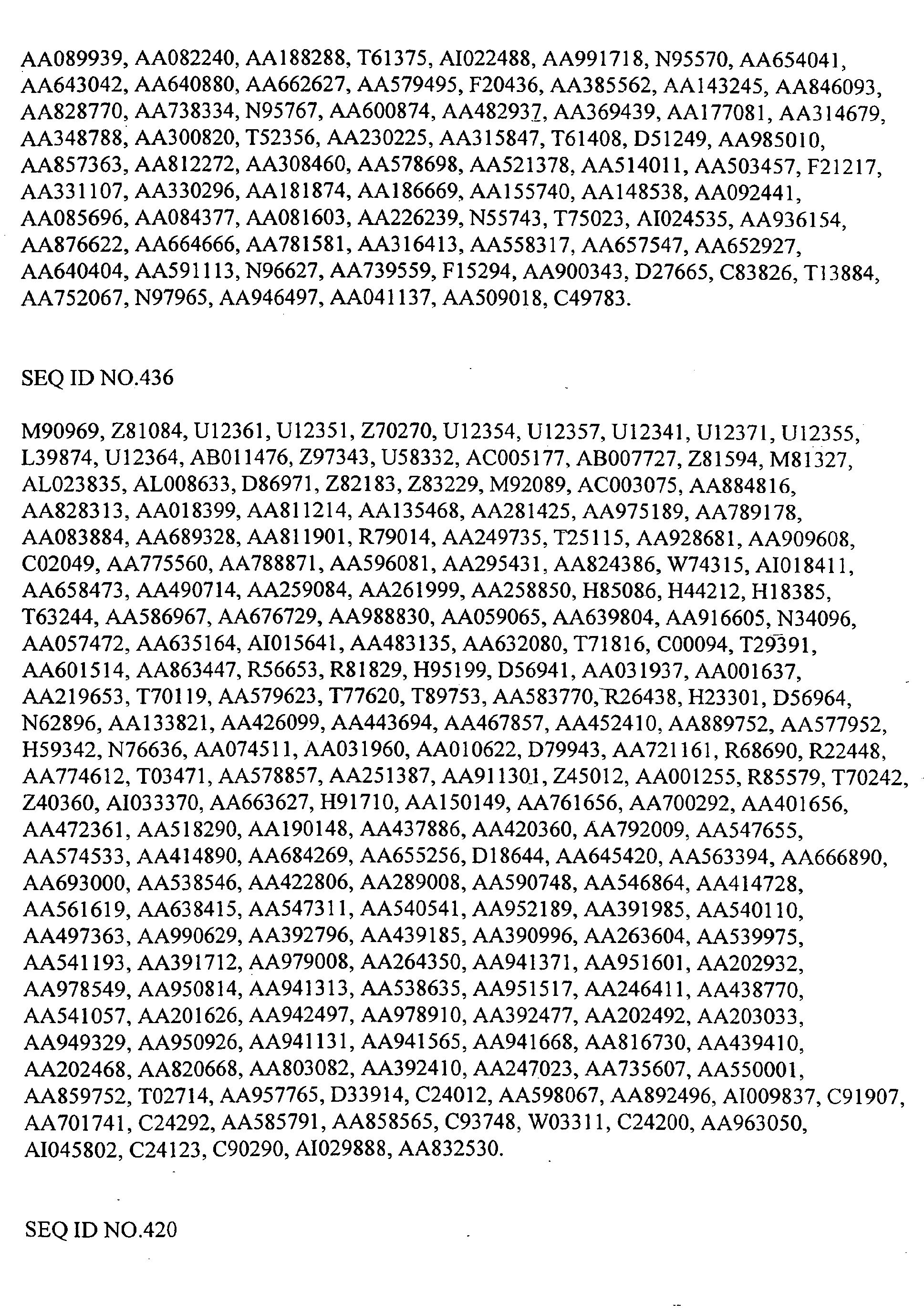

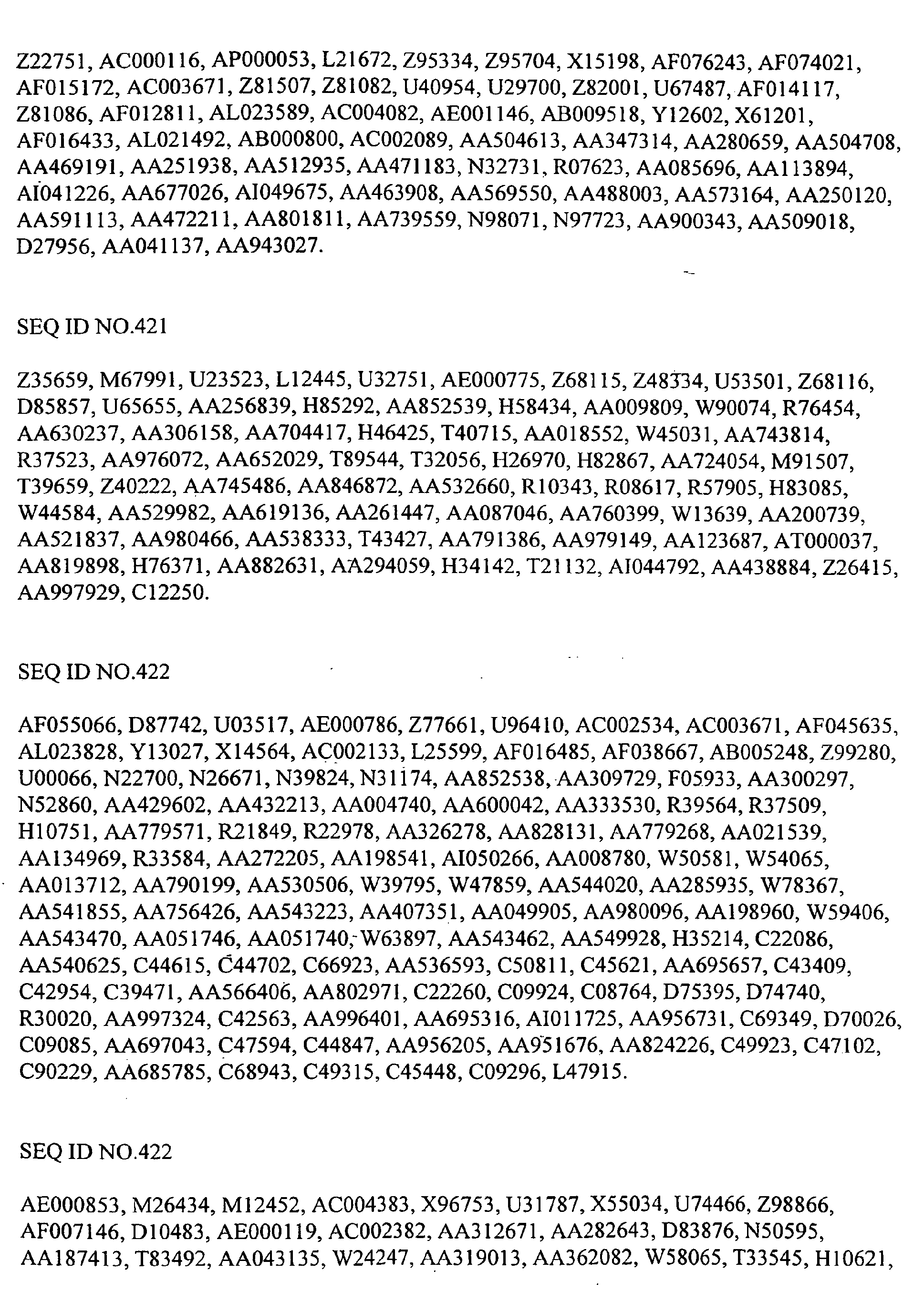

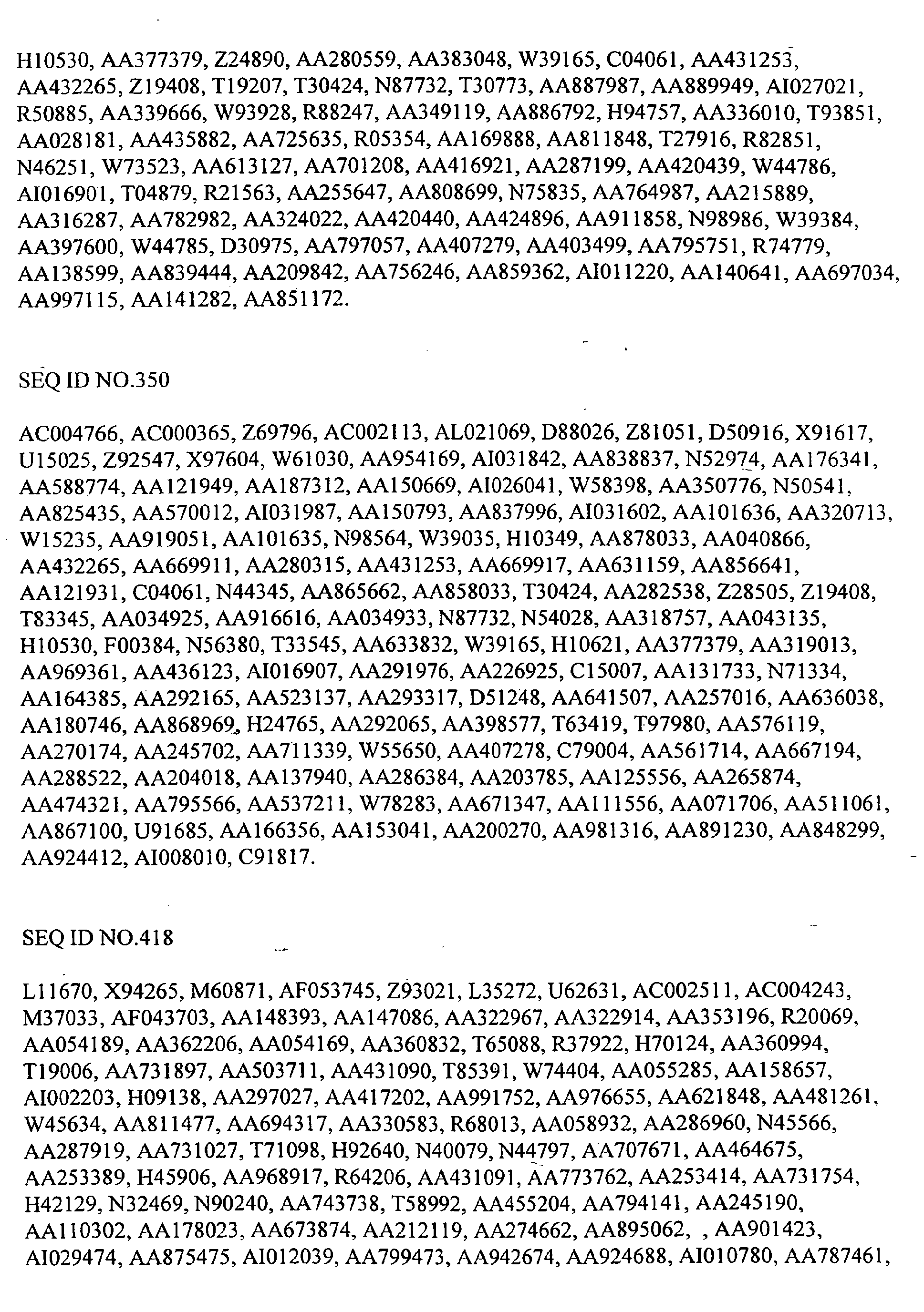

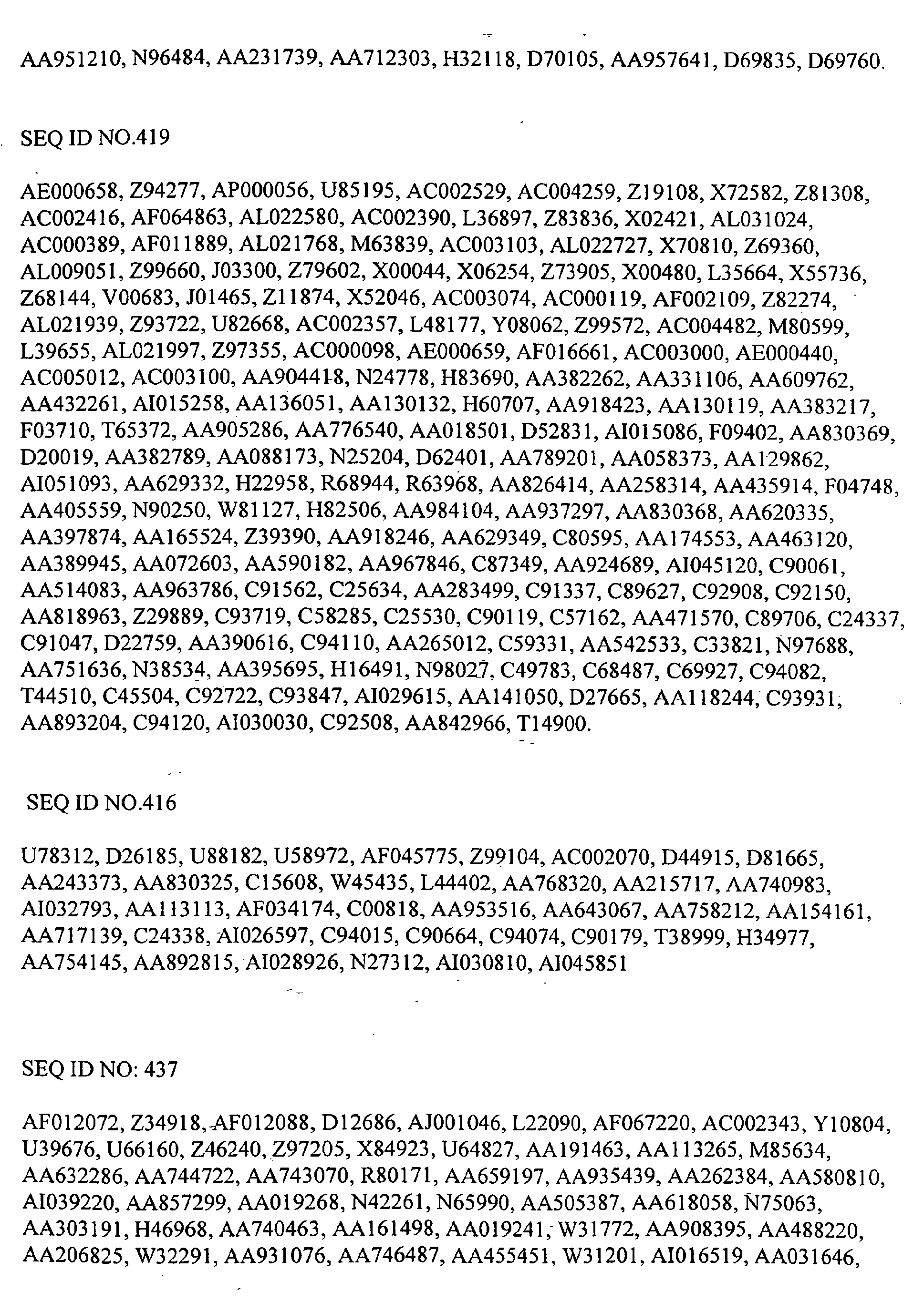

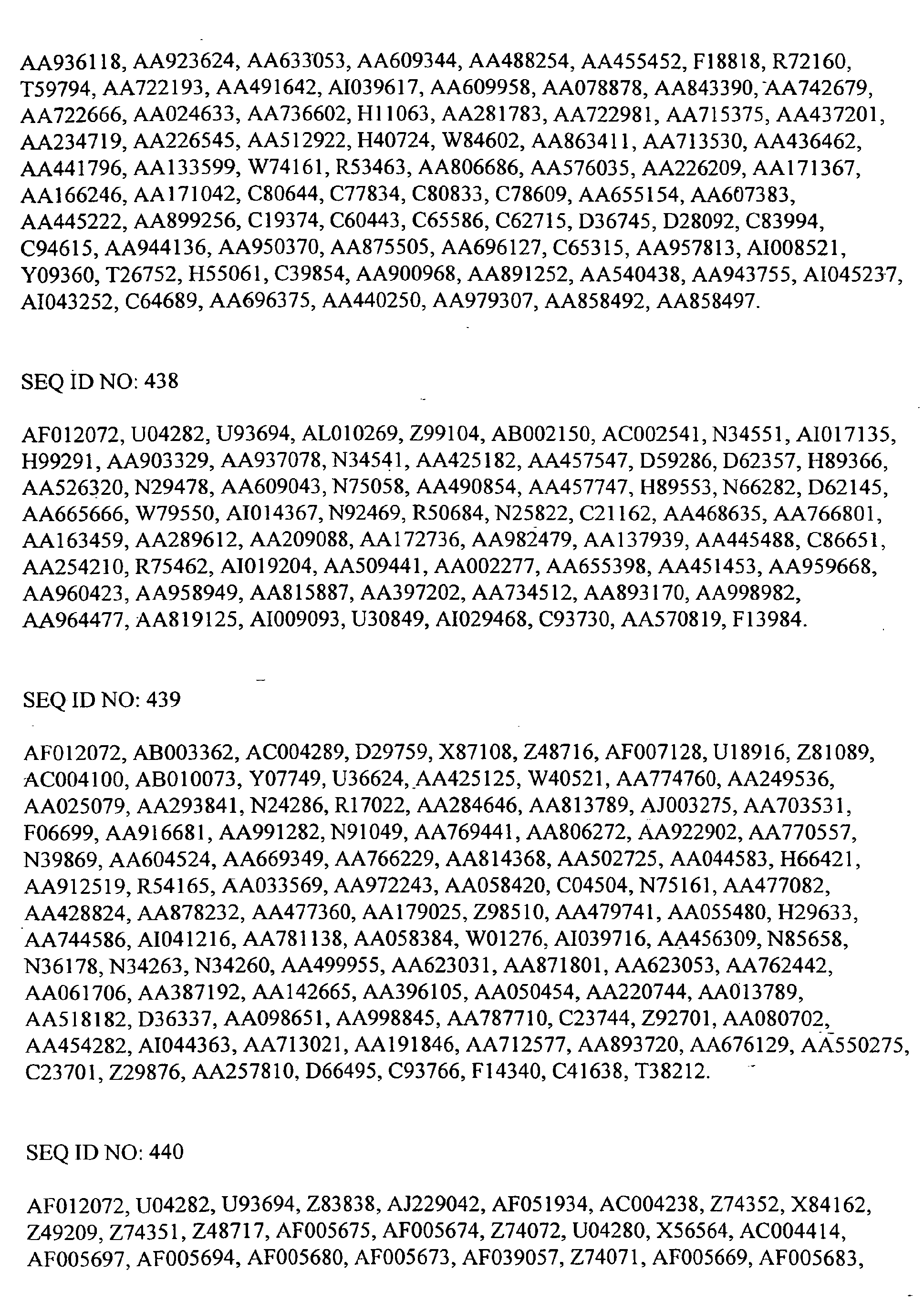

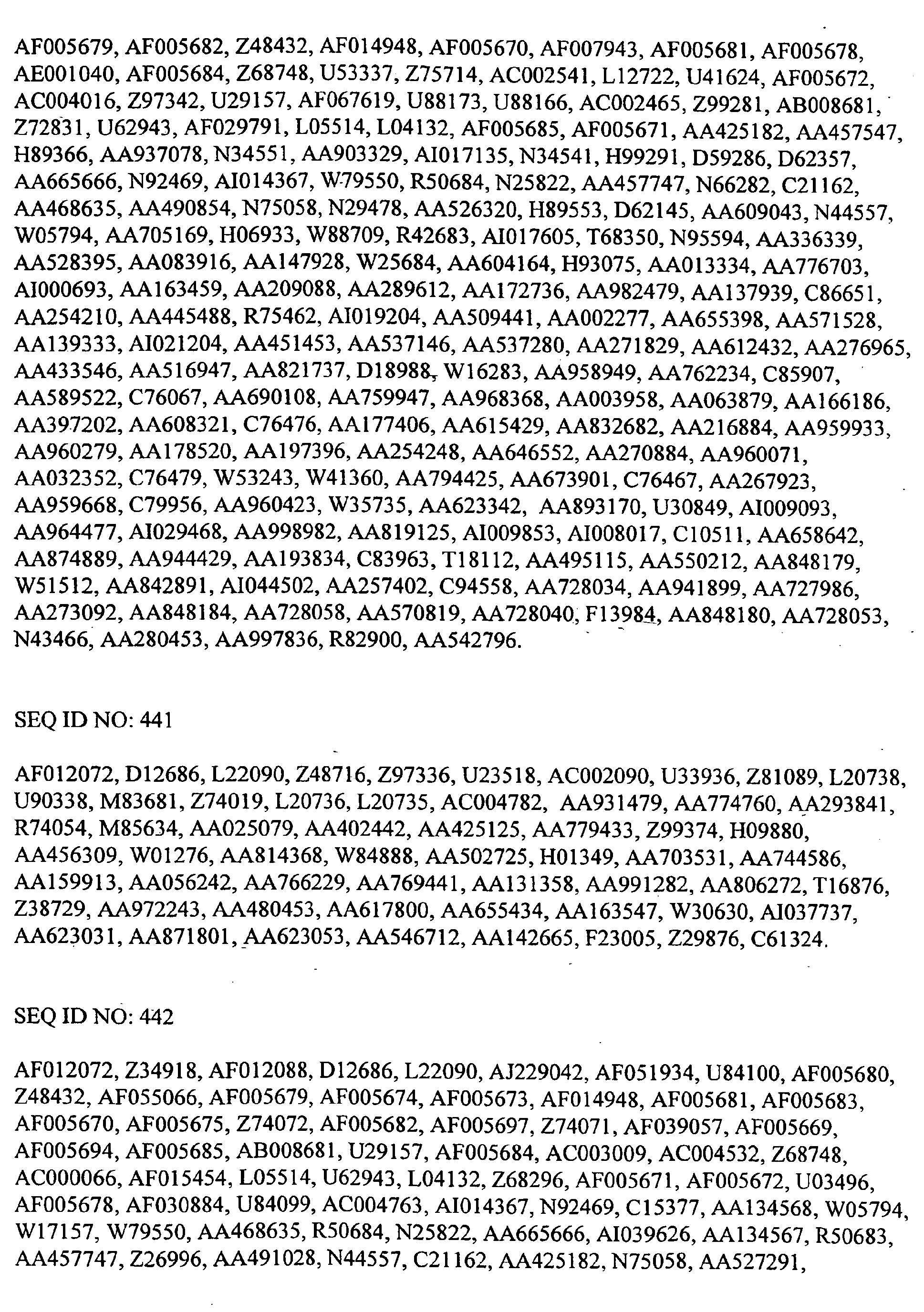

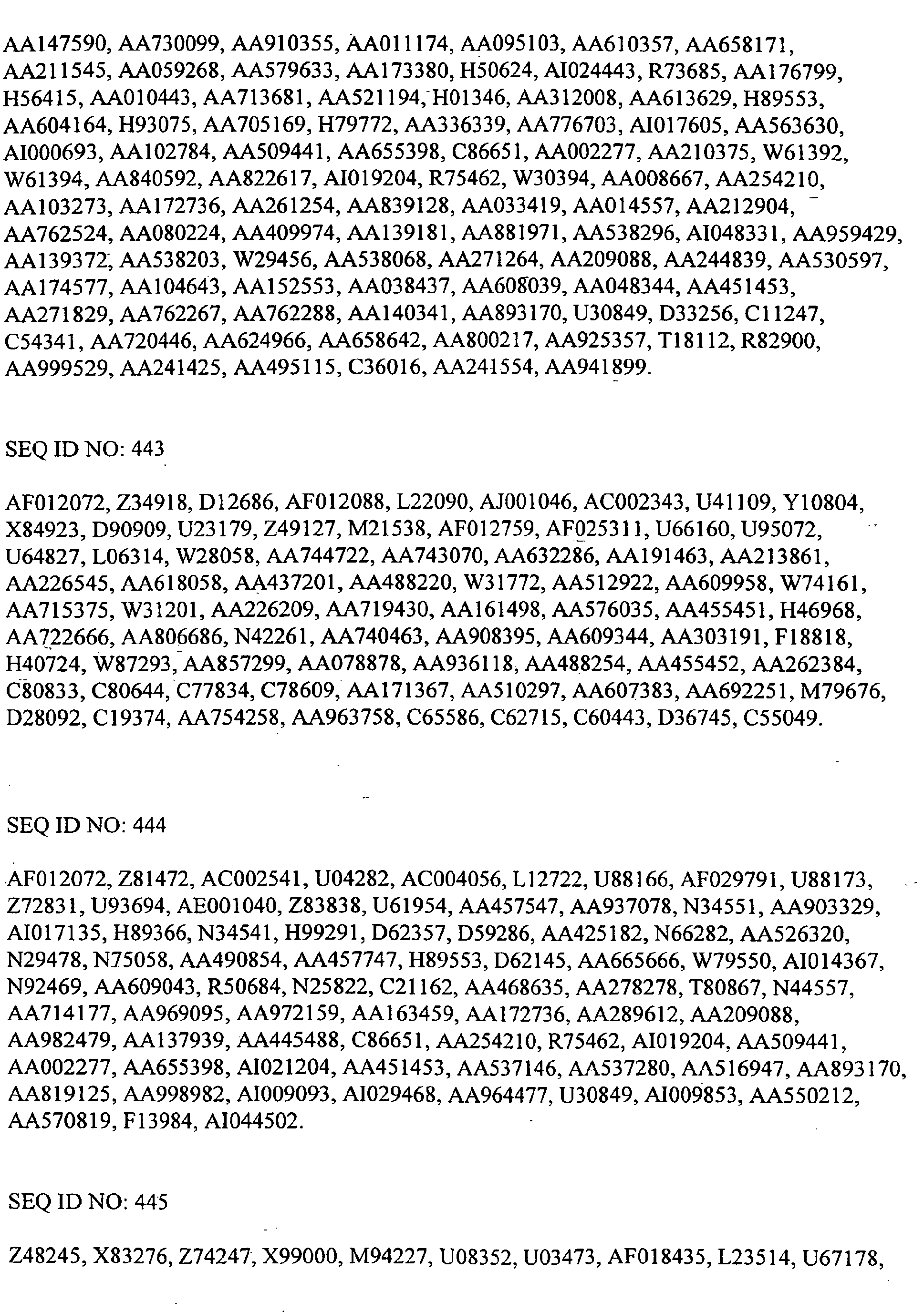

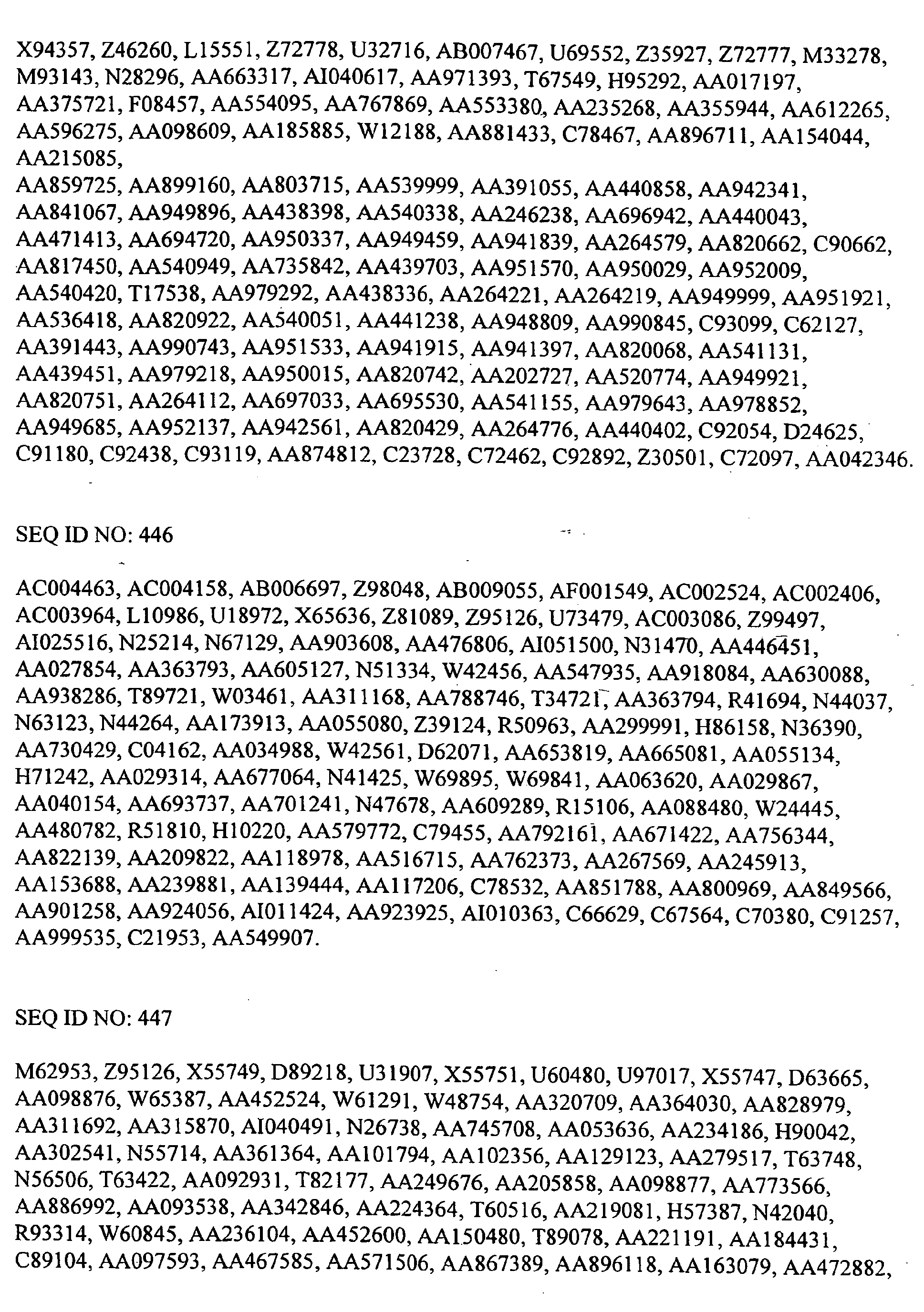

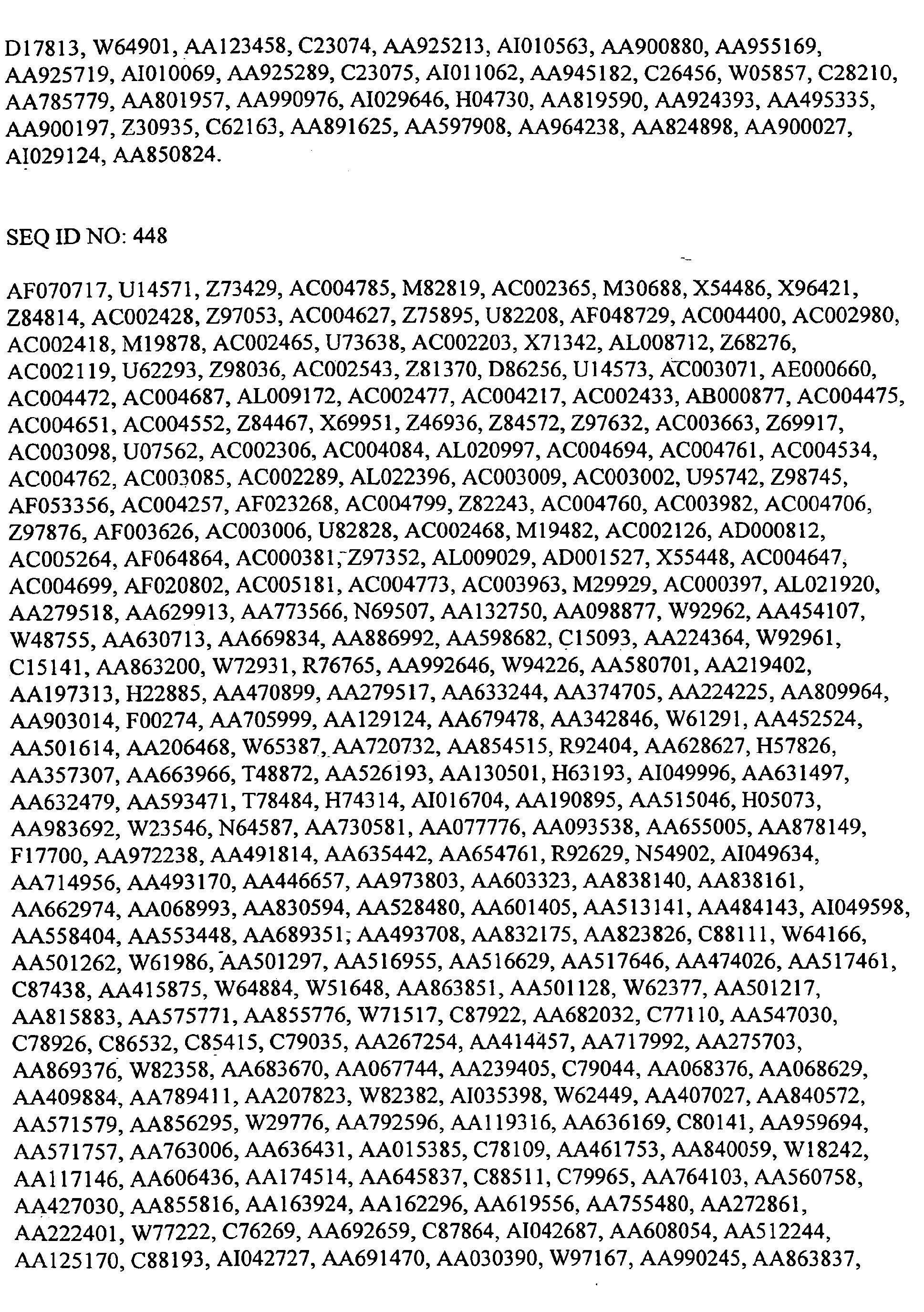

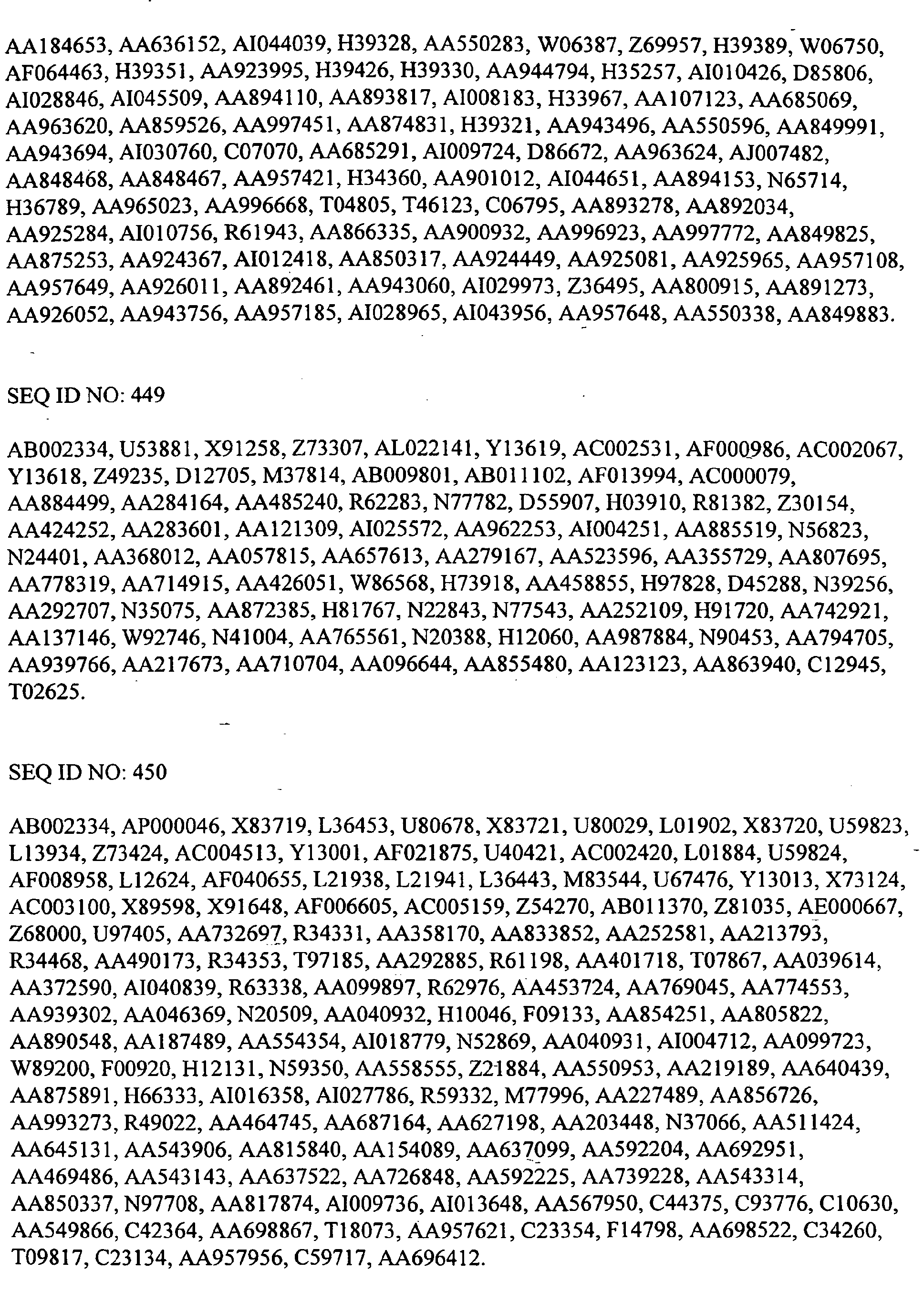

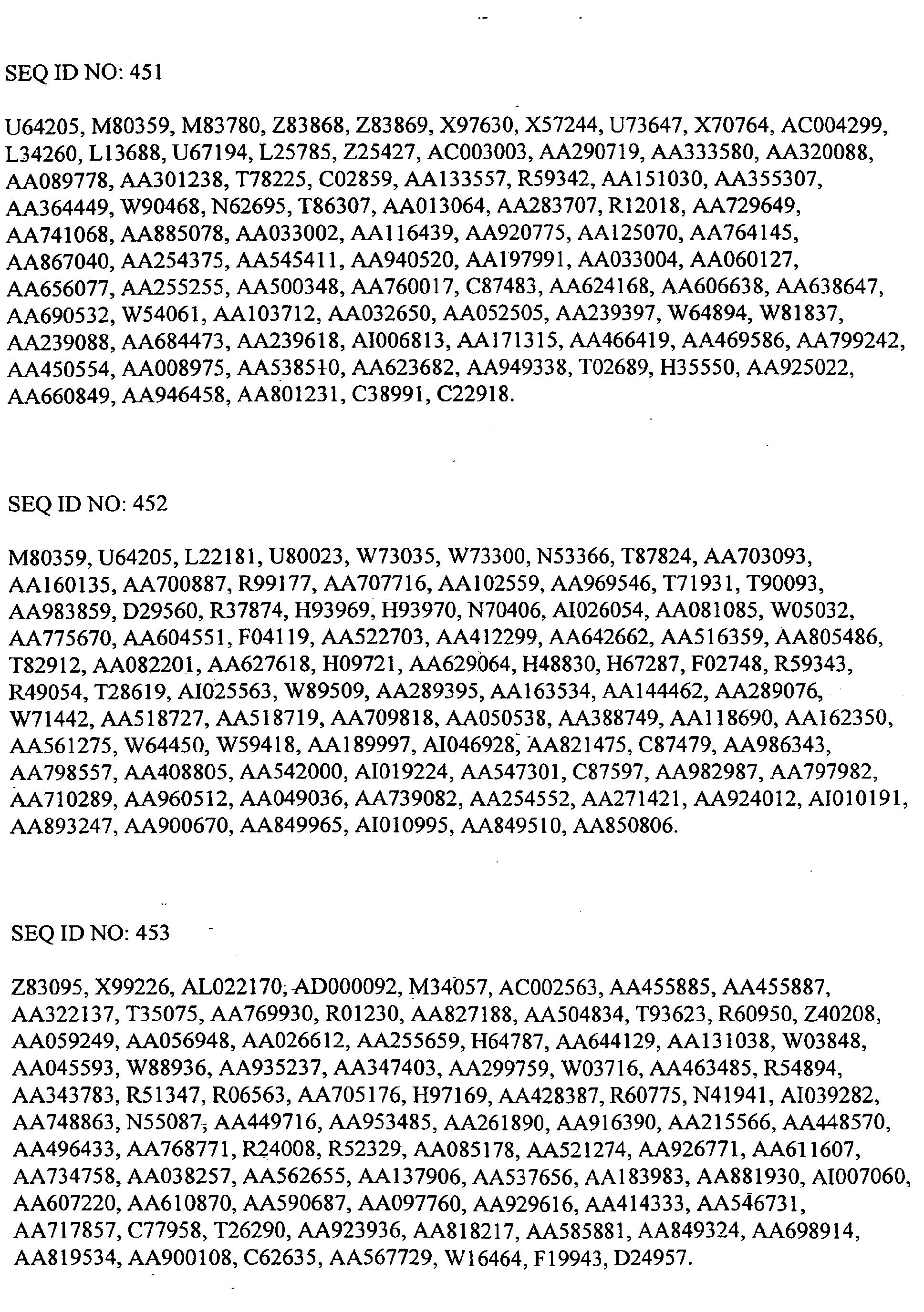

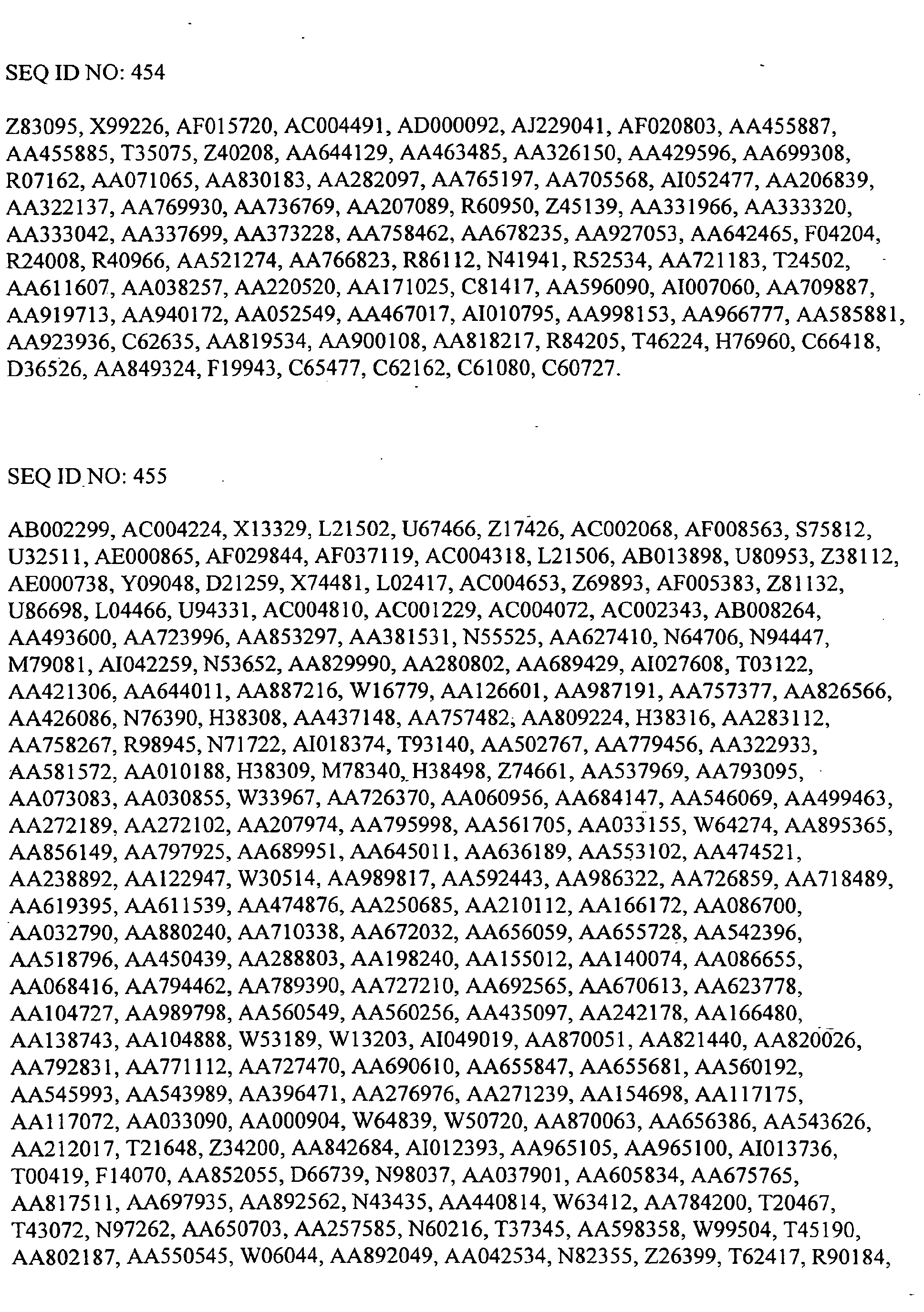

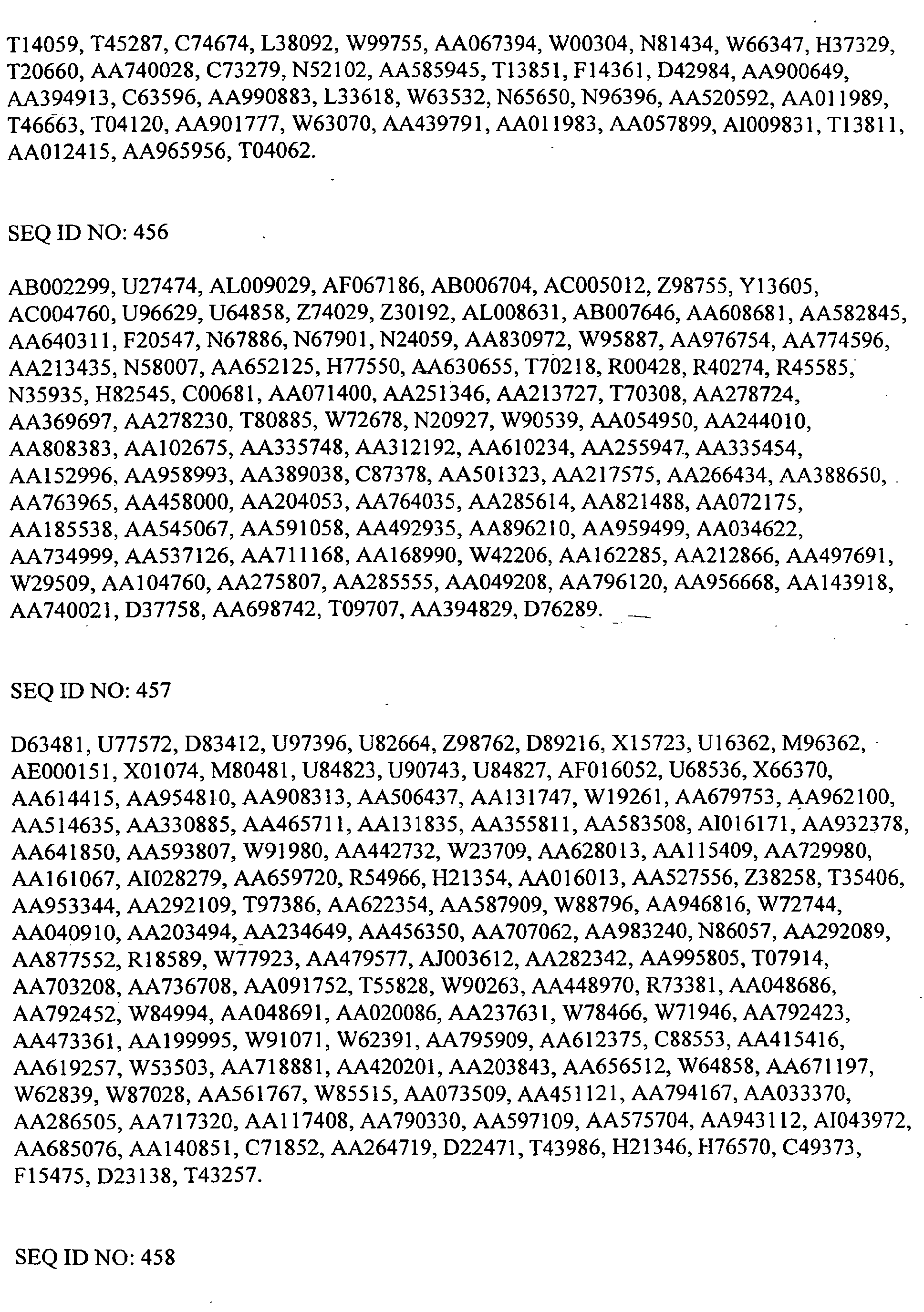

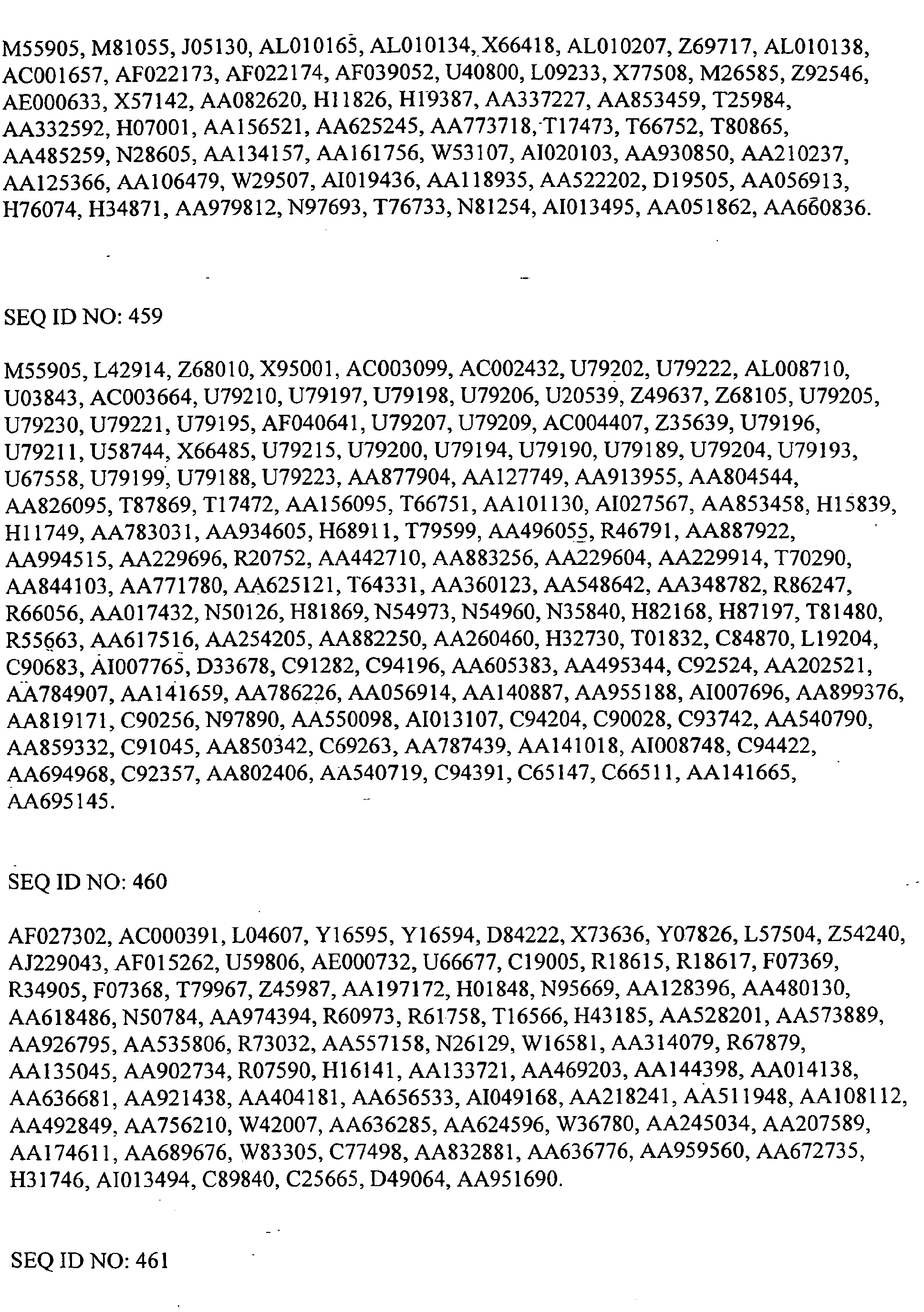

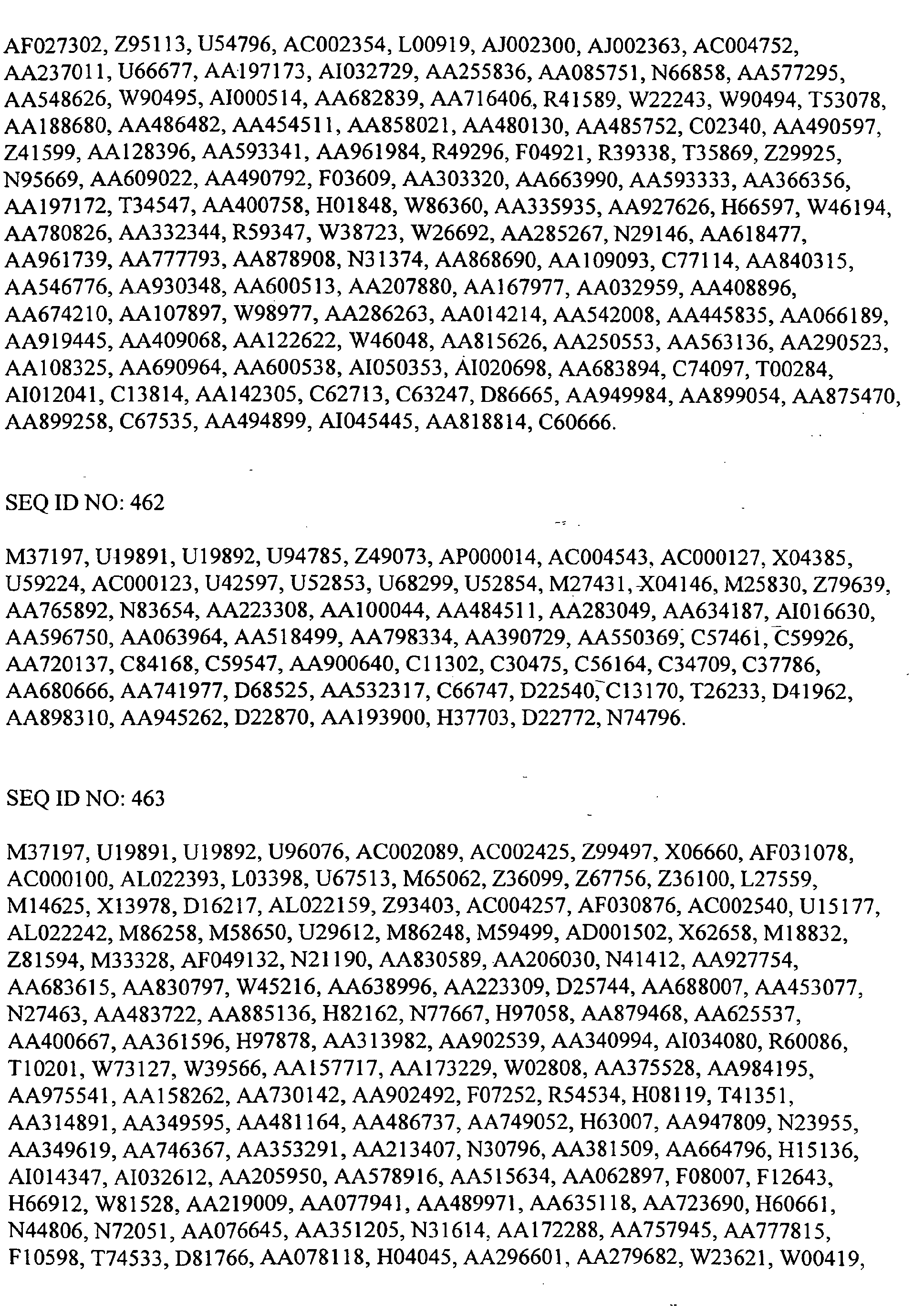

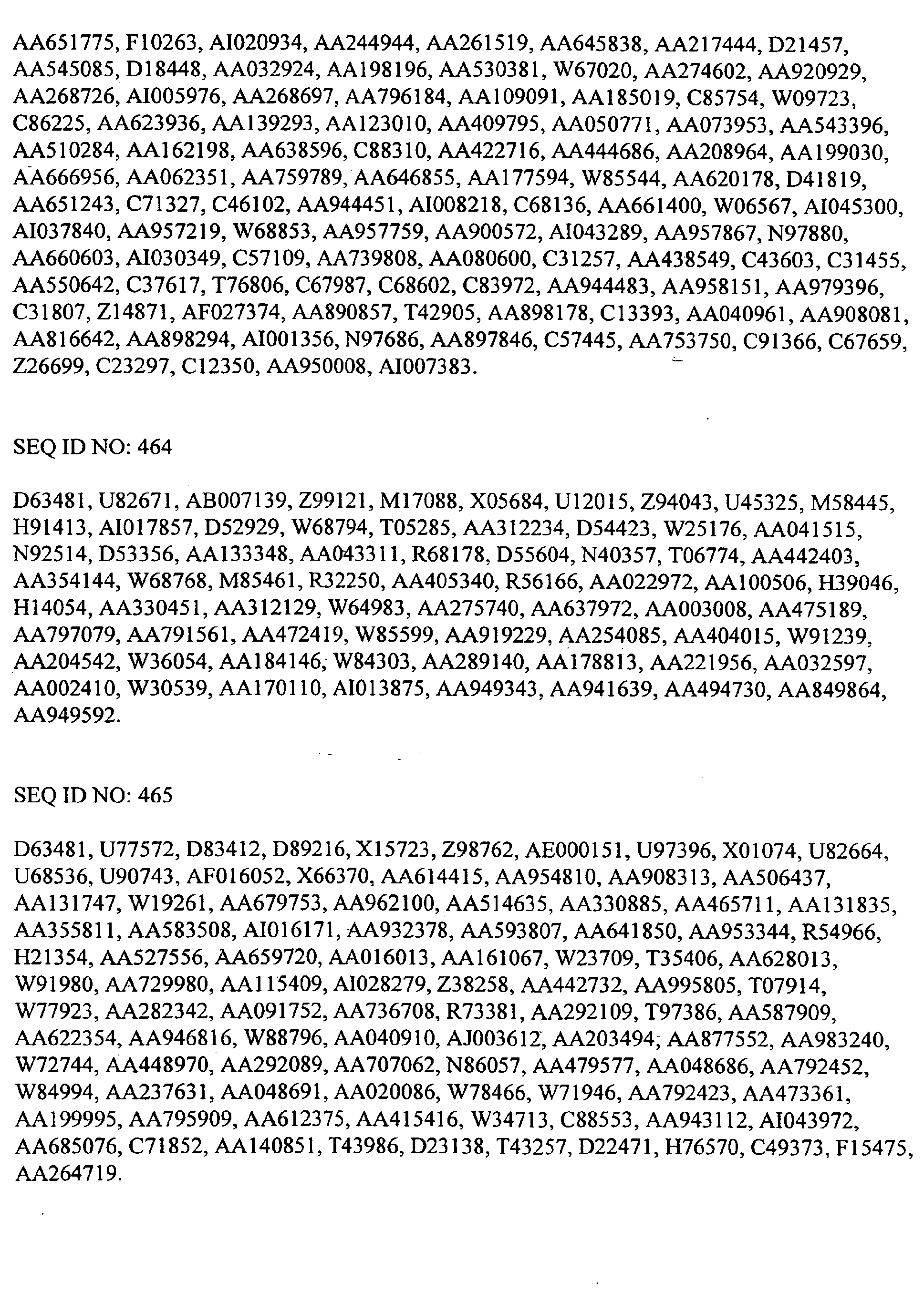

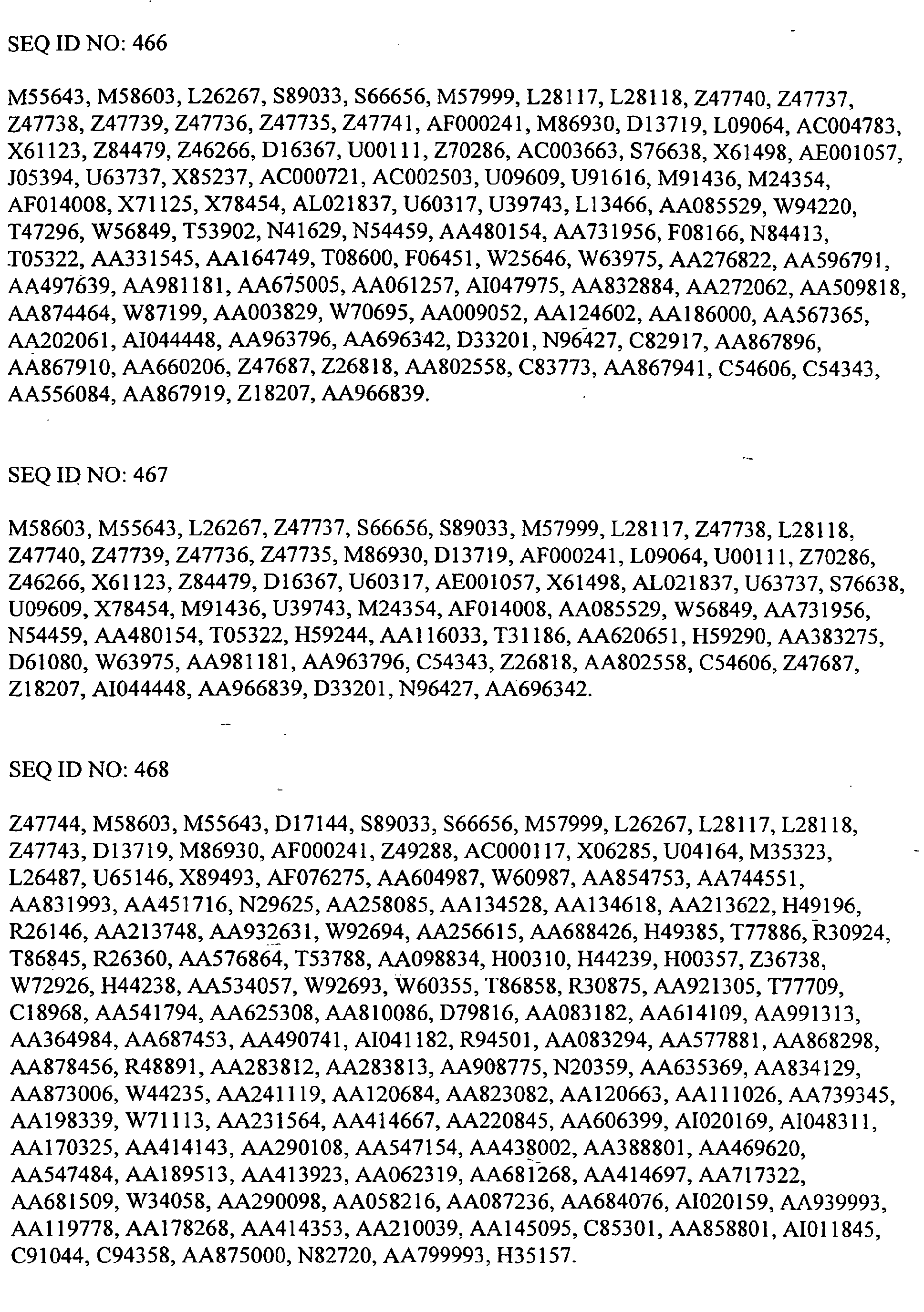

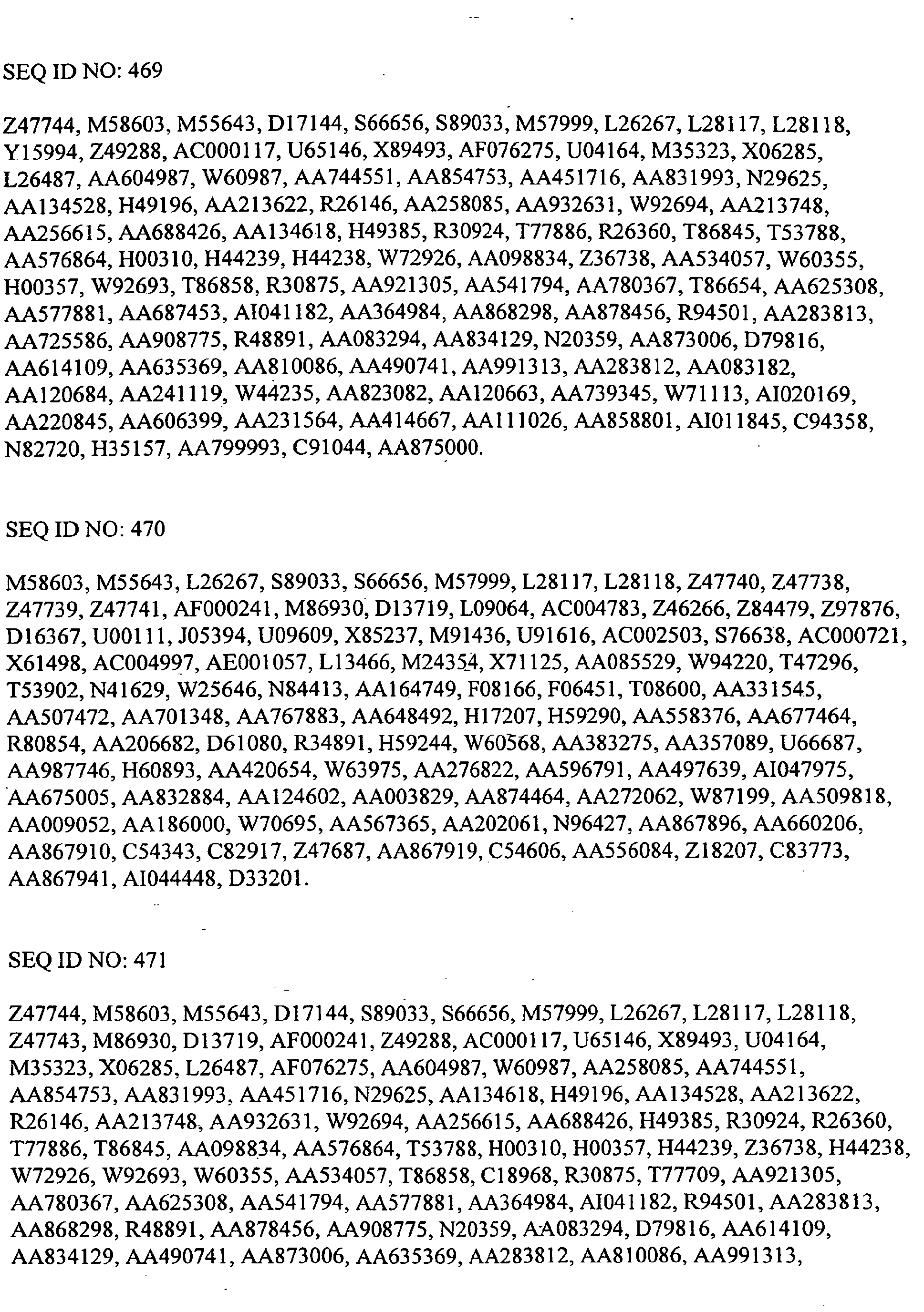

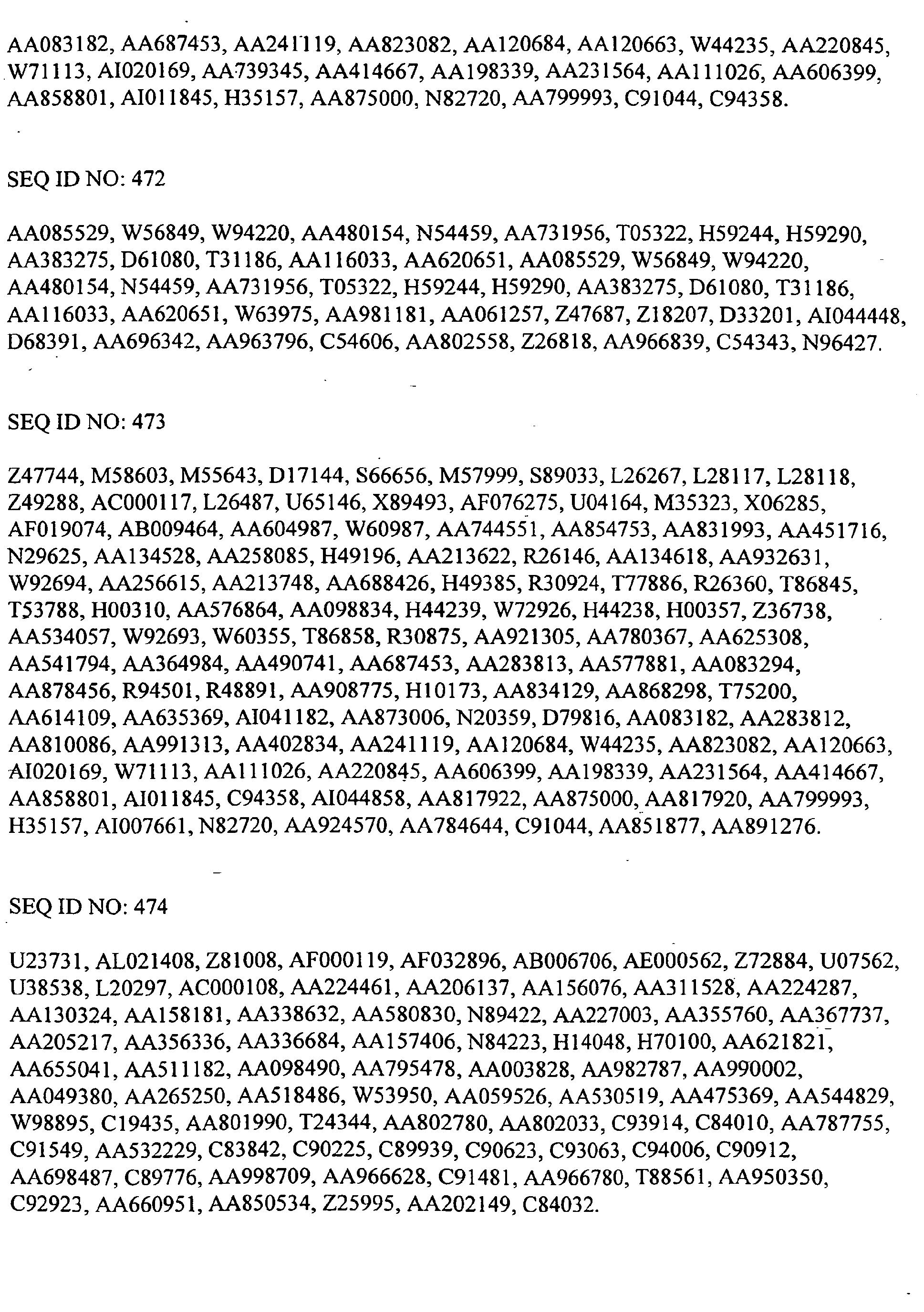

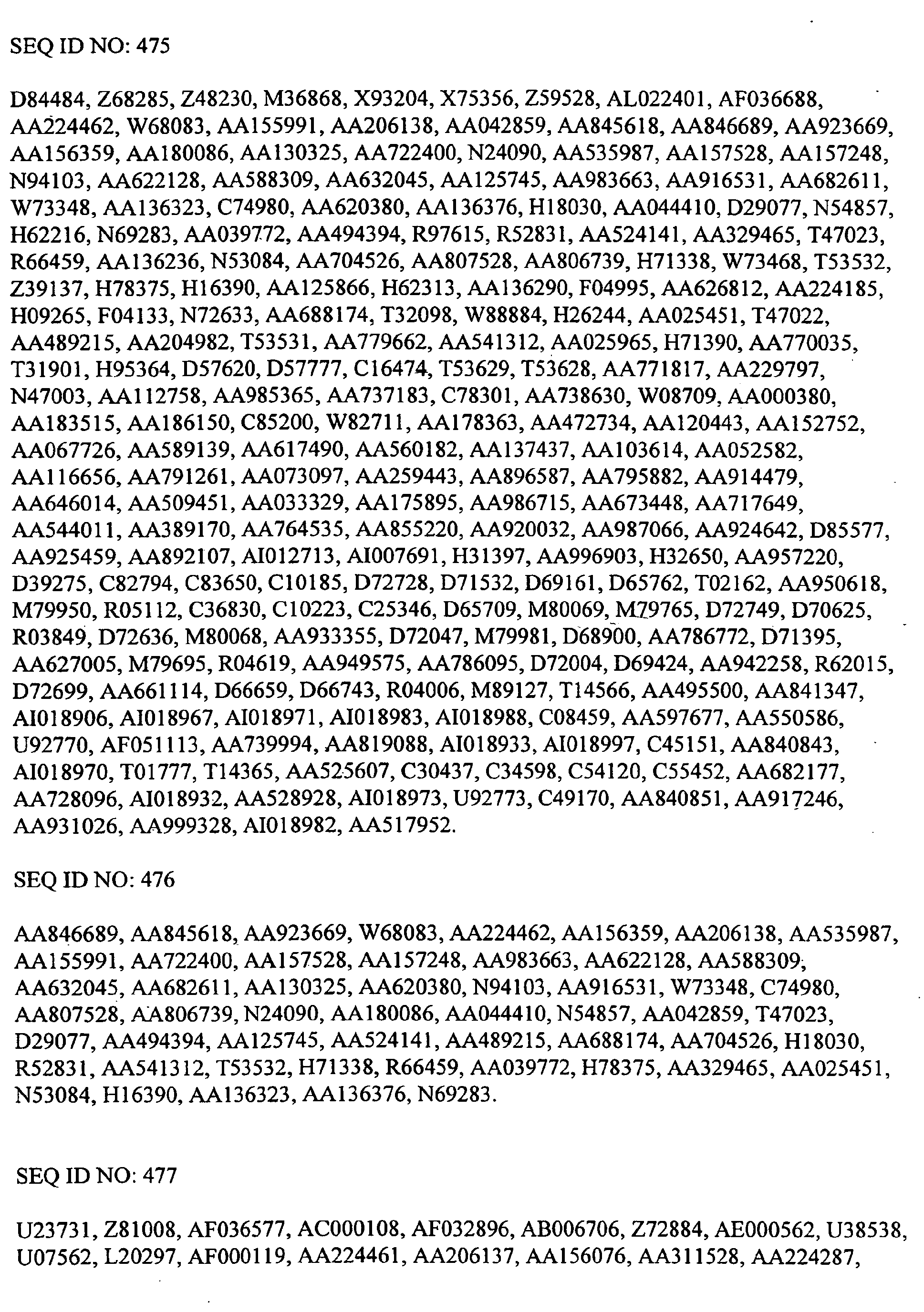

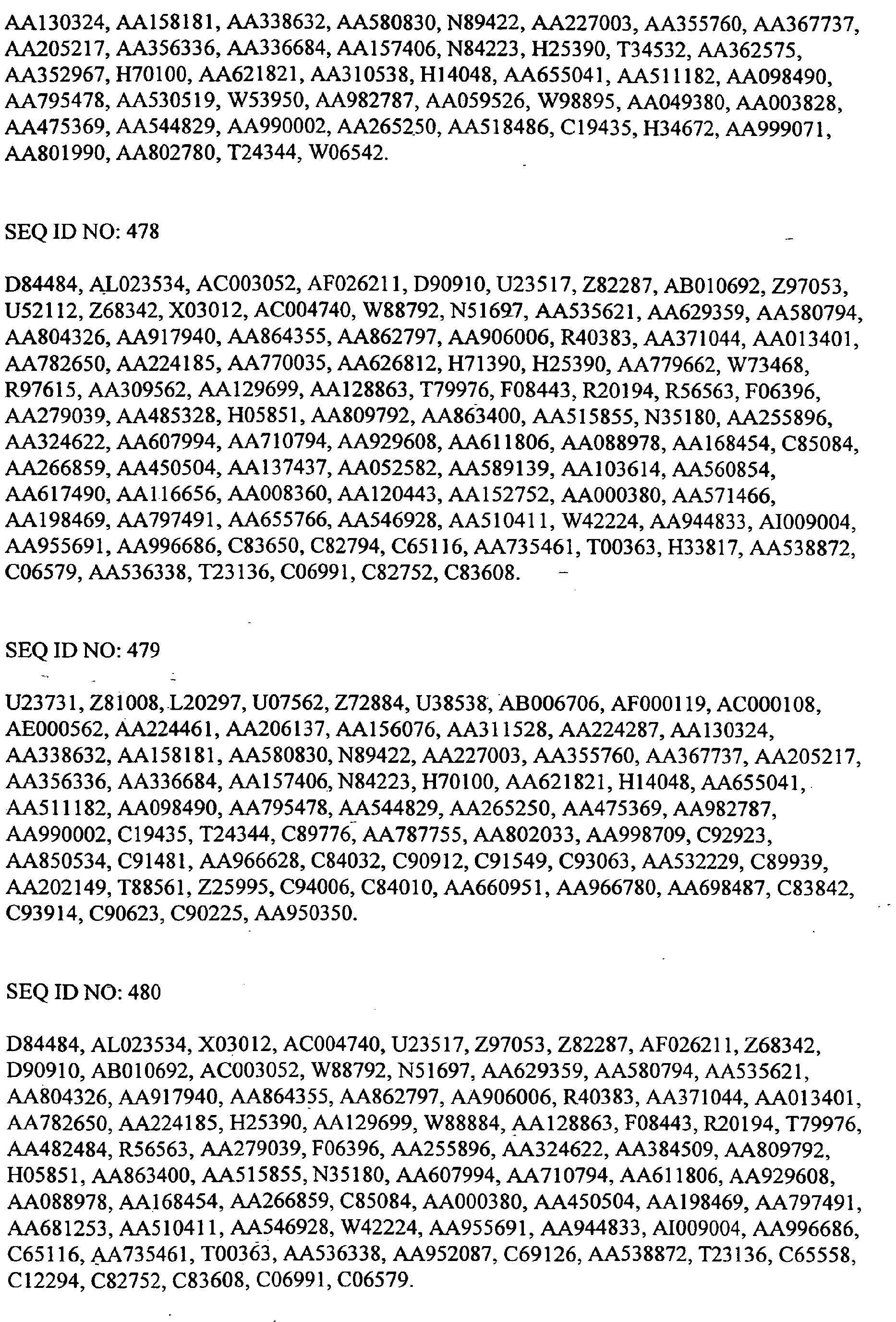

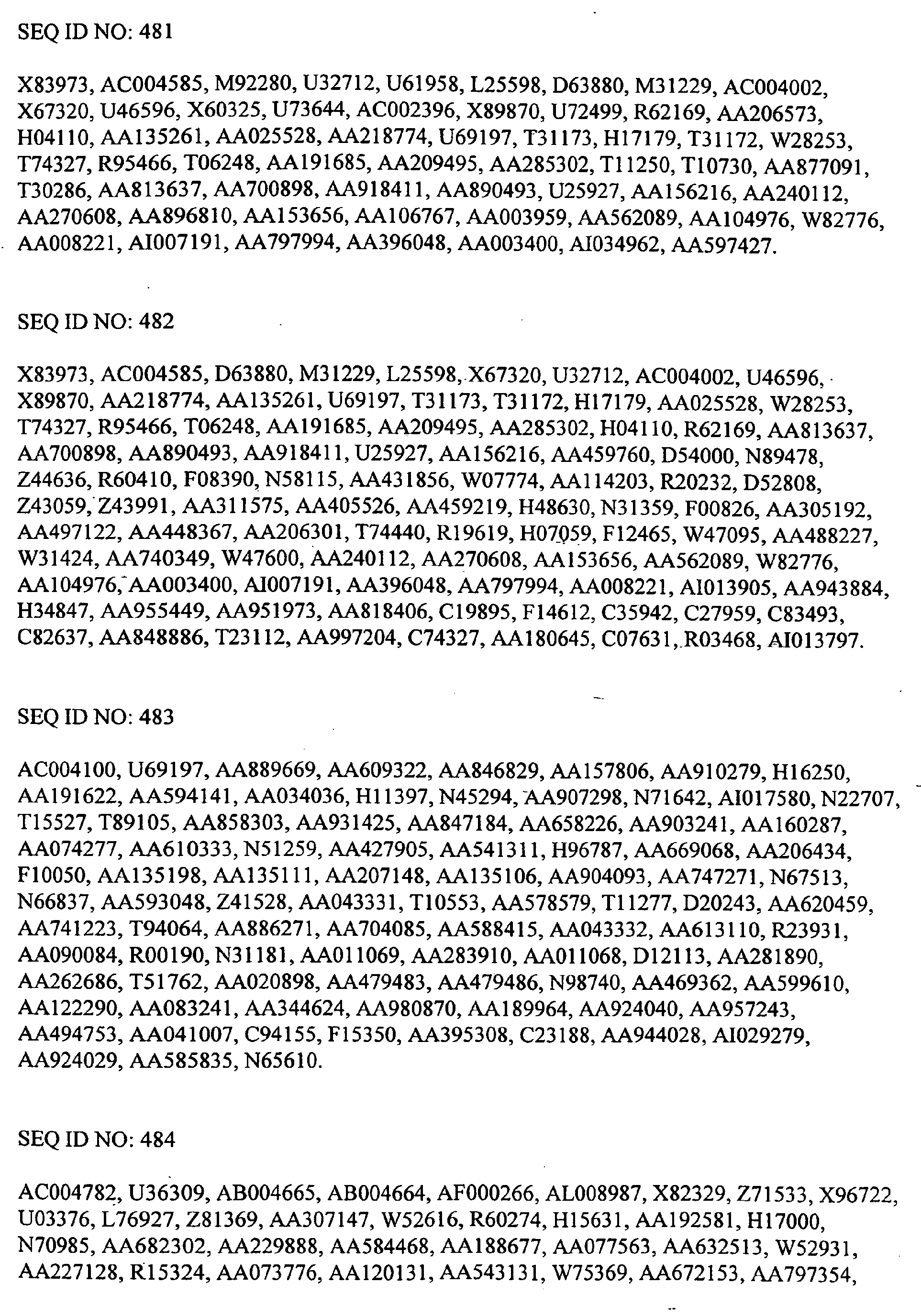

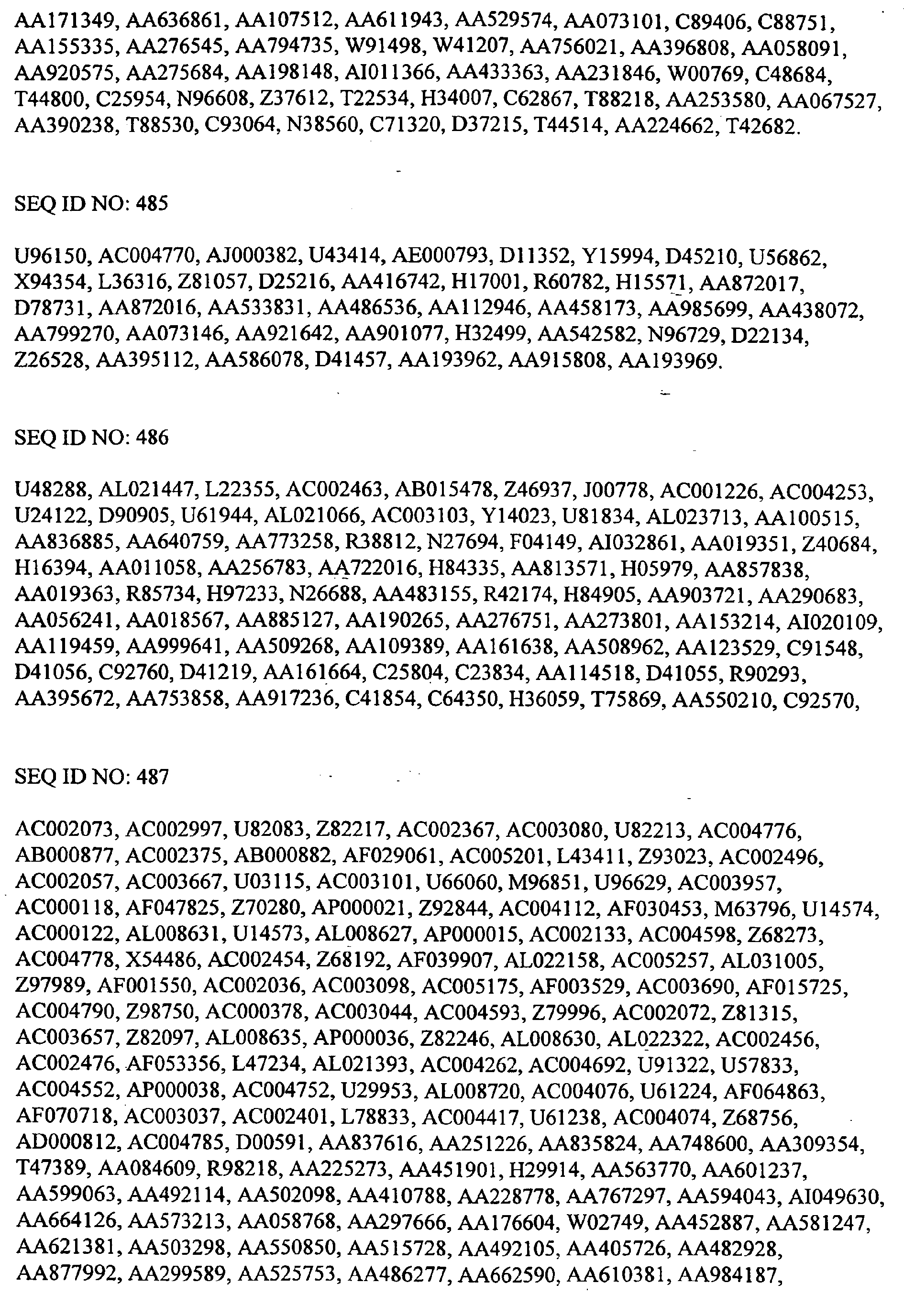

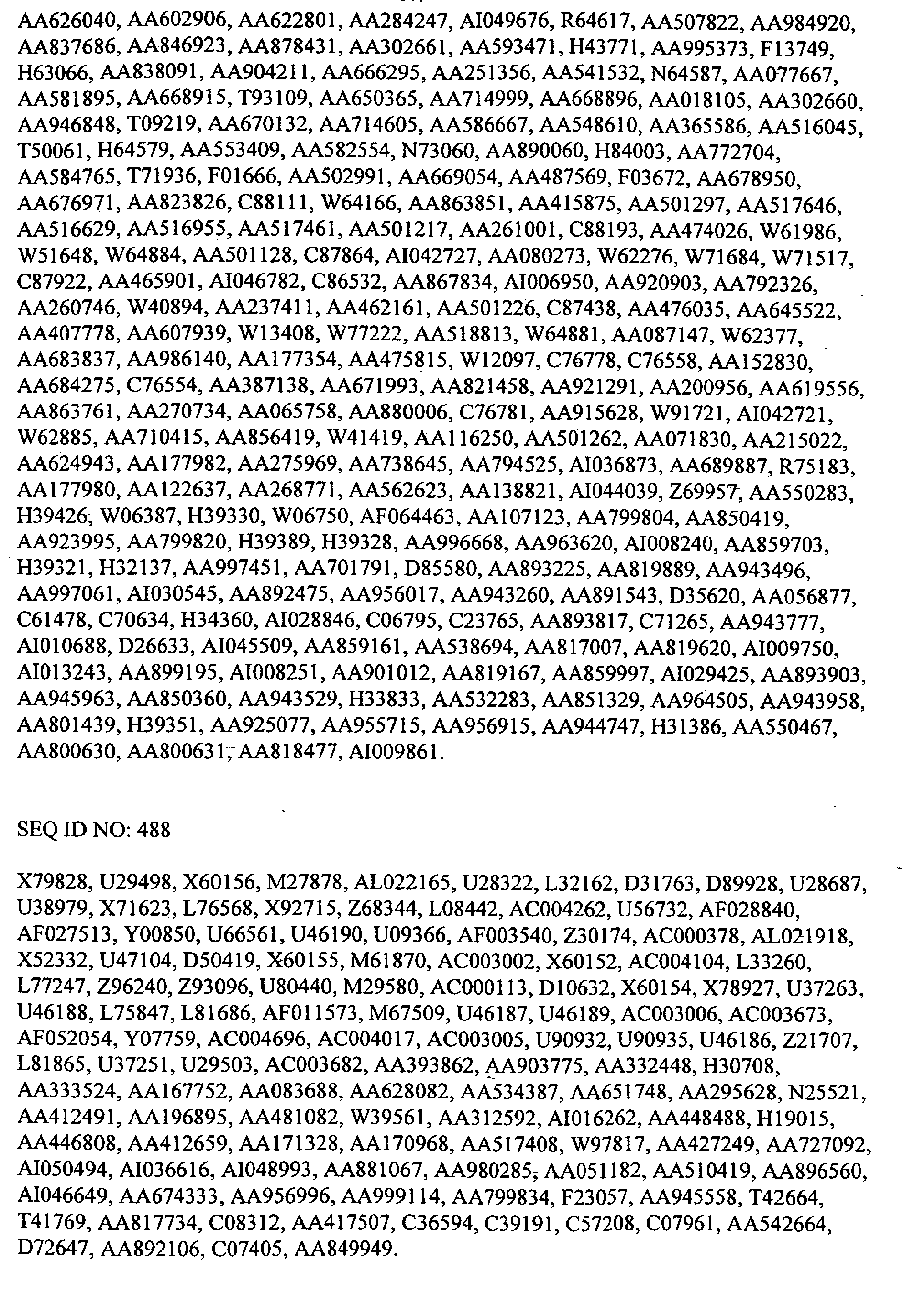

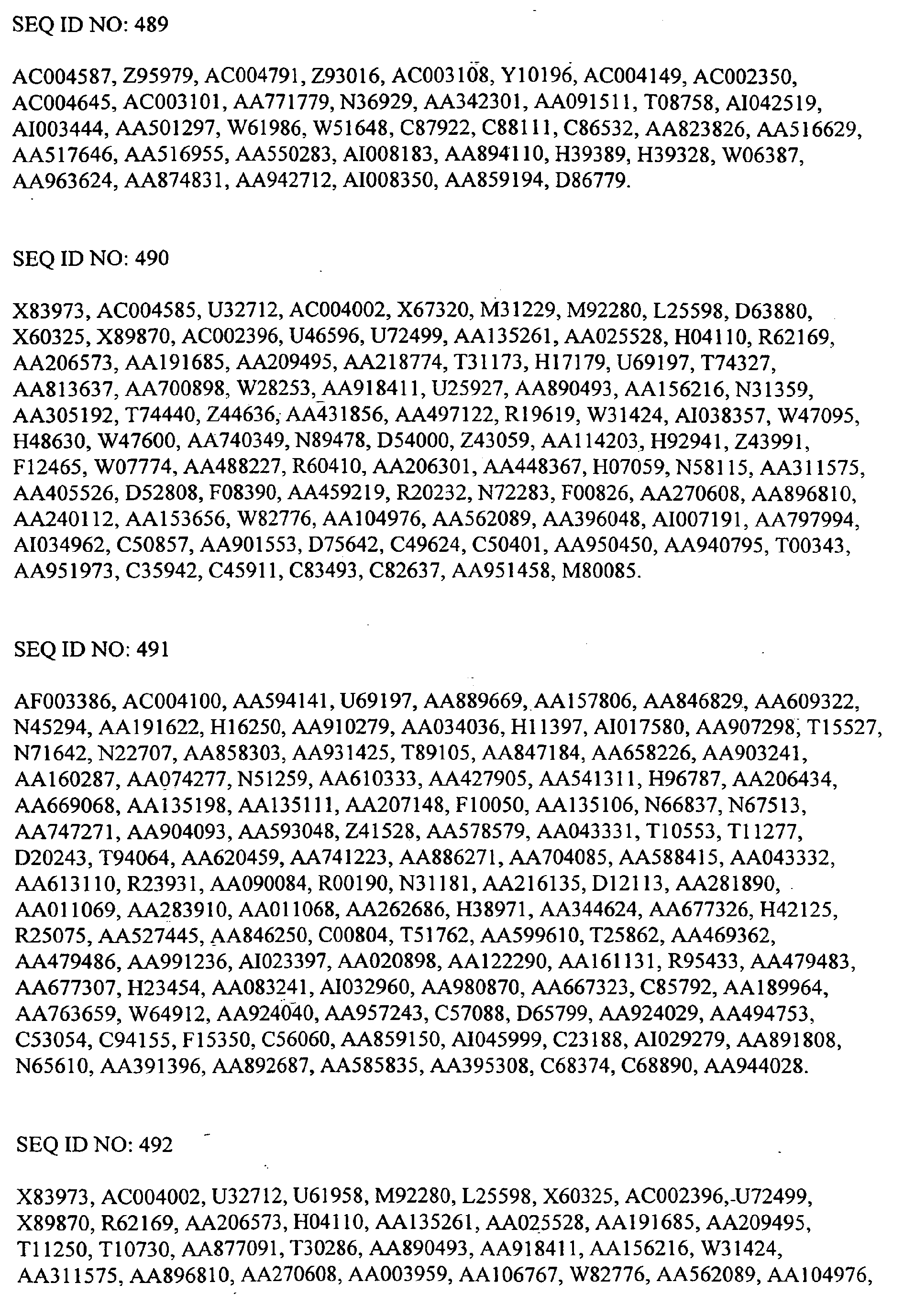

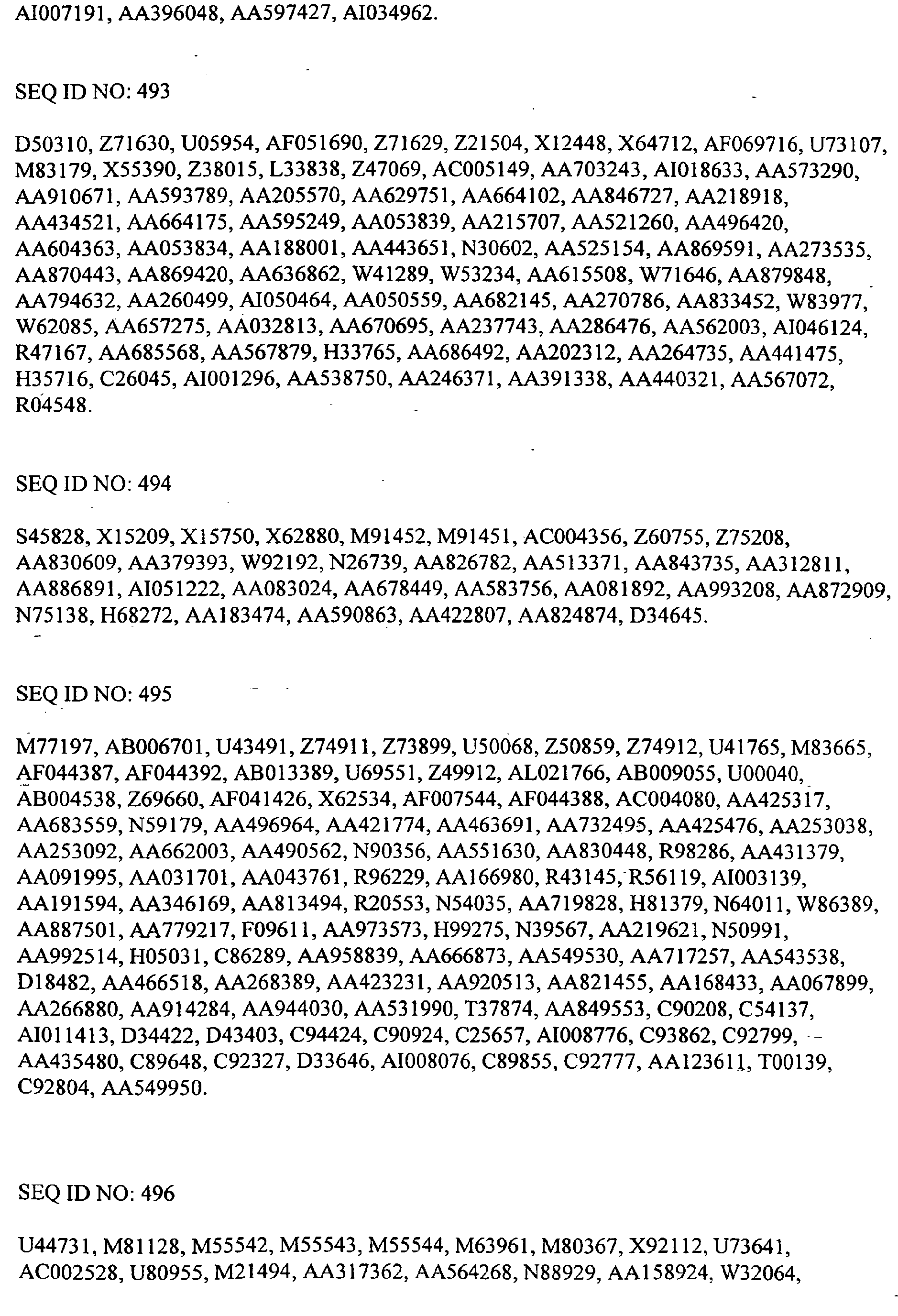

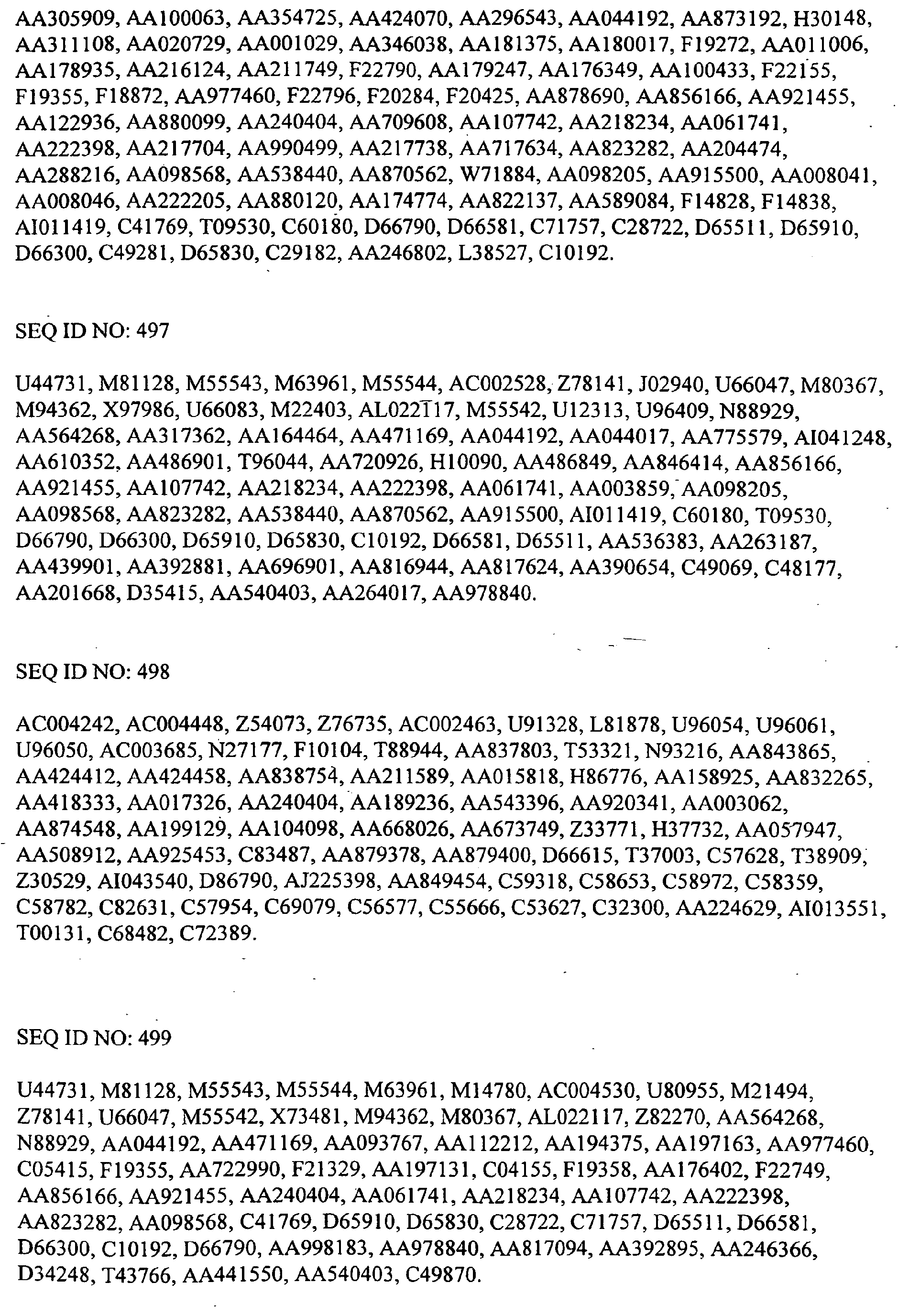

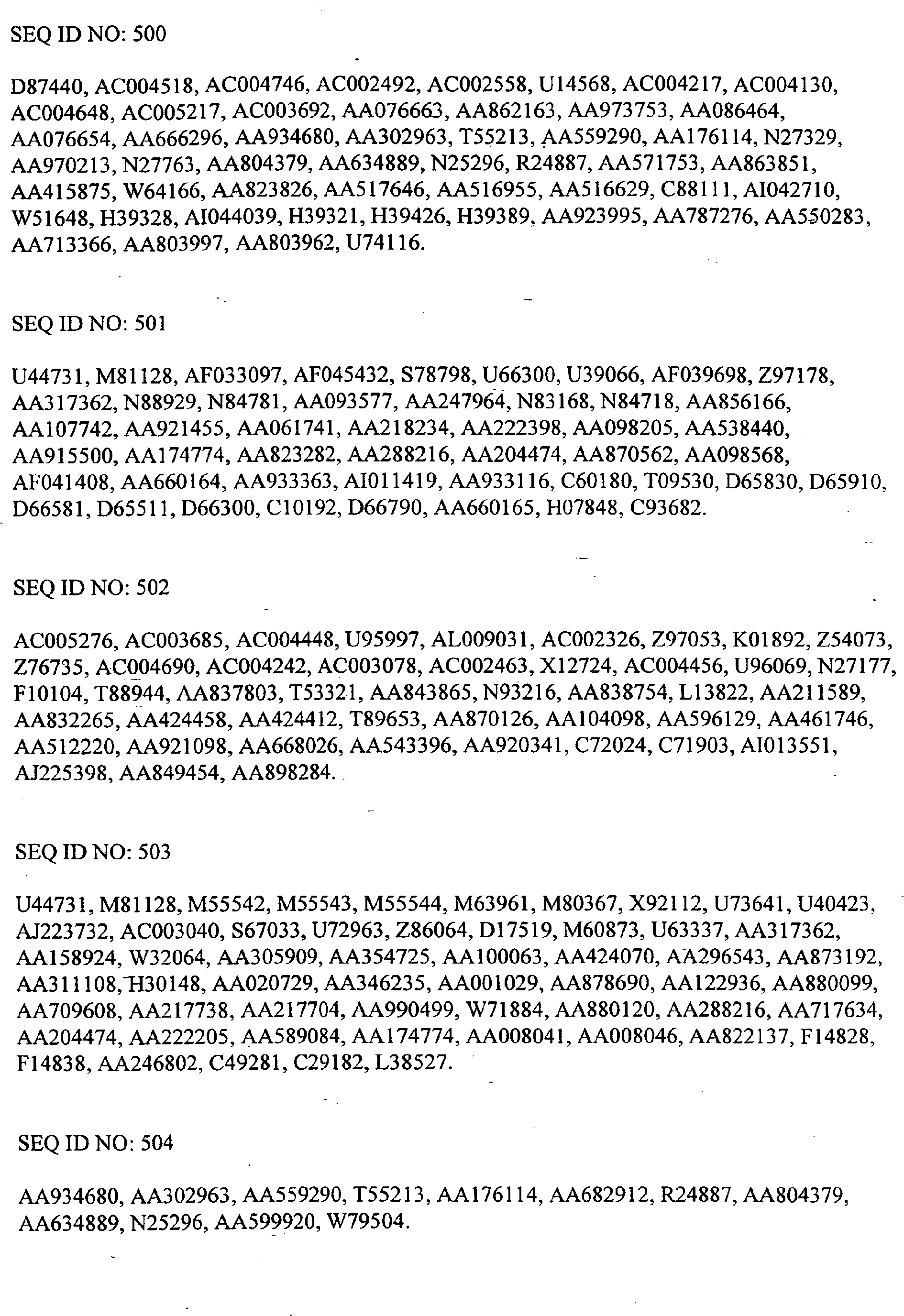

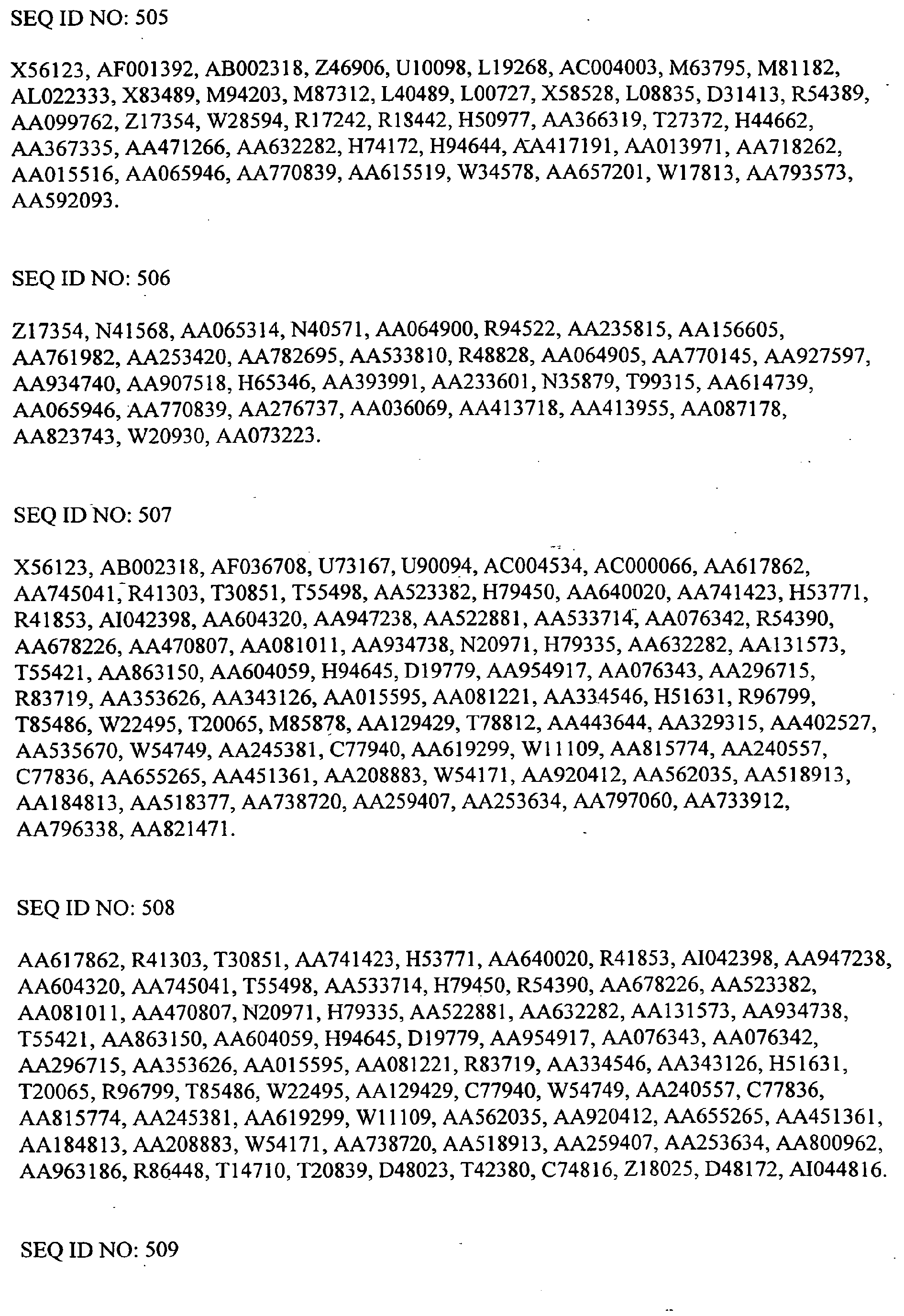

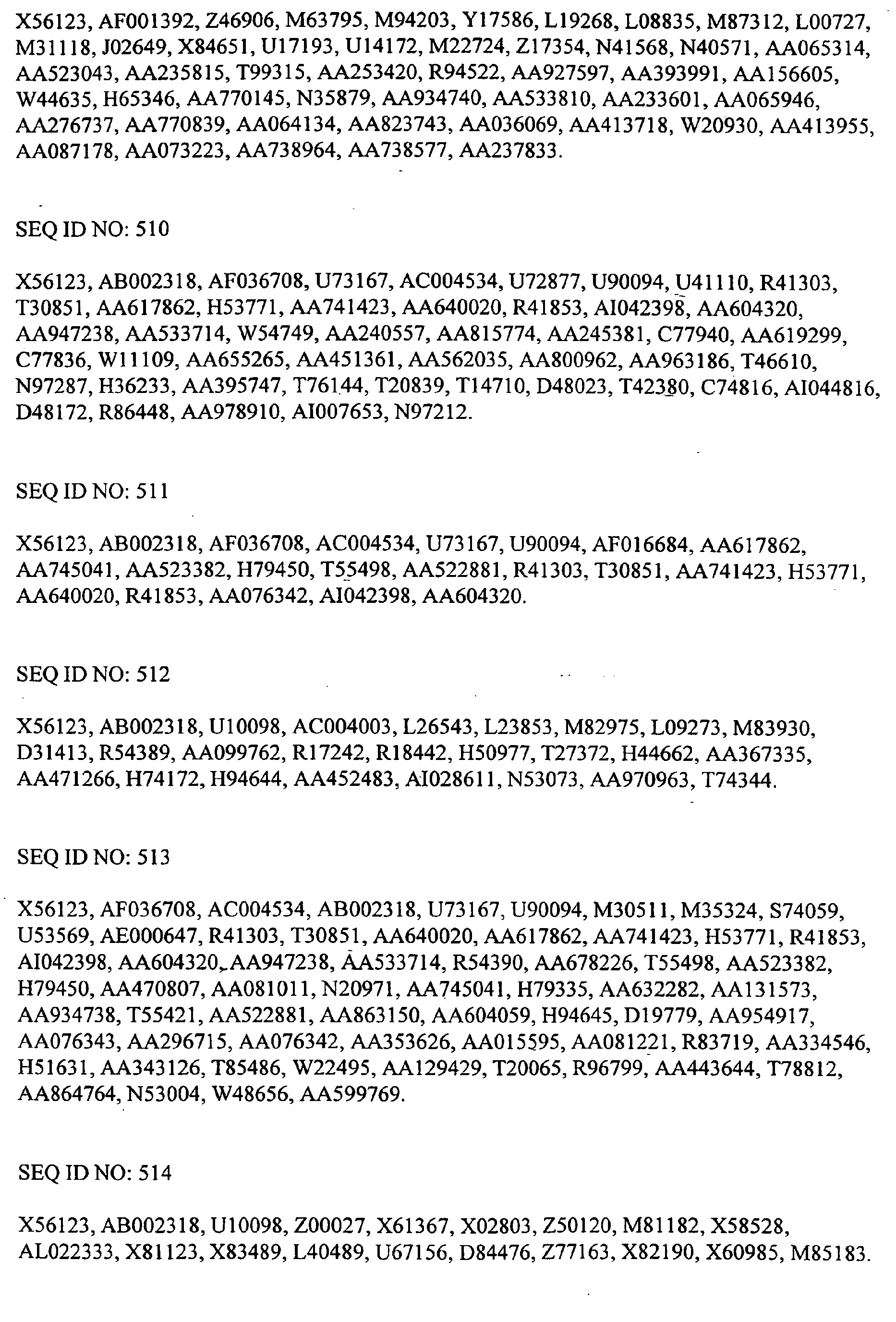

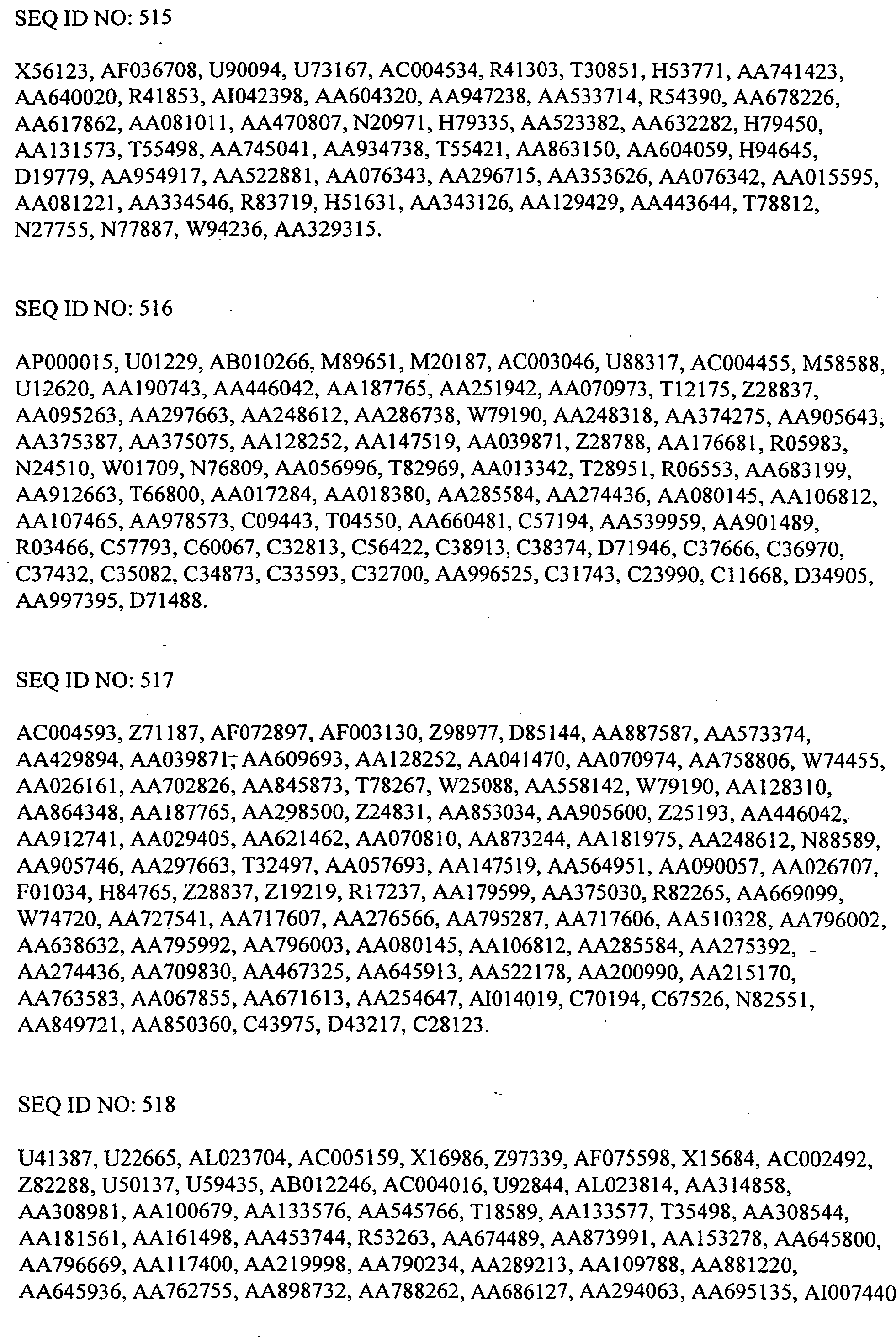

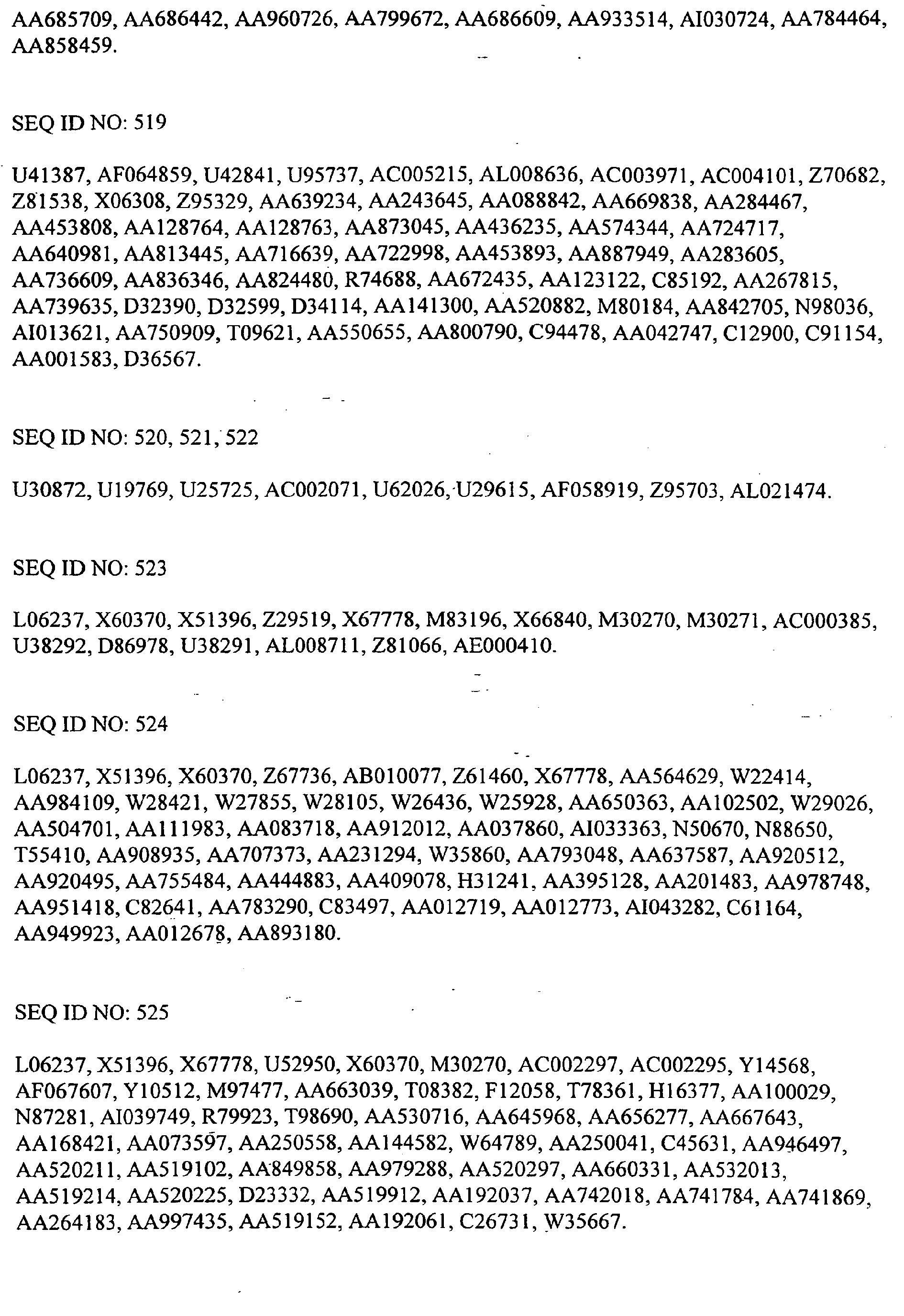

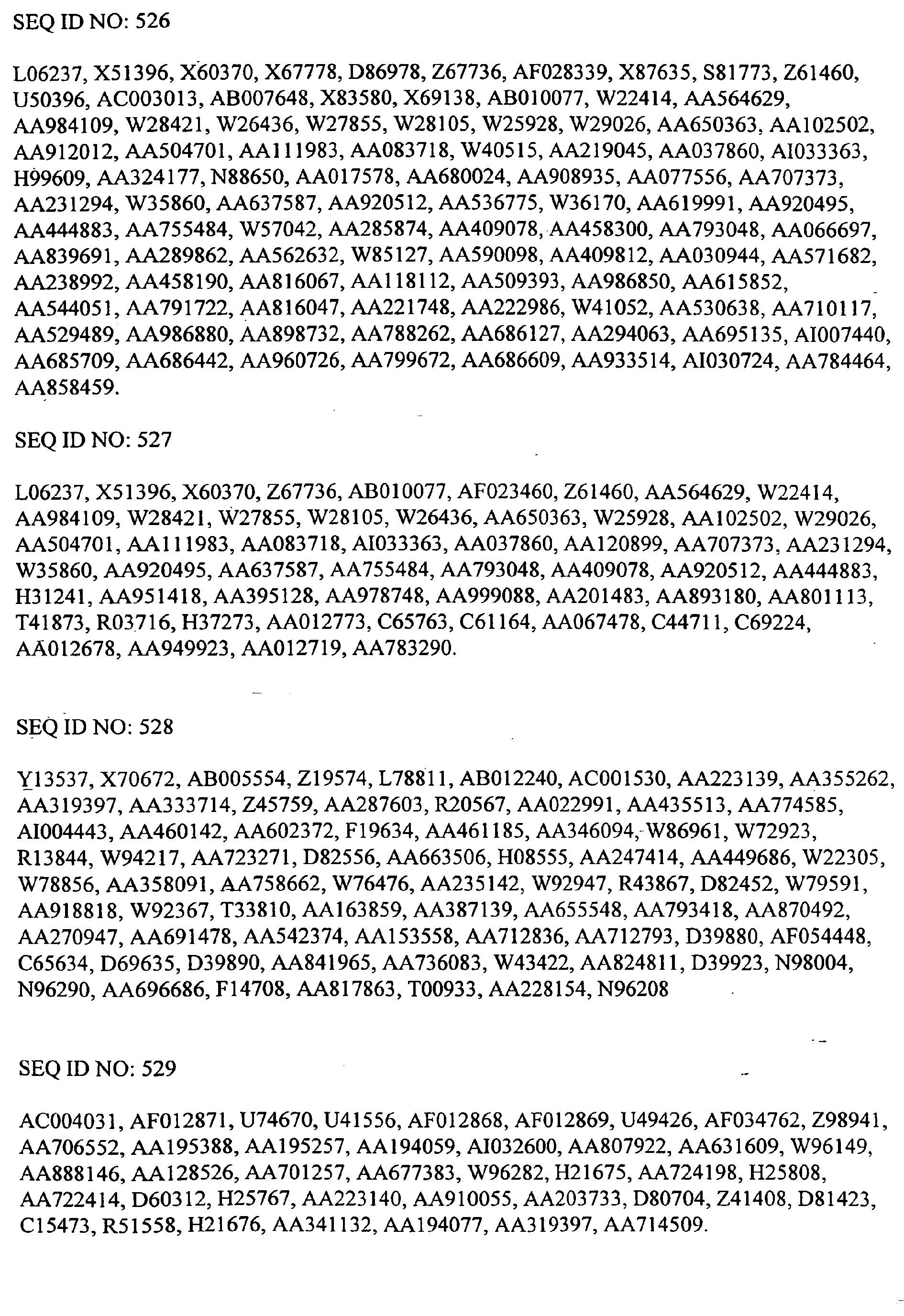

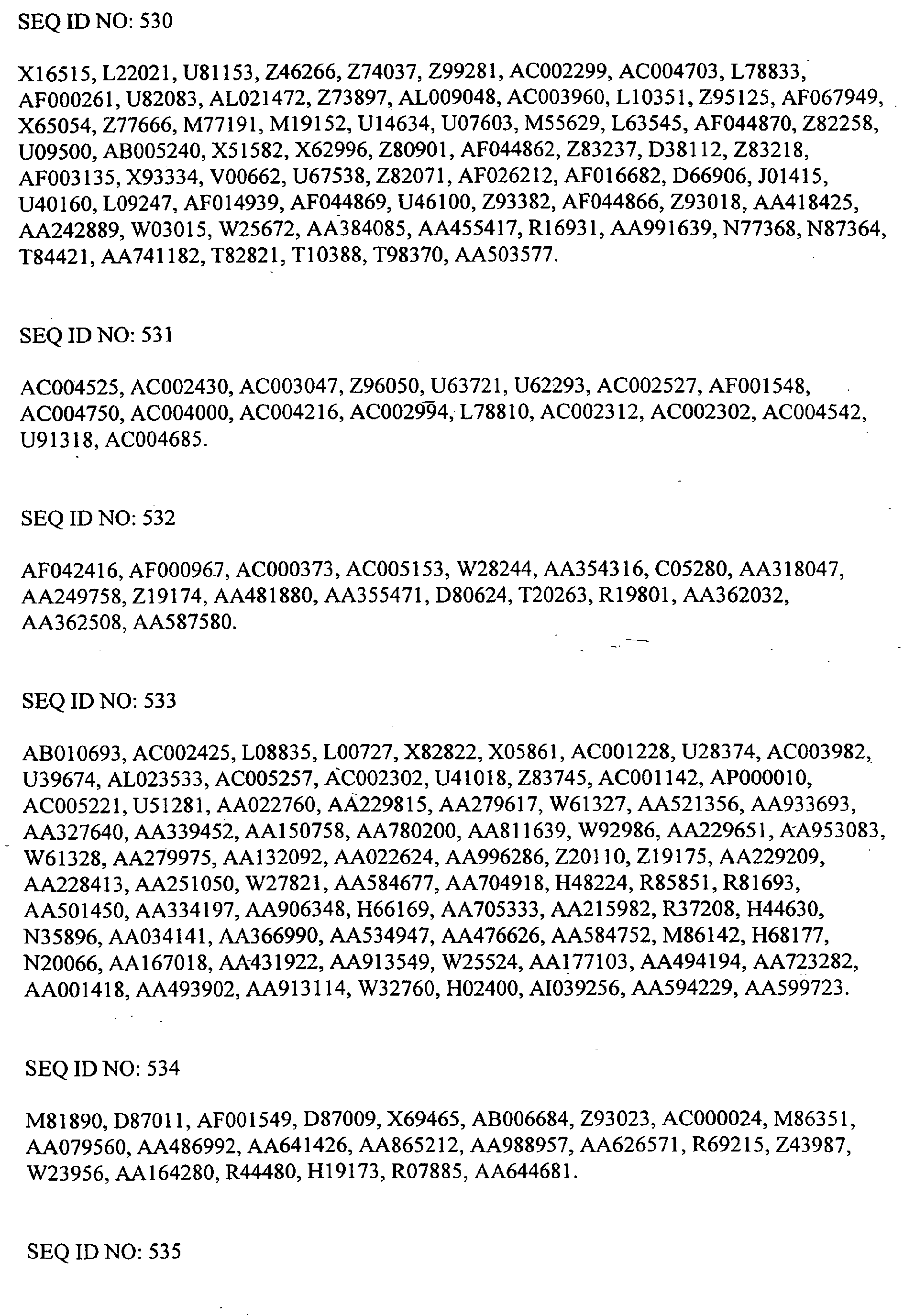

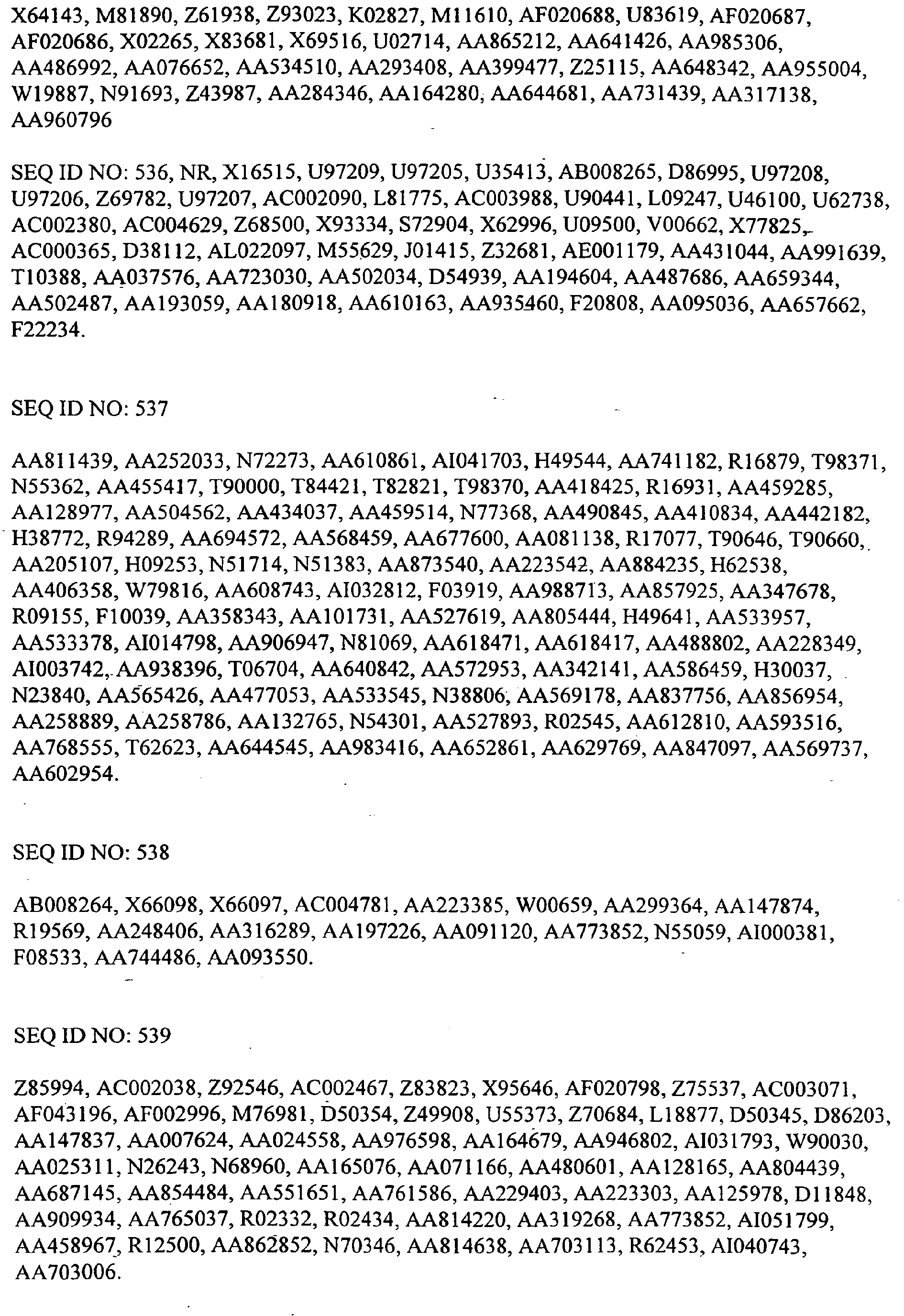

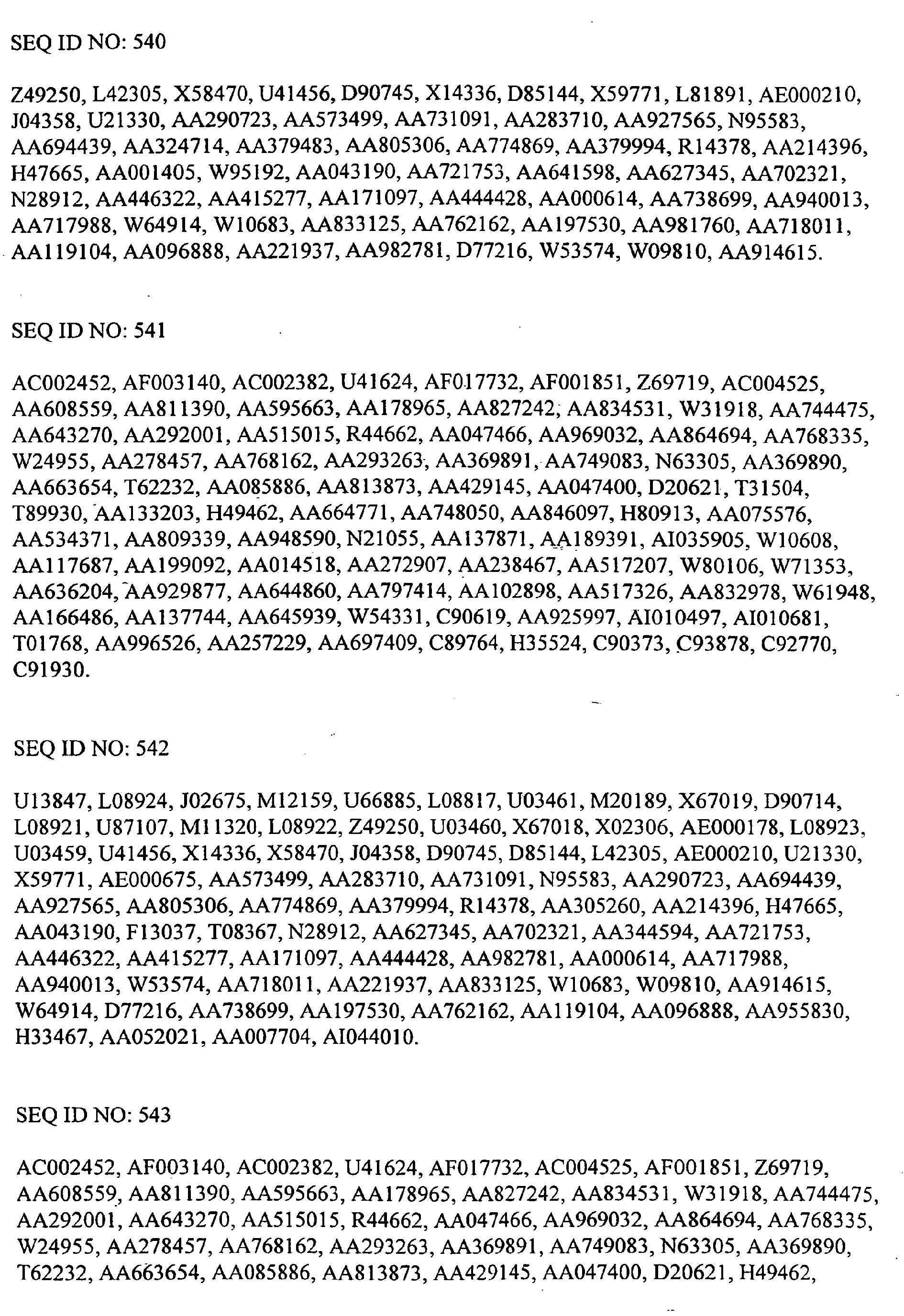

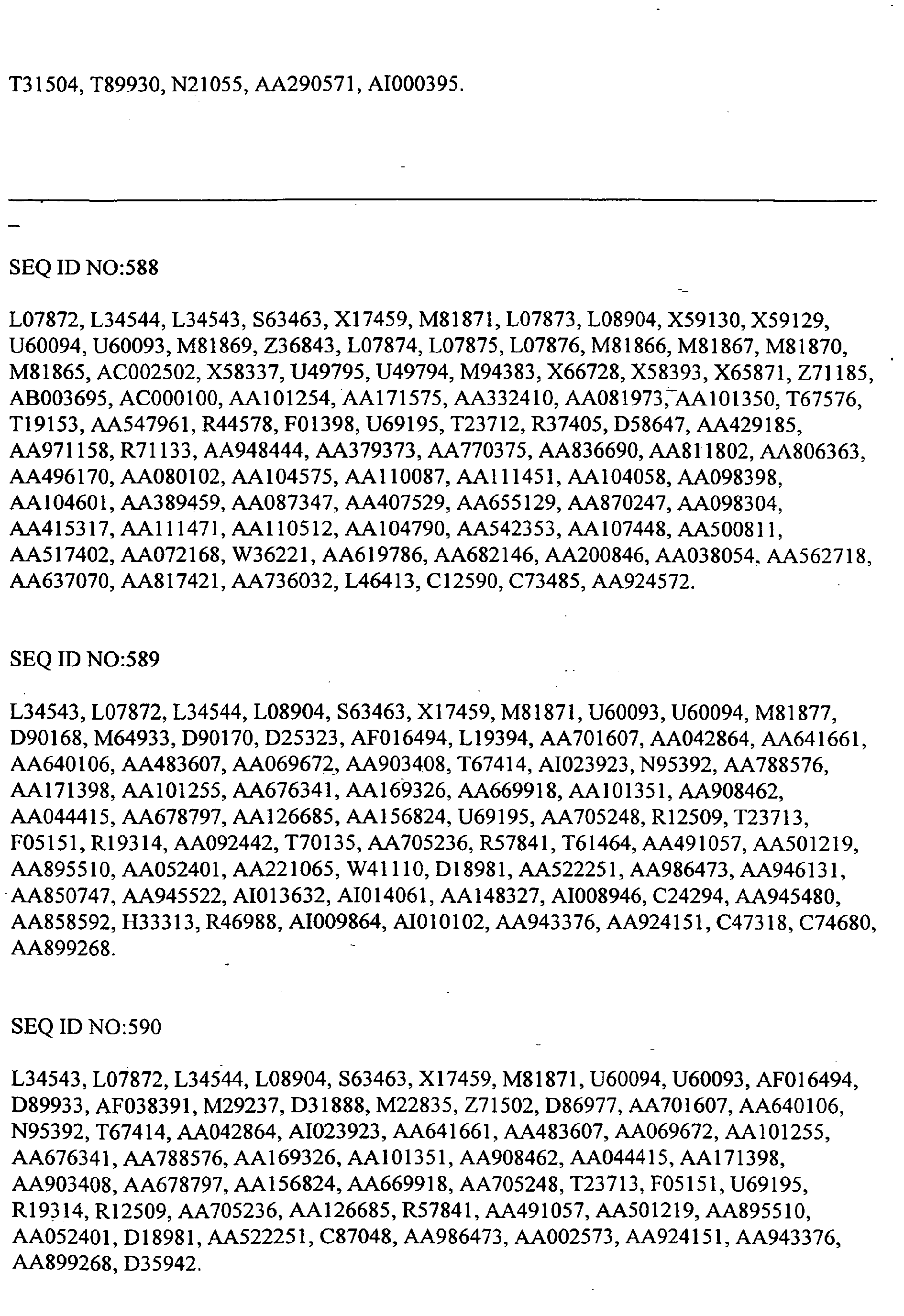

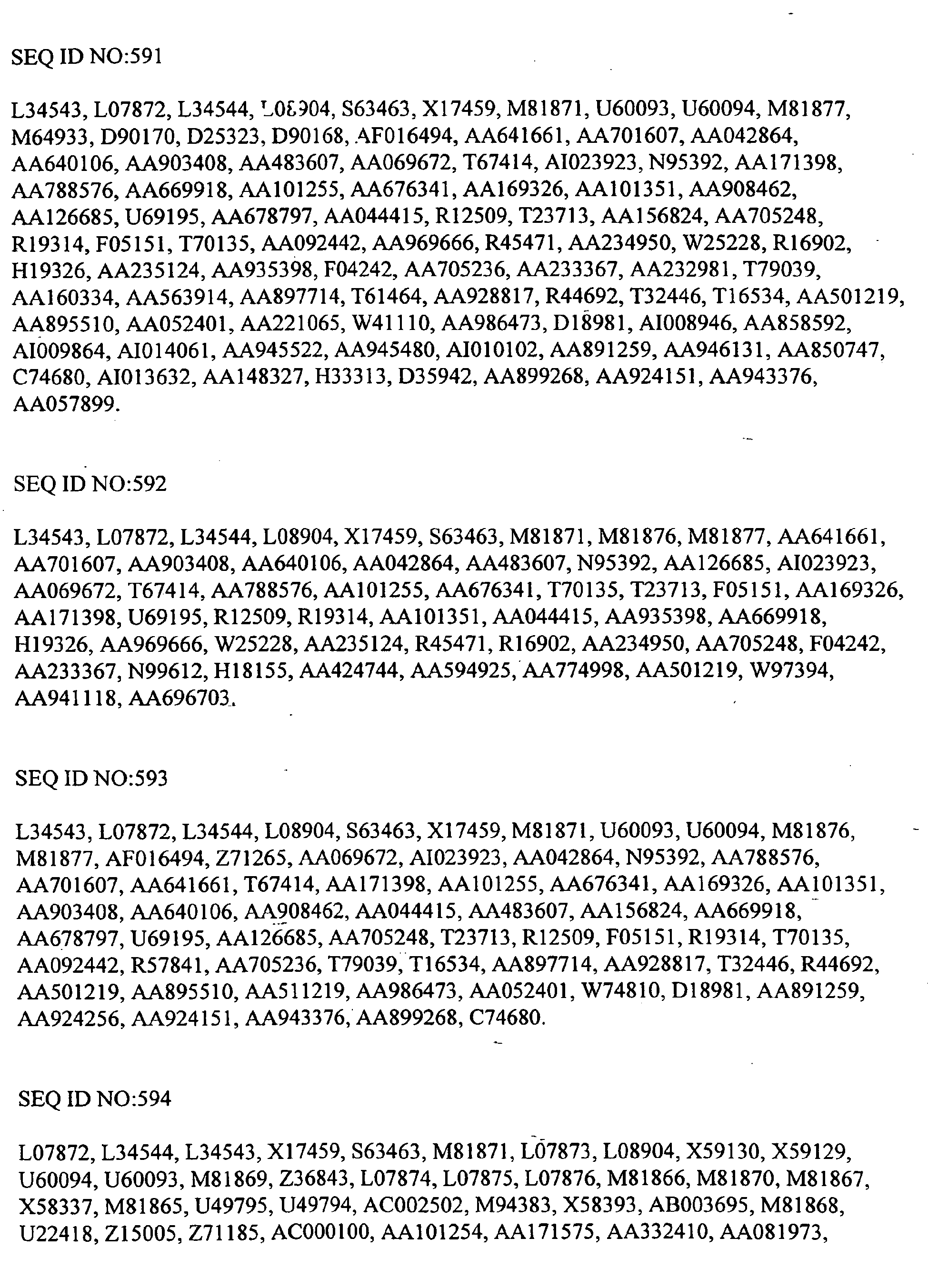

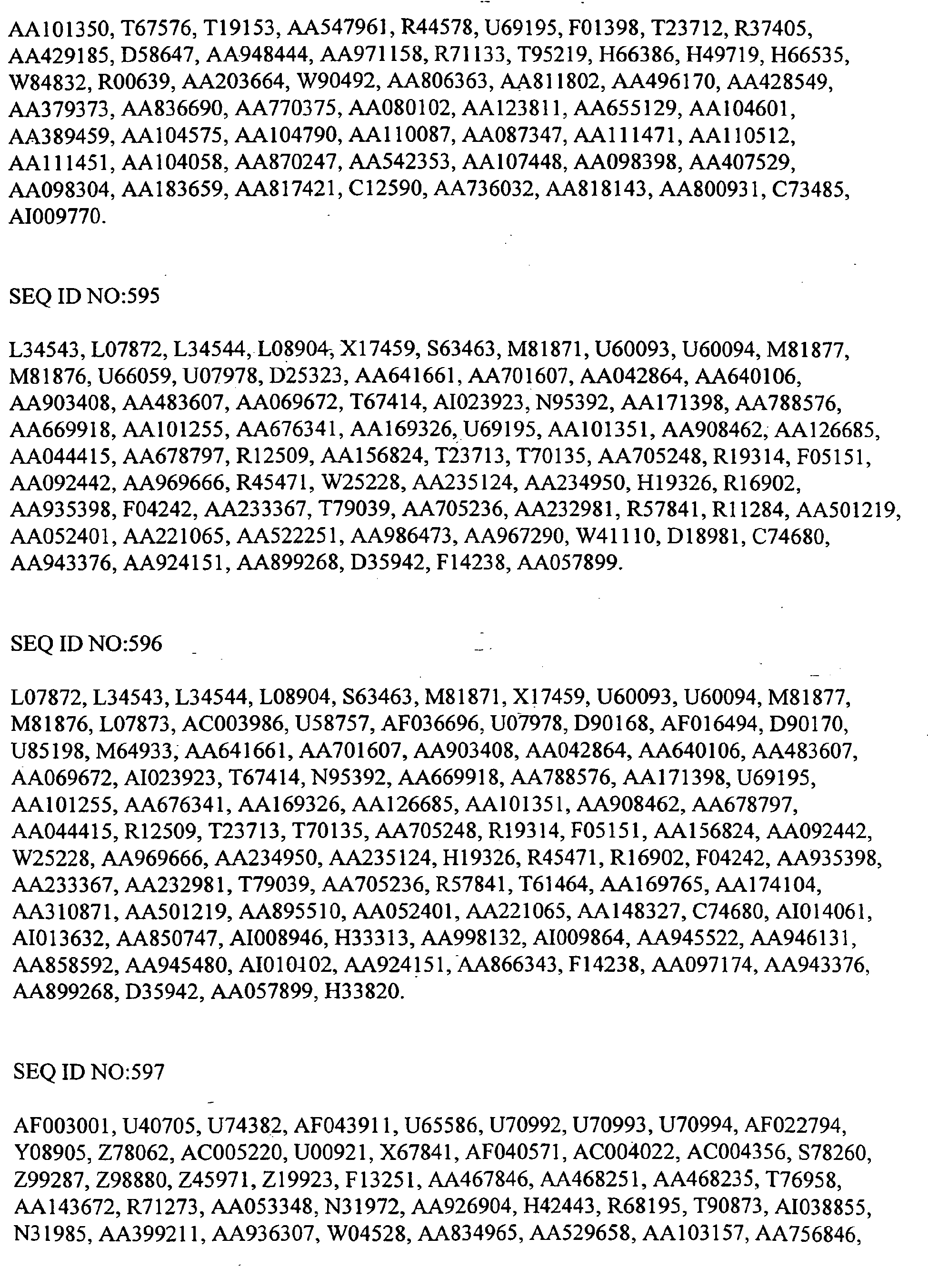

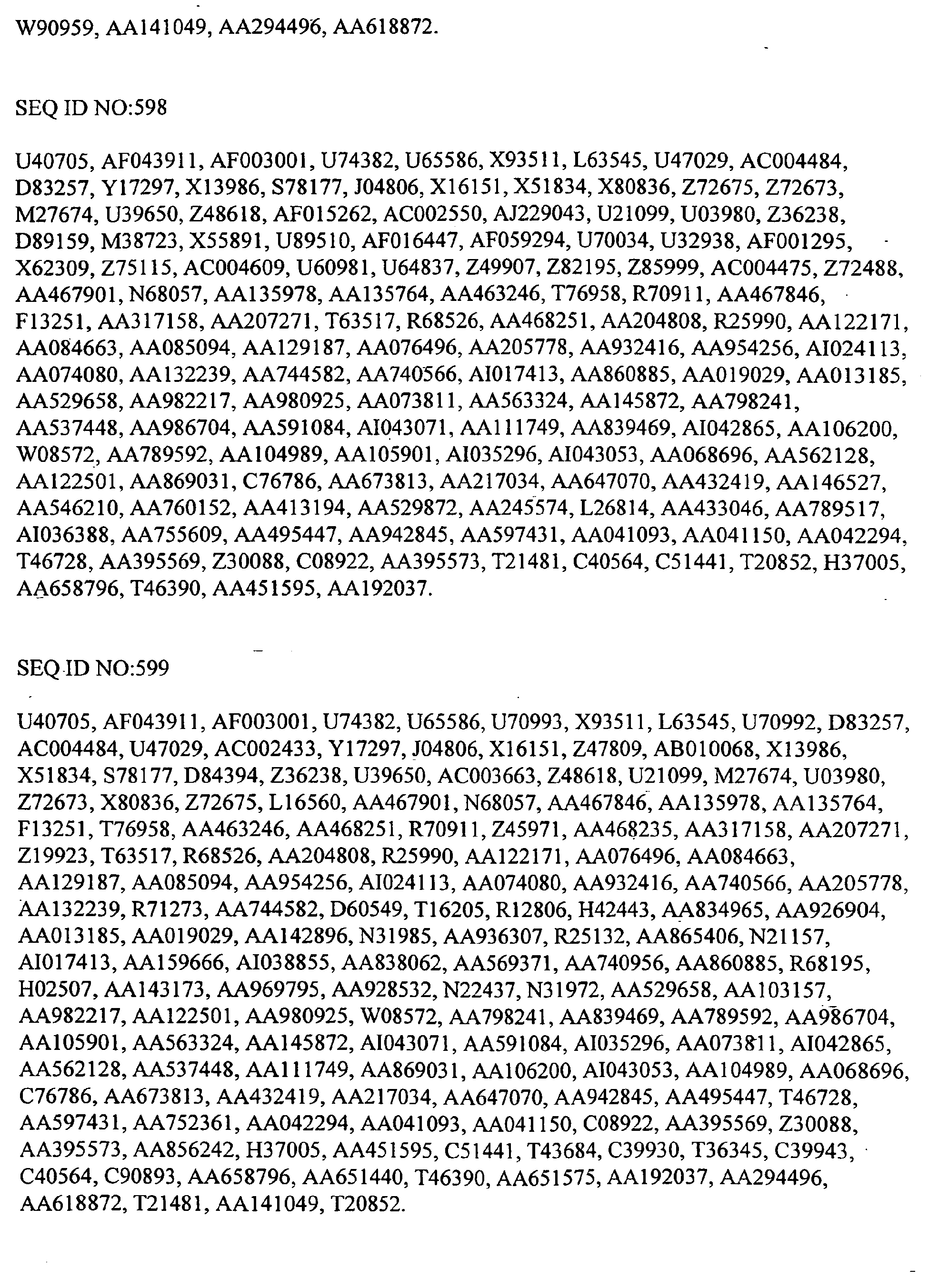

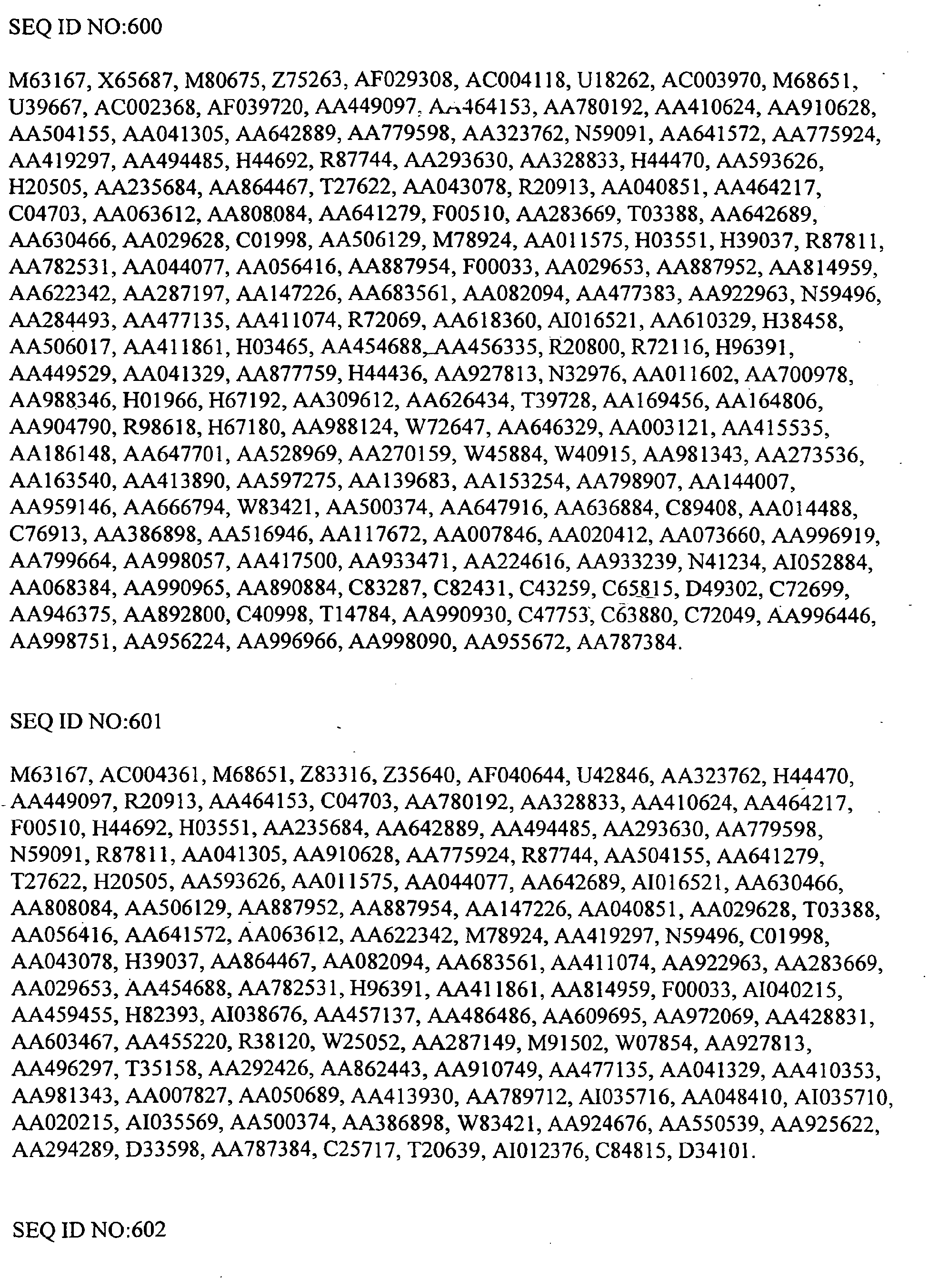

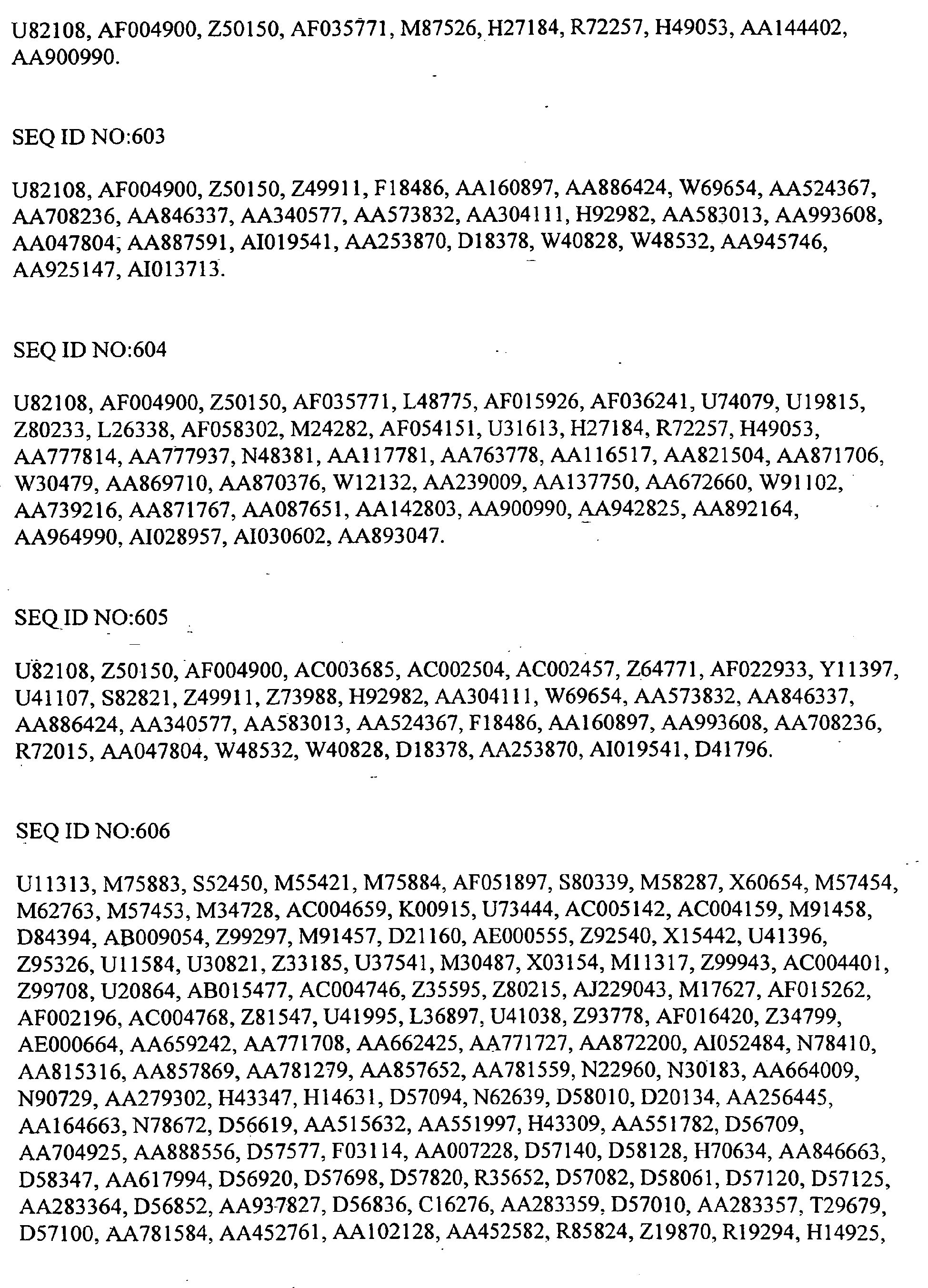

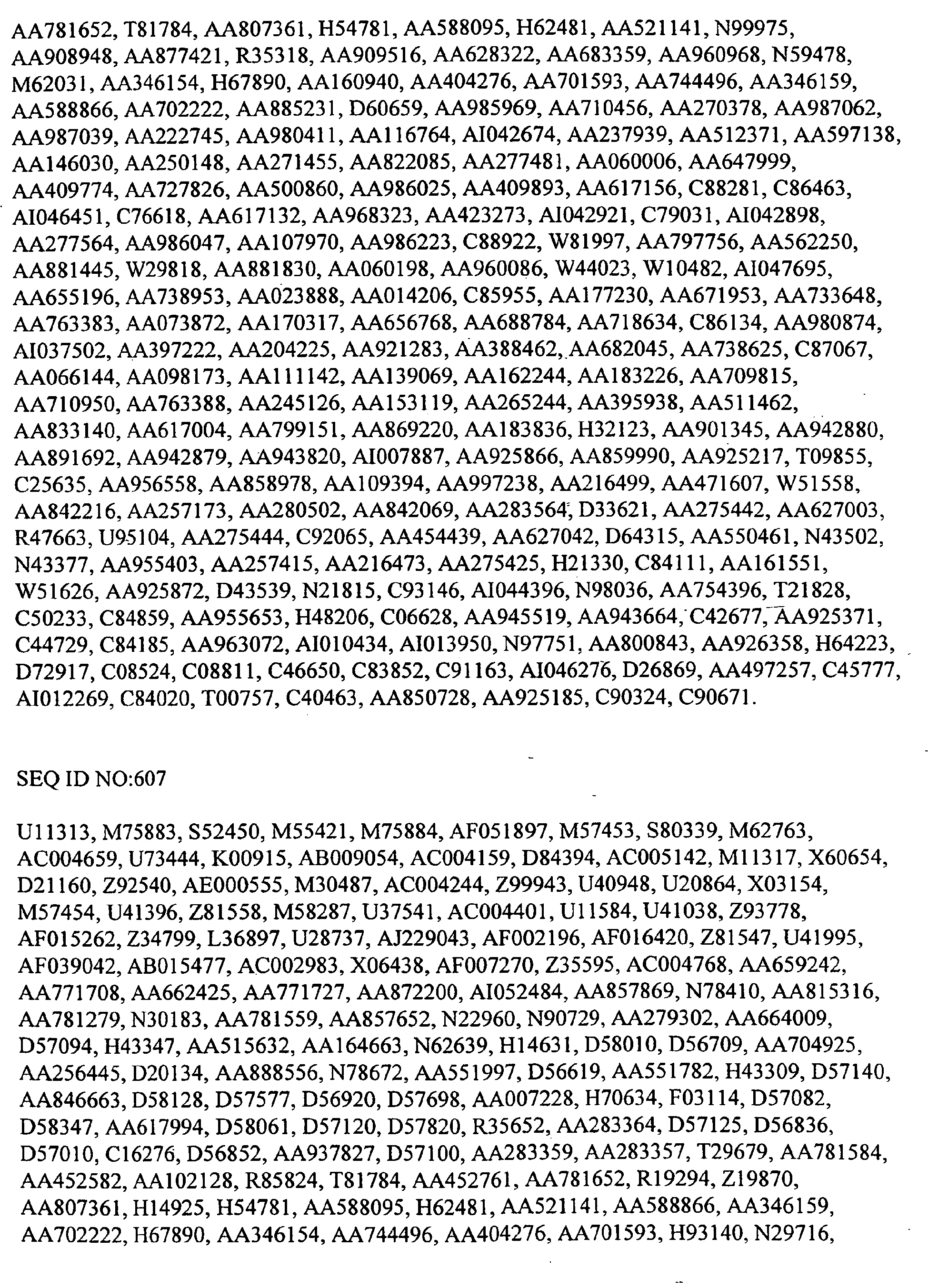

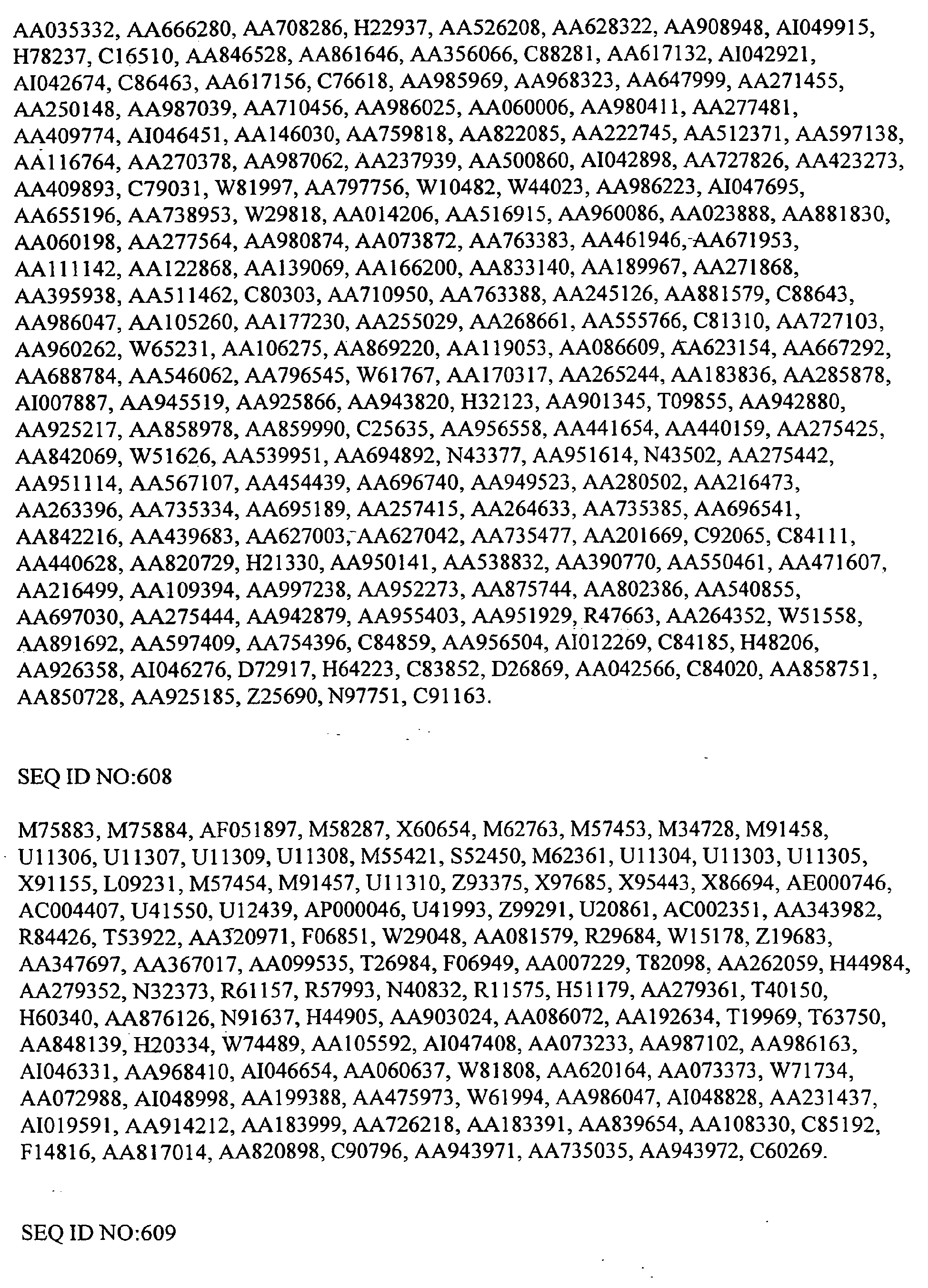

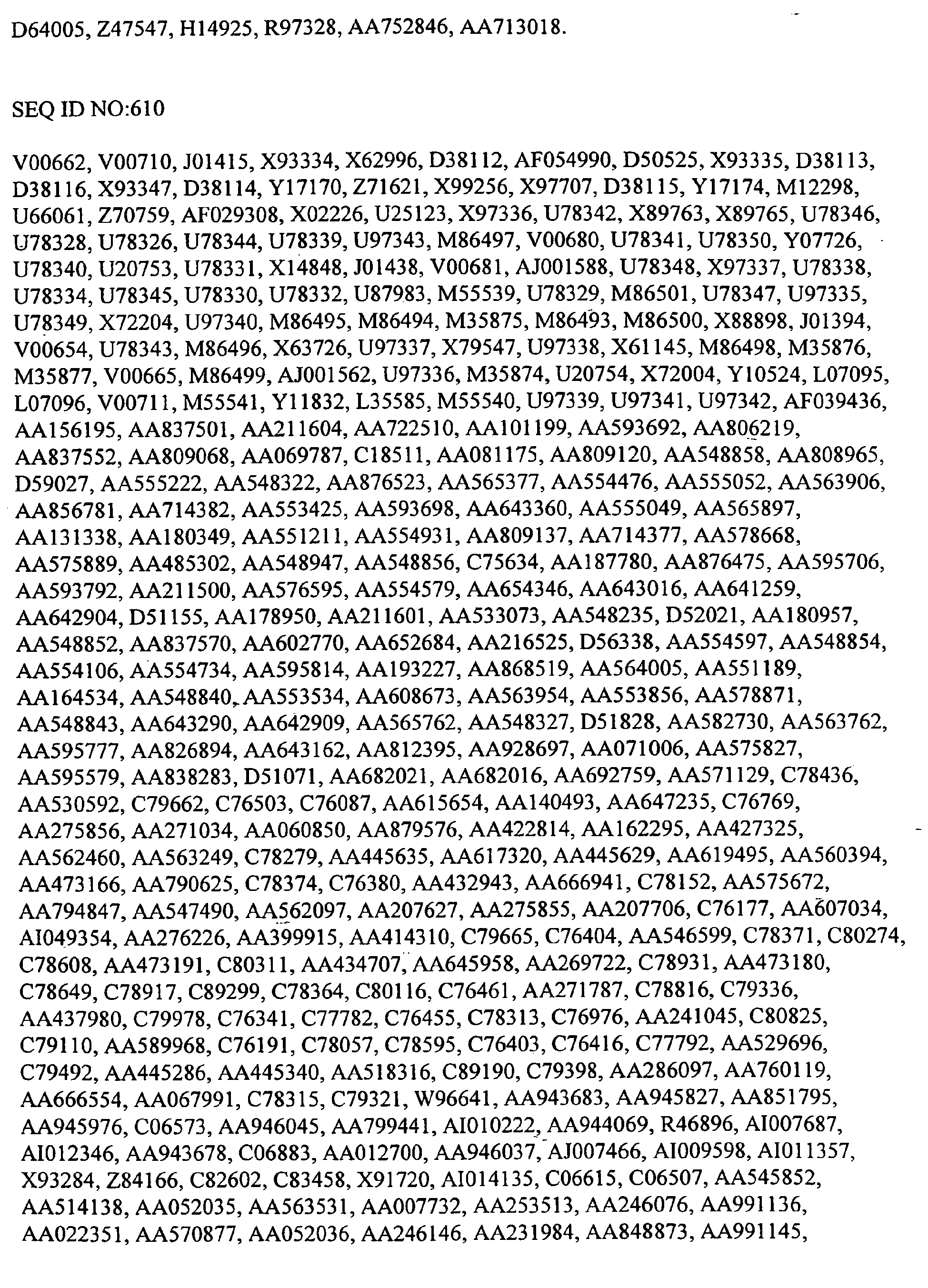

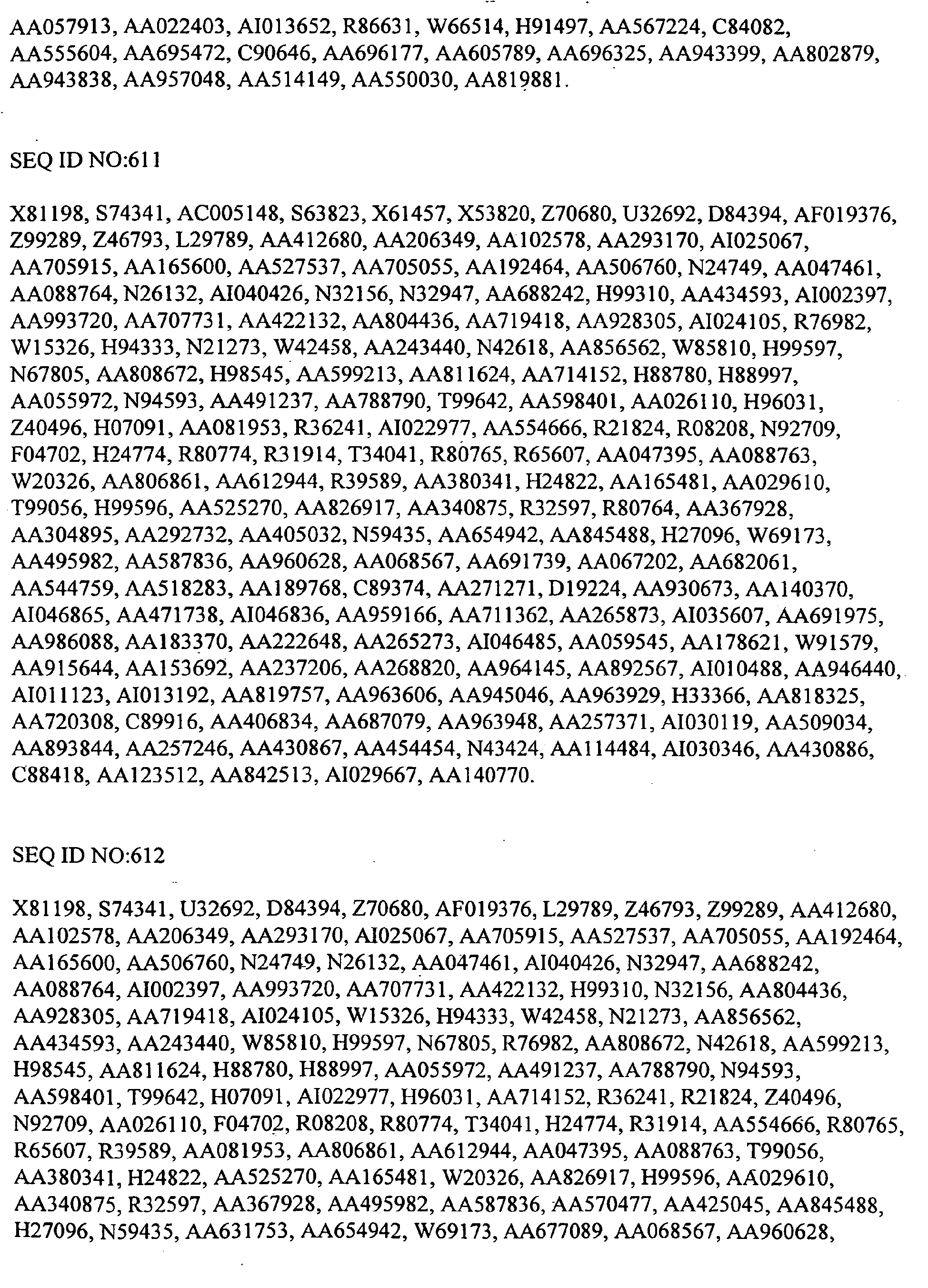

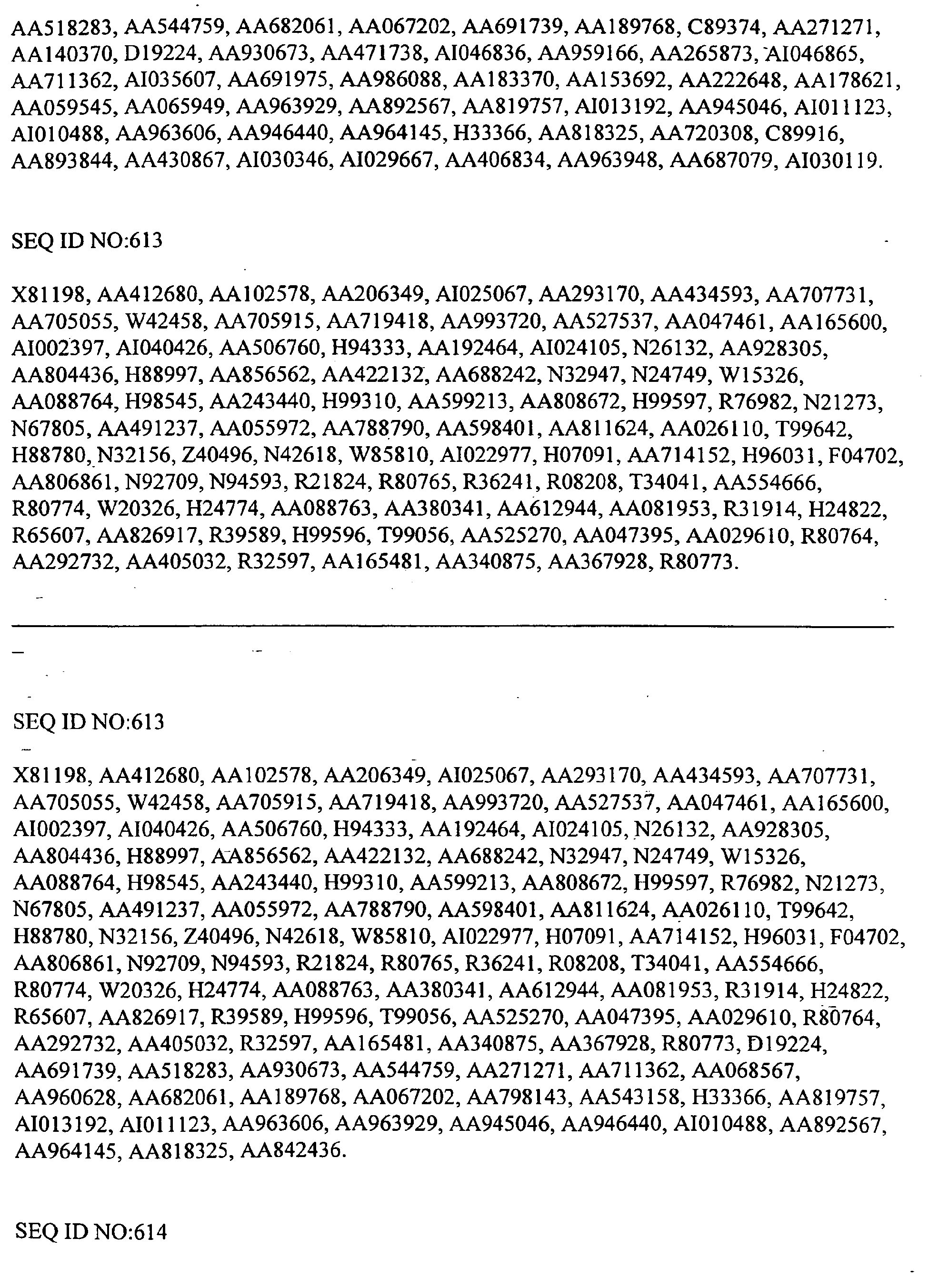

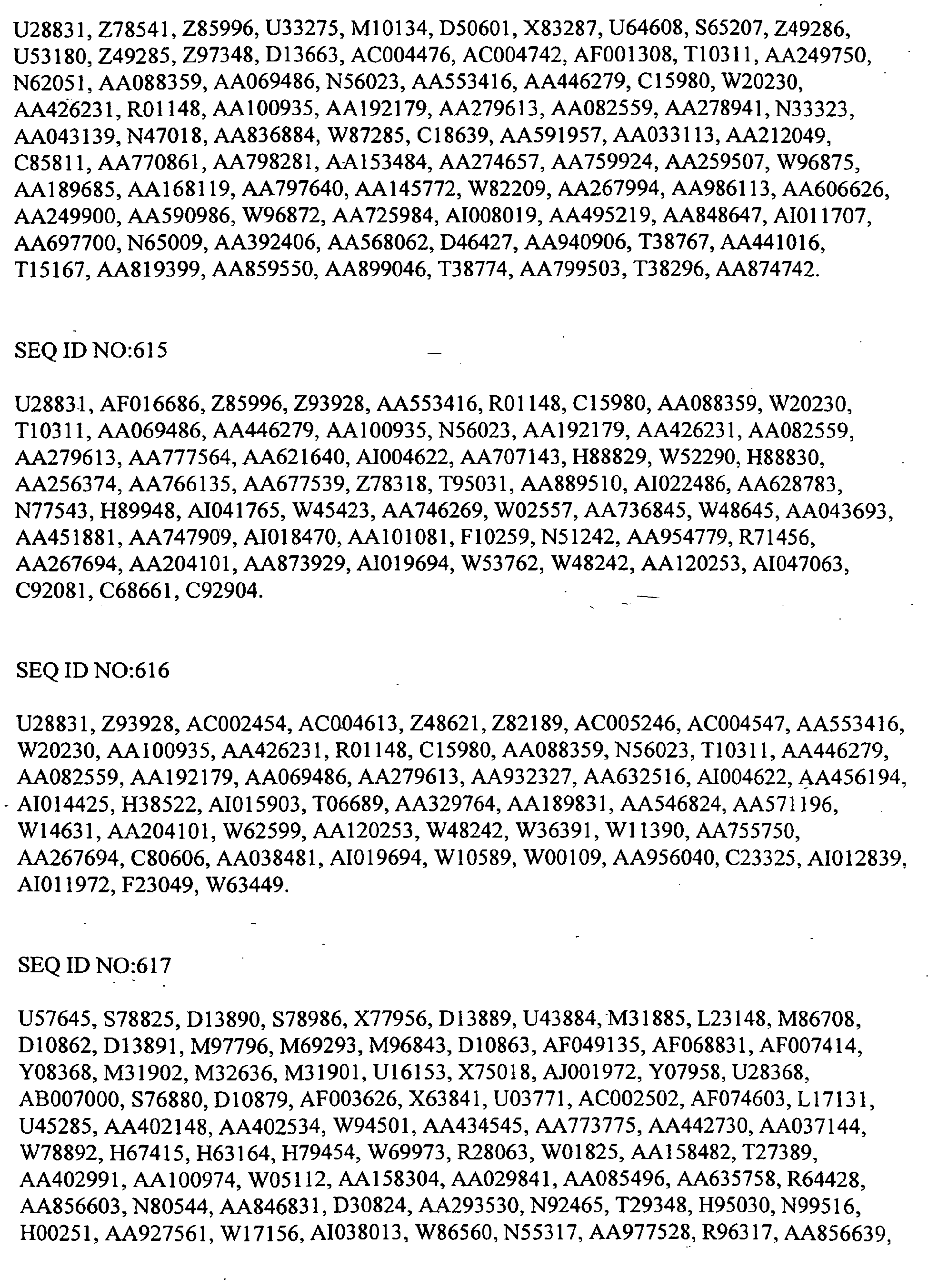

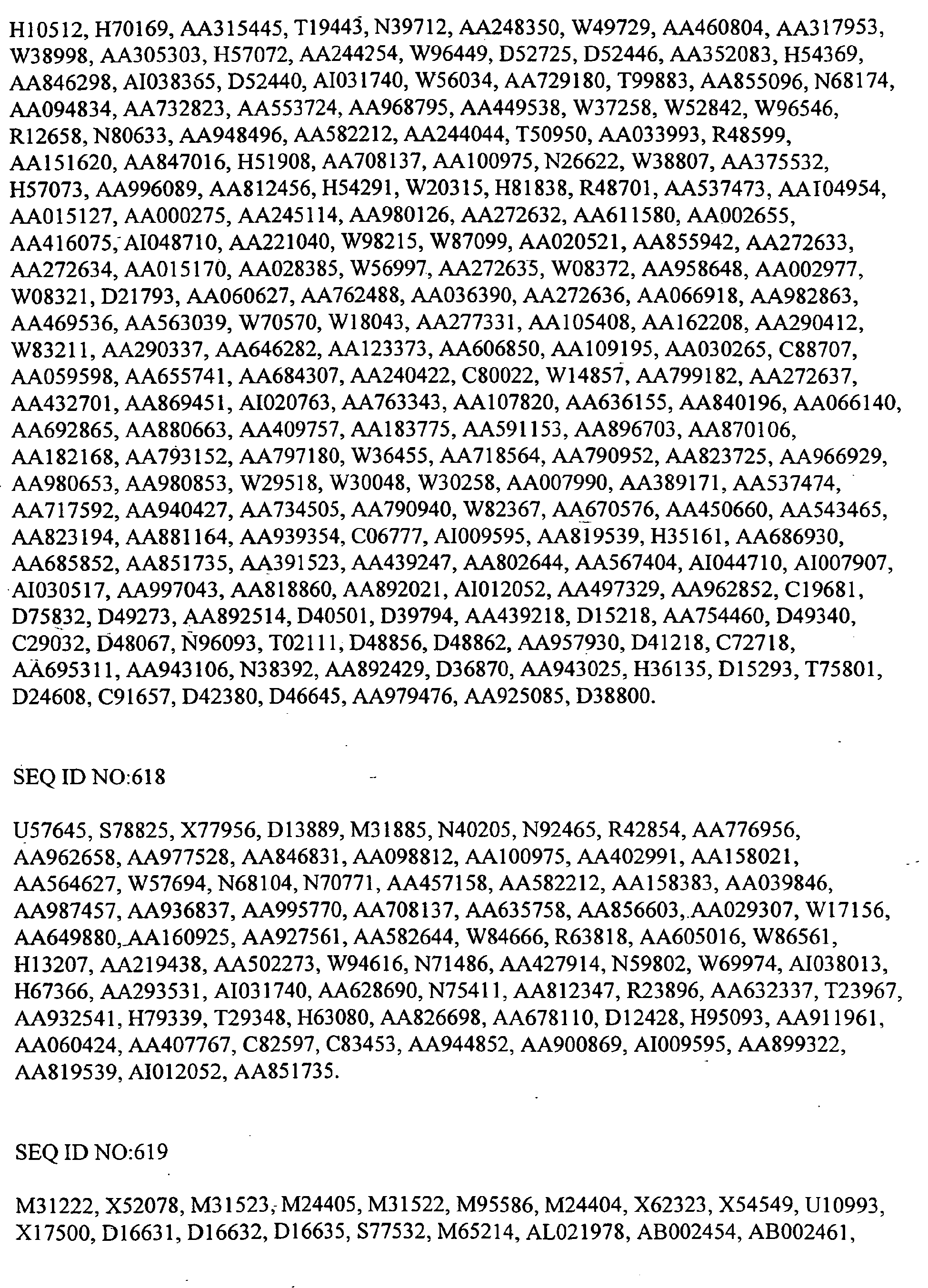

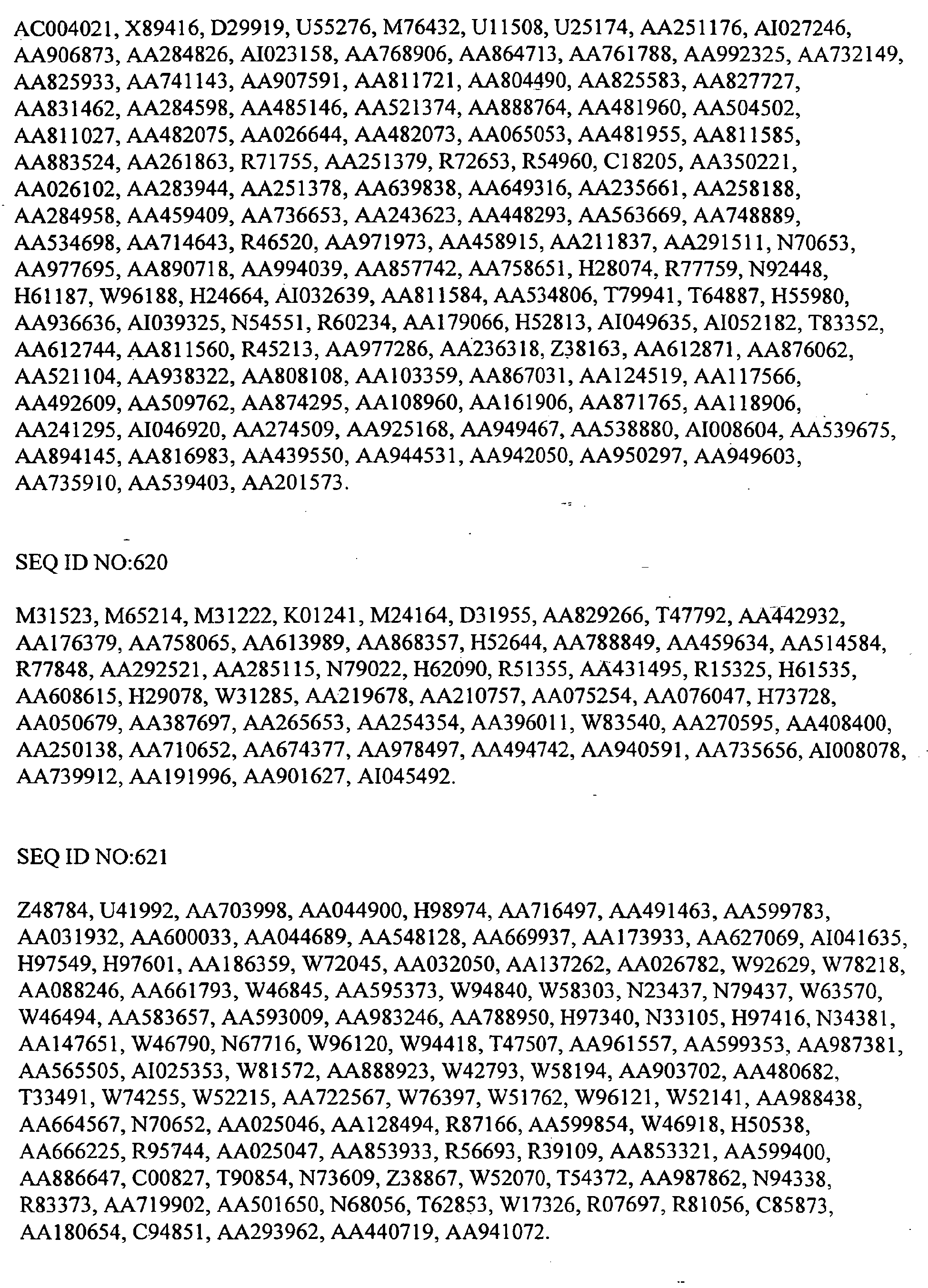

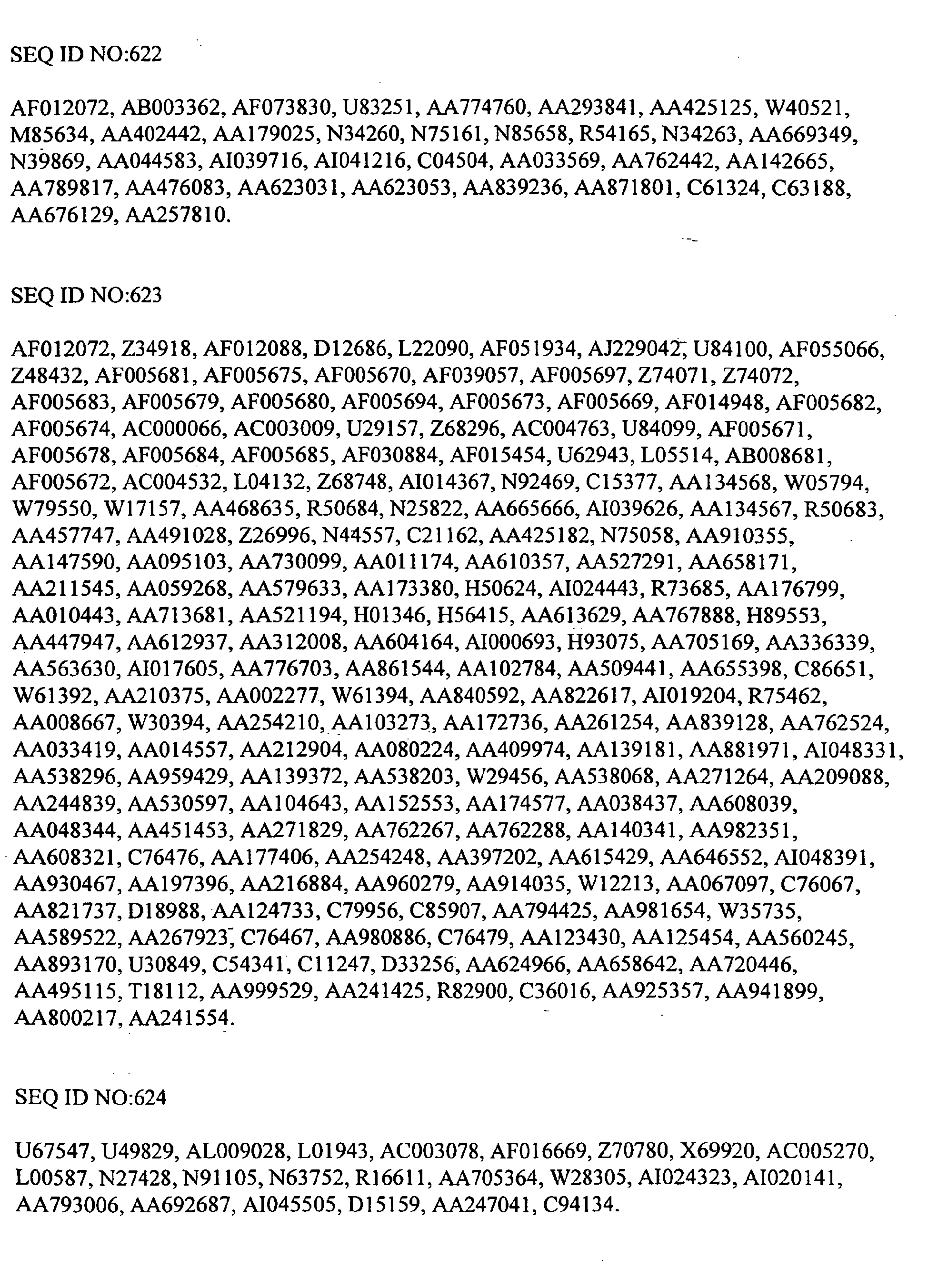

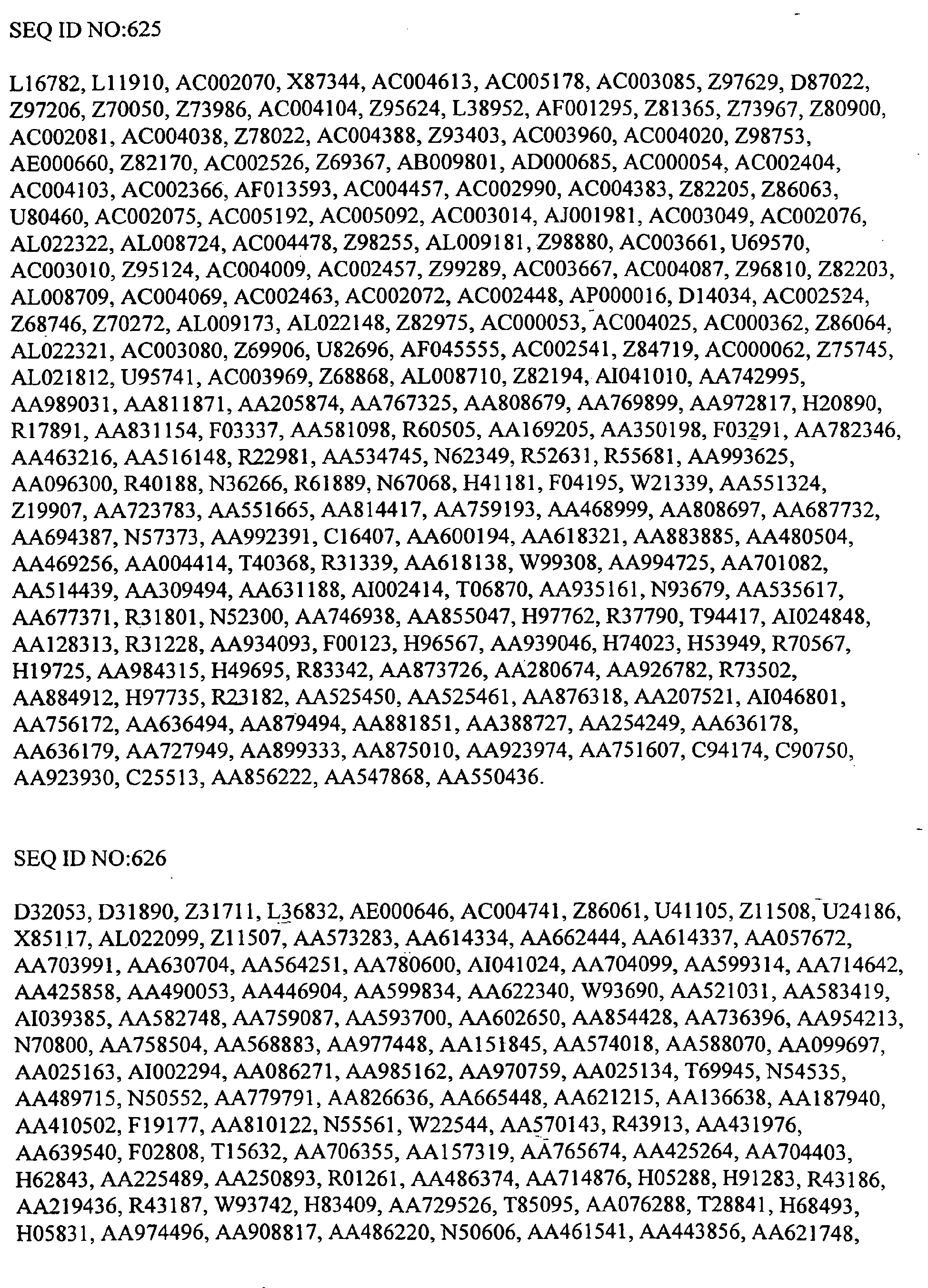

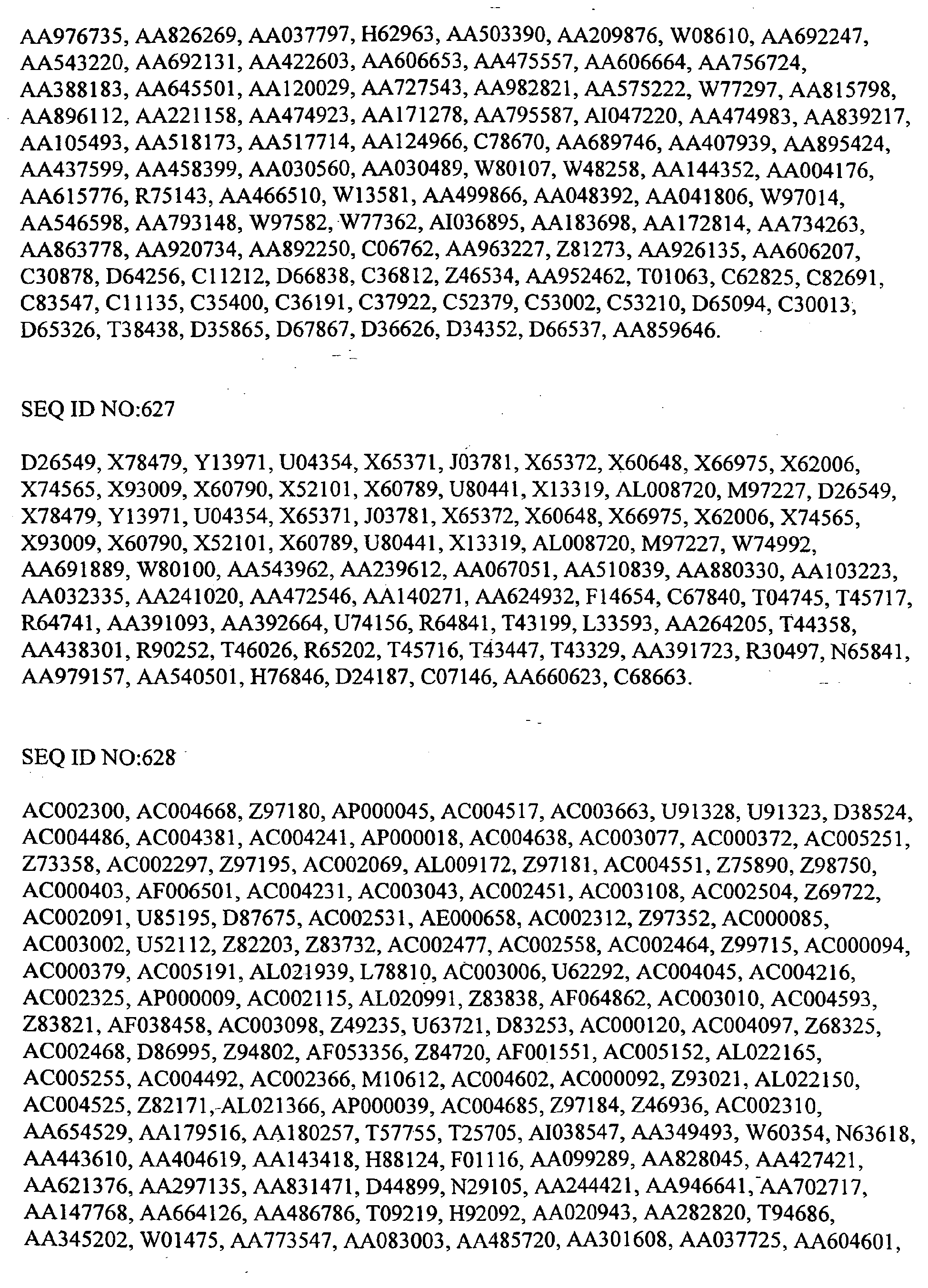

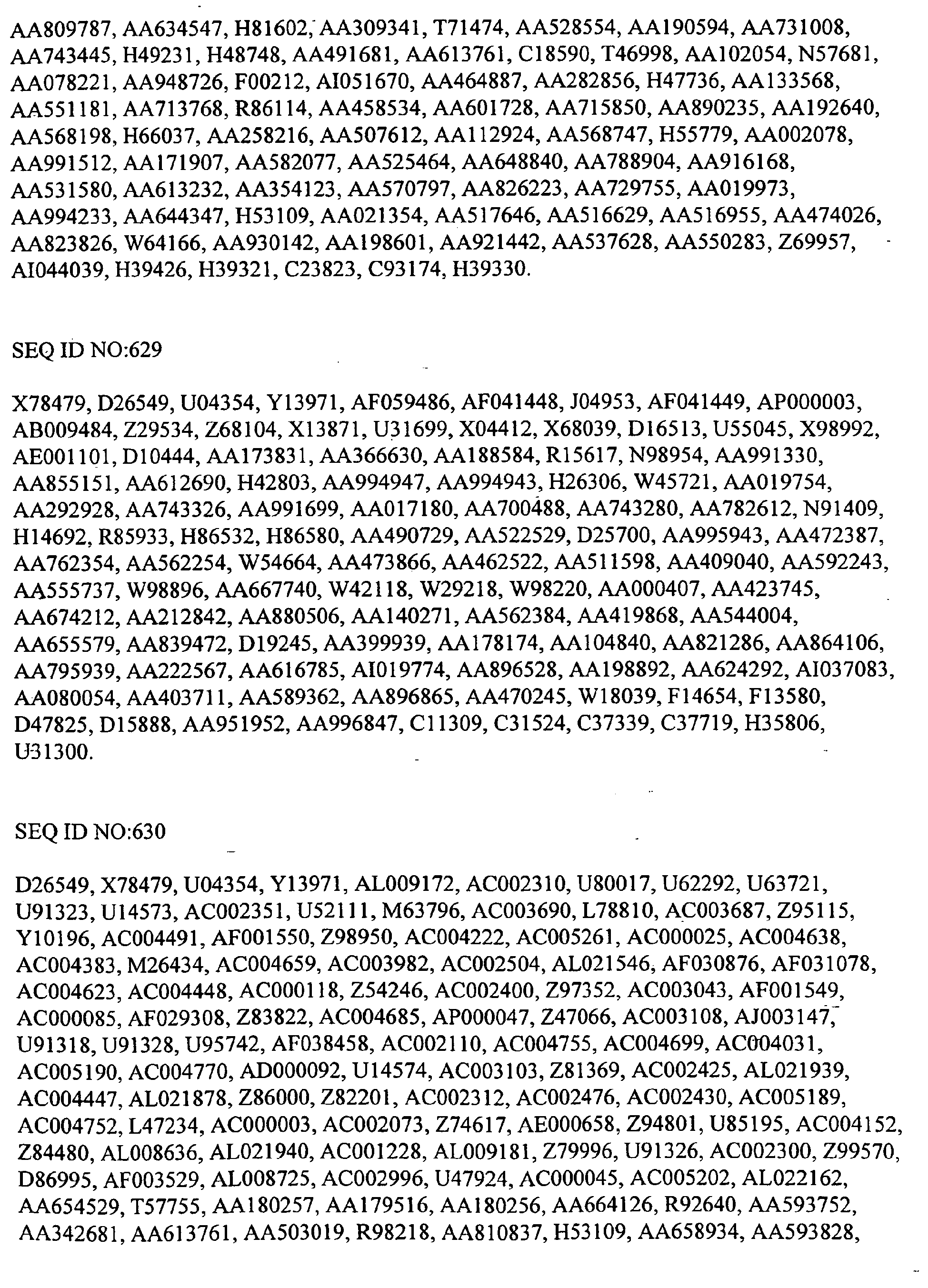

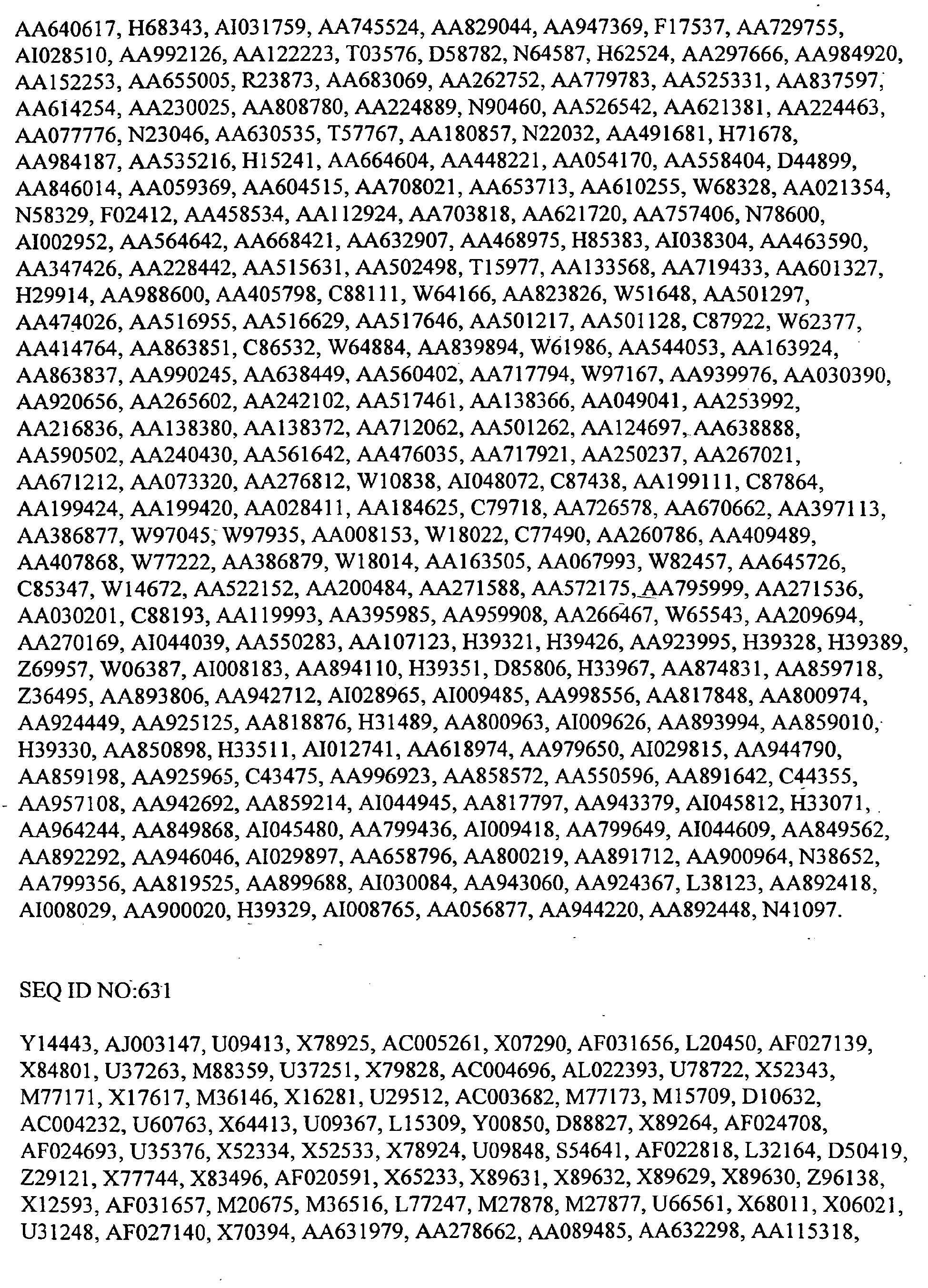

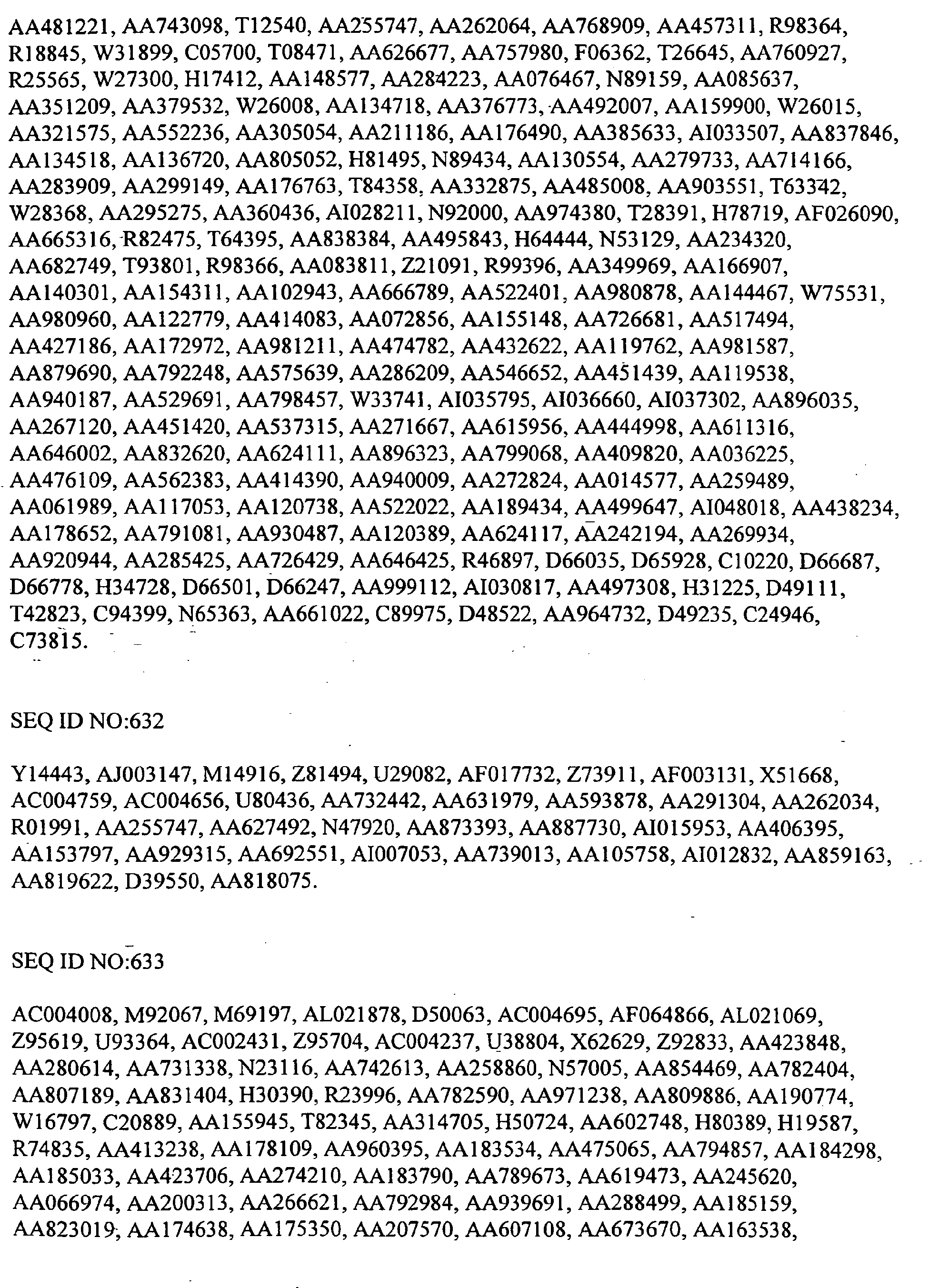

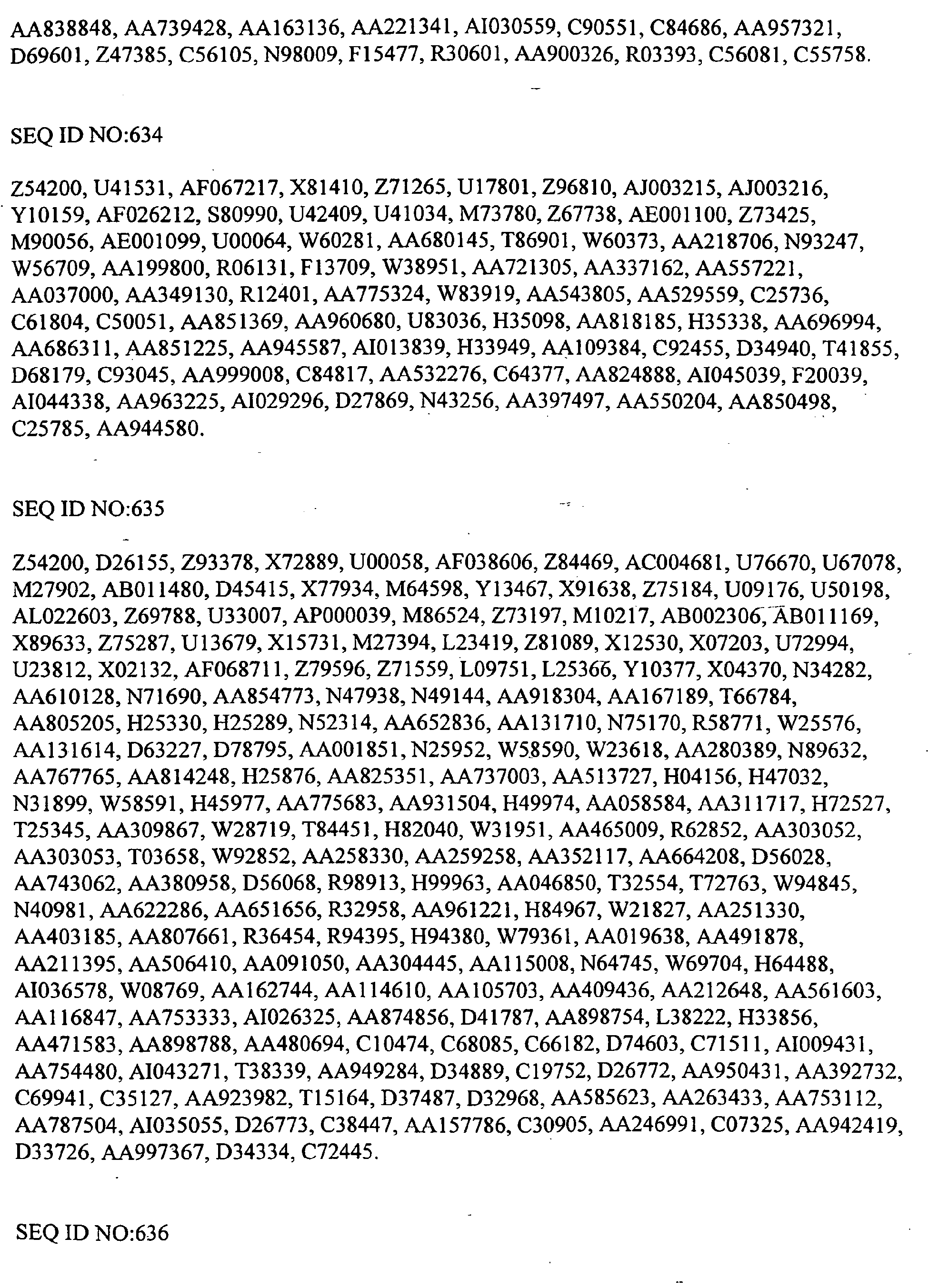

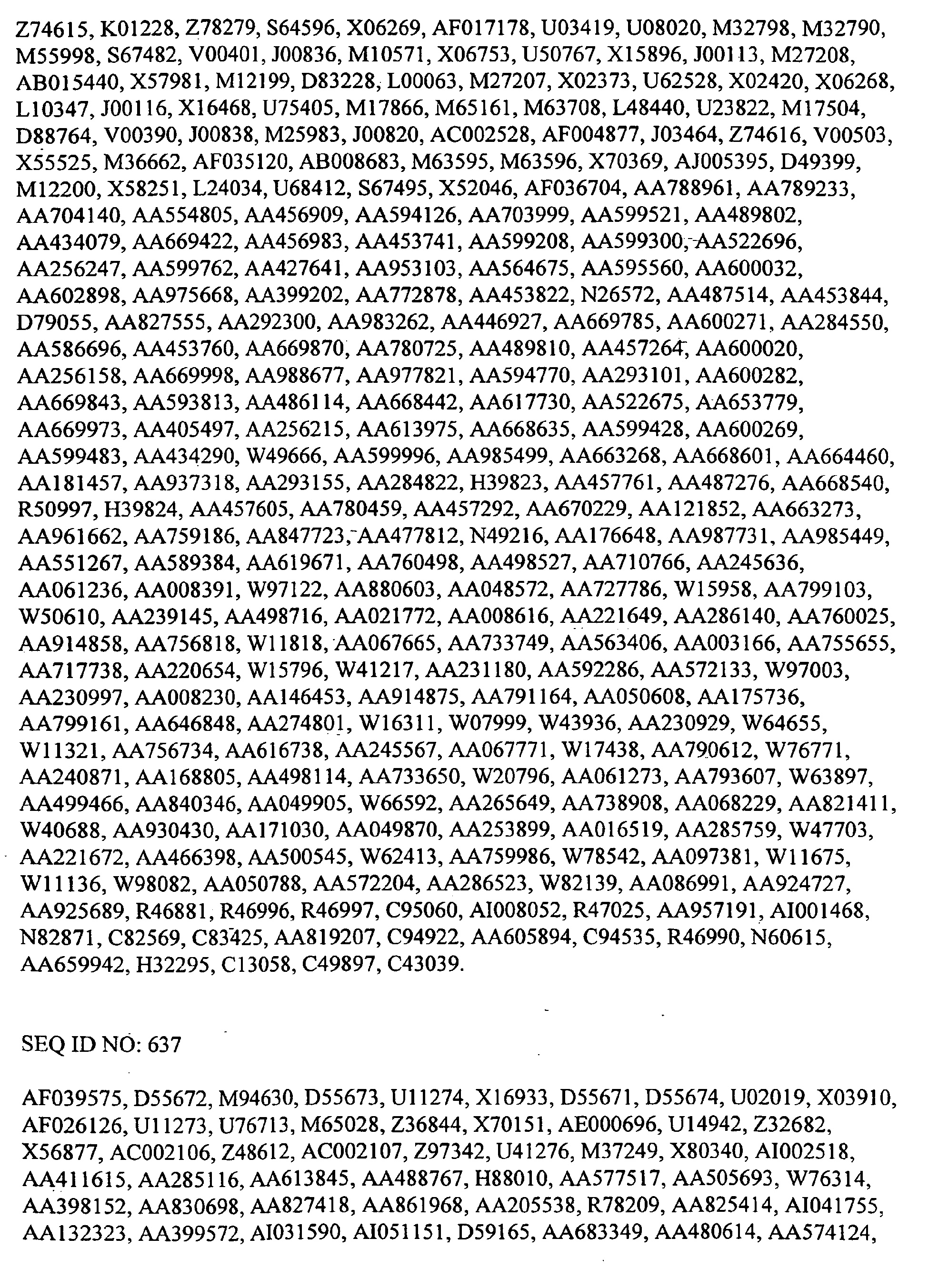

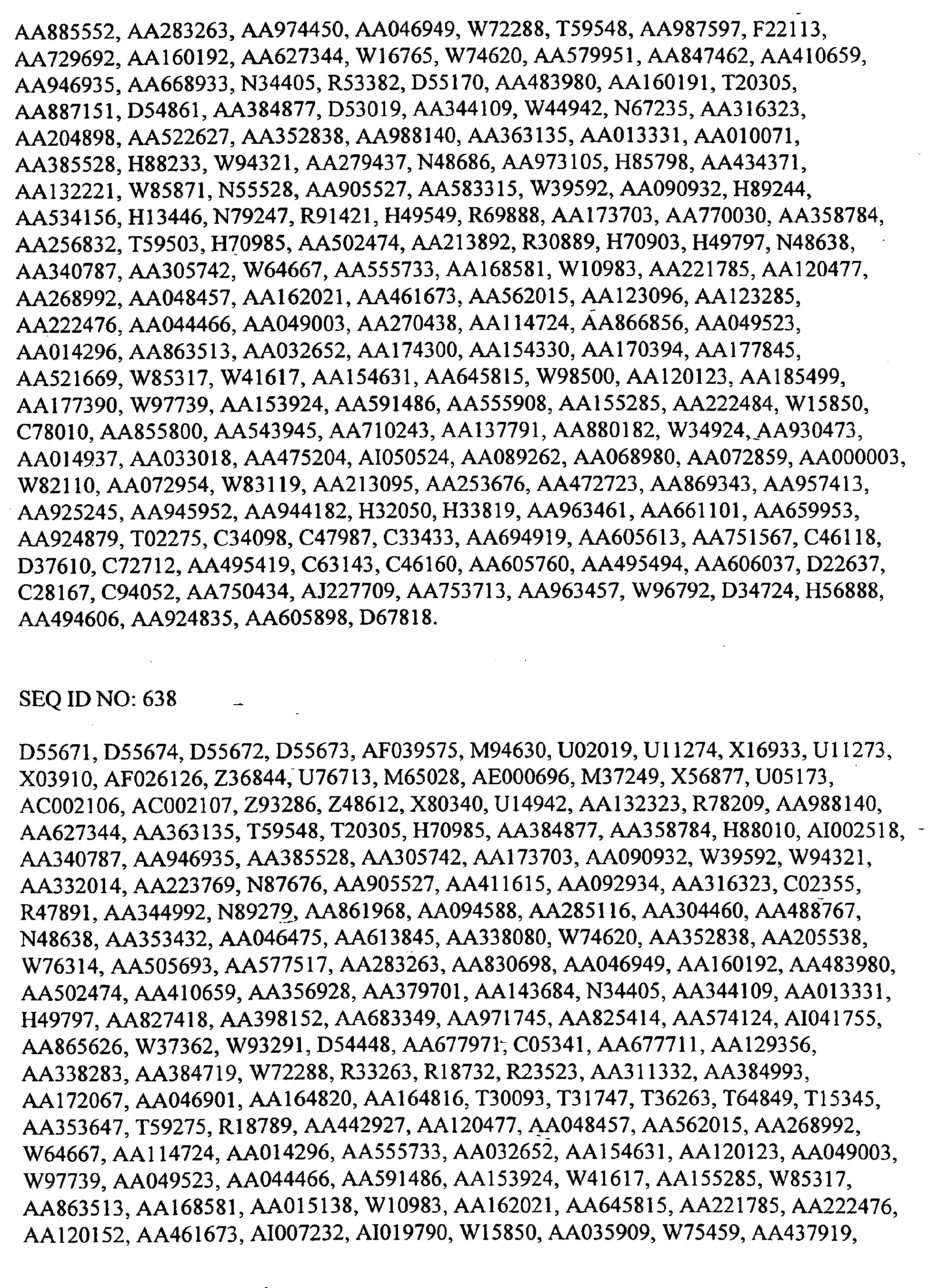

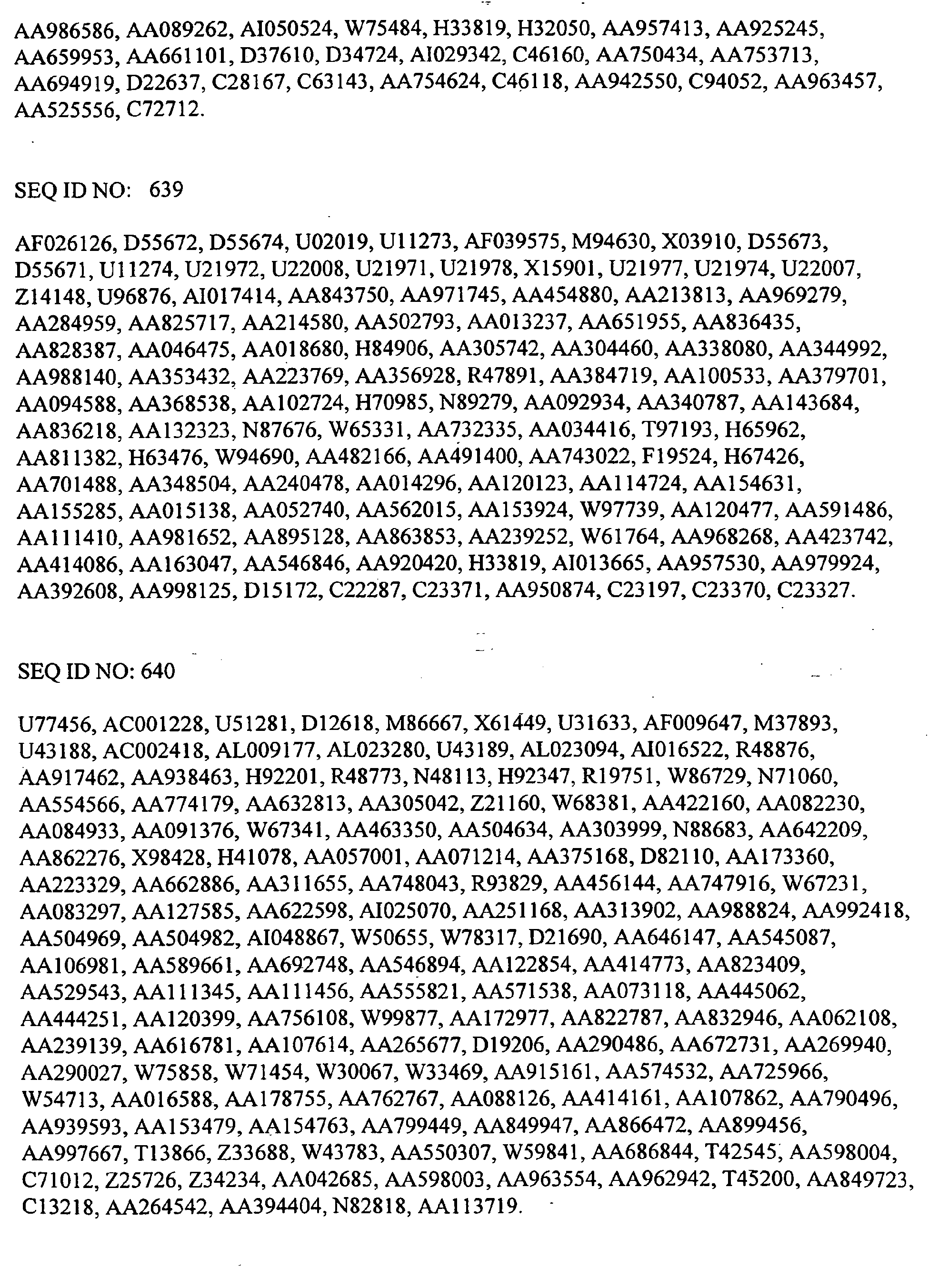

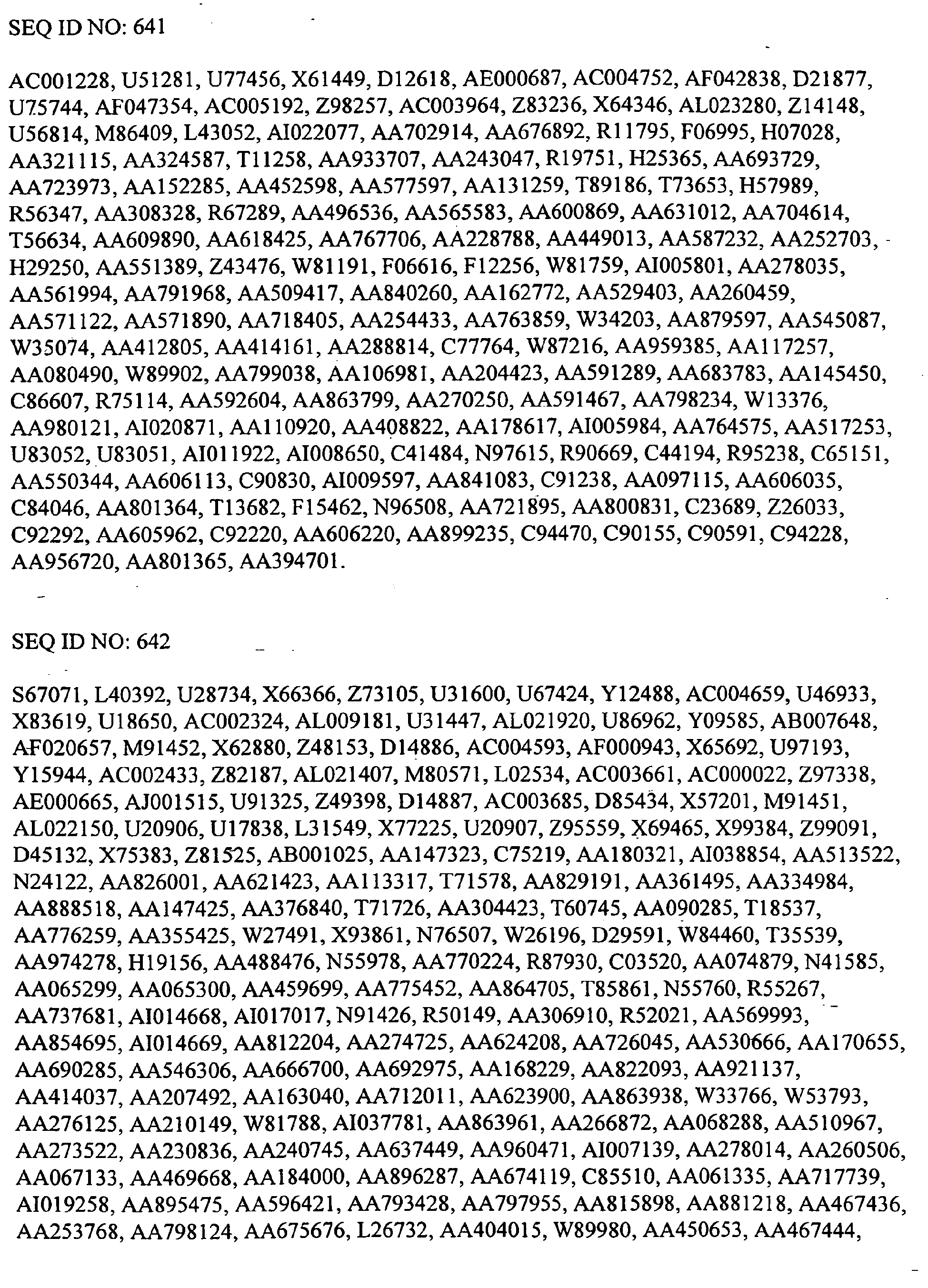

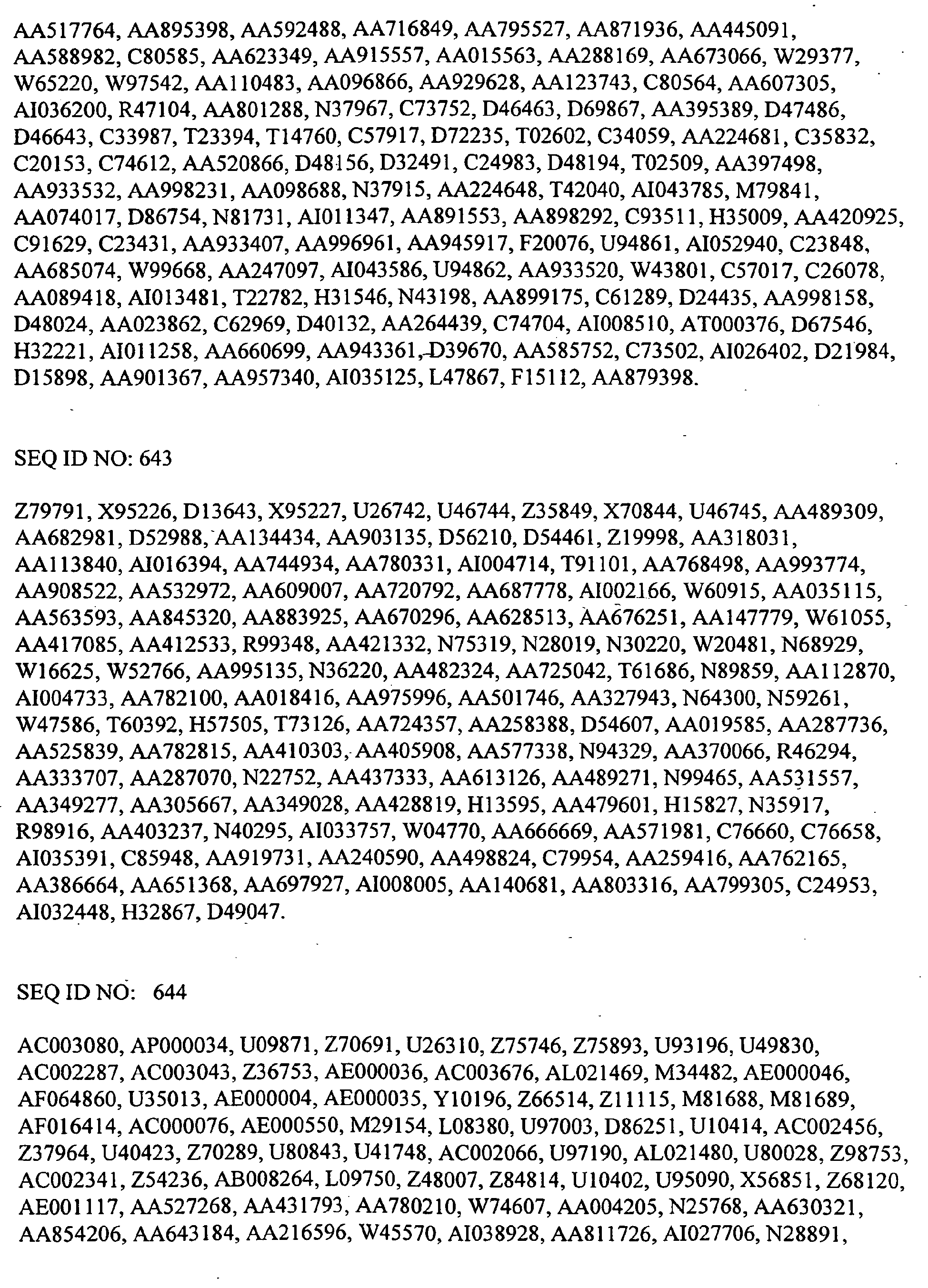

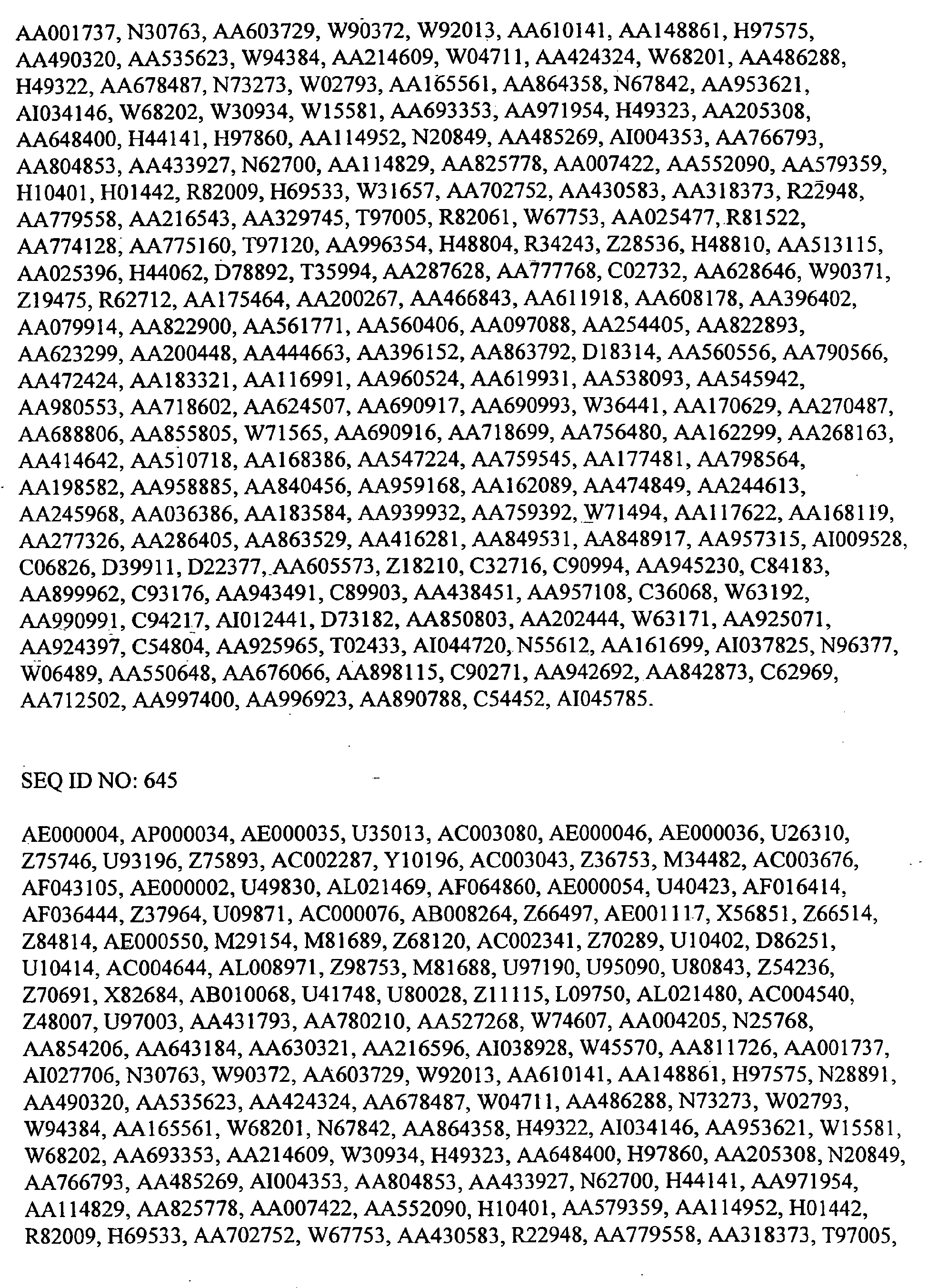

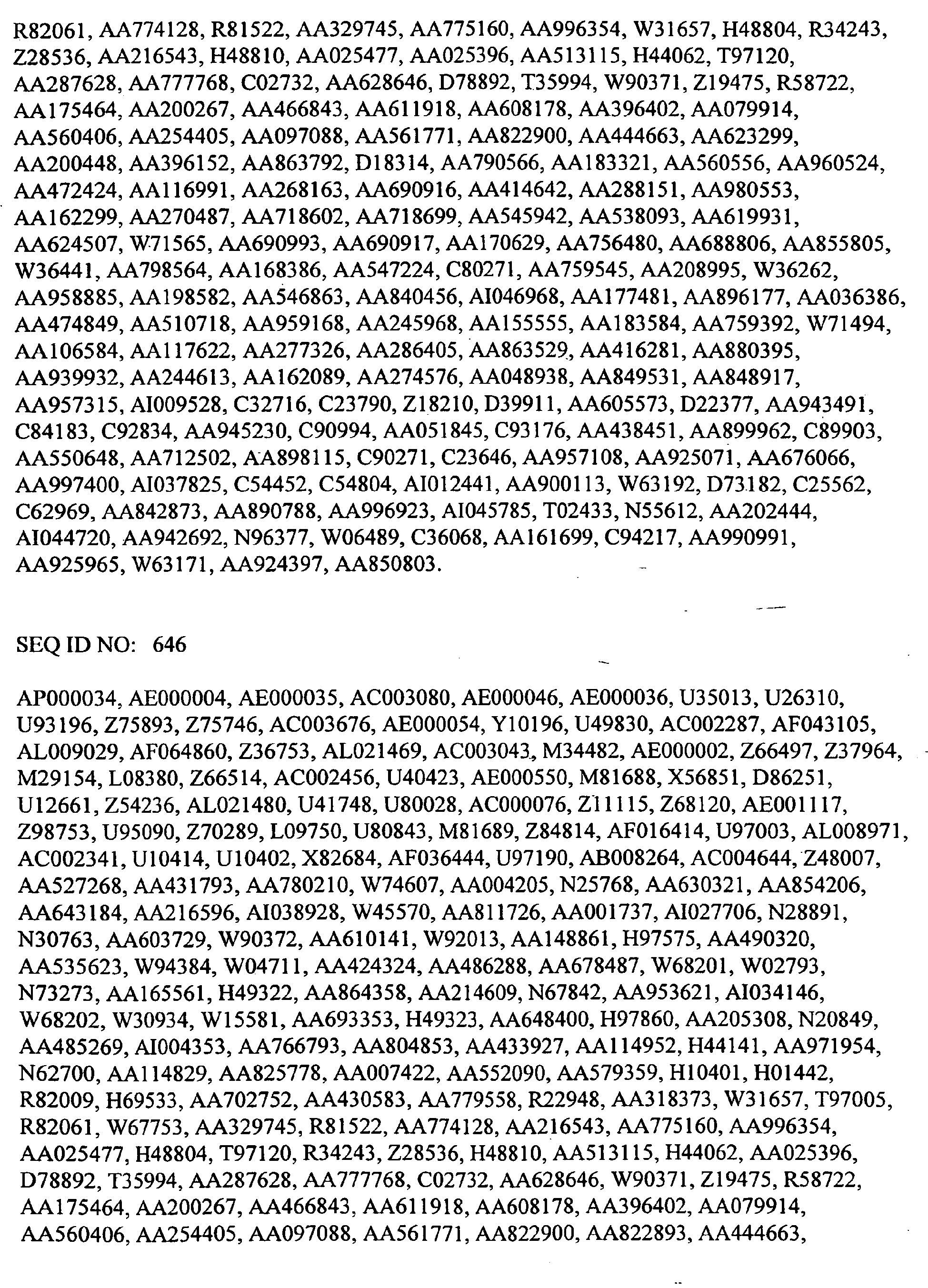

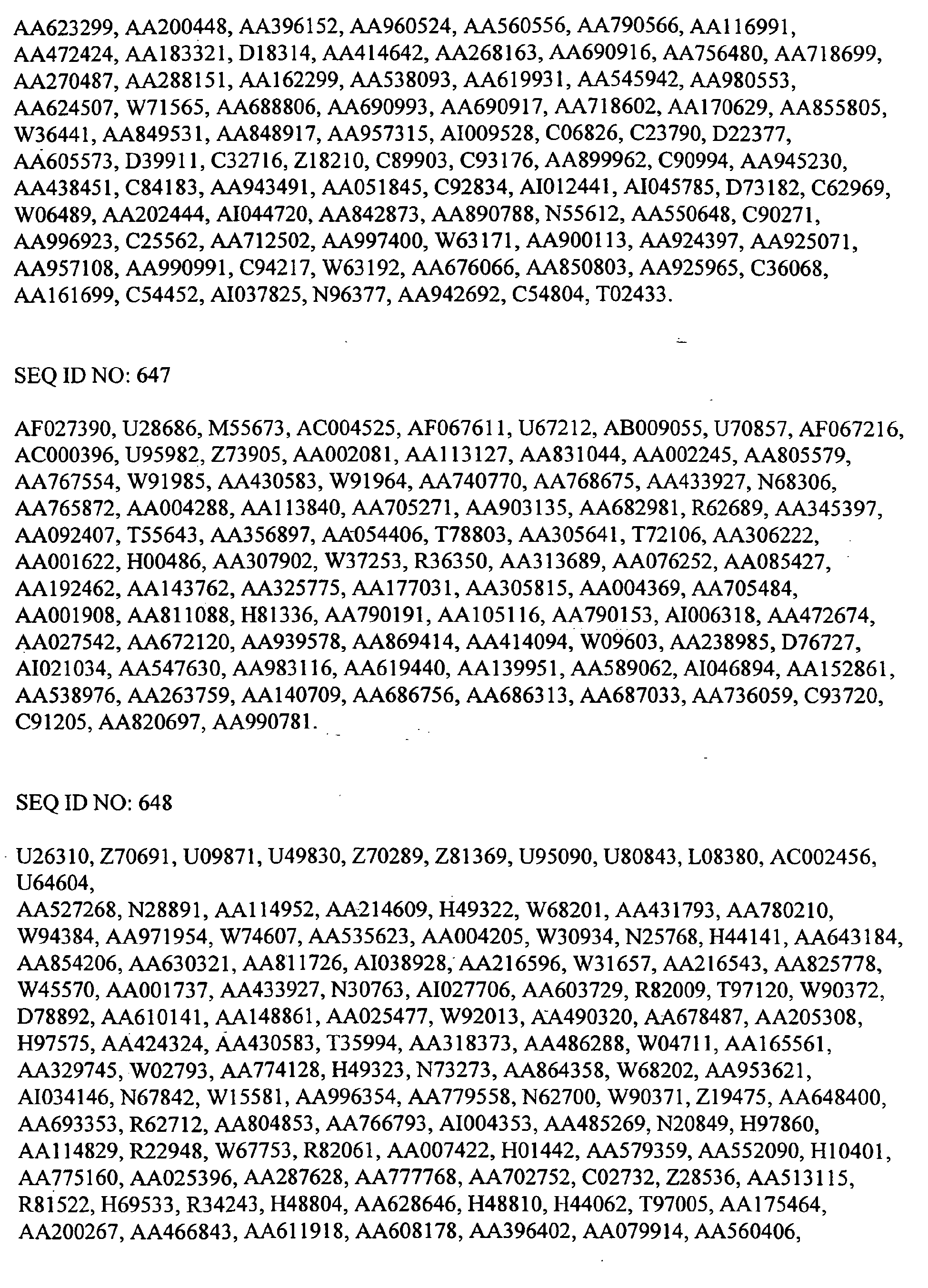

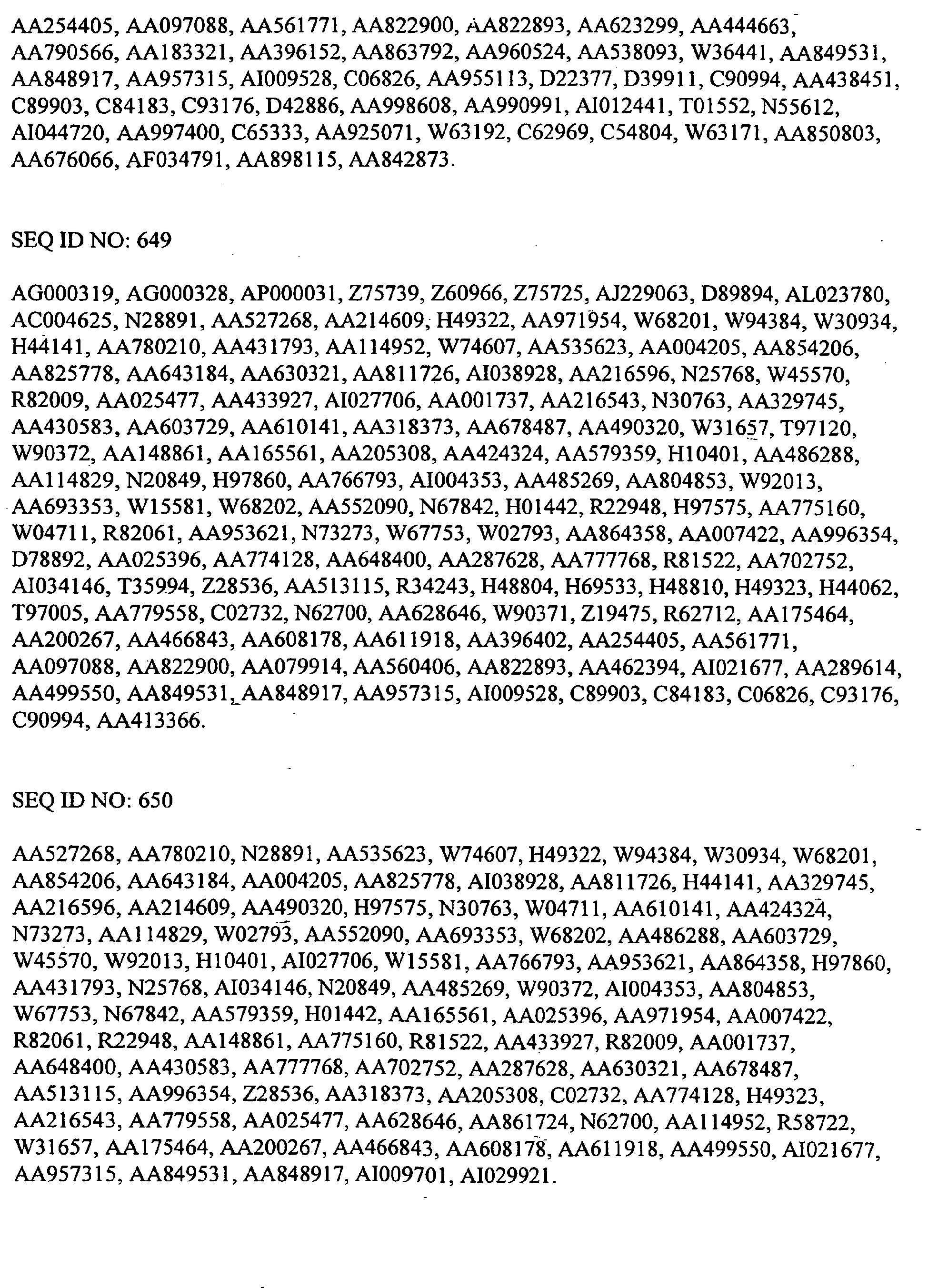

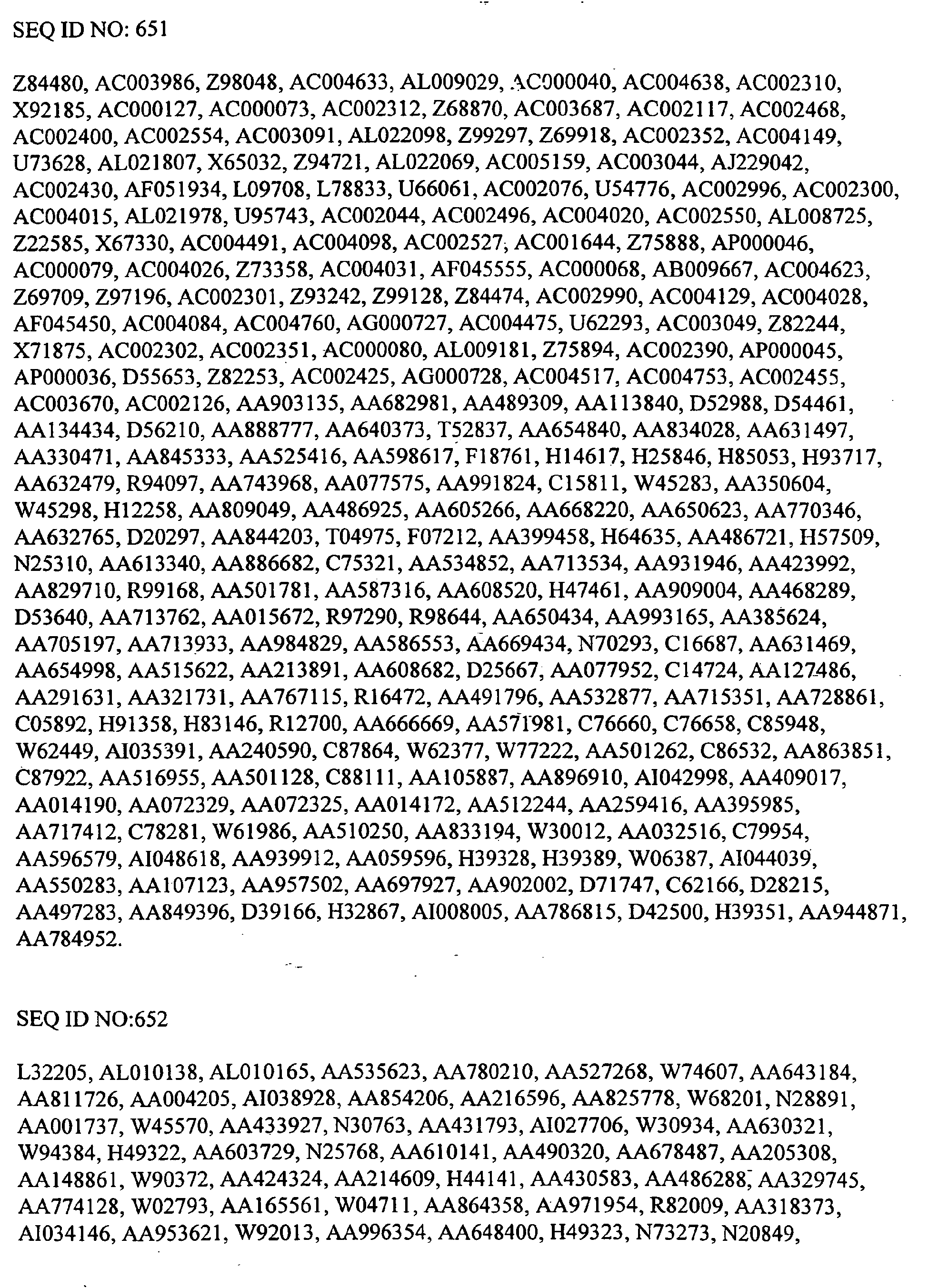

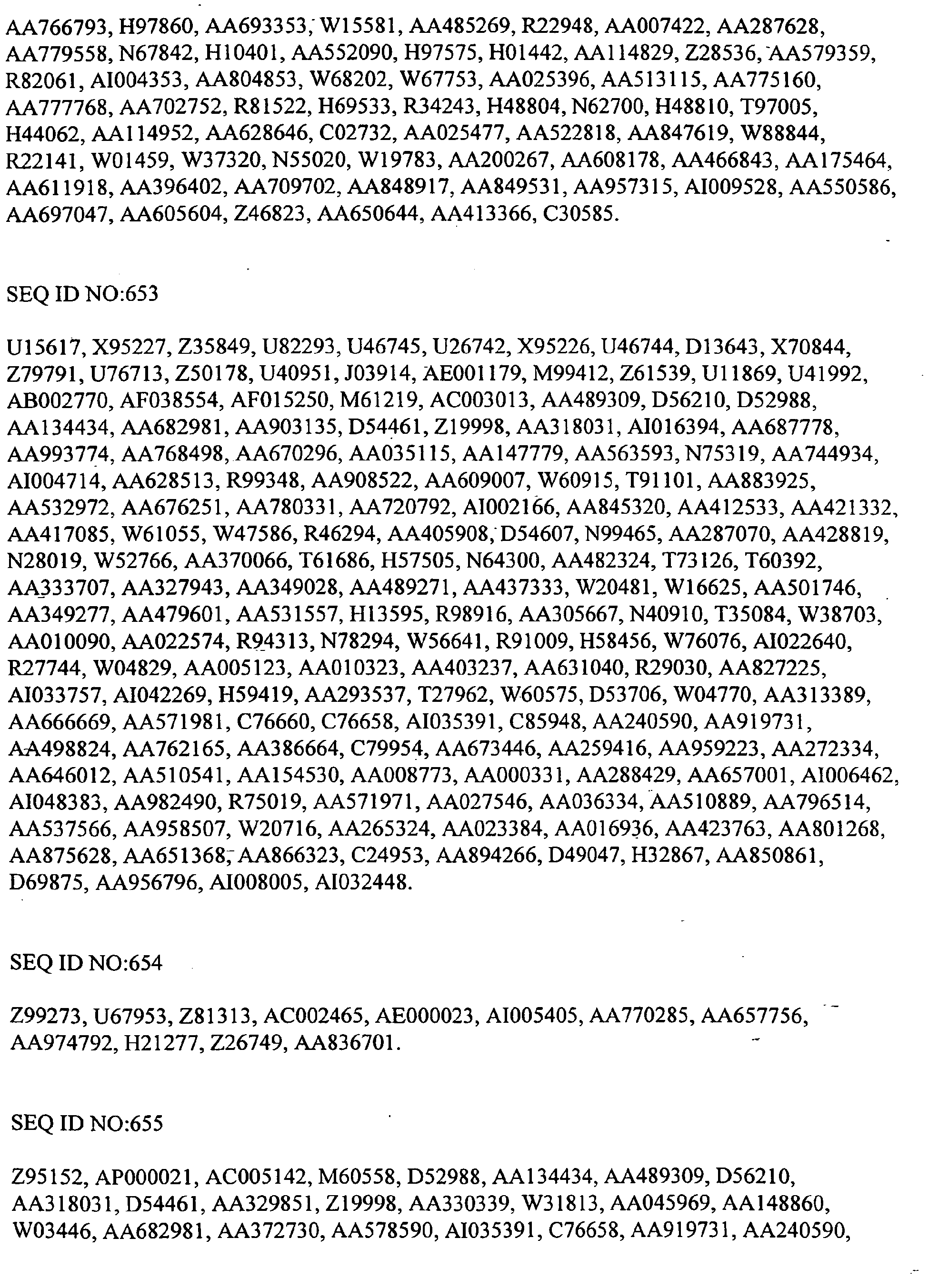

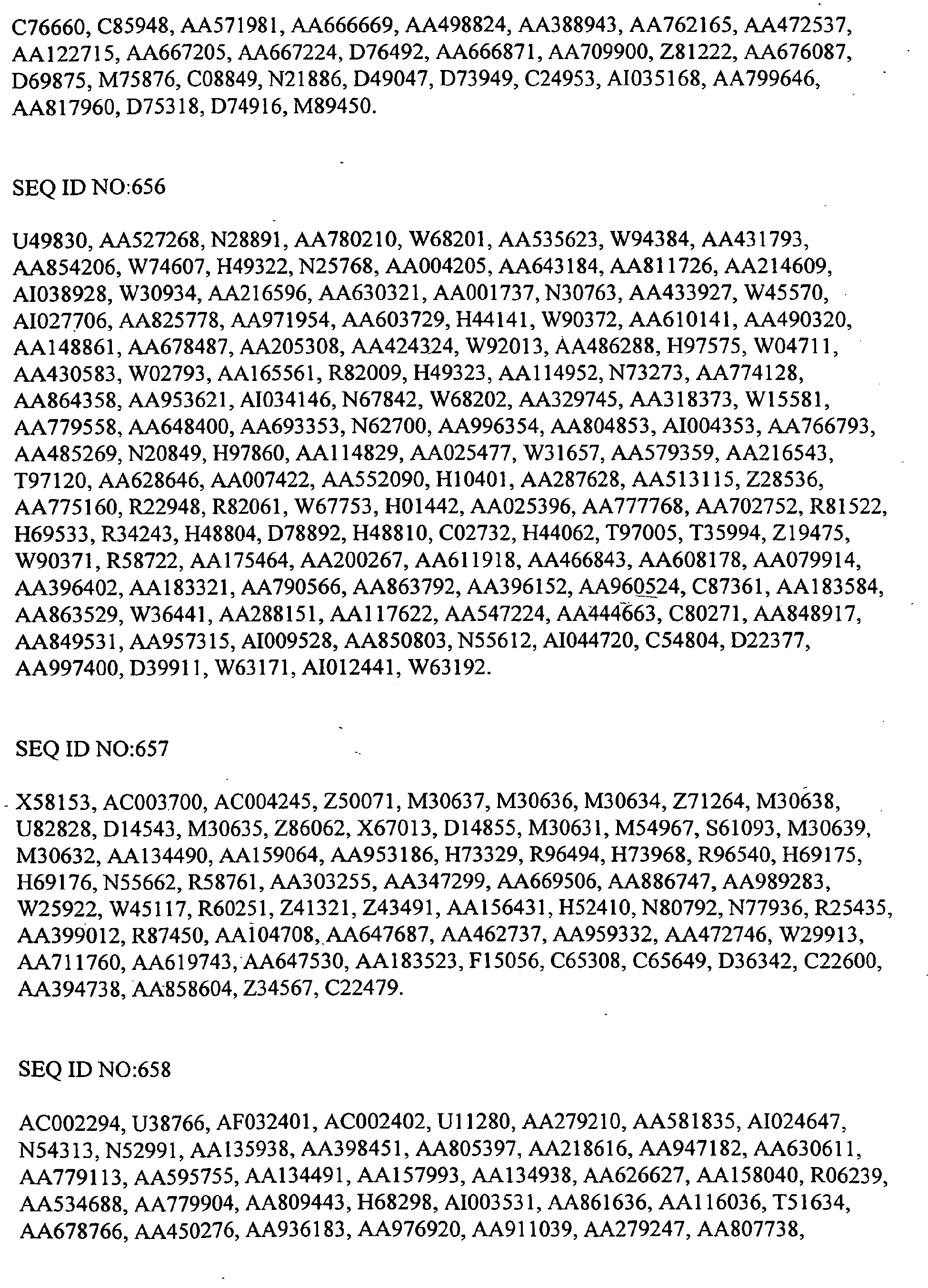

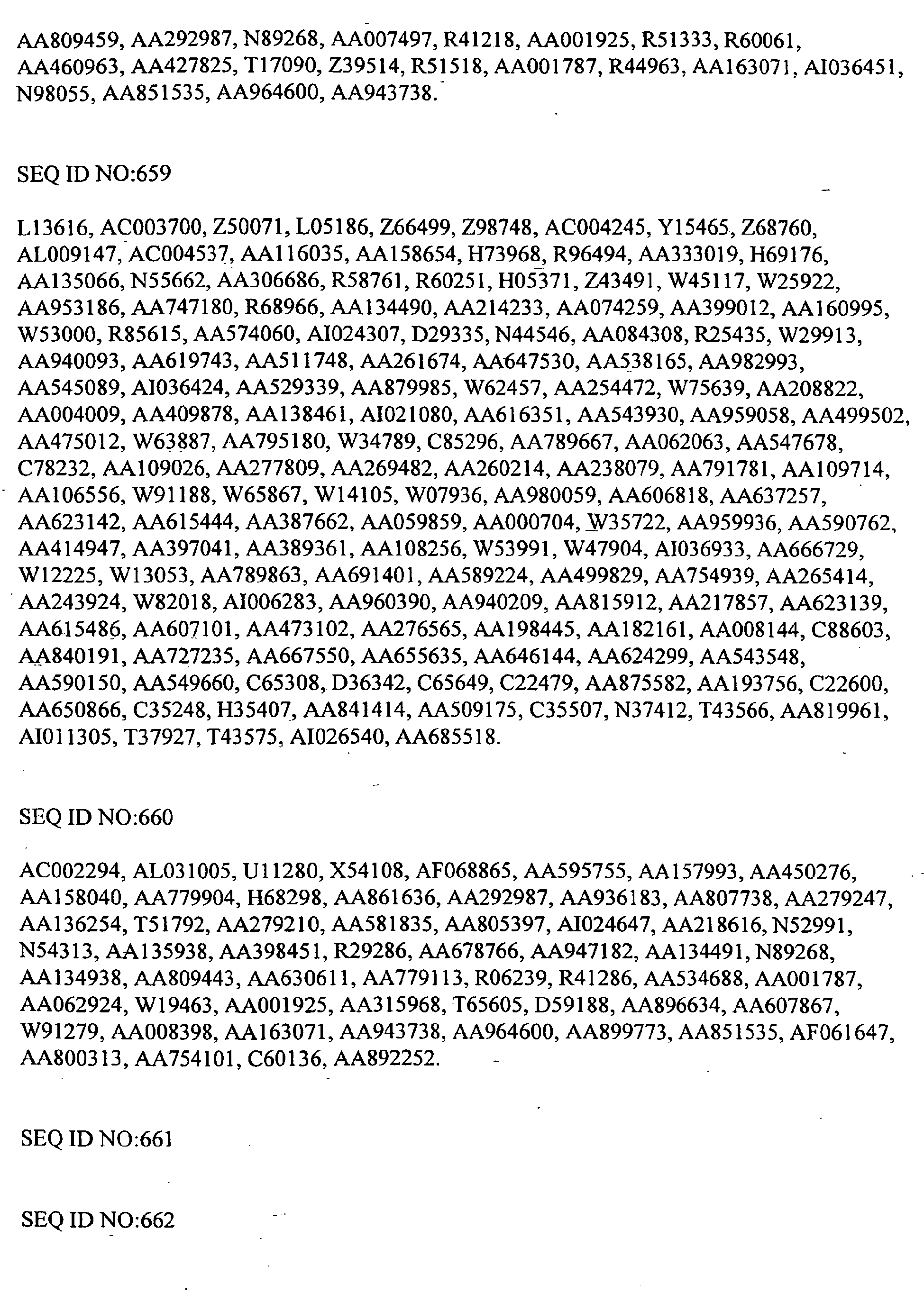

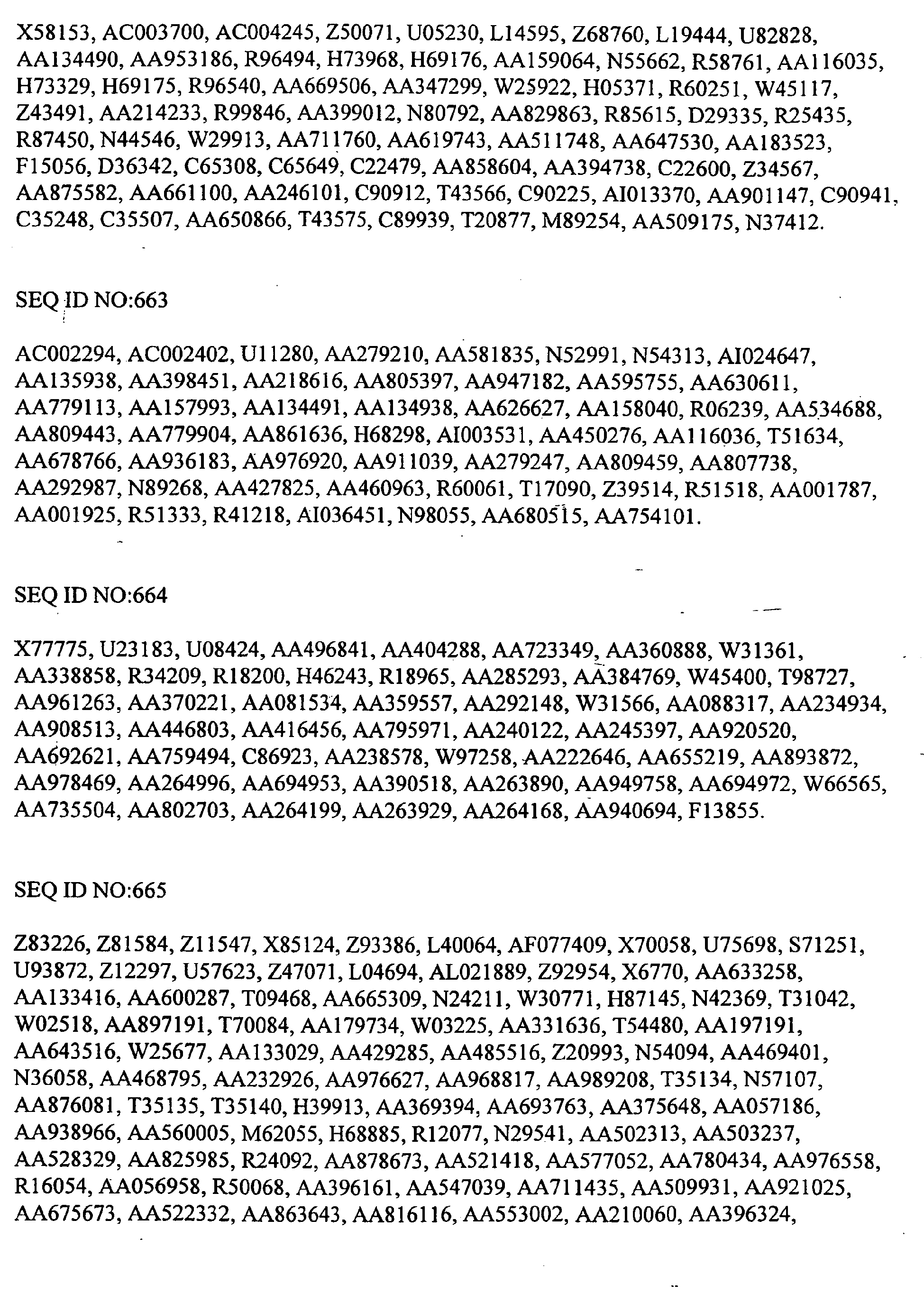

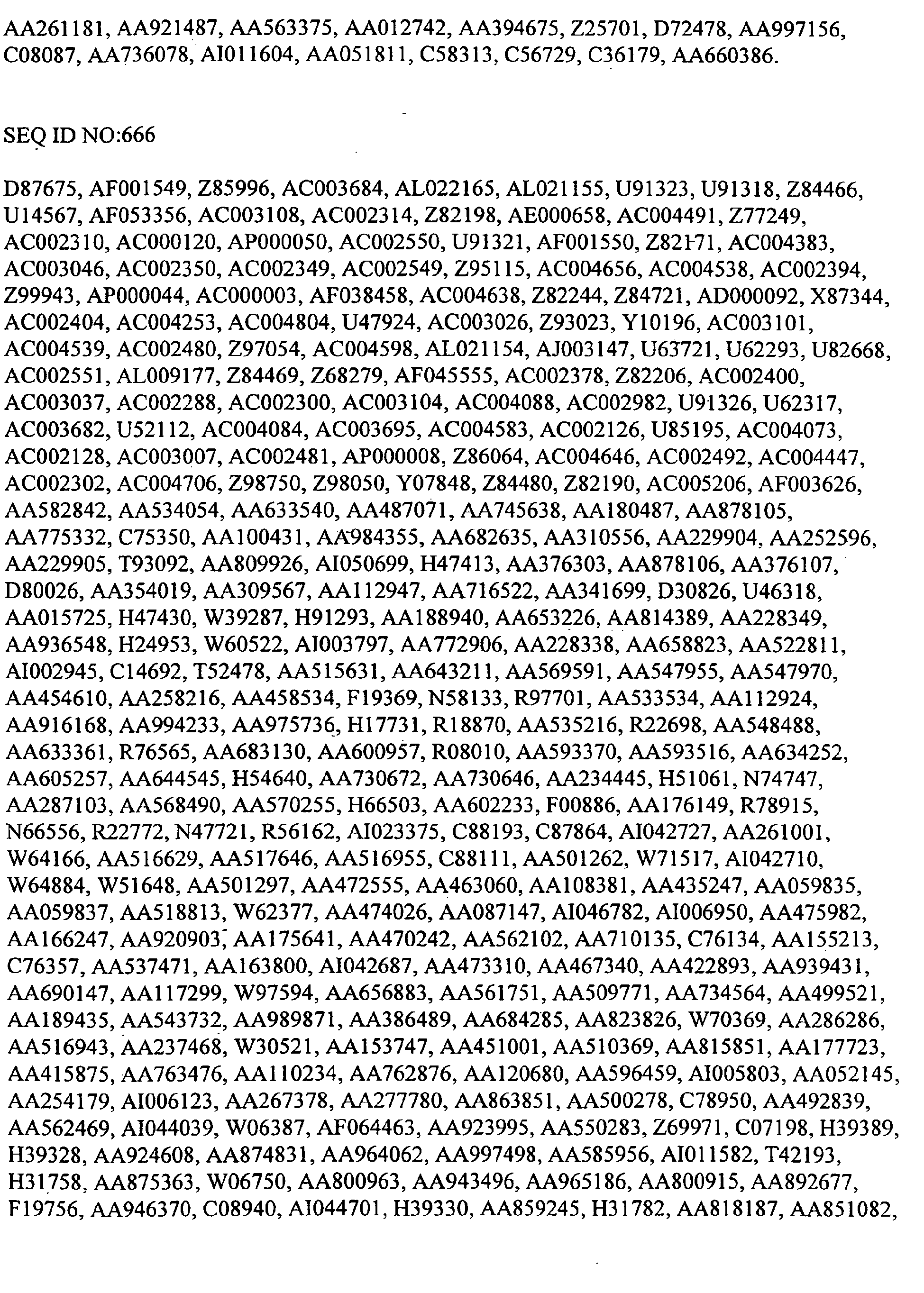

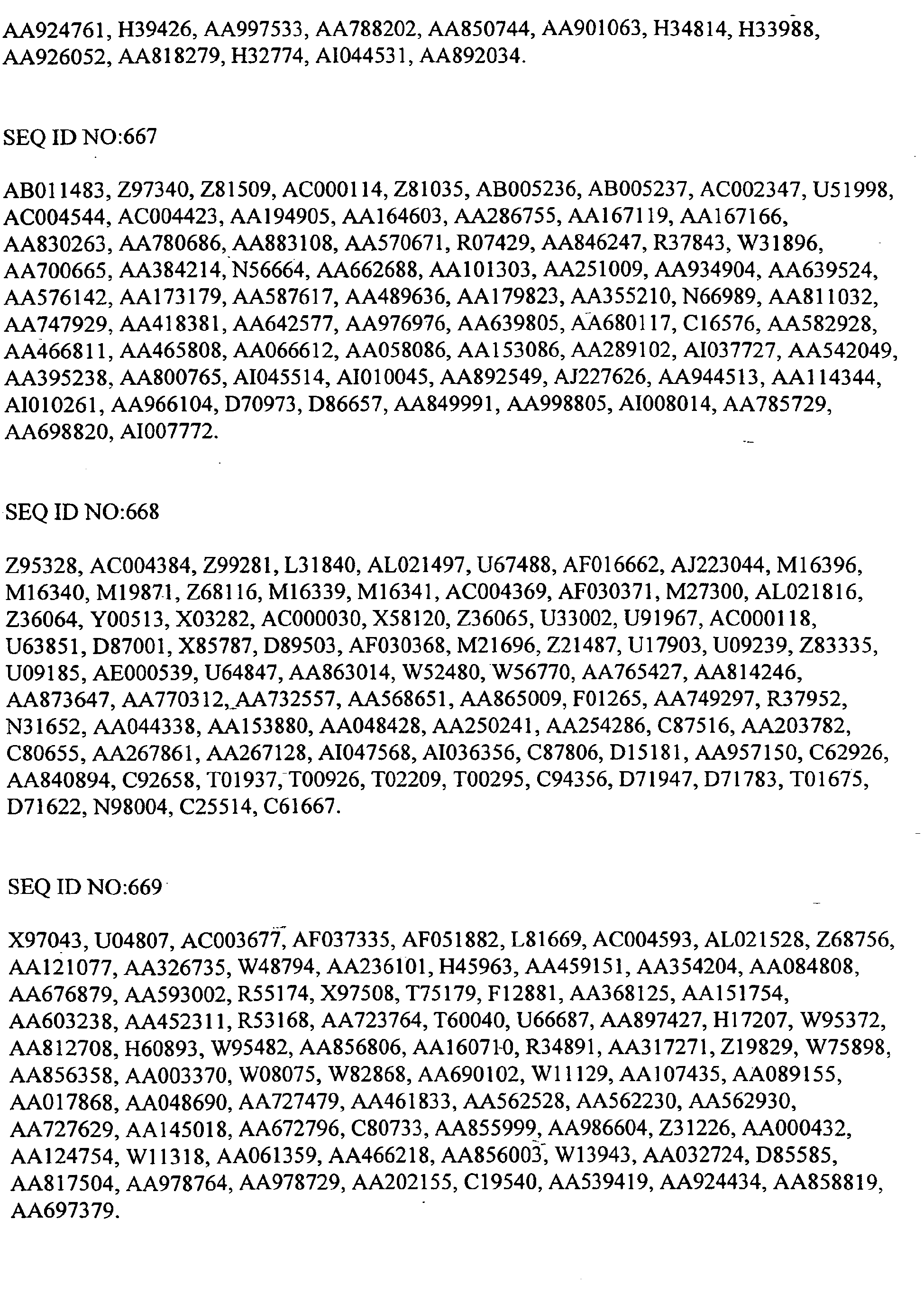

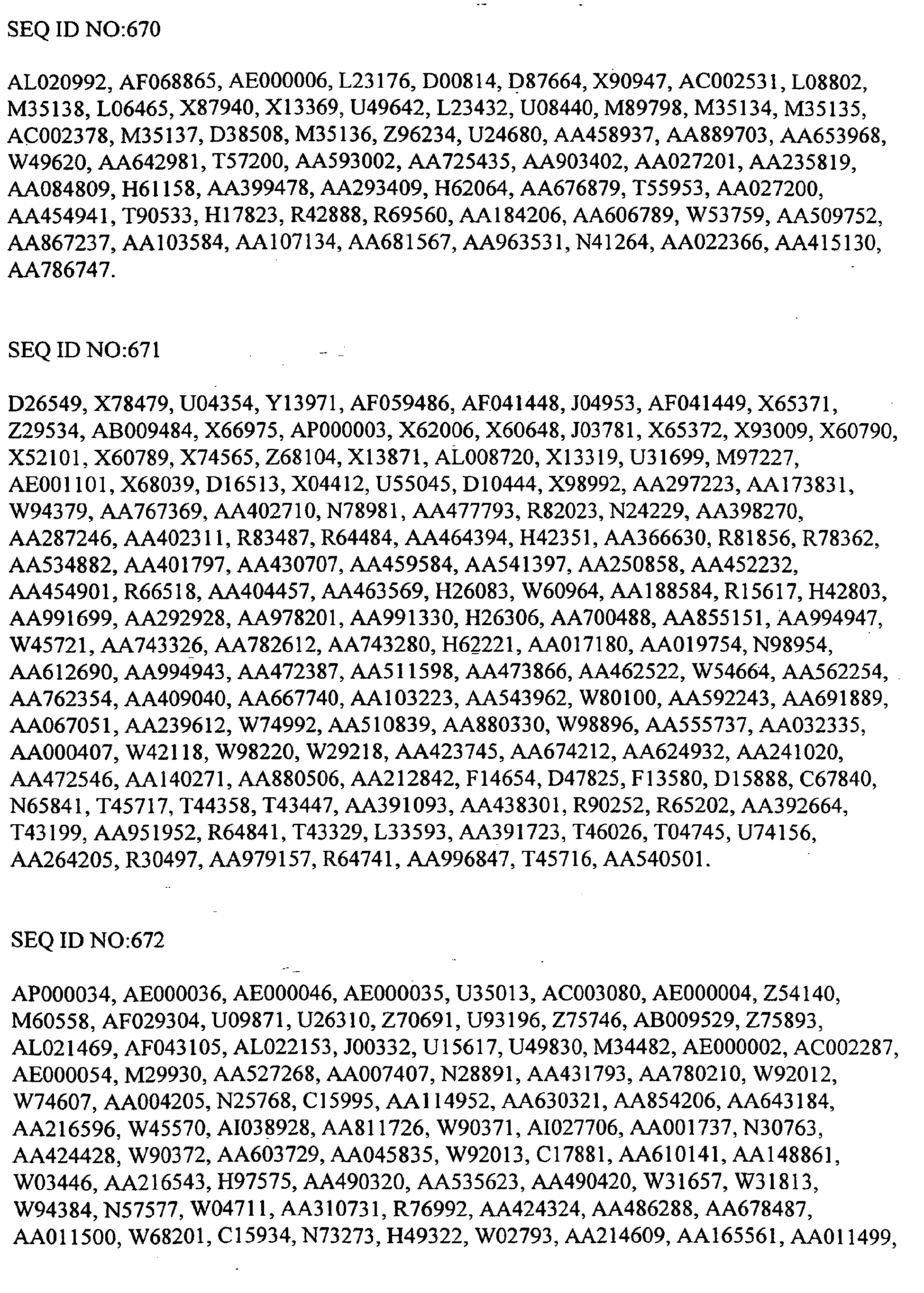

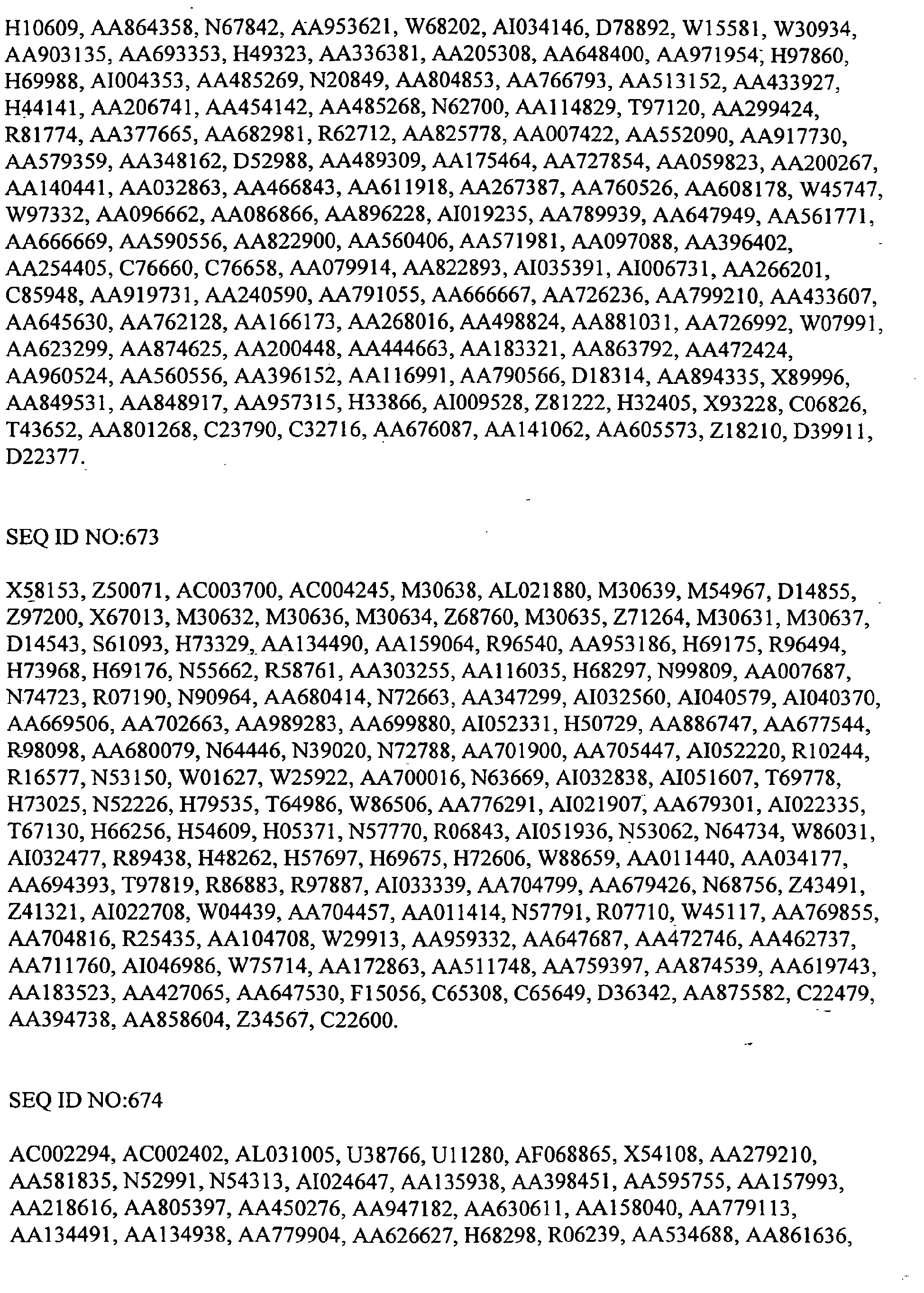

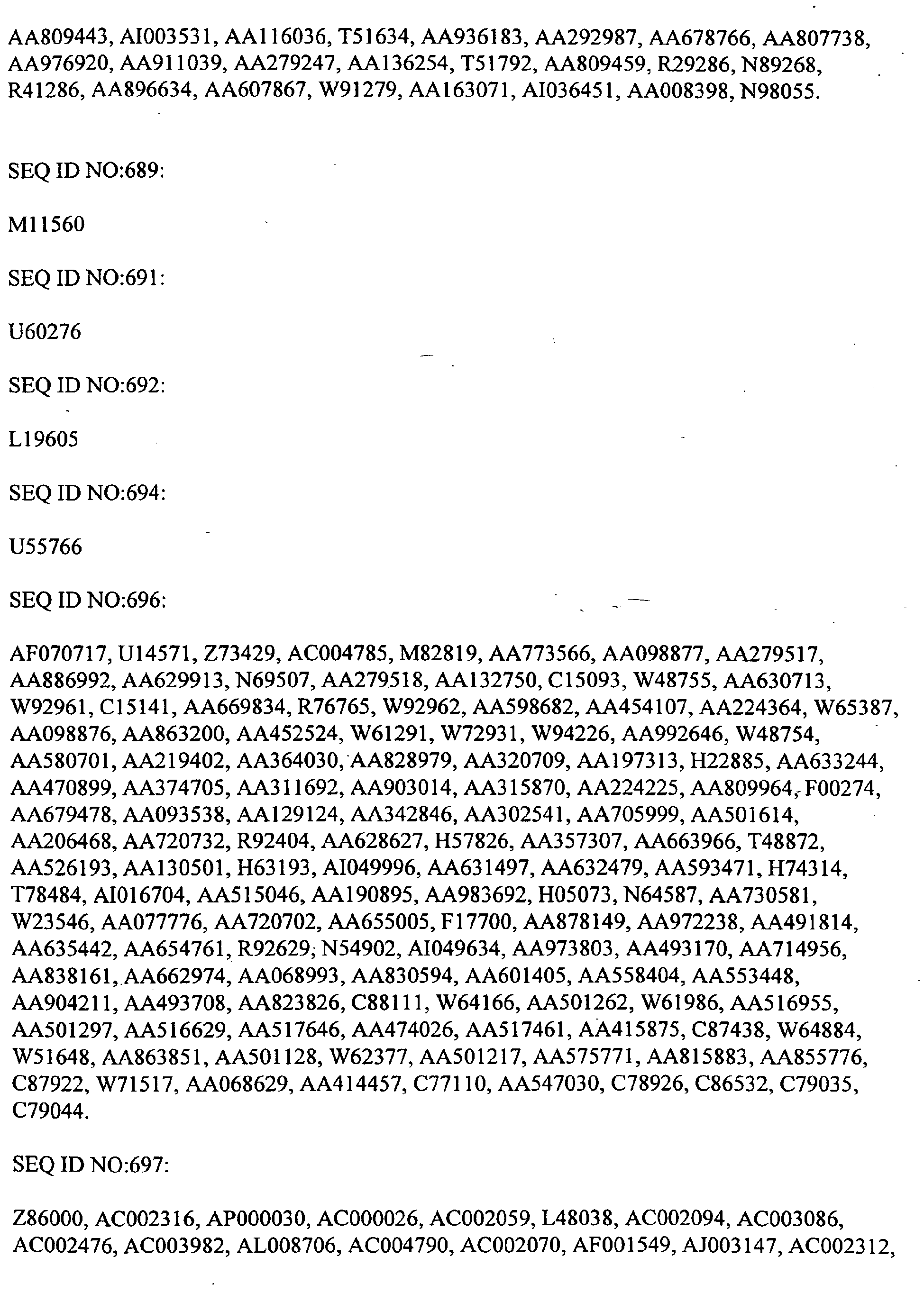

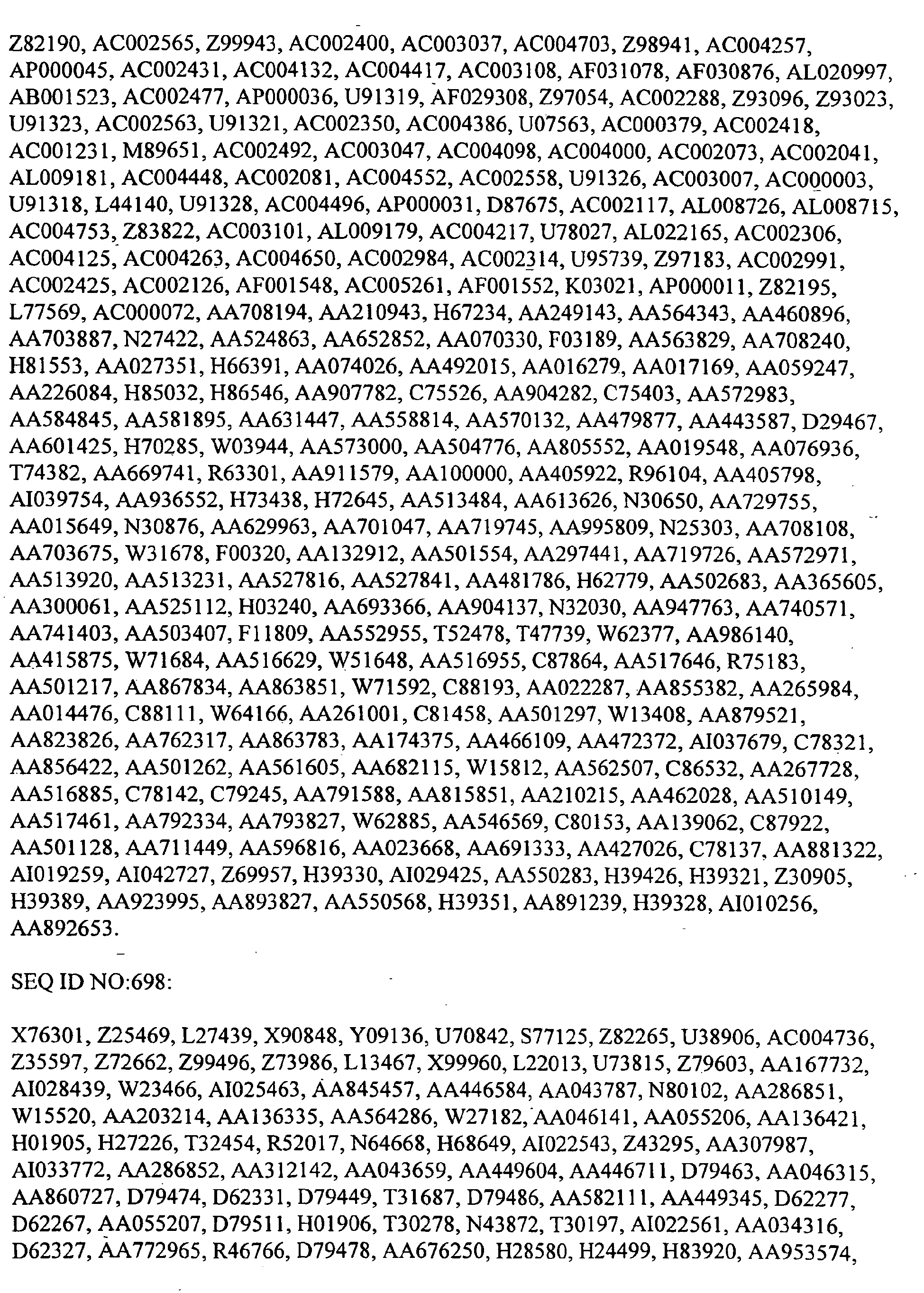

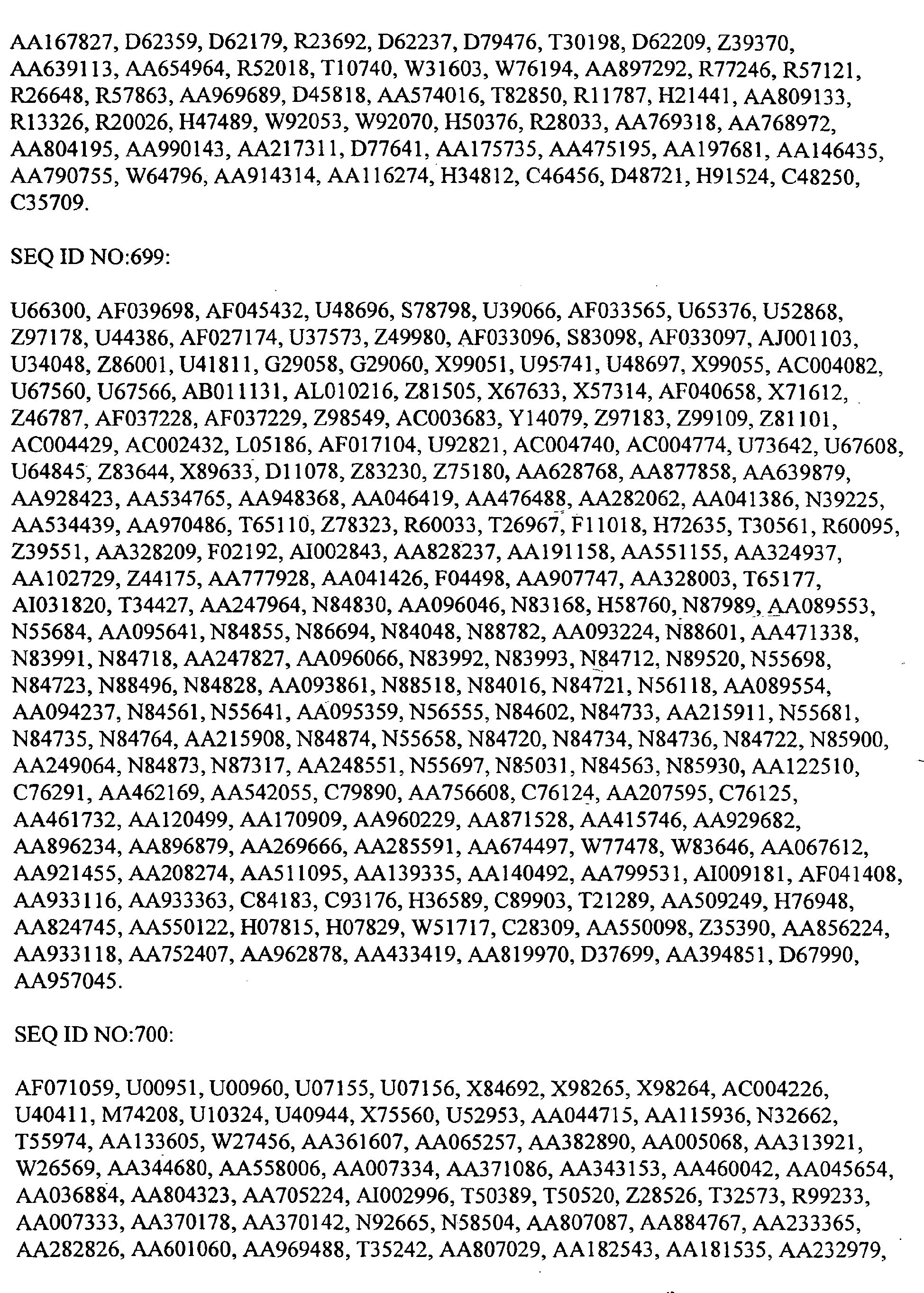

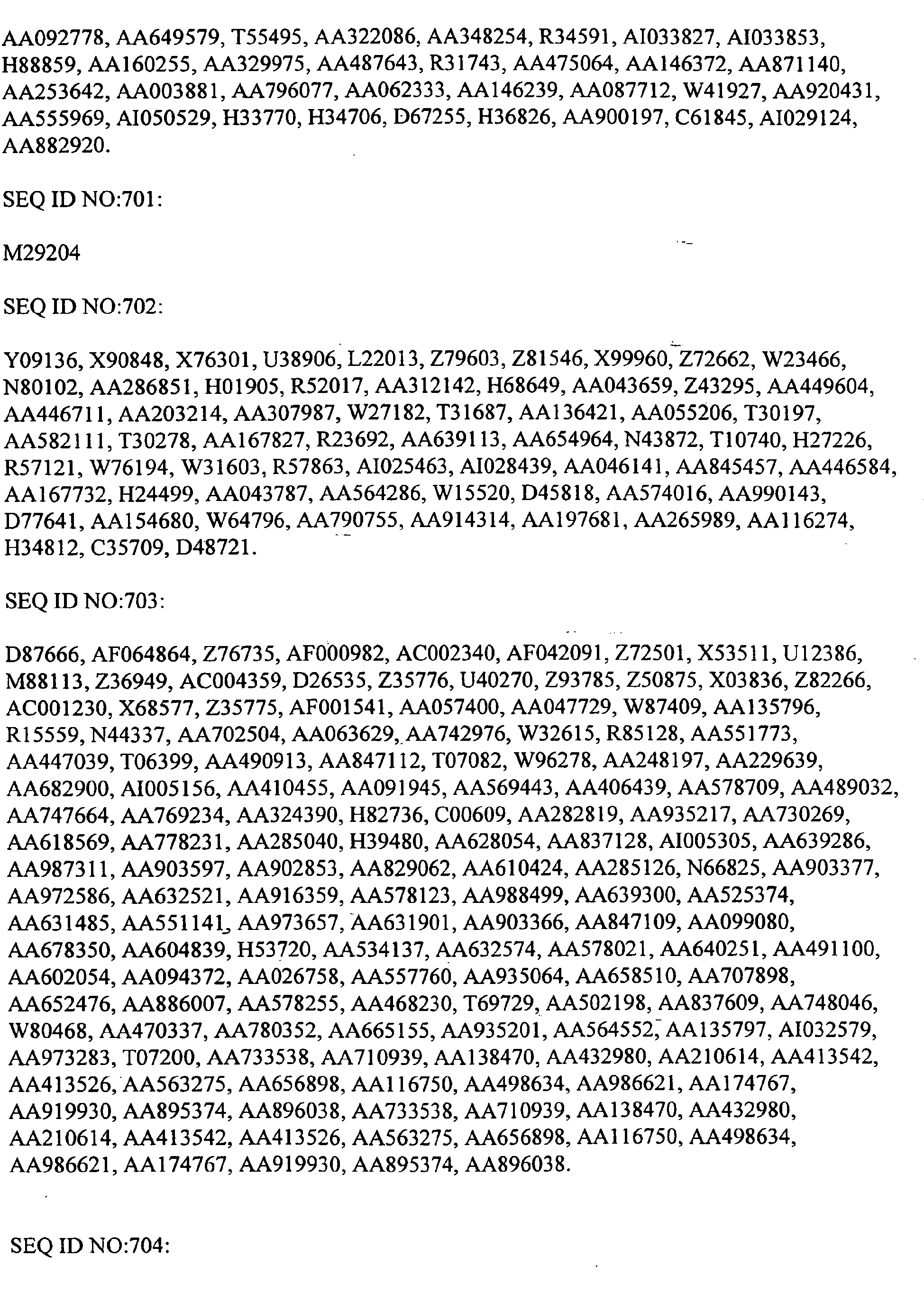

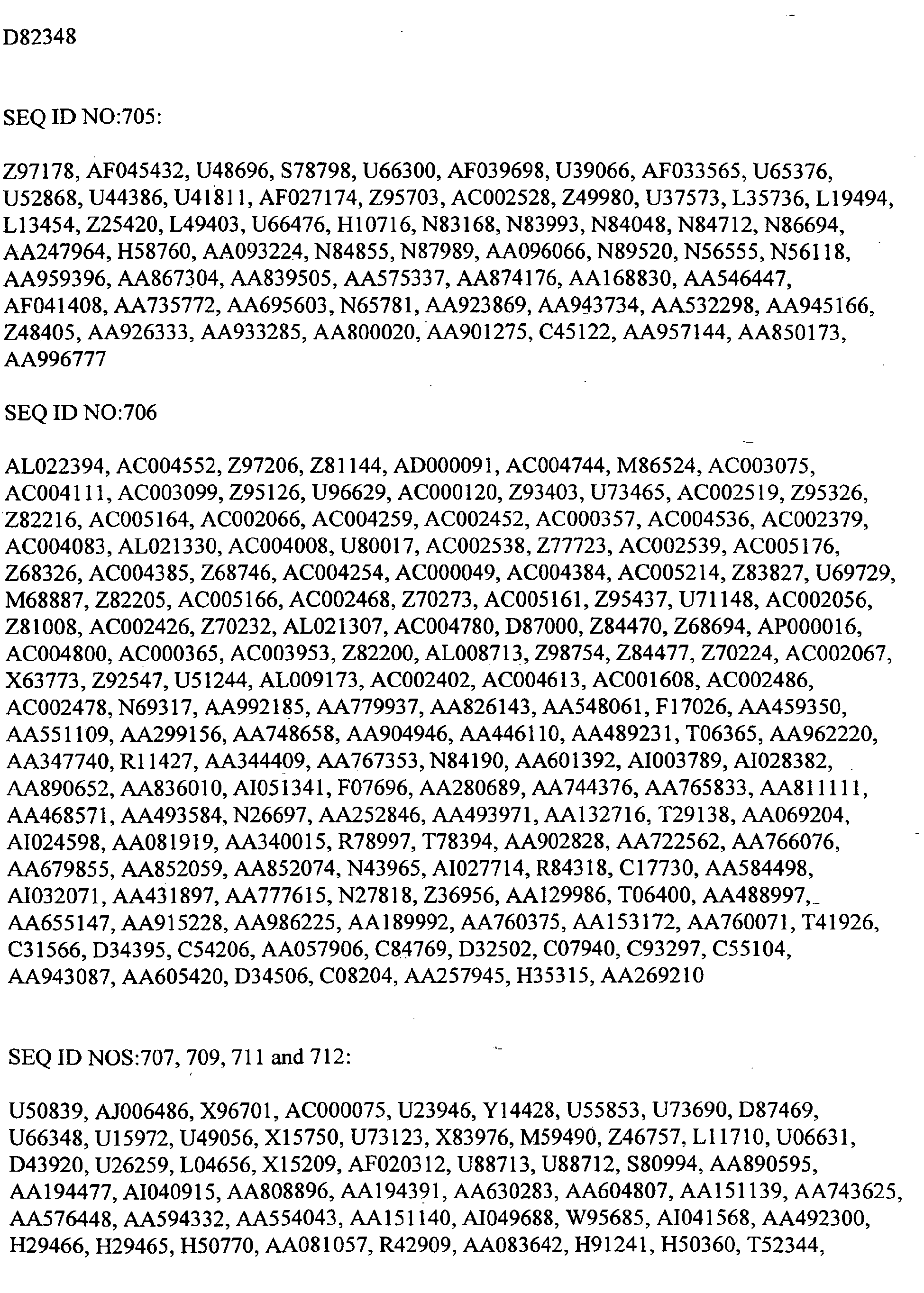

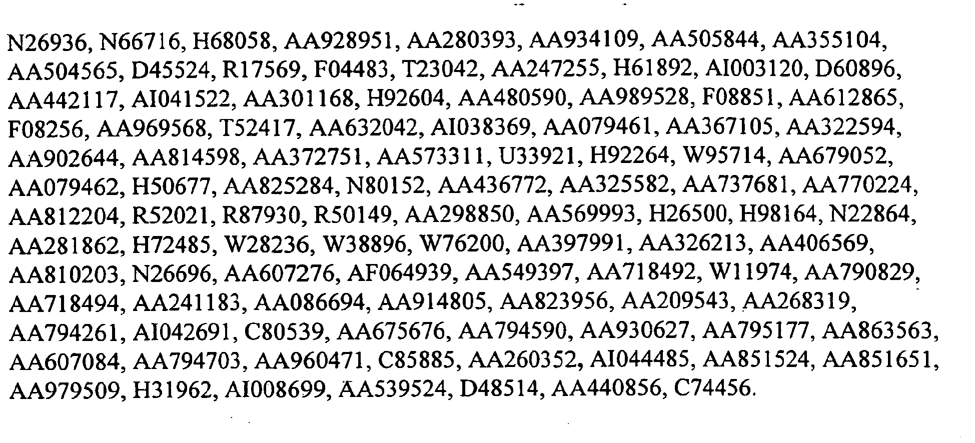

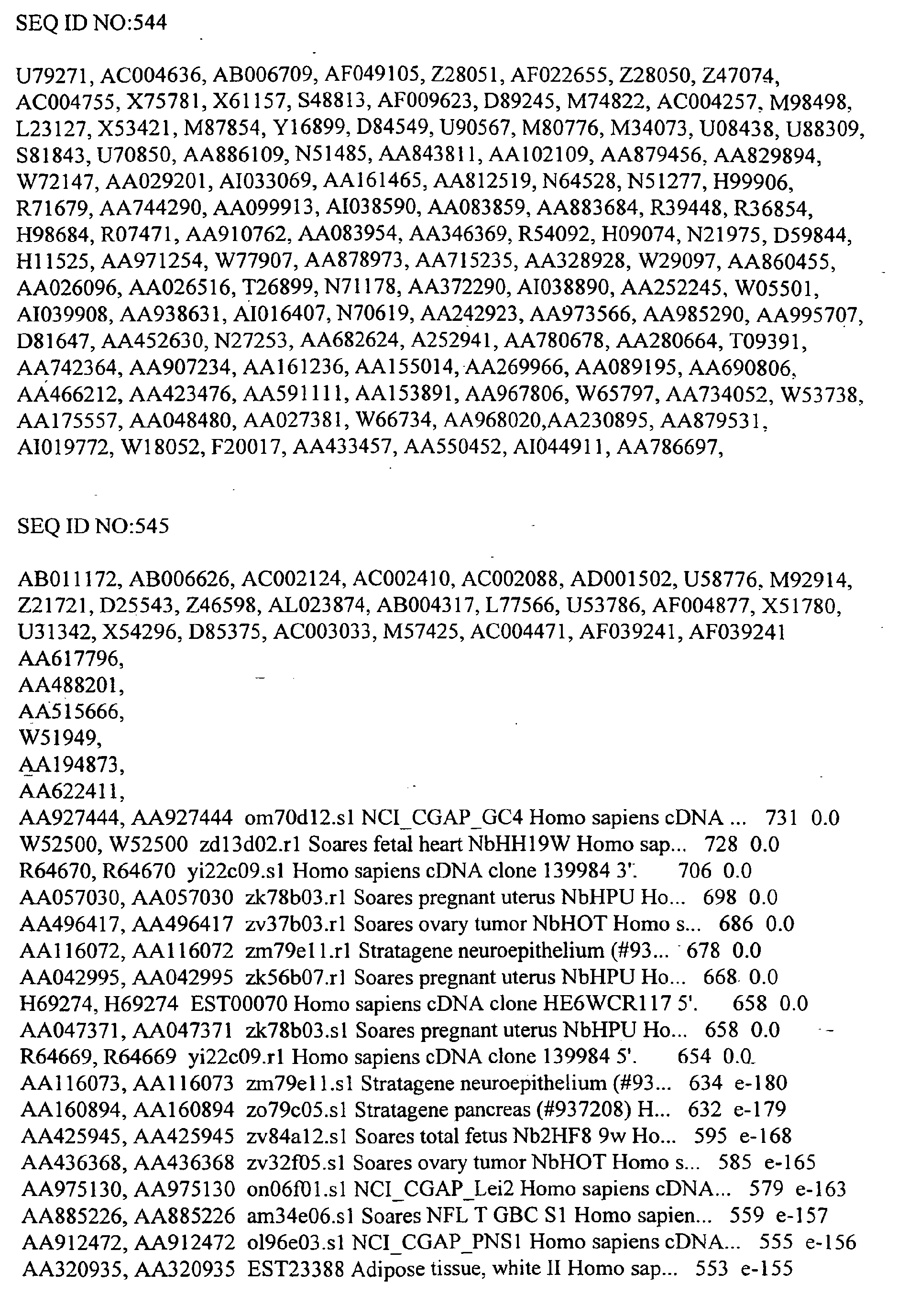

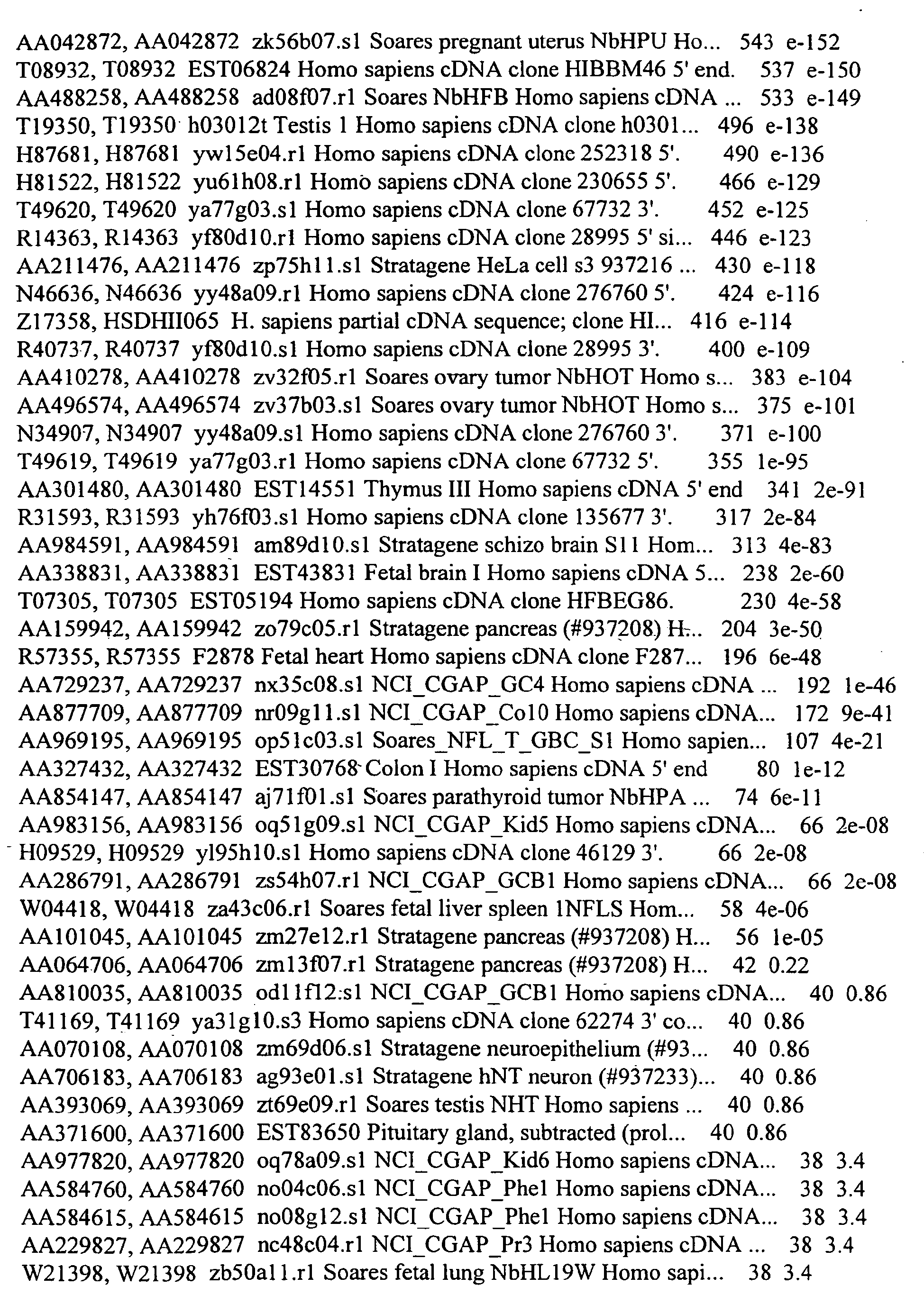

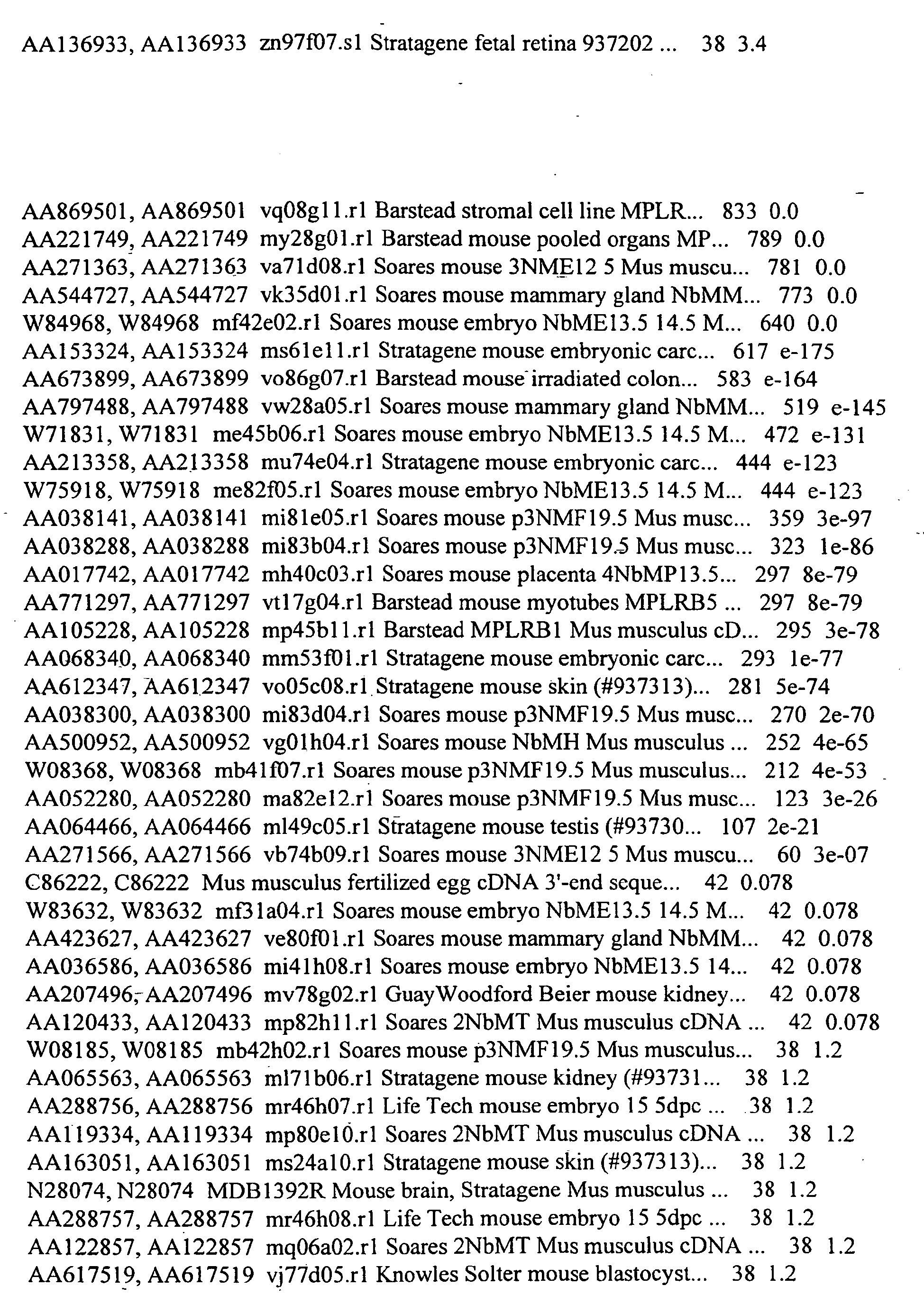

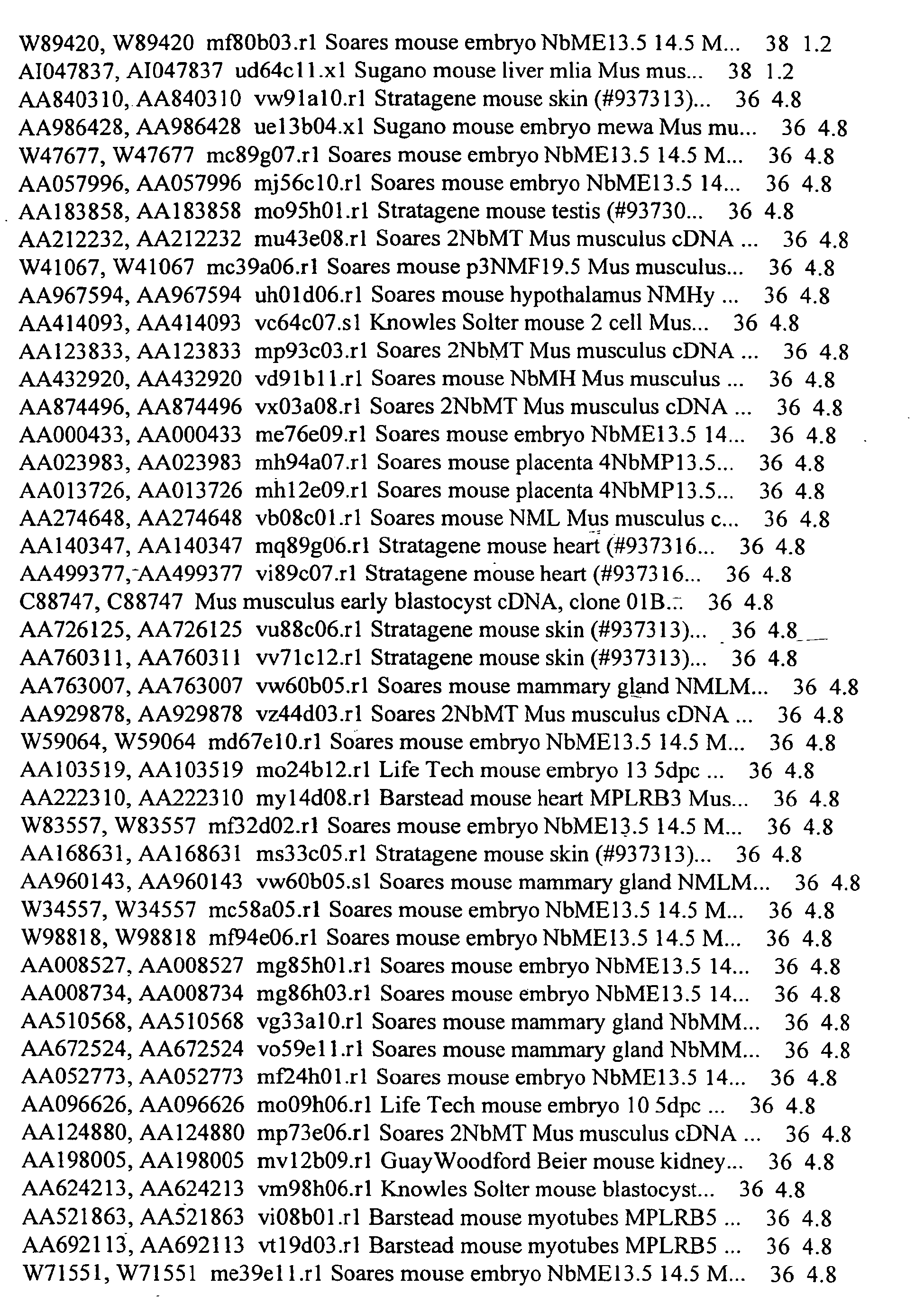

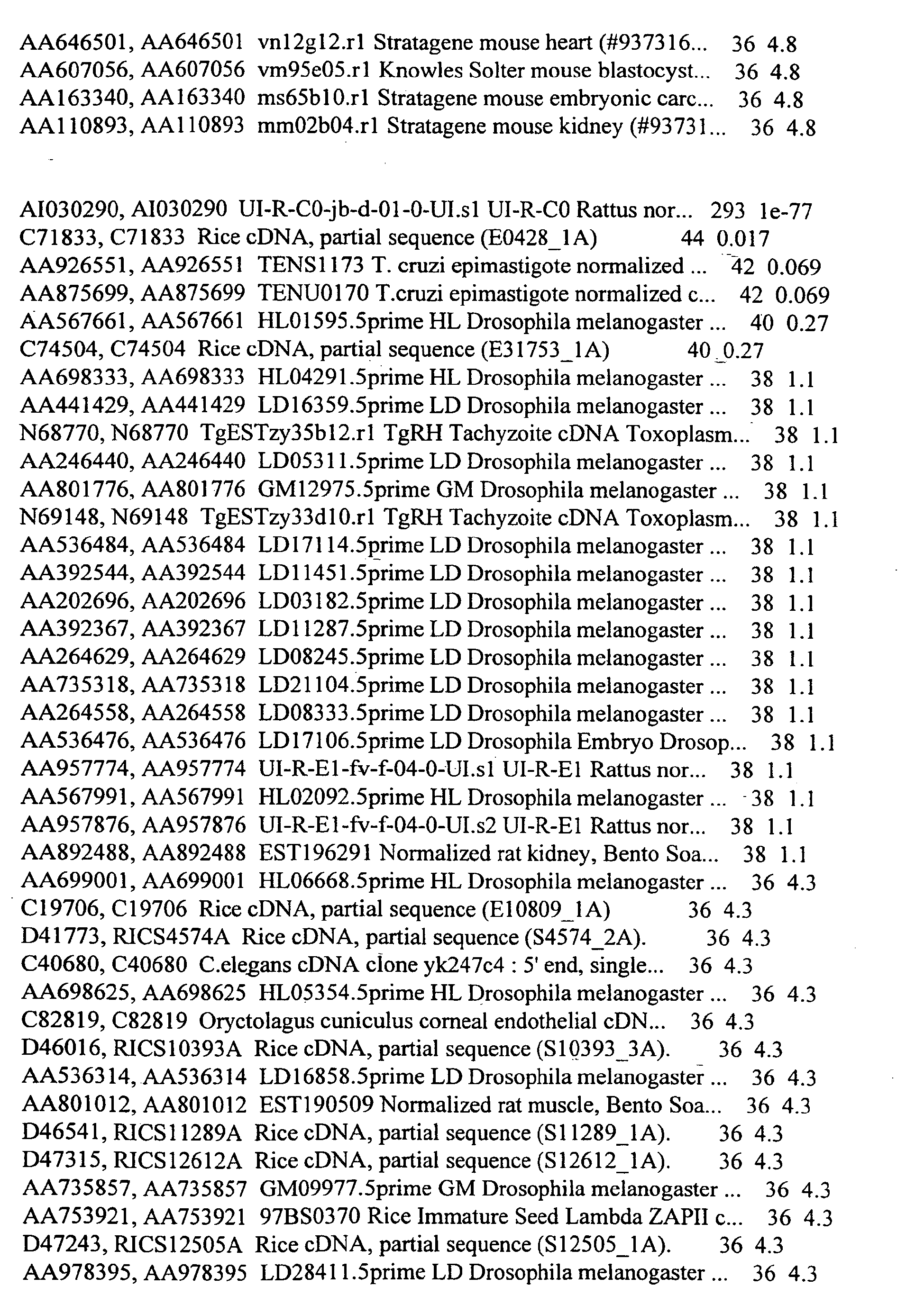

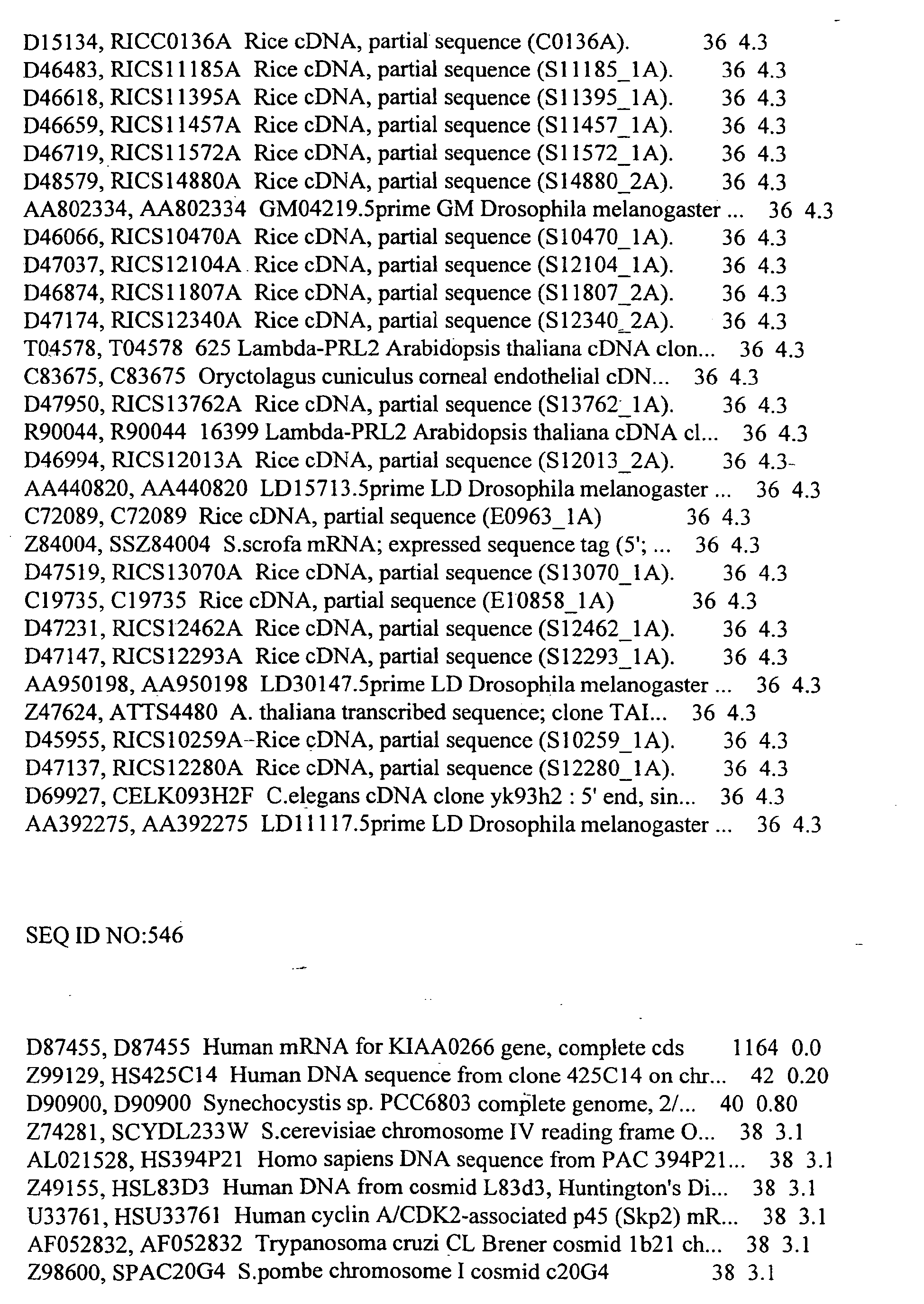

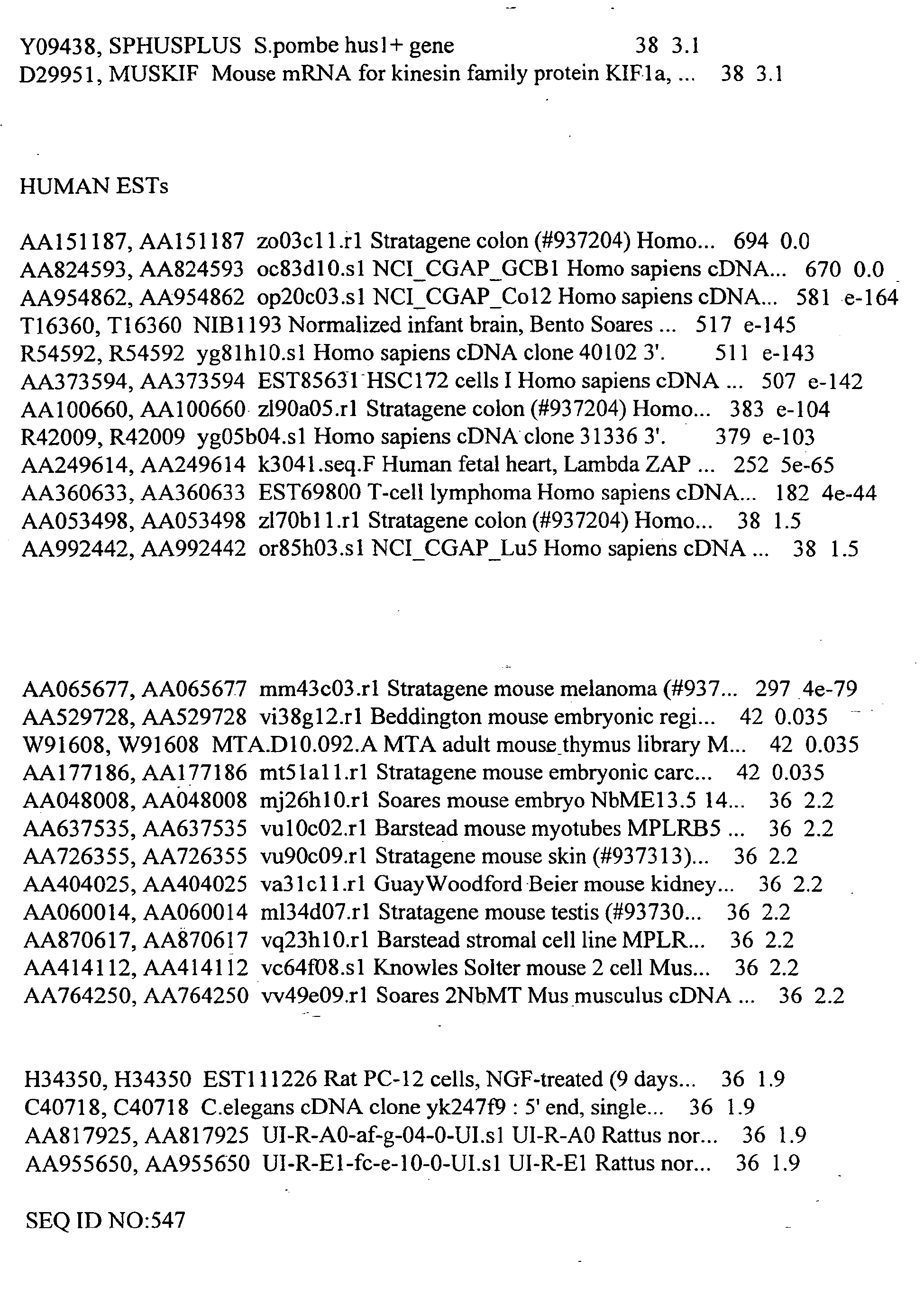

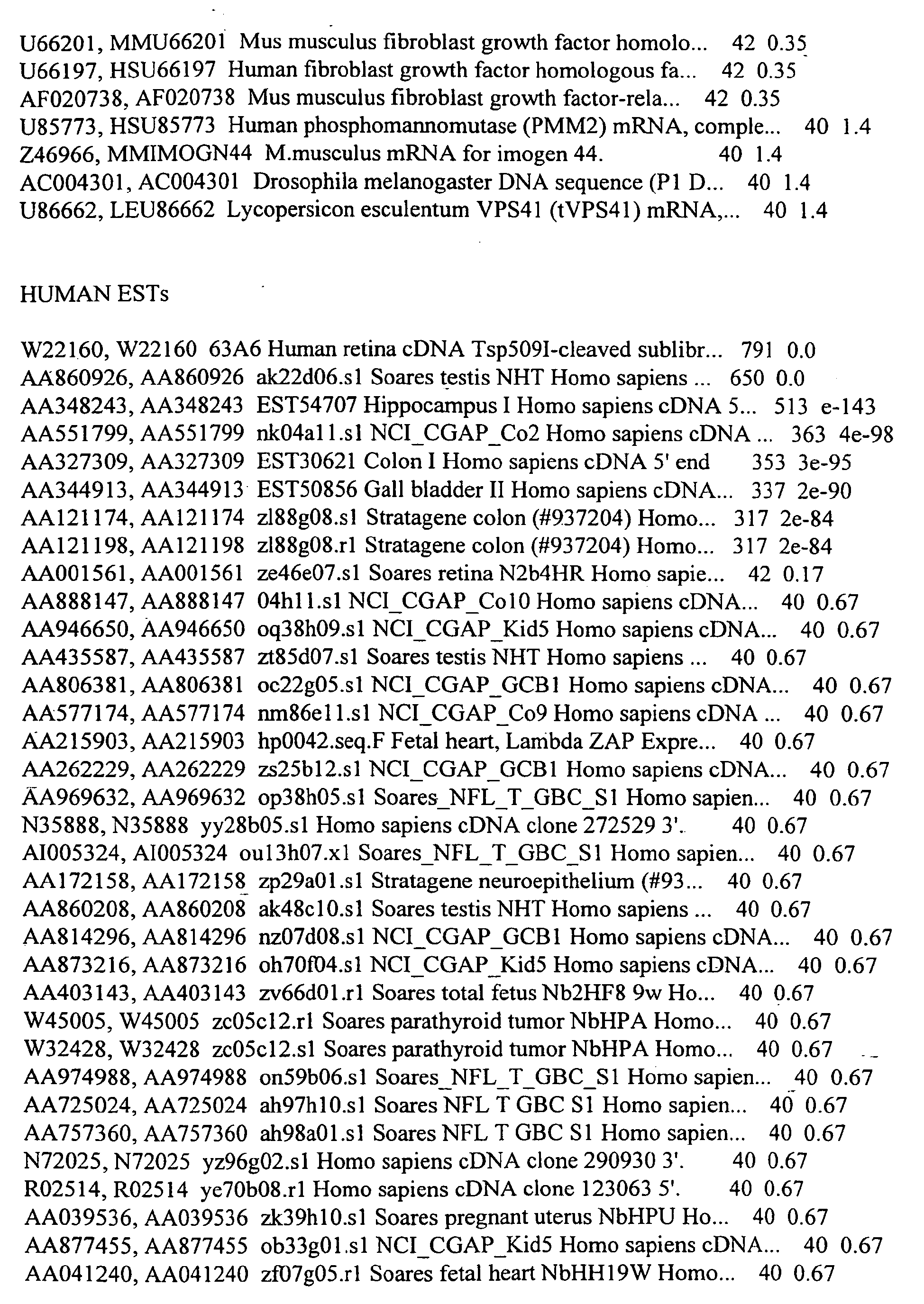

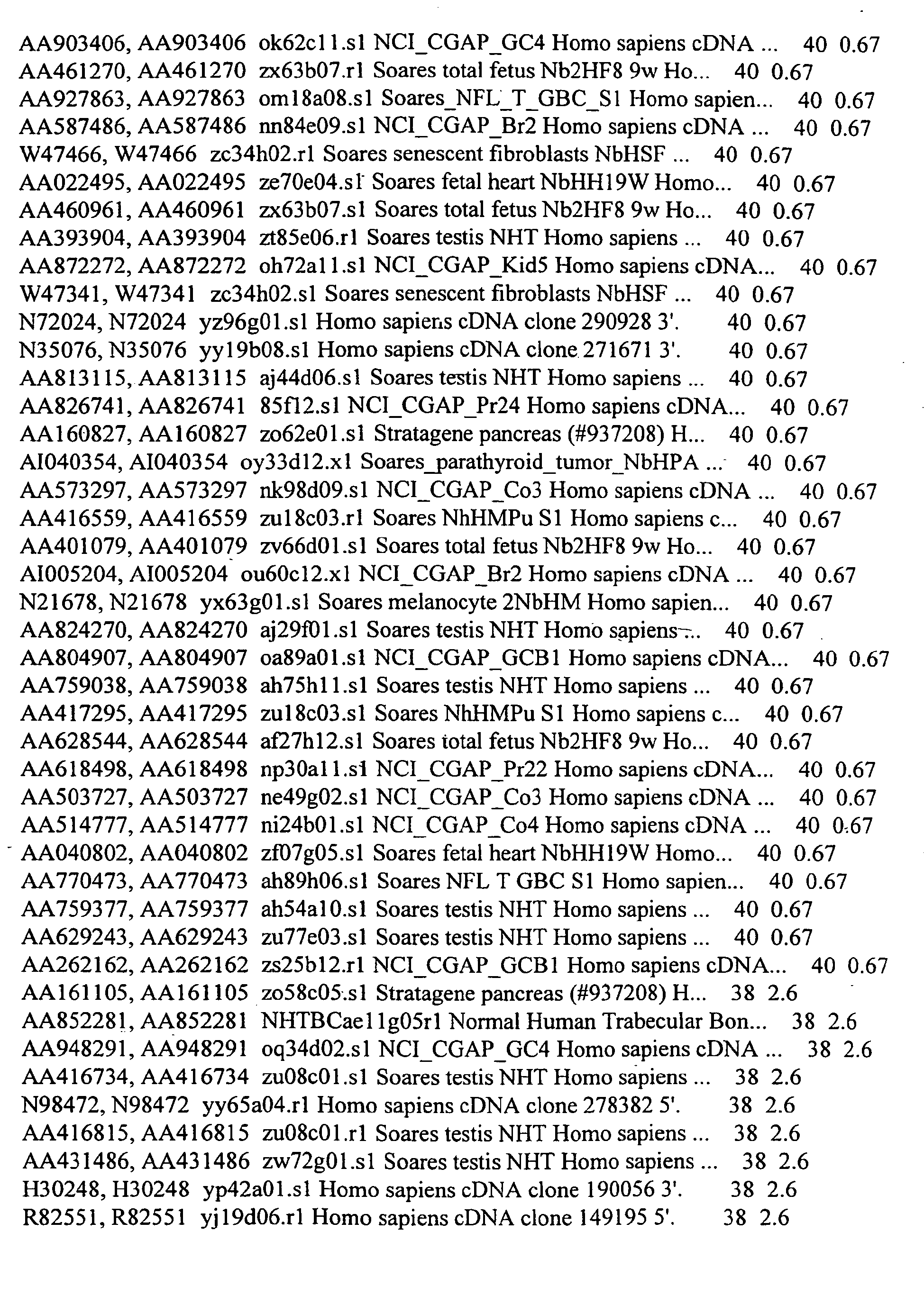

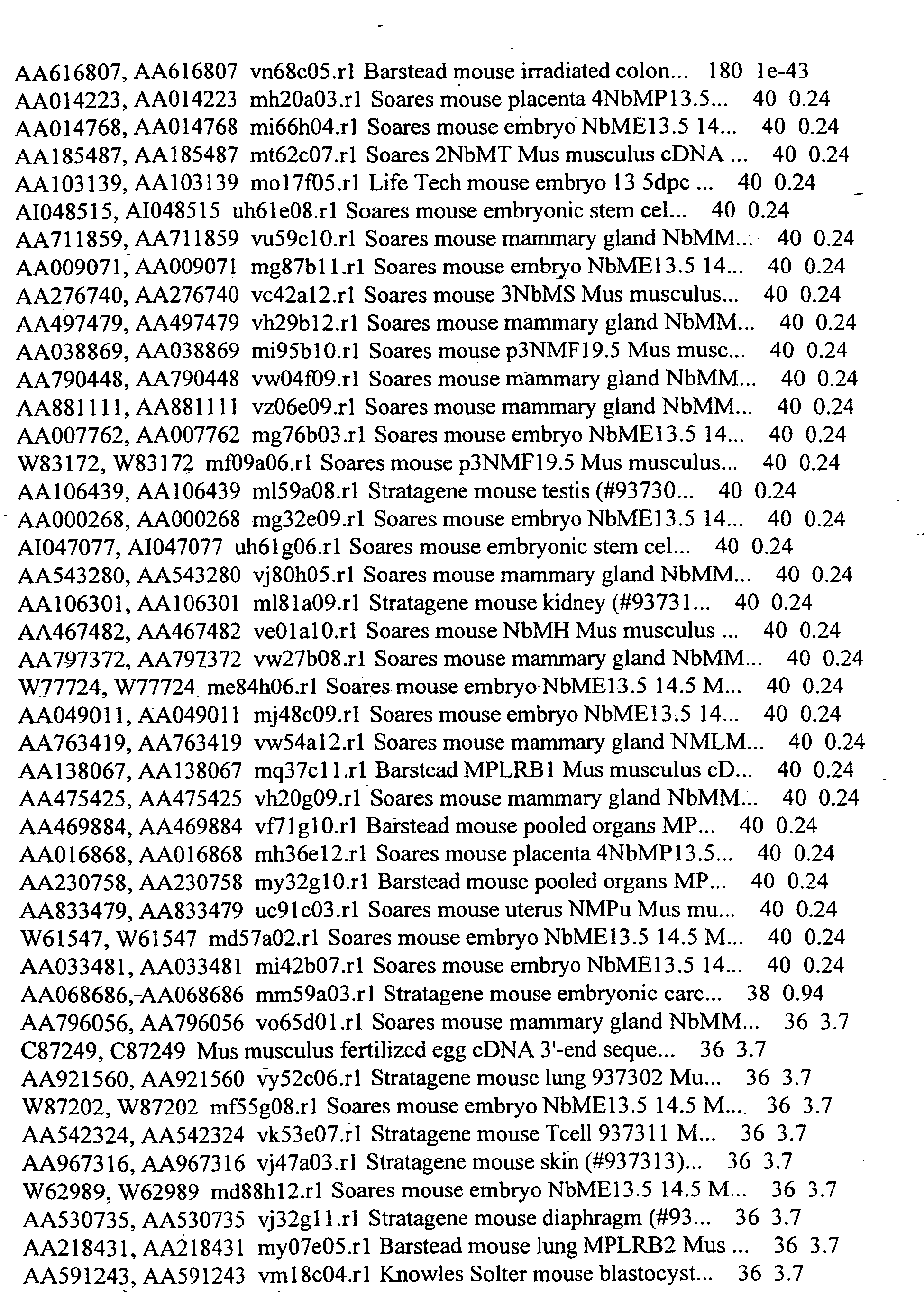

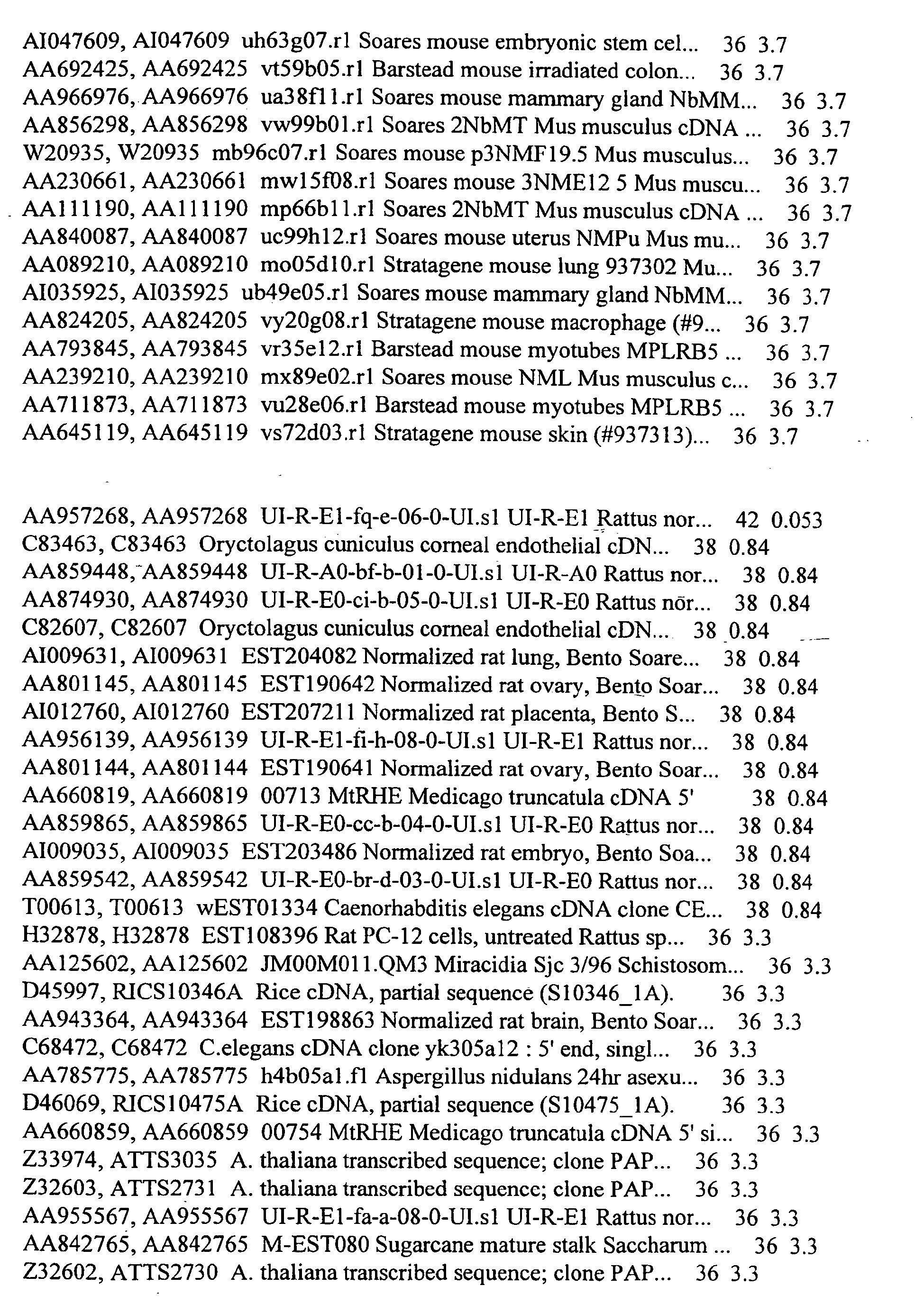

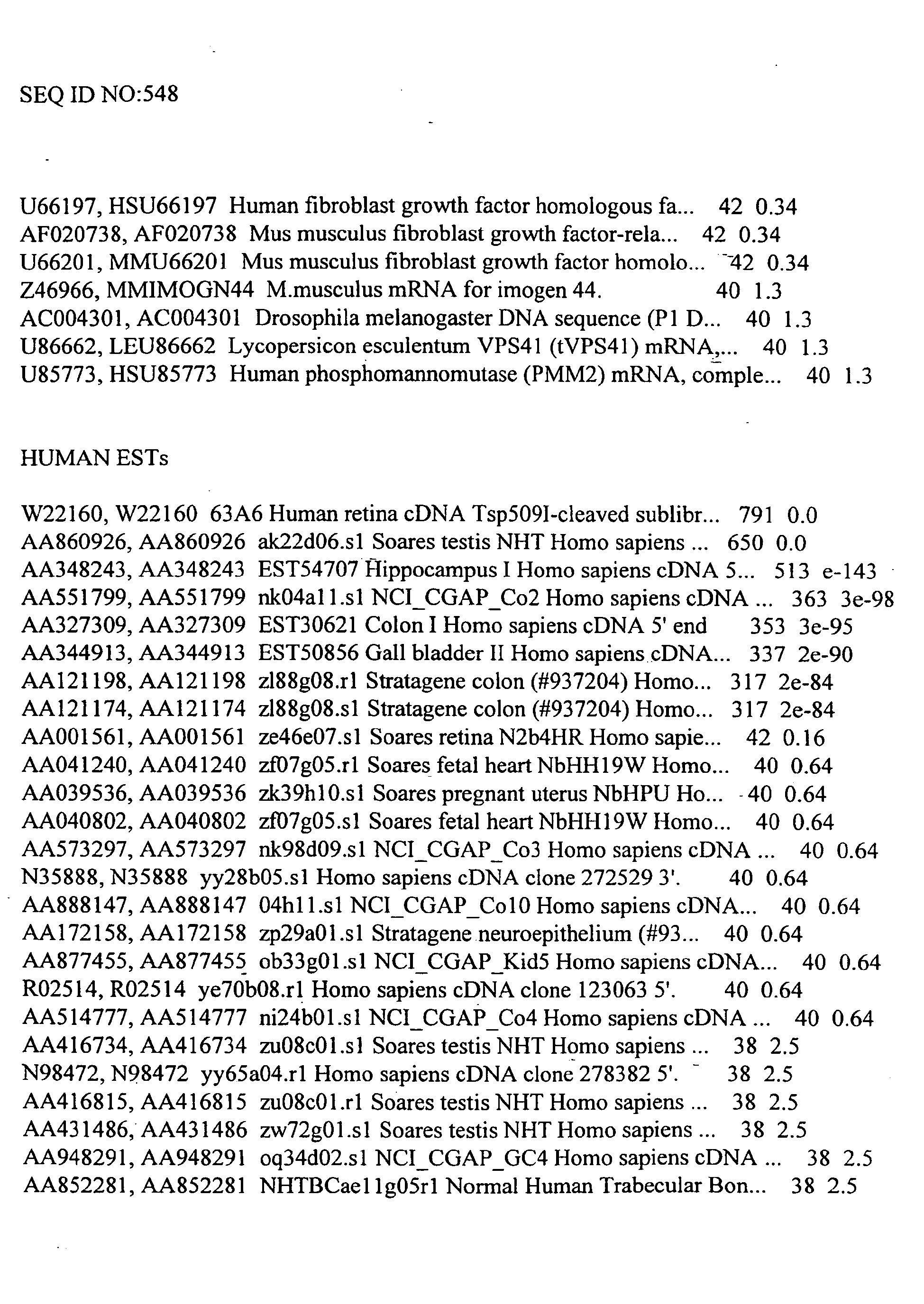

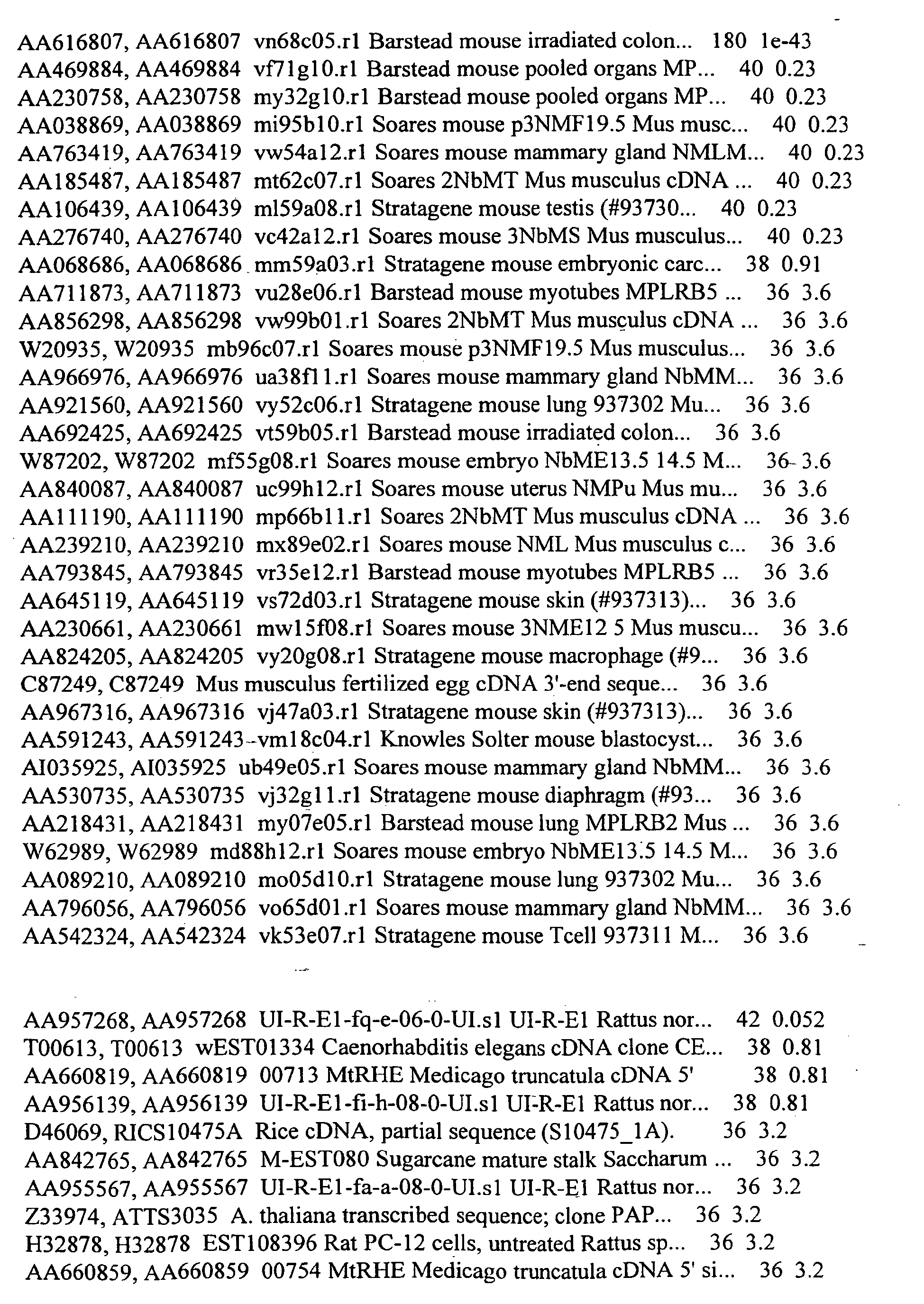

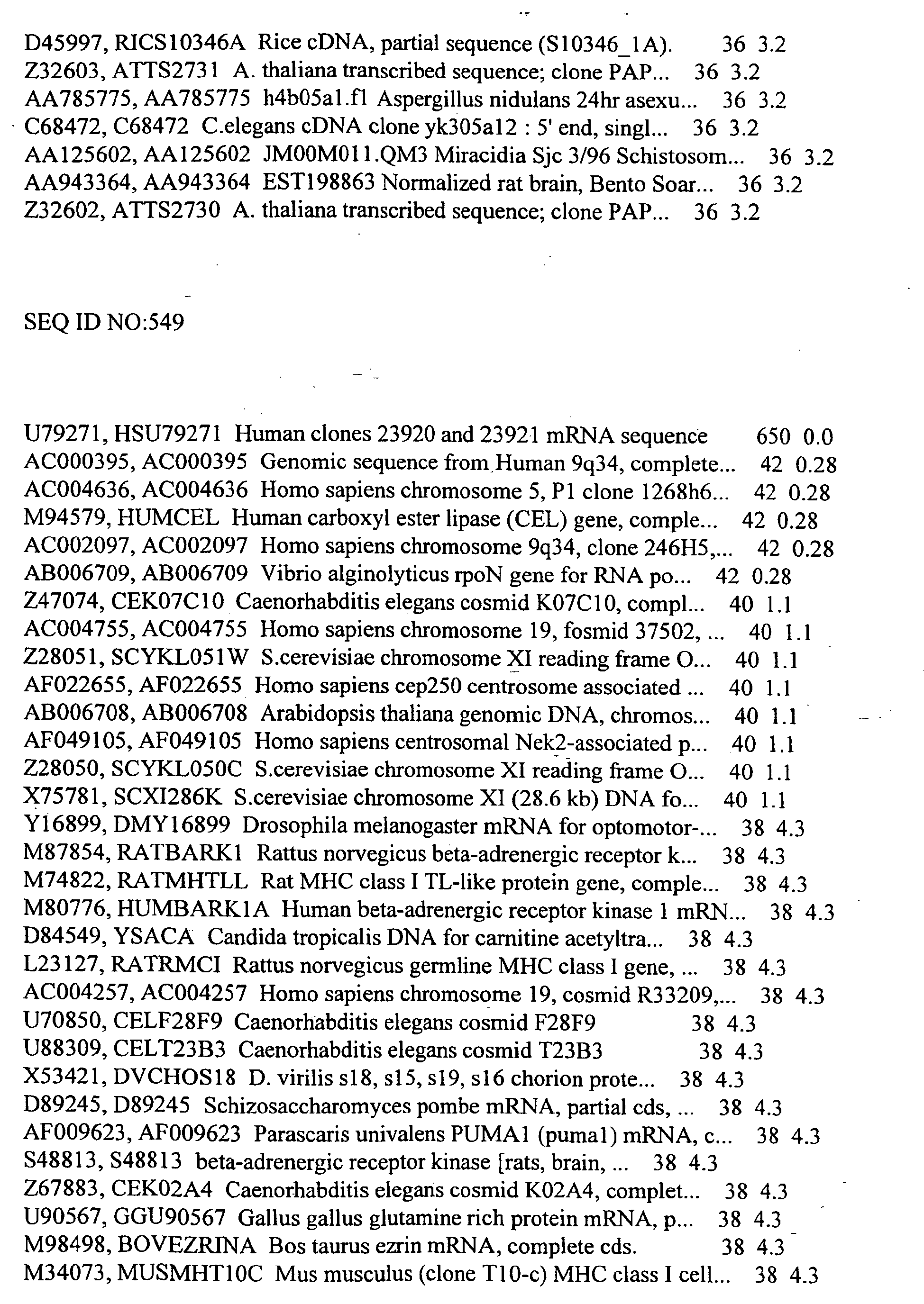

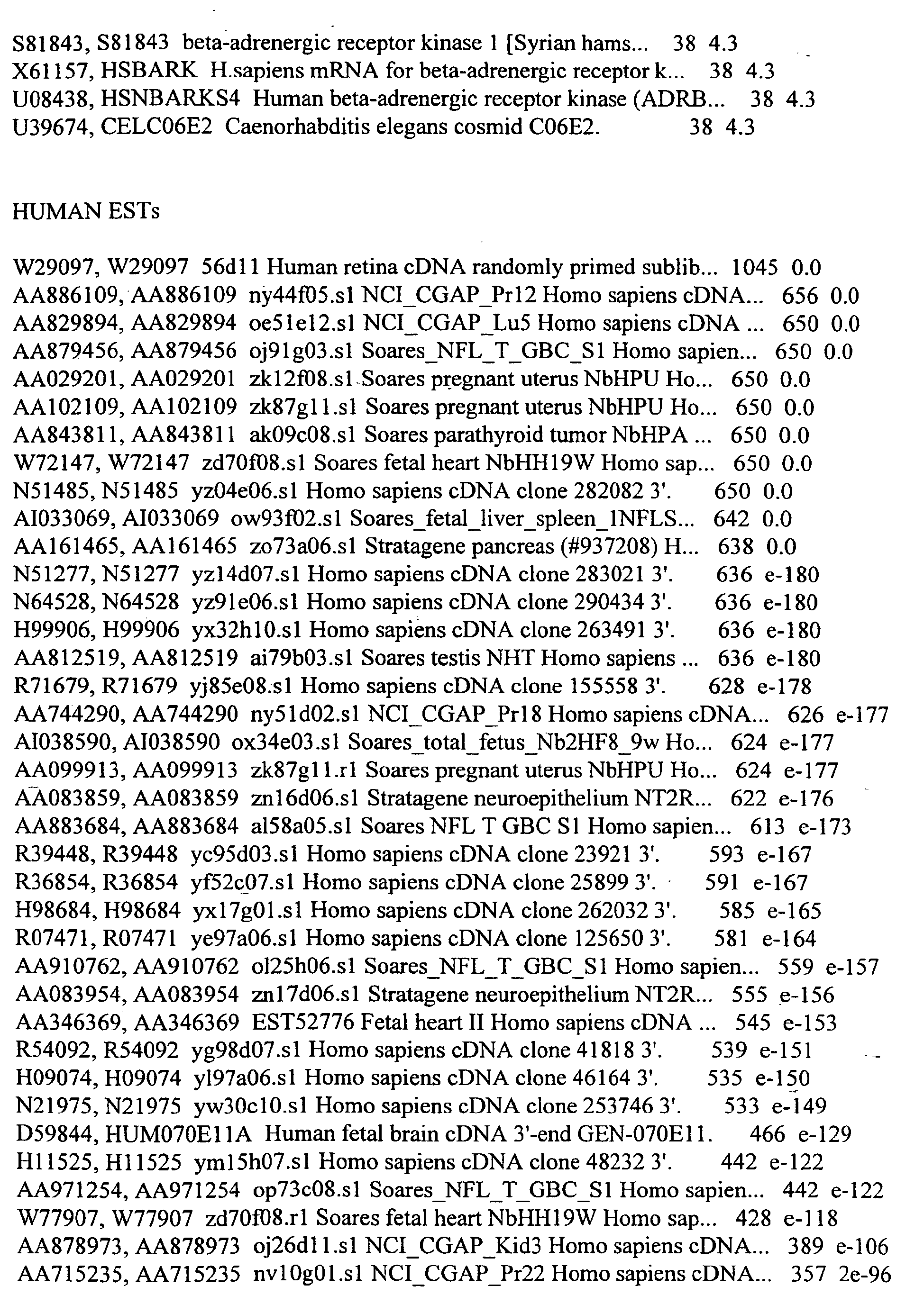

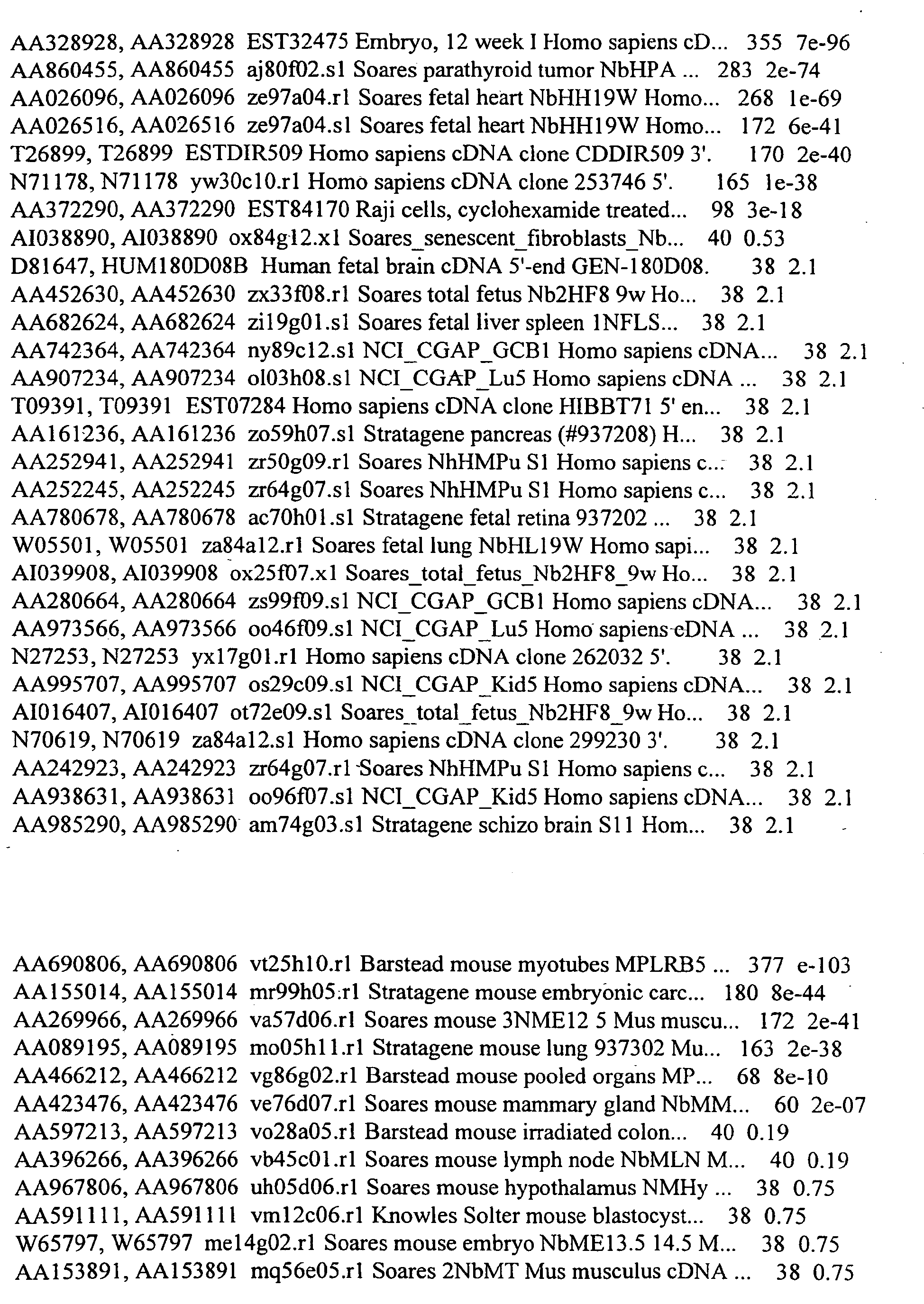

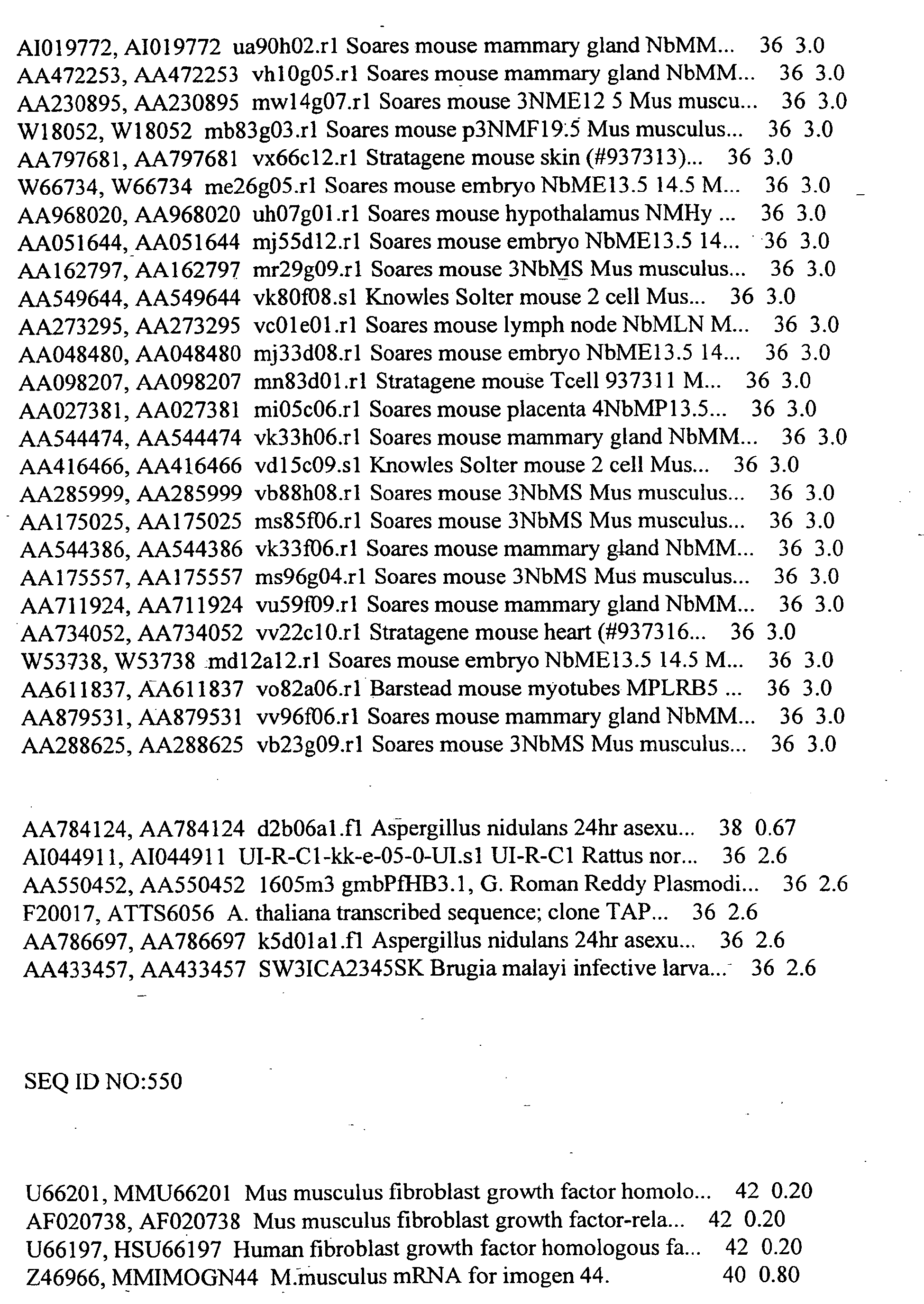

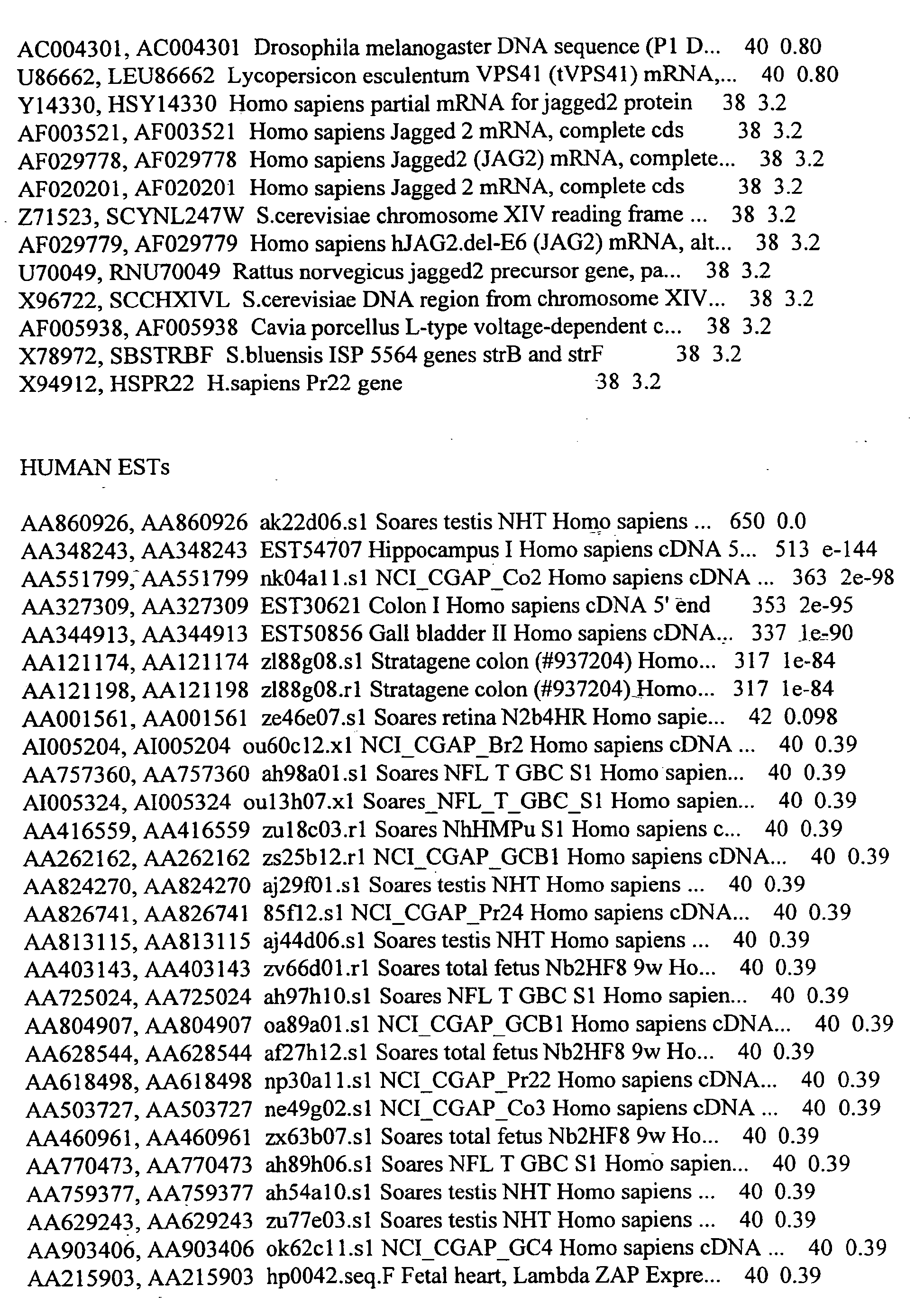

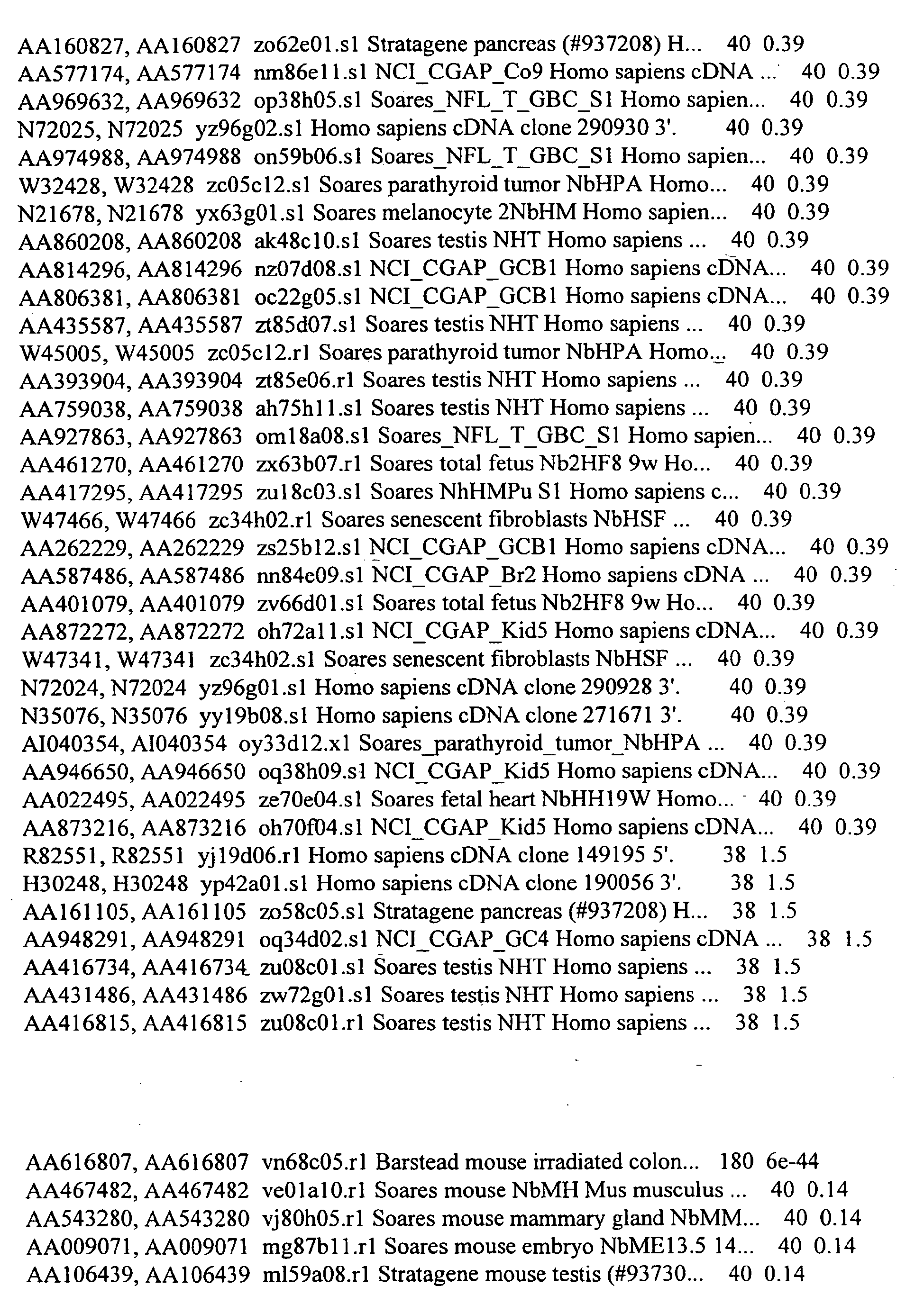

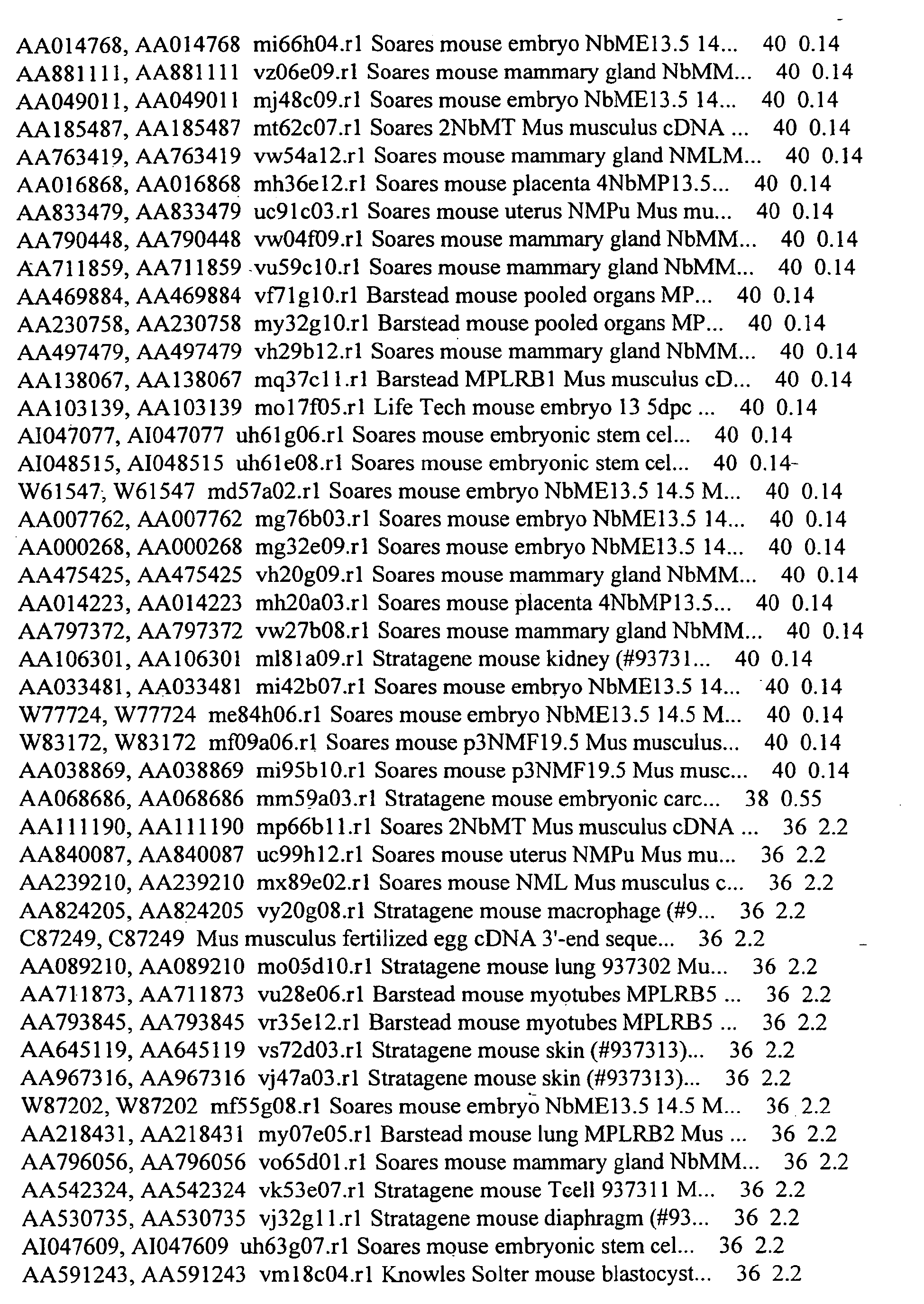

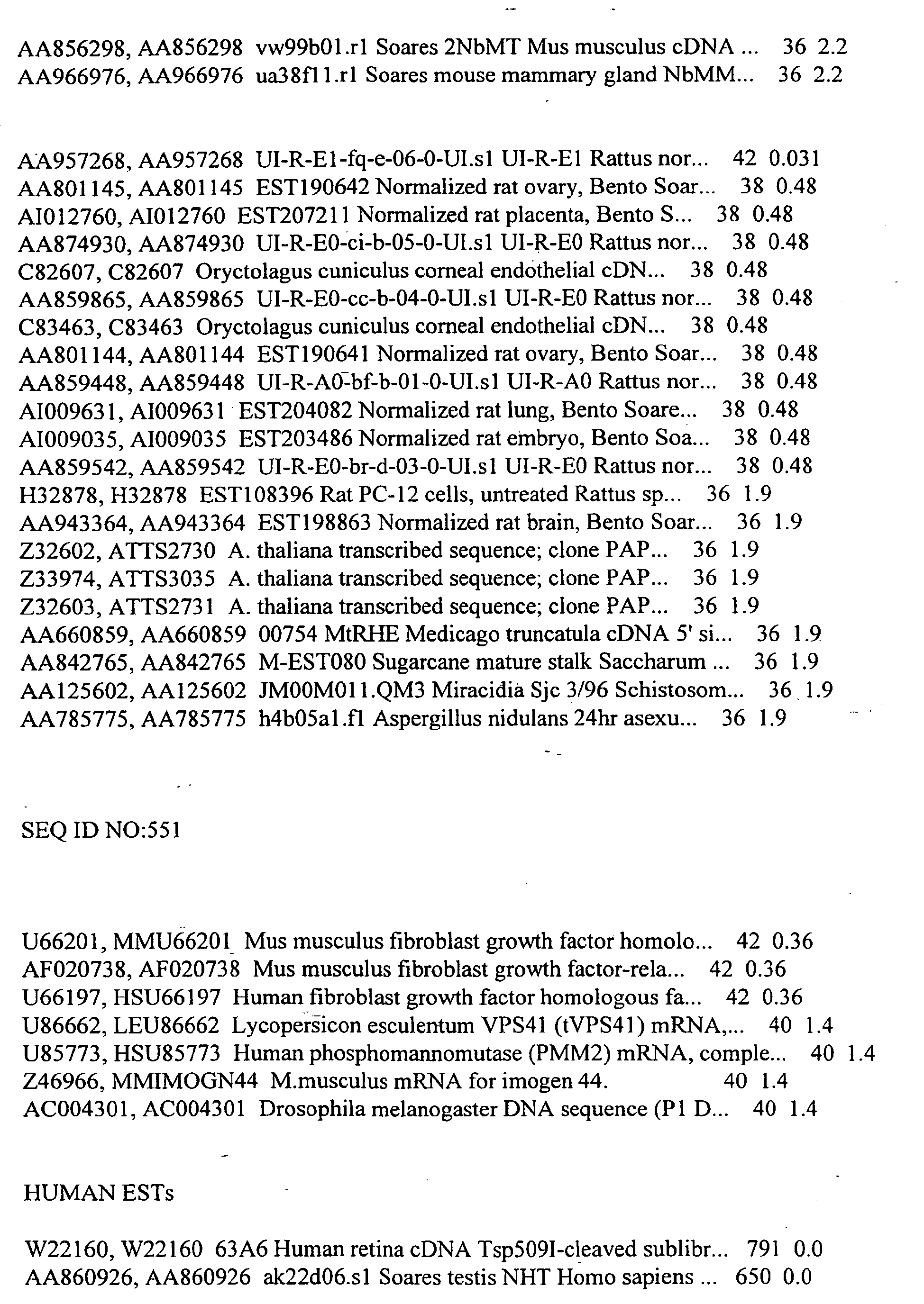

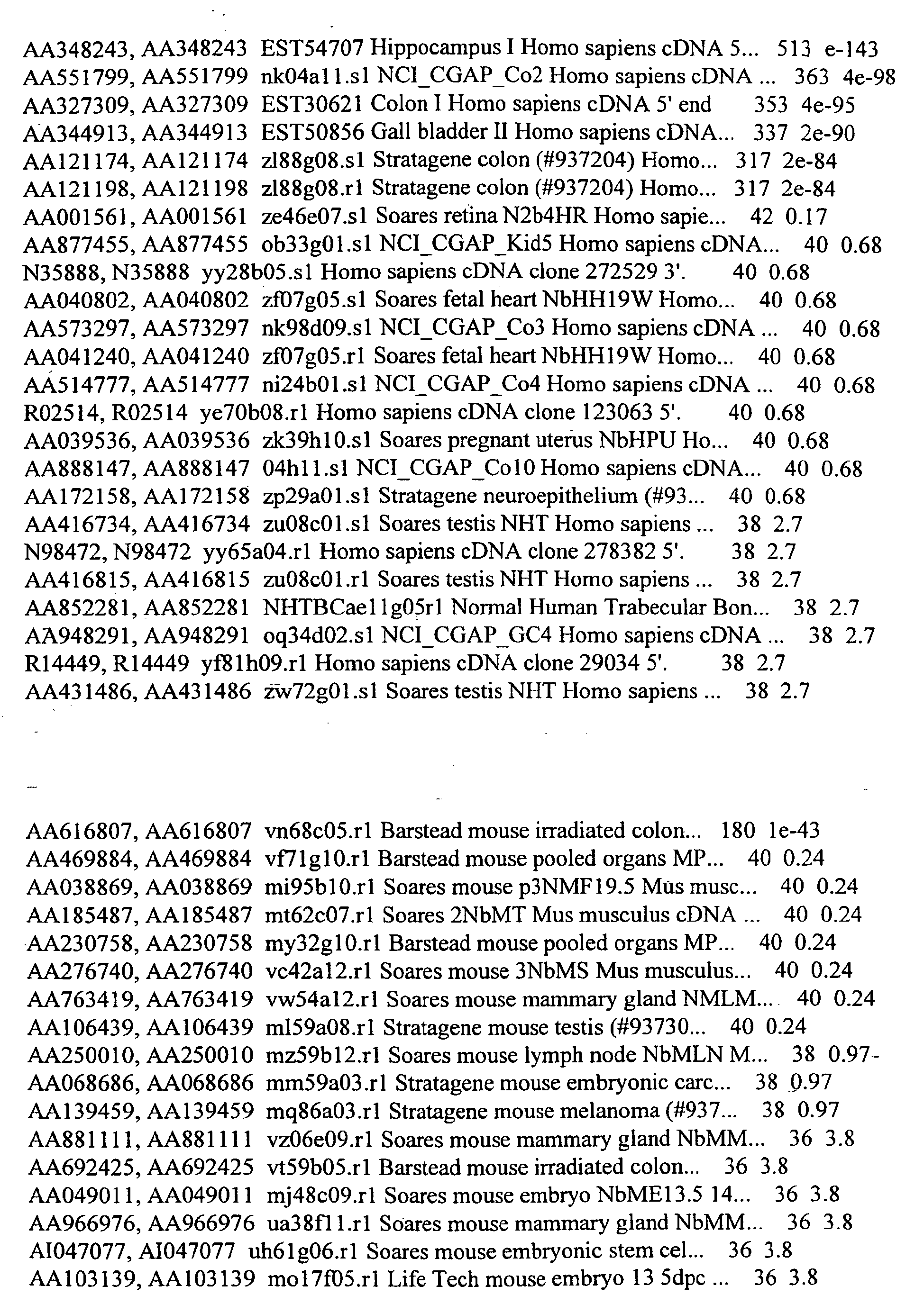

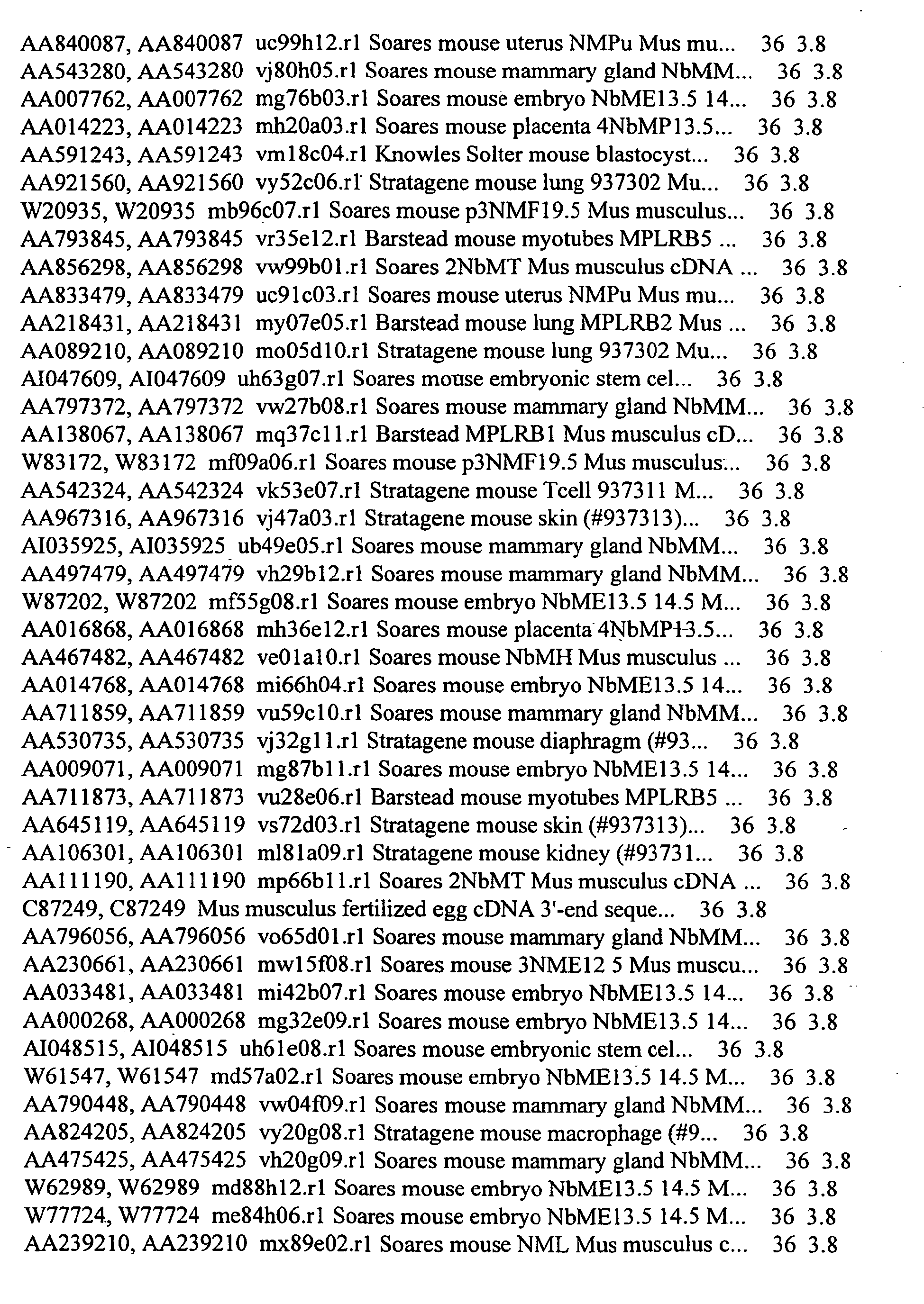

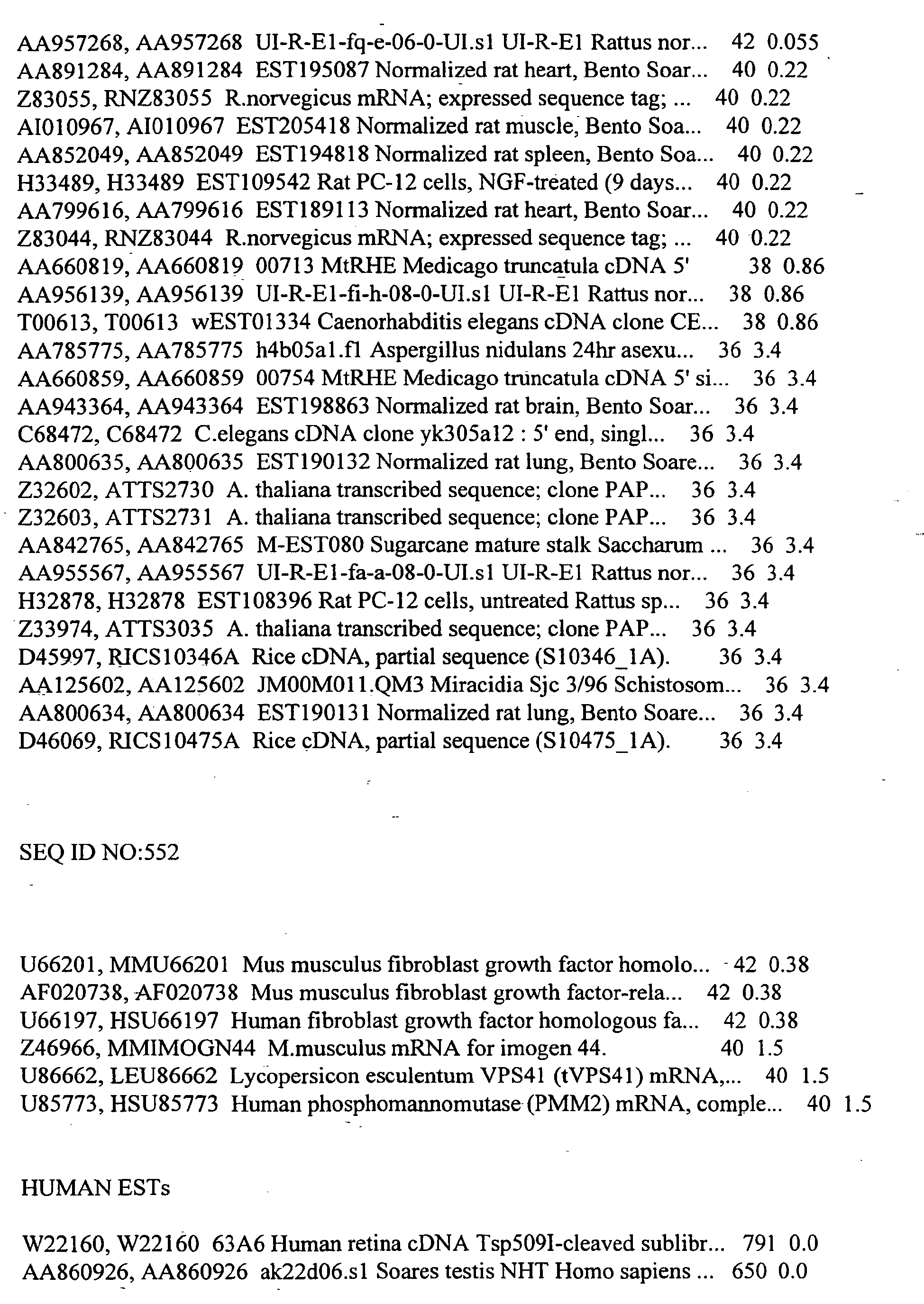

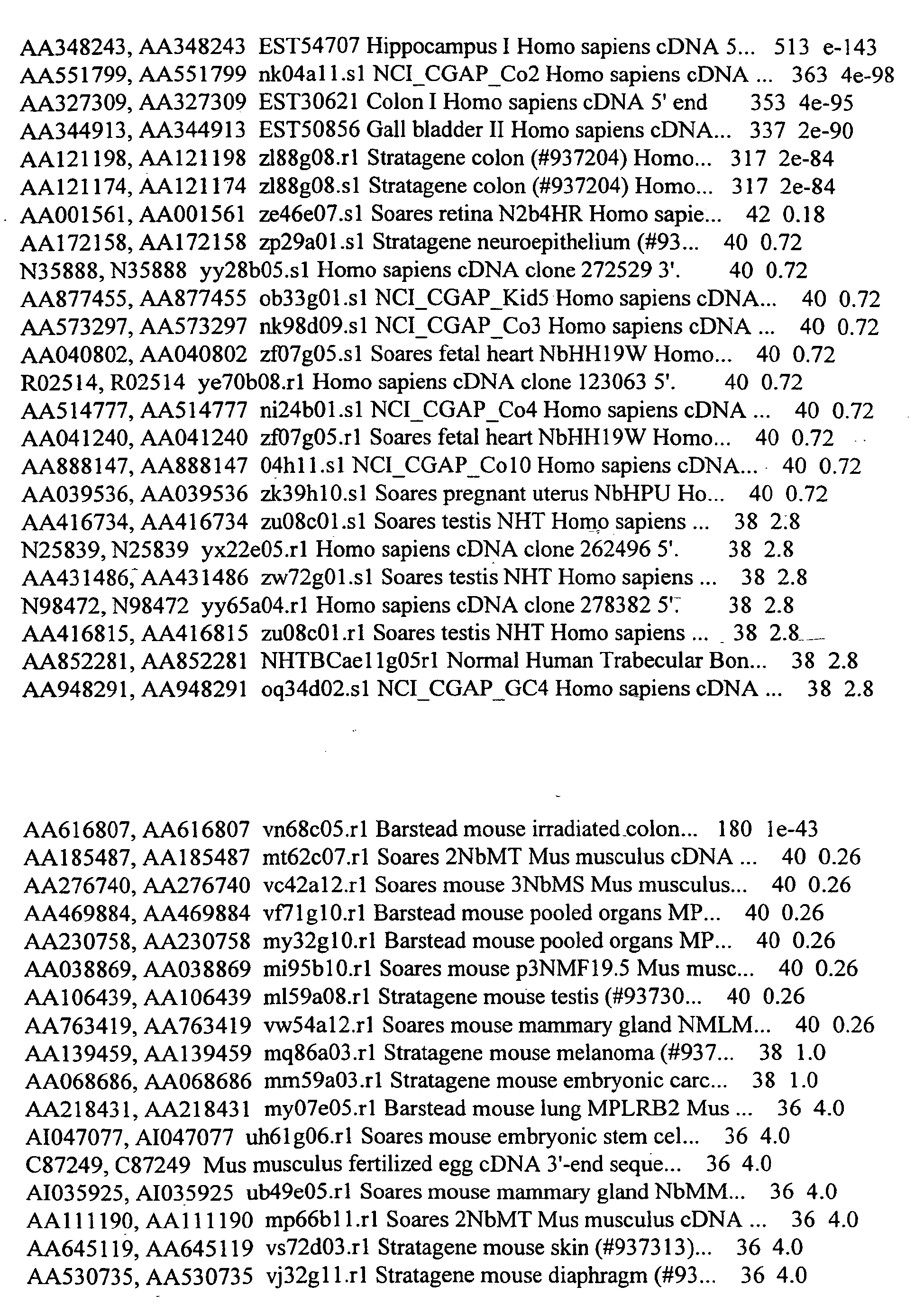

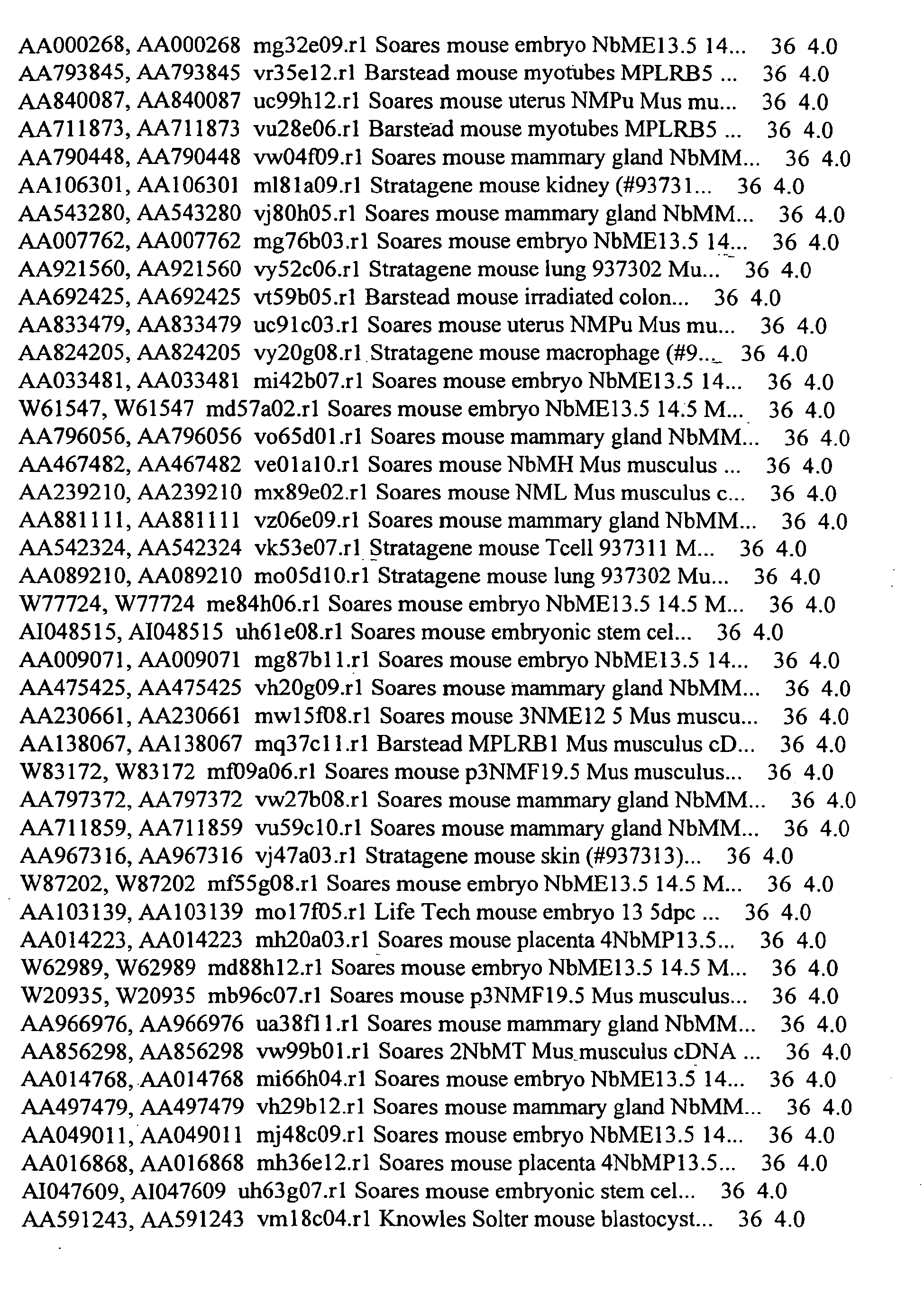

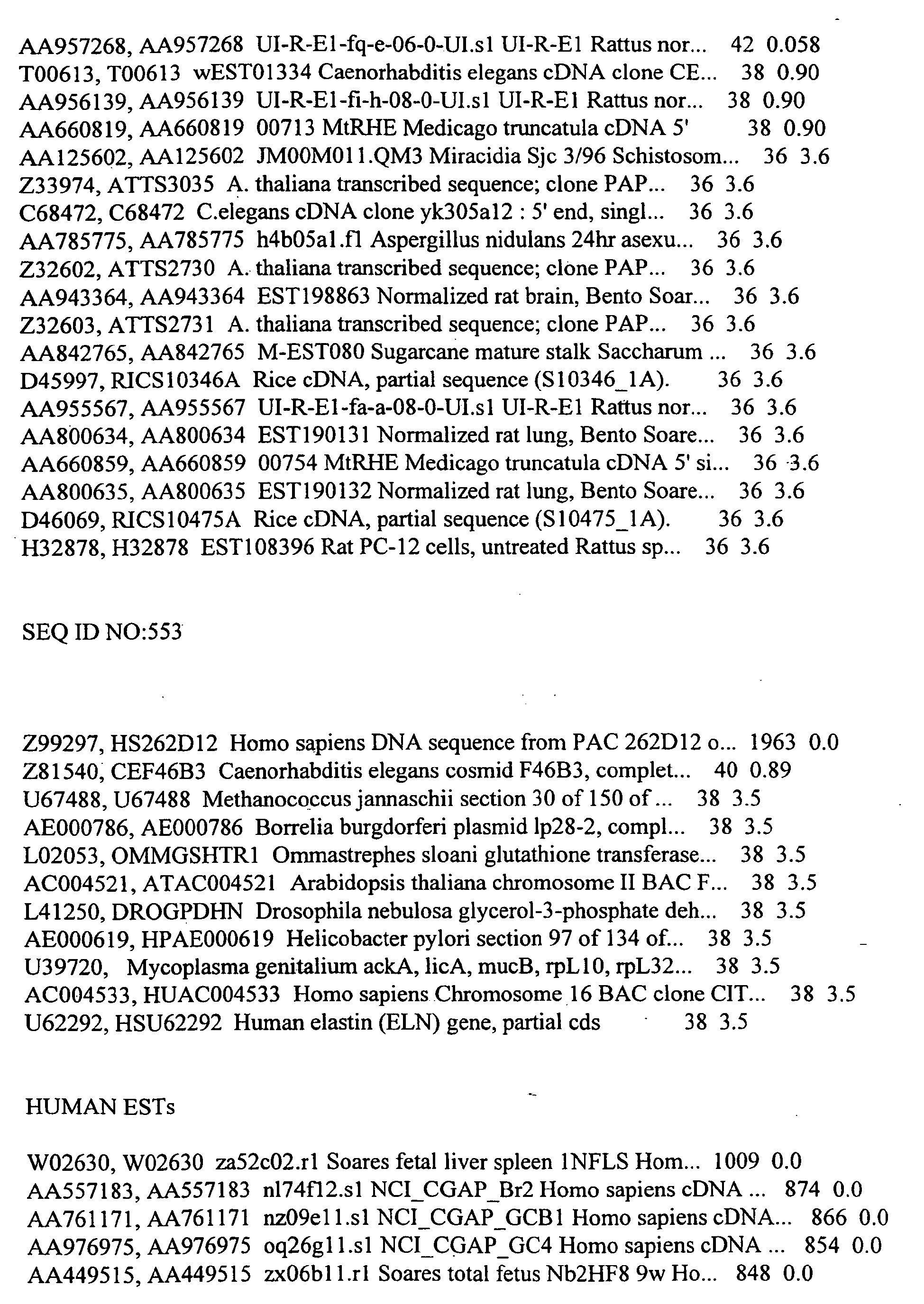

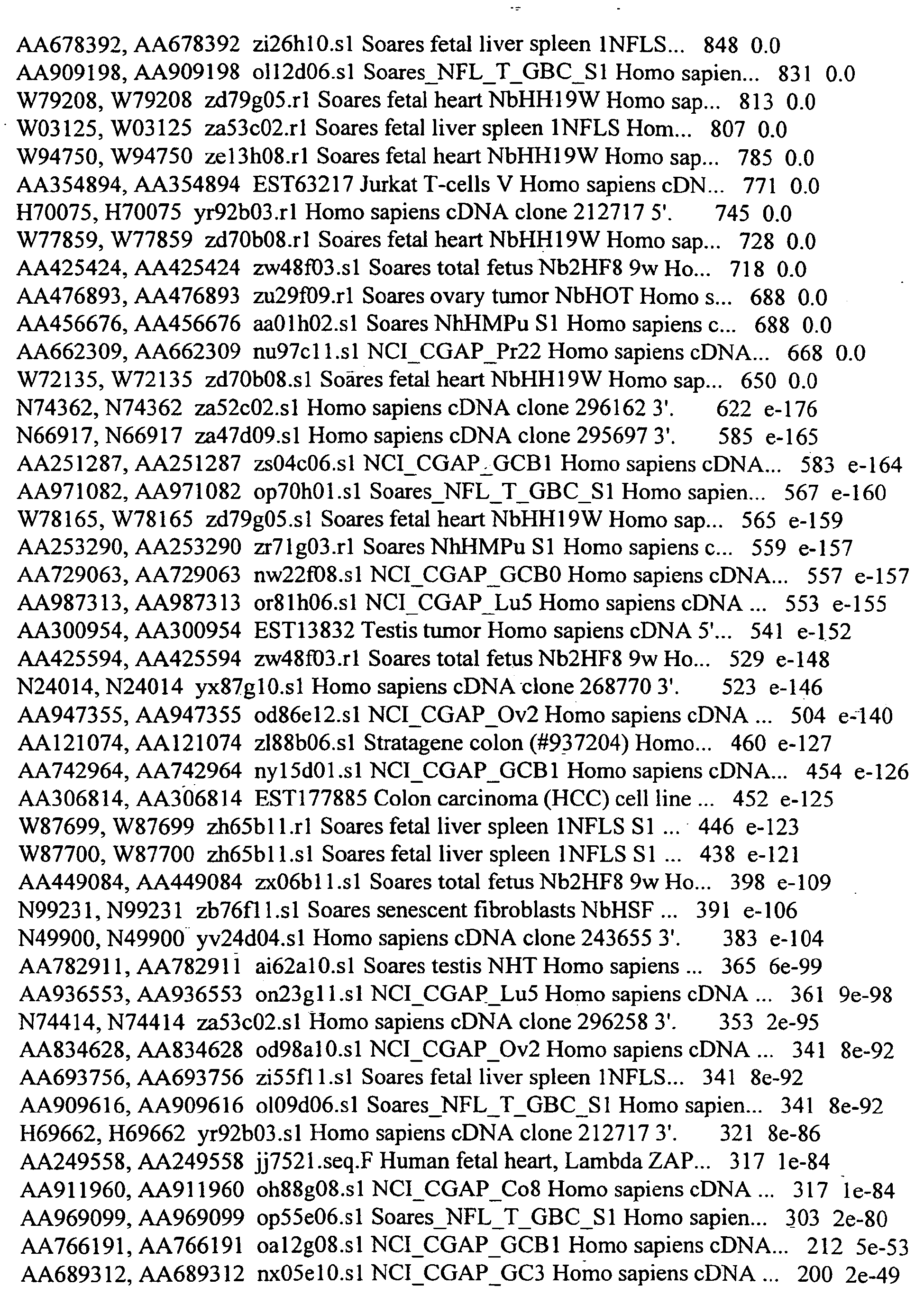

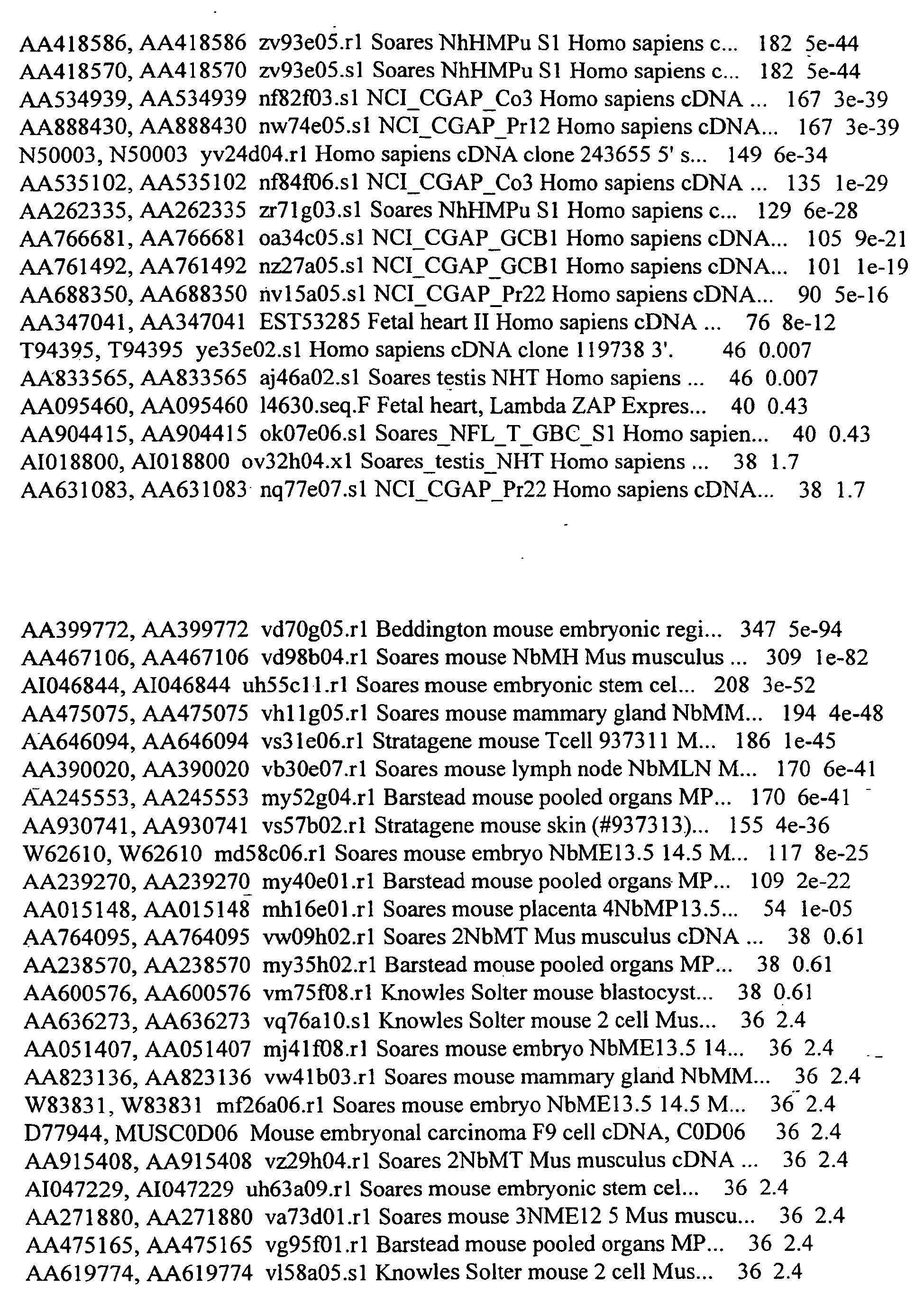

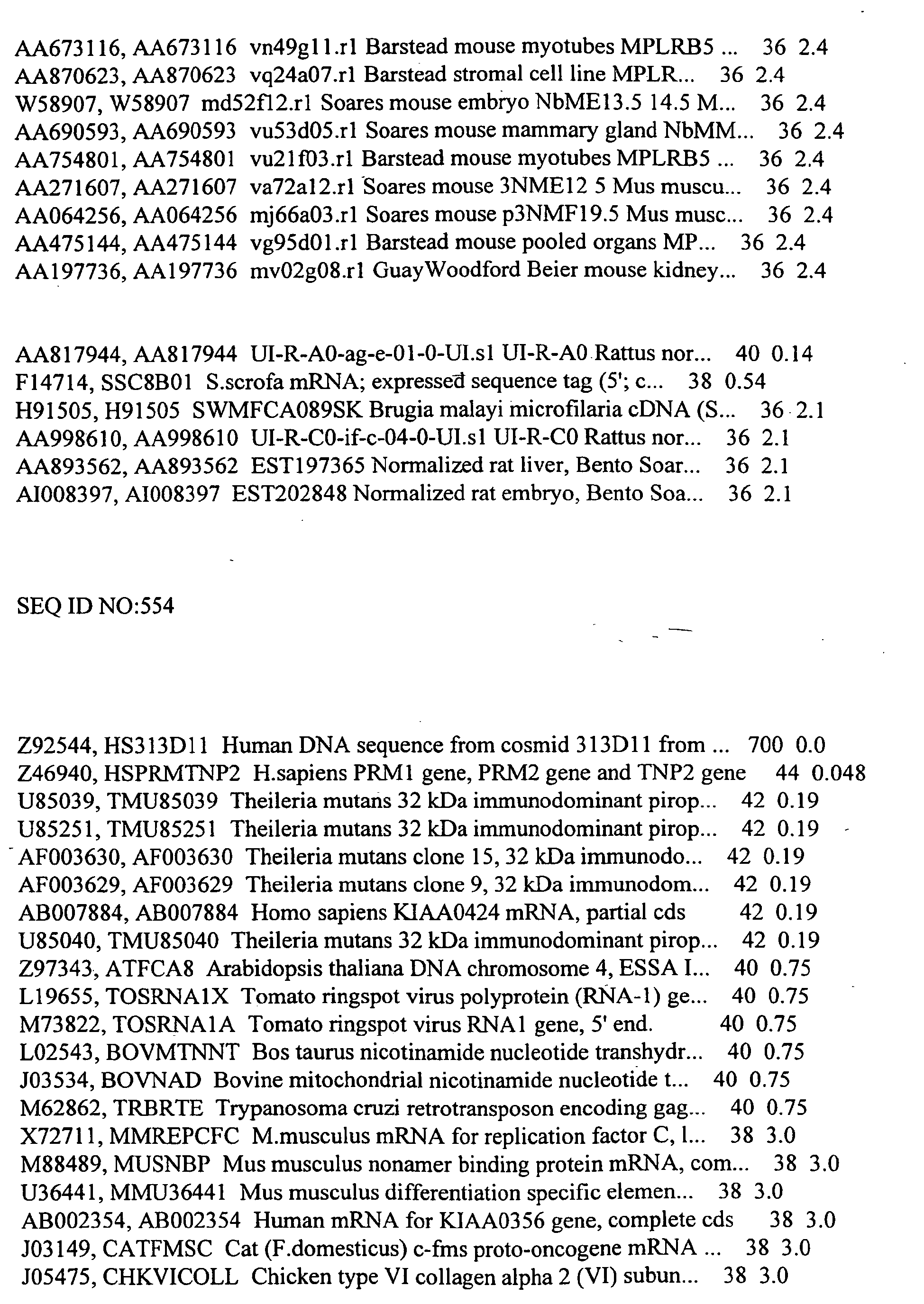

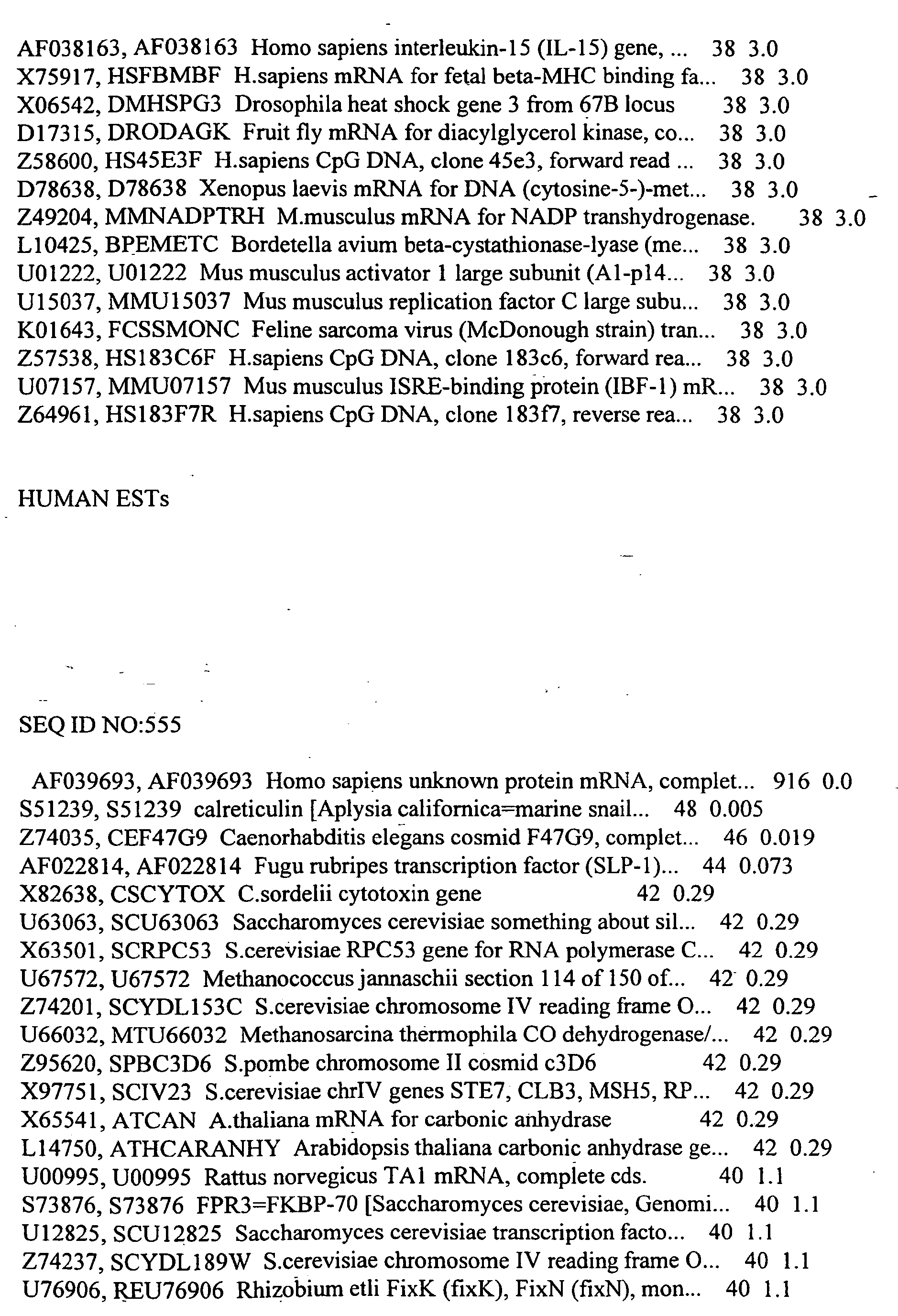

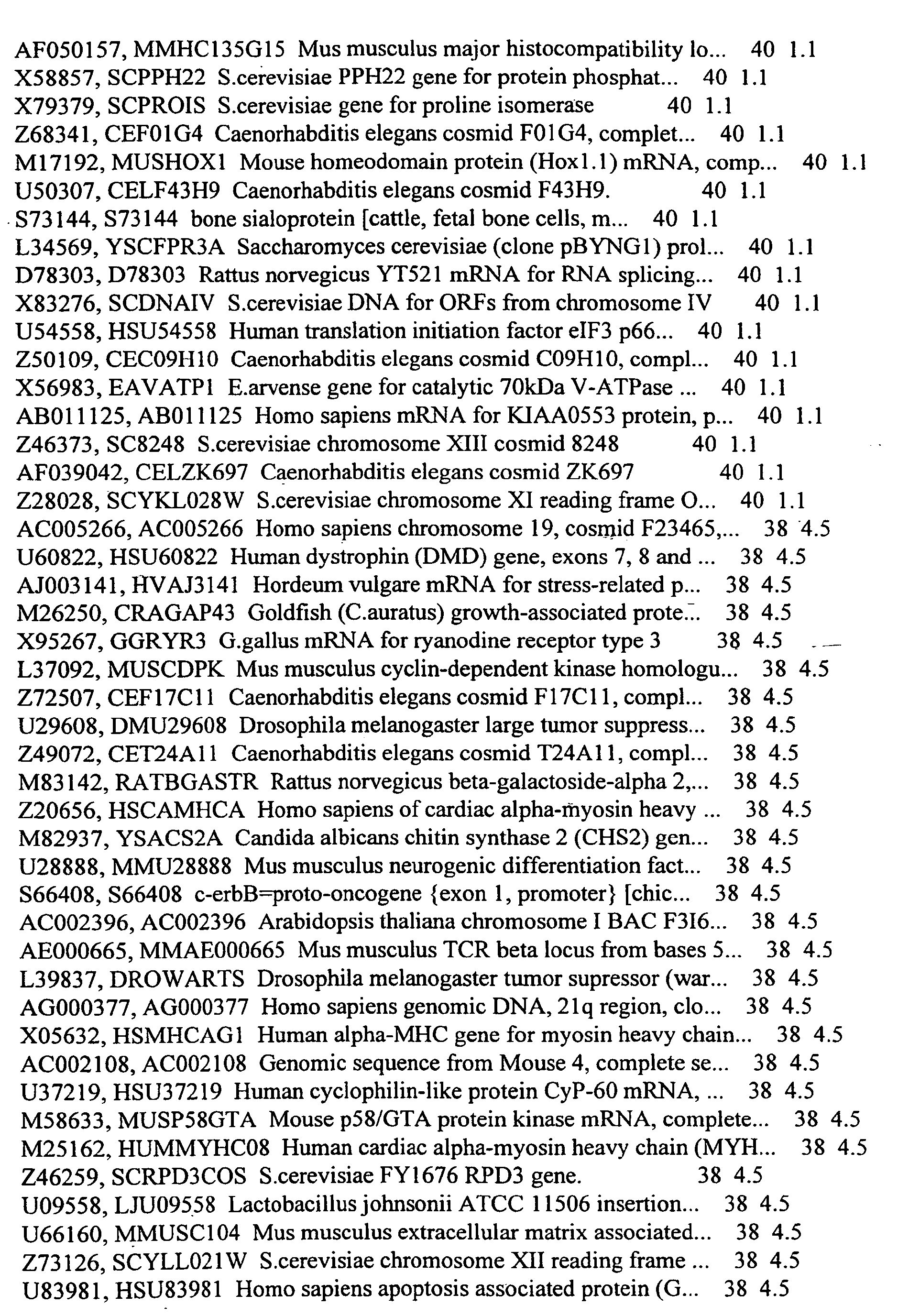

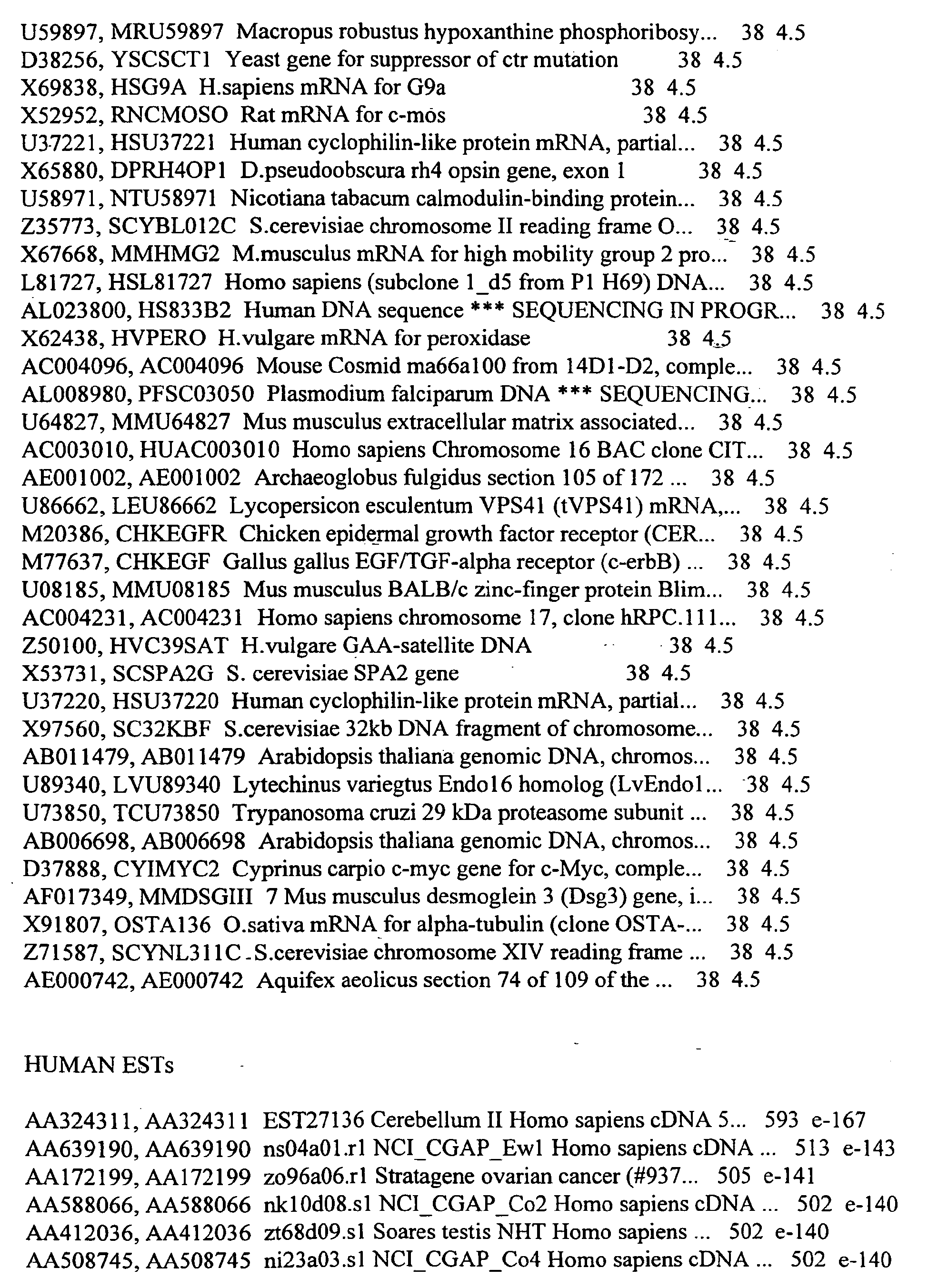

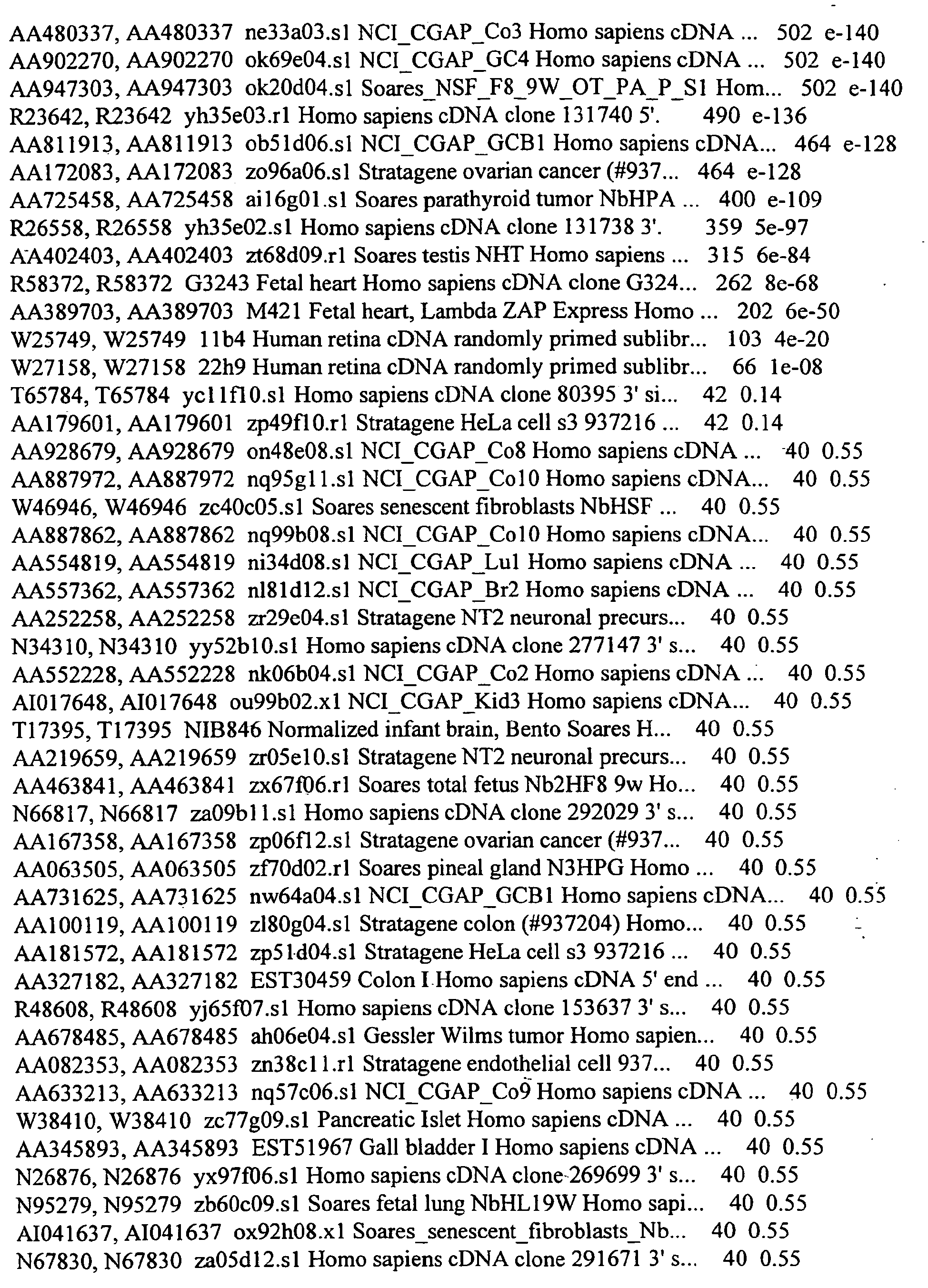

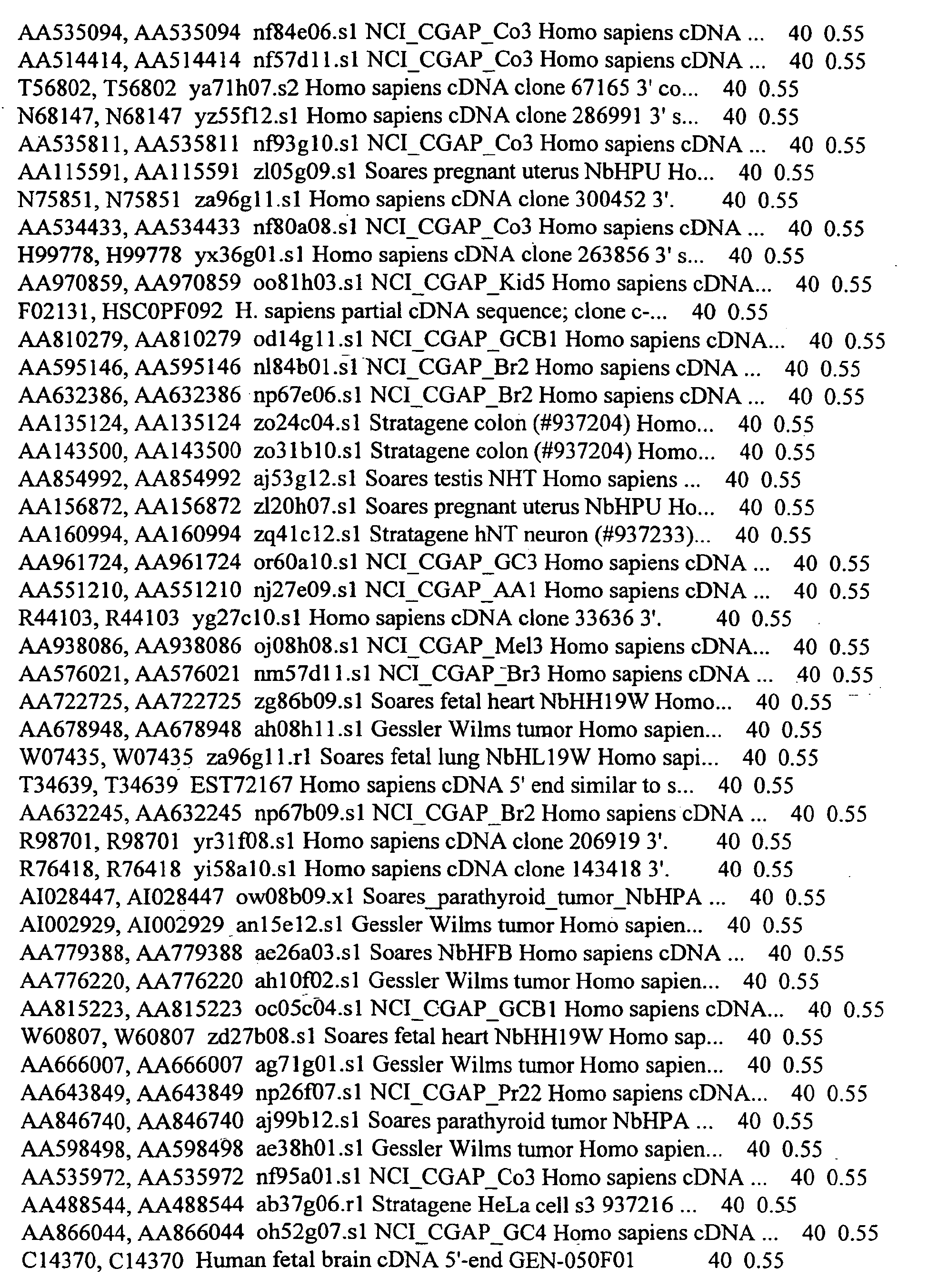

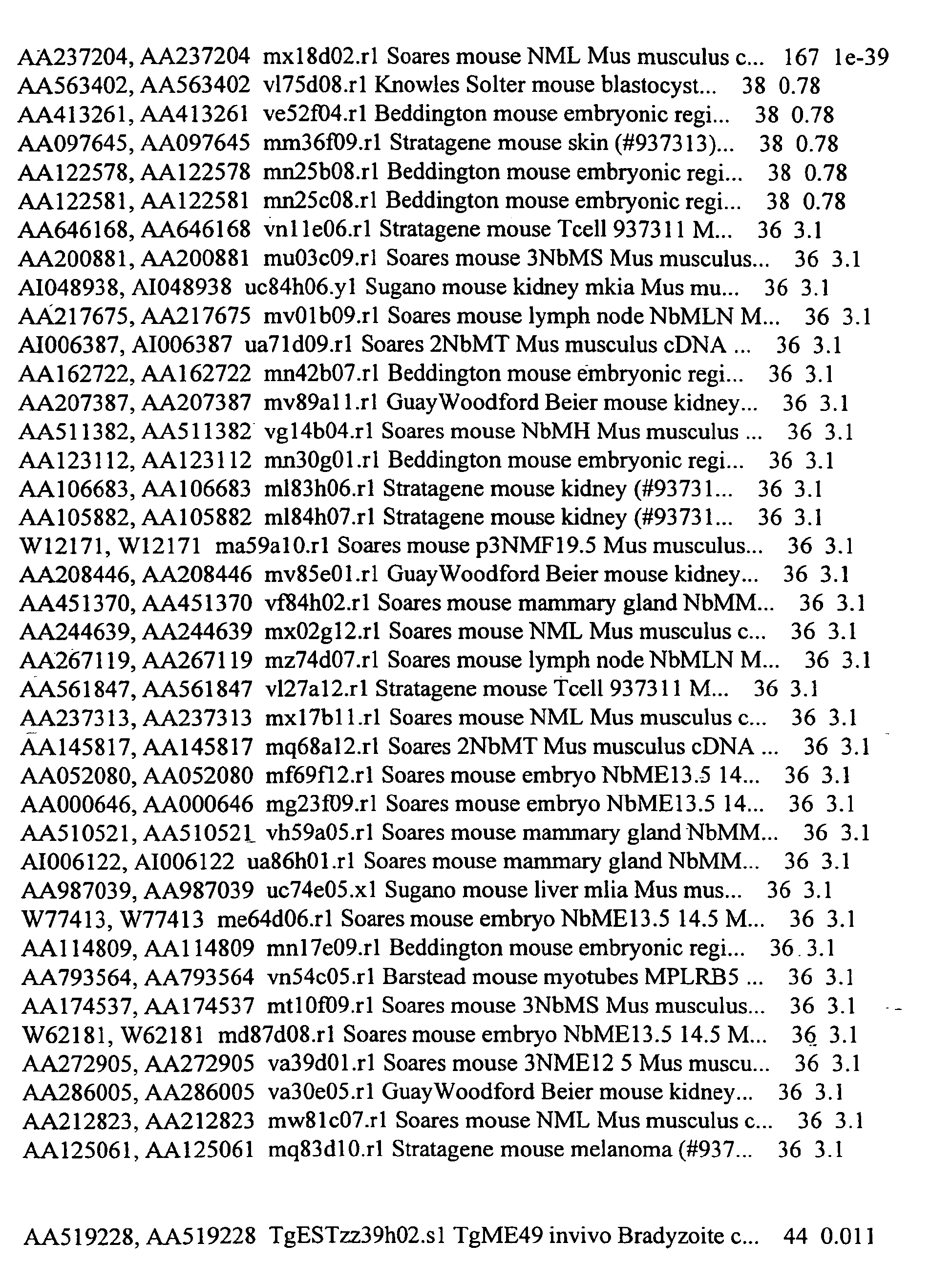

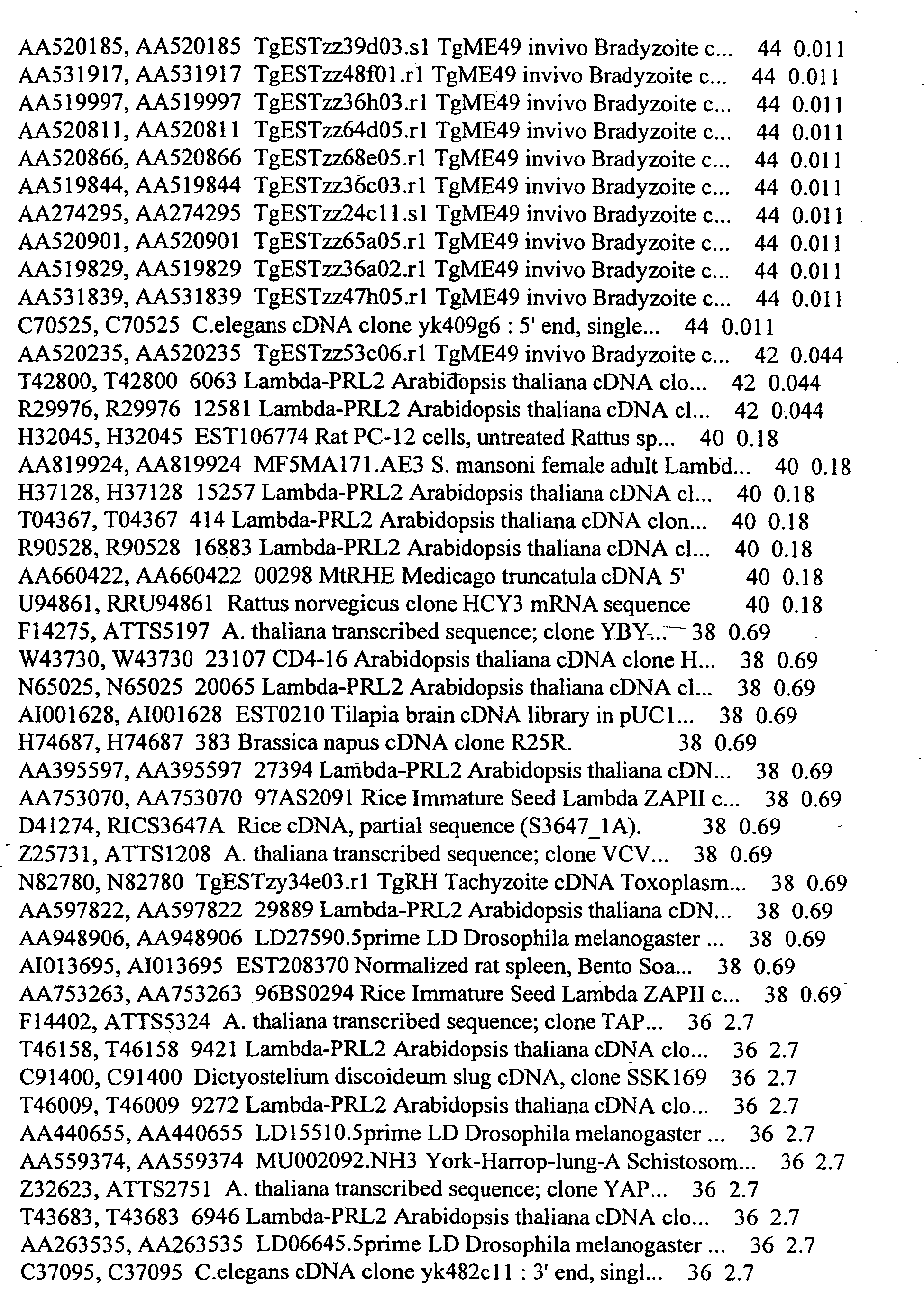

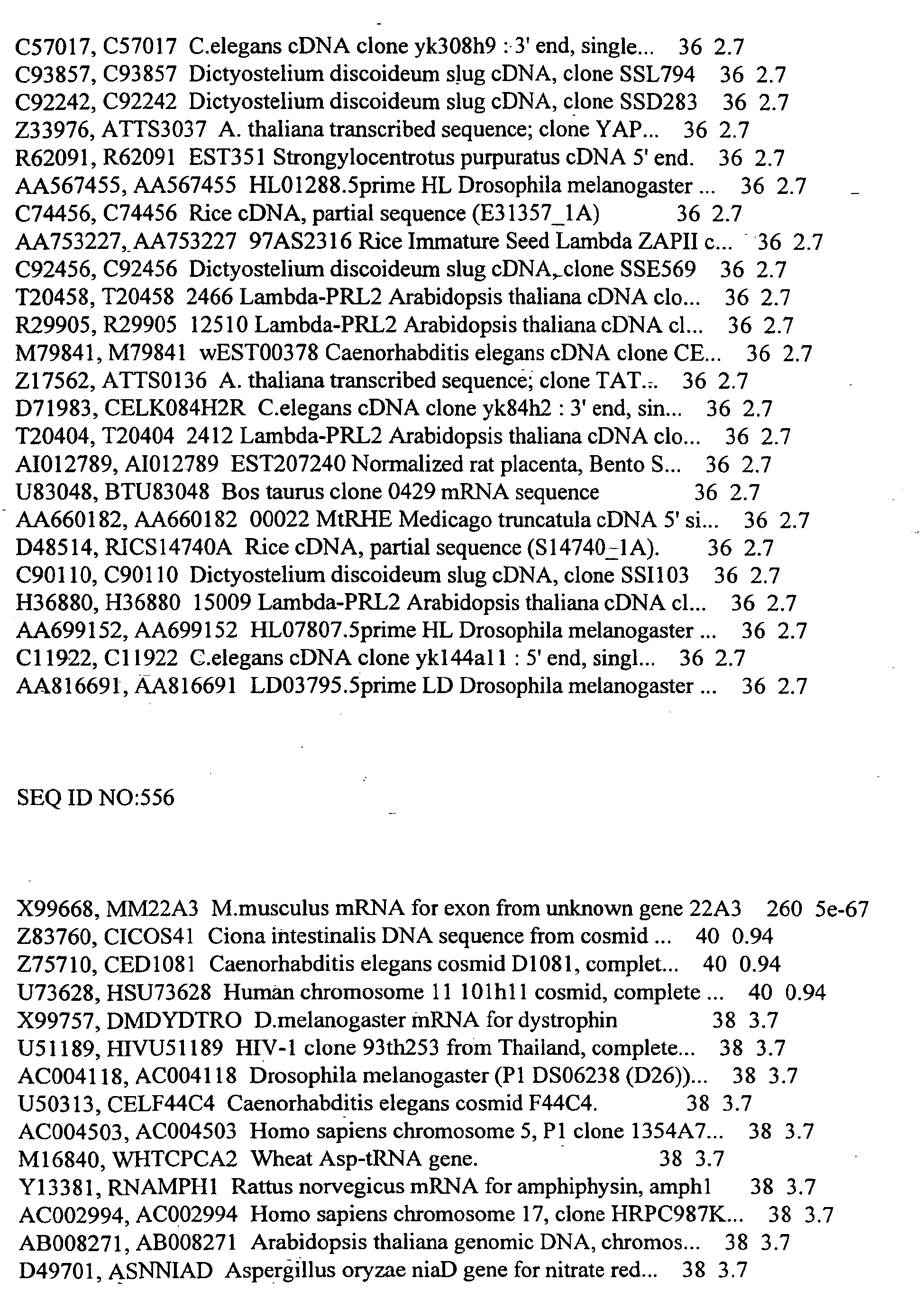

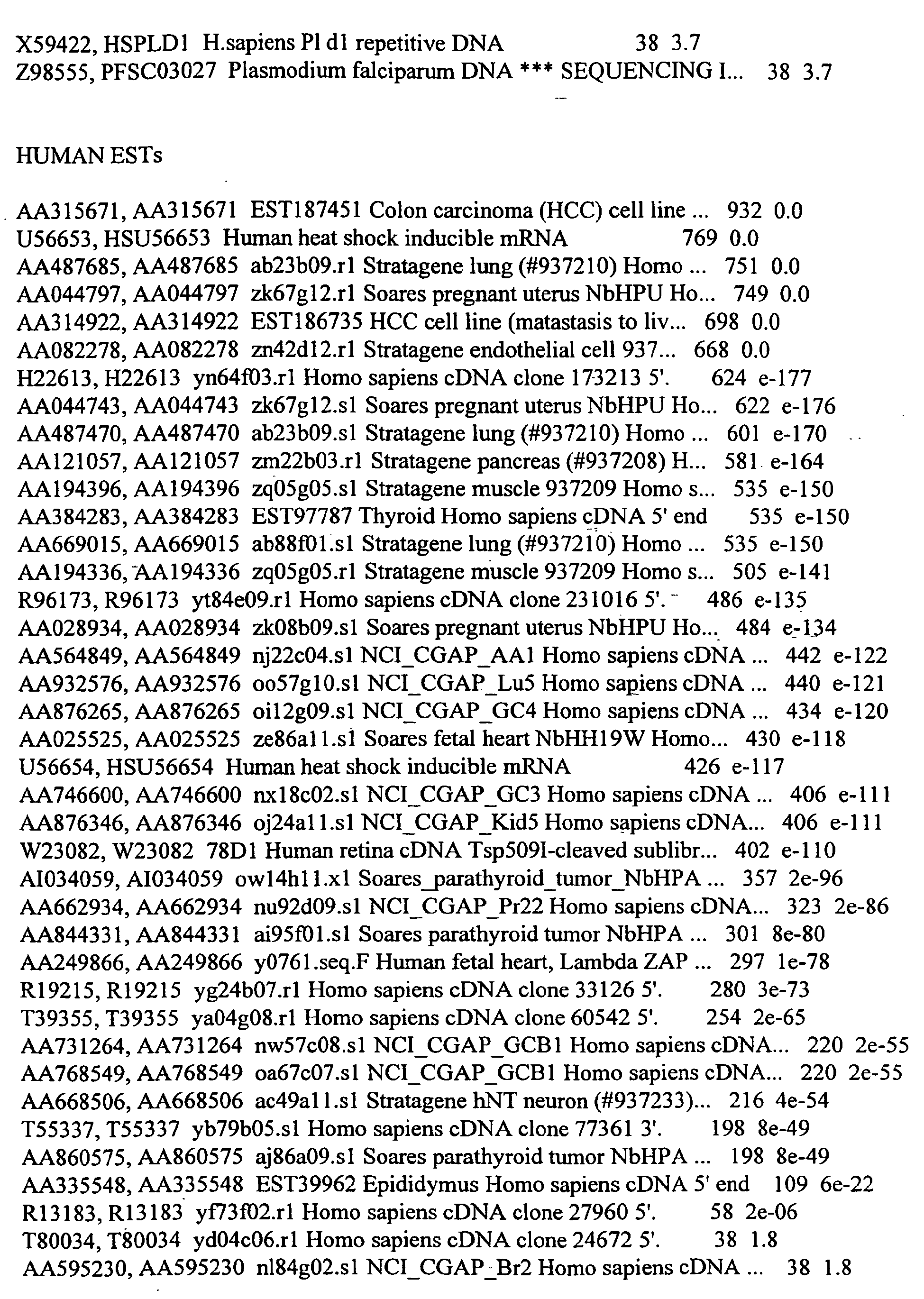

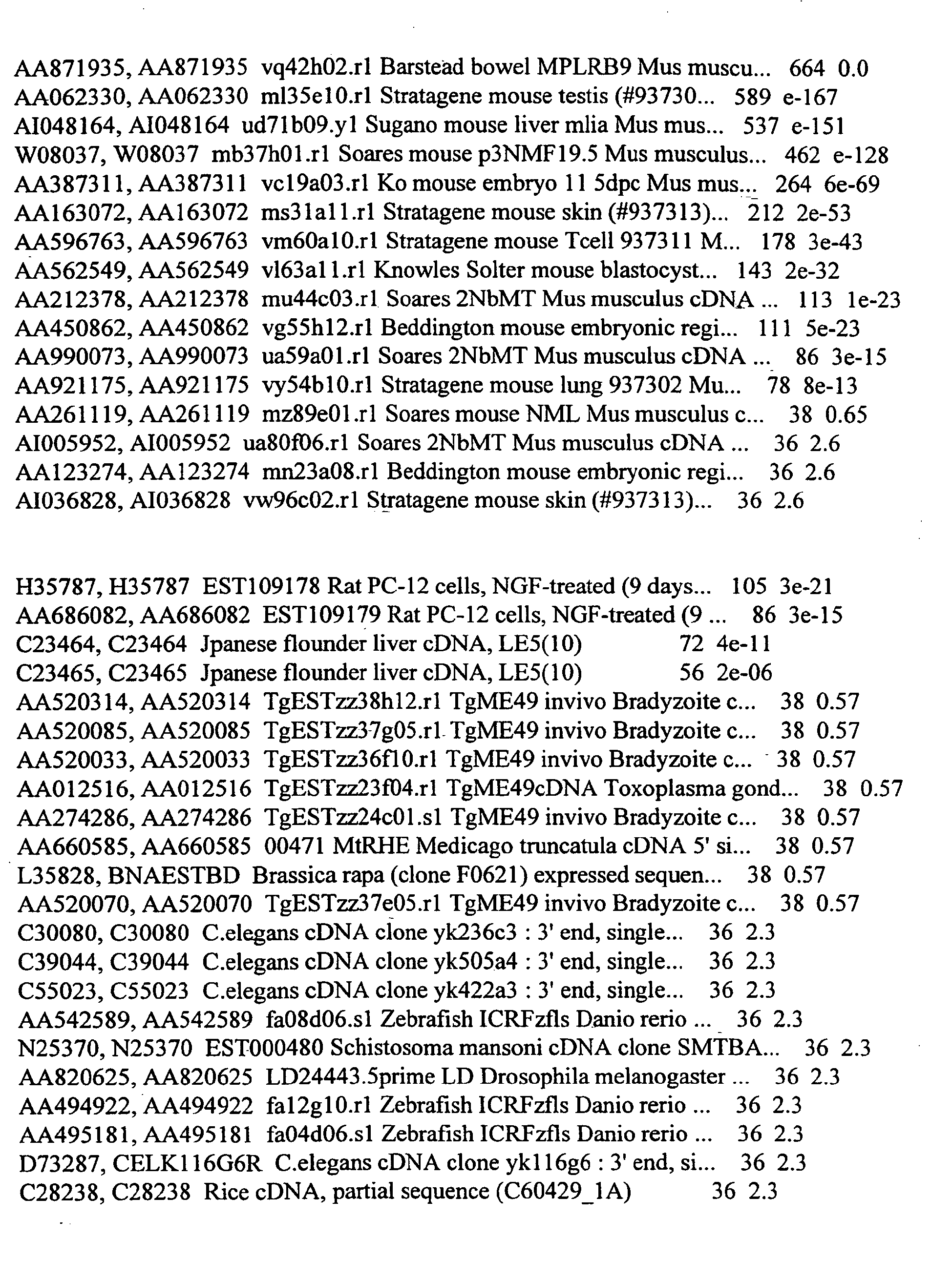

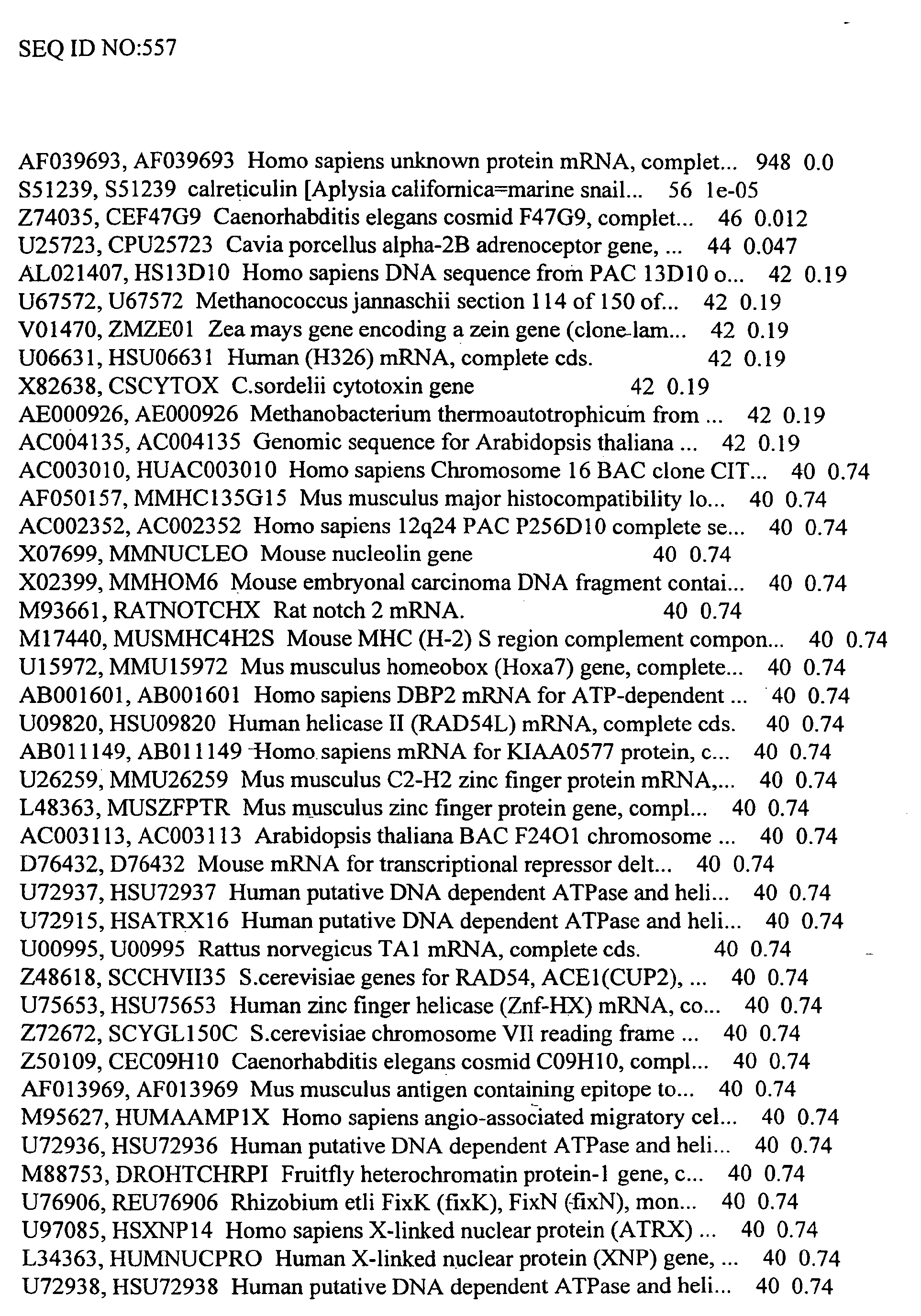

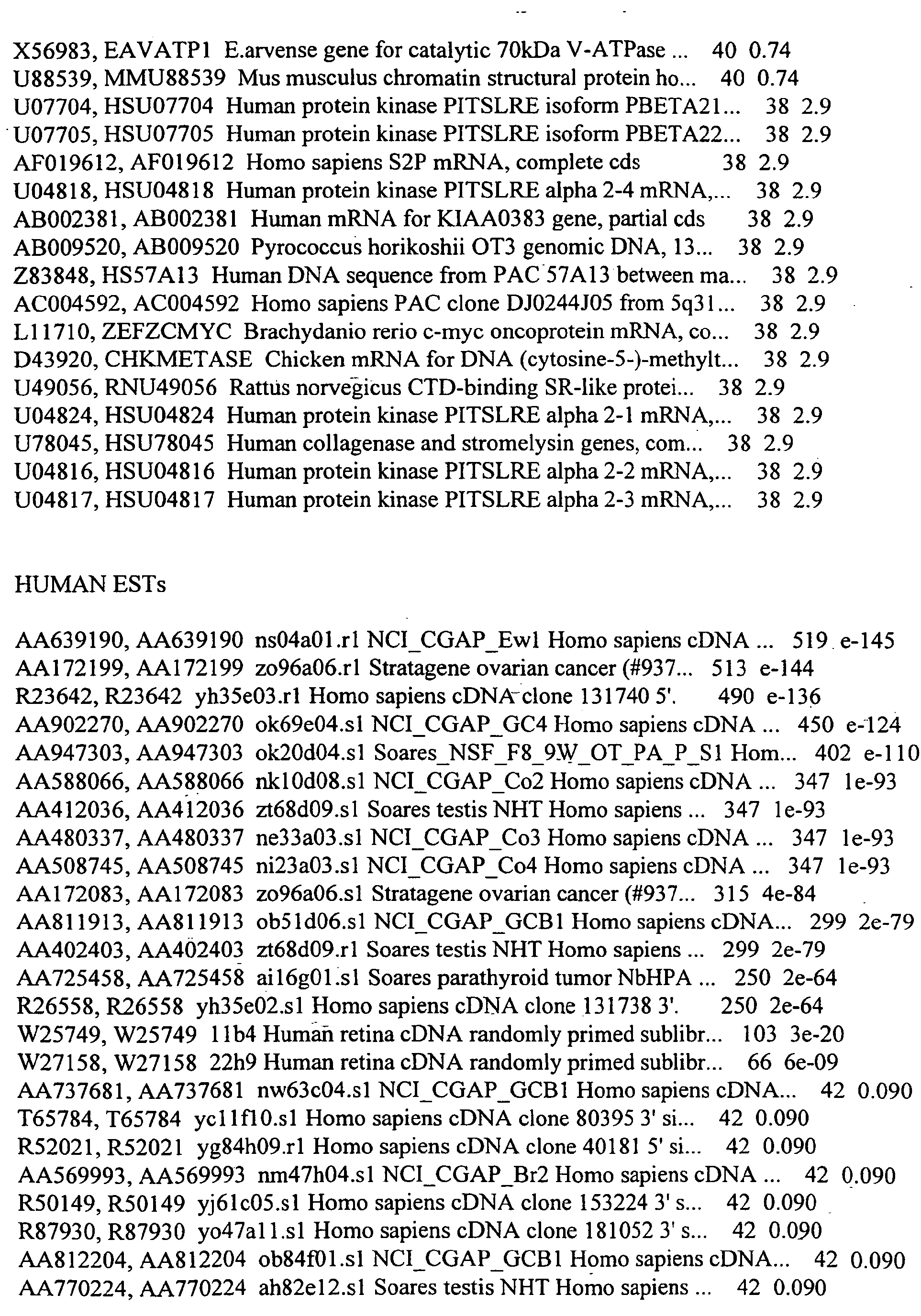

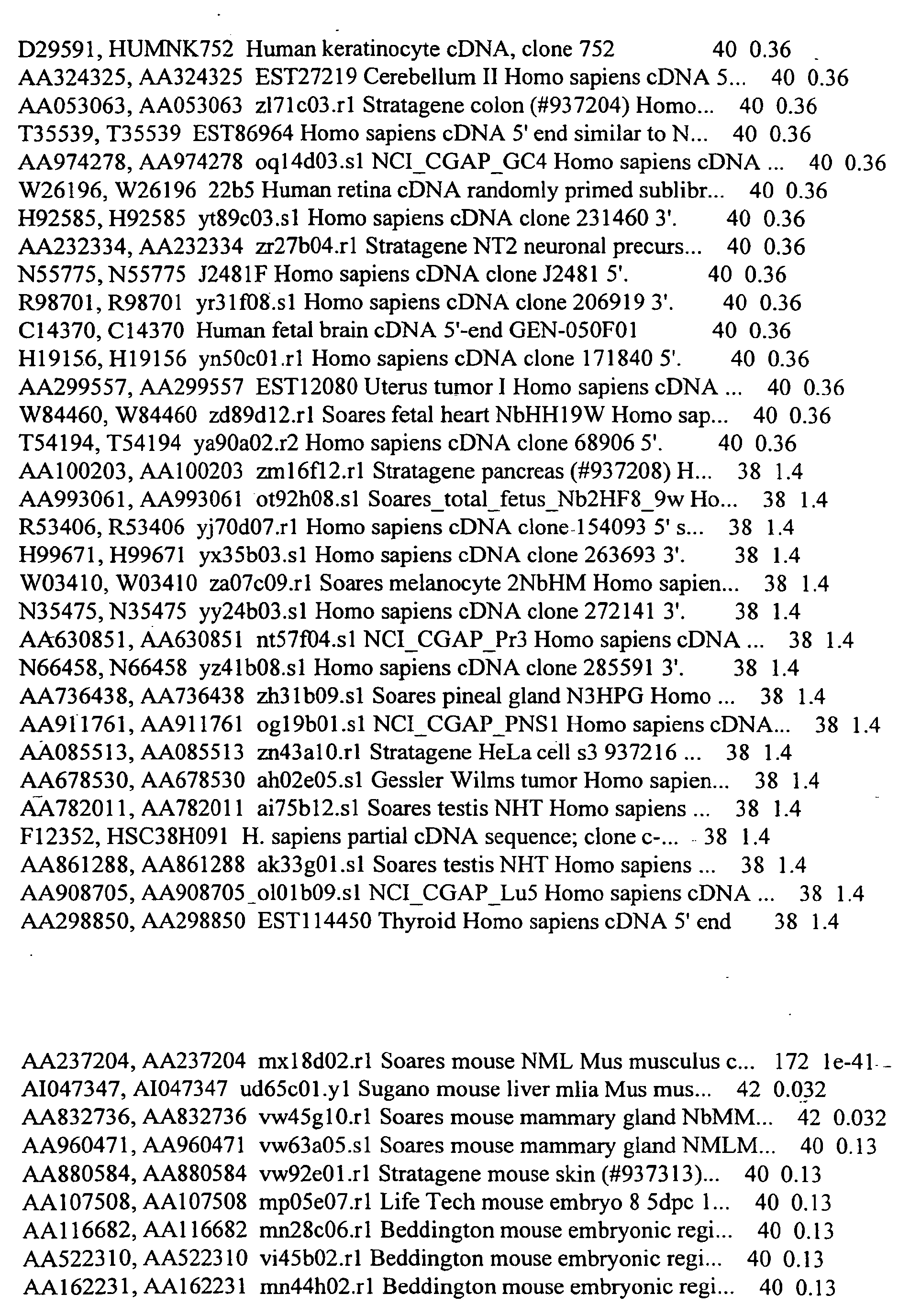

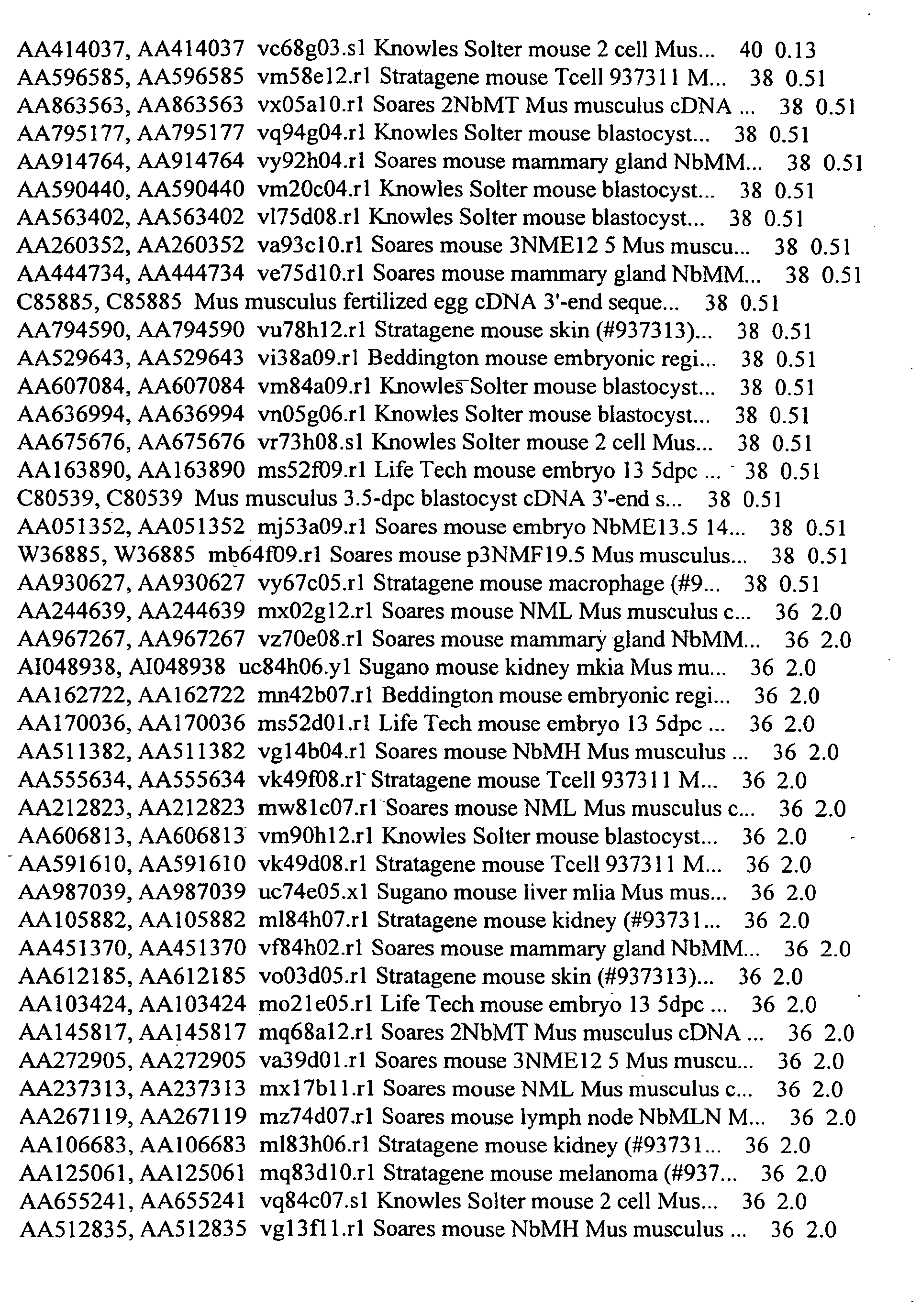

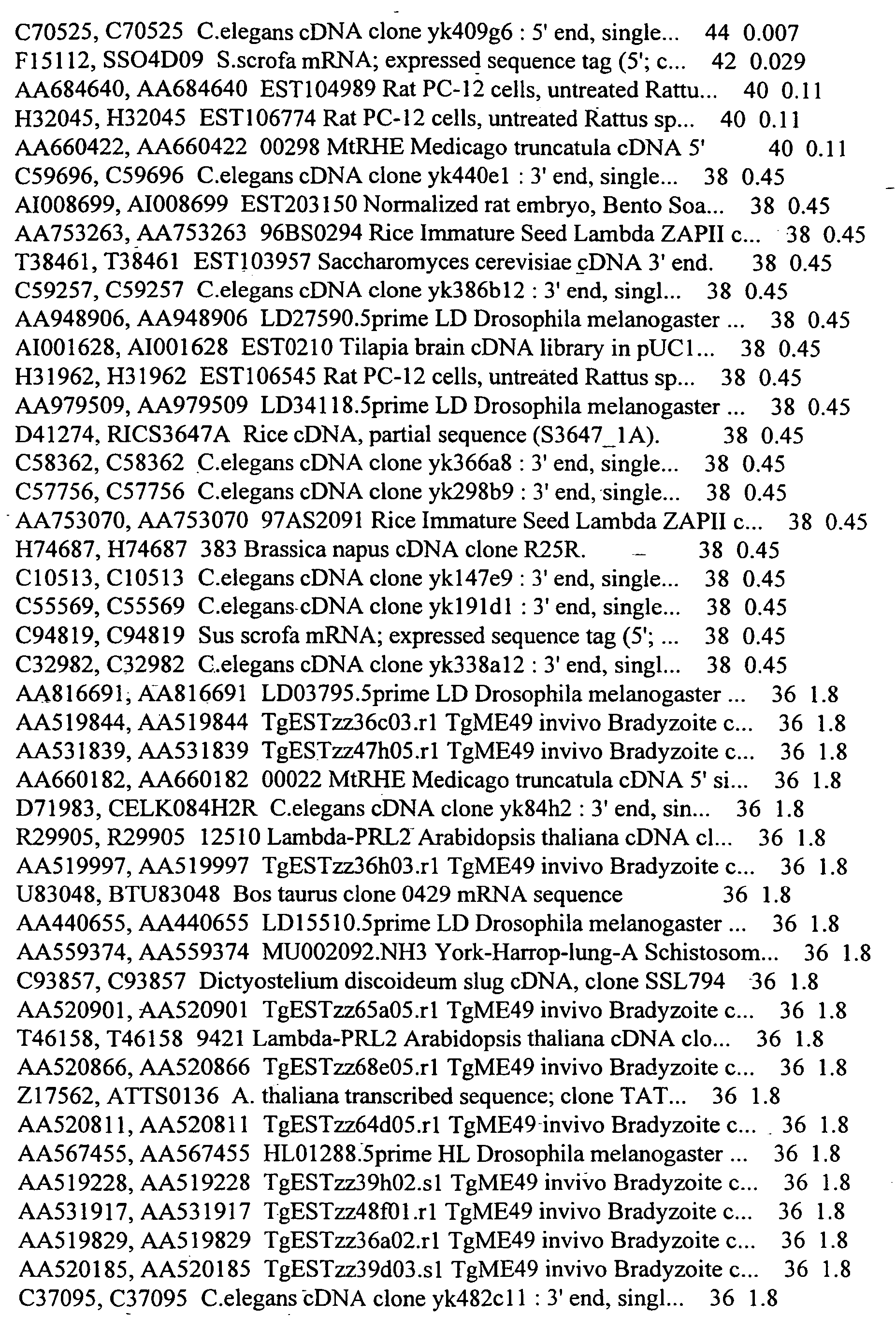

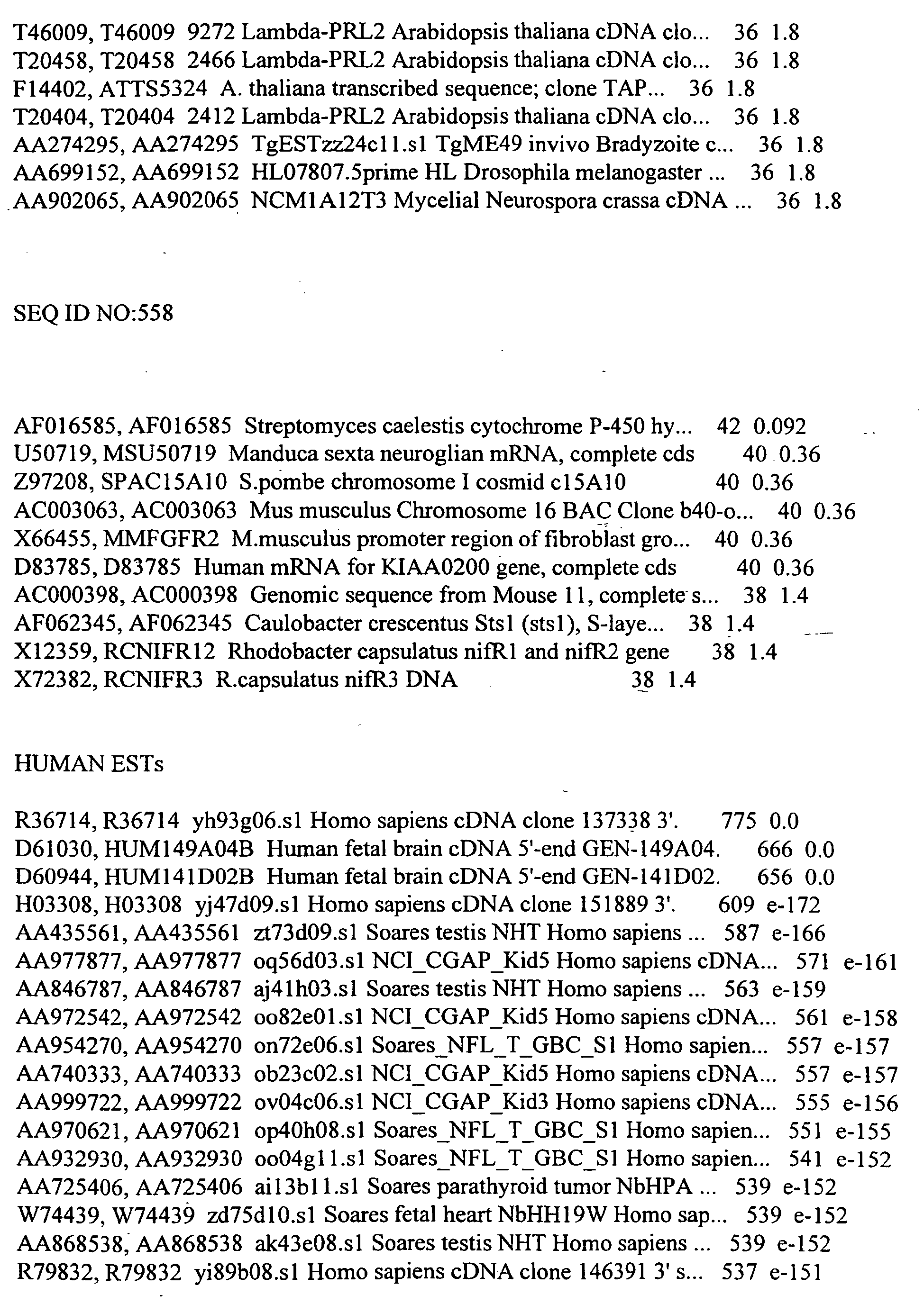

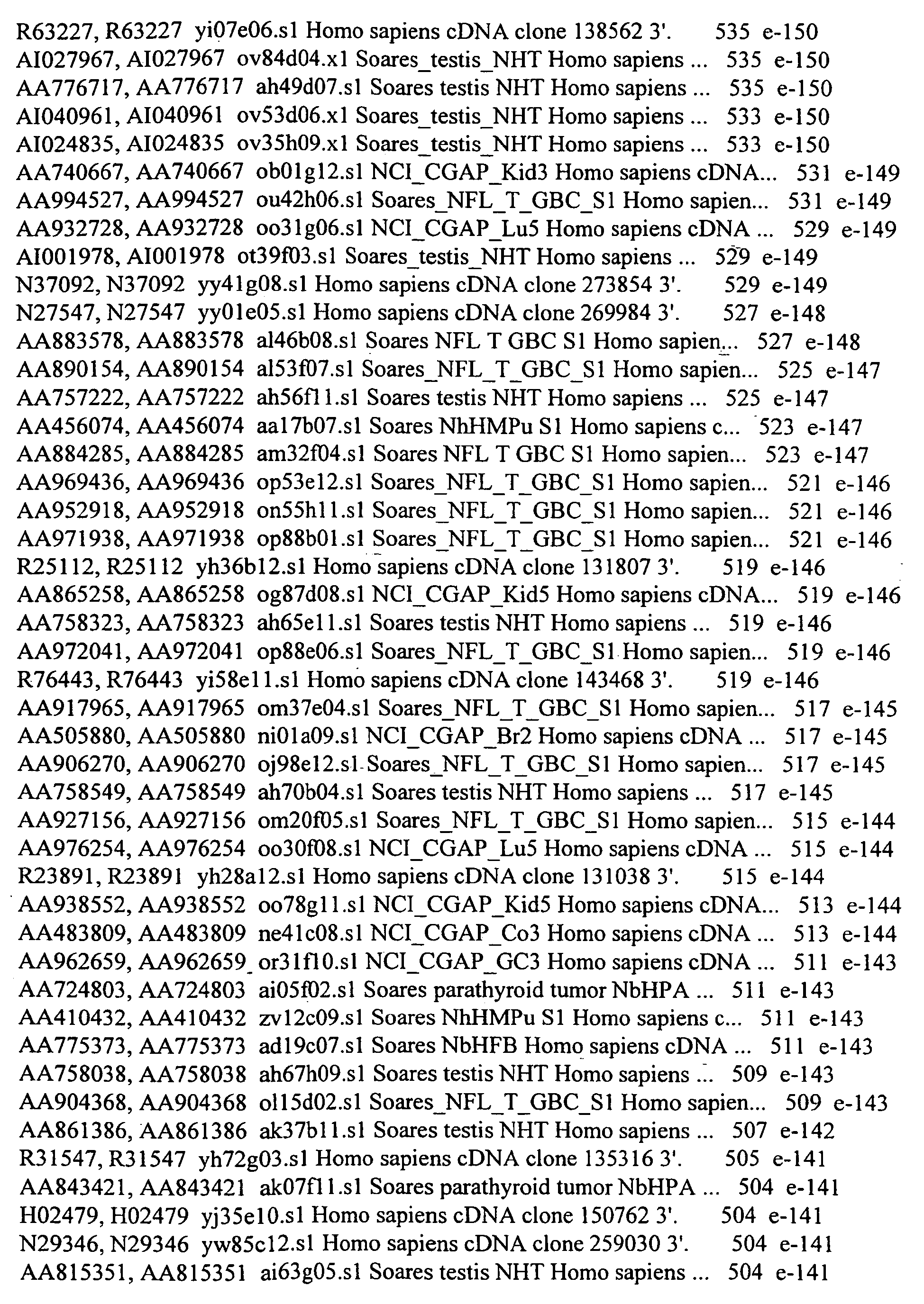

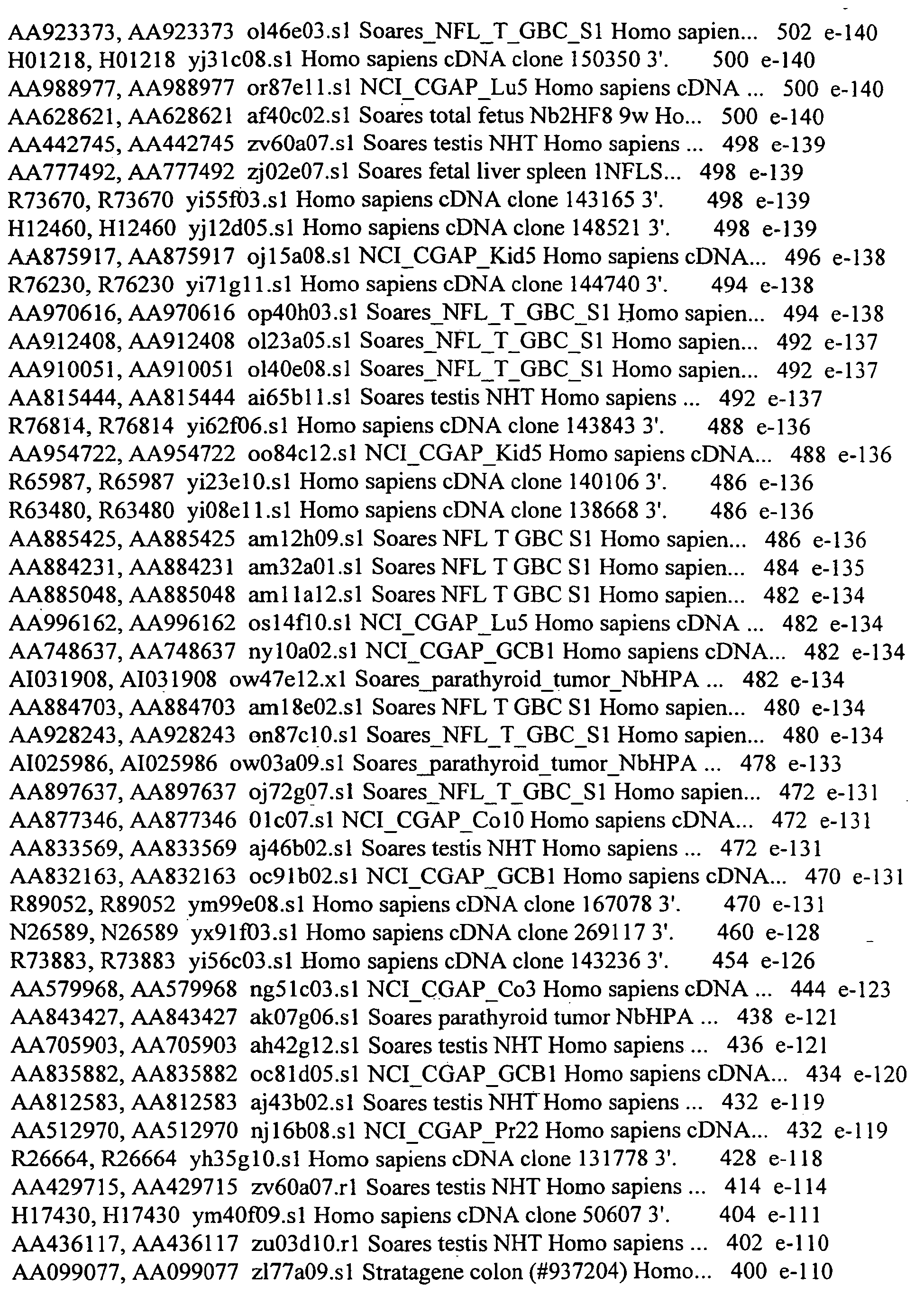

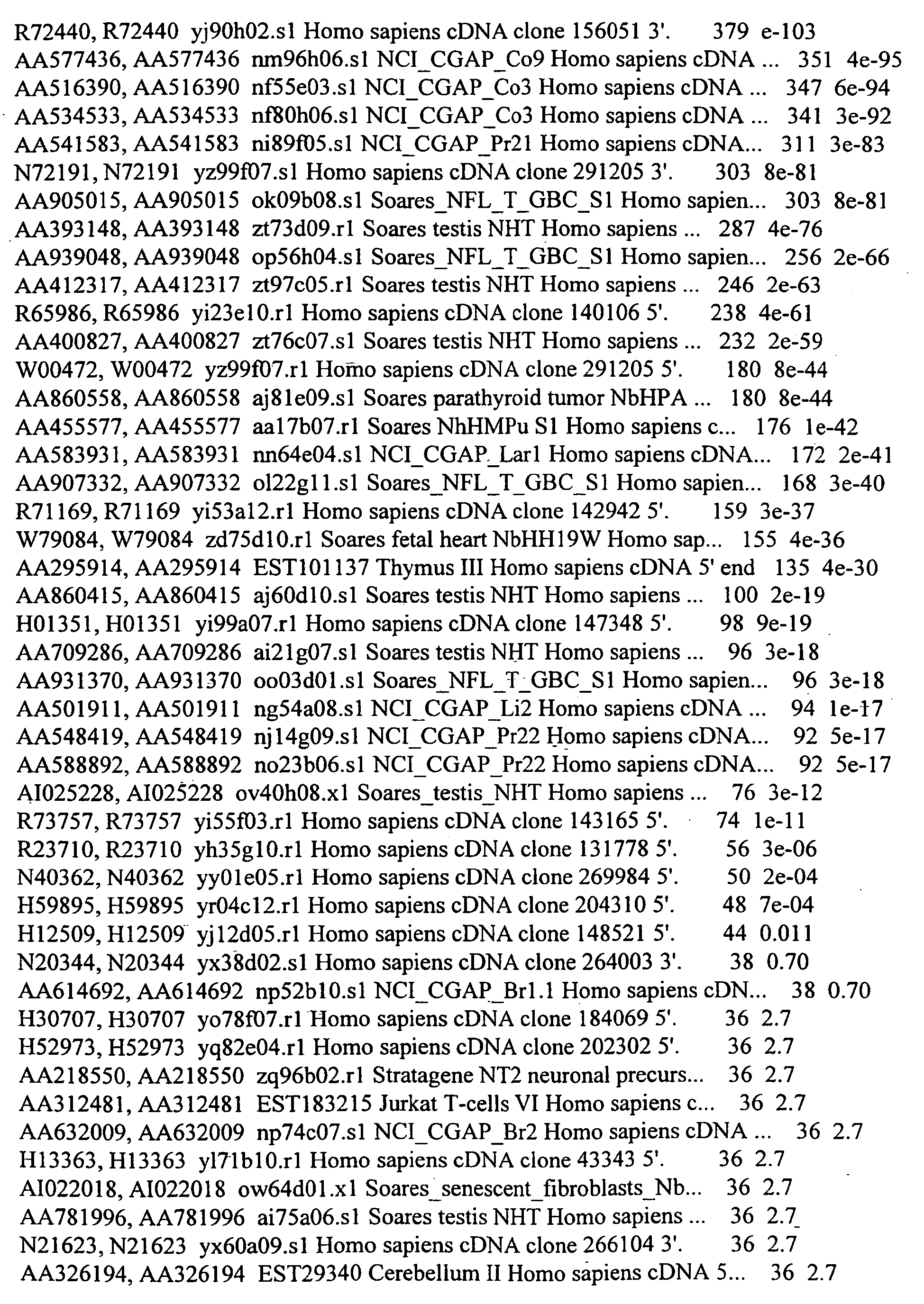

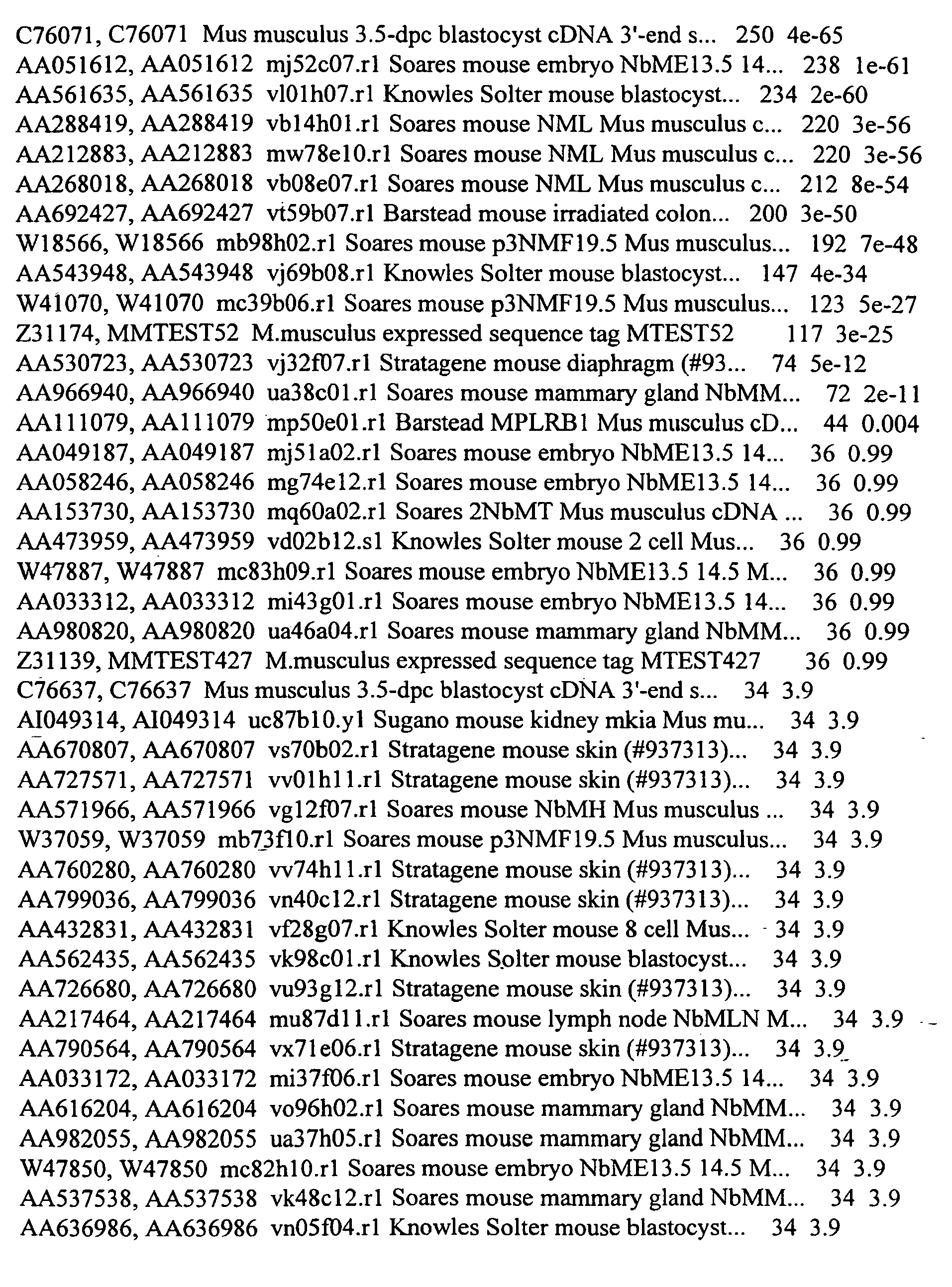

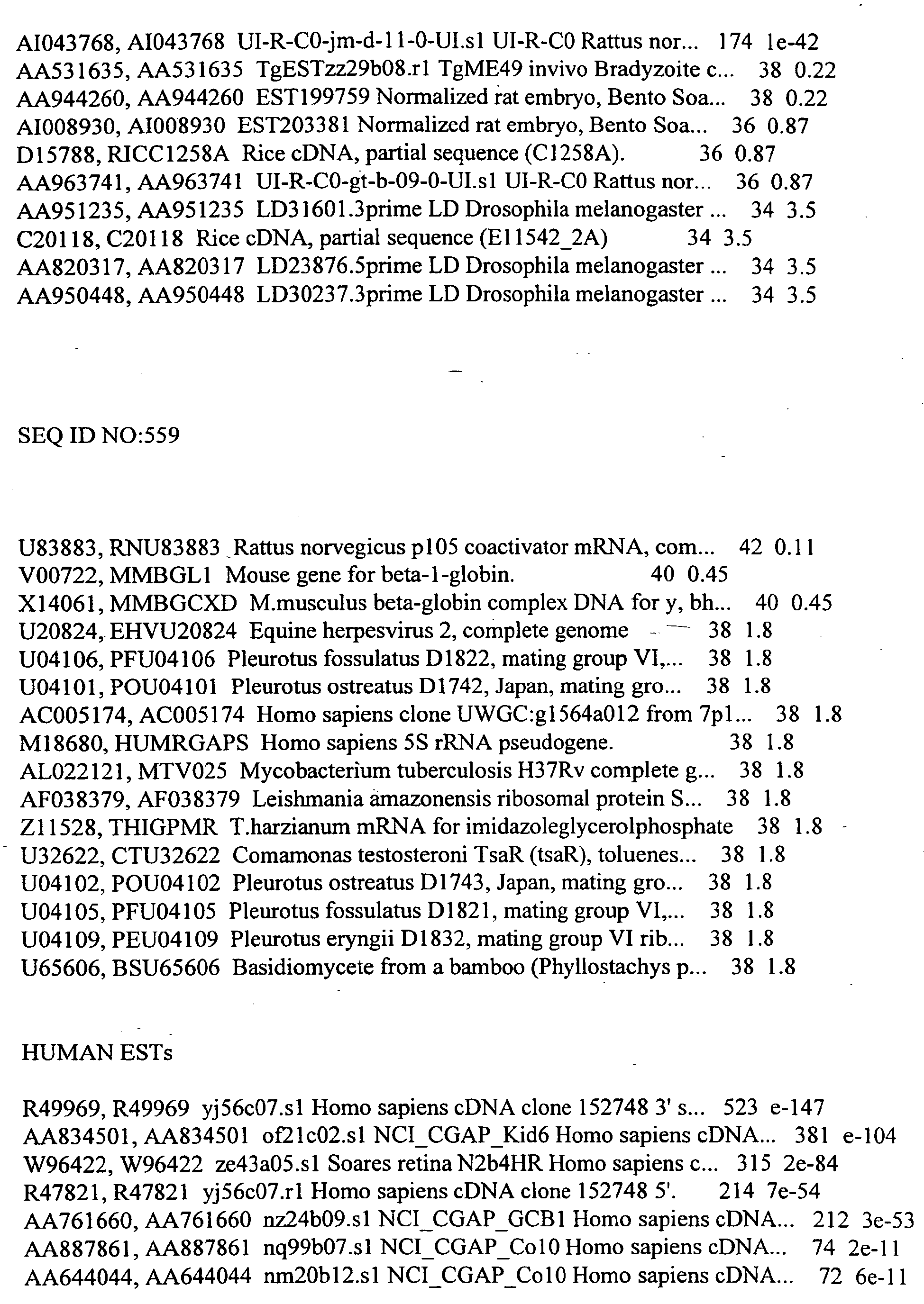

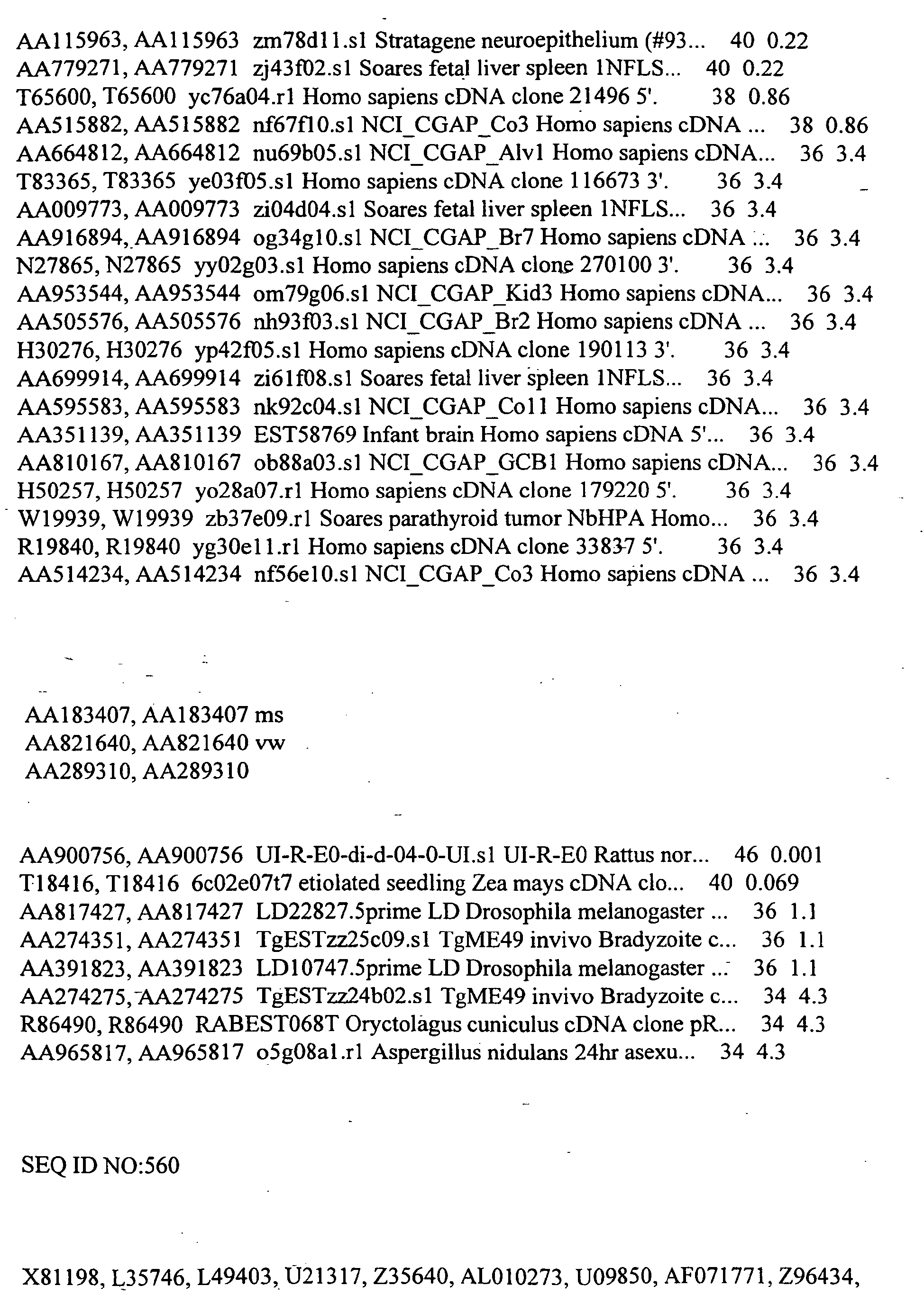

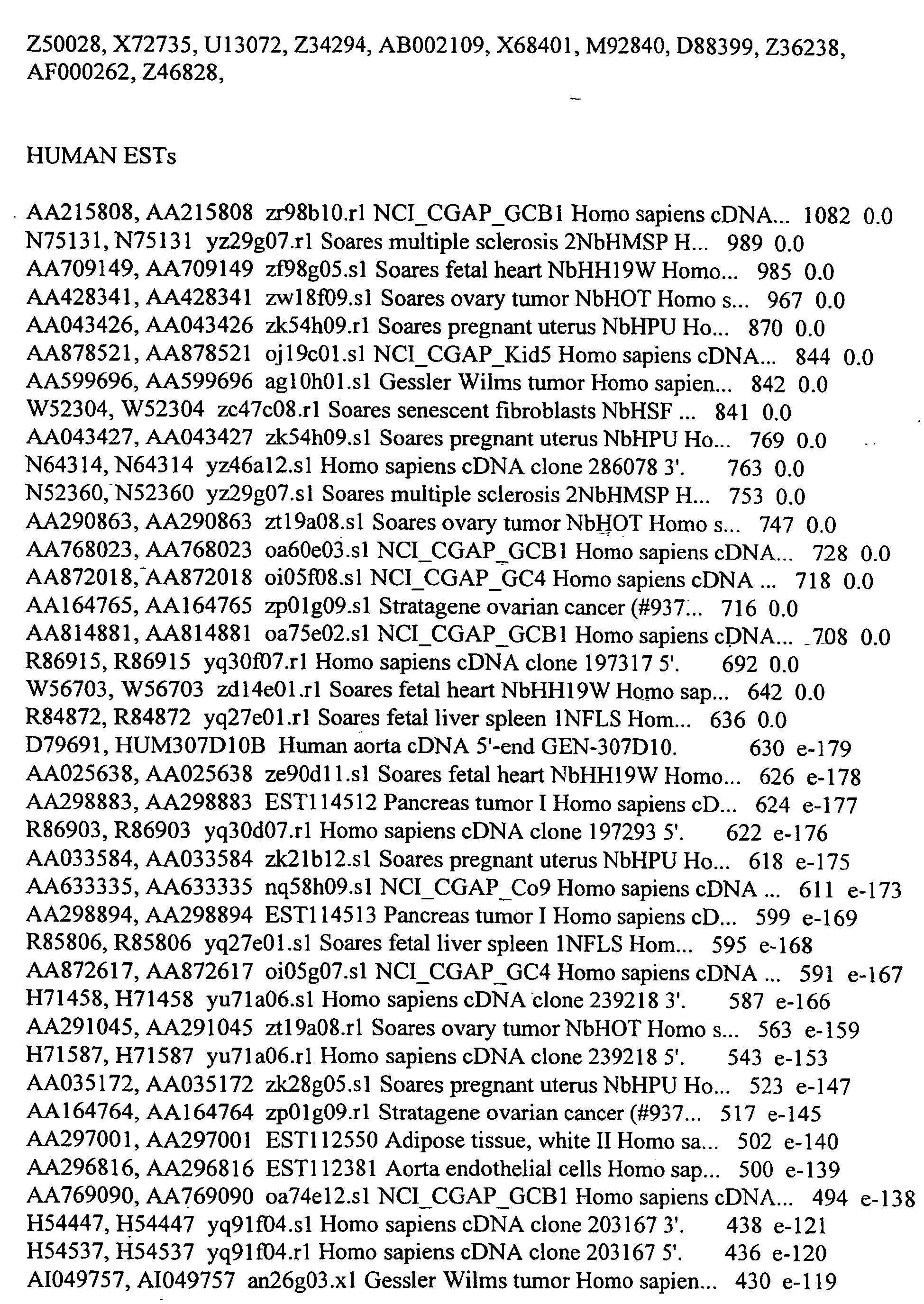

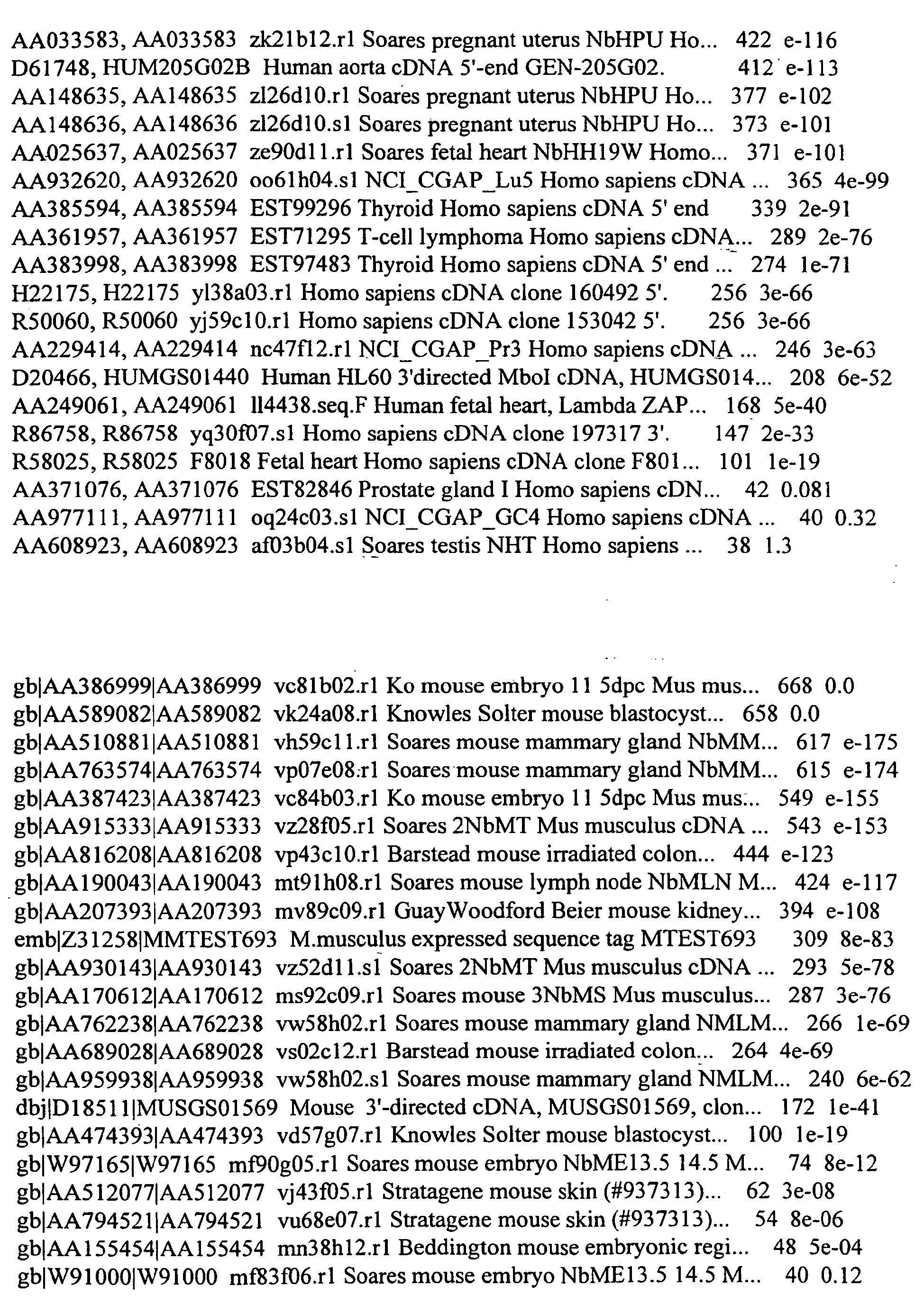

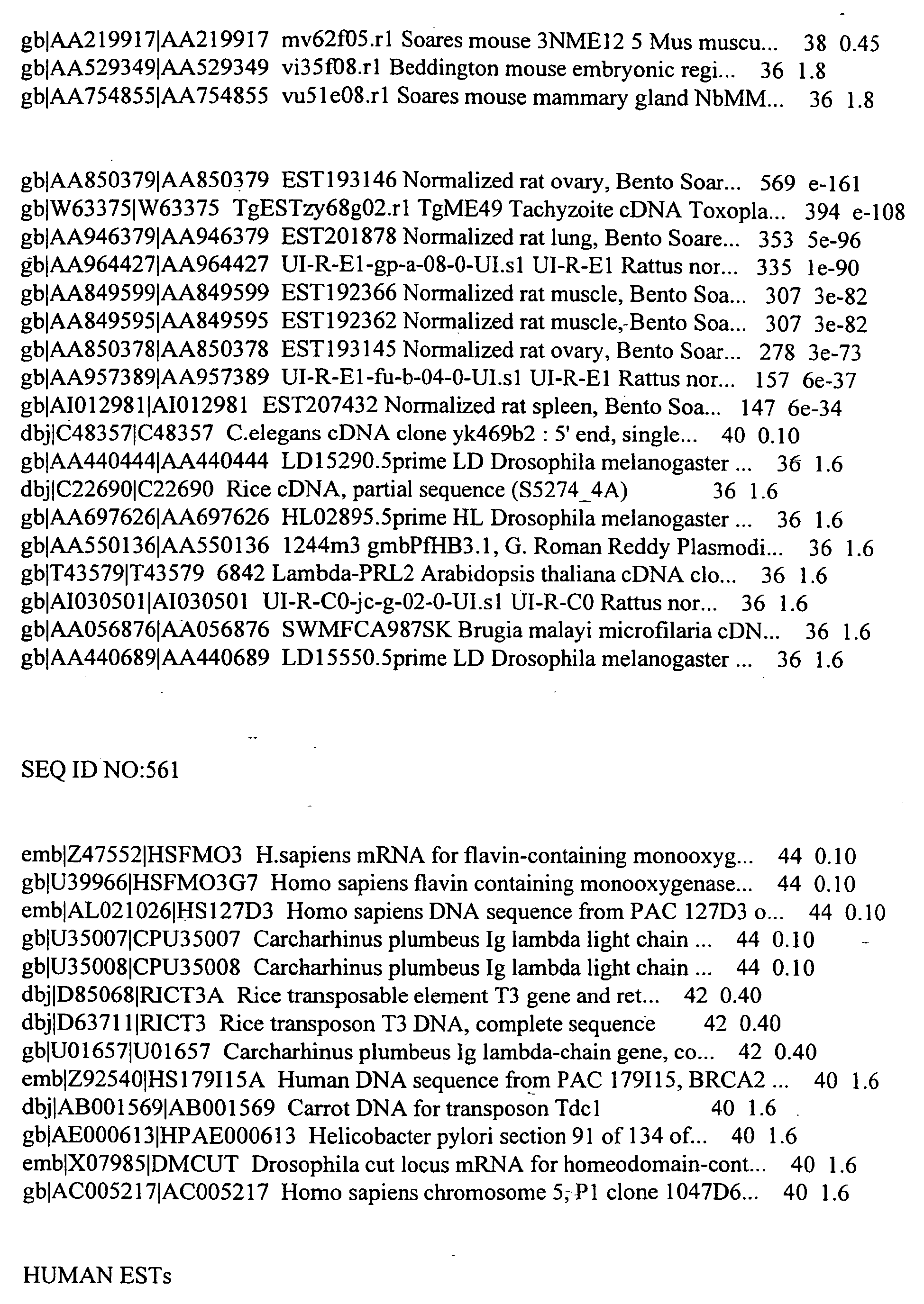

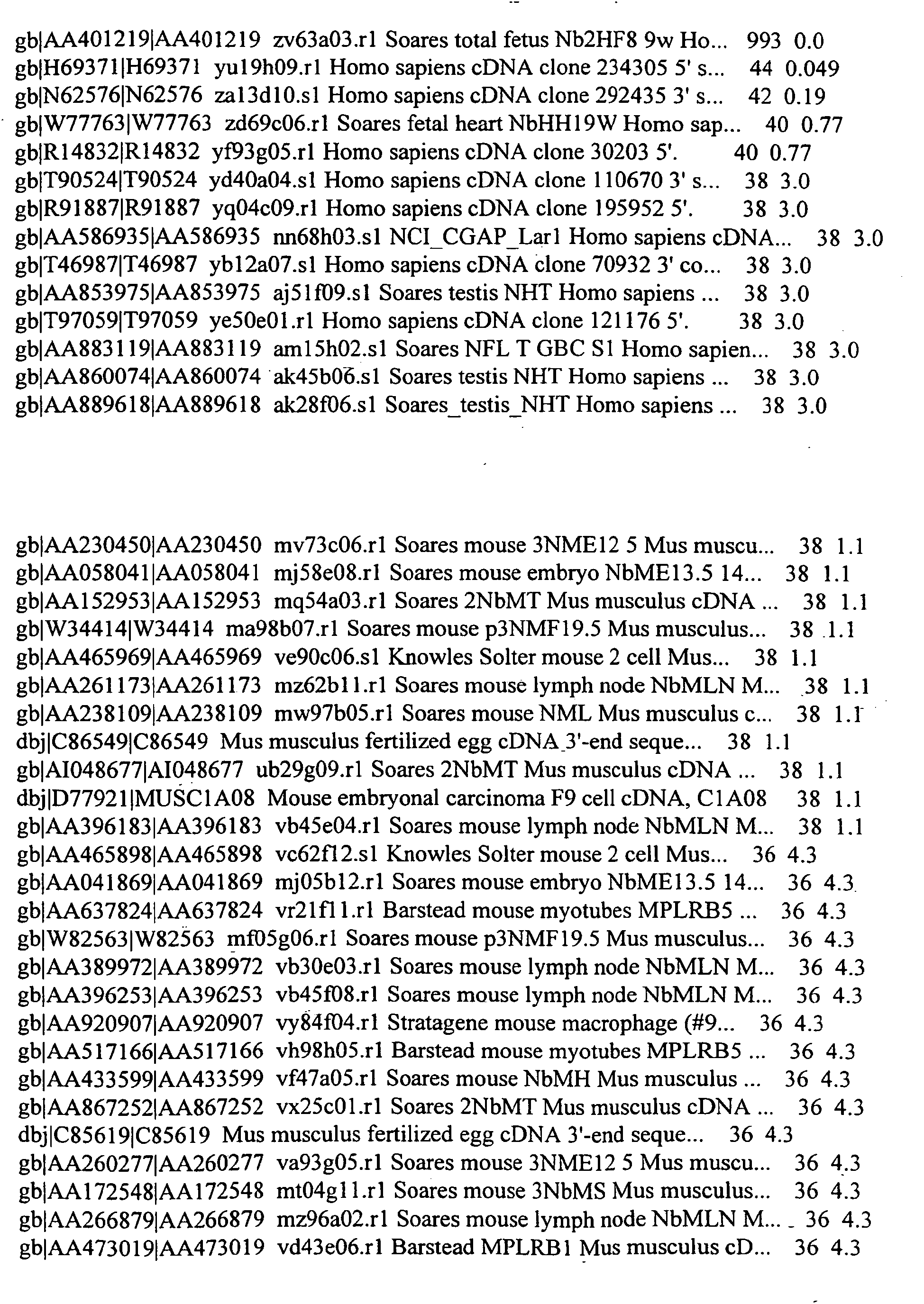

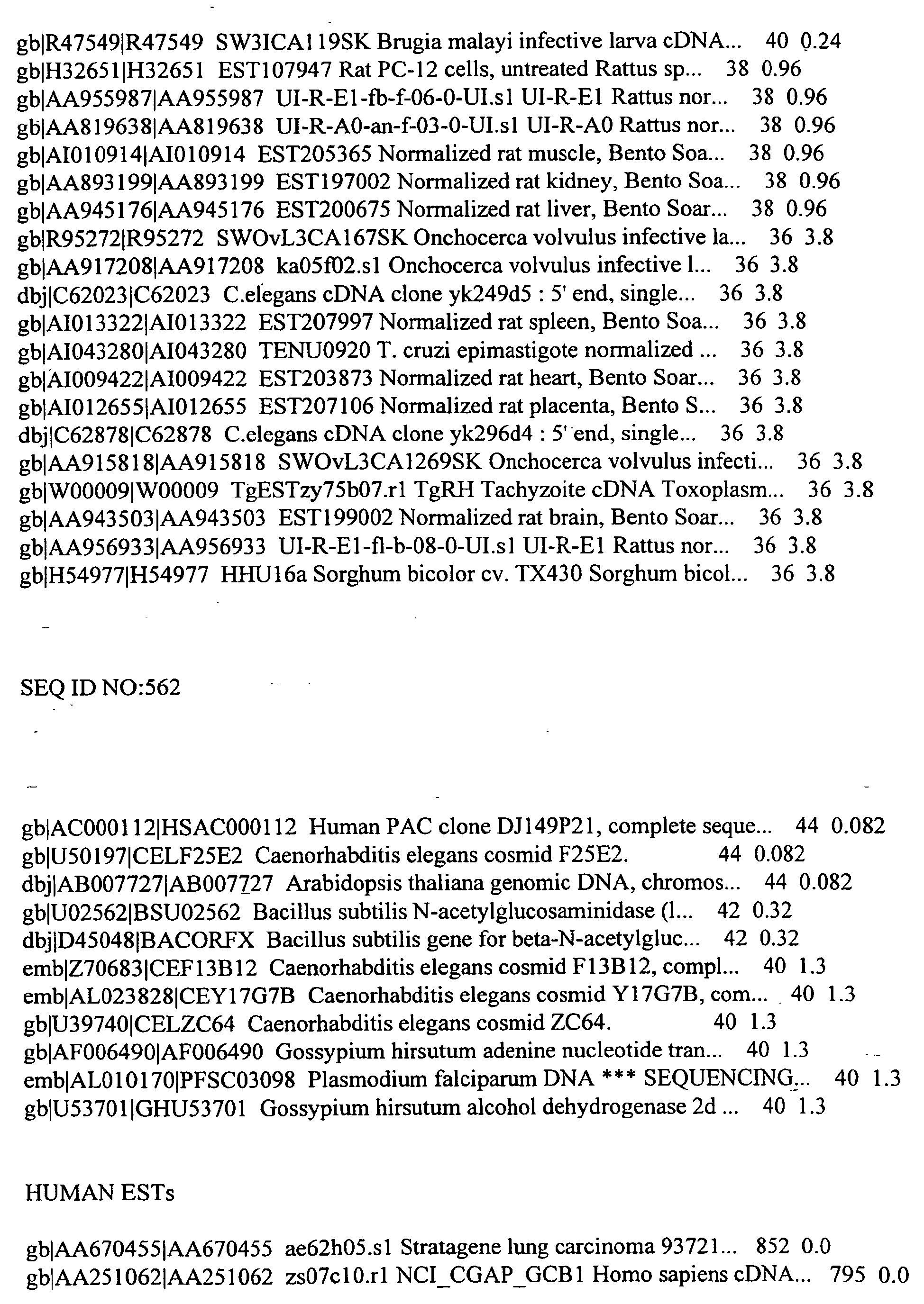

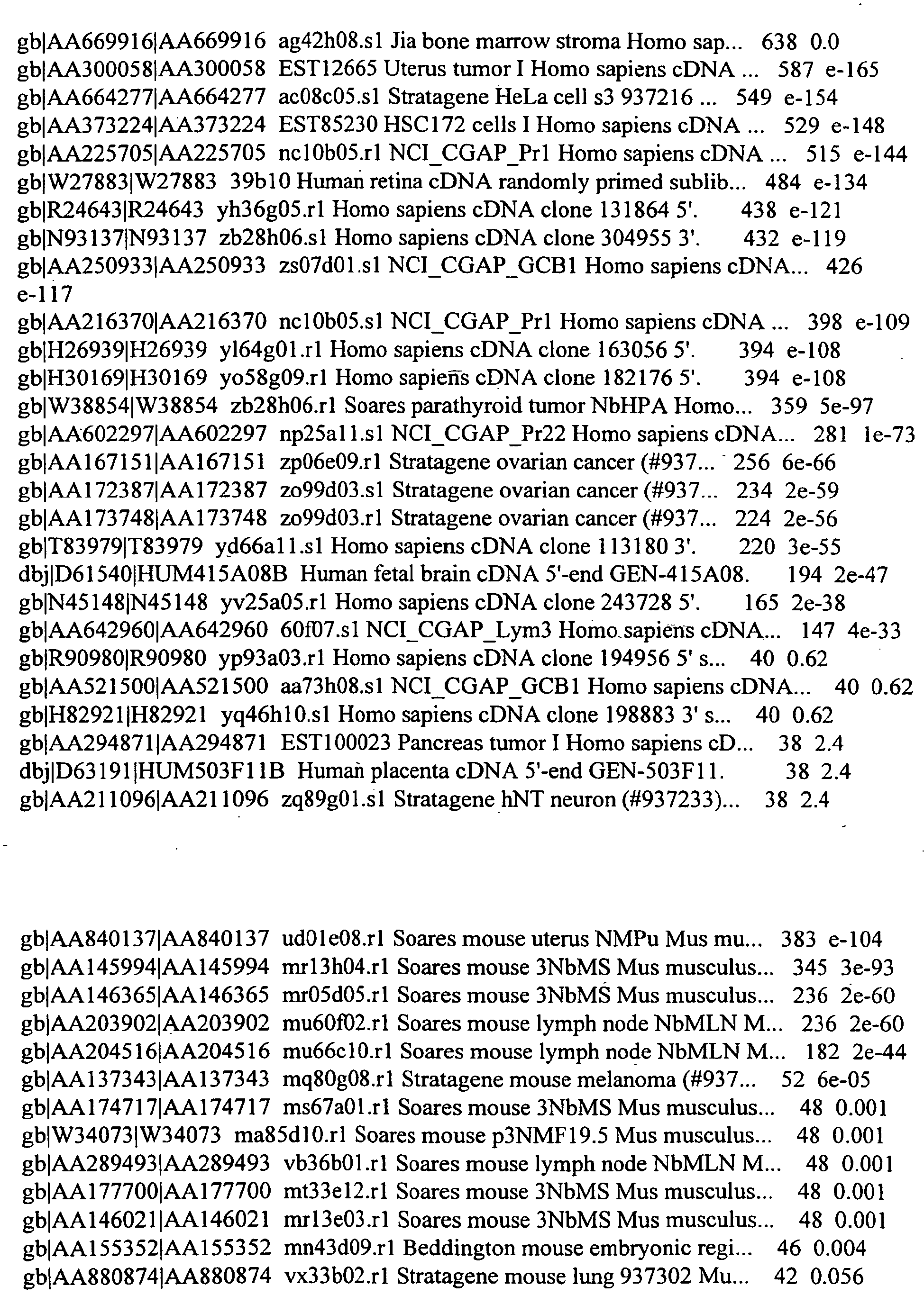

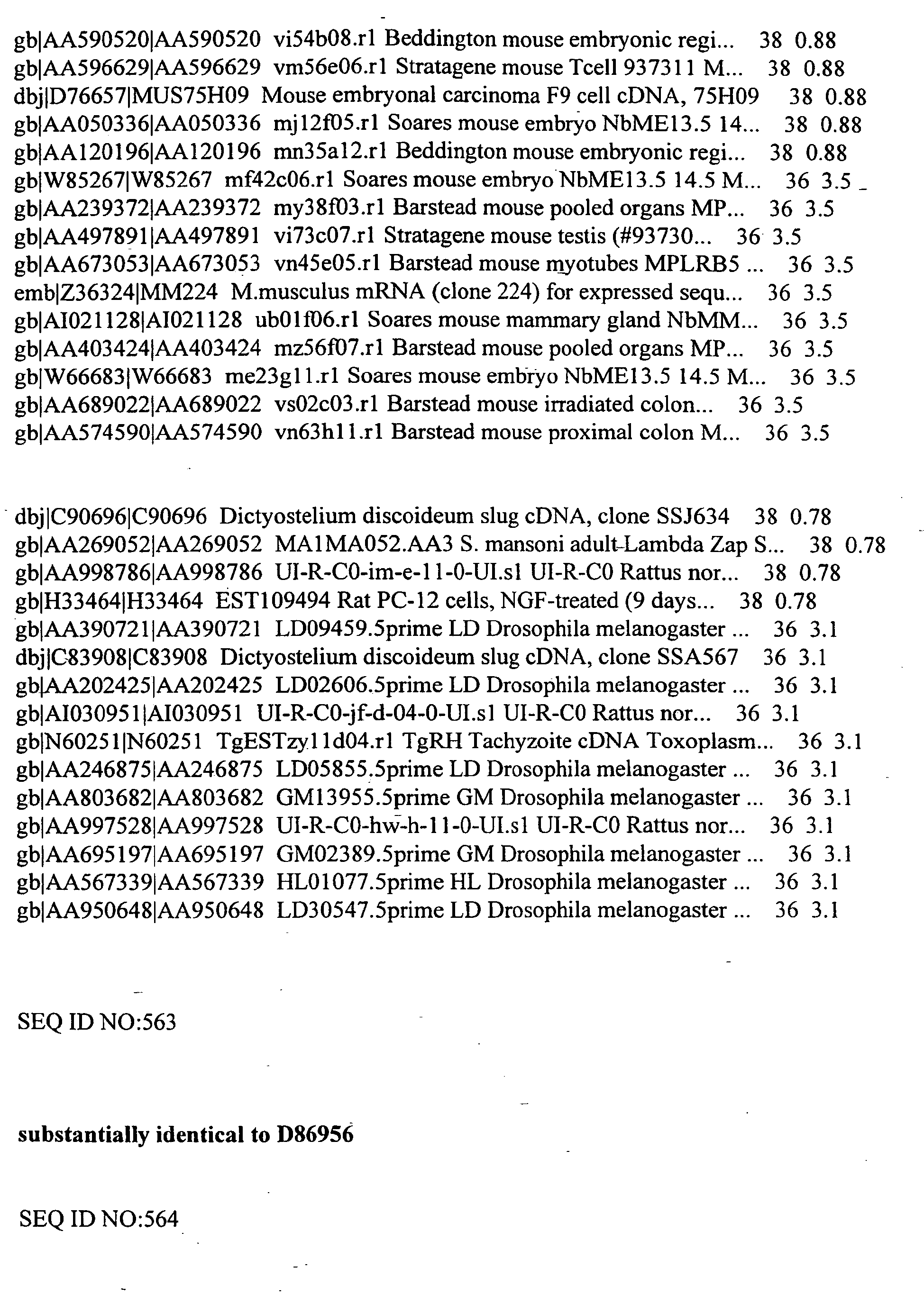

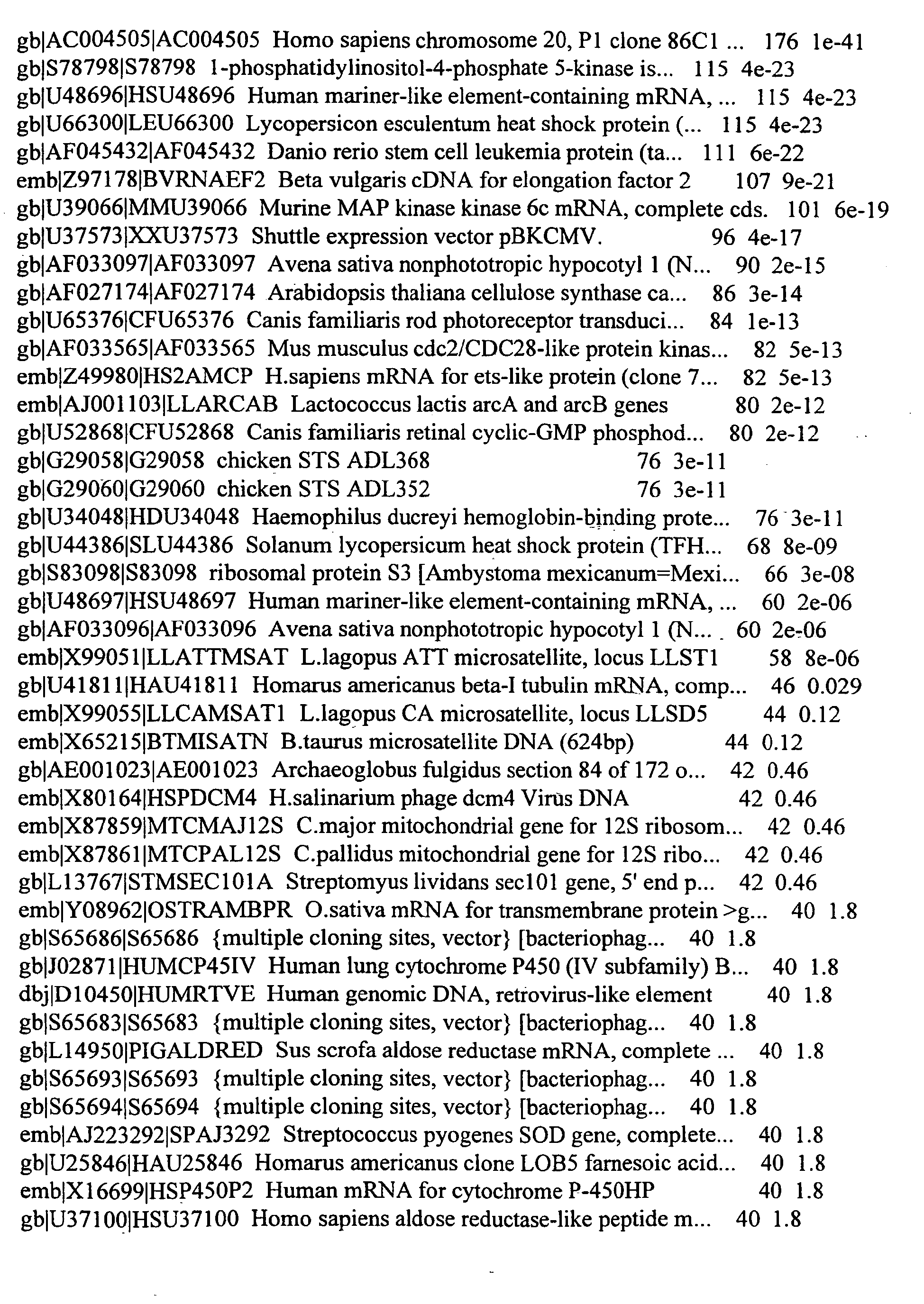

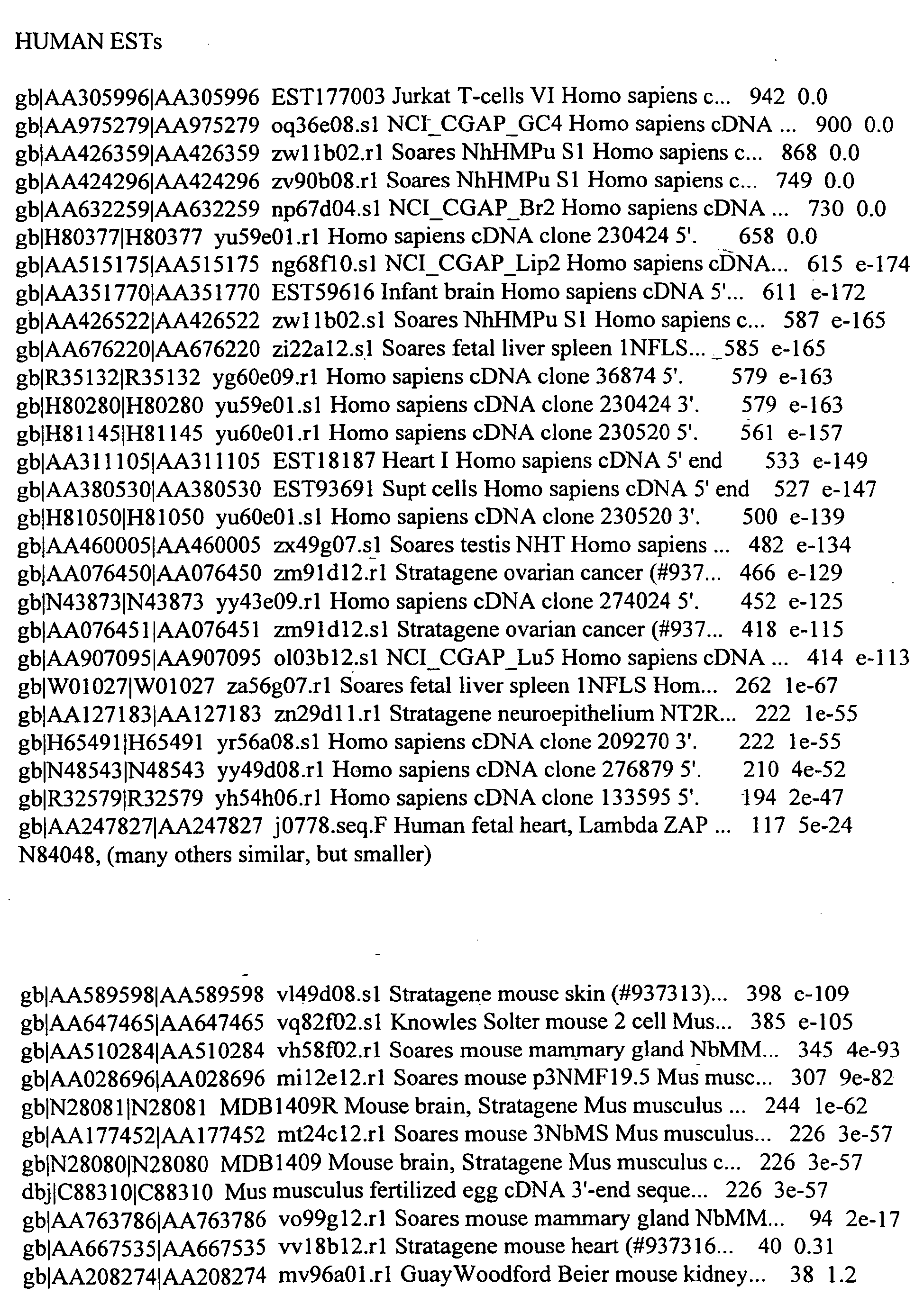

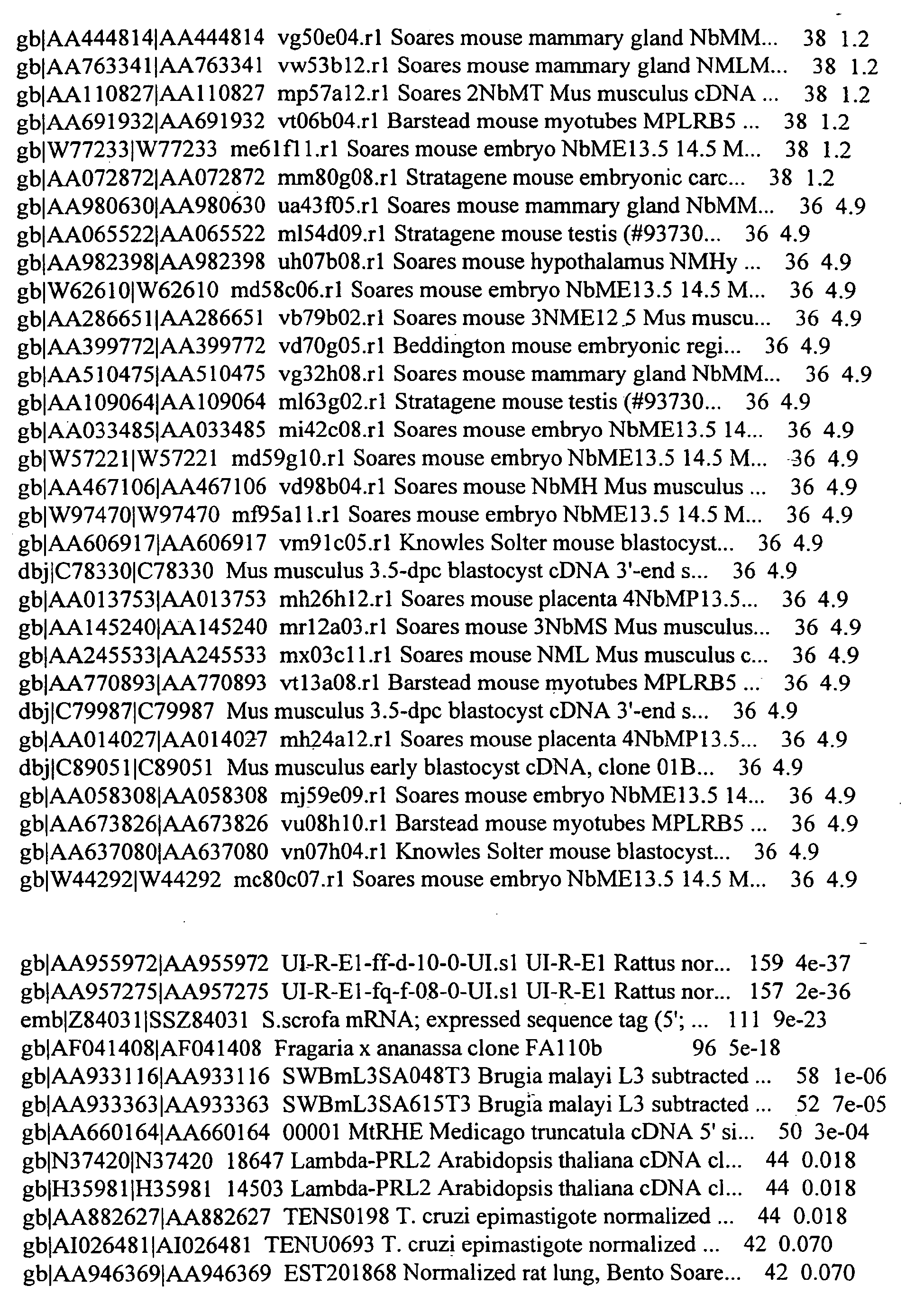

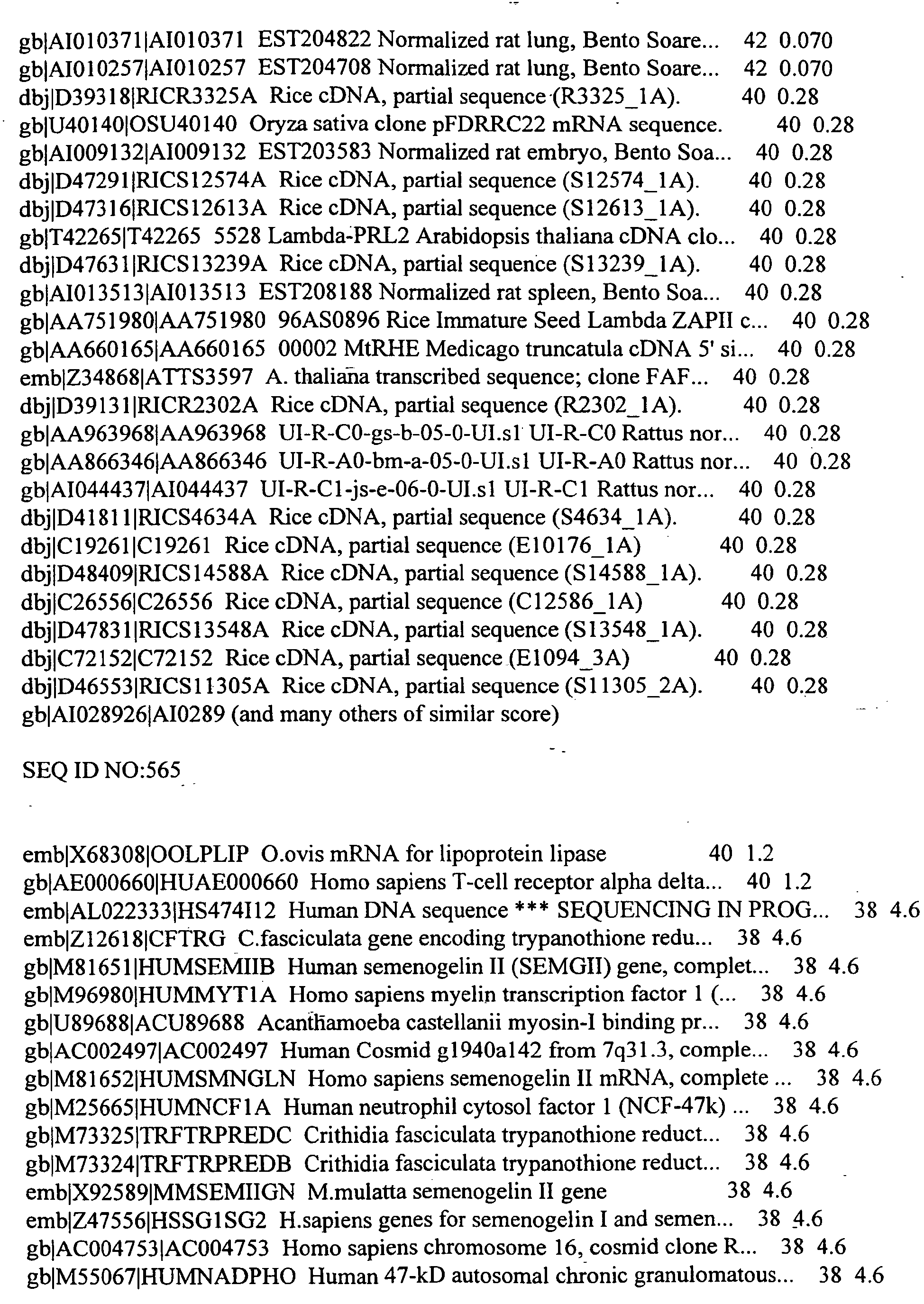

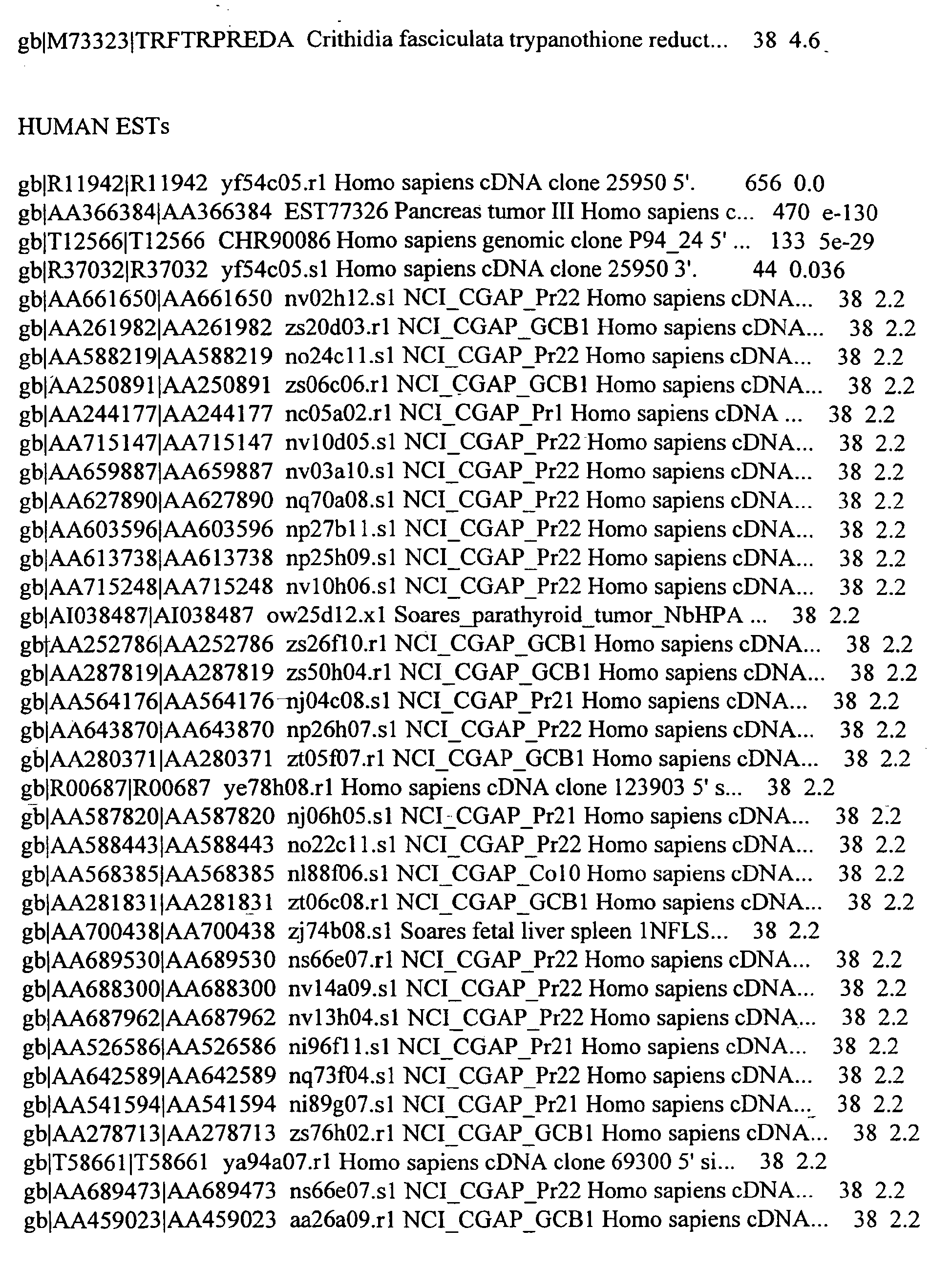

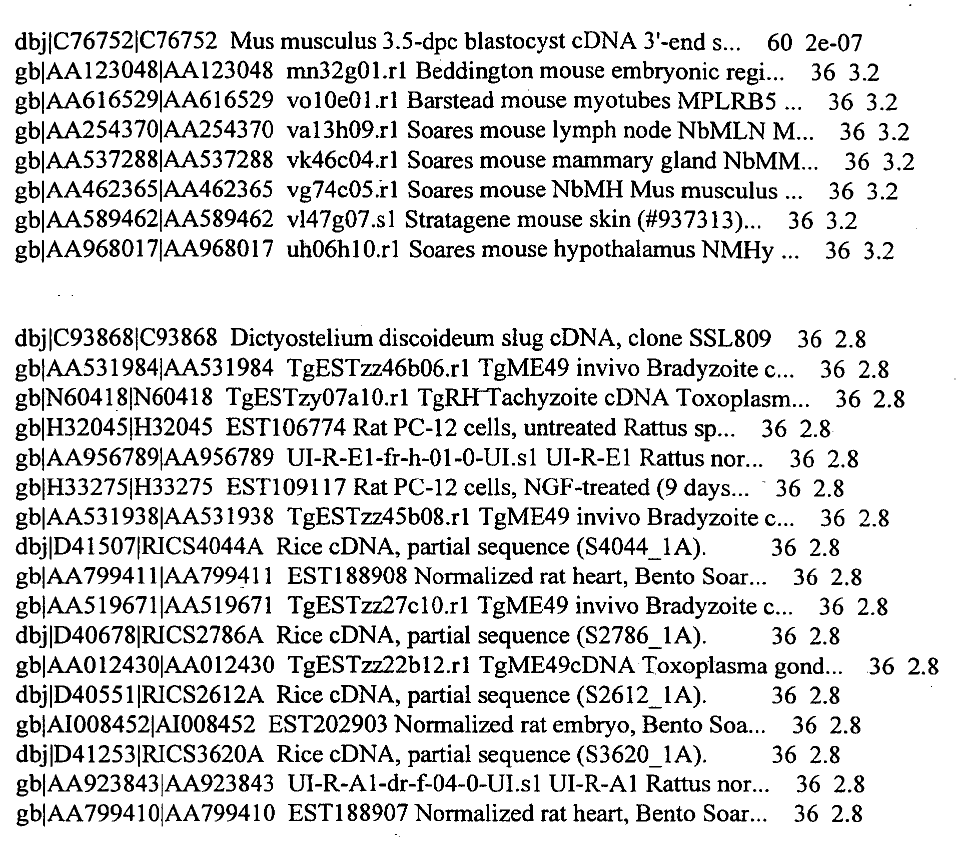

## Claims

1. An isolated nucleic acid molecule which is
(I) a nucleic acid molecule which hybridizes at 65°C in hybridisation buffer (3.5 x SSC, 0.02% Ficoll, 0.02% polyvinyl pyrrolidone, 0.02% Bovine Serum Albumin, 2.5mM NaH₂PO₄ (pH7), 0.5% SDS, 2mM EDTA) to a molecule consisting of SEQ ID NO: 681 and which codes for a cancer associated antigen precursor;
(II) a nucleic acid molecule that differs from the nucleic acid molecule of (I) in codon sequence due to the degeneracy of the genetic code; or
(III) a complement of (I) or (II).

2. An isolated nucleic acid molecule selected from the group consisting of:
(a) a fragment of SEQ ID NO: 681 of sufficient length to represent a sequence unique within the human genome identifying a nucleic acid encoding a human cancer associated antigen precursor; and
(b) complements of (a).

3. An isolated nucleic acid molecule of claim 2, wherein the fragment has a size selected from the group consisting of at least 8 nucleotides, 10 nucleotides, 12 nucleotides, 14 nucleotides, 16 nucleotides, 18 nucleotides, 20, nucleotides, 22 nucleotides, 24 nucleotides, 26 nucleotides, 28 nucleotides, 30 nucleotides, 50 nucleotides, 75 nucleotides, 100 nucleotides, and 200 nucleotides.

4. An isolated nucleic acid molecule of claim 3, wherein the molecule encodes a polypeptide, or a fragment thereof, which binds a human HLA receptor or a human antibody or an antigen-binding fragment thereof.

5. An isolated nucleic acid molecule as claimed in any of claims 1 to 4 consisting of a nucleic acid molecule having the sequence set out in SEQ ID NO: 681.

6. An expression vector comprising an isolated nucleic acid molecule of claims 1-5 operably linked to a promoter.

7. An expression vector as claimed in claim 6 wherein the nucleic acid codes for a polypeptide, or a portion thereof, which binds to an MHC molecule to form a complex recognised by an autologous antibody or lymphocyte.

8. An expression vector as claimed in claim 6 or claim 7 further comprising a nucleic acid encoding an HLA molecule.

9. A host cell transformed or transfected with an expression vector of claims 6-8.

10. A host cell transformed or transfected with an expression vector of claim 6 or claim 7 and further comprising a nucleic acid encoding HLA.

11. An isolated polypeptide encoded by the isolated nucleic acid molecule of claims 1-5.

12. A polypeptide as claimed in claim 11, which is immunogenic.

13. A polypeptide as claimed in claim 12, wherein the polypeptide; or a portion of the polypeptide, binds HLA or a human antibody or antigen-binding fragment thereof.

14. A polypeptide as claimed in claim 13, wherein said polypeptide is isolated.

15. A polypeptide of claim 14, wherein the polypeptide is part of a complex with HLA.

16. A polypeptide of claim 14, wherein the polypeptide is between 8 and 12 amino acids in length.

17. An isolated polypeptide comprising a fragment of the polypeptide of claim 14 of sufficient length to represent a sequence unique within the human genome identifying a polypeptide that is a human cancer associated antigen precursor.

18. A method of diagnosing a cancer, the method comprising contacting a biological sample isolated from a subject with an agent that specifically binds to a nucleic acid molecule as claimed in claim 1, and determining the interaction between the agent and the nucleic acid molecule as a determination of a cancer;
wherein the agent is a nucleic acid molecule which hybridizes at 65°C in hybridisation buffer (3.5 x SSC, 0.02% Ficoll, 0.02% polyvinyl pyrrolidone, 0.02% Bovine Serum Albumin, 2.5mM NaH₂PO₄ (pH7), 0.5% SDS, 2mM EDTA) to a molecule as claimed in claim 1 or fragment thereof.

19. A method of diagnosing a cancer, the method comprising contacting a biological sample isolated from a subject with an antibody or an antigen-binding fragment thereof that specifically binds to a polypeptide encoded by a nucleic acid molecule as claimed in claim 1, and determining the interaction between the agent and the polypeptide as a determination of a cancer

20. A method as claimed in claim 19, wherein the polypeptide is complexed with an HLA molecule.

21. A method of claims 18, 19 or 20 wherein the cancer is a breast, a gastric, a lung, a prostate, a renal or a colon cancer.

22. A method for determining regression, progression or onset of a cancer **characterized by** expression of abnormal levels of a protein encoded by a nucleic acid molecule as claimed in claim 1, the method comprising:
monitoring a sample from a patient who has or is suspected of having a cancer for a parameter selected from the group consisting of:
(i) the protein,
(ii) a peptide derived from the protein,
(iii) an antibody which selectively binds the protein or peptide, and
(iv) cytolytic T cells specific for a complex of the peptide derived from the protein and an MHC molecule,
as a determination of regression, progression or onset of said cancer,
wherein the sample is preferably a body fluid, a body effusion or a tissue.

23. A method of claim 22, wherein the step of monitoring comprises contacting the sample with a detectable agent selected from the group consisting of:
(a) an antibody or an antigen-binding fragment thereof which selectively binds the protein of (i), or the peptide of (ii);
(b) a protein or peptide which binds the antibody of (iii); and
(c) a cell which presents the complex of the peptide and MHC molecule of (iv).

24. A method of claim 23, wherein the antibody, fragment, protein, peptide or cell is labelled with a radioactive label or an enzyme.

25. A method of claim 22, comprising assaying the sample for the peptide.

26. A kit for detecting the presence of the expression of a human cancer associated antigen precursor comprising:
a pair of isolated nucleic acid molecules each of which consists of a molecule selected from the group consisting of:
(a) a 12-32 nucleotide contiguous segment of the nucleotide sequence of any of a nucleic acid molecule as claimed in claim 1; and
(b) complements of (a), wherein the contiguous segments are nonoverlapping;
and wherein the pair of isolated nucleic acid molecules selectively amplify an isolated nucleic acid molecule as claimed in claim 1.

27. An isolated antibody or an antigen-binding fragment thereof which selectively binds to a complex of:
(i) a peptide derived from a protein encoded by a nucleic acid molecule as claimed in claim 1, and
(ii) and an MHC molecule to which binds the peptide to form the complex,
wherein the isolated antibody does not bind to (i) or (ii) alone,
wherein the antibody is preferably a monoclonal antibody, a chimeric antibody or a humanized antibody.

## Patentansprüche

1. Isoliertes Nukleinsäuremolekül, bei dem es sich um Folgendes handelt:
(I) ein Nukleinsäuremolekül, das bei 65 °C in Hybridisierungspuffer (3,5X SSC, 0,02 % Ficoll, 0,02 % Polyvinylpyrrolidon, 0,02 % Rinderserumalbumin, 2,5 mM NaH₂PO₄ (pH 7), 0,5 % SDS, 2 mM EDTA) an ein Molekül hybridisiert, das aus SEQ ID NO: 681 besteht, und das für einen mit Krebs assoziierten Antigenvorläufer codiert;
(II) ein Nukleinsäuremolekül, das sich von dem Nukleinsäuremolekül von (I) in Bezug auf die Codonsequenz aufgrund der Degeneration des genetischen Codes unterscheidet; oder
(III) ein Komplement von (I) oder (II).

2. Isoliertes Nukleinsäuremolekül, das aus der Gruppe bestehend aus Folgendem ausgewählt ist:
(a) ein Fragment von SEQ ID NO: 681 von ausreichender Länge, um eine Sequenz darzustellen, die im menschlichen Genom einzigartig ist, und das eine Nukleinsäure identifiziert, die einen mit menschlichem Krebs assoziierten Antigenvorläufer codiert; und
(b) Komplemente von (a).

3. Isoliertes Nukleinsäuremolekül nach Anspruch 2, wobei das Fragment eine Größe aufweist, die aus der Gruppe bestehend aus mindestens 8 Nukleotiden, 10 Nukleotiden, 12 Nukleotiden, 14 Nukleotiden, 16 Nukleotiden, 18 Nukleotiden, 20 Nukleotiden, 22 Nukleotiden, 22 Nukleotiden, 24 Nukleotiden, 26 Nukleotiden, 28 Nukleotiden, 30 Nukleotiden, 50 Nukleotiden, 75 Nukleotiden, 100 Nukleotiden und 200 Nukleotiden ausgewählt ist.

4. Isoliertes Nukleinsäuremolekül nach Anspruch 3, wobei das Molekül ein Polypeptid oder ein Fragment davon codiert, das einen menschlichen HLA-Rezeptor oder einen menschlichen Antikörper oder ein antigenbindendes Fragment davon bindet.

5. Isoliertes Nukleinsäuremolekül nach einem der Ansprüche 1 bis 4, das aus einem Nukleinsäuremolekül mit der in SEQ ID NO: 681 dargelegten Sequenz besteht.

6. Expressionsvektor, der ein isoliertes Nukleinsäuremolekül nach den Ansprüchen 1 - 5 umfasst, das mit einem Promotor operativ verknüpft ist.

7. Expressionsvektor nach Anspruch 6, wobei die Nukleinsäure für ein Polypeptid oder einen Teil davon codiert, das bzw. der an ein MHC-Molekül bindet, um einen Komplex zu bilden, der von einem autologen Antikörper oder Lymphozyten erkannt wird.

8. Expressionsvektor nach Anspruch 6 oder 7, der weiterhin eine Nukleinsäure umfasst, die ein HLA-Molekül codiert.

9. Wirtszelle, die mit einem Expressionsvektor nach den Ansprüchen 6 - 8 transformiert oder transfiziert wird.

10. Wirtszelle, die mit einem Expressionsvektor nach Anspruch 6 oder 7 transformiert oder transfiziert wird und weiterhin eine Nukleinsäure umfasst, die HLA codiert.

11. Isoliertes Polypeptid, das von dem isolierten Nukleinsäuremolekül nach den Ansprüchen 1 - 5 codiert wird.

12. Polypeptid nach Anspruch 11, das immunogen ist.

13. Polypeptid nach Anspruch 12, wobei das Polypeptid oder ein Teil des Polypeptids HLA oder einen menschlichen Antikörper oder ein antigenbindendes Fragment davon bindet.

14. Polypeptid nach Anspruch 13, wobei das Polypeptid isoliert ist.

15. Polypeptid nach Anspruch 14, wobei das Polypeptid Teil eines Komplexes mit HLA ist.

16. Polypeptid nach Anspruch 14, wobei das Polypeptid zwischen 8 und 12 Aminosäuren lang ist.

17. Isoliertes Polypeptid, das ein Fragment des Polypeptids nach Anspruch 14 von ausreichender Länge umfasst, um eine Sequenz darzustellen, die im menschlichen Genom einzigartig ist, und das ein Polypeptid identifiziert, bei dem es sich um einen mit menschlichem Krebs assoziierten Antigenvorläufer handelt.

18. Verfahren zum Diagnostizieren eines Karzinoms, wobei das Verfahren das Inkontaktbringen einer biologischen Probe, die aus einem Probanden isoliert wurde, mit einem Agens, das spezifisch an ein Nukleinsäuremolekül nach Anspruch 1 bindet, und das Bestimmen der Wechselwirkung zwischen dem Agens und dem Nukleinsäuremolekül als eine Bestimmung eines Karzinoms umfasst;
wobei es sich bei dem Agens um ein Nukleinsäuremolekül, das bei 65 °C in Hybridisierungspuffer (3,5X SSC, 0,02 % Ficoll, 0,02 % Polyvinylpyrrolidon, 0,02 % Rinderserumalbumin, 2,5 mM NaH₂PO₄ (pH 7), 0,5 % SDS, 2 mM EDTA) an ein Molekül hybridisiert, nach Anspruch 1 oder ein Fragment davon handelt.

19. Verfahren zum Diagnostizieren eines Karzinoms, wobei das Verfahren das Inkontaktbringen einer biologischen Probe, die aus einem Probanden isoliert wurde, mit einem Antikörper oder einem antigenbindenden Fragment davon, der bzw. das spezifisch an ein Polypeptid bindet, das von einem Nukleinsäuremolekül nach Anspruch 1 codiert wird, und das Bestimmen der Wechselwirkung zwischen dem Agens und dem Polypeptid als eine Bestimmung eines Karzinoms umfasst.

20. Verfahren nach Anspruch 19, wobei das Polypeptid mit einem HLA-Molekül komplexiert wird.

21. Verfahren nach Anspruch 18, 19 oder 20, wobei es sich bei dem Karzinom um ein Brustkarzinom, ein Magenkarzinom, ein Lungenkarzinom, ein Prostatakarzinom, ein Nierenkarzinom oder ein Dickdarmkarzinom handelt.

22. Verfahren zum Bestimmen der Rückbildung, Weiterentwicklung oder Entstehung eines Karzinoms, die durch die Expression abnormer Niveaus eines Proteins **gekennzeichnet** ist, das von einem Nukleinsäuremolekül nach Anspruch 1 codiert wird, wobei das Verfahren Folgendes umfasst:
Überwachen einer Probe von einem Patienten, der ein Karzinom hat oder von dem vermutet wird, dass er ein Karzinom hat, auf einen Parameter, der aus der Gruppe bestehend aus Folgendem ausgewählt ist:
(i) dem Protein,
(ii) einem Peptid, das von dem Protein abgeleitet ist,
(iii) einem Antikörper, der selektiv das Protein oder das Peptid bindet, und
(iv) zytolytischen T-Zellen, die für einen Komplex aus dem Peptid, das von dem Protein abgeleitet ist, und einem MHC-Molekül spezifisch sind,
als eine Bestimmung der Rückbildung, Weiterentwicklung oder Entstehung eines Karzinoms,
wobei es sich bei der Probe vorzugsweise um eine Körperflüssigkeit, ein Körperexsudat oder ein Gewebe handelt.

23. Verfahren nach Anspruch 22, wobei der Schritt des Überwachens das Inkontaktbringen der Probe mit einem nachweisbaren Agens umfasst, das aus der Gruppe bestehend aus Folgendem ausgewählt ist:
(a) einem Antikörper oder einem antigenbindenden Fragment davon, der bzw. das selektiv das Protein von (i) oder das Peptid von (ii) bindet;
(b) einem Protein oder Peptid, das den Antikörper von (iii) bindet; und
(c) einer Zelle, die den Komplex aus dem Peptid und dem MHC-Molekül von (iv) darstellt.

24. Verfahren nach Anspruch 23, wobei der Antikörper, das Fragment, das Protein, das Peptid oder die Zelle mit einem radioaktiven Marker oder einem Enzym markiert ist.

25. Verfahren nach Anspruch 22, das das Testen der Probe auf das Peptid umfasst.

26. Kit zum Nachweisen des Vorliegens der Expression eines mit menschlichem Krebs assoziierten Antigenvorläufers, das Folgendes umfasst:
ein Paar isolierter Nukleinsäuremoleküle, von denen jedes aus einem Molekül besteht, das aus der Gruppe bestehend aus Folgendem ausgewählt ist:
(a) einem zusammenhängenden Segment aus 12 - 32 Nukleotiden der Nukleotidsequenz eines beliebigen Nukleinsäuremoleküls nach Anspruch 1 und
(b) Komplementen von (a), wobei die zusammenhängenden Segmente nichtüberlappend sind;
und wobei das Paar isolierter Nukleinsäuremoleküle selektiv ein isoliertes Nukleinsäuremolekül nach Anspruch 1 amplifiziert.

27. Isolierter Antikörper oder ein antigenbindendes Fragment davon, der bzw. das selektiv einen Komplex aus Folgendem bindet:
(i) einem Peptid, das von einem Protein abgeleitet ist, das von einem Nukleinsäuremolekül nach Anspruch 1 codiert wird, und
(ii) einem MHC-Molekül, an das das Peptid bindet, um den Komplex zu bilden,
wobei der isolierte Antikörper nicht an (i) oder (ii) allein bindet,
wobei es sich bei dem Antikörper vorzugsweise um einen monoklonalen Antikörper, einen chimären Antikörper oder einen humanisierten Antikörper handelt.

## Revendications

1. Molécule d'acide nucléique isolée qui est
(I) une molécule d'acide nucléique qui s'hybride à 65 °C dans du tampon d'hybridation (SSC 3,5x, 0,02 % de Ficoll, 0,02 % de polyvinylpyrrolidone, 0,02 % d'albumine de sérum bovin, NaH₂PO₄ 2,5 mM (pH 7), 0,5 % de SDS, EDTA 2 mM) à une molécule consistant en SEQ ID NO : 681 et qui code pour un précurseur d'antigène associé au cancer ;
(II) une molécule d'acide nucléique qui diffère de la molécule d'acide nucléique de (I) dans une séquence de codons du fait de la dégénérescence du code génétique ; ou
(III) un complément de (I) ou (II).

2. Molécule d'acide nucléique isolée choisie dans le groupe constitué par :
(a) un fragment de SEQ ID NO : 681 d'une longueur suffisante pour représenter une séquence unique à l'intérieur du génome humain identifiant un acide nucléique codant pour un précurseur d'antigène associé au cancer humain ; et
(b) les compléments de (a).

3. Molécule d'acide nucléique isolée de la revendication 2, dans laquelle le fragment a une taille choisie dans le groupe constitué par au moins 8 nucléotides, 10 nucléotides, 12 nucléotides, 14 nucléotides, 16 nucléotides, 18 nucléotides, 20 nucléotides, 22 nucléotides, 24 nucléotides, 26 nucléotides, 28 nucléotides, 30 nucléotides, 50 nucléotides, 75 nucléotides, 100 nucléotides et 200 nucléotides.

4. Molécule d'acide nucléique isolée de la revendication 3, dans laquelle la molécule code pour un polypeptide, ou un fragment de celui-ci, qui se lie à un récepteur de HLA humain ou un anticorps humain ou un fragment fixant un antigène de celui-ci.

5. Molécule d'acide nucléique isolée selon l'une quelconque des revendications 1 à 4 consistant en une molécule d'acide nucléique ayant la séquence présentée dans SEQ ID NO : 681.

6. Vecteur d'expression comprenant une molécule d'acide nucléique isolée des revendications 1-5 liée de façon fonctionnelle à un promoteur.

7. Vecteur d'expression selon la revendication 6
dans lequel l'acide nucléique code pour un polypeptide, ou une partie de celui-ci, qui se lie à une molécule MHC pour former un complexe reconnu par un anticorps ou lymphocyte autologue.

8. Vecteur d'expression selon la revendication 6 ou la revendication 7 comprenant en outre un acide nucléique codant pour une molécule HLA.

9. Cellule hôte transformée ou transfectée avec un vecteur d'expression des revendications 6-8.

10. Cellule hôte transformée ou transfectée avec un vecteur d'expression de la revendication 6 ou la revendication 7 et comprenant en outre un acide nucléique codant pour HLA.

11. Polypeptide isolé codé par la molécule d'acide nucléique isolée des revendications 1-5.

12. Polypeptide selon la revendication 11, lequel est immunogène.

13. Polypeptide selon la revendication 12, dans lequel le polypeptide, ou une partie du polypeptide, se lie à HLA ou un anticorps humain ou un fragment fixant un antigène de celui-ci.

14. Polypeptide selon la revendication 13, dans lequel ledit polypeptide est isolé.

15. Polypeptide de la revendication 14, dans lequel le polypeptide est une partie d'un complexe avec HLA.

16. Polypeptide de la revendication 14, dans lequel le polypeptide a une longueur comprise entre 8 et 12 acides aminés.

17. Polypeptide isolé comprenant un fragment du polypeptide de la revendication 14 de longueur suffisante pour représenter une séquence unique à l'intérieur du génome humain identifiant un polypeptide qui est un précurseur d'antigène associé au cancer humain.

18. Procédé de diagnostic d'un cancer, le procédé comprenant de mettre en contact un échantillon biologique isolé à partir d'un sujet avec un agent qui se lie spécifiquement à une molécule d'acide nucléique selon la revendication 1 et de déterminer l'interaction entre l'agent et la molécule d'acide nucléique en tant que détermination d'un cancer ;
dans lequel l'agent est une molécule d'acide nucléique qui s'hybride à 65 °C dans du tampon d'hybridation (SSC 3,5x, 0,02 % de Ficoll, 0,02 % de polyvinylpyrrolidone, 0,02 % d'albumine de sérum bovin, NaH₂PO₄ 2,5 mM (pH 7), 0,5 % de SDS, EDTA 2 mM) à une molécule selon la revendication 1 ou un fragment de celle-ci.

19. Procédé de diagnostic d'un cancer, le procédé comprenant de mettre en contact un échantillon biologique isolé à partir d'un sujet avec un anticorps ou un fragment fixant un antigène de celui-ci qui se lie spécifiquement à un polypeptide codé par une molécule d'acide nucléique selon la revendication 1 et de déterminer l'interaction entre l'agent et le polypeptide en tant que détermination d'un cancer.

20. Procédé selon la revendication 19, dans lequel le polypeptide est complexé avec une molécule HLA.

21. Procédé des revendications 18, 19 ou 20 dans lequel le cancer est un cancer du sein, de l'estomac, du poumon, de la prostate, du rein ou du colon.

22. Procédé pour déterminer la régression, la progression ou l'apparition d'un cancer **caractérisé par** l'expression de niveaux anormaux d'une protéine codée par une molécule d'acide nucléique selon la revendication 1, le procédé comprenant :
de suivre un échantillon provenant d'un patient qui a ou est suspecté d'avoir un cancer en ce qui concerne un paramètre choisi dans le groupe constitué par :
(i) la protéine,
(ii) un peptide dérivé de la protéine,
(iii) un anticorps qui se lie sélectivement à la protéine ou au peptide et
(iv) des lymphocytes T cytolytiques spécifiques pour un complexe du peptide dérivé de la protéine et d'une molécule MHC,
en tant que détermination de la régression, de la progression ou de l'apparition dudit cancer,
dans lequel l'échantillon est de préférence un fluide corporel, un épanchement corporel ou un tissu.

23. Procédé de la revendication 22, dans lequel l'étape de suivi comprend de mettre en contact l'échantillon avec un agent détectable choisi dans le groupe constitué par :
(a) un anticorps ou un fragment fixant un antigène de celui-ci qui se lie sélectivement à la protéine de (i), ou au peptide de (ii) ;
(b) une protéine ou un peptide qui se lie à l'anticorps de (iii) ; et
(c) une cellule qui présente le complexe du peptide et de la molécule MHC de (iv).

24. Procédé de la revendication 23, dans lequel l'anticorps, le fragment, la protéine, le peptide ou la cellule est marqué avec un marqueur radioactif ou une enzyme.

25. Procédé de la revendication 22, comprenant de doser le peptide dans l'échantillon.

26. Kit pour détecter la présence de l'expression d'un précurseur d'antigène associé au cancer humain comprenant :
une paire de molécules d'acide nucléique isolées chacune consistant en une molécule choisie dans le groupe constitué par :
(a) un segment contigu de 12-32 nucléotides de la séquence nucléotidique de l'une quelconque d'une molécule d'acide nucléique selon la revendication 1 ; et
(b) les compléments de (a), dans lesquels les segments contigus sont non chevauchants ;
et dans lequel la paire de molécules d'acide nucléique isolées amplifie sélectivement une molécule d'acide nucléique isolée selon la revendication 1.

27. Anticorps isolé ou fragment fixant un antigène de celui-ci qui se lie sélectivement à un complexe de :
(i) un peptide dérivé d'une protéine codée par une molécule d'acide nucléique selon la revendication 1 et
(ii) une molécule MHC à laquelle se lie le peptide pour former le complexe,
dans lequel l'anticorps isolé ne se lie pas à (i) ou (ii) seul,
dans lequel l'anticorps est de préférence un anticorps monoclonal, un anticorps chimère ou un anticorps humanisé.


SEQUENCE LISTING

<110> Ludwig Institute for Cancer Research
Old, Lloyd J.
Scanlan, Matthew J.
Stockert, Elisabeth
Gure, Ali
Chen, Yao-Tseng
Gout, Ivan
O'Hare, Michael
Obata, Yuichi
Pfreundschuh, Michael
Tureci, Ozlem
Sahin, Ugur

<120> CANCER-ASSOCIATED NUCLEIC ACIDS AND
POLYPEPTIDES

<130> L0461/7039/JRV/ERG

<140> Unknown
<141> 1998-07-15

<150> U.S. 08/896,164
<151> 1997-07-17

<150> U.S. 60/061,599
<151> 1997-10-10

<150> U.S. 60/061,765
<151> 1997-10-10

<150> U.S. 08/948,705
<151> 1997-10-10

<150> U.S. SNU (LUD5506.1)
<151> 1998-06-22

<150> U.K. 9721697.2
<151> 1997-10-11

<160> 816

<170> FastSEQ for Windows Version 3.0

<210> 1
<211> 474
<212> DNA
<213> Homo Sapiens

<400> 1

tctagactgt cctccccatc tgtggnacta acactatcat acagtcttgt cctatagggt 60
ctctggantc tgttcttcac acagagctgg gctcgagttt tcagtgcttt tgaatccang 120
cgctctgtct ctgggacngc ttcattcaag tcttgactta cgccatcttg gcaagatgtc 180
tgcttgccat ccaanttctc catggtatcc cctttanaga cttcaccaag agatgttctt 240
ctctcaaaga tgttgttatt attgntaact tgantcccat tttgcttcag taagcctgaa 300
tacctcagct tccaaatctc taatccgcac ttggaggcct tgcacttcca naagangngc 360
tttttcaaat cttcnagttt cnttcttctg ctcctgtctt tgnatagatc aanctcnttt 420
tgntgnnagt ccntnntnaa cntcnttgcn tgntggnngn tcntttncan nggc 474

<210> 2
<211> 1054
<212> DNA
<213> Homo Sapiens

<400> 2
tctagactgt cctccccatc tgtggangnn acactgatca tatcantctg ggtcctanca 60
gggtctctgg cgtctgtgct tcacnntant agctgggctc nantctncan ctgcttttga 120
atccacgngc tctgtctctg ggactgcttc attcnantct tgactnaggn catcttggca 180
agatgtctgc ttgccatccn agtnctcnat ggtatcccct ttcananact ccancnanta 240
gatnttctcc tctcnaanat gttgtccncc ntcgtnanct cgagtcccat tntgcttcan 300
taccctnant actcacnctc canatctccn ntccncacct ggngggcttn ctcttnccac 360
canatnactc tttcncnnaa tcttctacat tctctctttt ctgctccagt ctnttgatca 420
aaccnccctn nggttcnana tccncgatna acttctctgt cnngtngtnt ctcttttcct 480
cnggccgata ttggcctcnn ctcccntnnn aancatcccc ccncgtgtna nnttntttgg 540
ngccccccnt attcttccac ccctaaattn cctttntccc ccaattttcg ctgtantcgn 600
ttctccnctc ccannnccnc cncntatctn cccccncntt ngnntccccc cttnnaacnc 660
ccnctctctn ctaccnttct aacctaccct ctnntctncn annttccnct tctccttcta 720
nntnaancnt nttcacctac ngacattnat nnnntncncn ttcntcnnaa tcanattctn 780
tnctcccnnc ctccatctna tttctntnct ctatctnncn nattcnnctc attattccnt 840
ntactctnnc actcttctcn cnnntacatn ncancctccc anacancncc ctccanantn 900
tnttctctct tcctttctcc cctcctccnn ctccccactc ctccttcccc tntcttctnc 960
tacntntntt tcnctccttn natcnccctc atcttctccc ttctcccact catctantac 1020
ctccncnttc ttnantccnt ncctttntct antc 1054

<210> 3
<211> 441
<212> DNA
<213> Homo Sapiens

<400> 3
   attttgttgg aattttatct ttggcagata ggcagatata tttttgtgca tgtaaacaat 60
   cacatatata taaagctata taactgtaca tttttgacag ctttgtaatc acaggttgct 120
   ctgtcgtttc cctccataaa tggtttaatc tttgtttcct tcgctttttt tagagttgtc 180
   aattcatatt gccctctcca gtttgtacag tttcttagat gcttgttact tttaaaaagg 240
   atgttaggag aattcatttt ttntcataaa gaaatggaac actatttaaa ataaatttag 300
   cnccttgcaa ctagagcaat ctttttagtn ntacctctcc taaccgacta attcnacaga 360
   atctttgcan aaattacaca gtattacttt tgcntgtgtg tgatnaccac tgttcgtcng 420
   ccccttgaan gtttnacatc t 441

<210> 4
<211> 247
<212> DNA
<213> Homo Sapiens

<400> 4

accgctcacc gcagacgtgg tggctgcagt cagtcttccc gagtgaggga tttcgccgcc 60
cgctttcagg cccgtttggc ttaaataact gtgattgatg gccatgcngg anaaatatcc 120
aactgagggg atctctcncg tcacttcacc gagttccgat gtgattcana agggcanttc 180
cctggggact gaatggcata ccccantttt ctcggatccc tttcngagcc gcttcaatcg 240
ctgttct 247

<210> 5
<211> 486
<212> DNA
<213> Homo Sapiens

<400> 5
gaggaaagga acagatacct gccaagtcaa tcagagaatg gcggtaaaac ttgatggtta 60
gccgtctgtc tgttcatttt tatgaatgcc actatagaag taatctttag aaactggagc 120
tgttttcagt ttaatgacgt ccatggaatt aaccggattc attaataacg gcatcaggaa 180
gagcattgtt tgaaggatcc tctgcctagt ctattaatca gtttcctttt agacaggtgt 240
acaggtgatt tctggccctc attcacagaa tagaatagtg gtcgttgaga agcagaccta 300
gcacctagca agtcttcaga ccagtctttt aagtaatttt tattccctga gaagaaaatt 360
gaagaaacaa ttggggcaaa ganatttgtg gggttttact anaaagatct cnataaagca 420
aggactgtca ctctattcct cattcatatg agggttcaca ttaaaaatga cagtgttaca 480
cagggt 486

<210> 6
<211> 766
<212> DNA
<213> Homo Sapiens

<400> 6
ggggtcggag tctgggggtt cgcgcccgcc gacccgcgcc ctgctccctc tcagcacctg 60
ggcggacgaa atgaccatta agaagtagat gcccagatgc aaaagtgatg aaacagtcca 120
tttgtcataa agtaagatgc agctgtggca tgtcaaccag cttggaacaa aattgtatct 180
gtttttctca gaagagaatt ccacaaggtt aaatcagcaa acaaagaaaa catggtattt 240
tgaaatatga ttaaactcct gatgctgcag cagaggctaa gaatattaat ggccagatct 300
agtgcacaca tggtcttctg aagaagccat gggtagctgt tgtagctgtc cagataaaga 360
cactgtccca gataaccatc ggaacaagtt taaggtcatt aatgtggatg atgatgggaa 420
tgagttaggt tctggcataa tggaacttac agacacagaa ctgattttat acacccgcaa 480
acgtgactca gtaaaatggc actacctctg cctgcgacgc tatggctatg actcgaatct 540
cttttctttt gaaagtggtc caaggtgtca aactgggaca aggaatcttt gcctttaagt 600
gtgcccgtgc aaaaagaatt atttaacatg ttgcaaagag attatgcaaa aataatagta 660
taaatgttgg tggaagaagc caatttgtag aaagaaataa tcatcaagac aagaattgga 720
agtccctaan aacaacctcg aaacaactac aactccaaga ttttgc 766

<210> 7
<211> 567
<212> DNA
<213> Homo Sapiens

<400> 7
gagcacatgg cccaggggtg gagccgacct ccagacacca gaagaacaac ctaagttcct 60
cccacacagt cagacttgaa actagaggac agacagagaa ccaggaatgt ctactttgcc 120
cccatgagga atagaaacaa cttcctgact atcccttggt gggacaggag gactttcaag 180
aggatgaaac tctgaaaaag agtttaccct gtgattaatt aaatgaaatc ctcaatggcc 240
agagtaatta tacaaagatt aagtgtcaaa tgggagagag tttacctgga agcagtaaga 300
ttcgttgctt ttattggttc atttgttttg ttttctgctt tcggtagaaa tggatcttca 360
cgagtcagtt gaatttgatt aaacagaatt tagcatttca gccatttggg tgacagtggg 420

tgcattctaa agtcagcaca ccacccaaag tgatatatga ggtggaatgg gaggggtggc 480
aggtattcag cttaaaggac cgggatatca tgatcagtgg ctactgtttt acttttaaaa 540
accttccttt tctgggtcta gactcga 567

<210> 8
<211> 730
<212> DNA
<213> Homo Sapiens

<400> 8
gcgaggctca agcgggcctc tgcccccacc ttcgataacg actacagcct ctccgagctt 60
ctctcacagc tggactctgg agtttcccag gctgtcgagg gccccgagga gctcagccgc 120
agctcctctg agtccaagct gccatcgtcc ggcagtggga agaggctctc gggggtgtcc 180
tcggtggact ccgccttctc ttccagagga tcactgtcgc tgtcctttga gcgggaacct 240
tcaaccagcg atctgggtac cacagacgtc cagaagaaga agcttgtgga tgccatcgtg 300
tccggggaca ccagcaaact gatgaagatc ctgcagccgc aggacgtgga cctggcactg 360
gacagcggtg ccagcctgct gcacctggcg gtggaggccg ggcaagagga gtgcgccaag 420
tggctgctgc tcaacaatgc caaccccaac ctgagcaacc gtaggggctc caccccgttg 480
cacatggccg tggagaggag ggtgcggggt gtcgtggagc tcctgctggc acggnagatc 540
agtgtcaacg ccaaggatga ggaccagtgg acagccctcc actttgcanc ccanaacggg 600
ggatgagtct agcacacggc tgctgttggn naagaacgcc tcggtcaaac gaaggtggac 660
tttgagggcc ggacgcccat gcaagttggc ctgccaacaa cgggcaaggn gaatatcttg 720
cgcatcctgc 730

<210> 9
<211> 585
<212> DNA
<213> Homo Sapiens

<400> 9
ataatttgtt tcattttcaa ggcacaaaga gtttatgtca atcattttaa tgtctaagaa 60
tacaaagtta gcactagtaa catctggtag tctaatcatt tatcatgctt aaatgtaaca 120
ttacangnac tacattttaa aatctgcccc ctaaccagat gtgaaacaac gtggacaagg 180
gtgacatgtg ctagacccaa tctccaaaaa cgtatggttg acaaagacag ctgactgctg 240
gggtaaaact gcagcagtca taatcgaaga gcgaaagagg ccactctatt aaagactttg 300
tttcttttgc tagacatttt tcacctaatc ccaggatagt ttctgttaat gcatcttact 360
ctctttcaaa cgaatcgtcc ctagagcagg tgtacacatt aaaaatgagc tttatagcat 420
caaacacata ccacaaccaa ctctacaagg agggttttct gtaagatgtg tacgactgtc 480
cgaagaacac attctggctg ataagtctca agctcctgtg aggtcctgat gagtatctaa 540
acaacctcac atttctcttt ccacgcctan ggttaagggc agcac 585

<210> 10
<211> 661
<212> DNA
<213> Homo Sapiens

<400> 10
   tctagagttc gcacggaaac acgaagaata ggtggtgcat attttctggt ttggangtgg 60
   atccancctt ctgcatcctg tgacaaaata ttanttatac cttctaaagt atggcaatgt 120
   caancatttc atttanatan aananattca ccaccaaata gtttgacacc gtgtctaaag 180
   attcggaata tgtttgatcc cgttatggaa ataggggatc agtggcattt ggcaattcaa 240
   gaagcaattt tanaaanatg cantgataat gatggcattg ttcacattgc antanacaaa 300
   aattcacgtg agggttgtgt atatgttaaa tgtctgtctc cagaatatgc tngaaatgct 360
   tttaaatcat tgcatngctc ttgnttttat nggaaattgg ttacagtaaa atatttacga 420
   cnanatagat accaccatcg ctttccccan gctctcactt ncnacactcc attnaanncc 480
   atcaantatc atatnaactc natgtctcat cttcgtcttc ngactggnct aaccaattct 540
   caanggnant tcctgaaaan attttcttcc atttctaana ctgtttnttt acnatagnna 600
   aattccngtt tggctttttg tcttcccttt ttaaatcctt tttgtanntn atattttant 660
   g 661

<210> 11
<211> 1162
<212> DNA
<213> Homo Sapiens

<400> 11
tctagagggt ttttctcagg ggcttctctt agtgctgtng ctgctgcagg ctgcgcaggg 60
cctccgcaca tgcgcggata aggccacaca gctggatgct agtctccang gaggtgctag 120
agcccagttc agcagaggcc cangtcagct tctgcaagag ggctgtctgt gtgcaggcca 180
tgacatctgt atttggaggc acagcangag caggtggccc ctgggctgcc ttnagtcctg 240
ctgcagctcc ctcacagtgc tccggacnan gtactggggg ctggggcgca agtcnaganc 300
ccanggggan ctctgaggct gaggccanct ggtgctcccg agcttgggan aaggcaacct 360
gngcattcan ancgggatta tctttatcca cgtctgantc tagttcctga caagccacgc 420
aatanatttt ccgctgtttg tcttggagga ngatcgtccc gcagtccgca cacgtctcgc 480
ccancatgcg gtaaccgcgc aacanatant cncccatgan ccgggagatg cgatcttgcc 540
gctcccgtcn cgcctgcanc acttcgtctc cgcctcaatc gggggctccc aaganaantc 600
gtcnacttca actccgttca agggccatgt ttnccgtttt tcaaccgccc gggctcaaaa 660
ggaaatttac ttcaaaaccg gnancngccc tcccgcttcg gctccgcccc ttccaataac 720
ttccgccgga tnaanggcng accttnacnc cttaaccttn tcctcaanaa cctccccttt 780
tccccggggt tggnnccttt tccngntgct taccnaanaa acctttcttn tngggaannc 840
cttccnttna aacccgggaa ttcccaaggg gaacaaaaaa ctttcccnaa nnccccaaaa 900
gcctccctaa aggaancntt cccctttggt aaatncncct cccgnttccc aaaaaacctn 960
gaaaattgnc ctnannnggg ggntcacccc ctttcnnttn cctttcntgg nntttaaang 1020
gnnaacnacn tttgctttta aaaaaantnc ccnccttggc ntctannctn ccccctgggg 1080
gttccccttt ntgnnnncaa nncngnggnn natnnnnttc nttacaaaat nattnncanc 1140
cccnttttca aaagaaannt tg 1162

<210> 12
<211> 850
<212> DNA
<213> Homo Sapiens

<400> 12
tctagactgt cctccccatc tgtggaacta acactatcat acagtcttgt cctaaagggt 60
ctctggcgtc tgttcttcac agagagctgg gctcgagttt tcagtgcttt tgaatccagg 120
cgctctgtct ctgggactgc ttcattcaag tcttgactta ggccatcttg gcaagatgtc 180
tgcttgccat ccaagttctc catggtatcc cctttagaga cttcaccaag agatgttctt 240
ctctcaaaga tgttgttatt attgttaact tgagtcccat tttgcttcag tagcctgaat 300
acctcanctt ccaaatctct aatccgcact tggaggcctt gcacttccac aagatgagct 360
ttttccaaat cttctatttt ctttctttct gcttcctgtc ttttgatgaa atcaagctct 420
ttttgttgaa aatccttgat caacttcttt gccttgttgt atttcttttc caaggcctga 480
tactggcttt gagtctcttt gagatgctca ttcactgtgt ggcataatgt ttgggcctca 540
atccantanc tttccaactt caacattctt tccttantct cttctangtt ttgttgngan 600
ttgggttttt tctaanttcc caactcactt tctcctccgt gcccnaattc ccgggtacca 660
aacacaaatg ggnncgcctt cccnannaaa aannnaaaaa agctcntcca aaatctcngn 720
nnctttggnn taatcaatgg ncaananctg tttccctgnt ntnaaaattn nttntccgct 780
caaaaatttn ctnanantna cttttcaaat ttttttgcgc ccnccgttnc aanccnaana 840
ntannccaat 850

<210> 13

<211> 372
<212> DNA
<213> Homo Sapiens

<400> 13
tctagaatat ngaattttcc ctctttaaca cangggccct ccttgtcatt gaccttagct 60
aaaccatggc aattcataaa tagaggaaac attaatgaat taaaagcatt ccttattttt 120
taactaatat ttgtacattt tcttagtctc tttccaagtc tttgcctctt tttttctttt 180
atttttattt tttcctttga cagatggtat cccttcctgg atcattcatt tcaccttggt 240
ttctaacttt aggtttactt tcacttgtta tttgacttag caggtgcaac anaaacaaga 300
aacaaatgtg cccaccccac tttccgctta actgaaaagc ttaaaataaa tttctgaatt 360
atgtanaant tc 372

<210> 14
<211> 1167
<212> DNA
<213> Homo Sapiens

<400> 14
tctagactat ntgtcctttg ctcttcaaaa ccaggattcc ctaattctga actgcttata 60
agatagcaaa acaagtctgg ctaaactaca gtcaagagct taggatctat ggaagccaag 120
aaggcccagg gctccatgaa tgggctgggc aggggcacga ggccttgcct ttgtctgaga 180
agtcctcaca ccacagctga tcagatggaa acaatgggga ancagagttt cccggtgccg 240
tctttcccca gcccagattc caccaagcgc tggaaagcag agctgggaat ctcccaaggc 300
agagtccttc cagctttcct ccccctcaac ttcacactcc cctacctgtg gctctggaag 360
gagtcacact tgaacctcaa ccaaacttcc caatatcagt tggaagtcaa aaagatgaan 420
cctttctttc tggactttta gctacaaagg ggaacttcct gtgccaagcc ctggttcctc 480
ataccccaac gagtgctgtg ggtttcaaca ngtcttctct aaaaagggtg ccccanggtt 540
attgtgtgga gtctanggaa nctcatatcc tcaggatgat catttgtgtg ttggtccagt 600
gaaagcattt cccaactggt ccttccataa aactggggta tttcaantga ncacannaat 660
tcaacctatg gatttaaang ttnnanancc anaaacctta ngcaagtctt taanaantan 720
tgcanngtta attaaactta nctaagntgg ctaatttntt attcaangcg gggcataagg 780
tttcatcggc tctaaattgn ttaanatgnn ctgtaagntt gtngaanata tatccctggg 840
aagntnctaa aatangtttt ctngaactan cngtgctnta naanctnngn ggggntnang 900
ggggatccgg ggatccccan caatanaagc tntccngnnt ngcncctnca aaggngtaag 960
ccctgttngc ttnaaaanaa tccttncttg caancnaatg gatgtcntgg ggtancntgg 1020
naacntttgg tncccntnnn cctttgnnnn tatnaantnc nggnctgttc tccaactnna 1080
aaccttnnnn nnnnaagacc ccttngnaan accctttttg ntnanttttt cnncntgang 1140
ngaanatnnc tnnccncnnn antnttg 1167

<210> 15
<211> 1148
<212> DNA
<213> Homo Sapiens

<400> 15
tctagatttt tttttttttt ttttgaattc ttangcttgt tttacaaaac tttttattca 60
tcagagctgt agtgaaatat catcattgta attgatattc tagcactaca aaaggcacaa 120
tgaagcttat ttagttccag tactggaaat cagaggtaac agcacatcct tccttggaca 180
tgctttactc tgctgtagtg gtcatcacag ttttgatttt ctggataaga agttcaccac 240
agcatttgtg cattcatctg atagccatct tccctgaagg acattgcatt cttcagcatt 300
aacagcgtgt antttttctc tctctctttt cctgattacc tcttttgaaa ttctcaagac 360
atttggggga agctttgcaa atgccttcag cctggtccag acttctttct gaaagtgctt 420
atcagggaaa acttcagtaa caagtccttg nagcacatgc ctctcccgct gttaacttct 480
ttccaaaaat aagcatctct gttgccttgg ctgggctcat tatcttccgn aaagtgtaag 540

anggagcatc cttccgggac tttggnctan gtgacctaaa tggtgttatg aaaatgttgc 600
cctgtcagaa tgcatacacg ggaatcnnnt cncccaangg agggtnaacn ggnnatnccc 660
anaanctggn ccanttgnca nctgnannnc anangncttn gggnaaaatc naatnnnnnc 720
aancccaaca anatttcccc nccanttnna acnggnnatt aantttttaa cctttccncc 780
tccnantncc aaccaagggg ggaaanntcc aantnnnnnt tnncccnnat taattcccaa 840
nnancnggnn nntnnncnnc natttcccng gtnanaacna cttnntnntt nnnntccaac 900
ctttccngng naanntttna agtnnnacnc cannantttc cagtnnttna nnccnaannn 960
nttaagggnn nntttccctt ttttgggncc nnnnttannn annnnnnctt ttgnnnannc 1020
naaccttccg nngggncacn nncnagnnnt ttcaaaancc nanttnnntt cccnnnnnnn 1080
ntnnccaann nngnncanct gnntnnnngg tnccantnan nngnnntcna ancnngcnnn 1140
ncnnnttg 1148

<210> 16
<211> 1113
<212> DNA
<213> Homo Sapiens

<400> 16
tctagaaggt gctgccagac ggccccttga gctgggcttc ccgcacccac cttctgctcc 60
actgtcttct ccagcgactg gatgcgcatc tgctccttcc tcangctggc ctggagggcc 120
aacgcttccg cctgggcctt gctccggacc tgggcgatct cctcgtttgc canctgcagc 180
ttctcctccg cgtgggcctt canggcttgg tacctctggc cctcctgggt gatccttgcc 240
angtaatcct ccacgcactt cttcagtgac tcttcgttct tgcggtagcc ctcgatcacc 300
tctttctgtt tctcaaaacg cttgaagagg tcggagaagg acttctccat ggagttcana 360
tctgtggtaa gttggtcttt ttcttttana actttctgga tttcagcttt ggaaagttcc 420
ttctgcttct gaacttcctc catggcctgg tacacaacct cttcgaacct gtccatgatc 480
ttccccagtt ccaaggttct tcccgtggag ctcctcacac ctgctcctca gctcccggtt 540
ctcctcctgt gtcgccttta ccactgcatc caaagtcctt ctggctgtac tgggancaag 600
tccactatna ngtccgggtn gacaagggtg ggcccccaag cgcgggaaaa actggggggt 660
ctcntgccna attcccgggt acaacacaat tgngcgcttt cccganaaaa aaaaaaaaan 720
gctcgtccaa atttccggaa cttggcgtaa atcannggnc aataactgtt tccccngtgt 780
tnaaaatntt ttccgctcaa aanttgctaa anntactttt naaatttttn cnnccccctt 840
ttcaatccaa aatnanccca tngnannatt ccanccanna taanaacngg nttnaaatcc 900
cttnncnggg gggaatttgg cccaantatn aaanncntac cnaaaaaccn tccgnnttaa 960
ntccnaaaac ccttttcaaa anttaaacct aannnnanct ttnnnntaan ccaaanngtt 1020
ncccaaaaan ancttngggg tnaccaaggc aananngttt cccnggttaa aattttttac 1080
ccgcnaaaat tcananaaan tntnnnttan ntt 1113

<210> 17
<211> 731
<212> DNA
<213> Homo Sapiens

<400> 17
tctagagcct tccccggcca tctacaggca ggatgcggct gggaaaaaga caactggaat 60
ttctcgaagg ttgatggtcc gcacggttga ngattctacg tggttctctt ggttcccctg 120
gtgtgtgtgt gtgtgtggag gangccgcgg cccttagatn accttcttga gctcgtcgta 180
caggaccagc acgaangcgc cccccatgcc ccgcaggacg ttggaccacg cacccttgaa 240
naaggccttg cccccctcat ctctgaagat cttcctccaa cantcgacgg tgcccgtgta 300
catgatgtca gctcctttgc gcccggactg catcatcatt cgccgccgca ccgttcnaan 360
gggtangaca ccacgccggc cacngncgtc acggtctgcg cgatcatcca gctcaccacn 420
atgtgcgtgt tcttggggtc ngggancatn cccttggccg tatcgtanac nccgaantaa 480
gccgcccggt atatnatgat gccctgctcg tgccgaattc ccgggtncca acacaatggn 540
ncgcttcccg aanaaaaaaa ataaaaanct ncgtccaaat ctcggaactt tggcntantc 600
atgggcatan ctgttttccc tgtgtgaaat tntttccgct caacaatttg ctanaantaa 660

ttttcatttt tttgctcntc nnttatcanc caaanntact cccattantt attccaantn 720
tatnacaaag g 731

<210> 18
<211> 1145
<212> DNA
<213> Homo Sapiens

<400> 18
tctagactat cttcttcatc atcactctct gaatcatgaa cagtaattga ggttggtttt 60
actacacgct gaagaccact gggtcctgct tgttcctcat caacatccac aggaataatt 120
gcctggctgt gagctggagt attatttcca ctctcggcca ccacctctcc atcttcttgg 180
acgatgtctt ccatctcatt taatgcgtca gctgcaaact gctgctctcc tggctgcaga 240
tcttgccgaa agtcattctc ctcgtctgag tcatctggat gatggtgatt gttgtcgtgg 300
atgttggcat tgttattctg ggcattatta tcgtcattgt tggagttctg gttgctaaca 360
agagctgatg aaacaccaat tcctgtgcct gcactcagac caactcgagc acaacccttg 420
ggtggttcac gaaacatctg atccagtgaa ataaactcta ggctgggtaa taattcaata 480
aactggccaa aagagtccag cttcaaagca tggcaccgta ctaggttgat atcaaccaat 540
cgagtccatc ttgagtggtc tgagattcaa ttgtatgggt tgtgtanatn agggcaattg 600
taaattgcca gatatttgnt acaggnaaaa aacttcaatt gactgctttc atccctatat 660
caagtgatga taccaagatt tttctacaaa atcaagccaa accaatactt caccaagatt 720
cggcaattan ccaattccgg ctcctgttnn caaaaaactc atctncaatt atcccaanat 780
nnaaggggnt tcaaaactgg ggggaaaact ccaaantcct gncaaacctt tttnggggca 840
nntnnnnnnn caccaannct tttggtttan gggaangggt nggntttccg aanttttaag 900
gttancctaa cggggnnaan cnanttantg nntnnngggt nanccaannt tttannttta 960
annngncaat ttttttnnaa ncnccccaan nannaaanna annnntgnna anttttttna 1020
aaanccncct ggntttccaa antggtnntt ttaaaaaaan nttggggntn annncnnncc 1080
nangggnang gggnannccn nncnaatttt taannggttc caaanttttn gggganntnn 1140
ntttt 1145

<210> 19
<211> 1106
<212> DNA
<213> Homo Sapiens

<400> 19
tctagactat cttcttcatc atcactctct gaancatgaa cagtaattga ggttggtttt 60
actacacgct gaagaccact gggtcctgct tgttcctcat caacatccac aggaataatt 120
gcctggctgt gagctggagt attatttcca ctctcggcca ccacctctcc atcttcttgg 180
acgatgtctt ccatctcatt taatgcgtca gctgcaaact gctgctctcc tggctgcaga 240
tcttgccgaa agtcattctc ctcgtctgag tcatctggat gatggtgatt gttgtcgtgg 300
atgttggcat tgttattctg ggcattatta tcgtcattgt tggagttctg gtgctaacaa 360
gagctgatga aacaccaatt cctgtgcctg cactcagacc aactcgagca caacccttgg 420
gtggttcacg aaacatctga tccagtgaaa taaacctagg ctgggtaata attcaataaa 480
ctggccaaaa gagtccagct tcaaagcatg gcaccgtact angttgatat caaccaatcg 540
agtccatctt gagtggtctg agatccaatt gtatggggtt gtgtanatga nggcaattgt 600
aaattgccaa gatattttga tacaggaaaa aacttcaatg gactgctttc atccctatat 660
cantgatgat accaaggatt ttctancaca tcaagccaaa accaatactt naacaagatt 720
cgcaataacc attctgctcc tgtnncaaaa actcaatcct acaatatcct aaaatnaaag 780
ggnttcaaaa ctgggggaaa actccnaaat tcctgccaat cctttagggg aaaaaaaaag 840
acaccaatcc tttgggttaa gggaaaggnt ggattcngaa actttcaagt taaccttang 900
ggcaaacaan ttaangnatt aagggtaacc aaananttta aatttcaaag nngcaatttt 960
ttgcaancnt nccaaaanaa atanggttgn aaaattttct aaaaccaacc tgatttncaa 1020
aatggtcttt aaaaaaaatt ttggntnaaa accttcaaag gaaaagggaa cccaaccaan 1080
tttcaaannn nttccaaant tnngtt 1106

<210> 20
<211> 484
<212> DNA
<213> Homo Sapiens

<400> 20
acctgaaagc aagcccatta tgacaagctc agaggctttt gaacctccaa aatatttaat 60
gcttggtcaa caggcagtag gtggagttcc cattcagcct tccgtaagga ctcagatgtg 120
gcttacagag cagctgcgga caaatccttt ggaaggtaga aatacagagg attcttacag 180
tttagctcct tggcaacagc agcaaattga anactttcga caaggaagtg aaacaccaat 240
gcaggttttg actggatcat ctcgtcaaag ttattcacct ggctatcagg atttcagtaa 300
gtgggaatcn atgttgaaaa tnaaagaagg acttctaagg cagaaagaaa ttgtantcga 360
tcggcagaag caacnaatta cccacctgcn tnanangata agggataatg aattaccggc 420
tcnncatgcc atgttaggac attatgtnaa ttgtgaggat tcttatgtgg ctagtttgca 480
ccac 484

<210> 21
<211> 355
<212> DNA
<213> Homo Sapiens

<400> 21
cttccaagtt gctcttatca ggtactgctg atggtgcaga cctcaggaca gtagatccag 60
aaacacaggc tagactggaa gctttactag aagctgcagg aataggaaaa ttgtccacgg 120
ctgatggtaa agcctttgca gatcctgaag tacttcggag gttgacatcg tctgttagtt 180
gtgcgttgga tgaanctgct gctgcactta cccgtatgag agctgaaagc acagcaaatg 240
cagggcagtc ggacaaccgc agtttggcng aagcctgttc anaaggagat gtaantgctg 300
tgcgaaagtt actcattgaa gggcgaagtg tatttgaact cccngaggaa gggga 355

<210> 22
<211> 1070
<212> DNA
<213> Homo Sapiens

<400> 22
atgaaaaaga acaggataaa ccacctaatt tggttctgaa agataaagta aagcccaaac 60
aggatacaaa atacgatctt atattagatg agcaggccga agactcaaaa tcaagtcact 120
cacacacaag tnaaaaacac aagaagaaaa cccatcactg ttctgaagag aaagaagatg 180
aggactacat gccaatcaaa aatactaatc aggatatcta tagagaaatg gggtttggtc 240
actatgaaga agaagaaagc tgttgggaga aacaaaagag tgaaaagaga gaccgaactc 300
agaaccgaag tcgtagccga tctcgagaga gggatggcca ttatagtaat agtcataaat 360
caaaatacca aacagatctt tatgaaagag aaaggagtta aaagagagac cgaagcagaa 420
gtccnaagaa gtccnaagat aaagaaaaat ctaagtntng atgaaagatg aagaggcaga 480
attgagaggc taacatattt actcttgtct aacttaagag tgccaggaaa gcagatgctt 540
agattttgtg tccaagcttg ttattttttt canactagga ttatggtctt tagattaata 600
cngatnatat agagcacgga aagataaaga attgaacatt ttcttngtat acttttttac 660
actaatttca ttgctatacn taaanggtag tnttcatttt ctgaagtcta acattttcac 720
tcttttttta atgnagtatt tcatactaca aaaatacatn nacgtatata taaagggata 780
ataaangtan atatntgtgt antcatcagc cagcttaaga tacagatgtt gtcgacattt 840
tagaagttcc ctaaggccct ctccctctca aataattatt tggaattttg tgtttgtcat 900
ttgtctatta tagttttaca acanacgtat gtatntgtaa gtgaaatgtt aantttgtat 960
gtttctgaat tttatataaa tggcaaaang tttacttntg tgactttctt tcatttttat 1020
tgntanatag tattatataa atatactaca acttattcat ttcttgatgg 1070

<210> 23

<211> 861
<212> DNA
<213> Homo Sapiens

<400> 23
gaaagaccca ggagaagccg cccaaagaac tggtcaatga gtggtcattg aagataagaa 60
aggaaatgag agttgttgac aggcaaataa gggatatcca aagagaagaa gaaaaagtga 120
aacgatctgt gaaagatgct gccaagaagg gccagaagga tgtctgcata gttctggcca 180
aggagatgat caggtcaagg aaggctgtga gcaagctgtn tgcatccaaa gcacacatga 240
actcagtgct catggggatg aagaaccagc tcgcggtctt gcgagtggct ggttccctgc 300
agaagagcac agaagtgatg aaggccatgc aaagtcttgt gaagattcca gagattcagg 360
ccaccatgag ggagttgtcc aaagaaatga tgaaggctgg gatcatagag gagatgttag 420
aggacacttt tgaaagcatg gacgatcagg aagaaatgga ggaagaagca gaaatggaaa 480
ttgacagaat tctctttgaa attacagcag gggccttggg caaagcaccc agtaaagtga 540
ctgatgccct tccagagcca gaacctccag gagcgatggc tgcctcagag gatgaggagg 600
aggaggaaga ggntttggag gccatgcagt cccggctggc cacantccgc agctaggggc 660
tgccnacccc gctgggtgtg cacacactcc tntcaagagc tgccatttta tgtgtntctt 720
gcactacacc tctgttgtga ggactaccat tttggagaag gttctgtttg tctcttttca 780
ttctctgccc aggttttggg atcgcaaagg gattgttctt ataaaagtgg cataaataaa 840
tgcatcattt ttaggagtat a 861

<210> 24
<211> 985
<212> DNA
<213> Homo Sapiens

<400> 24
agggctcccg ggaagaaaca ctggcatttg tccctttgct tcggcttctg gaggcagana 60
ctctgagccc agggagagcc ttctgcagcc ccatttcctc aaaaatccaa cctgcccagg 120
tggcgggtca tgagctgtgc tcaggaagct ggaatctgac cctggtggcg tcgggcccag 180
tctccatggc agccgagcat ttattacccg ggcctccacc cagcttggca gactttanac 240
ttgaggctgg aggaaaggga actgaacgcg gttctgggag cagcaagccc acgggtagca 300
gcggaggccc cagaatggcc agtttntttc ccaagaccaa atttaatgag tacaaggatg 360
ttcttccctg tatgacaagc agcagagggg aagataaaag ccaccgactt catggtggcc 420
atgaggtgcc tgggggccag cccgacgcca ggggaggtgc agcggcacct gcagacccac 480
gggatagacg gaaatggaga gctggatttc tccacttttc tgaccattat gcacatgcaa 540
ataaaacaag aagacccaaa gaaagaaatt cttctagcca tgttgatggt ggacaaggag 600
aagaaaggtt acgtcatggc gtccgacctg cggtcaaaac tcacgagtnt gggggagaag 660
ctcacccaca aggaagtgga tgatcttttc agggaagcag atatcgaacc caatggcaaa 720
gtgaagtatg atgaatttat ccacaagatc acccttnctg gacgggacta ttgaaggagg 780
agaatgggag agcctcccct gggcctgaaa acttggagca attaattttt tttaaaaagt 840
gttcttttca cttgggagag atggcaaaca cagtggcaag acaacattac ccaactatag 900
aagagaggct aactagcaac aataatagat gatttcagcc atggtatgag tagatcttta 960
ataaaagatt tgtattgatt ttatt 985

<210> 25
<211> 545
<212> DNA
<213> Homo Sapiens

<400> 25
ctctctgctg catgcactat tgctgtaagt cttgctggaa tgagtacctg acaactcgga 60
tcgagcagaa ccttgttttg aattgcacct gccccattgc cgactgcccc gcccagccca 120
ccggagcctt cattcgtgcc atcgtctcct cgccagaggt catctccaag tatganaagg 180
cgctcctgcg tggctatgtg gagagctgct ccaacctgac ctggtgcacc aacccccagg 240

gctgcgaccg catcctgtgc cgccagggcc tgggctgtgg gaccacctgc tccaagtgtg 300
gctgggcctc ttgcttcaac tgtagcttcc ctgaggcaca ctaccctgct agctgtggcc 360
atatgtctca gtgggtcgat gacggtggct actatgacgg catgagcgtg gaggcgcana 420
ncaagcacct ggccaagctc atctccaagc gctgtcccag ctgtcaggct cccatcgaga 480
anaacgaagg gtgcctgcac atgacctgtg ccaaatgtaa ccatggattc tgctggcgct 540
gcctc 545

<210> 26
<211> 374
<212> DNA
<213> Homo Sapiens

<400> 26
gggaaaaggg cctccacatt gatcagctgg tttgtctggt gctggaggcc tgncagaagg 60
gtccaaatcc tcctggaacc ctgggccaca ctgttgctgg gggtgtggcc tgtaccacta 120
canatgtcct ctcttgcntc ctgcacctct taagccaggg ctacntgaaa cngcgtgatn 180
accggcccca aatcctgatg tatgccgctc catancccat ggggccctgc cggggtcntg 240
cacatntcnc tttctgtggc agccanagcc aaacctccca ncccncccca aaacctgtgg 300
ctaccctgnn atctctacan ctgccttgcn ngccgcttcn tnanccncca ctaaaatnta 360
acggttgatt aacc 374

<210> 27
<211> 552
<212> DNA
<213> Homo Sapiens

<400> 27
cacaaacctt gcaatccaag ggaaaaggag cgaatccaaa atgcaggagg cagcgtgatg 60
atacaacgtg ttaatggttc attagcagta tctcgtgctc tgggggacta tgattacaag 120
tgtgttgatg gcaagggccc aacagaacaa cttgtttctc cagagcctga ggtttatgaa 180
attttaagag cagaagagga tgaatttatc atcttggctt gtgatgggat ctgggatgtt 240
atgagtaatg aggagctctg tgaatatgtt aaatctaggc ttgaggtatc tgatgacctg 300
gaaaatgtgt gcaattgggt agtggacact tgtttacaca agggaagtcg agataacatg 360
agtattgtac tagtttgctt ttcaaatgct cccaaggtct cagatgaagc ggtgaaaaaa 420
gattcagagt tggataagca cttggaatca cgggttgaag anattatgga gaatctggcg 480
aagaangaat gcctgatctt gcccatgtca tgcgcatctt gtctgcagaa aatatcccaa 540
atttgcctcc tg 552

<210> 28
<211> 502
<212> DNA
<213> Homo Sapiens

<400> 28
ctgacctgcc cacactggaa gaccatcaga agcagagcca gcagcttaag gattctgagt 60
tgaagagcac agagctgcag gagaaagtga ctgagctgga gagtttgctg gaggagaccc 120
aggcaatctg cagagagaag gagattcaac tggaaagcct gaggcagaga gaagcagaat 180
tctcctccgc tggacatagc ctgcaagata aacagtctgt ggaggagacc agtggagaag 240
gtccagaagt ggaaatggag tcctggcaga agcgatacga ttcgctccaa aagattgtgg 300
agaagcagca gcagaagatg gatcagttgc gctcacaagt ncagagccta gagcaggaag 360
tggctcnaga agaaggaaca agccaggccc tgagagagga ggcccagcga agggattcag 420
ccctgcagca gctgcgcaca gccgtgaagg anctttcagt gcnaaaccag gacttgattg 480
agaagaatct gacactccag ga 502

<210> 29

<211> 537
<212> DNA
<213> Homo Sapiens

<400> 29
gctttgggga ctcagtggac tgctctgact gctggcttcc ggtggtgaaa ttcatcgagg 60
agcaatttga gcagtacctt agggatgaga gtggcctgaa ccggaagaac atccaggact 120
cccgagtcca ctgctgcctc tacttcatct cacccttcgg ccgggggctc cggcccctan 180
atgtggcctt cctccgggca gtacacgana aagtcaacat catcccagtc attggcaaag 240
cggatgctct gatgccccag gaaacccagg ccctcaagca gaagatccgg gatcagttga 300
aggaagagga gatccacatc taccagttcc ccgaatgtga ctctgatgaa gatgaagact 360
tcaagaggca ggatgcagan atgaaggaaa gcatcccttt tgcagtcgtg ggatcatgcc 420
aagtggtgag ggatggcggg aaccggccgg tgaggggacg ccgctactcc tgggggaacg 480
tggaagtgga naaccacatc nctgcgattt cctgaacctg cgacggatgc tggtgca 537

<210> 30
<211> 3872
<212> DNA
<213> Homo Sapiens

<400> 30
   ccattgcaca cagacaggca gcatggctag caaacgaaaa tctacaactc catgcatggt 60
   tcggacatca caagtagtag aacaagatgt gcccgaggca agtagacagg gccaaagaga 120
   aaggaatcgg cacaccacag cctgacgtgg ccaaggacag ttgggcanca gaacttgaaa 180
   actcttccaa agaaaacgaa gtgatagagg tgaaatctat gggggaaagc cagtccaaaa 240
   aactccaagg tggttatgag tgcaaatact gcccctactc cacgcaaaac ctgaacgagt 300
   tcacggagca tgtcgacatg cagcatccca acgtgattct caaccccctc tacgtgtgtg 360
   cagaatgtaa cttcacaacc aaaaagtacg actccctatc cgaccacaac tccaagttcc 420
   atcccgggga ggccaacttc aagctgaagt taattaaacg caataatcaa actgtcttgg 480
   aacagtccat cgaaaccacc aaccatgtcg tgtccatcac caccagtggc cctggaactg 540
   gtgacagtga ttctgggatc tcggtgagta aaacccccat catgaagcct ggaaaaccaa 600
   aagcggatgc caagaaggtg cccaagaagc ccgaggagat cacccccgag aaccacgtgg 660
   aagggaccgc ccgcctggtg acagacacag ctgagatcct ctcgagactc ggcggggtgg 720
   agctcctcca agacacatta ggacacgtca tgccttctgt acagctgcca ccaaatatca 780
   accttgtgcc caaggtccct gtcccactaa atactaccaa atacaactct gccctggata 840
   caaatgccac gatgatcaac tctttcaaca agtttcctta cccgacccag gctgagttgt 900
   cctggctgac agctgcctcc aaacacccag aggagcacat cagaatctgg tttgccaccc 960
   agcgcttaaa gcatggcatc agctggtccc cagaagaggt ggaggaggcc cggaagaaga 1020
   tgttcaacgg caccatccag tcagtacccc cgaccatcac tgtgctgccc gcccagttgg 1080
   cccccacaaa gatgacgcag cccatcctcc agacggctct accgtgccag atcctcggcc 1140
   agactagcct ggtgctgact caggtgacca gcgggtcaac aaccgtctct tgctccccca 1200
   tcacacttgc cgtggcagga gtcaccaacc atggccagaa gagacccttg gtgactcccc 1260
   aagctgcccc cgaacccaag cgtccacaca tcgctcaggt gccagagccc ccacccaagg 1320
   tggccaaccc cccgctcaca ccagccagtg accgcaagaa gacaaaggag cagatagcac 1380
   atctcaaggc cagctttctc cagagccagt tccctgacga tgccgaggtt taccggctca 1440
   tcgaggtgac tggccttgcc aggagcgaga tcaagaagtg gttcagtgac caccgatatc 1500
   ggtgtcaaag gggcatcgtc cacatcacca gcgaatccct tgccaaagac cagttggcca 1560
   tcgcggcctc ccgacacggt cgcacgtatc atgcgtaccc agactttgcc ccccagaagt 1620
   tcaaagagaa aacacagggt caggttaaaa tcttggaaga cagctttttg aaaagttctt 1680
   ttcctaccca agcagaactg gatcggctaa gggtggagac caagctgagc aggagagaga 1740
   tcgactcctg gttctcggag aggcggaagc ttcgagacag catggaacaa gctgtcttgg 1800
   attccatggg gtctgggcaa aaaaggccaa gatgtgggaa gcccccaatg gtgctctgtc 1860
   tcgactcgaa cagctctccg gtgcccagtt aacaagttct ctgcccagcc cttcgccagc 1920
   aatttgcaaa aagtcaagaa cangttcatc tcctgaagga gcacgtttgc aanaaaccca 1980
   nttgggctac tccccangag taacgaccag tttaaccggg ccaagancng gnctgggtcc 2040
   cgaactgaaa attgtgcntt tgggttcang gngaacaaga nngcttnccn gaaaacgggg 2100
   aaccgttaaa attggnttgg agcaaatnnc aagnaaccaa gcccaatggg caaaattgnt 2160
   caacgggtta ccnaatgccg nttcnaaggg aaanncaaca aanacccaan ggccgganan 2220
   gcccaaagaa acgggggntt aatgttggtt cccacaatta ttacaaggga cccccaaaaa 2280
   agctcttgcg aaggaggact ttgganaaan tttgttgacc agggtaaaan tagggcaggg 2340
   acccagcaaa aagactgttt tcccagcaaa gcccttcaga ggccaccttc agaccgttca 2400
   gagggcagca gccgggacgg ccagggtagc gacgagaacg aggagtcgag cgttgtggat 2460
   tacgtggagg tgacggtcgg ggaggaggat gccatcttca gatagatcag atagctggag 2520
   tcaggntgcg gcagaaggtg tgtcggaact ggctgaatca gactccgact gcgtccctgc 2580
   agaggctggc caggcctaga cagggaagtc tgttagaact gctgtgctga tcaacgggac 2640
   gctccgtctt tgaagaaaga agagatggtc tctccccagc catgggccac ccttgccagt 2700
   gactccaagt ggaactactt agctcgcgtg tgcctggagg gtgcgggaag tccagcgact 2760
   ctcagacgca cctcccagag gaccggtggg aattgttcat agtgccaaag tcctactact 2820
   gcgttttcaa tgggtccttg tacatagttt gctcctctgc cctagccctc acctcttgct 2880
   atactggaac cgatttgtac aatgtgggaa ttttgttacc tttttaatca agggcaactt 2940
   ccttttccag cactaccatt gtaaggtttt tttcaggagg gagggctaac caccttgctt 3000
   ttctcttttc tctttttctt tttttatttt ttgttttatt aatttgggga aaggggtgtt 3060
   agcattagtg ccatgatatc tactggattt taagtaggga gactttattt ttaaaggtag 3120
   gttgaaattt gggagatttc tcggcaggaa gggctgaaat ccaggcccct gtctcaactt 3180
   ggagagaggt gacagacggc agatcttcca aatcaaattc ctttccagtt cttcccctgg 3240
   ctgccttttt gggggtccct gccttagccc cacacaaggc tttctgaact gccaagaggg 3300
   gatctggctt ctcaactgct cggcctcttg ggccaggctg tgcccagcca gccctgggag 3360
   aactgggtag caggtggctg acttctttaa gcacctttct aaataccagc agaagaggct 3420
   cccgcctctg ttagcatgat cagtactatt gtgacattaa aacaacaaca ataagatctt 3480
   cctatctgga gggtacagag gtgaatggct ttggttttca tttctctttc ttcactgctt 3540
   ttctcggtgt ggtatttgac aagattttag ntnaaagcct caccatgaat tgattttttt 3600
   tgtttgngng ngtgtttgtt ttgggacaat tttagatacc tgngtgcant ttttcagtta 3660
   gtcctaantt ttaaaagaag ggnaaccaag nganatattt ggtgtaagtg ttgcagtatg 3720
   aanttctggt tgcaatccct ccccgtccca cantgccccc catttgagta caccgcacaa 3780
   gtcaaacgnt aggnagtttg nataaaacca atttttctaa nttgttgntc atttgttgta 3840
   antcaataaa gcnaaganta aacattttta tt 3872

<210> 31
<211> 655
<212> DNA
<213> Homo Sapiens

<400> 31
gaaatcatgg gagcacaaag aagaaataag tgaagcagag ccagggggtg gttccttggg 60
agatggaagg ccgccagagg aaagtgccca tgaaatgatg gaggaggaag aggaaatccc 120
aaaacctaag tctgtggttg caccgccagg tgctcctaag aaagagcatg taaatgtagt 180
attcattggg cacgtanatg ctggcaagtc aaccattgga ggacaaataa tgtatttgac 240
tggaatggtt gacaaaagga cgcttgaaaa gtatgaaaga gaagctaaag agaaaaacag 300
agaaacttgg tacttgtctt gggccttaga cacaaatcag gaagaacgag acaagggtaa 360
aacagtagaa gtgggtcgtg cctattttga aaccgaaaag aacatttcac aattctagat 420
atgaatccca gaacactgag ctcaaaaccc aaagcccaga atttgaagct caaagttccn 480
aattccanga aggtgcggag atgcttctga accccgagga aaagatcctt tgaatatctc 540
cgtaggagtt caccccctgg actccttcac tcaggggttt ggggagcacc cacaggggac 600
ctgcccatag ggccaccttt tgagatgccc acaggggccc tgctgtctac accgc 655

<210> 32
<211> 466
<212> DNA
<213> Homo Sapiens

<400> 32
gaaaggtggc cagaggaagg gacagctgac ctggcacaat ctgggcttga agggggcaca 60
acaagagcgt ctgtgagctg gtgctgtctg gagggatctt ggctcctctc cggctatctg 120
accttcctga agacctgctc gcacactgca tcccttgcag tcagttccag ctcgtgccga 180
attcggcacg agctcgtgcc gaattcggca cgagggaagc actactccca gcgctgggcc 240
caggaggacc tgctggagga gcagaaggat ggggcccggg cagcggctgt ggctgacaag 300
aagaaaggcc tcatggggcc actgaccgaa ctggacacta aagatgtgga tgccctgctg 360
aagaagtctg aggcccagca tgaacagccg gaagatggat gcccctttgg tgccctgacg 420
cagcgcctcc tgcaggccct ggtggaggaa aatattattt tttccc 466

<210> 33
<211> 293
<212> DNA
<213> Homo Sapiens

<400> 33
gtcggcgccc tacatgagaa gcatgatgca gtcgctgagc cagaatccag atttggctgc 60
acagatgatg ctgaatagcc cgctgtttac tgcaaatcct cagctgcagg agcagatgcg 120
gccacagctc ccagccttcc tgcagcagat gcagaatcca gacacactat cagccatgtc 180
aaacccaaga gcaatgcagg ctttaatgca gatccagcag gggctacaga cattagccac 240
tgaagcacct ggcctgattc cgagcttcac tccaggtgtg ggggtggggn tct 293

<210> 34
<211> 456
<212> DNA
<213> Homo Sapiens

<400> 34
caaagcctta agtcagagcc tgctagaatg tgtccaggtt tacagctata ctgcacagca 60
cttcccatgc tagtctctgt aaaacgcaaa aagccatttt caggagcagt aggcaagtca 120
cattcaattg aaatgcagga tggtgcactg ccattccaag ttccatcttc ctggcagatc 180
agcacagggt tccccagaag ttcatatcct ggattacagg tgtatgaaac catggtacca 240
tacagaaagt ttgatgaatg tgtagcagga gactcctttg tattttccca ggttttagcc 300
actgctccca aatgataagg agggtgagga gtcacatatg gaacttccat catgtcgtct 360
tcttgctcaa aatatccctg gtcatctttg agtttagtac agtctccaaa atctatatga 420
ggagggaggc cacagtctat tggcatacca aatttt 456

<210> 35
<211> 679
<212> DNA
<213> Homo Sapiens

<400> 35
ggcggcgttc gtgtccgagg tcactagttt cccggtagtt cagctgcaca tgaatagaac 60
agcaatgaga gccagtcaga aggactttga aaattcaata aatcaagtga aactcttgaa 120
aaaggatcca ggaaacgaag tgaagctaaa actctacgcg ctatataagc aggccactga 180
aggaccttgt aacatgccca aaccaggtgt atttgacttg atcaacaagg ccaaatggga 240
cgcatggaat gcccttggca gcctgcccaa ggaagctgcc aggcagaact atgtggattt 300
ggtgtccagt ttgagtcctt cattggaatc ctctagtcag gtggagcctg gaacagacag 360
gaaatcaact gggtttgaaa ctctggtggt gacctccgaa gatggcatca caaagatcat 420
gttcaaccgg cccaaaaaga aaaatgccat aaacactgag atgtatcatg aaattatgcg 480
tgcacttaaa gctgccagca aggatgactc aatcatcact gttttaacag gaaatggtga 540
ctattacagt agtgggaatg atctgactaa cttcactgat attccccctg gtggagtana 600
ggagaaagct aaaaataatg ccgttttact gaagggaatt tgtgggctgt tttatagaat 660
ttcctaagcc tctgattgc 679

<210> 36
<211> 689
<212> DNA
<213> Homo Sapiens

<400> 36
ctaaaccagt ggacatatca ggccatggtc cacgaactac taggcataaa caacaatcgg 60
attgatcttt ccagagtgcc gggaatcagt aaagacttaa gagaagtggt cctatctgct 120
gaaaatgatg aattctatgc taataatatg tacctgaact ttgctgagat tggtagcaat 180
ataaagaatc tcatggaaga ttttcagaag aagaaaccaa aagaacagca aaaactagaa 240
tcaatagcag acatgaaggc gtttgttgag aattatccac agttcaagaa aatgtctggg 300
actgtttcaa agcatgtgac agtggttgga gaactgtctc gattggtcag tgaacggaat 360
ctgctggagg tttcagaggt tgagcaagaa ctggcctgtc aaaatgacca ttctagtgct 420
ctccagaata taaaaaggct tctgcagaac cccaaagtga cagagtttga tgctgcccgc 480
ctggtgatgc tttatgcttt acattatgag cgacacagca gcaatagcct gccaggacta 540
atgatggncc tcaggaataa aggtgtttct gagaagtatc gaaagctcgt gtctgcagtt 600
gttgaatatg gtggtaaaac gagtcagagg aagtgacctc ctcagcccca aagatgctgt 660
tggctatcac caaacaattc ctcaaaggg 689

<210> 37
<211> 443
<212> DNA
<213> Homo Sapiens

<400> 37
ccacgcccgg ccccggagca ggcttttacg catgccccgc gcgccccctt gtgtccggaa 60
tttattcctt ccggtgggtt cgcggtctag ctgaccaaga acggaactgg ggactttcgc 120
agtgagagtt acagctctta aagatggcac cgacccaggc cgggcgcggt ggctcaggcc 180
tgcaatccca gcactttggg aggcggaggc aggtgaatca cgaggtcagg aaatcgagac 240
catcctggct aacatggtga aaccccgtct ccactaaaaa tacaaaaaat tagccaggca 300
tggtggctgg cacctgtagt cccagctact tgggaggctg agccaggaaa gtggcatgaa 360
cccgcgaggc agagcttgca ataagccgag atcgtgccaa tgcactccag cctgggcaac 420
agaaggagac actgtctcaa aaa 443

<210> 38
<211> 442
<212> DNA
<213> Homo Sapiens

<400> 38
ctcgcctcgg agcagccatg atggaaggcc tggacgacgg cccggacttc ctctcagaag 60
aggaccgcgg acttaaagca ataaatgtag atcttcaaag tgatgctgct ctgcaggtgg 120
acatttctga tgctcttagt gagcgggata aagtaaaatt cactgttcac acaaagattc 180
caccagcacc accaagacct gattttgatg cttcaaggga aaaactacag aagcttggtg 240
aaggagaagg gtcaatgacg aaggaagaat tcacaaagat gaaacaggaa ctggaagctg 300
aatatttggc aatattcaag aagacagttg cgatgcatga agtgttcctg tgtcgtgtgg 360
cagcacatcc tattttgaga agagatttaa atttccatgt cttcttggaa tataatcaag 420
atttgagtgt gcgaggaaaa aa 442

<210> 39
<211> 692
<212> DNA
<213> Homo Sapiens

<400> 39

cagggacagg ccctatctta tatttttttc catcttcatc atccacttct gcttacagtt 60
tgctgcttac aataacttaa tgatggattg agttatctgg gtggtctcta gccatctggg 120
cagtgtggtt ctgtctaacc aaagggcatt ggcctcaaac cctgcatttg gtttaggggc 180
taacagagct cctcagataa tcttcacaca catgtaactg ctggagatct tattctatta 240
tgaataagaa acgagaagtt tttccaaagt gttagtcagg atctgaaggc tgtcattcag 300
ataacccagc ttttcctttt ggcttttagc ccattcagac tttgccagag tcaagccaag 360
gattgctttt ttgctacagt tttctgccaa atggcctagt tcctgagtac ctggaaacca 420
gagagaaaga ggatccagga tgtacttgga tgaggaggcc tggcttatct aggaagtcgt 480
gtctggggtg cttattgctg ctccatacag ctgtacgtca gccccttggc cttctctgta 540
ggttcttggc ancaatgagc agctttcact caagtgacac aagtaattac tgagtcctaa 600
tttgatagcc accaactgta cctgggtang caaagtcaga tttttgagaa nctttttcct 660
gatttgaagt tttaattacc ttaatttcct tt 692

<210> 40
<211> 619
<212> DNA
<213> Homo Sapiens

<400> 40
gaggcaccag attctgctga ggggaccacc cttacagtgc tgcctgaagg tgaggagttg 60
cccctgtgtg tgagtgagag caatggcctg gagctcccac cctcagcagc atctgatgag 120
ccacttcagg agccactgga ggctgacagg acctcggaag agctgacaga ggccaagacc 180
ccaacctcca gcccagagaa gccacaggaa ctcgttacag ctgaggttgc agctccatcc 240
acctcatctt cagccacttc ctcgcctgag ggtccttcac ctgcccgacc tcctcggcgt 300
cgcaccagtg ctgatgtgga aattaggggt caagggactg gtcggccagg acaaccacca 360
ggccccaaag tgcttcgaaa gctgccagga cggctggtaa ctgtggtaga ggaaaaggaa 420
ctggtgcggc ggcggcggca gcagcgggga gctgccaanc accctagtgc ctggggtctc 480
tgagactagt gccagcccgg gaagcccgtc tgtccgcagc atgtcanggc canaatcctc 540
ccctcccatt ggtgggccct gtgaaagctg ctccttcatc cncactgcnc actccanccc 600
agnagccctt cattgcncg 619

<210> 41
<211> 153
<212> PRT
<213> Homo Sapiens

<400> 41
Pro Glu Ser Lys Pro Ile Met Thr Ser Ser Glu Ala Phe Glu Pro Pro
1               5                   10                  15
Lys Tyr Leu Met Leu Gly Gln Gln Ala Val Gly Gly Val Pro Ile Gln
            20                  25                  30
Pro Ser Val Arg Thr Gln Met Trp Leu Thr Glu Gln Leu Arg Thr Asn
        35                  40                  45
Pro Leu Glu Gly Arg Asn Thr Glu Asp Ser Tyr Ser Leu Ala Pro Trp
    50                  55                  60
Gln Gln Gln Gln Ile Glu Phe Arg Gln Gly Ser Glu Thr Pro Met Gln
65                  70                  75                  80
Val Leu Thr Gly Ser Ser Arg Gln Ser Tyr Ser Pro Gly Tyr Gln Asp
                85                  90                  95
Phe Ser Lys Trp Glu Ser Met Leu Lys Lys Glu Gly Leu Leu Arg Gln
            100                 105                 110
Lys Glu Ile Val Asp Arg Gln Lys Gln Ile Thr His Leu Ile Arg Asp
        115                 120                 125
Asn Glu Leu Pro Ala His Ala Met Leu Gly His Tyr Val Asn Cys Glu
    130                 135                 140

Asp Ser Tyr Val Ala Ser Leu His His
145                 150

<210> 42
<211> 95
<212> PRT
<213> Homo Sapiens

<400> 42
Ile Leu Leu Glu Phe Tyr Leu Trp Gln Ile Gly Arg Tyr Ile Phe Val
1               5                   10                  15
His Val Asn Asn His Ile Tyr Ile Lys Leu Tyr Asn Cys Thr Phe Leu
            20                  25                  30
Thr Ala Leu Ser Gln Val Ala Leu Ser Phe Pro Ser Ile Asn Gly Leu
        35                  40                  45
Ile Phe Val Ser Phe Ala Phe Phe Arg Val Val Asn Ser Tyr Cys Pro
    50                  55                  60
Leu Gln Phe Val Gln Phe Leu Arg Cys Leu Leu Leu Leu Lys Arg Met
65                  70                  75                  80
Leu Gly Glu Phe Ile Phe His Lys Glu Met Glu His Tyr Leu Lys
                85                  90                  95

<210> 43
<211> 114
<212> PRT
<213> Homo Sapiens

<400> 43
Ser Lys Leu Leu Leu Ser Gly Thr Ala Asp Gly Ala Asp Leu Arg Thr
1               5                   10                  15
Val Asp Pro Glu Thr Gln Ala Arg Leu Glu Ala Leu Leu Glu Ala Ala
            20                  25                  30
Gly Ile Gly Lys Leu Ser Thr Ala Asp Gly Lys Ala Phe Ala Asp Pro
        35                  40                  45
Glu Val Leu Arg Arg Leu Thr Ser Ser Val Ser Cys Ala Leu Asp Glu
    50                  55                  60
Ala Ala Ala Leu Thr Arg Met Arg Ala Glu Ser Thr Ala Asn Ala Gly
65                  70                  75                  80
Gln Ser Asp Asn Arg Ser Leu Ala Glu Ala Cys Ser Gly Asp Val Ala
                85                  90                  95
Val Arg Lys Leu Leu Ile Glu Gly Arg Ser Val Phe Glu Leu Pro Glu
            100                 105                 110
Glu Gly

<210> 44
<211> 132
<212> PRT
<213> Homo Sapiens

<400> 44
Gly Glu Lys Glu Gln Asp Lys Pro Pro Asn Leu Val Leu Lys Asp Lys
1               5                   10                  15
Val Lys Pro Lys Gln Asp Thr Lys Tyr Asp Leu Ile Leu Asp Glu Gln
            20                  25                  30

Ala Glu Asp Ser Lys Ser Ser His Ser His Thr Ser Lys His Lys Lys
        35                  40                  45
Lys Thr His His Cys Ser Glu Glu Lys Glu Asp Glu Asp Tyr Met Pro
    50                  55                  60
Ile Lys Asn Thr Asn Gln Asp Ile Tyr Arg Glu Met Gly Phe Gly His
65                  70                  75                  80
Tyr Glu Glu Glu Glu Ser Cys Trp Glu Lys Gln Lys Ser Glu Lys Arg
                85                  90                  95
Asp Arg Thr Gln Asn Arg Ser Arg Ser Arg Ser Arg Glu Arg Asp Gly
            100                 105                 110
His Tyr Ser Asn Ser His Lys Ser Lys Tyr Gln Thr Asp Leu Tyr Glu
        115                 120                 125
Arg Glu Arg Ser
    130

<210> 45
<211> 214
<212> PRT
<213> Homo Sapiens

<400> 45
Lys Thr Gln Glu Lys Pro Pro Lys Glu Leu Val Asn Glu Trp Ser Leu
1               5                   10                  15
Lys Ile Arg Lys Glu Met Arg Val Val Asp Arg Gln Ile Arg Asp Ile
            20                  25                  30
Gln Arg Glu Glu Glu Lys Val Lys Arg Ser Val Lys Asp Ala Ala Lys
        35                  40                  45
Lys Gly Gln Lys Asp Val Cys Ile Val Leu Ala Lys Glu Met Ile Arg
    50                  55                  60
Ser Arg Lys Ala Val Ser Lys Leu Ala Ser Lys Ala His Met Asn Ser
65                  70                  75                  80
Val Leu Met Gly Met Lys Asn Gln Leu Ala Val Leu Arg Val Ala Gly
                85                  90                  95
Ser Leu Gln Lys Ser Thr Glu Val Met Lys Ala Met Gln Ser Leu Val
            100                 105                 110
Lys Ile Pro Glu Ile Gln Ala Thr Met Arg Glu Leu Ser Lys Glu Met
        115                 120                 125
Met Lys Ala Gly Ile Ile Glu Glu Met Leu Glu Asp Thr Phe Glu Ser
    130                 135                 140
Met Asp Asp Gln Glu Glu Met Glu Glu Glu Ala Glu Met Glu Ile Asp
145                 150                 155                 160
Arg Ile Leu Phe Glu Ile Thr Ala Gly Ala Leu Gly Lys Ala Pro Ser
                165                 170                 175
Lys Val Thr Asp Ala Leu Pro Glu Pro Glu Pro Pro Gly Ala Met Ala
            180                 185                 190
Ala Ser Glu Asp Glu Glu Glu Glu Glu Glu Leu Glu Ala Met Gln Ser
        195                 200                 205
Arg Leu Ala Thr Arg Ser
    210

<210> 46
<211> 248
<212> PRT
<213> Homo Sapiens

<400> 46
Gly Ser Arg Glu Glu Thr Leu Ala Phe Val Pro Leu Leu Arg Leu Leu
1               5                   10                  15
Glu Ala Thr Leu Ser Pro Gly Arg Ala Phe Cys Ser Pro Ile Ser Ser
            20                  25                  30
Lys Ile Gln Pro Ala Gln Val Ala Gly His Glu Leu Cys Ser Gly Ser
        35                  40                  45
Trp Asn Leu Thr Leu Val Ala Ser Gly Pro Val Ser Met Ala Ala Glu
    50                  55                  60
His Leu Leu Pro Gly Pro Pro Pro Ser Leu Ala Asp Phe Leu Glu Ala
65                  70                  75                  80
Gly Gly Lys Gly Thr Glu Arg Gly Ser Gly Ser Ser Lys Pro Thr Gly
                85                  90                  95
Ser Ser Gly Gly Pro Arg Met Ala Ser Phe Pro Lys Thr Lys Phe Asn
            100                 105                 110
Glu Tyr Lys Asp Val Leu Pro Cys Met Thr Ser Ser Arg Gly Gly Lys
        115                 120                 125
Ile Lys Ala Thr Asp Phe Met Val Ala Met Arg Cys Leu Gly Ala Ser
    130                 135                 140
Pro Thr Pro Gly Glu Val Gln Arg His Leu Gln Thr His Gly Ile Asp
145                 150                 155                 160
Gly Asn Gly Glu Leu Asp Phe Ser Thr Phe Leu Thr Ile Met His Met
                165                 170                 175
Gln Ile Lys Gln Glu Asp Pro Lys Lys Glu Ile Leu Leu Ala Met Leu
            180                 185                 190
Met Val Asp Lys Glu Lys Lys Gly Tyr Val Met Ala Ser Asp Leu Arg
        195                 200                 205
Ser Lys Leu Thr Ser Gly Glu Lys Leu Thr His Lys Glu Val Asp Asp
    210                 215                 220
Leu Phe Arg Glu Ala Asp Ile Glu Pro Asn Gly Lys Val Lys Tyr Asp
225                 230                 235                 240
Glu Phe Ile His Lys Ile Thr Leu
                245

<210> 47
<211> 177
<212> PRT
<213> Homo Sapiens

<400> 47
Leu Cys Cys Met His Tyr Cys Cys Lys Ser Cys Trp Asn Glu Tyr Leu
1               5                   10                  15
Thr Thr Arg Ile Glu Gln Asn Leu Val Leu Asn Cys Thr Cys Pro Ile
            20                  25                  30
Ala Asp Cys Pro Ala Gln Pro Thr Gly Ala Phe Ile Arg Ala Ile Val
        35                  40                  45
Ser Ser Pro Glu Val Ile Ser Lys Tyr Lys Ala Leu Leu Arg Gly Tyr
    50                  55                  60
Val Glu Ser Cys Ser Asn Leu Thr Trp Cys Thr Asn Pro Gln Gly Cys
65                  70                  75                  80
Asp Arg Ile Leu Cys Arg Gln Gly Leu Gly Cys Gly Thr Thr Cys Ser
                85                  90                  95
Lys Cys Gly Trp Ala Ser Cys Phe Asn Cys Ser Phe Pro Glu Ala His
            100                 105                 110
Tyr Pro Ala Ser Cys Gly His Met Ser Gln Trp Val Asp Asp Gly Gly

115                 120                 125
Tyr Tyr Asp Gly Met Ser Val Glu Ala Lys His Leu Ala Lys Leu Ile
    130                 135                 140
Ser Lys Arg Cys Pro Ser Cys Gln Ala Pro Ile Glu Asn Glu Gly Cys
145                 150                 155                 160
Leu His Met Thr Cys Ala Lys Cys Asn His Gly Phe Cys Trp Arg Cys
                165                 170                 175
Leu

<210> 48
<211> 102
<212> PRT
<213> Homo Sapiens

<400> 48
Glu Lys Gly Leu His Ile Asp Gln Leu Val Cys Leu Val Leu Glu Ala
 1               5                  10                  15
Gln Lys Gly Pro Asn Pro Pro Gly Thr Leu Gly His Thr Val Ala Gly
            20                  25                  30
Gly Val Ala Cys Thr Thr Thr Val Leu Ser Cys Leu His Leu Leu Ser
        35                  40                  45
Gln Gly Tyr Lys Arg Asp Arg Pro Gln Ile Leu Met Tyr Ala Ala Pro
    50                  55                  60
Pro Met Gly Pro Cys Arg Gly Ala His Phe Cys Gly Ser Ser Gln Thr
65                  70                  75                  80
Ser Pro Pro Lys Pro Val Ala Thr Leu Ser Leu Leu Pro Cys Pro Leu
                85                  90                  95
Pro Pro Leu Lys Asn Gly
            100

<210> 49
<211> 179
<212> PRT
<213> Homo Sapiens

<400> 49
His Lys Pro Cys Asn Pro Arg Glu Lys Glu Arg Ile Gln Asn Ala Gly
 1               5                  10                  15
Gly Ser Val Met Ile Gln Arg Val Asn Gly Ser Leu Ala Val Ser Arg
            20                  25                  30
Ala Leu Gly Asp Tyr Asp Tyr Lys Cys Val Asp Gly Lys Gly Pro Thr
        35                  40                  45
Glu Gln Leu Val Ser Pro Glu Pro Glu Val Tyr Glu Ile Leu Arg Ala
    50                  55                  60
Glu Glu Asp Glu Phe Ile Ile Leu Ala Cys Asp Gly Ile Trp Asp Val
65                  70                  75                  80
Met Ser Asn Glu Glu Leu Cys Glu Tyr Val Lys Ser Arg Leu Glu Val
                85                  90                  95
Ser Asp Asp Leu Glu Asn Val Cys Asn Trp Val Val Asp Thr Cys Leu
            100                 105                 110
His Lys Gly Ser Arg Asp Asn Met Ser Ile Val Leu Val Cys Phe Ser
        115                 120                 125
Asn Ala Pro Lys Val Ser Asp Glu Ala Val Lys Lys Asp Ser Glu Leu
    130                 135                 140

Asp Lys His Leu Glu Ser Ile Met Glu Asn Leu Ala Lys Glu Cys Leu
145                 150                 155                 160
Ile Leu Pro Met Ser Cys Ala Ser Cys Leu Gln Lys Ile Ser Gln Ile
                165                 170                 175
Cys Leu Leu

<210> 50
<211> 163
<212> PRT
<213> Homo Sapiens

<400> 50
Asp Leu Pro Thr Leu Glu Asp His Gln Lys Gln Ser Gln Gln Leu Lys
1               5                   10                  15
Asp Ser Glu Leu Lys Ser Thr Glu Leu Gln Glu Lys Val Thr Glu Leu
            20                  25                  30
Glu Ser Leu Leu Glu Glu Thr Gln Ala Ile Cys Arg Glu Lys Glu Ile
        35                  40                  45
Gln Leu Glu Ser Leu Arg Gln Arg Glu Ala Glu Phe Ser Ser Ala Gly
    50                  55                  60
His Ser Leu Gln Asp Lys Gln Ser Val Glu Glu Thr Ser Gly Glu Gly
65                  70                  75                  80
Pro Glu Val Glu Met Glu Ser Trp Gln Lys Arg Tyr Asp Ser Leu Gln
                85                  90                  95
Lys Ile Val Glu Lys Gln Gln Gln Lys Met Asp Gln Leu Arg Ser Gln
            100                 105                 110
Val Gln Ser Leu Glu Gln Glu Val Ala Glu Glu Gly Thr Ser Gln Ala
        115                 120                 125
Leu Arg Glu Glu Ala Gln Arg Arg Asp Ser Ala Leu Gln Gln Leu Arg
    130                 135                 140
Thr Ala Val Lys Leu Ser Val Asn Gln Asp Leu Ile Glu Lys Asn Leu
145                 150                 155                 160
Thr Leu Gln

<210> 51
<211> 164
<212> PRT
<213> Homo Sapiens

<400> 51
Phe Gly Asp Ser Val Asp Cys Ser Asp Cys Trp Leu Pro Val Val Lys
1               5                   10                  15
Phe Ile Glu Glu Gln Phe Glu Gln Tyr Leu Arg Asp Glu Ser Gly Leu
            20                  25                  30
Asn Arg Lys Asn Ile Gln Asp Ser Arg Val His Cys Cys Leu Tyr Phe
        35                  40                  45
Ile Ser Pro Phe Gly Arg Gly Leu Arg Pro Leu Ala Phe Leu Arg Ala
    50                  55                  60
Val His Lys Val Asn Ile Ile Pro Val Ile Gly Lys Ala Asp Ala Leu
65                  70                  75                  80
Met Pro Gln Glu Thr Gln Ala Leu Lys Gln Lys Ile Arg Asp Gln Leu
                85                  90                  95
Lys Glu Glu Glu Ile His Ile Tyr Gln Phe Pro Glu Cys Asp Ser Asp

                100                 105                 110
Glu Asp Glu Asp Phe Lys Arg Gln Asp Ala Met Lys Glu Ser Ile Pro
        115                 120                 125
Phe Ala Val Val Gly Ser Cys Gln Val Val Arg Asp Gly Gly Asn Arg
    130                 135                 140
Pro Val Arg Gly Arg Arg Tyr Ser Trp Gly Asn Val Glu Val Asn His
145                 150                 155                 160
Ile Ala Ile Ser

<210> 52
<211> 600
<212> PRT
<213> Homo Sapiens

<400> 52
Met Cys Pro Arg Gln Val Asp Arg Ala Lys Glu Lys Gly Ile Gly Thr
 1               5                  10                  15
Pro Gln Pro Asp Val Ala Lys Asp Ser Trp Ala Glu Leu Glu Asn Ser
            20                  25                  30
Ser Lys Glu Asn Glu Val Ile Glu Val Lys Ser Met Gly Glu Ser Gln
        35                  40                  45
Ser Lys Lys Leu Gln Gly Gly Tyr Glu Cys Lys Tyr Cys Pro Tyr Ser
    50                  55                  60
Thr Gln Asn Leu Asn Glu Phe Thr Glu His Val Asp Met Gln His Pro
65                  70                  75                  80
Asn Val Ile Leu Asn Pro Leu Tyr Val Cys Ala Glu Cys Asn Phe Thr
                85                  90                  95
Thr Lys Lys Tyr Asp Ser Leu Ser Asp His Asn Ser Lys Phe His Pro
            100                 105                 110
Gly Glu Ala Asn Phe Lys Leu Lys Leu Ile Lys Arg Asn Asn Gln Thr
        115                 120                 125
Val Leu Glu Gln Ser Ile Glu Thr Thr Asn His Val Val Ser Ile Thr
    130                 135                 140
Thr Ser Gly Pro Gly Thr Gly Asp Ser Asp Ser Gly Ile Ser Val Ser
145                 150                 155                 160
Lys Thr Pro Ile Met Lys Pro Gly Lys Pro Lys Ala Asp Ala Lys Lys
                165                 170                 175
Val Pro Lys Lys Pro Glu Glu Ile Thr Pro Glu Asn His Val Glu Gly
            180                 185                 190
Thr Ala Arg Leu Val Thr Asp Thr Ala Glu Ile Leu Ser Arg Leu Gly
        195                 200                 205
Gly Val Glu Leu Leu Gln Asp Thr Leu Gly His Val Met Pro Ser Val
    210                 215                 220
Gln Leu Pro Pro Asn Ile Asn Leu Val Pro Lys Val Pro Val Pro Leu
225                 230                 235                 240
Asn Thr Thr Lys Tyr Asn Ser Ala Leu Asp Thr Asn Ala Thr Met Ile
                245                 250                 255
Asn Ser Phe Asn Lys Phe Pro Tyr Pro Thr Gln Ala Glu Leu Ser Trp
            260                 265                 270
Leu Thr Ala Ala Ser Lys His Pro Glu Glu His Ile Arg Ile Trp Phe
        275                 280                 285
Ala Thr Gln Arg Leu Lys His Gly Ile Ser Trp Ser Pro Glu Glu Val
    290                 295                 300
Glu Glu Ala Arg Lys Lys Met Phe Asn Gly Thr Ile Gln Ser Val Pro

305                 310                 315                 320
Pro Thr Ile Thr Val Leu Pro Ala Gln Leu Ala Pro Thr Lys Met Thr
                325                 330                 335
Gln Pro Ile Leu Gln Thr Ala Leu Pro Cys Gln Ile Leu Gly Gln Thr
            340                 345                 350
Ser Leu Val Leu Thr Gln Val Thr Ser Gly Ser Thr Thr Val Ser Cys
        355                 360                 365
Ser Pro Ile Thr Leu Ala Val Ala Gly Val Thr Asn His Gly Gln Lys
    370                 375                 380
Arg Pro Leu Val Thr Pro Gln Ala Ala Pro Glu Pro Lys Arg Pro His
385                 390                 395                 400
Ile Ala Gln Val Pro Glu Pro Pro Pro Lys Val Ala Asn Pro Pro Leu
                405                 410                 415
Thr Pro Ala Ser Asp Arg Lys Lys Thr Lys Glu Gln Ile Ala His Leu
            420                 425                 430
Lys Ala Ser Phe Leu Gln Ser Gln Phe Pro Asp Asp Ala Glu Val Tyr
        435                 440                 445
Arg Leu Ile Glu Val Thr Gly Leu Ala Arg Ser Glu Ile Lys Lys Trp
    450                 455                 460
Phe Ser Asp His Arg Tyr Arg Cys Gln Arg Gly Ile Val His Ile Thr
465                 470                 475                 480
Ser Glu Ser Leu Ala Lys Asp Gln Leu Ala Ile Ala Ala Ser Arg His
                485                 490                 495
Gly Arg Thr Tyr His Ala Tyr Pro Asp Phe Ala Pro Gln Lys Phe Lys
            500                 505                 510
Glu Lys Thr Gln Gly Gln Val Lys Ile Leu Glu Asp Ser Phe Leu Lys
        515                 520                 525
Ser Ser Phe Pro Thr Gln Ala Glu Leu Asp Arg Leu Arg Val Glu Thr
    530                 535                 540
Lys Leu Ser Arg Arg Glu Ile Asp Ser Trp Phe Ser Glu Arg Arg Lys
545                 550                 555                 560
Leu Arg Asp Ser Met Glu Gln Ala Val Leu Asp Ser Met Gly Ser Gly
                565                 570                 575
Gln Lys Arg Pro Arg Cys Gly Lys Pro Pro Met Val Leu Cys Leu Asp
            580                 585                 590
Ser Asn Ser Ser Pro Val Pro Ser
        595                 600

<210> 53
<211> 163
<212> PRT
<213> Homo Sapiens

<400> 53
Arg Lys Ser Trp Glu His Lys Glu Glu Ile Ser Glu Ala Glu Pro Gly
1               5                   10                  15
Gly Gly Ser Leu Gly Asp Gly Arg Pro Pro Glu Glu Ser Ala His Glu
            20                  25                  30
Met Met Glu Glu Glu Glu Glu Ile Pro Lys Pro Lys Ser Val Val Ala
        35                  40                  45
Pro Pro Gly Ala Pro Lys Lys Glu His Val Asn His Val Ala Gly Lys
    50                  55                  60
Ser Thr Ile Gly Gly Gln Ile Met Tyr Leu Thr Gly Met Val Asp Lys
65                  70                  75                  80
Arg Thr Leu Glu Lys Tyr Glu Arg Glu Ala Lys Glu Lys Asn Arg Glu

85                    90                    95
Thr Trp Tyr Leu Ser Trp Ala Leu Asp Thr Asn Gln Glu Glu Arg Asp
            100                 105                 110
Lys Gly Lys Thr Val Glu Val Gly Arg Ala Tyr Phe Glu Thr Glu Lys
        115                 120                 125
Lys His Phe Thr Ile Leu Asp Met Asn Pro Arg Thr Leu Ser Ser Lys
    130                 135                 140
Pro Lys Ala Gln Asn Leu Lys Leu Lys Val Pro Asn Ser Lys Val Arg
145                 150                 155                 160
Arg Cys Phe

<210> 54
<211> 155
<212> PRT
<213> Homo Sapiens

<400> 54
Glu Arg Trp Pro Glu Glu Gly Thr Ala Asp Leu Ala Gln Ser Gly Leu
1               5                   10                  15
Glu Gly Gly Thr Thr Arg Ala Ser Val Ser Trp Cys Cys Leu Glu Gly
            20                  25                  30
Ser Trp Leu Leu Ser Gly Tyr Leu Thr Phe Leu Lys Thr Cys Ser His
        35                  40                  45
Thr Ala Ser Leu Ala Val Ser Ser Ser Ser Cys Arg Ile Arg His Glu
    50                  55                  60
Leu Val Pro Asn Ser Ala Arg Gly Lys His Tyr Ser Gln Arg Trp Ala
65                  70                  75                  80
Gln Glu Asp Leu Leu Glu Glu Gln Lys Asp Gly Ala Arg Ala Ala Ala
                85                  90                  95
Val Ala Asp Lys Lys Lys Gly Leu Met Gly Pro Leu Thr Glu Leu Asp
            100                 105                 110
Thr Lys Asp Val Asp Ala Leu Leu Lys Lys Ser Glu Ala Gln His Glu
        115                 120                 125
Gln Pro Glu Asp Gly Cys Pro Phe Gly Ala Leu Thr Gln Arg Leu Leu
    130                 135                 140
Gln Ala Leu Val Glu Glu Asn Ile Ile Phe Ser
145                 150                 155

<210> 55
<211> 112
<212> PRT
<213> Homo Sapiens

<400> 55
Ser Glu Arg Ala Leu Ala Pro Arg Thr Tyr Arg Met Glu Thr Ala Arg
1               5                   10                  15
Ser Ala Pro Tyr Met Arg Ser Met Met Gln Ser Leu Ser Gln Asn Pro
            20                  25                  30
Asp Leu Ala Ala Gln Met Met Leu Asn Ser Pro Leu Phe Thr Ala Asn
        35                  40                  45
Pro Gln Leu Gln Glu Gln Met Arg Pro Gln Leu Pro Ala Phe Leu Gln
    50                  55                  60
Gln Met Gln Asn Pro Asp Thr Leu Ser Ala Met Ser Asn Pro Arg Ala
65                  70                  75                  80

Met Gln Ala Leu Met Gln Ile Gln Gln Gly Leu Gln Thr Leu Ala Thr
                85                  90                  95
Glu Ala Pro Gly Leu Ile Pro Ser Phe Thr Pro Gly Val Gly Val Gly
            100                 105                 110

<210> 56
<211> 151
<212> PRT
<213> Homo Sapiens

<400> 56
Lys Phe Gly Met Pro Ile Asp Cys Gly Leu Pro Pro His Ile Asp Phe
1               5                   10                  15
Gly Asp Cys Thr Lys Leu Lys Asp Asp Gln Gly Tyr Phe Glu Gln Glu
            20                  25                  30
Asp Asp Met Met Glu Val Pro Tyr Val Thr Pro His Pro Pro Tyr His
        35                  40                  45
Leu Gly Ala Val Ala Lys Thr Trp Glu Asn Thr Lys Glu Ser Pro Ala
    50                  55                  60
Thr His Ser Ser Asn Phe Leu Tyr Gly Thr Met Val Ser Tyr Thr Cys
65                  70                  75                  80
Asn Pro Gly Tyr Glu Leu Leu Gly Asn Pro Val Leu Ile Cys Gln Glu
                85                  90                  95
Asp Gly Thr Trp Asn Gly Ser Ala Pro Ser Cys Ile Ser Ile Glu Cys
            100                 105                 110
Asp Leu Pro Thr Ala Pro Glu Asn Gly Phe Leu Arg Phe Thr Glu Thr
        115                 120                 125
Ser Met Gly Ser Ala Val Gln Tyr Ser Cys Lys Pro Gly His Ile Leu
    130                 135                 140
Ala Gly Ser Asp Leu Arg Leu
145                 150

<210> 57
<211> 220
<212> PRT
<213> Homo Sapiens

<400> 57
Ala Ala Phe Val Ser Glu Val Thr Ser Phe Pro Val Val Gln Leu His
1               5                   10                  15
Met Asn Arg Thr Ala Met Arg Ala Ser Gln Lys Asp Phe Glu Asn Ser
            20                  25                  30
Ile Asn Gln Val Lys Leu Leu Lys Lys Asp Pro Gly Asn Glu Val Lys
        35                  40                  45
Leu Lys Leu Tyr Ala Leu Tyr Lys Gln Ala Thr Glu Gly Pro Cys Asn
    50                  55                  60
Met Pro Lys Pro Gly Val Phe Asp Leu Ile Asn Lys Ala Lys Trp Asp
65                  70                  75                  80
Ala Trp Asn Ala Leu Gly Ser Leu Pro Lys Glu Ala Ala Arg Gln Asn
                85                  90                  95
Tyr Val Asp Leu Val Ser Ser Leu Ser Pro Ser Leu Glu Ser Ser Ser
            100                 105                 110
Gln Val Glu Pro Gly Thr Asp Arg Lys Ser Thr Gly Phe Glu Thr Leu
        115                 120                 125
Val Val Thr Ser Glu Asp Gly Ile Thr Lys Ile Met Phe Asn Arg Pro

130                 135                 140
Lys Lys Lys Asn Ala Ile Asn Thr Glu Met Tyr His Glu Ile Met Arg
145                 150                 155                 160
Ala Leu Lys Ala Ala Ser Lys Asp Asp Ser Ile Ile Thr Val Leu Thr
                165                 170                 175
Gly Asn Gly Asp Tyr Tyr Ser Ser Gly Asn Asp Leu Thr Asn Phe Thr
            180                 185                 190
Asp Ile Pro Pro Gly Gly Val Glu Lys Ala Lys Asn Asn Ala Val Leu
        195                 200                 205
Leu Lys Gly Ile Cys Gly Leu Phe Tyr Arg Ile Ser
    210                 215                 220

<210> 58
<211> 101
<212> PRT
<213> Homo Sapiens

<400> 58
Trp Pro Asp Leu Val His Thr Trp Ser Ser Glu Glu Ala Met Gly Ser
1               5                   10                  15
Cys Cys Ser Cys Pro Asp Lys Asp Thr Val Pro Asp Asn His Arg Asn
            20                  25                  30
Lys Phe Lys Val Ile Asn Val Asp Asp Asp Gly Asn Glu Leu Gly Ser
        35                  40                  45
Gly Ile Met Glu Leu Thr Asp Thr Glu Leu Ile Leu Tyr Thr Arg Lys
    50                  55                  60
Arg Asp Ser Val Lys Trp His Tyr Leu Cys Leu Arg Arg Tyr Gly Tyr
65                  70                  75                  80
Asp Ser Asn Leu Phe Ser Phe Glu Ser Gly Pro Arg Cys Gln Thr Gly
                85                  90                  95
Thr Arg Asn Leu Cys
            100

<210> 59
<211> 43
<212> PRT
<213> Homo Sapiens

<400> 59
Ala His Gly Pro Gly Val Glu Pro Thr Ser Arg His Gln Lys Asn Asn
1               5                   10                  15
Leu Ser Ser Ser His Thr Val Arg Leu Glu Thr Arg Gly Gln Thr Glu
            20                  25                  30
Asn Gln Glu Cys Leu Leu Cys Pro His Glu Glu
        35                  40

<210> 60
<211> 210
<212> PRT
<213> Homo Sapiens

<400> 60
Leu Asn Gln Trp Thr Tyr Gln Ala Met Val His Glu Leu Leu Gly Ile
1               5                   10                  15
Asn Asn Asn Arg Ile Asp Leu Ser Arg Val Pro Gly Ile Ser Lys Asp

20                  25                  30
Leu Arg Glu Val Val Leu Ser Ala Glu Asn Asp Glu Phe Tyr Ala Asn
        35                  40                  45
Asn Met Tyr Leu Asn Phe Ala Glu Ile Gly Ser Asn Ile Lys Asn Leu
    50                  55                  60
Met Glu Asp Phe Gln Lys Lys Lys Pro Lys Glu Gln Gln Lys Leu Glu
65                  70                  75                  80
Ser Ile Ala Asp Met Lys Ala Phe Val Glu Asn Tyr Pro Gln Phe Lys
                85                  90                  95
Lys Met Ser Gly Thr Val Ser Lys His Val Thr Val Val Gly Glu Leu
            100                 105                 110
Ser Arg Leu Val Ser Glu Arg Asn Leu Leu Glu Val Ser Glu Val Glu
        115                 120                 125
Gln Glu Leu Ala Cys Gln Asn Asp His Ser Ser Ala Leu Gln Asn Ile
    130                 135                 140
Lys Arg Leu Leu Gln Asn Pro Lys Val Thr Glu Phe Asp Ala Ala Arg
145                 150                 155                 160
Leu Val Met Leu Tyr Ala Leu His Tyr Glu Arg His Ser Ser Asn Ser
                165                 170                 175
Leu Pro Gly Leu Met Met Leu Arg Asn Lys Gly Val Ser Glu Lys Tyr
            180                 185                 190
Arg Lys Leu Val Ser Ala Val Val Glu Tyr Gly Gly Lys Thr Ser Gln
        195                 200                 205
Arg Lys
    210

<210> 61
<211> 40
<212> PRT
<213> Homo Sapiens

<400> 61
Thr Pro Gly Pro Gly Ala Gly Phe Tyr Ala Cys Pro Ala Arg Pro Leu
1               5                   10                  15
Val Ser Gly Ile Tyr Ser Phe Arg Trp Val Arg Gly Leu Ala Asp Gln
            20                  25                  30
Glu Arg Asn Trp Gly Leu Ser Gln
        35                  40

<210> 62
<211> 238
<212> PRT
<213> Homo Sapiens

<400> 62
His Glu Ala Arg Leu Lys Arg Ala Ser Ala Pro Thr Phe Asp Asn Asp
1               5                   10                  15
Tyr Ser Leu Ser Glu Leu Leu Ser Gln Leu Asp Ser Gly Val Ser Gln
            20                  25                  30
Ala Val Glu Gly Pro Glu Glu Leu Ser Arg Ser Ser Ser Glu Ser Lys
        35                  40                  45
Leu Pro Ser Ser Gly Ser Gly Lys Arg Leu Ser Gly Val Ser Ser Val
    50                  55                  60
Asp Ser Ala Phe Ser Ser Arg Gly Ser Leu Ser Leu Ser Phe Glu Arg
65                  70                  75                  80

Glu Pro Ser Thr Ser Asp Leu Gly Thr Thr Asp Val Gln Lys Lys Lys
                85                  90                  95
Leu Val Asp Ala Ile Val Ser Gly Asp Thr Ser Lys Leu Met Lys Ile
            100                 105                 110
Leu Gln Pro Gln Asp Val Asp Leu Ala Leu Asp Ser Gly Ala Ser Leu
        115                 120                 125
Leu His Leu Ala Val Glu Ala Gly Gln Glu Glu Cys Ala Lys Trp Leu
    130                 135                 140
Leu Leu Asn Asn Ala Asn Pro Asn Leu Ser Asn Arg Arg Gly Ser Thr
145                 150                 155                 160
Pro Leu His Met Ala Val Glu Arg Arg Val Arg Gly Val Val Glu Leu
                165                 170                 175
Leu Leu Ala Arg Ile Ser Val Asn Ala Lys Asp Glu Asp Gln Trp Thr
            180                 185                 190
Ala Leu His Phe Ala Asn Gly Gly Val His Thr Ala Ala Val Gly Glu
        195                 200                 205
Arg Leu Gly Gln Thr Lys Val Asp Phe Glu Gly Arg Thr Pro Met Gln
    210                 215                 220
Val Gly Leu Pro Thr Thr Gly Lys Asn Ile Leu Arg Ile Leu
225                 230                 235

<210> 63
<211> 146
<212> PRT
<213> Homo Sapiens

<400> 63
Arg Leu Gly Ala Ala Met Met Glu Gly Leu Asp Asp Gly Pro Asp Phe
1               5                   10                  15
Leu Ser Glu Glu Asp Arg Gly Leu Lys Ala Ile Asn Val Asp Leu Gln
            20                  25                  30
Ser Asp Ala Ala Leu Gln Val Asp Ile Ser Asp Ala Leu Ser Glu Arg
        35                  40                  45
Asp Lys Val Lys Phe Thr Val His Thr Lys Ile Pro Pro Ala Pro Pro
    50                  55                  60
Arg Pro Asp Phe Asp Ala Ser Arg Glu Lys Leu Gln Lys Leu Gly Glu
65                  70                  75                  80
Gly Glu Gly Ser Met Thr Lys Glu Glu Phe Thr Lys Met Lys Gln Glu
                85                  90                  95
Leu Glu Ala Glu Tyr Leu Ala Ile Phe Lys Lys Thr Val Ala Met His
            100                 105                 110
Glu Val Phe Leu Cys Arg Val Ala Ala His Pro Ile Leu Arg Arg Asp
        115                 120                 125
Leu Asn Phe His Val Phe Leu Glu Tyr Asn Gln Asp Leu Ser Val Arg
    130                 135                 140
Gly Lys
145

<210> 64
<211> 63
<212> PRT
<213> Homo Sapiens

<400> 64
Glu Arg Gly His Ser Ile Lys Asp Phe Val Ser Phe Ala Arg His Phe

1                   5                   10                  15
Ser Pro Asn Pro Arg Ile Val Ser Val Asn Ala Ser Tyr Ser Leu Ser
                20                  25                  30
Asn Glu Ser Ser Leu Glu Gln Val Tyr Thr Leu Lys Met Ser Phe Ile
            35                  40                  45
Ala Ser Asn Thr Tyr His Asn Gln Leu Tyr Lys Glu Gly Phe Leu
    50                  55                  60

<210> 65
<211> 199
<212> PRT
<213> Homo Sapiens

<400> 65
Glu Ala Pro Asp Ser Ala Glu Gly Thr Thr Leu Thr Val Leu Pro Glu
1               5                   10                  15
Gly Glu Glu Leu Pro Leu Cys Val Ser Glu Ser Asn Gly Leu Glu Leu
            20                  25                  30
Pro Pro Ser Ala Ala Ser Asp Glu Pro Leu Gln Glu Pro Leu Glu Ala
        35                  40                  45
Asp Arg Thr Ser Glu Glu Leu Thr Glu Ala Lys Thr Pro Thr Ser Ser
    50                  55                  60
Pro Glu Lys Pro Gln Glu Leu Val Thr Ala Glu Val Ala Ala Pro Ser
65                  70                  75                  80
Thr Ser Ser Ser Ala Thr Ser Ser Pro Glu Gly Pro Ser Pro Ala Arg
                85                  90                  95
Pro Pro Arg Arg Arg Thr Ser Ala Asp Val Glu Ile Arg Gly Gln Gly
            100                 105                 110
Thr Gly Arg Pro Gly Gln Pro Pro Gly Pro Lys Val Leu Arg Lys Leu
        115                 120                 125
Pro Gly Arg Leu Val Thr Val Val Glu Glu Lys Glu Leu Val Arg Arg
    130                 135                 140
Arg Arg Gln Gln Arg Gly Ala Ala Ser Thr Leu Val Pro Gly Val Ser
145                 150                 155                 160
Glu Thr Ser Ala Ser Pro Gly Ser Pro Ser Val Arg Ser Met Ser Gly
                165                 170                 175
Pro Glu Ser Ser Pro Pro Ile Gly Gly Pro Cys Glu Ala Ala Pro Ser
            180                 185                 190
Ser Ser Leu Pro Thr Pro Pro
        195

<210> 66
<211> 1599
<212> DNA
<213> Homo Sapiens

<400> 66
ttctttgaaa cattattatt cagaacgaag gagaatgata cagatacact ggctgaggtg      60
ttttgaggtg cattgaaatg ttccatgctg ttacttaggt taacatgttc ttgaggtacc     120
atgccatgga ttaaaaggaa atttggtaag tggcttccac ctaaacgact tactagggaa     180
gctatgcgaa attatttaaa agggtaaggg gatcaaatag tacttatcct tcatgcaaaa     240
gttgtacaga agtcatatgg caatcaaaaa attttttttt gccctccccc ttgtgtatat     300
cttatgggca gtggatggaa gaaaaaaaaa gaacaaatga aatgcgatgg ttgttctgaa     360
cacagctctc atccatgtgc atttattggg ataggaaata gtgaccaaga aatgcagcag     420
ctaaacttgg aaggaaagaa ctattgcaca gccaaaacat tgtacatatc tgattcagac     480

aagcaaaagc acttcatttt ttctgtaaag gtgttctatg gcaacggtga tgacattggt 540
gtgttcctca gcaagtagat aaaagtcatc tccaaacctt ccaaaaagaa gcagtcattg 600
aaaaatgctg acttatgcat tgtctcagga acaaaggtgg ctctgtttaa tcgactacga 660
tcccagacag ttagtaccag atacttgcat gtagaaggag gtaattttca tgccagttca 720
cagcagtggg gagcatttta cattcaattc ttggatgatg atggatcaga aggagaagaa 780
ttcacagtct gagatgccta cattcattat ggacaaacat gcaaacttgt gtgctcagtt 840
actggcatgg cactcccaag attgataatt atgaaagttg ataagcatac cgcattattg 900
gatgcagatg atcctgtgtc acaactccat aaatgtgcat tttaccttaa ggatacagaa 960
agaatgtatt tgtgcctttc tcaagaaaga ataattcaat ttcaggccac tccatgtcca 1020
agagaaccaa ataaagagat gataaatgat ggcgcttcct ggacaatcat tagcacagat 1080
aaggcagggt atacatttta tgagggaatg ggccctgtcc ttgccccagt cactcctgtg 1140
cctgtggtag agagccttca gttgaatggc ggtggggacg tagcaatgct tgaacttaca 1200
ggacagaatt tcactccaaa tttacgagtg tggtttgggg gggtagaagc tgaaactatg 1260
tacaggtgtg gagagagtat gctctgtgtc gtcccagaca tttctgcatt ccgagaaggt 1320
tggagatggg tccggcaacc agtccaggtt ccagtaactt tggtccgaaa tgatggaatc 1380
atttattcca ccagccttac ctttacctac acaccagaac cagggccgcg gccacattgc 1440
agtgcagcag gagcaatcct tctagccaat tcaagccagg tgccccctaa cgaatcaaac 1500
acaaacagcg agggaagtta cacaaacgcc agcacaaatt caaccagtgt cacatcatct 1560
acagccacag tggtatccta actaccgtct tttgctag 1599

<210> 67
<211> 729
<212> PRT
<213> Homo Sapiens

<400> 67
Met Gly Lys Lys Tyr Lys Asn Ile Val Leu Leu Lys Gly Leu Glu Val
1               5                   10                  15
Ile Asn Asp Tyr His Phe Arg Met Val Lys Ser Leu Leu Ser Asn Asp
            20                  25                  30
Leu Lys Leu Asn Leu Lys Met Arg Glu Glu Tyr Asp Lys Ile Gln Ile
        35                  40                  45
Ala Asp Leu Met Glu Glu Lys Phe Arg Gly Asp Ala Gly Leu Gly Lys
    50                  55                  60
Leu Ile Lys Ile Phe Glu Asp Ile Pro Thr Leu Glu Asp Leu Ala Glu
65                  70                  75                  80
Thr Leu Lys Lys Glu Lys Leu Lys Val Lys Gly Pro Ala Leu Ser Arg
                85                  90                  95
Lys Arg Lys Lys Glu Val His Ala Thr Ser Pro Ala Pro Ser Thr Ser
            100                 105                 110
Ser Thr Val Lys Thr Glu Gly Ala Glu Ala Thr Pro Gly Ala Gln Lys
        115                 120                 125
Arg Lys Lys Ser Thr Lys Glu Lys Ala Gly Pro Lys Gly Ser Lys Val
    130                 135                 140
Ser Glu Glu Gln Thr Gln Pro Pro Ser Pro Ala Gly Ala Gly Met Ser
145                 150                 155                 160
Thr Ala Met Gly Arg Ser Pro Ser Pro Lys Thr Ser Leu Ser Ala Pro
                165                 170                 175
Pro Asn Ser Ser Ser Thr Glu Asn Pro Lys Thr Val Ala Lys Cys Gln
            180                 185                 190
Val Thr Pro Arg Arg Asn Val Leu Gln Lys Arg Pro Val Ile Val Lys
        195                 200                 205
Val Leu Ser Thr Thr Lys Pro Phe Glu Tyr Glu Thr Pro Glu Met Glu
    210                 215                 220
Lys Lys Ile Met Phe His Ala Thr Val Ala Thr Gln Thr Gln Phe Phe

225                 230                 235                 240
His Val Lys Val Leu Asn Thr Ser Leu Lys Glu Lys Phe Asn Gly Lys
                245                 250                 255
Lys Ile Ile Ile Ile Ser Asp Tyr Leu Glu Tyr Asp Ser Leu Leu Glu
            260                 265                 270
Val Asn Glu Glu Ser Thr Val Ser Glu Ala Gly Pro Asn Gln Thr Phe
        275                 280                 285
Glu Val Pro Asn Lys Ile Ile Asn Arg Ala Lys Glu Thr Leu Lys Ile
    290                 295                 300
Asp Ile Leu His Lys Gln Ala Ser Gly Asn Ile Val Tyr Gly Val Phe
305                 310                 315                 320
Met Leu His Lys Lys Thr Val Asn Gln Lys Thr Thr Ile Tyr Glu Ile
                325                 330                 335
Gln Asp Asp Arg Gly Lys Met Asp Val Val Gly Thr Gly Gln Cys His
            340                 345                 350
Asn Ile Pro Cys Glu Glu Gly Asp Lys Leu Gln Leu Phe Cys Phe Arg
        355                 360                 365
Leu Arg Lys Lys Asn Gln Met Ser Lys Leu Ile Ser Glu Met His Ser
    370                 375                 380
Phe Ile Gln Ile Lys Lys Lys Thr Asn Pro Arg Asn Asn Asp Pro Lys
385                 390                 395                 400
Ser Met Lys Leu Pro Gln Glu Gln Arg Gln Leu Pro Tyr Pro Ser Glu
                405                 410                 415
Ala Ser Thr Thr Phe Pro Glu Ser His Leu Arg Thr Pro Gln Met Pro
            420                 425                 430
Pro Thr Thr Pro Ser Ser Ser Phe Phe Thr Lys Lys Ser Glu Asp Thr
        435                 440                 445
Ile Ser Lys Met Asn Asp Phe Met Arg Met Gln Ile Leu Lys Glu Gly
    450                 455                 460
Ser His Phe Pro Gly Pro Phe Met Thr Ser Ile Gly Pro Ala Glu Ser
465                 470                 475                 480
His Pro His Thr Pro Gln Met Pro Pro Ser Thr Pro Ser Ser Ser Phe
                485                 490                 495
Leu Thr Thr Leu Lys Pro Arg Leu Lys Thr Glu Pro Glu Glu Val Ser
            500                 505                 510
Ile Glu Asp Ser Ala Gln Ser Asp Leu Lys Glu Val Met Val Leu Asn
        515                 520                 525
Ala Thr Glu Ser Phe Val Tyr Glu Pro Lys Glu Gln Lys Lys Met Phe
    530                 535                 540
His Ala Thr Val Ala Thr Glu Asn Glu Val Phe Arg Val Lys Val Phe
545                 550                 555                 560
Asn Ile Asp Leu Lys Glu Lys Phe Thr Pro Lys Lys Ile Ile Ala Ile
                565                 570                 575
Ala Asn Tyr Val Cys Arg Asn Gly Phe Leu Glu Val Tyr Pro Phe Thr
            580                 585                 590
Leu Val Ala Asp Val Asn Ala Asp Ala Asn Met Glu Ile Pro Lys Gly
        595                 600                 605
Leu Ile Arg Ser Ala Ser Val Thr Pro Lys Ile Asn Gln Leu Cys Ser
    610                 615                 620
Gln Thr Lys Gly Ser Phe Val Asn Gly Val Phe Glu Val His Lys Lys
625                 630                 635                 640
Asn Val Arg Gly Glu Phe Thr Tyr Tyr Glu Ile Gln Asp Asn Thr Gly
                645                 650                 655
Lys Met Glu Val Val Val His Gly Arg Leu Asn Thr Ile Asn Cys Glu
            660                 665                 670

Glu Gly Asp Lys Leu Lys Leu Thr Ser Phe Glu Leu Ala Pro Lys Ser
        675                 680                 685
Gly Asn Thr Gly Glu Leu Arg Ser Val Ile His Ser His Ile Lys Val
    690                 695                 700
Ile Lys Thr Lys Lys Asn Lys Lys Asp Ile Leu Asn Pro Asp Ser Ser
705                 710                 715                 720
Met Glu Thr Ser Pro Asp Phe Phe Phe
                725

<210> 68
<211> 754
<212> PRT
<213> Homo Sapiens

<400> 68
   Met Ala Ser Val Pro Ala Leu Gln Leu Thr Pro Ala Asn Pro Pro Pro
   1 5 10 15
   Pro Glu Val Ser Asn Pro Lys Lys Pro Gly Arg Val Thr Asn Gln Leu
   20 25 30
   Gln Tyr Leu His Lys Val Val Met Lys Ala Leu Trp Lys His Gln Phe
   35 40 45
   Ala Trp Pro Phe Arg Gln Pro Val Asp Ala Val Lys Leu Gly Leu Pro
   50 55 60
   Asp Tyr His Lys Ile Ile Lys Gln Pro Met Asp Met Gly Thr Ile Lys
   65 70 75 80
   Arg Arg Leu Glu Asn Asn Tyr Tyr Trp Ala Ala Ser Glu Cys Met Gln
   85 90 95
   Asp Phe Asn Thr Met Phe Thr Asn Cys Tyr Ile Tyr Asn Lys Pro Thr
   100 105 110
   Asp Asp Ile Val Leu Met Ala Gln Thr Leu Glu Lys Ile Phe Leu Gln
   115 120 125
   Lys Val Ala Ser Met Pro Gln Glu Glu Gln Glu Leu Val Val Thr Ile
   130 135 140
   Pro Lys Asn Ser His Lys Lys Gly Ala Lys Leu Ala Ala Leu Gln Gly
   145 150 155 160
   Ser Val Thr Ser Ala His Gln Val Pro Ala Val Ser Ser Val Ser His
   165 170 175
   Thr Ala Leu Tyr Thr Pro Pro Pro Glu Ile Pro Thr Thr Val Leu Asn
   180 185 190
   Ile Pro His Pro Ser Val Ile Ser Ser Pro Leu Leu Lys Ser Leu His
   195 200 205
   Ser Ala Gly Pro Pro Leu Leu Ala Val Thr Ala Ala Pro Pro Ala Gln
   210 215 220
   Pro Leu Ala Lys Lys Lys Gly Val Lys Arg Lys Ala Asp Thr Thr Thr
   225 230 235 240
   Pro Thr Pro Thr Ala Ile Leu Ala Pro Gly Ser Pro Ala Ser Pro Pro
   245 250 255
   Gly Ser Leu Glu Pro Lys Ala Ala Arg Leu Pro Pro Met Arg Arg Glu
   260 265 270
   Ser Gly Arg Pro Ile Lys Pro Pro Arg Lys Asp Leu Pro Asp Ser Gln
   275 280 285
   Gln Gln His Gln Ser Ser Lys Lys Gly Lys Leu Ser Glu Gln Leu Lys
   290 295 300
   His Cys Asn Gly Ile Leu Lys Glu Leu Leu Ser Lys Lys His Ala Ala
   305 310 315 320
   Tyr Ala Trp Pro Phe Tyr Lys Pro Val Asp Ala Ser Ala Leu Gly Leu
   325 330 335
   His Asp Tyr His Asp Ile Ile Lys His Pro Met Asp Leu Ser Thr Val
   340 345 350
   Lys Arg Lys Met Glu Asn Arg Asp Tyr Arg Asp Ala Gln Glu Phe Ala
   355 360 365
   Ala Asp Val Arg Leu Met Phe Ser Asn Cys Tyr Lys Tyr Asn Pro Pro
   370 375 380
   Asp His Asp Val Val Ala Met Ala Arg Lys Leu Gln Asp Val Phe Glu
   385 390 395 400
   Phe Arg Tyr Ala Lys Met Pro Asp Glu Pro Leu Glu Pro Gly Pro Leu
   405 410 415
   Pro Val Ser Thr Ala Met Pro Pro Gly Leu Ala Lys Ser Ser Ser Glu
   420 425 430
   Ser Ser Ser Glu Glu Ser Ser Ser Glu Ser Ser Ser Glu Glu Glu Glu
   435 440 445
   Glu Glu Asp Glu Glu Asp Glu Glu Glu Glu Glu Ser Glu Ser Ser Asp
   450 455 460
   Ser Glu Glu Glu Arg Ala His Arg Leu Ala Glu Leu Gln Glu Gln Leu
   465 470 475 480
   Arg Ala Val His Glu Gln Leu Ala Ala Leu Ser Gln Gly Pro Ile Ser
   485 490 495
   Lys Pro Lys Arg Lys Arg Glu Lys Lys Glu Lys Lys Lys Lys Arg Lys
   500 505 510
   Ala Glu Lys His Arg Gly Arg Ala Gly Ala Asp Glu Asp Asp Lys Gly
   515 520 525
   Pro Arg Ala Pro Arg Pro Pro Gln Pro Lys Lys Ser Lys Lys Ala Ser
   530 535 540
   Gly Ser Gly Gly Gly Ser Ala Ala Leu Gly Pro Ser Gly Phe Gly Pro
   545 550 555 560
   Ser Gly Gly Ser Gly Thr Lys Leu Pro Lys Lys Ala Thr Lys Thr Ala
   565 570 575
   Pro Pro Ala Leu Pro Thr Gly Tyr Asp Ser Glu Glu Glu Glu Glu Ser
   580 585 590
   Arg Pro Met Ser Tyr Asp Glu Lys Arg Gln Leu Ser Leu Asp Ile Asn
   595 600 605
   Lys Leu Pro Gly Glu Lys Leu Gly Arg Val Val His Ile Ile Gln Ala
   610 615 620
   Arg Glu Pro Ser Leu Arg Asp Ser Asn Pro Glu Glu Ile Glu Ile Asp
   625 630 635 640
   Phe Glu Thr Leu Lys Pro Ser Thr Leu Arg Glu Leu Glu Arg Tyr Val
   645 650 655
   Leu Ser Cys Leu Arg Lys Lys Pro Arg Lys Pro Tyr Thr Ile Lys Lys
   660 665 670
   Pro Val Gly Lys Thr Lys Glu Glu Leu Ala Leu Glu Lys Lys Arg Glu
   675 680 685
   Leu Glu Lys Arg Leu Gln Asp Val Ser Gly Gln Leu Asn Ser Thr Lys
   690 695 700
   Lys Pro Pro Lys Lys Ala Asn Glu Lys Thr Glu Ser Ser Ser Ala Gln
   705 710 715 720
   Gln Val Ala Val Ser Arg Leu Ser Ala Ser Ser Ser Ser Ser Asp Ser
   725 730 735
   Ser Ser Ser Ser Ser Ser Ser Ser Ser Ser Asp Thr Ser Asp Ser Asp
   740 745 750
   Ser Gly

<210> 69
<211> 210
<212> PRT
<213> Homo Sapiens

<400> 69
Met Asp Asp Glu Glu Glu Thr Tyr Arg Leu Trp Lys Ile Arg Lys Thr
1               5                   10                  15
Ile Met Gln Leu Cys His Asp Arg Gly Tyr Leu Val Thr Gln Asp Glu
            20                  25                  30
Leu Asp Gln Thr Leu Glu Glu Phe Lys Ala Gln Phe Gly Asp Lys Pro
        35                  40                  45
Ser Glu Gly Arg Pro Arg Arg Thr Asp Leu Thr Val Leu Val Ala His
    50                  55                  60
Asn Asp Asp Pro Thr Asp Gln Met Phe Val Phe Phe Pro Glu Glu Pro
65                  70                  75                  80
Lys Val Gly Ile Lys Thr Ile Lys Val Tyr Cys Gln Arg Met Gln Glu
                85                  90                  95
Glu Asn Ile Thr Arg Ala Leu Ile Val Val Gln Gln Gly Met Thr Pro
            100                 105                 110
Ser Ala Lys Gln Ser Leu Val Asp Met Ala Pro Lys Tyr Ile Leu Glu
        115                 120                 125
Gln Phe Leu Gln Gln Glu Leu Leu Ile Asn Ile Thr Glu His Glu Leu
    130                 135                 140
Val Pro Glu His Val Val Met Thr Lys Glu Glu Val Thr Glu Leu Leu
145                 150                 155                 160
Ala Arg Tyr Lys Leu Arg Glu Asn Gln Leu Pro Arg Ile Gln Ala Gly
                165                 170                 175
Asp Pro Val Ala Arg Tyr Phe Gly Ile Lys Arg Gly Gln Val Val Lys
            180                 185                 190
Ile Ile Arg Pro Ser Glu Thr Ala Gly Arg Tyr Ile Thr Tyr Arg Leu
        195                 200                 205
Val Gln
    210

<210> 70
<211> 621
<212> PRT
<213> Homo Sapiens

<400> 70
Met Leu Leu Leu Pro Ser Ala Ala Glu Gly Gln Gly Thr Ala Ile Thr
1               5                   10                  15
His Ala Leu Thr Ser Ala Ser Ser Val Cys Gln Val Glu Pro Val Gly
            20                  25                  30
Arg Trp Phe Glu Ala Phe Val Lys Arg Arg Asn Arg Asn Ala Ser Thr
        35                  40                  45
Ser Phe Gln Glu Leu Glu Asp Lys Lys Glu Leu Ser Glu Glu Ser Glu
    50                  55                  60
Asp Glu Glu Leu Gln Leu Glu Glu Phe Pro Met Leu Lys Thr Leu Asp
65                  70                  75                  80
Pro Lys Asp Trp Lys Asn Gln Asp His Tyr Ala Val Leu Gly Leu Gly
                85                  90                  95

His Val Arg Tyr Thr Ala Thr Gln Arg Gln Ile Lys Ala Ala His Lys
            100                 105                 110
Ala Met Val Leu Lys His His Pro Asp Lys Arg Lys Ala Ala Gly Glu
        115                 120                 125
Pro Ile Lys Glu Gly Asp Asn Asp Tyr Phe Thr Cys Ile Thr Lys Ala
    130                 135                 140
Tyr Glu Met Leu Ser Asp Pro Val Lys Arg Arg Ala Phe Asn Ser Val
145                 150                 155                 160
Asp Pro Thr Phe Asp Asn Ser Val Pro Ser Lys Ser Glu Ala Lys Asp
                165                 170                 175
Asn Phe Phe Gln Val Phe Ser Pro Val Phe Glu Arg Asn Ser Arg Trp
            180                 185                 190
Ser Asn Lys Lys Asn Val Pro Lys Leu Gly Asp Met Asn Ser Ser Phe
        195                 200                 205
Glu Asp Val Asp Ala Phe Tyr Ser Phe Trp Tyr Asn Phe Asp Ser Trp
    210                 215                 220
Arg Glu Phe Ser Tyr Leu Asp Glu Glu Glu Lys Glu Lys Ala Glu Cys
225                 230                 235                 240
Arg Asp Glu Arg Lys Trp Ile Glu Lys Gln Asn Arg Ala Thr Arg Ala
                245                 250                 255
Gln Arg Lys Lys Glu Glu Met Asn Arg Ile Arg Thr Leu Val Asp Asn
            260                 265                 270
Ala Tyr Ser Cys Asp Pro Arg Ile Lys Lys Phe Lys Glu Glu Glu Lys
        275                 280                 285
Ala Lys Lys Glu Ala Glu Lys Lys Ala Lys Ala Glu Ala Arg Arg Lys
    290                 295                 300
Glu Gln Glu Ala Lys Glu Lys Gln Arg Gln Ala Glu Leu Glu Ala Val
305                 310                 315                 320
Arg Leu Ala Lys Glu Lys Glu Glu Glu Glu Val Arg Gln Gln Ala Leu
                325                 330                 335
Leu Ala Lys Lys Glu Lys Asp Ile Gln Lys Lys Ala Ile Lys Lys Glu
            340                 345                 350
Arg Gln Lys Leu Arg Asn Ser Cys Lys Ser Trp Asn His Phe Ser Asp
        355                 360                 365
Asn Glu Ala Asp Arg Val Lys Met Met Glu Glu Val Glu Lys Leu Cys
    370                 375                 380
Asp Arg Leu Glu Leu Ala Ser Leu Gln Gly Leu Asn Glu Ile Leu Ala
385                 390                 395                 400
Ser Ser Thr Arg Glu Val Gly Lys Ala Ala Leu Glu Lys Gln Ile Glu
                405                 410                 415
Glu Val Asn Glu Gln Met Arg Arg Glu Lys Glu Glu Ala Asp Ala Arg
            420                 425                 430
Met Arg Gln Ala Ser Lys Asn Ala Glu Lys Ser Thr Gly Gly Ser Gly
        435                 440                 445
Ser Gly Ser Lys Asn Trp Ser Glu Asp Asp Leu Gln Leu Leu Ile Lys
    450                 455                 460
Ala Val Asn Leu Phe Pro Ala Gly Thr Asn Ser Arg Trp Glu Val Ile
465                 470                 475                 480
Ala Asn Tyr Met Asn Ile His Ser Ser Ser Gly Val Lys Arg Thr Ala
                485                 490                 495
Lys Asp Val Ile Ser Lys Ala Lys Ser Leu Gln Lys Leu Asp Pro His
            500                 505                 510
Gln Lys Asp Asp Ile Asn Lys Lys Ala Phe Asp Lys Phe Lys Lys Glu
        515                 520                 525
His Gly Val Ala Ser Gln Ala Asp Ser Ala Ala Pro Ser Glu Arg Phe

        530                 535                 540
Glu Gly Pro Cys Ile Asp Ser Thr Pro Trp Thr Thr Glu Glu Gln Lys
545                 550                 555                 560
Leu Leu Glu Gln Ala Leu Lys Thr Tyr Pro Val Asn Thr Pro Glu Arg
                565                 570                 575
Trp Glu Lys Ile Ala Glu Ala Val Pro Gly Arg Thr Lys Lys Asp Cys
            580                 585                 590
Met Arg Arg Tyr Lys Glu Leu Val Glu Met Val Lys Ala Lys Lys Ala
        595                 600                 605
Ala Gln Glu Gln Val Leu Asn Ala Ser Arg Ala Arg Lys
    610                 615                 620

<210> 71
<211> 267
<212> PRT
<213> Homo Sapiens

<400> 71

<210> 72
<211> 1752

<212> PRT
<213> Homo Sapiens

<400> 72
Arg Glu Lys Arg Arg Arg Lys Ser Val Glu Asp Arg Phe Asp Gln Gln
1               5                   10                  15
Lys Asn Asp Tyr Asp Gln Leu Gln Lys Ala Arg Gln Cys Glu Lys Glu
            20                  25                  30
Asn Leu Gly Trp Gln Lys Leu Glu Ser Glu Lys Ala Ile Lys Glu Lys
        35                  40                  45
Glu Tyr Glu Ile Glu Arg Leu Arg Val Leu Leu Gln Glu Glu Gly Thr
    50                  55                  60
Arg Lys Arg Glu Tyr Glu Asn Glu Leu Ala Lys Val Arg Asn His Tyr
65                  70                  75                  80
Asn Glu Glu Met Ser Asn Leu Arg Asn Lys Tyr Glu Thr Glu Ile Asn
                85                  90                  95
Ile Thr Lys Thr Thr Ile Lys Glu Ile Ser Met Gln Lys Glu Asp Asp
            100                 105                 110
Ser Lys Asn Leu Arg Asn Gln Leu Asp Arg Leu Ser Arg Glu Asn Arg
        115                 120                 125
Asp Leu Lys Asp Glu Ile Val Arg Leu Asn Asp Ser Ile Leu Gln Ala
    130                 135                 140
Thr Glu Gln Arg Arg Arg Ala Glu Glu Asn Ala Leu Gln Gln Lys Ala
145                 150                 155                 160
Cys Gly Ser Glu Ile Met Gln Lys Lys Gln His Leu Glu Ile Glu Leu
                165                 170                 175
Lys Gln Val Met Gln Gln Arg Ser Glu Asp Asn Ala Arg His Lys Gln
            180                 185                 190
Ser Leu Glu Glu Ala Ala Lys Thr Ile Gln Asp Lys Asn Lys Glu Ile
        195                 200                 205
Glu Arg Leu Lys Ala Glu Phe Gln Glu Glu Ala Lys Arg Arg Trp Glu
    210                 215                 220
Tyr Glu Asn Glu Leu Ser Lys Val Arg Asn Asn Tyr Asp Glu Glu Ile
225                 230                 235                 240
Ile Ser Leu Lys Asn Gln Phe Glu Thr Glu Ile Asn Ile Thr Lys Thr
                245                 250                 255
Thr Ile His Gln Leu Thr Met Gln Lys Glu Glu Asp Thr Ser Gly Tyr
            260                 265                 270
Arg Ala Gln Ile Asp Asn Leu Thr Arg Glu Asn Arg Ser Leu Ser Glu
        275                 280                 285
Glu Ile Lys Arg Leu Lys Asn Thr Leu Thr Gln Thr Thr Glu Asn Leu
    290                 295                 300
Arg Arg Val Glu Glu Asp Ile Gln Gln Gln Lys Ala Thr Gly Ser Glu
305                 310                 315                 320
Val Ser Gln Arg Lys Gln Gln Leu Glu Val Glu Leu Arg Gln Val Thr
                325                 330                 335
Gln Met Arg Thr Glu Glu Ser Val Arg Tyr Lys Gln Ser Leu Asp Asp
            340                 345                 350
Ala Ala Lys Thr Ile Gln Asp Lys Asn Lys Glu Ile Glu Arg Leu Lys
        355                 360                 365
Gln Leu Ile Asp Lys Glu Thr Asn Asp Arg Lys Cys Leu Glu Asp Glu
    370                 375                 380
Asn Ala Arg Leu Gln Arg Val Gln Tyr Asp Leu Gln Lys Ala Asn Ser
385                 390                 395                 400
Ser Ala Thr Glu Thr Ile Asn Lys Leu Lys Val Gln Glu Gln Glu Leu

                405                 410                 415
Thr Arg Leu Arg Ile Asp Tyr Glu Arg Val Ser Gln Glu Arg Thr Val
            420                 425                 430
Lys Asp Gln Asp Ile Thr Arg Phe Gln Asn Ser Leu Lys Glu Leu Gln
        435                 440                 445
Leu Gln Lys Gln Lys Val Glu Glu Glu Leu Asn Arg Leu Lys Arg Thr
    450                 455                 460
Ala Ser Glu Asp Ser Cys Lys Arg Lys Lys Leu Glu Glu Glu Leu Glu
465                 470                 475                 480
Gly Met Arg Arg Ser Leu Lys Glu Gln Ala Ile Lys Ile Thr Asn Leu
                485                 490                 495
Thr Gln Gln Leu Glu Gln Ala Ser Ile Val Lys Lys Arg Ser Glu Asp
            500                 505                 510
Asp Leu Arg Gln Gln Arg Asp Val Leu Asp Gly His Leu Arg Glu Lys
        515                 520                 525
Gln Arg Thr Gln Glu Glu Leu Arg Arg Leu Ser Ser Glu Val Glu Ala
    530                 535                 540
Leu Arg Arg Gln Leu Leu Gln Glu Gln Glu Ser Val Lys Gln Ala His
545                 550                 555                 560
Leu Arg Asn Glu His Phe Gln Lys Ala Ile Glu Asp Lys Ser Arg Ser
                565                 570                 575
Leu Asn Glu Ser Lys Ile Glu Ile Glu Arg Leu Gln Ser Leu Thr Glu
            580                 585                 590
Asn Leu Thr Lys Glu His Leu Met Leu Glu Glu Glu Leu Arg Asn Leu
        595                 600                 605
Arg Leu Glu Tyr Asp Asp Leu Arg Arg Gly Arg Ser Glu Ala Asp Ser
    610                 615                 620
Asp Lys Asn Ala Thr Ile Leu Glu Leu Arg Ser Gln Leu Gln Ile Ser
625                 630                 635                 640
Asn Asn Arg Thr Leu Glu Leu Gln Gly Leu Ile Asn Asp Leu Gln Arg
                645                 650                 655
Glu Arg Glu Asn Leu Arg Gln Glu Ile Glu Lys Phe Gln Lys Gln Ala
            660                 665                 670
Leu Glu Ala Ser Asn Arg Ile Gln Glu Ser Lys Asn Gln Cys Thr Gln
        675                 680                 685
Val Val Gln Glu Arg Glu Ser Leu Leu Val Lys Ile Lys Val Leu Glu
    690                 695                 700
Gln Asp Lys Ala Arg Leu Gln Arg Leu Glu Asp Glu Leu Asn Arg Ala
705                 710                 715                 720
Lys Ser Thr Leu Glu Ala Glu Thr Arg Val Lys Gln Arg Leu Glu Cys
                725                 730                 735
Glu Lys Gln Gln Ile Gln Asn Asp Leu Asn Gln Trp Lys Thr Gln Tyr
            740                 745                 750
Ser Arg Lys Glu Glu Ala Ile Arg Lys Ile Glu Ser Glu Arg Glu Lys
        755                 760                 765
Ser Glu Arg Glu Lys Asn Ser Leu Arg Ser Glu Ile Glu Arg Leu Gln
    770                 775                 780
Ala Glu Ile Lys Arg Ile Glu Glu Arg Cys Arg Arg Lys Leu Glu Asp
785                 790                 795                 800
Ser Thr Arg Glu Thr Gln Ser Gln Leu Glu Thr Glu Arg Ser Arg Tyr
                805                 810                 815
Gln Arg Glu Ile Asp Lys Leu Arg Gln Arg Pro Tyr Gly Ser His Arg
            820                 825                 830
Glu Thr Gln Thr Glu Cys Glu Trp Thr Val Asp Thr Ser Lys Leu Val
        835                 840                 845

Phe Asp Gly Leu Arg Lys Lys Val Thr Ala Met Gln Leu Tyr Glu Cys
    850                 855                 860
Gln Leu Ile Asp Lys Thr Thr Leu Asp Lys Leu Leu Lys Gly Lys Lys
865                 870                 875                 880
Ser Val Glu Glu Val Ala Ser Glu Ile Gln Pro Phe Leu Arg Gly Ala
                885                 890                 895
Gly Ser Ile Ala Gly Ala Ser Ala Ser Pro Lys Glu Lys Tyr Ser Leu
            900                 905                 910
Val Glu Ala Lys Arg Lys Lys Leu Ile Ser Pro Glu Ser Thr Val Met
        915                 920                 925
Leu Leu Glu Ala Gln Ala Ala Thr Gly Gly Ile Ile Asp Pro His Arg
    930                 935                 940
Asn Glu Lys Leu Thr Val Asp Ser Ala Ile Ala Arg Asp Leu Ile Asp
945                 950                 955                 960
Phe Asp Asp Arg Gln Gln Ile Tyr Ala Ala Glu Lys Ala Ile Thr Gly
                965                 970                 975
Phe Asp Asp Pro Phe Ser Gly Lys Thr Val Ser Val Ser Glu Ala Ile
            980                 985                 990
Lys Lys Asn Leu Ile Asp Arg Glu Thr Gly Met Arg Leu Leu Glu Ala
        995                 1000                1005
Gln Ile Ala Ser Gly Gly Val Val Asp Pro Val Asn Ser Val Phe Leu
    1010                1015                1020
Pro Lys Asp Val Ala Leu Ala Arg Gly Leu Ile Asp Arg Asp Leu Tyr
1025 1030 1035 104
Arg Ser Leu Asn Asp Pro Arg Asp Ser Gln Lys Asn Phe Val Asp Pro
                1045                1050                1055
Val Thr Lys Lys Lys Val Ser Tyr Val Gln Leu Lys Glu Arg Cys Arg
            1060                1065                1070
Ile Glu Pro His Thr Gly Leu Leu Leu Leu Ser Val Gln Lys Arg Ser
        1075                1080                1085
Met Ser Phe Gln Gly Ile Arg Gln Pro Val Thr Val Thr Glu Leu Val
    1090                1095                1100
Asp Ser Gly Ile Leu Arg Pro Ser Thr Val Asn Glu Leu Glu Ser Gly
1105 1110 1115 112
Gln Ile Ser Tyr Asp Glu Val Gly Glu Arg Ile Lys Asp Phe Leu Gln
                1125                1130                1135
Gly Ser Ser Cys Ile Ala Gly Ile Tyr Asn Glu Thr Thr Lys Gln Lys
            1140                1145                1150
Leu Gly Ile Tyr Glu Ala Met Lys Ile Gly Leu Val Arg Pro Gly Thr
        1155                1160                1165
Ala Leu Glu Leu Leu Glu Ala Gln Ala Ala Thr Gly Phe Ile Val Asp
    1170                1175                1180
Pro Val Ser Asn Leu Arg Leu Pro Val Glu Glu Ala Tyr Lys Arg Gly
1185 1190 1195 120
Leu Val Gly Ile Glu Phe Lys Glu Lys Leu Leu Ser Ala Glu Arg Ala
                1205                1210                1215
Val Thr Gly Tyr Asn Asp Pro Glu Thr Gly Asn Ile Ile Ser Leu Phe
            1220                1225                1230
Gln Ala Met Asn Lys Glu Leu Ile Glu Lys Gly His Gly Ile Arg Leu
        1235                1240                1245
Leu Glu Ala Gln Ile Ala Thr Gly Gly Ile Ile Asp Pro Lys Glu Ser
    1250                1255                1260
His Arg Leu Pro Val Asp Ile Ala Tyr Lys Arg Gly Tyr Phe Asn Glu
1265 1270 1275 128
Glu Leu Ser Glu Ile Leu Ser Asp Pro Ser Asp Asp Thr Lys Gly Phe

                    1285                    1290                    1295
Phe Asp Pro Asn Thr Glu Glu Asn Leu Thr Tyr Leu Gln Leu Lys Glu
                1300                    1305                    1310
Arg Cys Ile Lys Asp Glu Glu Thr Gly Leu Cys Leu Leu Pro Leu Lys
            1315                    1320                    1325
Glu Lys Lys Lys Gln Val Gln Thr Ser Gln Lys Asn Thr Leu Arg Lys
        1330                    1335                    1340
Arg Arg Val Val Ile Val Asp Pro Glu Thr Asn Lys Glu Met Ser Val
1345                    1350                    1355                    136
Gln Glu Ala Tyr Lys Lys Gly Leu Ile Asp Tyr Glu Thr Phe Lys Glu
                    1365                    1370                    1375
Leu Cys Glu Gln Glu Cys Glu Trp Glu Glu Ile Thr Ile Thr Gly Ser
                1380                    1385                    1390
Asp Gly Ser Thr Arg Val Val Leu Val Asp Arg Lys Thr Gly Ser Gln
            1395                    1400                    1405
Tyr Asp Ile Gln Asp Ala Ile Asp Lys Gly Leu Val Asp Arg Lys Phe
        1410                    1415                    1420
Phe Asp Gln Tyr Arg Ser Gly Ser Leu Ser Leu Thr Gln Phe Ala Asp
1425                    1430                    1435                    144
Met Ile Ser Leu Lys Asn Gly Val Gly Thr Ser Ser Ser Met Gly Ser
                    1445                    1450                    1455
Gly Val Ser Asp Asp Val Phe Ser Ser Ser Arg His Glu Ser Val Ser
                1460                    1465                    1470
Lys Ile Ser Thr Ile Ser Ser Val Arg Asn Leu Thr Ile Arg Ser Ser
            1475                    1480                    1485
Ser Phe Ser Asp Thr Leu Glu Glu Ser Ser Pro Ile Ala Ala Ile Phe
        1490                    1495                    1500
Asp Thr Glu Asn Leu Glu Lys Ile Ser Ile Thr Glu Gly Ile Glu Arg
1505                    1510                    1515                    152
Gly Ile Val Asp Ser Ile Thr Gly Gln Arg Leu Leu Glu Ala Gln Ala
                    1525                    1530                    1535
Cys Thr Gly Gly Ile Ile His Pro Thr Thr Gly Gln Lys Leu Ser Leu
                1540                    1545                    1550
Gln Asp Ala Val Ser Gln Gly Val Ile Asp Gln Asp Met Ala Thr Ser
            1555                    1560                    1565
Val Lys Pro Ala Gln Lys Ala Phe Ile Gly Phe Glu Gly Val Lys Gly
        1570                    1575                    1580
Lys Lys Lys Met Ser Ala Ala Glu Ala Val Lys Glu Lys Trp Leu Pro
1585                    1590                    1595                    160
Tyr Glu Ala Gly Gln Arg Phe Leu Glu Phe Gln Tyr Leu Thr Gly Gly
                    1605                    1610                    1615
Leu Val Asp Pro Glu Val His Gly Arg Ile Ser Thr Glu Glu Ala Ile
                1620                    1625                    1630
Arg Lys Gly Phe Ile Asp Gly Arg Ala Ala Gln Arg Leu Gln Asp Thr
            1635                    1640                    1645
Ser Ser Tyr Ala Lys Ile Leu Thr Cys Pro Lys Thr Lys Leu Lys Ile
        1650                    1655                    1660
Ser Tyr Lys Asp Ala Ile Asn Arg Ser Met Val Glu Asp Ile Thr Gly
1665                    1670                    1675                    168
Leu Arg Leu Leu Glu Ala Ala Ser Val Ser Ser Lys Gly Leu Pro Ser
                    1685                    1690                    1695
Pro Tyr Asn Met Ser Ser Ala Pro Gly Ser Arg Ser Gly Ser Arg Ser
                1700                    1705                    1710
Gly Ser Arg Ser Gly Ser Arg Ser Gly Ser Arg Ser Gly Ser Arg Arg
            1715                    1720                    1725

Gly Ser Phe Asp Ala Thr Gly Asn Ser Ser Tyr Ser Tyr Ser Tyr Ser
    1730                1735                1740
Phe Ser Ser Ser Ser Ile Gly His
1745                1750

<210> 73
<211> 1978
<212> PRT
<213> Homo Sapiens

<400> 73

<210> 74
<211> 366
<212> PRT
<213> Homo Sapiens

<400> 74
Met Arg Val Met Ala Pro Arg Thr Leu Ile Leu Leu Leu Ser Gly Ala
1               5                   10                  15
Leu Ala Leu Thr Glu Thr Trp Ala Gly Ser His Ser Met Arg Tyr Phe
            20                  25                  30
Tyr Thr Ala Val Ser Arg Pro Gly Arg Gly Glu Pro His Phe Ile Ala
        35                  40                  45
Val Gly Tyr Val Asp Asp Thr Gln Phe Val Arg Phe Asp Ser Asp Ala
    50                  55                  60
Ala Ser Pro Arg Gly Glu Pro Arg Ala Pro Trp Val Glu Gln Glu Gly

65                  70                  75                  80
Pro Glu Tyr Trp Asp Arg Glu Thr Gln Lys Tyr Lys Arg Gln Ala Gln
                85                  90                  95
Thr Asp Arg Val Ser Leu Arg Asn Leu Arg Gly Tyr Tyr Asn Gln Ser
            100                 105                 110
Glu Ala Gly Ser His Ile Ile Gln Arg Met Tyr Gly Cys Asp Val Gly
        115                 120                 125
Pro Asp Gly Arg Leu Leu Arg Gly Tyr Asp Gln Tyr Ala Tyr Asp Gly
    130                 135                 140
Lys Asp Tyr Ile Ala Leu Asn Glu Asp Leu Arg Ser Trp Thr Ala Ala
145                 150                 155                 160
Asp Thr Ala Ala Gln Ile Thr Gln Arg Lys Trp Glu Ala Ala Arg Glu
                165                 170                 175
Ala Glu Gln Leu Arg Ala Tyr Leu Glu Gly Leu Cys Val Glu Trp Leu
            180                 185                 190
Arg Arg Tyr Leu Lys Asn Gly Lys Glu Thr Leu Gln Arg Ala Glu His
        195                 200                 205
Pro Lys Thr His Val Thr His His Pro Val Ser Asp His Glu Ala Thr
    210                 215                 220
Leu Arg Cys Trp Ala Leu Gly Phe Tyr Pro Ala Glu Ile Thr Leu Thr
225                 230                 235                 240
Trp Gln Trp Asp Gly Glu Asp Gln Thr Gln Asp Thr Glu Leu Val Glu
                245                 250                 255
Thr Arg Pro Ala Gly Asp Gly Thr Phe Gln Lys Trp Ala Ala Val Val
            260                 265                 270
Val Pro Ser Gly Glu Glu Gln Arg Tyr Thr Cys His Val Gln His Glu
        275                 280                 285
Gly Leu Pro Glu Pro Leu Thr Leu Arg Trp Glu Pro Ser Ser Gln Pro
    290                 295                 300
Thr Ile Pro Ile Val Gly Ile Val Ala Gly Leu Ala Val Leu Ala Val
305                 310                 315                 320
Leu Ala Val Leu Gly Ala Val Val Ala Val Val Met Cys Arg Arg Lys
                325                 330                 335
Ser Ser Gly Gly Lys Gly Gly Ser Cys Ser Gln Ala Ala Ser Ser Asn
            340                 345                 350
Ser Ala Gln Gly Ser Asp Glu Ser Leu Ile Ala Cys Lys Ala
        355                 360                 365

<210> 75
<211> 240
<212> PRT
<213> Homo Sapiens

<400> 75
Met Gly Leu Glu Leu Tyr Leu Asp Leu Leu Ser Gln Pro Cys Arg Ala
1               5                   10                  15
Val Tyr Ile Phe Ala Lys Lys Asn Asp Ile Pro Phe Glu Leu Arg Ile
            20                  25                  30
Val Asp Leu Ile Lys Gly Gln His Leu Ser Asp Ala Phe Ala Gln Val
        35                  40                  45
Asn Pro Leu Lys Lys Val Pro Ala Leu Lys Asp Gly Asp Phe Thr Leu
    50                  55                  60
Thr Glu Ser Val Ala Ile Leu Leu Tyr Leu Thr Arg Lys Tyr Lys Val
65                  70                  75                  80
Pro Asp Tyr Trp Tyr Pro Gln Asp Leu Gln Ala Arg Ala Arg Val Asp

85                  90                  95
Glu Tyr Leu Ala Trp Gln His Thr Thr Leu Arg Arg Ser Cys Leu Arg
            100                 105                 110
Ala Leu Trp His Lys Val Met Phe Pro Val Phe Leu Gly Gly Pro Val
        115                 120                 125
Ser Pro Gln Thr Leu Ala Ala Thr Leu Ala Glu Leu Asp Val Thr Leu
    130                 135                 140
Gln Leu Leu Glu Asp Lys Phe Leu Gln Asn Lys Ala Phe Leu Thr Gly
145                 150                 155                 160
Pro His Ile Ser Leu Ala Asp Leu Val Ala Ile Thr Glu Leu Met His
                165                 170                 175
Pro Val Gly Ala Gly Cys Gln Val Phe Glu Gly Arg Pro Lys Leu Ala
            180                 185                 190
Thr Trp Arg Gln Arg Val Glu Ala Ala Val Gly Glu Asp Leu Phe Gln
        195                 200                 205
Glu Ala His Glu Val Ile Leu Lys Ala Lys Asp Phe Pro Pro Ala Asp
    210                 215                 220
Pro Thr Ile Lys Gln Lys Leu Met Pro Trp Val Leu Ala Met Ile Arg
225                 230                 235                 240

<210> 76
<211> 953
<212> PRT
<213> Homo Sapiens

<400> 76

<210> 77
<211> 335
<212> PRT
<213> Homo Sapiens

<400> 77
Met Gly Lys Val Lys Val Gly Val Asn Gly Phe Gly Arg Ile Gly Arg
 1               5                  10                  15
Leu Val Thr Arg Ala Ala Phe Asn Ser Gly Lys Val Asp Ile Val Ala
            20                  25                  30
Ile Asn Asp Pro Phe Ile Asp Leu Asn Tyr Met Val Tyr Met Phe Gln
        35                  40                  45
Tyr Asp Ser Thr His Gly Lys Phe His Gly Thr Val Lys Ala Glu Asn
    50                  55                  60
Gly Lys Leu Val Ile Asn Gly Asn Pro Ile Thr Ile Phe Gln Glu Arg
65                  70                  75                  80
Asp Pro Ser Lys Ile Lys Trp Gly Asp Ala Gly Ala Glu Tyr Val Val
                85                  90                  95

Glu Ser Thr Gly Val Phe Thr Thr Met Glu Lys Ala Gly Ala His Leu
            100                 105                 110
Gln Gly Gly Ala Lys Arg Val Ile Ile Ser Ala Pro Ser Ala Asp Ala
        115                 120                 125
Pro Met Phe Val Met Gly Val Asn His Glu Lys Tyr Asp Asn Ser Leu
    130                 135                 140
Lys Ile Ile Ser Asn Ala Ser Cys Thr Thr Asn Cys Leu Ala Pro Leu
145                 150                 155                 160
Ala Lys Val Ile His Asp Asn Phe Gly Ile Val Glu Gly Leu Met Thr
                165                 170                 175
Thr Val His Ala Ile Thr Ala Thr Gln Lys Thr Val Asp Gly Pro Ser
            180                 185                 190
Gly Lys Leu Trp Arg Asp Gly Arg Gly Ala Leu Gln Asn Ile Ile Pro
        195                 200                 205
Ala Ser Thr Gly Ala Ala Lys Ala Val Gly Lys Val Ile Pro Glu Leu
    210                 215                 220
Asn Gly Lys Leu Thr Gly Met Ala Phe Arg Val Pro Thr Ala Asn Val
225                 230                 235                 240
Ser Val Val Asp Leu Thr Cys Arg Leu Glu Lys Pro Ala Lys Tyr Asp
                245                 250                 255
Asp Ile Lys Lys Val Val Lys Gln Ala Ser Glu Gly Pro Leu Lys Gly
            260                 265                 270
Ile Leu Gly Tyr Thr Glu His Gln Val Val Ser Ser Asp Phe Asn Ser
        275                 280                 285
Asp Thr His Ser Ser Thr Phe Asp Ala Gly Ala Gly Ile Ala Leu Asn
    290                 295                 300
Asp His Phe Val Lys Leu Ile Ser Trp Tyr Asp Asn Glu Phe Gly Tyr
305                 310                 315                 320
Ser Asn Arg Val Val Asp Leu Met Ala His Met Ala Ser Lys Glu
                325                 330                 335

<210> 78
<211> 117
<212> PRT
<213> Homo Sapiens

<400> 78
Met Val Gln Arg Leu Thr Tyr Arg Arg Arg Leu Ser Tyr Asn Thr Ala
1               5                   10                  15
Ser Asn Lys Thr Arg Leu Ser Arg Thr Pro Gly Asn Arg Ile Val Tyr
            20                  25                  30
Leu Tyr Thr Lys Lys Val Gly Lys Ala Pro Lys Ser Ala Cys Gly Val
        35                  40                  45
Cys Pro Gly Lys Leu Arg Gly Val Arg Pro Val Arg Pro Lys Val Leu
    50                  55                  60
Met Arg Leu Ser Lys Thr Lys Lys His Val Ser Arg Ala Tyr Gly Gly
65                  70                  75                  80
Ser Met Cys Ala Lys Cys Val Arg Asp Arg Ile Lys Arg Ala Phe Leu
                85                  90                  95
Ile Glu Glu Gln Lys Ile Ile Val Lys Val Leu Lys Ala Gln Ala Gln
            100                 105                 110
Ser Gln Lys Ala Lys
        115

<210> 79

<211> 614
<212> PRT
<213> Homo Sapiens

<400> 79
Arg Ser Gly Gln Pro Arg Ala Glu Gly Leu Gly Ala Gly Ala Ala Gly
1               5                   10                  15
Pro Leu Arg Ala Met Ala Ala Pro Val Lys Gly Asn Arg Lys Gln Ser
            20                  25                  30
Thr Glu Gly Asp Ala Leu Asp Pro Pro Ala Ser Pro Lys Pro Ala Gly
        35                  40                  45
Lys Gln Asn Gly Ile Gln Asn Pro Ile Ser Leu Glu Asp Ser Pro Glu
    50                  55                  60
Ala Gly Gly Glu Arg Glu Glu Glu Gln Glu Arg Glu Glu Glu Gln Ala
65                  70                  75                  80
Phe Leu Val Ser Leu Tyr Lys Phe Met Lys Glu Arg His Thr Pro Ile
                85                  90                  95
Glu Arg Val Pro His Leu Gly Phe Lys Gln Ile Asn Leu Trp Lys Ile
            100                 105                 110
Tyr Lys Ala Val Glu Lys Leu Gly Ala Tyr Glu Leu Val Thr Gly Arg
        115                 120                 125
Arg Leu Trp Lys Asn Val Tyr Asp Glu Leu Gly Gly Ser Pro Gly Ser
    130                 135                 140
Thr Ser Ala Ala Thr Cys Thr Arg Arg His Tyr Glu Arg Leu Val Leu
145                 150                 155                 160
Pro Tyr Val Arg His Leu Lys Gly Glu Asp Asp Lys Pro Leu Pro Thr
                165                 170                 175
Ser Lys Pro Arg Lys Gln Tyr Lys Met Ala Lys Glu Asn Arg Gly Asp
            180                 185                 190
Asp Gly Ala Thr Glu Arg Pro Lys Lys Ala Lys Glu Glu Arg Arg Met
        195                 200                 205
Asp Gln Met Met Pro Gly Lys Thr Lys Ala Asp Ala Ala Asp Pro Ala
    210                 215                 220
Pro Leu Pro Ser Gln Glu Pro Pro Arg Asn Ser Thr Glu Gln Gln Gly
225                 230                 235                 240
Leu Ala Ser Gly Ser Ser Val Ser Phe Val Gly Ala Ser Gly Cys Pro
                245                 250                 255
Glu Ala Tyr Lys Arg Leu Leu Ser Ser Phe Tyr Cys Lys Gly Thr His
            260                 265                 270
Gly Ile Met Ser Pro Leu Ala Lys Lys Lys Leu Leu Ala Gln Val Ser
        275                 280                 285
Lys Val Glu Ala Leu Gln Cys Gln Glu Glu Gly Cys Arg His Gly Ala
    290                 295                 300
Glu Pro Gln Ala Ser Pro Ala Val His Leu Pro Glu Ser Pro Gln Ser
305                 310                 315                 320
Pro Lys Gly Leu Thr Glu Asn Ser Arg His Arg Leu Thr Pro Gln Glu
                325                 330                 335
Gly Leu Gln Ala Pro Gly Gly Ser Leu Arg Glu Glu Ala Gln Ala Gly
            340                 345                 350
Pro Cys Pro Ala Ala Pro Ile Phe Lys Gly Cys Phe Tyr Thr His Pro
        355                 360                 365
Thr Glu Val Leu Lys Pro Val Ser Gln His Pro Arg Asp Phe Phe Ser
    370                 375                 380
Arg Leu Lys Asp Gly Val Leu Leu Gly Pro Pro Gly Lys Glu Gly Leu
385                 390                 395                 400

Ser Val Lys Glu Pro Gln Leu Val Trp Gly Gly Asp Ala Asn Arg Pro
                405                 410                 415
Ser Ala Phe His Lys Gly Gly Ser Arg Lys Gly Ile Leu Tyr Pro Lys
            420                 425                 430
Pro Lys Ala Cys Trp Val Ser Pro Met Ala Lys Val Pro Ala Glu Ser
        435                 440                 445
Pro Thr Leu Pro Pro Thr Phe Pro Ser Ser Pro Gly Leu Gly Ser Lys
    450                 455                 460
Arg Ser Leu Glu Glu Glu Gly Ala Ala His Ser Gly Lys Arg Leu Arg
465                 470                 475                 480
Ala Val Ser Pro Phe Leu Lys Glu Ala Asp Ala Lys Lys Cys Gly Ala
                485                 490                 495
Lys Pro Ala Gly Ser Gly Leu Val Ser Cys Leu Leu Gly Pro Ala Leu
            500                 505                 510
Gly Pro Val Pro Pro Glu Ala Tyr Arg Gly Thr Met Leu His Cys Pro
        515                 520                 525
Leu Asn Phe Thr Gly Thr Pro Gly Pro Leu Lys Gly Gln Ala Ala Leu
    530                 535                 540
Pro Phe Ser Pro Leu Val Ile Pro Ala Phe Pro Ala His Phe Leu Ala
545                 550                 555                 560
Thr Ala Gly Pro Ser Pro Met Ala Ala Gly Leu Met His Phe Pro Pro
                565                 570                 575
Thr Ser Phe Asp Ser Ala Leu Arg His Arg Leu Cys Pro Ala Ser Ser
            580                 585                 590
Ala Trp His Ala Pro Pro Val Thr Thr Tyr Ala Ala Pro His Phe Phe
        595                 600                 605
His Leu Asn Thr Lys Leu
    610

<210> 80
<211> 114
<212> PRT
<213> Homo Sapiens

<400> 80
Met Ala Ser Val Ser Glu Leu Ala Cys Ile Tyr Ser Ala Leu Ile Leu
1               5                   10                  15
His Asp Asp Glu Val Thr Val Thr Glu Asp Lys Ile Asn Ala Leu Ile
            20                  25                  30
Lys Ala Ala Gly Val Asn Val Glu Pro Phe Trp Pro Gly Leu Phe Ala
        35                  40                  45
Lys Ala Leu Ala Asn Val Asn Ile Gly Ser Leu Ile Cys Asn Val Gly
    50                  55                  60
Ala Gly Gly Pro Ala Pro Ala Ala Gly Ala Ala Pro Ala Gly Gly Pro
65                  70                  75                  80
Ala Pro Ser Thr Ala Ala Ala Pro Ala Glu Glu Lys Lys Val Glu Ala
                85                  90                  95
Lys Lys Glu Glu Ser Glu Glu Ser Asp Asp Asp Met Gly Phe Gly Leu
            100                 105                 110
Phe Asp

<210> 81
<211> 596
<212> PRT

<213> Homo Sapiens

<400> 81
Met Arg Arg Ala His Glu Gly Arg Glu Ile Pro Ser Leu Gly Gly Ala
1               5                   10                  15
Arg Arg Arg Glu Val Leu Gln Ala Gly Arg Ser Gln Arg Ala Ala Gly
            20                  25                  30
Arg Arg Arg Arg Arg Gln Glu Leu Glu Leu Gly Val Gly Ser Gly Arg
        35                  40                  45
Pro Gly Gly Pro Pro Pro Gly Pro Gly Arg Arg Gly Thr Cys Ala Ala
    50                  55                  60
Ala Leu Pro Pro Glu Trp Pro Arg Arg Arg Thr Gly Leu Pro Arg Arg
65                  70                  75                  80
Gly Pro Arg Pro Pro Leu Ala Met Ala Lys Trp Leu Asn Lys Tyr Phe
                85                  90                  95
Ser Leu Gly Asn Ser Lys Thr Lys Ser Pro Pro Gln Pro Pro Arg Pro
            100                 105                 110
Asp Tyr Arg Glu Gln Arg Arg Arg Gly Glu Arg Pro Ser Gln Pro Pro
        115                 120                 125
Gln Ala Val Pro Gln Ala Ser Ser Ala Ala Ser Ala Ser Cys Gly Pro
    130                 135                 140
Ala Thr Ala Ser Cys Phe Ser Ala Ser Ser Gly Ser Leu Pro Asp Asp
145                 150                 155                 160
Ser Gly Ser Thr Ser Asp Leu Ile Arg Ala Tyr Arg Ala Gln Lys Glu
                165                 170                 175
Arg His Phe Gln Asp Pro Tyr Asn Gly Pro Gly Ser Ser Leu Arg Lys
            180                 185                 190
Leu Arg Ala Met Cys Arg Leu Asp Tyr Cys Gly Gly Ser Gly Glu Pro
        195                 200                 205
Gly Gly Val Gln Arg Ala Phe Ser Ala Ser Ser Ala Ser Gly Ala Ala
    210                 215                 220
Gly Cys Cys Cys Ala Ser Ser Gly Ala Gly Ala Ala Ala Ser Ser Ser
225                 230                 235                 240
Ser Ser Ser Gly Ser Pro His Leu Tyr Arg Ser Ser Ser Glu Arg Arg
                245                 250                 255
Pro Ala Thr Pro Ala Glu Val Arg Tyr Ile Ser Pro Lys His Arg Leu
            260                 265                 270
Ile Lys Val Glu Ser Ala Ala Gly Gly Gly Ala Gly Asp Pro Leu Gly
        275                 280                 285
Gly Ala Cys Ala Gly Gly Arg Thr Trp Ser Pro Thr Ala Cys Gly Gly
    290                 295                 300
Lys Lys Leu Leu Asn Lys Cys Ala Ala Ser Ala Ala Glu Glu Ser Gly
305                 310                 315                 320
Ala Gly Lys Lys Asp Lys Val Thr Ile Ala Asp Asp Tyr Ser Asp Pro
                325                 330                 335
Phe Asp Ala Lys Asn Asp Leu Lys Ser Lys Ala Gly Lys Gly Glu Ser
            340                 345                 350
Ala Gly Tyr Met Glu Pro Tyr Glu Ala Gln Arg Ile Met Thr Glu Phe
        355                 360                 365
Gln Arg Gln Glu Ser Val Arg Ser Gln His Lys Gly Ile Gln Leu Tyr
    370                 375                 380
Asp Thr Pro Tyr Glu Pro Glu Gly Gln Ser Val Asp Ser Asp Ser Glu
385                 390                 395                 400
Ser Thr Val Ser Pro Arg Leu Arg Glu Ser Lys Leu Pro Gln Asp Asp
                405                 410                 415

Asp Arg Pro Ala Asp Glu Tyr Asp Gln Pro Trp Glu Trp Asn Arg Val
                420                 425                 430
Thr Ser Pro Ala Leu Ala Ala Gln Phe Asn Gly Asn Glu Lys Arg Gln
        435                 440                 445
Ser Ser Pro Ser Pro Ser Arg Asp Arg Arg Arg Gln Leu Arg Ala Pro
    450                 455                 460
Gly Gly Gly Phe Lys Pro Ile Lys His Gly Ser Pro Glu Phe Cys Gly
465                 470                 475                 480
Ile Leu Gly Glu Arg Val Asp Pro Ala Val Pro Leu Glu Lys Gln Ile
                485                 490                 495
Trp Tyr His Gly Ala Ile Ser Arg Gly Asp Ala Glu Asn Leu Leu Arg
            500                 505                 510
Leu Cys Lys Glu Cys Ser Tyr Leu Val Arg Asn Ser Gln Thr Ser Lys
        515                 520                 525
His Asp Tyr Pro Leu Ser Leu Arg Ser Asn Gln Gly Phe Met His Met
    530                 535                 540
Lys Leu Ala Lys Thr Lys Glu Lys Tyr Val Leu Gly Gln Asn Ser Pro
545                 550                 555                 560
Pro Phe Asp Ser Val Pro Glu Val Ile His Tyr Tyr Thr Thr Arg Lys
                565                 570                 575
Leu Pro Ile Lys Gly Ala Glu His Leu Ser Leu Leu Tyr Pro Val Ala
            580                 585                 590
Val Arg Thr Leu
        595

<210> 82
<211> 207
<212> PRT
<213> Homo Sapiens

<400> 82
Met Ser Pro Leu Leu Arg Arg Leu Leu Leu Ala Ala Leu Leu Gln Leu
 1               5                  10                  15
Ala Pro Ala Gln Ala Pro Val Ser Gln Pro Asp Ala Pro Gly His Gln
                20                  25                  30
Arg Lys Val Val Ser Trp Ile Asp Val Tyr Thr Arg Ala Thr Cys Gln
        35                  40                  45
Pro Arg Glu Val Val Val Pro Leu Thr Val Glu Leu Met Gly Thr Val
    50                  55                  60
Ala Lys Gln Leu Val Pro Ser Cys Val Thr Val Gln Arg Cys Gly Gly
65                  70                  75                  80
Cys Cys Pro Asp Asp Gly Leu Glu Cys Val Pro Thr Gly Gln His Gln
                85                  90                  95
Val Arg Met Gln Ile Leu Met Ile Arg Tyr Pro Ser Ser Gln Leu Gly
            100                 105                 110
Glu Met Ser Leu Glu Glu His Ser Gln Cys Glu Cys Arg Pro Lys Lys
        115                 120                 125
Lys Asp Ser Ala Val Lys Pro Asp Arg Ala Ala Thr Pro His His Arg
    130                 135                 140
Pro Gln Pro Arg Ser Val Pro Gly Trp Asp Ser Ala Pro Gly Ala Pro
145                 150                 155                 160
Ser Pro Ala Asp Ile Thr His Pro Thr Pro Ala Pro Gly Pro Ser Ala
                165                 170                 175
His Ala Ala Pro Ser Thr Thr Ser Ala Leu Thr Pro Gly Pro Ala Ala
            180                 185                 190

Ala Ala Ala Asp Ala Ala Ala Ser Ser Val Ala Lys Gly Gly Ala
        195                 200                 205

<210> 83
<211> 429
<212> PRT
<213> Homo Sapiens

<400> 83
Glu Cys Asp Val Met Thr Tyr Val Arg Glu Thr Cys Gly Cys Cys Asp
1               5                   10                  15
Cys Glu Lys Arg Cys Gly Ala Leu Asp Val Val Phe Val Ile Asp Ser
            20                  25                  30
Ser Glu Ser Ile Gly Tyr Thr Asn Phe Thr Leu Glu Lys Asn Phe Val
        35                  40                  45
Ile Asn Val Val Asn Arg Leu Gly Ala Ile Ala Lys Asp Pro Lys Ser
    50                  55                  60
Glu Thr Gly Thr Arg Val Gly Val Val Gln Tyr Ser His Glu Gly Thr
65                  70                  75                  80
Phe Glu Ala Ile Gln Leu Asp Asp Glu His Ile Asp Ser Leu Ser Ser
                85                  90                  95
Phe Lys Glu Ala Val Lys Asn Leu Glu Trp Ile Ala Gly Gly Thr Trp
            100                 105                 110
Thr Pro Ser Ala Leu Lys Phe Ala Tyr Asp Arg Leu Ile Lys Glu Ser
        115                 120                 125
Arg Arg Gln Lys Thr Arg Val Phe Ala Val Val Ile Thr Asp Gly Arg
    130                 135                 140
His Asp Pro Arg Asp Asp Asp Leu Asn Leu Arg Ala Leu Cys Asp Arg
145                 150                 155                 160
Asp Val Thr Val Thr Ala Ile Gly Ile Gly Asp Met Phe His Glu Lys
                165                 170                 175
His Glu Ser Glu Asn Leu Tyr Ser Ile Ala Cys Asp Lys Pro Gln Gln
            180                 185                 190
Val Arg Asn Met Thr Leu Phe Ser Asp Leu Val Ala Glu Lys Phe Ile
        195                 200                 205
Asp Asp Met Glu Asp Val Leu Cys Pro Asp Pro Gln Ile Val Cys Pro
    210                 215                 220
Asp Leu Pro Cys Gln Thr Glu Leu Ser Val Ala Gln Cys Thr Gln Arg
225                 230                 235                 240
Pro Val Asp Ile Val Phe Leu Leu Asp Gly Ser Glu Arg Leu Gly Glu
                245                 250                 255
Gln Asn Phe His Lys Ala Arg Arg Phe Val Glu Gln Val Ala Arg Arg
            260                 265                 270
Leu Thr Leu Ala Arg Arg Asp Asp Asp Pro Leu Asn Ala Arg Val Ala
        275                 280                 285
Leu Leu Gln Phe Gly Gly Pro Gly Glu Gln Gln Val Ala Phe Pro Leu
    290                 295                 300
Ser His Asn Leu Thr Ala Ile His Glu Ala Leu Glu Thr Thr Gln Tyr
305                 310                 315                 320
Leu Asn Ser Phe Ser His Val Gly Ala Gly Val Val His Ala Ile Asn
                325                 330                 335
Ala Ile Val Arg Ser Pro Arg Gly Gly Ala Arg Arg His Ala Glu Leu
            340                 345                 350
Ser Phe Val Phe Leu Thr Asp Gly Val Thr Gly Asn Asp Ser Leu His
        355                 360                 365

Glu Ser Ala His Ser Met Arg Asn Glu Asn Val Val Pro Thr Val Leu
    370                 375                 380
Ala Leu Gly Ser Asp Val Asp Met Asp Val Leu Thr Thr Leu Ser Leu
385                 390                 395                 400
Gly Asp Arg Ala Ala Val Phe His Glu Lys Asp Tyr Asp Ser Leu Ala
                405                 410                 415
Gln Pro Gly Phe Phe Asp Arg Phe Ile Arg Trp Ile Cys
            420                 425

<210> 84
<211> 113
<212> PRT
<213> Homo Sapiens

<400> 84
Met Ser Ala Ser Val Val Ser Val Ile Ser Arg Phe Leu Glu Glu Tyr
 1               5                  10                  15
Leu Ser Ser Thr Pro Gln Arg Leu Lys Leu Leu Asp Ala Tyr Leu Leu
            20                  25                  30
Tyr Ile Leu Leu Thr Gly Ala Leu Gln Phe Gly Tyr Cys Leu Leu Val
        35                  40                  45
Gly Thr Phe Pro Phe Asn Ser Phe Leu Ser Gly Phe Ile Ser Cys Val
    50                  55                  60
Gly Ser Phe Ile Leu Ala Val Cys Leu Arg Ile Gln Ile Asn Pro Gln
65                  70                  75                  80
Asn Lys Ala Asp Phe Gln Gly Ile Ser Pro Glu Arg Ala Phe Ala Asp
                85                  90                  95
Phe Leu Phe Ala Ser Thr Ile Leu His Leu Val Val Met Asn Phe Val
            100                 105                 110
Gly

<210> 85
<211> 258
<212> PRT
<213> Homo Sapiens

<400> 85

<210> 86
<211> 569
<212> PRT
<213> Homo Sapiens

<400> 86
Met Ser Thr Met Val Tyr Ile Lys Glu Asp Lys Leu Glu Lys Leu Thr
1               5                   10                  15
Gln Asp Glu Ile Ile Ser Lys Thr Lys Gln Val Ile Gln Gly Leu Glu
            20                  25                  30
Ala Leu Lys Asn Glu His Asn Ser Ile Leu Gln Ser Leu Leu Glu Thr
        35                  40                  45
Leu Lys Cys Leu Lys Lys Asp Asp Glu Ser Asn Leu Val Glu Glu Lys
    50                  55                  60
Ser Asn Met Ile Arg Lys Ser Leu Glu Met Leu Glu Leu Gly Leu Ser
65                  70                  75                  80
Glu Ala Gln Val Met Met Ala Leu Ser Asn His Leu Asn Ala Val Glu
                85                  90                  95
Ser Glu Lys Gln Lys Leu Arg Ala Gln Val Arg Arg Leu Cys Gln Glu
            100                 105                 110
Asn Gln Trp Leu Arg Asp Glu Leu Ala Asn Thr Gln Gln Lys Leu Gln
        115                 120                 125
Lys Ser Glu Gln Ser Val Ala Gln Leu Glu Glu Glu Lys Lys His Leu
    130                 135                 140
Glu Phe Met Asn Gln Leu Lys Lys Tyr Asp Asp Asp Ile Ser Pro Ser
145                 150                 155                 160
Glu Asp Lys Asp Thr Asp Ser Thr Lys Glu Pro Leu Asp Asp Leu Phe
                165                 170                 175
Pro Asn Asp Glu Asp Asp Pro Gly Gln Gly Ile Gln Gln Gln His Ser
            180                 185                 190
Ser Ala Ala Ala Ala Ala Gln Gln Gly Gly Tyr Glu Ile Pro Ala Arg
        195                 200                 205
Leu Arg Thr Leu His Asn Leu Val Ile Gln Tyr Ala Ser Gln Gly Arg
    210                 215                 220
Tyr Glu Val Ala Val Pro Leu Cys Lys Gln Ala Leu Glu Asp Leu Glu
225                 230                 235                 240
Lys Thr Ser Gly His Asp His Pro Asp Val Ala Thr Met Leu Asn Ile

                245                 250                 255
Leu Ala Leu Val Tyr Arg Asp Gln Asn Lys Tyr Lys Asp Ala Ala Asn
            260                 265                 270
Leu Leu Asn Asp Ala Leu Ala Ile Arg Glu Lys Thr Leu Gly Lys Asp
        275                 280                 285
His Pro Ala Val Ala Ala Thr Leu Asn Asn Leu Ala Val Leu Tyr Gly
    290                 295                 300
Lys Arg Gly Lys Tyr Lys Glu Ala Glu Pro Leu Cys Lys Arg Ala Leu
305                 310                 315                 320
Glu Ile Arg Glu Lys Val Leu Gly Lys Asp His Pro Asp Val Ala Lys
                325                 330                 335
Gln Leu Asn Asn Leu Ala Leu Leu Cys Gln Asn Gln Gly Lys Tyr Glu
            340                 345                 350
Glu Val Glu Tyr Tyr Tyr Gln Arg Ala Leu Glu Ile Tyr Gln Thr Lys
        355                 360                 365
Leu Gly Pro Asp Asp Pro Asn Val Ala Lys Thr Lys Asn Asn Leu Ala
    370                 375                 380
Ser Cys Tyr Leu Lys Gln Gly Lys Phe Lys Gln Ala Glu Thr Leu Tyr
385                 390                 395                 400
Lys Glu Ile Leu Thr Arg Ala His Glu Arg Glu Phe Gly Ser Val Asp
                405                 410                 415
Asp Glu Asn Lys Pro Ile Trp Met His Ala Glu Glu Arg Glu Glu Cys
            420                 425                 430
Lys Gly Lys Gln Lys Asp Gly Thr Ser Phe Gly Glu Tyr Gly Gly Trp
        435                 440                 445
Tyr Lys Ala Cys Lys Val Asp Ser Pro Thr Val Thr Thr Thr Leu Lys
    450                 455                 460
Asn Leu Gly Ala Leu Tyr Arg Arg Gln Gly Lys Phe Glu Ala Ala Glu
465                 470                 475                 480
Thr Leu Glu Glu Ala Ala Met Arg Ser Arg Lys Gln Gly Leu Asp Asn
                485                 490                 495
Val His Lys Gln Arg Val Ala Glu Val Leu Asn Asp Pro Glu Asn Met
            500                 505                 510
Glu Lys Arg Arg Ser Arg Glu Ser Leu Asn Val Asp Val Val Lys Tyr
        515                 520                 525
Glu Ser Gly Pro Asp Gly Gly Glu Glu Val Ser Met Ser Val Glu Trp
    530                 535                 540
Asn Gly Gly Val Ser Gly Arg Ala Ser Phe Cys Gly Lys Arg Gln Gln
545                 550                 555                 560
Gln Gln Trp Pro Gly Arg Arg His Arg
                565

<210> 87
<211> 736
<212> PRT
<213> Homo Sapiens

<400> 87
Met Glu Ala Leu Ile Pro Val Ile Asn Lys Leu Gln Asp Val Phe Asn
1               5                   10                  15
Thr Val Gly Ala Asp Ile Ile Gln Leu Pro Gln Ile Val Val Val Gly
            20                  25                  30
Thr Gln Ser Ser Gly Lys Ser Ser Val Leu Glu Ser Leu Val Gly Arg
        35                  40                  45
Asp Leu Leu Pro Arg Gly Thr Gly Ile Val Thr Arg Arg Pro Leu Ile

    50                  55                  60
Leu Gln Leu Val His Val Thr Gln Glu Asp Lys Arg Lys Thr Thr Gly
65                  70                  75                  80
Glu Glu Asn Gly Val Glu Ala Glu Glu Trp Gly Lys Phe Leu His Thr
                85                  90                  95
Lys Asn Lys Leu Tyr Thr Asp Phe Asp Glu Ile Arg Gln Glu Ile Glu
            100                 105                 110
Asn Glu Thr Glu Arg Ile Ser Gly Asn Asn Lys Gly Val Ser Pro Glu
        115                 120                 125
Pro Ile His Leu Lys Ile Phe Ser Pro Asn Val Val Asn Leu Thr Leu
    130                 135                 140
Val Asp Leu Pro Gly Met Thr Lys Val Pro Val Gly Asp Gln Pro Lys
145                 150                 155                 160
Asp Ile Glu Leu Gln Ile Arg Glu Leu Ile Leu Arg Phe Ile Ser Asn
                165                 170                 175
Pro Asn Ser Ile Ile Leu Ala Val Thr Ala Ala Asn Thr Asp Met Ala
            180                 185                 190
Thr Ser Glu Ala Leu Lys Ile Ser Arg Glu Val Asp Pro Asp Gly Arg
        195                 200                 205
Arg Thr Leu Ala Val Ile Thr Lys Leu Asp Leu Met Asp Ala Gly Thr
    210                 215                 220
Asp Ala Met Asp Val Leu Met Gly Arg Val Ile Pro Val Lys Leu Gly
225                 230                 235                 240
Ile Ile Gly Val Val Asn Arg Ser Gln Leu Asp Ile Asn Asn Lys Lys
                245                 250                 255
Ser Val Thr Asp Ser Ile Arg Asp Glu Tyr Ala Phe Leu Gln Lys Lys
            260                 265                 270
Tyr Pro Ser Leu Ala Asn Arg Asn Gly Thr Lys Tyr Leu Ala Arg Thr
        275                 280                 285
Leu Asn Arg Leu Leu Met His His Ile Arg Asp Cys Leu Pro Glu Leu
    290                 295                 300
Lys Thr Arg Ile Asn Val Leu Ala Ala Gln Tyr Gln Ser Leu Leu Asn
305                 310                 315                 320
Ser Tyr Gly Glu Pro Val Asp Asp Lys Ser Ala Thr Leu Leu Gln Leu
                325                 330                 335
Ile Thr Lys Phe Ala Thr Glu Tyr Cys Asn Thr Ile Glu Gly Thr Ala
            340                 345                 350
Lys Tyr Ile Glu Thr Ser Glu Leu Cys Gly Gly Ala Arg Ile Cys Tyr
        355                 360                 365
Ile Phe His Glu Thr Phe Gly Arg Thr Leu Glu Ser Val Asp Pro Leu
    370                 375                 380
Gly Gly Leu Asn Thr Ile Asp Ile Leu Thr Ala Ile Arg Asn Ala Thr
385                 390                 395                 400
Gly Pro Arg Pro Ala Leu Phe Val Pro Glu Val Ser Phe Glu Leu Leu
                405                 410                 415
Val Lys Arg Gln Ile Lys Arg Leu Glu Glu Pro Ser Leu Arg Cys Val
            420                 425                 430
Glu Leu Val His Glu Glu Met Gln Arg Ile Ile Gln His Cys Ser Asn
        435                 440                 445
Tyr Ser Thr Gln Glu Leu Leu Arg Phe Pro Lys Leu His Asp Ala Ile
    450                 455                 460
Val Glu Val Val Thr Cys Leu Leu Arg Lys Arg Leu Pro Val Thr Asn
465                 470                 475                 480
Glu Met Val His Asn Leu Val Ala Ile Glu Leu Ala Tyr Ile Asn Thr
                485                 490                 495

Lys His Pro Asp Phe Ala Asp Ala Cys Gly Leu Met Asn Asn Asn Ile
            500                 505                 510
Glu Glu Gln Arg Arg Asn Arg Leu Ala Arg Glu Leu Pro Ser Ala Val
        515                 520                 525
Ser Arg Asp Lys Ser Ser Lys Val Pro Ser Ala Leu Ala Pro Ala Ser
    530                 535                 540
Gln Glu Pro Ser Pro Ala Ala Ser Ala Glu Ala Asp Gly Lys Leu Ile
545                 550                 555                 560
Gln Asp Ser Arg Arg Glu Thr Lys Asn Val Ala Ser Gly Gly Gly Gly
                565                 570                 575
Val Gly Asp Gly Val Gln Glu Pro Thr Thr Gly Asn Trp Arg Gly Met
            580                 585                 590
Leu Lys Thr Ser Lys Ala Glu Glu Leu Leu Ala Glu Glu Lys Ser Lys
        595                 600                 605
Pro Ile Pro Ile Met Pro Ala Ser Pro Gln Lys Gly His Ala Val Asn
    610                 615                 620
Leu Leu Asp Val Pro Val Pro Val Ala Arg Lys Leu Ser Ala Arg Glu
625                 630                 635                 640
Gln Arg Asp Cys Glu Val Ile Glu Arg Leu Ile Lys Ser Tyr Phe Leu
                645                 650                 655
Ile Val Arg Lys Asn Ile Gln Asp Ser Val Pro Lys Ala Val Met His
            660                 665                 670
Phe Leu Val Asn His Val Lys Asp Thr Leu Gln Ser Glu Leu Val Gly
        675                 680                 685
Gln Leu Tyr Lys Ser Ser Leu Leu Asp Asp Leu Leu Thr Glu Ser Glu
    690                 695                 700
Asp Met Ala Gln Arg Arg Lys Glu Ala Ala Asp Met Leu Lys Ala Leu
705                 710                 715                 720
Gln Gly Ala Ser Gln Ile Ile Ala Glu Ile Arg Glu Thr His Leu Trp
                725                 730                 735

<210> 88
<211> 37
<212> PRT
<213> Homo Sapiens

<400> 88
Met Gly Asp His Ala Trp Ser Phe Leu Lys Asp Phe Leu Ala Gly Gly
 1               5                  10                  15
Val Ala Ala Ala Val Ser Lys Thr Ala Val Ala Pro Ile Glu Arg Val
            20                  25                  30
Lys Leu Leu Leu Gln
        35

<210> 89
<211> 1381
<212> DNA
<213> Homo Sapiens

<400> 89
ccgcagccct agagccgccc aagggatggc gatggcgtac ttggcttgga gactggcgcg      60
gcgttcgtgt ccgagttctc tgcaggtcnc tantttcccg gtagttcanc tgcncatgaa     120
tanaacagca atgagagccn ctcncaaaga ctttgaaaat tcactgaatc nagtgaaact     180
ctngaaaaag gatccangaa acgaaatgaa nctnaaactc tncgcgctat atnancangc     240
cnctgaanga cttgtntcat gcccnaacca ngtgtntttg acttgatcna caaggggcca     300

atgggacaca tggaatgccc ttggcancct gcccnaagaa ctgccaggca naactatgtg 360
gatttggtgt ccantttgan tccntccttg gaatcctcna atcnngtgga ncctggaaca 420
nacaggaaat ccactgggtt tgaaactctg gtggtgacct ccgaagatgg catcacaaag 480
atcatgttca accggcccaa aaagaaaaat gccataaaca ctgagatgta tcatgaaatt 540
atgcgtgcac ttaaagctgc cagcaaggat gactcaatca tcactgtttt aacaggaaat 600
ggtgactatt acagtagtgg gaatgatctg actaacttca ctgatattcc ccctggtgga 660
gtagaggaga aagctaaaaa taatgccgtt ttactgaggg aatttgtggg ctgttttata 720
gattttccta agcctctgat tgcagtggtc aatggtccag ctgtgggcat ctccgtcacc 780
ctccttgggc tattcgatgc cgtgtatgca tctgacaggg caacatttca tacaccattt 840
agtcacctag gccaaagtcc ggaaggatgc tcctcttaca cttttccgaa gataatgagc 900
ccagccaagg caacagagat gcttattttt ggaaagaagt taacagcggg agaggcatgt 960
gctcaaggac ttgttactga agttttccct gatagcactt ttcagaaaga agtctggacc 1020
aggctgaagg catttgcaaa gcttccccca aatgccttga gaatttcaaa agaggtaatc 1080
aggaaaagag agagagaaaa actacacgct gttaatgctg aagaatgcaa tgtccttcag 1140
ggaagatggc tatcagatga atgcacaaat gctgtggtga acttcttatc cagaaaatca 1200
aaactgtgat gaccactaca gcagagtaaa gcatgtccaa ggaaggatgt gctgttacct 1260
ctgatttcca gtactggaac taaataagct tcattgtgcc ttttgtagtg ctagaatatc 1320
aattacaatg atgatatttc actacagctc tgatgaataa aaagttttgt aaaacaagaa 1380
a 1381

<210> 90
<211> 298
<212> PRT
<213> Homo Sapiens

<400> 90
Thr Cys Met Pro Pro Val Phe Asp Leu Ile Lys Gly Pro Met Gly His
1               5                   10                  15
Met Glu Cys Pro Trp Pro Ala Arg Thr Ala Arg Asn Tyr Val Asp Leu
            20                  25                  30
Val Ser Leu Pro Ser Leu Glu Ser Ser Asn Val Pro Gly Thr Arg Lys
        35                  40                  45
Ser Thr Gly Phe Glu Thr Leu Val Val Thr Ser Glu Asp Gly Ile Thr
    50                  55                  60
Lys Ile Met Phe Asn Arg Pro Lys Lys Lys Asn Ala Ile Asn Thr Glu
65                  70                  75                  80
Met Tyr His Glu Ile Met Arg Ala Leu Lys Ala Ala Ser Lys Asp Asp
                85                  90                  95
Ser Ile Ile Thr Val Leu Thr Gly Asn Gly Asp Tyr Tyr Ser Ser Gly
            100                 105                 110
Asn Asp Leu Thr Asn Phe Thr Asp Ile Pro Pro Gly Gly Val Glu Glu
        115                 120                 125
Lys Ala Lys Asn Asn Ala Val Leu Leu Arg Glu Phe Val Gly Cys Phe
    130                 135                 140
Ile Asp Phe Pro Lys Pro Leu Ile Ala Val Val Asn Gly Pro Ala Val
145                 150                 155                 160
Gly Ile Ser Val Thr Leu Leu Gly Leu Phe Asp Ala Val Tyr Ala Ser
                165                 170                 175
Asp Arg Ala Thr Phe His Thr Pro Phe Ser His Leu Gly Gln Ser Pro
            180                 185                 190
Glu Gly Cys Ser Ser Tyr Thr Phe Pro Lys Ile Met Ser Pro Ala Lys
        195                 200                 205
Ala Thr Glu Met Leu Ile Phe Gly Lys Lys Leu Thr Ala Gly Glu Ala
    210                 215                 220
Cys Ala Gln Leu Val Thr Glu Val Phe Pro Asp Ser Thr Phe Gln Lys

225                 230                     235                     240
Glu Val Trp Thr Lys Leu Lys Ala Phe Ala Lys Ala Ser Pro Lys Cys
                245                     250                     255
Leu Glu Asn Phe Lys Arg Gly Asn Gln Gly Lys Glu Arg Glu Lys Asn
            260                     265                     270
Tyr Thr Pro Leu Met Leu Lys Lys Cys Asn Val Pro Ser Arg Lys Gly
        275                     280                     285
Tyr Gln Asp Glu Cys Thr Lys Cys Leu Trp
    290                     295

<210> 91
<211> 1514
<212> DNA
<213> Homo Sapiens

<400> 91
gccgcgcgct gtgtctccgc tgcgtccgcc gaggcccccg agtgtcaggg acaaaagcct      60
ccgcctgctc ccgcagccgg ggctcatctg ccgccgccgc cgcgctgagg agagttcgcc     120
gccgtcgccg cccgtgagga tctgagagcc atgtcggcca gcagcctctt ggagcagaga     180
ccaaaaggtc aaggaaacaa agtacaaaat ggatctgtac atcaaaagga tggattaaac     240
gatgatgatt ttgaacctta cttgagtcca caggcaaggc ccaataatgc atatactgcc     300
atgtcagatt cctacttacc cagttactac agtccctcca ttggcttctc ctattctttg     360
ggtgaagctg cttggtctac ggggggtgac acagccatgc cctacttaac ttcttatgga     420
cagctgagca acggagagcc ccacttccta ccagatgcaa tgtttgggca accaggagcc     480
ctaggtagca ctccatttct tggtcagcat ggttttaatt tctttcccag tgggattgac     540
ttctcagcat ggggaaataa cagttctcag ggacagtcta ctcagagctc tggatatagt     600
agcaattatg cttatgcacc tagctcctta ggtggagcca tgattgatgg acagtcagct     660
tttgccaatg agaccctcaa taaggctcct ggcatgaata ctatagacca agggatggca     720
gcactgaagt tgggtagcac agaagttgca agcaatgttc caaaagttgt aggttctgct     780
gttggtagcg ggtccattac tagtaacatc gtggcttcca atagtttgcc tccagccacc     840
attgctcctc caaaaccagc atcttgggct gatattgcta gcaagcctgc aaaacagcaa     900
cctaaactga agaccaagaa tggcattgca gggtcaagtc ttccgccacc cccgataaag     960
cataacatgg atattggaac ttgggataac aagggtcccg ttgcaaaagc cccctcacag    1020
gctttggttc agaatatagg tcagccaacc caggggtctc ctcagcctgt aggtcagcag    1080
gctaacaata gcccaccagt ggctcaggca tcagtagggc aacagacaca gccattgcct    1140
ccacctccac cacagcctgc ccagctttca gtccagcaac aggcagctca gccaacccgc    1200
tgggtagcac ctcggaaccg tggcagtggg ttcggtcata atggggtgga tggtaatgga    1260
gtaggacagt ctcaggctgg ttctggatct actccttcag aaccccaccc agtgttggag    1320
aagcttcggt ccattaataa ctataacccc aaagattttg actgggaaat ctgaaacatg    1380
ggcgggtttt catcattaaa gaactactct gangacgata ttcaccgttc catttaagtt    1440
ataatatttg gtggancaca anagcaatgg taacaagaga atgggatgcc ngcttatcgt    1500
ttccatgaac gggg                                                      1514

<210> 92
<211> 407
<212> PRT
<213> Homo Sapiens

<400> 92
Met Ser Ala Ser Ser Leu Leu Glu Gln Arg Pro Lys Gly Gln Gly Asn
 1               5                  10                  15
Lys Val Gln Asn Gly Ser Val His Gln Lys Asp Gly Leu Asn Asp Asp
            20                  25                  30
Asp Phe Glu Pro Tyr Leu Ser Pro Gln Ala Arg Pro Asn Asn Ala Tyr
        35                  40                  45

Thr Ala Met Ser Asp Ser Tyr Leu Pro Ser Tyr Tyr Ser Pro Ser Ile
    50                  55                  60
Gly Phe Ser Tyr Ser Leu Gly Glu Ala Ala Trp Ser Thr Gly Gly Asp
65                  70                  75                  80
Thr Ala Met Pro Tyr Leu Thr Ser Tyr Gly Gln Leu Ser Asn Gly Glu
                85                  90                  95
Pro His Phe Leu Pro Asp Ala Met Phe Gly Gln Pro Gly Ala Leu Gly
            100                 105                 110
Ser Thr Pro Phe Leu Gly Gln His Gly Phe Asn Phe Phe Pro Ser Gly
        115                 120                 125
Ile Asp Phe Ser Ala Trp Gly Asn Asn Ser Ser Gln Gly Gln Ser Thr
    130                 135                 140
Gln Ser Ser Gly Tyr Ser Ser Asn Tyr Ala Tyr Ala Pro Ser Ser Leu
145                 150                 155                 160
Gly Gly Ala Met Ile Asp Gly Gln Ser Ala Phe Ala Asn Glu Thr Leu
                165                 170                 175
Asn Lys Ala Pro Gly Met Asn Thr Ile Asp Gln Gly Met Ala Ala Leu
            180                 185                 190
Lys Leu Gly Ser Thr Glu Val Ala Ser Asn Val Pro Lys Val Val Gly
        195                 200                 205
Ser Ala Val Gly Ser Gly Ser Ile Thr Ser Asn Ile Val Ala Ser Asn
    210                 215                 220
Ser Leu Pro Pro Ala Thr Ile Ala Pro Pro Lys Pro Ala Ser Trp Ala
225                 230                 235                 240
Asp Ile Ala Ser Lys Pro Ala Lys Gln Gln Pro Lys Leu Lys Thr Lys
                245                 250                 255
Asn Gly Ile Ala Gly Ser Ser Leu Pro Pro Pro Pro Ile Lys His Asn
            260                 265                 270
Met Asp Ile Gly Thr Trp Asp Asn Lys Gly Pro Val Ala Lys Ala Pro
        275                 280                 285
Ser Gln Ala Leu Val Gln Asn Ile Gly Gln Pro Thr Gln Gly Ser Pro
    290                 295                 300
Gln Pro Val Gly Gln Gln Ala Asn Asn Ser Pro Pro Val Ala Gln Ala
305                 310                 315                 320
Ser Val Gly Gln Gln Thr Gln Pro Leu Pro Pro Pro Pro Pro Gln Pro
                325                 330                 335
Ala Gln Leu Ser Val Gln Gln Gln Ala Ala Gln Pro Thr Arg Trp Val
            340                 345                 350
Ala Pro Arg Asn Arg Gly Ser Gly Phe Gly His Asn Gly Val Asp Gly
        355                 360                 365
Asn Gly Val Gly Gln Ser Gln Ala Gly Ser Gly Ser Thr Pro Ser Glu
    370                 375                 380
Pro His Pro Val Leu Glu Lys Leu Arg Ser Ile Asn Asn Tyr Asn Pro
385                 390                 395                 400
Lys Asp Phe Asp Trp Glu Ile
                405

<210> 93
<211> 2236
<212> DNA
<213> Homo Sapiens

<400> 93
cctggcccgg tcgcggtcgc ggctctttcc agctcctggc agccgggcac ccgaaggaac      60
gggtcgtgca acgacgcagc tggacctggc ccagccatgg accgaaaagt ggcccgagaa     120

ttccggcata aggtggattt tctgattgaa aatgatgcag agaaggacta tctctatgat 180
gtgctgcgaa tgtaccacca gaccatggac gtggccgtgc tcgtgggaga cctgaagctg 240
gtcatcaatg aacccagccg tctgcctctg tttgatgcca ttcggccgct gatcccactg 300
aagcaccagg tggaatatga tcagctgacc ccccggcgct ccaggaagct gaaggaggtg 360
cgtctggacc gtctgcaccc cgaaggcctc ggcctgagtg tgcgtggtgg cctggagttt 420
ggctgtgggc tcttcatctc ccacctcatc aaaggcggtc aggcagacag cgtcgggctc 480
caggtagggg acgagatcgt ccggatcaat ggatattcca tctcctcctg tacccatgag 540
gaggtcatca acctcattcg aaccaagaaa actgtgtcca tcaaagtgag acacatcggc 600
ctgatccccg tgaaaagctc tcctgatgag cccctcactt ggcagtatgt ggatcagttt 660
gtgtcggaat ctgggggcgt gcgaggcagc ctgggctccc ctggaaatcg ggaaaacaag 720
gagaagaagg tcttcatcag cctggtaggc tcccgaggcc ttggctgcag catttccagc 780
ggccccatcc agaagcctgg catctttatc agccatgtga aacctggctc cctgtctgct 840
gaggtgggat tggagatagg ggaccagatt gtcgaagtca atggcgtcga cttctctaac 900
ctggatcaca aggaggctgt aaatgtgctg aaaaatagcc gcagcctgac catctccatt 960
gtagctgcag ctggccggga gctgttcatg acagaccggg agcggctggc agaggcgcgg 1020
cagcgtgagc tgcagcggca ggagcttctc atgcagaagc ggctggcgat ggagtccaac 1080
aagatcctcc aggagcagca ggagatggag cggcaaagga gaaaagaaat tgcccagaag 1140
gcagcagagg aaaatgagag ataccggaag gagatggaac agattgtaga ggaggaagag 1200
aagtttaaga agcaatggga agaagactgg ggctcaaagg aacagctact cttgcctaaa 1260
accatcactg ctgaggtaca cccagtaccc cttcgcaagc caaagtatga tcagggagtg 1320
gaacctgagc tcgagcccgc agatgacctg gatggaggca cggaggagca gggagagcag 1380
gatttccgga aatatgagga aggctttgac ccctactcta tgttcacccc agagcagatc 1440
atggggaagg atgtccggct cctacgcatc aagaaggagg gatccttaga cctggccctg 1500
gaaggcggtg tggactcccc cattgggaag gtggtcgttt ctgctgtgta tgagcgggga 1560
gctgctgagc ggcatggtgg cattgtgaaa ggggacgaga tcatggcaat caacggcaag 1620
attgtgacag actacaccct ggctgaggct gacgctgccc tgcagaaggc ctggaatcag 1680
ggcggggact ggatcgacct tgtggttgcc gtctgccccc caaaggagta tgacgatgag 1740
ctgaccttct tgctgaagtc caaaagggga aaccaaattc acgcgttagg aaacagtgag 1800
ctccggcccc acctcgtgaa cacaaagcct cggaccagcc ttgagagagg ccacatgaca 1860
cacaccagat ggcatccttg ggacctgaat ctatcaccca ggaatctcaa actccctttg 1920
gccctgaacc agggccagat aaggaacagc tcgggccact tttttgaagg ccaatgtgga 1980
ggaaagggag cagccagccg tttgggagaa gatctcaagg atccagactc tcattccttt 2040
cctctggccc agtgaatttg gtctctccca gctttggggg actccttcct tgaaccctaa 2100
taagacccca ctggagtctc tctctctcca tccctctcct ctgccctctg ctctaattgc 2160
tgccaggatt gtcactccaa accttactct gagctcatta ataaaataaa cagatttatt 2220
ttccagctta aaaaaa 2236

<210> 94
<211> 652
<212> PRT
<213> Homo Sapiens

<400> 94
Met Asp Arg Lys Val Ala Arg Glu Phe Arg His Lys Val Asp Phe Leu
1               5                   10                  15
Ile Glu Asn Asp Ala Glu Lys Asp Tyr Leu Tyr Asp Val Leu Arg Met
            20                  25                  30
Tyr His Gln Thr Met Asp Val Ala Val Leu Val Gly Asp Leu Lys Leu
        35                  40                  45
Val Ile Asn Glu Pro Ser Arg Leu Pro Leu Phe Asp Ala Ile Arg Pro
    50                  55                  60
Leu Ile Pro Leu Lys His Gln Val Glu Tyr Asp Gln Leu Thr Pro Arg
65                  70                  75                  80
Arg Ser Arg Lys Leu Lys Glu Val Arg Leu Asp Arg Leu His Pro Glu
                85                  90                  95

Gly Leu Gly Leu Ser Val Arg Gly Gly Leu Glu Phe Gly Cys Gly Leu
            100                 105                 110
Phe Ile Ser His Leu Ile Lys Gly Gly Gln Ala Asp Ser Val Gly Leu
        115                 120                 125
Gln Val Gly Asp Glu Ile Val Arg Ile Asn Gly Tyr Ser Ile Ser Ser
    130                 135                 140
Cys Thr His Glu Glu Val Ile Asn Leu Ile Arg Thr Lys Lys Thr Val
145                 150                 155                 160
Ser Ile Lys Val Arg His Ile Gly Leu Ile Pro Val Lys Ser Ser Pro
                165                 170                 175
Asp Glu Pro Leu Thr Trp Gln Tyr Val Asp Gln Phe Val Ser Glu Ser
            180                 185                 190
Gly Gly Val Arg Gly Ser Leu Gly Ser Pro Gly Asn Arg Glu Asn Lys
        195                 200                 205
Glu Lys Lys Val Phe Ile Ser Leu Val Gly Ser Arg Gly Leu Gly Cys
    210                 215                 220
Ser Ile Ser Ser Gly Pro Ile Gln Lys Pro Gly Ile Phe Ile Ser His
225                 230                 235                 240
Val Lys Pro Gly Ser Leu Ser Ala Glu Val Gly Leu Glu Ile Gly Asp
                245                 250                 255
Gln Ile Val Glu Val Asn Gly Val Asp Phe Ser Asn Leu Asp His Lys
            260                 265                 270
Glu Ala Val Asn Val Leu Lys Asn Ser Arg Ser Leu Thr Ile Ser Ile
        275                 280                 285
Val Ala Ala Ala Gly Arg Glu Leu Phe Met Thr Asp Arg Glu Arg Leu
    290                 295                 300
Ala Glu Ala Arg Gln Arg Glu Leu Gln Arg Gln Glu Leu Leu Met Gln
305                 310                 315                 320
Lys Arg Leu Ala Met Glu Ser Asn Lys Ile Leu Gln Glu Gln Gln Glu
                325                 330                 335
Met Glu Arg Gln Arg Arg Lys Glu Ile Ala Gln Lys Ala Ala Glu Glu
            340                 345                 350
Asn Glu Arg Tyr Arg Lys Glu Met Glu Gln Ile Val Glu Glu Glu Glu
        355                 360                 365
Lys Phe Lys Lys Gln Trp Glu Glu Asp Trp Gly Ser Lys Glu Gln Leu
    370                 375                 380
Leu Leu Pro Lys Thr Ile Thr Ala Glu Val His Pro Val Pro Leu Arg
385                 390                 395                 400
Lys Pro Lys Tyr Asp Gln Gly Val Glu Pro Glu Leu Glu Pro Ala Asp
                405                 410                 415
Asp Leu Asp Gly Gly Thr Glu Glu Gln Gly Glu Gln Asp Phe Arg Lys
            420                 425                 430
Tyr Glu Glu Gly Phe Asp Pro Tyr Ser Met Phe Thr Pro Glu Gln Ile
        435                 440                 445
Met Gly Lys Asp Val Arg Leu Leu Arg Ile Lys Lys Glu Gly Ser Leu
    450                 455                 460
Asp Leu Ala Leu Glu Gly Gly Val Asp Ser Pro Ile Gly Lys Val Val
465                 470                 475                 480
Val Ser Ala Val Tyr Glu Arg Gly Ala Ala Glu Arg His Gly Gly Ile
                485                 490                 495
Val Lys Gly Asp Glu Ile Met Ala Ile Asn Gly Lys Ile Val Thr Asp
            500                 505                 510
Tyr Thr Leu Ala Glu Ala Asp Ala Ala Leu Gln Lys Ala Trp Asn Gln
        515                 520                 525
Gly Gly Asp Trp Ile Asp Leu Val Val Ala Val Cys Pro Pro Lys Glu

530                 535                 540
Tyr Asp Asp Glu Leu Thr Phe Leu Leu Lys Ser Lys Arg Gly Asn Gln
545                 550                 555                 560
Ile His Ala Leu Gly Asn Ser Glu Leu Arg Pro His Leu Val Asn Thr
                565                 570                 575
Lys Pro Arg Thr Ser Leu Glu Arg Gly His Met Thr His Thr Arg Trp
            580                 585                 590
His Pro Trp Asp Leu Asn Leu Ser Pro Arg Asn Leu Lys Leu Pro Leu
        595                 600                 605
Ala Leu Asn Gln Gly Gln Ile Arg Asn Ser Ser Gly His Phe Phe Glu
    610                 615                 620
Gly Gln Cys Gly Gly Lys Gly Ala Ala Ser Arg Leu Gly Glu Asp Leu
625                 630                 635                 640
Lys Asp Pro Asp Ser His Ser Phe Pro Leu Ala Gln
                645                 650

<210> 95
<211> 831
<212> DNA
<213> Homo Sapiens

<400> 95
cataactggg actcgcgcgc ctgcaggtcg acactagtgg gatccaaaga attcggcacg      60
agaaaccaca atgcccagat ctcaagtaga tgaagagttt ttgaagcaaa gtttaaagga     120
aaaacnattg cagaaaacat ttagattnta tgaaatatat aatnanancc aaaanccatt     180
tgaanttaat nganccttac ctgtcntcac taaatcaggg ttntctgcgc caccnaaggg     240
cngcccancg cctgctgtgt tggcttanta ggcctnagca tangggcagn tgcaatcctt     300
tcctcctnng gcggcanatg ggcttctgga anaacccttn ccttatcccc ancgcaaggc     360
ggcccctccc ctgccctnaa aggaaacctc ntggacncag ggaatatang gccaccttga     420
agggtggact ggctatcntg gaagatcaga taccaccaag caatttggag acagttcctg     480
ttgagaataa ccacggtttc catgaaaaga cagcagcgct gaagcttgag gccgagggcg     540
aggccatgga agatgcagcc gcgccaggga acgaccgagg cggcacacag gagccagccc     600
cagtgcctgc tgagccgttt gacaacacta cctacaagaa cctgcagcat catgactaca     660
gcacgtacac cttcttagac ctcaacctcg aactctcaaa attcaggatg cctcagccct     720
cctcaggccg ggagtcacct cgacactgag ggccctcggt gtgaagatga accttccacc     780
gtcttcactg catcctggag tgcaaaaata aaatccactc aagagtcaaa a              831

<210> 96
<211> 184
<212> PRT
<213> Homo Sapiens

<400> 96
Arg Lys Asn Cys Arg Lys His Leu Asp Met Lys Tyr Ile Lys His Leu
1               5                   10                  15
Leu Pro Tyr Leu Ser Ser Leu Asn Gln Gly Leu Arg His Arg Ala Ala
            20                  25                  30
Arg Leu Leu Cys Trp Leu Arg Pro His Gly Cys Asn Pro Phe Leu Leu
        35                  40                  45
Arg Met Gly Phe Trp Asn Pro Leu Ile Pro Ala Arg Arg Pro Leu Pro
    50                  55                  60
Cys Pro Arg Lys Pro Gly Arg Glu Tyr Ala Thr Leu Lys Gly Gly Leu
65                  70                  75                  80
Ala Ile Glu Asp Gln Ile Pro Pro Ser Asn Leu Glu Thr Val Pro Val
                85                  90                  95

Glu Asn Asn His Gly Phe His Glu Lys Thr Ala Ala Leu Lys Leu Glu
            100                 105                 110
Ala Glu Gly Glu Ala Met Glu Asp Ala Ala Ala Pro Gly Asn Asp Arg
        115                 120                 125
Gly Gly Thr Gln Glu Pro Ala Pro Val Pro Ala Glu Pro Phe Asp Asn
    130                 135                 140
Thr Thr Tyr Lys Asn Leu Gln His His Asp Tyr Ser Thr Tyr Thr Phe
145                 150                 155                 160
Leu Asp Leu Asn Leu Glu Leu Ser Lys Phe Arg Met Pro Gln Pro Ser
                165                 170                 175
Ser Gly Arg Glu Ser Pro Arg His
            180

<210> 97
<211> 1008
<212> DNA
<213> Homo Sapiens

<400> 97
gcaaggtctc caagtcccag ctcaaggtcc tttcccataa cctgtgcacg gtgctgaagg      60
ttcctcatga cccagttgcc cttgaagagc acttcaggga tgatgatgag ggtccagtgt     120
ccaaccaggg ctacatgcct tatttaaaca ggttcatttt ggaaaaggtc caagacaact     180
ttgacaagat tgaattcaat aggatgtgtt ggaccctctg tgtcaaaaaa aacctcacaa     240
agaatcccct gctcattaca gaagaanatg catttaaaat atgggttatt ttcaactttt     300
tatctgagga caagtatcca ttaattattg tgtcagaana gattgaatac ctgcttaaga     360
agcttacaga agctatggga ggaggttggc agcaagaaca atttgaacat tataaaatca     420
actttgatga cagtaaaaat ggcctttctg catgggaact tattgagctt attggaaatg     480
gacagtttag caaaggcatg gaccggcaga ctgtgtctat ggcaattaat gaagtcttta     540
atgaacttat attagatgtg ttaaagcagg gttacatgat gaaaaagggc cacagacgga     600
aaaactggac tgaacgatgg tttgtactaa aacccaacat aatttcttac tatgtgagtg     660
aggatctgaa ggataagaaa ggagacattc tcttggatga aaattgctgt gtagagtcct     720
tgcctgacaa agatggaaag aaatgccttt ttctcgtaaa atgttttgat aagacttttg     780
aaatcagtgc ttcagataag aanaanaaac aggagtggat tcaagccatt cattctacta     840
ttcatctgtt gaagctgngc agccctccac canacaaaga agccnnccag cttctnaaan     900
aactccggna gaatcatctg gctgaacaag angaactgga gcgacaaatg aangaactcc     960
aagcccgcca atgaaagcaa ncagcaagag ctggaaggcc ttncggaa                 1008

<210> 98
<211> 312
<212> PRT
<213> Homo Sapiens

<400> 98
Lys Val Ser Lys Ser Gln Leu Lys Val Leu Ser His Asn Leu Cys Thr
1               5                   10                  15
Val Leu Lys Val Pro His Asp Pro Val Ala Leu Glu Glu His Phe Arg
            20                  25                  30
Asp Asp Asp Glu Gly Pro Val Ser Asn Gln Gly Tyr Met Pro Tyr Leu
        35                  40                  45
Asn Arg Phe Ile Leu Glu Lys Val Gln Asp Asn Phe Asp Lys Ile Glu
    50                  55                  60
Phe Asn Arg Met Cys Trp Thr Leu Cys Val Lys Lys Asn Leu Thr Lys
65                  70                  75                  80
Asn Pro Leu Leu Ile Thr Glu Glu Ala Phe Lys Ile Trp Val Ile Phe
                85                  90                  95

Asn Phe Leu Ser Glu Asp Lys Tyr Pro Leu Ile Ile Val Ser Glu Ile
            100                 105                 110
Glu Tyr Leu Leu Lys Lys Leu Thr Glu Ala Met Gly Gly Gly Trp Gln
        115                 120                 125
Gln Glu Gln Phe Glu His Tyr Lys Ile Asn Phe Asp Asp Ser Lys Asn
    130                 135                 140
Gly Leu Ser Ala Trp Glu Leu Ile Glu Leu Ile Gly Asn Gly Gln Phe
145                 150                 155                 160
Ser Lys Gly Met Asp Arg Gln Thr Val Ser Met Ala Ile Asn Glu Val
                165                 170                 175
Phe Asn Glu Leu Ile Leu Asp Val Leu Lys Gln Gly Tyr Met Met Lys
            180                 185                 190
Lys Gly His Arg Arg Lys Asn Trp Thr Glu Arg Trp Phe Val Leu Lys
        195                 200                 205
Pro Asn Ile Ile Ser Tyr Tyr Val Ser Glu Asp Leu Lys Asp Lys Lys
    210                 215                 220
Gly Asp Ile Leu Leu Asp Glu Asn Cys Cys Val Glu Ser Leu Pro Asp
225                 230                 235                 240
Lys Asp Gly Lys Lys Cys Leu Phe Leu Val Lys Cys Phe Asp Lys Thr
                245                 250                 255
Phe Glu Ile Ser Ala Ser Asp Lys Lys Gln Glu Trp Ile Gln Ala Ile
            260                 265                 270
His Ser Thr Ile His Leu Leu Lys Leu Ser Pro Pro Pro Lys Glu Ala
        275                 280                 285
Gln Leu Leu Lys Leu Arg Asn His Leu Ala Glu Gln Glu Leu Glu Arg
    290                 295                 300
Gln Met Glu Leu Gln Ala Arg Gln
305                 310

<210> 99
<211> 1009
<212> DNA
<213> Homo Sapiens

<400> 99
ggctaatgta acatactcta ccacttggtc tgaagcccag cagtatctga tggataatcc 60
aacttttgca gaagatgagg agttacaaaa tatggacaaa gaagatgcat taatttgctt 120
tgaagaacac attcgggctt tagaaaagga ggaagaagaa gaaaaacaga agagtttgct 180
gagagaaagg agacgacagc gaaaaaatag ggaatctttc cagatatttt tagatgaatt 240
acatgaacat ggacaactgc attctatgtc atcttggatg gaattgtatc caactattag 300
ttctgatatt agattcacta atatgcttgg tcagcctgga tcaactgcac ttgatctttt 360
caagttttat gttgaggatc ttaaagcacg ttatcatgac gagaagaaga taataaaaga 420
cattctaaag gataaaggat ttgtagttga agtaaacact acttttgaag attttgtggc 480
gataatcagt tcaactaaaa gatcaactac attagatgct ggaaatatca aattggcttt 540
caatagttta ctagaaaagg cagaagcccg tgaacgtgaa agagaaaaag aagaggctcg 600
gaagatgaaa cgaaaagaat ctgcatttaa gagtatgtta aaacaagctg ctcctccgat 660
agaattggat gctgtctggg aagatatccg tgagagattt gtaaaagagc cagcatttga 720
ggacataact ctagaatctg aaagaaaacg aatatttaaa gattttatgc atgtgcttga 780
gcatgaatgt cagcatcatc attcaaagaa caagaaacat tctaagaaat ctaaaaaaca 840
tcataggaaa cgttcccgct ctcgatcggg gtcagattca ngatgatgat gatagccatt 900
caaagaaaaa aagacagcga tgagaagtct cggtctgntt canaacattc ttccantngc 960
agagtctgag agaagtntaa aaagtcaaaa nagcatagan aggaaagtt 1009

<210> 100
<211> 292

<212> PRT
<213> Homo Sapiens

<400> 100
Ala Asn Val Thr Tyr Ser Thr Thr Trp Ser Glu Ala Gln Gln Tyr Leu
1               5                   10                  15
Met Asp Asn Pro Thr Phe Ala Glu Asp Glu Glu Leu Gln Asn Met Asp
            20                  25                  30
Lys Glu Asp Ala Leu Ile Cys Phe Glu Glu His Ile Arg Ala Leu Glu
        35                  40                  45
Lys Glu Glu Glu Glu Glu Lys Gln Lys Ser Leu Leu Arg Glu Arg Arg
    50                  55                  60
Arg Gln Arg Lys Asn Arg Glu Ser Phe Gln Ile Phe Leu Asp Glu Leu
65                  70                  75                  80
His Glu His Gly Gln Leu His Ser Met Ser Ser Trp Met Glu Leu Tyr
                85                  90                  95
Pro Thr Ile Ser Ser Asp Ile Arg Phe Thr Asn Met Leu Gly Gln Pro
            100                 105                 110
Gly Ser Thr Ala Leu Asp Leu Phe Lys Phe Tyr Val Glu Asp Leu Lys
        115                 120                 125
Ala Arg Tyr His Asp Glu Lys Lys Ile Ile Lys Asp Ile Leu Lys Asp
    130                 135                 140
Lys Gly Phe Val Val Glu Val Asn Thr Thr Phe Glu Asp Phe Val Ala
145                 150                 155                 160
Ile Ile Ser Ser Thr Lys Arg Ser Thr Thr Leu Asp Ala Gly Asn Ile
                165                 170                 175
Lys Leu Ala Phe Asn Ser Leu Leu Glu Lys Ala Glu Ala Arg Glu Arg
            180                 185                 190
Glu Arg Glu Lys Glu Glu Ala Arg Lys Met Lys Arg Lys Glu Ser Ala
        195                 200                 205
Phe Lys Ser Met Leu Lys Gln Ala Ala Pro Pro Ile Glu Leu Asp Ala
    210                 215                 220
Val Trp Glu Asp Ile Arg Glu Arg Phe Val Lys Glu Pro Ala Phe Glu
225                 230                 235                 240
Asp Ile Thr Leu Glu Ser Glu Arg Lys Arg Ile Phe Lys Asp Phe Met
                245                 250                 255
His Val Leu Glu His Glu Cys Gln His His His Ser Lys Asn Lys Lys
            260                 265                 270
His Ser Lys Lys Ser Lys Lys His His Arg Lys Arg Ser Arg Ser Arg
        275                 280                 285
Ser Gly Ser Asp
    290

<210> 101
<211> 983
<212> DNA
<213> Homo Sapiens

<400> 101
aggtgacaat agatatagaa gtacgttgat gtgcgaagat gtattttgtt ttagccagcg      60
aggaaaaaag aatcagtttg attatacatt taccaaacat taagaattta atatggtaac     120
ttttatttca gtattaaaat agcaatttta tttattactt ttttatatat agaatttgac     180
accaaatttt ggaacttaaa aagaagattc ttaaaactta caatccagat tacgatgagg     240
acctggtgca ggaagcttca tctgaagatg tcctgggcgt tcatatggtg gacaaagaca     300
cagagagaga cattgagatg aaacggcaac tacggcgact acgggagctc cacctataca     360

gcacatggaa gaagtaccaa gaggcgatga agacatcctt gggagttcca caacgtgagc 420
gtgacgaagg ctccttgggc aagccattgt gtccacccga gatactctcg gagacgttgc 480
caggctctgt gaagaaaagg gtatgctttc catcagaaga tcatctagag gagtttatag 540
cagaacatct ccctgaagca tccaatcaga gtctcctcac tgttgcccat gcagacgcag 600
gcacccaaac caacggtgac ctggaagacc tggaggagca tgggccaggg cagacagtct 660
ctgaggaagc cacagaagtt cacatgatgg agggggaccc agacacactg gccgaacttc 720
tgatcaggga tgtacttcag gagctgtcca gttacaacgg cgaggaggag gacccanagg 780
aggtgaagac atccttggga gttccacaac gtggtgacct ggaagacctg gaggagcatg 840
tgncagggca gnnnttctct gaggaagcca caggggttca catgatgcag gtggacccag 900
ccacgctggc aaagagtgac ctggaagacc tggaggagca tgtgccagag cagacagtct 960
ctgaggaagc cacaggggtt cac 983

<210> 102
<211> 230
<212> PRT
<213> Homo Sapiens

<400> 102
Met Val Asp Lys Asp Thr Glu Arg Asp Ile Glu Met Lys Arg Gln Leu
1               5                   10                  15
Arg Arg Leu Arg Glu Leu His Leu Tyr Ser Thr Trp Lys Lys Tyr Gln
            20                  25                  30
Glu Ala Met Lys Thr Ser Leu Gly Val Pro Gln Arg Glu Arg Asp Glu
        35                  40                  45
Gly Ser Leu Gly Lys Pro Leu Cys Pro Pro Glu Ile Leu Ser Glu Thr
    50                  55                  60
Leu Pro Gly Ser Val Lys Lys Arg Val Cys Phe Pro Ser Glu Asp His
65                  70                  75                  80
Leu Glu Glu Phe Ile Ala Glu His Leu Pro Glu Ala Ser Asn Gln Ser
                85                  90                  95
Leu Leu Thr Val Ala His Ala Asp Ala Gly Thr Gln Thr Asn Gly Asp
            100                 105                 110
Leu Glu Asp Leu Glu Glu His Gly Pro Gly Gln Thr Val Ser Glu Glu
        115                 120                 125
Ala Thr Glu Val His Met Met Glu Gly Asp Pro Asp Thr Leu Ala Glu
    130                 135                 140
Leu Leu Ile Arg Asp Val Leu Gln Glu Leu Ser Ser Tyr Asn Gly Glu
145                 150                 155                 160
Glu Glu Asp Pro Glu Val Lys Thr Ser Leu Gly Val Pro Gln Arg Gly
                165                 170                 175
Asp Leu Glu Asp Leu Glu Glu His Val Gly Gln Phe Ser Glu Glu Ala
            180                 185                 190
Thr Gly Val His Met Met Gln Val Asp Pro Ala Thr Leu Ala Lys Ser
        195                 200                 205
Asp Leu Glu Asp Leu Glu Glu His Val Pro Glu Gln Thr Val Ser Glu
    210                 215                 220
Glu Ala Thr Gly Val His
225                 230

<210> 103
<211> 843
<212> DNA
<213> Homo Sapiens

<400> 103

aatncccgct gcaggtcgac actagtggat ccaaagaatt cggcacgagg caagttctgg 60
gagctggaca cggaccacga cctgctcatc gacgcggacg acctggcgcg gcacaatgac 120
cacgcccttt ctaccaagat gatagacagg atcttctcag gagcagtcac acgaggcaga 180
aaagtgcaga aggaagggaa gatcagctat gccgactttg tctggttttt gatctctgag 240
gaagacaaaa aaacaccgac cagcatcgag tactggttcc gctgcatgga cctggacggg 300
gacggcgccc tgtccatgtt cgagctcgag tacttctacg aggagcagtg ccgaaggctg 360
gacagcatgg ccatcgaggc cctgcccttc caggactgcc tctgccagat gctggacctg 420
gtcaagccga ggactgaagg gaagatcacg ctgcaggacc tgaagcgctg caagctggcc 480
aacgtcttct tcgacacctt cttcaacatc gagaagtncc tcgaccacga gcagaaagag 540
cagatctccc tgctcaggga cggtgacagc ggcgggcccg agctctcgga ctgggagaag 600
tnccggccga agagtncgac atcctggtgg ccgangaaac cgtggggana nccctgggga 660
agacgggttc naaggcgaac tcacccccnt ggancanaaa ctgantgcgc tgcgctcccc 720
gctgggccan aggccttctt ccaagcgcct cccgctgggg cgccgtggaa ctgttncaaa 780
ttcccctgcg gggacaagaa cttgaaaccg ctgtganncc ccccncnana acccnccccg 840
gnt 843

<210> 104
<211> 197
<212> PRT
<213> Homo Sapiens

<400> 104
Arg Cys Arg Ser Thr Leu Val Asp Pro Lys Asn Ser Ala Arg Gly Lys
1               5                   10                  15
Phe Trp Glu Leu Asp Thr Asp His Asp Leu Leu Ile Asp Ala Asp Asp
            20                  25                  30
Leu Ala Arg His Asn Asp His Ala Leu Ser Thr Lys Met Ile Asp Arg
        35                  40                  45
Ile Phe Ser Gly Ala Val Thr Arg Gly Arg Lys Val Gln Lys Glu Gly
    50                  55                  60
Lys Ile Ser Tyr Ala Asp Phe Val Trp Phe Leu Ile Ser Glu Glu Asp
65                  70                  75                  80
Lys Lys Thr Pro Thr Ser Ile Glu Tyr Trp Phe Arg Cys Met Asp Leu
                85                  90                  95
Asp Gly Asp Gly Ala Leu Ser Met Phe Glu Leu Glu Tyr Phe Tyr Glu
            100                 105                 110
Glu Gln Cys Arg Arg Leu Asp Ser Met Ala Ile Glu Ala Leu Pro Phe
        115                 120                 125
Gln Asp Cys Leu Cys Gln Met Leu Asp Leu Val Lys Pro Arg Thr Glu
    130                 135                 140
Gly Lys Ile Thr Leu Gln Asp Leu Lys Arg Cys Lys Leu Ala Asn Val
145                 150                 155                 160
Phe Phe Asp Thr Phe Phe Asn Ile Glu Lys Leu Asp His Glu Gln Lys
                165                 170                 175
Glu Gln Ile Ser Leu Leu Arg Asp Gly Asp Ser Gly Gly Pro Glu Leu
            180                 185                 190
Ser Asp Trp Glu Lys
        195

<210> 105
<211> 2264
<212> DNA
<213> Homo Sapiens

<400> 105
   ctagcacaag tacacaggcc ccagccgctt cccctactgg tgtagttcct ggtaccaaat 60
   atgcagtacc tgacacgtcc acttaccagt atgatgaatc ttcaggatat tactatgatc 120
   cgacaacagg gctctattat gaccccaact cgcaatacta ctataattcc ttgacccagc 180
   agtaccttta ctgggatggg gaaaaagaga cctacgtgcc agctgcagag tctagctccc 240
   accagcagtc gggcctgcct cctgcaaaag aggggaaaga gaagaaggag aaacccaaga 300
   gcaaaacagc ccagcagatt gccaaagaca tggaacgctg ggctaagagt ttgaataagc 360
   agaaagaaaa ctttaaaaat agctttcagc ctgtcaattc cttgagggaa gaagaaagga 420
   gagaatctgc tgcagcagac gctggctttg ctctctttga gaagaaggga gccttagctg 480
   aaaggcagca gctcatccca gaattggtgc gaaatggaga tgaggagaat cccctcaaaa 540
   ggggtctggt tgctgcttac agtggtgaca gtgacaatga ggaggagctg gtggagagac 600
   ttgagagtga ggaagagaag ctagctgact ggaagaagat ggcctgtctg ctctgccggc 660
   gccagttccc gaacaaagat gccctagtca ggcaccagca actctcagac cttcacaagc 720
   aaaacatgga catctaccga cgatccaggc tgagcgagca ggagctggaa gccttggagc 780
   taagggagag agagatgaaa taccgagacc gagctgcaga aagacgggag aagtacggca 840
   ttccagaacc tccagagccc aagcgcaaga agcagtttga tgccggcact gtgaattacg 900
   agcaacccac caaagatggc attgaccaca gtaacattgg caacaagatg ctgcaggcca 960
   tgggctggcg ggaaggctct ggcttgggac gaaagtgtca aggcattacg gctcccattg 1020
   aggctcaagt tcggctaaag ggagctggcc taggagccaa aggcagcgca tatggtttgt 1080
   cgggcgccga ttcctacaaa gatgctgtcc ggaaagccat gtttgcccgg ttcactgaga 1140
   tggagtgaga gagagagaga gagagagatg acaaggagca caagaagtgg tccatctccc 1200
   gaattcgctg ttaccgcctg tctctttaag ggcatgcctt gtgctgttaa tagatcttag 1260
   ggtgaaccac ttcattctgc agggttctcc ctcccacctt aaagaagttc cccttatgtg 1320
   ggttgcctgg tgaatggcct tccttcccgc cagagggctt gtgaacagac cggagaggac 1380
   agtggattgt ttatactcca gtgtacatag tgtaatgtag cgtgtttaca tgtgtagcct 1440
   atgttgtggt ccatcagccc ctcacattcc taggggtttg agatgctgta ggtggtatgt 1500
   gacaccaaag ccacctctgt catttgttgt gatgtctttt cttggcaaaa gccttgtgta 1560
   tatttgtata ttacacattt gtacagaatt ttggaagatt ttcaatccaa gttgccaaat 1620
   ctggctcctt tacaaaagaa ataccttgag aaaaaaaann aannaaaaaa aanncccnan 1680
   nnntttttaa aangggncgg gggccaannn ttttccnncc ggggngggna nnaagtaaan 1740
   ngtcccaaat ncccccaaaa nggagcccnn ttaaaattaa angggccgcn nttttaaaan 1800
   nttcngaatn gggnaaaccc tnggggtttn ccaaatttaa cccctttgaa aaaaaanccc 1860
   ctttcncaaa anngggntaa tanccaaaaa gggcccccan ccatttttgc ccnttccaaa 1920
   aaaatttgnc caanccnnaa atgggnaaan ggggaatcca attttttaaa gggnnaaaan 1980
   gggtttaaac nnacgggntt ccaaanttgn ttgggggaat ttttaaattc ccaannnccc 2040
   aagggggnca atttagnggn ccccnaatcc cccaaaaant ggttcnnggn tnaaancngc 2100
   cnnnnccnaa tttntanggg tttacttngn tttaaaaaac ccncccaaaa actcccccnn 2160
   gaaccnaaaa aanaaaagga ngccattttt ngnngnaaac ttttttaann nnccnnttaa 2220
   angggttaaa aaannnnnnn tnnncccnaa tttttcaaan aang 2264

<210> 106
<211> 381
<212> PRT
<213> Homo Sapiens

<400> 106
Ser Thr Ser Thr Gln Ala Pro Ala Ala Ser Pro Thr Gly Val Val Pro
1               5                   10                  15
Gly Thr Lys Tyr Ala Val Pro Asp Thr Ser Thr Tyr Gln Tyr Asp Glu
            20                  25                  30
Ser Ser Gly Tyr Tyr Tyr Asp Pro Thr Thr Gly Leu Tyr Tyr Asp Pro
        35                  40                  45
Asn Ser Gln Tyr Tyr Tyr Asn Ser Leu Thr Gln Gln Tyr Leu Tyr Trp
    50                  55                  60
Asp Gly Glu Lys Glu Thr Tyr Val Pro Ala Ala Glu Ser Ser Ser His
65                  70                  75                  80

Gln Gln Ser Gly Leu Pro Pro Ala Lys Glu Gly Lys Glu Lys Lys Glu
                85                  90                  95
Lys Pro Lys Ser Lys Thr Ala Gln Gln Ile Ala Lys Asp Met Glu Arg
            100                 105                 110
Trp Ala Lys Ser Leu Asn Lys Gln Lys Glu Asn Phe Lys Asn Ser Phe
        115                 120                 125
Gln Pro Val Asn Ser Leu Arg Glu Glu Glu Arg Arg Glu Ser Ala Ala
    130                 135                 140
Ala Asp Ala Gly Phe Ala Leu Phe Glu Lys Lys Gly Ala Leu Ala Glu
145                 150                 155                 160
Arg Gln Gln Leu Ile Pro Glu Leu Val Arg Asn Gly Asp Glu Glu Asn
                165                 170                 175
Pro Leu Lys Arg Gly Leu Val Ala Ala Tyr Ser Gly Asp Ser Asp Asn
            180                 185                 190
Glu Glu Glu Leu Val Glu Arg Leu Glu Ser Glu Glu Glu Lys Leu Ala
        195                 200                 205
Asp Trp Lys Lys Met Ala Cys Leu Leu Cys Arg Arg Gln Phe Pro Asn
    210                 215                 220
Lys Asp Ala Leu Val Arg His Gln Gln Leu Ser Asp Leu His Lys Gln
225                 230                 235                 240
Asn Met Asp Ile Tyr Arg Arg Ser Arg Leu Ser Glu Gln Glu Leu Glu
                245                 250                 255
Ala Leu Glu Leu Arg Glu Arg Glu Met Lys Tyr Arg Asp Arg Ala Ala
            260                 265                 270
Glu Arg Arg Glu Lys Tyr Gly Ile Pro Glu Pro Pro Glu Pro Lys Arg
        275                 280                 285
Lys Lys Gln Phe Asp Ala Gly Thr Val Asn Tyr Glu Gln Pro Thr Lys
    290                 295                 300
Asp Gly Ile Asp His Ser Asn Ile Gly Asn Lys Met Leu Gln Ala Met
305                 310                 315                 320
Gly Trp Arg Glu Gly Ser Gly Leu Gly Arg Lys Cys Gln Gly Ile Thr
                325                 330                 335
Ala Pro Ile Glu Ala Gln Val Arg Leu Lys Gly Ala Gly Leu Gly Ala
            340                 345                 350
Lys Gly Ser Ala Tyr Gly Leu Ser Gly Ala Asp Ser Tyr Lys Asp Ala
        355                 360                 365
Val Arg Lys Ala Met Phe Ala Arg Phe Thr Glu Met Glu
    370                 375                 380

<210> 107
<211> 1367
<212> DNA
<213> Homo Sapiens

<400> 107
gcgacacagg cctcgaggct gtctctgaca agtgttcaca ggaggtgggg acgcctctgc      60
gcgaggaacg aggagctacg ggcctgggcc cggttattgc catgggcagc ggctgccgca     120
tcgaatgcat attcttcagc gagttccacc ccacgctggg acccaagatc acctatcagg     180
tccctgaaga cttcatctcc cgagagctgt ttgacacagt ccaagtgtac atcatcacca     240
agccagagct gcagaacaag cttatcactg tcacagctat ggaaaagaag ctgatcggct     300
gtcctgtgtg catcgaacac aagaagtaca gccgcaatgc tctcctcttc aacctgggct     360
tcgtgtgtga tgcccaggcc aagacctgcg ccctcgagcc cattgttaaa aagctggctg     420
gctatctgac cacactagag ctagagagca gcttcgtgtc catggaggag agcaagcaga     480
agttggtgcc catcatgacc atcttgctgg aggagctaaa tgcctcaggc cggtgcactc     540
tgcccattga tgagtccaac accatccact tgaaggtgat tgagcagcgg ccagaccctc     600

cggtggccca ggagtatgat gtacctgtct ttaccaaaga caaggaggat ttcttcaact 660
cacagtggga cctcactaca caacaaatcc tgccctacat tgatgggttc cgccacatcc 720
agaagatttc agcagaggca gatgtggagc tcaacctggt gcgcattgct atccagaacc 780
tgctgtacta cggcgttgtg acactggtgt ccatcctcca gtactccaat gtatactgcc 840
caacgcccaa ggtccaggac ctggtagatg acaagtccct gcaagaggca tgtctatcct 900
acgtgaccaa gcaagggcac aagagggcca gtctccggga tgtgttccag ctatactgca 960
gcctgagccc tggcactacc gtgcgagacc tcattggccg ccacccccag cagctgcagc 1020
atgttgatga acggaagctg atccagttcg ggcttatgaa gaacctcatc aggcgactac 1080
agaagtatcc tgtgcgggtg actcgggaag agcagagcca ccctgcccgg ctttatacag 1140
gctgccacag ctatgacgag atctgctgca agacaggcat gagctaccat gagctggatg 1200
agcggcttga aaatgacccc aacatcatca tctgctggaa gtgaggctgg tagtgactgg 1260
atggacacat tgctgtgggt agtccctcct actaggaggc ttgtcatact gtctagaggt 1320
tgactcttag ttctgtaaat aaagacatcc atttcaaaca gccaaaa 1367

<210> 108
<211> 413
<212> PRT
<213> Homo Sapiens

<400> 108
Asp Thr Gly Leu Glu Ala Val Ser Asp Lys Cys Ser Gln Glu Val Gly
1               5                   10                  15
Thr Pro Leu Arg Glu Glu Arg Gly Ala Thr Gly Leu Gly Pro Val Ile
            20                  25                  30
Ala Met Gly Ser Gly Cys Arg Ile Glu Cys Ile Phe Phe Ser Glu Phe
        35                  40                  45
His Pro Thr Leu Gly Pro Lys Ile Thr Tyr Gln Val Pro Glu Asp Phe
    50                  55                  60
Ile Ser Arg Glu Leu Phe Asp Thr Val Gln Val Tyr Ile Ile Thr Lys
65                  70                  75                  80
Pro Glu Leu Gln Asn Lys Leu Ile Thr Val Thr Ala Met Glu Lys Lys
                85                  90                  95
Leu Ile Gly Cys Pro Val Cys Ile Glu His Lys Lys Tyr Ser Arg Asn
            100                 105                 110
Ala Leu Leu Phe Asn Leu Gly Phe Val Cys Asp Ala Gln Ala Lys Thr
        115                 120                 125
Cys Ala Leu Glu Pro Ile Val Lys Lys Leu Ala Gly Tyr Leu Thr Thr
    130                 135                 140
Leu Glu Leu Glu Ser Ser Phe Val Ser Met Glu Glu Ser Lys Gln Lys
145                 150                 155                 160
Leu Val Pro Ile Met Thr Ile Leu Leu Glu Glu Leu Asn Ala Ser Gly
                165                 170                 175
Arg Cys Thr Leu Pro Ile Asp Glu Ser Asn Thr Ile His Leu Lys Val
            180                 185                 190
Ile Glu Gln Arg Pro Asp Pro Pro Val Ala Gln Glu Tyr Asp Val Pro
        195                 200                 205
Val Phe Thr Lys Asp Lys Glu Asp Phe Phe Asn Ser Gln Trp Asp Leu
    210                 215                 220
Thr Thr Gln Gln Ile Leu Pro Tyr Ile Asp Gly Phe Arg His Ile Gln
225                 230                 235                 240
Lys Ile Ser Ala Glu Ala Asp Val Glu Leu Asn Leu Val Arg Ile Ala
                245                 250                 255
Ile Gln Asn Leu Leu Tyr Tyr Gly Val Val Thr Leu Val Ser Ile Leu
            260                 265                 270
Gln Tyr Ser Asn Val Tyr Cys Pro Thr Pro Lys Val Gln Asp Leu Val

        275                 280                 285
Asp Asp Lys Ser Leu Gln Glu Ala Cys Leu Ser Tyr Val Thr Lys Gln
    290                 295                 300
Gly His Lys Arg Ala Ser Leu Arg Asp Val Phe Gln Leu Tyr Cys Ser
305                 310                 315                 320
Leu Ser Pro Gly Thr Thr Val Arg Asp Leu Ile Gly Arg His Pro Gln
                325                 330                 335
Gln Leu Gln His Val Asp Glu Arg Lys Leu Ile Gln Phe Gly Leu Met
            340                 345                 350
Lys Asn Leu Ile Arg Arg Leu Gln Lys Tyr Pro Val Arg Val Thr Arg
        355                 360                 365
Glu Glu Gln Ser His Pro Ala Arg Leu Tyr Thr Gly Cys His Ser Tyr
    370                 375                 380
Asp Glu Ile Cys Cys Lys Thr Gly Met Ser Tyr His Glu Leu Asp Glu
385                 390                 395                 400
Arg Leu Glu Asn Asp Pro Asn Ile Ile Ile Cys Trp Lys
                405                 410

<210> 109
<211> 2113
<212> DNA
<213> Homo Sapiens

<400> 109
gtgcggttgg gaacgcggag cggacggatt cgattcaacg gggttccgga ccgcgctgcg 60
ctatggagca ggtcaatgag ctgaaggaga aaggcaacaa ggccctgagc gtgggtaaca 120
tcgatgatgc cttacagtgc tactccgaag ctattaagct ggatccccac aaccacgtgc 180
tgtacagcaa ccgttctgct gcctatgcca agaaaggaga ctaccagaag gcttatgagg 240
atggctgcaa gactgtcgac ctaaagcctg actggggcaa gggctattca cgaaaagcag 300
cagctctaga gttcttaaac cgctttgaag aagccaagcg aacctatgag gagggcttaa 360
aacacgaggc aaataaccct caactgaaag agggtttaca gaatatggag gccaggttgg 420
cagagagaaa attcatgaac cctttcaaca tgcctaatct gtatcagaag ttggagagtg 480
atcccaggac aaggacacta ctcagtgatc ctacctaccg ggagctgata gagcagctac 540
gaaacaagcc ttctgacctg ggcacgaaac tacaagatcc ccggatcatg accactctca 600
gcgtcctcct tggggtcgat ctgggcagta tggatgagga ggaagagatt gcaacacctc 660
caccaccacc ccctcccaaa aaggagacca agccagagcc aatggaagaa gatcttccag 720
agaataagaa gcaggcactg aaagaaaaag agctggggaa cgatgcctac aagaagaaag 780
actttgacac agccttgaag cattacgaca aagccaagga gctggacccc actaacatga 840
cttacattac caatcaagca gcggtatact ttgaaaaggg cgactacaat aagtgccggg 900
agctttgtga gaaggccatt gaagtgggga gagaaaaccg agaagactat cgacagattg 960
ccaaagcata tgctcgaatt ggcaactcct acttcaaaga agaaaagtac aaggatgcca 1020
tccatttcta taacaagtct ctggcagagc accgaacccc agatgtgctc aagaaatgcc 1080
agcaggcaga gaaaatcctg aaggagcaag agcggctggc ctacataaac cccgacctgg 1140
ctttggagga gaagaacaaa ggcaacgagt gttttcagaa aggggactat ccccaggcca 1200
tgaagcatta tacagaagcc atcaaaagga acccgaaaga tgccaaatta tacagcaatc 1260
gagctgcctg ctacaccaaa ctcctggagt tccagctggc actcaaggac tgtgaggaat 1320
gtatccagct ggagccgacc ttcatcaagg gttatacacg gaaagccgct gcgctggaag 1380
cgatgaagga ctacaccaaa gccatggatg tgtaccagaa ggcgctagac ctggactcca 1440
gctgtaagga ggcggcagac ggctaccagc gctgtatgat ggcgcagtac aaccggcacg 1500
acagccccga agatgtgaag cgacgagcca tggccgaccc tgaggtgcag cagatcatga 1560
gtgacccagc catgcgcctt atcctggaac agatgcagaa ggacccccag gcactcagcg 1620
aacacttaaa gaatcctgta atagcacaga agatccagaa gctgatggat gtgggtctga 1680
ttgcaattcg gtgatgactt gttcatcccc ccttcccttc gccctcatgt ggaaagagga 1740
gctgggaccg cggcgagcag cacggagcgg aagggagagc aggggagaga aggcctcatc 1800
tctctatatt tatacataac cccggggaag acacagagac tcgtacctgc gctgtttgtg 1860

ccgccgctgc ctctgggccc tcccagcaca cgcatggtct cttcaccgct gccctcgagt 1920
tccatgtctc tttcccctgc ccctagttgc tgtctcggct gctctcccat agttggtttt 1980
tttttatttt ggggcagtgg gcatgttatg gggaggggag ggggttcttc cagcctcagg 2040
tcccagctgt ctcacgttgt ttattctgcg tccccttctc caataaaaca agccagttgg 2100
gcgtggttat aac 2113

<210> 110
<211> 543
<212> PRT
<213> Homo Sapiens

<400> 110
Met Glu Gln Val Asn Glu Leu Lys Glu Lys Gly Asn Lys Ala Leu Ser
1               5                   10                  15
Val Gly Asn Ile Asp Asp Ala Leu Gln Cys Tyr Ser Glu Ala Ile Lys
            20                  25                  30
Leu Asp Pro His Asn His Val Leu Tyr Ser Asn Arg Ser Ala Ala Tyr
        35                  40                  45
Ala Lys Lys Gly Asp Tyr Gln Lys Ala Tyr Glu Asp Gly Cys Lys Thr
    50                  55                  60
Val Asp Leu Lys Pro Asp Trp Gly Lys Gly Tyr Ser Arg Lys Ala Ala
65                  70                  75                  80
Ala Leu Glu Phe Leu Asn Arg Phe Glu Glu Ala Lys Arg Thr Tyr Glu
                85                  90                  95
Glu Gly Leu Lys His Glu Ala Asn Asn Pro Gln Leu Lys Glu Gly Leu
            100                 105                 110
Gln Asn Met Glu Ala Arg Leu Ala Glu Arg Lys Phe Met Asn Pro Phe
        115                 120                 125
Asn Met Pro Asn Leu Tyr Gln Lys Leu Glu Ser Asp Pro Arg Thr Arg
    130                 135                 140
Thr Leu Leu Ser Asp Pro Thr Tyr Arg Glu Leu Ile Glu Gln Leu Arg
145                 150                 155                 160
Asn Lys Pro Ser Asp Leu Gly Thr Lys Leu Gln Asp Pro Arg Ile Met
                165                 170                 175
Thr Thr Leu Ser Val Leu Leu Gly Val Asp Leu Gly Ser Met Asp Glu
            180                 185                 190
Glu Glu Glu Ile Ala Thr Pro Pro Pro Pro Pro Pro Pro Lys Lys Glu
        195                 200                 205
Thr Lys Pro Glu Pro Met Glu Glu Asp Leu Pro Glu Asn Lys Lys Gln
    210                 215                 220
Ala Leu Lys Glu Lys Glu Leu Gly Asn Asp Ala Tyr Lys Lys Lys Asp
225                 230                 235                 240
Phe Asp Thr Ala Leu Lys His Tyr Asp Lys Ala Lys Glu Leu Asp Pro
                245                 250                 255
Thr Asn Met Thr Tyr Ile Thr Asn Gln Ala Ala Val Tyr Phe Glu Lys
            260                 265                 270
Gly Asp Tyr Asn Lys Cys Arg Glu Leu Cys Glu Lys Ala Ile Glu Val
        275                 280                 285
Gly Arg Glu Asn Arg Glu Asp Tyr Arg Gln Ile Ala Lys Ala Tyr Ala
    290                 295                 300
Arg Ile Gly Asn Ser Tyr Phe Lys Glu Glu Lys Tyr Lys Asp Ala Ile
305                 310                 315                 320
His Phe Tyr Asn Lys Ser Leu Ala Glu His Arg Thr Pro Asp Val Leu
                325                 330                 335
Lys Lys Cys Gln Gln Ala Glu Lys Ile Leu Lys Glu Gln Glu Arg Leu

            340                 345                 350
Ala Tyr Ile Asn Pro Asp Leu Ala Leu Glu Glu Lys Asn Lys Gly Asn
        355                 360                 365
Glu Cys Phe Gln Lys Gly Asp Tyr Pro Gln Ala Met Lys His Tyr Thr
    370                 375                 380
Glu Ala Ile Lys Arg Asn Pro Lys Asp Ala Lys Leu Tyr Ser Asn Arg
385                 390                 395                 400
Ala Ala Cys Tyr Thr Lys Leu Leu Glu Phe Gln Leu Ala Leu Lys Asp
                405                 410                 415
Cys Glu Glu Cys Ile Gln Leu Glu Pro Thr Phe Ile Lys Gly Tyr Thr
            420                 425                 430
Arg Lys Ala Ala Ala Leu Glu Ala Met Lys Asp Tyr Thr Lys Ala Met
        435                 440                 445
Asp Val Tyr Gln Lys Ala Leu Asp Leu Asp Ser Ser Cys Lys Glu Ala
    450                 455                 460
Ala Asp Gly Tyr Gln Arg Cys Met Met Ala Gln Tyr Asn Arg His Asp
465                 470                 475                 480
Ser Pro Glu Asp Val Lys Arg Arg Ala Met Ala Asp Pro Glu Val Gln
                485                 490                 495
Gln Ile Met Ser Asp Pro Ala Met Arg Leu Ile Leu Glu Gln Met Gln
            500                 505                 510
Lys Asp Pro Gln Ala Leu Ser Glu His Leu Lys Asn Pro Val Ile Ala
        515                 520                 525
Gln Lys Ile Gln Lys Leu Met Asp Val Gly Leu Ile Ala Ile Arg
    530                 535                 540

<210> 111
<211> 2765
<212> DNA
<213> Homo Sapiens

<400> 111
   gggctgcgac tgacaagcgg ctctgcccgg gaccttctcg ctttcatcta gcgctgcact 60
   caatggaggg gcgggcaccg cagtgcttaa tgctgtctta actagtgtag gaaaacggct 120
   caacccaccg ctgccgaaat gaagtataag aatcttatgg caagggcctt atatgacaat 180
   gtcccagagt gtgccgagga actggccttt cgcaagggag acatcctgac cgtcatagag 240
   cagaacacag ggggactgga aggatggtgg ctgtgctcgt tacacggtcg gcaaggcatt 300
   gtcccaggca accgggtgaa gcttctgatt ggtcccatgc aggagactgc ctccagtcac 360
   gagcagcctg cctctggact gatgcagcag acctttggcc aacagaagct ctatcaagtg 420
   ccaaacccac aggctgctcc ccgagacacc atctaccaag tgccaccttc ctaccaaaat 480
   cagggaattt accaagtccc cactggccac ggcacccaag aacaagaggt atatcaggtg 540
   ccaccatcag tgcagagaag cattggggga accagtgggc cccacgtggg taaaaaggtg 600
   ataacccccg tgaggacagg ccatggctac gtatacgagt acccatccag ataccaaaag 660
   gatgtctatg atatccctcc ttctcatacc actcaagggg tatacgacat ccctccctca 720
   tcagcaaaag gccctgtgtt ttcagttcca gtgggagaga taaaacctca aggggtgtat 780
   gacatcccgc ctacaaaagg ggtatatgcc attccgccct ctgcttgccg ggatgaagca 840
   gggcttaggg aaaaagacta tgacttcccc cctcccatga gacaagctgg aaggccggac 900
   ctcagaccgg aggggtttta tgacattcct ccaacctgca ccaagccagc agggaaggac 960
   cttcatgtaa aatacaactg tgacattcca ggagctgcag aaccggtggc tcgaaggcac 1020
   cagagcctgt ccccgaatca cccacccccg caactcggac agtcagtggg ctctcagaac 1080
   gacgcatatg atgtcccccg aggcgttcag tttcttgagc caccagcaga aaccagtgag 1140
   aaagcaaacc cccaggaaag ggatggtgtt tatgatgtcc ctctgcataa cccgccagat 1200
   gctaaaggct ctcgggactt ggtggatggg atcaaccgat tgtctttctc cagtacaggc 1260
   agcacccgga gtaacatgtc cacgtcttcc acctcctcca aggagtcctc actgtcagcc 1320
   tccccagctc aggacaaaag gctcttcctg gatccagaca cagctattga gagacttcag 1380
   cggctccagc aggcccttga gatgggtgtc tccagcctaa tggcactggt cactaccgac 1440
   tggcggtgtt acggatatat ggaaagacac atcaatgaaa tacgcacagc agtggacaag 1500
   gtggagctgt tcctgaagga gtacctccac tttgtcaagg gagctgttgc aaatgctgcc 1560
   tgcctcccgg aactcatcct ccacaacaag atgaagcggg agctgcaacg agtcgaagac 1620
   tcccaccaga tcctgagtca aaccagccat gacttaaatg agtgcagctg gtccctgaat 1680
   atcttggcca tcaacaagcc ccagaacaag tgtgacgatc tggaccggtt tgtgatggtg 1740
   gcaaagacgg tgcccgatga cgccaagcag ctcaccacaa ccatcaacac caacgcagag 1800
   gccctcttca gacccggccc tggcagcttg catctgaaga atgggccgga gagcatcatg 1860
   aactcaacgg agtacccaca cggtggctcc cagggacagc tgctgcatcc tggtgaccac 1920
   aaggcccagg cccacaacaa ggcactgccc ccaggcctga gcaaggagca ggcccctgac 1980
   tgtagcagca gtgatggttc tgagaggagc tggatggatg actacgatta cgtccaccta 2040
   cagggtaagg aggagtttga gaggcaacag aaagagctat tggaaaaaga gaatatcatg 2100
   aaacagaaca agatgcagct ggaacatcat cagctgagcc agttccagct gttggaacaa 2160
   gagattacaa agcccgtgga gaatgacatc tcgaagtgga agccctctca gagcctaccc 2220
   accacaaaca gtggcgtgag tgctcaggat cggcagttgc tgtgcttcta ctatgaccaa 2280
   tgtgagaccc atttcatttc ccttctcaac gccattgacg cactcttcag ttgtgtcagc 2340
   tcagcccagc ccccgcgaat cttcgtggca cacagcaagt ttgtcatcct cagtgcacac 2400
   aaactggtgt tcattggaga cacgctgaca cggcaggtga ctgcccagga cattcgcaac 2460
   aaagtcatga actccagcaa ccagctctgc gagcagctca agactatagt catggcaacc 2520
   aagatggccg ccctccatta ccccagcacc acggccctgc aggaaatggt gcaccaagtg 2580
   acagaccttt ctagaaatgc ccagctgttc aagcgctctt tgctggagat ggcaacgttc 2640
   tgagaagaaa aaaaagagga aggggactgc gttaacggtt actaaggaaa actggaaata 2700
   ctgtctggtt tttgtaaatg ttatctattt ttgtagatat tttatataaa aatgaaatat 2760
   tttcc 2765

<210> 112
<211> 834
<212> PRT
<213> Homo Sapiens

<400> 112
Met Lys Tyr Lys Asn Leu Met Ala Arg Ala Leu Tyr Asp Asn Val Pro
1               5                   10                  15
Glu Cys Ala Glu Glu Leu Ala Phe Arg Lys Gly Asp Ile Leu Thr Val
            20                  25                  30
Ile Glu Gln Asn Thr Gly Gly Leu Glu Gly Trp Trp Leu Cys Ser Leu
        35                  40                  45
His Gly Arg Gln Gly Ile Val Pro Gly Asn Arg Val Lys Leu Leu Ile
    50                  55                  60
Gly Pro Met Gln Glu Thr Ala Ser Ser His Glu Gln Pro Ala Ser Gly
65                  70                  75                  80
Leu Met Gln Gln Thr Phe Gly Gln Gln Lys Leu Tyr Gln Val Pro Asn
                85                  90                  95
Pro Gln Ala Ala Pro Arg Asp Thr Ile Tyr Gln Val Pro Pro Ser Tyr
            100                 105                 110
Gln Asn Gln Gly Ile Tyr Gln Val Pro Thr Gly His Gly Thr Gln Glu
        115                 120                 125
Gln Glu Val Tyr Gln Val Pro Pro Ser Val Gln Arg Ser Ile Gly Gly
    130                 135                 140
Thr Ser Gly Pro His Val Gly Lys Lys Val Ile Thr Pro Val Arg Thr
145                 150                 155                 160
Gly His Gly Tyr Val Tyr Glu Tyr Pro Ser Arg Tyr Gln Lys Asp Val
                165                 170                 175
Tyr Asp Ile Pro Pro Ser His Thr Thr Gln Gly Val Tyr Asp Ile Pro
            180                 185                 190

Pro Ser Ser Ala Lys Gly Pro Val Phe Ser Val Pro Val Gly Glu Ile
        195                 200                 205
Lys Pro Gln Gly Val Tyr Asp Ile Pro Pro Thr Lys Gly Val Tyr Ala
    210                 215                 220
Ile Pro Pro Ser Ala Cys Arg Asp Glu Ala Gly Leu Arg Glu Lys Asp
225                 230                 235                 240
Tyr Asp Phe Pro Pro Pro Met Arg Gln Ala Gly Arg Pro Asp Leu Arg
                245                 250                 255
Pro Glu Gly Val Tyr Asp Ile Pro Pro Thr Cys Thr Lys Pro Ala Gly
            260                 265                 270
Lys Asp Leu His Val Lys Tyr Asn Cys Asp Ile Pro Gly Ala Ala Glu
        275                 280                 285
Pro Val Ala Arg Arg His Gln Ser Leu Ser Pro Asn His Pro Pro Pro
    290                 295                 300
Gln Leu Gly Gln Ser Val Gly Ser Gln Asn Asp Ala Tyr Asp Val Pro
305                 310                 315                 320
Arg Gly Val Gln Phe Leu Glu Pro Pro Ala Glu Thr Ser Glu Lys Ala
                325                 330                 335
Asn Pro Gln Glu Arg Asp Gly Val Tyr Asp Val Pro Leu His Asn Pro
            340                 345                 350
Pro Asp Ala Lys Gly Ser Arg Asp Leu Val Asp Gly Ile Asn Arg Leu
        355                 360                 365
Ser Phe Ser Ser Thr Gly Ser Thr Arg Ser Asn Met Ser Thr Ser Ser
    370                 375                 380
Thr Ser Ser Lys Glu Ser Ser Leu Ser Ala Ser Pro Ala Gln Asp Lys
385                 390                 395                 400
Arg Leu Phe Leu Asp Pro Asp Thr Ala Ile Glu Arg Leu Gln Arg Leu
                405                 410                 415
Gln Gln Ala Leu Glu Met Gly Val Ser Ser Leu Met Ala Leu Val Thr
            420                 425                 430
Thr Asp Trp Arg Cys Tyr Gly Tyr Met Glu Arg His Ile Asn Glu Ile
        435                 440                 445
Arg Thr Ala Val Asp Lys Val Glu Leu Phe Leu Lys Glu Tyr Leu His
    450                 455                 460
Phe Val Lys Gly Ala Val Ala Asn Ala Ala Cys Leu Pro Glu Leu Ile
465                 470                 475                 480
Leu His Asn Lys Met Lys Arg Glu Leu Gln Arg Val Glu Asp Ser His
                485                 490                 495
Gln Ile Leu Ser Gln Thr Ser His Asp Leu Asn Glu Cys Ser Trp Ser
            500                 505                 510
Leu Asn Ile Leu Ala Ile Asn Lys Pro Gln Asn Lys Cys Asp Asp Leu
        515                 520                 525
Asp Arg Phe Val Met Val Ala Lys Thr Val Pro Asp Asp Ala Lys Gln
    530                 535                 540
Leu Thr Thr Thr Ile Asn Thr Asn Ala Glu Ala Leu Phe Arg Pro Gly
545                 550                 555                 560
Pro Gly Ser Leu His Leu Lys Asn Gly Pro Glu Ser Ile Met Asn Ser
                565                 570                 575
Thr Glu Tyr Pro His Gly Gly Ser Gln Gly Gln Leu Leu His Pro Gly
            580                 585                 590
Asp His Lys Ala Gln Ala His Asn Lys Ala Leu Pro Pro Gly Leu Ser
        595                 600                 605
Lys Glu Gln Ala Pro Asp Cys Ser Ser Ser Asp Gly Ser Glu Arg Ser
    610                 615                 620
Trp Met Asp Asp Tyr Asp Tyr Val His Leu Gln Gly Lys Glu Glu Phe

625                 630                 635                 640
Glu Arg Gln Gln Lys Glu Leu Leu Glu Lys Glu Asn Ile Met Lys Gln
                645                 650                 655
Asn Lys Met Gln Leu Glu His His Gln Leu Ser Gln Phe Gln Leu Leu
            660                 665                 670
Glu Gln Glu Ile Thr Lys Pro Val Glu Asn Asp Ile Ser Lys Trp Lys
        675                 680                 685
Pro Ser Gln Ser Leu Pro Thr Thr Asn Ser Gly Val Ser Ala Gln Asp
    690                 695                 700
Arg Gln Leu Leu Cys Phe Tyr Tyr Asp Gln Cys Glu Thr His Phe Ile
705                 710                 715                 720
Ser Leu Leu Asn Ala Ile Asp Ala Leu Phe Ser Cys Val Ser Ser Ala
                725                 730                 735
Gln Pro Pro Arg Ile Phe Val Ala His Ser Lys Phe Val Ile Leu Ser
            740                 745                 750
Ala His Lys Leu Val Phe Ile Gly Asp Thr Leu Thr Arg Gln Val Thr
        755                 760                 765
Ala Gln Asp Ile Arg Asn Lys Val Met Asn Ser Ser Asn Gln Leu Cys
    770                 775                 780
Glu Gln Leu Lys Thr Ile Val Met Ala Thr Lys Met Ala Ala Leu His
785                 790                 795                 800
Tyr Pro Ser Thr Thr Ala Leu Gln Glu Met Val His Gln Val Thr Asp
                805                 810                 815
Leu Ser Arg Asn Ala Gln Leu Phe Lys Arg Ser Leu Leu Glu Met Ala
            820                 825                 830
Thr Phe

<210> 113
<211> 3429
<212> DNA
<213> Homo Sapiens

<400> 113
gagacaaagc agcgcccgtc tgcttcgggc ctctggaatt tagcgctcgc ccagctagcc      60
gcagaaatga ctgctgtcca tgcaggcaac ataaacttca agtgggatcc taaaagtcta     120
gagatcagga ctctggcagt tgagagactg ttggagcctc ttgttacaca ggttacaacc     180
cttgtaaaca ccaatagtaa agggccctct aataagaaga gaggtcgttc taagaaggcc     240
catgttttgg ctgcatctgt tgaacaagca actgagaatt tcttggagaa gggggataaa     300
attgcgaagg agagccagtt tctcaaggag gagcttgtgg ctgctgtaga agatgttcga     360
aaacaaggtg atttgatgaa ggctgctgca ggagagttcg cagatgatcc ctgctcttct     420
gtgaagcgag gcaacatggt tcgggcagct cgagctttgc tctctgctgt tacccggttg     480
ctgattttgg ctgacatggc agatgtctac aaattacttg ttcagctgaa agttgtggaa     540
gatggtatct tgaagttgag gaatgctggc aatgaacaag acttaggaat ccagtataaa     600
gccctaaaac ctgaagtgga taagctgaac attatggcag ccaaaagaca acaggaattg     660
aaagatgttg gccatcgtga tcagatggct gcagctagag gaatcctgca gaagaacgtt     720
ccgatcctct atactgcatc ccaggcatgc ctacagcacc ctgatgtcgc agcctataag     780
gccaacaggg acctgatata caagcagctg cagcaggcgg tcacaggcat ttccaatgca     840
gcccaggcca ctgcctcaga cgatgcctca cagcaccagg gtggaggagg aggagaactg     900
gcatatgcac tcaataactt tgacaaacaa atcattgtgg accccttgag cttcagcgag     960
gagcgcttta ggccttccct ggaggagcgt ctggaaagca tcattagtgg ggctgccttg    1020
atggccgact cgtcctgcac gcgtgatgac cgtcgtgagc gaattgtggc agagtgtaat    1080
gctgtccgcc aggccctgca ggacctgctt tcggagtaca tgggcaatgc tggacgtaaa    1140
gaaagaagtg atgcactcaa ttctgcaata gataaaatga ccaagaagac cagggacttg    1200
cgtagacagc tccgcaaagc tgtcatggac cacgtttcag attctttcct ggaaaccaat    1260

gttccacttt tggtattgat tgaagctgca aagaatggaa atgagaaaga agttaaggag 1320
tatgcccaag ttttccgtga acatgccaac aaattgattg aggttgccaa cttggcctgt 1380
tccatctcaa ataatgaaga aggtgtaaag cttgttcgaa tgtctgcaag ccagttagaa 1440
gccctctgtc ctcaggttat taatgctgca ctggctttag cagcaaaacc acagagtaaa 1500
ctggcccaag agaacatgga tctttttaaa gaacaatggg aaaaacaagt ccgtgttctc 1560
acagatgctg tcgatgacat tacttccatt gatgacttct tggctgtctc agagaatcac 1620
attttggaag atgtgaacaa atgtgtcatt gctctccaag agaaggatgt ggatggcctg 1680
gaccgcacag ctggtgcaat tcgaggccgg gcagcccggg tcattcacgt agtcacctca 1740
gagatggaca actatgagcc aggagtctac acagagaagg ttctggaagc cactaagctg 1800
ctctccaaca cagtcatgcc acgttttact gagcaagtag aagcagccgt ggaagccctc 1860
agctcggacc ctgcccagcc catggatgag aatgagttta tcgatgcttc ccgcctggta 1920
tatgatggca tccgggacat caggaaagca gtgctgatga taaggacccc tgaggagttg 1980
gatgactctg actttgagac agaagatttt gatgtcagaa gcaggacgag cgtccagaca 2040
gaagacgatc agctgatagc tggccagagt gcccgggcga tcatggctca gcttccccag 2100
gagcaaaaag cgaagattgc ggaacaggtg gccagcttcc aggaagaaaa gagcaagctg 2160
gatgctgaag tgtccaaatg ggacgacagt ggcaatgaca tcattgtgct ggccaagcag 2220
atgtgcatga ttatgatgga gatgacagac tttacccgag gtaaaggacc actcaaaaat 2280
acatcggatg tcatcagtgc tgccaagaaa attgctgagg caggatccag gatggacaag 2340
cttggccgca ccattgcaga ccattgcccc gactcggctt gcaagcagga cctgctggcc 2400
tacctgcaac gcatcgccct ctactgccac cagctgaaca tctgcagcaa ggtcaaggcc 2460
gaggtgcaga atctcggcgg ggagcttgtt gtctctgggg tggacagcgc catgtccctg 2520
atccaggcag ccaagaactt gatgaatgct gtggtgcaga cagtgaaggc atcctacgtc 2580
gcctctacca aataccaaaa gtcacagggt atggcttccc tcaaccttcc tgctgtgtca 2640
tggaagatga aggcaccaga gaaaaagcca ttggtgaaga gagagaaaca ggatgagaca 2700
cagaccaaga ttaaacgggc atctcagaag aagcacgtga acccggtgca ggccctcagc 2760
gagttcaaag ctatggacag catctaagtc tgcccaggcc ggccgccccc acccctcggg 2820
gctcctgaat atcagtcact gttcgtcact caaatgaatt tgctaaatac aacactgata 2880
ctagattcca cagggaaatg ggcagactga accagtccag gtggtgaatt ttccaagaac 2940
atagtttaag ttgattaaaa atgcttttag aatgcaggag cctacttcta gctgtatttt 3000
ttgtatgctt aaataaaaat aaaaattcat aaccaaagag aatcccacat tagcttgtta 3060
gtaatgctct gaccaagccg agatgcccat tctcttagtg atggcggcgt tagggtttga 3120
gagaagggaa tttggctcaa cttcagttga gagggtgcag tccagacagc ttgactgctt 3180
ttaaatgacc aaagatgacc tgtggtaagc aacctgggca tcttagaagc agtccctgga 3240
gaaggcatgt tcccagaaag gtctctggag ggacaaactc actcagtaaa acataatgta 3300
tcatcatgaa gaaaactgat tctctatgac atgaaatgaa aattttaatg cattgttata 3360
attactaatg tacgctgctg caggacatta ataaagttgc ttttttaggc tacagtgtct 3420
cgatgccat 3429

<210> 114
<211> 906
<212> PRT
<213> Homo Sapiens

<400> 114
Met Thr Ala Val His Ala Gly Asn Ile Asn Phe Lys Trp Asp Pro Lys
1               5                   10                  15
Ser Leu Glu Ile Arg Thr Leu Ala Val Glu Arg Leu Leu Glu Pro Leu
            20                  25                  30
Val Thr Gln Val Thr Thr Leu Val Asn Thr Asn Ser Lys Gly Pro Ser
        35                  40                  45
Asn Lys Lys Arg Gly Arg Ser Lys Lys Ala His Val Leu Ala Ala Ser
    50                  55                  60
Val Glu Gln Ala Thr Glu Asn Phe Leu Glu Lys Gly Asp Lys Ile Ala
65                  70                  75                  80
Lys Glu Ser Gln Phe Leu Lys Glu Glu Leu Val Ala Ala Val Glu Asp

                85                  90                  95
Val Arg Lys Gln Gly Asp Leu Met Lys Ala Ala Ala Gly Glu Phe Ala
            100                 105                 110
Asp Asp Pro Cys Ser Ser Val Lys Arg Gly Asn Met Val Arg Ala Ala
        115                 120                 125
Arg Ala Leu Leu Ser Ala Val Thr Arg Leu Leu Ile Leu Ala Asp Met
    130                 135                 140
Ala Asp Val Tyr Lys Leu Leu Val Gln Leu Lys Val Val Glu Asp Gly
145                 150                 155                 160
Ile Leu Lys Leu Arg Asn Ala Gly Asn Glu Gln Asp Leu Gly Ile Gln
                165                 170                 175
Tyr Lys Ala Leu Lys Pro Glu Val Asp Lys Leu Asn Ile Met Ala Ala
            180                 185                 190
Lys Arg Gln Gln Glu Leu Lys Asp Val Gly His Arg Asp Gln Met Ala
        195                 200                 205
Ala Ala Arg Gly Ile Leu Gln Lys Asn Val Pro Ile Leu Tyr Thr Ala
    210                 215                 220
Ser Gln Ala Cys Leu Gln His Pro Asp Val Ala Ala Tyr Lys Ala Asn
225                 230                 235                 240
Arg Asp Leu Ile Tyr Lys Gln Leu Gln Gln Ala Val Thr Gly Ile Ser
                245                 250                 255
Asn Ala Ala Gln Ala Thr Ala Ser Asp Asp Ala Ser Gln His Gln Gly
            260                 265                 270
Gly Gly Gly Gly Glu Leu Ala Tyr Ala Leu Asn Asn Phe Asp Lys Gln
        275                 280                 285
Ile Ile Val Asp Pro Leu Ser Phe Ser Glu Glu Arg Phe Arg Pro Ser
    290                 295                 300
Leu Glu Glu Arg Leu Glu Ser Ile Ile Ser Gly Ala Ala Leu Met Ala
305                 310                 315                 320
Asp Ser Ser Cys Thr Arg Asp Asp Arg Arg Glu Arg Ile Val Ala Glu
                325                 330                 335
Cys Asn Ala Val Arg Gln Ala Leu Gln Asp Leu Leu Ser Glu Tyr Met
            340                 345                 350
Gly Asn Ala Gly Arg Lys Glu Arg Ser Asp Ala Leu Asn Ser Ala Ile
        355                 360                 365
Asp Lys Met Thr Lys Lys Thr Arg Asp Leu Arg Arg Gln Leu Arg Lys
    370                 375                 380
Ala Val Met Asp His Val Ser Asp Ser Phe Leu Glu Thr Asn Val Pro
385                 390                 395                 400
Leu Leu Val Leu Ile Glu Ala Ala Lys Asn Gly Asn Glu Lys Glu Val
                405                 410                 415
Lys Glu Tyr Ala Gln Val Phe Arg Glu His Ala Asn Lys Leu Ile Glu
            420                 425                 430
Val Ala Asn Leu Ala Cys Ser Ile Ser Asn Asn Glu Glu Gly Val Lys
        435                 440                 445
Leu Val Arg Met Ser Ala Ser Gln Leu Glu Ala Leu Cys Pro Gln Val
    450                 455                 460
Ile Asn Ala Ala Leu Ala Leu Ala Ala Lys Pro Gln Ser Lys Leu Ala
465                 470                 475                 480
Gln Glu Asn Met Asp Leu Phe Lys Glu Gln Trp Glu Lys Gln Val Arg
                485                 490                 495
Val Leu Thr Asp Ala Val Asp Asp Ile Thr Ser Ile Asp Asp Phe Leu
            500                 505                 510
Ala Val Ser Glu Asn His Ile Leu Glu Asp Val Asn Lys Cys Val Ile
        515                 520                 525

Ala Leu Gln Glu Lys Asp Val Asp Gly Leu Asp Arg Thr Ala Gly Ala
        530                 535                 540
Ile Arg Gly Arg Ala Ala Arg Val Ile His Val Val Thr Ser Glu Met
545                 550                 555                 560
Asp Asn Tyr Glu Pro Gly Val Tyr Thr Glu Lys Val Leu Glu Ala Thr
                565                 570                 575
Lys Leu Leu Ser Asn Thr Val Met Pro Arg Phe Thr Glu Gln Val Glu
            580                 585                 590
Ala Ala Val Glu Ala Leu Ser Ser Asp Pro Ala Gln Pro Met Asp Glu
        595                 600                 605
Asn Glu Phe Ile Asp Ala Ser Arg Leu Val Tyr Asp Gly Ile Arg Asp
    610                 615                 620
Ile Arg Lys Ala Val Leu Met Ile Arg Thr Pro Glu Glu Leu Asp Asp
625                 630                 635                 640
Ser Asp Phe Glu Thr Glu Asp Phe Asp Val Arg Ser Arg Thr Ser Val
                645                 650                 655
Gln Thr Glu Asp Asp Gln Leu Ile Ala Gly Gln Ser Ala Arg Ala Ile
            660                 665                 670
Met Ala Gln Leu Pro Gln Glu Gln Lys Ala Lys Ile Ala Glu Gln Val
        675                 680                 685
Ala Ser Phe Gln Glu Glu Lys Ser Lys Leu Asp Ala Glu Val Ser Lys
    690                 695                 700
Trp Asp Asp Ser Gly Asn Asp Ile Ile Val Leu Ala Lys Gln Met Cys
705                 710                 715                 720
Met Ile Met Met Glu Met Thr Asp Phe Thr Arg Gly Lys Gly Pro Leu
                725                 730                 735
Lys Asn Thr Ser Asp Val Ile Ser Ala Ala Lys Lys Ile Ala Glu Ala
            740                 745                 750
Gly Ser Arg Met Asp Lys Leu Gly Arg Thr Ile Ala Asp His Cys Pro
        755                 760                 765
Asp Ser Ala Cys Lys Gln Asp Leu Leu Ala Tyr Leu Gln Arg Ile Ala
    770                 775                 780
Leu Tyr Cys His Gln Leu Asn Ile Cys Ser Lys Val Lys Ala Glu Val
785                 790                 795                 800
Gln Asn Leu Gly Gly Glu Leu Val Val Ser Gly Val Asp Ser Ala Met
                805                 810                 815
Ser Leu Ile Gln Ala Ala Lys Asn Leu Met Asn Ala Val Val Gln Thr
            820                 825                 830
Val Lys Ala Ser Tyr Val Ala Ser Thr Lys Tyr Gln Lys Ser Gln Gly
        835                 840                 845
Met Ala Ser Leu Asn Leu Pro Ala Val Ser Trp Lys Met Lys Ala Pro
    850                 855                 860
Glu Lys Lys Pro Leu Val Lys Arg Glu Lys Gln Asp Glu Thr Gln Thr
865                 870                 875                 880
Lys Ile Lys Arg Ala Ser Gln Lys Lys His Val Asn Pro Val Gln Ala
                885                 890                 895
Leu Ser Glu Phe Lys Ala Met Asp Ser Ile
            900                 905

<210> 115
<211> 1701
<212> DNA
<213> Homo Sapiens

<400> 115

cggccggccg ccatggctaa cgtggctgac acgaagctgt acgacatcct gggcgttccc 60
gcgggcgcca gcgagaacga gctgaagaag gcatacagaa agttagccaa ggaatatcat 120
cctgataaga atccccaaat gcaggagaca aactttaaag aaataagttt tgcatatgaa 180
gtactatcaa atcctgagaa gcgtgagtta tatgacagat acggagagca aggtcttcgg 240
gaaggcagcg gcggaggtgg gtggcatgga ttgatatttt ctctcaccgt tttttgtggg 300
ggattgttcg gcttcatggg caatcagagt agaagtcgaa atggcagaag aagaggagag 360
gacatgatgc atccactcaa agtatcttta gaagatctgt ataatggcaa gacaaccaaa 420
ctacaactta gcaagaatgt gctctgtagt gcatgcagtg gccaaggcgg aaagtctgga 480
gctgtccaaa agtgtagtgc ttgtcgaggt cgaggtgtgc gcatcatgat cagacagctg 540
gctccaggga tggtacaaca gatgcagtct gtgtgctctg attgtaatgg tgaaggagag 600
gtaattaatg aaaaagaccg ctgtaaaaaa tgtgaaggga agaaggtgat taaagaagtc 660
aagattcttg aagtccacgt agacaaaggc atgaaacatg gacagagaat tacattcact 720
ggggaagcag accaggcccc agagtggaac ccggagacat tgttcttttt gctaccagga 780
gaaaagaaca tggaggtatt tcagagagat gggaatgatt tgcacatgac atataaaata 840
ggacttgttg aagctctatg tggatttcag ttcacattaa gccaccttga tggacgtcag 900
attgtggtga aatacccccc tggcaaagta attgaaccag ggtgtgttcg tgtagttcga 960
ggtgaaggga tgccgcagta tcgtaatccc tttgaaaaag gtgggcttta cataaagttt 1020
gatgtgcagt ttcctgaaaa caactggatc aacccagaca agctttctga actagaagat 1080
cttctgccat ctagaccgga agttcctaac ataattggag aaacagagga ggtagagctt 1140
caggaatttg atagcactcg aggctcagga ggtggtcaga ggcgtgaagc ctataatgat 1200
agctctgatg aagaaagcag cagccatcat ggacctggag tgcagtgtgc ccatcagtaa 1260
actctgcaaa caaattgcac aggtggattt tctttccaca tttgcctgat ttgttctcag 1320
caatccagct ggagtgtctt atcaatccag atgaactgag ggacatctgt tggtctatgt 1380
ataactttta aaattggtat agtatctaca gagtgtataa tttaaactaa ccacaaagct 1440
ttacatcttc attttgactg ttccatagca gaataaagca cttgaaagga aacaagactc 1500
cctttcacac atggattatt ataagtttca atcctggtat ctgtgcttga tttttatcag 1560
ttttgtgtag atttttatgt ttcatatttt aaatttaaat cccacattgt aaagtttgta 1620
caatttgtcc tgaagctttg tgtttggctg cacctgcata agctgctaca aatagaataa 1680
agaatttcat agcctgtaaa a 1701

<210> 116
<211> 415
<212> PRT
<213> Homo Sapiens

<400> 116
Met Ala Asn Val Ala Asp Thr Lys Leu Tyr Asp Ile Leu Gly Val Pro
1               5                   10                  15
Ala Gly Ala Ser Glu Asn Glu Leu Lys Lys Ala Tyr Arg Lys Leu Ala
            20                  25                  30
Lys Glu Tyr His Pro Asp Lys Asn Pro Gln Met Gln Glu Thr Asn Phe
        35                  40                  45
Lys Glu Ile Ser Phe Ala Tyr Glu Val Leu Ser Asn Pro Glu Lys Arg
    50                  55                  60
Glu Leu Tyr Asp Arg Tyr Gly Glu Gln Gly Leu Arg Glu Gly Ser Gly
65                  70                  75                  80
Gly Gly Gly Trp His Gly Leu Ile Phe Ser Leu Thr Val Phe Cys Gly
                85                  90                  95
Gly Leu Phe Gly Phe Met Gly Asn Gln Ser Arg Ser Arg Asn Gly Arg
            100                 105                 110
Arg Arg Gly Glu Asp Met Met His Pro Leu Lys Val Ser Leu Glu Asp
        115                 120                 125
Leu Tyr Asn Gly Lys Thr Thr Lys Leu Gln Leu Ser Lys Asn Val Leu
    130                 135                 140
Cys Ser Ala Cys Ser Gly Gln Gly Gly Lys Ser Gly Ala Val Gln Lys

145                 150                 155                 160
Cys Ser Ala Cys Arg Gly Arg Gly Val Arg Ile Met Ile Arg Gln Leu
                165                 170                 175
Ala Pro Gly Met Val Gln Gln Met Gln Ser Val Cys Ser Asp Cys Asn
            180                 185                 190
Gly Glu Gly Glu Val Ile Asn Glu Lys Asp Arg Cys Lys Lys Cys Glu
        195                 200                 205
Gly Lys Lys Val Ile Lys Glu Val Lys Ile Leu Glu Val His Val Asp
    210                 215                 220
Lys Gly Met Lys His Gly Gln Arg Ile Thr Phe Thr Gly Glu Ala Asp
225                 230                 235                 240
Gln Ala Pro Glu Trp Asn Pro Glu Thr Leu Phe Phe Leu Leu Pro Gly
                245                 250                 255
Glu Lys Asn Met Glu Val Phe Gln Arg Asp Gly Asn Asp Leu His Met
            260                 265                 270
Thr Tyr Lys Ile Gly Leu Val Glu Ala Leu Cys Gly Phe Gln Phe Thr
        275                 280                 285
Leu Ser His Leu Asp Gly Arg Gln Ile Val Val Lys Tyr Pro Pro Gly
    290                 295                 300
Lys Val Ile Glu Pro Gly Cys Val Arg Val Val Arg Gly Glu Gly Met
305                 310                 315                 320
Pro Gln Tyr Arg Asn Pro Phe Glu Lys Gly Gly Leu Tyr Ile Lys Phe
                325                 330                 335
Asp Val Gln Phe Pro Glu Asn Asn Trp Ile Asn Pro Asp Lys Leu Ser
            340                 345                 350
Glu Leu Glu Asp Leu Leu Pro Ser Arg Pro Glu Val Pro Asn Ile Ile
        355                 360                 365
Gly Glu Thr Glu Glu Val Glu Leu Gln Glu Phe Asp Ser Thr Arg Gly
    370                 375                 380
Ser Gly Gly Gly Gln Arg Arg Glu Ala Tyr Asn Asp Ser Ser Asp Glu
385                 390                 395                 400
Glu Ser Ser Ser His His Gly Pro Gly Val Gln Cys Ala His Gln
                405                 410                 415

<210> 117
<211> 1821
<212> DNA
<213> Homo Sapiens

<400> 117
cgcgtgaact gcttcctgca ggctggccat ggcgcttcac gttcccaagg ctccgggctt      60
tgcccagatg ctcaaggagg gagcgaaaca cttttcagga ttagaagagg ctgtgtatag     120
aaacatacaa gcttgcaagg agcttgccca aaccactcgt acagcatatg gaccaaaagg     180
aatgaacaaa atggttatca accacttgga gaagttgttt gtgacaaacg atgcagcaac     240
tattttaaga gaactagaag tacagcatcc tgctgcaaaa atgattgtaa tggcttctca     300
tatgcaagag caagaagttg gagatggcac aaactttgtt ctggtatttg ctggagctct     360
cctggaatta gctgaagaac ttctgaggat tggcctgtca gtttcagagg tcatagaagg     420
ttatgaaata gcctgcagaa aagctcatga gattcttcct aatttggtat gttgttctgc     480
aaaaaacctt cgagatattg atgaagtctc atctctactt cgtacctcca taatgagtaa     540
acaatatggt aatgaagtat ttctggccaa gcttattgct caggcatgcg tatctatttt     600
tcctgattcc ggccatttca atgttgataa catcagagtt tgtaaaattc tgggctctgg     660
tatcagttcc tcttcagtat tgcatggcat ggtttttaag aaggaaaccg aaggtgatgt     720
aacatctgtc aaagatgcaa aaatagcagt gtactcttgt ccttttgatg gcatgataac     780
agaaactaag ggaacagtgt tgataaagac tgctgaagaa ttgatgaatt ttagtaaggg     840
agaagaaaac ctcatggatg cacaagtcaa agctattgct gatactggtg caaatgtcgt     900

agtaacaggt ggcaaagtgg cagacatggc tcttcattat gcaaataaat ataatatcat 960
gttagtgagg ctaaactcaa aatgggatct ccgaagactt tgtaaaactg ttggtgctac 1020
agctcttcct agattgacac ctcctgtcct tgaagaaatg ggacactgtg acagtgttta 1080
cctctcagaa gttggagata ctcaggtggt ggtttttaag catgaaaagg aagatggcgc 1140
catttctacc atagtacttc gaggctctac agacaatctg atggatgaca tagaaagggt 1200
agtagacgat ggtgttaata ctttcaaagt tcttacaagg gataaacgtc ttgtacccgg 1260
aggtggagca acagaaattg aattagccaa acagatcaca tcatatggag agacatgtcc 1320
tggacttgaa cagtatgcta ttaagaagtt tgctgaggca tttgaagcta ttccccgcgc 1380
actggcagaa aactctggag ttaaggccaa tgaagtaatc tctaaacttt atgcagtaca 1440
tcaagaagga aataaaaacg ttggattaga tattgaggct gaagtccctg ctgtaaagga 1500
catgctggaa gctggtattc tagatactta cctgggaaaa tattgggcta tcaaactcgc 1560
tactaatgct gcagtcactg tacttagagt ggatcagatc atcatggcaa aaccagctgg 1620
tgggcccaag cctccaagtg ggaagaaaga ctgggatgat gaccaaaatg attgaaattg 1680
gcttaatttt tactgtaggt gaaggctgta tttgtagtag tactcaagaa tcacctgatg 1740
ttttcttatt ctccttaaat taagagttat tttgtgtttg tattcttggc tggatgttat 1800
aataaacata ttgttactgt c 1821

<210> 118
<211> 548
<212> PRT
<213> Homo Sapiens

<400> 118
Met Ala Leu His Val Pro Lys Ala Pro Gly Phe Ala Gln Met Leu Lys
1               5                   10                  15
Glu Gly Ala Lys His Phe Ser Gly Leu Glu Glu Ala Val Tyr Arg Asn
            20                  25                  30
Ile Gln Ala Cys Lys Glu Leu Ala Gln Thr Thr Arg Thr Ala Tyr Gly
        35                  40                  45
Pro Lys Gly Met Asn Lys Met Val Ile Asn His Leu Glu Lys Leu Phe
    50                  55                  60
Val Thr Asn Asp Ala Ala Thr Ile Leu Arg Glu Leu Glu Val Gln His
65                  70                  75                  80
Pro Ala Ala Lys Met Ile Val Met Ala Ser His Met Gln Glu Gln Glu
                85                  90                  95
Val Gly Asp Gly Thr Asn Phe Val Leu Val Phe Ala Gly Ala Leu Leu
            100                 105                 110
Glu Leu Ala Glu Glu Leu Leu Arg Ile Gly Leu Ser Val Ser Glu Val
        115                 120                 125
Ile Glu Gly Tyr Glu Ile Ala Cys Arg Lys Ala His Glu Ile Leu Pro
    130                 135                 140
Asn Leu Val Cys Cys Ser Ala Lys Asn Leu Arg Asp Ile Asp Glu Val
145                 150                 155                 160
Ser Ser Leu Leu Arg Thr Ser Ile Met Ser Lys Gln Tyr Gly Asn Glu
                165                 170                 175
Val Phe Leu Ala Lys Leu Ile Ala Gln Ala Cys Val Ser Ile Phe Pro
            180                 185                 190
Asp Ser Gly His Phe Asn Val Asp Asn Ile Arg Val Cys Lys Ile Leu
        195                 200                 205
Gly Ser Gly Ile Ser Ser Ser Ser Val Leu His Gly Met Val Phe Lys
    210                 215                 220
Lys Glu Thr Glu Gly Asp Val Thr Ser Val Lys Asp Ala Lys Ile Ala
225                 230                 235                 240
Val Tyr Ser Cys Pro Phe Asp Gly Met Ile Thr Glu Thr Lys Gly Thr
                245                 250                 255

Val Leu Ile Lys Thr Ala Glu Glu Leu Met Asn Phe Ser Lys Gly Glu
            260                 265                 270
Glu Asn Leu Met Asp Ala Gln Val Lys Ala Ile Ala Asp Thr Gly Ala
        275                 280                 285
Asn Val Val Val Thr Gly Gly Lys Val Ala Asp Met Ala Leu His Tyr
    290                 295                 300
Ala Asn Lys Tyr Asn Ile Met Leu Val Arg Leu Asn Ser Lys Trp Asp
305                 310                 315                 320
Leu Arg Arg Leu Cys Lys Thr Val Gly Ala Thr Ala Leu Pro Arg Leu
                325                 330                 335
Thr Pro Pro Val Leu Glu Glu Met Gly His Cys Asp Ser Val Tyr Leu
            340                 345                 350
Ser Glu Val Gly Asp Thr Gln Val Val Val Phe Lys His Glu Lys Glu
        355                 360                 365
Asp Gly Ala Ile Ser Thr Ile Val Leu Arg Gly Ser Thr Asp Asn Leu
    370                 375                 380
Met Asp Asp Ile Glu Arg Val Val Asp Asp Gly Val Asn Thr Phe Lys
385                 390                 395                 400
Val Leu Thr Arg Asp Lys Arg Leu Val Pro Gly Gly Gly Ala Thr Glu
                405                 410                 415
Ile Glu Leu Ala Lys Gln Ile Thr Ser Tyr Gly Glu Thr Cys Pro Gly
            420                 425                 430
Leu Glu Gln Tyr Ala Ile Lys Lys Phe Ala Glu Ala Phe Glu Ala Ile
        435                 440                 445
Pro Arg Ala Leu Ala Glu Asn Ser Gly Val Lys Ala Asn Glu Val Ile
    450                 455                 460
Ser Lys Leu Tyr Ala Val His Gln Glu Gly Asn Lys Asn Val Gly Leu
465                 470                 475                 480
Asp Ile Glu Ala Glu Val Pro Ala Val Lys Asp Met Leu Glu Ala Gly
                485                 490                 495
Ile Leu Asp Thr Tyr Leu Gly Lys Tyr Trp Ala Ile Lys Leu Ala Thr
            500                 505                 510
Asn Ala Ala Val Thr Val Leu Arg Val Asp Gln Ile Ile Met Ala Lys
        515                 520                 525
Pro Ala Gly Gly Pro Lys Pro Pro Ser Gly Lys Lys Asp Trp Asp Asp
    530                 535                 540
Asp Gln Asn Asp
545

<210> 119
<211> 1321
<212> DNA
<213> Homo Sapiens

<400> 119
cccccaagat ggctgctgag gacgagttac agctgccgcg gctccccgag ctgttcgaaa      60
ctggtagaca gttactggac gaagtagaag tggcgactga acccgccggt tcccggatag     120
tccaggagaa ggtgttcaag ggcttggacc tccttgagaa ggctgccgaa atgttatcgc     180
agctcgactt gttcagccga aatgaagatt tggaagagat tgcttccacc gacctgaagt     240
accttttggt gccagcgttt caaggagccc tcaccatgaa acaagtcaac cccagcaagc     300
gtctagatca tttgcagcgg gctcgagaac actttataaa ctacttaact cagtgccatt     360
gctatcatgt ggcagagttt gagctgccca aaaccatgaa caactctgct gaaaatcaca     420
ctgccaattc ctccatggct tatcctagtc tcgttgctat ggcatctcaa agacaggcta     480
aaatacagag atacaagcag aagaaggagt tggagcatag gttgtctgca atgaaatctg     540
ctgtggaaag tggtcaagca gatgatgagc gtgttcgtga atattatctt cttcaccttc     600

agaggtggat tgatatcagc ttagaagaga ttgagagcat tgaccaggaa ataaagatcc 660
tgagagaaag agactcttca agagaggcat caacttctaa ctcatctcgc caggagaggc 720
ctccagtgaa acccttcatt ctcactcgga acatggctca agccaaagta tttggagctg 780
gttatccaag tctgccaact atgacggtga gtgactggta tgagcaacat cggaaatatg 840
gagcattacc ggatcaggga atagccaagg cagcaccaga ggaattcaga aaagcagctc 900
agcaacagga agaacaagaa gaaaaggagg aagaggatga tgaacaaaca ctccacagag 960
cccgggagtg ggatgactgg aaggacaccc atcctagggg ctatgggaac cgacagaaca 1020
tgggctgatc ttcccacaac accacaggac tgcagggtgc acaactccct gccaaggaaa 1080
accatgcagt cctcccctcc ctggtctcct gcttcagctc tgtacaacga gggcaaagat 1140
gctaaatctt gctttgcatt cagtaaagtg tcaagtgatt aagtgtgtat ttgtacccta 1200
gatgatatga accagcagtc ttgttttggc atcatcctca tcatgttgta ttccagcttc 1260
ttaagtggaa ggaaaagagt gctgagaaat ggctctgtat aatctatggc tatccgaatt 1320
c 1321

<210> 120
<211> 339
<212> PRT
<213> Homo Sapiens

<400> 120
Met Ala Ala Glu Asp Glu Leu Gln Leu Pro Arg Leu Pro Glu Leu Phe
1               5                   10                  15
Glu Thr Gly Arg Gln Leu Leu Asp Glu Val Glu Val Ala Thr Glu Pro
            20                  25                  30
Ala Gly Ser Arg Ile Val Gln Glu Lys Val Phe Lys Gly Leu Asp Leu
        35                  40                  45
Leu Glu Lys Ala Ala Glu Met Leu Ser Gln Leu Asp Leu Phe Ser Arg
    50                  55                  60
Asn Glu Asp Leu Glu Glu Ile Ala Ser Thr Asp Leu Lys Tyr Leu Leu
65                  70                  75                  80
Val Pro Ala Phe Gln Gly Ala Leu Thr Met Lys Gln Val Asn Pro Ser
                85                  90                  95
Lys Arg Leu Asp His Leu Gln Arg Ala Arg Glu His Phe Ile Asn Tyr
            100                 105                 110
Leu Thr Gln Cys His Cys Tyr His Val Ala Glu Phe Glu Leu Pro Lys
        115                 120                 125
Thr Met Asn Asn Ser Ala Glu Asn His Thr Ala Asn Ser Ser Met Ala
    130                 135                 140
Tyr Pro Ser Leu Val Ala Met Ala Ser Gln Arg Gln Ala Lys Ile Gln
145                 150                 155                 160
Arg Tyr Lys Gln Lys Lys Glu Leu Glu His Arg Leu Ser Ala Met Lys
                165                 170                 175
Ser Ala Val Glu Ser Gly Gln Ala Asp Asp Glu Arg Val Arg Glu Tyr
            180                 185                 190
Tyr Leu Leu His Leu Gln Arg Trp Ile Asp Ile Ser Leu Glu Glu Ile
        195                 200                 205
Glu Ser Ile Asp Gln Glu Ile Lys Ile Leu Arg Glu Arg Asp Ser Ser
    210                 215                 220
Arg Glu Ala Ser Thr Ser Asn Ser Ser Arg Gln Glu Arg Pro Pro Val
225                 230                 235                 240
Lys Pro Phe Ile Leu Thr Arg Asn Met Ala Gln Ala Lys Val Phe Gly
                245                 250                 255
Ala Gly Tyr Pro Ser Leu Pro Thr Met Thr Val Ser Asp Trp Tyr Glu
            260                 265                 270
Gln His Arg Lys Tyr Gly Ala Leu Pro Asp Gln Gly Ile Ala Lys Ala

        275                 280                 285
Ala Pro Glu Glu Phe Arg Lys Ala Ala Gln Gln Gln Glu Glu Gln Glu
    290                 295                 300
Glu Lys Glu Glu Glu Asp Asp Glu Gln Thr Leu His Arg Ala Arg Glu
305                 310                 315                 320
Trp Asp Asp Trp Lys Asp Thr His Pro Arg Gly Tyr Gly Asn Arg Gln
                325                 330                 335
Asn Met Gly

<210> 121
<211> 2965
<212> DNA
<213> Homo Sapiens

<400> 121
gcggaggtcg gcggtcgggt ccgtctctgc ccgcggctgt ggcggcgccg gcggatccag 60
ccttagcgtt cctctctggg cggcggcggc ggcggctcgg ttgacgcctc ctccgccagc 120
tgagcccgcg ggagcccagg acgccgcttc cccgcccatc cccgctcccc gaggccggcc 180
gcctggtcat ggcgcagccg ggcccggctt cccagcctga cgtttctctt cagcaacggg 240
tagcagaatt ggaaaaaatt aatgcagaat ttttacgtgc acaacagcag cttgaacaag 300
aatttaatca aaagagagca aaatttaagg agttatattt ggctaaagag gaggatctga 360
agaggcaaaa tgcagtatta caagctgcac aagatgattt gggacacctt cgaacccagc 420
tgtgggaagc tcaagcagag atggagaata ttaaggcgat tgccacagtc tctgagaaca 480
ccaagcaaga agctatagat gaagtgaaaa gacagtggag agaagaagtt gcttcacttc 540
aggctgttat gaaagaaaca gttcgtgact atgagcacca gttccacctt aggctggagc 600
aggagcgaac acagtgggca cagtatagag aatacgcaga gagggaaata gctgatttaa 660
gaagaaggct gtctgaaggt caagaggagg aaaatttaga aaatgaaatg aaaaaggccc 720
aagaggatgc tgagaaactt cggtccgttg tgatgccaat ggaaaaggaa attgcagctt 780
tgaaggataa actgacagag gctgaagaca aaattaaaga gctggaggcc tcaaaggtta 840
aagaactgaa tcattatctg gaagctgaga aatcttgtag gactgatcta gagatgtatg 900
tagctgtttt gaatactcag aaatctgttc tacaggaaga tgctgagaaa ctgcggaaag 960
aattgcatga agtttgccat ctcttggagc aagagcgaca acaacacaac cagttaaaac 1020
atacgtggca gaaggccaat gaccagtttc tggaatctca gcgtttactg atgagagaca 1080
tgcagcgaat ggagattgtg ctaacttcag aacagctccg acaagttgaa gaactgaaga 1140
agaaagatca ggaggatgat gaacaacaaa gactcaataa gagaaaggat cacaaaaaag 1200
cagatgttga ggaagaaata aaaataccag tagtgtgtgc tttaactcaa gaagaatctt 1260
cagcccagtt atcaaatgaa gaggagcatt tagacagcac ccgtggctca gttcattcct 1320
tagatgcagg cttgctgttg ccatctggag atcctttcag taaatcggac aatgacatgt 1380
ttaaagatgg actcaggaga gcacagtcta cagacagctt gggaacctcg ggctcattgc 1440
aatccaaagc tttaggctat aactacaaag caaaatctgc tggaaacctg gacgagtcag 1500
attttggacc actggtagga gcagattcag tgtctgagaa ctttgatact gcatcccttg 1560
ggtcactcca gatgccaagt gggtttatgt taaccaaaga tcaggaaaga gcaatcaagg 1620
cgatgacacc agaacaagaa gagacagcgt ccctcctctc cagcgttacc cagggcatgg 1680
agagtgccta tgtgtcccct agtggttatc gtttagttag tgaaacagaa tggaatctct 1740
tgcagaaaga ggtacataat gctggaaata aacttggtag acgttgtgat atgtgttcca 1800
attacgaaaa acagttacaa ggaattcaga ttcaggaggc tgaaacgaga gaccaggtga 1860
aaaaactaca gctgatgcta aggcaagcta atgaccagtt agagaagaca atgaaagata 1920
agcaggagct ggaagacttc ataaagcaaa gcagcgaaga ttcgagtcac cagatctctg 1980
cactcgtcct aagagcccag gcctccgaga tcttacttga agagttacag caggggcttt 2040
cccaggcaaa gagggatgtt caggaacaga tggcggtgct gatgcagtca cgggaacagg 2100
tttcagaaga gctggtgagg ttacagaaag ataatgacag tctccaggga aagcacagcc 2160
tgcatgtgtc attacagcaa gcagaagact tcatcctccc agacactaca gaggcactgc 2220
gggagttggt attaaaatac cgtgaggaca tcattaatgt gcggacagca gcagaccacg 2280
tagaagaaaa gctgaaggct gagatacttt tcctaaaaga gcagatccaa gcagaacagt 2340

gtttaaaaga aaatcttgaa gaaactctgc aactagaaat agaaaactgc aaggaggaaa 2400
tagcttctat ttctagccta aaagctgaat tagaaagaat aaaagtggaa aaaggacagt 2460
tggagtccac attaagagag aagtctcaac agcttgagag tcttcaggaa ataaagatca 2520
gtttggaaga gcagttaaag aaagagactg ctgctaaggc taccgttgaa cagctaatgt 2580
ttgaagagaa gaacaaagct cagagattac agacagaatt agatgtcagt gagcaagtcc 2640
agagagattt tgtaaagctt tcacagaccc ttcaggtgca gttagagcgg atccggcaag 2700
ctgactcctt ggagagaatc cgggcaattc tgaatgatac taaactgaca gacattaacc 2760
agcttcctga gacatgacac cctcatggca ggattctagc ctgcactttg ggtttttaac 2820
tcatctttag agcaacagta attattattt aactcttaac tgaagaaaga gaagtcacaa 2880
caaaaggaag actggagaaa tgcttacttc tagagggaga agactgtgcg gcacaggaaa 2940
cagcaaacag tggggtgatc tgcag 2965

<210> 122
<211> 862
<212> PRT
<213> Homo Sapiens

<400> 122
Met Ala Gln Pro Gly Pro Ala Ser Gln Pro Asp Val Ser Leu Gln Gln
1               5                   10                  15
Arg Val Ala Glu Leu Glu Lys Ile Asn Ala Glu Phe Leu Arg Ala Gln
            20                  25                  30
Gln Gln Leu Glu Gln Glu Phe Asn Gln Lys Arg Ala Lys Phe Lys Glu
        35                  40                  45
Leu Tyr Leu Ala Lys Glu Glu Asp Leu Lys Arg Gln Asn Ala Val Leu
    50                  55                  60
Gln Ala Ala Gln Asp Asp Leu Gly His Leu Arg Thr Gln Leu Trp Glu
65                  70                  75                  80
Ala Gln Ala Glu Met Glu Asn Ile Lys Ala Ile Ala Thr Val Ser Glu
                85                  90                  95
Asn Thr Lys Gln Glu Ala Ile Asp Glu Val Lys Arg Gln Trp Arg Glu
            100                 105                 110
Glu Val Ala Ser Leu Gln Ala Val Met Lys Glu Thr Val Arg Asp Tyr
        115                 120                 125
Glu His Gln Phe His Leu Arg Leu Glu Gln Glu Arg Thr Gln Trp Ala
    130                 135                 140
Gln Tyr Arg Glu Tyr Ala Glu Arg Glu Ile Ala Asp Leu Arg Arg Arg
145                 150                 155                 160
Leu Ser Glu Gly Gln Glu Glu Glu Asn Leu Glu Asn Glu Met Lys Lys
                165                 170                 175
Ala Gln Glu Asp Ala Glu Lys Leu Arg Ser Val Val Met Pro Met Glu
            180                 185                 190
Lys Glu Ile Ala Ala Leu Lys Asp Lys Leu Thr Glu Ala Glu Asp Lys
        195                 200                 205
Ile Lys Glu Leu Glu Ala Ser Lys Val Lys Glu Leu Asn His Tyr Leu
    210                 215                 220
Glu Ala Glu Lys Ser Cys Arg Thr Asp Leu Glu Met Tyr Val Ala Val
225                 230                 235                 240
Leu Asn Thr Gln Lys Ser Val Leu Gln Glu Asp Ala Glu Lys Leu Arg
                245                 250                 255
Lys Glu Leu His Glu Val Cys His Leu Leu Glu Gln Glu Arg Gln Gln
            260                 265                 270
His Asn Gln Leu Lys His Thr Trp Gln Lys Ala Asn Asp Gln Phe Leu
        275                 280                 285
Glu Ser Gln Arg Leu Leu Met Arg Asp Met Gln Arg Met Glu Ile Val

290                 295                 300
Leu Thr Ser Glu Gln Leu Arg Gln Val Glu Glu Leu Lys Lys Lys Asp
305                 310                 315                 320
Gln Glu Asp Asp Glu Gln Gln Arg Leu Asn Lys Arg Lys Asp His Lys
                325                 330                 335
Lys Ala Asp Val Glu Glu Glu Ile Lys Ile Pro Val Val Cys Ala Leu
            340                 345                 350
Thr Gln Glu Glu Ser Ser Ala Gln Leu Ser Asn Glu Glu Glu His Leu
        355                 360                 365
Asp Ser Thr Arg Gly Ser Val His Ser Leu Asp Ala Gly Leu Leu Leu
    370                 375                 380
Pro Ser Gly Asp Pro Phe Ser Lys Ser Asp Asn Asp Met Phe Lys Asp
385                 390                 395                 400
Gly Leu Arg Arg Ala Gln Ser Thr Asp Ser Leu Gly Thr Ser Gly Ser
                405                 410                 415
Leu Gln Ser Lys Ala Leu Gly Tyr Asn Tyr Lys Ala Lys Ser Ala Gly
            420                 425                 430
Asn Leu Asp Glu Ser Asp Phe Gly Pro Leu Val Gly Ala Asp Ser Val
        435                 440                 445
Ser Glu Asn Phe Asp Thr Ala Ser Leu Gly Ser Leu Gln Met Pro Ser
    450                 455                 460
Gly Phe Met Leu Thr Lys Asp Gln Glu Arg Ala Ile Lys Ala Met Thr
465                 470                 475                 480
Pro Glu Gln Glu Glu Thr Ala Ser Leu Leu Ser Ser Val Thr Gln Gly
                485                 490                 495
Met Glu Ser Ala Tyr Val Ser Pro Ser Gly Tyr Arg Leu Val Ser Glu
            500                 505                 510
Thr Glu Trp Asn Leu Leu Gln Lys Glu Val His Asn Ala Gly Asn Lys
        515                 520                 525
Leu Gly Arg Arg Cys Asp Met Cys Ser Asn Tyr Glu Lys Gln Leu Gln
    530                 535                 540
Gly Ile Gln Ile Gln Glu Ala Glu Thr Arg Asp Gln Val Lys Lys Leu
545                 550                 555                 560
Gln Leu Met Leu Arg Gln Ala Asn Asp Gln Leu Glu Lys Thr Met Lys
                565                 570                 575
Asp Lys Gln Glu Leu Glu Asp Phe Ile Lys Gln Ser Ser Glu Asp Ser
            580                 585                 590
Ser His Gln Ile Ser Ala Leu Val Leu Arg Ala Gln Ala Ser Glu Ile
        595                 600                 605
Leu Leu Glu Glu Leu Gln Gln Gly Leu Ser Gln Ala Lys Arg Asp Val
    610                 615                 620
Gln Glu Gln Met Ala Val Leu Met Gln Ser Arg Glu Gln Val Ser Glu
625                 630                 635                 640
Glu Leu Val Arg Leu Gln Lys Asp Asn Asp Ser Leu Gln Gly Lys His
                645                 650                 655
Ser Leu His Val Ser Leu Gln Gln Ala Glu Asp Phe Ile Leu Pro Asp
            660                 665                 670
Thr Thr Glu Ala Leu Arg Glu Leu Val Leu Lys Tyr Arg Glu Asp Ile
        675                 680                 685
Ile Asn Val Arg Thr Ala Ala Asp His Val Glu Glu Lys Leu Lys Ala
    690                 695                 700
Glu Ile Leu Phe Leu Lys Glu Gln Ile Gln Ala Glu Gln Cys Leu Lys
705                 710                 715                 720
Glu Asn Leu Glu Glu Thr Leu Gln Leu Glu Ile Glu Asn Cys Lys Glu
                725                 730                 735

Glu Ile Ala Ser Ile Ser Ser Leu Lys Ala Glu Leu Glu Arg Ile Lys
            740                 745                 750
Val Glu Lys Gly Gln Leu Glu Ser Thr Leu Arg Glu Lys Ser Gln Gln
        755                 760                 765
Leu Glu Ser Leu Gln Glu Ile Lys Ile Ser Leu Glu Glu Gln Leu Lys
    770                 775                 780
Lys Glu Thr Ala Ala Lys Ala Thr Val Glu Gln Leu Met Phe Glu Glu
785                 790                 795                 800
Lys Asn Lys Ala Gln Arg Leu Gln Thr Glu Leu Asp Val Ser Glu Gln
                805                 810                 815
Val Gln Arg Asp Phe Val Lys Leu Ser Gln Thr Leu Gln Val Gln Leu
            820                 825                 830
Glu Arg Ile Arg Gln Ala Asp Ser Leu Glu Arg Ile Arg Ala Ile Leu
        835                 840                 845
Asn Asp Thr Lys Leu Thr Asp Ile Asn Gln Leu Pro Glu Thr
    850                 855                 860

<210> 123
<211> 544
<212> DNA
<213> Homo Sapiens

<400> 123
gggagtggcg tggcgcaggg atggcacaaa agaaatatct tcaagcaaaa ttgacccagt      60
ttttaaggga agacaggatt caactttgga aacctccata tacagatgaa aataaaaaag      120
ttggtttggc attaaaggac cttgctaagc agtactctga cagactagaa tgctgtgaaa      180
atgaagtaga aaaggtaata gaagaaatac gttgcaaggc aattgagcgt ggaacaggaa      240
atgacaatta tagaacaacg ggaattgcta caatcgaggt gtttttacca ccaagactaa      300
aaaaagatag gaaaaacttg ttggagaccc gattgcacat cactggcaga gaactgaggt      360
ccaaaatagc tgaaaccttt ggacttcaag aanattatat caaaattgtc ataaataaga      420
agcaactacn actagggaaa acccttgaag ancaaggcgt ggctcacaat gtgaaagcga      480
tggtgcttga actaaaacaa tctgaagagg acgcgaggaa aaacttccag ttagaggaag      540
agga                                                                  544

<210> 124
<211> 178
<212> PRT
<213> Homo Sapiens

<400> 124
Glu Trp Arg Gly Ala Gly Met Ala Gln Lys Lys Tyr Leu Gln Ala Lys
1               5                   10                  15
Leu Thr Gln Phe Leu Arg Glu Asp Arg Ile Gln Leu Trp Lys Pro Pro
            20                  25                  30
Tyr Thr Asp Glu Asn Lys Lys Val Gly Leu Ala Leu Lys Asp Leu Ala
        35                  40                  45
Lys Gln Tyr Ser Asp Arg Leu Glu Cys Cys Glu Asn Glu Val Glu Lys
    50                  55                  60
Val Ile Glu Glu Ile Arg Cys Lys Ala Ile Glu Arg Gly Thr Gly Asn
65                  70                  75                  80
Asp Asn Tyr Arg Thr Thr Gly Ile Ala Thr Ile Glu Val Phe Leu Pro
                85                  90                  95
Pro Arg Leu Lys Lys Asp Arg Lys Asn Leu Leu Glu Thr Arg Leu His
            100                 105                 110
Ile Thr Gly Arg Glu Leu Arg Ser Lys Ile Ala Glu Thr Phe Gly Leu

        115                 120                 125
Gln Glu Tyr Ile Lys Ile Val Ile Asn Lys Lys Gln Leu Leu Gly Lys
    130                 135                 140
Thr Leu Glu Gln Gly Val Ala His Asn Val Lys Ala Met Val Leu Glu
145                 150                 155                 160
Leu Lys Gln Ser Glu Glu Asp Ala Arg Lys Asn Phe Gln Leu Glu Glu
                165                 170                 175
Glu Glu

<210> 125
<211> 1302
<212> DNA
<213> Homo Sapiens

<400> 125
atggaggtgg tggacccgca gcagctgggc atgttcacgg agggcgagct gatgtcggtg 60
ggtatggaca cgttcatcca ccgcatcgac tccaccgagg tcatctacca gccgcgccgc 120
aagcgggcca agctcatcgg caagtacctg atgggggacc tgctggggga aggctcttac 180
ggcaaggtga aggaggtgct ggactcggag acgctgtgca ggagggccgt caagatcctc 240
aagaagaaga agttgcgaag gatccccaac ggggaggcca acgtgaagaa ggaaattcaa 300
ctactgagga ggttacggca caaaaatgtc atccagctgg tggatgtgtt atacaacgaa 360
gagaagcaga aaatgtatat ggtgatggag tactgcgtgt gtggcatgca ggaaatgctg 420
gacagcgtgc cggagaagcg tttcccagtg tgccaggccc acgggtactt ctgtcagctg 480
attgacggcc tggagtacct gcatagccag ggcattgtgc acaaggacat caagccgggg 540
aacctgctgc tcaccaccgg tggcaccctc aaaatctccg acctgggcgt ggccgaggca 600
ctgcacccgt tcgcggcgga cgacacctgc cggaccagcc agggctcccc ggctttccag 660
ccgcccgaga ttgccaacgg cctggacacc ttctccggct tcaaggtgga catctggtcg 720
gctggggtca ccctctacaa catcaccacg ggtctgtacc ccttcgaagg ggacaacatc 780
tacaagttgt ttgagaacat cgggaagggg agctacgcca tcccgggcga ctgtggcccc 840
ccgctctctg acctgctgaa agggatgctt gagtacgaac cggccaagag gttctccatc 900
cggcagatcc ggcagcacag ctggttccgg aagaaacatc ctccggctga agcaccagtg 960
cccatcccac cgagcccaga caccaaggac cggtggcgca gcatgactgt ggtgccgtac 1020
ttggaggacc tgcacggcgc ggacgaggac gaggacctct tcgacatcga ggatgacatc 1080
atctacactc aggacttcac ggtgcccgga caggtcccag aagaggaggc cagtcacaat 1140
ggacagcgcc ggggcctccc caaggccgtg tgtatgaacg gcacagaggc ggcgcagctg 1200
agcaccaaat ccagggcgga gggccgggcc cccaaccctg cccgcaaggc ctgctccgcc 1260
agcagcaaga tccgccggct gtcggcctgc aagcagcagt ga 1302

<210> 126
<211> 433
<212> PRT
<213> Homo Sapiens

<400> 126

<210> 127
<211> 1488
<212> DNA
<213> Homo Sapiens

<400> 127
gaggggcggg gcggtgccgg caagatggct gcgcccgaga agatgacgtt tcccgagaaa      60
ccaagccaca aaaagtacag ggccgccctg aagaaggaga aacgaaagaa acgtcggcag     120

gaacttgctc gactgagaga ctcaggactc tcacagaagg aggaagagga ggacactttt 180
attgaagaac aacaactaga agaagagaag ctattggaaa gagagaggca aagattacat 240
gaggagtggt tgctaagaga gcagaaggca caagaagaat tcagaataaa gaaggaaaag 300
gaagaggcgg ctaaaaaacg gcaagaagaa caagagagaa agttaaagga acaatgggaa 360
gaacagcaga ggaaagagag agaagaggag gagcagaaac gacaggagaa gaaagaaaaa 420
gaggaagctt tgcagaagat gctggatcag gctgaaaatg agttggaaaa tggtaccaca 480
tggcaaaacc cagaaccacc cgtggatttc agagtaatgg agaaggatcg agctaattgt 540
cccttctaca gtaaaacagg agcttgcaga tttggagata gatgttcacg taaacataat 600
ttcccaacat ccagtcctac ccttcttatt aagagcatgt ttacgacgtt tggaatggag 660
cagtgcagga gggatgacta tgaccctgac gcaagcctgg agtacagcga ggaagaaacc 720
taccaacagt tcctagactt ctatgaggat gtgttgcccg agttcaagaa cgtggggaaa 780
gtgattcagt tcaaggtcag ctgcaatttg gaacctcacc tgaggggcaa tgtatatgtt 840
cagtaccagt cggaagaaga atgccaagca gccctttctc tgtttaacgg acgatggtat 900
gcaggacgac agctgcagtg tgaattctgc cccgtgaccc ggtggaaaat ggcgatttgt 960
ggtttatttg aaatacaaca atgtccaaga ggaaagcact gcaactttct tcatgtgttc 1020
agaaatccca acaatgaatt ctgggaagct aatagagaca tctacttgtc tccagatcgg 1080
actggctcct cctttgggaa gaactccgaa aggagggaga ggatggccca ccacgacgac 1140
tactacagca ggctgcgggg aaggagaaac cctagtccag accactccta caaaagaaat 1200
ggggaatccg agaggaaaag tagtcgtcac agggggaaga aatctcacaa acgcacatca 1260
aagagtcggg agaggcacaa ttcacgaagc agaggaagaa atagggaccg cagcagggac 1320
cgcagccggg gccggggcag ccggagccgg agccggagcc ggagccgcag gagccgccgc 1380
agccggagcc aaagttcctc taggtcccga agtcgtggca ggaggaggtc gggtaataga 1440
gacagaactg ttcagagtcc caaatccaaa taaactagtt ttgttctt 1488

<210> 128
<211> 482
<212> PRT
<213> Homo Sapiens

<400> 128
Met Ala Ala Pro Glu Lys Met Thr Phe Pro Glu Lys Pro Ser His Lys
1               5                   10                  15
Lys Tyr Arg Ala Ala Leu Lys Lys Glu Lys Arg Lys Lys Arg Arg Gln
            20                  25                  30
Glu Leu Ala Arg Leu Arg Asp Ser Gly Leu Ser Gln Lys Glu Glu Glu
        35                  40                  45
Glu Asp Thr Phe Ile Glu Glu Gln Gln Leu Glu Glu Glu Lys Leu Leu
    50                  55                  60
Glu Arg Glu Arg Gln Arg Leu His Glu Glu Trp Leu Leu Arg Glu Gln
65                  70                  75                  80
Lys Ala Gln Glu Glu Phe Arg Ile Lys Lys Glu Lys Glu Glu Ala Ala
                85                  90                  95
Lys Lys Arg Gln Glu Glu Gln Glu Arg Lys Leu Lys Glu Gln Trp Glu
            100                 105                 110
Glu Gln Gln Arg Lys Glu Arg Glu Glu Glu Glu Gln Lys Arg Gln Glu
        115                 120                 125
Lys Lys Glu Lys Glu Glu Ala Leu Gln Lys Met Leu Asp Gln Ala Glu
    130                 135                 140
Asn Glu Leu Glu Asn Gly Thr Thr Trp Gln Asn Pro Glu Pro Pro Val
145                 150                 155                 160
Asp Phe Arg Val Met Glu Lys Asp Arg Ala Asn Cys Pro Phe Tyr Ser
                165                 170                 175
Lys Thr Gly Ala Cys Arg Phe Gly Asp Arg Cys Ser Arg Lys His Asn
            180                 185                 190
Phe Pro Thr Ser Ser Pro Thr Leu Leu Ile Lys Ser Met Phe Thr Thr

195                 200                 205
Phe Gly Met Glu Gln Cys Arg Arg Asp Asp Tyr Asp Pro Asp Ala Ser
    210                 215                 220
Leu Glu Tyr Ser Glu Glu Glu Thr Tyr Gln Gln Phe Leu Asp Phe Tyr
225                 230                 235                 240
Glu Asp Val Leu Pro Glu Phe Lys Asn Val Gly Lys Val Ile Gln Phe
                245                 250                 255
Lys Val Ser Cys Asn Leu Glu Pro His Leu Arg Gly Asn Val Tyr Val
            260                 265                 270
Gln Tyr Gln Ser Glu Glu Glu Cys Gln Ala Ala Leu Ser Leu Phe Asn
        275                 280                 285
Gly Arg Trp Tyr Ala Gly Arg Gln Leu Gln Cys Glu Phe Cys Pro Val
    290                 295                 300
Thr Arg Trp Lys Met Ala Ile Cys Gly Leu Phe Glu Ile Gln Gln Cys
305                 310                 315                 320
Pro Arg Gly Lys His Cys Asn Phe Leu His Val Phe Arg Asn Pro Asn
                325                 330                 335
Asn Glu Phe Trp Glu Ala Asn Arg Asp Ile Tyr Leu Ser Pro Asp Arg
            340                 345                 350
Thr Gly Ser Ser Phe Gly Lys Asn Ser Glu Arg Arg Glu Arg Met Gly
        355                 360                 365
His His Asp Asp Tyr Tyr Ser Arg Leu Arg Gly Arg Arg Asn Pro Ser
    370                 375                 380
Pro Asp His Ser Tyr Lys Arg Asn Gly Glu Ser Glu Arg Lys Ser Ser
385                 390                 395                 400
Arg His Arg Gly Lys Lys Ser His Lys Arg Thr Ser Lys Ser Arg Glu
                405                 410                 415
Arg His Asn Ser Arg Ser Arg Gly Arg Asn Arg Asp Arg Ser Arg Asp
            420                 425                 430
Arg Ser Arg Gly Arg Gly Ser Arg Ser Arg Ser Arg Ser Arg Ser Arg
        435                 440                 445
Arg Ser Arg Arg Ser Arg Ser Gln Ser Ser Ser Arg Ser Arg Ser Arg
    450                 455                 460
Gly Arg Arg Arg Ser Gly Asn Arg Asp Arg Thr Val Gln Ser Pro Lys
465                 470                 475                 480
Ser Lys

<210> 129
<211> 1663
<212> DNA
<213> Homo Sapiens

<400> 129
aggccctgag ccaactccgg gtgctctgct gtgagtggct gaggcccgag atccacacca      60
aggagcagat cctggagcta ctggtgctgg agcagttcct gaccatcctg ccccaggagc     120
tccaggcctg ggtgcaggag cattgcccgg agagcgctga agaggctgtc actctcctcg     180
aagatctgga gcgggaactg gatgagccag gacaccaggt ctcaactcct ccaaacgaac     240
agaaaccggt gtgggagaag atatcctcct caggaactgc aaaggaatcc ccgagcagca     300
tgcagccaca gcccttggag accagtcaca aatacgagtc ttgggggccc ctgtacatcc     360
aagagtctgg tgaggagcag gagttcgctc aagatccaag aaaggtccga gattgcagat     420
tgagtaccca gcacgaggaa tcagcagatg agcagaaagg ttctgaagca gaggggctca     480
aaggggatat aatttctgtg attatcgcca ataaacctga ggccagctta gagaggcagt     540
gcgtaaacct tgaaaatgaa aaaggaacaa aaccccctct tcaagaggca ggctccaaga     600
aaggtagaga atcagttcct actaaaccta ccccaggaga gagacgttat atatgtgctg     660

aatgtggcaa agcctttagt aatagctcaa atctcaccaa acacaggaga acacacactg 720
gggagaaacc ttacgtgtgc accaagtgtg ggaaagcttt cagccacagc tcaaacctca 780
ccctccacta cagaacacac ttggtggacc ggccctatga ctgtaagtgt ggaaaagctt 840
ttgggcagag ctcagacctt cttaaacatc agagaatgca cacagaagag gcgccatatc 900
agtgcaaaga ttgtggcaag gctttcagcg ggaaaggcag cctcattcgt cactatcgga 960
tccacactgg ggagaagcct tatcagtgta acgaatgtgg gaagagcttc agtcagcatg 1020
cgggcctcag ctcccaccag agactccaca ccggagagaa gccatataag tgtaaggagt 1080
gtgggaaagc cttcaaccac agctccaact tcaataaaca ccacagaatc cacaccgggg 1140
aaaagcccta ctggtgtcat cactgtggaa agaccttctg tagcaagtcc aatctttcca 1200
aacatcagcg agtccacact ggagagggag aagcaccgta actttcaagc gctcctgttg 1260
ttgtcgttgt tttaaacttt agaatctgaa aaccagaaag aagtcttgtc attgcagcag 1320
catcgattcc ggtgatagag tttgtatcac tcaacatcag gggatgcctg aggagtgcga 1380
gctccacagc aacatggcag gcaggaggtc ctcagaaggt gtcaggaggt tccacactcg 1440
ccagttcact ggagcagagt cccttcgcca cacttagggt cccagtaagc catgccagca 1500
ttaccttttg cgtagttaaa cagacgtgta tccagtctag ttaaggaaga aacattaaga 1560
ttgtttaatt tttaacatat attcaagaat tttaatttgt aaagaattga gccacattga 1620
acacaattga atgagattca gaataaactt ataacatctt aaa 1663

<210> 130
<211> 412
<212> PRT
<213> Homo Sapiens

<400> 130
Ala Leu Ser Gln Leu Arg Val Leu Cys Cys Glu Trp Leu Arg Pro Glu
1               5                   10                  15
Ile His Thr Lys Glu Gln Ile Leu Glu Leu Leu Val Leu Glu Gln Phe
            20                  25                  30
Leu Thr Ile Leu Pro Gln Glu Leu Gln Ala Trp Val Gln Glu His Cys
        35                  40                  45
Pro Glu Ser Ala Glu Glu Ala Val Thr Leu Leu Glu Asp Leu Glu Arg
    50                  55                  60
Glu Leu Asp Glu Pro Gly His Gln Val Ser Thr Pro Pro Asn Glu Gln
65                  70                  75                  80
Lys Pro Val Trp Glu Lys Ile Ser Ser Ser Gly Thr Ala Lys Glu Ser
                85                  90                  95
Pro Ser Ser Met Gln Pro Gln Pro Leu Glu Thr Ser His Lys Tyr Glu
            100                 105                 110
Ser Trp Gly Pro Leu Tyr Ile Gln Glu Ser Gly Glu Glu Gln Glu Phe
        115                 120                 125
Ala Gln Asp Pro Arg Lys Val Arg Asp Cys Arg Leu Ser Thr Gln His
    130                 135                 140
Glu Glu Ser Ala Asp Glu Gln Lys Gly Ser Glu Ala Glu Gly Leu Lys
145                 150                 155                 160
Gly Asp Ile Ile Ser Val Ile Ile Ala Asn Lys Pro Glu Ala Ser Leu
                165                 170                 175
Glu Arg Gln Cys Val Asn Leu Glu Asn Glu Lys Gly Thr Lys Pro Pro
            180                 185                 190
Leu Gln Glu Ala Gly Ser Lys Lys Gly Arg Glu Ser Val Pro Thr Lys
        195                 200                 205
Pro Thr Pro Gly Glu Arg Arg Tyr Ile Cys Ala Glu Cys Gly Lys Ala
    210                 215                 220
Phe Ser Asn Ser Ser Asn Leu Thr Lys His Arg Arg Thr His Thr Gly
225                 230                 235                 240
Glu Lys Pro Tyr Val Cys Thr Lys Cys Gly Lys Ala Phe Ser His Ser

                245                 250                 255
Ser Asn Leu Thr Leu His Tyr Arg Thr His Leu Val Asp Arg Pro Tyr
            260                 265                 270
Asp Cys Lys Cys Gly Lys Ala Phe Gly Gln Ser Ser Asp Leu Leu Lys
        275                 280                 285
His Gln Arg Met His Thr Glu Glu Ala Pro Tyr Gln Cys Lys Asp Cys
    290                 295                 300
Gly Lys Ala Phe Ser Gly Lys Gly Ser Leu Ile Arg His Tyr Arg Ile
305                 310                 315                 320
His Thr Gly Glu Lys Pro Tyr Gln Cys Asn Glu Cys Gly Lys Ser Phe
                325                 330                 335
Ser Gln His Ala Gly Leu Ser Ser His Gln Arg Leu His Thr Gly Glu
            340                 345                 350
Lys Pro Tyr Lys Cys Lys Glu Cys Gly Lys Ala Phe Asn His Ser Ser
        355                 360                 365
Asn Phe Asn Lys His His Arg Ile His Thr Gly Glu Lys Pro Tyr Trp
    370                 375                 380
Cys His His Cys Gly Lys Thr Phe Cys Ser Lys Ser Asn Leu Ser Lys
385                 390                 395                 400
His Gln Arg Val His Thr Gly Glu Gly Glu Ala Pro
                405                 410

<210> 131
<211> 724
<212> DNA
<213> Homo Sapiens

<400> 131
ggagaatgaa aagcagaaag tggcagagct gtattctatc cataactctg gagacaaatc 60
tgatattcag gacctcctgg agagtgtcag gctggacaaa gaaaaagcag agactttggc 120
tagtagcttg caggaagatc tggctcatac ccgaaatgat gccaatcgat tacaggatgc 180
cattgctaag gtagaggatg aataccgagc cttccaagaa gaagctaaga aacaaattga 240
agatttgaat atgacgttag aaaaattaag atcagacctg gatgaaaaag aaacagaaag 300
gagtgacatg aaagaaacca tctttgaact tgaagatgaa gtagaacaac atcgtgctgt 360
gaaacttcat gacaacctca ttatttctga tctagagaat acagttaaaa aactccagga 420
ccaaaagcac gacatggaaa gagaaataaa gacactccac agaagacttc gggaagaatc 480
tgcggaatgg cggcagtttc aggctgatct ccagactgca gtagtcattg caaatgacat 540
taaatctgaa gcccaagagg agattggtga tctaaagcgc cgggtacatg aggctcaaga 600
aaaaaatgag aaactcacaa aagaattgga ggaaataagt ccgccaagcc agaagangac 660
gangccggta ttccantaca tgnatgcccg tgagagagaa tttggcaggc cttaaggcag 720
ggaa 724

<210> 132
<211> 218
<212> PRT
<213> Homo Sapiens

<400> 132
Glu Asn Glu Lys Gln Lys Val Ala Glu Leu Tyr Ser Ile His Asn Ser
1               5                   10                  15
Gly Asp Lys Ser Asp Ile Gln Asp Leu Leu Glu Ser Val Arg Leu Asp
            20                  25                  30
Lys Glu Lys Ala Glu Thr Leu Ala Ser Ser Leu Gln Glu Asp Leu Ala
        35                  40                  45
His Thr Arg Asn Asp Ala Asn Arg Leu Gln Asp Ala Ile Ala Lys Val

    50                  55                  60
Glu Asp Glu Tyr Arg Ala Phe Gln Glu Glu Ala Lys Lys Gln Ile Glu
65                  70                  75                  80
Asp Leu Asn Met Thr Leu Glu Lys Leu Arg Ser Asp Leu Asp Glu Lys
                85                  90                  95
Glu Thr Glu Arg Ser Asp Met Lys Glu Thr Ile Phe Glu Leu Glu Asp
            100                 105                 110
Glu Val Glu Gln His Arg Ala Val Lys Leu His Asp Asn Leu Ile Ile
        115                 120                 125
Ser Asp Leu Glu Asn Thr Val Lys Lys Leu Gln Asp Gln Lys His Asp
    130                 135                 140
Met Glu Arg Glu Ile Lys Thr Leu His Arg Arg Leu Arg Glu Glu Ser
145                 150                 155                 160
Ala Glu Trp Arg Gln Phe Gln Ala Asp Leu Gln Thr Ala Val Val Ile
                165                 170                 175
Ala Asn Asp Ile Lys Ser Glu Ala Gln Glu Glu Ile Gly Asp Leu Lys
            180                 185                 190
Arg Arg Val His Glu Ala Gln Glu Lys Asn Glu Lys Leu Thr Lys Glu
        195                 200                 205
Leu Glu Glu Ile Ser Pro Pro Ser Gln Lys
    210                 215

<210> 133
<211> 719
<212> DNA
<213> Homo Sapiens

<400> 133
gagaactaca gagctgggtg cggggccaac ggccagaaag tggcgaggag gcagtgacgc 60
tggtggaggg tttgcagaaa caacccagga gaccaaggcg gtgactgtcc atgttcacgg 120
ccaggaagtc ctgtcagagg agacggtgca tttaggagcg gagcctgagt cacctaatga 180
gctgcaggat cctgtgcaaa gctcgacccc cgagcagtct cctgaggaaa ccacacagag 240
cccagatctg ggggcaccgg cagagcagcg tccacaccag gaagaggagc tccagaccct 300
gcaggagagc gaggtcccag tgcccgagga cccagacctt cctgcagaga ggagctctgg 360
agactcagag atggttgctc ttcttactgc tctgtcacag ggactggtaa cgttcaagga 420
tgtggccgta tgcttttccc aggaccagtg gagtgatctg gacccaacac agaaagagtt 480
ctatggagaa tatgtcttgg aagaagactg tggaattgtt gtctctctgt catttccaat 540
ccccagacct gatgagatct cccaggttag agaggaagag cccttgggtc ccagatatcc 600
aagagcctna ggagactcaa gagccagaaa tcctgagttt tacctacaca ggagatagga 660
gtnaagatga aggaaaatgt ctggagccag gaagaatctg agtttggagg atataccca 719

<210> 134
<211> 217
<212> PRT
<213> Homo Sapiens

<400> 134
Arg Thr Thr Glu Leu Gly Ala Gly Pro Thr Ala Arg Lys Trp Arg Gly
1               5                   10                  15
Gly Ser Asp Ala Gly Gly Gly Phe Ala Glu Thr Thr Gln Glu Thr Lys
            20                  25                  30
Ala Val Thr Val His Val His Gly Gln Glu Val Leu Ser Glu Glu Thr
        35                  40                  45
Val His Leu Gly Ala Glu Pro Glu Ser Pro Asn Glu Leu Gln Asp Pro
    50                  55                  60

Val Gln Ser Ser Thr Pro Glu Gln Ser Pro Glu Glu Thr Thr Gln Ser
65                  70                  75                  80
Pro Asp Leu Gly Ala Pro Ala Glu Gln Arg Pro His Gln Glu Glu Glu
                85                  90                  95
Leu Gln Thr Leu Gln Glu Ser Glu Val Pro Val Pro Glu Asp Pro Asp
            100                 105                 110
Leu Pro Ala Glu Arg Ser Ser Gly Asp Ser Glu Met Val Ala Leu Leu
        115                 120                 125
Thr Ala Leu Ser Gln Gly Leu Val Thr Phe Lys Asp Val Ala Val Cys
    130                 135                 140
Phe Ser Gln Asp Gln Trp Ser Asp Leu Asp Pro Thr Gln Lys Glu Phe
145                 150                 155                 160
Tyr Gly Glu Tyr Val Leu Glu Glu Asp Cys Gly Ile Val Val Ser Leu
                165                 170                 175
Ser Phe Pro Ile Pro Arg Pro Asp Glu Ile Ser Gln Val Arg Glu Glu
            180                 185                 190
Glu Pro Leu Gly Pro Arg Tyr Pro Arg Ala Gly Asp Ser Arg Ala Arg
        195                 200                 205
Asn Pro Glu Phe Tyr Leu His Arg Arg
    210                 215

<210> 135
<211> 1027
<212> DNA
<213> Homo Sapiens

<400> 135
gcgagggcga gggcgaggcg gtgctcatgg aggaggacct gatccagcag agcctggacg      60
actacgacgc cggcaggtac agcccgcggc tgctcacggc gcacgagctg ccactggacg     120
cgcacgtgct ggaaccggat gaggacctgc agcgcctgca gctctcgcgc cagcagctcc     180
aggtcacggg agacgccagc gagagcgccg aggacatctt cttccggcgg gccaaggagg     240
gcatgggcca ggacgaggcg cagttcagcg tggagatgcc actcaccggc aaggcctacc     300
tgtgggccga caagtaccgg ccacgcaagc cgcgcttctt caaccgcgtg cacacgggct     360
tcgagtggaa caagtacaac cagacgcact acgactttga caacccaccg cccaagatcg     420
tgcagggata caagttcaac atcttctacc ccgacctcat cgacaagcgc tccacgcccg     480
agtacttcct ggaggcctgc gccgacaaca aggatttcgc catcctgcgc ttcacgcggg     540
gccgcctacg aggacatcgc tttcaagatc gtcaaccgcg agtgggaata ctngcaccgc     600
cacggcttcc gctgccagtt tgccaacggc attttccanc tgngctttca cttcaagcgc     660
tnccgctatc ggcggtgacg gccctgggga acggcaggcc aggagggccg agggccacac     720
gggtgccaca gcccaggtcg gagtggccca gccggcaggc ttgtttttca gcatccgacg     780
ggaacatctc caacagaagc aaaacggaaa gtgcctcccg gacccccaga gggccaccca     840
acctcaccag tcaccagccc cagaccaccc acagcccctc ccagacaccc cgcctcatct     900
ggaaatagtt ccgtttgttt ctctaaaaag acttgtaggt gggaaaaaaa atcttttggt     960
ctcatggaat tggcctattg gcaagatcgc atgttttttt aataaacgtt gtattttaga    1020
ataaaaa                                                              1027

<210> 136
<211> 299
<212> PRT
<213> Homo Sapiens

<400> 136
Glu Gly Glu Gly Glu Ala Val Leu Met Glu Glu Asp Leu Ile Gln Gln
1               5                   10                  15
Ser Leu Asp Asp Tyr Asp Ala Gly Arg Tyr Ser Pro Arg Leu Leu Thr

.20                  25                  30
Ala His Glu Leu Pro Leu Asp Ala His Val Leu Glu Pro Asp Glu Asp
        35                  40                  45
Leu Gln Arg Leu Gln Leu Ser Arg Gln Gln Leu Gln Val Thr Gly Asp
    50                  55                  60
Ala Ser Glu Ser Ala Glu Asp Ile Phe Phe Arg Arg Ala Lys Glu Gly
65                  70                  75                  80
Met Gly Gln Asp Glu Ala Gln Phe Ser Val Glu Met Pro Leu Thr Gly
                85                  90                  95
Lys Ala Tyr Leu Trp Ala Asp Lys Tyr Arg Pro Arg Lys Pro Arg Phe
            100                 105                 110
Phe Asn Arg Val His Thr Gly Phe Glu Trp Asn Lys Tyr Asn Gln Thr
        115                 120                 125
His Tyr Asp Phe Asp Asn Pro Pro Pro Lys Ile Val Gln Gly Tyr Lys
    130                 135                 140
Phe Asn Ile Phe Tyr Pro Asp Leu Ile Asp Lys Arg Ser Thr Pro Glu
145                 150                 155                 160
Tyr Phe Leu Glu Ala Cys Ala Asp Asn Lys Asp Phe Ala Ile Leu Arg
                165                 170                 175
Phe Thr Arg Gly Arg Leu Arg Gly His Arg Phe Gln Asp Arg Gln Pro
            180                 185                 190
Arg Val Gly Ile Leu Ala Pro Pro Arg Leu Pro Leu Pro Val Cys Gln
        195                 200                 205
Arg His Phe Pro Leu Ser Leu Gln Ala Leu Pro Leu Ser Ala Val Thr
    210                 215                 220
Ala Leu Gly Asn Gly Arg Pro Gly Gly Pro Arg Ala Thr Arg Val Pro
225                 230                 235                 240
Gln Pro Arg Ser Glu Trp Pro Ser Arg Gln Ala Cys Phe Ser Ala Ser
                245                 250                 255
Asp Gly Asn Ile Ser Asn Arg Ser Lys Thr Glu Ser Ala Ser Arg Thr
            260                 265                 270
Pro Arg Gly Pro Pro Asn Leu Thr Ser His Gln Pro Gln Thr Thr His
        275                 280                 285
Ser Pro Ser Gln Thr Pro Arg Leu Ile Trp Lys
    290                 295

<210> 137
<211> 766
<212> DNA
<213> Homo Sapiens

<400> 137
caaaggttta cacagtaaac aatgtgaatg tgatcaccaa aatacgcaca gaacatctga      60
ccgaggagga aaaaaagaga tataaagaca ggaacccgct ggaatctttg ctgggaactg     120
tggaacacca atttggtgca caaggggacc tcaccacgga atgtgctact gcaaacaacc     180
ccacagccat cacgcctgat gagtacttca atgaagagtt tgatctgaaa gacaggacaa     240
ttggaaggcc gaaagagctg acgattagaa cacagaagtt taaagcaatg ttgtggatgt     300
gtgaagagtt tcccctctct ctggtggagc aggtcattcc catcattgac ctaatggctc     360
gaacgagtgc tcattttgca agactgagag atttcatcaa attggaattc ccacctggat     420
ttcctgtcaa aatagcttcc cacatcacaa actttgaggt tgatcaatct gtgtttgaaa     480
ttcccgaatc ttactatgtt caagacaatg gcagaaatgt gcatttgcaa gatgaagatt     540
acgagataat gcagtttgcc atccagcaaa gtctgctgga gtccagcagg agccaggaac     600
tttcaggacc agcttcgaat ggagggatca gccagacaaa cacctatgac gcccagtatg     660
agagggccat ncaggagagc cttctaccag cacagaaagc ctgtgccccc agcgcccctg     720
agcgagacna gccgttttga taatggactt gcagctaagc catgga                    766

<210> 138
<211> 243
<212> PRT
<213> Homo Sapiens

<400> 138
Lys Val Tyr Thr Val Asn Asn Val Asn Val Ile Thr Lys Ile Arg Thr
1               5                   10                  15
Glu His Leu Thr Glu Glu Glu Lys Lys Arg Tyr Lys Asp Arg Asn Pro
            20                  25                  30
Leu Glu Ser Leu Leu Gly Thr Val Glu His Gln Phe Gly Ala Gln Gly
        35                  40                  45
Asp Leu Thr Thr Glu Cys Ala Thr Ala Asn Asn Pro Thr Ala Ile Thr
    50                  55                  60
Pro Asp Glu Tyr Phe Asn Glu Glu Phe Asp Leu Lys Asp Arg Asp Ile
65                  70                  75                  80
Gly Arg Pro Lys Glu Leu Thr Ile Arg Thr Gln Lys Phe Lys Ala Met
                85                  90                  95
Leu Trp Met Cys Glu Glu Phe Pro Leu Ser Leu Val Glu Gln Val Ile
            100                 105                 110
Pro Ile Ile Asp Leu Met Ala Arg Thr Ser Ala His Phe Ala Arg Leu
        115                 120                 125
Arg Asp Phe Ile Lys Leu Glu Phe Pro Pro Gly Phe Pro Val Lys Ile
    130                 135                 140
Ala Ser His Ile Thr Asn Phe Glu Val Asp Gln Ser Val Phe Glu Ile
145                 150                 155                 160
Pro Glu Ser Tyr Tyr Val Gln Asp Asn Gly Arg Asn Val His Leu Gln
                165                 170                 175
Asp Glu Asp Tyr Glu Ile Met Gln Phe Ala Ile Gln Gln Ser Leu Leu
            180                 185                 190
Glu Ser Ser Arg Ser Gln Glu Leu Ser Gly Pro Ala Ser Asn Gly Gly
        195                 200                 205
Ile Ser Gln Thr Asn Thr Tyr Asp Ala Gln Tyr Glu Arg Ala Gln Glu
    210                 215                 220
Ser Leu Leu Pro Ala Gln Lys Ala Cys Ala Pro Ser Ala Pro Glu Arg
225                 230                 235                 240
Asp Pro Phe

<210> 139
<211> 3060
<212> DNA
<213> Homo Sapiens

<400> 139
ccgggcggga gtgcggcgag agccggctgg ctgagcttag cgtccgagga ggcggcggcg      60
gcggcggcgg cagcggcggc ggcggggctg tggggcggtg cggaagcgag aggcgaggag     120
cgcgcgggcc gtggccagag tctggcggcg gcctggcgga gcggagagca gcgcccgcgc     180
ctcgccgtgc ggaggagccc cgcacacaat agcggcgcgc gcagcccgcg cccttccccc     240
cggcgcgccc cgccccgcgc gccgagcgcc ccgctccgcc tcacctgcca ccagggagtg     300
ggcgggcatt gttcgccgcc gccgccgccg cgcggggcca tgggggccgc ccggcgcccg     360
gggccgggcc tggcgaggcc gccgcgccgc cgctgagacg ggccccgcgc gcagcccggc     420
ggcgcaggta aggccggccg cgccatggtg gacccggtgg gcttcgcgga ggcgtggaag     480
gcgcagttcc cggactcaga gcccccgcgc atggagctgc gctcagtggg cgacatcgag     540
caggagctgg agcgctgcaa ggcctccatt cggcgcctgg agcaggaggt gaaccaggag     600

cgcttccgca tgatctacct gcagacgttg ctggccaagg aaaagaagag ctatgaccgg 660
cagcgatggg gcttccggcg cgcggcgcag gcccccgacg gcgcctccga gccccgagcg 720
tccgcgtcgc gcccgcagcc agcgcccgcc gacggagccg acccgccgcc cgccgaggag 780
cccgaggccc ggcccgacgg cgagggttct ccgggtaagg ccaggcccgg gaccgcccgc 840
aggcccgggg cagccgcgtc gggggaacgg gacgaccggg gaccccccgc cagcgtggcg 900
gcgctcaggt ccaacttcga gcggatccgc aagggccatg gccagcccgg ggcggacgcc 960
gagaagccct tctacgtgaa cgtcgagttt caccacgagc gcggcctggt gaaggtcaac 1020
gacaaagagg tgtcggaccg catcagctcc ctgggcagcc aggccatgca gatggagcgc 1080
aaaaagtccc agcacggcgc gggctcgagc gtgggggatg catccaggcc cccttaccgg 1140
ggacgctcct cggagagcag ctgcggcgtc gacggcgact acgaggacgc cgagttgaac 1200
ccccgcttcc tgaaggacaa cctgatcgac gccaatggcg gtagcaggcc cccttggccg 1260
cccctggagt accagcccta ccagagcatc tacgtcgggg gcatgatgga aggggagggc 1320
aagggcccgc tcctgcgcag ccagagcacc tctgagcagg agaagcgcct tacctggccc 1380
cgcaggtcct actccccccg gagttttgag gattgcggag gcggctatac cccggactgc 1440
agctccaatg agaacctcac ctccagcgag gaggacttct cctctggcca gtccagccgc 1500
gtgtccccaa gccccaccac ctaccgcatg ttccgggaca aaagccgctc tccctcgcag 1560
aactcgcaac agtccttcga cagcagcagt ccccccacgc cgcagtgcca taagcggcac 1620
cggcactgcc cggttgtcgt gtccgaggcc accatcgtgg gcgtccgcaa gaccgggcag 1680
atctggccca acgatggcga gggcgccttc catggagacg cagatggctc gttcggaaca 1740
ccacctggat acggctgcgc tgcagaccgg gcagaggagc agcgccggca ccaagatggg 1800
ctgccctaca ttgatgactc gccctcctca tcgccccacc tcagcagcaa gggcaggggc 1860
agccgggatg cgctggtctc gggagccctg gagtccacta aagcgagtga gctggacttg 1920
gaaaagggct tggagatgag aaaatgggtc ctgtcgggaa tcctggctag cgaggagact 1980
tacctgagcc acctggaggc actgctgctg cccatgaagc ctttgaaagc cgctgccacc 2040
acctctcagc cggtgctgac gagtcagcag atcgagacca tcttcttcaa agtgcctgag 2100
ctctacgaga tccacaagga gttctatgat gggctcttcc cccgcgtgca gcagtggagc 2160
caccagcagc gggtgggcga cctcttccag aagctggcca gccagctggg tgtgtaccgg 2220
gccttcgtgg acaactacgg agttgccatg gaaatggctg agaagtgctg tcaggccaat 2280
gctcagtttg cagaaatctc cgagaacctg agagccagaa gcaacaaaga tgccaaggat 2340
ccaacgacca agaactctct ggaaactctg ctctacaagc ctgtggaccg tgtgacgagg 2400
agcacgctgg tcctccatga cttgctgaag cacactcctg ccagccaccc tgaccacccc 2460
ttgctgcagg acgccctccg catctcacag aacttcctgt ccagcatcaa tgaggagatc 2520
acaccccgac ggcagtccat gacggtgaag aagggagagc accggcagct gctgaaggac 2580
agcttcatgg tggagctggt ggagggggcc cgcaagctgc gccacgtctt cctgttcacc 2640
gagctgcttc tctgcaccaa gctcaagaag cagagcggag gcaaaacgca gcagtatgac 2700
tgcaaatggt acattccgct cacggatctc agcttccaga tggtggatga actggaggca 2760
gtgcccaaca tccccctggt gcccgatgag gagctggacg ctttgaagat caagatctcc 2820
cagatcaaga gtgacatcca gagagagaag agggcgaaca agggcagcaa ggctacggag 2880
aggctgaaga agaagctgtc ggagcaggag tcactgctgc tgcttatgtc tcccagcatg 2940
gccttcaggg tgcacagccg caacggcaag agttacacgt tcctgatctc ctctgactat 3000
gagcgtgcag agtggaggga gaacatccgg gagcagcaga agaagtgttt cagaagcttc 3060

<210> 140
<211> 872
<212> PRT
<213> Homo Sapiens

<400> 140
Met Val Asp Pro Val Gly Phe Ala Glu Ala Trp Lys Ala Gln Phe Pro
1               5                   10                  15
Asp Ser Glu Pro Pro Arg Met Glu Leu Arg Ser Val Gly Asp Ile Glu
            20                  25                  30
Gln Glu Leu Glu Arg Cys Lys Ala Ser Ile Arg Arg Leu Glu Gln Glu
        35                  40                  45
Val Asn Gln Glu Arg Phe Arg Met Ile Tyr Leu Gln Thr Leu Leu Ala

    50                  55                  60
Lys Glu Lys Lys Ser Tyr Asp Arg Gln Arg Trp Gly Phe Arg Arg Ala
65                  70                  75                  80
Ala Gln Ala Pro Asp Gly Ala Ser Glu Pro Arg Ala Ser Ala Ser Arg
                85                  90                  95
Pro Gln Pro Ala Pro Ala Asp Gly Ala Asp Pro Pro Pro Ala Glu Glu
            100                 105                 110
Pro Glu Ala Arg Pro Asp Gly Glu Gly Ser Pro Gly Lys Ala Arg Pro
        115                 120                 125
Gly Thr Ala Arg Arg Pro Gly Ala Ala Ala Ser Gly Glu Arg Asp Asp
    130                 135                 140
Arg Gly Pro Pro Ala Ser Val Ala Ala Leu Arg Ser Asn Phe Glu Arg
145                 150                 155                 160
Ile Arg Lys Gly His Gly Gln Pro Gly Ala Asp Ala Glu Lys Pro Phe
                165                 170                 175
Tyr Val Asn Val Glu Phe His His Glu Arg Gly Leu Val Lys Val Asn
            180                 185                 190
Asp Lys Glu Val Ser Asp Arg Ile Ser Ser Leu Gly Ser Gln Ala Met
        195                 200                 205
Gln Met Glu Arg Lys Lys Ser Gln His Gly Ala Gly Ser Ser Val Gly
    210                 215                 220
Asp Ala Ser Arg Pro Pro Tyr Arg Gly Arg Ser Ser Glu Ser Ser Cys
225                 230                 235                 240
Gly Val Asp Gly Asp Tyr Glu Asp Ala Glu Leu Asn Pro Arg Phe Leu
                245                 250                 255
Lys Asp Asn Leu Ile Asp Ala Asn Gly Gly Ser Arg Pro Pro Trp Pro
            260                 265                 270
Pro Leu Glu Tyr Gln Pro Tyr Gln Ser Ile Tyr Val Gly Gly Met Met
        275                 280                 285
Glu Gly Glu Gly Lys Gly Pro Leu Leu Arg Ser Gln Ser Thr Ser Glu
    290                 295                 300
Gln Glu Lys Arg Leu Thr Trp Pro Arg Arg Ser Tyr Ser Pro Arg Ser
305                 310                 315                 320
Phe Glu Asp Cys Gly Gly Gly Tyr Thr Pro Asp Cys Ser Ser Asn Glu
                325                 330                 335
Asn Leu Thr Ser Ser Glu Glu Asp Phe Ser Ser Gly Gln Ser Ser Arg
            340                 345                 350
Val Ser Pro Ser Pro Thr Thr Tyr Arg Met Phe Arg Asp Lys Ser Arg
        355                 360                 365
Ser Pro Ser Gln Asn Ser Gln Gln Ser Phe Asp Ser Ser Ser Pro Pro
    370                 375                 380
Thr Pro Gln Cys His Lys Arg His Arg His Cys Pro Val Val Val Ser
385                 390                 395                 400
Glu Ala Thr Ile Val Gly Val Arg Lys Thr Gly Gln Ile Trp Pro Asn
                405                 410                 415
Asp Gly Glu Gly Ala Phe His Gly Asp Ala Asp Gly Ser Phe Gly Thr
            420                 425                 430
Pro Pro Gly Tyr Gly Cys Ala Ala Asp Arg Ala Glu Glu Gln Arg Arg
        435                 440                 445
His Gln Asp Gly Leu Pro Tyr Ile Asp Asp Ser Pro Ser Ser Ser Pro
    450                 455                 460
His Leu Ser Ser Lys Gly Arg Gly Ser Arg Asp Ala Leu Val Ser Gly
465                 470                 475                 480
Ala Leu Glu Ser Thr Lys Ala Ser Glu Leu Asp Leu Glu Lys Gly Leu
                485                 490                 495

Glu Met Arg Lys Trp Val Leu Ser Gly Ile Leu Ala Ser Glu Glu Thr
            500                 505                 510
Tyr Leu Ser His Leu Glu Ala Leu Leu Leu Pro Met Lys Pro Leu Lys
        515                 520                 525
Ala Ala Ala Thr Thr Ser Gln Pro Val Leu Thr Ser Gln Gln Ile Glu
    530                 535                 540
Thr Ile Phe Phe Lys Val Pro Glu Leu Tyr Glu Ile His Lys Glu Phe
545                 550                 555                 560
Tyr Asp Gly Leu Phe Pro Arg Val Gln Gln Trp Ser His Gln Gln Arg
                565                 570                 575
Val Gly Asp Leu Phe Gln Lys Leu Ala Ser Gln Leu Gly Val Tyr Arg
            580                 585                 590
Ala Phe Val Asp Asn Tyr Gly Val Ala Met Glu Met Ala Glu Lys Cys
        595                 600                 605
Cys Gln Ala Asn Ala Gln Phe Ala Glu Ile Ser Glu Asn Leu Arg Ala
    610                 615                 620
Arg Ser Asn Lys Asp Ala Lys Asp Pro Thr Thr Lys Asn Ser Leu Glu
625                 630                 635                 640
Thr Leu Leu Tyr Lys Pro Val Asp Arg Val Thr Arg Ser Thr Leu Val
                645                 650                 655
Leu His Asp Leu Leu Lys His Thr Pro Ala Ser His Pro Asp His Pro
            660                 665                 670
Leu Leu Gln Asp Ala Leu Arg Ile Ser Gln Asn Phe Leu Ser Ser Ile
        675                 680                 685
Asn Glu Glu Ile Thr Pro Arg Arg Gln Ser Met Thr Val Lys Lys Gly
    690                 695                 700
Glu His Arg Gln Leu Leu Lys Asp Ser Phe Met Val Glu Leu Val Glu
705                 710                 715                 720
Gly Ala Arg Lys Leu Arg His Val Phe Leu Phe Thr Glu Leu Leu Leu
                725                 730                 735
Cys Thr Lys Leu Lys Lys Gln Ser Gly Gly Lys Thr Gln Gln Tyr Asp
            740                 745                 750
Cys Lys Trp Tyr Ile Pro Leu Thr Asp Leu Ser Phe Gln Met Val Asp
        755                 760                 765
Glu Leu Glu Ala Val Pro Asn Ile Pro Leu Val Pro Asp Glu Glu Leu
    770                 775                 780
Asp Ala Leu Lys Ile Lys Ile Ser Gln Ile Lys Ser Asp Ile Gln Arg
785                 790                 795                 800
Glu Lys Arg Ala Asn Lys Gly Ser Lys Ala Thr Glu Arg Leu Lys Lys
                805                 810                 815
Lys Leu Ser Glu Gln Glu Ser Leu Leu Leu Leu Met Ser Pro Ser Met
            820                 825                 830
Ala Phe Arg Val His Ser Arg Asn Gly Lys Ser Tyr Thr Phe Leu Ile
        835                 840                 845
Ser Ser Asp Tyr Glu Arg Ala Glu Trp Arg Glu Asn Ile Arg Glu Gln
    850                 855                 860
Gln Lys Lys Cys Phe Arg Ser Phe
865                 870

<210> 141
<211> 691
<212> DNA
<213> Homo Sapiens

<400> 141

gacccctcac actcacctag ccaccatgga catcgccatc caccacccct ggatccgccg 60
cccttctttt cctttccact cccccagccg cctctttgac cagttcttcg gagagcacct 120
gttggagtct gatcttttcc cgacgtctac ttccctgagt cccttctacc ttcggccacc 180
ctccttcctg cgggcaccca gctggtttga cactggactc tcagagatgc gcctggagaa 240
ggacaggttc tctgtcaacc tggatgtgaa gcacttctcc ccagaggaac tcaaagttaa 300
ggtgttggga gatgtgattg aggtgcatgg aaaacatgaa gagcgccagg atgaacatgg 360
tttcatctcc agggagttcc acaggaaata ccggatccca gctgatgtag accctctcac 420
cattacttca tccctgtcat ctgatggggt cctcactgtg aatggaccaa ggaaacaggt 480
ctctggccct gagcgcacca ttcccatcac ccgtgaagag aagcctgctg tcaccgcagc 540
ccccaagaaa tagatgccct ttcttgaatt gcatttttta aaacaagaaa gtttccccac 600
cagtgaatga aagtcttgtg actagtgctg aagcttatta atgctaaggg caggcccaaa 660
ttatcaagct aataaaatat cattcagcaa c 691

<210> 142
<211> 175
<212> PRT
<213> Homo Sapiens

<400> 142

<210> 143
<211> 1300
<212> DNA
<213> Homo Sapiens

<400> 143
atctgctggg aatttcttgg gttgacagct cttggatccc tattttgaac agtggtagtg 60
tcctggatta cttttcagaa agaagtaatc ctttttatga cagaacatgt aataatgaag 120
tggtcaaaat gcagaggcta acattagaac acttgaatca gatggttgga atcgagtaca 180
tccttttgca tgctcaagag cccattcttt tcatcattcg gaagcaacag cggcagtccc 240
ctgcccaagt tatcccacta gctgattact atatcattgc tggagtgatc tatcaggcac 300
cagacttggg atcagttata aactctagag tgcttactgc agtgcatggt attcagtcag 360
cttttgatga agctatgtca tactgtcgat atcatccttc caaagggtat tggtggcact 420

tcaaagatca tgaagagcaa gataaagtca gacctaaagc caaaaggaaa gaagaaccaa 480
gctctatttt tcagagacaa cgtgtggatg ctttactttt agacctcaga caaaaatttc 540
cacccaaatt tgtgcagcta aagcctggag aaaagcctgt tcaagtggat caaacaaaga 600
aagaggcaga acctatacca gaaactgtaa aacctgagga gaaggagacc cccnnagaat 660
gtacaaccag accgggagtg ctaaaggccc ccctgaaaaa cggatgagac ttcagtgagt 720
actggacaaa agagaagcct ggaagactcc tcatgctagt tatcatacct cagtactgtg 780
gctcttgagc tttgaagtac tttattgtaa ccttcttatt tgtatggaat gcgcttattt 840
tttgaaagga tattaggccg gatgtggtgg ctcacgcctg taatcccagc actttgggag 900
gccatggcgg gtggatcact tgaggtcaga agttcaagac cagcctgacc aatatggtga 960
aaccccgtct ctactaaaaa tacaaaaatt agccgggcgt ggtggcgggc gcccgtagtc 1020
ccagctactc gggaggctga gacaggagac ttgcttgaac ccgggaggtg gaggttgccc 1080
tgagctgatt atcatgctgt tgcactccag cttgggcgac agagcgagac tttgtctcaa 1140
aaaagaagaa aagatattac tcccatcatg atttcttgtg aatatttgtt atatgtcttc 1200
tgtaaccttt cctctcccgg acttgagcaa cctacacact cacatgttta ctggtagata 1260
tgtttaaaag caaaataaag gtatttgtat atattgaaaa 1300

<210> 144
<211> 233
<212> PRT
<213> Homo Sapiens

<400> 144
Leu Leu Gly Ile Ser Trp Val Asp Ser Ser Trp Ile Pro Ile Leu Asn
1               5                   10                  15
Ser Gly Ser Val Leu Asp Tyr Phe Ser Glu Arg Ser Asn Pro Phe Tyr
            20                  25                  30
Asp Arg Thr Cys Asn Asn Glu Val Val Lys Met Gln Arg Leu Thr Leu
        35                  40                  45
Glu His Leu Asn Gln Met Val Gly Ile Glu Tyr Ile Leu Leu His Ala
    50                  55                  60
Gln Glu Pro Ile Leu Phe Ile Ile Arg Lys Gln Gln Arg Gln Ser Pro
65                  70                  75                  80
Ala Gln Val Ile Pro Leu Ala Asp Tyr Tyr Ile Ile Ala Gly Val Ile
                85                  90                  95
Tyr Gln Ala Pro Asp Leu Gly Ser Val Ile Asn Ser Arg Val Leu Thr
            100                 105                 110
Ala Val His Gly Ile Gln Ser Ala Phe Asp Glu Ala Met Ser Tyr Cys
        115                 120                 125
Arg Tyr His Pro Ser Lys Gly Tyr Trp Trp His Phe Lys Asp His Glu
    130                 135                 140
Glu Gln Asp Lys Val Arg Pro Lys Ala Lys Arg Lys Glu Glu Pro Ser
145                 150                 155                 160
Ser Ile Phe Gln Arg Gln Arg Val Asp Ala Leu Leu Leu Asp Leu Arg
                165                 170                 175
Gln Lys Phe Pro Pro Lys Phe Val Gln Leu Lys Pro Gly Glu Lys Pro
            180                 185                 190
Val Gln Val Asp Gln Thr Lys Lys Glu Ala Glu Pro Ile Pro Glu Thr
        195                 200                 205
Val Lys Pro Glu Glu Lys Glu Thr Pro Glu Cys Thr Thr Arg Pro Gly
    210                 215                 220
Val Leu Lys Ala Pro Leu Lys Asn Gly
225                 230

<210> 145
<211> 1528

<212> DNA
<213> Homo Sapiens

<400> 145
cccccctttt tttttaaact aaaatggagg ctggtttctt gccttaagga gcccattgcc 60
tttcccgctg aagtctagat gttgacatgt aataaagcgg gcagcaggat ggtggtggat 120
gcggccaact ccaatgggcc tttccagccc gtggtccttc tccatattcg agatgttcct 180
cctgctgatc aagagaagct ttttatccag aagttacgtc agtgttgcgt cctctttgac 240
tttgtttctg atccactaag tgacctaaag tggaaggaag taaaacgagc tgctttaagt 300
gaaatggtag aatatatcac ccataatcgg aatgtgatca cagagcctat ttacccagaa 360
gtagtccata tgtttgcagt taacatgttt cgaacattac caccttcctc caatcctacg 420
ggagcggaat ttgacccgga ggaagatgaa ccaacgttag aagcagcctg gcctcatcta 480
cagcttgttt atgaattttt cttaagattt ttagagtctc cagatttcca acctaatata 540
gcgaagaaat atattgatca gaagtttgta ttgcagcttt tagagctctt tgacagtgaa 600
gatcctcggg agagagattt tcttaaaacc acccttcaca gaatctatgg gaaattccta 660
ggcttgagag cttacatcag aaaacagata aataatatat tttataggtt tatttatgaa 720
acagagcatc ataatggcat agcagagtta ctggaaatat tgggaagtat aattaatgga 780
tttgccttac cactaaaaga agagcacaag attttcttat tgaaggtgtt actacctttg 840
cacaaagtga aatctctgag tgtctaccat ccccagctgg catactgtgt agtgcagttt 900
ttagaaaagg acagcaccct cacggaacca gtggtgatgg cacttctcaa atactggcca 960
aagactcaca gtccaaaaga agtaatgttc ttaaacgaat tagaagagat tttagatgtc 1020
attgaaccat cagaatttgt gaagatcatg gaacccctct tccggcagtt ggccaaatgt 1080
gtctccagcc cacacttcca ggtggcagag cgagctctct attactggaa taatgaatac 1140
atcatgagtt taatcagtga caacgcagcg aagattctgc ccatcatgtt tccttccttg 1200
taccgcaact caaagaccca ttggaacaag acaatacatg gcttgatata caacgccctg 1260
aagctcttca tggagatgaa ccaaaagcta tttgatgact gtacacaaca gttcaaagca 1320
gagaaactaa aagagaagct aaaaatgaaa gaacgggaag aagcatgggt taaaatagaa 1380
aatctagcca aagccaatcc ccaggtacta aaaaagagaa taacatgaaa aggcccaggg 1440
ttacttgaat gtttttataa gataggaata tatgtcttca ccatgggggg ggtctcgatt 1500
tcactaacgt tgtatatgaa aatgtctg 1528

<210> 146
<211> 449
<212> PRT
<213> Homo Sapiens

<400> 146
Met Leu Thr Cys Asn Lys Ala Gly Ser Arg Met Val Val Asp Ala Ala
1               5                   10                  15
Asn Ser Asn Gly Pro Phe Gln Pro Val Val Leu Leu His Ile Arg Asp
            20                  25                  30
Val Pro Pro Ala Asp Gln Glu Lys Leu Phe Ile Gln Lys Leu Arg Gln
        35                  40                  45
Cys Cys Val Leu Phe Asp Phe Val Ser Asp Pro Leu Ser Asp Leu Lys
    50                  55                  60
Trp Lys Glu Val Lys Arg Ala Ala Leu Ser Glu Met Val Glu Tyr Ile
65                  70                  75                  80
Thr His Asn Arg Asn Val Ile Thr Glu Pro Ile Tyr Pro Glu Val Val
                85                  90                  95
His Met Phe Ala Val Asn Met Phe Arg Thr Leu Pro Pro Ser Ser Asn
            100                 105                 110
Pro Thr Gly Ala Glu Phe Asp Pro Glu Glu Asp Glu Pro Thr Leu Glu
        115                 120                 125
Ala Ala Trp Pro His Leu Gln Leu Val Tyr Glu Phe Phe Leu Arg Phe
    130                 135                 140

Leu Glu Ser Pro Asp Phe Gln Pro Asn Ile Ala Lys Lys Tyr Ile Asp
145                 150                 155                 160
Gln Lys Phe Val Leu Gln Leu Leu Glu Leu Phe Asp Ser Glu Asp Pro
                165                 170                 175
Arg Glu Arg Asp Phe Leu Lys Thr Thr Leu His Arg Ile Tyr Gly Lys
            180                 185                 190
Phe Leu Gly Leu Arg Ala Tyr Ile Arg Lys Gln Ile Asn Asn Ile Phe
        195                 200                 205
Tyr Arg Phe Ile Tyr Glu Thr Glu His His Asn Gly Ile Ala Glu Leu
    210                 215                 220
Leu Glu Ile Leu Gly Ser Ile Ile Asn Gly Phe Ala Leu Pro Leu Lys
225                 230                 235                 240
Glu Glu His Lys Ile Phe Leu Leu Lys Val Leu Leu Pro Leu His Lys
                245                 250                 255
Val Lys Ser Leu Ser Val Tyr His Pro Gln Leu Ala Tyr Cys Val Val
            260                 265                 270
Gln Phe Leu Glu Lys Asp Ser Thr Leu Thr Glu Pro Val Val Met Ala
        275                 280                 285
Leu Leu Lys Tyr Trp Pro Lys Thr His Ser Pro Lys Glu Val Met Phe
    290                 295                 300
Leu Asn Glu Leu Glu Glu Ile Leu Asp Val Ile Glu Pro Ser Glu Phe
305                 310                 315                 320
Val Lys Ile Met Glu Pro Leu Phe Arg Gln Leu Ala Lys Cys Val Ser
                325                 330                 335
Ser Pro His Phe Gln Val Ala Glu Arg Ala Leu Tyr Tyr Trp Asn Asn
            340                 345                 350
Glu Tyr Ile Met Ser Leu Ile Ser Asp Asn Ala Ala Lys Ile Leu Pro
        355                 360                 365
Ile Met Phe Pro Ser Leu Tyr Arg Asn Ser Lys Thr His Trp Asn Lys
    370                 375                 380
Thr Ile His Gly Leu Ile Tyr Asn Ala Leu Lys Leu Phe Met Glu Met
385                 390                 395                 400
Asn Gln Lys Leu Phe Asp Asp Cys Thr Gln Gln Phe Lys Ala Glu Lys
                405                 410                 415
Leu Lys Glu Lys Leu Lys Met Lys Glu Arg Glu Glu Ala Trp Val Lys
            420                 425                 430
Ile Glu Asn Leu Ala Lys Ala Asn Pro Gln Val Leu Lys Lys Arg Ile
        435                 440                 445
Thr

<210> 147
<211> 1580
<212> DNA
<213> Homo Sapiens

<400> 147
atcccctccg gttttcctca gtctccacgt acgtccctca aagcgcgtcc taaaacccgg      60
ataaccggag cgctccccat ggaccacacg gagggcttgc ccgcggagga gccgcctgcg     120
catgctccat cgcctgggaa atttggtgag cggcctccac ctaaacgact tactagggaa     180
gctatgcgaa attatttaaa agagcgaggg gatcaaacag tacttattct tcatgcaaaa     240
gttgcacaga agtcatatgg aaatgaaaaa aggttttttt gcccacctcc ttgtgtatat     300
cttatgggca gcggatggaa gaaaaaaaaa gaacaaatgg aacgcgatgg ttgttctgaa     360
caagagtctc aaccgtgtgc atttattggg ataggaaata gtgaccaaga aatgcagcag     420
ctaaacttgg aaggaaagaa ctattgcaca gccaaaacat tgtatatatc tgactcagac     480

aagcgaaagc acttcatttt ttctgtaaag atgttctatg gcaacagtga tgacattggt 540
gtgttcctca gcaagcggat aaaagtcatc tccaaacctt ccaaaaagaa gcagtcattg 600
aaaaatgctg acttatgcat tgcctcagga acaaaggtgg ctctgtttaa tcgactacga 660
tcccagacag ttagtaccag atacttgcat gtagaaggag gtaattttca tgccagttca 720
cagcagtggg gagccttttt tattcatctc ttggatgatg atgaatcaga aggagaagaa 780
ttcacagtcc gagatgtcta catccattat ggacaaacat gcaaacttgt gtgctcagtt 840
actggcatgg cactcccaag attgataatt atgaaagttg ataagcatac cgcattattg 900
gatgcagatg atcctgtgtc acaactccat aaatgtgcat tttaccttaa ggatacagaa 960
agaatgtatt tgtgcctttc tcaagaaaga ataattcaat ttcaggccac tccatgtcca 1020
aaagaaccaa ataaagagat gataaatgat ggcgcttcct ggacaatcat tagcacagat 1080
aaggcagagt atacatttta tgagggaatg ggccctgtcc ttgccccagt cactcctgtg 1140
cctgtggtag agagccttca gttgaatggc ggtggggacg tagcaatgct tgaacttaca 1200
ggacagaatt tcactccaaa tttacgagtg tggtttgggg atgtagaagc tgaaactatg 1260
tacaggtgtg gagagagtat gctctgtgtc gtcccagaca tttctgcatt ccgagaaggt 1320
tggagatggg tccggcaacc agtccaggtt ccagtaactt tggtccgaaa tgatggaatc 1380
atttattcca ccagccttac ctttacctac acaccagaac cagggccacg gccacattgc 1440
agtgtagcag gagcaatcct tccagccaat tcaagccagg tgccccctaa cgaatcaaac 1500
acaaacagcg agggaagtta cacaaacgcc agcacaaatt caaccagtgt cacatcatct 1560
acagccacag tggtatccta 1580

<210> 148
<211> 500
<212> PRT
<213> Homo Sapiens

<400> 148
Met Asp His Thr Glu Gly Leu Pro Ala Glu Glu Pro Pro Ala His Ala
1               5                   10                  15
Pro Ser Pro Gly Lys Phe Gly Glu Arg Pro Pro Pro Lys Arg Leu Thr
            20                  25                  30
Arg Glu Ala Met Arg Asn Tyr Leu Lys Glu Arg Gly Asp Gln Thr Val
        35                  40                  45
Leu Ile Leu His Ala Lys Val Ala Gln Lys Ser Tyr Gly Asn Glu Lys
    50                  55                  60
Arg Phe Phe Cys Pro Pro Pro Cys Val Tyr Leu Met Gly Ser Gly Trp
65                  70                  75                  80
Lys Lys Lys Lys Glu Gln Met Glu Arg Asp Gly Cys Ser Glu Gln Glu
                85                  90                  95
Ser Gln Pro Cys Ala Phe Ile Gly Ile Gly Asn Ser Asp Gln Glu Met
            100                 105                 110
Gln Gln Leu Asn Leu Glu Gly Lys Asn Tyr Cys Thr Ala Lys Thr Leu
        115                 120                 125
Tyr Ile Ser Asp Ser Asp Lys Arg Lys His Phe Ile Phe Ser Val Lys
    130                 135                 140
Met Phe Tyr Gly Asn Ser Asp Asp Ile Gly Val Phe Leu Ser Lys Arg
145                 150                 155                 160
Ile Lys Val Ile Ser Lys Pro Ser Lys Lys Lys Gln Ser Leu Lys Asn
                165                 170                 175
Ala Asp Leu Cys Ile Ala Ser Gly Thr Lys Val Ala Leu Phe Asn Arg
            180                 185                 190
Leu Arg Ser Gln Thr Val Ser Thr Arg Tyr Leu His Val Glu Gly Gly
        195                 200                 205
Asn Phe His Ala Ser Ser Gln Gln Trp Gly Ala Phe Phe Ile His Leu
    210                 215                 220
Leu Asp Asp Asp Glu Ser Glu Gly Glu Glu Phe Thr Val Arg Asp Val

225                 230                 235                 240
Tyr Ile His Tyr Gly Gln Thr Cys Lys Leu Val Cys Ser Val Thr Gly
                245                 250                 255
Met Ala Leu Pro Arg Leu Ile Ile Met Lys Val Asp Lys His Thr Ala
            260                 265                 270
Leu Leu Asp Ala Asp Asp Pro Val Ser Gln Leu His Lys Cys Ala Phe
        275                 280                 285
Tyr Leu Lys Asp Thr Glu Arg Met Tyr Leu Cys Leu Ser Gln Glu Arg
    290                 295                 300
Ile Ile Gln Phe Gln Ala Thr Pro Cys Pro Lys Glu Pro Asn Lys Glu
305                 310                 315                 320
Met Ile Asn Asp Gly Ala Ser Trp Thr Ile Ile Ser Thr Asp Lys Ala
                325                 330                 335
Glu Tyr Thr Phe Tyr Glu Gly Met Gly Pro Val Leu Ala Pro Val Thr
            340                 345                 350
Pro Val Pro Val Val Glu Ser Leu Gln Leu Asn Gly Gly Gly Asp Val
        355                 360                 365
Ala Met Leu Glu Leu Thr Gly Gln Asn Phe Thr Pro Asn Leu Arg Val
    370                 375                 380
Trp Phe Gly Asp Val Glu Ala Glu Thr Met Tyr Arg Cys Gly Glu Ser
385                 390                 395                 400
Met Leu Cys Val Val Pro Asp Ile Ser Ala Phe Arg Glu Gly Trp Arg
                405                 410                 415
Trp Val Arg Gln Pro Val Gln Val Pro Val Thr Leu Val Arg Asn Asp
            420                 425                 430
Gly Ile Ile Tyr Ser Thr Ser Leu Thr Phe Thr Tyr Thr Pro Glu Pro
        435                 440                 445
Gly Pro Arg Pro His Cys Ser Val Ala Gly Ala Ile Leu Pro Ala Asn
    450                 455                 460
Ser Ser Gln Val Pro Pro Asn Glu Ser Asn Thr Asn Ser Glu Gly Ser
465                 470                 475                 480
Tyr Thr Asn Ala Ser Thr Asn Ser Thr Ser Val Thr Ser Ser Thr Ala
                485                 490                 495
Thr Val Val Ser
            500

<210> 149
<211> 1248
<212> DNA
<213> Homo Sapiens

<400> 149
caagatatcg aattccaaat ttgagggcct cccggctctg gcgccggagg gagagctcag      60
gccgccatgc gcgacaggac ccacgagctg agacaggggg atgacagctc ggacgaagag     120
gacaaggagc gggtcgcgct ggtggtgcac ccgggcacgg cacggctggg gagcccggac     180
gaggagttct tccacaaggt ccggacaatt cgtcagacta ttgtcaaact ggggaataaa     240
gtccaggagt tggagaaaca gcaggtcacc atcctggcca cgccccttcc cgaggagagc     300
atgaagcagg agctgcagaa cctgcgcgat gagatcaaac agctggggag ggagatccgc     360
ctgcagctga aggccataga gccccagaag gaggaagctg atgagaacta taactccgtc     420
aacacaagaa tgagaaaaac ccagcatggg gtcctgtccc agcaattcgt ggagctcatc     480
aacaagtgca attcaatgca gtccgaatac cgggagaaga acgtggagcg gattcggagg     540
cagctgaaga tcaccaatgc tggcatggtg tctgatgagg agttggatca gatgctggac     600
agtgggcaaa gcgaggtgtt tgtgtccaat atccttaagg acacgcaggt gactcgacag     660
gccttaaatg agatctcggc ccggcacagt gagatccagc agcttgaacg cagtattcgt     720
gagctgcacg acatattcac ttttctggct accgaagtgg agatgcaggg ggagatgatc     780

aatcggattg agaagaacat cctgagctca gcggactacg tggaacgtgg gcaggagcac 840
gtcaagacgg ccctggagaa ccagaagaag gtgaggaaga agaaagtctt gattgccatc 900
tgtgtgtcca tcaccgtcgt cctcctagca gtcatcattg gcgtcacagt ggttggataa 960
tgtcgcacat tgttggcact aggagcacca ggaacccagg gcctggcctt ctctcccagc 1020
agcctggggg gcaggcagag cctccagtcg gaccccttcc tcacacactg gcccctatgc 1080
agaagggcag acagttcttc tggggttggc agctgctcat tcatgatggc ctcctccttc 1140
aggcctcaat gcctggggga ggcctgcact gtcctgattg gccgggacac acggttttgt 1200
aaaaaattaa aaaacaaaaa aagagcatag aaaaaaaaaa aaccgagt 1248

<210> 150
<211> 297
<212> PRT
<213> Homo Sapiens

<400> 150
Met Arg Asp Arg Thr His Glu Leu Arg Gln Gly Asp Asp Ser Ser Asp
1               5                   10                  15
Glu Glu Asp Lys Glu Arg Val Ala Leu Val Val His Pro Gly Thr Ala
            20                  25                  30
Arg Leu Gly Ser Pro Asp Glu Glu Phe Phe His Lys Val Arg Thr Ile
        35                  40                  45
Arg Gln Thr Ile Val Lys Leu Gly Asn Lys Val Gln Glu Leu Glu Lys
    50                  55                  60
Gln Gln Val Thr Ile Leu Ala Thr Pro Leu Pro Glu Glu Ser Met Lys
65                  70                  75                  80
Gln Glu Leu Gln Asn Leu Arg Asp Glu Ile Lys Gln Leu Gly Arg Glu
                85                  90                  95
Ile Arg Leu Gln Leu Lys Ala Ile Glu Pro Gln Lys Glu Glu Ala Asp
            100                 105                 110
Glu Asn Tyr Asn Ser Val Asn Thr Arg Met Arg Lys Thr Gln His Gly
        115                 120                 125
Val Leu Ser Gln Gln Phe Val Glu Leu Ile Asn Lys Cys Asn Ser Met
    130                 135                 140
Gln Ser Glu Tyr Arg Glu Lys Asn Val Glu Arg Ile Arg Arg Gln Leu
145                 150                 155                 160
Lys Ile Thr Asn Ala Gly Met Val Ser Asp Glu Glu Leu Asp Gln Met
                165                 170                 175
Leu Asp Ser Gly Gln Ser Glu Val Phe Val Ser Asn Ile Leu Lys Asp
            180                 185                 190
Thr Gln Val Thr Arg Gln Ala Leu Asn Glu Ile Ser Ala Arg His Ser
        195                 200                 205
Glu Ile Gln Gln Leu Glu Arg Ser Ile Arg Glu Leu His Asp Ile Phe
    210                 215                 220
Thr Phe Leu Ala Thr Glu Val Glu Met Gln Gly Glu Met Ile Asn Arg
225                 230                 235                 240
Ile Glu Lys Asn Ile Leu Ser Ser Ala Asp Tyr Val Glu Arg Gly Gln
                245                 250                 255
Glu His Val Lys Thr Ala Leu Glu Asn Gln Lys Lys Val Arg Lys Lys
            260                 265                 270
Lys Val Leu Ile Ala Ile Cys Val Ser Ile Thr Val Val Leu Leu Ala
        275                 280                 285
Val Ile Ile Gly Val Thr Val Val Gly
    290                 295

<210> 151

<211> 1953
<212> DNA
<213> Homo Sapiens

<400> 151
acgcctgcca ggagcaagcc gaagagccag ccggccggcg cactccgact ccgagcagtc 60
tctgtccttc gacccgagcc ccgcgccctt tccgggaccc ctgccccgcg ggcagcgctg 120
ccaacctgcc ggccatggag accccgtccc agcggcgcgc cacccgcagc ggggcgcagg 180
ccagctccac tccgctgtcg cccacccgca tcacccggct gcaggagaag gaggacctgc 240
aggagctcaa tgatcgcttg gcggtctaca tcgaccgtgt gcgctcgctg gaaacggaga 300
acgcagggct gcgccttcgc atcaccgagt ctgaagaggt ggtcagccgc gaggtgtccg 360
gcatcaaggc cgcctacgag gccgagctcg gggatgcccg caagaccctt gactcagtag 420
ccaaggagcg cgcccgcctg cagctggagc tgagcaaagt gcgtgaggag tttaaggagc 480
tgaaagcgcg caataccaag aaggagggtg acctgatagc tgctcaggct cggctgaagg 540
acctggaggc tctgctgaac tccaaggagg ccgcactgag cactgctctc agtgagaagc 600
gcacgctgga gggcgagctg catgatctgc ggggccaggt ggccaagctt gaggcagccc 660
taggtgaggc caagaagcaa cttcaggatg agatgctgcg gcgggtggat gctgagaaca 720
ggctgcagac catgaaggag gaactggact tccagaagaa catctacagt gaggagctgc 780
gtgagaccaa gcgccgtcat gagacccgac tggtggagat tgacaatggg aagcagcgtg 840
agtttgagag ccggctggcg gatgcgctgc aggaactgcg ggcccagcat gaggaccagg 900
tggagcagta taagaaggag ctggagaaga cttattctgc caagctggac aatgccaggc 960
agtctgctga gaggaacagc aacctggtgg gggctgccca cgaggagctg cagcagtcgc 1020
gcatccgcat cgacagcctc tctgcccagc tcagccagct ccagaagcag ctggcagcca 1080
aggaggcgaa gcttcgagac ctggaggact cactggcccg tgagcgggac accagccggc 1140
ggctgctggc ggaaaaggag cgggagatgg ccgagatgcg ggcaaggatg cagcagcagc 1200
tggacgagta ccaggagctt ctggacatca agctggccct ggacatggag atccacgcct 1260
accgcaagct cttggagggc gaggaggaga ggctacgcct gtcccccagc cctacctcgc 1320
agcgcagccg tggccgtgct tcctctcact catcccagac acaggtgggg ggcagcgtca 1380
ccaaaaagcg caaactggag tccactgaga gccgcagcag cttctcacag cacgcacgca 1440
ctagcgggcg cgtggccgtg gaggaggtgg atgaggaggg caagtttgtc cggctgcgca 1500
acaagtccaa tgaggaccag tccatgggca attggcagat caagcgccag aatggagatg 1560
atcccttgct gacttaccgg ttcccaccaa agttcaccct gaaggctggg caggtggtga 1620
cgatctgggc tgcaggagct ggggccaccc acagcccccc taccgacctg gtgtggaagg 1680
cacagaacac ctggggctgc gggaacagcc tgcgtacggc tctcatcaac tccactgggg 1740
aagaagtggc catgcgcaag ctggtgcgct cagtgactgt ggttgaggac gacgaggatg 1800
aggatggaga tgacctgctc catcaccacc acgtgagtgg tagccgccgc tgaggccgag 1860
cctgcactgg ggccaccagc caggcctggg ggcagcctct ccccagcctc cccgtgccaa 1920
aaatcttttc attaaagaat gttttggaac ttt 1953

<210> 152
<211> 572
<212> PRT
<213> Homo Sapiens

<400> 152

<210> 153
<211> 1610
<212> DNA
<213> Homo Sapiens

<400> 153
ctgcaggaat tcggcacgag cggtcacgcc gagccagcgc ctgggcctgg aaccgggccg 60
tagcccccca gtttcgccca ccacctccct accatggacc cccgcaaagt gaacgagctt 120
cgggcctttg tgaaaatgtg taagcaggat ccgagcgttc tgtacaccga ggaaatgcgc 180
ttcctgaggg agtgggtgga gagcataggt ggtaaagtac cacctgctac tcagaaagct 240
atatcagaag aaaataccaa ggaagaaaaa cctgatagta agaaggtgga ggaagactta 300
aaggcagacg aaccatcaag tgaggaaagt gatctagaaa ttgataaaga aggtgtgatt 360
gaaccagaca ctgatgctcc tcaagaaatg ggagatgaaa atgcggagat aacggaggag 420
atgatggatc aggcaaatga taaaaaagtg gctgctattg aagccctaaa tgatggtgaa 480
ctccagaaag ccattgactt attcacagat gccatcaagc tgaatcctcg cttggccatt 540
ttgtatgcca agagggccag tgtcttcgtc aaattacaga agccaaatgc tgccatccga 600
gactgtgaca gagccattga aataaatcct gattcagctc agccttacaa gtggcggggg 660
aaagcacaca gacttctagg ccactgggaa gaagcagccc atgatcttgc ccttgcctgt 720
aaattggatt atgatgaaga tgctagtgca atgctgaaag aagttcaacc tagggcacag 780
aaaattgcag aacatcggag aaagtatgag cgaaaacgtg aagagcgaga gatcaaagaa 840
agaatagaac gagttaagaa ggctcgagaa gagcatgaga gagcccagag ggaggaagaa 900
gccagacgac agtcaggagc tcagtatggc tcttttccag gtggctttcc tgggggaatg 960
cctggtaatt ttcccggagg aatgcctgga atgggagggg gcatgcctgg aatggctgga 1020
atgcctggac tcaatgaaat tcttagtgat ccagaggttc ttgcagccat gcaggatcca 1080
gaagttatgg tggctttcca ggatgtggct cagaacccag caaatatgtc aaaataccag 1140
agcaacccaa aggttatgaa tctcatcagt aaattgtcag ccaaatttgg aggtcaagcg 1200
taatgtcctt ctgataaata aagcccttgc tgaaggaaaa gcaacctaga tcaccttatg 1260
gatgtcgcaa taatacaaac cagtgtacct ctgaccttct catcaagaga gctggggtgc 1320
tttgaagata atccctaccc ctctccccca aatgcagctg aagcatttta cagtggtttg 1380
ccattagggt attcattcag ataatgtttt cctactagga attacaaact ttaaacactt 1440
tttaaatctt caaaatattt aaaacaaatt taaagggcct gttaattctt atatttttct 1500
ttactaatca ttttggattt ttttctttga attattggca gggaatatac ttatgtatgg 1560
aagattactg ctctgagtga aataaaagtt attagtgcga ggcaaacata 1610

<210> 154
<211> 369
<212> PRT
<213> Homo Sapiens

<400> 154
Met Asp Pro Arg Lys Val Asn Glu Leu Arg Ala Phe Val Lys Met Cys
1               5                   10                  15
Lys Gln Asp Pro Ser Val Leu Tyr Thr Glu Glu Met Arg Phe Leu Arg
            20                  25                  30
Glu Trp Val Glu Ser Ile Gly Gly Lys Val Pro Pro Ala Thr Gln Lys
        35                  40                  45
Ala Ile Ser Glu Glu Asn Thr Lys Glu Glu Lys Pro Asp Ser Lys Lys

50                  55                  60
Val Glu Glu Asp Leu Lys Ala Asp Glu Pro Ser Ser Glu Glu Ser Asp
65                  70                  75                  80
Leu Glu Ile Asp Lys Glu Gly Val Ile Glu Pro Asp Thr Asp Ala Pro
                85                  90                  95
Gln Glu Met Gly Asp Glu Asn Ala Glu Ile Thr Glu Glu Met Met Asp
            100                 105                 110
Gln Ala Asn Asp Lys Lys Val Ala Ala Ile Glu Ala Leu Asn Asp Gly
        115                 120                 125
Glu Leu Gln Lys Ala Ile Asp Leu Phe Thr Asp Ala Ile Lys Leu Asn
    130                 135                 140
Pro Arg Leu Ala Ile Leu Tyr Ala Lys Arg Ala Ser Val Phe Val Lys
145                 150                 155                 160
Leu Gln Lys Pro Asn Ala Ala Ile Arg Asp Cys Asp Arg Ala Ile Glu
                165                 170                 175
Ile Asn Pro Asp Ser Ala Gln Pro Tyr Lys Trp Arg Gly Lys Ala His
            180                 185                 190
Arg Leu Leu Gly His Trp Glu Glu Ala Ala His Asp Leu Ala Leu Ala
        195                 200                 205
Cys Lys Leu Asp Tyr Asp Glu Asp Ala Ser Ala Met Leu Lys Glu Val
    210                 215                 220
Gln Pro Arg Ala Gln Lys Ile Ala Glu His Arg Arg Lys Tyr Glu Arg
225                 230                 235                 240
Lys Arg Glu Glu Arg Glu Ile Lys Glu Arg Ile Glu Arg Val Lys Lys
                245                 250                 255
Ala Arg Glu Glu His Glu Arg Ala Gln Arg Glu Glu Glu Ala Arg Arg
            260                 265                 270
Gln Ser Gly Ala Gln Tyr Gly Ser Phe Pro Gly Gly Phe Pro Gly Gly
        275                 280                 285
Met Pro Gly Asn Phe Pro Gly Gly Met Pro Gly Met Gly Gly Gly Met
    290                 295                 300
Pro Gly Met Ala Gly Met Pro Gly Leu Asn Glu Ile Leu Ser Asp Pro
305                 310                 315                 320
Glu Val Leu Ala Ala Met Gln Asp Pro Glu Val Met Val Ala Phe Gln
                325                 330                 335
Asp Val Ala Gln Asn Pro Ala Asn Met Ser Lys Tyr Gln Ser Asn Pro
            340                 345                 350
Lys Val Met Asn Leu Ile Ser Lys Leu Ser Ala Lys Phe Gly Gly Gln
        355                 360                 365
Ala

<210> 155
<211> 1323
<212> DNA
<213> Homo Sapiens

<400> 155
cacaaaggca ccaaaccaca aaacgtcaca cgtaaacatc atacgtggca accacaagcc      60
aatcagttgg atatttcatt cattggtata catatggact gtaaggtgtc tttcaggttg     120
cagaaaagat ggaaaaaagg acatgtgcac tctgccccaa agatgtcgaa tataatgtcc     180
tgtactttgc acaatcagag aatatagctg ctcatgagaa ttgtttgctg tattcttcag     240
gacttgtgga atgtgaggat caggatccac ttaatcctga tagaagtttt gatgtggaat     300
cagtaaagaa agaaatccag agaggaagga agttgaaatg caaattttgt cataaaagag     360
gagccaccgt gggatgtgat ttaaaaaact gtaacaagaa ttaccacttt ttctgtgcca     420

agaaggacga cgcagttcca cagtctgatg gagttcgagg aatttataaa ctgctttgcc 480
agcaacatgc tcaattcccg atcatcgctc aaagtgctaa attttcagga gtgaaaagaa 540
aaagaggaag gaagaaaccc ctctcaggca atcatgtaca gccacccgaa acaatgaaat 600
gtaatacatt cataagacaa gtgaaagaag agcatggcag acacacagat gcaactgtga 660
aagttccttt tcttaagaaa tgcaagggaa gcaggacttc ttaattactt acttgaagaa 720
atattagnca aagttcattc aattccagaa aaactcatgg atgagactta cttcagaatc 780
agactatgaa gaaatcggga gtgcactttt tgactgtaga ttgttcgaag acacatttgt 840
aaattttcaa gcagcaatag agaaaaaaat tcatgcatct caacaaaggt ggcagcagtt 900
gaaggaagag attgagctac ttcaggactt aaaacaaacc ttgtgctctt ttcaagaaaa 960
tagagatctt atgtcaagtt ctacatcaat atcatccctg tcttattagg gattaccatt 1020
tcctaagcca agagtcatgt caaattgcaa tcaggctcaa aaccagagac caggctgtga 1080
aatccacaca tctttagaac tagtcgtctc ctcttggcct cagcagctct tccctgttct 1140
tactggttga cattttgatc actctttgca cactcttgtg ttttttgctc actgtcacac 1200
tcccagcacc tagtatgctc agtaaatgtt tgtggaataa gtgcataaaa tgttcttaac 1260
ctttgattct acttacagcc catgatagcc tcttagatat aataaatttg gattatacta 1320
aaa 1323

<210> 156
<211> 191
<212> PRT
<213> Homo Sapiens

<400> 156
Met Glu Lys Arg Thr Cys Ala Leu Cys Pro Lys Asp Val Glu Tyr Asn
1               5                   10                  15
Val Leu Tyr Phe Ala Gln Ser Glu Asn Ile Ala Ala His Glu Asn Cys
            20                  25                  30
Leu Leu Tyr Ser Ser Gly Leu Val Glu Cys Glu Asp Gln Asp Pro Leu
        35                  40                  45
Asn Pro Asp Arg Ser Phe Asp Val Glu Ser Val Lys Lys Glu Ile Gln
    50                  55                  60
Arg Gly Arg Lys Leu Lys Cys Lys Phe Cys His Lys Arg Gly Ala Thr
65                  70                  75                  80
Val Gly Cys Asp Leu Lys Asn Cys Asn Lys Asn Tyr His Phe Phe Cys
                85                  90                  95
Ala Lys Lys Asp Asp Ala Val Pro Gln Ser Asp Gly Val Arg Gly Ile
            100                 105                 110
Tyr Lys Leu Leu Cys Gln Gln His Ala Gln Phe Pro Ile Ile Ala Gln
        115                 120                 125
Ser Ala Lys Phe Ser Gly Val Lys Arg Lys Arg Gly Arg Lys Lys Pro
    130                 135                 140
Leu Ser Gly Asn His Val Gln Pro Pro Glu Thr Met Lys Cys Asn Thr
145                 150                 155                 160
Phe Ile Arg Gln Val Lys Glu Glu His Gly Arg His Thr Asp Ala Thr
                165                 170                 175
Val Lys Val Pro Phe Leu Lys Lys Cys Lys Gly Ser Arg Thr Ser
            180                 185                 190

<210> 157
<211> 4065
<212> DNA
<213> Homo Sapiens

<400> 157
atgtcgactg ggacagtttt tgagactcga tttgaaaaaa tggacaacct gctgcgggat 60

cccaaatcgg aagtgaattc ggattgtttg ctggatggat tggatgcttt ggtatatgat 120
ttggattttc ctgccttaag aaaaaacaaa aatattgaca acttttaag cagatataaa 180
gacacaataa ataaaatcag agatttacga atgaaagctg aagattatga agtagtgaag 240
gtgattggta gaggtgcatt tggagaagtt caattggtaa ggcataaatc caccaggaag 300
gtatatgcta tgaagcttct cagcaaattt gaaatgataa agagatctga ttctgctttt 360
ttctgggaag aaagggacat catggctttt gccaacagtc cttgggttgt tcagcttttt 420
tatgcattcc aagatgatcg ttatctctac atggtgatgg aatacatgcc tggtggagat 480
cttgtaaact taatgagcaa ctatgatgtg cctgaaaaat gggcacgatt ctatactgca 540
gaagtagttc ttgcattgga tgcaatccat tccatgggtt ttattcacag agatgtgaag 600
cctgataaca tgctgctgga taaatctgga catttgaagt tagcagattt tggtacttgt 660
atgaagatga ataaggaagg catggtacga tgtgatacag cggttggaac acctgattat 720
atttcccctg aagtattaaa atcccaaggt ggtgatggtt attatggaag agaatgtgac 780
tggtggtcgg ttggggtatt tttatacgaa atgcttgtag gtgatacacc tttttatgca 840
gattctttgg ttggaactta cagtaaaatt atgaaccata aaaattcact tacctttcct 900
gatgataatg acatatcaaa agaagcaaaa aaccttattt gtgccttcct tactgacagg 960
gaagtgaggt tagggcgaaa tggtgtagaa gaaatcaaac gacatctctt cttcaaaaat 1020
gaccagtggg cttgggaaac gctccgagac actgtagcac cagttgtacc cgatttaagt 1080
agtgacattg atactagtaa ttttgatgac ttggaagaag ataaaggaga ggaagaaaca 1140
ttccctattc ctaaagcttt cgttggcaat caactacctt ttgtaggatt tacatattat 1200
agcaatcgta gatacttatc ttcagcaaat cctaatgata acagaactag ctccaatgca 1260
gataaaagct tgcaggaaag tttgcaaaaa acaatctata agctggaaga acagctgcat 1320
aatgaaatgc agttaaaaga tgaaatggag cagaagtgca gaacctcaaa cataaaacta 1380
gacaagataa tgaaagaatt ggatgaagag ggaaatcaaa gaagaaatct agaatctaca 1440
gtgtctcaga ttgagaagga gaaaatgttg ctacagcata gaattaatga gtaccaaaga 1500
aaagctgaac aggaaaatga gaagagaaga aatgtagaaa atgaagtttc tacattaaag 1560
gatcagttgg aagacttaaa gaaagtcagt cagaattcac agcttgctaa tgagaagctg 1620
tcccagttac aaaagcagct agaagaagcc aatgacttac ttaggacaga atcggacaca 1680
gctgtaagat tgaggaagag tcacacagag atgagcaagt caattagtca gttagagtcc 1740
ctgaacagag agttgcaaga gagaaatcga attttagaga attctaagtc acaaacagac 1800
aaagattatt accagctgca agctatatta gaagctgaac gaagagacag aggtcatgat 1860
tctgagatga ttggagacct tcaagctcga attacatctt tacaagagga ggtgaagcat 1920
ctcaaacata atctcgaaaa agtggaagga gaaagaaaag aggctcaaga catgcttaat 1980
cactcagaaa aggaaaagaa taatttagag atagatttaa actacaaact taaatcatta 2040
caacaacggt tagaacaaga ggtaaatgaa cacaaagtaa ccaaagctcg tttaactgac 2100
aaacatcaat ctattgaaga ggcaaagtct gtggcaatgt gtgagatgga aaaaaagctg 2160
aaagaagaaa gagaagctcg agagaaggct gaaaatcggg ttgttcagat tgagaaacag 2220
tgttccatgc tagacgttga tctgaagcaa tctcagcaga aactagaaca tttgactgga 2280
aataaagaaa ggatggagga tgaagttaag aatctaaccc tgcaactgga gcaggaatca 2340
aataagcggc tgttgttaca aaatgaattg aagactcaag catttgaggc agacaattta 2400
aaaggtttag aaaagcagat gaaacaggaa ataaatactt tattggaagc aaagagatta 2460
ttagaatttg agttagctca gcttacgaaa cagtatagag gaaatgaagg acagatgcgg 2520
gagctacaag atcagcttga agctgagcaa tatttctcga cactttataa aacccaggta 2580
aaggaactta aagaagaaat tgaagaaaaa aacagagaaa atttaaagaa aatacaggaa 2640
ctacaaaatg aaaaagaaac tcttgctact cagttggatc tagcagaaac aaaagctgag 2700
tctgagcagt tggcgcgagg ccttctggaa gaacagtatt ttgaattgac gcaagaaagc 2760
aagaaagctg cttcaagaaa tagacaagag attacagata aagatcacac tgttagtcgg 2820
cttgaagaag caaacagcat gctaaccaaa gatattgaaa tattaagaag agagaatgaa 2880
gagctaacag agaaaatgaa gaaggcagag gaagaatata aactggagaa ggaggaggag 2940
atcagtaatc ttaaggctgc ctttgaaaag aatatcaaca ctgaacgaac ccttaaaaca 3000
caggctgtta acaaattggc agaaataatg aatcgaaaag attttaaaat tgatagaaag 3060
aaagctaata cacaagattt gagaaagaaa gaaaaggaaa atcgaaagct gcaactggaa 3120
ctcaaccaag aaagagagaa attcaaccag atggtagtga aacatcagaa ggaactgaat 3180
gacatgcaag cgcaattggt agaagaatgt gcacatagga atgagcttca gatgcagttg 3240
gccagcaaag agagtgatat tgagcaattg cgtgctaaac ttttggacct ctcggattct 3300
acaagtgttg ctagttttcc tagtgctgat gaaactgatg gtaacctccc agagtcaaga 3360

attgaaggtt ggctttcagt accaaataga ggaaatatca aacgatatgg ctggaagaaa 3420
cagtatgttg tggtaagcag caaaaaaatt ttgttctata atgacgaaca agataaggag 3480
caatccaatc catctatggt attggacata gataaactgt ttcacgttag acctgtaacc 3540
caaggagatg tgtatagagc tgaaactgaa gaaattccta aaatattcca gatactatat 3600
gcaaatgaag gtgaatgtag aaaagatgta gagatggaac cagtacaaca agctgaaaaa 3660
actaatttcc aaaatcacaa aggccatgag tttattccta cactctacca ctttcctgcc 3720
aattgtgatg cctgtgccaa acctctctgg catgttttta agccaccccc tgccctagag 3780
tgtcgaagat gccatgttaa gtgccacaga gatcacttag ataagaaaga ggacttaatt 3840
tgtccatgta aagtaagtta tgatgtaaca tcagcaagag atatgctgct gttagcatgt 3900
tctcaggatg aacaaaaaaa atgggtaact catttagtaa agaaaatccc taagaatcca 3960
ccatctggtt ttgttcgtgc ttcccctcga acgctttcta caagatccac tgcaaatcag 4020
tctttccgga aagtggtcaa aaatacatct ggaaaaacta gttaa 4065

<210> 158
<211> 1354
<212> PRT
<213> Homo Sapiens

<400> 158
Met Ser Thr Gly Asp Ser Phe Glu Thr Arg Phe Glu Lys Met Asp Asn
1               5                   10                  15
Leu Leu Arg Asp Pro Lys Ser Glu Val Asn Ser Asp Cys Leu Leu Asp
            20                  25                  30
Gly Leu Asp Ala Leu Val Tyr Asp Leu Asp Phe Pro Ala Leu Arg Lys
        35                  40                  45
Asn Lys Asn Ile Asp Asn Phe Leu Ser Arg Tyr Lys Asp Thr Ile Asn
    50                  55                  60
Lys Ile Arg Asp Leu Arg Met Lys Ala Glu Asp Tyr Glu Val Val Lys
65                  70                  75                  80
Val Ile Gly Arg Gly Ala Phe Gly Glu Val Gln Leu Val Arg His Lys
                85                  90                  95
Ser Thr Arg Lys Val Tyr Ala Met Lys Leu Leu Ser Lys Phe Glu Met
            100                 105                 110
Ile Lys Arg Ser Asp Ser Ala Phe Phe Trp Glu Glu Arg Asp Ile Met
        115                 120                 125
Ala Phe Ala Asn Ser Pro Trp Val Val Gln Leu Phe Tyr Ala Phe Gln
    130                 135                 140
Asp Asp Arg Tyr Leu Tyr Met Val Met Glu Tyr Met Pro Gly Gly Asp
145                 150                 155                 160
Leu Val Asn Leu Met Ser Asn Tyr Asp Val Pro Glu Lys Trp Ala Arg
                165                 170                 175
Phe Tyr Thr Ala Glu Val Val Leu Ala Leu Asp Ala Ile His Ser Met
            180                 185                 190
Gly Phe Ile His Arg Asp Val Lys Pro Asp Asn Met Leu Leu Asp Lys
        195                 200                 205
Ser Gly His Leu Lys Leu Ala Asp Phe Gly Thr Cys Met Lys Met Asn
    210                 215                 220
Lys Glu Gly Met Val Arg Cys Asp Thr Ala Val Gly Thr Pro Asp Tyr
225                 230                 235                 240
Ile Ser Pro Glu Val Leu Lys Ser Gln Gly Gly Asp Gly Tyr Tyr Gly
                245                 250                 255
Arg Glu Cys Asp Trp Trp Ser Val Gly Val Phe Leu Tyr Glu Met Leu
            260                 265                 270
Val Gly Asp Thr Pro Phe Tyr Ala Asp Ser Leu Val Gly Thr Tyr Ser
        275                 280                 285

Lys Ile Met Asn His Lys Asn Ser Leu Thr Phe Pro Asp Asp Asn Asp
    290                 295                 300
Ile Ser Lys Glu Ala Lys Asn Leu Ile Cys Ala Phe Leu Thr Asp Arg
305                 310                 315                 320
Glu Val Arg Leu Gly Arg Asn Gly Val Glu Glu Ile Lys Arg His Leu
                325                 330                 335
Phe Phe Lys Asn Asp Gln Trp Ala Trp Glu Thr Leu Arg Asp Thr Val
            340                 345                 350
Ala Pro Val Val Pro Asp Leu Ser Ser Asp Ile Asp Thr Ser Asn Phe
        355                 360                 365
Asp Asp Leu Glu Glu Asp Lys Gly Glu Glu Glu Thr Phe Pro Ile Pro
    370                 375                 380
Lys Ala Phe Val Gly Asn Gln Leu Pro Phe Val Gly Phe Thr Tyr Tyr
385                 390                 395                 400
Ser Asn Arg Arg Tyr Leu Ser Ser Ala Asn Pro Asn Asp Asn Arg Thr
                405                 410                 415
Ser Ser Asn Ala Asp Lys Ser Leu Gln Glu Ser Leu Gln Lys Thr Ile
            420                 425                 430
Tyr Lys Leu Glu Glu Gln Leu His Asn Glu Met Gln Leu Lys Asp Glu
        435                 440                 445
Met Glu Gln Lys Cys Arg Thr Ser Asn Ile Lys Leu Asp Lys Ile Met
    450                 455                 460
Lys Glu Leu Asp Glu Glu Gly Asn Gln Arg Arg Asn Leu Glu Ser Thr
465                 470                 475                 480
Val Ser Gln Ile Glu Lys Glu Lys Met Leu Leu Gln His Arg Ile Asn
                485                 490                 495
Glu Tyr Gln Arg Lys Ala Glu Gln Glu Asn Glu Lys Arg Arg Asn Val
            500                 505                 510
Glu Asn Glu Val Ser Thr Leu Lys Asp Gln Leu Glu Asp Leu Lys Lys
        515                 520                 525
Val Ser Gln Asn Ser Gln Leu Ala Asn Glu Lys Leu Ser Gln Leu Gln
    530                 535                 540
Lys Gln Leu Glu Glu Ala Asn Asp Leu Leu Arg Thr Glu Ser Asp Thr
545                 550                 555                 560
Ala Val Arg Leu Arg Lys Ser His Thr Glu Met Ser Lys Ser Ile Ser
                565                 570                 575
Gln Leu Glu Ser Leu Asn Arg Glu Leu Gln Glu Arg Asn Arg Ile Leu
            580                 585                 590
Glu Asn Ser Lys Ser Gln Thr Asp Lys Asp Tyr Tyr Gln Leu Gln Ala
        595                 600                 605
Ile Leu Glu Ala Glu Arg Arg Asp Arg Gly His Asp Ser Glu Met Ile
    610                 615                 620
Gly Asp Leu Gln Ala Arg Ile Thr Ser Leu Gln Glu Glu Val Lys His
625                 630                 635                 640
Leu Lys His Asn Leu Glu Lys Val Glu Gly Glu Arg Lys Glu Ala Gln
                645                 650                 655
Asp Met Leu Asn His Ser Glu Lys Glu Lys Asn Asn Leu Glu Ile Asp
            660                 665                 670
Leu Asn Tyr Lys Leu Lys Ser Leu Gln Gln Arg Leu Glu Gln Glu Val
        675                 680                 685
Asn Glu His Lys Val Thr Lys Ala Arg Leu Thr Asp Lys His Gln Ser
    690                 695                 700
Ile Glu Glu Ala Lys Ser Val Ala Met Cys Glu Met Glu Lys Lys Leu
705                 710                 715                 720
Lys Glu Glu Arg Glu Ala Arg Glu Lys Ala Glu Asn Arg Val Val Gln

                725                 730                 735
Ile Glu Lys Gln Cys Ser Met Leu Asp Val Asp Leu Lys Gln Ser Gln
            740                 745                 750
Gln Lys Leu Glu His Leu Thr Gly Asn Lys Glu Arg Met Glu Asp Glu
        755                 760                 765
Val Lys Asn Leu Thr Leu Gln Leu Glu Gln Glu Ser Asn Lys Arg Leu
    770                 775                 780
Leu Leu Gln Asn Glu Leu Lys Thr Gln Ala Phe Glu Ala Asp Asn Leu
785                 790                 795                 800
Lys Gly Leu Glu Lys Gln Met Lys Gln Glu Ile Asn Thr Leu Leu Glu
                805                 810                 815
Ala Lys Arg Leu Leu Glu Phe Glu Leu Ala Gln Leu Thr Lys Gln Tyr
            820                 825                 830
Arg Gly Asn Glu Gly Gln Met Arg Glu Leu Gln Asp Gln Leu Glu Ala
        835                 840                 845
Glu Gln Tyr Phe Ser Thr Leu Tyr Lys Thr Gln Val Lys Glu Leu Lys
    850                 855                 860
Glu Glu Ile Glu Glu Lys Asn Arg Glu Asn Leu Lys Lys Ile Gln Glu
865                 870                 875                 880
Leu Gln Asn Glu Lys Glu Thr Leu Ala Thr Gln Leu Asp Leu Ala Glu
                885                 890                 895
Thr Lys Ala Glu Ser Glu Gln Leu Ala Arg Gly Leu Leu Glu Glu Gln
            900                 905                 910
Tyr Phe Glu Leu Thr Gln Glu Ser Lys Lys Ala Ala Ser Arg Asn Arg
        915                 920                 925
Gln Glu Ile Thr Asp Lys Asp His Thr Val Ser Arg Leu Glu Glu Ala
    930                 935                 940
Asn Ser Met Leu Thr Lys Asp Ile Glu Ile Leu Arg Arg Glu Asn Glu
945                 950                 955                 960
Glu Leu Thr Glu Lys Met Lys Lys Ala Glu Glu Glu Tyr Lys Leu Glu
                965                 970                 975
Lys Glu Glu Glu Ile Ser Asn Leu Lys Ala Ala Phe Glu Lys Asn Ile
            980                 985                 990
Asn Thr Glu Arg Thr Leu Lys Thr Gln Ala Val Asn Lys Leu Ala Glu
        995                 1000                1005
Ile Met Asn Arg Lys Asp Phe Lys Ile Asp Arg Lys Lys Ala Asn Thr
    1010                1015                1020
Gln Asp Leu Arg Lys Lys Glu Lys Glu Asn Arg Lys Leu Gln Leu Glu
1025 1030 1035 104
Leu Asn Gln Glu Arg Glu Lys Phe Asn Gln Met Val Val Lys His Gln
                1045                1050                1055
Lys Glu Leu Asn Asp Met Gln Ala Gln Leu Val Glu Glu Cys Ala His
            1060                1065                1070
Arg Asn Glu Leu Gln Met Gln Leu Ala Ser Lys Glu Ser Asp Ile Glu
        1075                1080                1085
Gln Leu Arg Ala Lys Leu Leu Asp Leu Ser Asp Ser Thr Ser Val Ala
    1090                1095                1100
Ser Phe Pro Ser Ala Asp Glu Thr Asp Gly Asn Leu Pro Glu Ser Arg
1105 1110 1115 112
Ile Glu Gly Trp Leu Ser Val Pro Asn Arg Gly Asn Ile Lys Arg Tyr
                1125                1130                1135
Gly Trp Lys Lys Gln Tyr Val Val Val Ser Ser Lys Lys Ile Leu Phe
            1140                1145                1150
Tyr Asn Asp Glu Gln Asp Lys Glu Gln Ser Asn Pro Ser Met Val Leu
        1155                1160                1165

Asp Ile Asp Lys Leu Phe His Val Arg Pro Val Thr Gln Gly Asp Val
    1170                1175                1180
Tyr Arg Ala Glu Thr Glu Glu Ile Pro Lys Ile Phe Gln Ile Leu Tyr
1185                1190                1195                120
Ala Asn Glu Gly Glu Cys Arg Lys Asp Val Glu Met Glu Pro Val Gln
                1205                1210                1215
Gln Ala Glu Lys Thr Asn Phe Gln Asn His Lys Gly His Glu Phe Ile
            1220                1225                1230
Pro Thr Leu Tyr His Phe Pro Ala Asn Cys Asp Ala Cys Ala Lys Pro
        1235                1240                1245
Leu Trp His Val Phe Lys Pro Pro Pro Ala Leu Glu Cys Arg Arg Cys
    1250                1255                1260
His Val Lys Cys His Arg Asp His Leu Asp Lys Lys Glu Asp Leu Ile
1265                1270                1275                128
Cys Pro Cys Lys Val Ser Tyr Asp Val Thr Ser Ala Arg Asp Met Leu
                1285                1290                1295
Leu Leu Ala Cys Ser Gln Asp Glu Gln Lys Lys Trp Val Thr His Leu
            1300                1305                1310
Val Lys Lys Ile Pro Lys Asn Pro Pro Ser Gly Phe Val Arg Ala Ser
        1315                1320                1325
Pro Arg Thr Leu Ser Thr Arg Ser Thr Ala Asn Gln Ser Phe Arg Lys
    1330                1335                1340
Val Val Lys Asn Thr Ser Gly Lys Thr Ser
1345                1350

<210> 159
<211> 683
<212> DNA
<213> Homo Sapiens

<400> 159
acaagctgga gttcgagcct gacagtgagg acaagatctc ggactgtgag gaaggattga      60
gtaatgtggc acttgaatgc agtgagccaa gcacaagtgt atctgcttat gaccagttga     120
aggcaccggc atcccctggt gctggaaacc cacctgggac cccaaaggga aagagagagc     180
tgatgagcaa tggcccaggt tccattattg gtgctaaagc tgggaagaat tctggcaaaa     240
agaagggcct taacaatgaa ctgaacaacc ttccagtaat ctccaacatg acggctgcgt     300
tagacagttg ctcggcagca gacggcagtt tggctgctga gatgcctaaa ctggaagcag     360
aaggattaat tgacaagaaa aatttaggag ataaagaaaa gggcaaaaaa gctaacaact     420
gcaaaacgga caaaaacctc tctaaactga aaagtgcccg gcccattgcc cctgccccag     480
cccccactcc cccgcagcta atcgctatac ccactgcaac ctttacaacg accaccactg     540
ggacaatacc cggactgccc tccctcacaa caactgttgt tcaggctaca ccaaagagtc     600
ctccgttaaa acccattcaa ccaaagccca caattatggg agagcccatc accgtgaacc     660
cagctctggt gtcactcaaa gac                                             683

<210> 160
<211> 227
<212> PRT
<213> Homo Sapiens

<400> 160
Lys Leu Glu Phe Glu Pro Asp Ser Glu Asp Lys Ile Ser Asp Cys Glu
1               5                   10                  15
Glu Gly Leu Ser Asn Val Ala Leu Glu Cys Ser Glu Pro Ser Thr Ser
            20                  25                  30
Val Ser Ala Tyr Asp Gln Leu Lys Ala Pro Ala Ser Pro Gly Ala Gly

35                  40                  45
Asn Pro Pro Gly Thr Pro Lys Gly Lys Arg Glu Leu Met Ser Asn Gly
    50                  55                  60
Pro Gly Ser Ile Ile Gly Ala Lys Ala Gly Lys Asn Ser Gly Lys Lys
65                  70                  75                  80
Lys Gly Leu Asn Asn Glu Leu Asn Asn Leu Pro Val Ile Ser Asn Met
                85                  90                  95
Thr Ala Ala Leu Asp Ser Cys Ser Ala Ala Asp Gly Ser Leu Ala Ala
            100                 105                 110
Glu Met Pro Lys Leu Glu Ala Glu Gly Leu Ile Asp Lys Lys Asn Leu
        115                 120                 125
Gly Asp Lys Glu Lys Gly Lys Lys Ala Asn Asn Cys Lys Thr Asp Lys
    130                 135                 140
Asn Leu Ser Lys Leu Lys Ser Ala Arg Pro Ile Ala Pro Ala Pro Ala
145                 150                 155                 160
Pro Thr Pro Pro Gln Leu Ile Ala Ile Pro Thr Ala Thr Phe Thr Thr
                165                 170                 175
Thr Thr Thr Gly Thr Ile Pro Gly Leu Pro Ser Leu Thr Thr Thr Val
            180                 185                 190
Val Gln Ala Thr Pro Lys Ser Pro Pro Leu Lys Pro Ile Gln Pro Lys
        195                 200                 205
Pro Thr Ile Met Gly Glu Pro Ile Thr Val Asn Pro Ala Leu Val Ser
    210                 215                 220
Leu Lys Asp
225

<210> 161
<211> 662
<212> DNA
<213> Homo Sapiens

<400> 161
acccacagca gttgcacttg ctgagcaggc agcttgagga cccaaatggt agcttttcta      60
acgctgagat gagtgaactg agtgtggcac agaaaccaga aaaacttttg gagcgctgca     120
agtactggcc tgcttgtaaa aatggggatg agtgtgccta ccatcacccc atctcaccct     180
gcaaagcctt ccccaattgt aaatttgctg aaaaatgttt gtttgttcac ccaaattgta     240
aatatgatgc aaagtgtact aaaccagatt gtcccttcac tcatgtgagt agaagaattc     300
cagtactgtc tccaaaacca gttgcaccac cagcaccacc ttccagtagt cagctctgcc     360
gttacttccc tgcttgtaag aagatggaat gtcccttcta tcatccaaaa cattgtaggt     420
ttaacactca atgtacaaga ccggactgca cattctacca tcccaccatt aatgtcccac     480
cacgacatgc cttgaaatgg attcgacctc aaaccagcga atagcaccca gtcctgcctg     540
gcagaagatc atgcagtttg gaagttttca tgtctgatga aagatctcta cagaacttgt     600
caaatctttg aaacttggaa tatattgctt tcataatatg aaggtttatt ggctatctaa     660
aa                                                                    662

<210> 162
<211> 173
<212> PRT
<213> Homo Sapiens

<400> 162
Pro Gln Gln Leu His Leu Leu Ser Arg Gln Leu Glu Asp Pro Asn Gly
1               5                   10                  15
Ser Phe Ser Asn Ala Glu Met Ser Glu Leu Ser Val Ala Gln Lys Pro
            20                  25                  30

Glu Lys Leu Leu Glu Arg Cys Lys Tyr Trp Pro Ala Cys Lys Asn Gly
        35                  40                  45
Asp Glu Cys Ala Tyr His His Pro Ile Ser Pro Cys Lys Ala Phe Pro
    50                  55                  60
Asn Cys Lys Phe Ala Glu Lys Cys Leu Phe Val His Pro Asn Cys Lys
65                  70                  75                  80
Tyr Asp Ala Lys Cys Thr Lys Pro Asp Cys Pro Phe Thr His Val Ser
                85                  90                  95
Arg Arg Ile Pro Val Leu Ser Pro Lys Pro Val Ala Pro Pro Ala Pro
            100                 105                 110
Pro Ser Ser Ser Gln Leu Cys Arg Tyr Phe Pro Ala Cys Lys Lys Met
        115                 120                 125
Glu Cys Pro Phe Tyr His Pro Lys His Cys Arg Phe Asn Thr Gln Cys
    130                 135                 140
Thr Arg Pro Asp Cys Thr Phe Tyr His Pro Thr Ile Asn Val Pro Pro
145                 150                 155                 160
Arg His Ala Leu Lys Trp Ile Arg Pro Gln Thr Ser Glu
                165                 170

<210> 163
<211> 2912
<212> DNA
<213> Homo Sapiens

<400> 163
cagttgcttc agcgtcccgg tgtggctgtg ccgttggtcc tgtgcggtca cttagccaag      60
atgcctgagg aaacccagac ccaagaccaa ccgatggagg aggaggaggt tgagacgttc     120
gcctttcagg cagaaattgc ccagttgatg tcattgatca tcaatacttt ctactcgaac     180
aaagagatct ttctgagaga gctcatttca aattcatcag atgcattgga caaaatccgg     240
tatgaaactt tgacagatcc cagtaaatta gactctggga aagagctgca tattaacctt     300
ataccgaaca aacaagatcg aactctcact attgtggata ctggaattgg aatgaccaag     360
gctgacttga tcaataacct tggtactatc gccaagtctg ggaccaaagc gttcatggaa     420
gctttgcagg ctggtgcaga tatctctatg attggccagt tcggtgttgg tttttattct     480
gcttatttgg ttgctgagaa agtaactgtg atcaccaaac ataacgatga tgagcagtac     540
gcttgggagt cctcagcagg gggatcattc acagtgagga cagacacagg tgaacctatg     600
ggtcgtggaa caaaagttat cctacacctg aaagaagacc aaactgagta cttggaggaa     660
cgaagaataa aggagattgt gaagaaacat tctcagttta ttggatatcc cattactctt     720
tttgtggaga aggaacgtga taaagaagta agcgatgatg aggctgaaga aaaggaagac     780
aaagaagaag aaaaagaaaa agaagagaaa gagtcggaag acaaacctga aattgaagat     840
gttggttctg atgaggaaga agaaaagaag gatggtgaca agaagaagaa gaagaagatt     900
aaggaaaagt acatcgatca agaagagctc aacaaaacaa agcccatctg gaccagaaat     960
cccgacgata ttactaatga ggagtacgga gaattctata agagcttgac caatgactgg    1020
gaagatcact tggcagtgaa gcatttttca gttgaaggac agttggaatt cagagccctt    1080
ctatttgtcc cacgacgtgc tccttttgat ctgtttgaaa acagaaagaa aaagaacaat    1140
atcaaattgt atgtacgcag agttttcatc atggataact gtgaggagct aatccctgaa    1200
tatctgaact tcattagagg ggtggtagac tcggaggatc tccctctaaa catatcccgt    1260
gagatgttgc aacaaagcaa aattttgaaa gttatcagga agaatttggt caaaaaatgc    1320
ttagaactct ttactgaact ggcggaagat aaagagaact acaagaaatt ctatgagcag    1380
ttctctaaaa acataaagct tggaatacac gaagactctc aaaatcggaa gaagctttca    1440
gagctgttaa ggtactacac atctgcctct ggtgatgaga tggtttctct caaggactac    1500
tgcaccagaa tgaaggagaa ccagaaacat atctattata tcacaggtga gaccaaggac    1560
caggtagcta actcagcctt tgtggaacgt cttcggaaac atggcttaga agtgatctat    1620
atgattgagc ccattgatga gtactgtgtc caacagctga aggaatttga ggggaagact    1680
ttagtgtcag tcaccaaaga aggcctggaa cttccagagg atgaagaaga gaaaaagaag    1740
caggaagaga aaaaaacaaa gtttgagaac ctctgcaaaa tcatgaaaga catattggag    1800

aaaaaagttg aaaaggtggt tgtgtcaaac cgattggtga catctccatg ctgtattgtc 1860
acaagcacat atggctggac agcaaacatg gagagaatca tgaaagctca agccctaaga 1920
gacaactcaa caatgggtta catggcagca aagaaacacc tggagataaa ccctgaccat 1980
tccattattg agaccttaag gcaaaaggca gaggctgata agaacgacaa gtctgtgaag 2040
gatctggtca tcttgcttta tgaaactgcg ctcctgtctt ctggcttcag tctggaagat 2100
ccccagacac atgctaacag gatctacagg atgatcaaac ttggtctggg tattgatgaa 2160
gatgacccta ctgctgatga taccagtgct gctgtaactg aagaaatgcc accccttgaa 2220
ggagatgacg acacatcacg catggaagaa gtagactaat ctctggctga gggatgactt 2280
acctgttcag tactctacaa ttcctctgat aatatatttt caaggatgtt tttctttatt 2340
tttgttaata ttaaaaagtc tgtatggcat gacaactact ttaaggggaa gataagattt 2400
ctgtctacta agtgatgctg tgatacctta ggcactaaag cagagctagt aatgcttttt 2460
gagtttcatg ttggttcttt cacagatggg gtaacgtgca ctgtaagacg tatgtaacat 2520
gatgttaact ttgtgtggtc taaagtgttt agctgtcaag ccggatgcct aagtagacca 2580
aatcttgtta ttgaagtgtt ctgagctgta tcttgatgtt tagaaaagta ttcgttacat 2640
cttgtaggat ctactttttg aacttttcat tccctgtagt tgacaattct gcatgtacta 2700
gtcctctaga aataggttaa actgaagcaa cttgatggaa ggatctctcc acagggcttg 2760
ttttccaaag aaaagtattg tttggaggag caaagttaaa agcctaccta agcatatcgt 2820
aaagctgttc aaatactcga gcccagtctt gtggatggaa atgtagtgct cgagtcacat 2880
tctgcttaaa gttgtaacaa atacagatga gt 2912

<210> 164
<211> 732
<212> PRT
<213> Homo Sapiens

<400> 164
Met Pro Glu Glu Thr Gln Thr Gln Asp Gln Pro Met Glu Glu Glu Glu
1               5                   10                  15
Val Glu Thr Phe Ala Phe Gln Ala Glu Ile Ala Gln Leu Met Ser Leu
            20                  25                  30
Ile Ile Asn Thr Phe Tyr Ser Asn Lys Glu Ile Phe Leu Arg Glu Leu
        35                  40                  45
Ile Ser Asn Ser Ser Asp Ala Leu Asp Lys Ile Arg Tyr Glu Thr Leu
    50                  55                  60
Thr Asp Pro Ser Lys Leu Asp Ser Gly Lys Glu Leu His Ile Asn Leu
65                  70                  75                  80
Ile Pro Asn Lys Gln Asp Arg Thr Leu Thr Ile Val Asp Thr Gly Ile
                85                  90                  95
Gly Met Thr Lys Ala Asp Leu Ile Asn Asn Leu Gly Thr Ile Ala Lys
            100                 105                 110
Ser Gly Thr Lys Ala Phe Met Glu Ala Leu Gln Ala Gly Ala Asp Ile
        115                 120                 125
Ser Met Ile Gly Gln Phe Gly Val Gly Phe Tyr Ser Ala Tyr Leu Val
    130                 135                 140
Ala Glu Lys Val Thr Val Ile Thr Lys His Asn Asp Asp Glu Gln Tyr
145                 150                 155                 160
Ala Trp Glu Ser Ser Ala Gly Gly Ser Phe Thr Val Arg Thr Asp Thr
                165                 170                 175
Gly Glu Pro Met Gly Arg Gly Thr Lys Val Ile Leu His Leu Lys Glu
            180                 185                 190
Asp Gln Thr Glu Tyr Leu Glu Glu Arg Arg Ile Lys Glu Ile Val Lys
        195                 200                 205
Lys His Ser Gln Phe Ile Gly Tyr Pro Ile Thr Leu Phe Val Glu Lys
    210                 215                 220
Glu Arg Asp Lys Glu Val Ser Asp Asp Glu Ala Glu Glu Lys Glu Asp

225                 230                 235                 240
Lys Glu Glu Glu Lys Glu Lys Glu Glu Lys Glu Ser Glu Asp Lys Pro
                245                 250                 255
Glu Ile Glu Asp Val Gly Ser Asp Glu Glu Glu Glu Lys Lys Asp Gly
                260                 265                 270
Asp Lys Lys Lys Lys Lys Lys Ile Lys Glu Lys Tyr Ile Asp Gln Glu
        275                 280                 285
Glu Leu Asn Lys Thr Lys Pro Ile Trp Thr Arg Asn Pro Asp Asp Ile
    290                 295                 300
Thr Asn Glu Glu Tyr Gly Glu Phe Tyr Lys Ser Leu Thr Asn Asp Trp
305                 310                 315                 320
Glu Asp His Leu Ala Val Lys His Phe Ser Val Glu Gly Gln Leu Glu
                325                 330                 335
Phe Arg Ala Leu Leu Phe Val Pro Arg Arg Ala Pro Phe Asp Leu Phe
                340                 345                 350
Glu Asn Arg Lys Lys Lys Asn Asn Ile Lys Leu Tyr Val Arg Arg Val
        355                 360                 365
Phe Ile Met Asp Asn Cys Glu Glu Leu Ile Pro Glu Tyr Leu Asn Phe
    370                 375                 380
Ile Arg Gly Val Val Asp Ser Glu Asp Leu Pro Leu Asn Ile Ser Arg
385                 390                 395                 400
Glu Met Leu Gln Gln Ser Lys Ile Leu Lys Val Ile Arg Lys Asn Leu
                405                 410                 415
Val Lys Lys Cys Leu Glu Leu Phe Thr Glu Leu Ala Glu Asp Lys Glu
            420                 425                 430
Asn Tyr Lys Lys Phe Tyr Glu Gln Phe Ser Lys Asn Ile Lys Leu Gly
        435                 440                 445
Ile His Glu Asp Ser Gln Asn Arg Lys Lys Leu Ser Glu Leu Leu Arg
    450                 455                 460
Tyr Tyr Thr Ser Ala Ser Gly Asp Glu Met Val Ser Leu Lys Asp Tyr
465                 470                 475                 480
Cys Thr Arg Met Lys Glu Asn Gln Lys His Ile Tyr Tyr Ile Thr Gly
                485                 490                 495
Glu Thr Lys Asp Gln Val Ala Asn Ser Ala Phe Val Glu Arg Leu Arg
            500                 505                 510
Lys His Gly Leu Glu Val Ile Tyr Met Ile Glu Pro Ile Asp Glu Tyr
        515                 520                 525
Cys Val Gln Gln Leu Lys Glu Phe Glu Gly Lys Thr Leu Val Ser Val
    530                 535                 540
Thr Lys Glu Gly Leu Glu Leu Pro Glu Asp Glu Glu Glu Lys Lys Lys
545                 550                 555                 560
Gln Glu Glu Lys Lys Thr Lys Phe Glu Asn Leu Cys Lys Ile Met Lys
                565                 570                 575
Asp Ile Leu Glu Lys Lys Val Glu Lys Val Val Val Ser Asn Arg Leu
            580                 585                 590
Val Thr Ser Pro Cys Cys Ile Val Thr Ser Thr Tyr Gly Trp Thr Ala
        595                 600                 605
Asn Met Glu Arg Ile Met Lys Ala Gln Ala Leu Arg Asp Asn Ser Thr
    610                 615                 620
Met Gly Tyr Met Ala Ala Lys Lys His Leu Glu Ile Asn Pro Asp His
625                 630                 635                 640
Ser Ile Ile Glu Thr Leu Arg Gln Lys Ala Glu Ala Asp Lys Asn Asp
                645                 650                 655
Lys Ser Val Lys Asp Leu Val Ile Leu Leu Tyr Glu Thr Ala Leu Leu
            660                 665                 670

Ser Ser Gly Phe Ser Leu Glu Asp Pro Gln Thr His Ala Asn Arg Ile
        675                 680                 685
Tyr Arg Met Ile Lys Leu Gly Leu Gly Ile Asp Glu Asp Asp Pro Thr
    690                 695                 700
Ala Asp Asp Thr Ser Ala Ala Val Thr Glu Glu Met Pro Pro Leu Glu
705                 710                 715                 720
Gly Asp Asp Asp Thr Ser Arg Met Glu Glu Val Asp
                725                 730

<210> 165
<211> 790
<212> DNA
<213> Homo Sapiens

<400> 165
ccgactcaga aatggcggcc tccatgttct acggcaggct agtggccgtg gccacccttc 60
ggaaccaccg gcctcggacg gcccagcggg ctgctgctca ggttctggga agttctggat 120
tgtttaataa ccatggactc caagtacagc agcaacagca aaggaatctc tcactacatg 180
aatacatgag tatggaatta ttgcaagaag ctggtgtctc cgttcccaaa ggatatgtgg 240
caaagtcacc agatgaagct tatgcaattg ccaaaaaatt aggttcaaaa gatgtcgtga 300
taaaggcaca ggttttagct ggtggtagag gaaaaggaac atttgaaagt ggcctcaaag 360
gaggagtgaa gatagttttc tctccagaag aagcaaaagc tgtttcttca caaatgattg 420
ggaaaaaatt gtttaccaag caaacgggag aaaagggcag aatatgcaat caagtattgg 480
tctgtgagcg aaaatatccc aggagagaat actactttgc aataacaatg gaaaggtcat 540
ttcaaggtcc tgtattaata ggaagttcac atggtggtgt caacattgaa gatgttgctg 600
ctgagtctcc tgaagcaata attaaagaac ctattgatat tgaagaaggc atcaaaaagg 660
aacaagctct tcagcttgca cagaagaatg ggatttcccc taatattgng ggaatcagca 720
gcaggaaaac atggtcaagc tttacagncn ttttcttgaa atacgatgca acccttgata 780
ggaaattaaa 790

<210> 166
<211> 259
<212> PRT
<213> Homo Sapiens

<400> 166
Asp Ser Glu Met Ala Ala Ser Met Phe Tyr Gly Arg Leu Val Ala Val
1               5                   10                  15
Ala Thr Leu Arg Asn His Arg Pro Arg Thr Ala Gln Arg Ala Ala Ala
            20                  25                  30
Gln Val Leu Gly Ser Ser Gly Leu Phe Asn Asn His Gly Leu Gln Val
        35                  40                  45
Gln Gln Gln Gln Gln Arg Asn Leu Ser Leu His Glu Tyr Met Ser Met
    50                  55                  60
Glu Leu Leu Gln Glu Ala Gly Val Ser Val Pro Lys Gly Tyr Val Ala
65                  70                  75                  80
Lys Ser Pro Asp Glu Ala Tyr Ala Ile Ala Lys Lys Leu Gly Ser Lys
                85                  90                  95
Asp Val Val Ile Lys Ala Gln Val Leu Ala Gly Gly Arg Gly Lys Gly
            100                 105                 110
Thr Phe Glu Ser Gly Leu Lys Gly Gly Val Lys Ile Val Phe Ser Pro
        115                 120                 125
Glu Glu Ala Lys Ala Val Ser Ser Gln Met Ile Gly Lys Lys Leu Phe
    130                 135                 140
Thr Lys Gln Thr Gly Glu Lys Gly Arg Ile Cys Asn Gln Val Leu Val

145                 150                 155                 160
Cys Glu Arg Lys Tyr Pro Arg Arg Glu Tyr Tyr Phe Ala Ile Thr Met
                165                 170                 175
Glu Arg Ser Phe Gln Gly Pro Val Leu Ile Gly Ser Ser His Gly Gly
            180                 185                 190
Val Asn Ile Glu Asp Val Ala Ala Glu Ser Pro Glu Ala Ile Ile Lys
        195                 200                 205
Glu Pro Ile Asp Ile Glu Glu Gly Ile Lys Lys Glu Gln Ala Leu Gln
    210                 215                 220
Leu Ala Gln Lys Asn Gly Ile Ser Pro Asn Ile Gly Ile Ser Ser Arg
225                 230                 235                 240
Lys Thr Trp Ser Ser Phe Thr Phe Leu Lys Tyr Asp Ala Thr Leu Asp
                245                 250                 255
Arg Lys Leu

<210> 167
<211> 5307
<212> DNA
<213> Homo Sapiens

<400> 167
   gaataacagt taagttttgg ggagtaaaaa ctgtttcaat ttttgactgt gttgggggtt      60
   ggtgctccta atccctgtgt tgttaaaggg tcgactatat tgtatttttg aaaattgcta     120
   gagagtggac gtaaagtgtt ctcactaaac aaattataac tatgtgaggt agtgcatata     180
   ttaagtagct agatttggtc attccacaat gtatatgtac ttcaaaacat catgttgtac     240
   atgagaaaca cagttttatc tgttagtcag ttttaaaaat aaaaaatatt ccaactagaa     300
   actctgttgt agtttttgaa attacaactt ggaggctttg aggaactgat tagaagtctc     360
   ctttctgttt caggctttca tatccaaacc atagatcttt agaagtaaca tctgttaatt     420
   aattattaat aaatagtttg agtctttatt aattcatgga taacttgacc attttctctc      480
   tccttttgct tagataatcc cagatcatgg ccgggcacag tagctcacgc ctgtattccc     540
   agcagtttgg gaggccgagg caggcagatc acttgaactc aggagtttga gaccagcttg     600
   ggcaacatgg caaaaccctg tctctattaa aaatacaaaa attagctggg catggtagtg     660
   catgcctgta gtcccagcta cctgggaggc tgaggtggga ggatcgcttg agcctgggag     720
   gttgaggctt ctgtgcgcga tgattgctcc agtgatcacg ccattgcact ccagcctggg     780
   tgacagagtg agaccctgtc tccaaaaaaa aaaaaaatta agcaagtagc agttacaaga     840
   ccaaaagtta ttttcctttt tttttttctc tataaaattg cccatttgga ccaaatctag      900
   ttataactta tttcagtgtc attaagaaag ttgatgaata agtcatatta ctcagatgtt     960
   agtagctatg catttattaa tagttttatt tataagtatt tagtttcact ctgttgcaga    1020
   ctattttatg ctaaaattag ctaaagccaa attactattt cttaaaacat attttttact     1080
   tttttttttt ttttaaatat tattaggtac ttcttgcaag ggatatgcat tagcacatac    1140
   tcaagaaggg gaagaaaaga agcaaacttc tggtacatca aataccagag gatcaagacg    1200
   aaaacctgca atgacaactc ctacaaggag gtctacacgt aacacaagag ctgaaacagc    1260
   cagtcagtct cagagatccc caatatcaga caattctggg tgtgatgccc caggtaacag    1320
   taatccatct ttaagtgttc cctcttcagc tgagtcagaa aagcaaacaa gacaggctcc    1380
   aaaacggaag tctgtaagaa gaggaagaaa accaccttta ctgaaaaaga aacttcggag    1440
   ctctgtagct gcccctgaaa aatcatcttc caatgattca gtagatgaag aaacagcaga    1500
   atctgacaca tcacctgtgt tagaaaaaga gcaccaacca gatgtagaca gtagtaacat    1560
   ttgtactgtg cagactcatg tagaaaacca gtctgctaat tgcttgaaaa gttgcaatga    1620
   gcaaatagaa gaaagtgaga agcatactgc aaattatgat acagaggaaa gagtaggatc    1680
   ttcatcttct gagtcttgtg ctcaagatct tcctgtgcta gttggtgagg aaggggaagt    1740
   taaaaaactc gagaatacag gtatagaggc taatgttttg tgtttggaaa gtgagatttc    1800
   tgaaaatatt cttgaaaaag gaggtgatcc attggaaaag caagaccaga tatctggact    1860
   ttcacaatca gaggtaaaga cagatgtatg tacagttcat cttccaaatg attttcctac    1920
   atgtttaaca tctgaaagca aagtgtacca acctgtatct tgtccctaag gtgacttatc    1980
   tgagaatgta gagtcagtgg ttaatgaaga aaaaataaca gagagttccc tagtagaaat 2040
   tactgaacat aaagatttta cactaaaaac agaggagctt atagagagcc ccaagttaga 2100
   atcttctgag ggtgaaatta tacagacagt ggacagacaa tctgttaaga gcccagaggt 2160
   tcaattgctt gggcatgttg aaactgaaga tgtagaaata attgcaacat gtgatacttt 2220
   tgggaatgaa gatttcaata atattcaaga ctctgaaaat aacttactaa aaaataatct 2280
   tctgaacacc aaattggaaa aatctttaga agaaaagaat gaatcgctga ccgaacatcc 2340
   tagatctaca gagttgccta aaacacacat tgaacagatt cagaagcatt ttagtgagga 2400
   caacaatgaa atgataccta tggagtgtga ttcattttgc agtgaccaaa atgaatctga 2460
   agttgaacca tctgtaaatg ctgatcttaa acaaatgaat gaaaattctg tgacacactg 2520
   ttctgaaaat aatatgccgt cttctgatct tgcggatgaa aaggttgaaa ctgtttctca 2580
   accatctgaa agcccaaaag ataccataga taaaaccaaa aagcctcgta ctcgaagatc 2640
   tagatttcat tctccatcta caacttggtc acccaacaaa gacactccac aagaaaagaa 2700
   gcggccccag tctccatctc ccagaagaga aactgggaaa gaaagcagga agtctcaatc 2760
   accatctcct aagaatgagt cagccagagg ccggaaaaaa tcccgttctc agtccccaaa 2820
   aaaggatatt gcaagagaaa ggaggcaatc tcagtctcgg tctccaaaaa gggatactac 2880
   tagggaaagc agaagatctg aatcactgtc cccaagaaga gaaacttcta gagagaacaa 2940
   aagatctcag ccaagagtga aagattcttc cccaggagaa aaatccaggt cccagagcag 3000
   agaacgagaa agtgatagag atggcagagg gagagagaga gaaaggagaa ccagaaagtg 3060
   gtctaggtcc agatctcatt ctaggtcccc ctcaagatgt agaacaaaaa gtaagagttc 3120
   atcatttggt agaattgaca gagatagtta ctctccccgg tggaaggaaa gatgggcaaa 3180
   tgatggttgg agatgtccac gaggaaatga tcggtacaga aagaatgacc cagagaaaca 3240
   gaatgaaaat acaagaaaag aaaaaaatga catccatcta gatgctgatg atccaaattc 3300
   tgctgacaaa catagaaatg actgtcccaa ttggataaca gaaaaaataa actctggccc 3360
   tgatccaaga accagaaatc cagaaaagtt gaaagagtct cattgggaag aaaatagaaa 3420
   tgaaaattca ggaaattctt ggaataaaaa ctttggttct ggttgggtat ctaaccgtgg 3480
   tagaggcaga ggcaaccgtg gcagaggcac ttacagaagt agttttgcct ataaagatca 3540
   gaatgaaaat cggtggcaaa atcgaaaacc cctctcaggg aattcaaaca gttcagggag 3600
   tgaatctttc aagtttgtgg aacagcaatc ctataagcga aaaagtgaac aggagttctc 3660
   atttgataca ccagcagata gatctggatg gacatctgca tccagctggg ccgtgagaaa 3720
   gactttgcca gcagatgtac aaaactacta ctcacgacga ggcagaaatt cttcaggtcc 3780
   acagtctgga tggatgaaac aagaggagga aacatctgga caggattcta gcctaaaaga 3840
   ccaaacaaac cagcaagttg atggttctca gctacctata aatatgatgc aaccgcaaat 3900
   gaatgtaatg cagcaacaaa tgaatgcaca acaccagcct atgaatatct tcccatatcc 3960
   agtgggtgtt catgctcctt tgatgaacat ccaacgcaat ccatttaaca ttcatcctca 4020
   gctacccttg catctccaca caggagtgcc cctcatgcag gtagccactc ctaccagtgt 4080
   atctcaggga ctaccaccac caccaccccc tcccccacca tcccaacaag tcaactacat 4140
   tgcttcacaa ccagatggaa agcaattgca gggtattcct agttcttctc atgtaagtaa 4200
   taacatgagt acaccagttt tgcctgctcc gacagcagcc ccaggaaata cgggaatggt 4260
   tcaggaccaa agttctggta atacttcgtc atcaagtcac agcaaagcct ctaatgctgc 4320
   tgtaaaattg gcagaaagca aagtaagtgt tgcagtggaa gccagcgcag atagctcgaa 4380
   gacagacaag aaattgcaaa ttcaagaaaa agcagcacaa gaggtaaaat tggccatcaa 4440
   gccattttac caaaataaag atatcaccaa ggaagaatat aaagaaattg tacggaaagc 4500
   agtagataaa gtttgtcata gtaagagtgg agaagtaaat tctactaaag tggcaaatct 4560
   ggttaaagcc tatgtagaca aatacaaata ttcacggaag gggagccaaa agaaaactct 4620
   ggaagaacct gtgtctactg aaaaaaacat aggctgaaat ggggaacgct gtcaaggaca 4680
   ttatcaggat atctgcaaag tgcaatttca acatgtacca ttaactgaaa atcatacata 4740
   actgtgattg aaatttggtt ttgataaaat tattttttta acataggata tgatgttttg 4800
   ttctaaataa atataggtct gcactgcaac ttctgtatcc ttccttcccc tccaccctcc 4860
   cccacaaaat tcaagggaaa gtaaagggtt taaaggaatg tgcatcttta ctaggactgt 4920
   gttatagtgt ggatactgga aaatgtatag ctttttgatt agggcaatgg agtgcataaa 4980
   ttagaaactt ctaagtgcac tggttttcaa agagatatat ataatgcatt tattctgtca 5040
   ggttaaaata taaagtatga tctttatgat tttttccctc taattataga aagttaaata 5100
   atgtattacc atgaaaaatg tttctaatat taaatagaac atatcagttg caaagttcct 5160
   aatgtgtatt tttaaagcac atatctgaat aaattgccta gatagaaaaa aaattatcac 5220
   gagtaaaatt tagtgttcaa aacattgaaa cactcttcac ctattgtatg accaaataaa 5280
   ggttatgctg cttgttacgc gaaggcc                                    5307

<210> 168
<211> 1148
<212> PRT
<213> Homo Sapiens

<400> 168
Met Thr Thr Pro Thr Arg Arg Ser Thr Arg Asn Thr Arg Ala Glu Thr
1               5                   10                  15
Ala Ser Gln Ser Gln Arg Ser Pro Ile Ser Asp Asn Ser Gly Cys Asp
            20                  25                  30
Ala Pro Gly Asn Ser Asn Pro Ser Leu Ser Val Pro Ser Ser Ala Glu
        35                  40                  45
Ser Glu Lys Gln Thr Arg Gln Ala Pro Lys Arg Lys Ser Val Arg Arg
    50                  55                  60
Gly Arg Lys Pro Pro Leu Leu Lys Lys Lys Leu Arg Ser Ser Val Ala
65                  70                  75                  80
Ala Pro Glu Lys Ser Ser Ser Asn Asp Ser Val Asp Glu Glu Thr Ala
                85                  90                  95
Glu Ser Asp Thr Ser Pro Val Leu Glu Lys Glu His Gln Pro Asp Val
            100                 105                 110
Asp Ser Ser Asn Ile Cys Thr Val Gln Thr His Val Glu Asn Gln Ser
        115                 120                 125
Ala Asn Cys Leu Lys Ser Cys Asn Glu Gln Ile Glu Glu Ser Glu Lys
    130                 135                 140
His Thr Ala Asn Tyr Asp Thr Glu Glu Arg Val Gly Ser Ser Ser Ser
145                 150                 155                 160
Glu Ser Cys Ala Gln Asp Leu Pro Val Leu Val Gly Glu Glu Gly Glu
                165                 170                 175
Val Lys Lys Leu Glu Asn Thr Gly Ile Glu Ala Asn Val Leu Cys Leu
            180                 185                 190
Glu Ser Glu Ile Ser Glu Asn Ile Leu Glu Lys Gly Gly Asp Pro Leu
        195                 200                 205
Glu Lys Gln Asp Gln Ile Ser Gly Leu Ser Gln Ser Glu Val Lys Thr
    210                 215                 220
Asp Val Cys Thr Val His Leu Pro Asn Asp Phe Pro Thr Cys Leu Thr
225                 230                 235                 240
Ser Glu Ser Lys Val Tyr Gln Pro Val Ser Cys Pro Leu Ser Asp Leu
                245                 250                 255
Ser Glu Asn Val Glu Ser Val Val Asn Glu Glu Lys Ile Thr Glu Ser
            260                 265                 270
Ser Leu Val Glu Ile Thr Glu His Lys Asp Phe Thr Leu Lys Thr Glu
        275                 280                 285
Glu Leu Ile Glu Ser Pro Lys Leu Glu Ser Ser Glu Gly Glu Ile Ile
    290                 295                 300
Gln Thr Val Asp Arg Gln Ser Val Lys Ser Pro Glu Val Gln Leu Leu
305                 310                 315                 320
Gly His Val Glu Thr Glu Asp Val Glu Ile Ile Ala Thr Cys Asp Thr
                325                 330                 335
Phe Gly Asn Glu Asp Phe Asn Asn Ile Gln Asp Ser Glu Asn Asn Leu
            340                 345                 350
Leu Lys Asn Asn Leu Leu Asn Thr Lys Leu Glu Lys Ser Leu Glu Glu

        355                 360                 365
Lys Asn Glu Ser Leu Thr Glu His Pro Arg Ser Thr Glu Leu Pro Lys
    370                 375                 380
Thr His Ile Glu Gln Ile Gln Lys His Phe Ser Glu Asp Asn Asn Glu
385                 390                 395                 400
Met Ile Pro Met Glu Cys Asp Ser Phe Cys Ser Asp Gln Asn Glu Ser
                405                 410                 415
Glu Val Glu Pro Ser Val Asn Ala Asp Leu Lys Gln Met Asn Glu Asn
            420                 425                 430
Ser Val Thr His Cys Ser Glu Asn Asn Met Pro Ser Ser Asp Leu Ala
        435                 440                 445
Asp Glu Lys Val Glu Thr Val Ser Gln Pro Ser Glu Ser Pro Lys Asp
    450                 455                 460
Thr Ile Asp Lys Thr Lys Lys Pro Arg Thr Arg Arg Ser Arg Phe His
465                 470                 475                 480
Ser Pro Ser Thr Thr Trp Ser Pro Asn Lys Asp Thr Pro Gln Glu Lys
                485                 490                 495
Lys Arg Pro Gln Ser Pro Ser Pro Arg Arg Glu Thr Gly Lys Glu Ser
            500                 505                 510
Arg Lys Ser Gln Ser Pro Ser Pro Lys Asn Glu Ser Ala Arg Gly Arg
        515                 520                 525
Lys Lys Ser Arg Ser Gln Ser Pro Lys Lys Asp Ile Ala Arg Glu Arg
    530                 535                 540
Arg Gln Ser Gln Ser Arg Ser Pro Lys Arg Asp Thr Thr Arg Glu Ser
545                 550                 555                 560
Arg Arg Ser Glu Ser Leu Ser Pro Arg Arg Glu Thr Ser Arg Glu Asn
                565                 570                 575
Lys Arg Ser Gln Pro Arg Val Lys Asp Ser Ser Pro Gly Glu Lys Ser
            580                 585                 590
Arg Ser Gln Ser Arg Glu Arg Glu Ser Asp Arg Asp Gly Gln Arg Arg
        595                 600                 605
Glu Arg Glu Arg Arg Thr Arg Lys Trp Ser Arg Ser Arg Ser His Ser
    610                 615                 620
Arg Ser Pro Ser Arg Cys Arg Thr Lys Ser Lys Ser Ser Ser Phe Gly
625                 630                 635                 640
Arg Ile Asp Arg Asp Ser Tyr Ser Pro Arg Trp Lys Gly Arg Trp Ala
                645                 650                 655
Asn Asp Gly Trp Arg Cys Pro Arg Gly Asn Asp Arg Tyr Arg Lys Asn
            660                 665                 670
Asp Pro Glu Lys Gln Asn Glu Asn Thr Arg Lys Glu Lys Asn Asp Ile
        675                 680                 685
His Leu Asp Ala Asp Asp Pro Asn Ser Ala Asp Lys His Arg Asn Asp
    690                 695                 700
Cys Pro Asn Trp Ile Thr Glu Lys Ile Asn Ser Gly Pro Asp Pro Arg
705                 710                 715                 720
Thr Arg Asn Pro Glu Lys Leu Lys Glu Ser His Trp Glu Glu Asn Arg
                725                 730                 735
Asn Glu Asn Ser Gly Asn Ser Trp Asn Lys Asn Phe Gly Ser Gly Trp
            740                 745                 750
Val Ser Asn Arg Gly Arg Gly Arg Gly Asn Arg Gly Arg Gly Thr Tyr
        755                 760                 765
Arg Ser Ser Phe Ala Tyr Lys Asp Gln Asn Glu Asn Arg Trp Gln Asn
    770                 775                 780
Arg Lys Pro Leu Ser Gly Asn Ser Asn Ser Ser Gly Ser Glu Ser Phe
785                 790                 795                 800

Lys Phe Val Glu Gln Gln Ser Tyr Lys Arg Lys Ser Glu Gln Glu Phe
                805                 810                 815
Ser Phe Asp Thr Pro Ala Asp Arg Ser Gly Trp Thr Ser Ala Ser Ser
            820                 825                 830
Trp Ala Val Arg Lys Thr Leu Pro Ala Asp Val Gln Asn Tyr Tyr Ser
        835                 840                 845
Arg Arg Gly Arg Asn Ser Ser Gly Pro Gln Ser Gly Trp Met Lys Gln
    850                 855                 860
Glu Glu Glu Thr Ser Gly Gln Asp Ser Ser Leu Lys Asp Gln Thr Asn
865                 870                 875                 880
Gln Gln Val Asp Gly Ser Gln Leu Pro Ile Asn Met Met Gln Pro Gln
                885                 890                 895
Met Asn Val Met Gln Gln Gln Met Asn Ala Gln His Gln Pro Met Asn
            900                 905                 910
Ile Phe Pro Tyr Pro Val Gly Val His Ala Pro Leu Met Asn Ile Gln
        915                 920                 925
Arg Asn Pro Phe Asn Ile His Pro Gln Leu Pro Leu His Leu His Thr
    930                 935                 940
Gly Val Pro Leu Met Gln Val Ala Thr Pro Thr Ser Val Ser Gln Gly
945                 950                 955                 960
Leu Pro Pro Pro Pro Pro Pro Pro Pro Pro Ser Gln Gln Val Asn Tyr
                965                 970                 975
Ile Ala Ser Gln Pro Asp Gly Lys Gln Leu Gln Gly Ile Pro Ser Ser
            980                 985                 990
Ser His Val Ser Asn Asn Met Ser Thr Pro Val Leu Pro Ala Pro Thr
        995                 1000                1005
Ala Ala Pro Gly Asn Thr Gly Met Val Gln Gly Pro Ser Ser Gly Asn
    1010                1015                1020
Thr Ser Ser Ser Ser His Ser Lys Ala Ser Asn Ala Ala Val Lys Leu
1025 1030 1035 104
Ala Glu Ser Lys Val Ser Val Ala Val Glu Ala Ser Ala Asp Ser Ser
                1045                1050                1055
Lys Thr Asp Lys Lys Leu Gln Ile Gln Glu Lys Ala Ala Gln Glu Val
            1060                1065                1070
Lys Leu Ala Ile Lys Pro Phe Tyr Gln Asn Lys Asp Ile Thr Lys Glu
        1075                1080                1085
Glu Tyr Lys Glu Ile Val Arg Lys Ala Val Asp Lys Val Cys His Ser
    1090                1095                1100
Lys Ser Gly Glu Val Asn Ser Thr Lys Val Ala Asn Leu Val Lys Ala
1105 1110 1115 112
Tyr Val Asp Lys Tyr Lys Tyr Ser Arg Lys Gly Ser Gln Lys Lys Thr
                1125                1130                1135
Leu Glu Glu Pro Val Ser Thr Glu Lys Asn Ile Gly
            1140                1145

<210> 169
<211> 597
<212> DNA
<213> Homo Sapiens

<400> 169
gagactttta atcatctatc ccttgtgctt tacgcagacc ctacaataca ctagaggctt 60
caaagaggtc aaaaattcac atgtgtagac aaattaggtc ccttaagatg ccaggcaaac 120
gaagtgctac caaaacacgc aatgactgtc ctaaaagtgc gttctgggat acacctgtaa 180
acttggatca agttccctcc cctctcctca aaatatatcg acttgtgctg aaagaaatca 240

cgaccgatgc tcacaattct gacctcgtaa ttatataggg ggtggttttg gtttctgcgt 300
ctttccctga ttcagtggca ggtaacatat ttcatgtaca aaatgaactg caacaccacg 360
gcaaacaagg gacaggccct caaagttgtc ggtagggagc caggaccccg ccagtggcgt 420
ggggagacac cgtactaaac aagcttgcaa acagcaggca ccttcctgcc actgaggagg 480
aagggctggc taagggaggc cggggcggag gaagccaagc tctgcaggcc ctgacaaagt 540
cctcccggcc tccacgcgtc gccatggcaa cgcggggtct gtgctgcccg ggattgg 597

<210> 170
<211> 3344
<212> DNA
<213> Homo Sapiens

<400> 170
ggtacagctg cgcgtctgcg ggaataggtg cagcgggccc ttggcggggg actctgaggg 60
aggagctggg gacggcgacc ctaggagagt tctttggggt gactttcaag atggactcta 120
ctctaacagc aagtgaaatc cggcagcgat ttatagattt cttcaagagg aacgagcata 180
cgtatgttca ctcgtctgcc accatcccat tggatgaccc cactttgctc tttgccaatg 240
caggcatgaa ccagtttaaa cccattttcc tgaacacaat tgacccatct caccccatgg 300
caaagctgag cagagctgcc aatacccaga agtgcatccg ggctgggggc aaacaaaatg 360
acctggacga tgtgggcaag gatgtctatc atcacacctt cttcgagatg ctgggctctt 420
ggtcttttgg agattacttt aaggaattgg catgtaagat ggctctggaa ctcctcaccc 480
aagagtttgg cattcccatt gaaagacttt atgttactta ctttggcggg gatgaagcag 540
ctggcttaga agcagatctg gaatgcaaac agatctggca aaatttgggg ctggatgaca 600
ccaaaatcct cccaggcaac atgaaggata acttctggga gatgggtgac acgggcccct 660
gtggtccttg cagtgagatc cactacgacc ggattggtgg tcgggacgcc gcacatcttg 720
tcaaccagga cgaccctaat gtgctggaga tctggaacct tgtgttcatc cagtataaca 780
gggaagctga tggcattctg aaacctcttc ccaagaaaag cattgacaca gggatgggcc 840
tggaacgact ggtatctgtg ctgcagaata agatgtccaa ctatgacact gacctttttg 900
tcccttactt tgaagccatt cagaagggca caggtgcccg accatacact gggaaagttg 960
gtgctgagga tgccgatggg attgacatgg cctaccgggt gctggctgac catgctcgga 1020
ccatcactgt ggcactggct gatggtggcc ggcctgacaa cacagggcgt ggatatgtgt 1080
tgagacggat tctccgccga gctgtccgat acgcccatga aaagctcaat gccagcaggg 1140
gcttctttgc tacgttagtg gatgttgtcg tccagtccct gggagatgca tttcctgagc 1200
tgaagaagga cccagacatg gtgaaggaca tcattaatga agaagaggtg cagtttctca 1260
agactctcag cagagggcgt cgcatcctgg acaggaaaat tcagagcctg ggagacagca 1320
agaccattcc cggagacact gcttggctcc tctatgacac ctatgggttt ccagtggatc 1380
tgactggact gattgctgaa gagaagggcc tggtggtaga catggatggc tttgaagagg 1440
agaggaaact ggcccagctg aaatcacagg gcaagggagc tggtggggaa gacctcatta 1500
tgctggacat ttacgctatc gaagagctcc gggcacgggg tctggaggtc acagatgatt 1560
ccccaaagta caattaccat ttggactcca gtggtagcta tgtatttgag aacacagtgg 1620
ctacggtgat ggctctgcgc agggagaaga tgttcgtgga agaggtgtcc acaggccagg 1680
agtgtggagt ggtgctggac aagacctgtt tctatgctga gcaaggaggc cagatctatg 1740
acgaaggcta cctggtgaag gtggatgaca gcagtgaaga taaaacagag tttacagtga 1800
agaatgctca ggtccgagga gggtatgtgc tacacattgg aaccatctac ggtgacctga 1860
aagtggggga tcaggtctgg ctgtttattg atgagccccg acgaagaccc atcatgagca 1920
accacacagc tacgcacatt ctgaacttcg ccctgcgctc agtgcttggg gaagctgacc 1980
agaaaggctc attggttgct cctgaccgcc tcagatttga ctttactgcc aagggagcca 2040
tgtccaccca acagatcaag aaggctgaag agattgctaa tgagatgatt gaggcagcca 2100
aggccgtcta tacccaggat tgccccctgg cagcagcgaa agccatccag ggcctacggg 2160
ctgtgtttga tgagacctat cctgaccctg tgcgagtcgt ctccattggg gtcccggtgt 2220
ccgagttgct ggatgacccc tctgggcctg ctggctccct gacttctgtt gagttctgtg 2280
ggggaacgca cctgcggaac tcgagtcatg caggagcttt tgtgatcgtg acggaagaag 2340
ccattgccaa gggtatccgg aggattgtgg ctgtcacagg tgccgaggcc cagaaggccc 2400
tcaggaaagc agagagcttg aagaaatgtc tctctgtcat ggaagccaaa gtgaaggctc 2460
agactgctcc aaacaaggat gtgcagaggg agatcgctga ccttggagag gccctggcca 2520

ctgcagtcat cccccagtgg cagaaggatg aattgcggga gactctcaaa tccctaaaga 2580
aggtcatgga tgacttggac cgagccagca aagccgatgt ccagaaacga gtgttagaga 2640
agacgaagca gttcatcgac agcaacccca accagcctct tgtcatcctg gagatggaga 2700
gcggcgcctc agccaaggcc ctgaatgaag ccttgaagct cttcaagatg cactcccctc 2760
agacttctgc catgctcttc acggtggaca atgaggctgg caagatcacg tgcctgtgtc 2820
aagtccccca gaatgcagcc aatcggggct taaaagccag cgagtgggtg cagcaggtgt 2880
caggcttgat ggacggtaaa ggtggtggca aggatgtgtc tgcacaggcc acaggcaaga 2940
acgttggctg cctgcaggag gcgctgcagc tggccacttc cttcgcccag ctgcgcctcg 3000
gggatgtaaa gaactgagtg gggaaggagg aggctcccac tggatccatc cgtccagcca 3060
agagctcttc atctgctaca agaacatttg aatcttggga cctttaaaga gcccctccta 3120
acccagcagt aactggaaca cacttgggag cagtcctatg tctcagtgcc ccttaaattt 3180
ctgccctgag ccctccacgt cagtgccatc ggtctagaac cactaacccc gcattgctgt 3240
tgatcgtcac gctcgcatct atagataacg gctctccaga cctgagcttt ccgcgtcagc 3300
aagtaggaat cgtttttgct gcagagaata aaaggaccac gtgc 3344

<210> 171
<211> 1004
<212> PRT
<213> Homo Sapiens

<400> 171
Tyr Ser Cys Ala Ser Ala Gly Ile Gly Ala Ala Gly Pro Trp Arg Gly
1               5                   10                  15
Thr Leu Arg Glu Glu Leu Gly Thr Ala Thr Leu Gly Glu Phe Phe Gly
            20                  25                  30
Val Thr Phe Lys Met Asp Ser Thr Leu Thr Ala Ser Glu Ile Arg Gln
        35                  40                  45
Arg Phe Ile Asp Phe Phe Lys Arg Asn Glu His Thr Tyr Val His Ser
    50                  55                  60
Ser Ala Thr Ile Pro Leu Asp Asp Pro Thr Leu Leu Phe Ala Asn Ala
65                  70                  75                  80
Gly Met Asn Gln Phe Lys Pro Ile Phe Leu Asn Thr Ile Asp Pro Ser
                85                  90                  95
His Pro Met Ala Lys Leu Ser Arg Ala Ala Asn Thr Gln Lys Cys Ile
            100                 105                 110
Arg Ala Gly Gly Lys Gln Asn Asp Leu Asp Asp Val Gly Lys Asp Val
        115                 120                 125
Tyr His His Thr Phe Phe Glu Met Leu Gly Ser Trp Ser Phe Gly Asp
    130                 135                 140
Tyr Phe Lys Glu Leu Ala Cys Lys Met Ala Leu Glu Leu Leu Thr Gln
145                 150                 155                 160
Glu Phe Gly Ile Pro Ile Glu Arg Leu Tyr Val Thr Tyr Phe Gly Gly
                165                 170                 175
Asp Glu Ala Ala Gly Leu Glu Ala Asp Leu Glu Cys Lys Gln Ile Trp
            180                 185                 190
Gln Asn Leu Gly Leu Asp Asp Thr Lys Ile Leu Pro Gly Asn Met Lys
        195                 200                 205
Asp Asn Phe Trp Glu Met Gly Asp Thr Gly Pro Cys Gly Pro Cys Ser
    210                 215                 220
Glu Ile His Tyr Asp Arg Ile Gly Gly Arg Asp Ala Ala His Leu Val
225                 230                 235                 240
Asn Gln Asp Asp Pro Asn Val Leu Glu Ile Trp Asn Leu Val Phe Ile
                245                 250                 255
Gln Tyr Asn Arg Glu Ala Asp Gly Ile Leu Lys Pro Leu Pro Lys Lys
            260                 265                 270

Ser Ile Asp Thr Gly Met Gly Leu Glu Arg Leu Val Ser Val Leu Gln
        275                 280                 285
Asn Lys Met Ser Asn Tyr Asp Thr Asp Leu Phe Val Pro Tyr Phe Glu
    290                 295                 300
Ala Ile Gln Lys Gly Thr Gly Ala Arg Pro Tyr Thr Gly Lys Val Gly
305                 310                 315                 320
Ala Glu Asp Ala Asp Gly Ile Asp Met Ala Tyr Arg Val Leu Ala Asp
                325                 330                 335
His Ala Arg Thr Ile Thr Val Ala Leu Ala Asp Gly Gly Arg Pro Asp
            340                 345                 350
Asn Thr Gly Arg Gly Tyr Val Leu Arg Arg Ile Leu Arg Arg Ala Val
        355                 360                 365
Arg Tyr Ala His Glu Lys Leu Asn Ala Ser Arg Gly Phe Phe Ala Thr
    370                 375                 380
Leu Val Asp Val Val Val Gln Ser Leu Gly Asp Ala Phe Pro Glu Leu
385                 390                 395                 400
Lys Lys Asp Pro Asp Met Val Lys Asp Ile Ile Asn Glu Glu Glu Val
                405                 410                 415
Gln Phe Leu Lys Thr Leu Ser Arg Gly Arg Arg Ile Leu Asp Arg Lys
            420                 425                 430
Ile Gln Ser Leu Gly Asp Ser Lys Thr Ile Pro Gly Asp Thr Ala Trp
        435                 440                 445
Leu Leu Tyr Asp Thr Tyr Gly Phe Pro Val Asp Leu Thr Gly Leu Ile
    450                 455                 460
Ala Glu Glu Lys Gly Leu Val Val Asp Met Asp Gly Phe Glu Glu Glu
465                 470                 475                 480
Arg Lys Leu Ala Gln Leu Lys Ser Gln Gly Lys Gly Ala Gly Gly Glu
                485                 490                 495
Asp Leu Ile Met Leu Asp Ile Tyr Ala Ile Glu Glu Leu Arg Ala Arg
            500                 505                 510
Gly Leu Glu Val Thr Asp Asp Ser Pro Lys Tyr Asn Tyr His Leu Asp
        515                 520                 525
Ser Ser Gly Ser Tyr Val Phe Glu Asn Thr Val Ala Thr Val Met Ala
    530                 535                 540
Leu Arg Arg Glu Lys Met Phe Val Glu Glu Val Ser Thr Gly Gln Glu
545                 550                 555                 560
Cys Gly Val Val Leu Asp Lys Thr Cys Phe Tyr Ala Glu Gln Gly Gly
                565                 570                 575
Gln Ile Tyr Asp Glu Gly Tyr Leu Val Lys Val Asp Asp Ser Ser Glu
            580                 585                 590
Asp Lys Thr Glu Phe Thr Val Lys Asn Ala Gln Val Arg Gly Gly Tyr
        595                 600                 605
Val Leu His Ile Gly Thr Ile Tyr Gly Asp Leu Lys Val Gly Asp Gln
    610                 615                 620
Val Trp Leu Phe Ile Asp Glu Pro Arg Arg Arg Pro Ile Met Ser Asn
625                 630                 635                 640
His Thr Ala Thr His Ile Leu Asn Phe Ala Leu Arg Ser Val Leu Gly
                645                 650                 655
Glu Ala Asp Gln Lys Gly Ser Leu Val Ala Pro Asp Arg Leu Arg Phe
            660                 665                 670
Asp Phe Thr Ala Lys Gly Ala Met Ser Thr Gln Gln Ile Lys Lys Ala
        675                 680                 685
Glu Glu Ile Ala Asn Glu Met Ile Glu Ala Ala Lys Ala Val Tyr Thr
    690                 695                 700
Gln Asp Cys Pro Leu Ala Ala Ala Lys Ala Ile Gln Gly Leu Arg Ala

705                 710                 715                 720
Val Phe Asp Glu Thr Tyr Pro Asp Pro Val Arg Val Val Ser Ile Gly
                725                 730                 735
Val Pro Val Ser Glu Leu Leu Asp Asp Pro Ser Gly Pro Ala Gly Ser
            740                 745                 750
Leu Thr Ser Val Glu Phe Cys Gly Gly Thr His Leu Arg Asn Ser Ser
        755                 760                 765
His Ala Gly Ala Phe Val Ile Val Thr Glu Glu Ala Ile Ala Lys Gly
    770                 775                 780
Ile Arg Arg Ile Val Ala Val Thr Gly Ala Glu Ala Gln Lys Ala Leu
785                 790                 795                 800
Arg Lys Ala Glu Ser Leu Lys Lys Cys Leu Ser Val Met Glu Ala Lys
                805                 810                 815
Val Lys Ala Gln Thr Ala Pro Asn Lys Asp Val Gln Arg Glu Ile Ala
            820                 825                 830
Asp Leu Gly Glu Ala Leu Ala Thr Ala Val Ile Pro Gln Trp Gln Lys
        835                 840                 845
Asp Glu Leu Arg Glu Thr Leu Lys Ser Leu Lys Lys Val Met Asp Asp
    850                 855                 860
Leu Asp Arg Ala Ser Lys Ala Asp Val Gln Lys Arg Val Leu Glu Lys
865                 870                 875                 880
Thr Lys Gln Phe Ile Asp Ser Asn Pro Asn Gln Pro Leu Val Ile Leu
                885                 890                 895
Glu Met Glu Ser Gly Ala Ser Ala Lys Ala Leu Asn Glu Ala Leu Lys
            900                 905                 910
Leu Phe Lys Met His Ser Pro Gln Thr Ser Ala Met Leu Phe Thr Val
        915                 920                 925
Asp Asn Glu Ala Gly Lys Ile Thr Cys Leu Cys Gln Val Pro Gln Asn
    930                 935                 940
Ala Ala Asn Arg Gly Leu Lys Ala Ser Glu Trp Val Gln Gln Val Ser
945                 950                 955                 960
Gly Leu Met Asp Gly Lys Gly Gly Gly Lys Asp Val Ser Ala Gln Ala
                965                 970                 975
Thr Gly Lys Asn Val Gly Cys Leu Gln Glu Ala Leu Gln Leu Ala Thr
            980                 985                 990
Ser Phe Ala Gln Leu Arg Leu Gly Asp Val Lys Asn
        995                 1000

<210> 172
<211> 659
<212> DNA
<213> Homo Sapiens

<400> 172
gcctgagcaa cgtctccgag caggcgctgg gctagaggcg ggtctcaacc agctactcat      60
tggaggcggg cttgagagcg gcggccaggg aggtgcggag cagcctcggc ggcggcggcc     120
gaaccaaccg agtcggatcc tgaccctaaa acctagtatt ttccacttgt tcatcaatat     180
ggaaaactca gattccaatg acaaaggaag tggtgatcag tctgcagcac agcgcagaag     240
tcagatggac cgattggatc gagaagaagc tttctatcaa tttgtaaata acctgagtga     300
agaagattat aggcttatga gagataacaa tttgctaggc accccaggtg aaagtactga     360
ggaagagttg ctgagacgac tacagcaaat taaagaaggc ccaccaccgc aaaactcaga     420
tgaaaataga ggaggagact cttcagatga tgtgtctaat ggtgactcta taatagactg     480
gcttaactct gtcagacaaa ctggaaatac aacaagaagt gggcaaagag gaaaccaatc     540
ttggagagca gtgagtcgga ctaatccaaa cagtgggtga tttcagattc agtttagaga     600
taaatgttaa cccgtaataa tgggagccaa aattcagaga atgaaaatga gccatctgc      659

<210> 173
<211> 192
<212> PRT
<213> Homo Sapiens

<400> 173
Pro Glu Gln Arg Leu Arg Ala Gly Ala Gly Leu Glu Ala Gly Leu Asn
1               5                   10                  15
Gln Leu Leu Ile Gly Gly Gly Leu Glu Ser Gly Gly Gln Gly Gly Ala
            20                  25                  30
Glu Gln Pro Arg Arg Arg Arg Pro Asn Gln Pro Ser Arg Ile Leu Thr
        35                  40                  45
Leu Lys Pro Ser Ile Phe His Leu Phe Ile Asn Met Glu Asn Ser Asp
    50                  55                  60
Ser Asn Asp Lys Gly Ser Gly Asp Gln Ser Ala Ala Gln Arg Arg Ser
65                  70                  75                  80
Gln Met Asp Arg Leu Asp Arg Glu Glu Ala Phe Tyr Gln Phe Val Asn
                85                  90                  95
Asn Leu Ser Glu Glu Asp Tyr Arg Leu Met Arg Asp Asn Asn Leu Leu
            100                 105                 110
Gly Thr Pro Gly Glu Ser Thr Glu Glu Glu Leu Leu Arg Arg Leu Gln
        115                 120                 125
Gln Ile Lys Glu Gly Pro Pro Pro Gln Asn Ser Asp Glu Asn Arg Gly
    130                 135                 140
Gly Asp Ser Ser Asp Asp Val Ser Asn Gly Asp Ser Ile Ile Asp Trp
145                 150                 155                 160
Leu Asn Ser Val Arg Gln Thr Gly Asn Thr Thr Arg Ser Gly Gln Arg
                165                 170                 175
Gly Asn Gln Ser Trp Arg Ala Val Ser Arg Thr Asn Pro Asn Ser Gly
            180                 185                 190

<210> 174
<211> 610
<212> DNA
<213> Homo Sapiens

<400> 174
gtactggcat cagtcaatgt tctggagtga tttgggcccc gatgttggct atgaagctat      60
tggtcttgtg gacagtagtt tgcccacagt tggtgttttt gcaaaagcaa ctgcacaaga     120
caaccccaaa tctgccacag agcagtcagg aactggtatc cgatcagaga gtgagacaga     180
gtccgaggcc tcagaaatta ctattcctcc cagcaccccg gcagttccac aggctcccgt     240
ccagggggag gactacggca aaggtgtcat cttctacctc agggacaaag tggtcgtggg     300
gattgtgcta tggaacatct ttaaccgaat gccaatagca aggaagatca ttaaggacgg     360
tgagcagcat gaagatctca atgaagtagc caaactattc aacattcatg aagactgaag     420
ccccacagtg gaattggcaa acccactgca gcccctgaga ggaggtcgaa tgggtaaagg     480
agcatttttt tattcagcag actttctctg tgtatgagtg tgaatgatca agtcctttgt     540
gaatattttc aactatgtag gtaaattctt aatgttcnca tagtgaaata aattctgatt     600
cttctaaaaa                                                            610

<210> 175
<211> 138
<212> PRT
<213> Homo Sapiens

<400> 175

Tyr Trp His Gln Ser Met Phe Trp Ser Asp Leu Gly Pro Asp Val Gly
1               5                   10                  15
Tyr Glu Ala Ile Gly Leu Val Asp Ser Ser Leu Pro Thr Val Gly Val
            20                  25                  30
Phe Ala Lys Ala Thr Ala Gln Asp Asn Pro Lys Ser Ala Thr Glu Gln
        35                  40                  45
Ser Gly Thr Gly Ile Arg Ser Glu Ser Glu Thr Glu Ser Glu Ala Ser
    50                  55                  60
Glu Ile Thr Ile Pro Pro Ser Thr Pro Ala Val Pro Gln Ala Pro Val
65                  70                  75                  80
Gln Gly Glu Asp Tyr Gly Lys Gly Val Ile Phe Tyr Leu Arg Asp Lys
                85                  90                  95
Val Val Val Gly Ile Val Leu Trp Asn Ile Phe Asn Arg Met Pro Ile
            100                 105                 110
Ala Arg Lys Ile Ile Lys Asp Gly Glu Gln His Glu Asp Leu Asn Glu
        115                 120                 125
Val Ala Lys Leu Phe Asn Ile His Glu Asp
    130                 135

<210> 176
<211> 805
<212> DNA
<213> Homo Sapiens

<400> 176
gggacagcca agtctgtgac ttgcacgtac tcccctgccc tcaacaagat gttttgccaa      60
ctggccaaga cctgccctgt gcagctgtgg gttgattcca cacccccgcc cggcacccgc     120
gtccgcgcca tggccatcta caagcagtca cagcacatga cggaggttgt gaggcgctgc     180
ccccaccatg agcgctgctc agatagcgat ggtctggccc ctcctcagca tcttatccga     240
gtggaaggaa atttgcgtgt ggagtatttg gatgacagaa acacttttcg acatagtgtg     300
gtggtgccct atgagccgcc tgaggttggc tctgactgta ccaccatcca ctacaactac     360
atgtgtaaca gttcctgcat gggcggcatg aaccggaggc ccatcctcac catcatcaca     420
ctggaagact ccagtggtaa tctactggga cggaacagct ttgaggtgcg tgtttgtgcc     480
tgtcctggga gagaccggcg cacagaggaa gagaatctcc gcaagaaagg ggagcctcac     540
cacgaagctg cccccaggga gcactaagcg agcactgccc aacaacacca agctcctctc     600
cccagccaaa gaagaaanca ctggatngag aatatttcac cccttcanat tcgttgggcg     660
tgagcgcttc cganaatgtt ccgaagagct gnaagaaggc cttgggaact caaaggatgc     720
ccaaggcttg ggaaaggagc caangggggg gaancaangg gctcaactnc aagccaacct     780
gaaagttcca aaaaangggt ccagt                                           805

<210> 177
<211> 626
<212> DNA
<213> Homo Sapiens

<400> 177
ctaatttgtc tgtttattcc cacaaggtag ccaggggtgg gggcgccgag ccaagcccag      60
caggccatgg gaccttcctc cggcggggtg cacgctggat ttcgggtctg gccccaccag     120
caggtttgca ggcaggccgt catgagtgcc ggtggaaggc tccgagggcg tgggcagggg     180
ctcgggcggg gccacacact tgtggagcta gaaatantgg ggcaggtcct tctctatcac     240
caggggctcc tccatgggtc cgtagcgctt caccacgcag ccgttcttgt cgatgaggaa     300
ctgtgganan acggtgtcca aactgtgggg ccacccctgc aaggggctga ggctgccctt     360
cctgtccgct gcccatctgg gccacggctg tggccagggg aaactggtcc cctacccccc     420
acagccccct tacctttggt gaagttccac ttgatggcac tggaaaanaa gcacatggac     480
gtgagcgtcc ccaggcagcc ccccacagtc cccaaagctt gtcctgtctc caaggaggcc     540

anaaaggttg tnagcttccc ccggtncctc cacangccac agtgccccca aanccccccc 600
aanagccatc tttaccccaa ggaggg 626

<210> 178
<211> 793
<212> DNA
<213> Homo Sapiens

<400> 178
gcgcgaggct gctgctgctg cccccggccc gcgcggctgg aaacggagag gccgagccaa 60
gcggcggccc ctcttatgct gggaggatgc tggagagtag cggctgcaaa gcgctgaagg 120
agggcgtgct ggagaagcgc agcgacgggt tgttgcagct ctggaagaaa aagtgttgca 180
tcctcaccga ggaagggctg ctgcttatcc cgcccaagca gctgcaacac cagcagcagc 240
agcaacagca gcagcagcag cagcaacaac agcccgggca ggggccggcc gagccgtccc 300
aacccagtgg ccccgctgtc gccagcctcg agccgccggt caagctcaag gaactgcact 360
tctccaacat gaagaccgtg gactgtgtgg agcgcaaggg caagtacatg tacttcactg 420
tggtgatggc agagggcaag gagatcgact ttcggtgccc gcaagaccag ggctggaacg 480
ccgagatcac gctgcagatg gtgcagtaca agaatcgtca ggccatcctg gcggtcaaat 540
ccacgcggca gaagcagcag cacctggtcc agcancagcc cccctcgcag ccgcagccgc 600
agccgcagct ccaagcccca accccagcct tcagcctcaa gccngcaacc ccaagcccca 660
attcacaaac cccaagccct caagccccaa cccaaagccc tcangcccca ngcaagntcc 720
aacccgttat ncggccatcc aacattcaan atccaanact ctcaangcct taactncgcn 780
acccaanaac nct 793

<210> 179
<211> 786
<212> DNA
<213> Homo Sapiens

<400> 179
aatatcagag ttttaatttc aaccagctgg cacaacaatg aaagtgtcag actttctgaa 60
agtactcgag aaataatgaa taaattctta atgttttccc ctccaccgcc cttttttatt 120
ctccaagatt aggaattact acggattagg tttttgaaaa taaagtttcc tttttggaaa 180
atggtctaca ttcagaaatg tcttagaaca agcatttaaa aaaaactaat aaataatcat 240
aaatcaaaat acattaaaat aaaattacag tacatcatcg ctcctagaaa attcaccata 300
caagacgatc ctttcaaagg ttcataaata aaagtcttct tgactcgaaa tcgtttcctg 360
catcgtgatg aaaagtatgc agaaaactaa gaagaatcgc aagttttcag tagggtgatg 420
tccaaactac ttgatctggt gcggggcgga gagactgttt tgcttttgat ccaagtgaag 480
acaatagaaa tgtgctcgtc ccacttcctc aagtcctcaa aaccttgtct tgcccgggag 540
ctgccccttt cangcagagt tgggaggtgc tgcgganaaa ccggtgcccg tgcggctgcc 600
aatgcggctg tgggtgtggg tgcngtattt ggtgccggat gcnggtgccg ggtnaaggtg 660
tggggtgcca antnaaggat gaaaatgtgg atnttngnat nttgattccg gatacggggt 720
gggaacctng cngggggccn naaggcttgg ggttggggct naanggctgg ggttttttaa 780
ttgggg 786

<210> 180
<211> 791
<212> DNA
<213> Homo Sapiens

<400> 180
aggacctcag agacccaggc tctgtgattg tggccttcaa ggaaggggaa cagaaggaga 60
aggagggtat cctgcagctg cgtcgcacca actcagccaa gcccagtcca ctggcaccat 120
ccctcatggc ctcttctccg acttctatct gtgtgtgtgg gcaggtgcca gctggggtgg 180
gagttctgca gtgtgacctg tgtcaggact ggttccatgg gcagtgtgtg tcagtgcccc 240

atctcctcac ctctccaaag cccagtctca cttcatctcc actgctagcc tggtgggaat 300
gggacacaaa attcctgtgt ccactgtgta tgcgctcacg acggccacgc ctagagacaa 360
tcctagcctt gctggttgcc ctgcagaggc tgcccgtgcg gctgcctgag ggtgaggccc 420
ttcagtgtct cacagagagg gccattggct ggcaagaccg tgccagaaag gctctggcct 480
ctgaagatgt gactgctctg ttgcgacagc tggctgagct tcgccaacag ctacaggcca 540
aacccanacc agaggaggcc tcagtctaca cttcagccac tgcctgtgac cctatcagag 600
aaggcagtgg caacaatatt tcnaangtcc aagggctgct ggagaatgga gacantgttg 660
accagtcctg agaacatggc tccaggaaag ggctctgacc tggagctacn gtcctcactg 720
ttgccgcaat ttgactggnc ctgtttttgg ganctgcctg aaggcaatcc cggggctccc 780
cctggaggga g 791

<210> 181
<211> 747
<212> DNA
<213> Homo Sapiens

<400> 181
agtatccaaa catactcatt gttttatttt taacaaaaga aatgaaatta aagatagacc 60
acaggtagag tcatgaaatt cttgtttttc cctattcttt ttggtaatta caacgtacat 120
tgtcttcttt tataataaga cccaagggga gaaaagaaaa ggatgtacaa tgaaggtaca 180
agttttgaag caccaaaata ttttatgaca gggacaaaaa aacaaaaaac aaacaaaaat 240
tgaagtacag aaagagggtg gtgggggcaa aaataaaggt acgcacttgg gcttcctcaa 300
gatttgtttg tccctattca gactagaatg aaactggttt aggaaatcac tcctgtatgc 360
tagcaggaat gttgctggca agacacttct gagcatcggg gtgtggactt tacgaaccaa 420
ccttttaaca gtaactctag gagagaggat atcaaaaatt ggcagtgaaa aattatagat 480
aggcaaaaag ctccttctga ggtccaggcc aggagatagt angatttaag aaacaaacaa 540
acaataacaa ccacaaatgg acctttggtg ccactgtcac aactgttgct catcagagta 600
ggagaattgt ancaaaggca ttaaagaagg gacaagcaag ctgaagagcc tgaatccttg 660
gggttgtaag ccnattttgg gnttcctttc aagaaaaggg ctgttggncg gtggaanggg 720
tcanggaaca ntatttcacg ggtcngc 747

<210> 182
<211> 909
<212> DNA
<213> Homo Sapiens

<400> 182
aaacagagag ccaaatcatg agtgaactcc cattcacaat tgcttccaag ataataaaat 60
acctaggaat ccaacttaca aaggatgtga aggacctctt caaggagaac tacaaaccac 120
tgctcaatga aataaaagag gatacaaaca aatggaagaa cattccatgc tcatgggtag 180
gaagaatcaa tatcgtgaaa atggccatac tgcccaaggt aatgtataga ttcaatgcca 240
tccccatcaa gctaccaatg actttcttca cagaattgga aaaaactact caaaagttca 300
tatggaacca aaaaagagcc cacattgcca agtcaatcct aagccaaaag aacaaagctg 360
gaggcatcac gctacctgac ttcaaactat actacaaggc tacagtaacc aaaacagcgt 420
ggtactggta ccaaaacaga gatataaatc aatgcaacag aacagagccc tcagaaataa 480
tgccacatat ctacaactat ctgatctttg acaaacctga gaaaaacaag caatggggaa 540
aggattccct atttaataaa tggtgctggg aaaactggct agccatatgt agaaagctga 600
aactggatct cttctttata ccttatacaa aaattaattg aagatggntt aaaggactta 660
aacgttagac ctaaaaccat aaaaacccta gaagaaaaac ctaggcatta ccattcangg 720
acataggctt gggcaaggac ttcctgtcta aaacaccaan agcaatggga ncaaaagcca 780
aaattgcaaa tggggattct aattaactaa agggcttttg cacagcnaag aagctccatc 840
agagngaaca ggaacntcaa antgggagaa attttgaacc taccatcnga naaggctaat 900
nccagaatc 909

<210> 183

<211> 708
<212> DNA
<213> Homo Sapiens

<400> 183
attatcatta tactttaagt tttaggttac atgtgcacaa tgtgcaggtt agttacatat 60
gtatacatgt gccatgctgg tgtgctgcac ccattaactc gttatttagc attaggtata 120
tctcctaatg ctatccctcc cgcctccccc caccccacaa cagtccccag agtgtgatgt 180
tccccttcct gtgtccatgt gttctcactg ttcaattccc acctatgagt gagaatatgc 240
ggtgtttggt ttttttgtcc ttgccatagt ttactgagaa tgatgatttc caatttcatc 300
cctgtcccta caaaggacat gaactcatca ttttttatgg ctgcatagta ttccatggtg 360
tatatgtgcc acattttctt aatccagtct atcattgttg gccatttggg ttggttccaa 420
gtctttgcta ttgtgaatac tgccgcaata aacatacgtg tgcatgtgtc tttatagcag 480
catgatttat antcctttgg gtatatactc agtaatggga tggctgggtc aaatggnatt 540
ccaantccan atcccttang aattgccaca cggactccac aanggttgaa ctantttaca 600
gtcccancaa cagngtnaaa gggtccnaan tcnccaaaat cctctccaag caccngttgt 660
tcccggactt tttaanggat tgncaattcc aaccgggngt caaaaggg 708

<210> 184
<211> 855
<212> DNA
<213> Homo Sapiens

<400> 184
agactcacag tctgctggtg ggcagagaag acagaaacga catgagcaca gcaggaaaag 60
taatcaaatg caaagcagct gtgctatggg aggtaaagaa acccttttcc attgaggatg 120
tggaggttgc acctcctaag gcttatgaag ttcgcattaa gatggtggct gtaggaatct 180
gtcgcacaga tgaccacgtg gttagtggca acctggtgac cccccttcct gtgattttag 240
gccatgaggc agccggcatc gtggagagtg ttggagaagg ggtgactaca gtcaaaccag 300
gtgataaagt catcccgctc tttactcctc agtgtggaaa atgcagagtt tgtaaaaacc 360
cggagagcaa ctactgcttg aaaaatgatc taggcaatcc tcgggggacc ctgcaggatg 420
gcaccaggag gttcacctgc agggggaagc ccattcacca cttccttggc accagcacct 480
tctcccagta cacggtggtg gatgagaatg cagtggccaa aattgatgca gcctcgcccc 540
tggagaaagt ctgcctcatt ggctgtggat tctcgactgg gttatgggtc tgcagttaac 600
gttgccaagg tcaccccagg ctctacctgt gctgtgtgtg gcctgggaag ggtcggccta 660
tctgctgtta tgggctgtta aagcaactgg aggcanccag aatcaattgc ggtggacatc 720
aacaaggaca aattttgcaa agggcaaaag agttgggtgc cactgaatgc catcaaccct 780
caagnctnca ngnaaaccca tccaggnaag tgctaaaang gaatttaccg attggagggt 840
ttggattttt ccgtt 855

<210> 185
<211> 865
<212> DNA
<213> Homo Sapiens

<400> 185
cacagatgtt caatcaactg atgaagcaag tgtcaggact tactgttgac acagaggagc 60
ggctgaaagg agttattgac ctggtctttg agaaggctat tgatgaaccc agtttctctg 120
tggcttacgc aaacatgtgt cgatgtctag taacgctgaa agtacccatg gcagacaagc 180
ctggtaacac agtgaatttc cggaagctgc tactgaaccg ttgccagaag gagtttgaaa 240
aagataaagc agatgatgat gtctttgaga agaagcagaa agaacttgag gctgccagtg 300
ctccagagga gaggacaagg cttcatgatg aactggaaga agccaaggac aaagcccggc 360
ggagatccat tggcaacatc aagtttattg gagaactctt taaactcaaa atgctgactg 420
aagccatcat gcatgactgt gtggtgaagc tgctaaagaa ccatgatgaa gaatccctgg 480
agtgcctgtg tcgcctgctc accaccattg gcaaagactt ggactttgaa aaagcaaagc 540

cacgtatgga ccagtacttt aatcaagatg gagaaaattg tnaaagaaag aaaaacctca 600
tctagggatt cggttcatgc ttcaaagatg ttatanacct aaggctgttg caattggggt 660
atctcgaaag agcagatnaa gggcctnaan ctatcgaaca gattcacaaa ganggctaaa 720
attgaaanaa caagaatagc caaagggaag gnccaacaac tcatggacca anggagaaat 780
agaataccaa ggtgttccaa aaanttggcc aaangnnggt tggaaanacn gttcaaaggg 840
ggccangaaa aantccgggt actgg 865

<210> 186
<211> 736
<212> DNA
<213> Homo Sapiens

<400> 186
aaatatttgt tctatgtatt tacaagcctt aaagttgctc taaagatttc aagagtatta 60
agagtacttt tctcagggta gcactttngt ttttttaaac aattcttgga gttctgtggt 120
ccacagcatt tccttctgtt tcaatgttat gtatgttttg attactattg tgatttttta 180
aattttctga agcaagctga gaggcaggca gaaagatttg atgccaaaaa aaaaaaaatc 240
tttcttacct tgttcacccc aaactttctc aaatctggac taaatgctat accttaaaac 300
aaacatgagg tgcatcttga aggggaggga aatttatttc tctgcttttc tattatacaa 360
gttgtttaca gaaactgcaa attaaaaaat tacactggca tttgcagtcc ttaaaataaa 420
ttaaaagttc tcaacttttt tttttttttg ctaaacattt ttttaagtat gagtccttgt 480
ttaaaaagaa aagattaaaa cagaaaatat tttctataaa taatacatgt attttggttt 540
tagtgctccc gccctaaggt ttgaagttta cttttancca ngtacctttt tcctccatga 600
tcaccttttt ttctctttcc cctctcccaa ntccgtgcac acgtgggggt ttccggcaan 660
aattggcctt gctgnactgt gattgggcga anaacgttga aaaacctttt taaaaaaaaa 720
tacttaaaat tgggtt 736

<210> 187
<211> 946
<212> DNA
<213> Homo Sapiens

<400> 187
tgaaggagct acaggccgag caggaggacc gggctttaag gagttttaag ctgagtgtca 60
ctgtagaccc caaataccat cccaagatta tcgggagaaa gggggcagta attacccaaa 120
tccggttgga gcatgacgtg aacatccagt ttcctgataa ggacgatggg aaccagcccc 180
aggaccaaat taccatcaca gggtacgaaa agaacacaga agctgccagg gatgctatac 240
tgagaattgt gggtgaactt gagcagatgg tttctgagga cgtcccgctg gaccaccgcg 300
ttcacgcccg catcattggt gcccgcggca aagccattcg caaaatcatg gacgaattca 360
aggtggacat tcgcttccca cagagcggag ccccagaccc caactgcgtc actgtgacgg 420
ggctcccaga gaatgtggag gaagccatcg accacatcct caatctggag gaggaatacg 480
tgagtctctg tgggccttgg agccctgagg cgccctggca cgtccaccgg cctgaggccc 540
agccaggagc ttcaggggac aaggtggcac ttgtgtttcc agaggcaagc naagtgcagg 600
ggtgagcaag cnggcgggat gctgggggtg ctggggcaaa ctgaccctgt cttcctgtct 660
tccgcctgca gctagcctga cgttgtggac agtnaangcg cctgcangtt atacatgaaa 720
cccccagcac acgaanaagc caanggnacc tttcaaaagg ctttnttggt gccgggacca 780
acctgggacc gccagcaacc aatnaaaaaa ggcnctgacn ttaaccaagc tcngagggaa 840
tttcccancc tttgggggcc caaggtggct cccaaagaac cctccccntt nggggccccc 900
aaacnaatna ttgttcaaaa anggaacaaa aacccctctc aagccc 946

<210> 188
<211> 802
<212> DNA
<213> Homo Sapiens

<400> 188
aaagtcaagg ncgtttattt ccngaggnca tgacacanga agtggaatcc naaccacggn 60
tgcggnnnaa aagtgatgaa ggccaaagtg ctgactgaca tgccgggtgg accaaganct 120
ggagtcngtt atcntaacac gaatgcccan gaccttggtt taatgttaaa cantggagca 180
ngtcctganc gggcacggcc angcctggag gancggccgc acacacancc angcgcnagg 240
ctccctgcgg gacctcngga agggggaana gcgtcaacaa tttacggngg gtccaaccgc 300
tgggtcaaat tgagacaaac cantgtgtgg ttgggttcgg gtcancangc tgganangg t 360
tcngttcntt ttgatcanta ncntttgggg ccccaaggga nggtcntggg anccacctga 420
nccccaaagc tgggaaattc ctcaaagctg cncatgtcaa gagccttcnc antgctgctg 480
gcggtccaag gtgcgtcccg caccacaaag cctctggaag gngccntggc ctcttcctgt 540
gccgggggtt tcatgtntac ctgcancgcc tcactgtcca ccaangtcag ctaactgcag 600
gcnnaagaca ggaatnacag ggtcagtctg cccaacaacc ccancatccc ggcccgccct 660
ggctcaaacc ctgcaacctt gcctgccttc cgggaancac aatttcccac ccttgtnccc 720
ctgaaanccn cctggnctgg ggccntcaaa ggccgttgga ncttccanag gncnccccca 780
ggggntccca angggcccac aa 802

<210> 189
<211> 807
<212> DNA
<213> Homo Sapiens

<400> 189
aaaatggcgg cggcagcggt gtcgctttgt ttccgcggct cctgcggcgg tggcagtggt 60
agcggccttt gagctgtggg gaggttccag cagcagctac agtgacgact aagactccag 120
tgcatttcta tcgtaaccgg gcgcggggga gcgcagatcg gcgcccagca atcacagaag 180
ccgacaaggc gttcaagcga aaacatgacc gctgagccca tgagtgaaag caagttgaat 240
acattggtgc agaagcttca tgacttcctt gcacactcat cagaagaatc tgaagaaaca 300
agttctcctc cacgacttgc aatgaatcaa aacacagata aaatcagtgg ttctggaagt 360
aactctgata tgatggaaaa cagcaaggaa gagggaacta gctcttcaga aaaatccaag 420
tcttcaggat cgtcacgatc aaagaggaaa ccttcaattg taacaaagta tgtagaatca 480
gatgatgaaa aacctttgga tgatgaaact gtaaatgaag atgcgtctaa tgaaaattca 540
gaaaatgata ttactatgca nagcttgcca aaaggtacag tgattgttca gccagagcca 600
gtgctgaatg aagacaaaga tgattttaaa ggggcctgaa tttagaagca gaagttaaaa 660
tgaaaactga naatctcaaa aaacgccgga gaanatgggc ttcatgggga ttgtgangcc 720
tgcactggcn tggtggacaa caaggtcaat caatttcaaa aaggttccat ttatagacaa 780
cccttcaatg caaggtcnta tttgtta 807

<210> 190
<211> 608
<212> DNA
<213> Homo Sapiens

<400> 190
ccagttcttt ttttcccttc ttctggctca tcatctgaag atccatcctc atcagaggaa 60
agattggctt taatttcttc taaaagcatc ttcttggcaa ttctattctc aggatcattg 120
tcgtcatcat catcatccac tgtgacaggc actgatttag ataaggcttc atctcctgaa 180
gattggcaaa atccagtatg tgaagacagc actaaatttt cagtcacagg cttaattttc 240
tgttcatcgc tgcttccctc acctatagaa ttctgatcat catcttctat atcagaagaa 300
gatgaggatg taatgtcagc ttgcttcctt ttagtgcttg ttcttaggga gtttctcttt 360
ttctccttga caatgactgc cttcttttta gatgaagttc tttgcttctt ctttttacta 420
tcttcangaa ctttcctcag catcagatga tgatgangcc actttgtatt tccttagtat 480
ttctctttga acttaaattt cttctttccc tcaattcgag tcttttcagt caccttatca 540
gaagagttac aancatcttc tttcatggga agtatcaaga tgatgaacaa tcttgtcnct 600
tccttgaa 608

<210> 191
<211> 786
<212> DNA
<213> Homo Sapiens

<400> 191
gcactttgct gatggtggac agtgaggagg agtacttccc tgaagagatc gccaagctcc 60
ggagggacgt ggacaacggc ctctcgctcg tcatcttcag tgactggtac aacacttctg 120
ttatgagaaa agtgaagttt tatgatgaaa acacaaggca gtggtggatg ccggataccg 180
gaggagctaa catcccagct ctgaatgagc tgctgtctgt gtggaacatg gggttcagcg 240
atggcctgta tgaaggggag ttcaccctgg ccaaccatga catgtattat gcgtcagggt 300
gcagcatcgc gaagtttcca gaagatggcg tcgtgataac acagactttc aaggaccaag 360
gattggaggt tttaaagcag gaaacagcag ttgttgaaaa cgtccccatt ttgggacttt 420
atcagattcc agctgagggt ggaggccgga ttgtactgta tggggactcc aattgcttgg 480
atgacagtca ccgacagaag gactgctttt ggcttctgga tgccctcctc cagtacacat 540
cgtatggggt gacaccgcct agcctcagtc actctgggaa ccgccagcgc cctcccantt 600
ggagcaagct cagtcactcc agagaggatg gaaggaaacc atctcatcgg tactccaagg 660
ttctggangg ccatttggga aaaccaaaac ctcgggctcn acaaccctgt ccangcctgt 720
nctgggccaa gccaanagcc tttaaaccan aacggngccc aattaaccct ttggaaaaca 780
tcagaa 786

<210> 192
<211> 819
<212> DNA
<213> Homo Sapiens

<400> 192
gacgggtaat acatatttat tgaaaatttt cttcaccgac aatggtgaaa tcaagacctc 60
aaattacaaa acatggtggc aggtgatact tacaaaaata aagcgaaggt ctatgtttta 120
cagatttgtg catgtttcct tcaaatctca gtctgtactg tcattaaaaa gatcatggaa 180
tctatgttgt tcctcatgat ggaatagtaa aaaaactgca ttccactgac aaaaaaaata 240
gctttgcttc caaatagcac aagtctttaa agtgactttt cccaacaata aatatagaaa 300
atagccttta acaagcgtct tttagcttgg tcagggttgt atcatttgtt tggaaagtac 360
atccttcccc tgcagtcaga agaccccaga cagcctttcc agttctcccg agtctttggt 420
gcgcacagct gccggcggga agtctcactg gcggcagagc cactaagtcc ctcctgacgg 480
gatccacagg aatcttctcg atgtaccagg agcctctgcc catcacagga gggcaggccc 540
atgtagaaca agactctaac aaacctgcag ctggaaactg gattcctttt aaaccaaccc 600
gccaacacag ctcggntcac ccaccancgc cgtccgtnaa aggggctctc tgggcctcac 660
gggtcagcca ggttgccggt cacaccgaaa ggggtccttg ggcgggtgaa cctgctgcat 720
gaanctggcg gggngcttca accctgggct tcctccggct ttcggcctgg ncctgggcct 780
tgttgaantt gntccacaaa agaaaggcca ggagcaaca 819

<210> 193
<211> 744
<212> DNA
<213> Homo Sapiens

<400> 193
cagtcccagc acaacctgca ggggcatctg tccagcctgt tggccaggct ccggcagcag 60
tgtctgctgt acctactggc agtcagattg caaatattgg tcagcaagca aacataccta 120
ctgcagtgca gcagccctct acccaggttc caccttcagt tattcagcag ggtgctcctc 180
catcttcgca agtggttcca cctgctcaaa ctgggattat tcatcaggga gttcaaacta 240
gtgctccaag ccttcctcaa caattggtta ttgcatccca aagttccttg ttaactgtgc 300
ctccccagcc acaaggagta gaatcagtag ctcaaggaat tgtttcacag cagttgcctg 360
cagttagttc tttgccctct gctagtagta tttctgttac aagtcaggtt agttcaactg 420

gtccttctgg aatgccttct gccccaacaa acttggttcc accacaaaat atagcacaaa 480
cccctgctac ccaaaatggt aatttggttc aaagtgttaa gtcaacctcc cttgatagca 540
actaatacaa atttgccttt ggcacaacag ataccactaa gttctaccca agttctccgc 600
acaatcatta gctcaggcaa ttggaagcca aattgaagat gccaggcgtt gcagcggagc 660
cctccttaag ttggcttacc tcaagactaa tcagttggtg acaattgggg ggaatgttca 720
gcaagtttca agattgggaa gtta 744

<210> 194
<211> 567
<212> DNA
<213> Homo Sapiens

<400> 194
atcaacattt atatgcttta ttgaaagttg acaagtgcaa cagttaaata cagtgacacc 60
ttacaattgt gtagagaaca tgcacagaaa catatgcata taactactat acaggtgata 120
tgcagaaacc cctactggga aatccatttc attagttaga actgagcatt tttcaaagta 180
ttcaaccagc tcaattgaaa gacttcagtg aacaaggatt tacttcagcg tattcagcag 240
ctagatttca ggattacaca aagtgagtaa ctgtgccaaa ttcttaaaat ttctttaggt 300
gtggtttttg tcatgtagca gtttttatgt agatcnatat ntaaaagtcc acacctcctc 360
agacangcca atgaaacnac taaatttcaa tctgtacaan ctaaatagta attacagtcc 420
tctangtgnn caangatact tacaccacat anacaaatnt acnntacgca naacaacctt 480
catggggaag gatagcccta ggtccccagc tancctgtca ccatttttgt cactctcata 540
gttttggtgt ccaatccatt ggttttg 567

<210> 195
<211> 771
<212> DNA
<213> Homo Sapiens

<400> 195
gagagaacag agcaacaaga gcacaaagaa aaaaagaaga aatgaacaga ataagaacat 60
tagttgacaa tgcatacagc tgtgatccaa ggataaaaaa gttcaaggaa gaagaaaaag 120
ccaagaaaga ancanaaaag aaagcaaaag cagaagctaa acggaaggag caagaagcta 180
aagaaaaaca aagacaagct gaattagaag ctgctcggtt agctaaggag aaagaagagg 240
aggaagtcag acagcaagca ttgctggcaa agaaggaaaa agatatccag aaaaaagcca 300
ttaagaagga aaggcaaaaa tttcgaaact catgcaagac ctggaatcat ttttctgata 360
atgaggcaga gcgggttaaa atgatggaag aagtggaaaa actttgtgat cggcttgaac 420
tggcaagctt acagtgcttg aatgaaacac tcacatcatg cacaaaagaa gtnggaaagg 480
ctgctttgga aaaacagata gaagaaataa atgagcaaat cagaaaagag aaagaggaag 540
ctgaggctcg tatgcgacaa gcatctaaga acacagagaa atcaactggt ggaaggtgga 600
aaatggaagt aaaaattggg cacaaagatg ntctacaatt actaatttna aagctgtgaa 660
tcctgttncc tgctggaaca aantcaagat gggaagttat tgccaantac atgaacatac 720
attcctcccn cngggngtcc aaaaagaaac tgccaaaagn atgtttattg g 771

<210> 196
<211> 561
<212> DNA
<213> Homo Sapiens

<400> 196
acagtatttt cagttttatt ataaaaatgc acacacaaca aagattgtca tttcttggct 60
ctacttgcat tcagcacttg ttcttgagca gctttctttg cttttaccat ctcgacaagt 120
tccttgtatc gtttcatgca gtccttcttt gtcctgccag gcaccgcttc tgctattttt 180
tcccatcttt caggtgtatt tactgggtat gttttcaaag cttgttccaa aagcttctgt 240
tcttctgttg tccaaggggt gaagtctgta tatggacctt caaatcgttc tgaaggcgtt 300

gcgttgtctg cttgaggtac cactccatgt tcttttttga acttatcaaa tgccttttta 360
tttangtcag cttttgatg agggtcaagt ttttggagac tctttgcttt gccaataaca 420
tctttggnan gttcttttga ctccaagagg aagaangtnt ngttcatgtn antangcaan 480
aacgtcccat ctggaanttt tgttcnacca gggaacanac tcacaagctt taactaagta 540
antgtngnat naccgncngn c 561

<210> 197
<211> 691
<212> DNA
<213> Homo Sapiens

<400> 197
cgccacaacc acaaccagca ccacagcctc caccacccca gcagcagccg caacagcagc 60
cgcagcctca gccccagcag cctccacccc caccccctcc ccagcagcag cccccgctgt 120
cacagtgtan tatgaataac agtttcaccc cagctcctat gatcatggag ataccagaat 180
ctggaagcac tgggaacata agtatctatg agaggattcc aggggatttt ggtgccggca 240
gctactctca accatcagcc accttcagcc tagccaagct gcagcagctg accaacacca 300
ttatggaccc tcatgccatg ccttatagcc attctcctgc tgtgacttcc tatgcaacca 360
gtgtttctct gtccaataca ggactggctc agctggctcc atctcatccc ttagctggga 420
ctcctcaagc acangccacc atgacgccac ccccaaactt ggcatccact accatgaacc 480
tcacatctcc tctgcttcag tgcaacatgt ctgccaccaa cattggcatt cctcacacgc 540
aggagattgc aagggcaaat gccagtgaag gggcacattt ccatccgctc caagtttggc 600
ggcactgccc tctgcngctg ctcaccanna ngcagctggt atgggccgtn tccccaatcg 660
ggcagtttgc caatgcaang gcttgggccc t 691

<210> 198
<211> 646
<212> DNA
<213> Homo Sapiens

<400> 198
acctatccct ggagcaagta ataggaagag aatgggcaaa ctggttgcac gagagaaaag 60
agaatggagt tgggagcaac acatgaactt gcgttataac attctgctgt ccagatctgc 120
cctactgtgc tggtggtcgg tctgtccctc ttctcattag ccactcacag gagaggtgct 180
tgtgcactct gattcacagg ggatgaactc aggatctcaa aagacataca aaaactanag 240
gtatgtatca cttaagtagc tacgaaactc acaccgtgat ctcccttctg acacacatct 300
gcgccatctc ttccaacata aaatanactg tttcaatggt ttgtcagtta tttttcaaat 360
cactaanatg tacagtcatc caccaacaat ttaagaaaga acctaagagg caaatcactg 420
gggactgcta tttgagtttt atcagtcaaa ggctcaagca tcaanaccct cagttancat 480
ttcaaagtac atactangaa acancgaggc tgggtggcgt tgtgtgcgtt anggctgatt 540
caccaggtgg taaancaaca aagnggttaa gnctccnctt tttggattgt taattgncca 600
tcctcnattc ctccaaaagg gctgggattt ggatttggca aagtca 646

<210> 199
<211> 811
<212> DNA
<213> Homo Sapiens

<400> 199
cggcggcgct ccaggtgctg acagcgcgag agagcgcggc cctcaggagc aaggcgaatg 60
tatgacaaca tgtccacaat ggtgtacata aaggaagaca agttggagaa gcttacacag 120
gatgaaatta tttctaagac aaagcaagta attcaggggc tggaagcttt gaagaatgag 180
cacaattcca ttttacaaag tttgctggag acactgaagt gtttgaagaa agatgatgaa 240
agtaatttgg tggaggagaa atcaaacatg atccggaagt cactggagat gttggagctc 300
ggcctgagtg aggcacaggt tatgatggct ttgtcaaatc acctgaatgc tgtggagtcc 360

gagaagcaga aactgcgtgc gcaggttcgt cgtctgtgcc aggagaatca gtggctacgg 420
gatgaactgg ccaacacgca gcagaaactg cagaagagtg agcagtctgt ggctcaactg 480
gaggaggaga agaagcatct ggagtttatg aatcagctaa aaaaatatga tgacgacatt 540
tccccatccg aggacaaaga cactgattct accaaagagc ctctggatga cctttttccc 600
aatgatgaag acgacccagg gcaaggaatc cagcagcagc acagcagtgc agccgcggct 660
gcccagcaag gcngctacna agattcccgc gcggctgcgg acgctccaca acctgggtga 720
ttcagttcgc ctcnncangg ggccgctacc aaggtaacct gttgccccct cctggcaaag 780
caaggnccct gggaagggan cctgggagga a 811

<210> 200
<211> 763
<212> DNA
<213> Homo Sapiens

<400> 200
acacagtaaa tggattttat taatacagtt tatattacta agtacatatc tggcaaagct 60
acatgtatac agaaatcagg aaccccccca aaaaggacag cagcaccgaa aggaatggcc 120
agttcacaga gaggtgcagc tctgacaaga tcctagaggc tgctagacac agcgggcagc 180
actggagaga gaagggaagc tgcgggaggc gccacccgtc atgcaggaga cagtgtgaga 240
gtcacgggcg gctaggccat gggacgctga gcaagtcagt taaccagccc gagcttcatt 300
ttcctcattt cctcccctcc gtcagggcca ctctcgtact tgaccacgtc cacgttgagg 360
ctctcacggc tcctgcgctt ctccatgttc tcagggtcat tgagcacttc tgccaccctc 420
tgtttgtgaa cattgtcaag accctgttta cgagacctca tagcagcttc ttctaacgtt 480
tctgcagctt caaatttgcc ttgacgtctg taaagtgccc caaggttttt tagagtggtt 540
gtaacagttg gnctatcaac tttgcangct ttgtaccaac cgccatactc tccaaaaaga 600
tgtcccatcc ttttgctttc ctttgcattc ttctctttcc tcaacaatgc atccaaatgg 660
gtttaatttc aacatctaca gaaccaaact ccctttcatg tgcacaagtg agaatcnctt 720
tgtacantgt ttccgccttc cttgaacntt ccctgtttca aaa 763

<210> 201
<211> 717
<212> DNA
<213> Homo Sapiens

<400> 201
ggcgaatgta tgacaacatg tccacaatgg tgtacataaa ggaagacaag ttggagaagc 60
ttacacagga tgaaattatt tctaagacaa agcaagtaat tcaggggctg gaagctttga 120
agaatganca caattccatt ttacaaagtt tgctggagac actgaagtgt ttgaagaaag 180
atgatgaaag taatttggtg gaggagaaat caaacatgat ccggaagtca ctggagatgt 240
tggagctcgg cctgagtgag gcacaggtta tgatggcttt gtcaaatcac ctgaatgctg 300
tggagtccga gaagcagaaa ctgcgtgcgc aggttcgtcg tctgtgccag gagaatcagt 360
ggctacggga tgaactggcc aacacgcagc aagaaactgc agaagagtga gcagtctgtg 420
gctcaactgg aggaggagaa gaagcatctg gagtttatga atcagctaaa aaaatatgat 480
gacgacattt ccccatccga gggacaaaga cactgattct accaaagagc ctccggatga 540
ccttttcccc aatgatgaag acgaccccag ggcaagggaa tccancagca gcacagcaan 600
ttgcagccgc ggctgcccaa gcaaggcggc tacgagattc ccgccgcggc tgccggacgc 660
tccacaacct ggtnatccaa tacgccctcn caaggggcgc taccaagggt aactgtt 717

<210> 202
<211> 647
<212> DNA
<213> Homo Sapiens

<400> 202
cagtcggagt gagtttatta gaagttagaa agacacaaat acacaaatca ctgagcactt 60

caagattagt agagaaaagc agaatgccca aatttcacac acagactaca cagcaaatgc 120
tactggggca tatcctaggg agacccggag tccgagcggg gcccccaggg ctctaagtac 180
cacggagcac gtgcggcaca tgccttgctg taaggcttag ttacgtcaac aggtcaccgt 240
catgccattg caacaacacc ttgtgtgaca cttaactacc tgttaccaaa gtgaacagct 300
aatcgctctt aatttttaaa ctcgtgtatt acacagtaaa tggattttan taatacagtt 360
tatattacta agtacatatc tggcaaagct acatgtatac agaaatcagg aaccccccca 420
aaaaggacag cagcaccgaa aggaatggcc agttcacaga nangtgcagc tctgacaaga 480
tcctagangc tgctagacac agcgggcagc actggganaa gagaagggaa gctgcgggag 540
gcgccaaccc gtcatgccag gggacagtgt ganagtcacg ggncgggcta ngccaatggg 600
aacncctgan gcaangcagt ttaaccangc cccgnggctt caatttt 647

<210> 203
<211> 786
<212> DNA
<213> Homo Sapiens

<400> 203
cagccatgga cgccatcaag aagaagatgc agatgctgaa gctggacaag gagaacgcca 60
tcgaccgcgc cgagcaggcc gaagccgaca agaagcaagc tgaggaccgc tgcaagcagc 120
tggaggagga gcagcaggcc ctccagaaga agctgaaggg gacagaggat gaggtggaaa 180
agtattctga atccgtgaag gaggcccagg agaaactgga gcaggccgag aagaaggcca 240
ctgatgctga ggcagatgtg gcctccctga accgccgcat tcagctggtt gaggaggagc 300
tggaccgggc ccaggagcgc ctggctacag ccctgcagaa gctggaggag gccgagaagg 360
cggctgatga gagcgagaga ggaatgaagg tcatcgaaaa ccgggccatg aaggatgagg 420
agaagatgga actgcaggag atgcagctga aggaggccaa gcacatcgct gaggattcag 480
accgcaaata tgaagaggtg gccaggaagc tggtgatcct ggaaggagag ctggagcgct 540
cggaggagan ggctgaggtg gccgagagcc gagccagaca gctggaggag gaacttcgaa 600
ccatggacca ngccctcaag tccctgatgg cctcanagga ggagtattcc accaaagaag 660
attaatatga agaggagatn aaactgttgg anggagaagc tgaanggagg ctganacccc 720
aagcaaaagt ttgccnaaaa ggtctgtggg caaaaatttg ggngaaaaac catcnaatga 780
acctta 786

<210> 204
<211> 738
<212> DNA
<213> Homo Sapiens

<400> 204
   ggctagtaac atcagtttta ttgggttggg gtggcaacca tagcctggct gggggtgggg 60
   ctggccctca caggttgttg agttccagca gggtctggtc caaggtctgg tgaatctcga 120
   cgttctcctc cttggcactg gccaaggtct cttctaggtc atcgatggtt ttctccaact 180
   ttgccacaga cctctcggca aactctgctc gggtctcagc ctccttcagc ttctcctcca 240
   acagtttgat ctcctcttca tatttatctt ctttggtgga atactcctcc tctgaggcca 300
   tcagggactt gagggcctgg tccatggttc gaagttcctc ctccagctgt ctggctcggc 360
   tctcggccac ctcagccctc tcctccgagc gctccagctc tccttccagg atcaccagct 420
   tcctggccac ctcttcatat ttgcggtctg aatcctcagc gatgtgcttg gcctccttca 480
   gctgcatctc ctgcagttcc atcttctcct catccttcat ggcccggttt tcnatgacct 540
   tcattcctct ctcgctctca tcagcccgcc ttctcggctc ctccagcttc tgcanggctg 600
   tanccaangc gctcctgggc ccggtcaanc tcctcctcaa caagctgaat gcggcggttc 660
   aaggaaggca anatctgcct caacaacaat tggccttctt cncggccngc tccaattttc 720
   nccnggggcc tccttcaa 738

<210> 205
<211> 818
<212> DNA

<213> Homo Sapiens

<400> 205
gctagtaaca tcagttttat tgggttgggg tggcaaccat agcctggctg ggggtggggc 60
tggccctcac aggttgttga gttccagcag ggtctggtcc aaggtctggt gaatctcgac 120
gttctcctcc ttggcactgg ccaaggtctc ttctaggtca tcgatggttt tctccaactt 180
tgccacagac ctctcggcaa actctgctcg ggtctcagcc tccttcagct tctcctccaa 240
cagtttgatc tcctcttcat atttatcttc tttggtggaa tactcctcct ctgaggccat 300
cagggacttg agggcctggt ccatggttcg aagttcctcc tccagctgtc tggctcggct 360
ctcggccacc tcagccctct cctccgagcg ctccagctct ccttccagga tcaccanctt 420
cctggccacc tcttcatatt tgcggtctga atcctcagcg atgtgcttgg cctccttcag 480
ctgcatctcc tgcagttcca tcttctcctc atccttcaag gcccggtttt cgatgancct 540
tcattcctct ctcggctctc atcagccgcc ttctcgggct cntccaagct tctgcaaggc 600
tgtanncann ggctcctggg gcccgggtnc aagntcctcc tcaaacangc tnaaatncca 660
gagggtttca nggaagggcc aaaatctggc ctnnagnatc aattggcttt cttncncggg 720
nctngcncca attttctccn ggggcctncc tttcangggg tnaagaanaa atttcaaatt 780
caacctcggt tccccttnaa cntcntnctg gaagggct 818

<210> 206
<211> 927
<212> DNA
<213> Homo Sapiens

<400> 206
cagccatgga cgccatcaag aagaagatgc agatgctgaa gctggacaag gagaacgcca 60
tcgaccgcgc cgagcaggcc gaagccgaca agaagcaagc tgaggaccgc tgcaagcagc 120
tggaggagga gcagcaggcc ctccagaaga agctgaaggg gacagaggat gaggtggaaa 180
agtattctga atccgtgaag gaggcccagg agaaactgga gcaggccgag aagaaggcca 240
ctgatgctga ggcagatgtg gcctccctga accgccgcat tcagctggtt gaggaggagc 300
tggaccgggc ccaggagcgc ctggctacag ccctgcagaa gctggaggag gccgagaagg 360
cggctgatga gagcgagaga ggaatgaagg tcatcgaaaa ccgggccatg aaggatgagg 420
agaagatgga actgcaggag atgcagctga aggaggccaa gcacatcgct gaggattcag 480
accgcaaata tgaagaggtg gccaggaagc tggtgatcct ggaaggagag ctggagcgct 540
cggaggagag ggctgaggtg gccgagagcc gagccagaca gctggaggag gaacttcgaa 600
ccatggacca ggccctcaag tccctgatgg cctcagagga ggagtattcc accaaagaag 660
ataaatatga agaggagatc aaactgttgg aggagaagct gaaggaggct gagacccgag 720
cagagtttgc cgagaggtct gtggcaaagt tggagaaaac catcgatgac ctagaagaga 780
ccttggccag tgccaaggag gagaacgtcg agattcacca gaccttggac cagaccctgc 840
tggaactcaa caacctgtga gggccagccc cacccccagc caggctatgg ttgccacccc 900
aacccaataa aactgatgtt actagcc 927

<210> 207
<211> 910
<212> DNA
<213> Homo Sapiens

<400> 207
ggaagatggc ggcggccgtt ccacagcggg cgtggaccgt ggagcagctg cgcagtgagc 60
agctgcccaa gaaggacatt atcaagtttc tgcaggaaca cggttcagat tcgtttcttg 120
cagaacataa attattagga aacattaaaa atgtggccaa gacagctaac aaggaccact 180
tggttacagc ctataaccat ctttttgaaa ctaagcgttt taagggtact gaaagtataa 240
gtaaagtgtc tgagcaagta aaaaatgtga agcttaatga agataaaccc aaagaaacca 300
agtctgaaga gaccctggat gagggtccac caaaatatac taaatctgtt ctgaaaaagg 360
gagataaaac caactttccc aaaaagggag atgttgttca ctgctggtat acaggaacac 420
tacaagatgg gactgttttt gatactaata ttcaaacaag tgcaaagaag aagaaaaatg 480

ccaagccttt aagttttaag gtcggagtag gcaaagttat cagaggatgg gatgaagctc 540
tcttgactat gagtaaagga gaaaangctc gactggagat tgaaccagaa tgggcttacg 600
gaaagaaagg acagcctgat gccaaaattc caccaaatgc aaaactcact tttgaagtgg 660
nantatggga tattgattga aatagcagtg cntcagctcn aggntattag caacaatgat 720
taaaacntgg ncttgaaaga aaatttcaca actagttnag aaacttgtta ccaaatggta 780
aaggaaaaag tcaactggga aaaattcaag ggngttaana aaaanttggt ttacctgggg 840
cccaagcctt ttgngaaaaa aaaancccct tatgaaancc ccngggccca aaaanacttt 900
tccnaaaacc 910

<210> 208
<211> 745
<212> DNA
<213> Homo Sapiens

<400> 208
gacagtggat caatttttat tgagccactt aagtttacaa catgaggtaa aaggaaaaag 60
ttctccttga ccagtatttt acacagctgt aggaaagtat tttagaccag ggattcataa 120
gggatttatc tctcaaaagc tgggaccaag taaacaaatt ttattaactc cttgaatttt 180
ccagttgact cttcctttac aatagtaaca agttctaact agttgtgtaa atttcttcaa 240
ggccaagttt tatcattgtt gctaatatcc ttagagctga agcactgcta tttcaatcaa 300
tatccactaa ttccacttca aaagtgagtt ttgcatttgg tggaattttg gcatcaggct 360
gtcctttctt tccgtaagcc cattctggtt caatctccag tcgagccttt tctcctttac 420
tcatagtcaa gagagcttca tcccatcctc tgataacttt gcctactccg accttaaaac 480
ttaaaggctt ggcatttttc ttcttctttg cacttgtttg aatattagta tcaaaaacag 540
tcccatcttg tagtgttcct gtataccaag caagtgaaca acatcnccct ttttgggaaa 600
gttggtttaa cccccttttt cagaacagat ttaagtanat tttgggggac cctcanccaa 660
ggggtcnctt canaactggg tttccttggg gtttaacctt cattnagcct canaattttt 720
tacntggccn cagacacttt tactt 745

<210> 209
<211> 965
<212> DNA
<213> Homo Sapiens

<400> 209
ggaagatggc ggcggccgtt ccacagcggg cgtggaccgt ggagcagctg cgcagtgagc 60
agctgcccaa gaaggacatt atcaagtttc tgcaggaaca cggttcagat tcgtttcttg 120
cagaacataa attattagga aacattaaaa atgtggccaa gacagctaac aaggaccact 180
tggttacagc ctataaccat ctttttgaaa ctaagcgttt taagggtact gaaagtataa 240
gtaaagtgtc tgagcaagta aaaaatgtga agcttaatga agataaaccc aaagaaacca 300
agtctgaaga gaccctggat gagggtccac caaaatatac taaatctgtt ctgaaaaagg 360
gagataaaac caactttccc aaaaagggag atgttgttca ctgctggtat acaggaacac 420
tacaagatgg gactgttttt gatactaata ttcaaacaag tgcaaagaag aagaaaaatg 480
ccaagccttt aagttttaag gtcggagtag gcaaagttat cagaggatgg gatgaagctc 540
tcttgactat gagtaaagga gaaaaggctc gactggagat tgaaccaaga atgggcttac 600
ggaaagaaag gacagcctga tgccaaaatt ccaccaaatg caaaactcac ttttgaagtg 660
gaattagtgg atattgattg aaatagcagt gcttcagcct ccaagggata ttagcaacaa 720
tgaataaaac tttggncttg angaaaattt acacaaccta gtttagaacc ttgttactat 780
tgttaaagga aagaagtcaa ctgggnaaaa ttcaagggag ttaataaaat ttgtttactt 840
ggncccagcc ttttgagaga taaatccctt angaaancct ggtccnaaaa tactttccta 900
aagnctgtgt taaataccng ggncaagggn gaaacttttt ccctttaccn caagggtggt 960
aaact 965

<210> 210
<211> 867

<212> DNA
<213> Homo Sapiens

<400> 210
caagacagtg gatcaatttt tattgagcca cttaagttta caacatgagg taaaaggaaa 60
aagttctcct tgaccagtat tttacacagc tgtaggaaag tattttagac cagggattca 120
taagggattt atctctcaaa agctgggacc aagtaaacaa attttattaa ctccttgaat 180
tttccagttg actcttcctt tacaatagta acaagttcta actagttgtg taaatttctt 240
caaggccaag ttttatcatt gttgctaata tccttagagc tgaagcactg ctatttcaat 300
caatatccac taattccact tcaaaagtga gttttgcatt tggtggaatt ttggcatcag 360
gctgtccttt ctttccgtaa gcccattctg gttcaatctc cagtcgagcc ttttctcctt 420
tactcatagt caagagagct tcatcccatc ctctgataac tttgcctact ccgaccttaa 480
aacttaaagg cttggcattt ttcttcttct ttgcacttgt ttgaatatta gtatcaaaaa 540
cagtcccatc ttgtagtgtt cctgtatacc angcagtgaa caacatctcc ctttttggga 600
aagtttgggt ttaactccct tttttcagaa caagatttag taaaattttg gnnggaccct 660
caatccaagg gtctcttcaa nacttgggtt cctttggggt ttaancctca attaagcctc 720
acaatttttt acttggctca agaaancntt tacttaaacc tttcaggtac cctttaaaaa 780
nccttangtt ttaaaaaaaa tgggttataa gggctggtaa ccnaaggttg ggcccttggt 840
aaccngttct tggggcaaaa tttttaa 867

<210> 211
<211> 972
<212> DNA
<213> Homo Sapiens

<400> 211
ggaagatggc ggcggccgtt ccacagcggg cgtggaccgt ggagcagctg cgcagtgagc 60
agctgcccaa gaaggacatt atcaagtttc tgcaggaaca cggttcagat tcgtttcttg 120
cagaacataa attattagga aacattaaaa atgtggccaa gacagctaac aaggaccact 180
tggttacagc ctataaccat ctttttgaaa ctaagcgttt taagggtact gaaagtataa 240
gtaaagtgtc tgagcaagta aaaaatgtga agcttaatga agataaaccc aaagaaacca 300
agtctgaaga gaccctggat gagggtccac caaaatatac taaatctgtt ctgaaaaagg 360
gagataaaac caactttccc aaaaagggag atgttgttca ctgctggtat acaggaacac 420
tacaagatgg gactgttttt gatactaata ttcaaacaag tgcaaagaag aagaaaaatg 480
ccaagccttt aagttttaag gtcggagtag gcaaagttat cagaggatgg gatgaagctc 540
tcttgactat gagtaaagga gaaaaggctc gactggagat tgaaccagaa tgggcttacg 600
gaaagaaagg acagcctgat gccaaaattc caccaaatgc aaaactcact tttgaagtgg 660
aattagtgga tattgattga aatagcagtg cttcagctct aaggatatta gcaacaatga 720
taaaacttgg ccttgaagaa atttacacaa ctagttagaa cttgttacta ttgtaaagga 780
agagtcaact ggaaaattca aggagttaat aaaatttgtt tacttggtcc cagcttttga 840
gagataaatc ccttatgaat ccctggtcta aaatactttc ctacagctgt gtaaaatact 900
ggtcaaggag aactttttcc ttttacctca tgttgtaaac ttaagtggct caataaaaat 960
tgatccactg tc 972

<210> 212
<211> 817
<212> DNA
<213> Homo Sapiens

<400> 212
aacggctcta agggttatgc ctttgtccac ttcgagaccc aagaggctgc cgacaaggcc 60
atcgagaaga tgaatggcat gctcctcaat gaccgcaaag tatttgtggg cagattcaag 120
tctcgcaaag agcgggaagc tgagcttgga gccaaagcca aggaattcac caatgtttat 180
atcaaaaact ttggggaaga ggtggatgat gagagtctga aagagctatt cagtcagttt 240
ggtaagaccc taagtgtcaa ggtgatgaga gatcccaatg ggaaatccaa aggctttggc 300

tttgtgagtt acgaaaaaca cgaggatgcc aataaggctg tggaagagat gaatggaaaa 360
gaaataagtg gtaaaatcat atttgtaggc cgtgcacaaa agaaagtaga acggcaggca 420
gagttaaaac ggaaatttga acagttgaaa caggagagaa ttagtcgata tcagggggtg 480
aatctctaca ttaagaactt ggatgacact attgatgatg agaaattaag gaaagaattt 540
tctccttttg gatcaattac cagtgctaag gtaatgctgg aggatggaag aagcaaaggg 600
tttggcttcg tctgcttctc atctcctgaa gaancaacca aagcagtcac tggagatgaa 660
tggacgcatt ttggggctcc aaccactata tgttgccctg gccccanagg aagggaanag 720
agaaaggntc accttgacca accagtttta tgcaacgaan tggctgggaa tngagaacca 780
cttcccngcc aatgccaatc tttaaantca gnttcca 817

<210> 213
<211> 756
<212> DNA
<213> Homo Sapiens

<400> 213
ctttgatgtg attaagggaa agccaatccg catcatgtgg tctcagaggg atccctcttt 60
gagaaaatct ggtgtgggaa acgtcttcat caagaacctg gacaaatcta tagataacaa 120
ggcactttat gatacttttt ctgcttttgg aaacatactg tcctgcaagg tggtgtgtga 180
tgagaacggc tctaagggtt atgcctttgt ccacttcgag acccaagagg ctgccgacaa 240
ggccatcgag aagatgaatg gcatgctcct caatgaccgc aaagtatttg tgggcagatt 300
caagtctcgc aaagagcggg aagctgagct tggagccaaa gccaaggaat tcaccaatgt 360
ttatatcaaa aactttgggg aagaggtgga tgatgagagt ctgaaagagc tattcagtca 420
gtttggtaag accctaagtg tcaaggtgat gagagatccc aatggaaaat ccaaaggctt 480
tggctttgtg agttacgaaa aacacgagga tgccaataag gctgtggaag agatgaatgg 540
aaaagaaata agtggtaaaa tcatatttgt aggccgtgca caaaagaaag tagaacggca 600
agcagagtta aaacggaaat ttgaacagtt gaaacaggag agaattagtc gatatcangg 660
ggtgaatccc cacattaaga acttggatga cactattgat gatgaagaaa attaaggaaa 720
agaattttcn ccntttggga tnaattaaca agttgc 756

<210> 214
<211> 728
<212> DNA
<213> Homo Sapiens

<400> 214
atggagattt tttttcttta ttgggaaacg taagacttgg gtacatcaaa taaaaccaat 60
ttctggggga aaaaatcaaa ncccacaata aaaaaaaagt taacactgtc tgggccacag 120
cagaacccaa agaacatatt cgtataattg aaaaattcta ggtgcttcat aattgacctt 180
ttgatacaaa atgacctatt aaatttgcaa tttgtaatcc ttggtgttga ggtccatagg 240
acaagctagg aagtcttcaa accttgagtt gaattccata aggggttatt tggcttttga 300
atcggttttt ccttgtctaa gaggtagcag cagcaacagc gcccaccttc tgggcagctt 360
ctttcttggc atgatgagcc tgtagaactg ctacagcttc atccaccttg gagcggagag 420
actcggggga ctctaacatg tgcagcagct canagttgtc tatctccagc agcattcccg 480
tgatcttccc agccagattt gaatgcattg tttggatgan tgggaacaag cgttctccca 540
gcatctgctt ctgttcctgg gggggtgctg canccaacag gaggcaatca ntggntccng 600
gccctgcaca tggaccgcaa ggctggggtg cctgcaaaan gctgtatggc aaggatgaag 660
ggctgccgac actgggaagg cggtattngt aggggggcaa aaancccggg gaagcancag 720
caacaaca 728

<210> 215
<211> 710
<212> DNA
<213> Homo Sapiens

<400> 215
atgganattt tttttcttta ttgggaaacg taagacttgg gtacatcaaa taaaaccaaa 60
atctggggga aaaaatcaaa acccacaata aaaaaaaagt taacactgtc tgggccacag 120
canaacccaa agaacatatt cgtataattg aaaaattcta ggtgcttcan aattgacctt 180
ttgatacaaa atgacctatt aaatttgcaa tttgtaancc ttggtgttga ggtccatagg 240
acaagctagg aagtcttcaa accttgagtt gaattccana aggggttatt tggcttttga 300
atcggttttt ccttgtctaa naggtagcag cagcaacagc gcccaccttc tgggcagctt 360
ctttcttggc atgatgagcc tgtanaactg ctacagcttc atccaccttg gagcgganag 420
actcggggga ctctaacatg tgcagcagct canagttgtc tatctccagc agcattcccg 480
tgatcttccc agccagattt gaatgcattg tttggatgan tgggaacaag cgttctccca 540
gcatctgctt ctgttcctgn gggggtgctg canccangca tggaggcaan tcagtggctc 600
ctgcccctgc acaatggacc gcaaggctgg ggggtgcctg canaaggctg tttgggcaag 660
gangaagggc ctgcggaana ctgggangcg tatttgttan ggggggcaaa 710

<210> 216
<211> 824
<212> DNA
<213> Homo Sapiens

<400> 216
catggcctcc ctgtacgtgg gcgacctgca ttcggacgtc accgaggcca tgctgtacga 60
aaagttcagc cccgcggggc ctgtgctgtc catccgggtc tgccgcgata tgatcacccg 120
ccgctccctg ggctatgcct acgtcaactt ccagcagccg gccgacgctg agcgggcttt 180
ggacaccatg aactttgatg tgattaaggg aaagccaatc cgcatcatgt ggtctcagag 240
ggatccctct ttgagaaaat ctggtgtggg aaacgtcttc atcaagaacc tggacaaatc 300
tatagataac aaggcacttt atgatacttt ttctgctttt ggaaacatac tgtcctgcaa 360
ggtggtgtgt gatgagaacg gctctaaggg ttatgccttt gtccacttcg agacccaaga 420
ggctgccgac aaggccatcg agaagatgaa tggcatgctc ctcaatgacc gcaaagtatt 480
tgtgggcaga ttcaagtctc gcaaagagcg ggaagctgag cttggagcca aagccaagga 540
attcaccaat gtttatatca aaaactttgg ggaanaggtg gatgatgaga gtctgaaaga 600
agctattcan tcaagtttgg taagacccta agtgtcaang tgatgagaga tccaatggga 660
aatccaaaag gctttgggct ttgtgagttn acgaaaaaca cnaggatgcc aataaggctg 720
ttggaaagaa atgaatggga aaagaaataa antggtaaaa tcataatttg tagggccgtn 780
cacaaaaaga aagtttaaac gggnaggcaa aatttaaaac cggg 824

<210> 217
<211> 749
<212> DNA
<213> Homo Sapiens

<400> 217
atggagattt tttttcttta ttgggaaacg taagacttgg gtacatcaaa taaaaccaat 60
ttctggggga aaaaatcaaa acccacaata aaaaaaaagt taacactgtc tgggccacag 120
cagaacccaa agaacatatt cgtataattg aaaaattcta ggtgcttcat aattgacctt 180
ttgatacaaa atgacctatt aaatttgcaa tttgtaatcc ttggtgttga ggtccatagg 240
acaagctagg aagtcttcaa accttgagtt gaattccata aggggttatt tggcttttga 300
atcggttttt ccttgtctaa gaggtagcag cagcaacagc gcccaccttc tgggcagctt 360
ctttcttggc atgatgagcc tgtagaactg ctacagcttc atccaccttg gagcggagag 420
actcggggga ctctaacatg tgcagcagct cagagttgtc tatctccagc agcattcccg 480
tgatcttccc agccaagatt tgaatgcatt gtttggatga gtgggaacaa gcgttctccc 540
agcatctgcn tctgttcctg ggggggtgct gcatccagca tgggangcan tcagtggctc 600
ctgcccctgc acatgggacc gcaaggctgg ggtgcctgca naggctgtat gggaaggatg 660
nagggctgcc ggncaactgg ganggcgtat ttgtaggggg caaacaagcc cggggaagca 720
nccagcagca acancaacng cttggcgcc 749

<210> 218
<211> 600
<212> DNA
<213> Homo Sapiens

<400> 218
   ctttattggg aaacgtaaga cttgggtaca tcaaataaaa ccaatttctg ggggaaaaaa 60
   tcaaaaccca caataaaaaa aaagttaaca ctgtctgggc cacagcagaa cccaaagaac 120
   atattcgtat aattgaaaaa ttctaggtgc ttcataattg accttttgat acaaaatgac 180
   ctattaaatt tgcaatttgt aatccttggt gttgaggtcc ataggacaag ctaggaagtc 240
   ttcaaacctt gagttgaatt ccataagggg ttatttggct tttgaatcgg tttttccttg 300
   tctaagaggt agcancagca acagcgccca ccttctgggc agcttctttc ttggcatgat 360
   gancctgtag aactgctaca gcttcatcna ccttggagcg gngagactcg ggggactcta 420
   acatgtgcag cagctcagag ttgtcnatct ccaagcagca ttcccgtgat cttcccagcc 480
   anatttgaat gcattgtttg ggatgangtg gggaanaagc gttctcncag canncngctt 540
   cnggtnccnn ggaggggggt gcntgcaagc ccagcattga aggcaagttc antggctcct 600

<210> 219
<211> 1077
<212> DNA
<213> Homo Sapiens

<400> 219
catggcctcc ctgtacgtgg gcgacctgca ttcggacgtc accgaggcca tgctgtacga 60
aaagttcagc cccgcggggc ctgtgctgtc catccgggtc tgccgcgata tgatcacccg 120
ccgctccctg ggctatgcct acgtcaactt ccagcagccg gccgacgctg agcgggcttt 180
ggacaccatg aactttgatg tgattaaggg aaagccaatc cgcatcatgt ggtctcagag 240
ggatccctct ttgagaaaat ctggtgtggg aaacgtcttc atcaagaacc tggacaaatc 300
tatagataac aaggcacttt atgatacttt ttctgctttt ggaaacatac tgtcctgcaa 360
ggtggtgtgt gatgagaacg gctctaaggg ttatgccttt gtccacttcg agacccaaga 420
ggctgccgac aaggccatcg agaagatgaa tggcatgctc ctcaatgacc gcaaagtatt 480
tgtgggcaga ttcaagtctc gcaaagagcg ggaagctgag cttggagcca aagccaagga 540
attcaccaat gtttatatca aaaactttgg ggaagaggtg gatgatgaga gtctgaaaga 600
gctattcagt cagtttggta agaccctaag tgtcaaggtg atgagagatc ccaatgggaa 660
atccaaaggc tttggctttg tgagttacga aaaacacgag gatgccaata aggctgtgga 720
agagatgaat ggaaaagaaa taagtggtaa aatcatattt gtaggccgtg cacaaaagaa 780
agtagaacgg caagcagagt taaaacggaa atttgaacag ttgaaacagg agagaattag 840
tcgatatcan ggggtgaatc cccacattaa gaacttggat gacactattg atgatgagaa 900
attaaggaaa gaattttctc cttttggatc aattaccagt gctaaggtaa tgctggagga 960
tggaagaagc aaagggtttg gcttcgtctg cttctcatct cctgaagaan caaccaaagc 1020
agtcactgga gatgaatgga cgcatttggg ggctccaacc actatatgtt gccctgg 1077

<210> 220
<211> 1007
<212> DNA
<213> Homo Sapiens

<400> 220
actacatcga tcgcgtggac gagcccttgt cctgctctta tgtgctgacc attcgcactc 60
ctcggctctg cccccaccct ctcctccggc ccccacccag tgctgcaccg caggccatcc 120
tctgtcaccc ttccctacag cctgaggagt acatggccta cgttcagagg caagccgact 180
caaagcagta tggagataaa atcatagagg agctgcaaga tctaggcccc caagtgtgga 240
gtgagaccaa gtctggggtg gcaccccaaa agatggcagg tgcgagcccg accaaggatg 300
acagtaagga ctcagatttc tggaagatgc ttaatgagcc agaggaccag gccccaggag 360
gggaggaggt gccggctgag gagcaggacc caagccctga ggcagcagat tcagcttctg 420

gtgctcccaa tgattttcag aacaacgtgc aggtcaaagt cattcgaagc cctgcggatt 480
tgattcgatt catagaggag ctgaaaggtg gaacaaaaaa ggggaagcca aatataggcc 540
aagagcagcc tgtggatgat gctgcagaag tccctcagag ggaaccagag aaggaaaggg 600
gtgatccaga acggcagaga gagatggaag aagaggagga tgaggatgag gatgaggatg 660
aagatgagga tgaacggcag ttactgggag aatttgagaa ngaactggaa gggatcctgc 720
ttccgtcaga ccgagaccgg ctccgttcgg aggtgaangc tggcatggag ccgggaactg 780
gnaaacatca tccaggagac angagaaaga nctgggaccc anatggggct gaagaangga 840
tcagaatccg ggatcggcaa atgctggctc tcaaaatcaa ctctcaacaa antcattaaa 900
aagactggag ggaaaaacaa gagttccaaa ncctggtgaa nnaagcncat aaaaaagaag 960
gttgtcccaa aaaagnctcc cccatcaanc caaccctnca gggaaaa 1007

<210> 221
<211> 833
<212> DNA
<213> Homo Sapiens

<400> 221
ccgactcaaa gcagtatgga gataaaatca tagaggagct gcaagatcta ggcccccaag 60
tgtggagtga gaccaagtct ggggtggcac cccaaaagat ggcaggtgcg agcccgacca 120
aggatgacag taaggactca gatttctgga agatgcttaa tgagccagag gaccaggccc 180
caggagggga ggaggtgccg gctgaggagc aggacccaag ccctgaggca gcagattcag 240
cttctggtgc tcccaatgat tttcagaaca acgtgcaggt caaagtcatt cgaagccctg 300
cggatttgat tcgattcata gaggagctga aaggtggaac aaaaaagggg aagccaaata 360
taggccaaga gcagcctgtg gatgatgctg cagaagtccc tcagagggaa ccagagaagg 420
aaaggggtga tccagaacgg cagagagaga tggaagaaga ggaggatgag gatgaggatg 480
aggatgaaga tgaggatgaa cggcagttac tgggagaatt tgagaangaa ctggaaggga 540
tcctgcttcc gtcagaccga gaccggctcc gttcggaggt gaangctggc atggagccgg 600
gaactggnaa acatcatcca ggagacanga gaaaganctg ggacccanat ggggctgaag 660
aanggatcag aatccgggat cgggcaatgc tggctctcaa aatcaactct caacaaantc 720
attaaaaaga ctggagggaa aaacaagagt tccaaancct ggtgaannaa gcncataaaa 780
aagaaggttg tcccaaaaaa gnctccccca tcaanccaac cctncaggga aaa 833

<210> 222
<211> 745
<212> DNA
<213> Homo Sapiens

<400> 222
ggattgatgg tccagttgtt tatttagaaa cctgattgtt caagaacatg gtgggtgctt 60
cacacctttt tcactgggat tgtgctggag gtgataggca gcattctacc atttcctcag 120
caacagaggt gaaggctcct caactcagaa gcacaaattg taggggacag ggtgggcagg 180
gaaagggaga aggaaatccc aaggcaattc aatagaagag ggtaaaacga ctccaaacat 240
cactaagggc aggtgggggc ctgcttgctc agtgcctgct aagtgtcctg ccctccttgc 300
tctctctacc cacctccact caaaagatcc tactgaatct ccaggtaggc agcagggaat 360
atcctatcat taggggacaa taacaggaaa agccacagag gagaggaaga ggattgagtg 420
agagttcagg agagcaaata tcacaggccc ggtgaggtct caaggtggct gccagcaggg 480
gcagcaagca ttcacccagg gcccccacac ccacagagtt gcccgagang tccacaagct 540
cagctccact ctgctgtttg gccctcaagg gttccagggt ggggaagtgg ggaagaggca 600
ngccagtcca ggaagatctg gattccgtga angggtcaag tgtagtgttg gtctcagaag 660
tcaaattntc caagtcccct gttgccctcc ccacctggag aagccccana cccggnggta 720
attgctcncc anctccttct gccgc 745

<210> 223
<211> 747
<212> DNA

<213> Homo Sapiens

<400> 223
actacatcga tcgcgtggac gagcccttgt cctgctctta tgtgctgacc attcgcactc 60
ctcggctctg cccccaccct ctcctccggc ccccacccag tgctgcaccg caggccatcc 120
tctgtcaccc ttccctacag cctgaggagt acatggccta cgttcagagg caagccgact 180
caaagcagta tggagataaa atcatagagg agctgcaaga tctaggcccc caagtgtgga 240
gtgagaccaa gtctggggtg gcaccccaaa agatggcagg tgcgagcccg accaaggatg 300
acagtaagga ctcagatttc tggaagatgc ttaatgagcc agaggaccag gccccaggag 360
gggaggaggt gccggctgag gagcaggacc caagccctga ggcagcagat tcagcttctg 420
gtgctcccaa tgattttcag aacaacgtgc aggtcaaagt cattcgaagc cctgcggatt 480
tgattcgatt catagaggag ctgaaaggtg gaacaaaaaa ggggaagcca aatataggcc 540
aagagcagcc tgtggatgat gctgcagaag tccctcagag ggaaccagag aangaaaggg 600
gtgatccaga acggcagaga gagatgggaa ngaagangan gatgaggatg aggatgaggg 660
atgaaagann aaggatgaaa cgggcaagtt actggggaan aattttgana aagggaactg 720
ggaaagggat tcctggcttt ccgttca 747

<210> 224
<211> 618
<212> DNA
<213> Homo Sapiens

<400> 224
gatggtccag ttgtttattt agaaacctga ttgttcaaga acatggtggg tgcttcacac 60
ctttttcgct gggattgtgc tggaggtgat aggcagcatt ctaccatttc ctcagcaaca 120
gaggtgaagg ctcctcaact cagaagcaca aattgtaggg gacagggtgg gcagggaaag 180
ggagaaggaa atcccaaggc aattcaatag aagagggtaa aacgactcca aacatcacta 240
agggcaggtg ggggcctgct tgctcagtgc ctgctaagtg tcctgccctc cttgctctct 300
ctacccacct ccactcaaaa gatcctactg aatctccagg tangcancan ggaatatcct 360
atcattaggg gacaatanca ggaaaagcca cagaggagag gaagaggatt gagtganaag 420
ttcangacag caaattatca caggcccggt gaggtctcaa ngtgngctgc caacaagggg 480
caancagcat tcacccangg gccccacacc cacnnnagtt gccccagagg tccacanctc 540
ancctcccan ctgccngttt ggccctcaag gggttccaan gttcgngaaa gtgggggagg 600
aaggcanccc antcccag 618

<210> 225
<211> 765
<212> DNA
<213> Homo Sapiens

<400> 225
caaacatcag agactgcatg ctggagagaa acttgaagaa tgtgagaaaa ccttcagcaa 60
ggatgaggag cttagaaaag agcagagaac tcaccaggaa aagaaagttt attggtgtaa 120
tcagtgtagt aggaccttcc agggcagctc agatctcatc agacatcagg taactcatac 180
aagagagaaa ccatatgaat gtaaagaatg tgggaaaact caatcagagc tcagaccttc 240
tgagacatca tagaattcac agtggagaaa aaccttacgt atgcaataaa tgtggggaat 300
cttttaggag cagctcagat cttattaaac accatcgtgt tcatactgga gagaaacctc 360
atgaatgtag tgaatgtggg aaagtcttta gccagaggtc ccaccttgtc acacaccaga 420
aaatccacac tggagagaag ccctatcagt gcactgaatg tgaaaaagcc ttcaggcggc 480
gttcactcct tattcaacgt cggagaattc atagtggtga gaaaccctat gaatgtaagg 540
aatgtgggaa actcttcatg tggcacacag ctttcctcaa acatcagaga ctgcatgctg 600
gagagaaact tgaagaatgt gagaaaacct tcagcaagga tganggagct taggggagag 660
cagaaaattc accanggaag agaaagcctt attgggngta atcagtgtgg tanggctttc 720
caagggcagc tcangacctc atcgggccat caggtaactc aatac 765

<210> 226
<211> 791
<212> DNA
<213> Homo Sapiens

<400> 226
tggatccaaa gcacccctgg cactgttgtt tatggcccac ctcctgctgg ggcccccatg 60
gtgtatgggc ctccaccccc caacttctcc atccccttca tccctatggg tgtgctgcat 120
tgcaacgtcc ctgaacacca taacttagag aatgaagttt ctagattaga agacataatg 180
cagcatttaa aatcaaagaa gcgggaagaa aggtggatga gagcatccaa gcggcagtcg 240
gagaaagaaa tggaagaact gcatcataat attgatgatc ttttgcaaga gaagaaaagc 300
ttagagtgtg aagtagaaga attacataga actgtccaga aacgtcaaca gcaaaaggac 360
ttcattgatg gaaatgtaga gagtcttatg actgaactag aaatagaaaa atcactcaaa 420
catcatgaag atattgtaga tgaaattgag tgcattgaga agactcttct gaaacgtcgc 480
tcanagctca gggaagctga ccgactcctg gcagaggctg agagtgaact ttcatgcact 540
aaagaaaaga caaaaaatgc tgttgaaaag ttcactgatg ccaagagaag tttattgcaa 600
actgagtcag atgctgaggg aattagaaag gagagctcan gaaactgctg ttaanctcgt 660
caaanctgat cagcagctaa gatcgctcca agctgatgca aaaggatttg gancancaca 720
angatcaagc aagaagaaat cttgaaaaga aattaacnaa aatttntnca gcaaaagact 780
cagacttcaa a 791

<210> 227
<211> 687
<212> DNA
<213> Homo Sapiens

<400> 227
gattgttatc ttttattttc atatgaaaaa tagattttaa gcaaaattca aaaataactc 60
gacactataa aaanagaggg ccttaagtac attctttttg ttaataagat ttaccagttt 120
gtaggttcaa atatgcagtt aaaatcactg ttttttttta aacatgttac gaagattaaa 180
aaaaaaaagg ctcagccaca tgttggttta aattcccata tgcaactatt cccatatgta 240
ctatgtacaa gtgatttata aaaacattgg cattaatggt acaggcaaag taaactacag 300
tggagtttca naatctcagt tcactgcatc ttgattaaaa aaaccatgtg acattccaat 360
tatgaagtca gtgaggtagt ggaggtgttt tccttgaata tatttacaca agacagtatt 420
cctcatctgg ctgaggcatt cttttccgga ttttgtccaa gttganagtc ctctgtgagg 480
gaagactcca agctgagaca gactgggtga tgacgctgaa tctgcaaagg tgcctggtga 540
ccaattcccc ctaanagcat cctacttgtc tccncaaact gtgntaaagt gccctctgtc 600
ctgccgcttt cctttaatna aaacttctgg cttngcttgg ggcanacagt gtcgganttg 660
gggccttgag tcnggcttcc cggggaa 687

<210> 228
<211> 810
<212> DNA
<213> Homo Sapiens

<400> 228
gtctgggcag cgccaggcga tggccctgct gctggtgctc ctcgcctctt ggggcctggg 60
gcagtgaggg ggccggcggg cgtgggccga gtggccgcgg gcgccatgga gggggtgctg 120
tacaantgga ccaactatct gagcggttgg cagcctcgat ggttccttct ctgtggggga 180
atattgtcct attatgattc tcctgaagat gcctggaaag gttgcaaagg gagcatacaa 240
atggcagtct gtgaaattca agttcattct gtagataata cacgcatgga cctgataatc 300
cctggggaac agtatttcta cctgaaggcc agaagtgtgg ctgaaagaca gcggtggctg 360
gtggccctgg gatcagccaa ggcttgcctg actgacagta ggacccagaa ggagaaagag 420
tttgctgaaa acactgaaaa cttgaaaacc aaaatgtcan aactaagact ctactgtgac 480
ctccttgttc ancaagtaga ttaaaacata agaagtgacc acaactggtg tgtccaattc 540

tgaggtaaag gagtcttcca ctctggttgt ttcgtangag ggaattgatg tgggaacttt 600
gctgaaatca anctgntata ctttttctga aagaccttgg taagaattca tgcanatngc 660
aaattgcagc cttnaanctc ctgaagcctn cttctaaccg gcactccaac canggaatna 720
anctnaagct gggccaatgg ctccaaagtt ccaacnaaag gttaaaanat cccagctcaa 780
atttgggcng caaacaaagg gcaatccaac 810

<210> 229
<211> 552
<212> DNA
<213> Homo Sapiens

<400> 229
gtaaatttgt ttgagttcat tgtagattct ggatattagc ccttttgtca gatgagtaga 60
ttgcaaaaat tttctcccat tctgtaggtt gcctgttcac tctgatggta gtttcccttg 120
ctgtgcggaa gctctttagt ttaattagat cccatttgtc aatttcggct tttgttgcca 180
ttgctttcgg tgttttagac atgaagtcct tgcccatgcc tatgtcctga atggttttcc 240
taggttttct tctagggttt ttatggtttt aggtctaaca tttaagtctc gaatccatct 300
tgaattaatt tttgtataag gtgtaaggaa gggatccact ttcagctttc tacgtatggc 360
tagccagttt tcccancacc atttattaaa tagggaatcc tttccccant tcctgttttt 420
gtcangtttg tcaaagatca natggctgta natatgcanc attatttccg agggctctgt 480
tcngttccat tggtctacat ttccgttttg gttccngtac catgctgttt tttgttacng 540
gtanaccttg gt 552

<210> 230
<211> 842
<212> DNA
<213> Homo Sapiens

<400> 230
ctcatcagtt agaagaaaaa gaaaatcaaa ttaagagcat gaaggctgat attgaaagtc 60
ttgtaacaga aaaagaagcc ttacagaagg aaggaggcaa tcagcaacag gctgcttctg 120
aaaaggagtc ttgtataaca cagttgaaga aagagttatc tgaaaacatc aatgctgtca 180
cattgatgaa agaagagctt aaagaaaaaa aagttgagat tagcagtctt agtaaacaac 240
taactgattt gaatgttcag cttcaaaata gcatcagcct atccgaaaaa gaagcagcca 300
tttcatcact aagaaagcag tatgatgaag aaaaatgtga attgctggat caggtgcaag 360
atttatcttt taaagttgac actctgagta aagagaaaat ttctgctctt gagcaggtag 420
atgactggtc caataaattc tcagaatgga agaagaaagc acagtcaaga tttacacagc 480
atcaaaacac tgttaaagaa ttgcagatcc agcttgagtt aaaatcaaag gaagcttatg 540
aaaaggatga gcagataaat ttattgaagg aagagcttga tcagcaaaat aaaagatttg 600
attgtttaaa gggtgaaatg gaagacgaca agagcaagat gggagaaaaa ggagtctaat 660
ttagaaacag agttaaagtc tcaaacagca agaattatgg gattagagga ccatattanc 720
caagaaaact atttggaaat tagagtcctt aaatngaaag ttccttaaaa aattacaatc 780
aacaaaaagg atattggacc acaaagnaat tgggtcaaaa aaccttcaac aantttcaag 840
ga 842

<210> 231
<211> 781
<212> DNA
<213> Homo Sapiens

<400> 231
atatagtaaa taaactttat ttatctgttt ctcagagatg acactgccaa caatcacaga 60
tttgcataca atacagttat gtattggcta ttcacaattt acagtagtgt tttttcctct 120
gaaaaatata agtacaaaag ctaagtaaac aatgaggtac tgccatttgg gattttttac 180
atgtcttagc ttaaagaact ggtctttagc aaatattcaa cagatcaacc tgaataaaat 240

agtcaattaa atgctctaat ttatcagaaa aaatccacta agtttcacct caaaatgtat 300
tgcacaagtc tttttaaaaa atcaccctaa aaataaatag gaaaggtaag ccgttcttta 360
aaaagaatgg atgaaaggaa tattatgtaa gcccataaag caggttaagt tatcaaaata 420
tcttttaaac aacataaaac tcttcccaag agaaaactga agaaaaaact atcaccattt 480
ctccactgat aaaatctatt ttaaaggcag tctgcaactt atctgtgggc cagatttttc 540
ttgggtcttt tggctacatg aggggccctg aatgacaact tcattctcaa agagtagcaa 600
agtgtggaca agttttccaa gcagcangtc acccaatgtc actcttcctc aagatgaagg 660
atcggagcca tgacacatgt ttaactaagc acagaccgga tgggtttacc cagaagatac 720
cactggcaan ggtgaagtaa acatcaggcc gaggcaacct tccccntttc aaaaantttt 780
c 781

<210> 232
<211> 767
<212> DNA
<213> Homo Sapiens

<400> 232
gttatatagt aaataaactt tatttatctg tttctcagag atgacactgc caacaatcac 60
agatttgcat acaatacagt tatgtattgg ctattcacaa tttacagtag tgttttttcc 120
tctgaaaaat ataagtacaa aagctaagta aacaatgagg tactgccatt tgggattttt 180
tacatgtctt agcttaaaga actggtcttt agcaaatatt caacagatca acctgaataa 240
aatagtcaat taaatgctct aatttatcag aaaaaatcca ctaagtttca cctcaaaatg 300
tattgcacaa gtctttttaa aaaatcaccc taaaaataaa taggaaaggt aagccgttct 360
ttaaaaagaa tggatgaaag gaatattatg taagcccata aagcaggtta agttatcaaa 420
atatctttta aacaacataa aactcttccc aagagaaaac tgaagaaaaa actatcacca 480
tttctccact gataaaatct attttanagg cagtctgcaa cttatctgtg ggccagattt 540
ttcttggtct tttggctaca tgaggggccc tgaatgaaaa cttcattctc aaaggagtag 600
caagtgtggg acagttttcc aagcagcagt cacccaatgt cactcttctt caagatgaaa 660
gatcggagnc atgacacatg ttaacctaag nacangactg gagggtttac ncangaagat 720
acactgcgaa ggtgaaagtt aaacatcaag ccgaggaacc tcccctt 767

<210> 233
<211> 879
<212> DNA
<213> Homo Sapiens

<400> 233
gggagtttaa tacacagctg gcacaaaagg aacaagagct ggaaatgacc ataaaagaaa 60
ctatcaataa ggcccaggag gtggaggctg aacttttaga aagccatcaa gaagagacaa 120
atcagttact taaaaaaatt gctgagaaag atgatgatct aaaacgaaca gccaaaagat 180
atgaagaaat ccttgatgct cgtgaagaag aaatgactgc aaaagtaagg gacctgcaga 240
ctcaacttga ggagctgcag aagaaatacc agcaaaagct agagcaggag gagaaccctg 300
gcaatgataa tgtaacaatt atggagctac agacacagct agcacagaag acgactttaa 360
tcagtgattc gaaattgaaa gagcaagagt tcagagaaca gattcacaat ttagaagacc 420
gtttgaagaa atatgaaaag aatgtatatg caacaactgt ggggacacct tacaaaggtg 480
gcaatttgta ccatacggat gtctcactct ttggagaacc taccgaattt gagtatttgc 540
gaaaagtgct ttttgagtat atgatgggtc gtgagactaa gaccatggca aaagttataa 600
ccaccgtact gaagttccct gatgatcaga ctcagaaaat tttgggaaaa gagaagatct 660
cggctgatgt ttacttcacc tcgcagtggt atcctcngag taaaccatca gtcgtgccta 720
agtttacatg tgtcatgggt ccgattcttc atcctttgaa gaaagagtgg acattggggt 780
naccggctgc cttgggaaaa ctgtccanac nttgcnaacn ccttggggaa atggaagntt 840
ttccanttca agggccccct caangnttgc ccaaacagg 879

<210> 234
<211> 780

<212> DNA
<213> Homo Sapiens

<400> 234
aaactttatt tatctgtttc tcagagatga cactgccaac aatcacagat ttgcatacaa 60
tacagttatg tattggnnng gcacaattta cagtagtgtt ttttcctctg aaaaatataa 120
gtacaaaagc taagtaaaca atgaggtact gccatttggg attttttaca tgtcttagct 180
taaagaactg gtctttagca aatattcaac agatcaacct gaataaaata gtcaattaaa 240
tgctctaatt tatcagaaaa aatccactaa gtttcacctc aaaatgtatt gcacaagtct 300
ttttaaaaaa tcaccctaaa aataaatagg aaaggtaagc cgttctttaa aaagaatgga 360
tgaaaggaat attatgtaag cccataaagc aggttaagtt atcaaaatat cttttaaaca 420
acataaaact cttcccaaga gaaaactgaa gaaaaaacta tcaccatttc tccactgata 480
aaatctattt taaaggcagt ctgcaactta tctgtgggcc agatttttct tggtcttttg 540
gctacatgag gggccctgaa tgaaaacttc attctcaaag agtagcaagt gtggacaagt 600
tttccaagca gcagtcancc aatgtcactc ttcttcaaga tgaaagatcg gagccatgac 660
acatgttaac taagcacaga cntgatggtt tactncagaa gattaccact gcnaaggtga 720
aagttaaaca tcaagncgag catncntctc tttccaaaaa ttttccggng tccggattca 780

<210> 235
<211> 780
<212> DNA
<213> Homo Sapiens

<400> 235
attctgaggg tatattaagt cagagtcagg ataaatcact tcggagaata gcagaattaa 60
gagaggagct ccaaatggac cagcaggcaa agaaacatct gcaagaggag tttgatgcat 120
ctttagagga gaaagatcag tatatcagtg ttctccaaac tcaggtttct ctactgaaac 180
aacgattacg aaatggcccg atgaatgttg atgtactgaa accacttcct cagctggaac 240
cacaggctga agtcttcact aaagaagaga atccagaaag tgatggagag ccagtagtgg 300
aagatggaac ttctgtaaaa acactggaaa cactccagca aagagtgaag cgtcaagaga 360
acctacttaa gcgttgtaag gaaacaattc agtcacataa ggaacaatgt acactattaa 420
ctagtgaaaa agaagctctg caagaacaac tggatgaaag acttcaagaa ctagaaaaga 480
taaaggacct tcatatggcc gagaagacta aacttatcac tcagttgcgt gatgcaaaga 540
acttaattga acagcttgaa caaggataag ggaatggtaa tcgcagagac aaaacgtcag 600
atgcatgaaa ccctggaaat gaaagaagaa gaaattgctc aactccgtag tcgcatcaaa 660
cagatgacta cccaagggag aggaattacg ggaacaagan agaaaagtcc gaaagaactg 720
cntttgaggg aacttgaaaa agccttgagt acagnccaaa aanacagngg aagccaccgg 780

<210> 236
<211> 711
<212> DNA
<213> Homo Sapiens

<400> 236
cttggttttt aaatttggtt tcatattcct cattcaaaat atgaatactg tcctccttgg 60
ctgacaattt ctgtgtgagt atctcaattt ctttcttctg tccttctctc atttgtaaaa 120
tcatattttc cttttccacc aagatttgct ttgtctgttc ctgttctttg ttaccatctt 180
caagtttgga ctcatagact tgggttaaag attttacttt ttgctccatt tcactatttt 240
gtttttcaag ttgctgcatt aagtcctgca cctggatttt gtgagcatct aactcagtac 300
aaacatcttt cttttgtgct tcaacttcag caacctgttt ggtaagaaga attctttctg 360
tttccaaatc caacaacttc tgctgcaatt gggccaactg ttcctcatat gcttttgtct 420
gctcatgtgt ggcactctgg taagactgaa aaacgtccag cttagcagat gcctgctgga 480
gttccccttc agacctttta atatctgcct ccaaattttc tacatgagcc tgatgctctt 540
tcaaatgctt gtccctttcc ttcaagagaa gctcaagttg nttaanttga tcttttaaag 600
ccttctcaan tcctccggga tanaaaacnt cgtgttcttt naatgagaac ggtcaacntg 660

ccggctgggt gataantttt ccgttcancc anccttgggg ctccaaattc c 711

<210> 237
<211> 658
<212> DNA
<213> Homo Sapiens

<400> 237
atagtaaata aactttattt atctgtttct cagagatgac actgccaaca atcacagatt 60
tgcatacaat acagttatgt attggctatt cacaatttac agtagtgttt tttcctctga 120
aaaatataag tacaaaagct aagtaaacaa tgaggtactg ccatttggga ttttttacat 180
gtcttagctt aaagaactgg tctttagcaa atattcaaca gatcaacctg aataaaatag 240
tcaattaaat gctctaattt atcagaaaaa atccactaag tttcacctca aaatgtattg 300
cacaagtctt tttaaaaaat caccctaaan ataaatagga aaggtaagcc gttctttaaa 360
aagaatggat gaaaggaata ttatgtaagc ccataagagc aggttaagtt atcaaaatat 420
cttttaaaca ncataaaact cttcccanga gaaaactgaa gaaaaaacta tcaccatttc 480
tccactgata aaatctattt taaaggcagt ctgcanctta tctgtgggcc aagatttttc 540
ttggnctttt ggctacatga gggggccctg gaatgaaaaa cttcattccc aanggagttn 600
gcnaggtgtg ggacaggttt tccaaggcaa gcaagtnagc caaatngtca gctcttcc 658

<210> 238
<211> 678
<212> DNA
<213> Homo Sapiens

<400> 238
   gttatatagt aaataaactt tatttatctg tttctcagag atgacactgc caacaatcac 60
   agatttgcat acaatacagt tatgtattgg ctattcacaa tttacagtag tgttttttcc 120
   tctgaaaaat ataagtacaa aagctaagta aacaatgagg tactgccatt tgggattttt 180
   tacatgtctt agcttaaaga actggtcttt agcaaatatt caacagatca acctgaataa 240
   aatagtcaat taaatgctct aatttatcag aaaaaatcca ctaagtttca cctcaaaatg 300
   tattgcacaa gtctttttaa aaaatcaccc taaaaataaa taggaaaggt aanccgttct 360
   ttaaaaagaa tggatgaaag gaatattatg taagcccata aagcaggtta agttatcaaa 420
   atatctttta aacaacataa gaactcttcc caaggagaaa actgaannaa aaaactatca 480
   ncatttcnnc actgataaaa tctantttaa agggnagtcn gcaacttanc tgtgggccag 540
   atttttccgt ggggcttttg ggctacantn agggggccct gaatgaaaaa nttcaattcc 600
   ncaaatgnng tagcaaattg tgggncangt ttttccaaag cagncaantt cancccnana 660
   tgtcactcct tccttcaa 678

<210> 239
<211> 1402
<212> DNA
<213> Homo Sapiens

<400> 239
gggagtttaa tacacagctg gcacaaaagg aacaagagct ggaaatgacc ataaaagaaa 60
ctatcaataa ggcccaggag gtggaggctg aacttttaga aagccatcaa gaagagacaa 120
atcagttact taaaaaaatt gctgagaaag atgatgatct aaaacgaaca gccaaaagat 180
atgaagaaat ccttgatgct cgtgaagaag aaatgactgc aaaagtaagg gacctgcaga 240
ctcaacttga ggagctgcag aagaaatacc agcaaaagct agagcaggag gagaaccctg 300
gcaatgataa tgtaacaatt atggagctac agacacagct agcacagaag acgactttaa 360
tcagtgattc gaaattgaaa gagcaagagt tcagagaaca gattcacaat ttagaagacc 420
gtttgaagaa atatgaaaag aatgtatatg caacaactgt ggggacacct tacaaaggtg 480
gcaatttgta ccatacggat gtctcactct ttggagaacc taccgaattt gagtatttgc 540
gaaaagtgct ttttgagtat atgatgggtc gtgagactaa gaccatggca aaagttataa 600

ccaccgtact gaagttccct gatgatcaga ctcagaaaat tttggaaaga gaagatgctc 660
ggctgatgtt tacttcacct cgcagtggta tcttctgagt aaaccatcag tctgtgctta 720
gttaacatgt gtcatggctc cgatcttcat cttgaagaag agtgacattg ggtgactgct 780
gcttggaaaa ctgtccacac ttgctactct ttgagaatga agttttcatt cagggcccct 840
catgtagcca aaagaccaag aaaaatctgg cccacagata agttgcagac tgcctttaaa 900
atagatttta tcagtggaga aatggtgata gtttttcttt cagttttctc ttgggaagag 960
ttttatgttg tttaaaagat attttgataa cttaacctgc tttatgggct tacataatat 1020
tcctttcatc cattcttttt aaagaacggc ttacctttcc tatttatttt tagggtgatt 1080
ttttaaaaag acttgtgcaa tacattttga ggtgaaactt agtggatttt ttctgataaa 1140
ttagagcatt taattgacta ttttattcag gttgatctgt tgaatatttg ctaaagacca 1200
gttctttaag ctaagacatg taaaaaatcc caaatggcag tacctcattg tttacttagc 1260
ttttgtactt atatttttca gaggaaaaaa cactactgta aattgtgaat agccaataca 1320
taactgtatt gtatgcaaat ctgtgattgt tggcagtgtc atctctgaga aacagataaa 1380
taaagtttat ttactatata ac 1402

<210> 240
<211> 760
<212> DNA
<213> Homo Sapiens

<400> 240
gtgcagtttc tcttatattc ctcacatatg tgctttcatt catctttcgc aagtggagaa 60
aaaataatgg cttttggtct tttggctttt ttattatctt aatatgtgta tccacaatta 120
tggtatcaac tcaatatgaa aaactcaact taattttgtg catgattttc ataccttcct 180
tcactttgct ggggtatgtc atgttattga tccagctcga ctttatgaga aacttggaca 240
gtctggacaa tagaataaat gaagtcaata aaaccattct tttaacaacc ttaataccat 300
accttcagag tgttattttc ctttttgtca taaggtgtct ggaaatgaag tatggaaatg 360
aaataatgaa taaagaccca gttttcagaa tctctccacg gagtagagaa actcatccca 420
atccggaaga gcccgaagaa gaagatgaag atgttcaagc tgaaagagtc caagcagcaa 480
atgcactcac tgctccaaac ttggaggagg aaccagtcat aactgcaagc tgtttacaca 540
aggaatatta tgagacaaag aaaagttgct tttcaacaag aaagaagaaa atagccatca 600
gaaatgtttc cntttgtgtt aaaaaaggtg aaagttttgg ggattaccta ggacacaatg 660
ggagctggta aaagtacttc cattaaaatg ataacntggg tgcacaaagc caaactgcan 720
ggagtggtgg gtgttacaaa ggnagcagan gcatcnggta 760

<210> 241
<211> 745
<212> DNA
<213> Homo Sapiens

<400> 241
aaaagtccan caaagtttta tttctaagaa ataaacttgc atataacccg aacgtaacaa 60
cncnggtatt acatcaatac agctataaca ttaatgcagc aattatataa cacaaaagtg 120
ctataatgac atgggaaatg ttcatgaact gtgaggtgaa aagatacaga aaatgactat 180
gcctacngat actacctttg aaaaaggatc cataaaaaat acattgaata taagttggct 240
aaagaaaata ttaactgcgg tactttctta cagattangg ctancttctt ccatataact 300
tcaatatgta ctaaaattca catgcattta ttttataatc agaatgtcat tataattaaa 360
tgttangctg tgccatttca tcagtttatc anaccttctt atagtcaatg tcacattaaa 420
ttagaatccg agtaaataan gtttaaaaat anctgataca tttgaagttc aggctaaaaa 480
cctcatattt ttatttgtaa aatgttctca ntgttagctt tattgataat aaccgataac 540
caacctaata ttgtangatt tttaaattat ttttaagcac aaantagacc catgttgggg 600
atgaataaca tgtcngattt tgtnaatttt ggtcnacnac ttttcccaaa aatttccttg 660
tttccttcan ccnaaatttt taaaantgaa aactgtatca attatggaan ggtttattaa 720
aangtttncc tttggtaacc ngaag 745

<210> 242
<211> 818
<212> DNA
<213> Homo Sapiens

<400> 242
gcaacgccgg ggcgcccgag gtctggaagg cgcagaaatg gagcaagagc cacaaaatgg 60
agaacctgct gaaattaaga tcatcagaga agcatataag aaggcctttt tatttgttaa 120
caaaggtctg aatacagatg aattaggtca gaaggaagaa gcaaagaact actataagca 180
aggaatagga cacctgctca gagggatcag catttcatca aaagagtctg aacacacagg 240
tcctgggtgg gaatctgcta gacagatgca acagaaaatg aaagaaactc tacagaatgt 300
acgcaccagg ctggaaattc tagagaaggg tcttgccact tctctgcaga atgatcttca 360
ggaggtgccc aagttatatc cagaatttcc acctaaagac atgtgtgaaa aattaccaga 420
gcctcagtct tttagttcag ctcctcagca tgctgaagta aatggaaaca cctcaactcc 480
aagtgcaggg gcagttgctg cacctgcttc tctgtcttta ccatcacaaa gttgtccagc 540
agaagctcct cctgcttata ctcctcaagc tgctgaaggt cactacactg tatcctatgg 600
aacagattct ggggagtttt catcagttgg agaggagttt tatagggaat cattctcagc 660
caacggcctc ttnagaacct taagggctgg gattcangat gaaattgatt ttgataccaa 720
atgggagtac annttttttt tgtaaatcct gcaangggga ngttatgcan cttcgtancc 780
ccggggtacc ttcnaattgt gaagggtttt gggntaaa 818

<210> 243
<211> 799
<212> DNA
<213> Homo Sapiens

<400> 243
aatttcttga agtacttttt taatccaatt aagctgataa taatcacttc gaattttaat 60
acaatacaat catgttccca aatttccnag gctcataaca atacagtctc aatacaaaag 120
acgtaataat ctatttttat tcattttaaa tcaaagaaac cattccattt cctaacaaac 180
aggtaagtta caaaagtagt ccattttact tttcatcagt ctttccctgt tttgaacaag 240
tctttttgag aattcttagt tttagttttt gtttagctta cacactgaaa attttgagaa 300
gcatctaaaa aaatccacaa ttagtgcaaa aagaggggac aatactttaa gtcattcctt 360
ctataaaaag aattaaggtt actaaatgcc aatttttaag caaatatata gtttcctatt 420
tgccttctga aagacagcag atataaaaat agttcaatat taggtttaac aaggtttgaa 480
caacacatgt actatcagct ttattttacc tgcaaaaata ttttagctac acttggaaaa 540
aaaataaact tgagaatata acttcacatt tctaaggcca gatgcaagaa tacttaatct 600
tttcctttta aatagaagac atgccataaa atttatgaaa agttaatttg taggaatggn 660
atacatttaa aaaatacngg ttaaaccngg tgagggaatt ccacatttgg cctatttaac 720
aaaaatttta aaccaatttt caaaaggggc tttggggtaa aaagtngatt cccaagcaac 780
ntcaancant ttaaccttc 799

<210> 244
<211> 726
<212> DNA
<213> Homo Sapiens

<400> 244
gtgagttgag cgctgctgct ccgcggtgga gtcaccgcac cgctcccggg atcatggtgt 60
tctacttcac cagcagcagc gttaattcat ctgcctacac tatttacatg ggaaaagata 120
aatatgaaaa tgaagatctg atcaagcatg gctggcctga agatatctgg tttcatgtgg 180
acaaactctc ttcggctcat gtataccttc gattacataa gggagagaat atagaagaca 240
tcccaaagga agtgctgatg gactgtgccc accttgtgaa ggccaatagc attcaaggct 300
gcaagatgaa caacgttaat gtggtatata cgccgtggtc taacctgaag aaaacagctg 360
acatggatgt ggggcagata ggctttcaca ggcagaagga tgtaaaaatt gtgacagtgg 420

agaagaaagt aaatgagatc ctgaaccgat tagaaaagac caaagtcgag cggttcccag 480
acctancagc agagaaagaa tgcagagatc gtgaagagag gaatgagaaa aaagcccaaa 540
ttcaggaaat gaaaaagaga gaaanagaag aaatgaagaa gaanagggaa atggatgaac 600
ttangagcta ttcatcacta atgaaagttt gaaaatatgt cttcanatca ggatggcaat 660
ggattcagat gaattcatgt taaaaggaga aaaggngaaa aaggaccttt gaaaaatttg 720
aatgtt 726

<210> 245
<211> 592
<212> DNA
<213> Homo Sapiens

<400> 245
ccagattaaa aaaatggtat tttattataa cttttaaaat tgcggaacat cagactgaat 60
atcatcagac acatacacaa aaccactcat ctctaaagtc attttctata ccctctcaaa 120
atttggccag tgagttttgc ctcagggaat tttccagttc aaccccatac accaacatgg 180
aataaatgga aacactagcc ttttggtttt gcccanagtt ccaaagtgct attacaggtg 240
gaatatctgc tgcaggaagt cattcttgct gctgtgggtg tgagtaaaat gcttagttcc 300
ttctaaaatc ataattgcaa tatggacttc tgcttcacgc tgcatcctaa ggcacaaatc 360
aggtaaccta catctcccaa atgatcaaca ggagcactcc atcctatttt accctcaatg 420
cnganaaatt acncctgggc ccanaagttg tcacataggt ggcttgggtt acttggggct 480
caggcaacaa ctgccacagg ccccagcttg atgaanacca tcnatttctt taaaatatgt 540
tggnnactaa gatggaggcc tccggcncan agggaancan nggacataaa ac 592

<210> 246
<211> 821
<212> DNA
<213> Homo Sapiens

<400> 246
aggatgaaga gctggagagc gccgaggacg acgagcgcag ctgtcggggc cgcgagtcgg 60
acgaagacac tgaggatgct agtgaaactg acctggcaaa gcatgatgaa gaagactatg 120
tagaaatgaa ggaacagatg tatcaggaca aactggcttc tctcaagagg cagttgcaac 180
aactgcaaga aggtacatta caggaatatc agaagagaat gaaaaaacta gatcagcagt 240
acaaagagag gatacggaat gcagaactct tcctccagct ggaaactgaa caagtggaac 300
gaaattacat taaagaaaag aaggcagcag tgaaagaatt tgaagacaag aaggttgagc 360
tgaaagagaa cctgattgct gagctagaag aaaagaagaa aatgattgaa aatgaaaagc 420
tgacaatgga actgactgga gattctatgg aggtgaaacc tatcatgacc agaaagttgc 480
ggaggcgacc aaatgatccc gtccccatcc cagacaagag gaggaaacct gctccagccc 540
agctaaacta tttgttaaca ggatgaacag atcatggagg atctgagaac attaaataag 600
cttaagtcac ccaagagacc agcatctcca tcctctcctg agcacttgcc tgcaacaccc 660
gccggaatct ccaagcccca gaggttcnaa agccccggat anaagaatgg caaacctgtt 720
actatgacaa aaagatggtt accacaagag ccaaggccat cctatcctgg angtcaaagg 780
gacaaaccan gaaactgaag cctgcctnat taagtttccg t 821

<210> 247
<211> 639
<212> DNA
<213> Homo Sapiens

<400> 247
gttacacaaa gcatttattt ctctgagaag gccgagagcc acgagaattc atcatctcct 60
gctaggacct ctgccccaag cttctggcca aatagtgaat tggacgcgac agggaaagta 120
gctacgtgat ccactaatca gattcaaaac atgaaaatgc actggagagt gtatcccttc 180
ctgctcttct ccatggtaga gagacttaaa gataatcaat aaaaatagct gtcccttcaa 240

actcagagga ggttttcaaa aacaagtata agcaaaaaat aaagaaataa aaggaaagta 300
aatcaaaccc cccaatacgc ctgaaagtaa aacagtctca tggtgactga tgtctggaan 360
aagttgaggc agaaaagact gacaaagttg gaangcatcc cggccacaaa agtgcccnaa 420
aagaattcan tgcagtgctc tccatttcca aggctgagta actattccca gntaagttaa 480
catttttcna nttaaggana nancgaanac anntncatnt ctanatccca ctccagaaat 540
anggtcaatg agaangangc actgtannna aagtcaagna gctgganc nc ccgggcggnt 600
tnacccaaga gcccggcgct nnaagcctgg gcccaagct 639

<210> 248
<211> 846
<212> DNA
<213> Homo Sapiens

<400> 248
aacaggatgt caaaaattaa actgcgcttt ccatcacaat agaggacgat atgttgatgg 60
ccttttccta cctccgagca aaactgtgtt gcccactgtg cctgagtcac cagaagagga 120
agtgaaggct agccaacttt cagttcagca gaacaaattg tctgtccagt ccaatccttc 180
ccctcagctg cggagcgtta tgaaagtaga aagttccgaa aatgttccta gccccacgca 240
tccaccagtt gtaattaatg ctgcagatga tgatgaagat gatgatgatc agttttctga 300
ggaaggtgat gaaaccaaaa cacctaccct gcaaccaact cctgaagttc acaatggatt 360
acgagtgact tctgtccgga aacctgcagt caatataaag caaggtgaat gtttgaattt 420
tggaataaaa actcttgagg aaattaagtc aaagaaaatg aaggaaaaat ctaagaagca 480
aggtgagggt tcttcaggag tttccagtct tttactccac cctgagcccg ttccaggtcc 540
tgaaaaagaa aatgtcagga ctgtggtgag gacagtaact ctctccacca aacaaggaga 600
agaacccttg gttagattga gtcttactga gagactgggg aaacgaaaat tttcagcagg 660
cggtgacagt gatcctccat taaagcgtag cctggcacan aggctaaggg aagaaagttg 720
aagctccaga aactaacant gacaaaacac caangaaagc tcaagtttcc aagtccccct 780
aaaggggcga attaggcatg tcagccngga ttcaagataa tnagggatgc aacaagatta 840
aaggtt 846

<210> 249
<211> 763
<212> DNA
<213> Homo Sapiens

<400> 249
gactttccta catcagtttt atttaaaaca caaacaagta tttctctttc tgtaagggca 60
aatggttcaa ataatgcgga acacgaaaca ttgactaata caagtgcttt aaatatgaaa 120
caaaattatt ttttaaaaaa gcaaaagaat aaagaatata tacaaaaggg acctggaatc 180
tgtaagctga ttccaaaaat gaaataagta gaaaatccat ggtgaaacct gaacattcta 240
cctctgcttt ggagaagggc tatcatacaa cattcagtca gctgaagatg gattggtaga 300
ggtgtgtcta tacataaact tcagtcattt ttgcttgtgc agaatcatcc caatcttccc 360
aagactgaat gggcagtcct gtggctttct tccttttcca tattcccaac aaggctacgt 420
gaagttcaac tcttgatgag ccgcttacaa cagcagttcc ttaggagcca acatgacagg 480
tgggtcagat ttccctatga gaaacaaaac tggccaccta cagcaaaata tcaaaatggg 540
taagtccttc cttcctcttc ctcctgatta tatacaacat atctcctttc aagactatta 600
tttccatcat gccttattcc ttcacaaatc taaaccttga ngtgatatga angaaaccaa 660
catcaagaaa agaaaactca attcagaaat gaanaaaacg ggcaggtata caatacaccc 720
cagagcatct caatatcccc tgggacagnt acaattcagt gtt 763

<210> 250
<211> 899
<212> DNA
<213> Homo Sapiens

<400> 250
attcaagtca agagatgtga gaccatgaga gagaagcaca tgcagaaaca gcaggagagg 60
gaaaaatcag tcttgacacc tcttcgggga gatgtagcct cttgcaatac ccaagtggca 120
gagaaaccag tgctcactgc tgtgccagga atcacacggc acctgaccaa gcggcttccc 180
acaaagtcat cccagaaggt ggaggtagaa acctcaggga ttggagactc attattgaat 240
gtgaaatgtg cagcacagac cttggaaaaa aggggtaaag ctaaacccaa agtgaacgtg 300
aagccatctg tggttaaagt tgtgtcatcc cccaaattgg ccccaaaacg taaggcagtg 360
gagatgcacg ctgctgtcat tgccgctgtg aagccactca gctccagcag tgtcctacag 420
gaacccccag ccaaaaaggc agctgtggct gttgtcccgc ttgtctctga ggacaaatca 480
gtcactgtgc ctgaagcaga aaatcctaga gacagtcttg tgctgcctcc aacccagtcc 540
tcttcagatt cctcaccccc ggaggtgtct ggcccttcct catcccaaat gagcatgaaa 600
actcgccgac tcagctctgc ctcaacaagg aaagccccca ctctctgtgg aggatgattt 660
tgagaaacta atatgggaga tttcaaggag gcaaaattgg naactganat tgacctggat 720
tctgggaaaa gatgaagatg acccttccgg cttngngcct atcaannaaa ngattgntan 780
cctgaaaggg tggtaattga nggacncctt naaaaaaaaa atccnccaaa aaaactnggg 840
ccttaanttc naccaaatgg taacaatttn acctgagaat gnttaatttc ctttaggcc 899

<210> 251
<211> 755
<212> DNA
<213> Homo Sapiens

<400> 251
cctacatcag ttttatttaa aacactaaca agtatttctc tttctgtaag ggcaaatggt 60
tcaaataatg cggaacacga aacattgana nagacaagtg ctttaaatat gaaacaaaat 120
tattttttaa aaaagcaaaa gaataaagaa tatatacaaa agggacctgg aatctgtaag 180
gtgattccaa aaacgaaata agtagaaaat ccatggtgaa acctgaacat tctacctctg 240
ctttggagaa gggctatcat acaacattca gtcagctgaa gatggattgg tagaggtgtg 300
tctatacata aacttcagtc atttttgctt gtgcagaatc atcccaatct tcccaagact 360
gaatgggcag tcctgtggct ttcttccttt tccatattcc caacaaggct acgtgaagtt 420
caactcttga tgagccgctt acaacagcag ttccttagga gccaacatga caggtgggtc 480
agatttccct atgagaaaca aaactggcca cctacagcaa aatatcaaaa tgggtaagtc 540
cttccttcct cttcctcctg attatataca acatatctcc tttcaaagac tattatttcc 600
atcatgctta ntccttcaca aatctaaacc ttgaggtgat atgaaggaaa ccaacatcan 660
gaaaagaaaa ctcaattcag aaatgaagaa aacgggcang tatacaattc anccccagag 720
caacccaata atccctgggc aaaagttcaa ttcaa 755

<210> 252
<211> 753
<212> DNA
<213> Homo Sapiens

<400> 252
cctacatcag ttttatttaa aacactaaca agtatttctc tttctgtaag ggcaaatggt 60
tcaaataatg cggaacacga aacattgact aatacaagtg ctttaaatat gaaacaaaat 120
tattttttaa aaaagcaaaa gaataaagaa tatatacaaa agggacctgg aatctgtaag 180
gtgattccaa aaacgaaata agtagaaaat ccatggtgaa acctgaacat tctacctctg 240
ctttggagaa gggctatcat acaacattca gtcagctgaa gatggattgg tagaggtgtg 300
tctatacata aacttcagtc atttttgctt gtgcagaatc atcccaatct tcccaagact 360
gaatgggcag tcctgtggct ttcttccttt tccatattcc caacaaggct acgtgaagtt 420
caactcttga tgagccgctt acaacagcag ttccttagga gccaacatga caggtgggtc 480
agatttccct atgagaaaca aaactggcca cctacagcaa aatatcaaaa tgggtaagtc 540
cttccttcct cttcctcctg gattatatac aacatatctc ctttcaagac tattatttcc 600
atcatgcnta atccttcaca aatctaaaac cttgaggtgg atatgaaagg aaaccaacat 660
canagaaaag aaaactcaat tcaagaaaat taagaaaacc tggcaaggta tacaaataca 720

cccccaggag catcccaaat aatccctggg aaa 753

<210> 253
<211> 793
<212> DNA
<213> Homo Sapiens

<400> 253
gactttccta catcagtttt atttaaaaca ctaacaagta tttcnctttc ngtaagggca 60
aatggttcaa ataatgcgga acacgaaaca ttgactaata caagtgcttt aaatatgaaa 120
caaaattatt ttttaaaaaa gcaaaagaat aaagaatata tacaaaaggg acctggaatc 180
tgtaaggnga ttccaaaaac gaaataagta gaaaatccat ggtgaaacct gaanattcta 240
cctctgcttt gganaagggc tatcatacaa cattcagtca gctgaanatg gattggtaaa 300
ggtgtgtcta tacataaact tcagtcattt ttgcttgtgc anaatcatcc caatcttccc 360
aagactgaat gggcagtcct gtggctttct tccttttcca nattcccaac aaggctacgt 420
gaagttcaac tcttgatgag ccgcttacaa cagcagttcc ttaggagcca acatgacagg 480
tgggtcagat ttccctatga gaaacaaaac tggccaccta cagcaaaata tcaaaatggg 540
taagtccttc cttcctcttc cncctgatta tatacaanat atctcctttc aagactatta 600
tttccatcat gcttattcct tcacanatct aaaccttgan gtgatatgaa nggnaaccaa 660
catcangaaa agaaaactca attcagnaat gaangaaaac tgggaggtat ttaatanacc 720
cccangnnga atccaaatac cctggnaana gttcaattca antgtacngc naaagnccat 780
aantaantat tgg 793

<210> 254
<211> 625
<212> DNA
<213> Homo Sapiens

<400> 254
cctacatcag ttttatttaa aacactaaca agtatttctc tttctgtaag ggcaaatggt 60
tcaaataatg cggaacacga aacattgact aatacaagtg ctttaaatat gaaacaaaat 120
tattttttaa aaaagcaaaa gaataaagaa tatatacaaa agggacctgg aatctgtaag 180
gtgattccaa aaacgaaata agtagaaaat ccatggtgaa acctgaacat tctacctctg 240
ctttggagaa gggctatcat acaacattca gtcagctgaa gatggattgg tanaggtgtg 300
tctatacata aacttcagtc atttttgctt gtgcagaatc atcccaatct tcccaagact 360
gaatgggcag tcctgtggct ttcttccttt tccatattcc caacaaggct acgtgaagtt 420
caactcttga tgagccgctt acaacancaa gttccttang agccaacatg acaggtgggg 480
tcangatttc cctatgagaa acaanactgg ccacctacag caaaaatatn aaaatggggt 540
aagtccttcc ttcctcttcc tcctgaatta tatncaacat ntctcctttt caagacnatt 600
anttccatca gggcttaatc cttca 625

<210> 255
<211> 907
<212> DNA
<213> Homo Sapiens

<400> 255
gccaacagca gcggagaaac gtttctcttt cctctcagtt tgcgcacacc atggcggccc 60
ctgcccagca gactactcag cctggcggcg ggaagcgcaa aggcaaggct cagtatgtgc 120
tggccaagcg cgctcggcgc tgcgacgctg gcgggccccg tcagctagag cccgggctac 180
agggcatcct catcacctgc aatatgaacg agcgcaagtg cgtggaggag gcctacagcc 240
tcctcaacga atacggcgac gacatgtatg ggccagaaaa gtttacagac aaggatcagc 300
agccctctgg aagtgaggga gaggatgatg atgcggaggc tgccttgaag aaagaagttg 360
gtgacattaa ggcatctaca gagatgaggt taagaagatt ccagtcagtg gaaagtggag 420
caaataacgt tgtcttcatc aggacacttg gatagagcct tgagaaattg gtgcatcata 480

ttctccagga tatgtacaaa accaagaaaa agaagactcg agttattttg cgaatgttac 540
ccatctcagg cacatgcaag gcttttttag aagatatgaa aaaatatgca gaaacatttt 600
tggaaccctg gtttaaagct ccaaacaaag ggacatttca gattgtgtac aaaatctcga 660
nataacagtc atgtnaatag agaagaagtt atcaagagaa tttgcangga atagtgtgca 720
acctcaattc agnaaataaa gtgggtntca acaatccaca agtacacaat ngtaatanaa 780
atcatcaaan ctgtcngttc cctganngtt tgttaaagga ttacaaggtt ggtttannaa 840
aattcaatcn ccaagaaggt tggtnaanaa ncccctaang ggntccttca naggcnttaa 900
ctcaaag 907

<210> 256
<211> 794
<212> DNA
<213> Homo Sapiens

<400> 256
aataacgcaa aatgacttat ggagacaacc actgatgggg caccaggagt gtagatacca 60
gacctctggt tatcagatat gatgtcacaa aanagagata ttggcctttg ttctggcagg 120
ctcctagcaa tagaaaaagt tttctttgaa tttcatcatt tacaaatctt acaaatgcta 180
cagcatgaca aatattagtg aaacctgttg actcatcatc ctggatagag aagctgctac 240
ttttcagtta atgacacaaa accttttttg catcatatga catatcatca gtaaatcaac 300
ttattgagaa taaagtctct tcaactttgt actgcatctt gccccagcat tttaatgtta 360
ttagattctc accaaccatg catattttcc tttcctgaga taagttctgc tactaaataa 420
tttgcttctt aaaccttttg actaaaggtg atttctgaac aaaagcctta ctgtttttga 480
tagtccaaaa gccatttgaa aataatgaat atcctttctt gtcaagtggc tgtgatttat 540
tgttacaatt gctaagtttt gtaagttgca tgtcacagac aatgcacaat gggacaagan 600
aaccttggac ctgagtccac ataaataccc cttgagaagt tancttttcc ttaattaaga 660
caagaatttc ctttggtgtc cccttggttg cactaagtat acttgaaagt ntnctccagn 720
angactggaa gttcttcaat caaccaanct ttttcaagaa aatgtccngt agtttcaang 780
gcctaaaaat gggt 794

<210> 257
<211> 885
<212> DNA
<213> Homo Sapiens

<400> 257
gacgccaaca gcagcggaga aacgtttctc tttcctctca gtttgcgcac accatggcgg 60
cccctgccca gcagactact cagcctggcg gcgggaagcg caaaggcaag gctcagtatg 120
tgctggccaa gcgcgctcgg cgctgcgacg ctggcgggcc ccgtcagcta gagcccgggc 180
tacagggcat cctcatcacc tgcaatatga acgagcgcaa gtgcgtggag gaggcctaca 240
gcctcctcaa cgaatacggc gacgacatgt atgggccaga aaagtttaca gacaaggatc 300
agcagccctc tggaagtgag ggagaggatg atgatgcgga ggctgccttg aagaaagaag 360
ttggtgacat taaggcatct acagagatga ggttaagaag attccagtca gtggaaagtg 420
gagcaaataa cgttgtcttc atcaggacac ttgggataga gcctgagaaa ttggtgcatc 480
atattctcca ggatatgtac aaaaccaaga aaaagaagac tcgagttatt ttgcgaatgt 540
tacccatctc aggcacatgc aaggcttttt tagaagatat gaaaaaatat gcagaaacat 600
ttttggaacc ctgggtttaa agctccaaac aaagggacat ttcagattgt gtacaaatct 660
cgaaataaca gtcatgtgaa tngagaaaga agttatcaga gaaattggca aggaatagtt 720
gtgcaccctc aattcagaaa attaaaggtg ggntctcaac caatccacag ttcacagntg 780
gtagttagaa atcaatcaaa acctgtcngt ttgcccgaan ttgnttgtta aaagaattca 840
angttggttt tanaaanaat naaatcccca aagaaggtgg gtgaa 885

<210> 258
<211> 798
<212> DNA

<213> Homo Sapiens

<400> 258
   aacatttttg cataaatggg tctttgatac aggtaaccag ttttgtaaca ttattcagaa 60
   cttcactgta tcttcaagtt tttgatatca gnagcactgt ggagaaagca gtgtgctata 120
   atgtcaacat caggatttct tttttttttt ttaataacgc aaaatgactt atggagacaa 180
   ccactgatgg ggcaccagga gtgtagatac cagacctctg gttatcagat atgatgtcac 240
   aacattatat attggccttt gttctggcag gctcctagca atagaaaaag ttttctttga 300
   atttcatcat ttacaaatct tacaaatgct acagcatgac aaatattagt gaaacctgtt 360
   gactcatcat cctggataga gaagctgcta cttttcagtt aatgacacaa aacctttttt 420
   gcatcatatg acatatcatc aagtaaatca acttattgag aataaagtct cttcaacttt 480
   gtactgcatc ttgccccagc attttaatgt tattaagatt ctcaccaacc atgcatattt 540
   tcctttcctg agataagttc tgctactaaa taatttgctt cttaaacctt ttgactaaag 600
   gtgatttctg aacaaaagcc ttactgtttt tgataagtcc caaaaagcca tttgaaaaat 660
   aatgaatatc ctttcntgtc aagtggctgt gaatttaatg ttacaattgc caagttttgt 720
   aagttgcatn gtcacangac aatgcacaat ggggacaagg agaaccttgg gcctgagtcc 780
   acaataanta ccccttga 798

<210> 259
<211> 831
<212> DNA
<213> Homo Sapiens

<400> 259
gccggcggta gacgaggacg ccaacagcag cggagaaacg tttctctttc ctctcagttt 60
gcgcacacca tggcggcccc tgcccagcag actactcagc ctggcggcgg gaagcgcaaa 120
ggcaaggctc agtatgtgct ggccaagcgc gctcggcgct gcgacgctgg cgggccccgt 180
cagctagagc ccgggctaca gggcatcctc atcacctgca atatgaacga gcgcaagtgc 240
gtggaggagg cctacagcct cctcaacgaa tacggcgacg acatgtatgg gccagaaaag 300
tttacagaca aggatcagca gccctctgga agtgagggag aggatgatga tgcggaggct 360
gccttgaaga aagaagttgg tgacattaag gcatctacag agatgaggtt aagaagattc 420
cagtcagtgg aaagtggagc aaataacgtt gtcttcatca ggacacttgg gatanagcct 480
gagaaattgg tgcatcatat tctccaggat atgtacaaaa ccaagaaaaa gaagactcga 540
gttattttgc gaatgttacc catctcaggc acatgcaang cttttttaga agatatgaaa 600
aaatatgcan aaacattttt ggaancctgg tttaaagctc caaacaaagg gacatttcag 660
attgtgttca aatctcgaaa ataacagtca tgttgaatag aagaagaagt tatcagagaa 720
nttggcaagg aataatgntg caacctcaat tcagaaaata aaagtggatt tcaccaattc 780
cacagtncac aantggtagt agaaatcatc aaaagctntc tgtttgcccg a 831

<210> 260
<211> 772
<212> DNA
<213> Homo Sapiens

<400> 260
aataacgcaa aatgacttat ggagacaacc actgatgggg caccaggagt gtagatacca 60
gacctctggt tatcagatat gatgtcacaa cattatatat tggcctttgt tctggcaggc 120
tcctagcaat agaaaaagtt ttctttgaat ttcatcattt acaaatctta caaatgctac 180
agcatgacaa atattagtga aacctgttga ctcatcatcc tggatagaga agctgctact 240
tttcagttaa tgacacaaaa ccttttttgc atcatatgac atatcatcag taaatcaact 300
tattgagaat aaagtctctt caactttgta ctgcatcttg ccccagcatt ttaatgttat 360
tagattctca ccaaccatgc atattttcct ttcctgagat aagttctgct actaaataat 420
ttgcttctta aaccttttga ctaaaggtga tttctgaaca aaagccttac tgtttttgat 480
agtccaaaag ccatttgaaa ataatgaata tcctttcttg tcaagtggcn gtgatttatt 540
gttacaattg ctagttttgt nagttgcatg tcacagacaa tgcacaatgg gacangagag 600

cctgggactg agtccacata atacccntga gaagtannct ttctttatta agacagaant 660
tctttgtgtc ccttgttgca caagtntact gaagtntcnc aagaaggact ggangtcntc 720
ataancaacc ttttagaaat gtccgtattc ctaaggccca aaaangggtc cc 772

<210> 261
<211> 753
<212> DNA
<213> Homo Sapiens

<400> 261
agacgaggac gccaacagca gcggagaaac gtttctcttt cctctcagtt tgcgcacacc 60
atggcggccc ctgcccagca gactactcag cctggcggcg ggaagcgcaa aggcaaggct 120
cagtatgtgc tggccaagcg cgctcggcgc tgcgacgctg gcgggccccg tcagctagag 180
cccgggctac agggcatcct catcacctgc aatatgaacg agcgcaagtg cgtggaggag 240
gcctacagcc tcctcaacga atacggcgac gacatgtatg ggccagaaaa gtttacagac 300
aaggatcagc agccctctgg aagtgaggga gaggatgatg atgcggaggc tgccttgaag 360
aaagaagttg gtgacattaa ggcatctaca gagatgaggt taagaagatt ccagtcagtg 420
gaaagtggag caaataacgt tgtcttcatc aggacacttg ggatagagcc tgagaaattg 480
gtgcatcata ttctccagga tatgtacaaa accaagaaaa agaagactcg agttattttg 540
cgaatgttac ccatctcagg cacatgcaag gcttttttag aaagatatga anaaatatgc 600
anaaaacatt tttggaaccc tgggtttaaa gctccaaaca aagggacatt tcagaattgt 660
ggtacaaatc tcgaaatanc agtcatgtta antagagaan naagtttttc agaagaattt 720
ggcaaggaat nagtnntgca accctcaatt tca 753

<210> 262
<211> 659
<212> DNA
<213> Homo Sapiens

<400> 262
aataacgcaa aatgacttat ggagacaacc actgatgggg caccaggagt gtagatacca 60
gacctctggt tatcagatat gatgtcacaa cattatatat tggcctttgt tctggcaggc 120
tcctagcaat agaaaaagtt ttctttgaat ttcatcattt acaaatctta caaatgctac 180
agcatgacaa atattagtga aacctgttga ctcatcatcc tggatagaga agctgctact 240
tttcagttaa tgacacaaaa ccttttttgc atcatatgac atatcatcag taaatcaact 300
tattgagaat aaagtctctt caactttgta ctgcatcttg ccccagcatt ttaatgttat 360
tagattctca ccangccatg catattttcc tttcctgaga taagttctgc tactaaagaa 420
tttgcttctt aaaccttttg actaaaggtg atttctgaac aaaagcctta ctgtttttga 480
nnagtccana agccatttga aaaataatga atatcctttc cttgtcaagt ggcngtgatt 540
tantgttaca atttgcnagg ttttgtaagt tgcatggtca cagnanaatg cacantnggg 600
acanngagan cntgggncng aagtccacat tatanccctt tgagnaangt agctttccc 659

<210> 263
<211> 673
<212> DNA
<213> Homo Sapiens

<400> 263
gagattttga tcacggtaac cgatcagaat gacaacaagc ccgaattcac ccaggaggtc 60
tttaaggggt ctgtcatgga aggtgctctt ccaggaacct ctgtgatgga ggtcacagcc 120
acagacgcgg acgatgatgt gaacacctac aatgccgcca tcgcttacac catcctcagc 180
caagatcctg agctccctga caaaaatatg ttcaccatta acaggaacac aggagtcatc 240
agtgtggtca ccactgggct ggaccgagag agtttcccta cgtataccct ggtggttcaa 300
gctgctgacc ttcaaggtga ggggttaagc acaacagcaa cagctgtgat cacagtcact 360
gacaccaacg ataatcctcc gatcttcaat cccaccacgt acaagggtca ggtgcctgaa 420

aacgaggcta acgtcgtaat caccacactg aaagtgactg atgctgatgc ccccaatacc 480
ccagcgttgg gaggctgtat acaccatatt gaatgatgat ggtgggacaa tttgtcgtca 540
ccacaaatcc agtgaacaac gatggcattt tgaaaaacag caaagttgaa gtcaagtgat 600
tttgctggtt cngaatcaat tgttgcctcn gttgggagaa aggtntccaa cacatacccc 660
gggattngtt att 673

<210> 264
<211> 661
<212> DNA
<213> Homo Sapiens

<400> 264
ccatccaaga taactttatt ccattttgca ttatttgata actatttcct tcccctcccc 60
acctccaact gcatctccta ctctgaaatn cctcttgagc agccaagggt ggccagttct 120
gctcctcatt ttcctgaaga anaatctcag cctgaaagaa tatagagcta ggtgacatat 180
gggtggccaa ccgcttctcc tcaagttcca anagagtggg caattagtga aattccatca 240
gtcatgttaa aatatacttt caccaggtan acatccttct ttcaatgcta gaggacagtg 300
aaaaatgtag attaatgaga tctgtaactg tcttctctta actgtacacc cctcaggctg 360
aacgcgggag tgctgaacac atgccctcgg aagggaccct gaagacccaa gtgacctgca 420
ccataaaacc accccgaggg tcagccatgc tgccagcact caagaagcag cagggccacc 480
tgctggaaaa ctgggcacgg ctctgggtgc ctggccctgc ctgcctcctc cacgtccttg 540
gagccaggtc tacggcaggg aacatgatct tcttctccag cttctgtgga aggaacanga 600
aatttttcat gatgtcntcc agctcttcta nggccaactg ggcatgganc ttggccacgt 660
c 661

<210> 265
<211> 659
<212> DNA
<213> Homo Sapiens

<400> 265
ccatccaana taactttatt ccattttgca ttatttgata actatttcct tcccctcccc 60
acctccaact gcatctccta ctctgaaatg cctcttgagc agccaagggt ggccagttct 120
gctcctcatt ttcctgaana anaatctcag cctgaaagaa tatanagcta ggtgacatat 180
gggtggccaa ccgcttctcc tcaagttcca ananagtggg caattagtga aattccatca 240
gtcatgttaa aatatacttt caccaggtan acatccttct ttcaatgcta gaggacagtg 300
aaaaatgtag attaatgaga tctgtaactg tcttctctta actgtacacc cctcaggctg 360
aacgcgggag tgctgaacac atgccctcgg aagggaccct gaagacccaa gtgacctgca 420
ccataaaacc accccgaggg tcagccatgc tgccagcact caaaaagcag cagggccacc 480
tgctggaana actgggcacg gctctgggtg cctggccctg cctgcctcct ccacgtcctt 540
gganccaggt ctacggnagg accatgatct tcttctccan cttctgtgga aggaacanga 600
antttttcat gatgtcntcc actcttctag ggccaactgg gcatggactt ggccacgtc 659

<210> 266
<211> 620
<212> DNA
<213> Homo Sapiens

<400> 266
   ccatccaaga taactttatt ccattttgca ttatttgata actatttcct tcccctcccc 60
   acctccaact gcatctccta ttntnaaatg cctcttgagc agccaagggt ggccagttct 120
   gctcctcatt ttcctgaana anaatctcag cctgaaagaa tatagagcta ggtgacatat 180
   gggtggccaa ccgcttctcc tcaagttcca ananagtggg caattagtga aattccatca 240
   gtcatgttaa aatatacttt caccaggtan acatccttct ttcaatgcta gaggacagtg 300
   aaaaatgtag attaatgaga tctgtaactg tcttctctta actgtacacc cctcaggctg 360
   aacgcgggag tgctgaacac atgccctcgg aagggaccct gaagacccaa gtgacctgca 420
   ccataaaacc accccgaggg tcagccatgc tgccagcact caagaagcag cagggccacc 480
   tgctggaaga cctgggcacg gctctgggtg cctggccctg cctgcctcct ccacgtcctt 540
   ggagccaggt ctacngcang aacatgatct tcttctccac ttctgtggaa ggaacaggaa 600
   ntttttcatg atgtcatcca 620

<210> 267
<211> 745
<212> DNA
<213> Homo Sapiens

<400> 267
   ccccccagac aggcctgcag tcaaatgctc caatcattcc tcaaggagtc aatgagccca 60
   gcactactac aagtcagaaa tctggaagcg taaccacaga acagctccaa gaggttcttt 120
   tgtcagctta tgaccctcaa attccaacac gggctgctgc cctgcgtact ctttcccact 180
   ggatagagca gagagaagca aaagcccttg agatgcaaga gaagcttctc aagatattct 240
   tggaaaactt ggaacatgaa gacacttttg tatatctatc tgcaattcag ggggttgccc 300
   tgctgtcaga cgtctatcct gagaaaatct tgccggactt gttggctcaa tatgacagca 360
   gcaaagacaa gcacacacca gagaccaaga atgaaagtcg gggaagtcct tatgcgaatc 420
   gtcagggcat taggagacat ggtctcaaag taccgagaac ctttgatcca taccttcctg 480
   aggggagtga gagatcctga tggtgctcac agggccagca gcttgggcaa ccttggggag 540
   ctgtgccaga ggctggactt tctgctgggc tccgtggtcc atgaggtaac agcttgcctg 600
   attgctgtgg ccaaaaacat tntntgaaag ttcaagttcg cannagctgg ccaanacaat 660
   gtggggttgt gcctgcnngc tttcggggga actcaaccca agaaaaagct tantgtaagg 720
   gtggnttaan ccnccggtcc ttcaa 745

<210> 268
<211> 676
<212> DNA
<213> Homo Sapiens

<400> 268
ccatccaaga taactttatt ccattttgca ttatttgata actatttcct tcccctcccc 60
acctccaact gcatctccta ctctgaaatg cctcttgagc agccaagggt ggccagttct 120
gctcctcatt ttcctgaana anaatctcag cctgaaagaa tatagagcta ggtgacatat 180
gggtggccaa ccgcttctcc tcaagttcca ananagtggg caattagtga aattccatca 240
gtcatgttaa aatatacttt caccaggtag acatccttct ttcaatgcta gaggacagtg 300
aaaaatgtag attaatgaga tctgtaactg tcttctctta actgtacacc cctcaggctg 360
aacgcgggag tgctgaacac atgccctcgg aagggaccct gaagacccaa gtgacctgca 420
ccataaaacc accccgaggg tcagccatgc tgccagcact caagaagcag cagggccacc 480
tgctggaana cctgggcacg gctctgggtg cctggccctg cctgcctcct ccacgtcctt 540
gggagccagg tctacggcag ggaacatgat cttcttctcc agcttctgtg gaaggaacag 600
gaagtttttc atgatgtcat ccanctcttc taaggccaac tgggcatgga acttggccac 660
gtcatcgggc tccaaa 676

<210> 269
<211> 737
<212> DNA
<213> Homo Sapiens

<400> 269
aacaaagaca aagaaggcaa ggttttctac agcatcactg gccaaggagc tgacacaccc 60
cctgttggtg tctttattat tgaaagagaa acaggatggc tgaagctctt ctctcacgct 120
gtgtcatcca acgggaatgc agttgaggat ccaatggaga ttttgatcac ggtaaccgat 180
cagaatgaca acaagcccga attcacccag gaggtcttta aggggtctgt catggaaggt 240

gctcttccag gaacctctgt gatggaggtc acagccacag acgcggacga tgatgtgaac 300
acctacaatg ccgccatcgc ttacaccatc ctcagccaag atcctgagct ccctgacaaa 360
aatatgttca ccattaacag gaacacagga gtcatcagtg tggtcaccac tgggctggac 420
cgagagagtt tccctacgta taccctggtg gttcaagctg ctgaccttca aggtgagggg 480
ttaagcacaa cagcaacagc tgtgatcaca gtcactgaca ccaacgataa tcctccgatc 540
ttcaatccca ccacgtacaa gggtcangtg cctganaaag aaggctaacg tcgttatcac 600
caacactgaa aagtgactga tgcctgatgc cccccaatta ncccanccgt gggaagctgt 660
ntacaccata tngaaatgat gatgggtggg cnaatttgtn cgttcaccaa caaatnccan 720
gtggaacaac caatggg 737

<210> 270
<211> 726
<212> DNA
<213> Homo Sapiens

<400> 270
ccatccaaga taactttatt ccattttgca ttatttgata actatttcct tcccctcccc 60
acctccaact gcatctccta ctctgaaatg cctcttgagc agccaagggt ggccagttct 120
gctcctcatt ttcctgaana anaatctcag cctgaaagaa tatanagcta ggtgacatat 180
gggtggccaa ccgcttctcc tcaagttcca ananagtggg caattagtga aattccatca 240
gtcatgttaa aatatacttt caccaggtan acatccttct ttcaatgcta gaggacagtg 300
aaaaatgtag attaatgaga tctgtaactg tcttctctta actgtacacc cctcaggctg 360
aacgcgggag tgctgaacac atgccctcgg aagggaccct gaagacccaa gtgacctgca 420
ccataaaacc accccgaggg tcngccatgc tgccagcact caanaagcag cagggccacc 480
tgctggaana cctgggcacg gctctgggtg cctggccctg cctgcctcct ccacgtcctt 540
ggagccaggt ctacggcagg aacatgatct tcttctccac ttctgtggaa ggaacangaa 600
atttttcatg atgtctccan ctcttctagg gccactgggc atggancttg ggcncntcat 660
cgggctccaa anacactact gcttcancag gtgggtanaa atccttgaag angggctcac 720
acctcc 726

<210> 271
<211> 814
<212> DNA
<213> Homo Sapiens

<400> 271
ccatccaaga taactttatt ccattttgca ttatttgata actatttcct tcccctcccc 60
acctccaact gcatctccta ctctgaaatg cctcttgagc agccaagggt ggccagttct 120
gctcctcatt ttcctgaaga agaatctcag cctgaaagaa tatagagcta ggtgacatat 180
gggtggccaa ccgcttctcc tcaagttcca agagagtggg caattagtga aattccatca 240
gtcatgttaa aatatacttt caccaggtag acatccttct ttcaatgcta gaggacagtg 300
aaaaatgtag attaatgaga tctgtaactg tcttctctta actgtacacc cctcaggctg 360
aacgcgggag tgctgaacac atgccctcgg aagggaccct gaagacccaa gtgacctgca 420
ccataaaacc accccgaggg tcagccatgc tgccagcact caagaggcag cagggccacc 480
tgctggaaga cctgggcacg gctctgggtg cctggccctg cctgcctcct ccacgtcctt 540
ggagccaggt ctacggcagg accatgatct tcttctccag cttctgtggg agggaacagg 600
gaagtttttc aatgatgtca tccagctctt cctanggcca actgggcaag ggagcttggg 660
caacgtcatc ggggctccag acaaaactac gtgcttcanc aanggtggta aaanatcctt 720
gaaggacggg ggctcaacaa cccaagtanc ctttccnggg ctgaatcccc ngaagcaagc 780
aagnacaaac cacatgtttt gggaagctcc ggcg 814

<210> 272
<211> 862
<212> DNA
<213> Homo Sapiens

<400> 272
gtacactgaa cagaaaagat ctggaaggga aaatagaaga gcagcaacaa accagtcatg 60
aaagacccac tgatgtagct catagccacc ttgaacaaca gcagagccat gagacagccc 120
cccagacagg cctgcagtca aatgctccaa tcattcctca aggagtcaat gagcccagca 180
ctactacaag tcagaaatct ggaagcgtaa ccacagaaca gctccaagag gttcttttgt 240
cagcttatga ccctcaaatt ccaacacggg ctgctgccct gcgtactctt tcccactgga 300
tagagcagag agaagcaaaa gcccttgaga tgcaagagaa gcttctcaag atattcttgg 360
aaaacttgga acatgaagac acttttgtat atctatctgc aattcagggg gttgccctgc 420
tgtcagacgt ctatcctgag aaaatcttgc cggacttgtt ggctcaatat gacagcagca 480
aagacaagca cacaccagag accaagaatg aaagtcgggg aagtccttat gcgaatcgtc 540
agggcattag ggagacatgg tctcaaagta ccgagaacct ttgattcata ccttcctgan 600
gggagtgaga gattctggat ggtgctcaca agggcagcaa cttgggcaan cttgggggaa 660
ctggtgccag aggctggact ttcngctggg gctccgtggg ccaatggagg gtacaanctt 720
gccctgaatt gctgtgggcc aaaaacaaga tnggtgaaag tttaaagtta cgcaaaactg 780
ccaatacaat gttgggttgt tgccnggctg gnnttccggg ggaatcaagc ccaggaaaag 840
cctaccggan ggggccttaa ac 862

<210> 273
<211> 677
<212> DNA
<213> Homo Sapiens

<400> 273
ccatccaaga taactttatt ccattttgca ttatttgata actatttcct tcccctcccc 60
acctccaact gcatctccta ctctgaaatg cctcttgagc agccaagggt ggccagttct 120
gctcctcatt ttcctgaaga agaatctcag cctgaaagaa tatagagcta ggtgacatat 180
gggtggccaa ccgcttctcc tcaagttcca agagagtggg caattagtga aattccatca 240
gtcatgttaa aatatacttt caccaggtan acatccttct ttcaatgcta gaggacagtg 300
aaaaatgtag attaatgaga tctgtaactg tcttcnctta actgtacacc cctcaggctg 360
aacgcgggag tgctgaacac atgccctcgg aagggaccct gaagacccaa gtgacctgca 420
ccataaaacc accccgaggg tcagccatgc tgccaagcac tcaagaggca gcagggccac 480
ctgctggaan acctgggcac ggntctgggt gcctgggccc tgcctgcctc ctccangtcc 540
ttggggccaa gtctaaggga agggaccaat gatcttcttc cccaaacttc tgtggagggg 600
aaaaaaggaa ntttttcaag gnggtcatcc nangctcctc caaggggnca aaatgggggc 660
antggaacct tgggcaa 677

<210> 274
<211> 863
<212> DNA
<213> Homo Sapiens

<400> 274
gaaaacagca aagttgaagt caagtgattt tgctgttctg aagcagttgt tgcctctgtt 60
ggagaaggta tccaacacat accctgatcc ggtcatccaa gaactcgctg ttgatctccg 120
catcaccatc tctacccatg gagcctttgc cactgaggcc gtcagcatgg ctgcccaaag 180
tacactgaac agaaaagatc tggaagggaa aatagaagag cagcaacaaa ccagtcatga 240
aagacccact gatgtagctc atagccacct tgaacaacag cagagccatg agacagcccc 300
ccagacaggc ctgcagtcaa atgctccaat cattcctcaa ggagtcaatg agcccagcac 360
tactacaagt cagaaatctg gaagcgtaac cacagaacag ctccaagagg ttcttttgtc 420
agcttatgac cctcaaattc caacacgggc tgctgccctg cgtactcttt cccactggat 480
agagcagaga gaagcaaaag cccttgagat gcaagagaag cttctcaaga tattcttgga 540
aaacttggaa catgaagaca cttttgtata tctatctgca attcaggggg ttgccctgct 600
gtcagacgtc tatcctgaga aaatcttgcc ggacttgttg gctcaatatg acagcagcaa 660
agacaagcac acaccaagag accaagaatg aaagtcgggg aagtccttat gccaatcgtc 720
anggcattag ggagacatgg tctcaaagta accgagaacc tttgattcat accttcctga 780

aggggaatta gagattctga atggtgctca cagggccaac aaccttggcn aaccttgggg 840
aacctgtgcc anaaggctng gac 863

<210> 275
<211> 821
<212> DNA
<213> Homo Sapiens

<400> 275
ccatccaaga taactttatt ccattttgca ttatttgata actatttcct tcccctcccc 60
acctccaact gcatctccta ctctgaaatg cctcttgagc agccaagggt ggccagttct 120
gctcctcatt ttcctgaaga agaatctcag cctgaaagaa tatagagcta ggtgacatat 180
gggtggccaa ccgcttctcc tcaagttcca agagagtggg caattagtga aattccatca 240
gtcatgttaa aatatacttt caccaggtag acatccttct ttcaatgcta gaggacagtg 300
aaaaatgtag attaatgaga tctgtaactg tcttctctta actgtacacc cctcaggctg 360
aacgcgggag tgctgaacac atgccctcgg aagggaccct gaagacccaa gtgacctgca 420
ccataaaacc accccgaggg tcagccatgc tgccagcact caagaggcag cagggccacc 480
tgctggaaga cctgggcacg gctctgggtg cctggccctg cctgcctcct ccacgtcctt 540
ggagccaggt ctacggcagg accatgatct tcttctccaa gcttctgtgg agggaacagg 600
aagtttttca tgatgtcatc caagctcttc tanggccaac tgggcatgga gcttgggcac 660
gtcatcgggc tccagacaca ctacgtgctt cancaaggtg gtaaaagatt cttganggac 720
ggngctcanc acctcagtaa nctttctggc tgagtccccc gaaagcaaca gcacaancca 780
catgtntngg aaaccctgcg ttacttngaa cttcaacaac c 821

<210> 276
<211> 722
<212> DNA
<213> Homo Sapiens

<400> 276
aacagctgtg atcacagtca ctgacaccaa cgataatcct ccgatcttca atcccaccac 60
gtacaagggt caggtgcctg agaacgaggc taacgtcgta atcaccacac tgaaagtgac 120
tgatgctgat gcccccaata ccccagcgtg ggaggctgta tacaccatat tgaatgatga 180
tggtggacaa tttgtcgtca ccacaaatcc agtgaacaac gatggcattt tgaaaacagc 240
aaagttgaag tcaagtgatt ttgctgttct gaagcagttg ttgcctctgt tggagaaggt 300
atccaacaca taccctgatc cggtcatcca agaactcgct gttgatctcc gcatcaccat 360
ctctacccat ggagcctttg ccactgaggc cgtcagcatg gctgcccaaa gtacactgaa 420
cagaaaagat ctggaaggga aaatagaaga gcagcaacaa accagtcatg aaagacccac 480
tgatgtagct catagccacc ttgaacaaca gcagagccat gaagacagcc ccccagacag 540
gcctgcagtc aaatgctcca atcattcctc aaggagtcaa tgagcccagc actactacaa 600
gtcagaaatc tggaagcgtt accacagaac agctccaaga ggttcntttg tcagctttat 660
gaacctcaaa ttccaacacg gggctggtgc ctgcgttact cnttcccact gggntagaag 720
ca 722

<210> 277
<211> 805
<212> DNA
<213> Homo Sapiens

<400> 277
ccatccaaga taactttatt ccattttgca ttatttgata actatttcct tcccctcccc 60
acctccaact gcatctccta ctctgaaatg cctcttgagc agccaagggt ggccagttct 120
gctcctcatt ttcctgaaga agaatctcag cctgaaagaa tatagagcta ggtgacatat 180
gggtggccaa ccgcttctcc tcaagttcca agagagtggg caattagtga aattccatca 240
gtcatgttaa aatatacttt caccaggtag acatccttct ttcaatgcta gaggacagtg 300

aaaaatgtag attaatgaga tctgtaactg tcttctctta actgtacacc cctcaggctg 360
aacgcgggag tgctgaacac atgccctcgg aagggaccct gaagacccaa gtgacctgca 420
ccataaaacc accccgaggg tcagccatgc tgccagcact caagaggcag cagggccacc 480
tgctgggaag acctgggcac ggctctgggt gcctgggccc tgcctgcctc ctccacgtcc 540
ttggagccaa ggtctacggc aggaccatga tcttcttctc cagcttctgt ggagggaaca 600
ngaagttttt caagatgtca tccaactcct ccaagggcca actggggcat gggagccttg 660
gcacgtcatn cgggctccag acacactacg gtgcttcaac aagggnggta nagattcttg 720
anggacgggg ctcaaacaat gaacctcant tacctttcng gctgagtccc cnaaagcaac 780
aagtacaaac cacatgtttt gggaa 805

<210> 278
<211> 1358
<212> DNA
<213> Homo Sapiens

<400> 278
agaactcaga gctgctcttc ctctgtggcc agttggggac cagcatcatg aagtggatgg 60
tggtggtctt ggtctgcctc cagctcttgg aggcagcagt ggtcaaagtg cccctgaaga 120
aatttaagtc tatccgtgag accatgaagg agaagggctt gctgggggag ttcctgagga 180
cccacaagta tgatcctgct tggaagtacc gctttggtga cctcagcgtg acctacgagc 240
ccatggccta catggatgct gcctactttg gtgagatcag catcgggact ccaccccaga 300
acttcctggt cctttttgac accggctcct ccaacttgtg ggtgccctct gtctactgcc 360
agagccaggc ctgcaccagt cactcccgct tcaaccccag cgagtcgtcc acctactcca 420
ccaatgggca aaccttctcc ctgcagtatg gcagtggcag cctcaccggc ttctttggct 480
atgacaccct gactgtccag agcatccaan gtccccaacc aggagttcgg cttgagtgag 540
aatnagcctg ggtaccaact tcgtctaagc gcagtttgat ggcatcatgg gcctggcctt 600
accctgctct gtccgtggat gaggccacca cagtatgcag ggcatgtgca ggagggcgcc 660
ctnaaccagc cccgtnttca gggtttacnt cagcaaccag cagggctccc agcgggggag 720
cggttgtcct ttgggggtgt ggatagcagc ntgtacacgg ggcagatcta ctgggcgcnt 780
gtcacccagg aactctactg gcagattggc attgaagagt tcctcatcgg cggccaggcc 840
tccggctggt gttctgaggg ttgccaggcc atcgtggaca caggcacctc tctgctcact 900
gtgccccagc agtacatgag tgctcttctg caggccacag ggcccaggaa ggatgagtat 960
ggacagtttc tcgtgaactg taacagcatt cagaatctgc ccagcttgac cttcatcatc 1020
aatggtgtgg agttccctct gccaccttcc tcctatatcc tcagtaacaa cggctactgc 1080
accgtgggag tcgagcccac ctacctgtcc tcccagaacg gccagcccct gtggatcctc 1140
ggggatgtct tcctcaggtc ctactattcc gtctacgact tgggcaacaa cagagtaggc 1200
tttgccactg ccgcctagac ttgctgcctc gacacgtggg ctcccctctt cctcttgacc 1260
ctgcaccctc ctagggcatt gtatctgtct ttccactctg gattcagcct tctttttctg 1320
gactctggac tttctctaat aataaatagt tcttcttt 1358

<210> 279
<211> 702
<212> DNA
<213> Homo Sapiens

<400> 279
gaagcaatga atacgcaatt agaactttca gaacaactta aatttcagaa caactctgaa 60
gataatgtta aaaaactaca agaagagatt gagaaaatta ggccaggctt tgaggagcaa 120
attttatatc tgcaaaagca attagacgct accactgatg aaaagaagga aacagttact 180
caactccaaa atatcattga ggctaattct cagcattacc aaaaaaatat taatagtttg 240
caggaagagc ttttacagtt gaaagctata caccaagaag aggtgaaaga gttgatgtgc 300
cagattgaag catcagctaa ggaacatgaa gcagagataa ataagttgaa cgagctaaaa 360
gagaacttag taaaacaatg tgaggcaagt gaaaagaaca tccagaagaa atatgaatgt 420
gagttagaaa atttaaggaa agccacctca aatgcaaacc aagacaatca gatatgttct 480
attctcttgc aagaaaatac atttgtagaa caaagtagta aatgaaaaag tcaaacactt 540

agaagatacc ttaaaaagaa cttgaatctc aacacagtat cttaaaaaga tgagggtaac 600
ttatatgaat aatccttaag tttaaaactt gaaaatggga tgcctcaacc attttaaagg 660
gtngaggttt tttccangna accgggggaa gaccttaaaa gg 702

<210> 280
<211> 874
<212> DNA
<213> Homo Sapiens

<400> 280
aactcaaaac agtgttaagt tcctatgctg ttagtactgt atcttgtcca cacctcaaac 60
aacagtgaga tctctgagca catggtctgt acctcaacca cttttctatc accagggtct 120
agaatagttg ggcatttaaa taaaatttgc taaatgaatg aaaaatccaa aataaatcat 180
gaagccattt ataaatcaca ccaatcttgc ttgggttaaa caatagaaag taacactttt 240
gaaagagaag gcaaacaggt gttagagggg caagaatgtg agctcgagga aaagacagct 300
acgaactgtg tttttaacaa ctcattattt ggctactata tttcccaatc tattctaaca 360
ctaagaagaa tctgtctaat taattgtgac aacatctgca aaaccatagt tacctatttt 420
ttcttccaac tcttttactg aagacagagg atcatttttt acagaaggtg attttgctaa 480
ggaatcctan attttacagg ggggaaaaaa aaacacnaaa caaaacaaaa accagaatca 540
gaattcattt tccataatga actggccatc ntgttaagca taanaaaatc actatcaaag 600
anaattccta cagaaaccaa tttggtcaca gaatttccct tgttanacca gaaaattaat 660
actgaactta ctatgcatat ggcatttact attaaaaaaa aaaaagtant aaccaaggcc 720
aaganaaaca acctgaaaca ttaaatacat ntttataagg aaaaantaaa tgaattttaa 780
tcttaatttt aaanaaaaac cnaaaatttt nncatacccc cccgctctta cttaaaaant 840
gncttaccaa aatactaanc ctttccccaa aacc 874

<210> 281
<211> 730
<212> DNA
<213> Homo Sapiens

<400> 281
acaaaacagc agctggaaag agaaatgtag gtggcagacg agccaggcac gaggtttcag 60
attggaaggg accaagatga ggaccaaggt gtggctgcct gactaggaac gctgtgggct 120
ggcccaggct ctcgccacac atcctgggan aactgccata ggccctagaa ggagggatga 180
aaggcgtatg ggagggaana cagcggtccc cggatcagca gcagcaccac catcctctga 240
tggcccctgg gcagtccgcc agctcggaag cactcagggc tggagcctgg gctctaagca 300
tgggccccag gagccanaca ggagggaggc agcaggaang gctggcatgg aagggctgag 360
ttctattggg gtcccacgcg ggcaagggaa ccaggactca tccctgcttg tcagccaatc 420
agcttcttca ggaagcctcc aactgatcct catccttgat gcccacaaac ttgtccacca 480
cgtccccatt cttcatggcc agcacagtgg gcaccgctga cacctcatac tcaatggcga 540
agtctgtgtg gtcntcaata tccaccttgg ccatcaccac cttcccgtgc tgcttggcca 600
ccatcttctc taacctccgn cccangatct tcagggtcca caccactgtg cgtggaaatc 660
cacaaccact ggtgtctcct gtttgaacac tccgtcttga aantcngtcc ntcctgnata 720
ttaaaggttg 730

<210> 282
<211> 699
<212> DNA
<213> Homo Sapiens

<400> 282
agaactcaga gctgctcttc ctctgtggcc agttggggac cagcatcatg aagtggatgg 60
tggtggtctt ggtctgcctc cagctcttgg aggcagcagt ggtcaaagtg cccctgaaga 120
aatttaagtc tatccgtgag accatgaagg agaaggcctt gctgggggag ttcctgagga 180

cccacaagta tgatcctgct tggaagtacc gctttggtga cctcagcgtg acctacgagc 240
ccatggccta catggatgct gcctactttg gtgagatcag catcgggact ccaccccaga 300
acttcctggt cctttttgac accggctcct ccaacttgtg ggtgccctct gtctactgcc 360
agagccaggc ctgcaccagt cactcccgct tcaaccccag cgagtcgtcc acctactcca 420
ccaatgggca aaccttctcc ctgcagtatg gcagtggcag cctcaccggc ttctttggct 480
atgacaccct gactgtccag agcatccaan gtccccaacc aggagttcgg cttgagtgag 540
aatnagcctg ggtaccaact tcgtctaagc gcanttttga tgggatcaag ggcctgggcc 600
taacctggct ctgtcccgtt ggattaaggc caccacaagc tatntagggc nattnggntc 660
aaggatgggt gtcnctttat nnagcccccg tnctttcaa 699

<210> 283
<211> 759
<212> DNA
<213> Homo Sapiens

<400> 283
gaaattgaga actgatttaa tactaaagtt ctgaataaag gtgtgcactt tatgattgat 60
tctatctttt tgcacaagtt ggatactcca gtttcccatc ccaacatgtt gttcgcaatg 120
tgtgagaacg tgatgaaaga cgatatcccc gtttacacac aaattcaact gattcacctg 180
ttctcgaata aagcttctgt ttggctgtcc accttaatgc tatgttataa ttttccataa 240
tttctcggga tattacacac ggatgtaagc attttggtgg ttctgaccat tgtccatttc 300
tacatgttat tcgcttgtta ccctcaagtt gatacaagtt ctggcattgg tactcaactg 360
atgaagctgg agcatatact gacaacggga atgaagtaat gtccccattg tcaataggtg 420
gagggggccc acattttcct gtagaatctt tgcattgagg tggttccgtc cagtttccat 480
ttaaacacat cacttcttca tccccaaaca tttcataagg gctcctacat tgataacgta 540
ctctctcacc agatggatat ttactcatct gtctcgacac tatataagca ttttgtactg 600
tgggcggatt ccacangang tgtctctgca tgttgggctt cctgtccact gctattaatg 660
catgttacat tactggctcc accattttgt aatatgttgc acaagtttta gtccttgctc 720
acccccttat acacatcctt ctctctccat gggtttggc 759

<210> 284
<211> 764
<212> DNA
<213> Homo Sapiens

<400> 284
   ggaccgcgat gacgcagact ggagggaggt gatgatgccc tattcgacag aactgatatt 60
   ttatattgaa atggatcctc cagctcttcc accaaagcca cctaagccaa tgacttcagc 120
   agttccaaca tggaatgaag gacagttctg tttctcttca ggatgcagaa tggtactggg 180
   gggatatttc aagggaggag gtaaatgaca aattgcggga tatgccagat gggaccttct 240
   tggtccgaga tgcctcaaca aaaatgcagg gagattatac tttgactttg cggaagggag 300
   gcaataataa gttaataaag atctatcacc gggatggtaa atatggcttt tctgatcctc 360
   tgacatttaa ttccgtggtg gagctcatta accactatca ccatgaatct cttgctcagt 420
   acaatcccaa acttgatgtg aagctgatgt acccaagtgt ccagatacca acaggatcag 480
   ttggtaaaag aagataatat tgatgcagta ngtaaaaaac tgcaagaata ccactctcaa 540
   gtatcaggag aagagtaaag gagtatgata ngctgtatga agaatatact agaacatccc 600
   aaggaaatac agatgaagag gactgcaata gaaagctttt aatgaaaaca ttaaaatatt 660
   tggaagagca ntgtcacaca caaggaacca acattnccaa agaatatatt gagnngattt 720
   cncaaaanaa ggggaaatga aaagggggan ttgaacgaaa ttta 764

<210> 285
<211> 586
<212> DNA
<213> Homo Sapiens

<400> 285
gcattgcacc ttttctttac ccatacaaac aagttacaaa ggtttcaaac aacagntcat 60
tctttaggct aaggaaacac catacaagca ccaacttcat tttangattc aaagctcacc 120
atccccacaa aaagaatgct attccncatc tcagagaaac aggcaggaag gacanaaggg 180
gttagttaca gtgatcaatt ttagcgtttg ctaaaacnca caaattcnag nctttttaag 240
ttcaagtttt ggtacagaag tatacattca actatgagtg ccacgttttc ccatcaaaca 300
ttggnctggc aacaaactgt tttgttggct tctgaacata atacttcttc anagggaggg 360
gctggtgaaa tgctgaancc taaattatgt tggnaagaaa caaagtacct tcanttgaag 420
gtttttttta acanctnggc ttaaattatt taaatgaaan cccaagcctc ccnatttncc 480
tttggtngcc ttttncanaa aatcccattc natcacaaaa ccctaaaaag ccttcttcgt 540
nggggggaaa aaananactg ccaaangcaa aaacaaaaac ncccaa 586

<210> 286
<211> 666
<212> DNA
<213> Homo Sapiens

<400> 286
gcctggagtt cagtgggtgc agcctgcttg cgagctgagg ccagacaggg gggcgcctac 60
ggacggaaaa gaaaagttga ttacaaacgg gaccatattt tgcttcgaaa tggaaccagc 120
agttagcgag ccaatgagag accaagtcgc acggactcat ttgacagagg acactcccaa 180
agtgaatgct gacatagaaa aggttaacca gaatcaggcc aagagatgca cagtgatcgg 240
gggctctgga ttcctggggc agcacatggt ggagcagttg ctggcaagag gatatgctgt 300
caatgtattt gatatccagc aagggtttga taatccccag gtgcggttct ttctgggtga 360
cctctgcagc cgacaggatc tgtacccagc tctgaaaggt gtaaacacag ttttccactg 420
tgcgtcaccc ccaccatcca gtaacaacaa ggagctcttt tatagaagtg aattacattg 480
gcaccaagaa tgtcattgaa acttgcaaag aggctggggt tcagaaactc attttaacca 540
gcagtgccat gtcatctttg agggcgtcga tatcaagaat ggaactgaaa gaccttccct 600
nagccattga aaccaattga cctactacac aaganactaa agatcttaca ngagaaggca 660
atttct 666

<210> 287
<211> 782
<212> DNA
<213> Homo Sapiens

<400> 287
gacagagaac aaatcggtat aatatgaagc tgcctgcttc aagaaatcca aatccagttc 60
catgaaggaa gaaatgtctg tttttgccgc cctcatcgtc acggaaagag tagggtgcgc 120
tctctgccta gcagaaggag tcacaggctc agagcaaact cattcaaagg atgttatttc 180
atcaatccac aggggaagga gtgactggct gagcaacgtg tcgagagagc ccagcctcca 240
gtgtccctca cttgaccctc cgcaggtggc gaaagctctg cacggtcctc tccatagcat 300
catccatggt cactagtggc tggtagccca tggccttttt ggctctctcg cagctgtagt 360
agtggaatgt gccagccagt gcgacccgca tgggtgtgaa ggtgggctgc agctggatga 420
caggactgat caccatcacc agcagggata gcaggagggc caggtagtag gccacccagt 480
aggggatgtg gtacttgggg gcctcataat tgaggcctgt caaggatgcg agacaggaat 540
gtccaaaaag ggatgggctc atcattggtg atgtgaaatg ccttcccacc cagtgtcgag 600
tctcngggan anctgctctg ccgccaagat tgtccatggg accaaggttc tcacaaaggt 660
gaaagtccac caagttcctc ccaatttcca atcacgaaac ttcaaccttg ccgttcctgg 720
ctgcctccat gaaggatggg ttacaaactg ccgggttccc tttggggccg aaaaattgcc 780
aa 782

<210> 288
<211> 707
<212> DNA

<213> Homo Sapiens

<400> 288
gtggttccag cgccggtttt gaccgccaca ttaccatttt ttcacccgag ggtcggctct 60
accaagtaga atatgctttt aaggctatta accagggtgg ccttacatca gtagctgtca 120
gagggaaaga ctgtgcagta attgtcacac agaagaaagt acctgacaaa ttattggatt 180
ccagcacagt gactcactta ttcaagataa ctgaaaacat tggttgtgtg atgaccggaa 240
tgacagctga cagcagatcc caggtacaga gggcacgcta tgaggcagct aactggaaat 300
acaagtatgg ctatgagatt cctgtggaca tgctgtgtaa aagaattgcc gatatttctc 360
aggtctacac acagaatgct gaaatgaggc ctcttggttg ttgtatgatt ttaattggta 420
tagatgaaga gcaaggccct caggtatata agtgtgatcc tgcaggttac tactgtgggt 480
ttaaagccac tgcagcggga gttaaacaaa ctgagtcaac cagcttcctt gaaaaaaaag 540
tgaagaagaa atttgattgg acatttgaac agacagtgga aactgcaatt acatgcctgt 600
ctactgttcc atcaattgan ttcaaacctt cagaaataga aattgggagt aatgacagtt 660
gaaaatccta aattcangan tcctacagaa gcagagattg atgctca 707

<210> 289
<211> 673
<212> DNA
<213> Homo Sapiens

<400> 289
atggcaccat cacaacaaag gaacttggaa ctgtcatgag gtcactgggt cagaacccaa 60
cagaagctga attgcaggat atgatcaatg aagtggatgc tgatggtaat ggcaccattg 120
acttccccnn atttttgact atgatggcta gaaaaatgaa agatacagat agtgaagaag 180
aaatccgtga ggcattccga gtctttgaca aggatggcaa tggttatatc agtgcagcag 240
aactacgtca cgtcatgaca aacttaggag aaaaactaac agatgaagaa gtagatgaaa 300
tgatcagaga agcagatatt gatggagacg gacaagtcaa ctatgaagaa ttcgtacaga 360
tgatgactgc aaaatgaaga cctactttca actccttttt cccccctcta gaagaatcaa 420
attgaatctt ttacttacct cttgcaaaaa aaaaaaaaat aagncanaaa annnataaaa 480
aaaaaaacnc gagagtactt ctaaagcggc cgcgggccna tcgattttcc acccgggtgg 540
ggtaccaggt aagtgtccca attcgcccta taggggagtc gtattacaat tcacggggcc 600
gtcgttttta aaacgtcntg acgggggaaa accctggngt taccaactta atcccccttg 660
caacaaatnc ccc 673

<210> 290
<211> 573
<212> DNA
<213> Homo Sapiens

<400> 290
gcaagaggta agtaaaagat tcaatttgat tcttctanag gggggaaaaa ggagttgaaa 60
gtaggtcttc attttgcagt catcatctgt acgaattctt canagttgac ttgtccgtct 120
ccatcaatat ctgcttcnct gatcatttca tctacttctt catctgttag tttttcnccn 180
aagtttgtca tgacgtgacg tagttctgct gcactgatat aaccattgcc atccttgtca 240
aagactcgga atgcctcacg gatttcttct tcactatctg tatctttcan ttttcnagcc 300
atcatagtca aaaattcggg gaantcaatg gngccattac catcagcatc cacttcattg 360
atcatatcct gnaattcaan cttctgttgg gttntgaccc antgaccnca nggacaagtt 420
ccaagttccc tttggttgtg aagggtgcca nctcgtgccc gaattccttt gggntccnac 480
gangggtcna accctgcana ggngccgcga ancctccaan cttttggttc ccctttanat 540
ngagggttaa atttcgaact ttggnttttt tcc 573

<210> 291
<211> 819
<212> DNA

<213> Homo Sapiens

<400> 291
aaagaagaac tatttattat tagagaaagt ccagagtcca gaaaaagaag gctgaatcca 60
gagtggaaag acagatacaa tgccctagga gggtgcaggg tcaagaggaa gaggggagcc 120
cacgtgtcga ggcagcaagt ctaggcggca gtggcaaagc ctactctgtt gttgcccaag 180
tcgtagacgg aatagtagga cctgaggaag acatccccga ggatccacag gggctggccg 240
ttctgggagg acaggtaggt gggctcgact cccacggtgc agtagccgtt gttactgagg 300
atataggagg aaggtggcag agggaactcc acaccattga tgatgaaggt caagctgggc 360
agattctgaa tgctgttaca gttcacgaga aactgtccat actcatcctc ctgggcccct 420
gtggcctgca gaagagcact catgtactgc tggggcacag tgagcagaga ggtgcctgtg 480
tccacgatgg cctggcaacc ctcagaacac cagccggagg cctggccgcc gatgaggaac 540
tcttcaatgc caatctgcca gtagagttcc tgggtgacan gcgcccagta gatctgcccc 600
gtgtacangc tgctatccac acccccaaag gacaaccgct cccccgctgg gagccctgct 660
ggttgctgan gtaaaccctg aanacggggc tggttnaggg cgccctcctg cacatgccct 720
gcatactgtg gtggcctcat ccacggncna aaccanggta aggcaaggcc catgatgcca 780
tcaaactgcc ataacaaatt tgtacaaggc tcaatccca 819

<210> 292
<211> 664
<212> DNA
<213> Homo Sapiens

<400> 292
ctcgcgctcg cgctggtggc ggtcgcctgg gtccgcgccg aggaagagct aaggagcaaa 60
tccaagatct gtgccaatgt gttttgtgga gccggccggg aatgtgcagt cacagagaaa 120
ggggaaccca cctgtctctg cattgagcaa tgcaaacctc acaagaggcc tgtgtgtggc 180
agtaatggca agacctacct caaccactgt gaactgcatc gagatgcctg cctcactgga 240
tccaaaatcc aggttgatta cgatggacac tgcaaagaga agaaatccgt aagtccatct 300
gccagcccag ttgtttgcta tcagtccaac cgtgatgagc tccgacgtcg catcatccaa 360
tggctggaan ctgagatcat tccagatggc tggttctcta aaggcagcaa ctacagtgaa 420
atcctagaca agtattttaa agaactttga taatggtgat tctcgcctgg actccaagtg 480
aattcctgaa gtttgtggga acangaatga aactgccatc aatattacaa cgtttccagn 540
accaagggag aacaacaagt ttgcctaang ggactccggt ngttgatgcc tctcaatttg 600
aactggtctg gatgaaaaat gcctgattgg gnaattnaag cttcccaant agtttcncca 660
aatg 664

<210> 293
<211> 719
<212> DNA
<213> Homo Sapiens

<400> 293
cactttaatt tctttattca tcaatagtat ccgaaaagga agaatcagga gttacaaaaa 60
caagttaaat gcaatatana agcctactaa atacaaatac aagttcacaa acacatatgc 120
aacagaaact tgtttanatt gtttcttgaa gtttgactac ttaaaaacat aggtgtaaag 180
gaaagacatt cagactggtc cacgtgggct tgttagcagg canaggaacc ctgctttcca 240
aaaactgata tagtccaaag tcacggcatg tgggaatgtt tccatggaca ctggatctta 300
acagatgcta tagtgtttac aaaactacac acacagagaa agcccaagga agcctgcagg 360
ctaagcccta tgcttttaga gggctgaagg aaccaaacct agtttaatcc tgtttgtttg 420
ctccatgcaa aactttatgg aagactcccc agactaggct atttagcagc ttccatgaat 480
ggtcctcaga tcatgtgatt ctacggcata nacgacagct gccctattta cacagaagct 540
gcagaactca agaagaatgt ggatttgctc ttggganttc aatgttgcag ggtanantaa 600
tcttgggatg ataaccatgt tctaaatgac tagtgaanaa acctgtggtt tcttgctttt 660
aacaaattgg tgtactcttg cccctcccat aatgtccaag ggctgggtaa aacctttga 719

<210> 294
<211> 762
<212> DNA
<213> Homo Sapiens

<400> 294
agctaaggag caaatccaag atctgtgcca atgtgttttg tggagccggc cgggaatgtg 60
cagtcacaga gaaaggggaa cccacctgtc tctgcattga gcaatgcaaa cctcacaaga 120
ggcctgtgtg tggcagtaat ggcaagacct acctcaacca ctgtgaactg catcgagatg 180
cctgcctcac tggatccaaa atccaggttg attacgatgg acactgcaaa gagaagaaat 240
ccgtaagtcc atctgccagc ccagttgttt gctatcagtc caaccgtgat gagctccgac 300
gtcgcatcat ccagtggctg gaagctgaga tcattccaga tggctggttc tctaaaggca 360
gcaactacag tgaaatccta gacaagtatt ttaagaactt tgataatggt gattctcgcc 420
tggactccag tgaattcctg aagtttgtgg aacagaatga aactgccatc aatattacaa 480
cgtatccaga ccaggagaac aacaaagttg cttaggggac tctgtgttga tgccctcatt 540
gaactgtctg gatgaaaatg ctgattggna actcagcttc caagagtttc tcaaagtgcc 600
ctcaaaccca tctttcaacc ctcctgagaa agaagtgtgc cctgngaggg attaaacgta 660
atgcagatgg agnctgagac cnaaggtgga ccngttnacc gcctgtgtcc ggtgcccggt 720
ggaaattggg tcnggtncag ccatgaacct gttacgggaa ag 762

<210> 295
<211> 708
<212> DNA
<213> Homo Sapiens

<400> 295
cactttaatt tctttattca tcaatagtat ccgaaaagga agaatcagga gttacaaaaa 60
caagttaaat gcaatataga agcctactaa atacaaatac aagttcacaa acacatatgc 120
aacagaaact tgtttanatt gtttcttgaa gtttgactac ttaaaaacat aggtgtaaag 180
gaaagacatt cagactggtc cacgtgggct tgttagcagg cagaggaacc ctgctttcca 240
aaaactgata tagtccagag tcacggcatg tgggaatgtt tccatggaca ctggatctta 300
acagatgcta tagtgtttac aaaactacac acacagagaa agcccaagga agcctgcagg 360
ctaagcccta tgcttttaga gggctgaagg aaccaaacct agtttaatcc tgtttgtttg 420
ctccatgcaa aactttatgg aagactcccc agactaggct atttagcagc ttccatgaat 480
ggtcctcaga tcatgtgatt ctacggcata gacgacagct gccctattta cacagaagct 540
gcagaactca agaggaatgt ggatttgctc ttgggagttc aatgttgcag ggtaaaagta 600
gtcctggatg ataaccatgt tccaaatgac taagtgaaga gacactgtgg gttcctgcct 660
tttaacaaaa tgggggtact cctgcccctc ctccccanaa atgtccaa 708

<210> 296
<211> 652
<212> DNA
<213> Homo Sapiens

<400> 296
cactttaatt tctttattca tcaatagtat ccgaaaagga agaatcagga gttacaaaaa 60
caagttaaat gcaatataga agcctactaa atacaaatac aagttcacaa acacatatgc 120
aacagaaact tgtttanatt gtttcttgaa gtttgactac ttaaaaacat aggtgtaaag 180
gaaagacatt cagactggtc cacgtgggct tgttagcagg cagaggaacc ctgctttcca 240
aaaactgata tagtccagag tcacggcatg tgggaatgtt tccatggaca ctggatctta 300
acagatgcta tagtgtttac aaaactacac acacagagaa agcccaagga agcctgcagg 360
ctaagcccta tgcttttaga gggctgaagg aaccaaacct agtttaatcc tgtttgtttg 420
ctccatgcaa aactttatgg aagactcccc aagactaggc tatttagcag cttccatgaa 480
tggtcctcag atcaagtgat tctacggnat anacgacaag ctgccctatt tacacagaag 540
ctgcangaac tcaagaggga atgtgggatt gcccctgggg agttcaatgg ttgcangggt 600

aaaagttant cttgggntga ataaccaggt ttctaaaatg accaaattga aa 652

<210> 297
<211> 879
<212> DNA
<213> Homo Sapiens

<400> 297
cactttaatt tctttattca tcaatagtat ccgaaaagga agaatcagga gttacaaaaa 60
caagttaaat gcaatataga agcctactaa atacaaatac aagttcacaa acacatatgc 120
aacagaaact tgtttagatt gtttcttgaa gtttgactac ttaaaaacat aggtgtaaag 180
gaaagacatt cagactggtc cacgtgggct tgttagcagg cagaggaacc ctgctttcca 240
aaaactgata tagtccagag tcacggcatg tgggaatgtt tccatggaca ctggatctta 300
acagatgcta tagtgtttac aaanctacac acacagagaa agcccaagga agcctgcagg 360
ctaagcccta tgcttttaga gggctgaagg aaccaaacct agtttaatcc tgtttgtttg 420
ctccatgcaa aactttatgg aagactcccc agactaggct atttagcagc ttccatgaat 480
ggtcctcaga tcatgtgatt ctacggcata gacgacagct gccctattta cacagaagct 540
gcagaactca agaggaatgt ggatttgctc ttgggagttc aatgttgcag ggtagaagta 600
gtcctggatg ataaccatgt tccnaaatga ctagtgaaga gacactgtgg tttcctgcct 660
ttaacaaant ggtgtactcc ttgccctcct ccaatantgt ccaaagggct ggtaaaaacc 720
ctttgattaa aggcgtgctg cctgttgagt tccccaangg nacttgggac anggganccg 780
catttcaaga ccggaacaaa ttgggagttt tgaaaaaagt ttttaaatng ggaatgggtt 840
acataaaaan gcttgaaatg gctaaaacaa aggngggaa 879

<210> 298
<211> 697
<212> DNA
<213> Homo Sapiens

<400> 298
aaagaatcgg atatgaaggt gccaactgta agtttgaaag tatctgaaag tgtaattgat 60
gtgaaaacaa ctatggaaag tatatctaat acgtctacgc agtctctcac agcagaaaca 120
aaggacatag ctttggaacc taaggaacaa aaacatgaag acaggcagag caatacacct 180
tctcctcctg ttagtacctt ttcatcaggt acttctacca ccagtgatat tgaagtttta 240
gatcatgaaa gtgtaataag tgagagctca gcgagctcga gacaagagac tacagattca 300
aaatcaagtc ttcacttgat gcagacatct tttcagcttc tctctgcatc tgcttgtcct 360
gaatataatc gtttagatga tttccaaaaa ctcactgaga gttgctgttc atctgatgct 420
tttgaaagaa tagactcatt tagtgtacag tcattagata gccggagtgt aagtgaaatc 480
aattcaagat gatgaattgt caggcaaggg gatatgcttt agtgcctatt ataagttaat 540
tcttcaactc caaaagtcta aaacagttga atctgccgaa ggaaaatctg aagaagtaaa 600
tgaaacatta agttatacca ctgaggaagc agaaatggga agaaaagtgg gcgaaagtgg 660
caactccccg gttaacngng aaaangcctg gatatcc 697

<210> 299
<211> 510
<212> DNA
<213> Homo Sapiens

<400> 299
aaanaatnaa ttatgttaan aactttatta ttttcnantc cttttaaang gntgtnaaat 60
aatacttcnt ccaaatcntt taaatgttnt naangccntt gcnaaatcct tataaataaa 120
ttttcnccct tatccaancn catcnanaaa acattgaata tgttcaggtt tcncnggann 180
ggtnccnaaa ggnnccncnt tttatacnga cttaattgtn aaagcngggt gaaataaatt 240
ttccnatcna aatttttttt aagtttaaat cnttcccncn ttaaatttcn nanagtgtcc 300
gtgtnactcc tacttttaaa ggaaaaaaat tantttaaaa tttaatancc cccgatttaa 360

taatttttta ctttaacncn taatgttcnt tttcctgaac nntaattaan aaatgttgaa 420
attttaaatg tnaaanantc caantttccg tntgttaaca ttacncctcc aatgttcnta 480
atatatntnt taacccntnc caattatnga 510

<210> 300
<211> 625
<212> DNA
<213> Homo Sapiens

<400> 300
attagatagc cggagtgtaa gtgaaatcaa ttcagatgat gaattgtcag gcaaggata 60
tgctttagtg cctattatag ttaattcttc aactccaaag tctaaaacag ttgaatctgc 120
tgaaggaaaa tctgaagaag taaatgaaac attagttata cccactgagg aagcagaaat 180
ggaagaaagt ggacgaagtg caactcctgt taactgtgaa cagcctgata tcttggtttc 240
ttctacacca ataaatgaag gacagactgt gttagacaag gtggctganc agtgtgaacc 300
tgctgaaagt cagccanaan cactttctga caaggaanat gtttgcaata cagttgaatt 360
tctgaatgaa aaagcnggaa aaaagggang ctcagttatt atctcttagt aaggaaaaag 420
cacttctagg aagaagcttt ttgatacctg aananatgaa atgttcacag tngaaaggaa 480
naanngcagt ancatttccn tccttgaaan gattnngttt actcaaagga attngnnnaa 540
nccngtanta gaaaagtttc aaacctaagn ccggnaaaag aggaagagat gcctggccta 600
aaaaaaggga aatccacnga ccatt 625

<210> 301
<211> 792
<212> DNA
<213> Homo Sapiens

<400> 301
aaaaantaaa ttatnttaaa aactttatta tttncnatnc attttatagg gtantaaaat 60
aatactnctn caaaatcatt taaatnttat tgatgccatt gcaaaatcat tataaataaa 120
tttnctccat tatccaatca catctaaata acattgaata tntacaggtt nctctggata 180
ggtaccaaaa ggtaccacnt tttatacaaa cttaattgtg aaanctgggt gaaataaatt 240
tncaaatcaa aatttttttt aantttaaat catncactct ttaaatttca aacagtgtca 300
gtgtgacnct tacttttaaa ggaaaaaaat tagtttaaaa tttaatancc acanatttaa 360
taatttttta ctttaacact taatgtacat tttcatganc agtaattaaa atatnttgaa 420
attttaaatn tgaaaaattt caaagtttca gtatnttaac attacncttc aaatgttctt 480
aatatatata taaacactta caaattataa atacaactag ttgtntntct acaatacata 540
tntgaacacc attcttcttc tctagccatn tttatntgan gataaagtaa taaatctctg 600
tgctattcaa gggaaaaaaa atgaatgctt taaaaaataa atctttaaaa aataattcca 660
aaaataaagt tcaaatattg cacaaaaata atttaactgt aaatattact ncntagtgta 720
aacaatttta aaaaaatttt acactctaca ntaaatccnc ttctnattct ttaaaaaaat 780
tatgggaaat cc 792

<210> 302
<211> 738
<212> DNA
<213> Homo Sapiens

<400> 302
aaagagtaaa ttatgttaag aactttatta ttttcgattc attttatagg gtagtaaaat 60
aatacttctt caaaatcatt taaatgttat tgatgccatt gcaaaatcat tataaataaa 120
ttttctccat tatccaatca catctagata acattgaata tgtacaggtt tcnctggata 180
ggtaccaaaa ggtaccacat tttatacaga cttaattgtg aaagctgggt gaaataaatt 240
ttcagatcaa aatttttttt aagtttaaat cattcactct ttaaatttca gacagtgtca 300
gtgtgactct tacttttaaa ggaaaaaaat tagtttaaaa tttaatagcc acagatttaa 360

taatttttta ctttaacact taatgtacat tttcatgagc agtaattaag atatgttgaa 420
attttaaatg tgaaagattt caaaggtttc agtatgttaa cattactctt caaatgttct 480
taatatatat ataaacactt acaaattata gatacaacta gttgtatatc tacaatacat 540
atatgaacac cattcttctt ccccnagcca tatttatatg agggataaag taataaatct 600
ctggtgctat tcaaggnaaa aaaatggaat gccttaaaaa aataaaatcc ttaaagaata 660
ggttcaaaaa ataaagttca aaatantngc ccaaaaataa attaacnngg taatattaac 720
tacataaggg taaaacaa 738

<210> 303
<211> 635
<212> DNA
<213> Homo Sapiens

<400> 303
gaacggccga gggtaacatc ccgggctcgc gggaggctgt cggggtaatg gccacacgct 60
gacagaacca gccgagtgga aaaggggagc gaagccgttc ctctgcaccc ttccccaggc 120
ctgaggcctt cccgcttggt gctgccgccg ccactgccgg ctgaggaggg gcgatgagtt 180
ggttcaacgc ctcccagctc tccagcttcg ctaagcaggc cctgtcccag gcccagaagt 240
ctattgacag ggttctggac atccaggaag aggagccgag catctgggcc gagaccattc 300
cgtatggaga gccgggaata agttcccctg tcagtggagg atgggatact tcaacctggg 360
ggttgaaatc aaacactgaa cctcagagtc caccaatagc ctctcctaaa gcaatcacaa 420
agccagttcn gaggactgtg gtcgatgaat ctgaaaattt cttcagtgcc tttctctcgc 480
caactgatgt ccagaccatt cagaagagtc cagtggtatc aaaacctcca ncataatcac 540
aacnaccang nagaangaan tgaaaancan cttacatgaa tccttgcaca ttggncaant 600
caagaaactt cctgaaacaa ctgaaatcac aaagt 635

<210> 304
<211> 847
<212> DNA
<213> Homo Sapiens

<400> 304
gagacggagt ctttctctgt cacccatgct ggagtgcagt ggcacaatct tggctcattg 60
taacctccac ctcccaggtt caagcaatgc tcctgcctca gcctcccgag tagctaggat 120
tacaggcgca caccaccacg ccaggctaat ttttgtattt ttagtagaga tggggtttca 180
ccaaactgct ggccatgctg gtcttgaact cctgacatca ggtgatatgc ccgccttggc 240
ctcccaaagt gctgggatta caggcatgag ccacagcacc tggccgtaaa tgagagtttt 300
tatgtgcaag taaaggcagt taaataactt tcagtaataa aatgcatcac aatatttcac 360
aggtttaaaa cacaacctgg ttaccttttt gaataaaata acatttggaa gaaggcatag 420
ctacttttaa aagctattct atgctttcct tgtgtttgaa atttcaagaa aaaataaaat 480
gataaatcac aaaattaaaa atgccaaatt caagttaatt cctataattc ttccattttg 540
ttatgaatat tctgtaatat caaacattca tttttaatgt gctaaaaata tgggtttaca 600
aaatatgaac aggtaatttt taaaagagta aattatgtta aagaacttta ataantttcg 660
attcatttta tagggtanta aaataatact tcttcaaaat caattaaatg ttattgaatg 720
ccatttgcaa aatcattata aataaatttt cncaattatc caatcacaat tctagataac 780
attgaataag tncaaggttt ccccgggata ngttccaaaa nggtnccaca attttatnca 840
gacctaa 847

<210> 305
<211> 767
<212> DNA
<213> Homo Sapiens

<400> 305
cccccttcgt ctcagctgtg cgggaacggc cgagggtaac atcccgggct cgcgggaggc 60

tgtcggggta atggccacac gctgacagaa ccagccgagt ggaaaagggg agcgaagccg 120
ttcctctgca cccttcccca ggcctgaggc cttcccgctt ggtgctgccg ccgccactgc 180
cggctgagga ggggcgatga gttggttcaa cgcctcccag ctctccagct tcgctaagca 240
ggccctgtcc caggcccaga agtctattga cagggttctg gacatccagg aagaggagcc 300
gagcatctgg gccgagacca ttccgtatgg agagccggga ataagttccc ctgtcagtgg 360
aggatgggat acttcaacct gggggttgaa atcaaacact gaacctcaga gtccaccaat 420
agcctctcct aaagcaatca caaagccagt tcggaggact gtggtcgatg aatctgaaaa 480
tttcttcagt gcctttctct cgccaactga tgtccagacc attcagaaga gtccagtggt 540
atcaaaacct ccaacaaaat cacaacgacc aagaaagaag aagtgaaaag caacttacat 600
gaatcccttg cacattggcc aatcaagaac tcctgaaaca actgaatcac aagtaaaaag 660
actccctcct tgtgtgtttc aaggggaaaa ctctgggcaa caaggtactt catcacctaa 720
aactgaaagg naaacaacga agaaaactgt ttaatnaaag aatccgg 767

<210> 306
<211> 1659
<212> DNA
<213> Homo Sapiens

<400> 306
cccccttcgt ctcagctgtg cgggaacggc cgagggtaac atcccgggct cgcgggaggc 60
tgtcggggta atggccacac gctgacagaa ccagccgagt ggaaaagggg agcgaagccg 120
ttcctctgca cccttcccca ggcctgaggc cttcccgctt ggtgctgccg ccgccactgc 180
cggctgagga ggggcgatga gttggttcaa cgcctcccag ctctccagct tcgctaagca 240
ggccctgtcc caggcccaga agtctattga cagggttctg gacatccagg aagaggagcc 300
gagcatctgg gccgagacca ttccgtatgg agagccggga ataagttccc ctgtcagtgg 360
aggatgggat acttcaacct gggggttgaa atcaaacact gaacctcaga gtccaccaat 420
agcctctcct aaagcaatca caaagccagt tcggaggact gtggtcgatg aatctgaaaa 480
tttcttcagt gcctttctct cgccaactga tgtccagacc attcagaaga gtccagtggt 540
atcaaaacct ccaacaaaat cacaacgacc aagaaagaag aagtgaaaag caacttacat 600
gaatcccttg cacattggcc aatcaagaac tcctgaaaca actgaatcac aagtaaaaag 660
actccctcct tgtgtgtttc aaggggaaaa ctctgggcaa caaggtactt catcacctaa 720
aactgaaagg naaacaacga agaaaactgt ttaataaaga atcggatatg aaggtgccaa 780
ctgtaagttt gaaagtatct gaaagtgtaa ttgatgtgaa aacaactatg gaaagtatat 840
ctaatacgtc tacgcagtct ctcacagcag aaacaaagga catagctttg gaacctaagg 900
aacaaaaaca tgaagacagg cagagcaata caccttctcc tcctgttagt accttttcat 960
caggtacttc taccaccagt gatattgaag ttttagatca tgaaagtgta ataagtgaga 1020
gctcagcgag ctcgagacaa gagactacag attcaaaatc aagtcttcac ttgatgcaga 1080
catcttttca gcttctctct gcatctgctt gtcctgaata taatcgttta gatgatttcc 1140
aaaaactcac tgagagttgc tgttcatctg atgcttttga aagaatagac tcatttagtg 1200
tacagtcatt agatagccgg agtgtaagtg aaatcaattc agatgatgaa ttgtcaggca 1260
agggatatgc tttagtgcct attatagtta attcttcaac tccaaagtct aaaacagttg 1320
aatctgctga aggaaaatct gaagaagtaa atgaaacatt agttataccc actgaggaag 1380
cagaaatgga agaaagtgga cgaagtgcaa ctcctgttaa ctgtgaacag cctgatatct 1440
tggtttcttc tacaccaata aatgaaggac agactgtgtt agacaaggtg gctgancagt 1500
gtgaacctgc tgaaagtcag ccanaancac tttctgacaa ggaanatgtt tgcaatacag 1560
ttgaatttct gaatgaaaaa gcnggaaaaa agggangctc agttattatc tcttagtaag 1620
gaaaaagcac ttctaggaag aagctttttg atacctgaa 1659

<210> 307
<211> 831
<212> DNA
<213> Homo Sapiens

<400> 307
ctaagcattc catattggaa gaagagattt ctacacatga aaaaaatgcc tttgtttagt 60

aaatcacaca aaaatccagc agaaattgtg aaaatcctga aagacaattt ggccattttg 120
gaaaagcaag acaaaaagac agacaaggct tcagaagaag tgtctaaatc actgcaagca 180
atgaaagaaa ttctgtgtgg tacaaacgag aaagaacccc caacagaagc agtggctcag 240
ctagcacaag aactctacag cagtggcctg ctagtgacac tgatagctga cctgcagctg 300
atagactttg agggaaaaaa agatgtgacc cagatattta acaacatctt gagaagacag 360
ataggcactc ggagtcctac tgtggagtat attagtgctc atcctcatat cctgtttatg 420
ctcctcaaag gatatgaagc cccacagatt gccttacgtt gtgggattat gctgagagaa 480
tgtattcgac atgaaccact tgccaaaatc atcctctttt ctaatcaatt cagagatttc 540
tttaagtacg tggagttgtc aacatttgat attgcttcag atgcctttgc tactttcaag 600
ggatttacta accagacata aagtgttggt agcaagactt cttagaacaa aattacgaca 660
ctanttttga agactatgag aaattgcttc agtctgagaa attatgttac caagagacag 720
tccttaaagc ctgctaaggg aactgattct ggaccgtcan aactttgcca tcaangcaaa 780
agtttatcaa caagccnggg gaaaccggaa acncaaggag gaacctcctt c 831

<210> 308
<211> 833
<212> DNA
<213> Homo Sapiens

<400> 308
ccattcaaat gtttatactc catctaccca gaacaattac agcagaaaaa ataggcacct 60
ccaaagtctt cccaagaatg atgactttct gaaatgacac actgtacaaa ctggacaaat 120
gagacgactg actgtgacag gggccgggga gctcttcaag gggccgtttt cttcaagtct 180
cggatctgtt taatcaagta gttcttctcg tcagcgaact gctcatcatc cgtcctttct 240
ttttggaagc tgctcagaaa ctcaatgagt ttgggctgat tttttaacag gatctccaca 300
ataggctgtg ttttgtgagg actggccaca aacaccttaa aaacatgaaa ggcttcaaac 360
tggatgttgg gacttttatc ccgaaggagg ttcatcatga gtttcaggtt ctccggcttg 420
ctgatatact ttgtcatgat ggcaaagttg tgacggtcca ggatcagctc ccctagcagc 480
tttaaagact gtctcttagt aacataattc tcagactgaa gcaatttctc atagtcttca 540
aaaatagtgt cgtaattttg ttctaagaag tctgctacca acactttaag gtcnggttag 600
taaatccttg aaagtagcaa aggcatctga agcaatatca aatgttgaca actccacgtt 660
acttaaagaa atctctgaat tgattagaaa aagaggatga ntttgggcaa ntgggtcaag 720
tcgaatanat tcctctcaag cataaaccca caaacgttaa ggnaaaccgg tgggggcttc 780
aaaanccntt gagggagcat aaancangga tattagggat nagcacccaa ata 833

<210> 309
<211> 1320
<212> DNA
<213> Homo Sapiens

<400> 309
gcatcaccac catccacgag gatgaggtgg ccaagaccca gaaggcagag ggaggtggcc 60
gcctgcaggc tctgcgttcc ctgtctggca ccccaaagtc catgacctcc ctatccccac 120
gttcctctct ctcctccccc tccccaccct gttcccctct catggctgac cccctcctgg 180
ctggtgatgc cttcctcaac tccttggagt ttgaagaccc ggagctgagt gccactcttt 240
gtgaactgag ccttggtaac agcgcccagg aaagataccg gctggaggaa ccaggaacgg 300
agggcaagca gctgggccaa gctgtgaata cggcccaggg gtgtggcctg aaagtggcct 360
gtgtctcagc cgccgtatcg gacgagtcag tggctggaga cagtggtgtg tacgaggctt 420
ccgtgcagag actgggtgct tcagaagctg ctgcatttga cagtgacgaa tcggaagcag 480
tgggtgcgac ccgaattcag attgccctga agtatgatga gaagaataag caatttgcaa 540
tattaatcat ccagctgagt aacctttctg ctctgttgca gcaacaagac cagaaagtga 600
atatccgcgt ggctgtcctt ccttgctctg aaagcacaac ctgcctgttc cggacccggc 660
ctctggacgc ctcaagacac tctagtgttc aatgaggtgt tctgggtatc catgtcctat 720
ccaagccctt caccaagaag accttaagag tcgatgtctg taccaccgac aggagccatc 780
tgggaaaagt gcctgggagg cgcccaaatn agcctggcgg aggtctgccg gtctggggga 840

aaagtcgact cgcttggtac aactttctca gntacaaaat acttgaagaa acagagcagg 900
gagctcaagc cagtgggagt catggccctt gcttcagggc ntgccagcac ggacgctgtg 960
tcttgctctg ttggaacaga cagcagtgga gttggagaag aggcaggagg gcaggagcag 1020
cacacagaca ctggaagaca gctggtgagt gagcccgccc ttgggcccca ggagctgccc 1080
tgcctggacc taggcccagc aatgagatcc cccaatgcca gtgcaactaa gagaagggtt 1140
ccactgggaa ggctgagaac ccctctcctc atgggttctc tacaggcaaa aaggcaatgt 1200
aacctagtac gatggttccc agaattcctt tcgaatttgc catttcgttt cccatgaatc 1260
acctatgcta gttcacacct aatgttattc tttatcttga tatagtgaca tttattttgc 1320

<210> 310
<211> 1030
<212> DNA
<213> Homo Sapiens

<400> 310
aacatttctg tgatcaacat tgcttactgc gtttctactg tcaacaaaat gagcccaaca 60
tgacaactca gaaaggacct gaaaacttac attatgatca gggttgtcag acatctcgaa 120
ccaaaatgac aggttcagca ccaccccctt ctccaacacc taacaaagag atgaagaaca 180
aagcagttct ttgcaaacct ttaacaatga caaaagctac ttactgtaaa cctcacatgc 240
agaccaaatc ttgtcagaca gatgatactt ggaggacaga atatgttcca gtgcctatcc 300
ctgtgcctgt gtatatccca gttcctatgc acatgtacag tcagaatatt cctgttccta 360
ctacagttcc tgttcctgtg ccagttcctg tttttctgcc tgctccattg gacagcagtg 420
agaagattcc tgcagcaatt gaggagctaa aaagcaaggt ttcttcagat gctcttgata 480
cagagttgct tacaatgacg gatatgatga gtgaagacga ggggaaaaca gagacaacca 540
acatcaacag tgtaattatt gaaacagata taattggttc agaccttttg aagaactctg 600
acccagagac acagtccagc atgcctgatg taccatatga accaagattt ggatatcgaa 660
atagattttc ccagagctgc tgaggagctt gatatggaaa atgaattttt attaccacct 720
gtttttggcg aagaatatga ggaacagccc aagacctcga tctaaaaaaa aagggagcca 780
agagaaangc tgtatcaagg ataccaagtc tcatgatgat aagtctgaca atttcagaat 840
gcagcnttcc tttcaaatta tacgtatggg cgtaaatgca tgggnaacac cgggtcaaaa 900
actaagnnac ttggatgaaa gatcntccgg gnaattagaa tgagttaaaa tccttccaaa 960
tccantnaag tttaaaagag ggtntaatcc cctcaaaacc anagctggng ccttaacaag 1020
ggggttaacc 1030

<210> 311
<211> 546
<212> DNA
<213> Homo Sapiens

<400> 311
gtttctgttt tcagaagaat tgggaaaact tctgtgaaag aagaatgcag aaacaaagaa 60
atatgaagtc ttgggagtat actgattaaa aagcacacat tgggagtgat agtaagaaga 120
gctaaaataa aaagcacaga aggaaaaaat aattgatttg tacataagct aaattataat 180
tcctttaaaa ttgtttataa caagatggaa tacagaatga cgattagatt tataacgtgt 240
gtttatatga atatgttgtt aacagtgaga tttctgatat ggtataacaa agtatatgat 300
tggaggacct gcaaaatgta tactcgggtt gtttttcttt ttaaaaatat tgtnaaacag 360
gcaagtgagg cttaacagca ttatggttca ttacngggtt tgggntatat acctttttca 420
gcttctgtna tgagcaagtt gtgttttcaa tccccacttt caatgtctat gggaagggcg 480
cnttttgctn tgttttgttt tgtctttaaa ncnttttnaa acnggggaca canatggang 540
ccggcc 546

<210> 312
<211> 518
<212> DNA
<213> Homo Sapiens

<400> 312
aaaattatta ntntaaaagg ggaaataggt nggattnccn tnttnagggc aataattntg 60
gggaggaatg gggtggggct nacccctgna acccatnata aacctattct nctnagggtg 120
ctgggaaana attggggtct ggaataaanc tncaaatggg tcnccngctt cactaaaacc 180
ttggcaacta aggctcattt ttccaaaggg gttnctnang tcnnctccct ntnaaatcnt 240
tttattatnc cagggtggct gttgctaang cttnggtggg aaancangaa nttnctgctn 300
ctnctgctgc tgttgctgct gggcantnca agggaaaacc cccccgacaa actgggataa 360
ngtgacctgn ttgcncacnt ctngggccct attnccntac ctgncctgna aatncttccc 420
nctctgcccc ctttactnnt gccaannctt tcccccccgg ttaggataaa aattcccctn 480
aacctccnac ctttggttan cgggggtccc ctnccccc 518

<210> 313
<211> 660
<212> DNA
<213> Homo Sapiens

<400> 313
gccaagctgt gaatacggcc caggggtgtg gcctgaaagt ggcctgtgtc tcagccgccg 60
tatcggacga gtcagtggct ggagacagtg gtgtgtacga ggcttccgtg cagagactgg 120
gtgcttcaga agctgctgca tttgacagtg acgaatcgga agcagtgggt gcgacccgaa 180
ttcagattgc cctgaagtat gatgagaaga ataagcaatt tgcaatatta atcatccagc 240
tgagtaacct ttctgctctg ttgcagcaac aagaccagaa agtgaatatc cgcgtggctg 300
tccttccttg ctctgaaagc acaacctgcc tgttccggac ccggcctctg gacgcctcaa 360
gacactctag tgttcaatga ggtgttctgg gtatccatgt cctatccaag cccttcacca 420
agaagacctt aagagtcgat gtctgtacca ccgacaggag ccatctggga aaagtgcctg 480
ggaggcgccc aaatnagcct ggcggaggtc tgccggtctg gggggaaagt cgactcgctn 540
gtacaacctt ctcagctaca aatacttgaa gaaacaagac aangggactc aagccantgg 600
gagtcatggg ccctggcctc angggctgcc aacaacgggc cccgtgttct ggccccgttt 660

<210> 314
<211> 516
<212> DNA
<213> Homo Sapiens

<400> 314
gaaaggccac tttattgatg gagataaaac tgaatggagt tccccacagc cctcccctca 60
ctcatgttag tggcttnact gggcatctga gaccagcgtg gcctgtcacc cacatanact 120
aggctgctta gcccacccag cctatcacac tgcccgctcc acgttgggca gccacataaa 180
aacacgtcac agctcaanaa natccgtgga tgcacctctg aatccccccc aatggtttct 240
gtgcattttt ttaatattgt acaaaatatg ttaactagga aaaattagct gtactgtgac 300
aagtgcggga cgtcctatta ggattaccgt cccccaggca ttacttctta ttgcagtaag 360
acctctaaaa ggtggagctg tncaaaccaa aaaaaatcta aacgatttta agaanagcag 420
caactcaata ctgctttagt tcatttaaat tttctttccc aaaaatacac tcctaaatat 480
acaaactata caatcttatt attttaatgc tggttt 516

<210> 315
<211> 677
<212> DNA
<213> Homo Sapiens

<400> 315
   tcagaatggc agattcagga gagagtttgt gccagaatag cattgaagaa cttgatggtg 60
   tccttacatc catattcaaa catgagatac catattatga gttccagtct cttcaaactg 120
   aaatttgttc tcaaaacaaa tatactcatt tcaaagaact tccaactctt ctccactgtg 180
   cagcaaaatt tggcttaaag aacctggcta ttcatttgct tcaatgttca ggagcaacct 240
   gggcatctaa gatgaaaaat atggagggtt cagaccccac acatattgct gaaaggcatg 300
   gtcacaaaga actcaagaaa atcttcgaag acttttcaat ccaagaaatt gacataaata 360
   atgagcaaga aaatgattat gaagaggata ttgcctcatt ttccacatat attccttcca 420
   cacagaaccc agcatttcat catgaaagca ggaagacata cgggcaagag tgcaaatgga 480
   gctgaggcaa atgaaatgga aggggaaggn aaacagaatg ggntcaggca tggagaccaa 540
   acacagccca ctaagaggtt ggcagtgaga gttctgaaag accagtatga tgacttgtan 600
   gtgttcaatc cctgggngct gattcaagaa aaataattcc acaagggtgc tattcntngt 660
   ttttacaaga cntcctt 677

<210> 316
<211> 843
<212> DNA
<213> Homo Sapiens

<400> 316
agctttaaac attcaattta tttgtggcat ttgtacatga aaattatatg acgataacat 60
tgctttctat tctaagctag taaattgttt ctaagaaata atagattgat aaaattgcaa 120
gtcttaatac aaaggtaggt tatgaaaatg tatattaatt tgagatatag aaaagttttc 180
aaataataat gttttcaggg ttatatgcaa atagacacta aataagacaa ggtttctgca 240
aacatgatgt aacaataatg actggaactc tgaatgtgag aaattcagaa aatgaaccag 300
ctacttaaaa agcaaaaatg tgctaagtaa atttgtattt tcatggttat tctaaggaga 360
ggaggaataa tctgttgagg ttagtgccct caagcagacc ccataacttt gctacaccgc 420
atttaacttc tctgtgctgt tttcttttaa ttttcaaaat ggaaattagc tgtttcattg 480
gtgaagtgca ttgtaaaatg agagaatttt caaataatgc aattactcta tggtattctg 540
ttttaatagt aatataccca tatgaagcag gtataatgag aataaatttt gccaataaca 600
aattctgaaa tctgaanttt gtttctgctg tcatagtatg aattcgcttt aaagananca 660
ggcaatccaa attcaacttg ctcacctgaa aacaaaatgt ccgtanatcg tgagttcata 720
taataacctc cttaatgatc ttcctgcaca naaaccaaat tcttttcaac ttggggtcaa 780
caagaaccta ttgctgaatt ttcatataaa actatttcct gttggcagtt tcctaccccc 840
gga 843

<210> 317
<211> 835
<212> DNA
<213> Homo Sapiens

<400> 317
acaagacacg cctgcgtagt ggtagtgccc tcctgcagtc ccagtctagt actgaggacc 60
cgaaggatga gcctgcggag ctaaaaccag attctgagga cttatcctcc cagtcctcag 120
cttcaaaggc atctcaggag gatgccaatg aaatcaagtc taaacgggat gaagaagaac 180
gagaacgaga aaggagggag aaggagaggg aacgagaaag agaacgggag aaggagaagg 240
agagagaacg agagaagcag aagctaaaag agtcagaaaa agagagagat tctgctaagg 300
ataaagagaa aggcaaacat gatgatggac ggaaaaagga agcagaaatt atcaaacaat 360
tgaagattga actcaagaag gcacaggaga gccaaaagga gatgaaacta ttgctggata 420
tgtaccgttc tgccccaaag gaacagagag acaaagttca gctgatggca gctgagaaga 480
agtctaaggc agagttggaa gatctaaggc aaagactcaa ggatctggaa gataaagaga 540
agaaagagaa caaagaaaat ggctgatgaa ggatgccttg aggaagatcc gggcagtgga 600
gggacaagat agaataccta cagaagaagc taagccatgg gcaagcagga agaagaagca 660
ctcctctctg aaatgggatg tcacaaggcc aagcctttga agacatgcag gagcaaaaat 720
atccgntttg attgcagcaa nttgccggga anaanggatg atgccaaatt ttcaaagccc 780
aatgtcaaaa gccgttttca agttccaaat ccagnttcat naagnttgcc ttaaa 835

<210> 318
<211> 582
<212> DNA

<213> Homo Sapiens

<400> 318
caaactgaat cctgctttaa ttcaagcttg nggagaacaa agtcctacag aaacattcca 60
nanaattttc nggaaaagag ggatcacaac aaccctgtaa aaaggagact ganagtaatt 120
canagctcac caagttcncn ccgtatcaaa tttccanaat acccacaaga tttcttcacc 180
anctcantcc tgactcaacc tcttcaatct ttanttcatt agaagacaaa gggtcanatt 240
atttaaaatt antcnantcc caagaaattt aaagacttga agtagtagag cattcaaaac 300
ttaaataact ttaacaagaa agccanctga tcttaacaag ttacncngcn antaaatggg 360
aaatagactg aatcanccta nacataattt cattagggnt gcaaaccacc cangggaaag 420
tagcacaatt ataccanttt gtaatccaca ttcacaagaa gtttgcnaca caaatgaaga 480
aaactttgng cccatagaca acttattttt taaaatatca ctccccaaaa gtagccatgt 540
ttccactttt ggtccccttt ccanatcaaa aataccaact tg 582

<210> 319
<211> 827
<212> DNA
<213> Homo Sapiens

<400> 319
gaagccattc gatgttcatc agattggcca tttcagccat accttgtgtt tgatgttgga 60
gatggttcag aaagacggga taatgactca tatataaatg ttcaagaaat aaaactggtg 120
atggaaataa ttaagcttat taaagacaaa agaaaggatg ttagttttcg aaacattggc 180
ataataactc attacaaggc ccagaagacg atgattcaga aggatttgga caaagagttc 240
gatagaaaag gaccagcaga agtagacact gtggatgcat tccagggtcg gcagaaggat 300
tgtgttattg ttacgtgtgt cagagcaaat agcatccaag gttcaattgg attcctggca 360
agtttgcaga gattgaatgt caccatcaca cgagccaagt acagcctctt catcctcgga 420
catttgagga ccctgatgga aaaccagcat tggaatcagc tgattcagga tgctcagaag 480
cgtggtgcca ttattaagac ctgtgacaaa aactatagac atgatgcagt gaagattctg 540
aaactcaagc ctgtgctgca gagaagtctc actcancctc ctaccatagc cccaaagggg 600
tccaaacccc aagggtggnt tgcccaagca ncaagctaga cagttggatt ttgccaaaga 660
caatcctggt tgccggcttc tccaatacca aaacaaccct ccggactccc aagggaaaat 720
tacncctaac ggtttacctt caaaggaccc ctgaaaagac ccncctggtt caatgaccaa 780
cnttcanggg ncccacgaan tggctgaaaa agggatgggc aatttag 827

<210> 320
<211> 598
<212> DNA
<213> Homo Sapiens

<400> 320
aaattttaaa aggattttgt tatttgctat acaaatatac atttcaactt ttacaacatt 60
cactccagtc tgacctcctt gtctatagaa gactaagaga tcaacatttc cagtctctga 120
cttcaaggac attattacgg atacacaatg ccctctgaaa gcttttgcaa atgacagaaa 180
atactgaaga tgaccagagg ctcaggtgtt aaggatgcat tttccatgtt ttccaacagc 240
acacaaactc cttacaaaaa acaagcttat ctagatggtc ccacgagctg gtcatcttca 300
gtttacaata tgctgtggct gctggcccat gtcactgggc tttcctataa aagctttctt 360
ttcttgggaa ctgctgtcct cctgctccaa gtgtcctctt gtcccaccta gagttcctcc 420
tggtgtgatg ggtctcggaa ccacacttct cctgctcccc ttcactgaaa gccctggcct 480
ctctcctgtg acagagctcc tcttccgggt catcacattt gctctgacac gtgggnagcc 540
tcggggaact gggcanctgg gaggntccgt ttttttttgg gaaggtttgt tggctgcc 598

<210> 321
<211> 808
<212> DNA

<213> Homo Sapiens

<400> 321
gcatcaccac catccacgag gatgaggtgg ccaagaccca gaaggcagag ggaggtggcc 60
gcctgcaggc tctgcgttcc ctgtctggca ccccaaagtc catgacctcc ctatccccac 120
gttcctctct ctcctccccc tccccaccct gttcccctct catggctgac cccctcctgg 180
ctggtgatgc cttcctcaac tccttggagt ttgaagaccc ggagctgagt gccactcttt 240
gtgaactgag ccttggtaac agcgcccagg aaagataccg gctggaggaa ccaggaacgg 300
agggcaagca gctgggccaa gctgtgaata cggcccaggg gtgtggcctg aaagtggcct 360
gtgtctcagc cgccgtatcg gacgagtcag tggctggaga cagtggtgtg tacgaggctt 420
ccgtgcagag actgggtgct tcagaagctg ctgcatttga cagtgacgaa tcggaagcag 480
tgggtgcgac ccgaattcag attgccctga agtatgatga gaagaataag caatttgcaa 540
tattaatcat ccagctgagt aacctttctg ctctgttgca ncaacaaaga ccagaaagtg 600
aatatccgcg tggctgtcct tccttgctct gaaaagcaca aactgcctgt tccgggaccc 660
gggctctgga cgcctcaaac actccaagtg ttcaatgaag gtgttctggg tatccatggt 720
ccctatccaa acccnttaac aagaaagacc tttaaanaag tccaatgtcc ngtnaccaac 780
cggacaaggg agccaatctt gggaaaaa 808

<210> 322
<211> 629
<212> DNA
<213> Homo Sapiens

<400> 322
agcaaaataa atgtcactat atcaagataa agaataacat taggtgtgaa ctagcatagg 60
tgattcatgg gaaacgaaat ggcaaattcg aaaggaattc tgggaaccat cgtactaggt 120
tacattgcct ttttgcctgt agagaaccca tgaggagagg ggttctcagc cttcccagtg 180
gaacccttct cttagttgca ctggcattgg gggatctcat tgctgggcct aggtccaggc 240
agggcagctc ctggggccca agggcgggct cactcaccag ctgtcttcca gtgtctgtgt 300
gctgctcctg ccctcctgcc tcttctccaa ctccactgct gtctgttcca acagagcaag 360
acacagcgtc cgtgctggca ngccctgaag caagggccat gactcccact ggcttgagct 420
ccctgctctg tttcttcaag tattttgtan ctgagaaagt tgtaccaanc gaatcnacct 480
ctccccaaga ccgggaagac ctcccgccaa ggctgatttg gggcgcctcc caagcactct 540
tccaaaatgg ctcccgtccg ttgggacana catccnactt tttaangcct tccggggnaa 600
agggctgggn taaggacatt gggtncccc 629

<210> 323
<211> 798
<212> DNA
<213> Homo Sapiens

<400> 323
aacatttctg tgatcaacat tgcttactgc gtttctactg tcaacaaaat gagcccaaca 60
tgacaactca gaaaggacct gaaaacttac attatgatca gggttgtcag acatctcgaa 120
ccaaaatgac aggttcagca ccaccccctt ctccaacacc taacaaagag atgaagaaca 180
aagcagttct ttgcaaacct ttaacaatga caaaagctac ttactgtaaa cctcacatgc 240
agaccaaatc ttgtcagaca gatgatactt ggaggacaga atatgttcca gtgcctatcc 300
ctgtgcctgt gtatatccca gttcctatgc acatgtacag tcagaatatt cctgttccta 360
ctacagttcc tgttcctgtg ccagttcctg tttttctgcc tgctccattg gacagcagtg 420
agaagattcc tgcagcaatt gaggagctaa aaagcaaggt ttcttcagat gctcttgata 480
cagagttgct tacaatgacg gatatgatga gtgaagacga ggggaaaaca gagacaacca 540
acatcaacag tgtaattatt gaaacagata taattggttc agaccttttg aagaactctg 600
acccagagac acagtccagc atgcctgatg taccatatga accagatttg gatatcgaan 660
tagattttcc cagagctgct gaaggagcct tgatatggga aaatgaattt ttattaccaa 720
ccngtttttg ggcgaaagaa tatgaaggaa caagcccaaa cctcgattct aaaaaaaagg 780

ggagccaagg agaaaagg 798

<210> 324
<211> 754
<212> DNA
<213> Homo Sapiens

<400> 324
aaaaggacac taaggtttta ataaggggaa caaaaaattg ttttcaccag catagattca 60
cattacagta caccaatatt gacagcattc tcttgtctat ttttggtaca gaagatggta 120
tctctctaca taaccttgta aggcttcagt aactaaaatg taaaaccaaa caaaacaaaa 180
ccccaaaaca aaacaaaaac cccagcctat tagtttacag tttattttaa aaattccgaa 240
agacactgca agttctaaac ttttagtagt gctacccata cacaaccatc tggttaagaa 300
cccagtaaaa gagccccctt ccaaggaagc tttgcaacag tagagttgtg caatatggat 360
gtttcttact acaagaaaaa aattatacat ggcacattct cattcatatt ctgtaatgta 420
aaaagttaca aacataccta atcaaataaa taataataaa aaaagaattt gaatgtattt 480
gttaagtatc ctaaaaccac tacatagaat aatggcaact ttcactcaca gattatttac 540
atggtaatac ccagcgtggg tacactgcta caaaactcaa aacagaagga gtaaacttga 600
aatgttttcc ataataaaga tctagcanca tgactatcct aatgccgttt tatcccgaat 660
gcttctggca acgttccctt ttaatccggt gtctcatcca attcaaaaan tggcctttac 720
caaaaaatat ccttttacaa gaaagaaacc cgtt 754

<210> 325
<211> 854
<212> DNA
<213> Homo Sapiens

<400> 325
ggtcaggggt gagagctgga atctctgcac gggccttgga aaacgactgt cttcttctgc 60
caaaatgtca ggaattggaa ataaaagagc agctggagaa cctggcacct ccatgcctcc 120
tgagaagaag gcagctgttg aagattcagg gaccacagtg gaaacaatta agctaggagg 180
tgtctcttca acggaggaac tagacattag aacactgcaa accaaaaatc gcaagctggc 240
agaaatgttg gatcagcggc aggccattga agatgaactt cgtgagcaca ttgaaaaact 300
ggaacgacga caggccactg atgatgcctc actattgatt gtcaaccgat actggagtca 360
gtttgatgaa aacatccgta tcatccttaa acgttatgat ctggagcagg gcttgggaga 420
cctactcaca gaacgaaaag cccttgttgt gcctgaacca gaaccagact ctgatagcaa 480
tcaggagcgt aaagatgacc gagagagagc agttccagtg aagagatgga gtctcagctg 540
caggaacgtg tggagtcttc ccgccgagcc gtgtcccaga ttgtgactgt ttatgataaa 600
ttgcaagaaa aagtggagct cttatcccgg gaagctaaac agtgggagat aatctgatag 660
tggagggaag canttgcaag gagctgaact ctttcctcgc acaaggagaa tattaaggct 720
acanggaatt gacaagatct tcctcaggaa aaagcatcgc aaccatggtc tcaaggngtt 780
cctccaaagt tgcaagaggt aaaattgggg naaaagccga attcaccaan tttccggtcc 840
tggaagtcca anga 854

<210> 326
<211> 760
<212> DNA
<213> Homo Sapiens

<400> 326
caaactgaat cctgctttaa ttcaagcttg tggagaacaa agtcctacag aaacattcca 60
cagaattttc tggaaaagag ggatcacaac aaccctgtaa aaaggagact gagagtaatt 120
catagctcac caagttctct ccgtatcaaa tttccagaat acccacaaga tttcttcacc 180
agctcagtcc tgactcaacc tcttcaatct ttatttcatt agaagacaaa gggtcatatt 240
atttaaaatt attctagtct caagaaattt aaagacttga agtagtagag cattcaaaac 300

ttaaataact ttaacaagaa agccagctga tcttaacaag ttactctgct agtaaatggg 360
aaatagactg aatcatccta gacataattt cattagggct gcaaaccacc caggggagag 420
tagcacaatt ataccatttt gtaatccaca ttcacaagaa gtttgctaca caaatgaaga 480
aaactttgtg cccatagaca acttattttt taaaatatca ctccccaaaa gtagccatgt 540
ttccactttt gttccctttt ccacatcaaa aataccaact tgatttcttc aggaggaatg 600
gacaatccaa gtttatacaa gtgggctggg aaaaagaaaa cactgaaaag tctaaaagca 660
caagataaac aaagcctggg aagggaagac agttaagagt tatttgtttc caantcaatc 720
cnaaaaccca anggcttgta attaacaagt cctttccggc 760

<210> 327
<211> 852
<212> DNA
<213> Homo Sapiens

<400> 327
caaagcagtt ctttgcaaac ctttaacaat gacaaaagct acttactgta aacctcacat 60
gcagaccaaa tcttgtcaga cagatgatac ttggaggaca gaatatgttc cagtgcctat 120
ccctgtgcct gtgtatatcc cagttcctat gcacatgtac agtcagaata ttcctgttcc 180
tactacagtt cctgttcctg tgccagttcc tgtttttctg cctgctccat tggacagcag 240
tgagaagatt cctgcagcaa ttgaggagct aaaaagcaag gtttcttcag atgctcttga 300
tacagagttg cttacaatga cggatatgat gagtgaagac gaggggaaaa cagagacaac 360
caacatcaac agtgtaatta ttgaaacaga tataattggt tcagaccttt tgaagaactc 420
tgacccagag acacagtcca gcatgcctga tgtaccatat gaaccaagat ttggatatcg 480
aaatagattt tcccagagct gctgaggagc ttgatatgga aaatgaattt ttattaccac 540
ctgtttttgg cgaagaatat gaggaacagc ccaagacctc gatctaaaaa aaaagggagc 600
caagagaaan gctgtatcaa ggataccaag tctcatgatg ataagtctga caatttcaga 660
atgcagcntt cctttcaaat tatacgtatg ggcgtaaatg catgggnaac accgggtcaa 720
aaactaagnn acttggatga aagatcntcc gggnaattag aatgagttaa aatccttcca 780
aatccantna agtttaaaag agggtntaat cccctcaaaa ccanagctgg ngccttaaca 840
agggggttaa cc 852

<210> 328
<211> 799
<212> DNA
<213> Homo Sapiens

<400> 328
aaaaggacac taaggtttta ataaggggaa caaaaaattg ttttcaccag catagattca 60
cattacagta caccaatatt gacagcattc tcttgtctat ttttggtaca gaagatggta 120
tctctctaca taaccttgta aggcttcagt aactaaaatg taaaaccaaa caaaacaaaa 180
ccccaaaaca aaacaaaaac cccagcctat tagtttacag tttattttaa aaattccgaa 240
agacactgca agttctaaac ttttagtagt gctacccata cacaaccatc tggttaagaa 300
cccagtaaaa gagccccctt ccaaggaagc tttgcaacag tagagttgtg caatatggat 360
gtttcttact acaagaaaaa aattatacat ggcacattct cattcatatt ctgtaatgta 420
aaaagttaca aacataccta atcaaataaa taataataaa aaaagaattt gaatgtattt 480
gttaagtatc ctaaaaccac tacatagaat aatggcaact ttcactcaca gattatttac 540
atggtaatac ccagcgtggg tacactgcta caaaactcaa aacagaanga gtaaacttga 600
aatgttttcc ataataaaga tctagcaaca tgactatcca atgctgtttt atcccgattg 660
cttctgcaac gttcctttta atccgtgtct catccagttc anaantgtcc ttatcaanaa 720
taacctttac tagaagaaac cgtncaagca tattttcaan gggtttccgg tccaattgaa 780
gttanacgtn taccaaaca 799

<210> 329
<211> 978
<212> DNA

<213> Homo Sapiens

<400> 329
ggaagatggc ggcggccgtt ccacagcggg cgtggaccgt ggagcagctg cgcagtgagc 60
agctgcccaa gaaggacatt atcaagtttc tgcaggaaca cggttcanat tcgtttcttg 120
cagaacataa attattagga aacattaaaa atgtggccaa gacagctaac aaggaccact 180
tggttacagc ctataaccat ctttttgaaa actaagcgtt ttaagggtac tgaaagtata 240
agtaaagtgt ctgagcaagt aaaaaatgtg aagcttaaat gaagataaac ccaaagaaac 300
caagtctgaa gagaccctgg atgagggtcc cccaaaatat actaaatcct gttctgaaaa 360
agggagataa aaccaacttt cccaaaaagg gagatgttgt tcactgctgg tatacaggaa 420
cactacaaga tgggactgtt tttgatacta atattcaaac aagtgcaaag aagaagaaaa 480
atgccaagcc tttaagtttt aaggtcggag taggcaaagt tatcagagga tgggatgaag 540
ctctcttgac tatgagtaaa ggagaaaagg ctcgactgga gattgaacca gaatggcttt 600
acggaaagaa aggacagcct gatgccaaaa ttccnccaaa tgcaaaactc acttttgaag 660
tggaattagt ggatattgat tgaaatagca gtgcttcagc tctaaggata ttagcaacaa 720
tgataaaact tggccttgaa gaaatttacn caactagtta gaacttgtta ctattgtaaa 780
ggaagagtca actggaaaat tcaaggagtt aataaaattt gtttacttgg tcccagcttt 840
tgagagataa atcccttatg aatccctggt ctaaaatact ttcctacagc tgtgtaaaat 900
actggtcaag gagaactttt tccttttacc tcatgttgta aacttaagtg gctcaataaa 960
aattgatccn ctgtcttg 978

<210> 330
<211> 1017
<212> DNA
<213> Homo Sapiens

<400> 330
cgatcggcgg agctcccacc tccgcttaca gctcgctgcc gccgtcctgc cccgcgcccc 60
caggagacct ggaccagacc acgatgtgga aacgctggct cgcgctcgcg ctcgcgctgg 120
tggcggtcgc ctgggtccgc gccgaggaag agctaaggag caaatccaag atctgtgcca 180
atgtgttttg tggagccggc cgggaatgtg cagtcacaga gaaaggggaa cccacctgtc 240
tctgcattga gcaatgcaaa cctcacaaga ggcctgtgtg tggcagtaat ggcaagacct 300
acctcaacca ctgtgaactg catcgagatg cctgcctcac tggatccaaa atccaggttg 360
attacgatgg acactgcaaa gagaagaaat ccgtaagtcc atctgccagc ccagttgttt 420
gctatcagtc caaccgtgat gagctccgac gtcgcatcat ccagtggctg gaagctgaga 480
tcattccaga tggctggttc tctaaaggca gcaactacag tgaaatccta gacaagtatt 540
ttaagaactt tgataatggt gattctcgcc tggactccag tgaattcctg aagtttgtgg 600
aacagaatga aactgccatc aatattacaa cgtatccaga ccaggagaac aacaagttgc 660
ttaggggact ctgtgttgat gctctcattg aactgtctga tgaaaatgct gattggaaac 720
tcagcttcca agagtttctc aagtgcctca acccatcttt caaccctcct gagaagaagt 780
gtgccctgga ggatgaaacg tatgcagatg gagctgagac cgangtggac tgtaacccgc 840
tgtgtctgtg cctgtggaaa ttgggtctgt cagccatgac ctgtgacnga aagaatcaga 900
aggggcccca gacccagacn gaggangaga tgancngata tgtccaggag ctccaaagct 960
taggaaacag cttgaaaaga nccagagagg gagcccccaa agagattatg aggaggc 1017

<210> 331
<211> 799
<212> DNA
<213> Homo Sapiens

<400> 331
cccagaaaga tcatcacagt ttctgtaaaa gaagatgtac acctgaaaaa ggcagaaaat 60
gcctggaagc caagccaaaa acgagacagc caagccgatg atcccgaaaa cattaaaacc 120
caggagcttt ttagaaaagt tcgaagtatc ttaaataaat tgacaccaca gatgttcaat 180
caactgatga agcaagtgtc aggacttact gttgacacag aggagcggct gaaaggagtt 240

attgacctgg tctttgagaa ggctattgat gaacccagtt tctctgtggc ttacgcaaac 300
atgtgtcgat gtctagtaac gctgaaagta cccatggcag acaagcctgg taacacagtg 360
aatttccgga agctgctact gaaccgttgc cagaaggagt ttgaaaaaga taaagcagat 420
gatgatgtct ttgagaagaa gcagaaagaa cttgaggctg ccagtgctcc agaggagagg 480
acaaggcttc atgatgaact ggaagaagcc aaggacaaag cccggcggag atccattggc 540
aacatcaagt ttattggaga actctttaaa ctcaaaatgc tgactgaagc catcatgcat 600
gactgtgtgg tgaagctgct aaagaaccat gatgaagaat ccctggagtg cctgtgtcgc 660
ctgctcacca ccattggcaa agacttggac tttgaaaaag ccaaagccac gtatggaccc 720
cagtacttta atcagatgga gaaaattgtg aaaggaaaga aaaacctcat ctaggatcgg 780
gtcatgcttt caggaggtt 799

<210> 332
<211> 881
<212> DNA
<213> Homo Sapiens

<400> 332
cgatcggcgg agctcccacc tccgcttaca gctcgctgcc gccgtcctgc cccgcgcccc 60
caggagacct ggaccagacc acgatgtgga aacgctggct cgcgctcgcg ctcgcgctgg 120
tggcggtcgc ctgggtccgc gccgaggaag agctaaggag caaatccaag atctgtgcca 180
atgtgttttg tggagccggc cgggaatgtg cagtcacaga gaaaggggaa cccacctgtc 240
tctgcattga gcaatgcaaa cctcacaaga ggcctgtgtg tggcagtaat ggcaagacct 300
acctcaacca ctgtgaactg catcgagatg cctgcctcac tggatccaaa atccaggttg 360
attacgatgg acactgcaaa gagaagaaat ccgtaagtcc atctgccagc ccagttgttt 420
gctatcagtc caaccgtgat gagctccgac gtcgcatcat ccagtggctg gaagctgaga 480
tcattccaga tggctggttc tctaaaggca gcaactacag tgaaatccta gacaagtatt 540
ttaagaactt tgataatggt gattctcgcc tggactccag tgaattcctg aagtttgtgg 600
aacagaatga aactgccatc aatattacaa cgtatccaga ccaggagaac aacaagttgc 660
ttaagggact ctgtgttgat gctctcattg aactgtctga tgaaaatgct gantggaaac 720
ttagctttca agaagtttct caagngcctt naacccatct ttnaaccttc ttgagaagaa 780
tgtgcccttg gaggatgaaa cgtatgccan atggagcttg aaancgaggt ggactgtaan 840
ccgttggnct gggncctggg gaaaattggg tcttggacaa g 881

<210> 333
<211> 810
<212> DNA
<213> Homo Sapiens

<400> 333
   gtgcagtcac agagaaaggg gaacccacct gtctctgcat tgagcaatgc aaacctcaca 60
   agaggcctgt gtgtggcagt aatggcaaga cctacctcaa ccactgtgaa ctgcatcgag 120
   atgcctgcct cactggatcc aaaatccagg ttgattacga tggacactgc aaagagaaga 180
   aatccgtaag tccatctgcc agcccagttg tttgctatca gtccaaccgt gatgagctcc 240
   gacgtcgcat catccagtgg ctggaagctg agatcattcc agatggctgg ttctctaaag 300
   gcagcaacta cagtgaaatc ctagacaagt attttaagaa ctttgataat ggtgattctc 360
   gcctggactc cagtgaattc ctgaagtttg tggaacagaa tgaaactgcc atcaatatta 420
   caacgtatcc agaccaggag aacaacaagt tgcttagggg actctgtgtt gatgctctca 480
   ttgaactgtc tgatgaaaat gctgattgga aactcagctt ccaagagttt ctcaagtgcc 540
   tcaacccatc tttcaaccct cctgagaaga agtgtgccct ggaggatgaa acgtatgcag 600
   atggagctga gaccgangtg gactgtaacc cgctgtgtct gtgcctgtgg aaattgggtc 660
   tgtcagccat gacctgtgac ngaaagaatc agaagggggc ccagacccag acngaggang 720
   agatgancng atatgtccag gagctccaaa gcttaggaaa cagcttgaaa aganccagag 780
   agggagcccc caaagagatt atgaggaggc 810

<210> 334

<211> 808
<212> DNA
<213> Homo Sapiens

<400> 334
cactttaatt tctttattca tcaatagtat ccgaaaagga agaatcagga gttacaaaaa 60
caagttaaat gcaatataga agcctactaa atacaaatac aagttcacaa acacatatgc 120
aacagaaact tgtttagatt gtttcttgaa gtttgactac ttaaaaacat aggtgtaaag 180
gaaagacatt cagactggtc cacgtgggct tgttagcagg cagaggaacc ctgctttcca 240
aaaactgata tagtccagag tcacggcatg tgggaatgtt tccatggaca ctggatctta 300
acagatgcta tagtgtttac aaaactacac acacagagaa agcccaagga agcctgcagg 360
ctaagcccta tgcttttaga gggctgaagg aaccaaacct agtttaatcc tgtttgtttg 420
ctccatgcaa aactttatgg aagactcccc agactaggct atttagcagc ttccatgaat 480
ggtcctcaga tcatgtgatt ctacggcata gacgacagct gccctattta cacagaagct 540
gcagaactca agaggaatgt ggatttgctc ttgggaagtt caatgttgca gggtaaagta 600
agtcttggat gataaccatg ttctaaatga ctagtgaaga gacactgngg tttcttgctt 660
ttaacaaatt ggnggactct tggcccttct tcccatagng tccaagggct ggtaaaacct 720
ttggattaag gcgtgnctgc ttgggagttc ttccaaggca ctttggacca gggaacctgc 780
atttcaaact ggaccaagtg gaggtttg 808

<210> 335
<211> 758
<212> DNA
<213> Homo Sapiens

<400> 335
gcaattgggt atctcgaaga gcagatcaag ggcctaaaac tatcgaacag attcacaaag 60
aggctaaaat agaagaacaa gaagagcaaa ggaaggtcca gcaactcatg accaaagaga 120
agagaagacc aggtgtccag agagtggacg aaggtgggtg gaacactgta caaggggcca 180
agaacagtcg ggtactggac ccctcaaaat tcctaaaaat cactaagcct acaattgatg 240
aaaaaattca gctggtacct aaagcacagc taggcagctg gggaaaaggc agcagtggtg 300
gagcaaaggc aagtgagact gatgccttac ggtcaagtgc ttccagttta aacagattct 360
ctgccctgca acctccagca ccctcagggt ccacgccatc cacgcctgta gagtttgatt 420
cccgaaggac cttaactagt cgtggaagta tgggcaggga gaagaatgac aagccccttc 480
catctgcaac agctcggcca aatactttca tgaggggtgg cagcagtaaa gacctgctag 540
acaatcagtc tcaagaagag cagcggagag agatgctgga gaccgtgaag cagctnacan 600
gaggtgtgga tgtggagagg aacagccttg agctgaaccg aaataaacaa gggagtcagc 660
aaaaccccga aanttcagca atgtcagctt attgacaagg gttgattatc agaagaggac 720
tgganaggaa gtccaaatct atcatggtna attttttc 758

<210> 336
<211> 785
<212> DNA
<213> Homo Sapiens

<400> 336
aaacttgcaa tgtttgtctt tattttgttc tttatatttt caaagtgaaa agaaatagta 60
ctgagtcaat ttctttttgt ttttttaaat atttgttcta tgtatttaca agccttaaag 120
ttgctctaaa gatttcaaga gtattaagag tacttttctc agggtagcac tttttttttt 180
tttaaacaat tcttggagtt ctgtggtcca cagcatttcc ttctgtttca atgttatgta 240
cgttttgatt actattgnga ttttttaaat tttctgaagc aagctgagag gcaggcagaa 300
agatttgatg ccaaaaaaaa aaaaatcttt cttaccttgt tcaccccaaa ctttctcaaa 360
tctggactaa atgctatacc ttaaaacaaa catgaggngc atcttgaagg ggagggaaat 420
ttatttctct gcttttctat tatacaagtt gtttacagaa actgcaaatt aaaaaattac 480
actggcattt gcagtcctta aaataaatta aaagttctca actttttttt ttttgctaaa 540

cattttttta agtatgagtc cttgtttaaa aagaaaagat taaaacagaa aatattttct 600
ataaatacnt gnattttggg tttaagggct cccgccctaa ggnttgaagg ttacttttat 660
cccaggaccc tttttcctcc atggaacccc tttttttcnc ttttcccttt tcccacttcg 720
ngccncccnt ngggggtttc tggcaaaaaa tggcccttgc tgcnctgggg aattggccaa 780
aaacc 785

<210> 337
<211> 643
<212> DNA
<213> Homo Sapiens

<400> 337
ggaagatggc ggcggccgtt ccacagcggg cgtggaccgt ggagcagctg cgcagtgagc 60
agctgcccaa gaaggacatt atcaagtttc tgcaggaaca cggttcanat tcgtttcttg 120
cagaacataa attattagga aacattaaaa atgtggccaa gacagctaac aaggaccact 180
tggttacagc ctataaccat ctttttgaaa actaagcgtt ttaanggtac tgaangtnta 240
nntaaagtgt ctgancaagt naaaaatgnn aancttantg aagataancc caaagaaacc 300
aagtntgang agaccctgga tgagggtcca ccnaaatata ctaaatctgn tctgaaaaag 360
ggagataaaa ccaactttcc caaaaaggga gatgttgttc actgctggta tacaggaaca 420
ctacaagatg ggactgtttt tgatactaat attcaaacaa gtgcaaagaa naagaaaaat 480
gccaagcctt taagttttaa ggtcggagta cgcaaaagtt atcanaggat ggggatgaag 540
ctctcttgac tatgagtaaa ggagaaaagg ctngactgga aaatggaccc aaaatggctt 600
accggaaaga aagggacagc ctgatnccaa aatttcccca aat 643

<210> 338
<211> 831
<212> DNA
<213> Homo Sapiens

<400> 338
caagacagng gatcaatttt tattgagcca cttaagttta caacatgagg taaaaggaaa 60
aagttctcct tgaccagtat tttacacagc tgtaggaaag tattttagac cagggattca 120
taagggattt atctctcaaa agctgggacc aagtaaacaa attttattaa ctccttgaat 180
tttccagttg actcttcctt tacaatagta acaagttcta actagttgng taaatttctt 240
caaggccaag ttttatcatt gttgctaata tccttagagc tgaagcactg ctatttcaat 300
caatatccac taattccact tcaaaagtga gttttgcatt tggnggaatt ttggcatcag 360
gctgtccttt ctttccgtaa gcccattctg gttcaatctc cagtcgagcc ttttctcctt 420
tactcatagt caagagagct tcatcccatc ctctgataac tttgcctact ccgaccttaa 480
aacttaaagg cttggcattt ttcttcttct ttgcacttgt ttgaatatta gtatcaaaaa 540
cagtcccatc ttgtagtgtt cctgtatacc agcagtgaac aacatctccc tttttgggaa 600
agttggtttt atctcccttt ttcagaacag gatttagtat atttgggggg accctcatcc 660
agggtctctt cagacttggt ttctttgggt ttatcttcat ttaagcttca cattttttac 720
ttgctcagac actttactta tactttcagt acccttaaaa ccgcttaagt ttcaaaaaag 780
agggttatag gctgnaaccc aaggggggcc ttggtnagct ggccttgggc c 831

<210> 339
<211> 758
<212> DNA
<213> Homo Sapiens

<400> 339
ccaacatgtc ccgtggttcc agcgccggtt ttgaccgcca cattaccatt ttttcacccg 60
agggtcggct ctaccaagta gaatatgctt ttaaggctat taaccagggt ggccttacat 120
cagtagctgt cagagggaaa gactgtgcag taattgtcac acagaagaaa gtacctgaca 180
aattattgga ttccagcaca gtgactcact tattcaagat aactgaaaac attggttgtg 240

tgatgaccgg aatgacagct gacagcagat cccaggtaca gagggcacgc tatgaggcag 300
ctaactggaa atacaagtat ggctatgaga ttcctgtgga catgctgtgt aaaagaattg 360
ccgatatttc tcaggtctac acacagaatg ctgaaatgag gcctcttggt tgttgtatga 420
ttttaattgg tatagatgaa gagcaaggcc ctcaggtata taagtgtgat cctgcaggtt 480
actactgtgg gtttaaagcc actgcagcgg gagttaaaca aactgagtca accagcttcc 540
ttgaaaaaaa agtgaagaag aaatttgatt ggacatttga acagacagtg gaaactgcaa 600
ttacatgcct gtctactggt ctatcaattg atttcaaacc ttcagaaata gaagttggag 660
tagtgacagt tgaaaatcct aaattcagga ttcttacngg aagcagagat tgatgcttac 720
cttgtgnttt agcngagagg agacttaacc attggccg 758

<210> 340
<211> 840
<212> DNA
<213> Homo Sapiens

<400> 340
ccaaaagcct tgttttattt atatagagtc ctaaccactt cggtggtagg aggagtggga 60
gaggctcctt tttcaatcca gggacctcca tgatgttggt ttgttgttac caaacacaca 120
ggtaagtggc atcacggatc tggtaaacta acgacaatgt ttagtctctc tctgctagag 180
caacaaggtg agcatcaatc tctgcttctg taanaatcct gaatttagga ttttcaactg 240
tcactactcc aacttctatt tctgaaggtt tgaaatcaat tgatagaaca gtagacaggc 300
atgtaattgc agtttccact gtctgttcaa atgtccaatc aaatttcttc ttcacttttt 360
tttcaaggaa gctggttgac tcagtttgtt taactcccgc tgcagtggct ttaaacccac 420
agtagtaacc tgcaggatca cacttatata cctgagggcc ttgctcttca tctataccaa 480
ttaaaatcat acaacaacca agaggcctca tttcagcatt ctgtgtgtag acctgagaaa 540
tatcggcaat tcttttacac agcatgtcca caggaatctc atagccatac ttggatttcc 600
agttagctgc ctcatagccg tgcccttctg tacctgggat ctgctgtcag ctgcattccg 660
gtcatcacac aaccaatggt ttcagttatc ttggaataag tgaggtcact gngctggaat 720
nccaataatt tggcaggnac ctttctttct ggggngacaa ttactggccc agtcttttcc 780
tttggacagn tactggaggt aagggccacc ctgggttaat agccctttaa aggcntaatc 840

<210> 341
<211> 793
<212> DNA
<213> Homo Sapiens

<400> 341
cactttaatt tctttattca tnaatagtat ccgaaaagga agaatcagga gttacaaaaa 60
caagttaaat gcaatataga agcctactaa atacaaatac aagttcacaa acacatatgc 120
aacagaaact tgtttanatt gtttcttgaa gtttgactac ttaaaaacat aggngtaaag 180
gaaagacatt canactggtc cncgngggct tgntagcagg cagaggaacc ctgctttcca 240
aaaactgnta tagtccanan tcncggcatg ngggaatgnt tccatggacn ctggatctta 300
acagatgcta tagggtttac aaaactacnc acncagagaa agcccaagga agcctgcagg 360
ctaagcccta tgcttttaga gggctgaagg aaccaaacct agtttaatcc tgtttgnttg 420
ctccatgcaa aactttttgg aaactccccc agactaggct ttttancagn nttccattga 480
atggggcnnc aaancnttgg gaattttacg gntnaaancn aaagntngcc ttntttnccc 540
ccgaaagctt tgaaaaactt ttcagngggn atnggggaat ttggnttntt ggggnngttc 600
aattgttncc ngggtaaaaa ganacccttg gggaggnaaa cccctgngtt tnaannggcc 660
ttaggggaaa naacccntgg gggtntcntt ggnnttttaa caaaattggg gggncntttt 720
ggnccttcct cccaaaaggg ggcccanggn ctgnggaaaa aacctttggg antaaggggg 780
gncccnnctt gga 793

<210> 342
<211> 906
<212> DNA

<213> Homo Sapiens

<400> 342
ccaacatgtc ccgtggttcc agcgccggtt ttgaccgcca cattaccatt ttttcacccg 60
agggtcggct ctaccaagta gaatatgctt ttaaggctat taaccagggt ggccttacat 120
cagtagctgt cagagggaaa gactgtgcag taattgtcac acagaagaaa gtacctgaca 180
aattattgga ttccagcaca gtgactcact tattcaagat aactgaaaac attggttgtg 240
tgatgaccgg aatgacagct gacagcagat cccaggtaca gagggcacgc tatgaggcag 300
ctaactggaa atacaagtat ggctatgaga ttcctgtgga catgctgtgt aaaagaattg 360
ccgatatttc tcaggtctac acacagaatg ctgaaatgag gcctcttggt tgttgtatga 420
ttttaattgg tatagatgaa gagcaaggcc ctcaggtata taagtgtgat cctgcaggtt 480
actactgtgg gtttaaagcc actgcagcgg gagttaaaca aactgagtca accagcttcc 540
ttgaaaaaaa agtgaagaag aaatttgatt ggacatttga acagacagtg gaaactgcaa 600
ttacatgcct gtctactgtt ctatcaattg atttcaaacc ttcagaaata gaagttggag 660
tagtgacagt tgaaaatcct aaattcagga ttnttacaga agcagagatt gatgctcacc 720
ttgttgctct agcagagaga gactaaacat tgtcgttagt ttaccagatc cgtgatgcca 780
cttacctgtg tgtttggtaa caacaaacca acatcatgga ggtccctgga ttgaaaaagg 840
agcctctccc actcctccta ccaccgaagt ggttaggact ctatataaat aaaacaaggc 900
ttttgg 906

<210> 343
<211> 875
<212> DNA
<213> Homo Sapiens

<400> 343
gcaaggcaat tgagcgtgga acaggaaatg acaattatag aacaacggga attgctacaa 60
tcgaggtgtt tttaccacca agactaaaaa aagataggaa aaacttgttg gagacccgat 120
tgcacatcac tggcagagaa ctgaggtcca aaatagctga aacctttgga cttcaagaaa 180
attatatcaa aattgtcata aataagaagc aactacaact agggaaaacc cttgaagaac 240
aaggcgtggc tcacaatgtg aaagcgatgg tgcttgaact aaaacaatct gaagaggacg 300
cgaggaaaaa cttccagtta gaggaagagg agcaaaatga ggccaaactc aaagaaaaac 360
aaattcagag gaccaagaga ggactagaaa tactggcaaa gagagcagca gagacagtgg 420
tggatccaga aatgacaccg tacttagaca tagctaacca gacaggcaga tcaatcagaa 480
ttcccccatc agaaagaaaa gcccttatgt tagctatggg atatcatgag aagggcagag 540
ctttcctgaa aagaaaagaa tatggaatag ccttgccatg tctgttggac gctgacaaat 600
atttctgtga gtgttgcaga gagctgctgg acacagtgga taactatgcc cgtcttcagc 660
tggatatagt gtggtgttac tttcgcctgg aacagctgga atgccttgat gatgcagaaa 720
aaaaattaac ttggnccaga aatgctttaa aaattggtcc ggagaaatcn tcgaaactgg 780
tccccntaaa nggaattgtg gggaaaagag aangtctggt tctaagactn tacttacttt 840
nagggatccg aacttttcca gggggaatga tgtaa 875

<210> 344
<211> 629
<212> DNA
<213> Homo Sapiens

<400> 344
   atatttccca ccttttattt ccatcggtat catccgttta aaaagaatga caagaagatt 60
   cccatcagtc caaactggac cacccacact ttgaaaaagt tggagcattt cagccggctc 120
   cgcatgatcc atcctgtctt cagtcagtgc cttctggaag ggagggaaag tcttggatgc 180
   acctggcact caatccactc ggcacctggc tgctgctgcg gtcctggggc tggaaggaac 240
   tcccactggg cacacatcta cagaggagtg cgtggcgcag tgaggacggt tactgctgga 300
   gccgacacac agcgaactac atacttttag aaagagcctc tgtcacatgg ctagaacaac 360
   aacaacaaca aagaaaaccc acaaaaaacc tggagaaaat atatctaaat ctctgatagg 420
   tctcttagct agcagtgagt tcagtatgac agcacagagt ctaaaaatat taattaaaaa 480
   taaattgctt tggttagcat ttaaaccttt cccattcaat agaagatttc tgtaatgagg 540
   aatgctgaat atatataaag cctgccactc aatctttgaa tttcnggggg cgcaatttta 600
   ctgaactaag anccctaaaa caactggcg 629

<210> 345
<211> 724
<212> DNA
<213> Homo Sapiens

<400> 345
cttgggtggt tattttnctt ttctgngtcc ttccccanca gcagttggaa ttttcttttg 60
aacacaaagt aaattaatgt tnatactgnt ttttcacctg agtcatgtaa aaggtgactc 120
ctttcatttt aaaaagttat atttaatttt tgggggcctt aattaaaatt taacatttaa 180
ccatgngtnn tttttttgta aacagtctac atgtcaacaa atggataagg gttaacaaag 240
gcaaatnctg acttcatttg tgttttaaac acgattatat gaatttttct tttttaatta 300
aaaaaatgac ataaaaccat tcatataggt cctcttctct caactgcttt gagatatagc 360
tttaaatatg ggtagatcaa gacaagtaat gttggnaatc tcttatcttg catagaaaag 420
aaaaaaataa aggaacttat ttccttccta aggtctcagc tagtttctta ngtcttttct 480
tcagctccaa tggaaattnc tcatagcact tcttacagac tggcttcatg tcaaactcca 540
caaacttatt cttgantgtt aatttagtgt tgcaggtana acaggcaaag cagttcacgc 600
accaggcctt attaagagca gagaccccca tcaccttcta taacacgatt gcagtgggaa 660
gcaaacatca ccaaatagct gggttatagn gagtttcaca atatgcccag gcctttcctt 720
tcaa 724

<210> 346
<211> 907
<212> DNA
<213> Homo Sapiens

<400> 346
agagcgaaat tttaccactg agcaagtgac tgccatgctt ttgtccaaac tgaaggagac 60
agccgaaagt gttcttaaga agcctgtagt tgactgtgtt gtttcggttc cttgtttcta 120
tactgatgca gaaagacgat cagtgatgga tgcaacacag attgctggtc ttaattgctt 180
gcgattaatg aatgaaacca ctgcagttgc tcttgcatat ggaatctata agcaggatct 240
tcctgcctta gaagagaaac caagaaatgt agtttttgta gacatgggcc actctgctta 300
tcaagtttct gtatgtgcat ttaatagagg aaaactgaaa gttctggcca ctgcatttga 360
cacgacattg ggaggtagaa aatttgatga agtgttagta aatcacttct gtgaagaatt 420
tgggaagaaa tacaagctag acattaagtc caaaatccgt gcattattac gactctctca 480
ggagtgtgag aaactcaaga aattgatgag tgcaaatgct tcagatctcc ctttgagcat 540
tgaatgtttt atgaatgatg ttgatgtatc tggaactatg aatagaggca aatttctgga 600
gatgtgcaat gatctcttag ctagagtgga gccaccactt cgtagtgttt tggaacaaaa 660
ccaagttaaa gaaagaagat atttatgcag tggagatagt tggtggtgct acacgaatcc 720
ctgcggtaaa aggagaagat cagcaaaatt tttcggtaaa gaacttagta caaccnttaa 780
atgctgatga aactgcactc gaggctgggc cattgcantg ggccatctta tcgcctgctt 840
tcaaagtccg agaantttct atcactgatg tagtaccata tccatatctc tgaaaaggga 900
atcttcc 907

<210> 347
<211> 711
<212> DNA
<213> Homo Sapiens

<400> 347
ataatagnct gttttaatan aaacaagngt tggaatcaat caatgnccat ttcaggaagc 60

ttnttgtctg aatccgaagg cncagctgng tctgtaccct gctcancagc ctgggggcct 120
gggttgtctc cttgnccatc cactggtcca ttctgctctg catttttttg ttcctntttt 180
ggaggttcca ctttgggttt gggctttgaa attatagggc tacaagtact tgncagctcc 240
ttaattttag cttcaatctc ttttgacttg acaactggat ccatggncaa actntgcttg 300
ttctgcaaat ttagcttggt attcatccac tccattgctt catttgggct tttttctacc 360
tttgtcatgt cagcagcatc cgaatgatca tactggtcct ccttgntttt gaaagagctg 420
attattttca tatactgntg aatctgnttc cctagttctt caaataattt tggtcgttct 480
tnaaattcct ggaaacgtat nttaataggt tgacctaaat tttttaattc agccaactta 540
tcaacataaa cttgctttgg ctggcttctc catcctcata caaccaattt tcagtatctt 600
ccagtttcaa agtaaaactg ttacgancat nttcactnnc aaacttctca tattcnccac 660
taaagcttgg ctctcatttc ataccccata tttcctccca ctggggtctt a 711

<210> 348
<211> 862
<212> DNA
<213> Homo Sapiens

<400> 348
cttgagcctc atgtaaccgg cgctttagat caccaatctc ctcttgggct tcagatttaa 60
tgtcatttgc aatgactact gcagtctgga gatcagcctg aaactgccgc cattccgcag 120
attcttcccg aagtcttctg tggagtgtct ttatttctct ttccatgtcg tgcttttggt 180
cctggagttt tttaactgta ttctctagat cagaaataat gaggttgtca tgaagtttca 240
cagcacgatg ttgttctact tcatcttcaa gttcaaagat ggtttctttc atgtcactcc 300
tttctgtttc tttttcatcc aggtctgatc ttaatttttc taacgtcata ttcaaatctt 360
caatttgttt cttagcttct tcttggaagg ctcggtattc atcctctacc ttagcaatgg 420
catcctgtaa tcgattggca tcatttcggg tatgagccag atcttcctgc aagctactag 480
ccaaagtctc tgctttttct ttgtccagcc tgacactctc caggaggtcc tgaatatcag 540
atttgnctcc agagttatgg atagaataca gctctgccac tttctgcttt tcattctcca 600
gctgagcctt caggcgattc atctctatct ggtcactggc cactgnggct ttgnattcct 660
ctaacgtggc tgncaaggct gcttttcctt tctgctcnac tcaaataaat tcgctccata 720
tgggnggact ggcgttcctt tggagtgccc cctatcattt cttggngctt tccttantgg 780
ccttgggttc tggccatttt tccaaagtat tggctttaaa atggctggct tgggacnccc 840
aaggaaagct ggttcccggt tc 862

<210> 349
<211> 832
<212> DNA
<213> Homo Sapiens

<400> 349
aagactttcc tacatcagtt ttatttaaaa cacaaacaag tatttctctt tctgtaaggg 60
caaatggttc aaataatgcg gaacacgaaa cattgactaa tacaagtgct ttaaatatga 120
aacaaaatta ttttttaaaa aagcaaaaga ataaagaata tatacaaaag ggacctggaa 180
tctgtaagct gattccaaaa acgaaataag tagaaaatcc atggtgaaac ctgaacattc 240
tacctctgct ttggagaagg gctatcatac aacattcagt cagctgaaga tggattggta 300
gaggtgtgtc tatacataaa cttcagtcat ttttgcttgt gcagaatcat cccaatcttc 360
ccaagactga atgggcagtc ctgtggcttt cttccttttc catattccca acaaggctac 420
gtgaagttca actcttgatg agccgcttac aacagcagtt ccttaggagc caacatgaca 480
ggtgggtcag atttccctat gagaaacaaa actggccacc tacagcaaaa tatcaaaatg 540
ggtaagtcct tccttcctct tcctcctgat tatatacaac atatctcctt tcaagactat 600
tatttccatc atgcttattc cttcacaaat ctaaaccttg aggtgatatg aaggaaacca 660
acatcangaa aagaaaactc aattcagaaa tgaagaaaac tggcaggtat acaatacacc 720
cccagaacat ctcaatatcc ctggccagta caattcaagt gnactgggta caggcccata 780
ggattaaata attgggcagc ttgggaataa agctcatttt tttnccctca gg 832

<210> 350
<211> 782
<212> DNA
<213> Homo Sapiens

<400> 350
   ccnacatcag tttnattnaa aacacaanca agnatttctn tttcngnang gncaaagggt 60
   tnaaanaang cgnancacna ancatngact aatncaaggg cttnaaatat gaancaaaat 120
   nattttttaa aaaagcaaaa naataaanaa tatatncaaa ngggaccngn aatcngnaag 180
   cngatnccaa aaccnaaata agtaaaaaan ccanggggaa nccngancat tcnacctnng 240
   nttngnaaaa gggctatcat ncaacattca gncagntgaa nanggatngg nanaggnggg 300
   ncnatncata ancttcagnc attttngctn gggcaaaatc atcccaatnt tcccaanact 360
   gaanggncag cccnggggct ttcttccttt nccanattcc caacanggnt acgngaagtt 420
   caactntnga nganccgttt acaacagcag ttccttagga nccancatga caggggggnc 480
   aaatttccct atgagaanca aaacnggcca cctacagcaa aatatcaaaa ggggnaagnc 540
   cttccttcct cttcctccng attatatnca ccatatctcc tttcangact atnatttcca 600
   tcaggctnat tccttcacaa atntaaacct tgaggggata tgaaggaacc caacttcngg 660
   aaangaaaac tcaattcana aattgaagaa acctggcagg tatacaatac ccccccaggn 720
   catntcaana tccctggcac aagnnccaat tcagggncct ggtaccagcc ccatagaana 780
   aa 782

<210> 351
<211> 775
<212> DNA
<213> Homo Sapiens

<400> 351
ggcaaggcgg ctgctgcgaa tcaccaaaag aacagggatg aaagaagaga agaaccttca 60
ggaaggaaat gaagttgatt ctcagagcag tattagaaca gaagctaaag aggcttcagg 120
tgagaccaca ggagttgaca tcactaaaat tcaagtcaag agatgtgaga ccatgagaga 180
gaagcacatg cagaaacagc aggagaggga aaaatcagtc ttgacacctc ttcggggaga 240
tgtagcatct tgcaataccc aagtggcaga gaaaccagtg ctcactgctg tgccaggaat 300
cacacggcac ctgaccaagc ggcttcccac aaagtcatcc cagaaggtgg aggtagaaac 360
ctcagggatt ggagactcat tattgaatgt gaaatgtgca gcacagacct tggaaaaaag 420
gggtaaagct aaacccaaag tgaacgtgaa gccatctgtg gttaaagttg tgtcatcccc 480
caaattggcc ccaaaacgta aggcagtgga gatgcacgct gctgtcattg ccgctgtgaa 540
gccactcagc tccagcagtg tcctacagga acccccagcc aaaaaggcag ctgtggctgt 600
tgtcccgctt gtctctgagg acaaatcagt cactgtgcct gaagcagaaa atcctagaga 660
cagtctttgt gcttgncttc aacccagtcc ttnttcagat tccttacccc cagaggtgtc 720
ttggnccttt cttcatncca aatggagcct tgaaaaactt cggccgactt agctt 775

<210> 352
<211> 865
<212> DNA
<213> Homo Sapiens

<400> 352
   cctacatcag ttttatttaa aacacaaaca agtatttctc tttctgtaag ggcaaatggt 60
   tcaaataatg cggaacacga aacattgact aatacaagtg ctttaaatat gaaacaaaat 120
   tattttttaa aaaagcaaaa gaataaagaa tatatacaaa agggacctgg aatctgtaag 180
   gtgattccaa aaacgaaata agtagaaaat ccatggtgaa acctgaacat tctacctctg 240
   ctttggagaa gggctatcat acaacattca gtcagctgaa gatggattgg tagaggtgtg 300
   tctatacata aacttcagtc atttttgctt gtgcagaatc atcccaatct tcccaagact 360
   gaatgggcag tcctgtggct ttcttccttt tccatattcc caacaaggct acgtgaagtt 420
   caactcttga tgagccgctt acaacagcag ttccttagga gccaacatga caggtgggtc 480
   agatttccct atgagaaaca aaactggcca cctacagcaa aatatcaaaa tgggtaagtc 540
   cttccttcct cttcctcctg attatataca acatatctcc tttcaagact attatttcca 600
   tcatgcttaa tncttccaaa tctaaacctt gaggngatat tgaanggaaa cccaccttca 660
   nggaaaagaa aacctcaatt tcagaaatgg aagaaaaact ggcagggtat accaatacac 720
   ccccccagag catttttaaa atatccctgg ncacaagtnc caattcaagg gnacctggtt 780
   ccggnccata gaataaaana ntgggcactt tggaaaaaag cnccattttt ttcccttcag 840
   ggggggttaa aagggccccc aaacc 865

<210> 353
<211> 875
<212> DNA
<213> Homo Sapiens

<400> 353
gactttccta catcagtttt atttaaaaca caaacaagta tttctctttc tgtaagggca 60
aatggttcaa ataatgcgga acacgaaaca ttgactaata caagtgcttt aaatatgaaa 120
caaaattatt ttttaaaaaa gcaaaagaat aaagaatata tacaaaaggg acctggaatc 180
tgtaagctga ttccaaaaac gaaataagta gaaaatccat ggtgaaacct gaacattcta 240
cctctgcttt ggagaagggc tatcatacaa cattcagtca gctgaagatg gattggtaga 300
ggtgtgtcta tacataaact tcagtcattt ttgcttgtgc agaatcatcc caatcttccc 360
aagactgaat gggcagtcct gtggctttct tccttttcca tattcccaac aaggctacgt 420
gaagttcaac tcttgatgag ccgcttacaa cagcagttcc ttaggagcca acatgacagg 480
tgggtcagat ttccctatga gaaacaaaac tggccaccta cagcaaaata tcaaaatggg 540
gtaagtcctt ccttcctctt cctcctgatt atatacaaca tatctccttt caagactatt 600
atttccatca tgcttattcc ttccaaatct aaacccttga ggtgatatga aggaaaccaa 660
catcaagaaa aagaaaactc aattcagaaa atgaagaaaa ctggcaggga tacaatacac 720
ccccagagca tcttcaatat cccctgggca cagtncccaa ttcagggact gggtacaggc 780
ccataagaat naaataattg ggcagctttg gaataaagcc tcattttttt cccttcaggn 840
gggttaaagg ggccccccaa accaaaaact ggggc 875

<210> 354
<211> 705
<212> DNA
<213> Homo Sapiens

<400> 354
gactttccta catcagtttt atttaaaaca canacangta tttctctttc tgtaagggca 60
aatggttcaa ataatgcgga acacgaaaca ttgactaata caagngcttt aaatatgaaa 120
caaaattatt ttttaaaaaa gcaaaagaat aaagaatata tacaaaaggg acctggaatn 180
tgtaaggtga ttccaaaaac gaaataagta gaaaatccat ggtgaaacct gaacattcta 240
cctctgcttt ggagaagggc tatcatacaa cattcagtca gctgaagatg gattggtaga 300
ggtgtgtcta tacataaact tcagtcattt ttgcttgtgc agaatcatcc caatcttccc 360
aagactgaat gggcagtcct gtggctttct tccttttcca tattcccaac aaggctacgt 420
gaagttcaac tcttgatgag ccgcttacaa cagcagttcc ttaggagcca acatgacagg 480
tgggtcagat ttccctatga gaaacaaaac tggccaccta cagcaaaata tcaaaatggg 540
taagnccttc cttcctcttc ctnctgatta tatacnncat atctcctttc aagactatta 600
tttccatcat gcttattcct tccaaatcta aaccttgagg ngatatgaan ggaaaccaca 660
tcaggaaaag gaaactcaat tccgaaatga ngaaaactgg caggt 705

<210> 355
<211> 862
<212> DNA
<213> Homo Sapiens

<400> 355

ccatcagtga gagcgagctg agcgccagcg ccactgagct gctgcaggac tacatgctga 60
cgctgcgcac caagctgtca tcacaggaga tccagcagtt tgcagcactg ctgcacgagt 120
accgcaatgg ggcctctatc cacgagttct gcatcaacct gcggcagctc tacggggaca 180
gccgcaagtt cctgctgctt ggtctgaggc ccttcatccc tgagaaggac agccagcact 240
tcgagaactt cctggagacc attggcgtga aggatggccg cggcatcatc actgacagct 300
ttggcaggca ccggcgggcc ctgagcacca catccagttc caccaccaat gggaacaggg 360
ccacgggcag ctctgatgac cggtcggcac cctcagaggg ggatgagtgg gaccgcatga 420
tctcggacat cagcagcgac attgaggcgc tgggctgcag catggaccag gactcagcat 480
gatggacagt ggatgggggg gcacccacac cttccgcgca gtcgtcatag gccttcccag 540
aaggagctgc ccagacctgc gtgtcagccc ttggtggtgg ccaggganag gcgcccggtg 600
cagatggccc cgggcggccc aggtcctnta ctgtgaagga gcagggagct gccgagggac 660
acgagcctca gtgcggggtg gaaggctctt tgccttgtcc accagggntc agccaagccc 720
tgcagtgtgt ccccgctcgg ggagggcccg gccgagcggg cagggagagc cagtcctgtc 780
ggctgggccc ttggacggct gtcagttttg cacatgatgt tcctattgta actntcagag 840
accttaaaaa gaagtttact gc 862

<210> 356
<211> 750
<212> DNA
<213> Homo Sapiens

<400> 356
ccatcagtga gagcgagctg agcgccagcg ccactgagct gctgcaggac tacatgctga 60
cgctgcgcac caagctgtca tcacaggaga tccagcagtt tgcagcactg ctgcacgagt 120
accgcaatgg ggcctctatc cacgagttct gcatcaacct gcggcagctc tacggggaca 180
gccgcaagtt cctgctgctt ggtctgaggc ccttcatccc tgagaaggac agccagcact 240
tcgagaactt cctggagacc attggcgtga aggatggccg cggcatcatc actgacagct 300
ttggcaggca ccggcgggcc ctgagcacca catccagttc caccaccaat gggaacaggg 360
ccacgggcag ctctgatgac cggtcggcac cctcagaggg ggatgagtgg gaccgcatga 420
tctcggacat cagcagcgac attgaggcgc tgggctgcag catggaccag gactcagcat 480
gatggacagt ggatgggggg gcacccacac cttccgcgca gtcgtcatag gccttcccag 540
aaggagctgc cagacctgcg tgtcaaccct tggtggtggc cagggagagg cgcccggtgc 600
agatggcccc gggccggccc aagtcctcta ctgtgaagga acagggagct tgccgangga 660
cacgaacctc aatgccgggg tggaangctc tttggcttgt ccaccaaggc ttagcccagc 720
ccttgcaatg nggccccgct tcggggaagg 750

<210> 357
<211> 725
<212> DNA
<213> Homo Sapiens

<400> 357
gcagtaaact tctttttaag gtctctgana gttacaatag gaacatcatg tgcaaaactg 60
acagccgtcc aagggcccag ccgacaggac tggctctccc tgcccgctcg gccgggccct 120
ccccgagcgg ggacacactg cagggcttgg ctganccctg gtggacaagg caaagagcct 180
tccaccccgc actgaggctc gtgtccctcg gcagctccct gctccttcac agtanaggac 240
ctgggccgcc cggggccatc tgcaccgggc gcctntccct ggccaccacc aagggctgac 300
acgcaggtct gggcagctcc ttctgggaag gcctatgacg actgcgcgga aggtgtgggt 360
gcccccccat ccactgtcca tcatgctgag tcctggtcca tgctgcagcc cagcgcctca 420
atgtcgctgc tgatgtccga natcatgcgg tcccactcat ccccctctga gggtgccgac 480
cggtcatcag agctgcccgt ggccctgttc ccattggtgg tggaactgga tgtggtgctc 540
agggcccgcc ggtgcctgcc aaagctgtca gtgatgatgc cgcggccatc cttnacgcca 600
atggtctnca ggaagttctc gaantgctgg ctgncctttn tcaggatgaa anggccttan 660
accaagcagc anggaacttg cggnttntcc ccgaaaanct tgccncaggt tgatgcaaaa 720
acttc 725

<210> 358
<211> 813
<212> DNA
<213> Homo Sapiens

<400> 358
aaggcgacag ctgcccattc cgtcactgtg aagctgcaat aggaaatgaa actgtttgca 60
cattatggca agaagggcgc tgttttcgac aggtgtgcag gtttcggcac atggagattg 120
ataaaaaacg cagtgaaatt ccttgttatt gggaaaatca gccaacagga tgtcaaaaat 180
taaactgcgc tttccatcac aatagaggac gatatgttga tggccttttc ctacctccga 240
gcaaaactgt gttgcccact gtgcctgagt caccagaaga ggaagtgaag gctagccaac 300
tttcagttca gcagaacaaa ttgtctgtcc agtccaatcc ttcccctcag ctgcggagcg 360
ttatgaaagt agaaagttcc gaaaatgttc ctagccccac gcatccacca gttgtaatta 420
atgctgcaga tgatgatgaa gatgatgatg atcagttttc tgaggaaggt gatgaaacca 480
aaacacctac cctgcaacca actcctgaag ttcacaatgg attacgagtg acttctgtcc 540
ggaaacctgc agtcaatata aagcaaggtg aatgtttgaa ttttggaata aaaactcttg 600
aggaaattaa gtcaaagaaa atgaaggaaa aatctaagaa gcaaggtgag ggttcttcag 660
gagtttccag tcttttactt caccccttgag ccccgntcca ngtcctgaaa aagaaaatgt 720
caaggactgt ggtgangaca gtactntttt caccaaccaa ggagaagaac ccttggttag 780
atgagtctta ctgagagact ggggaaacca aaa 813

<210> 359
<211> 756
<212> DNA
<213> Homo Sapiens

<400> 359
cagcagagga gaggcagagg ataaaagagg aagagaaaag ggcagcagag gagaggcaaa 60
gggccagggc agaggaggaa gagaaggcta aggtagaaga gcagaaacgt aacaagcagc 120
tagaagagaa aaaacgtgcc atgcaagaga caaagataaa aggggaaaag gtagaacaga 180
aaatagaagg gaaatgggta aatgaaaaga aagcacaaga agataaactt cagacagctg 240
tcctaaagaa acagggagaa gagaagggaa ctaaagtgca agctaaaaga gaaaagctcc 300
aagaagacaa gcctaccttc aaaaaagaag agatcaaaga tgaaaagatt aaaaaggaca 360
aagaacccaa agaagaagtt aagagcttca tggatcgaaa gaagggattt acagaagtta 420
agtcgcagaa tggagaattc atgacccaca aacttaaaca tactgagaat actttcagcc 480
gccctggagg gagggccagc gtggacacca aggaggctga gggcgccccc caggtggaag 540
ccggcaaaag gctggaggag cttcgtcgtc gtcgcgggga gaccgagagc cgaagagttc 600
gagaagctca aacagaagca gcaggaggcg gctttggagc tggaggaact caaggaaaaa 660
ganggaggag agaaggaagg tcctgganga ggaagagcag aggaaggaac aggaggaaag 720
ccgatcggaa aaccttcaag aggaggaaga agaaga 756

<210> 360
<211> 706
<212> DNA
<213> Homo Sapiens

<400> 360
aatttcttcc atgctttatt ataaagngca naaacaacat gacttctgta tttaaaaaaa 60
caaaaactac ggttcatttt tctagatact gcacacattc cgcaggcaat tttaaacttg 120
gatcttctgt tgacttcana tgnggttggt atcactgctc aaatacagag ttatgatgat 180
cagtanaaaa gtctntattt cacagcatgg gtttctttan aaacaggctc ctgngcaaag 240
gcagtacttt taccatgaac atctntanac tgggattatt aaatatagng ataatataca 300
tgggtttact gggatattga aaaataaaag ataatgaacc caatttagta aatcaacata 360
aatacaaaac agagcgaatt agccctntac aactgagctc gtcctgcgtc ttgagcttgg 420
gttctttctg gaactgtctc aaaccttagt gggggaagtg accttatcca canattgctt 480

ttcccagagg ttccgcttgc tggataccgt ctcctggnct caagtcanaa ggtttgggag 540
caggtgactt gtttccatct ggggttttaa gttagccatt cattgatgcg gctagaaacc 600
cctaccttta agccagcagt ttnccttatt tgggggngcc ctgctgcant ggggggatga 660
aaacncattt cctttntcca catactcttg aaggttgcgg tacacc 706

<210> 361
<211> 726
<212> DNA
<213> Homo Sapiens

<400> 361
gccatgctac gcgcgctgag ccgcctgggc gcggggaccc cgtgcaggcc ccgggcccnt 60
ctgntgctgc cagcgcgcgg ccgcaagacc cgccacgacc cgctggccaa atccaagatc 120
gagcgagtga acatgccgcc cgcggtggac cctgcggagt tcttcgtgct gatggagcgt 180
taccagcact accgncagac cgtgcgcgcc ctcaggatgg anttcgtgtc cgaggtgcat 240
aggaaggtgc acgaggcccg agccggngtt ctggcgganc gcaaggccct gaaggacgcc 300
gccgagcacc gcnagctgat ggcctggaac caggcggaga accggcggct gnacgagctg 360
cggatagcga ggctgcggca ggagganc gg nagcaggagc agtgncaggc gttggagcan 420
gcccgcaagg ccgaagaggt gcangcctgg gcgcagcgca aggagcgnga antgctgcag 480
ctgcagnaag aggtgaaaaa cttcatcacc cgagagaacc tggaggcacg ggtggaagca 540
gcattggact cccggaagaa ctacaactgg gccatcacca gagaggggct ggtggtcagg 600
ccacaacgca nggacttcta agggcccagt aaggacagtg cccggcaggg accatgtatg 660
tatcatggcg gaagagttgc ccttgactgg aattaaagca attggtgttg cttatgagga 720
aaggtt 726

<210> 362
<211> 747
<212> DNA
<213> Homo Sapiens

<400> 362
gcaggaagga attccattna ttggggatgc attttcacaa tatatgttna tnggagcgat 60
ccattatcag ggaaaagtat caagggttna taaaattttt aggaanggca nattcacaga 120
acatgctagt cagctngcag ttttacctcg taaagatanc aganaattat agncaaacca 180
gtaaacangg aattnacttt tcaaaagatt aaatccaaac tgancaaaat tntaccctaa 240
aacttactcc atccaaatat tggaataaaa gtcagcaggg atncattctn ttctgaactt 300
tanattttnt anaaaaatat gtaatagnga tcaggaggag ctnttgttca aaagtncaac 360
aaagcaangt taccttacca taggccttaa ttcaaacttt gatccatttc actccaanga 420
cgggagtcaa ngctacctgg gacacttgta tttgtaaatt ctgatttagc ttatngtaaa 480
cttgggccta ctttgncatg agggtttgac ttcngcattn ttcggggntt tccttccttt 540
ggcttaggtt tgctaaagct agaanattca attgctcttt acagacttat gaggaanata 600
gactttgtaa cgcanatgtc acttttaatg ccagccctgc cctggttagc ncttctggag 660
gaatactgca gataagaaaa atagttattt gggaggctcc ctcagngggg tanggaattg 720
gggactaacc ncaattttng gttaaag 747

<210> 363
<211> 1227
<212> DNA
<213> Homo Sapiens

<400> 363
gtgaagaccc tgagtccgtt tatttgccgg taggagcagg ctccaacatt ttgtctccat 60
caaacgttga ctgggaagta gaaacagata attctgattt accagcaggt ggagacatag 120
gaccaccaaa tggtgccagc aaggaaatac cagaattgga agaagaaaaa acaattccta 180
ccaaagagcc tgagcagata aaatcagaat acaaggaaga aagatgcaca gagaagaatg 240

aagatcgtca tgcactacac atggattaca tacttgtaaa ccgtgaagaa aattcacact 300
caaagccaga gacctgtgaa gaaagagaaa gcatagctga attagaattg tatgtaggtt 360
ccaaagaaac agggctgcag ggaactcagt tagcaagctt cccagacaca tgtcagccag 420
cctccttaaa tgaaagaaaa ggtctctctg cagagaaaat gtcttctaaa ggcgatacga 480
gatcatcttt tgaaagccct gcacaagacc agagttggat gttcttgggc catagtgagg 540
ttggtgatcc atcactggat gccaggactc cagggcctgg gtggtctggc aagactgtgg 600
agccgttctc tgaactcggc ttgggtgagg gtccccagct gcagattctg gaagaaatga 660
agcctctaga atctttagca ctagaggaag cctctggtcc agtcagccaa tcacagaaga 720
gtaagagccg aggcagggct ggcccggatg cagttaccca tgacagtgaa tgggaaatgc 780
tttcaccaca gcctgttcag aaaaacatga tccctgacac ggaaatggag gaggagacag 840
agttccttga gctcggaacc aggatatcaa gaccaaatgg actactgtca gaggatgtag 900
gaatggacat cccctttgaa gagggcgtgc tgagtcccag tgctgcagac atgaggcctg 960
aacctcctaa ttctctggat cttaatgaca ctcatcctcg gagaatcaag ctcacagccc 1020
caaatatcaa tctttctctg gaccaaagtg aaggatctat tctctctgat gataactttg 1080
gacagtccag atgaaattga catcaatgtg gatgaacttg atacccccga tgaagcagat 1140
tcttttgagt accctggccc atgaagaatc ccacagccac aaagattctg gcccaagaag 1200
tcagagtcta tttcnggaat ataccgg 1227

<210> 364
<211> 831
<212> DNA
<213> Homo Sapiens

<400> 364
ctgacatcta caccgaggtc cgcgagctgg tgagcctcaa gcaggagcag caggctttca 60
aggaggcggc ccgacacgga gcggctcgcc ctgcaggccc tcacggagaa gcttctcagg 120
tctgaggagt ccgtctcccg cctcccggag gagatccgga gactggagga agagctccgc 180
cagctgaagt ccgattccca cgggccgaag gaggacggag gcttcagaca ctcggaagcc 240
tttgaggcac tccagcaaaa gagtcaggga ctggactcca ggctccagca cgtggaggat 300
ggggtgctct ccatgcaggt ggcttctgcg cgccagaccg agagcctgga gtccctcctg 360
tccaagagcc aggagcacga gcagcgcctg gccgccctgc aggggcgcct ggaaggcctc 420
gggtcctcag aggcagacca ggatggcctg gccagcacgg tgaggagcct gggcgagacc 480
cagctggtgc tctacggtga cgtggaggag ctgaagagga gtgtgggcga gctccccagc 540
acccgtggaa tcactccaga aggtgcagga acaggtgcac acgctgctca gtcaggacca 600
agcccaggcc cgccgtctgc cttctcagga ctttctggac agactttctt ctctagacaa 660
cctgaaagcc tcagtcaggc cagtggaagc cggacttgaa aatgctcaag aactgctgtg 720
gacaagttgg gtgcataact cggtcaaaat tagaaaccaa cgagnacaat tttggaatca 780
agcccanggt tactagatga ccttggggaa tgatcnggat aggttgtttg t 831

<210> 365
<211> 785
<212> DNA
<213> Homo Sapiens

<400> 365
acttgaaatc tgccagccag acaggatttc tgaggttaat ctgcttctgt taatcctcaa 60
tttaagcctt tatcattttt ctctgactag agacatccat gaaaagccac ctgttattca 120
caggggctgc gcttcaggaa accaaccaaa tgcagaagca gagaacttaa atattgtaaa 180
taagttaact gggcatgaaa atacaatgcc ttggtgttca ggtggtgaca actgctcttt 240
aagaggggac aagaaattgg ggggtagggg acacatggga aaaaaccaca cattttttgg 300
tcatgagaaa ttggacttta aatccgcgcc ctgcacacgc aattcattta gaccttttcg 360
tgaatcttct ccactttcac aaacaaccta tccagatcat tcctcaggtc atctagtaaa 420
cccttggctg attccagatt gttctcgttg gtttctattt tgaccgagta tgcaaccaaa 480
ctgtccacag cagtcctgag cattttcaag tccgcctcca cttggctgac tgaggctttc 540
aggttgtcta gagaagaaag tctgtccagg aagtcctgag gaggcagacg ggcggcctgg 600

gcttggtcct gactgagcag cgtgtgcacc tgctcctgcc ctttctggga gtgattccac 660
ggtgctgggg agctngccca cacttcctct tcagcttctt ccacgtcacc cgtaaaagca 720
cccagctggg tctcgnccaa gcttcntacc gtgctgggcc aggcccatcc tggnctggct 780
tttga 785

<210> 366
<211> 816
<212> DNA
<213> Homo Sapiens

<400> 366
gtcagccagc ctccttaaat gaaagaaaag gtctctctgc agagaaaatg tcttctaaag 60
gcgatacgag atcatctttt gaaagccctg cacaagacca gagttggatg ttcttgggcc 120
atagtgaggt tggtgatcca tcactggatg ccagggactc agggcctggg tggtctggca 180
agactgtgga gccgttctct gaactcggct tgggtgaggg tccccagctg cagattctgg 240
aagaaatgaa gcctctagaa tctttagcac tagaggaagc ctctggtcca gtcagccaat 300
cacagaagag taagagccga ggcagggctg gcccggatgc agttacccat gacagtgaat 360
gggaaatgct ttcaccacag cctgttcaga aaaacatgat ccctgacacg gaaatggagg 420
aggagacaga gttccttgag ctcggaacca ggatatcaag accaaatgga ctactgtcag 480
aggatgtagg aatggacatc ccctttgaag agggcgtgct gagtcccagt gctgcagaca 540
tgaggcctga acctcctaat tctctggatc ttaatgacac tcatcctcgg agaatcaagc 600
tcacagcccc aaatatcaat ctttctctgg accaaagtga aggatctatt ctctctgatg 660
ataactttgg acagtccaga tgaaattgac atcaatgtgg atgaacttga tacccccgat 720
gaagcagatt cttttgagta ccctggccca tgaagaatcc cacagccaca aagattctgg 780
cccaagaagt cagagtctat ttcnggaata taccgg 816

<210> 367
<211> 803
<212> DNA
<213> Homo Sapiens

<400> 367
aaaagaacca tggaagttct cctgaacagg tagtgaggcc aaaagttaga aaactgataa 60
gttcaagcca ggtggaccaa gaaacaggtt ttaataggca tgaggcgaaa caaagaagtg 120
ttcaaagatg gagagaggct ttggaagttg aggaaagtgg ctcagatgac ctcttaataa 180
aatgtgaaga atatgatgga gagcatgact gtatgttctt ggatccacca tactcaagag 240
ttattacaca aagggaaaca gaaaataacc aaatgacatc agaaagtgga gccacagcag 300
gaaggcaaga agtggataac accttttgga atggctgtgg agattattac caactctatg 360
acaaagatga agatagttct gaatgcagtg atggggaatg gtctgcttct ttgcctcatc 420
gattttctgg tacagaaaaa gatcaatcct caagtgatga aagctgggag actctgccag 480
gaaaagatga gaatgaacct gagctacaaa gtgatagcag tggccctgaa gaagaaaacc 540
aagaattatc tcttcaggaa ggggaacaga catccttgga agagggagaa attccttggt 600
tacagtacaa tgaagtcaat gaaagcagca gtgatgaagg gaaatgaacc tgccaatgaa 660
tttgcacagc cagctttcat gttggatggt aacaataacc tggangatga cttccgtgtg 720
aagtgaagac ttagatgtgg attggagcct attttgatgg ctttgcaaat gggcctagga 780
gttgctggaa gctttttcat aag 803

<210> 368
<211> 809
<212> DNA
<213> Homo Sapiens

<400> 368
attagaaatg accaccgagt atattctgtt tattgtttat gatttacaca gaaaatgatg 60
ggctggggtt atagaacaat aaaccaacca ttacatttag acctgggctt ttgaaaaact 120

tgcattccat tttaacaatt cgtatgtatc taacaaatac ataaatccag atcacaaata 180
atcttaagag ttaaacaatt aagaaacaca aagaatacca catagatcta cctttaaata 240
tcagcattca tattataaga aataagaaaa tgttaaaaaa ataaaattag gttaagtcac 300
aacataaaat agagaaataa gataaatgct attttattaa tattcatact tatttctaat 360
ttaccttcat atagtcttaa ctttttcaaa aggatccaag atatgatcaa ataatatttt 420
agtatctgaa cttgccagcc ttagcttata ccagagcttg ttaccatgaa aatcctaaaa 480
cctcaatttt ctttttcttt tttaaaattt aagccaactc ttattcaact tttcttcttc 540
acagcagctg tttatagata gtagggagcc aagaatgaag gacagtaaca gatggaaagc 600
aaaaagtaca acagctatct taagttcagc tctcaacatt gctggttgag tttggaaccc 660
aaaaccctct taacaactgg cagataatag cttaaatctt tacaggccaa ggaagaaata 720
ttttctttgg ggacagctgn tatctagaag aaaacccang ggccctttaa tataggccta 780
aaatattaan gggnggcttt aattttagg 809

<210> 369
<211> 826
<212> DNA
<213> Homo Sapiens

<400> 369
gtgaagaccc tgagtccgtt tatttgccgg taggagcagg ctccaacatt ttgtctccat 60
caaacgttga ctgggaagta gaaacagata attctgattt accagcaggt ggagacatag 120
gaccaccaaa tggtgccagc aaggaaatac cagaattgga agaagaaaaa acaattccta 180
ccaaagagcc tgagcagata aaatcagaat acaaggaaga aagatgcaca gagaagaatg 240
aagatcgtca tgcactacac atggattaca tacttgtaaa ccgtgaagaa aattcacact 300
caaagccaga gacctgtgaa gaaagagaaa gcatagctga attagaattg tatgtaggtt 360
ccaaagaaac agggctgcag ggaactcagt tagcaagctt cccagacaca tgtcagccag 420
cctccttaaa tgaaagaaaa ggtctctctg cagagaaaat gtcttctaaa ggcgatacga 480
gatcatcttt tgaaagccct gcacaagacc agagttggat gttcttgggc catagtgagg 540
ttggtgatcc atcactggat gccagggact cagggcctgg gtggtctggc aagactgtgg 600
agccgttctc tgaactcggc ttgggtgagg gtccccagct gcagattctg gaagaaatga 660
acctctagaa tctttagcac tagangaagc ctntggtcca gtcagcccat cacaggaaga 720
gttagaaccc gaggcanggc tgggcccgga tgcagtaccc cntgacagtg gaatgggnaa 780
tgcttttanc cacagcctgt tcagaaaaac atgatccttg ccccgg 826

<210> 370
<211> 783
<212> DNA
<213> Homo Sapiens

<400> 370
gcagaatcaa tttttatttc tgaattatac agtgaggcta tatagatata ttgtgtcatt 60
aaagactttt atattattaa tctacattat ggagaattta tttaccaaaa cgaagtctaa 120
cagacacttt attctgagca atccaatgca tgatagaaaa acctttagat atataaaaga 180
ttaatttgtg cacatctaaa tgtttctaag ggaacaaact actgaggcat tgtgataaga 240
cgagagttgc aaacatagta ccataactga atatttaaaa ttacatctta acaaaggcta 300
ggagtagtga cttcctcaca cacctcagag aatgtcttag agagtaaccc catagaacat 360
tgtatggctt caacagaaac ttcaggattt tcttccacac tgagctactg ccctcaaaca 420
aactttctca ctccttgaca ctatcttctg tgcaaatttc tgttctttct cttaatcaag 480
gagctttgag aaacaatgct tttgccccaa tgaccccttg gttcccttaa ctacagatct 540
ataggagaaa tgcaaagcag ttcccagaag tcagaaccaa agcaagaatg ttcagagtgc 600
aagagctaga gagctaaatc atgtgaatgg ttacctctgn ctacctatct gcttanggat 660
tatttttcta nggattcatc taggattcta tttaccttgg gggtgaaatg gacatggtag 720
cttttcctta gccccatgcc aattaaaatt naatttgggc ntttaaagaa taattaaaat 780
tgc 783

<210> 371
<211> 793
<212> DNA
<213> Homo Sapiens

<400> 371
ccacactgca ggatctgtct tcttctaaag aaccttctaa ttccctaaac ttacctcaca 60
gtaatgagct gtgttcatcc cttgtgcatc ccgaattgag tgaggtcagt tctaacgttg 120
caccaagcat ccctccagta atgtcaagac ctgttagctc ttcctccatt tccactccct 180
tgcccccaaa tcaaataact gtatttgtca cttccaatcc catcacaact tcagctaaca 240
catcagcagc tttgccaact cacttgcagt ctgcattgat gtcaacagtt gtcacaatgc 300
ccaatgcggg tagcaaggtt atggtttctg agggacagtc agctgctcag tctaatgccc 360
ggcctcagtt cattacacct gtctttatca attcatcctc aataattcag gttatgaaag 420
gatcacagcc aagcacaatt cctgcagccc cactgacaac caactctggc ctgatgcctc 480
cctctgttgc agttgttggc cctttacaca tacctcagaa cataaaattt tcttctgctc 540
ctgtaccgcc taatgccctc tccagtagtc ctgctccaaa catccagaca ggtcgacctt 600
tggtccttag ctcacgagcc acccctgttc agcttccttc ccttcttgna cgtcttctnc 660
agttgccctt ctnatcccct gtgcaacaag tgaaagaatt gaatncagat gangctagcc 720
ctnangtgaa caccttaaca gatcagacac tttttccttt tncagtcaac cccaatgggt 780
tcttcccttt tga 793

<210> 372
<211> 804
<212> DNA
<213> Homo Sapiens

<400> 372
cacattgtac aaatccttag attctcttta ttcactggtc catttctaca acaaatacat 60
ccaaaacact atataataaa attatttaca acatttccaa atgagaagat tgcttttgcc 120
cccactactg ctattcacac acagtacttc cacggcacaa tacattagga gatctaaaaa 180
tgctcaccct gtactctagg ctgcttagga aatgtgaaaa ctagtaacat ttataatggc 240
attagctcct ttcaatacaa gacaacattt tagaaacctt gaacttcaac tcgcaacacc 300
aaaagggctc aacagtcctg ctttccccat tgcactttat gaaacaggtt gcagggacta 360
ggaaaagggc cacattatta aaattactaa ctgtacagaa attgatttaa aaaagtcaca 420
gctcaaaatt gctctttgta aaagtcacac acatttccaa gtatcaagtc gcagtcctgc 480
ttgtttactt ggattttctt cgcttggatt gcaccgcact ggttatgtct ttagtagagc 540
tggaggctga agcaggtcga gaagatcgtt tacgatgtcc attttccaca ctttcagagg 600
ccacagttgg ctcttcagtt cgggagtttc ttcggcctgg gatttggact tttcaactat 660
ctctttgggc tcactgcttt gtccagagac tatggcagca tttacctccg ctttgggctg 720
gcaacagang cctgcaatgc tgngggttga agttcctttt gagactaaat tctggcgacn 780
gggctttgct gggggtaaag ttct 804

<210> 373
<211> 792
<212> DNA
<213> Homo Sapiens

<400> 373
gccggccgcc cgcgcccgcc cgccgctgcc cccagctcga ggaggacatc gcggccaagg 60
agaagttgct gcgggtgtcg gaggacgagc gggaccgggt gctggaggag ctgcacaagg 120
cggaggacag cctcctggcc gccgaagagg ccgccgccaa ggctgaagcc gacgtagctt 180
ctctgaacag acgcatccag ctggttgagg aagagttgga tcgtgcccag gagcgtctgg 240
caacagcttt gcagaagctg gaggaagctg agaaggcagc agatgagagt gagagaggca 300
tgaaagtcat tgagagtcga gcccaaaaag atgaagaaaa aatggaaatt caggagatcc 360
aactgaaaga ggcaaagcac attgctgaag atgccgaccg caaatatgaa gaggtggccc 420

gtaagctggt catcattgag agcgacctgg aacgtgcaga ggagcgggct gagctctcag 480
aaggccaagt ccgacagctg gaagaacaat taagaataat ggatcagacc ttgaaagcat 540
taatggctgc agaggataag tactcgcaga aggaagacag atatgaggaa gagatcaagg 600
tcctttccga caagctgaag gaggctgaga ctcgggctga gttttgcgga aaaggtcagt 660
aactaaantt ggagaaaaag catttgatga cttagaagaa gaaagtggct tcatgcccaa 720
agaagaaaan cttatatgca tcaanatgct ggatcagact ttactggagt taaaccacat 780
gtgaaaaact tc 792

<210> 374
<211> 745
<212> DNA
<213> Homo Sapiens

<400> 374
agccgataac agtagaactc tgaacgtgga ttccactgca atgacactac ctatgtctga 60
tccaactgca tgggccacag caatgaataa tcttggaatg gcaccgctgg gaattgccgg 120
acaaccaatt ttacctgact ttgatcctgc tcttggaatg atgactggaa ttccaccaat 180
aactccaatg atgcctggtt tgggaatagt acctccacca attcctccag atatgccagt 240
agtaaaagag atcatacact gtaaaagctg cacgctcttc cctccaaatc caaatctccc 300
acctcctgca acccgagaaa gaccaccagg atgcaaaaca gtatttgtgg gtggtctgcc 360
tgaaaatggg acagagcaaa tcattgtgga agttttcgag cagtgtggag agatcattgc 420
cattcgcaag agcaagaaga acttctgcca cattcgcttt gctgaggagt acatggtgga 480
caaagccctg tatctgtctg gttaccgcat tcgcctgggc tctagtactg acaagaagga 540
cacaggcaga ctccacgttg atttcgcaca ggctcgagat gacctgtatg agtgggagtg 600
taaacagcgt atgctagcca gagaggagcg ccatcgtaga agaatggaag aagaaagatt 660
gcgtncacca tnttcacccc cagtggtcac tatttagatc atgaatgcag cattggtgct 720
gaaaaaataa aaggaggatt ccaaa 745

<210> 375
<211> 734
<212> DNA
<213> Homo Sapiens

<400> 375
gaggtataaa aaggaatatt tatcttttaa aaatacaact ttgaacacta ctggcatctc 60
atttacaaag tatttttgtg aaatactctc cattggcttt gcttgctcag tacattcttt 120
tatcttcaat tgagactcaa gggagggtat gcttgcatta ttataaatac cacaaccacc 180
accacacaca ataaagacca tctctgcctc aggacattcg ccccaaacct ccatcctctc 240
tgtttacttt ccaccaagca gaagtttctg aatggtccac tcacatgctg ccattgcgat 300
ttgccgatgg gcactaccaa ggtgtctctg gcaattcgca ctccaggtgg agctgaccta 360
tttgtagaaa gcctcacaaa ccctagctca ttatttattc attgattcat tactattaat 420
acttatatca agtctttgca aacattcagc atgaagtaaa catagtattt acagcagtac 480
tcggtttgca attcaacaca ctgacaacag aagcaaaggg accaacagac tgtaagaagg 540
ccagagggga aagaatatta atataaatcc cttctgccac tgtgtgccgt gccgtgtgtg 600
tgtttgtgcg tgtgtgccca cacatgagca tattttaatt cacagaaaaa ctgaaacatg 660
ccctccttta aaagcagact atttacaagt gattctgaat agcatgaaca catgccagnc 720
atactggaaa cttg 734

<210> 376
<211> 822
<212> DNA
<213> Homo Sapiens

<400> 376
ggctgatcag tgttctagaa cagatcagac attttgtaat gatgcctgaa ataaacacta 60

accacctcga caagcaacag gttcaactcc tggcagagat gtgtatcctt attgatgaaa 120
atgacaataa aattggagct gagaccaaga agaattgtca cctgaacgag aacattgaga 180
aaggattatt gcatcgagct tttagtgtct tcttattcaa caccgaaaat aagcttctgc 240
tacagcaaag atcagatgct aagattacct ttccaggttg ttttacgaat acgtgttgta 300
gtcatccatt aagcaatcca gccgagcttg aggaaagtga cgcccttgga gtgaggcgag 360
cagcacagag acggctgaaa gctgagctag gaattccctt ggaagaggtt cctccagaag 420
aaattaatta tttaacacga attcactaca aagctcagtc tgatggtatc tggggtgaac 480
atgaaattga ttacattttg ttggtgagga agaatgtaac tttgaatcca gatcccaatg 540
agattaaaag ctattgttat gtgtcaaagg aagaactaaa agaacttctg aaaaaagcag 600
cccagtggtg aaattaagat aacgccatgg tttaaaatta ttgcagcgac ttttctcttt 660
aaatggtggg ataacttaaa tcatttgaat caagtttggt gacccatgag aaaatatacn 720
gaatggggaa tatgtaggta aatggattac ccgaaaaaan ttatctgntt aacaaactta 780
gaaaggcttt ttncctttta aattaagttc tatcattaaa tt 822

<210> 377
<211> 812
<212> DNA
<213> Homo Sapiens

<400> 377
gcaagaaata aatttttatt tttcttcatt atcatacagc atttaagaat aataaatctg 60
tcttgaggtt tcaaatctga gatatctatg gcaagtttat aaaaagtaca ttgatcaagg 120
tacaattttt aacattaata tacacattcc ataatctcat ctatttaaca ttaacacagg 180
cctttgttgt tgttattttt ttctccctac aatatttcct gactctgtag gacagtggtc 240
ctcagttggg ggttgactct gtcccctagg ggcatctggc aacatccggc ataactgtgg 300
gtgtcacatg agagggacgc tgctcaccat cctgcaatgc acagcacaga ccccaccaca 360
ggggttttat ccagcccaaa tgtcaacagt gtcaagttta agcaactctt accgagtggg 420
actcaattcc cattttatga acacctctgt gctcactgta attctgaaaa cacagacttt 480
gctaactggt aaatactatt tacaagaaga ttcaacctaa tcaatatcac ttatcaaaag 540
cagtggctga ctgtaagtat caacatgttt ccagaatgaa taaaccacac aatcaactca 600
gaatgataca aattagggtc catatcattt aatttccctt gaacctgctc tgctaggtta 660
atctgctaat atgaaagtta attaagactg gttttgaagg accgaggaca atagtttcct 720
ttgcacaatt ttctgaacta tgagaaaaat ttaaaggatc cntaaagcnc ctggcaaaaa 780
gccaaggccc tttgcaaagg gcttccggaa aa 812

<210> 378
<211> 870
<212> DNA
<213> Homo Sapiens

<400> 378
aaaatttaag ccaactctta ttcaactttt cttcttcaca gcagctgttt atagatagta 60
gggagccaag aatgaaggac agtaacagat ggaaagcaaa aagtacaaca gctatcttaa 120
gttcagctct caacattgct ggttgagttt ggaaccaaaa cctcttaaca actggcagat 180
aatagctaaa tcttaacaga caaagaagaa atattttctt tgggacagct gctatctaga 240
agaaaaccaa ggtcccttaa tatagtctaa atataatgtg tggcttatta tagagaaatc 300
tttagcaacg taagtttaac cagtaagtgt cacaactgat caacagtact taaaaggaaa 360
caaacaaaaa tcacactagc cacaaatttc caccatatac acatgaaatt aattttaatc 420
tgttttgact ccttgacact aactgatcat taatgaaata tgatatggaa agatcacaga 480
gtagaaaaca agcaaagatt agtttataca acagtgacta tatacatcag agggaaaaca 540
tgctagctaa tgcaacatta aggcctgaat gtaagcattt cccaagtcac agaagcccca 600
aagaactcct aaattacaaa ttcatcacat tacatgcatg caatggtcac ttttggttta 660
cccataaaag gatacncagt attttgctgn aaataccagg accacattta caatatatgc 720
aaaaaattag aatgcagngg taagntcctt anatttaagc cctcatatgn gncaacaggg 780
gaaaattcca tttattttta agaaaggaaa aanggagacn gggatataaa tactcggaga 840

aattccccga attaagaagn aacctctgca 870

<210> 379
<211> 837
<212> DNA
<213> Homo Sapiens

<400> 379
gaggagaggt caaccgtcgt agcgccaata acttctactc catgatccag tcggccaaca 60
gccatgtccg ccgcctggtg aacgagaaag ctgcccatga gaaagatatg gaagaagcaa 120
aggagaagtt caagcaggcc ctttctggaa ttctcattca atttgagcag atagtggctg 180
tgtaccattc cgcctccaag cagaaggcat gggaccactt cacaaaagcc cagcggaaga 240
acatcagcgt gtggtgcaaa caagctgagg aaattcgcaa cattcataat gatgaattaa 300
tgggaatcag gcgagaagaa gaaatggaaa tgtctgatga tgaaatagaa gaaatgacag 360
aaacaaaaga aactgaggaa tcagccttag tatcacaggc agaagctctg aaggaagaaa 420
atgacagcct ccgttggcag ctcgatgcct accggaatga agtagaactg ctcaagcaag 480
aacaaggcaa agtccacaga gaagatgacc ctaacaaaga acagcagctg aaactcctgc 540
aacaagccct gcaaggaatg caacagcatc tactcaaagt ccaagaggaa tacaaaaaga 600
aagaagctga acttgaaaaa ctcaaagatg acaagttaca ggtggaaaaa atgttggaaa 660
atcttaaaga aaaggaaagc tgtgcttcta ngctgtgtgc ctcaaaccag gatagcgaat 720
accctnttga gaaagaccat gaacagcagt cctatcaaaa tcttgaaccg tgaagcactg 780
gttagtgggg gattatcttc cacanttcct tcatggtcac cccatttgga gccagcc 837

<210> 380
<211> 793
<212> DNA
<213> Homo Sapiens

<400> 380
   gttgcttagt ttcaggagtt ctctacatat tctggatatt aattcctttt catatatatg 60
   atttgcaaat attttctccc attctgtggg gtttttttac tttgttgata ttgtcttttg 120
   agacacaatt ttttttaatt ttcatgaagt ccaatttgtc tatttttttt cttttgttgc 180
   ctattttgtg tcatcctcaa gaaaccatta ccaaatccag tgttttgaag ctttccccat 240
   atgttttatt ctaagagcct tatggtttta ggccttacat ttaggccttt gatccatttt 300
   gagttaattt ttgtatatgg tgttaggtaa ggacccaact tccttgtttg gcatgtggat 360
   atccaatttt cctaccacca tttgtttgaa aagattgtcc tttccccatt gaatggtctt 420
   ggtagccttg tcaaaagtca actgatcata catcttattt atttccggcc tccctaatct 480
   attctatcag actatatgtc tgtctttatg ccagtaccac attgttttga ttactgttag 540
   tccatcttta ttatataaaa tcatgattac aagctcatac tataatatta tattttatac 600
   ttttccaaat cttccatagc attgngttct tcttccacta aaaagcagac cgtttagagg 660
   tataataagt agcctgaagt gggcaagtaa tgaaacaaac ttgagaatta cataaccttn 720
   cagctataga gttcataatg gcccgaaagg gtaaagactg caggncgctt aattnccagg 780
   cttttcacca ggc 793

<210> 381
<211> 807
<212> DNA
<213> Homo Sapiens

<400> 381
agaacaccct cttagatgtc ttgttctgtg tgcccaagta catgccggaa tgtggagaag 60
aaatgggttc tctctagtaa accagattta ttactaccat aatgtgaaat gcagacgtga 120
gatgtttgac aaggatgtag taatgcttca gacaggtgtc tccatgatgg atccaaatca 180
tttcctgatg atcatgctca gccgctttga actttatcag attttcagta ctccagacta 240
tggaaaaaga tttagttctg agattaccca taaggatgtt gttcagcaga acaatactct 300

aatagaagaa atgctatacc tcattataat gcttgttgga gagagattta gtcctggagt 360
tggacaggta aatgctacag atgaaatcaa gcgagagatt atccatcagt tgagtatcaa 420
gcctatggct catagtgaat tggtaaagtc tttacctgaa gatgagaaca aggagactgg 480
catggagagt gtaatcgaag cagttgccca tttcaagaaa cctggattaa caggacgagg 540
catgtatgaa ctgaaaccag aatgtgccaa agagttcaac ttgnatttct atcacttttc 600
aagggcagaa cagtccaagg cagaagaagc gcaacggaaa ttgaaaagac naaatagaga 660
agatacagca cttccacctt ccggggttgn ctncattctg gcctctggtt gcaagcctgg 720
gtaacanttt gcagtcagat gtcatggtgn gcatcatggg gaaccaattn tgcaatgggc 780
tgtggaacca taaanggata tgcctgg 807

<210> 382
<211> 800
<212> DNA
<213> Homo Sapiens

<400> 382
aagtttaaat aaagaattta tttcgaaatt cagcagaact tctttctttc ttaaaaagcc 60
aactgggtta aaaaaatcca agtttgtgtt ttttggtggt gcaataatta taaatgttgc 120
cagtcaatgc caaccagtgt ctgattggct tcctgtgcat gtccaatttc ctctgtgaca 180
ctgtgttggt gccagagctt ctgaatcttc ttgaatcgct ctttgcataa atgtaaagga 240
tttccccgtc tgagtccctg gtcggtctcc ccatagtcat caaggtaagg aggagaataa 300
aaacagcctt tggttttgcc agctaaaaat agcacctgac attcccgtac tctcaggaag 360
atgcccactc cagagccaca ggagtaggtg tgagctgtgc aggctcctac atcctcccct 420
tccagttcag tctggcagca gtaactctgg gagcacagca gagatccgca cacaaggcac 480
agagttgggg ctctgctctt atcaccacct gatttcgggc acgagaaatt ggatgcttga 540
ttaatgaggc tgctgtaatc ctctggaagg tttattaatt tgttaagatt ctcttggata 600
tcttatagca tctcttttca ccttctagaa atcttttaac tttcactggt accggcacca 660
acntttcaat cagggaattc antatctcac tatttctttt gaaaaggcaa aatggaggtt 720
ggtttgggta agggaaagga aaggcttccn taaaaggttc aaaaagggct tngttnccag 780
gnaaccttgn aatgtcgggt 800

<210> 383
<211> 1203
<212> DNA
<213> Homo Sapiens

<400> 383
ctgacatcta caccgaggtc cgcgagctgg tgagcctcaa gcaggagcag caggctttca 60
aggaggcggc ccgacacgga gcggctcgcc ctgcaggccc tcacggagaa gcttctcagg 120
tctgaggagt ccgtctcccg cctcccggag gagatccgga gactggagga agagctccgc 180
cagctgaagt ccgattccca cgggccgaag gaggacggag gcttcagaca ctcggaagcc 240
tttgaggcac tccagcaaaa gagtcaggga ctggactcca ggctccagca cgtggaggat 300
ggggtgctct ccatgcaggt ggcttctgcg cgccagaccg agagcctgga gtccctcctg 360
tccaagagcc aggagcacga gcagcgcctg gccgccctgc aggggcgcct ggaaggcctc 420
gggtcctcag aggcagacca ggatggcctg gccagcacgg tgaggagcct gggcgagacc 480
cagctggtgc tctacggtga cgtggaggag ctgaagagga gtgtgggcga gctccccagc 540
acccgtggaa tcactccaga aggtgcagga gcaggtgcac acgctgctca gtcaggacca 600
agcccaggcc gcccgtctgc ctcctcagga cttcctggac agactttctt ctctagacaa 660
cctgaaagcc tcagtcagcc aagtggaggc ggacttgaaa atgctcagga ctgctgtgga 720
cagtttggtt gcatactcgg tcaaaataga aaccaacgag aacaatctgg aatcagccaa 780
gggtttacta gatgacctga ggaatgatct ggataggttg tttgtgaaag tggagaagat 840
tcacgaaaag gtctaaatga attgcgtgtg cagggcgcgg atttaaagtc caatttctca 900
tgaccaaaaa atgtgtggtt ttttcccatg tgtcccctac cccccaattt cttgtcccct 960
cttaaagagc agttgtcacc acctgaacac caaggcattg tattttcatg cccagttaac 1020
ttatttacaa tatttaagtt ctctgcttct gcatttggtt ggtttcctga agcgcagccc 1080

ctgtgaataa caggtggctt ttcatggatg tctctagtca gagaaaaatg ataaaggctt 1140
aaattgagga ttaacagaag cagattaacc tcagaaatcc tgtctggctg gcagatttca 1200
agt 1203

<210> 384
<211> 2651
<212> DNA
<213> Homo Sapiens

<400> 384
cctggctgca gagtacccca ccagcgcggt tcatagtggc gtcatgcacg cagactcctg 60
caagttcccc taagttctta gaggactgct ttgccttttg atctgagagt tgcaaagttc 120
cataaagaat ggcccttgtg gataagcaca aagtcaagag acagcgattg gacagaattt 180
gtgaaggtat ccgcccccag atcatgaacg gccccctgca cccccgcccc ctggtggcgc 240
tgctggacgg ccgcgactgc actgtggaga tgcccatcct gaaggacctg gccactgtgg 300
ccttctgtga cgcgcagtcg acgcaggaaa tccacgagaa ggttctaaac gaagccgtgg 360
gcgccatgat gtaccacacc atcaccctca ccagggagga cctggagaag ttcaaggccc 420
tgagagtgat cgtgcggata ggcagtggct atgacaacgt ggacatcaag gctgccggcg 480
agctcggaat tgccgtgtgc aacatcccgt ctgcagccgt ggaagagaca gcggactcta 540
ccatctgcca catcctcaac ctgtaccgga ggaacacgtg gctgtaccag gcactgcggg 600
aaggcacgcg ggttcagagc gtggagcaga tccgcgaggt ggcctcggga gcggcccgca 660
tccgtgggga gacgctgggc ctcattggct ttggtcgcac ggggcaggcg gttgcagttc 720
gagccaaggc ctttggattc agcgtcatat tttatgaccc ctacttgcag gatgggatcg 780
agcggtccct gggcgtgcag agggtctaca ccctgcagga tttgctgtat cagagcgact 840
gcgtctcctt gcactgcaat ctcaacgaac ataaccacca cctcatcaat gactttacca 900
taaagcagat gaggcaggga gcattccttg tgaacgcagc ccgtggcggc ctggtggacg 960
agaaagcctt agcacaagcc ctcaaggagg gcaggatacg aggggcagcc ctcgacgtgc 1020
atgagtcaga gccctttagc tttgctcagg gtccgttgaa agatgcaccg aatcttatct 1080
gcactcctca cactgcctgg tacagcaagc aggcgtcact ggagatgagg gaggcagctg 1140
ccactgagat ccgccgagcc atcacaggtc acatcccaga aagcttaaga aactgtgtga 1200
acaaggaatt atctgtcaca tcagcgcctt ggtcagtaat agaccagcaa gcaattcatc 1260
ctgagctcaa tggtgccaca tacagatatc cgccaggcat cgtgggcgtg gctccaggag 1320
gacttcctgc agccatggaa gggatcatcc cctgnaaggc atcccagtga ctcacaaccc 1380
tcccgacagt ggcacatcct ttcccaaggg ccttntccca accagcccac aaaacacggg 1440
gccaatcgag agcaccccaa cgagcaatag cagagaatgc cagaaggtaa tcactcagat 1500
acacttggga ccaagagnca gtgaaaaata gatgaactaa gagaaaaaga atcggatggt 1560
ctttgtaact tgattctgga catatgcatc attgatgttg cagtgttgaa actacaagag 1620
ctagaaaact gaagatgtcg tctgcttacg gaagcgctga aagactagga tgtgatttat 1680
taacgaccaa cttctgttat tgtgtgttaa gtttttcatc tgtgcatcaa atcacaaaaa 1740
gaataaatag agctttttcc tttatcagtc ccttgggcac agcaggtcct gaacaccctg 1800
ctctacaatg ttgcatcaag agttcaaaca acaaaataaa aaatattaag aggaaatccc 1860
catcctgtga cttgagtccc ttaagtctac aggggctggt gacctctttt tgctaatagg 1920
aaaatcacat tactacaaaa tggggagaaa actgtttgcc tgtggtagac acctgcacgc 1980
ataggattga agacagtaca ggctgctgta cagagaagcg cctctcacat ctgaactgca 2040
tactgagcgg gcaagtcggt tgtaagttca gtaaaaccct ctgatgatgc aaaaaaaaaa 2100
aaaaagtatt aagtttcaca agctgtttgt actcaaatat attttctcag tttcagatcc 2160
tctgctattt tattgagtgg aaagtcttga gctaaaaggg ttcaagaaga ataatgttgc 2220
atttccttat gtctcaggaa acacttttta tggtaacttg tcagattgtc tatgaacaaa 2280
cccacttttt tagacattga taaagtcttc ttcacgtgat attttataca agaacacttc 2340
agatgtatta gatgtgactg attttaacaa atcctattag atttgtatca actagttaca 2400
tgttctattc atagtctttt gtgaatcatt gcctttttgt ttaaaaagat ggcctatttt 2460
gagcctttgt ataggtacat tcctgttttt gtgacaaaag aaaaacttta aaattgtccc 2520
aaacagaaaa ataatggcta tcagaagtat gttttgtttt agtgtgagtt accgttactg 2580
tatttgttta ttgtaaaggt ggacatttag cgttcagtgc agttttcaat aaaaagtaat 2640
taaaatttgt t 2651

<210> 385
<211> 804
<212> DNA
<213> Homo Sapiens

<400> 385
cctggctgca gagtacccca ccagcgcggt tcatagtggc gtcatgcacg cagactcctg 60
caagttcccc taagttctta gaggactgct ttgccttttg atctgagagt tgcaaagttc 120
cataaagaat ggcccttgtg gataagcaca aagtcaagag acagcgattg gacagaattt 180
gtgaaggtat ccgcccccag atcatgaacg gccccctgca cccccgcccc ctggtggcgc 240
tgctggacgg ccgcgactgc actgtggaga tgcccatcct gaaggacctg gccactgtgg 300
ccttctgtga cgcgcagtcg acgcaggaaa tccacgagaa ggttctaaac gaagccgtgg 360
gcgccatgat gtaccacacc atcaccctca ccagggagga cctggagaag ttcaaggccc 420
tgagagtgat cgtgcggata ggcagtggct atgacaacgt ggacatcaag gctgccggcg 480
agctcggaat tgccgtgtgc aacatcccgt ctgcagccgt ggaagagaca gcggactcta 540
ccatctgcca catcctcaac ctgtaccgga ggaacacgtg gctgtaccag gcactgcggg 600
aaggcacgcg ggttcagagc gtggagcaga tcccgcgagg tggcctcggg agcgggccgc 660
atncgtgggg agacgcttgg gcctcattgg ctttggtccg caccggggca agccggttgc 720
agttcgagcc aaggcctttg gattcagcgc atattttatg accctacttt gcanggatgg 780
gatcgaaccg gtcccntggc cgtg 804

<210> 386
<211> 782
<212> DNA
<213> Homo Sapiens

<400> 386
gcatcatcag agggttttac tgaacttaca accgacttgc ccgctcagta tgcagttcag 60
atgtgagagg cgcttctctg tacagcagcc tgtactgtct tcaatcctat gcgtgcaggt 120
gtctaccaca ggcaaacagt tttctcccca ttttgtagta atgtgatttt cctattagca 180
aaaagaggtc accagcccct gtagacttaa gggactcaag tcacaggatg gggatttcct 240
cttaatattt tttattttgt tgtttgaact cttgatgcaa cattgtagag cagggtgttc 300
aggacctgct gtgcccaagg gactgataaa ggaaaaagct ctatttattc tttttgtgat 360
ttgatgcaca gatgaaaaac ttaacacaca ataacagaag ttggtcgtta ataaatcaca 420
tcctagtctt tcagcgcttc cgtaagcaga cgacatcttc agttttctag ctcttgtagt 480
ttcaacactg caacatcaat gatgcatatg tccagaatca gttacaaaga ccatccgatt 540
ctttttctct tagttcatct atttttcact ggctcttggt cccaagtgta tctgagtgat 600
taccttctgg cattctctgc tattgctcgg tggggtgctc tcgatggccc cgtggtttgn 660
gggctggttg ggaanagggc ncttgggaaa ggaagtgcca ctgtccggaa ggntggtgaa 720
gtcactggga ngcctccagg gatgannccc tttccatggg ntgcaaggaa agncttcctg 780
ga 782

<210> 387
<211> 865
<212> DNA
<213> Homo Sapiens

<400> 387
agattancnn cnggagctcg cgcgcctgca ggtcgacact agtggatcca aagctgtatc 60
agagcgactg cgtctccttg cactgcaatc tcaacgaaca taaccaccac ctcatcaatg 120
actttaccat aaagcagatg aggcagggag cattccttgt gaacgcaccc cgtggtggcc 180
tggtggacga gaaagcctta gcacaagctc tcaaggaggg caggatacga ggggcagccc 240
tctatgtgcg tgagtcggag ccctttagct ttgctcaggg tccgttgaaa gatgcaccga 300
atcttatctg cactcctcac actgcctggt acagcaagca ggcgtcactg gagatgaggg 360
aggcagctgc cactgagatc cgccgagcca tcacaggtca catcccagaa agcttaagaa 420

actgtgtgaa caaggaatta tctgtcacat cagcgccttg gtcagtaata gaccagcaag 480
caattcatcc tgagctcaat ggtgccacat acagatatcc gccaggcatc gtgggcgtgg 540
ctccaggagg acttcctgca gccttggaag ggatcatccc tggaggcatc ccagtgactc 600
acaacctccc aacagtggca catccttccc aggcgccctc tcccaaccag cccacaaaac 660
acggggacaa tcgagagcac ttcaacgagc aatagcagag aatgcccgga aggtaatcat 720
tcagatacat ttgggaccna gagatagtga aaaatgatga acttagagaa aaaggaatat 780
gaaggncttt ggaactggat cttggactta tgcatcattg atgcttgcaa gtggttaaaa 840
ctnccaggag ctttgaaaac tggaa 865

<210> 388
<211> 753
<212> DNA
<213> Homo Sapiens

<400> 388
gagtataang gttaaattct atttaaaaag aaagnccatt aaatcaactt caagttctta 60
nctcatagga ctatttngna ncacttcttt gnaaatatca tttngttagg tnatnggcaa 120
ancagtttca nggttcactt ccctcccttg anccaggncc aggncatttn gctttggggn 180
aaattaaaat canaattcta aaagttganc anctttgttt tttttnaatn gactnanctn 240
tancccacca ttacaactta nggacggcat gactngataa nganggactt gngtgaggtt 300
ttgagttttc aattaanctt tgnatcacat gaggnaatng ncagcattct tgagncnggt 360
tatggaatag gcagatanaa ccctgtagta ccaanagttg gaaatnggct aatngacaac 420
gcactngcct taaacatctc angtagagaa cttttacatt agngagangt ncttgaattt 480
canancctcac caaattttaa ttacttttta tngaaaactg cagngaangc taaaggtcta 540
cgtttacaat aaacaaatcc agtancagta actcacactg aaccaaanca tacttctgat 600
agccattatt tttcngcttg ggacaatttt taaagntttt cttttggccc aaaaaccngg 660
aatgtatccc aaacnaaggc tcaaaagagg cccatcnttt tcaaacaaaa aagggcantg 720
gattcncaaa aanactggng aaatagaaca tgg 753

<210> 389
<211> 737
<212> DNA
<213> Homo Sapiens

<400> 389
aggaaatcca cgagaaggtt ctaaacgaag ccgtgggcgc catgatgtac cacaccatca 60
ccctcaccag ggaggacctg gagaagttca aggccctgag agtgatcgtg cggataggca 120
gtggctatga caacgtggac atcaaggctg ccggcgagct cggaattgcc gtgtgcaaca 180
tcccgtctgc agccgtggaa gagacagcgg actctaccat ctgccacatc ctcaacctgt 240
accggaggaa cacgtggctg taccaggcac tgcgggaagg cacgcgggtt cagagcgtgg 300
agcagatccg cgaggtggcc tcgggagcgg cccgcatccg tggggagacg ctgggcctca 360
ttggctttgg tcgcacgggg caggcggttg cagttcgagc caaggccttt ggattcagcg 420
tcatatttta tgacccctac ttgcaggatg ggatcgagcg gtccctgggc gtgcagaggg 480
tctacaccct gcaggatttg ctgtatcaga gcgactgcgt ctccttgcac tgcaatctca 540
acgaacataa ccaccacctc atcaatgact ttaccataaa gcagatgagg cagggagcat 600
tccttgtgaa cgcagcccgt ggcggcctgg tggacgagaa agccttagca caagccctna 660
agganggcag gatacnaagg ggcaancctt gacgtgcatg agtcaaaanc ctttagcttt 720
tgcttaaggg tccgttg 737

<210> 390
<211> 775
<212> DNA
<213> Homo Sapiens

<400> 390

gcatcatcag agggttttac tgaacttaca accgacttgc ccgctcagta tgcagttcag 60
atgtgagagg cgcttctctg tacagcagcc tgtactgtct tcaatcctat gcgtgcaggt 120
gtctaccaca ggcaaacagt tttctcccca ttttgtagta atgtgatttt cctattagca 180
aaaagaggtc accagcccct gtagacttaa gggactcaag tcacaggatg gggatttcct 240
cttaatattt tttattttgt tgtttgaact cttgatgcaa cattgtagag cagggtgttc 300
aggacctgct gtgcccaagg gactgataaa ggaaaaagct ctatttattc tttttgtgat 360
ttgatgcaca gatgaaaaac ttaacacaca ataacagaag ttggtcgtta ataaatcaca 420
tcctagtctt tcagcgcttc cgtaagcaga cgacatcttc agttttctag ctcttgtagt 480
ttcaacactg caacatcaat gatgcatatg tccagaatca agttacaaag accatccgat 540
tctttttctc ttagttcatc tatttttcac tgnctcttgg tcccaagtgt atctgagtga 600
ttaccttctg gcattctctg ctattgctcg ttggggtgct ctcgattggc cccgtgtttt 660
gtgggctggt tggganaagg cccttgggaa aggatgtgcc actgtcggga gggttgtgag 720
tcactgggat gccttncagg ggatgatccc tttcatggct tggcaggaaa gtctt 775

<210> 391
<211> 776
<212> DNA
<213> Homo Sapiens

<400> 391
gtggataagc acaaagtcaa gagacagcga ttggacagaa tttgtgaagg tatccgcccc 60
cagatcatga acggccccct gcacccccgc cccctggtgg cgctgctgga cggccgcgac 120
tgcactgtgg agatgcccat cctgaaggac ctggccactg tggccttctg tgacgcgcag 180
tcgacgcagg aaatccacga gaaggttcta aacgaagccg tgggcgccat gatgtaccac 240
accatcaccc tcaccaggga ggacctggag aagttcaagg ccctgagagt gatcgtgcgg 300
ataggcagtg gctatgacaa cgtggacatc aaggctgccg gcgagctcgg aattgccgtg 360
tgcaacatcc cgtctgcagc cgtggaagag acagcggact ctaccatctg ccacatcctc 420
aacctgtacc ggaggaacac gtggctgtac caggcactgc gggaaggcac gcgggttcag 480
agcgtggagc agatccgcga ggtggcctcg ggagcggccc gcatccgtgg ggagacgctg 540
ggcctcattg gctttggtcg cacggggcaa gcggttgcag ttcgagccaa ggcctttgga 600
ttcagcgtca tattttatga cccctacttg caggatggga tcgagccggt ccctgggcgt 660
gcagaaggtc tacaccctgc aggatttgct gtatcagaac cgactgcgtc ttctttcact 720
tgcaatntta acgaacataa ccacccactt tatcaatgga cttttcccta aagcca 776

<210> 392
<211> 909
<212> DNA
<213> Homo Sapiens

<400> 392
aacaaatttt aattactttt tattgaaaac tgcactgaac gctaaatgtc cacctttaca 60
ataaacaaat acagtaacgg taactcacac taaaacaaaa catacttctg atagccatta 120
tttttctgtt tgggacaatt ttaaagtttt tcttttgtca caaaaacagg aatgtaccta 180
tacaaaggct caaaataggc catcttttta aacaaaaagg caatgattca caaaagacta 240
tgaatagaac atgtaactag ttgatacaaa tctaatagga tttgttaaaa tcagtcacat 300
ctaatacatc tgaagtgttc ttgtataaaa tatcacgtga agaagacttt atcaatgtct 360
aaaaaagtgg gtttgttcat agacaatctg acaagttacc ataaaaagtg tttcctgaga 420
cataaggaaa tgcaacatta ttcttcttga acccttttag ctcaagactt tccactcaat 480
aaaatagcag aggatctgaa actgagaaaa tatatttgag tacaaacagc ttgtgaaact 540
taatactttt tttttttttt tgcatcatca gagggtttta ctgaacttac aaccgacttg 600
cccgctcagt atgcagttca naagtganag gcgcttctct gtacagcaac ctggactggc 660
ttcaatccta tgcgtgcagg tgtctaccca gggcnaacag ttttctcccc attttggtag 720
taatggggat tttcctatta gccaaaaaag angtcaccag nccctgnaga cttaaaggga 780
cctcaaggtc nccaggaatg ggggatttcc ctcntaaaaa atttttaatt ttggggggtt 840
gnaactcttg gangccacca tttgtaaaac canggggttc aagaacctgg ntgggcccca 900

agggacctg                                                          909

<210> 393
<211> 769
<212> DNA
<213> Homo Sapiens

<400> 393
caaattttaa ttacttttta ttgaaaactg cactgaacgc taaatgtcca cctttacaat      60
aaacaaatac agtaacggta actcacacta aaacaaaaca tacttctgat agccattatt     120
tttctgtttg ggacaatttt aaagtttttc ttttgtcaca aaaacaggaa tgtacctata     180
caaaggctca aaataggcca tctttttaaa caaaaaggca atgattcaca aaagactatg     240
aatagaacat gtaactagtt gatacaaatc taataggatt tgttaaaatc agtcacatct     300
aatacatctg aagtgttctt gtataaaata tcacgtgaag aagactttat caatgtctaa     360
aaaagtgggt ttgttcatag acaatctgac aagttaccat aaaaagtgtt tcctgagaca     420
taaggaaatg caacattatt cttcttgaac ccttttagct caagactttc cactcaataa     480
aatagcagag gatctgaaac tgagaaaata tatttgagta caaacagctt gtgaaactta     540
atactttttt tttttttttg catcatcana gggttttact gaacttacaa ccgacttgcc     600
cgctcagtat gccagttcan atgtgaaagg cgcttttntg tcagcagcct gnactggctt     660
caatcctatg cgtgcaggng tttacccaca ggcaaacagg ttttctnccc catttttgga     720
agtaatgggg attttcctat tagcaaaaaa gaaggncacc aanccccctg                 769

<210> 394
<211> 813
<212> DNA
<213> Homo Sapiens

<400> 394
ggaagatggc ggagctgcgc gtgctcgtag ctgtcaagag ggtcatcgac tacgccgtga      60
agatccgagt gaagcctgac aggaccggtg tggtcacgga tggtgtgaag cactccatga     120
accccttctg tgagatcgcg gtggaggagg ctgtgcggct caaggagaag aagctggtga     180
aggaggtcat cgccgtcagc tgtgggcctg cacagtgcca ggagacgatt cgtaccgccc     240
tggccatggg tgcagaccga ggtatccacg tggaggtgcc cccagcagaa gcagaacgct     300
tgggtcccct gcaggtggct cgggtcctgg ccaagctggc agagaaggag aaggtggacc     360
tggtgctgct gggcaaacag gccatcgatg atgactgtaa ccagacaggg cagatgacag     420
ctggatttct tgactggcca cagggcacat tcgcctccca ggtgacgctg gagggggaca     480
agttgaaagt ggagcgggag atcgatgggg gcctggagac cctgcgcctg aagctgccag     540
ctgtggtgac agctgacctg aggctcaacg agccccgcta cgccacgctg cccaacatca     600
tgaaagccaa gaagaagaag atcgaggtga tcaagcctgg ggacctgggt gtggacctga     660
cctccaagct ctctgtgatc agtgtggagg acccgcccca gcgcacggcc ggcgtcaagg     720
tggagaccac tgaggacctg gtggccaagc tgaaggagat tgggcggatt tgagcccctc     780
ccagagatgg caataaaact gactctcaac atc                                  813

<210> 395
<211> 762
<212> DNA
<213> Homo Sapiens

<400> 395
ggaagatggc ggagctgcgc gtgctcgtag ctgtcaagag ggtcatcgac tacgccgtga      60
agatccgagt gaagcctgac aggaccggtg tggtcacgga tggtgtgaag cactccatga     120
accccttctg tgagatcgcg gtggaggagg ctgtgcggct caaggagaag aagctggtga     180
aggaggtcat cgccgtcagc tgtgggcctg cacagtgcca ggagacgatt cgtaccgccc     240
tggccatggg tgcagaccga ggtatccacg tggaggtgcc cccagcagaa gcagaacgct     300
tgggtcccct gcaggtggct cgggtcctgg ccaagctggc agagaaggag aaggtggacc     360

tggtgctgct gggcaaacag gccatcgatg atgactgtaa ccagacaggg cagatgacag 420
ctggatttct tgactggcca cagggcacat tcgcctccca ggtgacgctg gagggggaca 480
agttgaaagt ggagcgggag atcgatgggg gcctggagac cctgcgcctg aagctgccag 540
ctgtggtgac agctgacctg aggctnaacg agcccccgct acgccacgct tgccaacatc 600
atgaaagcca agaagaagaa gatcgangtg atcaacctgg gganctgggt gtggacctga 660
ctccagcttt tttgngatca gtgtgganga ccggcccacg cacgggcgcg tcaangtgga 720
gaccctgagg acctggtggn caactnaaag aaaatgggcg ga 762

<210> 396
<211> 822
<212> DNA
<213> Homo Sapiens

<400> 396
gagagtcagt tttattgcca tctntgggag gggctcaaat ccgcccaatc tccttcagct 60
tggccaccag gtcctcagng gtctccacct tgacgccggc cgtgcgctgg ggcgggtcct 120
ccacactgat cacagagagc ttggaggtca ggtccacacc caggtcccca ggcttgatca 180
cctcgatctt cttcttcttg gctttcatga tgttgggcag cgtggcgtag cggggctcgt 240
tgagcctcag gtcagctgtc accacagctg gcagcttcag gcgcagggtc tccaggcccc 300
catcgatctc ccgctccact ttcaacttgt ccccctccag cgtcacctgg gaggcgaatg 360
tgccctgtgg ccagtcaaga aatccagctg tcatctgccc tgtctggtta cagtcatcat 420
cgatggcctg tttgcccagc agcaccaggt ccaccttctc cttctctgcc agcttggcca 480
ggacccgagc cacctgcagg ggacccaagc gttctgcttc tgctgggggc acctccacgt 540
ggatacctcg gtctgcaccc atggccaggg cggtaccaat cgtctcctgg cactgtgcag 600
gcccacagnt gacggcgatg accttccttc accaagcttt tttctccttt gagccggaca 660
ggcctcttca acgggatctt caccanaaag gggttcatgg gagngcttaa aaccatccgn 720
gaacccaccg gnccttgtna ggctttactt cggatctttn acnggggaat cgatgacccn 780
ttttgacagg tacgaacccc cgccagnttc ggcattttcc tt 822

<210> 397
<211> 812
<212> DNA
<213> Homo Sapiens

<400> 397
gatgttgaga gtcagtttta ttgccatctc tgggaggggc tcaaatccgc ccaatctcct 60
tcagcttggc caccaggtcc tcagtggtct ccaccttgac gccggccgtg cgctggggcg 120
ggtcctccac actgatcaca gagagcttgg aggtcaggtc cacacccagg tccccaggct 180
tgatcacctc gatcttcttc ttcttggctt tcatgatgtt gggcagcgtg gcgtagcggg 240
gctcgttgag cctcaggtca gctgtcacca cagctggcag cttcaggcgc agggtctcca 300
ggcccccatc gatctcccgc tccactttca acttgtcccc ctccagcgtc acctgggagg 360
cgaatgtgcc ctgtggccag tcaagaaatc cagctgtcat ctgccctgtc tggttacagt 420
catcatcgat ggcctgtttg cccagcagca ccaggtccac cttctccttc tctgccagct 480
tggccaggac ccgagccacc tgcaggggac ccaagcgttc tgcttctgct gggggcacct 540
ccacgtggat acctcggtct gcacccatgg ccagggcggt acgaatcgtc tcctggcact 600
gtgcaggccc acaagctgac gggcgatgaa cctccttcac cagcttcttc tccttgagcc 660
cgcacagcct tcttcaccgc gatctcacag gaaggggttc atggagtgct tacaaccatc 720
cggngaccac accgggccct gtcaggcttt aactcggant ctttacgggg taatcgnntg 780
gacctttttg acaagctacc aagcaccccg ca 812

<210> 398
<211> 751
<212> DNA
<213> Homo Sapiens

<400> 398
gatgttgaga gtcagtttta ttgccatctc tgggaggggc tcaaatccgc ccaatctcct 60
ttngcttggc caccaggtcc tcagtggtct ccaccttgac gccggccgtg cgctggggcg 120
ggtcctccac actgatcaca gagagcttgg aggtcaggtc cacacccagg tccccaggct 180
tgatcacctc gatcttcttc ttcttggctt tcatgatgtt gggcagcgtg gcgtagcggg 240
gctcgttgag cctcaggtca gctgtcacca cagctggcag cttcaggcgc agggtctcca 300
ggcccccatc gatctcccgc tccactttca acttgtcccc ctccagcgtc acctgggagg 360
cgaatgtgcc ctgtggccag tcaagaaatc cagctgtcat ctgccctgtc tggttacagt 420
catcatcgat ggcctgtttg cccagcagca ccaggtccac cttctccttc tctgccagct 480
tggccaggac ccgagccacc tgcaggggac ccaagcgtnc tgcttctgct gggggcacct 540
ccacgtggat acctcggtct gcacccatgg ccagggcggt acnnaatcgn ctcctggcac 600
tgtgcaggcc cacaagntga cggggaatga cctccttnac caagcttntt ntccttgacc 660
cgaaaagctt cttcaccgng aacttncaga angggttcaa tggantgctt tacacattcg 720
ggaccacccc cgggccttgt caggctttaa t 751

<210> 399
<211> 800
<212> DNA
<213> Homo Sapiens

<400> 399
   agatgttgag agtcagtttt attgccatct ctgggagggg ctcaaatccg cccaatctcc 60
   ttcagcttgg ccaccaggtc ctcagtggtc tccaccttga cgccggccgt gcgctggggc 120
   gggtcctcca cactgatcac agagagcttg gaggtcaggt ccacacccag gtccccaggc 180
   ttgatcacct cgatcttctt cttcttggct ttcatgatgt tgggcagcgt ggcgtagcgg 240
   ggctcgttga gcctcaggtc agctgtcacc acagctggca gcttcaggcg cagggtctcc 300
   aggcccccat cgatctcccg ctccactttc aacttgtccc cctccagcgt cacctgggag 360
   gcgaatgtgc cctgtggcca gtcaagaaat ccagctgtca tctgccctgt ctggttacag 420
   tcatcatcga tggcctgttt gcccagcagc accaggtcca ccttctcctt ctctgccagc 480
   ttggccagga cccgagccac ctgcagggga cccaagcgtt ctgcttctgc tgggggcacc 540
   ttccacgtgg atacctcggt ctgacccatg gccagggcgg tacgaatcgt ctcctggcac 600
   tgngcangcc cacaagctga cggcgatgac ctncttnacc agcttcttct ncttgagccc 660
   ggacaagnct tcttcaaccg ggatctcaca agaaggggtc atggagtgct ttcacaccat 720
   tcgggancac aaccggncct gncaaggctt naacttggac ntttacggng taatccgatg 780
   aacccttttt gacagntacc 800

<210> 400
<211> 810
<212> DNA
<213> Homo Sapiens

<400> 400
ggaagactga attgaaagaa gatagctcta gcagtgaagc agaggaagaa gaggaggagg 60
aagatgatga aaaagaaaag gaggataata gcagtgaaga agaggaagaa atagaaccat 120
ttccagaaga aagggagaac tttcttcagc aattgtacaa atttatggaa gatagaggta 180
cacctattaa caaacgacct gtacttggat atcgaaattt gaatctcttt aagttattca 240
gacttgtaca caaacttgga ggatttgata atattgaaag tggagctgtt tggaaacaag 300
tctaccaaga tcttggaatc cctgtcttaa attcagctgc aggatacaat gttaaatgtg 360
cttataaaaa atnctnatnt ggctntgngg agtactgtac atcagccaac attgaatttc 420
agatggcatt gccagagaaa gttgttaaca agcaatgtaa ggagtgtgaa aatgtaaaag 480
aaataaaagt taaggaggaa aatgaaacag agatcaaaga aataaagatg gaggaggaga 540
ggaatataat accaagagaa gaaaagccta ttgaggatga aattgaaaga aaagaaaata 600
ttaagccctc tctgggaagt aaaaagaatt tattagaatc tatacctaca cattctgatc 660
aggaaaaaga agttacatta aaaaaccnga agacaatgaa aatctgggcc gaccaagatg 720
atgacncaac tagggtagat gaatccctca accntaaggt agaactgagg aagaaaaagc 780

caaatctgga tncnatgaat gggattaagc 810

<210> 401
<211> 860
<212> DNA
<213> Homo Sapiens

<400> 401
gggaggcccg cctagccacc ctgaccagcc gtgtagaaga agacagcaac agagattata 60
aaaaactcta tgagagtgct ctgactgaaa accaaaaact gaaaacaaaa cttcaggaag 120
cccagctaga gctagcagat ataaagtcca agcttgagaa ggtggcccag cagaaacaag 180
aaaagacctc tgaccgatca tcagtgctgg agatggagaa acgggagagg cgagccttgg 240
agcgcaaaat gtcagaaatg gaggaagaaa tgaaggtgtt aacagaactg aaatccgaca 300
accagaggct gaaagatgaa aatggtgccc tcatcagagt catcagcaaa ctgtccaagt 360
aggctaggct ccagatttat gaggaaagaa agggacagca tttgctgccc ccacccctct 420
tttccagtcc ttgccttcca accaaaagaa atggatgttt tggtggaagg acacttcttt 480
ctatcaccct cttcagtcac ctctatacac tctacatttt ctctgcactt tcaatgccct 540
gttcttccaa acccctatcc caagttttat gacagtttta attgaagcat gattgtggta 600
attcgagcca tctggagaat gctctgggga gtacaccagg ctcagctgtg gacccctcaa 660
cttcctgctg ctcagctact ttgtccacat tggatttggt ccaaacatgt aagactttct 720
accctnatca gtatccttca gctttttaca ttaacccagt gnccttctga tataggtgaa 780
gtccttgngg gtagccactt tcaggatcct ggaatggggt gcccaagaga aacngccagg 840
atggttgaat tggatcattc 860

<210> 402
<211> 779
<212> DNA
<213> Homo Sapiens

<400> 402
gagatggagt cttgctctgt cacccaggct ggagtgcagt ggcgtgatca aggctcactg 60
caagctccgc ctcccgggtt cacgccattc tcctgcctca gcctcccgag tagctgggac 120
tacaggcgcc cgccaccacg cctggctaat ttttgtattt tttagtagag attgggtttc 180
accgtgttag ccaggatggt ctcgaactcc tgacctcgtg atctgtccac ctcggcctcc 240
tgaagtgctg ggattacagg catgagccac tgcacctggt ggaattggga tcttgaatta 300
cagcttctag tttaaacagc atgtggtgtt tcagagggag gaccatggag agctacatgt 360
catgttagga aagaattaac agacagaggt agtatatatt aagggaatga accactctaa 420
acactgaata tcactggcaa ccctaaaatg atgaggattt aatgacttgc acactcaagt 480
gaaccaaggg ataaaactcc tacaaaaaga aaatactgta agtattaatg ctaggttatc 540
atcaaganct aatggtttaa ttttgcactg gatttgnatt cttttccagg cctggacatg 600
atattttaaa ggctggttnt ggctagagga ggatgggcca anatgtgaca gggangaaaa 660
gcatgcctta tgaggaatga cttaaaggga ctagaggtaa cagcagctca aaagtaagaa 720
ctgaggggga aaacccccca ctgnaccata tntnaagggc cgttaaagaa ttgcagaat 779

<210> 403
<211> 1443
<212> DNA
<213> Homo Sapiens

<400> 403
cttcaggaac tgttaaaaga aaaacaacaa gaagtaaagc agctacagaa ggactgcatc 60
aggtatcaag agaaaattag tgctctggag agaactgtta aagctctaga atttgttcaa 120
actgaatctc aaaaagattt ggaaataacc aaagaaaatc tggctcaagc agttgaacac 180
cgcaaaaagg cacaagcaga attagctagc ttcaaagtcc tgctagatga cactcaaagt 240
gaagcagcaa gggtcctagc agacaatctc aagttgaaaa aggaacttca gtcaaataaa 300

gaatcagtta aaagccagat gaaacaaaag gatgaagatc ttgagcgaag actggaacag 360
gcagaagaga agcacctgaa agagaagaag aatatgcaag agaaactgga tgctttgcgc 420
agagaaaaag tccacttgga agagacaatt ggagagattc aggttacttt gaacaagaaa 480
gacaaggaag ttcagcaact tcaggaaaac ttggacagta ctgtgaccca gcttgcagcc 540
tttactaaga gcatgtcttc ccttcaggat gatcgtgaca gggtgataga tgaagctaag 600
aaatgggaga ggaagtttag tgatgcgatt caaagcaaag aagaagaaat tagactcaaa 660
gaagataatt gcagtgttct aaaggatcaa cttagacaga tgtccatcca tatggaagaa 720
ttaaagatta acatttccag gcttgaacat gacaagcaga tttgggagtc caaggcccag 780
acagaggtcc agcttcagca gaaggtctgt gatactctac agggggaaaa caaagaactt 840
ttgtcccagc tagaagagac acgccaccta taccacagtt ctcagaatga attagctaag 900
ttggaatcag aacttaagag tctcaaagac cagttgactg atttaagtaa ctctttagaa 960
aaatgtaagg aacaaaaagg aaacttggaa gggatcataa ggcagcaaga ggctgatatt 1020
caaaattcta agttcagtta tgaacaactg gagactgatc ttcaggcctc cagagaactg 1080
accagtaggc tgcatgaaga aataaatatg aaagagcaaa agattataag cctgctttct 1140
ggcaaggaag aggcaatcca agtagctatt gctgaactgc gtcagcaaca tgataaagaa 1200
attaaagagc tggaaaacct gctgtnccag gaggaagagg agaatattgg tttagaagag 1260
gagaacaana angcttgtgg ttaaaaccca atcagcttat gggaacactt gaaaaccatc 1320
aaaanggaaa catttagnca aaaggcncag ttggattcct tggtnaaatc ctgncttctn 1380
ttccaaatgg atccgagaac cgcntagtgg ggggactatt caccagctgg gaanagccga 1440
ctt 1443

<210> 404
<211> 819
<212> DNA
<213> Homo Sapiens

<400> 404
gcgattcaaa gcaaagaaga agaaattaga ctcaaagaag ataattgcag tgttctaaag 60
gatcaactta gacagatgtc catccatatg gaagaattaa agattaacat ttccaggctt 120
gaacatgaca agcagatttg ggagtccaag gcccagacag aggtccagct tcagcagaag 180
gtctgtgata ctctacaggg ggaaaacaaa gaacttttgt cccagctaga agagacacgc 240
cacctatacc acagttctca gaatgaatta gctaagttgg aatcagaact taagagtctc 300
aaagaccagt tgactgattt aagtaactct ttagaaaaat gtaaggaaca aaaaggaaac 360
ttggaaggga tcataaggca gcaagaggct gatattcaaa attctaagtt cagttatgaa 420
caactggaga ctgatcttca ggcctccaga gaactgacca gtaggctgca tgaagaaata 480
aatatgaaag agcaaaagat tataagcctg ctttctggca aggaagaggc aatccaagta 540
gctattgctg aactgcgtca gcaacatgat aaagaaatta aagagctgga aaacctgctg 600
tnccaggagg aagaggagaa tattggttta gaagaggaga acaanaangc ttgtggttaa 660
aacccaatca gcttatggga acacttgaaa accatcaaaa nggaaacatt tagncaaaag 720
gcncagttgg attccttggt naaatcctgn cttctnttcc aaatggatcc gagaaccgcn 780
tagtgggggg actattcacc agctgggaan agccgactt 819

<210> 405
<211> 761
<212> DNA
<213> Homo Sapiens

<400> 405
ctgaaaataa ttttattatt ttacagttgt tcaggaaact tcccaggatg ttgtaaccaa 60
natttaatca ccacagtana tttanagcan atcagtcagc ccacttgtct tccctcttct 120
ttagggaang gctaggcagt gaacacatca tgtatgcaat ganaaaataa ccaactggta 180
ggatggggga ggggagggga ggcagggaat aggcncaaat ggaattctat cctggctgtc 240
cttctcaggt ctatctatat ttaattttgt cttctctata ttctccttcc attgccacag 300
agggcanaga caatggggct gaaaaactgt aataactgnc actaacagca aagtanctta 360
gtncttcaag aggtcaggag ttgcagtgtg gtgttanacc agtcanactc ctggctgaaa 420

gtcaatgcct aatattggct cccagnggcc cctgagcact gtctcagggt ccacattcca 480
ggaatnttca natnttcctg gaatgacaag aattggaacc ctgctgncca tagacacttc 540
tccctgccct ttggtgaaag gaaagacttt gggccccttt aataccttan tatcccatgt 600
gatcaagggc caaaagccaa aggggattct tatccttata gcctaagacc ctgaaattct 660
tcccttccca attatatctg gaaattggcc aggggaanaa aaatgctgnc cttcccatgn 720
ggaatctacc aggnttaaaa ccccnttaag ggagttccct t 761

<210> 406
<211> 758
<212> DNA
<213> Homo Sapiens

<400> 406
gatactgaac ttcagattat taggtttatt gaaaccatcc tcttggcttg gctgaaagac 60
attcctcagt atcttttaca ggaccacaaa agatcagggt cctgcaaaat ctcaacaaat 120
attaggctca acaaaccaaa tgtgattctc agattaagca gaagcgttca ggctcagggc 180
agtagaagaa agcagactcg ccagtccctg cagctccaac ctgtcctcgt atcacctctg 240
tttttgcagg cactttccgt gaagagttgg agagaagacc tgtaaatggg aagactgttc 300
cactggaatt gatgttctga tgttagaggt gagagaattc caagttttga ggggagtggt 360
ccaaagagta acaactaagt ctatagatgg cccgtaaaac acagaatgag caggacatga 420
atcattagaa agtagatggc tgctagaagt ggcactcggg tccgtgaatg acagagtgaa 480
cgcaggactc gcttccatcc aacgccactc cgggtccttc gacaactgtt gcttgtaaga 540
tctattaaca gtgcctgctc ctgagtgcca caggagccaa tgataggagt ccgggaaaga 600
gtcccattca ctgngctcta accggctgga tctgctcctc ggccacagga gagagcattt 660
ttcagcagcc actctttggc cncggtcttt cttccagcag cttcctttaa atcattcctt 720
tcttggctgg nggttgccat aactgctggt tggacctt 758

<210> 407
<211> 778
<212> DNA
<213> Homo Sapiens

<400> 407
cttcaggaac tgttaaaaga aaaacaacaa gaagtaaagc agctacagaa ggactgcatc 60
aggtatcaag agaaaattag tgctctggag agaactgtta aagctctaga atttgttcaa 120
actgaatctc aaaaagattt ggaaataacc aaagaaaatc tggctcaagc agttgaacac 180
cgcaaaaagg cacaagcaga attagctagc ttcaaagtcc tgctagatga cactcaaagt 240
gaagcagcaa gggtcctagc agacaatctc aagttgaaaa aggaacttca gtcaaataaa 300
gaatcagtta aaagccagat gaaacaaaag gatgaagatc ttgagcgaag actggaacag 360
gcagaagaga agcacctgaa agagaagaag aatatgcaag agaaactgga tgctttgcgc 420
agagaaaaag tccacttgga agagacaatt ggagagattc aggttacttt gaacaagaaa 480
gacaaggaag ttcagcaact tcaggaaaac ttggacagta ctgtgaccca gcttgcagcc 540
tttactaaga gcatgtcttc ccttcaggat gatcgtgaca gggtgataga tgaagctaag 600
aaatgggaga ggaagtttag tgatgcgatt caaagcaaag aagaagaaat tagactcaaa 660
gaagataatt gcagtgtcta aaggacactt agacagatgt ccttcntatg gaagaattaa 720
agantaccat ttcaggcttt gaccatgaca gcagatttgg agtccaggnc caaccaga 778

<210> 408
<211> 752
<212> DNA
<213> Homo Sapiens

<400> 408
canattatta ggttnatnga anccatcctn tnggntnggn tgaaanacnt tcctnagtnt 60
nttttacngg accncaaaan atcagggncc tgcaaaatct cancaaatnt taggctcanc 120

aaaccaaang ngattntnaa attaancaaa ancgttcagg ctcagggcag taaaaaaaag 180
caaactcgcc agnccntgca gctccaacct gncctcgtat cncctntgtt tttgcaggcn 240
ntttccgnga anagttggan anaaaacctg taaanggnaa aactgttcca ntggaatnga 300
ngttctgatg ttanaggnga nanaattcca agttttgagg ggagnggncc aaagagtacc 360
aactaagtnt ntananggcc cgtaaaacnc anantganca ggacntgaat cnttaaaaag 420
taaatggctg ntaaaagngg cnctcgggtc cgtgaatgac agagtgancn caggactcgn 480
ttccatccaa cgccantccg ggtccttcga caactgtngc ttgtaanatc tattaacagg 540
gcctgntcct gantgccaca ggagccaatg ntaggagtcc gggaagagtc ccatttcact 600
ggggctttaa ccgtctgaat ctggtccttg gccncagaga gagcnttttt nagnaggccc 660
ncnttttggg ccccgttntt ttttccagca ngcttccctt taattcattc ncttcccggg 720
ctgggggttg caaaacntgc tggntgacct tt 752

<210> 409
<211> 736
<212> DNA
<213> Homo Sapiens

<400> 409
ggcgtgtcaa aactaacgta cctgtcaagc tctttgcccg ctccacagct gtcaccacca 60
gctcagccaa gatcaagtta aagagcagtg agctgcaggc catcaagacg gagctgacac 120
agatcaagtc caatatcgat gccctgctga gccgcttgga gcagatcgct gcggagcaaa 180
aggccaatcc agatggcaag aagaagggtg atggaggtgg cgccagcggc ggcggcggcg 240
gtggtggtgg cagcggtggc ggtggcagtg gtggtggcgg tggcggtggc aacagccggc 300
caccagcccc ccaagagaac acaacttctg aggcaggcct gccccagggg gaagcacgga 360
cccgagacga cggcgatgag gaagggctcc tgacacacag cgaggaagag ctggaacaca 420
gccaggacac agacgcggat gatggggcct tgcagtaagc agcctgacag gagcaatggc 480
caccagcagg tgaagggcat cgctgcccag gcctcaagcc gggcacccaa ccctggatgc 540
caccccccag cgggtaccag aggaaagctg cagcaggccg cctcctcccc caacgcatnc 600
cagccagtgc catgtcctct gcaggtggag ttactggcct actccttccc atgaaccctt 660
ccttgtctgc acttgccagg ccagagggta gagcacangg gtttccccat acttaccttc 720
ccttcccagg acactt 736

<210> 410
<211> 766
<212> DNA
<213> Homo Sapiens

<400> 410
gggatccaat ctctttattg tcagggtccc ctccctgngg ccccccgcca aacctataga 60
aaaaacccaa gcctgggagt gtcctgggga ggggaggtag tatggggaaa cccctgngct 120
ctaccctctg gcctgggcag tgcanacagg gagggctcat ggggaaggag taggccagta 180
actccacctg cagaggacat ggcactggct gggatgcgtt gggggaggag gcgcctgctg 240
ccagctttcc tctggtaccc gctggggggt ggcatccagg gttgggtgcc cggcttgagg 300
cctggggcag cgatgccctt cacctgctgg nggccattgc tcctgtcagg ctgcttactg 360
caaggcccca tcatccgcgt ctgtgtcctg gctgtgttcc agctcttcct cgctgtgtgt 420
caggagccct tcctcatcgc cgtcgtctcg ggtccgtgct tccccctggg gcaggcctgc 480
ctcanaagtt gngttctctt ggggggctgg tggcccggct gttgccaccg gcaccggcac 540
caccactgnc accgncaccg ctgcaccacc accgncggcg cccgncgntt ggcgccaact 600
tcatnaccct tcttcttgca tctggaatgg ncttttgctt ncgcancgaa ctgntccaaa 660
cgggttaanc agggcatcna tatttggact tgaactgggn caancttccg ncttgaangg 720
ccttgcaagc ttnaatggtc tttaacttga actttggctt gaacct 766

<210> 411
<211> 812
<212> DNA

<213> Homo Sapiens

<400> 411
ggcgtgtcaa aactaacgta cctgtcaagc tctttgcccg ctccacagct gtcaccacca 60
gctcagccaa gatcaagtta aagagcagtg agctgcaggc catcaagacg gagctgacac 120
agatcaagtc caatatcgat gccctgctga gccgcttgga gcagatcgct gcggagcaaa 180
aggccaatcc agatggcaag aagaagggtg atggaggtgg cgccagcggc ggcggcggcg 240
gtggtggtgg cagcggtggc ggtggcagtg gtggtggcgg tggcggtggc aacagccggc 300
caccagcccc ccaagagaac acaacttctg aggcaggcct gccccagggg gaagcacgga 360
cccgagacga cggcgatgag gaagggctcc tgacacacag cgaggaagag ctggaacaca 420
gccaggacac agacgcggat gatggggcct tgcagtaagc agcctgacag gagcaatggc 480
caccagcagg tgaagggcat cgctgcccca ggcctcaagc cgggcaccca accctggatg 540
ccacccccca gcgggtacca gaggaaagct ggcagcaggc gcctcctccc ccaacgcatc 600
ccagccagtg ccatgtcctc tgcaggtgga gttactggcc tactccttcc ccatgagccc 660
tccctgtctg cactgcccag gccagagggt agagcacagg ggtttcccca tactacctcc 720
cctccccagg acactcccag gcttgggttt tttctatagg tttggcgggg ggccncaggg 780
aggggaccct gacaataaag agattggatc cc 812

<210> 412
<211> 857
<212> DNA
<213> Homo Sapiens

<400> 412
aaccatctta gcccccaaaa tgatgatgct ctggagacac gagctaagaa gtctgcatgc 60
tctgacatgc ttctcgaagg tggtcctact acagcttctg taagagaggc caaagaggat 120
gaagaagatg aggagaagat tcagaatgaa gattatcatc acgagctttc agatggagat 180
ctggatctgg atcttgttta tgaggatgaa gtaaatcagc tcgatggcag cagttcctct 240
gctagttcca cagcaacaag taatacagaa gaaaatgata ttgatgaaga aactatgtct 300
ggagaaaatg atgtggaata taacaacatg gaattagaag agggagaact catggaagat 360
gcagctgctg caggacccgc aggtagtagc catggttatg tgggttccag tagtagaata 420
tcaagaagaa cacatttatg ctccgctgct accagtagtt tactagacat tgatccatta 480
attttaatac atttgttgga ccttaaggac cggagcagta tagaaaattt gtggggctta 540
cagcctcgcc cacctgcttc acttctgcag cccacagcat catattctcg aaaagataaa 600
gaccaaagga agcaacaggc aatgtggcga agtgccctct gatttaaaga tgctaaaaag 660
actcaaaact caaatggccc gaagttcgat gtatgaaaac tgatgtaaag gaatacactt 720
tcagaaataa aaagcacagt gctgcttctg gagacatgcn gacaagnctt tttttgctga 780
nccagcagnt ntggctgatg tggactgaaa cttttggcag aatgcaggat ttggatggac 840
tcctggcnaa agtctta 857

<210> 413
<211> 790
<212> DNA
<213> Homo Sapiens

<400> 413
ctcaagtnga ttttattanc aaaaagngca aactattttg ancaaaagta aactatgagt 60
cacagcnttc agcaagacat canacncgga anagnganca atattcacta agtaaaatnc 120
agcanatgan atgtctntca catgtatatt naattattca tgctttttca atagtctntt 180
agtcaacttt cagngtaatt tccacaaata tatagcagnt caaacncaaa tgcaggancn 240
caanggcaaa gttnggcaac tgtttngggc taattatgag tntgaaagaa anccttatat 300
cacagtttca cgttcatgta anccactgng caacatgaat gaatntttaa angngttgac 360
nctgaaatca angtncaact aangaaanta aagaanaaaa gggggcttta aaatattngt 420
ngcnctacag tcgtatagta agaggcagaa aaaaatgaan gaattttaaa taatcttaca 480
cgtgtntaca gggccaggaa cgtaatgaat ccatgttaac ttaatttcat ttaaaattnc 540

atttgtagaa gtcncncaac agaaagatcc atgcggttga acagtgtgcc tgtncttgac 600
aagtgagaga agatccttct ccaaaaggga gattcagtct agggntactt cagttnttcc 660
catagnggct acagggcana atctttttca aaagcaattt tctggtccct aaatctacag 720
gcnctantgg gacctgtaat taaaancccc caattttaag gangattttt aaaccccact 780
taagctttta 790

<210> 414
<211> 1063
<212> DNA
<213> Homo Sapiens

<400> 414
gnnnnntncn gccanncnan agnntgntca cctccnagat nngggatggn ntggtgaccc 60
nggcnttgac tctgnnnngc gacntnttgc tagtcttcag gnctcctact acaggctttg 120
taatganctn nacttgnctt gagacagcct angggagacc acggatgntc tattannngn 180
gcangctgnn ctatngcaan ntgggnctna nnctgnanaa tcannngcng ccatgnnaga 240
tnaatagaag ctcatnntgt cataaatggn ccatgactta taaatnaagt ggactggata 300
tcttatgaca gnagcnatnt angcttngtg ngnagttaan gcttccacct nnggangata 360
agaggncnac cttgtntnan ctnntgcngc tgnaagancc agaganannt gccntgggag 420
attcatggcc natgatagta tatnatctct tacaccanat atgccttgct gnatcncaaa 480
tctggacata cacgntttcc ccatctcaga cttcnttgca gcagctgctt nccnacnnta 540
cccatgaacg acanntgctt acgntanagc ntgaacnatn tgatgagctt cntcagccca 600
gacctcatca tttcgagaag cacatgtccc tgcgtttcaa cctatggatg aggaaaagnc 660
ctngngctta aagctcttga aaatccttta cacnngaanc nttctgcata gcttnaatca 720
ctctgagntg cccacatngn gtnctggaag gcttccggnt annatggttc cgggacctnc 780
aacccttccg tttgaatnct nacntgaccg ganagggtnt gcctgggttc cttgngccnc 840
gaacttaacc ntcacaattn ggntgngant tcntggtaac ggcntaatct nccccaggaa 900
ttggccgctg cttcnacggg aattaanggg aatctttccc atcccnctta nnaccagtta 960
ggngcccntt tttcaatttt cngactcccg gagcttttaa aaaccggggg ccttaggttn 1020
cttggatggc nttgggggtn gcccccttta gggaattaaa ggg 1063

<210> 415
<211> 824
<212> DNA
<213> Homo Sapiens

<400> 415
gtttgattnt aacaaaannt attatgcaca aatnacnnag gntanagact ctnncatctn 60
anatnaaaat ancagttata attacacaca taatataggt accttataca atgattccaa 120
taaatatcac aggaaataca ntgcattttc aagntgnana gacnaatact tnctcattca 180
cagngnttga catanganag cctatttaca tancnatctg tataaagtca tgctctnant 240
ancaggntat ncagngctgn gccancacaa tgntttnaga angtgaagaa ccggncaaac 300
cactnntggn gctggggatc tgganaagcc acctgnanaa gcttcactct gagcangact 360
cannaatgnc ttgngccctt taggtggcac tggctgtgga agtggttaag ctgctgctga 420
actcaattcg tggactgnag aattaggaat ggganccagg cggttnggat gaccattgcc 480
cactcnanca natnccaaag nnctnagaan gggaacnctc caancctgct tnatggngat 540
taancatnct tcttcttttg cttaacccat ggattananc acancagcna gtacngactt 600
ggntttaccc ncttcngttg gaaataagga ttcttgatng actaaannnc agctggtnaa 660
aacntaactn tccctcaatt tagcnttatt ntatgaancc ggggcctant ntcntgttca 720
aaaangngnt tttaagttcc ggtaatccta ccggnaatta nttgggggct ntgaattcan 780
cncccttana anatttnggn ttaccatttn aatccaaagg ccac 824

<210> 416
<211> 838
<212> DNA

<213> Homo Sapiens

<400> 416
ctcaaaagtg gaaaatatgt acaatctgta atgagctttt tcctgaaaat gtctatagtg 60
tgcacttcga aaaagaacat aaagctgaga aagtcccagc agtagccaac tacattatga 120
aaatacacaa ttttactagc aaatgcctct actgtaatcg ctatttaccc acagatactc 180
tgctcaacca tatgttaatt catggtctgt cttgtccata ttgccgttca actttcaatg 240
atgtggaaaa gatggccgca cacatgcgga tggttcacat tgatgaagag atgggaccta 300
aaacagattc tactttgagt tttgatttga cattgcagca gggtagtcac actaacatcc 360
atctcctggt aactacatac aatctgaggg atgccccagc tgaatctgtt gcttaccatg 420
cccaaaataa tcctccagtt cctccaaagc cacagccaaa ggttcaggaa aaggcagata 480
tccctgtaaa aagttcacct caagctgcag tgccctataa aaaagatgtt gggaaaaccc 540
tttgtcctct ttgcttttca atcctaaaag gacccatatc tgatgcactt gcacatcact 600
tacgagagag gcaccaagtt attcagacgg tcatccagtt tgagaaaaag ctnacctaca 660
aatgnatcca ttggcttggt gngnatacca gcaacatgga ncggctnaac tatcacttct 720
gnatctagnt cactggangg gccgtttggn aagganccca aatgggccag gataagacaa 780
aaggcnccct ttngggttaa tcagncttcc aagtctngca cctgtgnaac gcacttac 838

<210> 417
<211> 880
<212> DNA
<213> Homo Sapiens

<400> 417
aagcacaata cagcaattta tttagatgct taaaatgaat acaaagggaa aataaagatc 60
acaaaattat acatactaca acagtgtgtc atatattaga tggtataaat gaatccacca 120
tgatggtgtt gaactaaaga taaaactaaa tatccaaaat gcagcactca ttggtttgct 180
gcttcaacac aacacacttt tatacagatc taaaaggtgt caaaattagt agctgcaaag 240
tcaattcttg catgtgattt tagcttaaaa gatttcagaa aacagatctg aaataccagt 300
ttttgttttt gacagctgta atgtcaagga tattcagaac aagaaaaatc ctataataca 360
agagagtcca gatatatatc ttacgtggct ggcctctgtt gcaagattgt acaaggttat 420
gtgcaaaaac taagtctgtc caaaaagtcc atactagcgc agttttgagc ttttgctagg 480
taaactagat agagcgttta ttacacagca agggcaacac taaaaaaaga aatctatgat 540
gggcacacag taacaggatc atgagcatca cttgaatagg tctaaaagac tgtcaaatat 600
acatttcaac tattcagaat gaatacatga aaaaaaatcg cttttcccaa aggtctacta 660
tacncattan actgggagct tgnatgttgg gccctacact accatgggga attangttta 720
acacttntta aaaacatttg gccaatcatt tcncagangg gaaagaaatg ttgaaaaggc 780
cgataaaata aacccttggg ttttcctcgg gggattcatg gagtcacccg ccttaatggg 840
ttttcacatt taagttaccc gggcttggca aaaaaaggtt 880

<210> 418
<211> 763
<212> DNA
<213> Homo Sapiens

<400> 418
agaagatggc ggaagcggaa tttaaggacc atagtacagc tatggatact gaaccaaacc 60
cgggaacatc ttctgtgtca acaacaacca gcagtaccac caccaccacc atcaccactt 120
cctcctctcg aatgcagcag ccacagatct ctgtctacag tggttcagac cgacatgctg 180
tacaggtaat tcaacaggca ttgcatcggc cccccagctc agctgctcag taccttcagc 240
aaatgtatgc agcccaacaa cagcacttga tgctgcatac tgcagctctt cagcagcagc 300
atttaagcag ctcccagctt cagagccttg ctgctgttca ggcaagtttg tccagtggaa 360
gaccatctac atctcccaca ggaagtgtca cacagcagtc aagtatgtcc caaacgtctg 420
tagaaattct tatggactgg aatcttcctc aaggcttact ttgttcctgg gatgcagtgg 480
tgcatagaag atagggcatt gactcactca gacctggctt gcccagcatg cattgcaaca 540

ataatgtgca agttattaaa gacatgagtg aattcgtgac agattgtcag aaaagaaaca 600
agagttttct acaacaaaaa actggcttat ggaacatata cttctgcttg agttgaatgt 660
gttggggctg agtgtaagaa aatgcaagct gcaaatctgg cttacatgtg gaaccaaagc 720
tggaaatgng tgctttaaan gcaacttgta aaattggatt tcc 763

<210> 419
<211> 753
<212> DNA
<213> Homo Sapiens

<400> 419
   ggactattta cttttaatgt aattatcaat acagtcggtt taaatctacc attttgttgt 60
   tggttttcta tttgtttcat ttgttctctc ttcctttttt tcacctcttc aggattattt 120
   tggattcact acttttttta nagngtcgtt ttaccactac tattggccta ttacctgtat 180
   ctcttttttt taatggcatt tctctaggat ttacaatatg catctttagc ttatagtatc 240
   ttgaaatagt agngtaacac ttcacaaata gagtaaaaac cttataatct tccatttttc 300
   ccttccttct tttgtgctat tgatgacnca tatttactcc tacagatatt ataaacaaat 360
   tgatatacnc acattatcat ttttgcttta catactcaat tatcttttaa ataaaataaa 420
   aattgaggag aaaatccgtt atattatcta cacatttact gtttccagca cttttcattt 480
   ctttgngtag attcaaattt ctgncatctt ccctttgccc aaagaacttc ttttcatctt 540
   tcttatagtt caggtctgct ggcaaccaat tagctcagcc tttggtttgc taaaaaagtt 600
   catatattat cttgattttc aaatggnatt taagctctat ataggaattc ttaggtgact 660
   ttaattcctt catcattggg aagangtcat aaagggcttg caaaggacta gaaatctgct 720
   tacatttttt natttggtaa tctttcttac cca 753

<210> 420
<211> 799
<212> DNA
<213> Homo Sapiens

<400> 420
gaaaaacgct ttgataccaa gaattaaaaa tgcttgtcta caaacatctt cccttgcggt 60
tcgtgtaaat tcattagtgt gcttaggaaa gattttggaa tacttggata agtggtttgt 120
acttgatgat atcctaccct tcttacaaca aattccatcc aaggaacctg cggtcctcat 180
gggaatttta ggtatttaca aatgtacttt tactcataag aagttgggaa tcaccaaaga 240
gcagctggcc ggaaaagtgt tgcctcatct tattcccctg agtattgaaa acaatcttaa 300
tcttaatcag ttcaattctt tcatttccgt cataaaagaa atgcttaata gattggagtc 360
tgaacataag actaaactgg agcaacttca tataatgcaa gaacagcaga aatctttgga 420
tataggaaat caaatgaatg tttctgagga gatgaaagtt acaaatattg ggaatcagca 480
aattgacaaa gtttttaaca acattggagc agaccttctg actggcagtg agtccgaaaa 540
taaagaggac gggttacaga ataaacataa aagagcatca cttacacttg aagaaaaaca 600
aaaattagca aaagaacaag agcaggcaca gaagctgaaa agccagcagc ctcttaaacc 660
ccaagtgcac acacctgttg ctactgttaa acagactaag gacttgacag acacactgat 720
ggataatatg tcatccttga ccagccnttc tggtagtacc cctaaatctt ctgcttcaag 780
tctttcactt ctggtcctt 799

<210> 421
<211> 770
<212> DNA
<213> Homo Sapiens

<400> 421
gttcaatatg ggggacattc tggctcatga atctgaatta cttggactag tgaaagagta 60
tttagatttt gctgaatttg aagacacctt gaaaacattt tcaaaagaat gcaaaataaa 120
aggaaaacca ttgtgtaaaa cagtaggcgg atctttcaga gactccaaat cattgacaat 180

tcagaaggat cttgtcgctg catttgacaa cggagaccag aaggtgttct tcgatctgtg 240
ggaggagcac atttcaagtt ccatccgaga tggggactcc tttgcccaga agctggaatt 300
ctatctccac atccattttg ccatctatct tttgaagtac tctgtgggga gaccggacaa 360
agaggagctg gatgaaaaga tttcctactt caaaacctac ctggagacca aaggggcagc 420
cttgagccag accacagagt ttcttccttt ctatgccctt ccttttgttc ccaaccctat 480
ggtgcacccc tcatttaaag aactcttcca ggattcctgg actccagagt taaagttgaa 540
gttggaaaag tttctagctt taatatctaa agccagcaac acgccaaagc ttttaacaat 600
atataaggag aatgggacan agtaacaaag aaatcttgca gcagcttcac cagcagctgg 660
ntgaagcttg aaccgtaggt caatgacata cctcaaacgg naccataaga tccaggcccg 720
actaccacaa tctcantgga gtcacagcan aactggtggg attctcttga 770

<210> 422
<211> 733
<212> DNA
<213> Homo Sapiens

<400> 422
caaaangaan gctttatttt gaattttaaa aatacataca tcttacactg taatcaaaac 60
aaagcttaag aaagtcaatt cccgcttcct ttagccctga cttacactgg gtacccgttt 120
ctgtggccgc cgggggtgac ggncctttgc aggggctcat ccccgctcca ctgcacatta 180
gccagcccct tccgccttgt cttccccgng ttggtcatga tccccaggta ctccgnggtc 240
anaagcttct ctcctgagag ttctccgagc tggggctgga tcagttcgtc tttgtccana 300
tcggcttcca tgatgtcatg gncctcttca tcatcttcat cttcatcatc atcagattca 360
agaacaccat ctggtagctc ttcggaattt agctgcttga tgatgaattc tatctggcgg 420
atcatttcag cattgccttc tttgatgaag cagcgtagga tgtcttccat tcccattgct 480
cttgcttcct cacgaatgga tggancagaa aggatgctgt acagagctcc attcacatac 540
ggctgtatct catggttttc atggccaaga agatccgaaa ggactttgag caccgaggcc 600
tgccaccttg gcacacatgg tcttccctgn gctgcggagg gcagaggttc atggagcaaa 660
agccaccgag tactccaacg gggnagccag acagggcagn cagggtcctt tcanaacatc 720
aacccagccc gaa 733

<210> 423
<211> 862
<212> DNA
<213> Homo Sapiens

<400> 423
catctgtcca gggtgcatcg agccgggagg gctcaccagc caggagcagc acgccactcc 60
actccccctc gcccatccgt gtgcacaccg tggtcgacag gcctcagcag cccatgaccc 120
atcgagaaac tgcacctgtt tcccagcctg aaaacaaacc agaaagtaag ccaggcccag 180
ttggaccaga actccctcct ggacacatcc caattcaagt gatccgcaaa gaggtggatt 240
ctaaacctgt ttcccagaag cccccacctc cctctgagaa ggtagaggtg aaagttcccc 300
ctgctccagt tccttgtcct cctcccagcc ctggcccttc tgctgtcccc tcttccccca 360
agagtgtggc tacagaagag agggcagccc ccagcactgc ccctgcagaa gctacacctc 420
caaaaccagg agaagccgag gctcccccaa aacatccagg agtgctgaaa gtggaagcca 480
tcctggagaa ggtgcagggg ctggagcagg ctgtagacaa ctttgaaggc aagaagactg 540
acaaaaagta cctgatgatc gaagagtatt tgaccaaaga gctgctggcc ctggattcag 600
tggaccccga gggacgaagc cgatgtgcgt caggccagga gagacggtgt caggaaggtt 660
cagaccatct tggaaaaact tgaacagaaa gccattgatg tccangtcaa gtccaggtct 720
atgaacttca agccaagcaa ccnttgaagc agatcaagcc cctggaggca atcatggaaa 780
agggtgccgt ggcagcaaga caagggcaag aaaaatgctt ggaaatggcn gaagatcccc 840
acacnggaaa ccagcaggcc cg 862

<210> 424
<211> 859

<212> DNA
<213> Homo Sapiens

<400> 424
gagttatatt attactttat tttctttttt taaaatgtag cattaaagtc atccaacata 60
cagatattcc tatggctcct ggcacatttt actctctcta aagtcaggta ttttaattat 120
gagatgaaga aaatcatctc attaaaatgg caacatttct gataaatgtt tcatatttat 180
gtgatgggta attgactccc catctacccc tccagtccag agctacaaaa gacagtgcac 240
aaccacagct aacaggtggt gggggtgccc aagtagacag ggctgcagaa caagcaacgg 300
ggttaaactt ctcaaacaac aagcaacttc tttatttgta cagagtaaga atatagaaga 360
aaagcatcat tttccttttt agccctttta ttagtgtttt gcctccaccc aagttactgc 420
ataccaagca gctaataaaa accaactgac ttaaagtctc tgaaatgcat gcaacttaaa 480
attccctaaa gcacacatcg gttccgagtc tgatttttac agggcagagg ctacggtgct 540
gctgggttac caggggtgtc tggcatgctg ctggggtttg aagtcgctgc tgctgnggct 600
tctggctgct gggtttctgt gtggggatct ttctgcattt ccagcatttt tcttggcctt 660
ggctgctgcc acggnaccca tcttcatgaa tgcctgcaaa tggctggacc tgnttcaaag 720
gttgctgggg ctggagttca ttagacctgg accttggccc tgggacatca aagggctttc 780
tggtcaaggt ttttccaaga agggcctgga accttcctgg acancggntt tttctgggcc 840
tggacgcnca attggggtt 859

<210> 425
<211> 837
<212> DNA
<213> Homo Sapiens

<400> 425
cagaatggag gtggagtccc taaacaaaat gcttgaggag ctaagacttg aacggaagaa 60
actaattgag gattatgaag gcaagttgaa taaagctcag tccttttatg aacgtgagct 120
tgatactttg aaaaggtcac agctttttac agcagaaagc ctacaggcca gcaaagaaaa 180
ggaagctgat cttagaaaag aatttcaggg acaagaagca attttacgaa aaactatagg 240
aaaattaaag acagagttac agatggtaca ggatgaagct ggaagtcttc ttgacaaatg 300
ccaaaagctt cagacggcac ttgccatagc agagaacaat gttcaggttc ttcaaaaaca 360
gcttgatgat gccaaggagg gagaaatggc cctattaagc aagcacaaag aagtggaaag 420
tgagctagca gctgccagag aacgtttaca acagcaagct tcagatcttg tcctcaaagc 480
tagtcatatt ggaatgcttc aagcaactca aatgacccag gaagttacaa ttaaagattt 540
agaatcagaa aaatcgagag tcaatgagag attatctcaa cttgaagagg aaagagcttt 600
tttgcgaagc caaaacccaa agtctggatg aagagcagaa gcnacagatt ctaagaactg 660
ggagaagaaa gtaaatgaac caagagactc agcaggaata ttatgaaagg gaacttaaaa 720
anctgcaagt agaatggaag aagaggggct taattaacga nggccattct aagacttttg 780
gaagaattag cttggaacnc cttttggcaa ttgaacttgt cncaggtaat gccattt 837

<210> 426
<211> 724
<212> DNA
<213> Homo Sapiens

<400> 426
gattctaaca aaatttatta tgcagtaatt acaaaggtta aagactcttc catctcaaat 60
aaaaataaca gttataatta cacacataat atagtacctt atagaatgat tccaataaat 120
atcacaggaa atacagtgca ttttcaagtt ggagagacaa atactttctc attcacagtg 180
tttgacatag gaaagcctat ttacataaca atctgtataa agtcatgctc ttagtaacag 240
tctatacaga gctgtgccaa cacaattctt tcagaatgtg aagtaccggg caaaccactc 300
ctggcgctgg ggatctggag aagccactgg agaagcttca ctctgagcag gactcaaaaa 360
tgtcttgggc cctttaggtg gcactggctg tggaagtggt ttgctgctgt tgaactcaat 420
atcgtggact ggagaattag gaatgggatc caggcggtta ggatgtccat tgcccactcc 480

accagattcc agagcactta nattgggaac actcacaaac ctgtttgttg gtgatttatc 540
attcttcttc ttttgcttag ccaatggatt aataacacca acagtaggac ttgagttaaa 600
cactttggtg aaagttagtt tctcgaattg actaattcca gctgataaaa cttattatcc 660
tcaattagtt tctttatgan ctgggcctct ttctgtaagc atggctttta attctggaat 720
catc 724

<210> 427
<211> 981
<212> DNA
<213> Homo Sapiens

<400> 427
aaaaacaaaa taaaacaaaa aanaaaaant atatatatnt atatatatat atatatatac 60
acacacacac acacaactca aagagttana atcattacnt ncaaatgaaa gtcgtaatga 120
tagatgatga tagntncaat gaanctgnga ncatanatta angaaacana naacantncn 180
aaaggtccac aaatctggtc ctatgaaaag agtaaaatta ccaagactng gtgaaaganc 240
ccannaaaan ncanagagag anagagagag agagaganac anagagagag aganaaaggg 300
aaggcacacn taancnatat cagcaataaa angggnnact ttantacana ttctgcaanc 360
attannnnna taatganagg atattatgaa cagttgtatg gcnatatgtt tgaaaactta 420
gatgccgata tgtttgaaaa cttaaatgaa acggaaaaat tccttgaaga accacaantt 480
aaatttgaca caggtagaaa atntgaatgc agttngncct tcagtatctg tggggaaatc 540
ggttncagaa ccactccccc antaccnaaa tttataattg ctcaagttcc tgatataaaa 600
tggcaaagta tttgcatata ncctatccct acccttttac atactttaaa taacctntga 660
gttncttnat tatacctaac ataatgtaca tttctgtggc aaatcgntnn taatattgga 720
ttttnaaaat tatnttantt ttggaatagg nngtantatt tcctggggct ttttttttcc 780
ccaaatattt tntaattccc caattnggtt ggaatcttgg gaaccccatg gnggggancc 840
catangattt tgggaanggn ccaacttggg gccttngtaa ctttttaaag aaatngggaa 900
ttctttgntn aanaattctt ncncccaaag aaaacccctt tggccccana agttntttna 960
aatggggaaa tttncccaaa c 981

<210> 428
<211> 655
<212> DNA
<213> Homo Sapiens

<400> 428
ataggacaac atgaacattg ttgagtcact gaagctaaac caaacttgct tttctgtaat 60
aaacccaatt tggtcatgat ttaatatttt ttggatcgct ctggatttgg tttgctaata 120
ttttattcat ccaagaaata ttcattagag aaattggcat gggatttttt tttcattgta 180
atgtccttgt caggtatcaa ggctttttca gcctgataaa gcatattaag aaatgcttcc 240
tcttttccta ttctctggaa aagattgtgt aatattgctg ttactacttc ctgtaatgtt 300
tggtgaaatt cacaattgaa gacatctggg cctagcgtgt tctttgtagg aagaatatta 360
agaaagaatt ccatttcttt aaaagttacg agcacagttg gccttccaga tctatggatc 420
ccacatgagt tccagattca accaattgtg tattaaaaat atttgggaaa aaaagccaca 480
agaaataata caactataca aaataatata atttttaaaa tacaatataa caacgattta 540
cacagaatgt nccattatgt taggnattat aagtaactca gaggntattt aaagnatgtg 600
agaggnnatg gataggctat atgccaaata ctttgccant cttatantca gggaa 655

<210> 429
<211> 788
<212> DNA
<213> Homo Sapiens

<400> 429
gagcagcaga gatttttgct gtgagaatta attaccagta acagttcaat atgggggaca 60

ttctggctca tgaatctgaa ttacttggac tagtgaaaga gtatttagat tttgctgaat 120
ttgaagacac cttgaaaaca ttttcaaaag aatgcaaaat aaaaggaaaa ccactgtgta 180
aaacagtagg cggatctttc agagactcca aatcattgac aattcagaag gatcttgtcg 240
ctgcatttga caacggagac cagaaggtgt tcttcgatct gtgggaggag cacatttcaa 300
gttccatccg agatggggac tcctttgccc agaagctgga attctatctc cacatccatt 360
ttgccatcta tcttttgaag tactctgtgg ggagaccgga caaagaggag ctggatgaaa 420
agatttccta cttcaaaacc tacctggaga ccaaaggggc agccttgagc cagaccacag 480
agtttcttcc tttctatgcc cttccttttg ttcccaaccc tatggtgcac ccctcattta 540
aagaactctt ccaggattcc tggactccag agttaaagtt gaagttggaa aagtttctag 600
ctttaatatc taaagccagc aacacgccna agcttttaac aatatataag gagaatggac 660
aaagtaccaa gaaatcttgc agcagcttca ccacagctgg ttgaagctga acgtaggtca 720
gngccttcct taaacgggcc aattaagaat ccaggccgac taccacaatc ttantggggg 780
tcccagca 788

<210> 430
<211> 655
<212> DNA
<213> Homo Sapiens

<400> 430
caaaatgaat gctttatttt gaattttaaa aatncatacn tnttacnctg naatcaaaac 60
aaagcttaan aaagtcaatt cccgnttcct ttanccctga cttacnctgg gtncccgttt 120
ntggggccnc cgggggngac gggcctttgc aggggctcat ccccgntcca ctggacatta 180
nccagcccct tccgccttgg cttccccgng ttggtcatga nccccaggtn ctccgnggtc 240
aaaagcttnt ntcctgaaag ttctccganc tggggctgga tcanttcgtc tttgnccaaa 300
ncggnttcca tgatgncatg ggcctnttca tcatcttcat tttcatcatc atcanattca 360
anaacnccat ntggnanctt ttcggaattt aactgcttga tgangaattc tatntggngg 420
ancatttcag cattgccttn tttgaagaac cancgtagga nggtttccat tcccattggt 480
nttgnttcct cacgaatgga tggaacanaa aggatgctnt acananctcc attcacatac 540
ggntgnatnt catggntttc atggccaana anaatcccaa aggctttgag cccaggnctg 600
gcccttggca caaatgttnt tcctggcttc cgaaggccaa ggttcattga ccaaa 655

<210> 431
<211> 844
<212> DNA
<213> Homo Sapiens

<400> 431
ggaagaagga agaggtaact ataactaccc aatattgcag ccatggagtc catgcttaat 60
aaattgaaga gtactgttac aaaagtaaca gctgatgtca ctagtgctgt aatgggaaat 120
cctgtcacta gagaatttga tgttggtcga cacattgcca gtggtggcaa tgggctagct 180
tggaagattt ttaatggcac aaaaaagtca acaaagcagg aagtggcagt tttgtctttt 240
gataaaaaac tgattgacaa gtatcaaaaa tttgaaaagg atcaaatcat tgattctcta 300
aaacgaggag tccaacagtt aactcggctt cgacaccctc gacttcttac tgtccagcat 360
cctttagaag aatccaggga ttgcttggca ttttgtacag aaccagtttt tgccagttta 420
gccaatgttc ttggtaactg ggaaaatcta ccttccccta tatctccaga cattaaggat 480
tataaacttt atgatgtaga aaccaaatat ggtttgcttc aggtttctga aggattgtca 540
ttcttgcata gcagtgtgaa aatgggtgca tggaaatatc actcctgaaa atataatttt 600
gaataaaagt ggagcctgga aaataatggg ttttgatttt tgngtatcat caaccaatcc 660
ttctgaacaa gagcctaaat ttccttgtaa agaatgggac ccaaatttac cttcattgng 720
tcttncaaat cctgaatatt tggcttctga atcctacttt ctgngaactt gtgaaaccag 780
ccagtggata tgggattcnt ttaggaactg gtatggaatg ccgggatttt aataaaaggg 840
gaaa 844

<210> 432

<211> 807
<212> DNA
<213> Homo Sapiens

<400> 432
atcaaagcta aaatttattt ggtgcatact cctcttgata tcaggtatgt tcgcatatac 60
ctttttcttt catgtgtaaa aacaaccatg tgaggtattt tacaggtcaa aagaaaacaa 120
aaactacttc cttattcagt gtaaaggagg cttataagca ttccaaaata aaaacaaaca 180
aaaaccagac aagtacatag tctatttcca tttcctttta tacatcctct ctatatatca 240
cacatttagc aataggagaa tagagaacta attcaaatgc aagggaatct tttttgtaga 300
ttctgttgac agatgctctt taacctaaac attttctact ctaaacataa cggacttaat 360
tgtcttcagt acgtgaaata attttaaggt gatctagtac tttgaaaatt tcattcactt 420
aagaacactt aagctgaaaa atagcactat ttttcagagg caatttctca acagaaaaag 480
gcaatggtaa cagttcaatt gatggaaatg gttgaaataa aatacctgaa gtagaaaaaa 540
ggtgtaggaa caattttgta aaaacatagc accattacct caacgaatga acaaatttta 600
catactggat ttttttcaaa tgacttattt tcatatttag tagttcaagg tctataagct 660
ggtatattaa gctttctttc tggttaagag ntcaacactt acatcatggt attttacnaa 720
attaaaaacc aatttcttaa ataaaccgng gctcctaaaa tggtaccaag gaaaaattct 780
tcaataccta atttaattcc ataagga 807

<210> 433
<211> 866
<212> DNA
<213> Homo Sapiens

<400> 433
cttcagccca gatgcagaat gggggcccct ccacaccccc tgcatcaccc cctgcagatg 60
gctcacctcc attgcttccc cctggggaac ctcccctgtt agggaccttt ccccgggacc 120
acacctcttt ggcactagtt cagaatggtg atgtgtcggc cccctctgcc atactcagaa 180
caccagaaag cacaaaaccg ggtcctgttt gtcagccacc agtgagtcag agccgctccc 240
tgttttcttc tgtcccgtcc aagccaccaa tgtctctgga gcctcaaaat gggacgtatg 300
caggaccagc gccagcattc cagccatttt tcttcactgg agcatttcca tttaatatgc 360
aagagctggt actcaaggtg agaattcaga acccatctct tcgagaaaat gatttcattg 420
aaattgaact ggaccgacag gagctcacct accaagagtt gctcagagtg tgttgctgtg 480
agctgggtgt taatccagat cangtggaga ngatcagaaa gttacccaat actctgttaa 540
ggaaggacaa ggatgttgct cgactccaag atttccagga gctggaactg gttctgatga 600
taagtgaaaa taattttctg ttcanaaatg ctgcatccac actgactgaa aggccttgct 660
ataacaggag agcttcaaaa actgacttac taatgcacag ggacttttat cactggagta 720
ttatgacagt gngcatcacc ttntgggccc aaggaccaag ccattggtct aaaaggcctc 780
aaaatgcccc ggganggcct ctggtggcca tggcattagt atatactaac catcattctg 840
gccaggtaag gaagcccctg gacccc 866

<210> 434
<211> 764
<212> DNA
<213> Homo Sapiens

<400> 434
caaaataacc tttatttttg atacaaaaat aaagatgcta actcctttag ctcagtttcc 60
cacaataacc tttaaaatag caacagattc agtctcaaaa attgcttttc atttgtagtg 120
gaaaatgaaa gtggagaaca tggaacagca atatttgngc tcttctcata ggatgcagtt 180
acacacacat atgactggaa tcacttcaga gtaaaaaaaa agtgggctgg gtgcagtggc 240
tcacacctgt aatcccagca ctttgggagg ccaaggacag gagcatcact taaggccaga 300
agtttgagac cagcctgggc cacatagtga gaccctgtct ctatgggcgg ggtgggggtg 360
gggggcattg taaaaaagca gttgttcttt tanaaggcat cagagagccc tntagtgacc 420

acgaagggga gttaatgcag agatgactcg agacagagaa gcagtcatga gtgtttacaa 480
aggaaaaagt gagggaggga aagctctttt ggttaacagc atatttacaa ttagttaact 540
gnattcttaa atacttttaa cctgagtaac atttataaat atgttatagg aaacctcaca 600
gtcacaagtc acactagaat ccatctgtcc agtatctggg ctttccccac accagaatcc 660
atctgtccag tatctgggct ttcccgagtc ttcctcttct cataagttcc caanggcagc 720
anaagtgtga agcatgcaca ccaaggaaaa acgcattcca gccc 764

<210> 435
<211> 834
<212> DNA
<213> Homo Sapiens

<400> 435
agattttgtt aattttccta caaaaaatgg atttgctact aaccaaaacc cctcctgatg 60
agataaagaa cagtgttcta cccatggttt acagagcact agaagctcct tccattcaga 120
tccaggagct ctgtctaaac atcattccaa cctttgcaaa tcttatagac tacccatcca 180
tgaaaaacgc tttgatacca agaattaaaa atgcttgtct acaaacatct tcccttgcgg 240
ttcgtgtaaa ttcattagtg tgcttaggaa agattttgga atacttggat aagtggtttg 300
tacttgatga tatcctaccc ttcttacaac aaattccatc caaggaacct gcggtcctca 360
tgggaatttt aggtatttac aaatgtactt ttactcataa gaagttggga atcaccaaag 420
agcagctggc cggaaaagtg ttgcctcatc ttattcccct gagtattgaa aacaatctta 480
atcttaatca gttcaattct ttcatttccg tcataaaaga aatgcttaat agattggagt 540
ctgaacataa gactaaactg gagcaacttc atataatgca agaacagcag aaatctttgg 600
atataggaaa tcaaatgaat gtttctgagg agatgaaagt tcaaatattg ggaatcagca 660
aattggcaaa gtttttaaca acattggagc agaccttntg actggcagtg agtccgaaaa 720
taaagangac gggttacaga ataaccttaa aagagcatcc ttaccacttg gaggaaaaac 780
caaaatttgc caaaagaacc aggaccggcn ccgaagctgg aaaagccgca ggct 834

<210> 436
<211> 812
<212> DNA
<213> Homo Sapiens

<400> 436
acagaagtaa agtttattac atttgaaaca atacagcaga aacctcaaaa gtttactcat 60
aaatatagtt taattcttac aaatcttctt ttgaaaatgc aattcatata tgctgcaacc 120
tcagaagttt gaatttgaaa tgaaatatga aggtagtagt cagggaagtc acatcagagt 180
gccttgtcaa atatccaaac aaatcagcac atacctcttc cttgatacag gaggaaaaaa 240
gtgattctaa atatatccaa gtgaatgcag aaaaatacat tactatttga ggcagaccat 300
gctaaaatat aatttacaat gattagtttg cacttaagat ggttaataac gcatttaaac 360
caatgaaatg aaggttaagt tgaattttgt agtatttgct cagtctctgt actaaacaat 420
agttcatctg aaaagtttgg aaaaagcaaa taacctgata cttctcttta tgcttatcat 480
tttctcactg tcatcttaaa tgcaaacaaa tcaatacagc atcaagattt tttacatatt 540
aaaatgaaga ctaatgactc atagactgng taccatatag tacttaatag atgagcttgc 600
aatgaccatc acctcaattt tttaaataac accaagatcc acaagccaaa ataaacattt 660
gattaaaaag ttatggtatt caagataact cagtttcctt tttctctttg agattgggna 720
anggctgggt ctttaaaaaa ccctggaaaa gggagttggg taaagaggga aaaaaatcct 780
tcaangcttt taaaaaaact tcnactgggt ta 812

<210> 437
<211> 842
<212> DNA
<213> Homo Sapiens

<400> 437

gtggaagagg cgtacctatt tgcaaagtgc agagcaggca tggattgcca attctggaac 60
agagcaaagc cccaacttgc cctccactgg tgatgtcaca cccacccatg aagagcctgc 120
ctctagggtt gttgaatgtt gggtcacgaa gatctcaacc tggccaaaga agagaaccca 180
gaaagatcat cacagtttct gtaaaagaag atgtacacct gaaaaaggca gaaaatgcct 240
ggaagccaag ccaaaaacga gacagccaag ccgatgatcc cgaaaacatt aaaacccagg 300
agctttttag aaaagttcga agtatcttaa ataaattgac accacagatg ttcaatcaac 360
tgatgaagca agtgtcagga cttactgttg acacagagga gcggctgaaa ggagttattg 420
acctggtctt tgagaaggct attgatgaac ccagtttctc tgtggcttac gcaaacatgt 480
gtcgatgtct agtaacgctg aaagtaccca tggcagacaa gcctggtaac acagtgaatt 540
tccggaagct gctactgaac cgttgccaga aggagtttga aaaagataaa gcagatgatg 600
atgtctttga gaagaagcag aaagaacttg aggctgccag tgctccagag gagaggacaa 660
ggcttcatga tgaactggaa gaagccaagg acaaaagccc ggcggagatc cattggcaac 720
atcaagttta ttggagaact cttttaaact caaaatgctt gacttgaagc catcattgca 780
tgactgtgtg gtgaagctgc ttaagaaccn ttgatgaaga atccctggaa tggcctgtgt 840
cg 842

<210> 438
<211> 678
<212> DNA
<213> Homo Sapiens

<400> 438
aaactngcan tgtntgtntt tattttgtnc tttatatttt caaagngaaa agaaatanna 60
ctgagncaat ntctttttgt ntttttaaan atttgtncta tgtatttaca ngccttaaag 120
nngctctaaa gatntcaaga gnattaanag nacttttntc agggnagcac tntttttttt 180
ttaaacantt nttggngttc tgtggnccac annatttcct tntgtntcaa ngtnatgtat 240
gtnttgatna cnatngngat nttttaaann ttntgaanca agctgagagg cnngcanaaa 300
gatntgangc cnnaaaaaaa aaaatctttn ttaccttgtn caccccaaac tttttcaaat 360
ctggnctaaa tgctntacct taaaacanac atgaggggca tcttgaaggg gagggaaant 420
tatttctctg cntttctatn atacangtng tttacanaaa ctgngaatta naaaattaca 480
ctggnatttg cngaccttaa aataaattaa aagtnctcaa ctnttttttt ttttgntaaa 540
cnttttttta agnatgannc cntggttaaa aagaaaagnt ttaaaccgaa aatattttct 600
ataaataata cctggatttt ggntttaggg cccccgccct aaggnttgna ggttactttt 660
ntccnangac ctttttcc 678

<210> 439
<211> 826
<212> DNA
<213> Homo Sapiens

<400> 439
gaccctttac caacaaatga aaatgatgat gatatatgca agaaaccctg tagtgtagca 60
cctaatgata ttccactggt ttctagtact aacctaatta atgaaataaa tggagttagc 120
gaaaaattat cagccacgga gagcattgtg gaaatagtaa aacaggaagt attgccattg 180
actcttgaat tggagattct cgaaaatccc ccagaagaaa tgaaactgga gtgtatccca 240
gctcccatca ccccttccac agttccttcc tttcctccaa ctcctccaac tcctccagct 300
tctcctcctc acactccagt cattgttcct gctgctgcca ctactgttag ttctccgagt 360
gctgccatca cagtccagag agtcctagag gaggacgaga gcataagaac ttgccttagt 420
gaagatgcaa aagagattca gaacaaaata gaggtagaag cagatgggca aacagaagag 480
attttggatt ctcaaaactt aaattcaaga aggagccctg tcccagctca aatagctata 540
actgtaccaa agacatggaa gaaaccaaaa gatcggaccc gaaccactga agagatgtta 600
gaggcagaat tggagcttaa agctgaagag gagctttcca ttggcaaagt acttgaatct 660
gaccaggata aaatgagcca ggggtttcat cctgaaagag acccctntgg cctaaaaaaa 720
gtgaaaagct gtggaagaaa atggagaaga actgagccag accgtaatgg ggcctgaaag 780
ggttctgang gtgaaggaat agatgcttaa ttcangcttc cccaga 826

<210> 440
<211> 689
<212> DNA
<213> Homo Sapiens

<400> 440
aaatatttgt tctatgtatt tacaagcctt aaagttgctc taaagatttc aagagtatta 60
agagtacttt tctcagggta gcactttttt ttttttaaac aattcttgga gttctgtggt 120
ccacagcatt tccttctgtt tcaatgttat gtatgttttg attactattg tgatttttta 180
aattttctga agcaagctga gaggcaggca gaaagatttg atgccaaaaa aaaaaaaatc 240
tttcttacct tgttcacccc aaactttctc aaatctggac taaatgctat accttaaaac 300
aaacatgagg tgcatcttga aggggaggga aatttatttc tctgcttttc tattatacaa 360
gttgtttaca gaaactgcaa attaaaaaat tacactggca tttgcagtcc ttaaaataaa 420
ttaaaagttc tcaacttttt ttttttgcta aacatttttt taagtatgag tccttgttta 480
aaaagaaaag attaaaacag aaaatatttt ctataaataa tacatgtatt ttggttttag 540
tgctcccgcc ctaaggtttg aagtttactt ttatccagta cctttttcct ccatgatcac 600
ctttttttct ctttcccctn ttccactcgg gcacacgtgg ggggtttctg cnanaattgg 660
ccttgctgca ctgngaatgg gcnaaaacc 689

<210> 441
<211> 883
<212> DNA
<213> Homo Sapiens

<400> 441
ctttttatcc tggaccagga cctggggact tccccaatgc ttatggaacg cctttttacc 60
caagtcagcc ggtgtatcag tcagcaccta tcatagtgcc tacgcagcaa cagccgcctc 120
cagccaagag agagaaaaaa actataagaa ttcgggatcc aaaccaggga ggtaaagaca 180
taacagagga gattatgtct ggaggtggca gcagaaatcc tactccaccc ataggaagac 240
ccacgtccac acctactcct cctcagcagc tgcccagcca ggtccccgag cacagccctg 300
tggtttatgg gactgtggag agcgctcatc ttgctgccag cacccctgtc actgcagcta 360
gcgaccagaa gcaagctcaa atagctataa ctgtaccaaa gacatggaag aaaccaaaag 420
atcggacccg aaccactgaa gagatgttag aggcagaatt ggagcttaaa gctgaagagg 480
agctttccat tgacaaagta cttgaatctg aacaagataa aatgagccag gggtttcatc 540
ctgaaagaga cccctctgac ctaaaaaaag tgaaagctgt ggaagaaaat ggagaagaag 600
ctgagccagt acgtaatggt gcttgagagt gtttcttgag ggtgaaggaa tagatgctaa 660
ttcaggcttc acagatagtt ctggtgatgg gggtacattt ccatttaaac cagaatnctg 720
gaagcctact ggtacttgaa ggtaagaaca gtatgaccag ggagtttctg gtggactttc 780
cagttcatgc ctggctgnat tccaaaancc naagggcctg gcttctatta anggatgngg 840
ttnttgacag gatcaaccaa ncccaaatgg ccaatgggga act 883

<210> 442
<211> 777
<212> DNA
<213> Homo Sapiens

<400> 442
gctaaacatt tttttaagta tgagtccttg tttaaaaaga aaagattaaa acagaaaata 60
ttttctataa ataatacatg tattttggtt ttagtgctcc cgccctaagg tttgaagttt 120
acttttatcc agtacctttt tcctccatga tcaccttttt ttctctttcc cctctcccac 180
tcgtgcacac gtgggggttt ctgcgagaat tggccttgct gcactgtgat tggcgaagac 240
gtgaaacttt ttaaaaaaat acttaaattg tttcttttgt ttcattttgt gtatttgaag 300
ttttagttat cctcagactc ctcttctgct tcccgcagcc acgtgaagaa tgccgtgaca 360
gatttcagag ccacgccctt cccattctgc tctgcagggt ccttgctgct ctcccatttg 420
tagaaggcat cctcggagat cacctcctcg tcatatagac aatcaaaaaa catccgcagc 480

aaattggcag gttgatcaag ttttactatc gatgcttgta gtgcataaag tgcttgcagt 540
tccttctctg natctgagtc taggtacttg agtaagatcg gcactctctg cttgaaacag 600
cagtgtccac ttcttgaang tagaagaagt cggctattaa tagctggttt acaaacagca 660
gtcatttaaa gctctaagga atggtaggtg aactcntctg ggatttcggc taagaataag 720
ccctttancc aggccaaaga acctggtcan tcaattcgct tttggccctc caataaa 777

<210> 443
<211> 875
<212> DNA
<213> Homo Sapiens

<400> 443
taacacagtg aatttccgga agctgctact gaaccgttgc cagaaggagt ttgaaaaaga 60
taaagcagat gatgatgtct ttgagaagaa gcagaaagaa cttgaggctg ccagtgctcc 120
agaggagagg acaaggcttc atgatgaact ggaagaagcc aaggacaaag cccggcggag 180
atccattggc aacatcaagt ttattggaga actctttaaa ctcaaaatgc tgactgaagc 240
catcatgcat gactgtgtgg tgaagctgct aaagaaccat gatgaagaat ccctggagtg 300
cctgtgtcgc ctgctcacca ccattggcaa agacttggac tttgaaaaag caaagccacg 360
tatggaccag tactttaatc agatggagaa aattgtgaaa gaaagaaaaa cctcatctag 420
gattcggttc atgcttcaag atgttataga cctaaggctg tgcaattggg tatctcgaag 480
agcagatcaa gggcctaaaa ctatcgaaca gattcacaaa gaggctaaaa tagaagaaca 540
agaagagcaa aggaaggtcc agcaactcat gaccaaagag aagagaagac caggtgtcca 600
gagagtggac gaaggtgggt ggaacactgt acaaggggcc caagaacagt cgggtactgg 660
acccctcaaa antcctaaaa atcactaagc ctacaattga tgaaaaaant cactggacct 720
aaagccagct aggcagctgg ggaaaaggca gcagtggtgg accaangcaa gtgaaactga 780
gccntacggc aagtgcttnc agttaaacag atctntgncc tgaaccttca gaaccttang 840
gtcccgccat cacgcctgta aagttggatt cccga 875

<210> 444
<211> 756
<212> DNA
<213> Homo Sapiens

<400> 444
   cttttaaact tgcaatgntt gnctttattt tggtctttat attttcaaag ngaaaagaaa 60
   tagtactgag tcaatttctt tttggttttt taaatatttg gtctatgnat ttacnagcct 120
   taaagttgct ctaaagattt caagagtatt aagagtactt ttctcagggt agcacttttt 180
   tttttttaaa caattcttgg agttctgngg nccacagcat ttccttctgn ttcaatgnta 240
   tgtatgtttt gattactatt gggatttttt aaattttctg aagcaagctg anaggcaggc 300
   ngaaagattt gatgccnaaa aaaaaaaaaa aatctttntt accttggtca ccccaaactt 360
   tntcaaatct ggactaaatg ctatacctta aaacaaacnt gaggggcatn ttgaagggga 420
   gggaaattta tttctctgnt tttctattat acnagttgnt taccgaaact gnaaattaaa 480
   aaattaccct ggcntttgca ggccttaaaa taaattaaaa gntctcaact tttttttttt 540
   gccaaacatt tttttaagta tgagnccttg nttaaaaaga aaagattnaa nccgaaaata 600
   ttttctataa ataatacntg nattttggtt ttaaggctcc cgccctaang nttgaaggtt 660
   acttttatcc nagnnccctt tttccctcca tgaanacccc tttttttcnc ctttcccctt 720
   ttcccacttn gggccccccc tnggggggtt tttgcg 756

<210> 445
<211> 783
<212> DNA
<213> Homo Sapiens

<400> 445
cagaaaatgg tgcttaccaa ctacatgttc cctcaacagc caaggactga ggatgttatg 60

tttatatcag ataatgaaag ttttaaccct tcattgtggg aggaacagag gaaacagcgg 120
gctcaagttg catttgaatg tgatgaagac aaagatgaaa gggaggcacc tcccagggag 180
ggaaatttaa aaagatatcc aacaccatac ccagatgagc ttaagaatat ggtcaaaact 240
gttcaaacca ttgtacatag attaaaagat gaagagacca atgaagactc aggaagagat 300
ttgaaaccac atgaagatca acaagatata aataaagatg tgggtgtgaa gacctcagaa 360
agtactacta cagtaaaaag caaagttgat gaaagagaaa aatatatgat aggaaactct 420
gtacagaaga tcagtgaacc tgaagctgag attagtcctg ggagtttacc agtgactgca 480
aatatgaaag cctctgagaa cttgaagcat attgttaacc atgatgatgt ttttgaggaa 540
tctgaagaac tttcttctga tgaagagatg aaaatggcgg agatgcgacc accattaatt 600
gaaacctcta ttaaccagcc aaaagtcgta gcacttagta ataacaaaaa agatgataca 660
aaggaaacag attctttatc agatgaagtt acacacaata gcaatcagaa taccagcaat 720
tggtcttctc catctcggat gtctgattca gttctcttaa tactgatagt agtcaagaca 780
cct 783

<210> 446
<211> 866
<212> DNA
<213> Homo Sapiens

<400> 446
agattacaac acacatacaa taagtgaatt ttatcaaaat acagcacatt tcttctacta 60
tatccataaa aatcaattcc tatgtaaata gtactgaaaa tcaactaaaa tgagttaaaa 120
tttacaaaga gttgttaaag ggtttcaatc aaaattatta aaactataca gtacaataac 180
caattgataa catcttgaaa gaagtgcaat atttgagttc acatattttt aaaagtgctg 240
cctacttact ctgactagca agaatggaaa gtgagtccaa ctcacttttg caaaaataat 300
gttggttggt gttttaagct agtcttataa aagtcttaat taaaatcaag gttgataaac 360
aaagcataac agattaaaaa ttcccaaatt gcatttctta gtaaataaaa atgaagtgca 420
ataaccaaat attgctctaa tgaaaggttc cagactagcc tcaactaaac agttattggt 480
cttctatggc acttttttct ggtccaaata accatgcatt aatccttacc attacatgtt 540
actcaaattt tatttgatta catagaacaa aaacaaataa aattaatggt ctggataaac 600
aaaattaata aacctctatc atcaaatatt tgttacagta actaggaaca aagaaaggca 660
gtttggtggg taaaacacta ttacactgat ccccatagga aaccccttta aagactctgg 720
aagtgttgag ttcacattta atggtacctg tagaaacagn cctttatttg gacaccttta 780
cccactggca ngccctaang gacccatccc tttgctctat aacttttcac aagcaattct 840
ctaatcctgg gccagtttnc aaaagc 866

<210> 447
<211> 789
<212> DNA
<213> Homo Sapiens

<400> 447
gtcacgttgg aatgcaaatt gagcacatca ttgaaaacat tgttgctgtc accaaaggac 60
tttcagaaaa attgccagag aagtgggaga gcgtgaaact cctgtttgtg aaaactgaga 120
aatcggctgc acttcccatc ttttcctcgt ttgtcagcaa ttgggatgaa gccaccaaaa 180
gatctttgct taataagaag aaaaaagagg caaggagaaa acgaagagaa agaaattttg 240
aaaaacaaaa ggagaggaag aagaagaggc agcaggctag gaagactgca tcagttctta 300
gtaaagatga tgtggcacct gaaagtggtg atactacagt gaagaaacct gaatcaaaga 360
aggaacagac cccagagcat gggaagaaaa aacgtggcag aggaaaagcc caagttaaag 420
caacaaatga atccgaagac gaaatcccac agctggtacc aataggaaag aagactccag 480
ctaatgaaaa agtagagatt caaaaacatg ccacaggaaa gaagtctcca gcaaagagtc 540
ctaatcccag cacacctcgt gggaagaaaa gaaaggcttt gccagcatct gagaccccaa 600
aagctgcaga gtctgagacc ccagggaaaa gcccagagaa gaagccaaaa atcaaagaag 660
agcagtgaag gaaaaaagtc cttcgctggg gaaaaaagat gccgaagaca gacttcaaaa 720
aagccagang ccaggttttc ccactcctag taaatctgtg agaaagcttt ccacaccccc 780

aaaaaatgg 789

<210> 448
<211> 820
<212> DNA
<213> Homo Sapiens

<400> 448
caggattact tatggaggtt ttattatttn tatttatttt tgagactgag tcttgctctg 60
tcatcaggct ggagtgcagt ggctcactgc aacctccgcc tcccaggttc aagcaattct 120
cctgcctcag cctccctagt agctgggatt acaggtgtcc accaccatgc ccaattaatt 180
tttgtatttt tggtacagac agggtttcac catgttggcc aggatggtct cgatctcgtt 240
gaccttgtga tccgcctgcc tcggcctccc aaagtgctgg gattacaggc gtgagccacc 300
gcccctggac tacttatgga ggttttaaaa aatcttttaa gtccaggcct gacgtttaga 360
gaaggttaca aaggcggcca ggatctgagt atttccaaaa agctctggag gcagcattga 420
ggtttccttc cagttgaatc actgacttta ggtcgactgg ggtactttgg gttttttggg 480
ccattttttg ggggtgtggg aagcttttct cacagattta ctaggagtgg tgaaaaactt 540
ggcctctggc ttttttggag tctgtctcgc atcttttttc cccagcgaag gacttttttc 600
cttcactgcc tcttctttga tttttggctt cttctcttgg gcttttccct ggggtctcag 660
actctgcagc tttttggggg tcttcaanat gctggcaaaa gccttttctt ttcttcccac 720
gagggggngc ctggggatta ggactctttt gcctgggana cttcttttct tgngggnang 780
tttttgaaac nntacttttt ccaatttagc ctggaggcct 820

<210> 449
<211> 936
<212> DNA
<213> Homo Sapiens

<400> 449
aaaagaagga aacagttact caactccaaa atatcattga ggctaattct cagcattacc 60
aaaaaaatat taatagtttg caggaagagc ttttacagtt gaaagctata caccaagaag 120
aggtgaaaga gttgatgtgc cagattgaag catcagctaa ggaacatgaa gcagagataa 180
ataagttgaa cgagctaaaa gagaacttag taaaacaatg tgaggcaagt gaaaagaaca 240
tccagaagaa atatgaatgt gagttagaaa atttaaggaa agccacctca aatgcaaacc 300
aagacaatca gatatgttct attctcttgc aagaaaatac atttgtagaa caagtagtaa 360
atgaaaaagt caaacactta gaagatacct taaaagaact tgaatctcaa cacagtatct 420
taaaagatga ggtaacttat atgaataatc ttaagttaaa acttgaaatg gatgctcaac 480
atataaagga tgagtttttt catgaacggg aagacttaga gtttaaaatt aatgaattat 540
tactagctaa agaagaacag ggctgtgtaa ttgaaaaatt aaaatctgag ctagcaggtt 600
taaataaaca gttttgctat actgtagaac agcataacag agaagtacag agtcttaagg 660
aacaccatca aaaagaaata tcagaactaa atgagacatt tttgtcagat tcagaaaaag 720
gaaaaattaa cattaatggt tgaaattcaa ggtcttaang gacagtgtga aaacctaccg 780
ccaggaaaag caagaagcca ttttaaantt ntgagagntt acccagagga ttttggaaat 840
ttcccaancn gaactggggg gaatctgctg ggaaaaatag gtcaggagtt cgaatcatgg 900
aaccaccagc aggcctttga ngtcatgacc tgagca 936

<210> 450
<211> 806
<212> DNA
<213> Homo Sapiens

<400> 450
aactcaaaac agtgttaagt tcctatgctg ttagtactgt atcttgtcca cacctcaaac 60
aacagtgaga tctctgagca catggtctgt acctcaacca cttttctatc accagggtct 120
agaatagttg ggcatttaaa taaaatttgc taaatgaatg aaaaatccaa aataaatcat 180

gaagccattt ataaatcaca ccaatcttgc ttgggttaaa caatagaaag taacactttt 240
gaaagagaag gcaaacaggt gttagagggg caagaatgtg agctcgagga aaagacagct 300
acgaactgtg tttttaacaa ctcattattt ggctactata tttcccaatc tattctaaca 360
ctaacaagaa tctgtctaat taattgtgac aacatctgca aaaccatagt tacctatttt 420
ttcttccaac tcttttactg aagacagagg atcatttttt acagaaggtg attttgctaa 480
ggaatccttt aatagtatca actctgctct cctatctcgt aattcttttt gntctagtag 540
tggctttagg ttttcatgtt cctttataaa acatttttct ttttcattat ggatttcact 600
tttgctacat gtttgagata cttctttcaa cttgaattaa aagaatctga ttttcaagcc 660
ttggtttttc attagcattc ttcatttcta gaagatccag actgcanggn ctctttttct 720
ggactggaat tcttctaact cttttccttt aagaagaacc tttttcttgg ntcataggcc 780
tcttcaatta aggacttaag gtcttt 806

<210> 451
<211> 909
<212> DNA
<213> Homo Sapiens

<400> 451
ctgagctctt ccagggcaag aaatatgacg ggccagaagt ggatgtgtgg agtctggggg 60
tcattttata cacactagtc agtggctcac ttccctttga tgggcaaaac ctaaaggaac 120
tgagagagag agtattaaga gggaaataca gaattccctt ctacatgtct acagactgtg 180
aaaaccttct caaacgtttc ctggtgctaa atccaattaa acgcggcact ctagagcaaa 240
tcatgaagga caggtggatc aatgcagggc atgaagaaga tgaactcaaa ccatttgttg 300
aaccagagct agacatctca gaccaaaaaa gaatagatat tatggtggga atgggatatt 360
cacaagaaga aattcaagaa tctcttagta agatgaaata cgatgaaatc acagctacat 420
atttgttatt ggggagaaaa tcttcagagc tggatgctag tgattccagt tctagcagca 480
atctttcact tgctaaggtt aggcccgagc agtgatctca acaacagtac tggccagtct 540
cctcaccaca aagtgcagag aagtgtttct tcaagccaaa agcaaagacg ctacagtgac 600
catgctggac cagctattcc ttctgttgtg gcgtatcccg aaaaggagtc agaccagcac 660
tgcagatagg tgaccctcaa agaagatggg aaatttcctt ccnggaaatc aaagtggcag 720
tgctggttgg aaggaaangg gaattgcttc cagccaggtc ccatgctttg ggnaatgcca 780
ggtaatncct aataaggcgg atattcctgg aacgccagga aaagctccac tggnccctag 840
tagtancnca gcatctggtg ggaatgacnc gaccgaaatt ncttaaggtt tgcagtggag 900
agaacttcc 909

<210> 452
<211> 672
<212> DNA
<213> Homo Sapiens

<400> 452
actgaaaaaa agtgaanttt naattatntt gtnaatnnac tnaaaaaacc ncacncaagc 60
aatgttcaca antntaaatt naaacctttt gcactaaaaa ancacaaaan ancaaacaca 120
aaaccacagg cntgaactgn aaacctgtct taactatgaa ctggncttaa ggttaattct 180
tannngccat tcantatttc nntccttggn aactgtaatg ttntagcacc ggatgatctc 240
ccgnanaggt nctagaanng acngnctgcc agngnangga gatncttccn tatacaccac 300
ttnanacnca taccgtcnan tttcanaccn acccagacgg nangcacatg gngatggggc 360
cncacnccna ctntnanggn aacggaagta gggcaggngg cgcatnggtt gcacatcttt 420
aatgtattgc attcgnaaaa aaaaggccag ntttcnatcc caggcgtgct ctngacctna 480
gactttaatn ncatgattta naanatncag nacgntattg cctaaatntt attctataca 540
tttccatcag tggttnagga aaacacttta aatgcaactn anttccacat cananncact 600
gnggttacag ntttagctca ttgggcaatt tttngaagca attttttnng aaangctntt 660
ggaatgnccc cc 672

<210> 453

<211> 834
<212> DNA
<213> Homo Sapiens

<400> 453
aagaagccaa gaagtctgaa gaaccaagaa ttcggaagaa gccgggaccc aagcccggat 60
ggaagaagaa gcttcgttgt gagagggagg agcttcccac catctacaag tgtccttacc 120
agggctgcac ggccgtgtac cgaggcgctg acggcatgaa gaagcacatc aaggagcacc 180
acgaggaggt ccgggagcgg ccctgccccc accctggctg caacaaggtt ttcatgatcg 240
accgctacct gcagcgccac gtgaagctca tccacacaga ggtgcggaac tatatctgtg 300
acgaatgtgg acaaaccttc aagcagcgga agcaccttct cgtccaccaa atgcgacatt 360
cgggagccaa gcctttgcag tgtgaggtct gtgggttcca gtgcaggcag cgggcatccc 420
tcaagtacca catgaccaaa cacaaggctg agactgagct ggactttgcc tgtgaccagt 480
gtggccggcg gtttgagaag gcccacaacc tcaatgtaca catgtccatg gtgcacccgc 540
tgacacagac ccaggacaag gccctgccct ggaggcggaa ccaccacctg ggccaccgag 600
cccctctgtg accacagacg gccaggcggt gaagcccgaa cccacctgag gacggcagtg 660
aggatgagca cctctagcag cctggacttc gcagtggctg tgtcaagcct cacccttcgt 720
gtgcacccgc atgggagggt cggagggttg cttgccgncc ttggtgctgg angcgggctt 780
ggtgtccggc tcaagtagcc ttctttgntc ttgggaccag tgggttattt tccc 834

<210> 454
<211> 703
<212> DNA
<213> Homo Sapiens

<400> 454
cccgtgtaaa taatttatta caagcataac atggagctct tgttgcacta aaaagtggat 60
tacaaatctc ctcgactgct ttagtgggga aaggaatcaa ttatttatga actgtccggc 120
cccaagtcac tcagcgtttg cgggaaaata aaccactggt cccagagcag aggaaggcta 180
cttgagccgg acaccaagcc cgcctccagc accaagggcg ggcagcaccc tccgaccctc 240
ccatgcgggt gcacacgaag ggtgaggctg acacagccac tgcggagtcc aggctgctan 300
aggtgctcat cctcactgcc gtcctcaggt gggttcgggc ttcaccgcct ggccgtctgt 360
ggtcacagag gggctcggtg gcccaggtgg tggttccgcc tccaggggca gggccttgtc 420
ctgggtctgt gtcagcgggt gcaccatgga catgtgtaca ttgaggttgt gggccttctc 480
aaaccgccgg ccacactggt cacaggcaaa gtccagctca gtctcagcct tgngtttggt 540
catgtggtac ttgagggatg cccgctgcct gcactggaac ccacagacct cacactgcaa 600
aggcttggct nccgaatgtc gcatttgggg gacgaaaaag gtgcttccgc tgcttgaaag 660
gnttggccca attnggtaca agatatagtt ccccaccttt ggg 703

<210> 455
<211> 825
<212> DNA
<213> Homo Sapiens

<400> 455
atggcaatca ggaaaaggtg ccagaacccg aggctttgga ccttccagat gacttgaacc 60
ttgacagtga agacaagaat ggtggtgagg acaccgacaa tgaagaagga gaagaagaga 120
atcctttgga gataaaagaa aaaccagaag aagcaggtca tgaagctgag gaaagaggag 180
agaccgagac cgaccagaac gaaagtcaga gtccacagga gcctgaggaa ggccccagtg 240
aagatgacaa ggcagaaggg gaagaggaaa tggacacagg agctgatgac caagatggag 300
atgctgctca gcatcctgaa gaacactctg aggagcagca gcagtctgtg gaggaaaaag 360
acaaggaagc cgatgaagaa ggtggagaga atggccctgc tgaccaaggt ttccagcccc 420
aggaggaaga agaacgggag gactctgata cagaggagca ggtgccagag gctttggaga 480
ggaaggagca tgcctcctgt gggcagactg gtgtggagaa catgcagaac acacaggcca 540
tggagctggc tggggccgca cctgagaagg agcaggggaa agaggaacac ggaagtggag 600

ctgcagatgc aaaccaggca gaaggccatg aatcgaattt cattgcccag ttggccttcc 660
agaacacacc aggaaaaaca cacagagttt taagaggaaa cctgggcagg cttgacaatt 720
gaacgttnca tgggtgatca caattgaacg tgtgcacaag aagctganga cttgtggaat 780
ccggacaggc attgccaacc aggggccagc ttaacaagcc ccagg 825

<210> 456
<211> 740
<212> DNA
<213> Homo Sapiens

<400> 456
acatcaacaa cagtggtata tgttttaata gttttcagaa tataagctgc atagcttttt 60
agaataaaaa atgatataac ttcaggtaca tgctttggga cacttggtta aacaaggaat 120
ctgtgtcttt gatgaccacc tcaaaagggt cgcagacttc acagtgtaac ttggaaacag 180
acaaggagat agatgattac atcatgacat actgcctaca aaagaacatt ctgacagaac 240
attaagtaga acagagcaca cagtttcaag tattcagcac tgctttctgg ccaagtaaaa 300
actgcctaaa gatcagtttc tttcgactgg aaaaaataga tggagctgct gagttctgga 360
cacagcgttt ctttcccaga atgagactgg ctcagtccag cttgaaagca gtgtgaggaa 420
tcactcttcc ccttgactgt taagaaaaaa aaaaatgaac taaacaaata aattactaca 480
acaacaggga ccatggcact gaatgaaata aaggggcaat caccttccca tcattgcata 540
gtctcccgaa gcagcaagtg tgaaagagga tactgaaaag ccacttcatt tttacacagc 600
ccaagggatc gtttttatng atgacctggg cacctataat gnccagttgc tttatgagaa 660
ccacacacac accacattct tcctaccctn taagagaagg taggttcctt tcacaataag 720
gaaaaccccc ccttatactt 740

<210> 457
<211> 726
<212> DNA
<213> Homo Sapiens

<400> 457
aaaatgtagt caactttatt ctccttaaac cacaaaatag agtctttggt tgtacaaaca 60
tcactagtta cagtctcgcc gaggtctcgg ctggggtggg gcagttagtt agtcacaggc 120
cagaactcct gtggggtctc tttaaaatgc taacacccag gttaaaagac ttggggcaag 180
ggtggtgctg gagctggcag ggcccccacc ccaagtctgg gggaggtgcc tgctcctcta 240
ggagggcaca gggcccaggc cacggcgccc aggccttacg gggcggcggc tgctgcacag 300
tgccacatct tcagggccca cagcgccggg tgagggcctg cccagaagca ccagagccac 360
ttctccatcc tcctcctgcg ggccagggct gggagatggt tccagggacc tcaactcctc 420
agcaaagtcc ggtgacaggc gtcccgggga ggtgctggtc tgggggccga ggtcttccac 480
aggggtgggc gacggggtgg gcccagggga aggggcctcg gccagtcgct ccaggggccc 540
ccgcgtgccc cggcctttct gggacctgct gaggaccatc tgtgctcgga gagcgtcctg 600
ttccaatgac ttcatcctgg ctggccttca caagcgcacg cttctcggnc ttcagggccc 660
cggacttcgg caaggggaca nggcacgctt cgggtgccgg tggcttccgg actttggacg 720
ccgcaa 726

<210> 458
<211> 870
<212> DNA
<213> Homo Sapiens

<400> 458
cgcggcctct ccgccgggtg taccacctgt cgcggcgcga gacctctggt gaaagaaaag 60
atgttgtccc ggttaagagt agtttccacc acttgtactt tggcatgtcg acatttgcac 120
ataaaagaaa aaggcaagcc acttatgctg aacccaagaa caaacaaggg aatggcattt 180
actttacaag aacgacaaat gcttggtctt caaggacttc tacctcccaa aatagagaca 240

caagatattc aagccttacg atttcataga aacttgaaga aaatgactag ccctttggaa 300
aaatatatct acataatggg aatacaagaa agaaatgaga aattgtttta tagaatactg 360
caagatgaca ttgagagttt aatgccaatt gtatatacac cgacggttgg tcttgcctgc 420
tcccagtatg gacacatctt tagaagacct aagggattat ttatttcgat ctcagacaga 480
ggtcatgtta gatcaattgt ggataactgg ccagaaaatc atgttaaggc tgttgtagtg 540
actgatggag agagaattct gggtcttgga gatctgggtg tctatggaat gggaattcca 600
gtaggaaaac tttgttgnat cagcttgtgc aggaatacgg cctgatagat gcctgccagt 660
gtgtattgat gtgggaactg ataatatcgc actcttaaaa ganccatttt acatgggctt 720
gaccagaaac gagatcgcac ccacagttga tganctgatg gatgagttta tgaaagcttt 780
actgacagat atggccggaa cacctttatt cagttcgaag acnttggaaa tcataangcc 840
ttcaggtctt tgagaaagtc cggggaaaaa 870

<210> 459
<211> 761
<212> DNA
<213> Homo Sapiens

<400> 459
aaatgtaaga tatttattaa ataaaaaggt tacactatga tttttataca ctgttgaaaa 60
caatgacttt tatttactta aagccagcag tagttcccat tactctcata atgttatagt 120
taaggcttga tttagttcca gaaaataaat agggtaaatt tttaatattt ccctagctct 180
gtctgctata gggaatttca gagtatgaag gtaagatgaa gcagatatat aagaacattt 240
ttagataatg acaatttttc cttaaaattt ggtgaaaatt tagtttcttc tcaaaattct 300
gtacttctat ccataaaagt aaatttctat tttagtagct ctgtaagaac taggccagag 360
aagagtatta cccataatag taaatagcaa atactttggc aagtctgaat tagagtacaa 420
gtgaagacat tcacaaacac actttttaca tctcctggat gtggtacggg ctgtatgtta 480
gaattaaagc atcacaacta tctgattgta gggtgctggt gggcaatgca atcaatcaac 540
acgtctaccc caacagatgt ggagacccat ggaaaaaata catcaaccaa agtggtcagg 600
gagaacaaaa ccccagaaaa cacccttaaa actgaagaca ttatctcttc ttggctgaaa 660
aaaggggttc cctggagcac angaaaggtt ttatcaaggg aggcttctat tcngtaatca 720
caggaaggct tgatgcanat tcctggccat tcatacccca t 761

<210> 460
<211> 876
<212> DNA
<213> Homo Sapiens

<400> 460
ctgagctcct gaagcgccct aaggagtaca ctgtgcgctt cacttttcca gaccccccac 60
cactcagccc tccagtgctg ggtctgcatg gtgtgacatt cggctaccag ggacagaaac 120
cactctttaa gaacttggat tttggcatcg acatggattc aaggatttgc attgtgggcc 180
ctaatggtgt ggggaagagt acgctactcc tgctgctgac tggcaagctg acaccgaccc 240
atggggaaat gagaaagaac caccggctga aaattggctt cttcaaccag cagtatgcag 300
agcagctgcg catggaggag acgcccactg agtacctgca gcggggcttc aacctgccct 360
accaggatgc ccgcaagtgc ctgggccgct tcggcctgga gagtcacgcc cacaccatcc 420
agatctgcaa actctctggt ggtcagaagg cgcgagttgt gtttgctgag ctggcctgtc 480
gggaacctga tgtcctcatc ttggacgagc caaccaataa cctggacata gagtctattg 540
atgctctagg ggaggccatc aatgaataca agggtgctgt gatcgttgtc agccatgatg 600
cccgactcat cacagaaacc aattgccagc ttgtgggtgg tggaggaaca gagtggtagc 660
ccaatcgatg gtgactttga agactacaag ccgggaggtg ttggaagccc tgggtgaagt 720
catgggcagc cnggccccga naagtgaagc tttnctttcc agaagtntcc gagagaacat 780
aattgggggg gcctaaaann cctctggggg cttcccttct tttgaanaat gctntggnct 840
gcaantgact tggcaaccat ttaggcccct taaagg 876

<210> 461

<211> 689
<212> DNA
<213> Homo Sapiens

<400> 461
gcaaacaaga tccatttagt ggggaagagg ggactattaa aagctgctag aaaactgaat 60
aaagcaaatc aagactgaga acagttccaa ctcccatcaa tctccaaaca gtgacaggtc 120
ggcagcaact cctttccttt atttcttccc cttgtaaagg gaaattcaag ttcagcagca 180
ttcctttcct gccccaagtc ctcaaccaga caagaggctg caggcaccaa atcttgggct 240
ggataatggc aaaggcctca gaagctcacc tccagctctg agcttcaaca gctgtttgta 300
ccagtgagtc agcattaaat ccaccagaaa agaacagcac cacccaaaga ctggggggca 360
gctgggcctg aagctgtagg gtaaatcaga ggcaggcttc tgagtgatga gagtcctgag 420
acaataggcc acataaactt ggctggatgg aacctcacaa taaggtggtc acctcttgtt 480
tgtttagggg gatgccaagg ataaggccag ctcagttata tgaagagaag cagaacaaac 540
aaagtctttc agagaaatgg atgcaatcag aagtgggatc cccggncaca tcaaggtcac 600
actccacctt catgtgcctg aaatggttgc caggtcagct gcaggcccan aggcagtctt 660
canaaggaag gggagaccac agaggactt 689

<210> 462
<211> 840
<212> DNA
<213> Homo Sapiens

<400> 462
aggagccttt ggagttccat gccaagcggc cttggcgccc cgaggaggca gtagaagatc 60
cggacgagga ggatgaggat aatactagtg aagccgagaa tgggttctcc ctggaggaag 120
tgttacggct cggaggcacc aagcaagatt accttatgct ggctactttg gatgagaatg 180
aggaagtgat agatggaggc aaaaaaggag caatcgatga ccttcagcaa ggtgaattgg 240
aagcatttat tcaaaatctt aatttggcga agtatacaaa agcttcctta attgaagaag 300
atgaaccagc tgaaaaagaa aattccagca aaaaagaagt aaaaatacct aaaataaata 360
ataaaaatac agcagaaagt caaaggacat cagttaataa ggtgaaaaat aagaataggc 420
cagaaccaca ttctgatgag aatggcagta ccacaccgaa agtaaagaaa gataaacaga 480
acatctttga attttttgag agacagactt tgttacttag gcctggaggc aaatggtatg 540
atctggagta cagcaatgaa tattctttga aaccccagcc tcaggatgtt gnatctaagt 600
acaaaaccct tgctcagaag ctgtatcagc atgaaatcaa cttattcaaa agtaagacga 660
atagtcaaaa gggagcctct tctacctgga tgaaaggcaa ttgtgtcatc ggggaccact 720
aggtgacagg atggcagcca ttgattcttc ttattcagga tgatgcccgt tcacaccact 780
ttcagnttgt agnaaactct tggggaaccc ttggtaaaaa ggaanggcna caaaacagca 840

<210> 463
<211> 784
<212> DNA
<213> Homo Sapiens

<400> 463
agatgtaagt agaattttaa tctataattt acattaataa ctcatttcct ttgtttttta 60
gttttttgag tggttttaat cctcttcttt ttaaaatgtt tctttttctt gatgatactt 120
tttgcatctc tgttgtgtag ccagtcatca cgttcagcct cccatctaag ctgtttgaga 180
cttgcattat ctttgttagc catggcattc atgccaatgt tatcaaactt ggatcccata 240
ttttcatcca atagatggcc aaactcttca gcagatacaa ataggctgga atcatttaag 300
tttcttttct tttttcttgg cccttgaaat gagccagcaa agtcaaaatc atctgtacct 360
tttctcttgc ttttcttagt actgactttg gagtggactt caagttctgg aacactctca 420
ctttcatcat ctaacacatc catgaatgtt cctccatctt catcaacttc agcaaattct 480
tcatcatcca tacttcctaa agaaacttca tcgtcatcca ggttaccaag ttcatcatca 540
ctaccttctg aatcttcatc taatgtgtta tccttagctc cttttggtct ctttttcacg 600

tttccagcaa aaatccatat catcctttnc agagctgaaa cagttatcat cttcaaatgt 660
gtcaatcagc tcttcaaatt ctttcatcat ccacgtcctt ctaatacttt cttcaatctg 720
catccccgtt tttggnttct cttttaanca gcaacttttt ttatnaaacc ctgggggaaa 780
aaac 784

<210> 464
<211> 850
<212> DNA
<213> Homo Sapiens

<400> 464
caggcatcgg ccaccggaac agcctggaga gcatctcttc catcgaccgg gagctgagcc 60
ctgagggccc aggcaaggag aaggagctgc ctggacagac cctgcactgg gggcccgagg 120
ccacagaagc cgcaggtcgg ggtctgcagc ccctgaagct ggactaccgc gccctggccg 180
ccgtgcccag cgctggcagc gtgcagaggg taccgtctgg agcagctgga gggaagatgg 240
ctgaatctcc ctgctcccct agtggccagc agccgccctc cccgccttct ccggatgagc 300
tgcccgccaa tgtgaagcag gcctacaggg ccttcgcggc cgtgcccact tctcacccgc 360
ctgaggatgc ccctgcccag ccccccacgc ctgggcctgc agcctccccg gagcagctgt 420
ccttccggga gcggcagaag tactttgagc tggaggtgcg cgtgccccag gccgagggcc 480
cccctaagcg cgtgtccctg gtgggtgctg acgacctgcg gaagatgcag gaggaggaag 540
ccagaaaact acagcagaag agagcgcaga tgctgcggga ggcggcagaa gctggggccg 600
aagcgaggct ngccctggac ggggagacgc tgggcgagga ggaacaggan gatgagcagc 660
caccctgggc cagcccgagc cccacttaag gcagaacccg gcgtcccccc ggccctggaa 720
gtggcgcccc ggtgcggacg gncaaaagct gaacggggcc ancaggaacc ggttgccctt 780
canagtnccg gacccaccgg gaccccancg tgccctggtc ccttgcccaa cttccgggcc 840
ctggaaggcc 850

<210> 465
<211> 759
<212> DNA
<213> Homo Sapiens

<400> 465
aaaatgtagt caactttatt ctccttaaac cacaaaatag agtctttggt tgtacaaaca 60
tcactagtta cagtctcgcc gaggtctcgg ctggggtggg gcagttagtt agtcacaggc 120
cagaactcct gtggggtctc tttaaaatgc taacacccag gttaaaagac ttggggcaag 180
ggtggtgctg gagctggcag ggcccccacc ccaagtctgg gggaggtgcc tgctcctcta 240
ggagggcaca gggcccaggc cacggcgccc aggccttacg gggcggcggc tgctgcacag 300
tgccacatct tcagggccca cagcgccggg tgagggcctg cccagaagca ccagagccac 360
ttntccatcc tcctcctgcg ggccagggct gggagatggt tccagggacc tcaactcctc 420
agcaaagtcc ggtgacaggc gtcccgggga ggtgctggtc tgggggccga ggtcttccac 480
aggggtgggc gacggggtgg gcgcagggga aggggcctcg gccagtcgct ccagggcccc 540
ccgcgtgccc cggcctttct gggacctgct gaggaccatc tgggctcngg aaagcgtcct 600
tgttccaatg acttcatcct ggctgccctt cacagngcac gcttntcggc ttcagggccc 660
ggagctttgg canggggaca aggcaacgct tcgggtgccc ggtgggttcc ggacttttga 720
acgcgccaan ccggttcctt ggngggcgcc cgtttcaac 759

<210> 466
<211> 1240
<212> DNA
<213> Homo Sapiens

<400> 466
gtggtagtgg tgccggagct ggaggcggag gcatgtttgg tagtggcggt ggaggagggg 60
gcactggaag tacaggtcca gggtatagct tcccacacta tggatttcct acttatggtg 120

ggattacttt ccatcctgga actactaaat ctaatgctgg gatgaagcat ggaaccatgg 180
acactgaatc taaaaaggac cctgaaggtt gtgacaaaag tgatgacaaa aacactgtaa 240
acctctttgg gaaagttatt gaaaccacag agcaagatca ggagcccagc gaggccaccg 300
ttgggaatgg tgaggtcact ctaacgtatg caacaggaac aaaagaagag agtgctggag 360
ttcaggataa cctctttcta gagaaggcta tgcagcttgc aaagaggcat gccaatgccc 420
ttttcgacta cgcggtgaca ggagacgtga agatgctgct ggccgtccag cgccatctca 480
ctgctgtgca ggatgagaat ggggacagtg tcttacactt agcaatcatc caccttcatt 540
ctcaacttgt gagggatcta ctagaagtca catctggttt gatttctgat gacattatca 600
acatgagaaa tgatctgtac cagacgccct tgcacttggc agtgatcact aagcaggaag 660
atgtggtgga ggatttgctg agggctgggg ccgacctgag ccttctggac cgcttgggta 720
actctgtttt gcacctagct gccaaagaag gacatgataa agttctcagt atcttactca 780
agcacaaaaa ggcagcacta cttcttgacc accccaacgg ggacggtctg aatgccattc 840
atctagccat gatgagcaat agcctgccat gtttgctgct gctggtggcc gctggggctg 900
acgtcaatgc tcaggagcag aagtccgggc gcacagcact gcacctggct gtggagcacg 960
acaacatctc attggcaggc tgcctgctcc tggagggtga tgcccatgtg gacagtacta 1020
cctacgatgg aaccacaccc ctgcatatag cagctgggaa agggtccacc aggctggcag 1080
ctcttcttaa agcagcagga gcagatcccc tggtgggaga ctttgagccc ttctatgacc 1140
tggatgactc ttgggaaaat gcaggaaaag gattgaagga gttggnctgg aancacgcct 1200
tttaganatg ggccnccaac tggcaggnat ttggcctatt 1240

<210> 467
<211> 885
<212> DNA
<213> Homo Sapiens

<400> 467
gtgccggagc tggaggcgga ggcatgtttg gtagtggcgg tggaggaggg ggcactggaa 60
gtacaggtcc agggtatagc ttcccacact atggatttcc tacttatggt gggattactt 120
tccatcctgg aactactaaa tctaatgctg ggatgaagca tggaaccatg gacactgaat 180
ctaaaaagga ccctgaaggt tgtgacaaaa gtgatgacaa aaacactgta aacctctttg 240
ggaaagttat tgaaaccaca gagcaagatc aggagcccag cgaggccacc gttgggaatg 300
gtgaggtcac tctaacgtat gcaacaggaa caaaagaaga gagtgctgga gttcaggata 360
acctctttct agagaaggct atgcagcttg caaagaggca tgccaatgcc cttttcgact 420
acgcggtgac aggagacgtg aagatgctgc tggccgtcca gcgccatctc actgctgtgc 480
aggatgagaa tggggacagt gtcttacact tagcaatcat ccaccttcat tctcaacttg 540
tgagggatct actagaagtc acatctggtt tgatttctga tgacattatc aacatgagaa 600
atgatctgta ccagacgccc ttgcacttgg cagtgatcac taagcaggaa gatgtggtgg 660
aggatttgct gagggctggg gcccgacctg agccttctgg acccgcttgg gtaactctgg 720
tttgcaccta gcttgcccaa agaaggacat gataaagttc tcaagtatct tacttaagcn 780
caaaaanggc agcactactt tnttgaccac ccccaacggg ggacggtctt gaatgccatt 840
catttaagcc atgatgagcc ataagcctgg catggtttgc tgctg 885

<210> 468
<211> 748
<212> DNA
<213> Homo Sapiens

<400> 468
gcaaatcaga gaaataacca cattagaaaa agcaatatgc cttttttttt aaaatggcac 60
atcaagtgac tctcatttta aaatatctct tttcttaacc cttaatttga atgcaaaatg 120
atgctgtggt cagaaggaat gccaggtggc gaccgtgata cctttaatga caataggaac 180
gtagcagagg gacaacagca atgacaacag aaagcagctg tgatccagca gcagctggca 240
aagcttagta agcaacctca tccccagatg catccgctca gccagtgttg tgattgctag 300
atactatctg taagtgaacc aaactaaaat tcatttatga accaagaaag gaagccaagt 360
tgaaaaggtc tcgagttaaa tcgagaatga ttcaggcggg ccggctctct gagcaccttt 420

ggatgcactt cagcttctgt cttgtggaaa cgcgtggaat tttagggctt tggtttacac 480
ggtgtgggaa attgtcagca ggctaaattt tgccttctag aggtccttcc tgcccataat 540
catggggcat tttgttgaga gttagcagtg aggcaccact ggtcagagac tcggtaaagc 600
tgagtttgcg gaaggatgtc tccacgccgc ttgtcgcaga cactgtcact ggcttcggag 660
ctcgnctatt tgctgccttg tggaggcagg cgaaanaagc agcgagtggg ccctgaaaag 720
gngggcnttc actgggctgg aaggcttg 748

<210> 469
<211> 770
<212> DNA
<213> Homo Sapiens

<400> 469
gcaaatcaga gaaataacca cattagaaaa agcaatatgc cttttttttt aaaatggcac 60
atcaagtgac tctcatttta aaatatctct tttcttaacc cttaatttga atgcaaaatg 120
atgctgtggt cagaaggaat gccaggtggc gaccgtgata cctttaatga caataggaac 180
gtagcagagg gacaacagca atgacaacag aaagcagctg tgatccagca gcagctggca 240
aagcttagta agcaacctca tccccagatg catccgctca gccagtgttg tgattgctag 300
atactatctg taagtgaacc aaactaaaat tcatttatga accaagaaag gaagccaagt 360
tgaaaaggtc tcgagttaaa tcgagaatga ttcaggcggg ccggctctct gagcaccttt 420
ggatgcactt cagcttctgt cttgtggaca acgcagtgga attttagggc tttggtttac 480
acggtgtggg aaattgtcag caggctaaat tttgccttct agaggtcctt cctgcccata 540
atcatggggc attttggtga gagntagcag tgaggcacca ctggtcagag acttcggtaa 600
agctgagttt gcgggaaagg atgtnttcca cgccgcttnt cgcanacact ggcactgnct 660
tgggagctcn gctattttgc ttgcccttgt ggangcaggc caaaanaagc caacgaatgg 720
ggccctgaaa aggngggcct tcanctgggc ttggaagctt gcctnggatc 770

<210> 470
<211> 892
<212> DNA
<213> Homo Sapiens

<400> 470
agagtgctgg agttcaggat aacctctttc tagagaaggc tatgcagctt gcaaagaggc 60
atgccaatgc ccttttcgac tacgcggtga caggagacgt gaagatgctg ctggccgtcc 120
agcgccatct cactgctgtg caggatgaga atggggacag tgtcttacac ttagcaatca 180
tccaccttca ttctcaactt gtgagggatc tactagaagt cacatctggt ttgatttctg 240
atgacattat caacatgaga aatgatctgt accagacgcc cttgcacttg gcagtgatca 300
ctaagcagga agatgtggtg gaggatttgc tgagggctgg ggccgacctg agccttctgg 360
accgcttggg taactctgtt ttgcacctag ctgccaaaga aggacatgat aaagttctca 420
gtatcttact caagcacaaa aaggcagcac tacttcttga ccaccccaac ggggacggtc 480
tgaatgccat tcatctagcc atgatgagca atagcctgcc atgtttgctg ctgctggtgg 540
ccgctggggc tgacgtcaat gctcaggagc agaagtccgg gcgcacagca ctgcacctgg 600
ctgtggagca cgacaacatc tcattggcag gctgcctgct cctggagggt gatgcccatg 660
tggacagtac tacctacgat ggaaccacac ccctgcatat agcagctggg aaagggtcca 720
ccaggctggc agctcttctt aaagcagcag gagcagatcc cctggtggga gactttgagc 780
ccttctatga cctggatgac tcttgggaaa atgcaggaaa aggattgaag gagttggnct 840
ggaancacgc cttttagana tgggccncca actggcaggn atttggccta tt 892

<210> 471
<211> 759
<212> DNA
<213> Homo Sapiens

<400> 471

gcaaatcaga gaaataacca cattagaaaa agcaatatgc cttttttttt aaaatggcac 60
atcaagtgac tctcatttta aaatatctct tttcttaacc cttaatttga atgcaaaatg 120
atgctgtggt cagaaggaat gccaggtggc gaccgtgata cctttaatga caataggaac 180
gtagcagagg gacaacagca atgacaacag aaagcagctg tgatccagca gcagctggca 240
aagcttagta agcaacctca tccccagatg catccgctca gccagtgttg tgattgctag 300
atactatctg taagtgaacc aaactaaaat tcatttatga accaagaaag gaagccaagt 360
tgaaaaggtc tcgagttaaa tcgagaatga ttcaggcggg ccggctctct gagcaccttt 420
ggatgcactt cagcttctgt cttgtggaca acgcagtgga attttagggc tttggtttac 480
acggtgtggg aaattgtcag caggctaaat tttgccttct agaggtcctt cctgcccata 540
atcatggggc attttgttga gagttagcag tgaggcacca ctggtcagag actcggtaaa 600
gctgagtttg cggaaggatg tctccacgcc gctgtccgca gacactgtca ctgnctcgga 660
gctcgtctat ttgctgcctt gtggaggcag gcgananagg caacgagtgg gccctgaaaa 720
gnggtcttca ctgggctgga agcttgnctg gatcacttt 759

<210> 472
<211> 852
<212> DNA
<213> Homo Sapiens

<400> 472
gtggtagtgg tgccggagct ggaggcggag gcatgtttgg tagtggcggt ggaggagggg 60
gcactggaag tacaggtcca gggtatagct tcccacacta tggatttcct acttatggtg 120
ggattacttt ccatcctgga actactaaat ctaatgctgg gatgaagcat ggaaccatgg 180
acactgaatc taaaaaggac cctgaaggtt gtgacaaaag tgatgacaaa aacactgtaa 240
acctctttgg gaaagttatt gaaaccacag agcaagatca ggagcccagc gaggccaccg 300
ttgggaatgg tgaggtcact ctaacgtatg caacaggaac aaaagaagag agtgctggag 360
ttcaggataa cctctttcta gagaaggcta tgcagcttgc aaagaggcat gccaatgccc 420
ttttcgacta cgcggtgaca ggagacgtga agatgctgct ggccgtccag cgccatctca 480
ctgctgtgca ggatgagaat ggggacagtg tcttacactt agcaatcatc caccttcatt 540
ctcaacttgt gagggatcta ctagaagtca catctggttt gatttctgat gacattatca 600
acatgagaaa tgatctgtac cagacgccct ttgcacttgg cagtgatcac taagcaggaa 660
gatgtggtgg aggatttgct gaagggctgg ggcccgacct tgagcctttc tggacccgct 720
tgggtaactc tgttttgcac cctaacttgc caaagaaggg cattgataaa ggtcttcaag 780
tatcttactt cagcccaaaa anggcagcac tacttntttg accaccccaa cgggggacgg 840
gcttgaatgc ca 852

<210> 473
<211> 804
<212> DNA
<213> Homo Sapiens

<400> 473
gcaaatcaga gaaataacca cattagaaaa agcaatatgc cttttttttt aaaatggcac 60
atcaagtgac tctcatttta aaatatctct tttcttaacc cttaatttga atgcaaaatg 120
atgctgtggt cagaaggaat gccaggtggc gaccgtgata cctttaatga caataggaac 180
gtagcagagg gacaacagca atgacaacag aaagcagctg tgatccagca gcagctggca 240
aagcttagta agcaacctca tccccagatg catccgctca gccagtgttg tgattgctag 300
atactatctg taagtgaacc aaactaaaat tcatttatga accaagaaag gaagccaagt 360
tgaaaaggtc tcgagttaaa tcgagaatga ttcaggcggg ccggctctct gagcaccttt 420
ggatgcactt cagcttctgt cttgtggaca acgcagtgga attttagggc tttggtttac 480
acggtgtggg aaattgtcag caggctaaat tttgccttct agaggtcctt ccttgcccat 540
aatcatgggg cattttgttg agagttagca gtgaggcacc acttggtcaa gagactcggt 600
naagctgagt tttgcggaag gatgtctcca cgcccgctgt cgcagacact gtcactgtct 660
tcggaactcg nctatttgct gncttgtgga agcaggcnaa nanaagcanc gaantggggc 720
cctgaaaagn gggtcttcac ttggnctgga aggcttgccc tgggatcnct ttnaatgggc 780

ttcggnggaa ccccattttg tctt 804

<210> 474
<211> 819
<212> DNA
<213> Homo Sapiens

<400> 474
ggctgggctg cgcttgggtc cgtcgctgct tcggtgtccc tgtcgggctt cccagcagcg 60
gcctagcggg aaaagtaaaa gatgtctgaa tatattcggg taaccgaaga tgagaacgat 120
gagcccattg aaataccatc ggaagacgat gggacggtgc tgctctccac ggttacagcc 180
cagtttccag gggcgtgtgg gcttcgctac aggaatccag tgtctcagtg tatgagaggt 240
gtccggctgg tagaaggaat tctgcatgcc ccagatgctg gctggggaaa tctggtgtat 300
gttgtcaact atccaaaaga taacaaaaga aaaatggatg agacagatgc ttcatcagca 360
gtgaaagtga aaagagcagt ccagaaaaca tccgatttaa tagtgttggg tctcccatgg 420
aaaacaaccg aacaggacct gaaagagtat tttagtacct ttggagaagt tcttatggtg 480
caggtcaaga aagatcttaa gactggtcat tcaaaggggt ttggctttgt tcgttttacg 540
gaatatgaaa cacaagtgaa agtaatgtca cagcgacata tgatagatgg acgatggtgt 600
gactgcaaac ttcctaattc taagcaaagc caagatgagc ctttgagaag cagaaaagtg 660
tttgtggggc gctgtcagag gacatgactg aggatgaagc tgcgggagtt cttcttttca 720
gtancgggga tgtgatggat ggtcttcatn cccaagccat tcagggcctt tggctttggt 780
catttgcaga tgaatcagat gcgccagtct ctttgtgga 819

<210> 475
<211> 721
<212> DNA
<213> Homo Sapiens

<400> 475
atttaaatca gttttattta agaatttcca acaatgacaa ctcttataaa aagcatccaa 60
gcacaggaca cagaactgca gcaaacagca ttcttatggg tagctaacag acattagaac 120
ttccaccctt ctttgagaca cctgagctca ctggtgaact ctgcttcaag tcctcctgca 180
aagcacacca caagctcagt ccatgttctc agcccatcag cttcagttca cattgccaca 240
cttacatatc agtaacagaa gagaacacac accatacagc attcacagca gttgacaaag 300
gggtaggggg agtacaagta tcatttcact taacacattc atctaatgtg ggttatctaa 360
gaacaaaaac tcacttaaaa gtcttccaac agatgtggat gtcctttgaa tgcaaaaaac 420
attcgtacat tatttgctat cattgctctc tgcacactct ctcaccaaag ccacaggatt 480
gagagacaca tctcgccaag ttaaaaaata tccattatgc accaccaagt ctctgcacgc 540
gctctctcct tttctcgctc atactagcct ttcatgcctc ggcaccacca tcaatcccac 600
acaaggtttc aaaagttcag acagccttct ggttccatat cacaggcctt gcgttcatag 660
cggtgatacg acttcctgga aattaagagt ancggataaa aatgggacac ccaccggtaa 720
a 721

<210> 476
<211> 442
<212> DNA
<213> Homo Sapiens

<400> 476
attnaaatca gtttnattna anantttcca ncanngncan ctntnataaa aggcntccan 60
ncncaggacn canancngca gcaancagcn ttntnanggg tagntancan acnttaaanc 120
ttccaccntt ntttganacn ccnganctna nngggganct nngnttcang ncctccngca 180
angcacacca cangctcagn ccatgttntn agcccatcag nttcagttna catngccaca 240
nttncntatc agtaccagaa gagaccncnc nccntncagc nttcncagca gtngncaaag 300
gggtaggggn agtccangta tcatttnant taccacattc atctaagggg ggttatctaa 360

naccaaaanc tcanttaaan gtnttccanc anangnggan gnccttngaa ngcaaaaanc 420
nttcgnccat nattggctat ca 442

<210> 477
<211> 878
<212> DNA
<213> Homo Sapiens

<400> 477
ggtggctggg ctgcgcttgg gtccgtcgct gcttcggtgt ccctgtcggg cttcccagca 60
gcggcctagc gggaaaagta aaagatgtct gaatatattc gggtaaccga agatgagaac 120
gatgagccca ttgaaatacc atcggaagac gatgggacgg tgctgctctc cacggttaca 180
gcccagtttc caggggcgtg tgggcttcgc tacaggaatc cagtgtctca gtgtatgaga 240
ggtgtccggc tggtagaagg aattctgcat gccccagatg ctggctgggg aaatctggtg 300
tatgttgtca actatccaaa agataacaaa agaaaaatgg atgagacaga tgcttcatca 360
gcagtgaaag tgaaaagagc agtccagaaa acatccgatt taatagtgtt gggtctccca 420
tggaaaacaa ccgaacagga cctgaaagag tattttagta cctttggaga agttcttatg 480
gtgcaggtca agaaagatct taagactggt cattcaaagg ggtttggctt tgttcgtttt 540
acggaatatg aaacacaagt gaaagtaatg tcacagcgac atatgataga tggacgatgg 600
tgtgactgca aacttcctaa ttctaagcaa agccaagatg agcctttgag aagcagaaaa 660
gtgtttgtgg ggcgctgtca gaggacatga ctgaggatga agctgcggga gttcttcttt 720
agtaccgggg atgtgatgga tgtctttatt ccccaagccc nttcaggggc ttttggcttt 780
ggtacatttg ccagatgatc agaatgccca gtctcttttg tggaaaagga ctttgatcat 840
ttaaagggaa tcagcggttc attatattcc aatggccc 878

<210> 478
<211> 768
<212> DNA
<213> Homo Sapiens

<400> 478
ggtgtcaaaa aaaaatttta tttatctggt tcaaaaaatt ttttagaatg aatgcattta 60
gattgaccaa atagattttt aaaaacaaat ctttgccaaa tagtttaagt acttttaaac 120
ttcaaaatct tcttagggta aaataaatac ccgtatctat gcagtaccat aaacatgtta 180
ataaaaggcc actcaacatt gaaagccttc tatgaccagt aactgaaatt tacacaagtg 240
taaagaaggg attaaaccat gccgttgaca agttaactta cccctgggct ccttgaaggc 300
ttgtcagttt agtctttgga ggtccccgag taccatttta agtgttacca tgttactgct 360
gctgagtaat agtgcaagtg cattttaggt gcggtcaccc agacttattc aaaactagat 420
ttcaaaagaa aaaaaaaaat tttcactttg gccaatgcaa gaacaaatac caattaagtc 480
tgggtatcag gtgtcaatgc atgacaggtg atgaatccat ttgacttgag acaacttttc 540
aaataagttt atttgaagca aaataaacta ctgccaagaa actttatgaa agttccatct 600
caaaagggtc aaaaaagggg aattaactgc tatgaattct ttgcattcag ggcgtcaaaa 660
gacgccggcc tgnggatgcc gtgatgacca attcttgaat gagaaagcat gtagaccgna 720
tttcctatgg cagaaatatt tacnggccta ctttcaatgg aagngctt 768

<210> 479
<211> 815
<212> DNA
<213> Homo Sapiens

<400> 479
gcgaagcggt ggctgggctg cgcttgggtc cgtcgctgct tcggtgtccc tgtcgggctt 60
cccagcagcg gcctagcggg aaaagtaaaa gatgtctgaa tatattcggg taaccgaaga 120
tgagaacgat gagcccattg aaataccatc ggaagacgat gggacggtgc tgctctccac 180
ggttacagcc cagtttccag gggcgtgtgg gcttcgctac aggaatccag tgtctcagtg 240

tatgagaggt gtccggctgg tagaaggaat tctgcatgcc ccagatgctg gctggggaaa 300
tctggtgtat gttgtcaact atccaaaaga taacaaaaga aaaatggatg agacagatgc 360
ttcatcagca gtgaaagtga aaagagcagt ccagaaaaca tccgatttaa tagtgttggg 420
tctcccatgg aaaacaaccg aacaggacct gaaagagtat tttagtacct ttggagaagt 480
tcttatggtg caggtcaaga aagatcttaa gactggtcat tcaaaggggt ttggctttgt 540
tcgttttacg gaatatgaaa cacaagtgaa agtaatgtca cagcgacata tgatagatgg 600
acgatggtgt gactgcaaac ttcctaattc taagcaaagc ccagatgaac ctttgagaag 660
cagaaaagtg tttgtggggg cgctgtacag angacatgac tgangataan cttcnggagt 720
tcttttttta ataccgggat gtgatggatg cttcatttcc caacccattc agggcctttg 780
nctttggtac catttgcaga tgatcanatt gccca 815

<210> 480
<211> 812
<212> DNA
<213> Homo Sapiens

<400> 480
gtggtgtcaa aaaaaatttt atttatctgg ttcaaaaaat tttttagaat gaatgcattt 60
agattgacca aatagatttt taaaaacaaa tctttgccaa atagtttaag tacttttaaa 120
cttcaaaatc ttcttagggt aaaataaata cccgtatcta tgcagtacca taaacatgtt 180
aataaaaggc cactcaacat tgaaagcctt ctatgaccag taactgaaat ttacacaagt 240
gtaaagaagg gattaaacca tgccgttgac aagttaactt acccctgggc tccttgaagg 300
cttgtcagtt tagtctttgg aggtccccga gtaccatttt aagtgttacc atgttactgc 360
tgctgagtaa tagtgcaagt gcattttagg tgcggtcacc cagacttatt caaaactaga 420
tttcaaaaga aaaaaaaaaa ttttcacttt ggccaatgca agaacaaata ccaattaagt 480
ctgggtatca ggtgtcaatg catgacaggt gatgaatcca tttgacttga gacaactttt 540
caaataagtt tatttgaagc aaaataaact actgccaaga aactttatga aaagttccat 600
cttcaaaagg ggtcaaaaaa ggggaattaa ctgctatgaa ttctttgcat tcanggctgc 660
aaaacaaaga ccccatatta tttaaaatcc agtttattta agaatttncc accntggaca 720
acttcttatt aaaaaggcnt tccaggccca nggaccacag aaactgnang ccaaacangc 780
atttcttatg ggtagcttta ccaggacctt tt 812

<210> 481
<211> 1127
<212> DNA
<213> Homo Sapiens

<400> 481
gaggacagca atttaatggc aaaggaaaga caagacaggc tgcgaaacac gatgctgctg 60
ccaaagcgtt gaggatcctg cagaatgagc ccctgccaga gaggctggag gtgaatggaa 120
gagaatccga agaagaaaat ctcaataaat ctgaaataag tcaagtgttt gagattgcac 180
ttaaacggaa cttgcctgtg aatttcgagg tggcccggga gagtggccca ccccacatga 240
agaactttgt gaccaaggtt tcggttgggg agtttgtggg ggaaggtgaa gggaaaagca 300
agaagatttc aaagaaaaat gccgccatag ctgttcttga ggagctgaag aagttaccgc 360
ccctgcctgc agttgaacga gtaaagccta gaatcaaaaa gaaaacaaaa cccatagtca 420
agccacagac aagcccagaa tatggccagg ggatcaatcc gattagccga ctggcccaga 480
tccagcaggc aaaaaaggag aaggagccag agtacacgct cctcacagag cgaggcctcc 540
cgcgccgcag ggagtttgtg atgcaggtga aggttggaaa ccacactgca gaaggaacgg 600
gcaccaacaa gaaggtggcc aagcgcaatg cagccgagaa catgctggag atccttggtt 660
tcaaagtccc gcaggcgcag cccaccaaac ccgcactcaa gtcagaggag aagacaccca 720
taaagaaacc aggggatgga agaaaagtaa ccttttttga acctggctct ggggatgaaa 780
atgggactag taataaagag gatgagttca ggatgcctta tctaagtcat cagcagctgc 840
ctgctggaat tcttcccatg gtgcccgagg tcgcccaggc tgtaggagtt agtcaaggac 900
atcacaccaa agattttacc agggcagctc cgaatcctgc caaggccacg gtaactgcca 960
tgatagcccg agagttgttg tatgggggca cctcgcccac agcccgagac cattttaaag 1020

aataacatct cttcaggcca cgtaccccat ggacctctca cgagaccctn tgagcaactg 1080
gactatcttt ncagagtcca gggattncag gttgaatacc aagactt 1127

<210> 482
<211> 773
<212> DNA
<213> Homo Sapiens

<400> 482
taccgcccct gcctgcagtt gaacgagtaa agcctagaat caaaaagaaa acaaaaccca 60
tagtcaagcc acagacaagc ccagaatatg gccaggggat caatccgatt agccgactgg 120
cccagatcca gcaggcaaaa aaggagaagg agccagagta cacgctcctc acagagcgag 180
gcctcccgcg ccgcagggag tttgtgatgc aggtgaaggt tggaaaccac actgcagaag 240
gaacgggcac caacaagaag gtggccaagc gcaatgcagc cgagaacatg ctggagatcc 300
ttggtttcaa agtcccgcag gcgcagccca ccaaacccgc actcaagtca gaggagaaga 360
cacccataaa gaaaccaggg gatggaagaa aagtaacctt ttttgaacct ggctctgggg 420
atgaaaatgg gactagtaat aaagaggatg agttcaggat gccttatcta agtcatcagc 480
agctgcctgc tggaattctt cccatggtgc ccgaggtcgc ccaggctgta ggagttagtc 540
aaggacatca caccaaagat tttaccaggg cagctccgaa tcctgccaag gccacggtaa 600
ctgccatgat agcccgagag ttgttgtatg ggggcacctc gcccacagcc cgagaccatt 660
ttaaagaata acatctcttc aggccacgta ccccatggac ctctcacgag accctntgag 720
caactggact atctttncag agtccaggga ttncaggttg aataccaaga ctt 773

<210> 483
<211> 794
<212> DNA
<213> Homo Sapiens

<400> 483
cattagtagc tgttnattga tcaanggttn gatataaagt tatttcanat cttcanactt 60
ttgcccagat ggaatcacaa gcattacaaa gttttttctt aaaaataaaa aaaggatagg 120
ggcaagttgg gaggggacca acctagcagt agnggcattt ganaataaat tancaaaaaa 180
atttagtatt accattnatt gatgacaaac acttaagttt tacttacatt ccatggggag 240
aaaaattcca gcgtaaacaa tgaatggaag cagtacttaa ctcgcagggc taccaggctt 300
tccatacgga ccacacgcag agcctcagng cacacacttc tgtgtncagt ancacaacat 360
caaaagcaac acagntgtat acagaaacgt aggtcattct tttcagccct aanggagatg 420
taattaacag tatcgagcac tntggaaaat cactctgcag gtttatatgg actacatgga 480
gatcatatcc tgtagtgtag tgaaagctaa gtcctcaaga gccatatgta tagatncaca 540
atgtttttta ataatcttta aaacagagat caaagttcat ttaagncctg tttgcattac 600
caaaaataaa aatgaaataa aaatggaacc aaatgaacat ctaangttta aaattcctaa 660
atnggccaat ttatncaact ggnggggaga cttattcaag ggttttgaaa gtccaggaac 720
tggtttcaag ctggaaccca ggggggcccc acaatttggc attcnctgga aactggccct 780
ggggttaagc caaa 794

<210> 484
<211> 788
<212> DNA
<213> Homo Sapiens

<400> 484
caagaccaga aggaaatgca cagttggata agatggggtt cacaattatc agaaaatgca 60
tcagtgccgt tgaaacacga ggtataaatg accaaggatt gtacagagtt gtgggggtga 120
gttcaaaggt ccagagactt ctgagtatgt tgatggatgt aaaaacatgc aatgaggtgg 180
acctggagaa ttctgcagat tgggaagtga agacaataac aagtgccttg aaacagtatt 240
tgaggagtct tccagagcct ctcatgacct atgagttaca tggagatttc attgttccag 300

ccaaaagcgg cagcccagaa tctcgtgtta atgcgatcca tttcttggta cacaaactgc 360
cagagaagaa taaagagatg ttggatattt tggtgaaaca cttaacaaat gtttcaaatc 420
actccaagca gaacctgatg actgtggcaa acttaggagt ggtgtttgga ccaactctga 480
tgaggccaca ggaagaaact gtcgctgcct catggacttg aagtttcaga atattgttgt 540
ggaaatctta attgaaaacc atgaaaagat ttttcggacg ccgnccgata ctacattccc 600
tgagcccacc tgcctgtcag catcaccccc aaatgcgcca ccaangcagt cnaagagaca 660
aggncagaga accaagaagg cccgtgggcc gtctacaatc tttggctgga gctggaaaga 720
tggtgacaat ccttaccctt tccanggagg acacccctta ccacagtctg gactcacttt 780
tcttcccg 788

<210> 485
<211> 430
<212> DNA
<213> Homo Sapiens

<400> 485
agtaaattac agtttatttc atttacagag accttgaggc aaaaaggtgg tgtttggaaa 60
acagcacacg ggtgaggagc accggagaag cctgttacaa atacgccagt gcacgctgcc 120
agtgcagtga gtgtggggtc ctgcaggtgg ccgtctagga agggcaggct tgagacgcgc 180
gtctctgctt ccctntgact tgagaccatc tcccttgnac caacagcagc ttntccaagc 240
taggctgcca cagccaagca cacactctgc aaacctatca ctgcgagtng tacagttccc 300
tttanaatcg nagcagcang tgctcaggga ggagagggag ccnnngtggc tctggtggcg 360
tgactgccag tgnaggcgga cacangtggc ataaggctgc ccgtcccctc tcattcttat 420
atgctgngat 430

<210> 486
<211> 831
<212> DNA
<213> Homo Sapiens

<400> 486
aaagtgtagt gccatcgaca caggctgtca cgttttcccc ttcttttcac aatcaagcaa 60
ttatggtgac aaaaccagtg caggaatata aaaaggaata cacagtgcag caggccttgt 120
tttgtacttc tggaattgtt acttctatac cggtgccctt ggcaggaagt gcccttctcc 180
catatcatat ttcatctact gcatgtcagg ccaaggctca tctgtcatct gatgatagta 240
attcaaatgg tgattctgcc caagtgcata ttgccacaaa aaacagagaa gaaaaagcag 300
cttgtctcag aaatatttgt ttaccttcag aacacaatcc aggtaatcag aatgatttta 360
aaccaactaa tgacgatatt gaaatgcaga gttcctcaaa attaccaaat gatcctgcaa 420
ttattagcaa cttttctgca gcagtggtgc atacgatagt aaatgaaact ttagagtcaa 480
tgacatcatt ggaagttaca aaaatggttg atgaacgtac agattattta actaaatctt 540
taaaggagaa aacccctcca ttttcccact gtgatcaggc agtgctgcaa tgcagtgaag 600
ctagtagcaa taaggacatg tttgctgacc ggttatctaa atctattatt aaacattcca 660
tagataagag caaatcagtg atcccaaata tagataaaaa tgcagtatac aaggaaagct 720
tgcctgtttc tggagaagaa tcacagttga cacccagaaa agtcttncca aatttnctga 780
ctcttcagaa tcagtttaac ttactggttc acttttagct gcaaaggaat g 831

<210> 487
<211> 728
<212> DNA
<213> Homo Sapiens

<400> 487
gacggagtct gtctctgtcg cccaggatgg agtacagtgg cacaatctca gctcactgca 60
atctctgcct cccaggttca agcaattctc ctgccttagc ctcccaagta gctgggatta 120
caggtgcctg ccaccacgcc tggctaattt ttgtattttt ggtagagacg gggtttcacc 180

atgttgccca ggctggtctt gaactcctga cctcaagtga tccaccccca cccccattgg 240
cttcccagag ttctgggatt acaggcgtga atcaccgcgc ccagcccaaa tcgccgaagt 300
ctttatctcc taccttgatc tctgtagcag aaaagaacag tatagatatc aattgtcatc 360
aacagatgca acatatcttg taaatcaata tattttcaag tgaggtctct gaatcacctg 420
cactgaaatc atctgtgatg cttatcaagc atgcagattc tcaggaccct tcactgactt 480
cataaatctt catctctgga ggtgagaccc tggacactgt atatgcaacg agcacaccac 540
caatcctgga tgagccccgc tttttctctg tgccagaacc ttaatgccac gcagcattac 600
attaagtcac attacaactt tggtcaatgg aaacacaggg tctttttctg acaaaatgcc 660
atcaagccag gtttggctcc ccacttaagt tcaaatnttt aatcattaat tttctgagcc 720
taaaatgc 728

<210> 488
<211> 788
<212> DNA
<213> Homo Sapiens

<400> 488
gtgggccctg tcctttctcc ccagctcctg ccccggagcc gggccctggc gaggcaggaa 60
tggccccgag gcctccgacc gccgcgcccc aggaatcagt gacattcaaa gatgtgtctg 120
tggacttcac ccaggaagaa tggtaccatg tcgaccctgc tcagaggagc ttatacaggg 180
atgtgatgct ggagaactat agccacctgg tttctcttgg atatcaagtt tccaagccag 240
aggtgatctt caaattggag caaggagaag agccatggat atcagaggga gaaatccaac 300
gacctttcta tccagactgg aagaccaggc ctgaagtcaa atcatcacat ttgcagcagg 360
atgtatcaga agtatcccac tgcacacatg atctcttaca tgctacatta gaagactcct 420
gggatgttag cagccagtta gacgggcaac aggaaaactg gaagagacat ctgggatcag 480
aggcatccac ccagaagaaa ataattacac cacaagaaaa ttttgagcaa aataaatttg 540
gtgaaaattc tagattgaac accaatttgg ttacacaact gaacattcct gcaagaataa 600
ggcctagtga atgtgagacc cttggaagca atttgggaca taatgcagac ttacttaatg 660
agaataatat tcttgcaaaa aagaaaccct tttagtgnga taatgtagaa aagnctttan 720
tcatagatca tcgnttacta aaccttgaga aaacccctta anggaaaagg gagctttcct 780
aatgggac 788

<210> 489
<211> 875
<212> DNA
<213> Homo Sapiens

<400> 489
aaagagatgg ggtttcacca tgttgtccag gctggtcttg aactctgggt tcaagcagtc 60
tatctgcctt agccacccaa agtgctggga ttacaggtgt gagacaccat acctagccaa 120
gttaattttt ttaatggtga aatcttttct ttgcacataa aatgagccag tgcatgttgc 180
ttctctgagt acaagacaaa atttatggca atgggcaatt agacttatac ttttctgcaa 240
gaaaattaac gggaaaattc tcctcttagt tttctgttgt tttcccattg atctgatact 300
gtaggcttaa gaaagtgctt tttcatgggc atgccataaa aagtacaata aggggactta 360
atagttctgt gaaactggca tatgttagct gaaagtataa ttgtaactgg gaaaagggga 420
aaaaagtcac tagtagttca accatctaca gtttctgtta aattgtggtt tgtaagcctc 480
caagaagttg ctttaaatag tttgtgataa atttgcatac attttgctcc cacttatact 540
tttaagaatt ctcaaagtgt ccaacccata ggtgcccatt aaatgtttgt gtatctgatc 600
atcttaaaat ttattttaaa gccctctgag tcccaaaaat aaccttttca ctggcaaggc 660
catggggccc caaatccagg aaaccctggc atttttaacc caacttttac ccttataggc 720
tggaatcata ctgngggaaa cccacttcac atcttttggc tttcagtctt caatctgncc 780
cnaatggaaa atgggttggg cctagttgga actaaattct tttgaatggg ggactttcct 840
ggaaattggg aactnggttt ccatggggga aagtt 875

<210> 490

<211> 844
<212> DNA
<213> Homo Sapiens

<400> 490
aagtgtttga gattgcactt aaacggaact tgcctgtgaa tttcgaggtg gcccgggaga 60
gtggcccacc ccacatgaag aactttgtga ccaaggtttc ggttggggag tttgtggggg 120
aaggtgaagg gaaaagcaag aagatttcaa agaaaaatgc cgccatagct gttcttgagg 180
agctgaagaa gttaccgccc ctgcctgcag ttgaacgagt aaagcctaga atcaaaaaga 240
aaacaaaacc catagtcaag ccacagacaa gcccagaata tggccagggg atcaatccga 300
ttagccgact ggcccagatc cagcaggcaa aaaaggagaa ggagccagag tacacgctcc 360
tcacagagcg aggcctcccg cgccgcaggg agtttgtgat gcaggtgaag gttggaaacc 420
acactgcaga aggaacgggc accaacaaga aggtggccaa gcgcaatgca gccgagaaca 480
tgctggagat ccttggtttc aaagtcccgc aggcgcagcc caccaaaccc gcactcaagt 540
cagaggagaa gacacccata aagaaaccag gggatggaag aaaagtaacc ttttttgaac 600
ctgctcttgg ggatgaaaat gggactagta ataaagagga tgagttcagg atgccttatc 660
taagtcatca gcagctgcct gctggaattc tttccatggt gcccgangtc gcccaagctg 720
taggaagtta gtcaaggaca tnacacccaa gattttacca ggcagcttcg aatcttgcca 780
nggcncngta ctgccatgat agcccanagt tgttgtattg gggcancttt gccccaggcc 840
ggga 844

<210> 491
<211> 825
<212> DNA
<213> Homo Sapiens

<400> 491
cattagtagc tgtttattga tcaatggttt gatataaagt tatttcanat cttcagactt 60
ttgcccagat ggaatcacaa gcattacaaa gtttttcttt aaaaataaaa aaaggatagg 120
ggcaagttgg gaggggacca acctagcagt agtggcattt gagaataaat taacaaaaaa 180
atttagtatt accatttatt gatgacaaac acttaagttt tacttacatt ccatggggag 240
aaaaattcca gcgtaaacaa tgaatggaag cagtacttaa ctcgcagggc taccaggctt 300
tccatacgga ccacacgcag agcctcagtg cacacacttc tgtgtacagt aacacaacat 360
caaaagcaac acagctgtat acagaaacgt aggtcattct tttcagccct aatggagatg 420
taattaacag tatcgagcac tctggaaaat cactctgcag gtttatatgg actacatgga 480
gatcatatcc tgtagtgtag tgaaagctaa gtcctcaaga gccatatgta tagatacaca 540
atgtttttta ataatcttta aaacagagat caaagttcat ttaaagtcct gtttgcatta 600
acaaaaataa aaatganaat aaaaatggac caaatgatca tctaaagttt aaaattccta 660
aatggtccaa tttatacaac tgggggagac ttattcaagg tttttgaaag tccaggactg 720
gtttcagctg aaccagangg cccccaattt gcatcactgg aactgncctg ggtttagcca 780
aggaaattaa aaaagnctta acccccttcc cctgggattt gaacc 825

<210> 492
<211> 946
<212> DNA
<213> Homo Sapiens

<400> 492
gaggacagca atttaatggc aaaggaaaga caagacaggc tgcgaaacac gatgctgctg 60
ccaaagcgtt gaggatcctg cagaatgagc ccctgccaga gaggctggag gtgaatggaa 120
gagaatccga agaagaaaat ctcaataaat ctgaaataag tcaagtgttt gagattgcac 180
ttaaacggaa cttgcctgtg aatttcgagg tggcccggga gagtggccca ccccacatga 240
agaactttgt gaccaaggtt tcggttgggg agtttgtggg ggaaggtgaa gggaaaagca 300
agaagatttc aaagaaaaat gccgccatag ctgttcttga ggagctgaag aagttaccgc 360
ccctgcctgc agttgaacga gtaaagccta gaatcaaaaa gaaaacaaaa cccatagtca 420

agccacagac aagcccagaa tatggccagg ggatcaatcc gattagccga ctggcccaga 480
tccagcaggc aaaaaaggag aaggagccag agtacacgct cctcacagag cgaggcctnc 540
cgcgccgcag ggagtttgtg atgcaggtga aggttggaaa ccacacttgc agaaggaacg 600
ggcaccaaca agaaggtggc caagcgcaat gcacccgaga acatgctgga gatccttggt 660
ttcaaaagtc ccgcangcgc agcccaccaa acccggactn aagtcagang agaagacccc 720
attaaggaaa ccangggatg gaagaaaagt ancnttttga anctggctnt tgggattaaa 780
atgggcttgt antaaagagg atgagttcag gatgncntat ctaagtcatn aacacttgct 840
gctggaaatc tttccatggg ggccgaggtc ncccagcttt taggagttat canggccntt 900
ccncccaaga attttcccgg gcagtttcca atctgccaag gccccg 946

<210> 493
<211> 804
<212> DNA
<213> Homo Sapiens

<400> 493
ggtctttatg tgcttaaata acgctgaatt ataattagcc acacaaataa tgagagtttt 60
attttttttt tctggctcac tccaaatcag cctgttaagg tatatttcct tctacagcct 120
ttcctgattt tgcatgttct cattcccaaa gtagtctacc ttagtttaca ctcaaaggta 180
gcacttgttg aaactacatg acagaaacag gctgcaaagg tggacaaggg gaagcatgtc 240
cctcttgtct tgataaatca gtgccacaca cagaacccac attttctgag acattatctt 300
cattatagag ccgtttgatt ccatcataga agtcatccac ttccatttcc tctactttgc 360
gtttagtaga ggtctgcttg cacccactgg cagctgggag atgatggtaa aaggctgctg 420
tacctctgac tggcacttct ggcttgctgt tgtccttgga gaagtctggg cctgggacag 480
aggagggatg taatctgaac actcctttgt cacaggtcac cagggtgtgc ttgaggggac 540
ggtagacata aacggaattc agaggcaggg aagactgcag agtanaaagg tgatgtgccc 600
aagcttccga ccatggatca actgggagct atncatctgg ctttctgaag cagntcaatt 660
gtaagagaaa gcccaatccn ggaatggagt tcntccattt tcagactaac cctgggcncn 720
aagcaaggca tgggatcccc tggaattgcc anaaanttgg gttgcagggn ccatacncgg 780
nggnaagtaa ttngctttgg gtaa 804

<210> 494
<211> 856
<212> DNA
<213> Homo Sapiens

<400> 494
gaaaggttgg aaagaataaa tagggccagg gaacaaggat ggagaaatgt gctaagtgct 60
ggtggaagtg gtgaagtaaa ggctcctttt ctgggcagtg gagggactat agctccatca 120
tctttttctt ctcgaggaca gtatgaacat taccatgcca tttttgacca aatgcagcaa 180
caaagagcag aagataatga agctaaatgg aaaagagaaa tatatggtcg aggtcttcca 240
gaaaggcaaa aagggcagct agctgtagaa agagctaaac aagtagaaga gttcctgcag 300
cgaaaacggg aagctatgca gaataaagct cgagccgaag gacatatggg aatcctgcaa 360
aacctggcag ctatgtatgg aggcaggccc agctcttcaa gaggagggaa gccaagaaac 420
aaagaggaag aggtttatct ggcaagactg aggcaaataa gactacagaa tttcaatgag 480
cgccaacaga ttaaagccaa acttcgtggt gaaaagaaag aagctaatca ttctgaagga 540
caagaaggaa gtgaagaggc tgacatgagg cgcaaaaaaa atcgaatcac tgaaggccca 600
tgcaaatgca cgtgctgctg tctaaaagaa cactagaacg aaagagaaag gaggcttatg 660
agagagaaaa aaaagtgtgg gaagagcatt tggtggctaa aggagttaag agtctgatgg 720
ttcttcccct ttgggaccag catgaaacaa ggtggctttt ccttcaaagc caccggatga 780
aaanctggta nttctggnac ttcacttttn aagaanttgg ccgtggnngt agtttaactg 840
gatacccggg aacttc 856

<210> 495
<211> 757

<212> DNA
<213> Homo Sapiens

<400> 495
agataataaa aatttaatag caatatcata aaataaacac acatattaaa aaatcaagta 60
tttagtttcg gatattagaa ataatataca taataaattc aacatactga tagtgctgca 120
agataagatt ttatttttca aattacatat tatgccaacc agcctgcttt ggactcagag 180
gttcaaaaac tttgctttta ttacgaagaa catntggact gtagacacct ntaacgaaac 240
caggttatac ttggcatatt gngattgaag ctgtgtgatc aacatcttaa tgacctaact 300
aaatcctntc ataacagaaa gaagttcaac aggcaaacat ttccctccct aggatcctag 360
ttaccaaaac tgtcacagng ncaaaataaa aataattatt tcctcctttt taacatctta 420
ttgnccttga agcttatgta tggaggaagt taaaaaccaa aagagcaact ttaagctata 480
tgctaagtca gngttaaatc cacagactaa tttttcgata tagnattcct ggntctggnc 540
cttaaagaga aataaaggca ttaaaccact tttttatatg tcaaggaaat ataatttngc 600
tattctttca taatcaaatc tttcaatgga tttctaagac tggnttctac agcctgngng 660
ctagttccag gggacacact gattgtaaaa nggacttggn ggaaatntaa aactttaagg 720
gctaaaaaat ttcatctttc aaaatgntnt agatgtt 757

<210> 496
<211> 1759
<212> DNA
<213> Homo Sapiens

<400> 496
cgaggatcca ggcgcaggag gacagagcaa tgggtgagag aactcttcac gctgcagtgc 60
ccacaccagg ttatccagaa tctgaatcca tcatgatggc ccccatttgt ctagtggaaa 120
accaggaaga gcagctgaca gtgaattcaa aggcattaga gattcttgac aagatttctc 180
agcccgtggt ggtggtggcc attgtagggc tataccgcac aggaaaatcc tatctcatga 240
atcgtcttgc aggaaagcgc aatggcttcc ctctgggctc cacggtgcag tctgaaacta 300
agggcatctg gatgtggtgt gtgccccacc tctctaagcc aaaccacacc ctggtccttc 360
tggacaccga gggcctgggc gatgtagaaa agagtaaccc taagaatgac tcgtggatct 420
ttgccctggc tgtgcttcta agcagcagct ttgtctataa cagcgtgagc accatcaacc 480
accaggccct ggagcagctg cactatgtga ctgagctagc agagctaatc agggcaaaat 540
cctgccccag acctgatgaa gctgaggact ccagcgagtt tgcgagtttc tttccagact 600
ttatttggac tgttcgggat tttaccctgg agctaaagtt agatggaaac cccatcacag 660
aagatgagta cctggagaat gccttgaagc tgattccagg caagaatccc aaaattcaaa 720
attcaaacat gcctagagag tgtatcaggc atttcttccg aaaacggaag tgctttgtct 780
ttgaccggcc tacaaatgac aagcaatatt taaatcatat ggacgaagtg ccagaagaaa 840
atctggaaag gcatttcctt atgcaatcag acaacttctg ttcttatatc ttcacccatg 900
caaagaccaa gaccctgaga gagggaatca ttgtcactgg aaagcggctg gggactctgg 960
tggtgactta tgtagatgcc atcaacagtg gagcagtacc ttgtctggag aatgcagtga 1020
cagcactggc ccagcttgag aacccagcgg ctgtgcagag ggcagccgac cactatagcc 1080
agcagatggc ccagcaactg aggctcccca cagacacgct ccaggagctg ctggacgtgc 1140
atgcagcctg tgagagggaa gccattgcag tcttcatgga gcactccttc aaggatgaaa 1200
accatgaatt ccagaagaag cttgtggaca ccatagagaa aaagaaggga gactttgtgc 1260
tgcagaatga agaggcatct gccaaatatt gccaggctga gcttaagcgg ctttcagagc 1320
acctgacaga aagcattttg agaggaattt tctctgttcc tggaggacac aatctctact 1380
tagaagaaaa gaaacaggtt gagtgggact ataagctagt gcccagaaaa ggagttaagg 1440
caaacgaggt cctccagaac ttcctgcagt cacaggtggt tgtagaggaa tccatcctgc 1500
agtcagacaa agccctcact gctggagaga aggccatagc agcggagcgg gccatgaagg 1560
aagcagctga gaaggaacag gagctgctaa gagaaaaaca gaaggagcag cagcaaatga 1620
tggaggctca agagagaagc tttcaggaat acatggncca aatggagaag aagttggagg 1680
angaaaggga aaaccntntc agagagcctt gaaaaggttg ctaaacacaa agcttgaagg 1740
tncagaagaa aatgcttaa 1759

<210> 497
<211> 842
<212> DNA
<213> Homo Sapiens

<400> 497
atgacaagca atatttaaat catatggacg aagtgccaga agaaaatctg gaaaggcatt 60
tccttatgca atcagacaac ttctgttctt atatcttcac ccatgcaaag accaagaccc 120
tgagagaggg aatcattgtc actggaaagc ggctggggac tctggtggtg acttatgtag 180
atgccatcaa cagtggagca gtaccttgtc tggagaatgc agtgacagca ctggcccagc 240
ttgagaaccc agcggctgtg cagagggcag ccgaccacta tagccagcag atggcccagc 300
aactgaggct ccccacagac acgctccagg agctgctgga cgtgcatgca gcctgtgaga 360
gggaagccat tgcagtcttc atggagcact ccttcaagga tgaaaaccat gaattccaga 420
agaagcttgt ggacaccata gagaaaaaga agggagactt tgtgctgcag aatgaagagg 480
catctgccaa atattgccag gctgagctta agcggctttc agagcacctg acagaaagca 540
ttttgagagg aatttctctg ttcctggagg acacaatctc ctacttagaa gaaaagaaac 600
aggttgagtg ggactataag ctagtgccca gaaaaggagt taaggcaaac gaggtcctcc 660
agaacttcct gcagtcacan gtggttgtag aggaatccat cctgcagtca gacaaagccc 720
tcactgctgg agagaaggcc atacaaccgg aaccgggcca tgaaggaagc acttgagaag 780
gaacaggagc tgcttagaga aaaaccgaag gagccagcag ccaaatggat ggaggctcaa 840
ga 842

<210> 498
<211> 707
<212> DNA
<213> Homo Sapiens

<400> 498
gagcaataaa gctttttaat cacctgggtg caggctggct gagtccgaaa agacagtcag 60
tgaagggaga tagggttggg accattttac aggatttggg ttggtaaagg aaaattacag 120
tcaaaggggg ttgttctctg gcgggcagag gtgggtgtca caagttgctt agtgggggag 180
cttttgagcc aggatgagcc aggagaagga atttcacaag gtaatgtcat cagttaaggc 240
aggaacaggc cattttcact tcttttgtga ttcttcactt gcttcaggcc atctggacgt 300
atgtacatgc aggtcacagg ggatatgatg gcttagcttg ggctcagagg cctgacattt 360
agtatattta ctggaatatt caggctctta aatacgtgag ccaagatatt ttgtccctac 420
tccaagtagc ttggaagccc caggtagagt gacaatcatt atgttgctag ccatgtcaag 480
gatctttaag agccttaact gttcattttt agtgctttca atttttcttt tcagttgatt 540
aatctcttta tttaactgct cagatttctt ttgaaattct tccttaagca tttcttcttg 600
naccttcagc ttggggttta acagcctttc atgctctctg aaaagggttt ncctttcctn 660
cttcaacttc ttctccattt gggccatgna ttcctggaag cttctct 707

<210> 499
<211> 772
<212> DNA
<213> Homo Sapiens

<400> 499
gtggagcagt accttgtctg gagaatgcag tgacagcact ggcccagctt gagaacccag 60
cggctgtgca gagggcagcc gaccactata gccagcagat ggcccagcaa ctgaggctcc 120
ccacagacac gctccaggag ctgctggacg tgcatgcagc ctgtgagagg gaagccattg 180
cagtcttcat ggagcactcc ttcaaggatg aaaaccatga attccagaag aagcttgtgg 240
acaccataga gaaaaagaag ggagactttg tgctgcagaa tgaagaggca tctgccaaat 300
attgccaggc tgagcttaag cggctttcag agcacctgac agaaagcatt ttgagaggaa 360
ttttctctgt tcctggagga cacaatctct acttagaaga aaagaaacag gttgagtggg 420
actataagct agtgcccaga aaaggagtta aggcaaacga ggtcctccag aacttcctgc 480

agtcacaggt ggttgtagag gaatccatcc tgcagtcaga caaagccctc actgctggag 540
agaaggccat agcagcggag cgggccatga aggaagcagc tgagaaggaa caggagctgc 600
taagagaaaa acagaaggag cagcagcaaa tgatggaggc tcaagagaga agctttcagg 660
aatacatggn ccaaatggag aagaagttgg aggangaaag ggaaaaccnt ntcagagagc 720
cttgaaaagg ttgctaaaac acaagcttga aggtncagaa gaaaatgctt aa 772

<210> 500
<211> 787
<212> DNA
<213> Homo Sapiens

<400> 500
ggctgttttt agtttttctt tgatttcaaa tcttcttttc aacacctccc tcttctctat 60
gcgattgaac agttcttgct ctctctcttt ctctgtcatc tgttccagac gggccctgtc 120
ttcctcatct cccatgaggt cttctccata gccatcatgg aactcttcat cttctgagga 180
agagtctgaa tctgaactgg aagaggagct gttgctgtca gagtctgaca cttcaccttc 240
ctcaggggct gagctctcag ctgaactgtc tttgtctgaa ctgcctgagg aggcagtttt 300
gttggcctgt ttcttcatgg ttcctttctt ctctattttt ctggctttcc ctttcttctt 360
atttttctcg tgccgaattc ggcacgagga actattcgag tttttttttt tttttttttt 420
tgagacggag tctcgctccg tcgcccaggc tggagtgcag cggcgcgatc tcgactcact 480
gcaagctccg cctcccgggc ccacgccatt ctcccgcccc agcctcccgt gtagctggga 540
ctacaggcgc gtgccaccac gcccggccaa tttttgcatt tttagcanag acggggtttc 600
accgggttag ccaggaaggg ctcgatcccc tgacctcgng atccacccgt cttggcctcc 660
caaagtgctg ggacccacag gcaatgagtt ggatttttaa ctactgggtt taaggccagg 720
caggccccag gcctgggttt tgggcctggc nctggcctgn ccggccttgg gtttaccttc 780
ctgggg 787

<210> 501
<211> 886
<212> DNA
<213> Homo Sapiens

<400> 501
agttntnacc gctcgnctcg cgcgcctgca ggtcgacact agtggatcca aagcgggatt 60
ttaccctgga gctaaagtta gatggaaacc ccatcacaga agatgagtac ctggagaatg 120
ccttgaagct gattccaggc aagaatccca aaattcaaaa ttcaaacatg cctagagagt 180
gtatcaggca tttcttccga aaacggaagt gctttgtctt tgaccggcct acaaatgaca 240
agcaatattt aaatcatatg gacgaagtgc cagaagaaaa tctggaaagg catttcctta 300
tgcaatcaga caacttctgt tcttatatct tcacccatgc aaagaccaag accctgagag 360
agggaatcat tgtcactgga aagcggctgg ggactctggt ggtgacttat gtagatgcca 420
tcaacagtgg agcagtacct tgtctggaga atgcagtgac agcactggcc cagcttgaga 480
acccagcggc tgtgcagagg gcagccgacc actatagcca gcagatggcc cagcaactga 540
ggctccccac agacacgctc caggagctgc tggacgtgca tgcagcctgt gagagggaag 600
ccattgcagt cttcatggag cactccttca aggatgaaaa ccatgaattc cagaagaagc 660
ttgtggacac catagagaaa aagaagggag actttgtgct gcagaatgaa gaggcatctg 720
ccaaatattg ccaggctgac ttaagcggct ttcagagcac ctgacagaaa gcattttgag 780
aggaattttc tctggtcctg gaggacacaa tctctactta gaagaaagga aacaggntga 840
gtggggacta ttagctagtg nccagaaaag gagttaaggc aaacga 886

<210> 502
<211> 626
<212> DNA
<213> Homo Sapiens

<400> 502

gggagcaata aagcttttta atcacctggg ngcaggctgg ctgagtccna aaagacagtc 60
agngaaggga nanagggttg ggaccatttt acaggatttg ggttggtaaa ggaaaattac 120
ngtcaaaggg ggttgttctn tggcgggcaa aggggggngt cacaagttgc ttannggggg 180
ancttttgag ccaggatgan ccnggaaaag gaatttcnca aggnaatggc atcagttaag 240
gcaggaacag gccattttca cttnttttgg gantcttcac ttgcttcagg ccatntggaa 300
nattcaggct nttaaanacn ngagccnana nattttggcc ctactccaag tagcttggaa 360
nccccaggta aagggacnat cattatgntg ctagccntgt caaggatntt taaaagcctt 420
aactggncat ttttanggct ttcaattttt tnttttagtn gattaancnc tttatttaac 480
ngctcaaatt tcttttgaaa ntnttcctta agcntttctt cttgnccttn ancttgggnt 540
ttancagcct ttcatgcnct ttgaaaaggn tttccctttc ctcctccaac ttcntctcca 600
tttggggcca tgntattncc tgggaa 626

<210> 503
<211> 884
<212> DNA
<213> Homo Sapiens

<400> 503
cgaggatcca ggcgcaggag gacagagcaa tgggtgagag aactcttcac gctgcagtgc 60
ccacaccagg ttatccagaa tctgaatcca tcatgatggc ccccatttgt ctagtggaaa 120
accaggaaga gcagctgaca gtgaattcaa aggcattaga gattcttgac aagatttctc 180
agcccgtggt ggtggtggcc attgtagggc tataccgcac aggaaaatcc tatctcatga 240
atcgtcttgc aggaaagcgc aatggcttcc ctctgggctc cacggtgcag tctgaaacta 300
agggcatctg gatgtggtgt gtgccccacc tctctaagcc aaaccacacc ctggtccttc 360
tggacaccga gggcctgggc gatgtagaaa agagtaaccc taagaatgac tcgtggatct 420
ttgccctggc tgtgcttcta agcagcagct ttgtctataa cagcgtgagc accatcaacc 480
accaggccct ggagcagctg cactatgtga ctgagctagc agagctaatc agggcaaaat 540
cctgccccag acctgatgaa gctgaggact ccagcgagtt tgcgagtttc tttccagact 600
ttatttggac tgttcgggat tttaccctgg agctaaagtt agatggaaac cccatcacag 660
aagatgagta cctggagaat gccttgaagc ttgantncag gcaagaatnc caaaantcaa 720
aattcaaaca tgcctagaga gtgnattaag gcantttctt ccgaaaaccg gaagtgcttt 780
tgcctttgac cgggctacaa atggacaagc caatatttaa aatcattntg gacnaantgc 840
cngaagaaaa tctggaaagg catttcctta tgccatcaga caac 884

<210> 504
<211> 612
<212> DNA
<213> Homo Sapiens

<400> 504
gagacggagt ttcgctctgt cgcccaggct ggagtgcagt ggcgcgatct cgactcactg 60
caagctccgc ctcctgggtt cacgccattc tcctgcctca gcctcccgtg tagctgggac 120
tacaggcgcg tgccaccatg cccggctaat ttttgtattt ttagtagaga cggggtttca 180
ccgtgttagc caggatggtc tcgatctcct gacctcgtga tccacccgtc tcggcctccc 240
aaagtgctgg gattacaggc aatgagttga tttttaacta ctgggtttag gccaggcagg 300
cccaggcctg gttttgggcc tggcgctggg ctgcctgtct ttggttttac ttccttggtg 360
ntttttctta aaacaggtac tgagtatcaa acaatataaa acaatataag aaggtctctc 420
tcttccctca attctagctg caagttttga gcactagaca gcagaaataa attcctaaaa 480
tgttgagttg agcaaatagt tcaatgctat ccctatcaaa ctaccaatga cattntttac 540
nagaaattag aaactacttt aaaaatttca tatgggaacn aaaaaagagc cttacccnag 600
gcnaanccta aa 612

<210> 505
<211> 2215
<212> DNA

<213> Homo Sapiens

<400> 505
ctcagatgct cactgcagtc caagagatct cccatctcat tgagccgctg gccaatgctg 60
cccgggctga agcctcccag ctgggacaca aggtgtccca gatggcgcag tactttgagc 120
cgctcaccct ggctgcagtg ggtgctgcct ccaagaccct gagccacccg cagcagatgg 180
cactcctgga ccagactaaa acattggcag agtctgccct gcagttgcta tacactgcca 240
aggaggctgg tggtaaccca aagcaagcag ctcacaccca ggaagccctg gaggaggctg 300
tgcagatgat gaccgaggcc gtagaggacc tgacaacaac cctcaacgag gcagccagtg 360
ctgctggggt cgtgggtggc atggtggact ccatcaccca ggccatcaac cagctagatg 420
aaggaccaat gggtgaacca gaaggttcct tcgtggatta ccaaacaact atggtgcgga 480
cagccaaggc cattgcagtg actgttcagg agatggttac caagtcaaac accagcccag 540
aggagctggg ccctcttgct aaccagctga ccagtgacta tggccgtctg gcctcggagg 600
ccaagcctgc agcggtggct gctgaaaatg aagagatagg ttcccatatc aaacaccggg 660
tacaggagct gggccatggc tgtgccgctc tggtcaccaa ggcaggcgcc ctgcagtgca 720
gccccagtga tgcctacacc aagaaggagc tcatagagtg tgcccggaga gtctctgaga 780
aggtctccca cgtcctggct gcgctccagg ctgggaatcg tggcacccag gcctgcatca 840
cagcagccag cgctgtgtct ggtatcattg ctgacctcga caccaccatc atgttcgcca 900
ctgctggcac gctcaatcgt gagggtactg aaactttcgc tgaccaccgg gagggcatcc 960
tgaagactgc gaaggtgctg gtggaggaca ccaaggtcct ggtgcaaaac gcagctggga 1020
gccaggagaa gttggcgcag gctgcccagt cctccgtggc gaccatcacc cgcctcgctg 1080
atgtggtcaa gctgggtgca gccagcctgg gagctgagga ccctgagacc caggtggtac 1140
taatcaacgc agtgaaagat gtagccaaag ccctgggaga cctcatcagt gcaacgaagg 1200
ctgcagctgg caaagttgga gatgaccctg ctgtgtggca gctaaagaac tctgccaagg 1260
tgatggtgac caatgtgaca tcattgctta agacagtaaa agccgtggaa gatgaggcca 1320
ccaaaggcac tcgggccctg gaggcaacca cagaacacat acggcaggag ctggcggttt 1380
tctgttcccc agagccacct gccaagacct ctaccccaga agacttcatc cgaatgacca 1440
agggtatcac catggcaacc gccaaggccg ttgctgctgg caattcctgt cgccaggaag 1500
atgtcattgc cacagccaat ctgagccgcc gtgctattgc agatatgctt cgggcttgca 1560
aggaagcagc ttaccaccca gaagtggccc ctgatgtgcg gcttcgagcc ctgcactatg 1620
gccgggagtg tgccaatggc tacctggaac tgctggacca tgtactgctg accctgcaga 1680
agccaagccc agaactgaag cagcagttga caggacattc aaagcgtgtg gctggttccg 1740
tcactgagct catccaggct gctgaagcca tgaagggaac agaatgggta gacccagagg 1800
accccacagt cattgctgag aatgagctcc tgggagctgc agccgccatt gaggctgcag 1860
ccaaaaagct agagcagctg aagccccggg ccaaacccaa ggaggcagat gagtccttga 1920
actttgagga gcagatacta gaagctgcca agtccattgc agcagccacc agtgcactgg 1980
taaaggctgc gtcggctgcc agagagaact agtggcccaa gggaaagtgg gtgccattcc 2040
aagcaatgca ctggacgatg ggcagtggtc ccangggcct catttctgct gcccngatgg 2100
tggcttgcgg ccaccaacaa nttgtgtgaa ggcagccaat gcaactgtcc aagggcatgc 2160
caagccngga anaactnatn ttattcagcc caacaggtaa cttgcctttc acaag 2215

<210> 506
<211> 742
<212> DNA
<213> Homo Sapiens

<400> 506
ggcacgaggt aacccaaagc aagcagctca cacccaggaa gccctggagg aggctgtgca 60
gatgatgacc gaggccgtag aggacctgac aacaaccctc aacgaggcag ccagtgctgc 120
tggggtcgtg ggtggcatgg tggactccat cacccaggcc atcaaccagc tagatgaagg 180
accaatgggt gaaccagaag gttccttcgt ggattaccaa acaactatgg tgcggacagc 240
caaggccatt gcagtgaccg ttcaggagat ggttaccaag tcaaacacca gcccagagga 300
gctgggccct cttgctaacc agctgaccag tgactatggc cgtctggcct cggaggccaa 360
gcctgcagcg gtggctgctg aaaatgaaga gataggttcc catatcaaac accgggtaca 420
ggagctgggc catggctgtg ccgctctggt caccaaggca ggcgccctgc agtgcagccc 480

cagtgatgcc tacaccaaga aggagctcat agagtgtgcc cggagagtct ctgagaaggt 540
ctcccacgtc ctggctgcgc tccaggctgg gaatcgtggc acccaggcct gcatcacagc 600
agccagcgct gtgtctggta tcattgctga cctcgacacc accatcatgt tcgccacttg 660
ctggcacgct caatcgtgag ggtactgaaa ctttcgctga ccaccgggan ggcatnctga 720
agactgcgaa ngtgctggtg ga 742

<210> 507
<211> 735
<212> DNA
<213> Homo Sapiens

<400> 507
gtaggtagaa tcatttttat tggagcatga cctgtttggg gcttataact ctgcagcccc 60
tatgggtagc tgggggtggg ggaagatagt atcaaaaaac ggtgaagaga gctgatgagg 120
ctgtggggac tggctggaag ctgctggcag ggtggagtgg gctggggccc cggcagattc 180
agatcgaggt acagcagcgt taataatact cttggagcgt taatactctg gggaggggca 240
ggcacttggg gggccctagg gcatgaaggc acttggggtt ggggagggga caggggatgt 300
actgcgggac tgggcggggc caggccctgg ggtttggcag gcactttggg gagtgctggg 360
gttgggcagg ttgggccccg acagcccaga aggctttggt agtggcacgc acagtctctg 420
ggccgggtct gcattaaata gaagaggctt ctttagtgct catctcgaag ctctgaaggc 480
agaaacttgt actgctgctg ccggatctgg gccagtttct tccgngcctc ttccagctct 540
cgttccttcc gaagcatttc ttcctgngct gcgatgatct gggcaatgcc cgccaaccat 600
cttctcttta ccaccactgg cttnattctc ctgctcttca aaggctgcaa ccttctgggc 660
tgntttnacc agattatctg angctcgctt cactgngttg ncagcaacct tgaatccgtt 720
tcatttgccc tccag 735

<210> 508
<211> 666
<212> DNA
<213> Homo Sapiens

<400> 508
gtaggtagaa tcatttttat tggagcatga cctgtttggg gcttataact ctgcagcccc 60
tatgggtagc tgggggtggg ggaagatagt atcaaaaaac ggtgaagaga gctgatgagg 120
ctgtggggac tggctggaag ctgctggcag ggtggagtgg gctggggccc cggcagattc 180
agatcgaggt acagcagcgt taataatact cttggagcgt taatactctg gggaggggca 240
ggcacttggg gggccctagg gcatgaaggc acttggggtt ggggagggga caggggatgt 300
actgcgggac tgggcggggc caggccctgg ggtttggcag gcactttggg gagtgctggg 360
gttgggcagg ttgggccccg acagcccaga aggctttggt agtggcacgc acagtctntg 420
ggccgggtct gcattaaata gaagaggctt ctttagtgct catctngaag ctctgaaggc 480
agaaacttgt actgctgctg ccggatctgg gccagttttc ttccgcgcct tttccagctc 540
tcgttccttt ccgaagcatt tcttcctgng ctgccatgat tctgggccat gcccgccaac 600
catcttctct tttacccanc attggcttna ttctcctgct ctttcaaaag gcttgnagnc 660
tttctg 666

<210> 509
<211> 818
<212> DNA
<213> Homo Sapiens

<400> 509
ctcagatgct cactgcagtc caagagatct cccatctcat tgagccgctg gccaatgctg 60
cccgggctga agcctcccag ctgggacaca aggtgtccca gatggcgcag tactttgagc 120
cgctcaccct ggctgcagtg ggtgctgcct ccaagaccct gagccacccg cagcagatgg 180
cactcctgga ccagactaaa acattggcag agtctgccct gcagttgcta tacactgcca 240

aggaggctgg tggtaaccca aagcaagcag ctcacaccca ggaagccctg gaggaggctg 300
tgcagatgat gaccgaggcc gtagaggacc tgacaacaac cctcaacgag gcagccagtg 360
ctgctggggt cgtgggtggc atggtggact ccatcaccca ggccatcaac cagctagatg 420
aaggaccaat gggtgaacca gaaggttcct tcgtggatta ccaaacaact atggtgcgga 480
cagccaaggc cattgcagtg actgttcagg agatggttac caagtcaaac accagcccag 540
aggagctggg ccctcttgct aaccagctga ccagtgacta tggccgtctg gcctcggagg 600
ccaagcctgc agcggtggct gctgaaaatg aagagatagg ttccatatca aacaccgggt 660
acaggagctg ggccatggct tgtgccgctc tggtcaccaa ngcangcgcc ctgantgcaa 720
gcccagtgat gcctacccaa gaaggagctc atagagtgtg cccggagaag tttttgaaag 780
gtcttccacg tnctggttgg cttcaagctt gggaatcg 818

<210> 510
<211> 651
<212> DNA
<213> Homo Sapiens

<400> 510
gtaggtagaa tcatttttat tggagcatga cctgtttggg gcttataact ctgcagcccc 60
tatgggtagc tgggggtggg ggaagatagt atcaaaaaac ggtgaagaga gctgatgagg 120
ctgtggggac tggctggaag ctgctggcag ggtggagtgg gctggggccc cggcagattc 180
agatcgaggt acagcagcgt taataatact cttggagcgt taatactctg gggaggggca 240
ggcacttggg gggccctagg gcatgaaggc acttggggtt ggggagggga caggggatgt 300
actgcgggac tgggcggggc caggccctgg ggtttggcag gcactttggg gagtgctggg 360
gttgggcagg ttgggccccg acagcccana aggctttggt agtggcacgc acagtctctg 420
ggccgggtct gcattaaata gaagaggctt ctttagtgct catctcgaag ctctgaaggc 480
aanaaacttg tactgctgct gcncggatct gggccanttt cttccgcgcc tcttccanct 540
ctcgttcctt ccgaagcatt tcttcctggc tgccgatgat ctggncaatg ccgccaacca 600
tcttctcttt caccaccact tggctcaatt cttcctggct ctttcaaagg c 651

<210> 511
<211> 712
<212> DNA
<213> Homo Sapiens

<400> 511
gtaggtagaa tcatttttat tggagcatga cctgtttggg gcttataact ctgcagcccc 60
tatgggtagc tgggggtggg ggaagatagt atcaaaaaac ggtgaagaga gctgatgagg 120
ctgtggggac tggctggaag ctgctggcag ggtggagtgg gctggggccc cggcagattc 180
agatcgaggt acagcagccg ttaataatac tcttggagcg ttaatactct ggggaggggc 240
aggcacttgg ggggccctag ggcatgaagg cacttggggt tggggagggg acaggggatg 300
tactgcggga ctgggcgggg ccaggccctg gggtttggca ggcactttgg ggagtgctgg 360
ggttgggcag gttgggcccc gacagcccag aaggctttgg tagtggcacg cacagtctct 420
gggccgggtc tgcattaaat agaagaggct tctttagtgc tcatctcgaa gctctgaagg 480
cagaaacttg tactgctgct gccggatctg ggccagtttc ttccgcgcct cttccagctc 540
tcgttccttc cgaagcattt cttcctgtgc tgccgatgat ctgggcaatg cccggcaacc 600
atcttctctt ttaccaccac tgggctcatt ctcctgctct tcaaaangct gcagcccttt 660
tgggctgntt ttcaccagaa ttaatcttga ngcntcgctt tnacttgcgt tg 712

<210> 512
<211> 850
<212> DNA
<213> Homo Sapiens

<400> 512
aggagctggc ggttttctgt tccccagagc cacctgccaa gacctctacc ccagaagact 60

tcatccgaat gaccaagggt atcaccatgg caaccgccaa ggccgttgct gctggcaatt 120
cctgtcgcca ggaagatgtc attgccacag ccaatctgag ccgccgtgct attgcagata 180
tgcttcgggc ttgcaaggaa gcagcttacc acccagaagt ggcccctgat gtgcggcttc 240
gagccctgca ctatggccgg gagtgtgcca atggctacct ggaactgctg gaccatgtac 300
tgctgaccct gcagaagcca agcccagaac tgaagcagca gttgacagga cattcaaagc 360
gtgtggctgg ttccgtcact gagctcatcc aggctgctga agccatgaag ggaacagaat 420
gggtagaccc agaggacccc acagtcattg ctgagaatga gctcctggga gctgcagccg 480
ccattgaggc tgcagccaaa aagctagagc agctgaagcc ccgggccaaa cccaaggagg 540
cagatgagtc cttgaacttt gaggagcaga tactagaagc tgccaagtcc attgcagcag 600
ccaccagtgc actggtaaag gctgcgtcgg ctgccagaga gaactagtgg cccaagggaa 660
agtgggtgcc attccaagca atgcactgga cgatgggcag tggtcccang ggcctcattt 720
ctgctgcccn gatggtggct tgcggccacc aacaanttgt gtgaaggcag ccaatgcaac 780
tgtccaaggg catgccaagc cnggaanaac tnatnttatt cagcccaaca ggtaacttgc 840
ctttcacaag 850

<210> 513
<211> 727
<212> DNA
<213> Homo Sapiens

<400> 513
gtaggtagaa tcatttttat tggagcatga cctgtttggg gcttataact ctgcagcccc 60
tatgggtagc tgggggtggg ggaagatagt atcaaaaaac ggtgaagaga gctgatgagg 120
ctgtggggac tggctggaag ctgctggcag ggtggagtgg gctggggccc cggcagattc 180
agatcgaggt acagcagcgt taataatact cttggagcgt taatactctg ggagggggca 240
ggcacttggg gggccctagg gcatgaaggc acttggggtt ggggagggga caggggatgt 300
actgcgggac tgggcggggc caggccctgg ggtttggcag gcactttggg gagtgctggg 360
gttgggcagg ttgggccccg acagcccana aggctttggt agtggcacgc acagtctctg 420
ggcccgggtc tgcattaaat agaagaggct tctttagtgc tcatctcgaa gctctgaagg 480
cagaaacttg tactgctgct gccggatctg ggccangttt cttccgngcc tcttccagct 540
tctcgttcct tccgaaagca tttcttnctg tgcttgcnat gaatcntggg caatgcccgn 600
ccaacccatc ttctctttca ccaccactgg tctnatttct cctnngtcnt tcaaaaggct 660
tgcaagcctt ctgggctggc ctttcaccca ganttaattt naagnctcgc tttacttggg 720
tttgcca 727

<210> 514
<211> 877
<212> DNA
<213> Homo Sapiens

<400> 514
cagcagccag cgctgtgtct ggtatcattg ctgacctcga caccaccatc atgttcgcca 60
ctgctggcac gctcaatcgt gagggtactg aaactttcgc tgaccaccgg gagggcatcc 120
tgaagactgc gaaggtgctg gtggaggaca ccaaggtcct ggtgcaaaac gcagctggga 180
gccaggagaa gttggcgcag gctgcccagt cctccgtggc gaccatcacc cgcctcgctg 240
atgtggtcaa gctgggtgca gccagcctgg gagctgagga ccctgagacc caggtggtac 300
taatcaacgc agtgaaagat gtagccaaag ccctgggaga cctcatcagt gcaacgaagg 360
ctgcagctgg caaagttgga gatgaccctg ctgtgtggca gctaaagaac tctgccaagg 420
tgatggtgac caatgtgaca tcattgctta agacagtaaa agccgtggaa gatgaggcca 480
ccaaaggcac tcgggccctg gaggcaacca cagaacacat acggcaggag ctggcggttt 540
tctgttcccc agagccacct gccaagacct ctaccccaga agacttcatc cgaatgacca 600
agggtatcac catggcaacc gccaagccgt tgctgctgca attcctgtcg ccaggaagat 660
gtcattgcca cagccaatct gagcccgccg tgctattgca gatatgcttc ggctttgcaa 720
ggaagcagct taccacccag aagtgggccc tgatgtgcgg nttcaancct gnactatggc 780
ccggagtgtg ccaatggcta cctgggaact ggttggacca ttgtacttgg tgacccttgc 840

aaaagcccag ccccagaact tgaagccagc agtttgc 877

<210> 515
<211> 685
<212> DNA
<213> Homo Sapiens

<400> 515
gtaggtagaa tcatttttat tggagcatga cctgtttggg gcttataact ctgcagcccc 60
tatgggtagc tgggggtggg ggaagatagt atcaaaaaac ggtgaagaga gctgatgagg 120
ctgtggggac tggctggaag ctgctggcag ggtggagtgg gctggggccc cggcagattc 180
agatcgaggt acagcagcgt taataatact cttggagcgt taatactctg gggaggggca 240
ggcacttggg gggccctagg gcatgaaggc acttggggtt ggggagggga caggggatgt 300
actgcgggac tgggcggggc caggccctgg ggtttggcag gcactttggg gagtgctggg 360
gttgggcagg ttgggccccg acagcccaaa aggctttggt agtggcacgc acagtctctg 420
ggccgggtct gcattaaata gaagaggctt ctttagtgct catctcgaaa ctcttgaagg 480
cagaaacttt gtactgctgc ttgccggatc tgggccagtt tcttccgcgc ctcttcagct 540
tntcgttcct ttccgaancc atttctttcc tgngcttgcc natgaatctt gggcaaatgc 600
ccgccaaccc atcttctctt ttcaccccac cacctggnct cattctcctg ctcttcaaaa 660
ngcttgcaac cctttcttgg ngctn 685

<210> 516
<211> 790
<212> DNA
<213> Homo Sapiens

<400> 516
ggttaacata cgaagaaaga atggctcgtc gactgctagg tgctgacagt gcaactgtct 60
ttaatattca ggagccagaa gaggaaacag ctaatcagga atacaaagtc tccagctgtg 120
aacagagact catcagtgaa atagagtaca ggctagaaag gtctcctgtg gatgaatcag 180
gtgatgaagt tcagtatgga gatgtgcctg tggaaaatgg aatggcacca ttctttgaga 240
tgaagctgaa acattacaag atctttgagg gaatgccagt aactttcaca tgtagagtgg 300
ctggaaatcc aaagccaaag atctattggt ttaaagatgg gaagcagatc tctccaaaga 360
gtgatcacta caccattcaa agagatctcg atgggacctg ctccctccat accacagcct 420
ccaccctaga tgatgatggg aattatacaa ttatggctgc aaaccctcag ggccgcatca 480
gttgtactgg acggctaatg gtacaggctg tcaaccaaag aggtcgaagt ccccggtctc 540
cctcaggcca tcctcatgtc agaaggcctc gttctagatc aagggacagt ggagacgaaa 600
atgaaccaat tcaggagcga ttcttcagac ctcacttctt gcaggctcct ggagatctga 660
ctgttcaaga aggaaaactc tgcagaatgg actgcaaagt cagtgggtta ccaaccccca 720
gatctaagct ggcaactaga tggaaagccc gtacgccctg acagtgctca caagaaagcc 780
tggtgcctga 790

<210> 517
<211> 747
<212> DNA
<213> Homo Sapiens

<400> 517
atagtcaaag gtatgtttct gccttttaca tantgtgaca aaggaatatg ttggtcaagg 60
caatggctgt ttcagtgttt cagctttaac aagaatgctg gattacaggt cctcactttc 120
taccaaggca gtattcagtg tcaggtgaga tgggttggcc tcaggttgga acgctgcttt 180
gatgtctagt ccctggtccg aaagtgctgc atagcgactg gctgagggcc gtactttttt 240
tggcttggtg ctctgtgact gctgatgcca ctgggtgtaa acgtccagcc tggcagtaca 300
ggacacaatc cctgcttcat tcttggctga cacagtatac cacccagcat cttcttttgt 360
ggctccctga atgagcaggc agatgtagcc gtggttgtcc tggtgcatgc tcactcggtc 420

agtgctgtga gtgagtgatt cattttcttt cttccaaaat atctgaggtg gtggcactcc 480
caatacacga cattccagcc gcactgggta cccatcagca actcctgtgt tttggagctt 540
ctcaataaac acagggggtt tgtgtgcttc tttagcagca accacaagct ccaggctgaa 600
tgagttctgt cctgctcggt tggtagctat acatgtgtag atgccggcat cacgtgacgt 660
gactggctct atgatcagag agtgcacccc gttctttacg caccagcatc ttgggagccc 720
tgtcaaggcg taccggcttt ccatcta 747

<210> 518
<211> 926
<212> DNA
<213> Homo Sapiens

<400> 518
agaaagcaga gccttctgaa gttgacatga attctcctaa atccaaaaag gcaaaaaaga 60
aagaggagcc atctcaaaat gacatttctc ctaaaaccaa aagtttgaga aagaaaaagg 120
agcccattga aaagaaagtg gtttcttcta aaaccaaaaa agtgacaaaa aatgaggagc 180
cttctgagga agaaatagat gctcctaagc ccaagaagat gaagaaagaa aaggaaatga 240
atggagaaac tagagagaaa agccccaaac tgaagaatgg atttcctcat cctgaaccgg 300
actgtaaccc cagtgaagct gccagtgaag aaagtaacag tgagatagag caggaaatac 360
ctgtggaaca aaaagaaggc gctttctcta attttcccat atctgaagaa actattaaac 420
ttctcaaagg ccgaggagtg accttcctat ttcctataca agcaaagaca ttccatcatg 480
tttacagcgg gaaggactta attgcacagg cacggacagg aactgggaag acattctcct 540
ttgccatccc tttgattgag aaacttcatg gggaactgca agacaggaag agaggccgtg 600
cccctcaggt actggttctt gcacctacaa gagagttggc aaatcaagta agcaaagact 660
tcagtgacat cacaaaaaaa gcttgtcagt gggcttggtt tttatggtgg aacttcctat 720
ggaggtcaat ttggaccgca tggangnaat gggaattgga taatcctggg ttggaacacc 780
angtcgtatc aaaggaccnc antacnggaa tgggcaaact aagatcttca cccaaacttt 840
aagccatgtt ggcccttggg atgaaagtgg ggncccagan tgtttgggaa atngggaatt 900
tgcttgatca aagtggggaa gaagaa 926

<210> 519
<211> 789
<212> DNA
<213> Homo Sapiens

<400> 519
acatactctt gagcaatgct aatctgcgcc ccttactccc ttaagtcctt cttggtaaat 60
aatgttaatc ttccaatagg aagaagtgga gtacattacc atttaagcac catttatcca 120
gcctacttac aaataaagct atggagccac cttatacatg tgaaattcct taaaaccctg 180
gctttctatt aaaatgtact tttatatata ctatctatga agaattcact aaagcatgaa 240
tcaccttata atgagaagct aaaaatgtat caaaacgaac ataagtatag gtaatccaca 300
tcaaacatac tacatcttcc aagtctagag catacactgg tataaactgt attacaaccc 360
agattagttt gaaatcttgt ttcaaaacat tgctcagtat taagtctcag tagacaaata 420
ataggaccac atgagaaact gttcggcagg tggctgagga aaccttaact tccaaaggct 480
caaagtggtc ctccagagac tgttacactc ccttaggtat ttatttcagg gaaggacact 540
attaagggac acttttgagt ataaagacag gtgaactcac aaagtatagg cagatcatgc 600
ttgattttat cttctaatct acaggataat acattagaat aaaaatgtaa tgaattcata 660
cacctttcaa aanggaaaaa ctggatgaag taacnnntaa agntataaat ggataatgga 720
tccggatgaa aataaatttt aaaatggaaa ccttggctgn gtctgaaaga agaccgggac 780
tttggcaag 789

<210> 520
<211> 827
<212> DNA
<213> Homo Sapiens

<400> 520
gtgatatagt gcttgtcatt ttaattgtaa catattacca aaaagcttta tatacatagc 60
tttatactat ttacattgca gtagaggaat ggcaatgcta acaggtgatc agtgcttcca 120
aactttttca atacctacac atgggagatc taaagagtac aatatattta agacttctaa 180
ggaattgttt tctcctcact aataaagcat gccctgacta aagagaagtc ctgtaggcac 240
agccttatct attcaatgac tggcacctcc caggggtact gacacacaaa gtgccttcac 300
tggaccttac agttctcact gcccttggac tccagtccag ctttggggct ggggacaagt 360
cggcctcgct tgaccctcag gccctctctg gggctgtcag tcggacttct ctcaggaaga 420
ttattgactg ggacggattt cgtggtgggt tctcggagga tggtgcctga atctactggg 480
ctccgctgag caactttgac cttttgtgat ctgctgccac cagctgttgg tttggaggac 540
tctgcaagat tttctttgcc gagactcagt ggggatagcg ctaacttctg tgcagccagg 600
cgggggctgg tccgagttgc catggttggt cttcgcagga tatatgggct aagtctttnc 660
tgtcgggatg tcagcaaacc ctttctttac aacttctgga agtccctctg gctcaaactt 720
agtaccttcg ngncttctgc anggtgaata ccactcatga ctgntttctt gcttttttta 780
gaaaagctct ctggggtaac aggtgtgggn ccttcaccat tcttccc 827

<210> 521
<211> 710
<212> DNA
<213> Homo Sapiens

<400> 521
gtgatatagt gcttgtcatt ttaattgtaa catattacca aaaagcttta tatacatagc 60
tttatactat ttacattgca gtagaggaat ggcaatgcta acaggtgatc agtgcttcca 120
aactttttca atacctacac atgggagatc taaagagtac aatatattta agacttctaa 180
ggaattgttt tctcctcact aataaagcat gccctgacta aagagaagtc ctgtaggcac 240
agccttatct attcaatgac tggcacctcc caggggtact gacacacaaa gtgccttcac 300
tggaccttac agttctcact gcccttggac tccagtccag ctttggggct ggggacaagt 360
cggcctcgct tgaccctcag gccctctctg gggctgtcag tcggacttct ctcaggaaga 420
ttattgactg ggacggattt cgtggtgggt tctcggagga tggtgcctga atctactggg 480
ctccgctgag caactttgac cttttgtgat ctgctgccac cagctgttgg tttggaggac 540
tctgcaagat tttctttgcc gagactcagt ggggatagcg ctaacttctg tgcagccagg 600
cgggggctgg tccgaagttg ccatgggttg ntcttccagg atatatgggc taagnctttc 660
ctgtcgggat gtcagcaaaa ccctttcttt acaacttctg gaaagcccct 710

<210> 522
<211> 638
<212> DNA
<213> Homo Sapiens

<400> 522
atagngcttg tcattttaat tgtaacatat taccaaaaag ctttatatac atagctttat 60
actatttaca ttgcagtaga ggaatggcaa tgctaacagg tgatcagtgc ttccaaactt 120
tttcaatacc tacacatggg agatctaaag agtacaatat atttaagact tctaaggaat 180
tgttttctcc tcactaataa agcatgccct gactaaagag aagtcctgta ggcacagcct 240
tatctattca atgactggca cctcccaggg gtactgacac acaaagngcc ttcactggac 300
cttacagttc tcactgccct tggactccag tccagctttg gggctgggga caagtcggcc 360
tcgcttgacc ctnaggccct ctctggggct gtcagtcgga cttctntcag gaagattatt 420
gactgggacg gatttcgtgg tgggttctcg gaggatggtg cctgaatcta ctgggctccg 480
ctgagcaact ttgacctttt gngatctgct gccaccagct gttggtttgg aggactntgc 540
aagatttctt ttgccgagac ttantggggg atagcgctaa cttctggngc agccangcgg 600
gggctggtcc naanttgcca tggntgntct tcncagga 638

<210> 523
<211> 833

<212> DNA
<213> Homo Sapiens

<400> 523
cgacacttag accgagtgga ctccatcctg ctcacccaca ttggggatga caatttgcct 60
ggaataaaca gcatgttaca gcggaaaatt gcagagctcg aggaagaaca gtcccagggc 120
tccaccacaa atagtgactg gatgaaaaac ctcatctccc ctgacttagg agttgtattt 180
ctcaatgtac ctgaaaatct caaaaatcca gagccaaaca tcaagatgaa gagaagcata 240
gaagaagcct gcttcactct ccagtaccta aacaaattgt ccatgaaacc agaacctctg 300
tttagaagtg taggcaatac tattgatcct gtcattcttt tccaaaaaat gggagtaggt 360
aaacttgaga tgtatgtgct taatccagtc aagagcagca aggaaatgca gtattttatg 420
cagcagtgga ctggtaccaa caaagacaag gctgaattca ttctgcctaa tggtcaagaa 480
gtagatctcc cgatttccta cttaacttca gtctcatctt tgattgtgtg gcatccagca 540
aaccctgcgg agaaaatcat ccgagtcctg tttcctggga acagcaccca gtacaacatc 600
ctggaagggt tggaaaagct caaacatcta gactttctga agcagccact ggccacccaa 660
aaggatctca ctggccaggt gcccactcct gtggtgaaac aaacaaaact gaacagaggg 720
cttgatagcc gagaaagtct gaagcccagc cgcaaaanca ctttctagca aaatccggcg 780
ccaaggagtc aaaagaagaa acccctgagg tcacaaaagg tggaatcacg tgg 833

<210> 524
<211> 766
<212> DNA
<213> Homo Sapiens

<400> 524
cacttccttc ttctcttcct tcttaacttc cttcttgact tcctttggcg gtgtttcttt 60
cttaacctct ttcttgggtt cttttttctc ttctttcttg atctcttttt tgacttcttt 120
tttcacctct tccttttttg gtttttcctc cttcttgata ggtgttttgt cctccttttt 180
agccacttct ttctttggct tttctttctc ctctttcttg tcttcaggct ttacctttgt 240
ttcctttttc accgtcttct ccttggcagc tttgggtttg acatctgtgg cttgcttctc 300
agccacctcg gctttcactg gagatggctc ttctttgctg ggaacctcct tttcagtcac 360
tgaaggtttg gtctctgttt ttattggctt gtcttttttc accattacct tttctttgct 420
ttcaactttg ggtggctttt ccacgtgatt cacttttgtg acctcagggg tttcttcttt 480
tgactccttg cgcacggatt tgctaggaag tggttttgcg gctggcttca gactttctcg 540
gctatcagcc ctctgtttca gttttgtttg tttcaccaca ggagtgggca cctggccagt 600
gaganccttt tgggtggcca gtggctggtt cagaaagtct aaanggtttg aggcttttnc 660
aacctttcag gaatggtgga ccggggtgct ggtcccagga aacaggactc ggatggattt 720
ttctccccaa gggtttgctg gaagccccca caaatcaaag gaagga 766

<210> 525
<211> 847
<212> DNA
<213> Homo Sapiens

<400> 525
cagcagccgg caggatggcg accgtggtgg tggaagccac cgagccggag ccgtccggca 60
gcatcgccaa cccggcggcg tccacctcgc ctagcctgtc gcaccgcttc cttgacagca 120
agttctactt gctggtggtc gtcggcgaga tcgtgaccga ggagcacctg cggcgtgcca 180
tcggcaacat cgagctcgga atccgatcat gggacacaaa cctgattgaa tgcaacttgg 240
accaagaact caaacttttt gtatctcgac actctgcaag attctctcct gaagtcccag 300
gacaaaagat ccttcatcac cgaagtgacg ttttagaaac agtggtcctg atcaaccctt 360
ctgatgaagc agtcagcacc gaggtgcgct taatgatcac tgatgctgcc cgacacaagc 420
tgctcgtgct gaccgggcag tgctttgaaa ataccggaga gctcattctc cagtccggct 480
ctttctcctt ccagaacttc atagagattt tcaccgatca agagatcggg gagttactaa 540
gcaccaccca tcctgccaac aaagccagct taaccctgtt ctgtcctgaa gaaggggact 600

ggaagaactc caatcttgac agacacaatc tccaagactt catcaatatt aaactcaatt 660
cagcttctat cttgccagaa atggaaggac tttctgagtt taccgagtat ctctcagaat 720
caagtggaag tcccatctcc ttttgacatc ttgggaacct tccacatcgg gtggatttct 780
gaagcttttc caagccctgt ggtataattt ttccaggang gaagggccaa ttttgccttg 840
gttgcaa 847

<210> 526
<211> 746
<212> DNA
<213> Homo Sapiens

<400> 526
cttgatctct tttttgactt cttttttcac ctcttccttt tttggttttt cctccttctt 60
gataggtgtt ttgtcctcct ttttagccac ttctttcttt ggcttttctt tctcctcttt 120
cttgtcttca ggctttacct ttgtttcctt tttcaccgtc ttctccttgg cagctttggg 180
tttgacatct gtggcttgct tctcagccac ctcggctttc actggagatg gctcttcttt 240
gctgggaacc tccttttcag tcactgaagg tttggtctct gttttattgg cttgtctttt 300
ttcaccatta ccttttcttt gctttcaact ttgggtggct tttccacgtg attcactttt 360
gtgacctcag gggtttcttc ttttgactcc ttgcgcacgg atttgctagg aagtggtttt 420
gcggctggct tcagactttc tcggctatca gccctctgtt tcagttttgt ttgtttcacc 480
acaggagtgg gcacctggcc agtgagatcc ttttgggtgg ccagtggctg cttcagaaag 540
tctagatgtt tgagcttttc caacccttcc aggatgttgt actgggtgct gttcccagga 600
aacaggactc ggatgatttt ctcccgcagg gtttgctgga agccacacaa tcaaagatga 660
gaactgaaag taaagtangg aaatcgggaa gaactacttc ttggaccatt taggcagaaa 720
ggaattcagc ccttggcttt ggtggg 746

<210> 527
<211> 837
<212> DNA
<213> Homo Sapiens

<400> 527
cacttccttc ttctcttcct tcttaacttc cttcttgact tcctttggcg gtgtttcttt 60
cttaacctct ttcttgggtt cttttttctc ttctttcttg atctcttttt tgacttcttt 120
tttcacctct tccttttttg gtttttcctc cttcttgata ggtgttttgt cctccttttt 180
agccacttct ttctttggct tttctttctc ctctttcttg tcttcaggct ttacctttgt 240
ttcctttttc accgtcttct ccttggcagc tttgggtttg acatctgtgg cttgcttctc 300
agccacctcg gctttcactg gagatggctc ttctttgctg ggaacctcct tttcagtcac 360
tgaaggtttg gtctctgttt ttattggctt gtcttttttc accattacct tttctttgct 420
ttcaactttg ggtggctttt ccacgtgatt cacttttgtg acctcagggg tttcttcttt 480
tgactccttg cgcacggatt tgctaggaag tggttttgcg gctggcttca gactttctcg 540
gctatcagcc ctctgtttca agttttgttt gnttcaccac aggagtgggc acctggccag 600
tgagaccttt tgggtggcca agtggctgct tcagaaaagt ctagaaggtt tgagcctttt 660
ccaacccttc caggaaggtt gggacctggg tgctggttcc canggaaacc aggacctcgg 720
gatgaatttt ctcccgcaag ggtttgcctg gaatgccccn acaatccaaa gaatgaaanc 780
tgaaagttta antagggaaa atccgggaga aactaccttc ntggaccatt naggccc 837

<210> 528
<211> 822
<212> DNA
<213> Homo Sapiens

<400> 528
ctcgggacgt gaaattgaca gtgaaaagta tggcagatga gcaagaaatc atgtgcaaat 60
tggaaagcat taaagagatc aggaacaaga ccctgcagat ggagaagatc aaggctcgtt 120

tgaaggctga gtttgaggca cttgagtcag aggaaaggca cctgaaggaa tacaagcagg 180
agatggacct tctgctacag gagaagatgg cccatgtgga ggaactccga ctgatccacg 240
ctgacatcaa tgtgatggaa aacactatca aacaatctga gaatgaccta aacaagctgc 300
tagagtctac aaggaggctg catgatgagt ataagccact gaaagaacat gtggatgccc 360
tgcgcatgac tctgggcctg cagaggctcc ctgacttgtg tgaagaagag gagaagcttt 420
ccttggatta ctttgagaag cagaaagcag aatggcagac agaacctcag gagcccccca 480
tccctgagtc cctggccgct gcagcccgct gccgcccaac agctccaagt ggctaggaag 540
caggatactc ggcagacggc caccttcagg cagcagcccc cacctatgaa ggcctgcttg 600
tcatgtcacc agcaaattca ccggaatgca cctatatgcc ctctttgcaa ggccaagagt 660
cggtcccgga accccaaaaa gccgaacgga agcaggatga ataaaggaaa gggagagccc 720
atgaagcttt gctaattata accccttcac cttgaccaga gtcattgatg tcctgatgtg 780
aaacaaccct tggcccaacc ccacgaagtc tcctatttaa tg 822

<210> 529
<211> 842
<212> DNA
<213> Homo Sapiens

<400> 529
actttcaaag agcagaggaa cattttatat agtgaacaca tacacacttg gcaatgtaaa 60
actacttaag gaaggaaaaa tatccccctc cccagccagg tactgagacc tggggctaaa 120
attttttgtc agtcagcccc catccccatc ccttatcttc gagtgacctt accaggaaac 180
ctggctttgg tggaaaggag agctgtgggg cttggggagc ctgatgcctt ttcttttggg 240
aggaaaggca cctgcacaat ccacaggaca ggagtggcca gcagctatcc tgagctgagg 300
ctccagaaga gttcagatcc aagagagcaa gggatgaatg gaaggaaagt cccacccacc 360
ttcatgtgta aagtgattgg catttactca aatctaaatc tactcctctc ctccctgcaa 420
tataccattg agcatgtgcc agagtaatgg ttctgaacaa aagccaacac agatgtcagc 480
ctgggggcac tctcagccaa ggaagcccct acagccgagc cctcagccct aatgacttag 540
gcagtaggtt aggcaggaga tgtagaagtt ggtctggctc actgatttca ctgtggaaat 600
cttctactag aatttgcaaa gactagatat tggggaaagg ttcattgatc ttaagaatcc 660
caagacacac agcctagtac ctaagaattt taagtatatg tggggagaca gaagtgggag 720
aaagctaaag aattaccggc catgccttcc aaatgattat gaaaanggag ggcttggtcc 780
aagcttacct ttgggccttt aaggatgaan atgangggta ggaagtangg gggatacatg 840
cc 842

<210> 530
<211> 815
<212> DNA
<213> Homo Sapiens

<400> 530
ggaaaaggga gaaagatagg gagaaatatt cccaaagaga acaagaaaga gatagacaac 60
aaaatgatca gaaccgaccc agtgagaaag gagagaagga agagaaaagc aaagcaaagg 120
aagagcatat gaaagtaagg aaggaaagat atgaaaataa tgataaatac agagatagag 180
aaaaacgaga ggtaggtgtt cagtcttcag aaagaaatca agacagaaag gaaagcagcc 240
caaattctag ggcaaaggat aaatttcttg accaagaaag atccaacaaa atgagaaaca 300
tggcaaagga caaagaaaga aaccaagaga aaccctctaa ttctgaatca tcactgggag 360
caaaacacag actcacagag gaagggcaag agaagggtaa agaacaagag agaccacctg 420
aggcagtgag caagtttgca aagcggaaca atgaagaaac tgtaatgtca gctagagaca 480
ggtacttggc caggcagatg gcgcgggtta atgcaaagac ctatattgag aaagaagatg 540
attgatggct accccaagag aaagatttaa ggaagcacag aaaactgtaa ttcctggaac 600
ctgctgcgta aaaccataaa ggagtgtgtt acccagtagt ttggagggca tttttaaatt 660
tattttcaaa attttaagtt aaaagtcagt cttaagcttg gatgttttgg aatgtggatg 720
tttggctgaa tttatatata gggngtactc atcaataccn cattctttgt gganttcaag 780
aacccgttaa gagtgtgctt aattccctga ngtac 815

<210> 531
<211> 857
<212> DNA
<213> Homo Sapiens

<400> 531
aaaatgtata agcatatcat tttattttca tttaagccaa ctatgctgta agctatttag 60
acaagatgat tcacatttta tacttaaata caaatttcag aacataaagt atattttctg 120
tttttcaaat ccatatttta tctgaaatac atttcctgca acaaaacatt attagaagag 180
ttaaattatt tatttaaaaa aaatttttta gagacagggt ctcattctgt tgcccaggtt 240
ggagtgcagt ggcatgatca tacctcactg taacatcaaa ttcctaggct caagtgatct 300
tcttgcctca gcctcttgaa cagctgggac tacaggcatg gactaccatg ctaggctttt 360
tgttttttaa atagagacaa ggtcttatta tcctgcctag gctggtcttg aatgcctagc 420
ctcaatatcc ttctgccttg gcctcccaaa atgttggtat tacaggcacg agctaccgta 480
tctggccaaa attatttttt aatggttgta gtggagcaaa ttttcctcat tatgtaccta 540
cagggaatta gcacactctt aacggttctt gaatcaacaa agaatgtggt attgatgagt 600
acacactata tataaattca gccaaacatc cacatccaaa catccaagct gtaagactga 660
cttttaactt aaaattttga aaataaattt aaaaagccct tcaaactact ggtaacacac 720
ttcnttatgg tttaccccac aggntncagg aattccagtt tctgggcttn ccttaaacct 780
ttccttgggg tagcccatca atcatctctt tctcaaaaaa aggcntttgc attaacccgg 840
gccatttggc ctggcca 857

<210> 532
<211> 736
<212> DNA
<213> Homo Sapiens

<400> 532
cctggatgct gtgctgattg aggatgagct ggaggaactc caccgctact gccaggaggt 60
gtttggaagg gtctcccggt tccaccggcg gctcacctcc tgcactccgg gcttggaaga 120
tgaaaaggag gcctctgaga atgaaacaga catggaagac cccagagaaa tccagactga 180
ttcttggcgt aaacggggag agagcgagga accgtcatct cctcagtccc tgtgtcatct 240
agtggcccca gggcacgagc ggtctggctg cgagacccct gtcagcgtgg actccatccc 300
cctggagtgg gaccacacag gcgacgtggg gggctcctcc tctcacgaag aggacgagga 360
gggcccatac tacagcgcac tgtcagatgt agaaatccct gaaaatcctg aggcatatct 420
taaaatgacc acaaaaactt tgaaagcgtc ttctggtaaa tccatttcgg atggccactc 480
gtggcatgtt cccgacagcc cttcctgtcc cgagcatcac tacaagcaaa tggaaggtga 540
caggaatgtt ccacctgttc cccctgcgtc cagcacccct tataaaccac cctatggaaa 600
gctactatta cctccaggca cggatggtgg caaagaaagc ccgcgagtcc tgaatggcaa 660
cccacagcag gaagacnggg gactggcccg gtattacaga gcaacagtca gggtgccttc 720
gacagatggg agatga 736

<210> 533
<211> 678
<212> DNA
<213> Homo Sapiens

<400> 533
ctggctaatt ttgtttttta atganaaaca tntgagttgt ncatatcaca aacagnttca 60
agtttntgnn ccaacccccc gcccccaccc ccgccgnggc caaacagtta aaacccaaag 120
caaagcatca ntttggatgt gaaaaagtnt taaaaaatta acttacaaaa ncatccctat 180
caagtcggta gttnggcatt tactttacat tagtcaaaag ctccagctaa aatctaattt 240
ttttaaaaaa aaatcgaagt ttacattatt catacanatt gggcattgtt aaaaaatatg 300
cncaaataac cacatccatg caatacaatt tntttaaaaa tttaaagcan tntaaaagag 360
cagagctagg tnctgaacan aacattttgg ngtataaccg gcagntcaaa attgccagct 420

gattggagta aaactgattn taagcgtatt aaatatgatn gatngtttcc atcagctaag 480
ggngcctatg agtttctgaa ccatttntag ggnggaatgt cctcgcttgc ttcnataata 540
tatgtgatgg acaccactgc tcattgncca tacctacatt ataataatgc tgttttacaa 600
acaaaccaga attcacaaag ngcttggctn ttcaggaaac tgacatttcc agagatccct 660
aaactaaatc aactagtt 678

<210> 534
<211> 789
<212> DNA
<213> Homo Sapiens

<400> 534
ggtggatgag ggtgctgggg acagtgctgc ggtggccagt ggtggtgccc agaccttggc 60
ccttgccggg tcccctgccc catcggggca ccccaaggct ggacacagtg agaacggggt 120
tgaggaggac acagaaggtc gaacggggcc caaagaaggt acccctggga gcccatcgga 180
gaccccaggc cccagcccag caggacctgc aggggacgag ccagccgaga gcccatcgga 240
gaccccaggc ccccgcccag caggacctgc aggggacgag ccagccgaga gcccatcgga 300
gaccccaggc ccccgcccgg caggacctgc aggggacgag ccagccgaga gcccatcgga 360
gaccccaggc cccagcccgg caggacctac aagggatgag ccagccgaga gcccatcgga 420
gaccccaggc ccccgcccgg caggacctgc aggggacgag ccagccgaga gcccatcgga 480
gaccccaggc ccccgcccgg caggacctgc aggggacgag ccagccgaga gcccatcgga 540
gaccccaggc cccagcccgg caggacctac aagggatgag ccagccaagg cgggggaggc 600
agcagagttg caggacgcag aggtggagtc ttctgccaag ttctgggaag ccnttaagga 660
aaggagttgc ccgtcggcgt cttggtcctc tggtccttgt tgaagggctt gggncttccg 720
gacttnttgn ggcttccctt aaggtttggt ttgtgaccct gaccatggan ccacaatgct 780
gggcttctt 789

<210> 535
<211> 802
<212> DNA
<213> Homo Sapiens

<400> 535
caaagtcaaa tgaatttatt cagaaaaggc cttgcttggt atcagactaa gaaaagcagc 60
cctgcccgcc gccccccact ccanaagggt caatttacaa agacaggggc gcaggggana 120
gctgggtggg gaagacacag ccaggccagg aggcttctgc aggccttggg cttccctgag 180
ggcctcgcgg cttctggtgg ctgctatagt ggccccacag gaggccagca ctgtgggtca 240
tgggtcacgg gtcacgaagc anagcctgag gggagcccgc agcagctccg gaggccccag 300
cccctgcagc agggacagga ggaccaagac gccgacgggc actcctttcc ttaaggcttc 360
ccanacttgg cagaagactc cacctctgcg tcctgcaact ctgctgcctc ccccgccttg 420
gctggctcat cccttgtagg tcctgccggg ctggggcctg gggtctccga tgggctctcg 480
gctggctcgt cccctgcagg tcctgccggg cgggggcctg gggtntccga tgggctctcg 540
gctggctcgt cccctgcagg tcctgccggg cgggggcctg gggtctccga tgggctctcg 600
gctggctcaa tcccttgtag gtccttgccg ggctggggcc tgggggtctt ccgaatgggc 660
ttctcggctg gcttcgtccc ttgcaagtcc ttgccgggcc gggggccctg ggggtcttcn 720
aatgggcttt ttgggttggg ttcggccccc tggaaggtcc ctggctgggc cggggggccc 780
tgggggtctt ccnaaagggg ct 802

<210> 536
<211> 901
<212> DNA
<213> Homo Sapiens

<400> 536
aaaagaatgg aaaagaaaat acagagagaa cgagaaatgg aaaaggggga gtttgatgat 60

aaagaagcat ttgtgacatc tgcatataag aaaaaactgc aagagagagc tgaagaagaa 120
gaaagagaaa agagggctgc tgcactggaa gcatgtttgg atgtaaccaa gcagaaagat 180
ctcagtggat tttataggca cctattaaat caagcagttg gtgaagagga agtacctaaa 240
tgcagctttc gtgaagccag atctggtata aaggaagaaa aatcaagggg cttctccaat 300
gaagtaagtt caaaaaacag aataccacaa gagaaatgca ttcttcaaac tgatgtgaaa 360
gtagaggaaa acccagatgc agacagtgac ttcgatgcta agagcagtgc ggatgatgaa 420
atagaagaaa ctagagtgaa ctgcagaagg gaaaaggtca tagagacccc tgagaatgac 480
ttcaagcacc acaggagtca aaaccactct cggtcaccta gtgaagaaag agggcacagt 540
accaggcacc acacgaaagg atcacgaacg tcgagaggac atgagaaaag ggaagatcag 600
caccaacaga agcaatccag agaccaaaga gaaccattac actgacccgt gantaccgga 660
aagaaaggga ttctcatagc acagagaggc cagtcattag agattcccat tggaagagcc 720
ttgaacagga agataaaccc anggccaagg gnccaaggag gaaagaagtg acngaagtnt 780
ggnaaaaggg agaaaggatt gggagaaata nttcccaagg aggaccagga aggagattgc 840
ccaccaaatn gatccgaaac cgacccaatg agaaaggaga gaaggaagag aaaagccaag 900
c 901

<210> 537
<211> 761
<212> DNA
<213> Homo Sapiens

<400> 537
   atgtataagc atatcatttt attttcattt aagccaacta tgctgtaagc tatttagaca 60
   agatgattca cattttatac ttaaatacaa atttcagaac ataaagtata ttttctgttt 120
   ttcaaatcca tattttatct gaaatacatt tcctgcaaca aaacattatt agaagagtta 180
   aattatttat ttaaaaaaaa ttttttagag acagggtctc attctgttgc ccaggttgga 240
   gtgcagtggc atgatcatac ctcactgtaa catcaaattc ctaggctcaa gtgatcttct 300
   tgcctcagcc tnttgaacag ctgggactac aggcatggac taccatgcta ggctttttgt 360
   tttttaaata gagacaaggt cttattatcc tgcctaggct ggtcttgaat gcctagcctc 420
   aatatccttc tgccttggcc tcccaaaatg ttggtattac aggcacgagc taccggatct 480
   ggccaaaatt attttttaat ggttgtagtg gagcaaattt tcctcattat gtacctacag 540
   ggaattaagc cactcttaac ggttcttgaa tcnncaaaga atgtggnatt gatgagttcn 600
   cactatatat aaattcagcc caaacatcca cattcnaaca tnccagctgt aagactgact 660
   tttaacttaa aattttgaaa natnaaattt aaaaatgccc tncaaaacta ctgggaacac 720
   cctcccttta tgggtttanc ccagcagggt tccaaggaat t 761

<210> 538
<211> 869
<212> DNA
<213> Homo Sapiens

<400> 538
cgggaacaag atggcagccc ccatacctca agggttctct tgtttatcga ggtttttggg 60
ctggtggttt cggcagccag ttctggtgac tcagtccgca gctatagttc cagtaagaac 120
taaaaaacgt ttcacacctc ctatttatca acctaaattt aaaacagaaa aggagtttat 180
gcaacatgcc cggaaagcag gattggttat tcctccagaa aaatcggacc gttccataca 240
tctggcctgt acagctggta tatttgatgc ctatgttcct cctgagggtg atgcacgcat 300
atcatctctt tcaaaggagg gactgataga gagaactgaa cgaatgaaga agactatggc 360
atcacaagtg tcaatccgga ggataaaaga ctatgatgcc aactttaaaa taaaggactt 420
ccctgaaaaa gctaaggata tctttattga agctcacctt tgtctaaata actcagacca 480
tgaccgactt cataccttgg taactgaaca ctgttttcca gacatgactt gggacatcaa 540
atataagacc gtccgctgga gctttgtgga atctttagag ccctctcatg ttgttcaagt 600
tcgctgttca agtatgatga accagggcaa cgtgtaccgn ccagatcacc gtaccgcatg 660
cacacccggc agactctggc catctatgac cgggtttggc ccggttgatg tatgggccag 720
gnagatgtcc ccaggatgtc ctggaagtat gttggantcg aaaagcagnt tgccaaancc 780

ctatggaagc tggagaagcn tacccaagac ggtnccccct gggcaccccc ttaagcaggc 840
catcctttaa aacggggatg atcccttgg 869

<210> 539
<211> 760
<212> DNA
<213> Homo Sapiens

<400> 539
aagggataaa ttatttcttt ggatttatat ttttccataa aatgcaaatg ctgattcatc 60
agtgagtcag tatatgaaaa agggcctctt aaatgtctta taaacactaa ttattcttcc 120
ccagtcttca tttccttaaa gtcacatcgc tcacaagtag gctcatcttc cacttctgcc 180
atctgaaggc tggtccatgc ccagcctgaa ccaggggaaa tgtgcagaac tcaccaaaat 240
ttttccaaca ccctgacaac atttcatttc aaactctgat ccctgccctg tgattacaaa 300
gaggatgctg ctggttgtct ctcacagtcc ctgctgtggg aaaaactgat atccaatgtt 360
ctctgaaaca tactgtcttt catctagact cagaagctag acataaaatt taaaaaagaa 420
gagtgtccat ggccatgtta tacctgccac ctgctagggc ccagtcatca gtcatggttg 480
ctgatgatga gactgctgaa aagacctgag caggatggga gagaacaaag gtagttcttt 540
ttatagcatg aggggaatgg gagacttcaa agcttncagg cagcctcatc accccaggct 600
tcaccctaga aagtcatttt tgncatcagg gctaacctga ngcttctggg gcctctcctt 660
gggcctcttc ataatcttct tctgggnttc agcttgaagg gccaggggat tcatnacccg 720
gctttaaagg gatggggcct gcttaagggg ggtgccccat 760

<210> 540
<211> 874
<212> DNA
<213> Homo Sapiens

<400> 540
ggagcactgc ctcaaacatg ggctgaaagt taagaagagt tttattggcc aaaataaatc 60
attctttggt cctttggagc tggtggagaa actttgtcca gaagcatcag atatagcgac 120
tagtgtcaga aatcttccag aattaaagac agctgtggga agaggccgag cgtggcttta 180
tcttgcactc atgcaaaaga aactggcaga ttatctgaaa gtgcttatag acaataaaca 240
tctcttaagc gagttctatg agcctgaggc tttaatgatg gaggaagaag ggatggtgat 300
tgttggtctg ctggtgggac tcaatgttct cgatgccaat ctctgcttga aaggagaaga 360
cttggattct caggttggag taatagattt ttccctctac cttaaggatg tgcaggatct 420
tgatggtggc aaggagcatg aaagaattac tgatgtcctt gatcaaaaaa attatgtgga 480
agaacttaac cggcacttga gctgcacagt tggggatctt caaaccaaga tagatggctt 540
ggaaaagact aactcaaagc ttcaagaaga gctttcagct gcaacagacc gaatttgctc 600
acttcaagaa gaacagcagc agttaaagag aacnaaatga attaattcga gaaagaagtn 660
aaaagagtgt agaagatacn aaacaggatc caaagttgag ctggagactt acagccaact 720
tcgcaaggtc tggatgaaat gtcnntgatg tgtggaagca cttaaagagg agaagaaagt 780
ccggttggaa ctggaaaaaa gaactggagn tccaaatggg aatgaaaacc caaatnggaa 840
atgccatgaa gttcctggna aaggcccccc ccaa 874

<210> 541
<211> 729
<212> DNA
<213> Homo Sapiens

<400> 541
gaaaaataaa tgattttatt gcagggccaa tgataggtag tcacaagggc atgaaatggc 60
agatctcttg tctgaagcag agaaggcaca ctggcagact ccatgtgtgt caaacgctgt 120
gcatgaatca ggtttttaga aggaaggtag gagaggaaaa ctactcacta gcagaactga 180
actgctgtaa aataggttaa attctttgaa aagtgaaaaa tgatagtagc aaaatcatga 240

agttgtatct gaaccagagc cgtgatgtaa ccaagtaaga tggaagtttc catccagagg 300
agttaattcc gaacaagtca cagaaaggtg agagctgccg gttccggcac gctgtcttct 360
ggagtgccag tgaccgggca agaaatttga ttctttcctt tgattctctt gggaaagaac 420
acatttccca agcccctgga gacccacagg gtttggcact gtccgtgagg ctgtgctcct 480
gaggacggac gttcaggagg ccgtggagga gcagcgctgc aggagcaggg tgtggcagct 540
gtcgcacact cgcaccggct tggggtagga gggcagggcc cagctcgttg ctgggagcag 600
gtgtttgcan aagatgtggc ccacagttcc ggcagtnggt gctttctccg gggaaaatgg 660
agaacttcct ttntcacacn tggctaccag tggggtcgnt ttcggcatct tttcaagcca 720
ggccgtggg 729

<210> 542
<211> 830
<212> DNA
<213> Homo Sapiens

<400> 542
gggacagcgg ggacggcacg gcgcgcgcag cttctaagtg ccagatgatg gaggagcgtg 60
ccaacctgat gcacatgatg aaactcagca tcaaggtgtt gctccagtcg gctctgagcc 120
tgggccgcag cctggatgcg gaccatgccc ccttgcagca gttctttgta gtgatggagc 180
actgcctcaa acatgggctg aaagttaaga agagttttat tggccaaaat aaatcattct 240
ttggtccttt ggagctggtg gagaaacttt gtccagaagc atcagatata gcgactagtg 300
tcagaaatct tccagaatta aagacagctg tgggaagagg ccgagcgtgg ctttatcttg 360
cactcatgca aaagaaactg gcagattatc tgaaagtgct tatagacaat aaacatctct 420
taagcgagtt ctatgagcct gaggctttaa tgatggagga agaagggatg gtgattgttg 480
gtctgctggt gggactcaat gttctcgatg ccaatctctg cttgaaagga gaagacttgg 540
attctcaggt tggagtaata gatttttccc tctaccttaa ggatgtgcag gatcttgatg 600
gtggcaagga gcatgaaaga attactgatg tccttgatca aaaaaattat gtggaagaac 660
ttaacccggc acttgagctg caccagttgg ggatctttca acccaagata gatggctttg 720
gaaaagacta actcaaagct tcagaagagc nttnagctgc accagaccga attttgctcc 780
tttcaagaaa nacagcaccn gttaagaaaa ccaaatggaa ttaatttcag 830

<210> 543
<211> 733
<212> DNA
<213> Homo Sapiens

<400> 543
gaaaaataaa tgattttatt gcagggccaa tgataggtag tcacaagggc atgaaatggc 60
agatctcttg tctgaagcag agaaggcaca ctggcagact ccatgtgtgt caaacgctgt 120
gcatgaatca ggtttttaga aggaaggtag gagaggaaaa ctactcacta gcagaactga 180
actgctgtaa aataggttaa attctttgaa aagtgaaaaa tgatagtagc aaaatcatga 240
agttgtatct gaaccagagc cgtgatgtaa ccaagtaaga tggaagtttc catccagagg 300
agttaattcc gaacaagtca cagaaaggtg anagctgccg gttccggcac gctgtcttct 360
ggagtgccag tgaccgggca agaaatttga ttctttcctt tgattctctt gggaaagaac 420
acatttccca agcccctgga gacccacagg gtttggcact gtccgtgagg ctgtgctcct 480
gaggacggac gttcaggagg cccgtggagg agcagcgctg caggagcagg gtgtggcagc 540
tgtcgcacac tcgcaccggc ttggggtagg anggcagggc tagctcgttg ctggancang 600
tgttgcaaaa naatgtggcc acagntncgg cagtgggtgc tttntccggg aaaagggaga 660
acttccttnt cacacttggc tacagnggng gncgctttcg ncatcttttt ancccaggcg 720
nnggcccttt caa 733

<210> 544
<211> 852
<212> DNA
<213> Homo Sapiens

<400> 544
gtggagaaat gcgctatcag ctgaataaaa ccaacatgga gaaggatgag gcagaaaagg 60
agcacagaga gttcagagca aaaactaaca gggatcttga aattaaagat caggaaatag 120
agaaattgag aatagaactg gatgaaagca aacaacactt ggaacaggag cagcagaagg 180
cagccctggc cagagaggag tgcctgagac taacagaact gctgggcgaa tctgagcacc 240
aactgcacct caccagatct gaaatagctc aactcagtca agaaaaaagg tatacatatg 300
ataaattggg aaagttacag agaagaaatg aagaattgga ggaacagtgt gtccagcatg 360
ggagagtaca tgagacgatg aagcaaaggc taaggcagct ggataagcac agccaggcca 420
cagcccagca gctggtgcag ctcctcagca agcagaacca gcttctcctg gagaggcaga 480
gcctgtcgga agaggtggac cggctgcgga cccagttacc cagcatgcca caatctgatt 540
gctgacctgg atggaacaga gtgaaataaa tgaattacaa agagatattt acattcatct 600
ggtttagact taatatgcca caacgcacca cgaccttccc agggtgacac cgcctcagcc 660
tgcagtgggg ctggtcctca tcaacgcggg cgctgtcccc gcacgcagtc gggctggagc 720
tggagtctga ctctagctga gcagactcct ggtgtatgtt ttcagaaatg gcttgaagtt 780
atgtgtttaa atctgctcat tcgtatgcta ggttatacat atgattttca ataaatgaac 840
tttttaaaga aa 852

<210> 545
<211> 414
<212> PRT
<213> Homo Sapiens

<400> 545
Leu Leu Asp Ala Ser Glu Lys Leu Lys Leu Thr Tyr Glu Glu Lys Cys
1               5                   10                  15
Glu Ile Glu Glu Ser Gln Leu Lys Phe Leu Arg Asn Asp Leu Ala Glu
            20                  25                  30
Tyr Gln Arg Thr Cys Glu Asp Leu Lys Glu Gln Leu Lys His Lys Glu
        35                  40                  45
Phe Leu Leu Ala Ala Asn Thr Cys Asn Arg Val Gly Gly Leu Cys Leu
    50                  55                  60
Lys Cys Ala Gln His Glu Ala Val Leu Ser Gln Thr His Thr Asn Val
65                  70                  75                  80
His Met Gln Thr Ile Glu Arg Leu Val Lys Glu Arg Asp Asp Leu Met
                85                  90                  95
Ser Ala Leu Val Ser Val Arg Ser Ser Leu Ala Asp Thr Gln Gln Arg
            100                 105                 110
Glu Ala Ser Ala Tyr Glu Gln Val Lys Gln Val Leu Gln Ile Ser Glu
        115                 120                 125
Glu Ala Asn Phe Glu Lys Thr Lys Ala Leu Ile Gln Cys Asp Gln Leu
    130                 135                 140
Arg Lys Glu Leu Glu Arg Gln Ala Glu Arg Leu Glu Lys Glu Leu Ala
145                 150                 155                 160
Ser Gln Gln Glu Lys Arg Ala Ile Glu Lys Asp Met Met Lys Lys Glu
                165                 170                 175
Ile Thr Lys Glu Arg Glu Tyr Met Gly Ser Lys Met Leu Ile Leu Ser
            180                 185                 190
Gln Asn Ile Ala Gln Leu Glu Ala Gln Val Glu Lys Val Thr Lys Glu
        195                 200                 205
Lys Ile Ser Ala Ile Asn Gln Leu Glu Glu Ile Gln Ser Gln Leu Ala
    210                 215                 220
Ser Arg Glu Met Asp Val Thr Lys Val Cys Gly Glu Met Arg Tyr Gln
225                 230                 235                 240
Leu Asn Lys Thr Asn Met Glu Lys Asp Glu Ala Glu Lys Glu His Arg
                245                 250                 255

Glu Phe Arg Ala Lys Thr Asn Arg Asp Leu Glu Ile Lys Asp Gln Glu
            260                 265                 270
Ile Glu Lys Leu Arg Ile Glu Leu Asp Glu Ser Lys Gln His Leu Glu
        275                 280                 285
Gln Glu Gln Gln Lys Ala Ala Leu Ala Arg Glu Glu Cys Leu Arg Leu
    290                 295                 300
Thr Glu Leu Leu Gly Glu Ser Glu His Gln Leu His Leu Thr Arg Ser
305                 310                 315                 320
Glu Ile Ala Gln Leu Ser Gln Glu Lys Arg Tyr Thr Tyr Asp Lys Leu
                325                 330                 335
Gly Lys Leu Gln Arg Arg Asn Glu Glu Leu Glu Glu Gln Cys Val Gln
            340                 345                 350
His Gly Arg Val His Glu Thr Met Lys Gln Arg Leu Arg Gln Leu Asp
        355                 360                 365
Lys His Ser Gln Ala Thr Ala Gln Gln Leu Val Gln Leu Leu Ser Lys
    370                 375                 380
Gln Asn Gln Leu Leu Leu Glu Arg Gln Ser Leu Ser Glu Glu Val Asp
385                 390                 395                 400
Arg Leu Arg Thr Gln Leu Pro Ser Met Pro Gln Ser Asp Cys
                405                 410

<210> 546
<211> 2885
<212> DNA
<213> Homo Sapiens

<400> 546
ggaattcctc ttgtcgaagt caaaggagcc cacaccaggc ggcctcaacc attccctccc      60
acagcacccc aaatgctggg gagcccacca tgcttctttg gaccagagtt cccctcccca     120
gagcggcccc cctgggacgc ctccctccta caaactgcct ttgcctgggc cctacgacag     180
tcgagacgac ttccccctcc gcaaaacagc ctctgaaccc aacttgaaag tgcgttcaag     240
gctaaaacag aaggtggctg agcggagaag cagtcccctc ctgcgtcgca aggatgggac     300
tgttattagc acctttaaga agagagctgt tgagatcaca ggtgccgggc ctggggcgtc     360
gtccgtgtgt aacagcgcac ccggctccgg ccccagctct cccaacagct cccacagcac     420
catcgctgag aatggcttta ctggctcagt ccccaacatc cccactgaga tgctccctca     480
gcaccgagcc ctccctctgg acagctcccc caaccagttc agcctctaca cgtctccttc     540
tctgcccaac atctccctag ggctgcaggc cacggtcact gtcaccaact cacacctcac     600
tgcctccccg aagctgtcga cacagcagga ggccgagagg caggccctcc agtccctgcg     660
gcagggtggc acgctgaccg gcaagttcat gagcacatcc tctattcctg gctgcctgct     720
gggcgtggca ctggagggcg acgggagccc ccacgggcat gcctccctgc tgcagcatgt     780
gctgttgctg gagcaggccc ggcagcagag caccctcatt gctgtgccac tccacgggca     840
gtccccacta gtgacgggtg aacgtgtggc caccagcatg cggacggtag gcaagctccc     900
gcggcatcgg cccctgagcc gcactcagtc ctcaccgctg ccgcagagtc cccaggccct     960
gcagcagctg gtcatgcaac aacagcacca gcagttcctg gagaagcaga agcagcagca    1020
gctacagctg ggcaagatcc tcaccaagac aggggagctg cccaggcagc ccaccaccca    1080
ccctgaggag acagaggagg agctgacgga gcagcaggag gtcttgctgg gggagggagc    1140
cctgaccatg ccccgggagg gctccacaga gagtgagagc acacaggaag acctggagga    1200
ggaggacgag gaagaggatg gggaggagga ggaggattgc atccaggtta aggacgagga    1260
gggcgagagt ggtgctgagg aggggcccga cttggaggag cctggtgctg gatacaaaaa    1320
actgttctca gatgcccaac cgctgcaacc tttgcaggtg taccaagcgc ccctcagcct     1380
ggccactgtg ccccaccaag ccctgggccg tacccaatcc tcccctgctg cccctggggg    1440
catgaagaac cccccagacc aacccgtcaa gcacctcttc accacaagtg tggtctacga    1500
cacgttcatg ctaaagcacc agtgcatgtg cgggaacaca cacgtgcacc ctgagcatgc    1560
tggccggatc cagagcatct ggtcccggct gcaggagaca ggcctgctta gcaagtgcga    1620
gcggatccga ggtcgcaaag ccacgctaga tgagatccag acagtgcact ctgaatacca    1680

caccctgctc tatgggacca gtcccctcaa ccggcagaag ctagacagca agaagttgct 1740
cggtcccatc agccagaaga tgtatgctgt gctgccttgt gggggcatcg gggtggacag 1800
tgacaccgtg tggaatgaga tgcactcctc cagtgctgtg cgcatggcag tgggctgcct 1860
gctggagctg gccttcaagg tggctgcagg agagctcaag aatggatttg ccatcatccg 1920
gcccccagga caccacgccg aggaatccac agccatggga ttctgcttct tcaactctgt 1980
agccatcacc gcaaaactcc tacagcagaa gttgaacgtg ggcaaggtcc tcatcgtgga 2040
ctgggacatt caccatggca atggcaccca gcaggcgttc tacaatgacc cctctgtgct 2100
ctacatctct ctgcatcgct atgacaacgg gaacttcttt ccaggctctg gggctcctga 2160
agaggttggt ggaggaccag gcgtggggta caatgtgaac gtggcatgga caggaggtgt 2220
ggaccccccc attggagacg tggagtacct tacagccttc aggacagtgg tgatgcccat 2280
tgcccacgag ttctcacctg atgtggtcct agtctccgcc gggtttgatg ctgttgaagg 2340
acatctgtct cctctgggtg gctactctgt caccgccaga tgttttggcc acttgaccag 2400
gcagctgatg accctggcag ggggccgggt ggtgctggcc ctggagggag gccatgactt 2460
gaccgccatc tgtgatgcct ctgaagcttg tgtctcggct ctgctcagtg taaagctgca 2520
gcccttggat gaggcagtct tgcagcaaaa gcccaacatc aacgcagtgg ccacgctaga 2580
gaaagtcatc gagatccaga gcaaacactg gagctgtgtg cagaagttcg ccgctggtct 2640
gggccggtcc ctgcgagggg cccaagcagg tgagaccgaa gaagccgaaa tgtgaacgcc 2700
atggccttgc tgttggtggg ggccgaacag gcccaagctg cggcagcccg ggaacacagc 2760
cccaggccgg cagaggagcc catggagcag gagcctgccc tgtgacgccc cggcccccat 2820
ccctttgggc ttcaccattg tgattttgtt tattttttct attaaaaaca aaaagttaaa 2880
aattt 2885

<210> 547
<211> 897
<212> PRT
<213> Homo Sapiens

<400> 547
Glu Phe Leu Leu Ser Lys Ser Lys Glu Pro Thr Pro Gly Gly Leu Asn
1               5                   10                  15
His Ser Leu Pro Gln His Pro Lys Cys Trp Gly Ala His His Ala Ser
            20                  25                  30
Leu Asp Gln Ser Ser Pro Pro Gln Ser Gly Pro Pro Gly Thr Pro Pro
        35                  40                  45
Ser Tyr Lys Leu Pro Leu Pro Gly Pro Tyr Asp Ser Arg Asp Asp Phe
    50                  55                  60
Pro Leu Arg Lys Thr Ala Ser Glu Pro Asn Leu Lys Val Arg Ser Arg
65                  70                  75                  80
Leu Lys Gln Lys Val Ala Glu Arg Arg Ser Ser Pro Leu Leu Arg Arg
                85                  90                  95
Lys Asp Gly Thr Val Ile Ser Thr Phe Lys Lys Arg Ala Val Glu Ile
            100                 105                 110
Thr Gly Ala Gly Pro Gly Ala Ser Ser Val Cys Asn Ser Ala Pro Gly
        115                 120                 125
Ser Gly Pro Ser Ser Pro Asn Ser Ser His Ser Thr Ile Ala Glu Asn
    130                 135                 140
Gly Phe Thr Gly Ser Val Pro Asn Ile Pro Thr Glu Met Leu Pro Gln
145                 150                 155                 160
His Arg Ala Leu Pro Leu Asp Ser Ser Pro Asn Gln Phe Ser Leu Tyr
                165                 170                 175
Thr Ser Pro Ser Leu Pro Asn Ile Ser Leu Gly Leu Gln Ala Thr Val
            180                 185                 190
Thr Val Thr Asn Ser His Leu Thr Ala Ser Pro Lys Leu Ser Thr Gln
        195                 200                 205
Gln Glu Ala Glu Arg Gln Ala Leu Gln Ser Leu Arg Gln Gly Gly Thr

210                 215                 220
Leu Thr Gly Lys Phe Met Ser Thr Ser Ser Ile Pro Gly Cys Leu Leu
225                 230                 235                 240
Gly Val Ala Leu Glu Gly Asp Gly Ser Pro His Gly His Ala Ser Leu
                245                 250                 255
Leu Gln His Val Leu Leu Leu Glu Gln Ala Arg Gln Gln Ser Thr Leu
            260                 265                 270
Ile Ala Val Pro Leu His Gly Gln Ser Pro Leu Val Thr Gly Glu Arg
        275                 280                 285
Val Ala Thr Ser Met Arg Thr Val Gly Lys Leu Pro Arg His Arg Pro
    290                 295                 300
Leu Ser Arg Thr Gln Ser Ser Pro Leu Pro Gln Ser Pro Gln Ala Leu
305                 310                 315                 320
Gln Gln Leu Val Met Gln Gln Gln His Gln Gln Phe Leu Glu Lys Gln
                325                 330                 335
Lys Gln Gln Gln Leu Gln Leu Gly Lys Ile Leu Thr Lys Thr Gly Glu
            340                 345                 350
Leu Pro Arg Gln Pro Thr Thr His Pro Glu Glu Thr Glu Glu Glu Leu
        355                 360                 365
Thr Glu Gln Gln Glu Val Leu Leu Gly Glu Gly Ala Leu Thr Met Pro
    370                 375                 380
Arg Glu Gly Ser Thr Glu Ser Glu Ser Thr Gln Glu Asp Leu Glu Glu
385                 390                 395                 400
Glu Asp Glu Glu Glu Asp Gly Glu Glu Glu Glu Asp Cys Ile Gln Val
                405                 410                 415
Lys Asp Glu Glu Gly Glu Ser Gly Ala Glu Glu Gly Pro Asp Leu Glu
            420                 425                 430
Glu Pro Gly Ala Gly Tyr Lys Lys Leu Phe Ser Asp Ala Gln Pro Leu
        435                 440                 445
Gln Pro Leu Gln Val Tyr Gln Ala Pro Leu Ser Leu Ala Thr Val Pro
    450                 455                 460
His Gln Ala Leu Gly Arg Thr Gln Ser Ser Pro Ala Ala Pro Gly Gly
465                 470                 475                 480
Met Lys Asn Pro Pro Asp Gln Pro Val Lys His Leu Phe Thr Thr Ser
                485                 490                 495
Val Val Tyr Asp Thr Phe Met Leu Lys His Gln Cys Met Cys Gly Asn
            500                 505                 510
Thr His Val His Pro Glu His Ala Gly Arg Ile Gln Ser Ile Trp Ser
        515                 520                 525
Arg Leu Gln Glu Thr Gly Leu Leu Ser Lys Cys Glu Arg Ile Arg Gly
    530                 535                 540
Arg Lys Ala Thr Leu Asp Glu Ile Gln Thr Val His Ser Glu Tyr His
545                 550                 555                 560
Thr Leu Leu Tyr Gly Thr Ser Pro Leu Asn Arg Gln Lys Leu Asp Ser
                565                 570                 575
Lys Lys Leu Leu Gly Pro Ile Ser Gln Lys Met Tyr Ala Val Leu Pro
            580                 585                 590
Cys Gly Gly Ile Gly Val Asp Ser Asp Thr Val Trp Asn Glu Met His
        595                 600                 605
Ser Ser Ser Ala Val Arg Met Ala Val Gly Cys Leu Leu Glu Leu Ala
    610                 615                 620
Phe Lys Val Ala Ala Gly Glu Leu Lys Asn Gly Phe Ala Ile Ile Arg
625                 630                 635                 640
Pro Pro Gly His His Ala Glu Glu Ser Thr Ala Met Gly Phe Cys Phe
                645                 650                 655

Phe Asn Ser Val Ala Ile Thr Ala Lys Leu Leu Gln Gln Lys Leu Asn
            660                 665                 670
Val Gly Lys Val Leu Ile Val Asp Trp Asp Ile His His Gly Asn Gly
        675                 680                 685
Thr Gln Gln Ala Phe Tyr Asn Asp Pro Ser Val Leu Tyr Ile Ser Leu
    690                 695                 700
His Arg Tyr Asp Asn Gly Asn Phe Phe Pro Gly Ser Gly Ala Pro Glu
705                 710                 715                 720
Glu Val Gly Gly Gly Pro Gly Val Gly Tyr Asn Val Asn Val Ala Trp
                725                 730                 735
Thr Gly Gly Val Asp Pro Pro Ile Gly Asp Val Glu Tyr Leu Thr Ala
            740                 745                 750
Phe Arg Thr Val Val Met Pro Ile Ala His Glu Phe Ser Pro Asp Val
        755                 760                 765
Val Leu Val Ser Ala Gly Phe Asp Ala Val Glu Gly His Leu Ser Pro
    770                 775                 780
Leu Gly Gly Tyr Ser Val Thr Ala Arg Cys Phe Gly His Leu Thr Arg
785                 790                 795                 800
Gln Leu Met Thr Leu Ala Gly Gly Arg Val Val Leu Ala Leu Glu Gly
                805                 810                 815
Gly His Asp Leu Thr Ala Ile Cys Asp Ala Ser Glu Ala Cys Val Ser
            820                 825                 830
Ala Leu Leu Ser Val Lys Leu Gln Pro Leu Asp Glu Ala Val Leu Gln
        835                 840                 845
Gln Lys Pro Asn Ile Asn Ala Val Ala Thr Leu Glu Lys Val Ile Glu
    850                 855                 860
Ile Gln Ser Lys His Trp Ser Cys Val Gln Lys Phe Ala Ala Gly Leu
865                 870                 875                 880
Gly Arg Ser Leu Arg Gly Ala Gln Ala Gly Glu Thr Glu Glu Ala Glu
                885                 890                 895
Met

<210> 548
<211> 1298
<212> DNA
<213> Homo Sapiens

<400> 548
ggctgctgaa atgactgcga accggcttgc agagagcctt ctggctttga gccancagga 60
agaactagcg gatttgccaa aagactacct cttgagtgag agtgaagatg agggggacaa 120
tgatggagag agaaagcatc naaagcttct ggaagcaatc agttcccttg atggaaagaa 180
taggcggaaa ttggctgana ggtctgaggc tagtctgaag gtgtcagagt tcaatgtcag 240
ttctgaagga tcaggagaaa agctggtcct tgcagatctg cttgagcctg ttaaaacttc 300
atcttctttg gccactgtga aaaagcaact gagtagagtc anatcaaaga anacagtgga 360
gttacctctg aacaaagaag agattgaacg gatccacaga gaatagcatt caataaaacg 420
cacaagtcct ctccaaatgg gaccctgtcg tcctgaagaa ccggcaggca gagcagctgg 480
tttttcccct ggagaaagag gagccagcca ttgctcccat tgaacatgtg ctcagtggct 540
ggaaggcaag aactcccctg gagcaggaaa ttttcaacct cctccataag aacaagcagc 600
cagtgacaga ccctttactg acccctgtgg aaaaggcctc tctccgagcc atgagcctag 660
aagaggcaaa gatgcgacga gcagagcttc agaggctcgg ggctctgcag tcctactatg 720
angccaaggc tcgaagagag aagaaaatcn aaagttaaaa gtatcacaaa gtcgtgaaga 780
aaggaaaggc caagaaagcc ctaaaagagt ttgagcagct gcggaaggtt aatccagctg 840
ccgcactaga agaacgaaga aaagaggaaa gaaggaggag gagaaagaag aagaacaagg 900
agaagaagaa agaagaaggg agaaggagaa gaaaagaagg agaagaggaa aaggaagaag 960

gagaaagaaa aggagaagga aaaggaaaag aaggagaaga aagaagaact aagaagaagg 1020
agaggaagaa taagaaggaa agaagaaaga aaaaagtnaa agaagaagaa agaaggaaga 1080
aggaaagaag aggaagaact nagaagaaga aagaggagga aagaagaaag aagaataagg 1140
aacnagaaag aaggagaaga aagaataaga agaggaagaa gaaaaagaag aaaagaagaa 1200
ggaaagaagg agaaaaagga agaaaaaagg aagaagaaag tagaaagcgg aagaaagaaa 1260
agaaagtata agaaggaaga agaagaaaga aggaaaaa 1298

<210> 549
<211> 236
<212> PRT
<213> Homo Sapiens

<400> 549
Ala Ala Glu Met Thr Ala Asn Arg Leu Ala Glu Ser Leu Leu Ala Leu
1               5                   10                  15
Ser Gln Glu Glu Leu Ala Asp Leu Pro Lys Asp Tyr Leu Leu Ser Glu
            20                  25                  30
Ser Glu Asp Glu Gly Asp Asn Asp Gly Glu Arg Lys His Lys Leu Leu
        35                  40                  45
Glu Ala Ile Ser Ser Leu Asp Gly Lys Asn Arg Arg Lys Leu Ala Arg
    50                  55                  60
Ser Glu Ala Ser Leu Lys Val Ser Glu Phe Asn Val Ser Ser Glu Gly
65                  70                  75                  80
Ser Gly Glu Lys Leu Val Leu Ala Asp Leu Leu Glu Pro Val Lys Thr
                85                  90                  95
Ser Ser Ser Leu Ala Thr Val Lys Lys Gln Leu Ser Arg Val Ser Lys
            100                 105                 110
Thr Val Glu Leu Pro Leu Asn Lys Glu Glu Ile Glu Arg Ile His Arg
        115                 120                 125
Glu Ile Ala Phe Asn Lys Thr His Lys Ser Ser Pro Asn Gly Thr Leu
    130                 135                 140
Ser Ser Val Leu Lys Asn Arg Gln Ala Glu Gln Leu Val Phe Pro Leu
145                 150                 155                 160
Glu Lys Glu Glu Pro Ala Ile Ala Pro Ile Glu His Val Leu Ser Gly
                165                 170                 175
Trp Lys Ala Arg Thr Pro Leu Glu Gln Glu Ile Phe Asn Leu Leu His
            180                 185                 190
Lys Asn Lys Gln Pro Val Thr Asp Pro Leu Leu Thr Pro Val Glu Lys
        195                 200                 205
Ala Ser Leu Arg Ala Met Ser Leu Glu Glu Ala Lys Met Arg Arg Ala
    210                 215                 220
Glu Leu Gln Arg Ala Arg Ala Leu Gln Ser Tyr Tyr
225                 230                 235

<210> 550
<211> 2236
<212> DNA
<213> Homo Sapiens

<400> 550
cctggcccgg tcgcggtcgc ggctctttcc agctcctggc agccgggcac ccgaaggaac 60
gggtcgtgca acgacgcagc tggacctggc ccagccatgg accgaaaagt ggcccgagaa 120
ttccggcata aggtggattt tctgattgaa aatgatgcag agaaggacta tctctatgat 180
gtgctgcgaa tgtaccacca gaccatggac gtggccgtgc tcgtgggaga cctgaagctg 240
gtcatcaatg aacccagccg tctgcctctg tttgatgcca ttcggccgct gatcccactg 300

aagcaccagg tggaatatga tcagctgacc ccccggcgct ccaggaagct gaaggaggtg 360
cgtctggacc gtctgcaccc cgaaggcctc ggcctgagtg tgcgtggtgg cctggagttt 420
ggctgtgggc tcttcatctc ccacctcatc aaaggcggtc aggcagacag cgtcgggctc 480
caggtagggg acgagatcgt ccggatcaat ggatattcca tctcctcctg tacccatgag 540
gaggtcatca acctcattcg aaccaagaaa actgtgtcca tcaaagtgag acacatcggc 600
ctgatccccg tgaaaagctc tcctgatgag cccctcactt ggcagtatgt ggatcagttt 660
gtgtcggaat ctgggggcgt gcgaggcagc ctgggctccc ctggaaatcg ggaaaacaag 720
gagaagaagg tcttcatcag cctggtaggc tcccgaggcc ttggctgcag catttccagc 780
ggccccatcc agaagcctgg catctttatc agccatgtga aacctggctc cctgtctgct 840
gaggtgggat tggagatagg ggaccagatt gtcgaagtca atggcgtcga cttctctaac 900
ctggatcaca aggaggctgt aaatgtgctg aaaaatagcc gcagcctgac catctccatt 960
gtagctgcag ctggccggga gctgttcatg acagaccggg agcggctggc agaggcgcgg 1020
cagcgtgagc tgcagcggca ggagcttctc atgcagaagc ggctggcgat ggagtccaac 1080
aagatcctcc aggagcagca ggagatggag cggcaaagga gaaaagaaat tgcccagaag 1140
gcagcagagg aaaatgagag ataccggaag gagatggaac agattgtaga ggaggaagag 1200
aagtttaaga agcaatggga agaagactgg ggctcaaagg aacagctact cttgcctaaa 1260
accatcactg ctgaggtaca cccagtaccc cttcgcaagc caaagtatga tcagggagtg 1320
gaacctgagc tcgagcccgc agatgacctg gatggaggca cggaggagca gggagagcag 1380
gatttccgga aatatgagga aggctttgac ccctactcta tgttcacccc agagcagatc 1440
atggggaagg atgtccggct cctacgcatc aagaaggagg gatccttaga cctggccctg 1500
gaaggcggtg tggactcccc cattgggaag gtggtcgttt ctgctgtgta tgagcgggga 1560
gctgctgagc ggcatggtgg cattgtgaaa ggggacgaga tcatggcaat caacggcaag 1620
attgtgacag actacaccct ggctgaggct gacgctgccc tgcagaaggc ctggaatcag 1680
ggcggggact ggatcgacct tgtggttgcc gtctgccccc caaaggagta tgacgatgag 1740
ctgaccttct tgctgaagtc caaaagggga aaccaaattc acgcgttagg aaacagtgag 1800
ctccggcccc acctcgtgaa cacaaagcct cggaccagcc ttgagagagg ccacatgaca 1860
cacaccagat ggcatccttg ggacctgaat ctatcaccca ggaatctcaa actccctttg 1920
gccctgaacc agggccagat aaggaacagc tcgggccact tttttgaagg ccaatgtgga 1980
ggaaagggag cagccagccg tttgggagaa gatctcaagg atccagactc tcattccttt 2040
cctctggccc agtgaatttg gtctctccca gctttggggg actccttcct tgaaccctaa 2100
taagacccca ctggagtctc tctctctcca tccctctcct ctgccctctg ctctaattgc 2160
tgccaggatt gtcactccaa accttactct gagctcatta ataaaataaa cagatttatt 2220
ttccagctta aaaaaa 2236

<210> 551
<211> 652
<212> PRT
<213> Homo Sapiens

<400> 551
Met Asp Arg Lys Val Ala Arg Glu Phe Arg His Lys Val Asp Phe Leu
1               5                   10                  15
Ile Glu Asn Asp Ala Glu Lys Asp Tyr Leu Tyr Asp Val Leu Arg Met
            20                  25                  30
Tyr His Gln Thr Met Asp Val Ala Val Leu Val Gly Asp Leu Lys Leu
        35                  40                  45
Val Ile Asn Glu Pro Ser Arg Leu Pro Leu Phe Asp Ala Ile Arg Pro
    50                  55                  60
Leu Ile Pro Leu Lys His Gln Val Glu Tyr Asp Gln Leu Thr Pro Arg
65                  70                  75                  80
Arg Ser Arg Lys Leu Lys Glu Val Arg Leu Asp Arg Leu His Pro Glu
                85                  90                  95
Gly Leu Gly Leu Ser Val Arg Gly Gly Leu Glu Phe Gly Cys Gly Leu
            100                 105                 110
Phe Ile Ser His Leu Ile Lys Gly Gly Gln Ala Asp Ser Val Gly Leu

115                 120                 125
Gln Val Gly Asp Glu Ile Val Arg Ile Asn Gly Tyr Ser Ile Ser Ser
    130                 135                 140
Cys Thr His Glu Glu Val Ile Asn Leu Ile Arg Thr Lys Lys Thr Val
145                 150                 155                 160
Ser Ile Lys Val Arg His Ile Gly Leu Ile Pro Val Lys Ser Ser Pro
                165                 170                 175
Asp Glu Pro Leu Thr Trp Gln Tyr Val Asp Gln Phe Val Ser Glu Ser
            180                 185                 190
Gly Gly Val Arg Gly Ser Leu Gly Ser Pro Gly Asn Arg Glu Asn Lys
        195                 200                 205
Glu Lys Lys Val Phe Ile Ser Leu Val Gly Ser Arg Gly Leu Gly Cys
    210                 215                 220
Ser Ile Ser Ser Gly Pro Ile Gln Lys Pro Gly Ile Phe Ile Ser His
225                 230                 235                 240
Val Lys Pro Gly Ser Leu Ser Ala Glu Val Gly Leu Glu Ile Gly Asp
                245                 250                 255
Gln Ile Val Glu Val Asn Gly Val Asp Phe Ser Asn Leu Asp His Lys
            260                 265                 270
Glu Ala Val Asn Val Leu Lys Asn Ser Arg Ser Leu Thr Ile Ser Ile
        275                 280                 285
Val Ala Ala Ala Gly Arg Glu Leu Phe Met Thr Asp Arg Glu Arg Leu
    290                 295                 300
Ala Glu Ala Arg Gln Arg Glu Leu Gln Arg Gln Glu Leu Leu Met Gln
305                 310                 315                 320
Lys Arg Leu Ala Met Glu Ser Asn Lys Ile Leu Gln Glu Gln Gln Glu
                325                 330                 335
Met Glu Arg Gln Arg Arg Lys Glu Ile Ala Gln Lys Ala Ala Glu Glu
            340                 345                 350
Asn Glu Arg Tyr Arg Lys Glu Met Glu Gln Ile Val Glu Glu Glu Glu
        355                 360                 365
Lys Phe Lys Lys Gln Trp Glu Glu Asp Trp Gly Ser Lys Glu Gln Leu
    370                 375                 380
Leu Leu Pro Lys Thr Ile Thr Ala Glu Val His Pro Val Pro Leu Arg
385                 390                 395                 400
Lys Pro Lys Tyr Asp Gln Gly Val Glu Pro Glu Leu Glu Pro Ala Asp
                405                 410                 415
Asp Leu Asp Gly Gly Thr Glu Glu Gln Gly Glu Gln Asp Phe Arg Lys
            420                 425                 430
Tyr Glu Glu Gly Phe Asp Pro Tyr Ser Met Phe Thr Pro Glu Gln Ile
        435                 440                 445
Met Gly Lys Asp Val Arg Leu Leu Arg Ile Lys Lys Glu Gly Ser Leu
    450                 455                 460
Asp Leu Ala Leu Glu Gly Gly Val Asp Ser Pro Ile Gly Lys Val Val
465                 470                 475                 480
Val Ser Ala Val Tyr Glu Arg Gly Ala Ala Glu Arg His Gly Gly Ile
                485                 490                 495
Val Lys Gly Asp Glu Ile Met Ala Ile Asn Gly Lys Ile Val Thr Asp
            500                 505                 510
Tyr Thr Leu Ala Glu Ala Asp Ala Ala Leu Gln Lys Ala Trp Asn Gln
        515                 520                 525
Gly Gly Asp Trp Ile Asp Leu Val Val Ala Val Cys Pro Pro Lys Glu
    530                 535                 540
Tyr Asp Asp Glu Leu Thr Phe Leu Leu Lys Ser Lys Arg Gly Asn Gln
545                 550                 555                 560

Ile His Ala Leu Gly Asn Ser Glu Leu Arg Pro His Leu Val Asn Thr
                565                 570                 575
Lys Pro Arg Thr Ser Leu Glu Arg Gly His Met Thr His Thr Arg Trp
            580                 585                 590
His Pro Trp Asp Leu Asn Leu Ser Pro Arg Asn Leu Lys Leu Pro Leu
        595                 600                 605
Ala Leu Asn Gln Gly Gln Ile Arg Asn Ser Ser Gly His Phe Phe Glu
    610                 615                 620
Gly Gln Cys Gly Gly Lys Gly Ala Ala Ser Arg Leu Gly Glu Asp Leu
625                 630                 635                 640
Lys Asp Pro Asp Ser His Ser Phe Pro Leu Ala Gln
                645                 650

<210> 552
<211> 2162
<212> DNA
<213> Homo Sapiens

<400> 552
cctggcccgg tcgcggtcgc ggctctttcc agctcctggc agccgggcac ccgaaggaac      60
gggtcgtgca acgacgcagc tggacctggc ccagccatgg accgaaaagt ggcccgagaa     120
ttccggcata aggtggattt tctgattgaa aatgatgcag agaaggacta tctctatgat     180
gtgctgcgaa tgtaccacca gaccatggac gtggccgtgc tcgtgggaga cctgaagctg     240
gtcatcaatg aacccagccg tctgcctctg tttgatgcca ttcggccgct gatcccactg     300
aagcaccagg tggaatatga tcagctgacc ccccggcgct ccaggaagct gaaggaggtg     360
cgtctggacc gtctgcaccc cgaaggcctc ggcctgagtg tgcgtggtgg cctggagttt     420
ggctgtgggc tcttcatctc ccacctcatc aaaggcggtc aggcagacag cgtcggcctc     480
caggtagggg acgagatcgt ccggatcaat ggatattcca tctcctcctg tacccatgag     540
gaggtcatca acctcattcg aaccaagaaa actgtgtcca tcaaagtgag acacatcggc     600
ctgatccccg tgaaaagctc tcctgatgag cccctcactt ggcagtatgt ggatcagttt     660
gtgtcggaat ctgggggcgt gcgaggcagc ctgggctccc ctggaaatcg ggaaaacaag     720
gagaagaagg tcttcatcag cctggtaggc tcccgaggcc ttggctgcag catttccagc     780
ggccccatcc agaagcctgg catctttatc agccatgtga aacctggctc cctgtctgct     840
gaggtgggat tggagatagg ggaccagatt gtcgaagtca atggcgtcga cttctctaac     900
ctggatcaca aggaggctgt aaatgtgctg aaaaatagcc gcagcctgac catctccatt     960
gtagctgcag ctggccggga gctgttcatg acagaccggg agcggctggc agaggcgcgg    1020
cagcgtgagc tgcagcggca ggagcttctc atgcagaagc ggctggcgat ggagtccaac    1080
aagatcctcc aggagcagca ggagatggag cggcaaagga gaaaagaaat tgcccagaag    1140
gcagcagagg aaaatgagag ataccggaag gagatggaac agattgtaga ggaggaagag    1200
aagtttaaga agcaatggga agaagactgg ggctcaaagg aacagctact cttgcctaaa    1260
accatcactg ctgaggtaca cccagtaccc cttcgcaagc caaagtgatt tccggaaata    1320
tgaggaaggc tttgacccct actctatgtt caccccagag cagatcatgg ggaaggatgt    1380
ccggctccta cgcatcaaga aggagggatc cttagacctg gccctggaag gcggtgtgga    1440
ctcccccatt gggaaggtgg tcgtttctgc tgtgtatgag cggggagctg ctgagcggca    1500
tggtggcatt gtgaaagggg acgagatcat ggcaatcaac ggcaagattg tgacagacta    1560
caccctggct gaggctgacg ctgccctgca gaaggcctgg aatcagggcg gggactggat    1620
cgaccttgtg gttgccgtct gccccccaaa ggagtatgac gatgagctga ccttcttgct    1680
gaagtccaaa aggggaaacc aaattcacgc gttaggaaac agtgagctcc ggccccacct    1740
cgtgaacaca aagcctcgga ccagccttga gagaggccac atgacacaca ccagatggca    1800
tccttgggac ctgaatctat cacccaggaa tctcaaactc cctttggccc tgaaccaggg    1860
ccagataagg aacagctcgg gccacttttt tgaaggccaa tgtggaggaa agggagcagc    1920
cagccgtttg ggagaagatc tcaaggatcc agactctcat tcctttcctc tggcccagtg    1980
aatttggtct ctcccagctt tgggggactc cttccttgaa ccctaataag accccactgg    2040
agtctctctc tctccatccc tctcctctgc cctctgctct aattgctgcc aggattgtca    2100
ctccaaacct tactctgagc tcattaataa aataaacaga tttattttcc agcttaaaaa    2160

aa

<210> 553
<211> 403
<212> PRT
<213> Homo Sapiens

<400> 553
Met Asp Arg Lys Val Ala Arg Glu Phe Arg His Lys Val Asp Phe Leu
1               5                   10                  15
Ile Glu Asn Asp Ala Glu Lys Asp Tyr Leu Tyr Asp Val Leu Arg Met
            20                  25                  30
Tyr His Gln Thr Met Asp Val Ala Val Leu Val Gly Asp Leu Lys Leu
        35                  40                  45
Val Ile Asn Glu Pro Ser Arg Leu Pro Leu Phe Asp Ala Ile Arg Pro
    50                  55                  60
Leu Ile Pro Leu Lys His Gln Val Glu Tyr Asp Gln Leu Thr Pro Arg
65                  70                  75                  80
Arg Ser Arg Lys Leu Lys Glu Val Arg Leu Asp Arg Leu His Pro Glu
                85                  90                  95
Gly Leu Gly Leu Ser Val Arg Gly Gly Leu Glu Phe Gly Cys Gly Leu
            100                 105                 110
Phe Ile Ser His Leu Ile Lys Gly Gly Gln Ala Asp Ser Val Gly Leu
        115                 120                 125
Gln Val Gly Asp Glu Ile Val Arg Ile Asn Gly Tyr Ser Ile Ser Ser
    130                 135                 140
Cys Thr His Glu Glu Val Ile Asn Leu Ile Arg Thr Lys Lys Thr Val
145                 150                 155                 160
Ser Ile Lys Val Arg His Ile Gly Leu Ile Pro Val Lys Ser Ser Pro
                165                 170                 175
Asp Glu Pro Leu Thr Trp Gln Tyr Val Asp Gln Phe Val Ser Glu Ser
            180                 185                 190
Gly Gly Val Arg Gly Ser Leu Gly Ser Pro Gly Asn Arg Glu Asn Lys
        195                 200                 205
Glu Lys Lys Val Phe Ile Ser Leu Val Gly Ser Arg Gly Leu Gly Cys
    210                 215                 220
Ser Ile Ser Ser Gly Pro Ile Gln Lys Pro Gly Ile Phe Ile Ser His
225                 230                 235                 240
Val Lys Pro Gly Ser Leu Ser Ala Glu Val Gly Leu Glu Ile Gly Asp
                245                 250                 255
Gln Ile Val Glu Val Asn Gly Val Asp Phe Ser Asn Leu Asp His Lys
            260                 265                 270
Glu Ala Val Asn Val Leu Lys Asn Ser Arg Ser Leu Thr Ile Ser Ile
        275                 280                 285
Val Ala Ala Ala Gly Arg Glu Leu Phe Met Thr Asp Arg Glu Arg Leu
    290                 295                 300
Ala Glu Ala Arg Gln Arg Glu Leu Gln Arg Gln Glu Leu Leu Met Gln
305                 310                 315                 320
Lys Arg Leu Ala Met Glu Ser Asn Lys Ile Leu Gln Glu Gln Gln Glu
                325                 330                 335
Met Glu Arg Gln Arg Arg Lys Glu Ile Ala Gln Lys Ala Ala Glu Glu
            340                 345                 350
Asn Glu Arg Tyr Arg Lys Glu Met Glu Gln Ile Val Glu Glu Glu Glu
        355                 360                 365
Lys Phe Lys Lys Gln Trp Glu Glu Asp Trp Gly Ser Lys Glu Gln Leu

370                 375                 380
Leu Leu Pro Lys Thr Ile Thr Ala Glu Val His Pro Val Pro Leu Arg
385                 390                 395                 400
Lys Pro Lys

<210> 554
<211> 1789
<212> DNA
<213> Homo Sapiens

<400> 554
cttctggatg catccgagaa gctaaaactt acttatgagg aaaagtgtga aattgaggaa      60
tcccaattga agtttttgag gaacgactta gctgaatatc agagaacttg tgaagatctt     120
aaagagcaac taaagcataa agaatttctt ctggctgcta atacttgtaa ccgtgttggt     180
ggtctttgtt tgaaatgtgc tcagcatgaa gctgttcttt cccaaaccca tactaatgtt     240
catatgcaga ccatcgaaag actggttaaa gaaagagatg acttgatgtc tgcactagtt     300
tccgtaagga gcagcttggc agatacgcag caaagagaag caagtgctta tgaacaggtg     360
aaacaagttt tgcaaatatc tgaggaagcc aattttgaaa aaaccaaggc tttaatccag     420
tgtgaccagt tgaggaagga gctggagagg caggcggagc gacttgaaaa agaacttgca     480
tctcagcaag agaaaagggc cattgagaaa gacatgatga aaaaggaaat aacgaaagaa     540
agggagtaca tgggatcaaa gatgttgatc ttgtctcaga atattgccca actggaggcc     600
caggtggaaa aggttacaaa ggaaaagatt tcagctatta atcaactgga ggaaattcaa     660
agccagctgg cttctcggga aatggatgtc acaaaggtgt gtggagaaat gcgctatcag     720
ctgaataaaa ccaacatgga gaaggatgag gcagaaaagg agcacagaga gttcagagca     780
aaaactaaca gggatcttga aattaaagat caggaaatag agaaattgag aatagaactg     840
gatgaaagca aacaacactt ggaacaggag cagcagaagg cagccctggc cagagaggag     900
tgcctgagac taacagaact gctgggcgaa tctgagcacc aactgcacct caccagacag     960
gaaaaagata gcattcagca gagctttagc aaggaagcaa aggcccaagc ccttcaggcc    1020
cagcaaagag agcaggagct gacacagaag atacagcaaa tggaagccca gcatgacaaa    1080
actgaaaatg aacagtattt gttgctgacc tcccagaata catttttgac aaagttaaag    1140
gaagaatgct gtacattagc caagaaactg gaacaaatct ctcaaaaaac cagatctgaa    1200
atagctcaac tcagtcaaga aaaaaggtat acatatgata aattgggaaa gttacagaga    1260
agaaatgaag aattggagga acagtgtgtc cagcatggga gagtacatga gacgatgaag    1320
caaaggctaa ggcagctgga taagcacagc caggccacag cccagcagct ggtgcagctc    1380
ctcagcaagc agaaccagct tctcctggag aggcagagcc tgtcggaaga ggtggaccgg    1440
ctgcggaccc agttacccag catgccacaa tctgattgct gacctggatg gaacagagtg    1500
aaataaatga attacaaaga gatatttaca ttcatctggt ttagacttaa tatgccacaa    1560
cgcaccacga ccttcccagg gtgacaccgc ctcagcctgc agtggggctg gtcctcatca    1620
acgcgggcgc tgtccccgca cgcagtcggg ctggagctgg agtctgactc tagctgagca    1680
gactcctggt gtatgttttc agaaatggct tgaagttatg tgtttaaatc tgctcattcg    1740
tatgctaggt tatacatatg attttcaata aatgaacttt ttaaagaaa                1789

<210> 555
<211> 493
<212> PRT
<213> Homo Sapiens

<400> 555
Leu Leu Asp Ala Ser Glu Lys Leu Lys Leu Thr Tyr Glu Glu Lys Cys
1               5                   10                  15
Glu Ile Glu Glu Ser Gln Leu Lys Phe Leu Arg Asn Asp Leu Ala Glu
            20                  25                  30
Tyr Gln Arg Thr Cys Glu Asp Leu Lys Glu Gln Leu Lys His Lys Glu
        35                  40                  45

Phe Leu Leu Ala Ala Asn Thr Cys Asn Arg Val Gly Gly Leu Cys Leu
    50                  55                  60
Lys Cys Ala Gln His Glu Ala Val Leu Ser Gln Thr His Thr Asn Val
65                  70                  75                  80
His Met Gln Thr Ile Glu Arg Leu Val Lys Glu Arg Asp Asp Leu Met
                85                  90                  95
Ser Ala Leu Val Ser Val Arg Ser Ser Leu Ala Asp Thr Gln Gln Arg
            100                 105                 110
Glu Ala Ser Ala Tyr Glu Gln Val Lys Gln Val Leu Gln Ile Ser Glu
        115                 120                 125
Glu Ala Asn Phe Glu Lys Thr Lys Ala Leu Ile Gln Cys Asp Gln Leu
    130                 135                 140
Arg Lys Glu Leu Glu Arg Gln Ala Glu Arg Leu Glu Lys Glu Leu Ala
145                 150                 155                 160
Ser Gln Gln Glu Lys Arg Ala Ile Glu Lys Asp Met Met Lys Lys Glu
                165                 170                 175
Ile Thr Lys Glu Arg Glu Tyr Met Gly Ser Lys Met Leu Ile Leu Ser
            180                 185                 190
Gln Asn Ile Ala Gln Leu Glu Ala Gln Val Glu Lys Val Thr Lys Glu
        195                 200                 205
Lys Ile Ser Ala Ile Asn Gln Leu Glu Glu Ile Gln Ser Gln Leu Ala
    210                 215                 220
Ser Arg Glu Met Asp Val Thr Lys Val Cys Gly Glu Met Arg Tyr Gln
225                 230                 235                 240
Leu Asn Lys Thr Asn Met Glu Lys Asp Glu Ala Glu Lys Glu His Arg
                245                 250                 255
Glu Phe Arg Ala Lys Thr Asn Arg Asp Leu Glu Ile Lys Asp Gln Glu
            260                 265                 270
Ile Glu Lys Leu Arg Ile Glu Leu Asp Glu Ser Lys Gln His Leu Glu
        275                 280                 285
Gln Glu Gln Gln Lys Ala Ala Leu Ala Arg Glu Glu Cys Leu Arg Leu
    290                 295                 300
Thr Glu Leu Leu Gly Glu Ser Glu His Gln Leu His Leu Thr Arg Gln
305                 310                 315                 320
Glu Lys Asp Ser Ile Gln Gln Ser Phe Ser Lys Glu Ala Lys Ala Gln
                325                 330                 335
Ala Leu Gln Ala Gln Gln Arg Glu Gln Glu Leu Thr Gln Lys Ile Gln
            340                 345                 350
Gln Met Glu Ala Gln His Asp Lys Thr Glu Asn Glu Gln Tyr Leu Leu
        355                 360                 365
Leu Thr Ser Gln Asn Thr Phe Leu Thr Lys Leu Lys Glu Glu Cys Cys
    370                 375                 380
Thr Leu Ala Lys Lys Leu Glu Gln Ile Ser Gln Lys Thr Arg Ser Glu
385                 390                 395                 400
Ile Ala Gln Leu Ser Gln Glu Lys Arg Tyr Thr Tyr Asp Lys Leu Gly
                405                 410                 415
Lys Leu Gln Arg Arg Asn Glu Glu Leu Glu Glu Gln Cys Val Gln His
            420                 425                 430
Gly Arg Val His Glu Thr Met Lys Gln Arg Leu Arg Gln Leu Asp Lys
        435                 440                 445
His Ser Gln Ala Thr Ala Gln Gln Leu Val Gln Leu Leu Ser Lys Gln
    450                 455                 460
Asn Gln Leu Leu Leu Glu Arg Gln Ser Leu Ser Glu Glu Val Asp Arg
465                 470                 475                 480
Leu Arg Thr Gln Leu Pro Ser Met Pro Gln Ser Asp Cys

485                    490

<210> 556
<211> 1306
<212> DNA
<213> Homo Sapiens

<400> 556
aaaaatagcc gcagcctgac catctccatt gtagctgcag ctggccggga gctgttcatg      60
acagaccggg agcggctggc agaggcgcgg cagcgtgagc tgcagcggca ggagcttctc     120
atgcagaagc ggctggcgat ggagtccaac aagatcctcc aggagcagca ggagatggag     180
cggcaaagga gaaaagaaat tgcccagaag gcagcagagg aaaatgagag ataccggaag     240
gagatggaac agattgtaga ggaggaagag aagtttaaga agcaatggga agaagactgg     300
ggctcaaagg aacagctact cttgcctaaa accatcactg ctgaggtaca cccagtaccc     360
cttcgcaagc caaagtatga tcagggagtg gaacctgagc tcgagcccgc agatgacctg     420
gatggaggca cggaggagca gggagagcag gatttccgga aatatgagga aggctttgac     480
ccctactcta tgttcacccc agagcagatc atggggaagg atgtccggct cctacgcatc     540
aagaaggagg gatccttaga cctggccctg gaaggcggtg tggactcccc cattgggaag     600
gtggtcgttt ctgctgtgta tgagcgggga gctgctgagc ggcatggtgg cattgtgaaa     660
ggggacgaga tcatggcaat caacggcaag attgtgacag actacaccct ggctgaggct     720
gacgctgccc tgcagaaggc ctggaatcag ggcggggact ggatcgacct tgtggttgcc     780
gtctgccccc caaaggagta tgacgatgag ctgaccttct tgctgaagtc caaaagggga     840
aaccaaattc acgcgttagg aaacagtgag ctccggcccc acctcgtgaa cacaaagcct     900
cggaccagcc ttgagagagg ccacatgaca cacaccagat ggcatccttg ggacctgaat     960
ctatcaccca ggaatctcaa actccctttg gccctgaacc agggccagat aaggaacagc    1020
tcgggccact tttttgaagg ccaatgtgga ggaaagggag cagccagccg tttgggagaa    1080
gatctcaagg atccagactc tcattccttt cctctggccc agtgaatttg gtctctccca    1140
gctttggggg actccttcct tgaaccctaa taagacccca ctggagtctc tctctctcca    1200
tccctctcct ctgccctctg ctctaattgc tgccaggatt gtcactccaa accttactct    1260
gagctcatta ataaaataaa cagatttatt ttccagctta aaaaaa                   1306

<210> 557
<211> 328
<212> PRT
<213> Homo Sapiens

<400> 557
Met Glu Ser Asn Lys Ile Leu Gln Glu Gln Gln Glu Met Glu Arg Gln
1               5                   10                  15
Arg Arg Lys Glu Ile Ala Gln Lys Ala Ala Glu Glu Asn Glu Arg Tyr
            20                  25                  30
Arg Lys Glu Met Glu Gln Ile Val Glu Glu Glu Glu Lys Phe Lys Lys
        35                  40                  45
Gln Trp Glu Glu Asp Trp Gly Ser Lys Glu Gln Leu Leu Leu Pro Lys
    50                  55                  60
Thr Ile Thr Ala Glu Val His Pro Val Pro Leu Arg Lys Pro Lys Tyr
65                  70                  75                  80
Asp Gln Gly Val Glu Pro Glu Leu Glu Pro Ala Asp Asp Leu Asp Gly
                85                  90                  95
Gly Thr Glu Glu Gln Gly Glu Gln Asp Phe Arg Lys Tyr Glu Glu Gly
            100                 105                 110
Phe Asp Pro Tyr Ser Met Phe Thr Pro Glu Gln Ile Met Gly Lys Asp
        115                 120                 125
Val Arg Leu Leu Arg Ile Lys Lys Glu Gly Ser Leu Asp Leu Ala Leu
    130                 135                 140

Glu Gly Gly Val Asp Ser Pro Ile Gly Lys Val Val Val Ser Ala Val
145                 150                 155                 160
Tyr Glu Arg Gly Ala Ala Glu Arg His Gly Gly Ile Val Lys Gly Asp
                165                 170                 175
Glu Ile Met Ala Ile Asn Gly Lys Ile Val Thr Asp Tyr Thr Leu Ala
            180                 185                 190
Glu Ala Asp Ala Ala Leu Gln Lys Ala Trp Asn Gln Gly Gly Asp Trp
        195                 200                 205
Ile Asp Leu Val Val Ala Val Cys Pro Pro Lys Glu Tyr Asp Asp Glu
    210                 215                 220
Leu Thr Phe Leu Leu Lys Ser Lys Arg Gly Asn Gln Ile His Ala Leu
225                 230                 235                 240
Gly Asn Ser Glu Leu Arg Pro His Leu Val Asn Thr Lys Pro Arg Thr
                245                 250                 255
Ser Leu Glu Arg Gly His Met Thr His Thr Arg Trp His Pro Trp Asp
            260                 265                 270
Leu Asn Leu Ser Pro Arg Asn Leu Lys Leu Pro Leu Ala Leu Asn Gln
        275                 280                 285
Gly Gln Ile Arg Asn Ser Ser Gly His Phe Phe Glu Gly Gln Cys Gly
    290                 295                 300
Gly Lys Gly Ala Ala Ser Arg Leu Gly Glu Asp Leu Lys Asp Pro Asp
305                 310                 315                 320
Ser His Ser Phe Pro Leu Ala Gln
                325

<210> 558
<211> 2289
<212> DNA
<213> Homo Sapiens

<400> 558
cctggcccgg tcgcggtcgc ggctctttcc agctcctggc agccgggcac ccgaaggaac      60
gggtcgtgca acgacgcagc tggacctggc ccagccatgg accgaaaagt ggcccgagaa     120
ttccggcata aggtggattt tctgattgaa aatgatgcag agaaggacta tctctatgat     180
gtgctgcgaa tgtaccacca gaccatggac gtggccgtgc tcgtgggaga cctgaagctg     240
gtcatcaatg aacccagccg tctgcctctg tttgatgcca ttcggccgct gatcccactg     300
aagcaccagg tggaatatga tcagctgacc ccccggcgct ccaggaagct gaaggaggtg     360
cgtctggacc gtctgcaccc cgaaggcctc ggcctgagtg tgcgtggtgg cctggagttt     420
ggctgtgggc tcttcatctc ccacctcatc aaaggcggtc aggcagacag cgtcgggctc     480
caggtagggg acgagatcgt ccggatcaat ggatattcca tctcctcctg tacccatgag     540
gaggtcatca acctcattcg aaccaagaaa actgtgtcca tcaaagtgag acacatcggc     600
ctgatccccg tgaaaagctc tcctgatgag cccctcactt ggcagtatgt ggatcagttt     660
gtgtcggaat ctgggggcgt gcgaggcagc ctgggctccc ctggaaatcg ggaaaacaag     720
gagaagaagg tcttcatcag cctggtaggc tcccgaggcc ttggctgcag catttccagc     780
ggccccatcc agaagcctgg catctttatc agccatgtga aacctggctc cctgtctgct     840
gaggtgggat tggagatagg ggaccagatt gtcgaagtca atggcgtcga cttctctaac     900
ctggatcaca aggaggctgt aaatgtgctg aaaaatagcc gcagcctgac catctccatt     960
gtagctgcag ctggccggga gctgttcatg acagaccggg agcggctggc agaggcgcgg    1020
cagcgtgagc tgcagcggca ggagcttctc atgcagaagc ggctggcgat ggagtccaac    1080
aagatcctcc aggagcagca ggagatggag cggcaaagga gaaaagaaat tgcccagaag    1140
gcagcagagg aaaatgagag ataccggaag gagatggaac agattgtaga ggaggaagag    1200
aagtttaaga agcaatggga agaagactgg ggctcaaagg aacagctact cttgcctaaa    1260
accatcactg ctgaggtaca cccagtaccc cttcgcaagc caaagtatga tcagggagtg    1320
gaacctgagc tcgagcccgc agatgacctg gatggaggca cggaggagca gggagagcag    1380
ccacaggaga tgttgaagag gatggtggtt tatcaagaca gcattcaaga caagatttcc    1440

ggaaatatga ggaaggcttt gacccctact ctatgttcac cccagagcag atcatgggga 1500
aggatgtccg gctcctacgc atcaagaagg agggatcctt agacctggcc ctggaaggcg 1560
gtgtggactc ccccattggg aaggtggtcg tttctgctgt gtatgagcgg ggagctgctg 1620
agcggcatgg tggcattgtg aaaggggacg agatcatggc aatcaacggc aagattgtga 1680
cagactacac cctggctgag gctgacgctg ccctgcagaa ggcctggaat cagggcgggg 1740
actggatcga ccttgtggtt gccgtctgcc ccccaaagga gtatgacgat gagctgacct 1800
tcttgctgaa gtccaaaagg ggaaaccaaa ttcacgcgtt aggaaacagt gagctccggc 1860
cccacctcgt gaacacaaag cctcggacca gccttgagag aggccacatg acacacacca 1920
gatggcatcc ttgggacctg aatctatcac ccaggaatct caaactccct ttggccctga 1980
accagggcca gataaggaac agctcgggcc acttttttga aggccaatgt ggaggaaagg 2040
gagcagccag ccgtttggga gaagatctca aggatccaga ctctcattcc tttcctctgg 2100
cccagtgaat ttggtctctc ccagctttgg gggactcctt ccttgaaccc taataagacc 2160
ccactggagt ctctctctct ccatccctct cctctgccct ctgctctaat tgctgccagg 2220
attgtcactc caaaccttac tctgagctca ttaataaaat aaacagattt attttccagc 2280
ttaaaaaaa 2289

<210> 559
<211> 481
<212> PRT
<213> Homo Sapiens

<400> 559
   Met Asp Arg Lys Val Ala Arg Glu Phe Arg His Lys Val Asp Phe Leu
   1 5 10 15
   Ile Glu Asn Asp Ala Glu Lys Asp Tyr Leu Tyr Asp Val Leu Arg Met
   20 25 30
   Tyr His Gln Thr Met Asp Val Ala Val Leu Val Gly Asp Leu Lys Leu
   35 40 45
   Val Ile Asn Glu Pro Ser Arg Leu Pro Leu Phe Asp Ala Ile Arg Pro
   50 55 60
   Leu Ile Pro Leu Lys His Gln Val Glu Tyr Asp Gln Leu Thr Pro Arg
   65 70 75 80
   Arg Ser Arg Lys Leu Lys Glu Val Arg Leu Asp Arg Leu His Pro Glu
   85 90 95
   Gly Leu Gly Leu Ser Val Arg Gly Gly Leu Glu Phe Gly Cys Gly Leu
   100 105 110
   Phe Ile Ser His Leu Ile Lys Gly Gly Gln Ala Asp Ser Val Gly Leu
   115 120 125
   Gln Val Gly Asp Glu Ile Val Arg Ile Asn Gly Tyr Ser Ile Ser Ser
   130 135 140
   Cys Thr His Glu Glu Val Ile Asn Leu Ile Arg Thr Lys Lys Thr Val
   145 150 155 160
   Ser Ile Lys Val Arg His Ile Gly Leu Ile Pro Val Lys Ser Ser Pro
   165 170 175
   Asp Glu Pro Leu Thr Trp Gln Tyr Val Asp Gln Phe Val Ser Glu Ser
   180 185 190
   Gly Gly Val Arg Gly Ser Leu Gly Ser Pro Gly Asn Arg Glu Asn Lys
   195 200 205
   Glu Lys Lys Val Phe Ile Ser Leu Val Gly Ser Arg Gly Leu Gly Cys
   210 215 220
   Ser Ile Ser Ser Gly Pro Ile Gln Lys Pro Gly Ile Phe Ile Ser His
   225 230 235 240
   Val Lys Pro Gly Ser Leu Ser Ala Glu Val Gly Leu Glu Ile Gly Asp
   245 250 255
   Gln Ile Val Glu Val Asn Gly Val Asp Phe Ser Asn Leu Asp His Lys
   260 265 270
   Glu Ala Val Asn Val Leu Lys Asn Ser Arg Ser Leu Thr Ile Ser Ile
   275 280 285
   Val Ala Ala Ala Gly Arg Glu Leu Phe Met Thr Asp Arg Glu Arg Leu
   290 295 300
   Ala Glu Ala Arg Gln Arg Glu Leu Gln Arg Gln Glu Leu Leu Met Gln
   305 310 315 320
   Lys Arg Leu Ala Met Glu Ser Asn Lys Ile Leu Gln Glu Gln Gln Glu
   325 330 335
   Met Glu Arg Gln Arg Arg Lys Glu Ile Ala Gln Lys Ala Ala Glu Glu
   340 345 350
   Asn Glu Arg Tyr Arg Lys Glu Met Glu Gln Ile Val Glu Glu Glu Glu
   355 360 365
   Lys Phe Lys Lys Gln Trp Glu Glu Asp Trp Gly Ser Lys Glu Gln Leu
   370 375 380
   Leu Leu Pro Lys Thr Ile Thr Ala Glu Val His Pro Val Pro Leu Arg
   385 390 395 400
   Lys Pro Lys Tyr Asp Gln Gly Val Glu Pro Glu Leu Glu Pro Ala Asp
   405 410 415
   Asp Leu Asp Gly Gly Thr Glu Glu Gln Gly Glu Gln Pro Gln Glu Met
   420 425 430
   Leu Lys Arg Met Val Val Tyr Gln Asp Ser Ile Gln Asp Lys Ile Ser
   435 440 445
   Gly Asn Met Arg Lys Ala Leu Thr Pro Thr Leu Cys Ser Pro Gln Ser
   450 455 460
   Arg Ser Trp Gly Arg Met Ser Gly Ser Tyr Ala Ser Arg Arg Arg Asp
   465 470 475 480
   Pro

<210> 560
<211> 2409
<212> DNA
<213> Homo Sapiens

<400> 560
cctggcccgg tcgcggtcgc ggctctttcc agctcctggc agccgggcac ccgaaggaac 60
gggtcgtgca acgacgcagc tggacctggc ccagccatgg accgaaaagt ggcccgagaa 120
ttccggcata aggtggattt tctgattgaa aatgatgcag agaaggacta tctctatgat 180
gtgctgcgaa tgtaccacca gaccatggac gtggccgtgc tcgtgggaga cctgaagctg 240
gtcatcaatg aacccagccg tctgcctctg tttgatgcca ttcggccgct gatcccactg 300
aagcaccagg tggaatatga tcagctgacc ccccggcgct ccaggaagct gaaggaggtg 360
cgtctggacc gtctgcaccc cgaaggcctc ggcctgagtg tgcgtggtgg cctggagttt 420
ggctgtgggc tcttcatctc ccacctcatc aaaggcggtc aggcagacag cgtcgggctc 480
caggtagggg acgagatcgt ccggatcaat ggatattcca tctcctcctg tacccatgag 540
gaggtcatca acctcattcg aaccaagaaa actgtgtcca tcaaagtgag acacatcggc 600
ctgatccccg tgaaaagctc tcctgatgag cccctcactt ggcagtatgt ggatcagttt 660
gtgtcggaat ctgggggcgt gcgaggcagc ctgggctccc ctggaaatcg ggaaaacaag 720
gagaagaagg tcttcatcag cctggtaggc tcccgaggcc ttggctgcag catttccagc 780
ggccccatcc agaagcctgg catctttatc agccatgtga aacctggctc cctgtctgct 840
gaggtgggat tggagatagg ggaccagatt gtcgaagtca atggcgtcga cttctctaac 900
ctggatcaca aggaggctgt aaatgtgctg aaaaatagcc gcagcctgac catctccatt 960
gtagctgcag ctggccggga gctgttcatg acagaccggg agcggctggc agaggcgcgg 1020
cagcgtgagc tgcagcggca ggagcttctc atgcagaagc ggctggcgat ggagtccaac 1080
aagatcctcc aggagcagca ggagatggag cggcaaagga gaaaagaaat tgcccagaag 1140

gcagcagagg aaaatgagag ataccggaag gagatggaac agattgtaga ggaggaagag 1200
aagtttaaga agcaatggga agaagactgg ggctcaaagg aacagctact cttgcctaaa 1260
accatcactg ctgaggtaca cccagtaccc cttcgcaagc caaagtatga tcagggagtg 1320
gaacctgagc tcgagcccgc agatgacctg gatggaggca cggaggagca gggagagcag 1380
acattttgcc caagcccaca gcctccacga ggccctggcg tgtccaccat ctccaaacct 1440
gtcatggtcc accaggagcc caatttcatc tacaggccag ctgtgaaatc tgaagttctg 1500
ccacaggaga tgttgaagag gatggtggtt tatcaagaca gcattcaaga caagatttcc 1560
ggaaatatga ggaaggcttt gacccctact ctatgttcac cccagagcag atcatgggga 1620
aggatgtccg gctcctacgc atcaagaagg agggatcctt agacctggcc ctggaaggcg 1680
gtgtggactc ccccattggg aaggtggtcg tttctgctgt gtatgagcgg ggagctgctg 1740
agcggcatgg tggcattgtg aaaggggacg agatcatggc aatcaacggc aagattgtga 1800
cagactacac cctggctgag gctgacgctg ccctgcagaa ggcctggaat cagggcgggg 1860
actggatcga ccttgtggtt gccgtctgcc ccccaaagga gtatgacgat gagctgacct 1920
tcttgctgaa gtccaaaagg ggaaaccaaa ttcacgcgtt aggaaacagt gagctccggc 1980
cccacctcgt gaacacaaag cctcggacca gccttgagag aggccacatg acacacacca 2040
gatggcatcc ttgggacctg aatctatcac ccaggaatct caaactccct ttggccctga 2100
accagggcca gataaggaac agctcgggcc acttttttga aggccaatgt ggaggaaagg 2160
gagcagccag ccgtttggga gaagatctca aggatccaga ctctcattcc tttcctctgg 2220
cccagtgaat ttggtctctc ccagctttgg gggactcctt ccttgaaccc taataagacc 2280
ccactggagt ctctctctct ccatccctct cctctgccct ctgctctaat tgctgccagg 2340
attgtcactc caaaccttac tctgagctca ttaataaaat aaacagattt attttccagc 2400
ttaaaaaaa 2409

<210> 561
<211> 521
<212> PRT
<213> Homo Sapiens

<400> 561
Met Asp Arg Lys Val Ala Arg Glu Phe Arg His Lys Val Asp Phe Leu
1               5                   10                  15
Ile Glu Asn Asp Ala Glu Lys Asp Tyr Leu Tyr Asp Val Leu Arg Met
            20                  25                  30
Tyr His Gln Thr Met Asp Val Ala Val Leu Val Gly Asp Leu Lys Leu
        35                  40                  45
Val Ile Asn Glu Pro Ser Arg Leu Pro Leu Phe Asp Ala Ile Arg Pro
    50                  55                  60
Leu Ile Pro Leu Lys His Gln Val Glu Tyr Asp Gln Leu Thr Pro Arg
65                  70                  75                  80
Arg Ser Arg Lys Leu Lys Glu Val Arg Leu Asp Arg Leu His Pro Glu
                85                  90                  95
Gly Leu Gly Leu Ser Val Arg Gly Gly Leu Glu Phe Gly Cys Gly Leu
            100                 105                 110
Phe Ile Ser His Leu Ile Lys Gly Gly Gln Ala Asp Ser Val Gly Leu
        115                 120                 125
Gln Val Gly Asp Glu Ile Val Arg Ile Asn Gly Tyr Ser Ile Ser Ser
    130                 135                 140
Cys Thr His Glu Glu Val Ile Asn Leu Ile Arg Thr Lys Lys Thr Val
145                 150                 155                 160
Ser Ile Lys Val Arg His Ile Gly Leu Ile Pro Val Lys Ser Ser Pro
                165                 170                 175
Asp Glu Pro Leu Thr Trp Gln Tyr Val Asp Gln Phe Val Ser Glu Ser
            180                 185                 190
Gly Gly Val Arg Gly Ser Leu Gly Ser Pro Gly Asn Arg Glu Asn Lys
        195                 200                 205

Glu Lys Lys Val Phe Ile Ser Leu Val Gly Ser Arg Gly Leu Gly Cys
    210                 215                 220
Ser Ile Ser Ser Gly Pro Ile Gln Lys Pro Gly Ile Phe Ile Ser His
225                 230                 235                 240
Val Lys Pro Gly Ser Leu Ser Ala Glu Val Gly Leu Glu Ile Gly Asp
                245                 250                 255
Gln Ile Val Glu Val Asn Gly Val Asp Phe Ser Asn Leu Asp His Lys
            260                 265                 270
Glu Ala Val Asn Val Leu Lys Asn Ser Arg Ser Leu Thr Ile Ser Ile
    275                 280                 285
Val Ala Ala Ala Gly Arg Glu Leu Phe Met Thr Asp Arg Glu Arg Leu
    290                 295                 300
Ala Glu Ala Arg Gln Arg Glu Leu Gln Arg Gln Glu Leu Leu Met Gln
305                 310                 315                 320
Lys Arg Leu Ala Met Glu Ser Asn Lys Ile Leu Gln Glu Gln Gln Glu
                325                 330                 335
Met Glu Arg Gln Arg Arg Lys Glu Ile Ala Gln Lys Ala Ala Glu Glu
            340                 345                 350
Asn Glu Arg Tyr Arg Lys Glu Met Glu Gln Ile Val Glu Glu Glu Glu
    355                 360                 365
Lys Phe Lys Lys Gln Trp Glu Glu Asp Trp Gly Ser Lys Glu Gln Leu
    370                 375                 380
Leu Leu Pro Lys Thr Ile Thr Ala Glu Val His Pro Val Pro Leu Arg
385                 390                 395                 400
Lys Pro Lys Tyr Asp Gln Gly Val Glu Pro Glu Leu Glu Pro Ala Asp
                405                 410                 415
Asp Leu Asp Gly Gly Thr Glu Glu Gln Gly Glu Gln Thr Phe Cys Pro
            420                 425                 430
Ser Pro Gln Pro Pro Arg Gly Pro Gly Val Ser Thr Ile Ser Lys Pro
    435                 440                 445
Val Met Val His Gln Glu Pro Asn Phe Ile Tyr Arg Pro Ala Val Lys
    450                 455                 460
Ser Glu Val Leu Pro Gln Glu Met Leu Lys Arg Met Val Val Tyr Gln
465                 470                 475                 480
Asp Ser Ile Gln Asp Lys Ile Ser Gly Asn Met Arg Lys Ala Leu Thr
                485                 490                 495
Pro Thr Leu Cys Ser Pro Gln Ser Arg Ser Trp Gly Arg Met Ser Gly
            500                 505                 510
Ser Tyr Ala Ser Arg Arg Arg Asp Pro
    515                 520

<210> 562
<211> 1445
<212> DNA
<213> Homo Sapiens

<400> 562
ctccggcagg gagtcctagc gcagactttg cggttcatgg agagtctctg ggagacaggc      60
acctgcggac gctgcagata agttacgacg cactgaaaga tgaaaattct aagctgagaa     120
gaaagctgaa tgaggttcag agcttctctg aagctcaaac agaaatggtg aggacgcttg     180
agcggaagtt agaagcaaaa atgatcaagg aggaaagcga ctaccacgac ctggagtcgg     240
tggttcagca ggtggagcag aacctggagc tgatgaccaa acgggctgta aaggcagaaa     300
accacgtcgt gaaactaaaa caggaaatca gtttgctcca ggcgcaggtc tccaacttcc     360
agcgagagaa tgaagccctg cggtgcggcc agggtgccag cctgaccgtg gtgaagcaga     420
acgccgacgt ggccctgcag aacctccggg tggtcatgaa cagtgcacag gcttccatca     480

agcaactggt ttccggagct gagacactga atcttgttgc cgaaatcctt aaatctatag 540
acagaatttc tgaagttaaa gacgaggagg aagactcttg aggacccctg ggtgttctca 600
gcatgaagct ccgtgtatac cctgaggtca ccaccgctcg atctaaatgt gcagttgtgt 660
ccttaaatat gcagtcttca cccagagtaa agtgttgatc gcaagagtcc agtgtcgtgc 720
cctcagccag ttcttggcca ccacaatggg agcagccctg gccgagttgt ctctgtggtt 780
tctatgcagc ccttcttggc gaaattcctg cgatcttata gattctaatg agctcttgga 840
agacattgtc ataaaagcca gtgattttaa gaaaaagagt ggttctggaa tcaatgtttt 900
ccagtcccat cccagaacat cagttgtaag ataagtacaa ttggttgtcc ttgatttcat 960
aagtagaaca aacactaaat gtgcctctga gatggccacc ccgggcaggg acctgtgcct 1020
tccgccgatg ctcagggctc cctctggctc ccgggtcact cttgtggccc cagtgggtgg 1080
tccctgcagt catggcctga gtgcgcaggg gccaccgcgt ggctgctgct gtcctcctcc 1140
ggggaccacg ggggaacaag gtcacacctt ccgtgctgtg aagctgtcca gatgtgcctc 1200
tttggctggg ggttttggtg gacgtttcaa gtggcatttt gtacaatgca ggttagaatt 1260
caggaatttc aagtatgtgc ccgggtntgt caggtcccag ttgcctttnt gacggccccc 1320
ctcagaggga cggcgatgag cactaaatgc ttttttgant attttcctat agattttttt 1380
taaaactttt ttttcctcct gttccaattg atagctttct tatttaataa attctgtagt 1440
tcacc 1445

<210> 563
<211> 192
<212> PRT
<213> Homo Sapiens

<400> 563
Pro Ala Gly Ser Pro Ser Ala Asp Phe Ala Val His Gly Glu Ser Leu
1               5                   10                  15
Gly Asp Arg His Leu Arg Thr Leu Gln Ile Ser Tyr Asp Ala Leu Lys
            20                  25                  30
Asp Glu Asn Ser Lys Leu Arg Arg Lys Leu Asn Glu Val Gln Ser Phe
        35                  40                  45
Ser Glu Ala Gln Thr Glu Met Val Arg Thr Leu Glu Arg Lys Leu Glu
    50                  55                  60
Ala Lys Met Ile Lys Glu Glu Ser Asp Tyr His Asp Leu Glu Ser Val
65                  70                  75                  80
Val Gln Gln Val Glu Gln Asn Leu Glu Leu Met Thr Lys Arg Ala Val
                85                  90                  95
Lys Ala Glu Asn His Val Val Lys Leu Lys Gln Glu Ile Ser Leu Leu
            100                 105                 110
Gln Ala Gln Val Ser Asn Phe Gln Arg Glu Asn Glu Ala Leu Arg Cys
        115                 120                 125
Gly Gln Gly Ala Ser Leu Thr Val Val Lys Gln Asn Ala Asp Val Ala
    130                 135                 140
Leu Gln Asn Leu Arg Val Val Met Asn Ser Ala Gln Ala Ser Ile Lys
145                 150                 155                 160
Gln Leu Val Ser Gly Ala Glu Thr Leu Asn Leu Val Ala Glu Ile Leu
                165                 170                 175
Lys Ser Ile Asp Arg Ile Ser Glu Val Lys Asp Glu Glu Glu Asp Ser
            180                 185                 190

<210> 564
<211> 1226
<212> DNA
<213> Homo Sapiens

<400> 564

ctgggccgcg aggcgcggag cttgggagcg gagcccaggc cgtgccgcgc ggcgccatga 60
agggcaagga ggagaaggag ggcggcgcac ggctgggcgc tggcggcgga agccccgaga 120
agagcccgag cgcgcaggag ctcaaggagc agggcaatcg tctgttcgtg ggccgaaagt 180
acccggaggc ggcggcctgc tacggccgcg cgatcacccg gaacccgctg gtggccgtgt 240
attacaccaa ccgggccttg tgctacctga agatgcagca gcacgagcag gccctggccg 300
actgccggcg cgccctggag ctggacgggc agtctgtgaa ggcgcacttc ttcctggggc 360
agtgccagct ggagatggag agctatgatg aggccatcgc caatctgcag cgagcttaca 420
gcctggccaa ggagcagcgg ctgaacttcg gggacgacat ccccagcgct cttcgaatcg 480
cgaagaagaa gcgctggaac agcattgagg agcggcgcat ccaccaggag agcgagctgc 540
actcctacct ctccaggctc attgccgcgg agcgtgagag ggagctggaa gagtgccagc 600
gaaaccacga gggtgatgag gacgacagcc acgtccgggc ccagcaggcc tgcattgagg 660
ccaagcacga caagtacatg gcggacatgg acgagctttt ttctcaggtg gatgagaaga 720
ggaagaagcg agacatcccc gactacctgt gtggcaagat cagctttgag ctgatgcggg 780
agccgtgcat cacgcccagt ggcatcacct acgaccgcaa ggacatcgag gagcacctgc 840
agcgtgtggg tcattttgac ccggtgaccg ggagccccct gacccaggaa cagttcatcc 900
ccaacttggc tatgaaggag gttattgacg cattcatctc tgagaatggc tgggtggagg 960
actactgagg ttccctgccc tacctggcgt cctggtccag gggagccctg ggcagaagcc 1020
cccggcccct aaacatagtt tatgtttttg gccaccccga ccgcttcccc caagttctgc 1080
tgttggactc tggactgttt cccctctcag catcgctttt gctgggccgt gattgtcccc 1140
tttgtgggct ggaaaagcag gtgagggtgg gctgggctga ggccattgcc gccactatct 1200
gtgtaataaa atccgtgagc acgaaa 1226

<210> 565
<211> 303
<212> PRT
<213> Homo Sapiens

<400> 565
Met Lys Gly Lys Glu Glu Lys Glu Gly Gly Ala Arg Leu Gly Ala Gly
1               5                   10                  15
Gly Gly Ser Pro Glu Lys Ser Pro Ser Ala Gln Glu Leu Lys Glu Gln
            20                  25                  30
Gly Asn Arg Leu Phe Val Gly Arg Lys Tyr Pro Glu Ala Ala Ala Cys
        35                  40                  45
Tyr Gly Arg Ala Ile Thr Arg Asn Pro Leu Val Ala Val Tyr Tyr Thr
    50                  55                  60
Asn Arg Ala Leu Cys Tyr Leu Lys Met Gln Gln His Glu Gln Ala Leu
65                  70                  75                  80
Ala Asp Cys Arg Arg Ala Leu Glu Leu Asp Gly Gln Ser Val Lys Ala
                85                  90                  95
His Phe Phe Leu Gly Gln Cys Gln Leu Glu Met Glu Ser Tyr Asp Glu
            100                 105                 110
Ala Ile Ala Asn Leu Gln Arg Ala Tyr Ser Leu Ala Lys Glu Gln Arg
        115                 120                 125
Leu Asn Phe Gly Asp Asp Ile Pro Ser Ala Leu Arg Ile Ala Lys Lys
    130                 135                 140
Lys Arg Trp Asn Ser Ile Glu Glu Arg Arg Ile His Gln Glu Ser Glu
145                 150                 155                 160
Leu His Ser Tyr Leu Ser Arg Leu Ile Ala Ala Glu Arg Glu Arg Glu
                165                 170                 175
Leu Glu Glu Cys Gln Arg Asn His Glu Gly Asp Glu Asp Asp Ser His
            180                 185                 190
Val Arg Ala Gln Gln Ala Cys Ile Glu Ala Lys His Asp Lys Tyr Met
        195                 200                 205
Ala Asp Met Asp Glu Leu Phe Ser Gln Val Asp Glu Lys Arg Lys Lys

210                 215                 220
Arg Asp Ile Pro Asp Tyr Leu Cys Gly Lys Ile Ser Phe Glu Leu Met
225                 230                 235                 240
Arg Glu Pro Cys Ile Thr Pro Ser Gly Ile Thr Tyr Asp Arg Lys Asp
                245                 250                 255
Ile Glu Glu His Leu Gln Arg Val Gly His Phe Asp Pro Val Thr Gly
            260                 265                 270
Ser Pro Leu Thr Gln Glu Gln Phe Ile Pro Asn Leu Ala Met Lys Glu
        275                 280                 285
Val Ile Asp Ala Phe Ile Ser Glu Asn Gly Trp Val Glu Asp Tyr
    290                 295                 300

<210> 566
<211> 1857
<212> DNA
<213> Homo Sapiens

<400> 566
gtgaggggct cctttgggca ggggtagtgt ttggtgtccc tgtcttgcgt gatattgaca      60
aactgaagct ttcctgcacc actggactta aggaanagtg tactcgtagg cggacagctt     120
tagtggccgg ccggccgctc tcatcccccg taaggagcag agtcctttgt actgaccaag     180
atgagcaaca tctacatcca ggagcctccc acgaatggga aggttttatt gaaaactaca     240
gctggagata ttgacataga gttgtggtcc aaagaagctc ctaaagcttg cagaaatttt     300
atcccaactt tgtttggaag cttattatga caataccatt tttcatagag ttgtgcctgg     360
tttcatagtc caaggcggag atcctactgg cacagggagt ggtggagagt ctatctatgg     420
agcgccattc aaagatgaat ttcattcacg gttgcgtttt aatcggagag gactggttgc     480
catggcaaat gctggttctc atgataatgg cacccacttt ttcttcacac tgggtcgagc     540
agatgaactt aacaataagc ataccatctt tggaaaggtt acaggggata cagtatataa     600
catgttgcga ctgtcagaag tagacattga tgatgacgaa agaccacata atccacacaa     660
aataaaaagc tgtgaggttt tgtttaatcc ttttgatgac atcattccaa gggaaattaa     720
aaggctgaaa aaagagaaac cagaggagga agtaaagaaa ttgaaaccca aaggcacaaa     780
aaattttagt ttactttcat ttggagagga agctgaggaa gaagaagagg aagtaaatcg     840
agttagtcag agcatgaagg gcaaaagcaa aagtagtcat gacttgctta aggatgatcc     900
acatctcagt tctgttccag ttgtagaaag tgaaaaaggt gatgcaccag atttagttga     960
tgatggagaa gatgaaagtg cagagcatga tgaatatatt gatggtgatg aaaagaacct    1020
gatgagagaa agaattgcca aaaaattaaa aaaggacaca agtgcgaatg ttaaatcagc    1080
tggagaagga gaagtggaga agaaatcagt cagccgcagt gaagagctca gaaaagaagc    1140
aagacaatta aaacgggaac tcttagcagc aaaacaaaaa aaagtagaaa atgcagcaaa    1200
acaagcagaa aaaagaagtg aagaggaaga agcccctcca gatggtgctg ttgccgaata    1260
cagaagagaa aagcaaaagt atgaagcttt gaggaagcaa cagtcaaaga agggaacttc    1320
ccgggaagat cagacccttg cactgctgaa ccagtttaaa tctaaactca ctcaagcaat    1380
tgctgaaaca cctgaaaatg acattcctga aacagaagta gaagatgatg aaggatggat    1440
gtcacatgta cttcagtttg aggataaaag cagaaaagtg aaagatgcaa gcatgcaaga    1500
ctcagataca tttgaaatct atgatcctcg gaatccagtg aataaaagaa ggagggaaga    1560
aagcaaaaag ctgatgagag agaaaaaaga aagaagataa aatgagaata atgataacca    1620
gaacttgctg gaaatgtgcc tacaatggcc ttgtaacagc cattgttccc aacagcatca    1680
cttaggggtg tgaaaagaag tatttttgaa cctgttgtct ggttttgaaa aacaattatc    1740
ttgttttgca aattgtggaa tgatgtaagc aaatgctttt ggttactggt acatgtgttt    1800
tttcctagct gaccttttat attgctaaat ctgaaataaa ataactttcc ttccaaa       1857

<210> 567
<211> 372
<212> PRT
<213> Homo Sapiens

<400> 567
Met Ala Asn Ala Gly Ser His Asp Asn Gly Thr His Phe Phe Phe Thr
1               5                   10                  15
Leu Gly Arg Ala Asp Glu Leu Asn Asn Lys His Thr Ile Phe Gly Lys
            20                  25                  30
Val Thr Gly Asp Thr Val Tyr Asn Met Leu Arg Leu Ser Glu Val Asp
        35                  40                  45
Ile Asp Asp Asp Glu Arg Pro His Asn Pro His Lys Ile Lys Ser Cys
    50                  55                  60
Glu Val Leu Phe Asn Pro Phe Asp Asp Ile Ile Pro Arg Glu Ile Lys
65                  70                  75                  80
Arg Leu Lys Lys Glu Lys Pro Glu Glu Glu Val Lys Lys Leu Lys Pro
                85                  90                  95
Lys Gly Thr Lys Asn Phe Ser Leu Leu Ser Phe Gly Glu Glu Ala Glu
            100                 105                 110
Glu Glu Glu Glu Glu Val Asn Arg Val Ser Gln Ser Met Lys Gly Lys
        115                 120                 125
Ser Lys Ser Ser His Asp Leu Leu Lys Asp Asp Pro His Leu Ser Ser
    130                 135                 140
Val Pro Val Val Glu Ser Glu Lys Gly Asp Ala Pro Asp Leu Val Asp
145                 150                 155                 160
Asp Gly Glu Asp Glu Ser Ala Glu His Asp Glu Tyr Ile Asp Gly Asp
                165                 170                 175
Glu Lys Asn Leu Met Arg Glu Arg Ile Ala Lys Lys Leu Lys Lys Asp
            180                 185                 190
Thr Ser Ala Asn Val Lys Ser Ala Gly Glu Gly Glu Val Glu Lys Lys
        195                 200                 205
Ser Val Ser Arg Ser Glu Glu Leu Arg Lys Glu Ala Arg Gln Leu Lys
    210                 215                 220
Arg Glu Leu Leu Ala Ala Lys Gln Lys Lys Val Glu Asn Ala Ala Lys
225                 230                 235                 240
Gln Ala Glu Lys Arg Ser Glu Glu Glu Glu Ala Pro Pro Asp Gly Ala
                245                 250                 255
Val Ala Glu Tyr Arg Arg Glu Lys Gln Lys Tyr Glu Ala Leu Arg Lys
            260                 265                 270
Gln Gln Ser Lys Lys Gly Thr Ser Arg Glu Asp Gln Thr Leu Ala Leu
        275                 280                 285
Leu Asn Gln Phe Lys Ser Lys Leu Thr Gln Ala Ile Ala Glu Thr Pro
    290                 295                 300
Glu Asn Asp Ile Pro Glu Thr Glu Val Glu Asp Asp Glu Gly Trp Met
305                 310                 315                 320
Ser His Val Leu Gln Phe Glu Asp Lys Ser Arg Lys Val Lys Asp Ala
                325                 330                 335
Ser Met Gln Asp Ser Asp Thr Phe Glu Ile Tyr Asp Pro Arg Asn Pro
            340                 345                 350
Val Asn Lys Arg Arg Arg Glu Glu Ser Lys Lys Leu Met Arg Glu Lys
        355                 360                 365
Lys Glu Arg Arg
    370

<210> 568
<211> 1537
<212> DNA
<213> Homo Sapiens

<400> 568
gccgcgcgcc gatcggtcgt taccgcgagg cgctggtggc cttcaggctg gacggcgcgg 60
gtcagccctg gttcgccggc ttctgggtct ttgaacagcc gcgatgtcga tcttcacccc 120
caccaaccag atccgcctaa ccaatgtggc cgtggtacgg atgaagcgtg ccgggaagcg 180
cttcgaaatc gcctgctaca aaaacaaggt cgtcggctgg cggagcggcg tggaaaaaga 240
cctcgatgaa gttctgcaga cccactcagt gtttgtaaat gtttctaaag gtcaggttgc 300
caaaaaggaa gatctcatca gtgcgtttgg aacagatgac caaactgaaa tctgtaagca 360
gattttgact aaaggagaag ttcaagtatc agataaagaa agacacacac aactggagca 420
gatgtttagg gacattgcaa ctattgtggc agacaaatgt gtgaatcctg aaacaaagag 480
accatacacc gtgatcctta ttgagagagc catgaaggac atccactatt cggtgaaaac 540
caacaagagt acaaaacagc aggctttgga agtgataaag cagttaaaag agaaaatgaa 600
gatagaacgt gctcacatga agcttcggtt catccttcca gtcaatgaag gcaagaactg 660
aaagaaaagc tcaagccact gatcaaggtc atagaaagtg aagattatgg ccaacagtta 720
gaaatcgtat gtctgattga cccgggctgc ttccgagaaa ttgatgagct aataaaaaag 780
gaaactaaag gcaaaggttc tttggaagta ctcaatctga aagatgtaga agaaggagat 840
gagaaatttg aatgacaccc atcaatctct tcacctctaa aacactaaag tgtttccgtt 900
tccgacggca ctgtttcatg tctgtggtct gccaaatact tgcttaaact atttgacatt 960
ttctatcttt gtgttaacag tggacacagc aaggctttcc tacataagta taataatgtg 1020
ggaatgattt ggttttaatt ataaactggg gtctaaatcc taaagcaaaa ttgaaactcc 1080
aagatgcaaa gtccagagtg gcattttgct actctgtctc atgccttgat agctttccaa 1140
aatgaaagtt acttgangca gctcttgtgg gtgaaaagtt atttgtacag tagagtaaga 1200
ttattagggg tatgtctata caacaaaagg gggggtcttt cctaaaaaag aaaacatatg 1260
atgcttcatt tctacttaat ggaacttgtg ttctgagggt cattatggta tcgtaatgta 1320
aagcttggat gatgttcctg attatttgag gaacagatat aggaaaattg tgccggaatt 1380
acctttcatt gaacatgctg ccataaatta ggttattttt ggttaaaaaa taaaagtcaa 1440
ttatttttaa tttttaaagt ttataatata tattaatata ggtaaaattg tatgtaatca 1500
ataaaaccaa ttttatgttt attaaactta aaaaaaa 1537

<210> 569
<211> 210
<212> PRT
<213> Homo Sapiens

<400> 569
Ala Ala Arg Arg Ser Val Val Thr Ala Arg Arg Trp Trp Pro Ser Gly
1               5                   10                  15
Trp Thr Ala Arg Val Ser Pro Gly Ser Pro Ala Ser Gly Ser Leu Asn
            20                  25                  30
Ser Arg Asp Val Asp Leu His Pro His Gln Pro Asp Pro Pro Asn Gln
        35                  40                  45
Cys Gly Arg Gly Thr Asp Glu Ala Cys Arg Glu Ala Leu Arg Asn Arg
    50                  55                  60
Leu Leu Gln Lys Gln Val Val Gly Trp Arg Ser Gly Val Glu Lys Asp
65                  70                  75                  80
Leu Asp Glu Val Leu Gln Thr His Ser Val Phe Val Asn Val Ser Lys
                85                  90                  95
Gly Gln Val Ala Lys Lys Glu Asp Leu Ile Ser Ala Phe Gly Thr Asp
            100                 105                 110
Asp Gln Thr Glu Ile Cys Lys Gln Ile Leu Thr Lys Gly Glu Val Gln
        115                 120                 125
Val Ser Asp Lys Glu Arg His Thr Gln Leu Glu Gln Met Phe Arg Asp
    130                 135                 140
Ile Ala Thr Ile Val Ala Asp Lys Cys Val Asn Pro Glu Thr Lys Arg
145                 150                 155                 160
Pro Tyr Thr Val Ile Leu Ile Glu Arg Ala Met Lys Asp Ile His Tyr

                165                 170                 175
Ser Val Lys Thr Asn Lys Ser Thr Lys Gln Gln Ala Leu Glu Val Ile
            180                 185                 190
Lys Gln Leu Lys Glu Lys Met Lys Ile Glu Arg Ala His Met Lys Leu
        195                 200                 205
Arg Phe
    210

<210> 570
<211> 1211
<212> DNA
<213> Homo Sapiens

<400> 570
accatctttg gaaaggttac aggggtatac agtatataac atgttgcgac tgtcagaagt      60
agacattgat gatgacgaaa gaccacataa tccacacaaa ataaaaagct gtgaggtttt     120
gtttaatcct tttgatgaca tcattccaag ggaaattaaa aggctgaaaa aagagaaacc     180
agaggaggaa gtaaagaaat tgaaacccaa aggcacaaaa aattttagtt tactttcatt     240
tggagaggaa gctgaggaag aagaggagga agtaaatcga gttagtcaga gcatgaaggg     300
caaaagcaaa agtagtcatg acttgcttaa ggatgatcca catctcagtt ctgttccagt     360
tgtagaaagt gaaaaaggtg atgcagcaga tttagttgat gatggagaag atgaaagtgc     420
agagcatgat gaatatattg atggtgatga aaagaacctg atgagagaaa gaattgccaa     480
aaaattaaaa aaggacacaa gtgcgaatgt taaatcagct ggagaaggag aagtggagaa     540
gaaatcagtc agccgcagtg aagagctcag aaaagaagca agacaattaa aacgggaact     600
cttagcagca gaacaaaaaa aagtagaaaa tgcagcaaaa caagcagaaa aaagaagtga     660
agaggaagaa gcccctccag atggtgctgt tgccgaatac agaagagaaa agcaaaagta     720
tgaagctctg aggaagcaac agtcaaagaa gggaacttcc cgggaagatc agacccttgc     780
actgctgaac cagtttaaat ctaaactcac tcaagcaatt gctgaaacgc ctgaaaatga     840
cattcctgaa acagaagtag aagatgatga aggatggatg tcacatgtac ttcagtttga     900
ggataaaagc agaaaagtga aagatgcaag catgcaagac tcagatacat ttgaaatcta     960
tgatcctcgg aatccagtga ataaaagaag gagggaagaa agcaaaaagc tgatgagaga    1020
gaaaaaagaa agaagataaa atgagaataa tgataaccag aacttgctgg aaatgtgcct    1080
acaatggcct tgtaacagcc attgttccca acagcatcac ttaggggtgt gaaaagaagt    1140
atttttgaac ctgttgtctg gttttgaaaa acaattatct tgttttgcaa attgtggaat    1200
gatgtaagca a                                                          1211

<210> 571
<211> 354
<212> PRT
<213> Homo Sapiens

<400> 571
   Pro Ser Leu Glu Arg Leu Gln Gly Tyr Thr Val Tyr Asn Met Leu Arg
   1               5                   10                  15
   Leu Ser Glu Val Asp Ile Asp Asp Asp Glu Arg Pro His Asn Pro His
               20                  25                  30
   Lys Ile Lys Ser Cys Glu Val Leu Phe Asn Pro Phe Asp Asp Ile Ile
           35                  40                  45
   Pro Arg Glu Ile Lys Arg Leu Lys Lys Glu Lys Pro Glu Glu Glu Val
       50                  55                  60
   Lys Lys Leu Lys Pro Lys Gly Thr Lys Asn Phe Ser Leu Leu Ser Phe
   65                  70                  75                  80
   Gly Glu Glu Ala Glu Glu Glu Glu Glu Glu Val Asn Arg Val Ser Gln
                   85                  90                  95
   Ser Met Lys Gly Lys Ser Lys Ser Ser His Asp Leu Leu Lys Asp Asp
                      100                 105                 110
   Pro His Leu Ser Ser Val Pro Val Val Glu Ser Glu Lys Gly Asp Ala
           115                 120                 125
   Ala Asp Leu Val Asp Asp Gly Glu Asp Glu Ser Ala Glu His Asp Glu
       130                 135                 140
   Tyr Ile Asp Gly Asp Glu Lys Asn Leu Met Arg Glu Arg Ile Ala Lys
   145                 150                 155                 160
   Lys Leu Lys Lys Asp Thr Ser Ala Asn Val Lys Ser Ala Gly Glu Gly
                   165                 170                 175
   Glu Val Glu Lys Lys Ser Val Ser Arg Ser Glu Glu Leu Arg Lys Glu
               180                 185                 190
   Ala Arg Gln Leu Lys Arg Glu Leu Leu Ala Ala Glu Gln Lys Lys Val
           195                 200                 205
   Glu Asn Ala Ala Lys Gln Ala Glu Lys Arg Ser Glu Glu Glu Glu Ala
       210                 215                 220
   Pro Pro Asp Gly Ala Val Ala Glu Tyr Arg Arg Glu Lys Gln Lys Tyr
   225                 230                 235                 240
   Glu Ala Leu Arg Lys Gln Gln Ser Lys Lys Gly Thr Ser Arg Glu Asp
                   245                 250                 255
   Gln Thr Leu Ala Leu Leu Asn Gln Phe Lys Ser Lys Leu Thr Gln Ala
               260                 265                 270
   Ile Ala Glu Thr Pro Glu Asn Asp Ile Pro Glu Thr Glu Val Glu Asp
           275                 280                 285
   Asp Glu Gly Trp Met Ser His Val Leu Gln Phe Glu Asp Lys Ser Arg
       290                 295                 300
   Lys Val Lys Asp Ala Ser Met Gln Asp Ser Asp Thr Phe Glu Ile Tyr
   305                 310                 315                 320
   Asp Pro Arg Asn Pro Val Asn Lys Arg Arg Arg Glu Glu Ser Lys Lys
                   325                 330                 335
   Leu Met Arg Glu Lys Lys Glu Arg Arg Ile Leu Pro Val Asn Glu Gly
               340                 345                 350
   Lys Asn

<210> 572
<211> 604
<212> DNA
<213> Homo Sapiens

<400> 572
ccttcggcaa aaaattttgg tcccaacttt ttgttccatt ccaaaagggc ttaccttcat 60
tccctttagc aacagggccc ccaagaagct cccgttcatt cacccttacc ttggccccca 120
ggttggaccc ccaaaggctc ccttacccca aagtgggtgg ttgaataaat cttctcagtt 180
ccctggctcc caaggcccat tgaagaagat tgtacaaggc gtgcctcaag taccccgagt 240
ggaaacagaa gcacctgcct cacttcaagc cgtggctgca cccggagcag agcccgttgc 300
cgagcctggc gctgtcggag ctgtcggtgc agcatgcgga ctcactggag aacatcgacg 360
agagcgcggt ggccgagagc agagaggagc ggatgggcgg cgcgggcggc gagggcagcg 420
acgacgacac cttcacctga gcccgcaccg cttcagggac ggagacagga ccgggcgagc 480
cctggggcgg cggccgctcc tgcactttct cccctccccc acccggcacc tggtggcacc 540
gggccaggcc caggcgggtg ctgcagcctg gctggacaga gcccaataaa cggatcccac 600
agcc 604

<210> 573
<211> 195
<212> PRT

<213> Homo Sapiens

<400> 573

<210> 574
<211> 742
<212> DNA
<213> Homo Sapiens

<400> 574
cccaccaggg ccccctcgat gcagagacag aggtcggtgc tgaccgctgc acgtcgactg      60
cctaccagga gcagaggccc caggtggagc aagttggcaa agtcgctcct ctctccccag     120
ggctgccggc aatggggggg cctggccccg gcccctgtga ggaccccgcg ggtgctgggg     180
gagcaggtgc agggggctcc gagcccctgg tgactgtcac cgtgcagtgc gccttcacag     240
tggccctgag ggcaggaaga ggagccgacc tgtccagcct gcgggcactg ctgggccaag     300
ccttccttca ccaggcccag cttgggcaat tcagttacct agccccaggt gaggacgggc     360
actgggtccc catccccgag gaggagtcgc tgcagagggc ctggcaggac gcagctgcct     420
gccccagggg gctgcagctg cagtgcaggg gagccggggg tcggccggtc ctttaccagg     480
tggtggccca gcacagatac tccgcccagg ggccagagga cctgggcttc cgacaggggg     540
acacggtgga cgtcctgtgt gaagtggacc aggcatggct ggagggccac tgtgacggcc     600
gcatcggcat cttccccaag tgcttcgtgg tccccgccgg ccctcggatg tcaggagccc     660
ccggccgcct gccccgatcc cagcagggag atcagcccta atgatgctgt gtccatgatg     720
cttttaataa aaacaacccc ca                                              742

<210> 575
<211> 232
<212> PRT
<213> Homo Sapiens

<400> 575
His Gln Gly Pro Leu Asp Ala Glu Thr Glu Val Gly Ala Asp Arg Cys
1               5                   10                  15
Thr Ser Thr Ala Tyr Gln Glu Gln Arg Pro Gln Val Glu Gln Val Gly
            20                  25                  30
Lys Val Ala Pro Leu Ser Pro Gly Leu Pro Ala Met Gly Gly Pro Gly
        35                  40                  45
Pro Gly Pro Cys Glu Asp Pro Ala Gly Ala Gly Gly Ala Gly Ala Gly
    50                  55                  60
Gly Ser Glu Pro Leu Val Thr Val Thr Val Gln Cys Ala Phe Thr Val
65                  70                  75                  80
Ala Leu Arg Ala Gly Arg Gly Ala Asp Leu Ser Ser Leu Arg Ala Leu
                85                  90                  95
Leu Gly Gln Ala Phe Leu His Gln Ala Gln Leu Gly Gln Phe Ser Tyr
            100                 105                 110
Leu Ala Pro Gly Glu Asp Gly His Trp Val Pro Ile Pro Glu Glu Glu
        115                 120                 125
Ser Leu Gln Arg Ala Trp Gln Asp Ala Ala Ala Cys Pro Arg Gly Leu
    130                 135                 140
Gln Leu Gln Cys Arg Gly Ala Gly Gly Arg Pro Val Leu Tyr Gln Val
145                 150                 155                 160
Val Ala Gln His Arg Tyr Ser Ala Gln Gly Pro Glu Asp Leu Gly Phe
                165                 170                 175
Arg Gln Gly Asp Thr Val Asp Val Leu Cys Glu Val Asp Gln Ala Trp
            180                 185                 190
Leu Glu Gly His Cys Asp Gly Arg Ile Gly Ile Phe Pro Lys Cys Phe
        195                 200                 205
Val Val Pro Ala Gly Pro Arg Met Ser Gly Ala Pro Gly Arg Leu Pro
    210                 215                 220
Arg Ser Gln Gln Gly Asp Gln Pro
225                 230

<210> 576
<211> 1087
<212> DNA
<213> Homo Sapiens

<400> 576
aagatgatgc ctagtaaatt acagaagaac aaacagagac tgcgaaacga tcctctcaat      60
caaaataagg gtaaaccaga cttgaataca acattgccaa ttagacaaac agcatcaatt     120
ttcaaacaac cggtaaccaa agtcacaaat catcctagta ataaagtgaa atcagaccca     180
caacgaatga atgaacagcc acgtcagctt ttctgggaga agaggctaca aggacttagt     240
gcatcagatg taacagaaca aattataaaa accatggaac tacccaaagg tcttcaagga     300
gttggtccag gtagcaatga tgagaccctt ttatctgctg ttgccagtgc tttgcacaca     360
agctctgcgc caatcacagg gcaagtctcc gctgctgtgg aaaagaaccc tgctgtttgg     420
cttaacacat ctcaacccct ctgcaaagct tttattgtca cagatgaaga catcaggaaa     480
caggaagagc gagtacagca agtacgcaag aaattggaag aagcactgat ggcagacatc     540
ttgtcgcgag ctgctgatac agaagagatg gatattgaaa tggacagtgg agatgaagcc     600
taagaatatg atcaggtaac tttcgaccga ctttccccaa gagaaaattc ctagaaattg     660
aacaaaaatg tttccactgg cttttgcctg taagaaaaaa aatgtacccg agcacataga     720
gctttttaat agcactaacc aatgcctttt tagatgtatt tttgatgtat atatctatta     780
ttcaaaaaat catgtttatt ttgagtccta ggacttaaaa ttagtctttt gtaatatcaa     840
gcaggaccct aagatgaagc tgagcttttg atgccaggtg caatttactg gaaatgtagc     900
acttacgtaa aacatttgtt tcccccacag ttttaataag aacagatcag gaattctaaa     960
taaatttccc agttaaagat tattgtgact tcactgtata taaacatatt tttatacttt    1020

attgaaaggg gacacctgta cattcttcca tcgtcactgt aaagacaaat aaatgattat 1080
attcaca 1087

<210> 577
<211> 200
<212> PRT
<213> Homo Sapiens

<400> 577
Lys Met Met Pro Ser Lys Leu Gln Lys Asn Lys Gln Arg Leu Arg Asn
1               5                   10                  15
Asp Pro Leu Asn Gln Asn Lys Gly Lys Pro Asp Leu Asn Thr Thr Leu
            20                  25                  30
Pro Ile Arg Gln Thr Ala Ser Ile Phe Lys Gln Pro Val Thr Lys Val
        35                  40                  45
Thr Asn His Pro Ser Asn Lys Val Lys Ser Asp Pro Gln Arg Met Asn
    50                  55                  60
Glu Gln Pro Arg Gln Leu Phe Trp Glu Lys Arg Leu Gln Gly Leu Ser
65                  70                  75                  80
Ala Ser Asp Val Thr Glu Gln Ile Ile Lys Thr Met Glu Leu Pro Lys
                85                  90                  95
Gly Leu Gln Gly Val Gly Pro Gly Ser Asn Asp Glu Thr Leu Leu Ser
            100                 105                 110
Ala Val Ala Ser Ala Leu His Thr Ser Ser Ala Pro Ile Thr Gly Gln
        115                 120                 125
Val Ser Ala Ala Val Glu Lys Asn Pro Ala Val Trp Leu Asn Thr Ser
    130                 135                 140
Gln Pro Leu Cys Lys Ala Phe Ile Val Thr Asp Glu Asp Ile Arg Lys
145                 150                 155                 160
Gln Glu Glu Arg Val Gln Gln Val Arg Lys Lys Leu Glu Glu Ala Leu
                165                 170                 175
Met Ala Asp Ile Leu Ser Arg Ala Ala Asp Thr Glu Glu Met Asp Ile
            180                 185                 190
Glu Met Asp Ser Gly Asp Glu Ala
        195                 200

<210> 578
<211> 2569
<212> DNA
<213> Homo Sapiens

<400> 578
aagagtaaaa gctactcttt cagagagaaa aataggagat tcatgtgaca aagatttgcc 60
tctgaaattt tgtgagttcc cacagaagac tataatgcct ggatttaaaa caactgtata 120
tgtttctcat ataaatgacc tttcagactt ttatgttcaa ctaatagaag atgaagctga 180
aattagtcat ctttcagaga gattaaacag tgttaaaaca aggcccgaat attatgtagg 240
tccacctttg caaagaggag atatgatatg tgctgttttc ccagaagata atttatggta 300
tcgtgctgtg atcaaggagc aacaacccaa tgaccttctc tctgtgcagt ttatagatta 360
tggcaatgtt tctgtggttc atactaacaa aataggtagg cttgaccttg ttaatgcaat 420
attgccgggg ttgtgcattc attgctcctt gcaggatttt gaggttcctg acaataaaaa 480
ttctaagaaa atgatgcatt acttttccca acggaccagc gaggctgcaa taagatgtga 540
atttgttaaa tttcaagaca gatgggaagt tattcttgct gatgaacatg ggatcatagc 600
agatgatatg attagcaggt atgctctcag tgaaaaatct caagtagaac tttctaccca 660
agtaattaaa agtgccagtt caaagtctgt taacaaatca gacattgaca cttcagtatt 720
tcttaactgg tataatccag aaaaaaaaat gataagagct tatgccactg tgatagatgg 780

acctgagtac ttttggtgtc agtttgctga tacggagaaa cttcagtgtt tagaagtaga 840
agtacagact gctggagaac aggtagcaga caggagaaat tgtatcccat gtccttatat 900
tggagatcct tgtatagtaa gatacagaga agatggacat tattataggg cacttatcac 960
taatatttgt gaagattatc ttgtatctgt caggcttgtg gactttggaa acattgaaga 1020
ctgtgtggac ccaaaagcac tctgggccat tccttctgaa cttctgtcgg ttcccatgca 1080
agcctttcca tgttgcctct cagggtttaa catttcagaa ggattatgtt ctcaagaggg 1140
aaatgactat ttctatgaaa taataacaga agatgtgttg gaaataacaa tactagaaat 1200
cagaagggat gtttgtgata tccctttagc aattgttgac ttgaaaagca aaggtaaaag 1260
tattaatgag aaaatggaga aatattctaa gactggtatt aaaagtgctc ttccctatga 1320
aaatattgac tcagagataa agcagactct tgggtcctac aatcttgatg taggacttaa 1380
gaaattaagt aataaagctg tacaaaataa aatatatatg gaacaacaga cagatgagct 1440
tgctgaaata actgaaaaag atgtaaacat tattggaacc aaaccaagta acttccgtga 1500
ccctaaaact gataacattt gtgaagggtt tgaaaacccc tgcaaagata aaattgatac 1560
tgaggaactg gaaggtgaat tagagtgcca tctggttgac aaagcagagt ttgatgataa 1620
atacctgatt acaggattta acacattact accacatgct aatgaaacaa aggagatact 1680
agaactgaat tcacttgagg tgccgctttc tcctgatgat gaatcaaaag aattcttaga 1740
actggaatct attgagttac agaattctct ggtggtggat gaagaaaaag gggagctaag 1800
cccggtgcca ccgaatgtgc cactctccca agagtgtgtc acaaaaggcg ccatggagct 1860
atttacactg cagcttcctc tcagctgtga agctgagaaa cagccagaac tagaactacc 1920
tacagcccag ctgcctttag atgacaagat ggatcctttg tctttaggag ttagtcagaa 1980
agcacaggaa tccatgtgta ctgaggacat gagaaagtca agttgtgtag aatcttttga 2040
tgaccagcgc aggatgtcat tgcatctaca tggagcagat tgtgatccta aaacacagaa 2100
tgaaatgaat atatgtgaag aagaatttgt agagtataaa aacagggatg ccatttcggc 2160
attgatgcct ttttctctga ggaagaaagc agtgatggaa gcaagcacaa taatggttta 2220
ccagatcata tttcagntca attacagaac acctacactn tgaaagcctt tactgttgga 2280
tctaaatgtg ttgtgtggtc aagtntaaga aacanatggt ctaaatgtga gattttagaa 2340
acagctgaag aaggnacaag ggttttgaac ctttcaaatg gtatggagga gatagtgaac 2400
cctgagaatg tctggaatgn nanacccaaa ttggataaga gtccacctga gaaaaggggt 2460
ttggaggtga tggagattta accgtggatn tatagctgtg gccaatcagt cagaagctgc 2520
ccntgaacaa gtggcatctt acgcagacca acagagtatt tgagaaaat 2569

<210> 579
<211> 752
<212> PRT
<213> Homo Sapiens

<400> 579
Arg Val Lys Ala Thr Leu Ser Glu Arg Lys Ile Gly Asp Ser Cys Asp
1               5                   10                  15
Lys Asp Leu Pro Leu Lys Phe Cys Glu Phe Pro Gln Lys Thr Ile Met
            20                  25                  30
Pro Gly Phe Lys Thr Thr Val Tyr Val Ser His Ile Asn Asp Leu Ser
        35                  40                  45
Asp Phe Tyr Val Gln Leu Ile Glu Asp Glu Ala Glu Ile Ser His Leu
    50                  55                  60
Ser Glu Arg Leu Asn Ser Val Lys Thr Arg Pro Glu Tyr Tyr Val Gly
65                  70                  75                  80
Pro Pro Leu Gln Arg Gly Asp Met Ile Cys Ala Val Phe Pro Glu Asp
                85                  90                  95
Asn Leu Trp Tyr Arg Ala Val Ile Lys Glu Gln Gln Pro Asn Asp Leu
            100                 105                 110
Leu Ser Val Gln Phe Ile Asp Tyr Gly Asn Val Ser Val Val His Thr
        115                 120                 125
Asn Lys Ile Gly Arg Leu Asp Leu Val Asn Ala Ile Leu Pro Gly Leu
    130                 135                 140

Cys Ile His Cys Ser Leu Gln Gly Phe Glu Val Pro Asp Asn Lys Asn
145                 150                 155                 160
Ser Lys Lys Met Met His Tyr Phe Ser Gln Arg Thr Ser Glu Ala Ala
                165                 170                 175
Ile Arg Cys Glu Phe Val Lys Phe Gln Asp Arg Trp Glu Val Ile Leu
            180                 185                 190
Ala Asp Glu His Gly Ile Ile Ala Asp Asp Met Ile Ser Arg Tyr Ala
        195                 200                 205
Leu Ser Glu Lys Ser Gln Val Glu Leu Ser Thr Gln Val Ile Lys Ser
    210                 215                 220
Ala Ser Ser Lys Ser Val Asn Lys Ser Asp Ile Asp Thr Ser Val Phe
225                 230                 235                 240
Leu Asn Trp Tyr Asn Pro Glu Lys Lys Met Ile Arg Ala Tyr Ala Thr
                245                 250                 255
Val Ile Asp Gly Pro Glu Tyr Phe Trp Cys Gln Phe Ala Asp Thr Glu
            260                 265                 270
Lys Leu Gln Cys Leu Glu Val Glu Val Gln Thr Ala Gly Glu Gln Val
        275                 280                 285
Ala Asp Arg Arg Asn Cys Ile Pro Cys Pro Tyr Ile Gly Asp Pro Cys
    290                 295                 300
Ile Val Arg Tyr Arg Glu Asp Gly His Tyr Tyr Arg Ala Leu Ile Thr
305                 310                 315                 320
Asn Ile Cys Glu Asp Tyr Leu Val Ser Val Arg Leu Val Asp Phe Gly
                325                 330                 335
Asn Ile Glu Asp Cys Val Asp Pro Lys Ala Leu Trp Ala Ile Pro Ser
            340                 345                 350
Glu Leu Leu Ser Val Pro Met Gln Ala Phe Pro Cys Cys Leu Ser Gly
        355                 360                 365
Phe Asn Ile Ser Glu Gly Leu Cys Ser Gln Glu Gly Asn Asp Tyr Phe
    370                 375                 380
Tyr Glu Ile Ile Thr Glu Asp Val Leu Glu Ile Thr Ile Leu Glu Ile
385                 390                 395                 400
Arg Arg Asp Val Cys Asp Ile Pro Leu Ala Ile Val Asp Leu Lys Ser
                405                 410                 415
Lys Gly Lys Ser Ile Asn Glu Lys Met Glu Lys Tyr Ser Lys Thr Gly
            420                 425                 430
Ile Lys Ser Ala Leu Pro Tyr Glu Asn Ile Asp Ser Glu Ile Lys Gln
        435                 440                 445
Thr Leu Gly Ser Tyr Asn Leu Asp Val Gly Leu Lys Lys Leu Ser Asn
    450                 455                 460
Lys Ala Val Gln Asn Lys Ile Tyr Met Glu Gln Gln Thr Asp Glu Leu
465                 470                 475                 480
Ala Glu Ile Thr Glu Lys Asp Val Asn Ile Ile Gly Thr Lys Pro Ser
                485                 490                 495
Asn Phe Arg Asp Pro Lys Thr Asp Asn Ile Cys Glu Gly Phe Glu Asn
            500                 505                 510
Pro Cys Lys Asp Lys Ile Asp Thr Glu Glu Leu Glu Gly Glu Leu Glu
        515                 520                 525
Cys His Leu Val Asp Lys Ala Glu Phe Asp Asp Lys Tyr Leu Ile Thr
    530                 535                 540
Gly Phe Asn Thr Leu Leu Pro His Ala Asn Glu Thr Lys Glu Ile Leu
545                 550                 555                 560
Glu Leu Asn Ser Leu Glu Val Pro Leu Ser Pro Asp Asp Glu Ser Lys
                565                 570                 575
Glu Phe Leu Glu Leu Glu Ser Ile Glu Leu Gln Asn Ser Leu Val Val

580                 585                 590
Asp Glu Glu Lys Gly Glu Leu Ser Pro Val Pro Pro Asn Val Pro Leu
        595                 600                 605
Ser Gln Glu Cys Val Thr Lys Gly Ala Met Glu Leu Phe Thr Leu Gln
    610                 615                 620
Leu Pro Leu Ser Cys Glu Ala Glu Lys Gln Pro Glu Leu Glu Leu Pro
625                 630                 635                 640
Thr Ala Gln Leu Pro Leu Asp Asp Lys Met Asp Pro Leu Ser Leu Gly
                645                 650                 655
Val Ser Gln Lys Ala Gln Glu Ser Met Cys Thr Glu Asp Met Arg Lys
            660                 665                 670
Ser Ser Cys Val Glu Ser Phe Asp Asp Gln Arg Arg Met Ser Leu His
        675                 680                 685
Leu His Gly Ala Asp Cys Asp Pro Lys Thr Gln Asn Glu Met Asn Ile
    690                 695                 700
Cys Glu Glu Glu Phe Val Glu Tyr Lys Asn Arg Asp Ala Ile Ser Ala
705                 710                 715                 720
Leu Met Pro Phe Ser Leu Arg Lys Lys Ala Val Met Glu Ala Ser Thr
                725                 730                 735
Ile Met Val Tyr Gln Ile Ile Phe Gln Asn Tyr Arg Thr Pro Thr Leu
            740                 745                 750

<210> 580
<211> 2077
<212> DNA
<213> Homo Sapiens

<400> 580
ctgttgattt tttggagaaa tatgggagaa acagtggaat atttttatga catttttagg      60
aaatcacctg gcttggttgg tagtcccaca ctgactttcc ttatgataat tctacagatg     120
gaggtgactc gagcagtgat gaggataaag aataacatga aactcctgtg gaagtagaac     180
tcatgactca ggttgaccaa gaggatatca ctcttcagag tggcagagat gaactaaatg     240
aggagctcat tcaggaagaa agctctgaag acgaaggaga atatgaagag gttagaaaag     300
atcaggattc tgttggtgaa atgaaggatg aaggggaaga gacttaaatt atcctgatac     360
taccattgac ttgtctcacc ttcaacccca aaggtccatc cagaaattgg cttcaaaaga     420
ggaatcttct aattctagtg acagtaaatc acagagccgg agacatttgt cagccaagga     480
aagaagggaa atgaaaaaga aaaaacttcc aagtgactca ggagatttag aagcgttaga     540
gggaaaggat aaagaaaaag aaagtactgt acacattgaa actcatcaga acacaagcaa     600
aaatgttgcg gctgtgcagc caatgaaacg aggacaaaag agtaaaatga aaaaaatgaa     660
agaaaaatac aaagaccagg atgaagaaga ccgtgaactt atcatgaagt tgctggggtc     720
tgcaggttca aacaaagaag aaaaagggaa gaaggggaag aaaggaaaaa caaaggacga     780
acctgtgaag aaacagcccc agaaacctag aggtggacag agggtctctg acaacattaa     840
gaaagaaact ccgttccttg aggttataac tcatgagtta caagactttg ctgtagatga     900
tccacatgat gacaaggaag agcaagatct ggatcaacag ggaaatgagg aaaacctatt     960
tgattctttg acaggccagc cacatcctga agatgtacta ctgtttgcca ttccaatatg    1020
tgccccttac accaccatga caaactacaa atataaagtg aaacttactc ctggagtgca    1080
gaaaaaggga aaagctgcaa aaacagcctt gaatagtttc atgcattcca aagaagcaac    1140
agcaagagaa aaagacttat tccgcagcgt aaaggacaca gatttatcaa gaaacattcc    1200
tggcaaagtg aaaagtgtct gcacccaatc ttctgaacgt aaaaaggaaa tagctgaaat    1260
gaaattctaa aatatttgag aagagccaat tttatagcct tttggaagtt caaagatgaa    1320
agcaccatgt atcaggattt ccgcattata aaaatgaact aaacattgcc ttgctatatt    1380
caccaaaagg acttaattct tgtttttttc ccagttttat atagaggaaa cactgtctat    1440
gataggattt ccaaaagtat ttgtggacag ttaaatgcta attatataca tctgtagtta    1500
ttctacattt tcttgaaatt tgggaggtta ataccaagta ttcatttcat gatgtaaaga    1560
aactgaacag tgaagtggct tgattgctta aactattgac ttggtaagtc tactgtatat    1620

aacatctaat atatatatta caggccaaat gaactaaaca ttgccttgct atattcacca 1680
aaaggactta attcttgttt ttttcccagt tttatataga ggaaacacta tgataggatt 1740
tcctaaagta tttgtggaca gttaaatgct aattatatac atctgtagtt attctacatt 1800
ttcttgaaat ttgagaggtt aataccaagt attcatttca tgatgtaaag aaactgaaca 1860
gtgaagtggc ttgattgctt aaactattga cttggtaagt ctactgtata taacatctaa 1920
tatatatata ttataggcca gctacaaggg gtttaaatat ttaggattgt gtcttgaaaa 1980
ctaagtattg gagtggattt tcttctgctt tcattgatac ttgtcagaaa aaaatattag 2040
accaaaatgt aaaatataag taataattct catgaaa 2077

<210> 581
<211> 312
<212> PRT
<213> Homo Sapiens

<400> 581
Arg Gly Arg Asp Leu Asn Tyr Pro Asp Thr Thr Ile Asp Leu Ser His
1               5                   10                  15
Leu Gln Pro Gln Arg Ser Ile Gln Lys Leu Ala Ser Lys Glu Glu Ser
            20                  25                  30
Ser Asn Ser Ser Asp Ser Lys Ser Gln Ser Arg Arg His Leu Ser Ala
        35                  40                  45
Lys Glu Arg Arg Glu Met Lys Lys Lys Lys Leu Pro Ser Asp Ser Gly
    50                  55                  60
Asp Leu Glu Ala Leu Glu Gly Lys Asp Lys Glu Lys Glu Ser Thr Val
65                  70                  75                  80
His Ile Glu Thr His Gln Asn Thr Ser Lys Asn Val Ala Ala Val Gln
                85                  90                  95
Pro Met Lys Arg Gly Gln Lys Ser Lys Met Lys Lys Met Lys Glu Lys
            100                 105                 110
Tyr Lys Asp Gln Asp Glu Glu Asp Arg Glu Leu Ile Met Lys Leu Leu
        115                 120                 125
Gly Ser Ala Gly Ser Asn Lys Glu Glu Lys Gly Lys Lys Gly Lys Lys
    130                 135                 140
Gly Lys Thr Lys Asp Glu Pro Val Lys Lys Gln Pro Gln Lys Pro Arg
145                 150                 155                 160
Gly Gly Gln Arg Val Ser Asp Asn Ile Lys Lys Glu Thr Pro Phe Leu
                165                 170                 175
Glu Val Ile Thr His Glu Leu Gln Asp Phe Ala Val Asp Asp Pro His
            180                 185                 190
Asp Asp Lys Glu Glu Gln Asp Leu Asp Gln Gln Gly Asn Glu Glu Asn
        195                 200                 205
Leu Phe Asp Ser Leu Thr Gly Gln Pro His Pro Glu Asp Val Leu Leu
    210                 215                 220
Phe Ala Ile Pro Ile Cys Ala Pro Tyr Thr Thr Met Thr Asn Tyr Lys
225                 230                 235                 240
Tyr Lys Val Lys Leu Thr Pro Gly Val Gln Lys Lys Gly Lys Ala Ala
                245                 250                 255
Lys Thr Ala Leu Asn Ser Phe Met His Ser Lys Glu Ala Thr Ala Arg
            260                 265                 270
Glu Lys Asp Leu Phe Arg Ser Val Lys Asp Thr Asp Leu Ser Arg Asn
        275                 280                 285
Ile Pro Gly Lys Val Lys Ser Val Cys Thr Gln Ser Ser Glu Arg Lys
    290                 295                 300
Lys Glu Ile Ala Glu Met Lys Phe
305                 310

<210> 582
<211> 3309
<212> DNA
<213> Homo Sapiens

<400> 582
cgcagaccga gacccgaggc ggaggcggac cgcgagccgg ccatgtcggt ggtggggttg 60
gacgtgggct cgcagagctg ctacatcgcg gtagcccggg ccgggggcat cgagaccatc 120
gccaatgagt tcagcgaccg gtgcaccccg tcagtcatat catttggatc aaaaaataga 180
acaatcggag ttgcagccaa aaatcagcaa atcactcatg caaacaatac ggtgtctaac 240
ttcaaaagat ttcatggccg agcattcaat gaccccttca ttcaaaagga gaaggaaaac 300
ttgagttacg atttggttcc attgaaaaat ggtggagttg gaataaaggt aatgtacatg 360
ggtgaagaac atctatttag tgtggagcag ataacagcca tgttgttgac taagctgaag 420
gaaactgctg aaaacagcct caagaaacca gtaacagatt gtgttatttc agtcccctcc 480
ttctttacag atgctgagag gcgatctgtg ttagatgctg cacagattgt tggcctaaac 540
tgtttaagac ttatgaatga catgacagct gttgctttga attacggaat ttataagcag 600
gatctcccaa gcctggatga gaaacctcgg atagtggttt ttgttgatat gggacattca 660
gcttttcaag tgtctgcttg tgcttttaac aagggaaaat tgaaggtact gggaacagct 720
tttgatcctt tcttaggagg aaaaaacttc gatgaaaagt tagtggaaca tttttgtgca 780
gaatttaaaa ctaagtacaa gttggatgca aaatccaaaa tacgagcact cctacgtctg 840
tatcaggaat gtgaaaaact gaaaaagcta atgagctcta acagcacaga ccttccactg 900
aatatcgaat gctttatgaa tgataaagat gtttccggaa agatgaacag gtcacaattt 960
gaagaactct gtgctgaact tctgcaaaag atagaagtac ccctttattc actgttggaa 1020
caaactcatc tcaaagtaga agatgtgagt gcagttgaga ttgttggagg cgctacacga 1080
attccagctg tgaaggaaag aattgccaaa ttctttggaa aagatattag cacaacactc 1140
aatgcagatg aagcagtagc cagaggatgt gcattacagt gtgcaatact ttccccggca 1200
tttaaagtta gagaattttc cgtcacagat gcagttcctt ttccaatatc tctgatctgg 1260
aaccatgatt cagaagatac tgaaggtgtt catgaagtct ttagtcgaaa ccatgctgct 1320
cctttctcca aagttctcac ctttctgaga aggggccttt ttgagctaga agctttctat 1380
tctgatcccc aaggagttcc atatccagaa gcaaaaatag gccgctttgt agttcagaat 1440
gtttctgcac agaaagatgg agaaaaatct agagtaaaag tcaaagtgcg agtcaacacc 1500
catggcattt tcaccatctc tacggcatct atggtggaga aagtcccaac tgaggagaat 1560
gaaatgtctt ctgaagctga catggagtgt ctgaatcaga gaccaccaga aaacccagac 1620
actgataaaa atgtccagca agacaacagt gaagctggaa cacagcccca ggtacaaact 1680
gatgctcaac aaacctcaca gtctccccct tcacctgaac ttacctcaga agaaaacaaa 1740
atcccagatg ctgacaaagc aaatgaaaaa aaagttgacc agcctccaga agctaaaaag 1800
cccaaaataa aggtggtgaa tgttgagctg cctattgaag ccaacttggt ctggcagtta 1860
gggaaagacc ttcttaacat gtatattgag acagagggta agatgataat gcaagataaa 1920
ttggaaaaag aaaggaatga tgctaaaaat gcagttgagg aatatgtgta tgagttcaga 1980
gacaagctgt gtggaccata tgaaaaattt atatgtgagc aggatcatca aaattttttg 2040
agactcctca cagaaactga agactggctg tatgaagaag gagaggacca agctaaacaa 2100
gcatatgttg acaagttgga agaattaatg aaaattggca ctccagttaa agttcggttt 2160
caggaagctg aagaacggcc aaaaatgttt gaagaactag gacagaggct gcagcattat 2220
gccaagatag cagctgactt cagaaataag gatgagaaat acaaccatat tgatgagtct 2280
gaaatgaaaa aagtggagaa gtctgttaat gaagtgatgg aatggatgaa taatgtcatg 2340
aatgctcagg ctaaaaagag tcttgatcag gatccagttg tacgtgctca ggaaattaaa 2400
acaaaaatca aggaattgaa caacacatgt gaacccgttg taacacaacc gaaaccaaaa 2460
attgaatcac ccaaactgga aagaactcca aatggcccaa atattgataa aaaggaagaa 2520
gatttagaag acaaaaacaa ttttggtgct gaacctccac atcagaatgg tgaatgttac 2580
cctaatgaga aaaattctgt taatatggac ttggactaga taaccttaaa ttggcctatt 2640
ccttcaatta ataaaatatt tttgccatag tatgtgactc tacataacat actgaaacta 2700
tttatatttt cttttttaag gatatttaga aattttgtgt attatatgga aaaagaaaaa 2760
aagcttaagt ctgtagtctt tatgatccta aaagggaaaa ttgccttggt aactttcaga 2820
ttcctgtgga attgtgaatt catactaagc tttctgtgca gtctcaccat ttgcatcact 2880
gaggatgaaa ctgacttttg tcttttggag aaaaaaaact gtactgcttg ttcaagaggg 2940

ctgtgattaa aatctttaag catttgttcc tgccaaggta gttttcttgc attttgctct 3000
ccattcagca tgtgtgtggg tgtggatgtt tataaacaag actaagtctg acttcataag 3060
ggctttctaa aaccatttct gtccaagaga aaatgacttt ttgctttgat attaaaaatt 3120
caatgagtaa aacaaaagct agtcaaatgt gttagcagca tgcagaacaa aaactttaaa 3180
ctttctctct cactatacag tatattgtca tgtgaaagtg tggaatggaa gaaatgtcga 3240
tcctgttgta actgattgtg aacactttta tgagctttaa aataaagttc atcttatggt 3300
gtcatttct 3309

<210> 583
<211> 872
<212> PRT
<213> Homo Sapiens

<400> 583
Arg Arg Pro Arg Pro Glu Ala Glu Ala Asp Arg Glu Pro Ala Met Ser
1               5                   10                  15
Val Val Gly Leu Asp Val Gly Ser Gln Ser Cys Tyr Ile Ala Val Ala
            20                  25                  30
Arg Ala Gly Gly Ile Glu Thr Ile Ala Asn Glu Phe Ser Asp Arg Cys
        35                  40                  45
Thr Pro Ser Val Ile Ser Phe Gly Ser Lys Asn Arg Thr Ile Gly Val
    50                  55                  60
Ala Ala Lys Asn Gln Gln Ile Thr His Ala Asn Asn Thr Val Ser Asn
65                  70                  75                  80
Phe Lys Arg Phe His Gly Arg Ala Phe Asn Asp Pro Phe Ile Gln Lys
                85                  90                  95
Glu Lys Glu Asn Leu Ser Tyr Asp Leu Val Pro Leu Lys Asn Gly Gly
            100                 105                 110
Val Gly Ile Lys Val Met Tyr Met Gly Glu Glu His Leu Phe Ser Val
        115                 120                 125
Glu Gln Ile Thr Ala Met Leu Leu Thr Lys Leu Lys Glu Thr Ala Glu
    130                 135                 140
Asn Ser Leu Lys Lys Pro Val Thr Asp Cys Val Ile Ser Val Pro Ser
145                 150                 155                 160
Phe Phe Thr Asp Ala Glu Arg Arg Ser Val Leu Asp Ala Ala Gln Ile
                165                 170                 175
Val Gly Leu Asn Cys Leu Arg Leu Met Asn Asp Met Thr Ala Val Ala
            180                 185                 190
Leu Asn Tyr Gly Ile Tyr Lys Gln Asp Leu Pro Ser Leu Asp Glu Lys
        195                 200                 205
Pro Arg Ile Val Val Phe Val Asp Met Gly His Ser Ala Phe Gln Val
    210                 215                 220
Ser Ala Cys Ala Phe Asn Lys Gly Lys Leu Lys Val Leu Gly Thr Ala
225                 230                 235                 240
Phe Asp Pro Phe Leu Gly Gly Lys Asn Phe Asp Glu Lys Leu Val Glu
                245                 250                 255
His Phe Cys Ala Glu Phe Lys Thr Lys Tyr Lys Leu Asp Ala Lys Ser
            260                 265                 270
Lys Ile Arg Ala Leu Leu Arg Leu Tyr Gln Glu Cys Glu Lys Leu Lys
        275                 280                 285
Lys Leu Met Ser Ser Asn Ser Thr Asp Leu Pro Leu Asn Ile Glu Cys
    290                 295                 300
Phe Met Asn Asp Lys Asp Val Ser Gly Lys Met Asn Arg Ser Gln Phe
305                 310                 315                 320
Glu Glu Leu Cys Ala Glu Leu Leu Gln Lys Ile Glu Val Pro Leu Tyr

                325                 330                 335
Ser Leu Leu Glu Gln Thr His Leu Lys Val Glu Asp Val Ser Ala Val
            340                 345                 350
Glu Ile Val Gly Gly Ala Thr Arg Ile Pro Ala Val Lys Glu Arg Ile
        355                 360                 365
Ala Lys Phe Phe Gly Lys Asp Ile Ser Thr Thr Leu Asn Ala Asp Glu
    370                 375                 380
Ala Val Ala Arg Gly Cys Ala Leu Gln Cys Ala Ile Leu Ser Pro Ala
385                 390                 395                 400
Phe Lys Val Arg Glu Phe Ser Val Thr Asp Ala Val Pro Phe Pro Ile
                405                 410                 415
Ser Leu Ile Trp Asn His Asp Ser Glu Asp Thr Glu Gly Val His Glu
            420                 425                 430
Val Phe Ser Arg Asn His Ala Ala Pro Phe Ser Lys Val Leu Thr Phe
        435                 440                 445
Leu Arg Arg Gly Pro Phe Glu Leu Glu Ala Phe Tyr Ser Asp Pro Gln
    450                 455                 460
Gly Val Pro Tyr Pro Glu Ala Lys Ile Gly Arg Phe Val Val Gln Asn
465                 470                 475                 480
Val Ser Ala Gln Lys Asp Gly Glu Lys Ser Arg Val Lys Val Lys Val
                485                 490                 495
Arg Val Asn Thr His Gly Ile Phe Thr Ile Ser Thr Ala Ser Met Val
            500                 505                 510
Glu Lys Val Pro Thr Glu Glu Asn Glu Met Ser Ser Glu Ala Asp Met
        515                 520                 525
Glu Cys Leu Asn Gln Arg Pro Pro Glu Asn Pro Asp Thr Asp Lys Asn
    530                 535                 540
Val Gln Gln Asp Asn Ser Glu Ala Gly Thr Gln Pro Gln Val Gln Thr
545                 550                 555                 560
Asp Ala Gln Gln Thr Ser Gln Ser Pro Pro Ser Pro Glu Leu Thr Ser
                565                 570                 575
Glu Glu Asn Lys Ile Pro Asp Ala Asp Lys Ala Asn Glu Lys Lys Val
            580                 585                 590
Asp Gln Pro Pro Glu Ala Lys Lys Pro Lys Ile Lys Val Val Asn Val
        595                 600                 605
Glu Leu Pro Ile Glu Ala Asn Leu Val Trp Gln Leu Gly Lys Asp Leu
    610                 615                 620
Leu Asn Met Tyr Ile Glu Thr Glu Gly Lys Met Ile Met Gln Asp Lys
625                 630                 635                 640
Leu Glu Lys Glu Arg Asn Asp Ala Lys Asn Ala Val Glu Glu Tyr Val
                645                 650                 655
Tyr Glu Phe Arg Asp Lys Leu Cys Gly Pro Tyr Glu Lys Phe Ile Cys
            660                 665                 670
Glu Gln Asp His Gln Asn Phe Leu Arg Leu Leu Thr Glu Thr Glu Asp
        675                 680                 685
Trp Leu Tyr Glu Glu Gly Glu Asp Gln Ala Lys Gln Ala Tyr Val Asp
    690                 695                 700
Lys Leu Glu Glu Leu Met Lys Ile Gly Thr Pro Val Lys Val Arg Phe
705                 710                 715                 720
Gln Glu Ala Glu Glu Arg Pro Lys Met Phe Glu Glu Leu Gly Gln Arg
                725                 730                 735
Leu Gln His Tyr Ala Lys Ile Ala Ala Asp Phe Arg Asn Lys Asp Glu
            740                 745                 750
Lys Tyr Asn His Ile Asp Glu Ser Glu Met Lys Lys Val Glu Lys Ser
        755                 760                 765

Val Asn Glu Val Met Glu Trp Met Asn Asn Val Met Asn Ala Gln Ala
    770                 775                 780
Lys Lys Ser Leu Asp Gln Asp Pro Val Val Arg Ala Gln Glu Ile Lys
785                 790                 795                 800
Thr Lys Ile Lys Glu Leu Asn Asn Thr Cys Glu Pro Val Val Thr Gln
                805                 810                 815
Pro Lys Pro Lys Ile Glu Ser Pro Lys Leu Glu Arg Thr Pro Asn Gly
            820                 825                 830
Pro Asn Ile Asp Lys Lys Glu Glu Asp Leu Glu Asp Lys Asn Asn Phe
        835                 840                 845
Gly Ala Glu Pro Pro His Gln Asn Gly Glu Cys Tyr Pro Asn Glu Lys
    850                 855                 860
Asn Ser Val Asn Met Asp Leu Asp
865                 870

<210> 584
<211> 2918
<212> DNA
<213> Homo Sapiens

<400> 584
ataactggag ctcgcgcgcc tgcaggtcga cactagtgga tccaaagaat tcggcacgag      60
gtgacgacaa cagggacaag gactccgaga agaccaagag gtggtccaag cccaggaagc     120
gctccctgat ggagatggag gggaaggagg atgcccttta aggtgctgaa gtgcatgtac     180
tgtggacact cctttgagtc cttgcaggac ctcagcgtcc acatgatcaa aaccaagcat     240
taccagaaag tgcctctgaa ggagccagtg ccagccatca ccaaactggt cccctccacc     300
aaaaagcggg cgcttcagga cctggcgccc ccctgctccc ctgagccagc aggaatggcc     360
gcagaggtgg ccctgagtga gtcagccaag gatcagaaag cagcgaaccc gtacgtcacg     420
cccaataacc gctatggcta ccagaatggc gccagctaca cctggcagtt tgaggcccgc     480
aaggcgcaga tcctcaagtg catggagtgt ggcagctccc acgacacgct gcagcagctc     540
accgcccaca tgatggtcac cgggcacttc ctgaaagtga ccacctcggc ttctaagaag     600
ggcaagcagt tggtgctgga ccctgtggtg gaagagaaga tccagtccat cccactaccg     660
cccaccaccc acacgcggct gccggcctcc agcatcaaaa agcagcccga ctctcccgcg     720
gggtccacga cttctgaaga aaagaaagag ccagagaagg agaagccgcc tgtggctggc     780
gacgcggaga agatcaagga ggagagtgag gacagcttgg agaaatttga gcccagcacc     840
ctgtacccgt acctgcgtga ggaggacctg gacgacagcc ccaagggagg gctggacatt     900
ctcaagtccc tggagaatac cgtctccacg gccattagca aagctcagaa tggtgcgccc     960
tcatggggtg gctaccccag catccatgca gcctaccagc tcccgggcac cgtgaagcca    1020
ctgccggcgg ccgtgcagag cgtgcaggtg cagccgtcct atgctggcgg cgtgaagtcg    1080
ctgtcttccg ccgagcacaa cgccctcctg cactccccag ggagcctcac gcccccaccg    1140
cacaagagca acgtgtctgc catggaggag ctggtggaga aggtcacggg caaggtcaac    1200
atcaagaagg aggagagacc ccctgagaag gagaagagct ccctggccaa ggctgcgtcc    1260
cccatagcaa aagagaataa agatttcccg aaaacggagg aagtcagcgg caaaccacag    1320
aagaagggcc ctgaggccga gacttgggaa gccaaaaagg agggaccgct ggacgttcac    1380
accccaaatg gcacagagcc tctcaaagca aaggtcacca acggctgtaa caacctgggg    1440
atcatcatgg accactcacc ggagccttcc ttcatcaacc cgctgagcgc tttgcagtcc    1500
atcatgaaca cccacctggg caaggtgtcc aagcccgtga gtccctcgct ggacccgctg    1560
gcgatgctgt acaagatcag caacagcatg ctggacaagc cggtgtaccc cgccacccct    1620
gtgaagcagg ccgatgccat cgaccgctac tattatgaaa acagcgacca gcccattgac    1680
ttaaccaagt ccaagaacaa gccgctggtg tccagcgtgg ctgattcggt ggcatcacct    1740
ctgcgggaga gcgcactcat ggacatctcc gacatggtga aaaacctcac aggccgcctg    1800
acgcccaagt cctccacgcc ctccacagtt tcagagaagt ccgatgctga tggcagcagc    1860
tttgaggagg cgttggacga gctgtcaccg gtccacaaga ggaagggccg gcagtccaac    1920
tggaacccgc agcaccttct catcctgcag gcccagttcg cctcgagctt gcgggagacc    1980
acagaaggca agtacatcat gtcggacttg ggcccgcagg agagggtgca catctcgaag    2040

tttactgggc tctccatgac caccatcagc cactggctgg ccaatgtgaa gtaccagttg 2100
aggaggacag ggggaacgaa attcctaaag aacctggaca cagggcatcc tgttttcttt 2160
tgcaacgatt gtgcctctca gttcagaact gcttctacat acataagtca tttggagaca 2220
cacttgggct tcagcctgaa ggatctctcc aagctgccac tcaatcagat tcaagaacag 2280
cagaatgttt cgaaagtcct caccaacaaa actctgggcc cactgggggc caccgaggaa 2340
gacttgggct ccacattcca atgtaagctc tgcaaccgga cttttgcgaa gcaagcacgc 2400
agtcaaactg caccttagta agacccacgg caagtctccc gaggaccacc tgatctatgt 2460
gactgagttg gagaaacagt agcgtccagg tatgcaagag accgcggaac attgcactaa 2520
acgtcgtcga gctgcactag gcatggcctg agcctctgaa atcagtcttt cctttgttgc 2580
tggcccgcct ctctggacct tggttttcct acacatattt tgtatattta tatgctttct 2640
gtccgatctg tgcatgttat ttttcttttt ccgtgagtca aagtctgacc tttattttca 2700
acatctgttt ttggtgttaa gctatctttt gtaggaaata gtggggcaca ctactcagag 2760
acattattta gcagtaaaga aagacacaaa taacaatgat aaaaagacat cctaaaatgg 2820
tgaagttgcc atgacaataa aggtcataga acctggtagt gtcaaattta accctttgag 2880
gactgtaatt gcatttctgt gcctttcact tgaaaaaa 2918

<210> 585
<211> 687
<212> PRT
<213> Homo Sapiens

<400> 585

<210> 586

<211> 1898
<212> DNA
<213> Homo Sapiens

<400> 586
ccgccttggg tcagcctgct cccctgcttc ctgccgcagt gggggccgtc agcctggcca 60
cctcccagct cccaagccca cccctggggc ccaccgtccc cccacagcca ccctcggccc 120
tggagtcgga tggggaaggg ccgcccccca gggtgggctt tgtggacagc accatcaaga 180
gcctggacga naagctgcgg actctgctct accaggagca cgtgcccacc tcctcagcct 240
cagctgggac ccctgtggag gtgggcgaca ganacttcac cctggagccc ctgagagggg 300
accagccccg ctcanaggtc tgcggggggg acctggccct gcccccagtg cctaaggagg 360
cggtctcagg gcgtgtccag ctgccccagc ccttggtgga gaagtcagaa ctggccccca 420
ctcgaggggc cgtgatggag cagggcacgt cctcgtcaat gacagagtcg tctcccagga 480
gtatgctagg ctatgacaga gatggaaggc aggtggcctc agactcccat gtggtcccca 540
gcgtccccca ggatgtacct gcttttgtga gacctgcacg tgtgganccc acanacaggg 600
atggtggana agctgganaa agctcggcan agcccccgcc gagtgacatg ggcanngtgg 660
ggggccaggc tagccacccc cagacactcg gcnctcgagc tttggggtcc cctcggaanc 720
gtccagatca ccaggatgtc agctcaccag ccaagactgt gggccgtttc tcggtggtca 780
gcactcagga cgagtggacc ctggcctccc cccacagcct gagatactct gccccacccg 840
acgtctacct ggacgaggcc ccctccagcc ccgacgtgaa gctggcagtg cggcgggcgc 900
agacggcctc ctccatcgag gtcggcgtgg gcgagcccgt gtccagcgac tctggggacg 960
agggccctcg ggcgagaccc ccggtgcaga agcaggcgtc cctgcccgtg agtggcagcg 1020
tggctggcga cttcgtgaag aaggccaccg cttcctgcag aggccttctc gggccggctt 1080
cgctgggccc cgagacaccc agcagggtgg gcatgaaggt ccccacgatc agcgtgacct 1140
ccttccattc ccagtcgtcc tacatcagca gcgacaatga ttcggagctc gaggatgctg 1200
acataaagaa ggagctgcan agtctgcggg agaagcacct gaaggagatc tcggagctgc 1260
agagccagca gaagcaggag atcgaagctc tgtnccgccg cctgggcaag ccactgcccc 1320
ccaacgtggg cttcttccac acggcacccc ccactggccg ccgganaaaa accancaaga 1380
ncaagctgaa ngcaggcaag ctgctaaatc ccctggtgcg gcagctcaag gtcgtggcct 1440
ccaacacagg tcacttggct gactccanca naagccctcc cgctaangac ctgcccnagc 1500
cagtgtgggg ctcactgcan acaacacggg cctgaacggg aangcagtgc anaccancan 1560
ccctgctccg tccggggctc cctgtcttcn gacatctgct ccggcttacc antgatggaa 1620
gcggaacgcg tngncaangg tcctccacca acaacctggc ccaggcctga accaagcccc 1680
acccgccctg cacgtccaag cgcangtgaa caacancaac nacaagaaag gttcttcncc 1740
gacgaactgc acaanctggt ggacnaatgg acaacaanan ngtggggggc gcgcactgaa 1800
acccacnctc nacccctnaa ncnnaaccnc aacttccana cattgaggcc cgcaggtggg 1860
ctgccctggc naagcccggc tttnaccccc ctccaaca 1898

<210> 587
<211> 399
<212> PRT
<213> Homo Sapiens

<400> 587
Ala Leu Gly Gln Pro Ala Pro Leu Leu Pro Ala Ala Val Gly Ala Val
1               5                   10                  15
Ser Leu Ala Thr Ser Gln Leu Pro Ser Pro Pro Leu Gly Pro Thr Val
            20                  25                  30
Pro Pro Gln Pro Pro Ser Ala Leu Glu Ser Asp Gly Glu Gly Pro Pro
        35                  40                  45
Pro Arg Val Gly Phe Val Asp Ser Thr Ile Lys Ser Leu Asp Lys Leu
    50                  55                  60
Arg Thr Leu Leu Tyr Gln Glu His Val Pro Thr Ser Ser Ala Ser Ala
65                  70                  75                  80
Gly Thr Pro Val Glu Val Gly Asp Arg Phe Thr Leu Glu Pro Leu Arg

                85                  90                  95
Gly Asp Gln Pro Arg Ser Val Cys Gly Gly Asp Leu Ala Leu Pro Pro
            100                 105                 110
Val Pro Lys Glu Ala Val Ser Gly Arg Val Gln Leu Pro Gln Pro Leu
        115                 120                 125
Val Glu Lys Ser Glu Leu Ala Pro Thr Arg Gly Ala Val Met Glu Gln
    130                 135                 140
Gly Thr Ser Ser Ser Met Thr Glu Ser Ser Pro Arg Ser Met Leu Gly
145                 150                 155                 160
Tyr Asp Arg Asp Gly Arg Gln Val Ala Ser Asp Ser His Val Val Pro
                165                 170                 175
Ser Val Pro Gln Asp Val Pro Ala Phe Val Arg Pro Ala Arg Val Pro
            180                 185                 190
Thr Arg Asp Gly Gly Ala Gly Ser Ser Ala Pro Pro Pro Ser Asp Met
        195                 200                 205
Gly Val Gly Gly Gln Ala Ser His Pro Gln Thr Leu Gly Arg Ala Leu
    210                 215                 220
Gly Ser Pro Arg Arg Pro Asp His Gln Asp Val Ser Ser Pro Ala Lys
225                 230                 235                 240
Thr Val Gly Arg Phe Ser Val Val Ser Thr Gln Asp Glu Trp Thr Leu
                245                 250                 255
Ala Ser Pro His Ser Leu Arg Tyr Ser Ala Pro Pro Asp Val Tyr Leu
            260                 265                 270
Asp Glu Ala Pro Ser Ser Pro Asp Val Lys Leu Ala Val Arg Arg Ala
        275                 280                 285
Gln Thr Ala Ser Ser Ile Glu Val Gly Val Gly Glu Pro Val Ser Ser
    290                 295                 300
Asp Ser Gly Asp Glu Gly Pro Arg Ala Arg Pro Pro Val Gln Lys Gln
305                 310                 315                 320
Ala Ser Leu Pro Val Ser Gly Ser Val Ala Gly Asp Phe Val Lys Lys
                325                 330                 335
Ala Thr Ala Ser Cys Arg Gly Leu Leu Gly Pro Ala Ser Leu Gly Pro
            340                 345                 350
Glu Thr Pro Ser Arg Val Gly Met Lys Val Pro Thr Ile Ser Val Thr
        355                 360                 365
Ser Phe His Ser Gln Ser Ser Tyr Ile Ser Ser Asp Asn Asp Ser Glu
    370                 375                 380
Leu Glu Asp Ala Asp Ile Lys Lys Glu Leu Ser Leu Arg Glu Lys
385                 390                 395

<210> 588
<211> 707
<212> DNA
<213> Homo Sapiens

<400> 588
agatggcgcc tgttgtgaca gggaaatttg gtgagcggcc tccacctaaa cgacttacta 60
gggaagctat gcgaaattat ttaaaagagc gaggggatca aacagtnctt attcttcatg 120
caaaagttgc acagaagtca tatggaaatg aaanaaggtt tttttgccca cctccttgtg 180
tatatcttat gggcantgga tggaagaaaa aaaangaaca aatggaacgc gatggttgtt 240
ctgaacaaaa gtctcaaccg tgtgcattta ttgggatagg aaatagtgac caaaaaatgc 300
agcagctana cttggaagga aagaactatt gcacagccaa aacattgtat atatctgact 360
cagacaagcg aaagcacttc atgttgtctg taaagatgtt ctatggcaac agtgatgaca 420
ttggtgtgtt cctcagcaag cgaataaaag tcntctccaa accttccaaa aagaacagtc 480
attgaaaaat gctgacttat gcattgcctc angaacaaag gtggctctgt ttaatcgact 540

acnatcccan acagttagta ccagatactt gcatgttana aggaggtnat tttcatgcca 600
gttcacagcn gtggggagcc ttttttattc anctcttgga tgatgatgan tccnaaggag 660
aagaattcac ngtccgagat ggctacatcc attatggaca aacagtc 707

<210> 589
<211> 551
<212> DNA
<213> Homo Sapiens

<400> 589
actgtggctt ctgcatttca aatcagcact tgcagggaga caacggggtt tttgaatagt 60
atcacctggt atgaaaagtt ttcccaagaa accacaaacg attgttcatt ttttctcctt 120
ttttgttaac tttttgccac actcaagtca gtttaagtcc tagcaaaaag acggtagtta 180
ggataccact gtggctgtan atgatgtgac actggttgaa tttgtgctgg cgtttgtgta 240
acttccctcg ctgtttgtgt ttgattcgtt agggggcacc tggcttgaat tggctcgaag 300
gattgctcct gctgcactgc aatgtggccg cggccctggt tctggtgtgt angtaaaggt 360
aaggctggtg gaataaatga ttccaccatt tcggaccaaa gttactggaa cctggactgg 420
ttgccggacc catctccaac cttctcggaa tgcanaaatg tctgggacga cacagaacat 480
acctctccac acctgtacat aatttcagct tctacatccc caaaccacac tcgtaaattt 540
ggantnaaaa t 551

<210> 590
<211> 478
<212> DNA
<213> Homo Sapiens

<400> 590
actgtggctt ctgcatttca aatcagcact tgcagggana caacggggtt tttgaatagt 60
atcacctggt atgaaaagtt ttcccaagaa accacaaacn attgttcatt ttttctcctt 120
ttttgttaac ttttngccac actcaantca gtttaagtcc tagcaaaaan acggtagtta 180
ggataccact gtggctgtaa atgatntgac actggttgaa tttgtgctgg cgtttgtgta 240
acttccctcg ctgtttgtgt ttgattcgtn agggggcacc tggcttgaat tggctcgaag 300
gattgctcct gctgcactgc aatgtggccg cgggcctgnt tcttatntgt tgtaaangtn 360
aggntggtgg aataaatgat tccatcatnt cggancgaag ttgctgggaa ctggganngg 420
tngncggaac catctccgac cncccggaaa ngcagaagtg ttngtggnag accggaac 478

<210> 591
<211> 707
<212> DNA
<213> Homo Sapiens

<400> 591
actgtggctt ctgcatttca aatcagcact tgcagggaga caacggggtt tttgaatagt 60
atcacctggt atgaaaagtt ttcccaanaa accacaaacg attgttcatt ttttctcctt 120
ttttgttaac tttttgccac actcaantca gtttaantcc tancaaaaag acggtagtta 180
ggataccact gtggctgtaa atgatgtgac actggttgaa tttgtgctgg cgtttgtgta 240
acttccctcg ctgtttgtgt ttgattcgtt agggggcacc tggcttgaat tggctcgaag 300
gattgctcct gctgcactgc aatgtggccg cggccctggt tctggtgtgt aggtaaaggt 360
aaggctggtg gaataaatga ttccatcatt tcggaccaaa gttactggaa cctggactgg 420
ttgccggacc catctccaac cttctcggaa tgcagaaatg tctgggacga cacagancat 480
actctctcca cacctgtaca tagtttcngc ttctacatcc ccaaaccaca ctcgtaaatt 540
tggantgaaa ttctgtcctg taagttcaag cattnctacg tccccacccg ccatttcaac 600
tgaaaggctc tctaccacan ggnacaggaa atgactgggg caaggacagg gcccattccc 660
tcattaaatg tnatactccg ccttatcngt cctaaangaa tgtncaa 707

<210> 592
<211> 541
<212> DNA
<213> Homo Sapiens

<400> 592
ggtaaacttt tggccacncn caattcantt taattcctac caaaaaaacg gtatttagna 60
tnccnctgtg gctgtaaata atttaacnct ggttaaattn ntnctggctt tngtntanct 120
cccccccctn ttngtttttn atccnttagg gggcacctgn cttnantngg cncaaaggat 180
ngcccctgct gcantgcaat ttggccncgg ccctggtcct ggtttntagg taaaggtaag 240
gcnggtgnaa taantaatcc caccattncg naccaaattt actgnaacct gaacnggttg 300
ccgnacccan cnccancctn cncgaaatgc aaaantttct ggnacaacnc aaaccntacn 360
cncnccaccc ctntncntat ttncagctnc tacntcccca aaccacacnc ntaaattngn 420
attaaaatcc tntcctgtaa ttccaagcat ggctacttcc ccaccgccat tcaactnaag 480
gcccnctacc acaggcncag nattaantgg ggcaaggaaa gggcccatcc ccccataaaa 540
t 541

<210> 593
<211> 605
<212> DNA
<213> Homo Sapiens

<400> 593
actgtggctt ctgcatttca aatcagcact tgcagggana caacggggtt tttgaatant 60
atcacctggt atgaaaagtt ttcccaanaa accacaaacn antgttcatt tttnctcctt 120
ttttgttaac tttttgccac actcaantca gtttaantcc tagcaaaaaa acggtagtta 180
ggataccact gtggctgtaa atgatgtnac actggttgaa tttgtgctgg cgtttgtgtn 240
acttccctcg ctgtttgtgt ttgattcgtt agggggcacc tggcttgaat tggctcgaan 300
gattgctcct gctgcactgc aatgtggccg cggccctggt tctggtgtgt aagtaaaggt 360
aaggctggtg gaataaatga ttccntcatt tcggancaaa gttactggaa cctggantgg 420
ttgncggacc atctccaacc ttctcggaat gcanaaatgt ctgggacaan acnnaacata 480
ctctctccnc acctggttca tantttcagc ttctacatcc cccaaaccac actcntaaat 540
ttggantgaa attctgtcct gttaattcaa acattgctac gtccccnccg ccattcaact 600
gaaag 605

<210> 594
<211> 666
<212> DNA
<213> Homo Sapiens

<400> 594
gaagagtttg tggaagatgg cgcctgttgt gacagggaaa tttggtgagc ggcctccacc 60
taaacgactt actagggaag ctatgcgaaa ttatttaaaa gagcgagggg atcaaacagt 120
acttattctt catgcaaaag ttgcacagaa gtcatatgga aatgaaaaaa ggtttttttg 180
cccacctcct tgtgtatatc ttatgggcag tggatggaag aaaaaaaaag aacaaatgga 240
acgcgatggt tgttctgaac aagagtctca accgtgtgca tttattggga taggaaatag 300
tgaccaagaa atgcagcagc taaacttgga aggaaagaac tattgcacag ccaaaacatt 360
gtatatatct gactcagaca agcgaaagca cttcatgttg tctgtaaaga tgttctatgg 420
caacagtgat gacattggtg tgttcctcan caagcggata aaagtcatct ccaaaccttc 480
caaaaagaac agtcattgaa aaatgctgac ttatgcattg cctcaggaac aaaggtggct 540
ctgtttaatc gactacgatc ccagacagtt ngtaccagat acttgcatgt anaaggaggt 600
aattttccat gccagttccc accagtgggg agcctttttt attcnctctt gggatgatga 660
tgaatc 666

<210> 595

<211> 600
<212> DNA
<213> Homo Sapiens

<400> 595
gccacactca agtcagttta agtcctagca aaaagacggt agttaggata ccactgtggc 60
tgtanatgat gtgacactgg ttgaatttgt gctggcgttt gtgtaacttc cctcgctgtt 120
tgtgtttgat tcgttagggg gcacctggct tgaattggct cgaaggattg ctcctgctgc 180
actgcaatgt ggccgcggcc ctggttctgg tgtgtaggta aaggtaaggc tggtggaata 240
aatgattcca tcatttcgga ccaaagttac tggaacctgg actggttgcc ggacccatct 300
ccaaccttct cggaatgcag aaatgtctgg gacgacacag ancatactct ctccacacct 360
gtacatagtt tcagcttcta catccccaaa ccacactcgt aaatttggag tgaaattctg 420
tcctgtaagt tcaagcattg ctacgtcccc accgccattc aactgaaggc tctctaccac 480
aggcacagga atgactgggg caaggacagg gcccattccc tncataaaat gtntaatttg 540
gggncaantg tggcccccaa ccccccccca aagggcatna tttaacnccn ctttaattgg 600

<210> 596
<211> 835
<212> DNA
<213> Homo Sapiens

<400> 596
actgtggctt ctgcatttca aatcagcact tgcagggaga caacggggtt tttgaatagt 60
atcacctggt atgaaaagtt ttcccaanaa accacaaacn attgttcatt ttttctcctt 120
ttttgttaac tttttgccac actcaantca gtttaagtcc tagcaaaaan acggtagtta 180
ggataccact gtggctgtaa atnatgtgac actggttgaa tttgtgctgg cgtttgtgta 240
acttccctcg ctgtttgtgt ttgattcgtt agggggcacc tggcttgaat tggctcgaag 300
gattgctcct gctgcactgc aatgtggccg cggccctggt tctggtgtgt aggtaaaggt 360
aaggctggtg gaataaatga ttccatcatt tcggaccaaa gttactggaa cctggactgg 420
ttgccggacc catctccaac cttctcggaa tgcagaaatg tctgggacga cacanancat 480
actctctcca cacctgtaca tagtttcagc ttctacatcc ccaaaccaca ctcgtaaatt 540
tggagtgaaa ttctgtcctg taagttcaag cattgctacg tccccaccgc cattcaactg 600
aaggcctcta cacaggcaca ggaatgactg gggcaaggan agggcccatt ccctcataaa 660
atgtatactc tgccttatct gtgctaatga ttgtccagga aacgccanca ttttaccacc 720
tcnttattgg ttcttttggg antggaatgg cctgaaattg aaatattctt ccttgaaaaa 780
aggccaaata cntcttctgt ttccttnaag ggtaaaatgc ccatttttgg aattg 835

<210> 597
<211> 443
<212> DNA
<213> Homo Sapiens

<400> 597
agcagttcga atgccaggaa actgctcgag tgccaggtgc aggtgggggc ccccgaggag 60
gaggaggagg aggaggagga cgcgggcctg gtggccgagg ccgangccgt ggctgccggc 120
tggatgctcg atttcctctg cctctctctt tgccgagctt tccgcnacgg ccgctccgag 180
gacttccncn ggacccgcaa cagcgcanag gctattattc atggactatc cagtctaaca 240
gcttgccagt gagaacgata tacatatgtc agtttttgac aagaattgca gcaggaaaaa 300
cccttgatgc ncagtttgaa aatgatgaac gaattacacc cttggaatcn gccctgatga 360
tttggggttc aattgaaaag gaacatgacn aacttcntga agaaatacag aatttaatta 420
aaattcangc tatngctgtt tgt 443

<210> 598
<211> 491
<212> DNA

<213> Homo Sapiens

<400> 598
gtactttgag gagttcctac tcttctttct ttcttattaa ggtcttgttg ctgggttcca 60
tgttgcaact tagataanaa aagattcttg tgagacctca ataaggatac tgtaccctct 120
gaggattcag ttaccgcaga ctgtttgtca ctaacacttt ttcttgtatc caaattagct 180
tcagtttcca tttcaacatc attaccacta ggtttatctt gagaagttat tgttcttgtc 240
cttttgcttt ctactacttt tgccgctgcc ttcattagaa aggttgatga tttttcactt 300
agcacataat tcacataact cttaattttc tccatcatgt ggttgtagct gaagtgttga 360
aaaaaggaat gaaatgtatc tttctgagan attatcataa gcaatttgct tttgaaaggc 420
atatgagaat ttggatcacc aaatattctt tcaaagactt cttctgcttc tttaaagttg 480
ccattttcca t 491

<210> 599
<211> 802
<212> DNA
<213> Homo Sapiens

<400> 599
gtactttgag gagttcctac tcttctttct ttcttattaa ggtcttgttg ctgggttcca 60
tgttgcaact taaataagaa aagattcttg tgagacctca ataaggatac tgtaccctct 120
gaggattcag ttaccgcaga ctgtttgtca ctaacacttt ttcttgtatc caaattagct 180
tcagtttcca tttcaacatc attaccacta ggtttatctt gagaagttat tgttcttgtc 240
cttttgcttt ctactacttt tgccgctgcc ttcattagaa aggttgatga tttttcactt 300
agcacataat tcacataact cttaattttc tccatcatgt ggttgtagct gaagtgttga 360
aaaaaggaat gaaatgtatc tttctgagag attatcataa gcaatttgct tttgaaaggc 420
atatgagaat ttggatcacc aaatattctt tcaaagactt cttctgcttc tttaaagttg 480
ccattttcca tacaaacagc tatagcctga attttaatta aattctgtat ttcttcatga 540
agtttgtcat gttccttttc aattgaaccc caaatcatca gggctgattc caanggtgta 600
attcgttcat cattttcaaa ctgtgcatca agggtttttc ctgctgcaat tcttgtcaaa 660
aactgacata tgtntatcgt tctcaactgg cnagcctgtt aaactggaaa atccatgaat 720
aataacctct ggcgctgttg cgggtcctgc ggaaattccn cggaaccggc cgtcncggaa 780
aactcngcaa aagaaaaaaa gc 802

<210> 600
<211> 523
<212> DNA
<213> Homo Sapiens

<400> 600
gaaaagcaac ttttattgaa naatttggag ggaaggttcc atattatatt ataatagtaa 60
aaatactaaa gttgaatgtt gtaaaaaaac nccgtggtgc agcggcagcg gcagcgtctg 120
gccaggaggc gtggaggggc ccagggatgg ccacccccac agggagtcag ggagggcctg 180
gggcgacagc ggaaaggtta agcgtcnaaa aggtcaagtg ctaccgtgga naaatcatct 240
gagggggagg ctcccggtgg gacagtcacc aanaactgtn acacacaagg ggaaggggga 300
gggctttcct gtcacaaana ttaaaaaccc ccnaaatgca tttgaacaac atnatacacn 360
ataacaaatt taaaccttgc tcctctgtcc cactgggtna accctggccc atcccccatc 420
cctggtccca tcccaggggc ccagcctccg atnactcctc anaaacacng ccttnntgct 480
ggggggctgc tgtntncctg ccacccccnn gaaaaggtgc tgg 523

<210> 601
<211> 530
<212> DNA
<213> Homo Sapiens

<400> 601
aaaaccaact tttattgaaa aatttggagg gaaggtncca tnttatntta taatantaaa 60
aatactaaat ttaaattttn taaaaaaacc ccntgntgca ccggcancgg cancttctgg 120
ccaaaangct tnaaggggcc cagggatngc cncccccnca gggattcngg gagggcctgg 180
ggcaanancg naaaggttaa ccntcnaaaa ggtcaattnc taccgtgnaa aaatnatctn 240
agggggangc tcccggtggg acactccccn aaaactntna cccaaaaggg gaagggggag 300
ggctttcctn tnncaaaaat tnaaancccc cnaaatgcct ttnaacnact ttntncccan 360
tnncaatttt naaccttgcn cctctntccc actgggtnaa ccctggccca tcccccatcc 420
ctggtcccnt cccnggggcc caccccccna taacttcctc aaaaaccngc cttnttnctg 480
gggggctgct nttttcttcc ccccccaana aaaggtnctg gcccccctcc 530

<210> 602
<211> 311
<212> DNA
<213> Homo Sapiens

<400> 602
gccnancagg nanccgccgc tgaagccacc gccgggtgcc cagcgccgcc gccgcccccg 60
agctcccccg cgcccctgcc cncgggcggn cggtgggcac cgggcgccat ggccgcgccg 120
gganccgctg cggntncgcn tgtgccnctt ggtgcncgga anancanggc tacngnttct 180
acctntacgt gtgananngg ccgccgcggg cacttcntcc ggcgcgtgna ncctctgttc 240
ccccgccgag gcngccgcgc tgtgctctgg ggatctnctg ntcnaggtca acntgcntca 300
acgtgnaggg c 311

<210> 603
<211> 289
<212> DNA
<213> Homo Sapiens

<400> 603
gcanagaaag gtttgtttta ttgcaattat ttaaatcncg tcccangggg gaggggaagg 60
gggangggaa ggggggggtn tctggtnttn attngatncc tgtctgccan cttnnacatc 120
tatnangaan anaaccatca ncncncntcc ctttcantca tctggcncct gcanaccatc 180
tttcgccctc tnccccccgc tgctctccna ctcccntgac cnctctcatc tctctccnct 240
ctgnctcctc nctctntctc tcatttctct gttncacnct ctctccccc 289

<210> 604
<211> 356
<212> DNA
<213> Homo Sapiens

<400> 604
ctgaagccac cgccgggtgc ccagcgccgc cgccgccccc gagctccccc gcgcccctgc 60
ccgcgggcgg ccggtgggca tcgggcgcca tggccgcncc gganccgctg cggccgcgcc 120
tgtgccgctt ggtgcgcgga nagcanggct acggcttcca cctgcacggt gagaanggcc 180
gccgcgggca nttcatccgg cgcgtggaac ccggttcccc cgccgaggcc nccgcnctgc 240
gcgctgggga ccgcntgntc naggtcnacn gcgtcaacnt ggagggcgat accaccncct 300
ngtgntgcnt acgatcnang ctgtngangg gcanactcgg ctgctggtgg tggacc 356

<210> 605
<211> 290
<212> DNA
<213> Homo Sapiens

<400> 605
   gcaaagaang gtttgtntta ttgcaattat ttanagcgcg tcccaagggg gaggggangg 60
   gggangggaa ggggggggtn tcttgctana aactggaaac ntgtttctta ccccnatntc 120
   nnantcgact nccaccaact gtnnntcttc cttcctttcc cnangtccct anntaccncc 180
   tnttgccctt ctnccccttn tttcccctcn cgctttccct nactctttat ctntcttntc 240
   ctctctctct ctcacctctt tctccccctc ccttcacnct cacnttgtct 290

<210> 606
<211> 714
<212> DNA
<213> Homo Sapiens

<400> 606
cgccagaaaa agttatttta attttctatt aaacattctt ctcaaagcat tattttatcc 60
tatatctcac tgaattttaa gaaataacat tagtattaga aaaactagga aaaaagataa 120
atgcagataa ttaaacttac atgaaaaagg aaaattataa caaaggactg agaacgttat 180
aaattgaaat gagattataa tttgaaaact gcatctgaaa gcaaacttta ttgttcaatt 240
atncttaatg atggtgtttt atgactaata cactgatttt tcaagaagga aacccatgtt 300
aaaaatattt ttattttaaa aataagcctg tgttcaagct ctgatcatat ttcttttatt 360
ttgatttggg aanaaaatac tgtttctgat agcatgaaat gcaaaatttt tagattttta 420
atctcactaa ttttaanaac tattgagaaa ttgattaatg acatgaagtg cacaacacta 480
attactggcc agctgttggc attgtgtttc ttacttagtt ctcccaaggg aaaactctta 540
aattgaatct tcagcagaat aatccttaaa tatactttgt aagcaaaaca aaagcttttt 600
tgtttacata gttctttggg attttactgt tcctaatttt attctgaaac tcaattttac 660
cccagaccat aattaccata ttaactttgt tntgcacagt tgtttgccaa ttca 714

<210> 607
<211> 687
<212> DNA
<213> Homo Sapiens

<400> 607
attttaattt tctattaaac attcttctca aagcattatt ttatcctata tctcactgaa 60
ttttaanaaa taacattagt attagaaaaa ctaggaaaaa agatnaatgc agataattaa 120
acttacatga aaaaggaaaa ttataacaaa ggactgagaa cgttataaat tgaaatgaga 180
ttataatttg aaaactgcat ctgaaagcaa actttattgt tcaattattc ttaatgatgg 240
tgttttatga ctaatacact gatttttcaa taaggaaacc catgttaaaa atatttttat 300
tttaaaaata agcctgtgtt caagctctga tcatatttct tttattttga tttgggaaga 360
aaatactgtt tctgatagca tgaaatgcaa aatttttaga tttttaatct cnctaatttt 420
aagaactatt gagaaattga ttaatgacat gaagtgcaca acactaatta ctggccagct 480
gttggcattg tgtttcttac ttagttctcc caaggaaaac tcttaaactg aatcttcagc 540
ngaataacct taaatatact ttgttagcca aacaaaactt ttttgtttac atagttcttt 600
ggattttact gttcctaatt ttattctgaa actccatttt tccccagacc ataattaccc 660
tatttaactt tgttatgcac agttgtt 687

<210> 608
<211> 994
<212> DNA
<213> Homo Sapiens

<400> 608
ctcacccagt tgctcctcag atgtttgggt atgctggaaa agaacatatg gaaaaatatg 60
gaacaaaaat tgaacacttt gcaaaaattg gatggaaaaa tcataaacat tcagttaata 120
acccgtattc ccagttccaa gatgaataca gtttagatga agtgatggca tctaaagaag 180
tttttgattt tttgactatc ttacaatgtt gtcccacttc agatggtgct gcagcagcaa 240
ttttggccag tgaagcattt gtacagaagt atggcctgca atccaaagct gtggaaattt 300

tggcacaaga aatgatgact gatttgccaa gctcgtttga agaaaaaagc attattaaaa 360
tggttggctt tgatatgagt aaagaagctg caagaaaatg ctatgagaaa tctggcctga 420
caccaaatga tattgacgta atagaacttc acgattgctt ttctaccaac gaactcctta 480
cttatgaagc actgggactc tgtccagaag gacaaggtgc aacgctggtt gatagaggag 540
ataatacata tggaggaaag tgggtcataa atcctagtgg tggactgatt tcaaagggac 600
acccactagg cgctacaggt cttgctcagt gtgcagaact ctgctggcag ctgagagggg 660
aagccggaaa agaggcaaag ttcctggtgc aaaggtggct ctgcngcata atttangcat 720
tggaggaact gtggttgtaa cactctacaa gatggggttt tcccggaagc cgccagttcc 780
ttttagaact catcaaaatt gaagccngtt ccaaccaagc tctgcaagtn atnggtttaa 840
ngnaaaatct ngttttaaag gnggattgag aaggaaacnt naaagaggga anggggaaca 900
atttgtgaaa gaaaaatncg gngggaattt ttgcccttca aggggaaana atggccctgg 960
ggggtaaaag anggccaccc tggggtggtg ggat 994

<210> 609
<211> 843
<212> DNA
<213> Homo Sapiens

<400> 609
ggccaaaaaa anttatttna atttcctatt aancntcctc cncaaancat tatttnaccc 60
tatnncncnc ngantttnan aaantacctt tnntnttaaa aaacctngga aaaaaaataa 120
tngcaaatan ttaaccttnc ttgaaaangg aaatttntac caanggacng aaancnttnt 180
aattngaant naaattatan ttngaaancg gcnncngaaa ccaancttna tggtccaatt 240
atcctnaang agggnntttn annactaatn cccngatttt ccaatangga ancccnnntt 300
aaaantnttt tnattttaaa aataacccng tntccaaccc cngatcanat tcctttnatt 360
tggattgggg aaaaaaatnc ngttccnnat accnngaann gcaaantttt taaattttta 420
acccccctan ttttaaaanc tatngaaaan tngattanng acttgaattg ccaaccctan 480
ttncnggcca ccngtgggcn tngtnttcct tacttantcc ccccaaggaa annccttaan 540
cngaanctcc nccaaaataa cccttaanta tccttggtaa ccaaancaaa acctttttng 600
tttacntant ccttgggatt taacgggtcc ccaatttnat ccngaaccca nttttccccc 660
naaccatant taccatttta ccttggtaag gcncagtngt ttgcantncc gcaaancagt 720
antnttcccc nggcnctttc ccccgancct tgggaaaaac gggatnggtc ccccccttaa 780
aaaacaacct tcccccncct ttggcccagg nnttnttccc gtctaaatcc gaacaataaa 840
aag 843

<210> 610
<211> 707
<212> DNA
<213> Homo Sapiens

<400> 610
ctagtctcga gttttttttt ttttttttaa cctttcctta tgagcatgcc tgtgttgggt 60
tgacagtgag ggtaataatg acttgttggt tgattgtana tattgggctg ttaattgtca 120
gttcagtgtt ttaatctgac gcaggcttat gcggaggana atgttttcat gttacttata 180
ctaacattag ttcttctata gggtgataga ttggtccaat tgggtgtgag gagttcagtt 240
atatgtttgg gattttttag gtantgggtg ttgagcttga acgctttctt aattggtggc 300
tgcttttagg cctactatgg gtgttaaatt ttttactctc tctacaaggt tttttcctag 360
tgtccaaana gctgttcctc tttggactaa cagttaaatt tacaagggga tttagagggt 420
tctgtgggca aatttaaagt tgaactaaga ttctatcttg gacaaccagc tatcaccagg 480
ctcggtaggt ttgtcgcctc tacctataaa tcttcccact attttgctac atagacgggg 540
tgtgctcttt tanctgttct tangtanctc gtctggtttc gggggtctta gctttggctc 600
tccttgcaaa gttatttcta agttnaattc attatgcnca angtataggg gttagtcctt 660
gctcatatta tgcttggtta taattttcca nctttcccct tgcggta 707

<210> 611

<211> 663
<212> DNA
<213> Homo Sapiens

<400> 611
ccattttata atgcgcttta tttgattaaa gaatttgcct tctttgtata cactggaatg 60
ttatattccc tatgtatttt acagggttac aaaatgtctc tcattttaaa tattacccca 120
aaagtaatct canaaaaaaa aggttttttg aaattaaact tgacttttaa aaaatcatac 180
ggacaaacaa ctttcaaaca aaactggatt agtaggattt cttgcctgct taactaacat 240
gacanacttc ttgtcccagg cccttctcan aaaaacctca tgtggaaacc aagctanaga 300
taanaattct tccctgatgc agttagggga aagggaaagg ctagaaactt ctttggcaag 360
caattccaca cacagccatt tatgtgtgag tgctctgctt caagcacagt acgctctttg 420
cagggacggc cagatgttca gagtgggagt ggtacttttc aaccagctaa aagtgcagaa 480
gtcatctant cgtctgcctc ttcccactgc cagtgcctgc agccttgcag caacttttaa 540
ccacccccta tgggactgga atnttgagtt aaaaagccaa ngctgaactg gctgacgctg 600
tantctccan tgaaaaggaa atgggatgaa atggaaaccg aaaaaccccc ngtnacntga 660
tga 663

<210> 612
<211> 621
<212> DNA
<213> Homo Sapiens

<400> 612
   cattttataa tgcgctttat ttgattaaag aatttgcctt ctttgtatac actggaatgt 60
   tatattccct atgtatttta cagggttaca aaatgtctct cattttaaat attaccccaa 120
   aagtaatctc anaaaaaaaa ggttttttga aattaaactt gacttttaaa aaatcatacg 180
   gacaaacaac tttcaaacaa aactggatta gtaggatttc ttgcctgctt aactaacatg 240
   acaaacttct tgtcccaggc ccttctcana aaaacctcat gtggaaacca agctananat 300
   aanaattctt ccctgatgca gttaggggaa agggaaaggc tagaaacttc tttggcaagc 360
   aattccacnc acagccattt atgtgtgagt gctctgcttc aagcacanta cgctctttgc 420
   agggacggcc anatgttcnn antgggagtg gtacttttca accagctaaa antgcanaag 480
   tcatctantc gtctgcctct tcccactgcc agttgcctgc agccttgcag catcttttaa 540
   ccacccctat nggactggaa tattgaatta taaacccngg ntgaactggc tgangctgtt 600
   tctcccttga aaaggaaatg g 621

<210> 613
<211> 637
<212> DNA
<213> Homo Sapiens

<400> 613
catttnataa tgcgctttat ntgattaaan aatnngcctt ctttgtatac gcnggattgt 60
tatctcccct ntntatttnn gggggttaca anttntcnct catttnaant atnnccccaa 120
tantntnctn aaaaaaaaga ggtttganga aattaaactt gacttttaaa anatcatgng 180
gacaaacnac tttcaaacaa agctggatta gnaggatttc tngnctgctt aactaacatn 240
aaanacttct tgtcccaggc cctnctnaaa aaaacctctt gtggaaaccn agcnaaaaat 300
aananttctc ccctgatgca ntggggggag anggagaggc taaaaacttc tntggcaanc 360
anttccacnc acngccattt ttntntnagt gcnctgctnc nancnnagta cgctctttgg 420
gnggacggcn anntnttnat agngggagtg gtnctttcaa ccagctaata ntgaagaaat 480
catctagtcg nctgcctctn cccactgcca gtgcctgcnt ccttgcaacn tcttttaacc 540
ccccctangg acnggattat nnagttaana ccgaggntga gctggntgac gctntctcct 600
ccatttgaaa angaaatgga taagatggaa ccgaaaa 637

<210> 614

<211> 673
<212> DNA
<213> Homo Sapiens

<400> 614
agattatgcc attgaggcta agaatagagt catttttgat ctaatttatg aatacgaaag 60
aaagagatat gaagatcttc ctataaatag caatccagtg tcttctcaga aacaaccagc 120
cttgaaggct acaagtggca aggaagattc tatttcaaat atagccacag aaataaagga 180
tggacaaaaa tctgggacag tgtcttctca gaaacaaccg gccttgaagg atacaagtga 240
caaggatgat tctgtttcga acacagccac agaaataaaa gatgaacaaa aatctgggac 300
agtgcttcct gctgttgaac agtgtttaaa caggagtctc tacagacctg atgctgttgc 360
acagcctgtg acagagaatg agttttcttt ggaatctgag attatttcaa aactatacat 420
cccaaagaga aagattattt ctccacgatc tataaaagat gtgcttcctc ctgttgaaga 480
ggctgttgac aggtgtctct acctactgga ccgttttgca cagcctgtga caaagggata 540
agtttgcttt ggaatctgag aatatttcag aaccatactt tacgaacaga aggactattc 600
tcaacaatct gcagaaaatt tagatgctgc atgtggcatt gacaaaacag aaaatggana 660
catgtttgaa gac 673

<210> 615
<211> 714
<212> DNA
<213> Homo Sapiens

<400> 615
cctctggcta tattcaaaac agaatctttc tcatcacttg aagccttcaa gcctggtggt 60
ttctcanaan acactgtcct agatttttct ccatccttgt tttctctggc tatacccaaa 120
acagaatctt cctcgtcact tgtacccttc aaggttggtg gtttctgana anacactttc 180
ctanatattt ctccatcctt ttttcctctg gttatattcg aaaaanaatc cttctcatca 240
cttgtagcct tcgaggctgg ttttttccga naagacactg tcctanattt ttctccatcc 300
ttgttttctc tggctatact caaaacagaa ccttcctcgt cacttgtanc cgtcaaggct 360
ggtggtttct ganaanacac tgtcccanat ttttctccat cctttatttc tgtggctatg 420
ttcgaaacag aatctttctc atcagttgta gccttcaagg ntggttgttt ctgaaaanan 480
ctgtcccana tttttctcca tcctttattt ctgtggctat nttcgaaaca gaatcttcct 540
cgtcagttgt accttcnagg ntggttgttt ctgaaaaaan actgtcccac actgtatcca 600
tccttttatt tntgttanct atatcnaagc aaaatctgtt ttgtcccttg ttaccntttg 660
aaggtnggtn gtttctgaaa aataanctgt tccanatttt cccaccaccc attt 714

<210> 616
<211> 688
<212> DNA
<213> Homo Sapiens

<400> 616
cctctggcta tattcaaaac agaatctttc tcgtcacttg tagccttcaa gcctgatggt 60
ttctcanaan acactgttct anatttttct ccatcctttt tttctctggc tatattcaaa 120
acanaatctt cctcgtcacc tgtagccttc aaggctggtg gtttctgaaa anacactgtc 180
ctanatgttt ctccatcctt tctttctctg gttatatttg aaaaanaatc tttctcatca 240
cttgtagcct tcaaggctgc ttttttccga naanacactt caagcctggt ggttgctctg 300
aaaacactgt tctaaatttt tctccatcct ttttttctct ggctatattc aaaacanaat 360
cttcctcgtc acttgtagcc ttcaaggctg gtggtttctg aaaananact gtcctanatg 420
tttctccatc ctttctttct ctggttatat ttgaaaaana atctttctca tcacttgtan 480
ccttcaaggn tgcttttttc cganaanaaa cttcaagcct ggtggttgct cngaaaaaac 540
tgtcctaaaa tttttctcca tccttttctt ctctnggcta tactcnaaac aaaatcntcc 600
tcgtcccttg ttncccttca anggtgggtg gtttctcgaa aaaaanactg tcctanaatt 660
ttcctccntc cctttttttc tctgggtt 688

<210> 617
<211> 721
<212> DNA
<213> Homo Sapiens

<400> 617
ttcgggcttc cacctcattt ttttcgcttt gcccattctg tttcagccag tcgccaagaa 60
tcatgaaagt cgccagtggc agcaccgcca ccgccgccgc gggccccagc tgcgcgctga 120
aggccggcaa gacagcgagc ggtgcgggcg aggtggtgcg ctgtctgtct gagcagagcg 180
tggccatctc gcgctgcgcc gggggcgccg gggcgcgcct gcctgccctg ctggacgagc 240
agcaggtaaa cgtgctgctc tacnacatga acggctgtta ctcacgcctc aaggagctgg 300
tgcccaccct gccccagaac cgcaaggtga gcaaggtgga gattctccag cacgtcatcg 360
actacatcag ggaccttcag ttggagctga actcggaatc cgaagttgga acccccgggg 420
gccgagggct gccggtccgg gctccgctca gcaccctcaa cggcgagatc agcgccctga 480
cggccgangt gagatccaga tccgaccact anatcatcct tataccgacg gggaaacnga 540
agccatanaa ggcgtgggcg cttgcaccac ttccgtccca tccttgcggg tacctggtct 600
atgcnggggt ncctaaggac cttggaaaaa acgctccccc gtcgttgctt cctggggaan 660
ggggcgttnc gctgcgcttc ggaacggggt tccttccaac ccgccggtct catttcttct 720
c 721

<210> 618
<211> 461
<212> DNA
<213> Homo Sapiens

<400> 618
ccaccancta anttattnnt ttaataacaa aaaaacancc ccacaaaact atngtaaaac 60
aatatttcca ntcggtnatc ntngtattnt acaatacaaa ncanttcccn caaaattctn 120
aaaancacca ancttnacca ttttttaaan tttctgcttt ncaaaaanta aaaacncnca 180
attgnantcc caccccctaa attctctggt nactattagg tntncaaaaa gnaccncccn 240
ctccncncca ttgcctcanc cncancccca ggctgnatnc atttaagggc ncattggccg 300
ccaatcggnc tnntccnccc ncaaatccgg caaggcnctt nggggnaaac ccacaaanca 360
cttattcccc ctngccccct gaatggctgg ggtccgccgg tccctggggn aggcnctcca 420
ccaacncaaa atgcaatcnt ccncagnaac ccntgccgcc t 461

<210> 619
<211> 751
<212> DNA
<213> Homo Sapiens

<400> 619
cccgagggac cacagctggc agctccgggg atgccctcgg caaagcactg gcctcgatct 60
actccccgga tcactcaagc aataacttct cgtccagccc ttctaccccc gtgggctccc 120
cccagggcct ggcaggaacg tcacagtggc ctcgagcagg agcccccggt gccttatcgc 180
ccatctacga cgggggtctc cacggcctgc agagtaagat agaagaccac ctggacgang 240
ccatccacgt gctccgcagc cacnccgtgg gcacagccgg cgacatgcac acgctgctgc 300
ctggccacgg ggcgctggcc tcaggtttca ccggccccat gtcactgggc gggcggcacg 360
caggcctggt tggaggcagc caccccgagg acggcctcgc aggcagcacc agcctcatgc 420
acaaccacgc ggccctcccc agccagccag gcaccctccc tgacctgtct cggcctcccg 480
actcctacag tgggctaggg cgancaggtg ccacngcggc cgccancgag atcaagcggg 540
aagagaagga ngacgangag aacacgtcag cggctganca ctcggaagaa ganaanaagg 600
aactgaaggc ccccgggccc ggaccattac ggaacaagtg ctgtcccttg naggagaaaa 660
actgaaggac cgggaaaagg cncatggcaa ttacnccccg ggaaccggtg cccttccggg 720
atattaacna aggcttccgg gaactggggg c 751

<210> 620
<211> 556
<212> DNA
<213> Homo Sapiens

<400> 620
aatacaacgt ttaatcatct ggttgatcaa aaaatgcaat gctcagtcta ggaacagcag 60
caaaaatagc ganagacacg ggacttttat acaaaaaaat ttgttgctta caaaacatat 120
gcaaaaaaag cttaaaaaaa ccaaaaacca aaggcagcat ccttgctaat tttcatctac 180
attaanaaaa aaaaaatctt gtaactaatg tttttatttn ccttaaaaaa aatatttcgc 240
ttaggcacaa tttgctggtg gctttaaaaa aataagccag gtttccacag catccccctt 300
gagtgatatn tttccatttc tccgcttttt atagttaagg cattttttnc tnctctgaca 360
aagtgtatgt tttgttgctt gctttcaggt tttgtttact ggaaaaaaaa aaaaatgccc 420
tgtcanccca ngcaanaggg ccaanatgca attcagggat ccntgggaca ggtccaaaat 480
gacccggggg ctgaaattcc gggacggggg aacaaggcnn tttaatngta ggccagggcc 540
canggaaccc tgaacc 556

<210> 621
<211> 708
<212> DNA
<213> Homo Sapiens

<400> 621
ccacttnaat tcctttatnc ancaatatta tccnaaaagg aaaaatcagg atttacaaaa 60
acaatttaan tgcaatataa aaccctacta aatacaaata caattncaca aacncntatg 120
caacaaaaac ttgtttaaat ngttccttna atttnnacta cttaaaanca taggtntaaa 180
ggaaaaacnt ncaaactggt ccacttgggc ttnttaccag gcaaagnaac cctgcttncc 240
aaaaactnat atattccaaa ttcncggcat ntggnaatnt tnccatggac nctgnatctt 300
aacaaatgct atantnttta caaaactacn cccncaaaaa aaccccaagg aacctgcagg 360
ctaanccccta tnctttttaaa gggctnaagg aaccaaacct atttaanccc tnttngtttg 420
cnccatgcaa aactttatgn aaaaccccca aactaggcta tttancnnct nccatnaatg 480
gnccccaaat catntnatnc tacggcataa acaacanctg ccctatttac ncggaacctg 540
caaanctcac aagnaatgtg aattngcnct ngggantcaa tgttnccggg tnaattatct 600
tggatnanaa ccnttttcta catnactatt gaaaaaacct gtggtttctt gctttttaac 660
aaatnnggtg ttcctttgcc cccccccctt atttttcaag ggctgggt 708

<210> 622
<211> 675
<212> DNA
<213> Homo Sapiens

<400> 622
atcacagtcc agagagtcct agaggaggac gagagcataa gancttgcct tagtgaagat 60
gcaaaagaga ttcagaacan aatagaggta gaagcagatg ggcnaacaga agagattttg 120
gattctcaaa acttaaattc aagaaggagc cctgtcccag ctcaaatagc tataactgta 180
ccaaagacnt ggaagaaacc aaaagatcgg acccgancca ctgaagagat gttagaggca 240
gaattggagc ttanagctga agaggagctt tccattgaca aagtacttga atctgancaa 300
gatntaatga gccaggggtt tcatcctgaa agagacccct ctgacctana aaaagtgaaa 360
gctgtggaag aaantggaga anaagctgag ccagtncgta ntggtgctga gagtgtctct 420
gagggtgaag gantagatgc tacttcaggc tccncagata gttctggtga tggggttacn 480
tntccatttn aaccngaatc ctggaagcct actgatnctg aaggtntgan gcnntntgac 540
ngggagttct gctggacttc cagttcatgc ctgcctggta tnctttnccc gagggcctgc 600
ctcctntcag tgatttggtt cttgacaaga tccnccntcc cccttttgcc aatgccgaac 660
tctgggatcc ttcga 675

<210> 623
<211> 713
<212> DNA
<213> Homo Sapiens

<400> 623
gctaaacatt tttttaagta tgagtccttg tttaaaaaga aaagattaaa acagaaaata 60
ttttctataa ataatacatg tattttggtt ttagtgctcc cgccctaagg tttgaagttt 120
acttttatcc agtacctttt tcctccatga tcaccttttt ttctctttcc cctctcccac 180
tcgtgcacac gtgggggttt ctgcgagaat tggccttgct gcactgtgat tggcgaanac 240
gtgaaacttt ttaaaaaaat acttaaattg tttcttttgt ttcattttgt gtatttgaag 300
ttttagttat cctcagactc ctcttctgct tcccgcagcc acgtgaagaa tgccgtgaca 360
gatttcagag ccacgccctt cccattctgc tctgcagggt ccttgctgct ctcccatttg 420
tagaaggcat cctcggagat cacctcctcg tcatatagac aatcaaaaaa catccgcagc 480
aaattggcag gttgatcaag ttttactatc gatgcttgta gtgcataaag tgctgcagtt 540
ccttctctgt atctgantct aggtacttga gtaagatcgg cactctctgc ttgataacag 600
cagtgtccac tctgaaggta naagaatcng gttattatag cttgctttaa caaacagcng 660
tcnttaaagc tctaaggaat gttangtgaa atncactgga tttcgtctaa att 713

<210> 624
<211> 554
<212> DNA
<213> Homo Sapiens

<400> 624
cattcnagaa agatnttaca cacggagttt nctcantatt gggctcaacg ggaagctgac 60
tttacggana ctctgcttca agtaacgana gatattanaa ganaatgctg gancgtcgtt 120
tggctatctt cnaggatttg gttggtaaat gtgaccctcg agaanaagca gcgaaagaca 180
tttntgccac caaagttgaa actgaagaag ctactgcttg tttagaacta actttnatcc 240
aattaaagct gaattagcta aaaccaatgg agaattaatc tcnaccncnc acnanttcnc 300
ccagaganaa natgaatccg attcattgat tcaagagctt gagacatctg ntaaganaat 360
aattncacan aatctggaga attnnagaat tgatnaatat nattgatcnn tcgaagatac 420
tatcancgaa tttcagaacc tnangtctca tatggaaaac tcntttaaat gcnatgacaa 480
ggctgataca tcttctttaa taataaacaa taaattgatt tgttatgaaa cagttgaagt 540
acctaaggga cagc 554

<210> 625
<211> 551
<212> DNA
<213> Homo Sapiens

<400> 625
gactgcatgt tctcatttat ttatgggatc taaaaataaa atcaattgac ctcatgggca 60
tacanantaa aaaaatggtt accagtggct ggtaagggta ctgacggttg cagggggagg 120
tggggatggt taatgggtac aaaaacaaat aagatnaaaa gaatgattta atatctgata 180
gcacaatana ntgactataa tcaataataa cttacttgta tatttttaaa tgatctaaaa 240
aatgtaattg gattatctgt aattcaaagg aaaaatgctt gaggggatgg atacctcatt 300
ctccatgata cacgtntttc acattgatgc ctgtgtcaaa acatctcaca taccccgtaa 360
atatatacat gtactatgta ccacaaaatg tttacaaaat aagtganaca ttctaattaa 420
agactgaaat ctttttctaa ataatgtata tacatgtttt gtgatctgta cacacttatt 480
ctccaaatcc taactntant cccaacanat atnttaaatc cttgtttanc ngaataagtt 540
aaaaaaatcc t 551

<210> 626
<211> 680

<212> DNA
<213> Homo Sapiens

<400> 626
atttggtaac aggattaaaa agaaattttt aattccttgt ctctcttctg atggctgaac 60
agaactgcgg tgtcaaatgg aaagcagcac acaagaattc ccttgcagac cttgatcttt 120
cgcanaaatg caaagacgcc tgagttatac aacttgcaat tattattttc tanacagaag 180
tgccaactgt tgtgctttcc agtgtatcag tggttgctac attctccttc ttgtcttcgg 240
gtttcatggc aggaaacaga agtacttcct tgatgttgtt ggagtccgtg agaaacatgg 300
cgactcgatc aatgcccatg ccccagccag ctgtgggggg cagcccatat tccagggcag 360
tacagaagtt ttcatctatg aacatggcct catcatcacc tgcagccttg gccttggcct 420
gttcttcaaa aanctgccgc tgccgcatgg gatcattcag ctcagtatac gcattgcata 480
tctctttctt catgacaaac agctcaaanc gctcagtcag acctctttaa ancggtgcca 540
tttaaccnaa gggccattat ctgtgggtga tcacagatga atgtnggatt gatgcaagtc 600
acttccanga actccccaac aancttgtca aggaacctgg ctgtggtcca angtggaagg 660
catccacanc ttttgccccc 680

<210> 627
<211> 753
<212> DNA
<213> Homo Sapiens

<400> 627
acaaatatga acgtctgaag gcaaaccagg tagctactgg cattcggtac aatgaaagga 60
aaggaaggtc tgaactaatt gtcgtggaan aaggaagtga accctcagaa cttataaagg 120
tcttagggga aaagccagag cttccagatg gaggtgatga tgatgacatt atancagaca 180
taagtaacag gaaaatggct aaactataca tggtttcaga tgcaagtggc tccatgagag 240
tgactgtggt ggcanaagaa aaccccttct cantggcaat gctgctgtct gaagaatgct 300
ttattttgga ccacggggct gccaaacaaa ttttcgtatg gaaaggtaaa gatgctaatc 360
cccaagagag gaaggctgca atgaagacag ctgaagaatt tctacagcaa atgaattatt 420
ccaagaatac ccaaattcaa gttcttccag aaggaggtga aacaccaatc ttcaaacagt 480
tttttaagga ctggagagat naacgatcag agtgatggct tcgggaaagt ttatgtcaca 540
gagaaagtgg ctcaantnna acnaattccc tttgatgcct cnnaattacn cagttctccg 600
cagatggcag cccagcacaa tatggtggat gatggttctg gccaagtgga aatttggcgt 660
gtncaaaaca atggtaggat ccaagttgac cnnaactcct atggtgactc ccatggtggt 720
gactgctact tcatactcta cacctatccc tga 753

<210> 628
<211> 675
<212> DNA
<213> Homo Sapiens

<400> 628
ggtgtttcca aaggctttta ataaggttaa aaaaaaaata aaatnccnct taaaaaataa 60
cncttancan ttaatgacat caaantcncn ttgactaaaa aaggaaaata ncaaccaatt 120
gttaaancca ccttaacata aaccttatng caattntaca cntcttttga acncaatcta 180
taaaaaaaaa aataactncc anggcattac aacttttnct ctggcatntt aaaaaacaac 240
tctnactaat ggctaatgca ttataaaatt ncctatctna caaatcttnc taaattatgc 300
atagtatttt acttttnaaa ggtcntaaaa aaaatataaa tcanttncca taaaanctaa 360
tatnggccca taacaaaant tccctncagg ttattttaat ntnttaacnt aaaaaaacnc 420
cagntgaaaa aaaattncaa nccaaaacta accnttaaaa aataggcntt nggttnaggt 480
taattttttt tttttttttt ttgnaaanaa antcnctntt gcccagnctg gattgtggtg 540
gcnccaatcc tggctcactg caacctcagc ctcctgggtt caagcaattt ncctgtctca 600
gccttccaan ttccngggaa tacaggggtn cnccaccacn cccagctaaa ttttttttgt 660
tttttttant aaaag 675

<210> 629
<211> 677
<212> DNA
<213> Homo Sapiens

<400> 629
aagatcagcg atatcacgcg tcccccggag catcgcgtgc aggagccatg gcgcgggagc 60
tataccacga agagttcgcc cgggcgggca agcaggcggg gctgcaggtc tggaggattg 120
agaagctgga gctggtgccc gtgccccaga gcgctcacgg cgacttctac gtcggggatg 180
cctacctggt gctgcacacg gccaagacga gccgaggctt cacctaccac ctgcacttct 240
ggctcggaaa ggagtgttcc caggatgaaa gcacagctgc tgccatcttc actgttcaga 300
tggatgacta tttgggtggc aagccagtgc agaatagaga acttcaagga tatgagtcta 360
atgactttgt tagctatttc aaaggcggtc tgaaatacaa ggctggaggc gtggcatctg 420
gattaaatca tgttcttacg aacgacctga cagccaagan gctcctacat gtgaagggtc 480
gtanagtggt gagagccaca gaattcccct tagctgggac agtttcaaca agggtgactg 540
cttcatcatt gaccttggca ccgaaattta tcanttggtg tggttcctcn tgcaacaaat 600
atgaacgtct gaaggcaaac cangtancta ctggcattcg gtncaatgaa aggaaaggaa 660
ggtctgaact aattgtc 677

<210> 630
<211> 665
<212> DNA
<213> Homo Sapiens

<400> 630
gagacagagt ctctgttgcc caggctggag tgtggtggcg caatcctggc tcactgcaac 60
ctcagcctcc tgggttcaag caattttcct gtctcagcct tccaagtagc agggactaca 120
ggcgtgcacc accacgcgca gctaattttt ttgtattttt agtaaaggcg aggtttcgcc 180
atgttggcca ggctggtctc gaaatcctga ccccagtgat ctgcctacct catcctctca 240
aagtgctggg attacaggtg tgagccaccg cgcccagcct taattttcaa aagacaaata 300
agcaaaaagc ttttcccgtt cctctcccaa aacagcaatg agataactgc cttgtaatgt 360
ttgtttgctt tttacaaata ccaatttacc acttgctgga atcccagccc aggaaccagc 420
ctgtgaatgt gggtggctca tggccctgtt ttatgatgac aattggtgtc ctcttgtctc 480
ttccagaagg gtctgtctca aggtacattt tggcanactt caaagattct tttttctcaa 540
cttcattagc atctttgcca atccaaataa atatctgttc ccaagcatct agtaacatga 600
catcatcttc agctaaatca tcctgggtga actctccctg gaatctcttc aataacaaat 660
ctccc 665

<210> 631
<211> 698
<212> DNA
<213> Homo Sapiens

<400> 631
ctgaggagct ggtggtcttt gaggatttga atgtatttca ctgccaggaa gaatgtgtga 60
gcttggatcc tactcaacaa ctcacgtcag agaaggaaga tgacagcagt gtcggggaaa 120
tgatgttact ggtcaatggc agtaatcctg aaggtgaaga tcctgagagg gaacctgtan 180
aaaatgaaga ttatagagaa aagtcttcag atgatgatga aatggattct tccttggtct 240
ctcagcagcc tcccgataac caggaaaagg aacgactaaa tacatccatt ccacaaaaaa 300
ggaaaatgag aaatctgtta gttaccattg agaatgatac tcctctagag gaactctcaa 360
aatatgtaga catcantatt attgccctta ctcgaaatcg gaggacaagg agatggtaca 420
cttgtccact gtgtgggaaa cagtttaatg aaagttctta cctcatttcc caccagagga 480
cccacactgg agaaaaaccc tatgactgtn ntcactgtgg gaaaagcttc aatcatnaaa 540
caaacctcaa taaacatgag cgaattcnta caggagagaa accttattcc tgttctcagt 600
gtggaaaaaa cttccgtcng aattctcatc ggagtcgtcc tgaaggaatc catntaacgg 660

agaagatatt aagtgtccan aatgtgggaa aacctccc 698

<210> 632
<211> 466
<212> DNA
<213> Homo Sapiens

<400> 632
atcacaaatt gtaaatatta ttgaaattga ttgcaaattt agatcacata caaatgagag 60
tctgacattc aactgttttc ctatattcca aagtaaacaa ttcctttcaa cactcaagac 120
ttaaacaggt attcttagag ggttatatga attgctatca gaagctgttg gctaacaagc 180
cagtaatttg gttctttcac canaacacag ttccagataa gcatctttgc actatttctc 240
aantatgaat ccccatgtgg ggggaaaacg gatatacttt caatagacac aagtcactct 300
ttgccttcca agtaagcana ctccagattc atcttcaaag tgttgggaaa ngggatctgt 360
gacctgtnca ttatcatata acttcaaaaa ggaaagctcc ttantccaaa aagcctanat 420
gctgaggtat agcccttgaa atgttttctt ccctgtnaat ttccta 466

<210> 633
<211> 734
<212> DNA
<213> Homo Sapiens

<400> 633
cacatacagt ctttgtttta atgtttattg gtagaaacag atcttcaatg catactttgt 60
gtttatataa actctacatt ctcttaaagg ttttcgtttt gttttcactg gagattttta 120
gcctccaagt gaacttaaca tattgcctat gcatctgatt ctttatanac ttttanattt 180
taaaactaaa tttganaaac catgcatact gtatacctta tttaataatc caaanaattg 240
tttgcacttt caaaaaagtt acaaaaaggc tgaacacaag ttaaataacc tatatgatgt 300
aaattttcca tttctgaata ctttttcagt attatatatt gcttgctgtc taataagtta 360
gattgtcaga nacgcttcag taaattatct ctactttaaa attatatctg aatccccttt 420
ctctganatg aacttgccaa tattaaacat tgtgccatat gcagtattan cccaaaagct 480
taaataagaa ccaaacttgt agactgaata ttttaacctt aaaattatat acctatatat 540
ncacctatgg tatgctgcat attaaattta acatttcaag taacatatat atagcaaaca 600
ttcagccaaa tactctttca tgaaaagata ctgtccttaa aataaaaagt tantgaaaag 660
cttatttaga ccnaatgtct aaatataant nctaagccta tgaaacttga anctaaagtc 720
tgctgtncta ttta 734

<210> 634
<211> 822
<212> DNA
<213> Homo Sapiens

<400> 634
ctcctgtgct tacacctgta gaaaaacacc agagcagaga gtatctcaag tgatgaagag 60
gttcatgaat ctgtggattc agacaatcag caaaataaaa aagttgaagg tggatatgaa 120
tgtaaatatt gtacttttca aactccagat ctaaatatgt ttacttttca tgtggattcg 180
gaacatccca atgtagtgct aaattcatcc tatgtttgtg tcgaatgcaa ttttcttacc 240
aaaaggtatg atgcactttc tgagcataat ctgaaatatc acccaggaga agagaatttt 300
aagttgacta tggtgaaacg taataaccag acaatctttg aacaaacaat aaatgatctg 360
acttttgatg gtagttttgt taaagaggag aatgcagagc aagcagaatc tacagaagtt 420
tcttcttcgg gaatatctat cagtaaaact cctatcatga aaatgatgaa aaataaagtg 480
gaaaataaac ggattgcagt tcatcataac tcagttgagg acgttcctga agagaaagag 540
aatgaaatca aaccagaccg tgaagaaatt gtagaaaatc caagttcttc agcttctgaa 600
tctaatacaa gtacttccat tgtaaacaga atacatccaa gtactgccag cacggtagtg 660
acccagcagc agttcctccc tggattgggc ccaggtgata actgctgtnt ctgctccgcc 720

agaattctaa tttgattccc naagtcttaa tccctgttna tancatcccc cctacaatgc 780
tgcnttggat aacaaccccc tttttactta acccctacan cc 822

<210> 635
<211> 819
<212> DNA
<213> Homo Sapiens

<400> 635
acccatttct aacaattttt actgtaaaat ttttggtcaa agttctaagc ttaatcacat 60
ctcaaagaat agaggcaata tatagcccat cttactagac atacagtatt aaactggact 120
gaatatgagg acaagctcta gtggtcatta aaccccctca gaaagtctaa gattcagaat 180
gtctccatca tattagaaga aaaatgtact gtattaaaat ttaaattgca tttttacaag 240
ttgtttttta attagtgttc tatttacatt gcanaacttc caccaactgc agtagtttaa 300
ctttggcaca acattaagtt ccatttcttt tgggtattgg atcctgcttt ttgagtgtgt 360
atgccccaaa acgttttcaa tgtcatcaaa gattgggcaa attcacagta aatcagacat 420
cttgagttga agaattgatt ctccttcaac gttttaggca gatttcagtc atctgattta 480
gacagcttcc gtttcacatg tcgtggaagt cccaagtgtc actatcatct gtttcttctt 540
catcctcttc ctggtcatca ataacttcat cttcctcctc attttcctca aataattcta 600
tacctaattc tgatcttctc tgtctttctg caaaccactc tctgacctgc tcatanccca 660
tatgtgattt gttaacaaat tcatcaaggt cttgctcatt aaaaaacttg tgcttcaggt 720
tataatcctt aanttttgcc gttccagttt taaattttat gaatnaatgg tcccctggtc 780
cccagttgtt aattcctttt ggctcctcca aggcgccca 819

<210> 636
<211> 704
<212> DNA
<213> Homo Sapiens

<400> 636
aaaaagttat ttatttattc tttttttttt tttttttttt ttggtaaggt tgaatgcact 60
tttggttttt ggtcatgttc ggttggtcaa anataaaaac taantttgan anatgaatgc 120
aaaggaaaaa aatattttcc aaantccatg tgaaattgtc tcccattttt tggcttttgg 180
gggggttcag tttgggttgc ttgtctgttt ccgggttggg gggaaagttg gttgggtggg 240
aggganccag gttgggatgg agggagttta caggaagcan acagggccaa cgtcnaagcc 300
naattcctgg tctggggcac caacgtccaa gggggccaca tcnatnatgg gcaggcggga 360
ggtcttggtg gttttgtatt caatcactgt cttgccccag gctccggtgt gactcgtgca 420
nccatcgaca gtgacgctgt aggtgaancg gctgttgccc tcggcgcgga tctcgatctc 480
gttggaaccc tggagganca gggccttctt gaggttgcca gtctgctggt ccatgtaggc 540
cacgctgttc ttgcantggt angtgatgtt ctgggagcct cggtggacat caggcgcagg 600
aaggtcacct ggatgccaca tcngcanggt cggaaccctg gccgccatac cccaactggg 660
aatccatcng tcatgctctc cccgaaacaa aacatcctct tgtt 704

<210> 637
<211> 693
<212> DNA
<213> Homo Sapiens

<400> 637
gaaagcaaat ttcttttaat ganaactcaa aattaaactt caaagggacc caacgtcata 60
cttccattca gggacttgat acaaaaaatt tagtttgaac tgctattagc aggtggcagg 120
agccaccttc aaatgaatct tcaaattgga aaatactgct tcaccacctg ttggggataa 180
nttgcaaatg gaataattta gtatggtttg tagctatttt gatnaccacc tcgcctgnat 240
accttcccat aaccactctg ctggtcacca cctcttccac aagctcttcc tgcaaatcct 300
cctctaaatc cccactgttg ctgttgctga tattgtncct tcgacatggc tacttttatt 360

tcacatttac taaaaccaac attgtggtat ttcttttcca ttatcttctt cactggttct 420
tcttccttaa aggtaataaa gcaaaaccca cgcctcttat tggtcttgtt gtccatgggg 480
agctctatgg attccacctc accaaaacca ccaaagtact cccttatttt ctcttcaggt 540
gtatctggan aaaggccacc ancnaaaatt ttttaaccgg ctcttttgtt tccatggctt 600
tgggcctttt angatcaatc accttcccca ttcaatttat gttctttttg gatccatgaa 660
cctttntcta cnccctcccg aattccttaa ata 693

<210> 638
<211> 619
<212> DNA
<213> Homo Sapiens

<400> 638
gcactctgaa gttagatcct atcacagggc gatcaagggg ttttggcttt gtgctattta 60
aagaatcgga gagtgtanat aaggtcatgg atcaaaaaga acataaattg aatgggaagg 120
tgattgatcc taaaagggcc aaagccatga aaacaaaaga gccggttaaa aaatttttgt 180
tggtggcctt tctccagata cacctgaaga gaaaataagg gagtactttg gtggttttgg 240
tgaggtggaa tccatagagc tccccatgga caacaagacc aataagaggc gtgggttctg 300
ctttattacc tttaaggaag aagaaccagt gaagaagata atggaaaaga aataccacaa 360
tgttggtctt agtaaatgtg aaataaaagt agccatgtcg aaggaacaat atcagcaaca 420
gcaacagtgg ggatctanag gaggatttgc angaagagct cgtggaagan gtggtggccc 480
cactcaaaac tggaaccang gatatantna ctattggaat cnaggctatg gcaactatgg 540
atatnacagc ccaggttacc gtggttntgg aagatatgac tncactggtt acnacaacta 600
ctatggatat ggtgattat 619

<210> 639
<211> 694
<212> DNA
<213> Homo Sapiens

<400> 639
cggcggcgcc attaaagcga ggaggangcg agagcggccg ccgctggtgc ttattctttt 60
ttagtgcagc gggagagagc gggagtgtgc gccgcgcgag agtgggaggc gaagggggca 120
ngccagggan aggcgcagga gcctttgcag ccacgcgcgc gccttccctg tcttgtgtgc 180
ttcgcgaggt acagcgggcg cgcggcancg gcggggatta ctttgctgct agtttcggtt 240
cgcggcagcg gcgggtgtat tctcggcggc agcggcggag acactatcac tatgtcggag 300
gancanttcg gcggggacgg ggcggcggca ncggcaacgg cggcggtagg cggctcggcg 360
ggcgaacang angganccat ggtggcggcg acacangggg cancggcggc ggcgggaacn 420
gaccgggacc gggggcggaa ccgcntctgg angctccnaa gggggcnncg ccnaatccga 480
aggggcgaaa attgaccccg tatgaaccaa gaagatgaat ggaaaatgtt tatangaagc 540
cttanctggg acactnccca gaaagatctg aaggactact tttccnaatt ttgggtgaaa 600
ttgttaaact gccctcttga aattttnatn ctatcccngg ggcnatcaaa ggggtttttg 660
gcttttttcc tattttaaac aaatcccgaa aaat 694

<210> 640
<211> 728
<212> DNA
<213> Homo Sapiens

<400> 640
cgccactgcn gcaggaggcg tgaggggata aaaacattca gatggcagat cacagttttt 60
cagatggggt tccttcagat tccgtggaag ctgctaaaaa tgcaagtaac acagaaaagc 120
tcacagatca ggtgatgcag aatcctcgag ttctggcagc tttacaggag cgacttgaca 180
atgtccctca caccccttcc agctacatcg aaactttacc taaagcagta aaaagaagaa 240
ttaatgcatt gaaacaactt caggtgagat gtgctcacat agaagccaag ttctatgaag 300

aggtacatga cttggaaaga aagtatgcag cgctatacca gcctctcttt gacaagagaa 360
gagaatttat caccggcgat gctgaaccaa cagatgcgga atcggaatgg cacagtgaaa 420
atgaagagga agagaaattg gctggagaca tgaaaagtaa agtagtcgtc acagaaaaag 480
cagcggcaac ggctgaagag ccagatccca naggaattcc agagttctgg tttaccatct 540
tcagaaatgt ggacatgctg agtgaattan tccaggaaat atgatgaacc aatcttgaaa 600
acacctgcag gatnttaaag ttgaaatttt ctgaccctgg acagcctatg tcttttgtgt 660
tagaattcca ctttgaaccc cacgactact ttaccaactc agtcctgaca aaaaccttac 720
cagatgaa 728

<210> 641
<211> 732
<212> DNA
<213> Homo Sapiens

<400> 641
   acctaattag atagaagttc aggaatttct atttcttttg ggttgatgaa ccacaggcta 60
   gcataagtcc actgtcaata aatgtttgtt gtggccagac ctccataaaa gagatattcc 120
   ctgtgttcac aagttccctg aagcttaggt tttgagagaa tattgttgag tcactaggca 180
   gggctcacat aggaaactgg caatcacctc tgaaactgct tcacagacac ctgcttttcc 240
   tgctctgttc ctcanacttc tcctcttcaa gcgtattccc cccacaacaa ggacagcagc 300
   ttggactaca tatctggctg atgatgtaat aaaaagatta ggcatggggg tttcctaagc 360
   cacaattcag ggccactctg caccaacaga gataagcacc caggtggaag ccccccttcc 420
   ccgagcctca tacattgtca tcatcttcta tggcctcccc agtgaagtac agcacagccc 480
   gcgggactat ccgctcacgg aaaaagtgtc caatttcaaa atcagaagct aatgtgaatt 540
   caaaatcttc atccagtgat ctccatcccc ggatgctttc caatggattg aagaaattga 600
   aaaaggactc attgggtact gtttcgtaat tgttctaaca gtgcctcaac cttatgcttc 660
   tgctttncnt ggaaggtntt gaaagtaaca ttcttncctt cttccaantc aattattnac 720
   ccccgttcac aa 732

<210> 642
<211> 582
<212> DNA
<213> Homo Sapiens

<400> 642
gcttttcttc ttcctcctct tctgattctg gctcttgctt tatttgtggc tgcctgcgcc 60
tctcagcctc ttgttccatc ctctggagct ctgcatctgg atctggatgc ccttctgcca 120
naaggcgctg cctgatttcc tcaagctcct ccttctctct cttcctatct cgttcatctg 180
cttccatttc cttttctcta tcacgcaacc ttttctgaaa agcacttcct ctgtaatatt 240
tggggtcatc tctatcatca tcatagtctt ctaanaattc ttttagtcgt ttagcttctt 300
tggccatttc tcttcttctt tcttcttctc tttcagcttc tttctcatat tcccgggttt 360
tctttcgttc tctgatttcc caattcttaa ggcgctcttg ataancagct tctttctctc 420
gganttttct ttcaagtttt cttcgttcgt atgcatcttc ttcatcttct tctcggtccc 480
gttttttgtc tttttctctt tctcgctccc gttcctctct cnctctctct ctcgctcccg 540
ttctcgttct cgctctcntt ctctctctct ctctctttcc cg 582

<210> 643
<211> 784
<212> DNA
<213> Homo Sapiens

<400> 643
aagaaaagct caagtttcca agtctcttaa gggagcgatt aggcatgtca gctgatccag 60
ataatgagga tgcaacagat aaagttaata aagttggtga gatccatgtg aagacattag 120
aagaaattct tcttgaaaga gccagtcaga aacgtggaga attgcaaact aaactcaaga 180

cagaaggacc ttcaaaaact gatgattcta cttcagggag caagaagctc ctccactatc 240
cgtatcaaaa ccttctctga ggtcctggct gaaaaaaaac atcggcagca gggaagcaga 300
gagacnaaaa agcnaaaagg gatacaactt gcatcaagct aaagattgat agtgaaatta 360
aaaaaacagt agttttgcca cccattgttg ccagcagagg acaatcagag gagcctgcag 420
gtaaaacaaa gtctatgcag ggaggtgcac atcaagacgc tggaaganat taaactggag 480
anggcactga gggtgcagca gagctctgag agcagcacca gctccccgtc tcaacacgag 540
gccactccag ggggcnaggc ggctgctgcg aatcaccnnn agaaccggga tgaaagaaga 600
gaagancctt ccgggaaggg aatgaagttg attctcagag cngtattaga acngaagcta 660
aagangctcc gggtgagaac nccggggttg acctccctaa aattccagtc cagagatgtn 720
agacctgaaa gagaacccct gccganaccg ccgggaaagg ganaaatccg tcttgacccc 780
cttc 784

<210> 644
<211> 749
<212> DNA
<213> Homo Sapiens

<400> 644
cctacatcag ttttatttaa aacacaaaca agtatttctc tttctgtaag ggcaaatggt 60
tcaaataatg cggaacacga aacattgact aatacaagtg ctttaaatat gaaacaaaat 120
tattttttaa aaaagcaaaa naataaagaa tatatacaaa agggacctgn aatctgtaag 180
gtgattccaa aaacnaaata antagaaaat ccatggtgaa acctgaacat tctacctctg 240
ctttggagaa gggctatcat acaacattca gtcagctgaa natggattgg tanaggtgtg 300
tctatacata aacttcagtc atttttgctt gtgcanaatc atcccaatct tcccaanact 360
gaatgggcag tcctgtggct ttcttccttt tccatattcc caacaaggct acgtgaagtt 420
caactcttga tgagccgctt acaacagcag ttccttaggg agccaacatg acaggtgggt 480
canatttccc tatgagaaac aaaactggcc acctacagca aaatatcaaa atgggtaagt 540
ccttccttcc tcttcctcct gattatatac aacatatctc ctttcaagac tattatttcc 600
atcatgctta ttccttcaca aatctaaacc ttgaggtgat atgaaggaaa ccancntcaa 660
aaaaaagaaa actcaattcc gaaatgaana aaactgggcn nggtatncaa tacnccccan 720
aacatctcca tatccctggc ccagttacc 749

<210> 645
<211> 751
<212> DNA
<213> Homo Sapiens

<400> 645
agactttcct acatcagttt tatttaaaac acaaacaagt atttctcttt ctgtaagggc 60
aaatggttca aataatgcgg aacacgaaac attgactaat acaagtgctt taaatatgaa 120
acaaaattat tttttaaaaa agcaaaagaa taaagaatat atacaaaagg gacctggaat 180
ctgtaaggtg attccaaaaa cgaaataagt agaaaatcca tggtgaaacc tgaacattct 240
acctctgctt tggagaaggg ctatcataca acattcagtc agctgaagat ggattggtag 300
aggtgtgtct atacataaac ttcagtcatt tttgcttgtg cagaatcatc ccaatcttcc 360
caanactgaa tgggcagtcc tgtggctttc ttccttttcc atattcccaa caaggctacg 420
tgaagttcaa ctcttgatga gccgcttaca acagcagttc cttaggancc caacatgaca 480
ggtgggtcag atttccctat gagaaacaaa actggncacc tacagcaaaa tntcaaaatg 540
ggtaagtcct tccttcctct tcctcctgat tatntacaac atatctcctt tcaagantat 600
tatttccatc atgcttattc cttcccaaat ctaaaccttg aaggtgattt gaagggaaac 660
cnccatccnn aaaaagaaaa acccattccc aaattgaaaa aaaactnggc agggtataca 720
atacaccccc canaaactcn ccaattttcc c 751

<210> 646
<211> 760
<212> DNA

<213> Homo Sapiens

<400> 646
cctacatcag ttttatttaa aacacaaaca agtatttctc tttctgtaag ggcaaatggt 60
tcaaataatg cggaacacga aacattgact aatacaagtg ctttaaatat gaaacaaaat 120
tattttttaa aaaagcaaaa gaataaagaa tatatacaaa agggacctgg aatctgtaag 180
gtgattccaa aaacnaaata agtagaaaat ccatggtgaa acctgaacat tctacctctg 240
ctttggagaa gggctatcat acaacattca gtcagctgaa natggattgg tagaggtgtg 300
tctatacata aacttcagtc atttttgctt gtgcanaatc atcccaatct tcccaanact 360
gaatgggcag tcctgtggct ttcttccttt tccatattcc caacaaggct acgtgaagtt 420
caactcttga tgagccgctt acaacagcag ttccttagga gccaacatga caggtgggtc 480
aaatttccct atganaaaca aaactggcca cctacagcaa aatatcaaaa tgggtaantc 540
cttccttcct cttcctcctg attatataca acatatctcc tttcaagact attattccat 600
catgcttatt ccttcacaaa tctaaacctt gaagtgatat gaangaaacc nccntccaga 660
aaagaaaact cnantcanaa atgaaaaaaa ctggcaggta tncaatacac cccaaaacnt 720
ctcaatntcc tggcacanta caatccattg ttctgctaca 760

<210> 647
<211> 1041
<212> DNA
<213> Homo Sapiens

<400> 647
caaaggcgac agctgcccat tccgtcactg tgaagctgca ataggaaatg aaactgtttg 60
cacattatgg caagaagggc gctgttttcg acaggtgtgc aggtttcggc acatggagat 120
tgataaaaaa cgcagtgaaa ttccttgtta ttgggaaaat cagccaacag gatgtcaaaa 180
attaaactgc gctttccatc acaatagagg acgatatgtt gatggccttt tcctacctcc 240
gagcaaaact gtgttgccca ctgtgcctga gtcaccagaa gaggaagtga aggctagcca 300
actttcagtt cagcagaaca aattgtctgt ccagtccaat ccttcccctc agctgcggag 360
cgttatgaaa gtagaaagtt ccgaaaatgt tcctagcccc acgcatccac cagttgtaat 420
taatgctgca gatgatgatg aagatgatga tgatcagttt tctgaggaag gtgatgaaac 480
caaaacacct accctgcaac caactcctga agttcacaat ggattacgag tgacttctgt 540
ccggaaacct gcagtcaata taaagcaagg tgaatgtttg aattttggaa taaaaactct 600
tgaggaaatt aagtcaaaga aaatgaagga aaaatctaag aagcaaggtg agggttcttc 660
aggagtttcc aagtctttta ctccaccctg agcccgttcc aagtcctgaa aaagaaaatg 720
tcaggactgt ggtgaaggac agtaactctc tccaacaaac aanggagaaa gaanccttgg 780
gtagattgag tcctactgan agacggggga aacgaaaant tcagcaagcg gtgacaagtg 840
atcctccaat aaagcgttac cctgcacaaa ggctagggaa aaaaanttaa ancccanaaa 900
actaacattg acaaaaccac caaagaaagc tcaagnttcc aagtccccta agggaccgan 960
taagcatgtc aaccggatca anataatgng gntgcaacag ttaaagntta aaaaattggg 1020
gaaattcagt taaaacattt g 1041

<210> 648
<211> 810
<212> DNA
<213> Homo Sapiens

<400> 648
ccctacatca gttttattta aaacacaaac aantatttct ctttctgtaa gggcaaatgg 60
ttcaaataat gcggaacacn aaacattgac taatacaant gctttaaata tgaaacaaaa 120
ttatttttta aaaaagcaaa agaataaana atatatacaa aagggacctg naatctgtaa 180
gctgattcca aaaacnaaat aantanaaaa tccatggtga aacctgaaca ttctacctct 240
gctttggana agggctatca tacaacattc antcagctga aaatggattg gtaaaggtgt 300
gtctatacat aaacttcant catttttgct tgtgcaaaat catcccaatc ttcccaaaac 360
tgaatgggca gtcctgtggc tttcttcctt ttccatattc ccaacaaggc tacntgaant 420

tcaactcttg atnagccgct tacaacagca gttccttagg agccaacatg acaggtgggt 480
caaatttccc tatgaanaaa caaaactggc cacctacagc aaaatatcaa aatgggtaag 540
tccttccttc ctcttcctcc tgattatata caacatatct cctttcaaga ctattatttc 600
catcatgctt attccttcac aaatctaaac cttgaggtga tatgaaggaa accancatca 660
agaaaagaaa accaattcan aaatgaanaa aactggcagg tntacaatac accccanan c 720
atctcaatat ccctggcaca gttacaattc agtgttctgc tacagcccat aaaataaata 780
ttggcagctt gaataancnc attttttccc 810

<210> 649
<211> 656
<212> DNA
<213> Homo Sapiens

<400> 649
cccnacatca nttttattta aaacacaaac aattatttct cttnctgtaa gggcaaatgg 60
ttcaaataat gcgnaacaca aaacnttgac taatacaatt gctttaaata tnaaacaaaa 120
ttatttttta aaaaancaaa aaaataaaaa atatntacaa aagggacctg aaatctgtaa 180
nctnatncca aaaacaaaat aattaaaaaa tccatggtna aacctnaacn tnctacctct 240
gcttnggaaa agggctatca tacaacntnc antcanctna aaatggatng gtaaaggtnt 300
ntctatacat aaacttcant cattttngct tgtgcaaaat cancccaatc tncccaaaac 360
tnaatgggca ntcctgtggc ttnctncctt tnccatatnc ccaacaaggc tacttnaatt 420
tcaactcttn ataanccgct tacaacagca ntnccttagn anccaacatn acaggtgggt 480
caaattcccc tataaaaaac aaaactggcc ncctacanca aaatatcaaa atgggtaatt 540
ccttcctncc tctnccncct nattatatac aacatttctc ctttcaaaac tattattncc 600
atcatgcttn ttcctncaca aatctaaacc ttgangtgat ttgaaggaac cacctc 656

<210> 650
<211> 645
<212> DNA
<213> Homo Sapiens

<400> 650
   gaacttcccn acnncatttt tatttaaaac ncaaacaatt nttncncttn ctntangggc 60
   aantggtnca aatantgcgn aacncaaaac tttnactaat acaattgctt taaatntaaa 120
   ncaaanttat tttttaaaaa accaaaaaaa taaaaaatnt ttccaaangg gacctgaaan 180
   ctntaaccta atcccaaaaa caaaataatt aaaaannccn nggtnaancc tnaacntnct 240
   nccnctnctt tgnaaaaggg ctatcanaca acntncattc ncctaaaaat gnatnggtaa 300
   aggtttttct anacataaac ttcattcatt ttggcttntn caaaancacc ccaanctncc 360
   caaaactnaa tgggcnncct ntggcttnct ccctttccca tntncccaac aaggctactt 420
   naattncaac ncttnataac ccccttacaa caccattncc ttagnaccaa cataacaggt 480
   gggtcaaatt ncccnataaa aaacaaanct ggcccctncc ccaaaatncc caaatgggta 540
   ttcctncctn ccctcccccc ngnatatata caacatntcc cctttcanaa atatattccc 600
   ccacgcttat tccncccaaa nntaancctt gaagttattt aagga 645

<210> 651
<211> 780
<212> DNA
<213> Homo Sapiens

<400> 651
ccttgacctc ccaaagtgct gagattacag gcctgagcca ctgcaccttg ccttccttac 60
ctcttttctc cgacattttt atgtttctaa cattgaactc taaggaagct ggtgaacaaa 120
cacgccatat gtatgcagaa cacttaacag aattatgcta tgttgtctgt ttttgtttgt 180
atttcttgtc cttgctgaag attgacttga aatcttaaac taagttctcc ctctttatag 240
gcggtgacag tgatcctcca ttaaagcgta gcctggcaca gaggctaggg aagaaagttg 300

aagctccaga aactaacatt gacaaaacac caaagaaagc tcaagtttcc aagtctctta 360
aggagcgatt aggcatgtca gctgatccag ataatgagga tgcaacagat aaagttaata 420
aagttggtga gatccatgtg aagacattag aagaaattct tcttgaaaga gccagtcaga 480
aacgtggaga attgcaaact aaactcaaga cagaaggacc ttcaaaaact gatgattcta 540
cttcaggagc aagaagctcc tccactatcc gtatcaaaac cttctctgag gtcctggctg 600
aaaaaaaaca tcngcagcag ggaactgaag agacaaaaaa gccnaaagga tacaacttgc 660
atcaagctaa agattgatag tgaaattaaa aaaaacagta attttngcca cccattgttg 720
ccngcagaag acaatcanaa gaacctgcag gtaaaacaaa ntctatgcag ggaggtgccc 780

<210> 652
<211> 518
<212> DNA
<213> Homo Sapiens

<400> 652
gnacttncct acatcatttt tatttaaaac acaaacaatt ntttcncttt ctgtangggc 60
aaatggttca aataatgcgg aacacaaaac nttnactaat acaattgctt taaatntnaa 120
acaaaattat tttttaaaaa ancaaaaaaa taaaaaatnt ttncaaangg gacctgaaat 180
ctntaanctn atnccaaaaa caaaataatt naaaaatcca nggtgaaacc tnaacntnct 240
nccnctgctt tggaaaaggg ctntcataca acnttcattc ncctaaaaat ggattggtaa 300
angtttttnt atacataaac tncattcatt tttgcttntg caaaatcanc ccaanctncc 360
caaaactnaa tgggcantcc tntggctttc tncctttccc anatncccaa caaggctact 420
tnaatttcaa cncttnataa nccgcttaca acancatttc cttaggancc aacatnacgg 480
tgggtcaaat cccctataaa aaacaaaact ggccncct 518

<210> 653
<211> 490
<212> DNA
<213> Homo Sapiens

<400> 653
gttaataaag ttggtgagat ccatgtgaag acattagaag aaattcttct tgaaagagcc 60
agtcagaaac gtggagaatt gcaaactaaa ctcaagacag aaggaccttc aaaaactgat 120
gattctactt caggagcaag aagctcctcc actatccgta tcaaaacctt ctctgaggtc 180
ctggctgaaa aaaaacatcg gcagcaggaa gcagagagac aaaaaagcaa aaaggataca 240
acttgcatca agctaaagat tgatagtgaa attaaaaaaa cagtagtttt gccacccatt 300
gttgccagca gaggacaatc agaggagcct gcaggtaaaa caaagtctat gcagggaggt 360
gcacatcaag acgctggaag aaattaaact ggagaaggca ctgagggtgc agcagagctc 420
tgagagcagc accagctccc cgtctcaaca cnaggccact ccaagggcaa ggcggctgct 480
gcnaatcccc 490

<210> 654
<211> 359
<212> DNA
<213> Homo Sapiens

<400> 654
   cccanctccn ttttanttna aancccaacc aattnttccc cttccgntan gggcaatngn 60
   tccaattatn ncgaacncca aaccttnaan natnccaatt ncttaaatnt taaaccaaat 120
   tnntttttta aaaagccaaa naattaagaa ttttttccaa agggaacnng aatccnttag 180
   ggtaatccca aaaccaaatt agttaaaaat ccctggntaa acccnaacnt tccnccnccn 240
   ccttggaaaa agggnnnccn ncnaccttcc atncncntaa aaatgaatgg ntaaagnttt 300
   ttcnnncctt aacntccatc ctttttgnct nttccaaanc ctccccancc tccccaaaa 359

<210> 655

<211> 611
<212> DNA
<213> Homo Sapiens

<400> 655
tgaaaaaaaa catcggcagc aggaancaga aagacnaaaa agcaaaaagg atactacttg 60
catcangcta angattgata gtgaaattaa aaaaacagta tttttgccac ccattgttgc 120
cancagagga caatcanagg agcctgcagg taaaannaag tctatgcagg aggtgcacat 180
caagacgctg gaagaaatta aactggagaa ggcactgagg gtgcagcana gctctgagag 240
cagcaccagc tccccgtctc aacacnaagc cactccatgg gcnangcggc tgctgcgant 300
cnccnaaaga ncagggatga angaagagaa gaaccttcag gaaggaaatg aatttgattc 360
tcagancatt attataactg aagctnnana ngcttcnggt gagaccacng ganttgacat 420
cactaaaatt ccagtcaaga gatgtgagac catgagagag aagcacatgc acaaaacanc 480
nngagaggga aaaatcagtc ttgacacctc ttcggggaga tgtagcatct tgcggnaccc 540
aantggcaga gaaaccagtg ctcactgctg tgccaggaat cacncggcac ctgaccaagc 600
ggcttcccac a 611

<210> 656
<211> 634
<212> DNA
<213> Homo Sapiens

<400> 656
ccnacatcag ttttatttaa aacacaaaca agtntttcnc tttctgtnag ggcaaatggt 60
tcaaataatg cggaacacna aacattgact aatacaantn ctttaaatat gaaacaaaat 120
tattttttaa aaaancnaaa naataaagaa tatntncaaa agggacctgg aatctgtnag 180
ctgattccaa aaacnaaata anttnaaaat ccntggtgaa acctgaacat tctacctctg 240
ctttggaaaa gggntatcat acaacattca gtcngctgaa aatggattgg taaaagtntn 300
tctatacata aacttcagtc atttttgctt gtncaaaatc atcccaatct tcccaaaant 360
gaatgggcag tcctgtggct ttcttccttt tccatattcc caacaaggnt acntnaantt 420
caactcttga nnanccgctt acaacagcag ttccttagga nccccatgac aggtgggtcn 480
aatttcccta tnaaaaacaa aactgggccc tacagcaaaa tatccaaatg ggtnagtcct 540
tccttcctct tcccctgant atatacacat atctcctttc aanaatanta tttccccatg 600
cttattcctt cccnaatcta aaccttgaag tgat 634

<210> 657
<211> 958
<212> DNA
<213> Homo Sapiens

<400> 657
gaaagaaaag catcatgtaa aaatgaaagc caagagatgt gccactcctg taatcatcga 60
tgaaattcta ccctctaaga aaatgaaagt ttctaacaac aaaaagaagc cagaggaaga 120
aggcagtgct catcaagata ctgctgaaaa gaatgcatct tccccagaga aagccaaggg 180
tagacatact gtgccttgta tgccacctgc aaagcagaag tttctaaaaa gtactgagga 240
gcaagagctg gagaagagta tgaaaatgca gcaagaggtg gtggagatgc ggaaaaagaa 300
tgaagaattc aagaaacttg ctctggctgg aatagggcaa cctgtgaaga aatcagtgag 360
ccaggtcacc aaatcagttg acttccactt ccgcacagat gagcgaatca aacaacatcc 420
taagaaccag gaggaatata aggaagtgaa ctttacatct gaactacgaa agcatccttc 480
atctcctgcc cgagtgacta agggatgtac cattgttaag cctttcaacc tgtcccaagg 540
aaagaaaaga acatttgatg aaacagtttc tacatatgtg ccccttgcac agcaagttga 600
agacttccat aaacgaaccc ctaacagata tcatttgagg agcaagaagg atgatattaa 660
cctgttaccc tccaaatctt ctgtgaccaa gatttgcaga gacccacagg actcctgtac 720
tgcaaacaan acaccgtgca cgggctgtga cctgcaaaaa gtacagcaga gctggaggct 780
gaggagctnc gagaaattgc aaccantaca anttccaaag cacgtngaac cttgattccc 840

agaataactt gangggtggg cccaaccttg cccaagaaaa ccaccngtga aancaancca 900
acggagccct antnggcttt gatttgggaa tttgggaaan gaatncaagg gaggngag 958

<210> 658
<211> 816
<212> DNA
<213> Homo Sapiens

<400> 658
gggagggaaa gacaaaacgt atttattcca ggccaggtct taaaatgcac actgcacggt 60
tccctgttgt tatcagcacc agtaaggaaa gaacgtgcct taacggcagc cccacccaga 120
gcctgctgcg tggctgctgt gaggctcccc atgaatccac gcagtcttct tcctcactgg 180
tgcagttggt gaggttttct accctcacag caaagggatc cttaactata aattcacggt 240
atgcagagaa gaggacagaa tctgatttac tgattgttcc tcatttaaac catgacttaa 300
tctctatctt aggatttaac tatctttatt ttctggttaa aatttttaaa aaaagtgggg 360
agagggtgag agtcgtaagg ggcaatagca atagagatta cactgtgctg acacagagac 420
taaattctag tcagagtgaa gaccatataa aaggccggct gatggtttaa aggaagtaac 480
tacatggagt ctaatcgaga cattcatgan ttacatctca ttattagcct tagtaatgta 540
agaaaacaat tctcaacaaa actgggagtc cacagttgtc aagtatgctt tctcangcac 600
gggtaggtaa aagtctggan aaatgggttc tctccatgcc caatgacaaa gcaagacggt 660
cctaggtttg aagttaaaaa caggtcccaa ttgcccgggc ggtatccgcc agctcacagc 720
tgaatttaan catggaaatc caatggaaaa attggganat acnggcacat tcanaaggct 780
ggtcctttga cttatctcca naacccgggt actggc 816

<210> 659
<211> 726
<212> DNA
<213> Homo Sapiens

<400> 659
tgagaaaagt ggtacaaata ctgggaaaaa cctgctcttc tgcgttaagt gggagacaat 60
gtcacaagtt aaaagctctt attcctatga tgccccctcg gatttcatca atttttcatc 120
cttggatgat gaaggagata ctcaaaacat agattcatgg tttgaggaga aggccaattt 180
ggagaataag ttactgggga agaatggaac tggagggctt tttcagggca aaactccttt 240
gagaaaggct aatcttcagc aagctattgt cacacctttg aaaccagttg acaacactta 300
ctacaaagag gcagaaaaag aaaatcttgt ggaacaatcc attccgtcaa atgcttgttc 360
ttccctggaa gttgaggcag ccatatcaag aaaaactcca gcccagcctc agagaagatc 420
tcttaggctt tctgctcaga aggatttgga acagaaagaa aagcatcatg taaaaatgaa 480
agccaagaga tgtgccactc ctgtaatcat cgatgaaatt ctaccctcta agaaaatgaa 540
agtttctaac ancacaaaga agccagagga agaaggcagt gctcatcaag atactgctga 600
aaagaatgca tcttccccaa gagaaagcca agggtagaca tactgtgcct tgtatgccac 660
ctgcanagca gaagtttcna aaangtactg angagcaang aatctggaga agagtatgaa 720
aaatgc 726

<210> 660
<211> 824
<212> DNA
<213> Homo Sapiens

<400> 660
aggatttaac tatctttatt ttctggttaa aatttttaaa aaaagtgggg agagggtgag 60
agtcgtaagg ggcaatagca atagagatta cactgtgctg acacagagac taaattctag 120
tcagagtgaa nacccatata aaaggccggc tgatggttta aaggaagtaa ctacatggag 180
tctaatcgag acattcatga gttacatctc attattagcc ttagtaatgt aagaaaacaa 240
ttctcaacaa aactggagtc cacagttgtc aagtatgctt tctcaggcac gggtaggtaa 300

aagtctggan aaatgggttc tctccatgcc caatgacaaa gcaagacggt cctaggtttg 360
aggttaagan caggtcccat tgccgggcgg tatccgcagc tcacagctga ntttagcagt 420
ggaatcgagt ggagaatttg gggagataca ggcncagtca gaggctggtc acttgacttt 480
atctccagac cctggtactt gcgtattgga tttgccttat gcaccagttc tctccgtagc 540
ctggccanct cctctttttt ctgctcttcc tcctgtagtc tggcctcctc caactgctgg 600
gctttctggg cttctacctc agccattctc ttctccagct ccctgccgct ctttggctct 660
ctctcagtag cccactgaaa angtccctga acnaaaaaaa ccanaaanng gccctcacaa 720
ctgatttcnt ctctttcttg ggaaccaagg ggcccctgaa aaaanaaacg gtgtttggaa 780
caaaccntga aacaagcngc ctccttctgc ctgtcccaat tcct 824

<210> 661
<211> 399
<212> DNA
<213> Homo Sapiens

<400> 661
ggtttgnagg gaaaaanaaa actttttttt cccagnccag ttcttaaant nccccnngcn 60
nggtcccctn tntttttcnc ccccattaag gaaaaaactt gcntnancgg nagccccccc 120
caaaacctnc tgcttggctg ctttaaggnc cccataannc cccccatnnt cctcccccac 180
tggtncattg gtnaggtttc ctcccccccn ccaaaggnnt ccttacntat aaatcccngg 240
tttncaaaaa aaaananaaa accaatttcn gatnntcccc cttnaancca gnacttaatc 300
cctntctnag gattnaacaa cctttttttn cgggttaaaa tttttaaaaa aattngggaa 360
anggttaaat ccttaggggg aatnccnata aaaattacc 399

<210> 662
<211> 826
<212> DNA
<213> Homo Sapiens

<400> 662
gtcaaatgct tgttcttccc tggaagttga ggcagccata tcaagaaaaa ctccagccca 60
gcctcagaga agatctctta ggctttctgc tcagaaggat ttggaacaga aagaaaagca 120
tcatgtaaaa atgaaagcca agagatgtgc cactcctgta atcatcgatg aaattctacc 180
ctctaagaaa atgaaagttt ctaacaacaa aaagaagcca gaggaagaag gcagtgctca 240
tcaagatact gctgaaaaga atgcatcttc cccagagaaa gccaagggta gacatactgt 300
gccttgtatg ccacctgcaa agcagaagtt tctaaaaagt actgaggagc aagagctgga 360
gaagagtatg aaaatgcagc aagaggtggt ggagatgcgg aaaaagaatg aagaattcaa 420
gaaacttgct ctggctggaa tagggcaacc tgtgaagaaa tcagtgagcc aggtcaccaa 480
atcagttgac ttccacttcc gcacagatga gcgaatcnaa caacatccta ngaaccagga 540
ggaatataag ggaagtgaac tttacatctg aactacgaaa gcatccttca tctcctgccc 600
gaantgacta aggggatgtt ccattgttaa gcctttcaac ctgtcccngg gaaagaanag 660
aacntttgat gaaacagttt ctacatatgt gccccttgcc cngcaagttg aagacttccn 720
taancgaacc ctnactgatt tcttttgang aaccagaang gntgattttn ccctgtttcc 780
ctccaatctt ctgtgaacaa gatttggccg aanacccccg aacccc 826

<210> 663
<211> 770
<212> DNA
<213> Homo Sapiens

<400> 663
gggaaagaca aaacgtattt attccaggcc aggtcttaaa atgcacactg cacggttccc 60
tgttgttatc agcaccagta aggaaagaac gtgccttaac ggcagcccca cccanagcct 120
gctgcgtggc tgctgtgagg ctccccatga atccacgcag tcttcttcct cactggtgca 180
gttggtgagg ttttctaccc tcacagcaaa gggatcctta actataaatt cacggtatgc 240

anagaanagg acagaatctg atttactgat tgttcctcat ttaaaccatg acttaatctc 300
tatcttagga tttaactatc tttattttct ggttaaaatt tttaaaaaaa gtggggagag 360
ggtgagagtc gtaaggggca atagcaatag agattacact gtgctgacac agagactaaa 420
ttctagtcag agtgaagacc catataaaag gccggctgat ggtttaaagg aagtaactac 480
atggagtcta atcgagacat tcatgagttn catctcatta ttagccttag taatgtaaga 540
aaacnattct caacaaaact ggagtccaca gttgtcaant ntgctttctc aggcacgggt 600
aggtnaaaat ctgganaaat gggttctctc catgcccaat gacaancaan anggtcctag 660
gtttgaagtt aaaaacangt cccattgccg gcggtatccg cagctcacag ctgaatttac 720
cngtggaatc aantggaaaa tttgggaaaa tacnggccca atcaaaaggt 770

<210> 664
<211> 593
<212> DNA
<213> Homo Sapiens

<400> 664
gaaganctga gcagcacagc actggtgaag aagagctgcc tggcggagct cctccggctt 60
tacaccaaaa gcagcagctc tgatgaggag tacatttata tgaacaaagt gaccatcaac 120
aagcaacaga atgcagagtc tcaaggcaaa gcgcctgagg agcagggcct gctacccaat 180
ggggagccca gccagcactc ctcggcccct cagaagagcc ttccagacct cccgccaccc 240
aagatgattc cagaacggaa acagcttgcc atcccaaaga cggagtctcc agagggctac 300
tatgaagagg ctgagccata tgacacatcc ctcaatgagg acggagaggc tgtgagcagc 360
tcctacgagt cctacgatga anaggacggc agcaagggca agtcggcccc ttaccantgg 420
ncctcgccgg aggccggcat cganctgatg cgtgacgccc gcntctgcgc cttcctgtgg 480
cgcaagaaag tggctgggac agtgggccaa gcagctctgt gtcatcnagg acaacaggct 540
tctgtgctnc naatcctcca aggaccccng ccctcagctg gacgtgaacc tac 593

<210> 665
<211> 1024
<212> DNA
<213> Homo Sapiens

<400> 665
aagagattga agcaaatgaa tggaagaaga aatacgaaga gacccggcaa gaagttttgg 60
agatgaggaa aattgtagct gaatatgaaa agactattgc tcaaatgatt gaagatgaac 120
aaaggacaag tatgacctct cagaagagct tccagcaact gaccatggag aaggaacagg 180
ccctggctga ccttaactct gtggaaaggt ccctttctga tctcttcagg agatatgaga 240
acctgaaagg tgttctggaa gggttcaaga agaatgaaga agccttgaag aaatgtgctc 300
aggattactt agccagagtt aaacaagagg agcagcgata ccaggccctg aaaatccacg 360
cagaagagaa actggacaaa gccaatgaag agattgctca ggttcgaaca aaagcaaagg 420
ctgagagtgc agctctccat gctggactcc gcaaagagca gatgaaggtg gagtccctgg 480
aaagggccct gcagcagaag aaccaagaaa ttgaaggaac tgacaaaaat ctgtgatgag 540
ctgattgcaa agctgggaaa gactgactga gacactcccc ctgttagctc aacagatctg 600
catttggctg cttctcttgt gaccacaatt atcttgcctt atccaggaat aattgcccct 660
ttgcaganga aaaaaatata cttaanaaaa gcacatgcct actgctgcct gtcccgcttt 720
gctgccaatg caacagccct ggaagaaaac cctatanggn tgcatagtct aaaaagggag 780
ttgtngactn gacagtgctg ggagcctnct agtttccccc cnatgaaagg ttcccttagg 840
ctgctgagtt tggggtttgt gatttaacct taagtttgtt ttaaagtcca ncttaacttt 900
cccaaattgt gtttaaaatt tgtaacnccc cctttggggt cttcccaaca accggtccga 960
tttttttggn gatcggttta acccttttaa ttttttagta nccagtgggg tttaatttag 1020
ggga 1024

<210> 666
<211> 734
<212> DNA

<213> Homo Sapiens

<400> 666
gagacaagat cttgctgtca cccaggatgg agtgcagtgg catgatcatg gctcactgca 60
gccttgacct cccaggctcc cacctcagcc tcccaagtag ctgggaccac aggcacgtgc 120
caccatgccc agctaatttt tattttggta nanacaaggt ttcaccatgt tgcctaggta 180
ggtttcaaac tcctggactc aagtgatcct cctgcctcgg ccttccacag tgttgggatt 240
acaggaataa gccactgtgc ccggcccttt ttctcttctg taacagantt tattactgcc 300
tagctagcag gttatttggc cctcacatgt gttgaggcaa actctatact atattcttac 360
tctccanagt tccaaaatcc tttattttta aanaaaaata aacaaacata cttcattctg 420
cccagtatat tctcttgatc tgtacaagct acgattttaa ttctctttgg gagaggaagc 480
atctgttaag ttcgaatggg ggatatttcc tcataacggt catggctgan aagccaggac 540
aattatcact taacgaaggt cctttggtgc tccctgtgca tcagcttcat tcactggggt 600
caggttctta aggggtctct tccaccaatg tgctagggaa gggctgccat cacctctgtt 660
taacacatag ctactttctt aaaccnataa gcttaaaaaa gangactatg gaattaccaa 720
tggaaggcnt ataa 734

<210> 667
<211> 592
<212> DNA
<213> Homo Sapiens

<400> 667
gttatgaana cctttccaaa ttcatttgta tttctgttaa atttattttt tacttttaga 60
gtggctatca ttataatgta atttaaaatt atatttgtaa aagtgactat tggagtgagt 120
acgaattttg tttatanatc tatgataaat gcattctccc tntaggaggt agaanagtat 180
acagctgtnt ataataagct tcgctatgaa catacatttc tcaagtcaga atttgaacac 240
cagaaggaag agtatgcacg tnttttagat gaangaaacn ataaactatg aatcagagat 300
ngcaanactg gaggaagatn aagaagaact acgtanccag ctgcttaatg tngatctcac 360
anaagacagc aaacgagtgg aacaacttgc tcgagaaaaa gtctatttgt gtccaaaatt 420
aagangttta gaggctgaag taccngaatt aaaggctgaa naggagaatt ctgangctca 480
ggtggaaaat gcccaaanaa tacacgtgcg gcagttggct gagatgcacg ctacagtcag 540
atccctggag gctgacaanc aatcanctaa tttacgggca naacgcttgg aa 592

<210> 668
<211> 373
<212> DNA
<213> Homo Sapiens

<400> 668
aaaaaaaaat taagctcttt aattatgtgc acacagattt tagaaaaggt agccttttgt 60
atatanatac ctttacattc tttaggntga nttttaaatt gtcatctttt ttcaactaca 120
gtttttgtnt atagtaaacc anaanatgtg tntggaccct gttatggnca agcatctcaa 180
agatgaagan agaattaatg atagttatat ttcactcaaa atgccaaaaa aaaaaattca 240
acaaagtaaa aattttaaaa cttgactcta actagttcct ttttgtttta cattctcaaa 300
ccattgtnaa atattctaaa tatctctgaa aatttctctt ttaatgcttc acttgtntaa 360
tcttaaaatc ctg 373

<210> 669
<211> 661
<212> DNA
<213> Homo Sapiens

<400> 669
cacacctggt ggtcctgaag acagcccagg acccagggat ctcccccagc cagagtctgt 60

gtgcggaaag ttccagaggc ctcagtgcag gctccctgtc ggagagtgca gttgggcccg 120
tggaggcatg ctgcctggtc atcctggctg cagagagcaa ggtcgctgcg gaggagcttt 180
gctgtctgct aggccaggtc ttccaggttg tttacacgga gtccaccatc gactttctgg 240
acagagcgat atttgatggg gcctctaccc cgacccacca cctgtccctg cacagcgatg 300
actcttctac aaaagtggac attaaggaga cctacgaggt ggaagccagc actttctgct 360
tccctgaatc tgtggatgtg ggtggtgcat caccccacag caagaccatc agtgagagcg 420
agctgagcgc cagcgccact gagctgctgc aggactacat gctgacgctg cgcaccaagc 480
tgtcatcaca ggagatccag cagtttgcag cactgctgca cgagtaccgc aatggggcct 540
ctatccacga nttctgcatc aacctgcggc agctctacgg ggacagccgc aagttcctgc 600
tgcttggtct gaagcccttc atccctgaaa angacagcca gcacttcnag aacttcctgg 660
a 661

<210> 670
<211> 401
<212> DNA
<213> Homo Sapiens

<400> 670
aaattattca cattgcagta aacttctttt taaggtctct gaaagttaca ataggaacat 60
catgtgcaaa actgacagcc gtccaagggc ccagccgaca ggactggctc tccctgcccg 120
ctcggccggg ccctccccga gcggggacac actgcagggc ttggctgaac cctggtggac 180
aaggcaaana nccttccacc ccgcactgag gctcgtgtcc ctcggcagct ccctgctcct 240
tcacagtaaa ngacctgggc cgcccggggc catctgcacc gggcgcctct ccctggccac 300
caccaagggc tgacacgcag gtctgggcag ctccttctgg gaaggcctat gacgactgcg 360
ccgaaggtgt gggtgccccc ccatccactg tccatcatgc c 401

<210> 671
<211> 1347
<212> DNA
<213> Homo Sapiens

<400> 671
aagatcagcg atatcacgcg tcccccggag catcgcgtgc aggagccatg gcgcgggagc 60
tataccacga agagttcgcc cgggcgggca agcaggcggg gctgcaggtc tggaggattg 120
agaagctgga gctggtgccc gtgccccaga gcgctcacgg cgacttctac gtcggggatg 180
cctacctggt gctgcacacg gccaagacga gccgaggctt cacctaccac ctgcacttct 240
ggctcggaaa ggagtgttcc caggatgaaa gcacagctgc tgccatcttc actgttcaga 300
tggatgacta tttgggtggc aagccagtgc agaatagaga acttcaagga tatgagtcta 360
atgactttgt tagctatttc aaaggcggtc tgaaatacaa ggctggaggc gtggcatctg 420
gattaaatca tgttcttacg aacgacctga cagccaagan gctcctacat gtgaagggtc 480
gtanagtggt gagagccaca gaattcccct tagctgggac agtttcaaca agggtgactg 540
cttcatcatt gaccttggca ccgaaattta tcanttggtg tggttcctcn tgcaacaaat 600
atgaacgtct gaaggcaaac caggtagcta ctggcattcg gtacaatgaa aggaaaggaa 660
ggtctgaact aattgtcgtg gaanaaggaa gtgaaccctc agaacttata aaggtcttag 720
gggaaaagcc agagcttcca gatggaggtg atgatgatga cattatanca gacataagta 780
acaggaaaat ggctaaacta tacatggttt cagatgcaag tggctccatg agagtgactg 840
tggtggcana agaaaacccc ttctcantgg caatgctgct gtctgaagaa tgctttattt 900
tggaccacgg ggctgccaaa caaattttcg tatggaaagg taaagatgct aatccccaag 960
agaggaaggc tgcaatgaag acagctgaag aatttctaca gcaaatgaat tattccaaga 1020
atacccaaat tcaagttctt ccagaaggag gtgaaacacc aatcttcaaa cagtttttta 1080
aggactggag agatnaacga tcagagtgat ggcttcggga aagtttatgt cacagagaaa 1140
gtggctcaan tnnaacnaat tccctttgat gcctcnnaat tacncagttc tccgcagatg 1200
gcagcccagc acaatatggt ggatgatggt tctggccaag tggaaatttg gcgtgtncaa 1260
aacaatggta ggatccaagt tgaccnnaac tcctatggtg actcccatgg tggtgactgc 1320
tacttcatac tctacaccta tccctga 1347

<210> 672
<211> 3441
<212> DNA
<213> Homo Sapiens

<400> 672
atgtttctaa cattgaactc taaggaagct ggtgaacaaa cacgccatat gtatgcagaa 60
cacttaacag aattatgcta tgttgtctgt ttttgtttgt atttcttgtc cttgctgaag 120
attgacttga aatcttaaac taagttctcc ctctttatag gcggtgacag tgatcctcca 180
ttaaagcgta gcctggcaca gaggctaggg aagaaagttg aagctccaga aactaacatt 240
gacaaaacac caagaaaagc tcaagtttcc aagtctctta agggagcgat taggcatgtc 300
agctgatcca gataatgagg atgcaacaga taaagttaat aaagttggtg agatccatgt 360
gaagacatta gaagaaattc ttcttgaaag agccagtcag aaacgtggag aattgcaaac 420
taaactcaag acagaaggac cttcaaaaac tgatgattct acttcaggag caagaagctc 480
ctccactatc cgtatcaaaa ccttctctga ggtcctggct gaaaaaaaac atcggcagca 540
ggaagcagag agacaaaaaa gcaaaaagga tacaacttgc atcaagctaa agattgatag 600
tgaaattaaa aaaacagtag ttttgccacc cattgttgcc agcagaggac aatcagagga 660
gcctgcaggt aaaacaaagt ctatgcaggg aggtgcacat caagacgctg gaagaaatta 720
aactggagaa ggcactgagg gtgcagcaga gctctgagag cagcaccagc tccccgtctc 780
aacacgaggc cactccaagg gcaaggcggc tgctgcgaat ccccaaaaga acagggatga 840
aagaagagaa gaaccttcag gaaggaaatg aatttgattc tcagagcatt attataactg 900
aagctaaaga agcttcaggt gagaccacag gagttgacat cactaaaatt ccagtcaaga 960
gatgtgagac catgagagag aagcacatgc acaaaacaac aggagaggga aaaatcagtc 1020
ttgacacctc ttcggggaga tgtagcctct tgcaataccc aagtggcaga gaaaccagtg 1080
ctcactgctg tgccaggaat cacacggcac ctgaccaagc ggcttcccac aaagtcatcc 1140
cagaaggtgg aggtagaaac ctcagggatt ggagactcat tattgaatgt gaaatgtgca 1200
gcacagacct tggaaaaaag gggtaaagct aaacccaaag tgaacgtgaa gccatctgtg 1260
gttaaagttg tgtcatcccc caaattggcc ccaaaacgta aggcagtgga gatgcacgct 1320
gctgtcattg ccgctgtgaa ccactcagct ccagcagtgt cctacaggaa cccccagcca 1380
aaaaggcagc tgtggctgtt gtcccgcttg tctctgagga caaatcagtc actgtgcctg 1440
aagcagaaaa tcctagagac agtcttgtgc tgcctccaac ccagtcctct tcagattcct 1500
cacccccgga ggtgtctggc ccttcctcat cccaaatgag catgaaaact cgccgactca 1560
gctctgcctc aacaggaaag cccccactct ctgtggagga tgattttgag aaactaatat 1620
gggagatttc aggaggcaaa ttggaagctg agattgacct ggatcctggg aaagatgaag 1680
atgaccttct gcttgagcta tcagaaatga ttgatagctg aagggtggta gtgaggacac 1740
tttaaaaaaa aatcgccaaa aaactggact tagtttcatc tattgtaaca tttacctgag 1800
atgatcattt ctttagtcta gaatttgccc caaatcagaa gtatacctct gaattatctg 1860
tatgtgtcct ggattccttg gggtcagatt tttaaagtta ctttataacc attttgtcca 1920
tttgatgcca ttgtttatca tcttttgaga aaaaagttct gtcataccct tctctccaca 1980
aaaaagagac tgagagggag atcaagtgaa agggtgcaag cgaacttagt gactccttga 2040
ggtgtttgtc agttttggct tttttcttct ttgttgtatt ctttatgtat tgtcttgatg 2100
tacttaatat tacctgagtt tgaaatggat gaagacagct gctaccatta aggaccaaat 2160
tttatgctac cactaaacaa aaatacccac tcagtctgtg ttaaattgta tgtcttttta 2220
aaggtattta aagattcaac taagctttaa agagggctga gcagctcagg aagcctgtaa 2280
tgtgggcata actctttgga cctgatcttg atgcttctgc tgctctgtta gcctctgaag 2340
agcaatatct aatttattat tactgtaatt ttttaaaagg ctttaaagtg cctcaggggt 2400
cccctgaaac taattttcta tttctgggat tccctggatt cattatatga gatggtgaca 2460
tgattagagg aattcttttt tagtatgaaa attgtccctt ttcttcttca gtacttgcct 2520
ccttgctggc attgaattaa cacagggaca aaatttggtt aattttttat ttctaactct 2580
cccaacaaac ccctgttgcc cagtatttgt ttggtggcct ttaaccacct gagggaaaaa 2640
atgagcttat tcaagctgcc aatatttatc tatgggctgt agcagtacac tgaattgtac 2700
tgtgccaggg atattgagat gctctggggg tgtattgtat acctgccagt tttcttcatt 2760
tctgaattga gttttctttt cttgatgttg gtttccttca tatcacctca aggtttagat 2820
ttgtgaagga ataagcatga tggaaataat agtcttgaaa ggagatatgt tgtatataat 2880
caggaggaag aggaaggaag gacttaccca ttttgatatt ttgctgtagg tggccagttt 2940

tgtttctcat agggaaatnt gacccacctg tcatgttggc tccctaagga actgctgttg 3000
taagcggctc atcaagagtt gaacttcacg tagccttgtt gggaatatgg aaaaggaaga 3060
aagccacagg actgcccatt cagttttggg aagattggga tgattttgca caagcaaaaa 3120
tgactgaagt ttatgtatag acacaccttt accaatccat nttcagctga ctgaatgttg 3180
tatgatagcc cttctccaaa gcagaggtag aatgttcagg tttcaccatg gattttctac 3240
ttatttcgtt tttggaatca ccttacagat tccaggtccc ttttgtatat attctttatt 3300
cttttgcttt tttaaaaaat aattttgttt catatttaaa gcacttgtat tagtcaatgt 3360
ttcgtgttcc gcattatttg aaccatttgc ccttacagaa agagaaatac ttgtttgtgt 3420
tttaaataaa actgatgtag g 3441

<210> 673
<211> 1016
<212> DNA
<213> Homo Sapiens

<400> 673
gtcaaatgct tgttcttccc tggaagttga ggcagccata tcaagaaaaa ctccagccca 60
gcctcagaga agatctctta ggctttctgc tcagaaggat ttggaacaga aagaaaagca 120
tcatgtaaaa atgaaagcca agagatgtgc cactcctgta atcatcgatg aaattctacc 180
ctctaagaaa atgaaagttt ctaacaacaa aaagaagcca gaggaagaag gcagtgctca 240
tcaagatact gctgaaaaga atgcatcttc cccagagaaa gccaagggta gacatactgt 300
gccttgtatg ccacctgcaa agcagaagtt tctaaaaagt actgaggagc aagagctgga 360
gaagagtatg aaaatgcagc aagaggtggt ggagatgcgg aaaaagaatg aagaattcaa 420
gaaacttgct ctggctggaa tagggcaacc tgtgaagaaa tcagtgagcc aggtcaccaa 480
atcagttgac ttccacttcc gcacagatga gcgaatcaaa caacatccta agaaccagga 540
ggaatataag gaagtgaact ttacatctga actacgaaag catccttcat ctcctgcccg 600
agtgactaag ggatgtacca ttgttaagcc tttcaacctg tcccaaggaa agaaaagaac 660
atttgatgaa acagtttcta catatgtgcc nccttgcaca gcaagttgaa gacttccata 720
aacgaacccc taacagatat catttgagga gcaagaagga tgatattaac ctgttaccct 780
ccaaatcttc tgtgaccaag atttgcagag acccacagac tcctgtactg caaaccaaac 840
accgtgcacg ggctgtgacc tgcaaaagtt acagcagagc tggaggctga ggagctcgag 900
aaattgcaac aatacaaatt caaagcacgt gaacttgatc ccagaatact tgaaggtggg 960
cccatcttgc ccaagaaacc acctgtgaaa ccacgccgag ccctatgcct cgtgcc 1016

<210> 674
<211> 1135
<212> DNA
<213> Homo Sapiens

<400> 674
aggaattggg acaggcagaa ggaggcngct tgtttcangg tttgttccaa acaccgtttn 60
ttttttcagg ggcccttggt tccccaagaa agagangaaa tcagttgtga gggccnnttt 120
ntggtttttt tngttcaggg acnttttcag tgggctactg agagagagcc aaagagcggc 180
agggagctgg agaagagaat ggctgaggta gaagcccaga aagcccagca gttggaggag 240
gccagactac aggaggaaga gcagaaaaaa gaggagntgg ccaggctacg gagagaactg 300
gtgcataagg caaatccaat acgcaagtac cagggtctgg agataaagtc aagtgaccag 360
cctctgactg ngcctgtatc tccccaaatt ctccactcga ttccactgct taaattcagc 420
tgtgagctgc ggataccgcc cggcaatggg acctgttttt aacttcaaac ctaggaccgt 480
cttgctttgt cattgggcat ggagagaacc catttntcca gacttttacc tacccgtgcc 540
tgagaaagca tacttgacaa ctgtggactc cagttttgtt gagaattgtt ttcttacatt 600
actaaggcta ataatgagat gtaactcatg aatgtctcga ttagactcca tgtagttact 660
tcctttaaac catcagccgg ccttttatat gggtcttcac tctgactaga atttagtctc 720
tgtgtcagca cagtgtaatc tctattgcta ttgcccctta cgactctcac cctctcccca 780
ctttttttaa aaattttaac cagaaaataa agatagttaa atcctaagat agagattaag 840
tcatggttta aatgaggaac aatcagtaaa tcagattctg tcctcttctc tgcataccgt 900

gaatttatag ttaaggatcc ctttgctgtg agggtagaaa acctcaccaa ctgcaccagt 960
gaggaagaag actgcgtgga ttcatgggga gcctcacagc agccacgcag caggctctgg 1020
gtggggctgc cgttaaggca cgttctttcc ttactggtgc tgataacaac agggaaccgt 1080
gcagtgtgca ttttaagacc tggcctggaa taaatacgtt ttgtctttcc ctccc 1135

<210> 675
<211> 1067
<212> DNA
<213> Homo Sapiens

<400> 675
attttaaaga aacttcacag agctgcttca gtcggggatt tgaagaagct gaaggaatac 60
cttcagatca agaaatatga tgtaaatatg caggacaaaa aatacagaac acctttgcac 120
ctagcctgtg ctaatggaca tacagatgtt gtacttttcc taattgagca acaatgcaaa 180
ataaatgtcc gggatagtga aaacaaatcc ccattgatta aggcagtaca gtgtcaaaat 240
gaggattgtg cctactattc ttctaaactt tggtgcagac ccagatctga gggatattcg 300
ttataatact gttcttcact atgctgtttg tggtcaaagt ttgtcattag ttgaaaaact 360
gcttgaatac gaagctgatc ttgaagcgaa aaataaggat gggtatactc cactattagt 420
tgccgttatt aacaataatc caaaaatggt aaaatttctt ctggagaaag gggctgatgt 480
gaatgcttca gataattatc aaagaacagc ccttattctt gctgtcagtg gtgaaccacc 540
atgtttagta aagcttcttc ttcagcaagg tgtggaatta tgttacgaag gtattgtgga 600
ttcacagctg aggaatatgt ttatttccat ggttttactg catagatacc cacaattcac 660
tgcgagccat ggaaagaaga aacatgctaa atagacacct tattcttggc actacatgtg 720
actaaaggaa gatatggaac ccatttctac aatttctttg ccgcttcctt gaattggaaa 780
aatgtacttt gaaagaaccg gttaagtgaa ctatgataat atttttgctg actacccagt 840
tgaagaaaaa gtttcgttaa ttggatggga tttttttttt tcacgttaga agaatgaatg 900
aagaaatttt aaaagataaa cattatattg tgaaccatca gctgaaaaga taaatttgtg 960
ttcaatatat aggagaaaaa atttgtgtca aaatgttgaa tggaataata atgagaaact 1020
gtgttaggca tgtattaaaa catttaaata aaataaaaat acatttc 1067

<210> 676
<211> 784
<212> DNA
<213> Homo Sapiens

<400> 676
aaaagaattc tacaagattg tggaattcac aatctagtat tgatcaaaaa ttggcaaatc 60
aaattaatga tcttagacaa actgtcattt ggatgggaga cagactcatg agcttagaac 120
atcgtttcca gttacaatgt gactggaata cgtcagattt ttgtattaca ccccaaattt 180
ataatgagtc tgagcatcac tgggacatgg ttagacgcca tctacaggga agagaagata 240
atctcacttt agacatttcc aaattaaaag aacaaatttt cgaagcatca aaagcccatt 300
taaatttggt gccaggaact gaggcaattg caggagttgc tgatggcctc gcaaatctta 360
accctgtcac ttgggttaag accattggaa gtactacgat tataaatctc atattaatcc 420
ttgtgtgcct gttttgtctg ttgttagtct gcaggtgtac ccaacagctc cgaagagaca 480
gcgaccatcg agaacgggcc atgatgacga tggcggtttt gtcgaaaaga aaagggggaa 540
atgtggggaa aagcaagaga gatcaaattg ttactgtgtc tgtgtagaaa gaagtagaca 600
tgggagactc catttgttta tgtgttaaga aaaattcttc tgccttgaga ttctgttaat 660
ctatgacctt acccccaacc ccgtgctctc tgaaacgtgt gctgtgtcaa ctcagggttg 720
aatggattaa gggcggtgca ggatgtgctt tgttaaacag atgcttgaag gcagcatgct 780
cctt 784

<210> 677
<211> 1362
<212> DNA
<213> Homo Sapiens

<400> 677
ggcacgagct gggcattaat gaggatcatt ctgagggtga tgaaaaatct gagaaggaaa 60
ctattatggc tcaccagccg actgatgtgg agtccacttt attgcaagtt gcaaggaaca 120
agaatactgc catccgtgaa gaactcaacc agctgaaaaa tgaaaacaga atgttaaagg 180
acaggttgaa tgcattgggc ttttccctag agcagaggtt agacaattct gaaaaactgt 240
ttggctatca gtccctgagc ccagaaatca cccctggtaa ccagagcgat ggaggaggaa 300
ctctgacttc ttcagtggaa ggctctgccc ctggctcagt ggaggatctc ttgagtcagg 360
atgaaaatac actaatggac catcagcaca gtaactccat ggacaattta gacagtgagt 420
gcagtgaggt ctaccagccc ctcacatcga gcgatgatgc gctggatgca cacatctctc 480
tctcagagtc ggaaggcatc tctcagcata gagcgctccc ggaaggggag cagcgggaat 540
gccagtgaag tgtccgtggc tctgcctgac ttnacgcata caccagatgg nagagaacca 600
acacagtaca agtgagggac tccaggcaac cctgcaagag ctagctgatt tacagcagat 660
tacccaggaa ctgaatagtg aaaacgaaag gcttggagaa gagaaggtta ttctgatgga 720
gtctttatgt cagcagagcg ataagttgga acactttagt cgacagattg aatacttccg 780
ctctcttcta gatgagcatc acatttctta tgtcatagat gaagatgtaa aaagtggcgg 840
ctatatggma ttagagcaac gttacatgga cctcgctgag aatgcccgtt ttgaacggga 900
gcagcttctt ggtgtccagc agcatttaag caatactttg aaaatggcag aacaagacaa 960
taaggaagct caagaaatga taggggcact caaagaacgc agtcaccata tggagcgaat 1020
tattgagtct gagcagaaag gaaaagcagc cttggcagcc acgttagagg aatacaaagc 1080
cacagtggcc agtgaccaga tagagatgaa tcgcctgaag gctcagctgg agaatgacaa 1140
gcagaaagtg gcagagctgt attctatcca taactctgga gacacatctg atattcagga 1200
cctcctggag agtgtcaggc tggacaaaga aaaagcagag actttggcta gtagcttgca 1260
ggaagatctg gctcataccc gacatgatgc caatcgatta caggatgcca ttgctaggta 1320
gaggatgata ccgagcctcc aagaagagct agaacaaatt ga 1362

<210> 678
<211> 1771
<212> DNA
<213> Homo Sapiens

<400> 678
agccagcggc agcaggctga gctcccaggc tgacatctgg gcagggctga tgggcagctt 60
ctggccatct ggtgaccagg tgtgccgcaa gtwktwwkta tatgcacagc ccctttccta 120
ataacccaca ttctaggtta cgtagacacg ttaaactcct attctagaac atcgtgcttg 180
aatgcagacc cctcagccca caatcgggct ggctgggcct cctgtgagcc ctcattgcat 240
ccagtctgtg gggcagtgac cccgcttcca ctggtggctg gtcttcctca tggtgctatg 300
caggtgccaa acacagttat attctcaaag gtacaggtct tgtctggaca ttgttcaaac 360
caatagctac tgcgttaggc acacgggaga tccctattcc caaaaatagc tgttgagttc 420
tggcctgaga gcatctccag tgaccacctt taaataaggc tttggttcaa acagcatgga 480
cccagcacct ggggagggtg ggcacagggg gcatggaccc agtacctggg gagggcgggc 540
atggtggtag gagacaactc aaccactgag tcttggaggt cctgccttgg ccacggaggg 600
cagtggctgc cctcacaaga agagtgaaga cactttcttt aactctgtcc taggagaatt 660
atgtgttagt gactcagtga gtttaaatga cactgcctgg rctccctaaa gttgtttact 720
tttstcctat ttrctgcktt awtccttgct ctcaccatgc taatgtacag atgttgttta 780
gatttctatg cttattgaaa caatgtaact gtgggactaa cagaacagga gcgaccttgt 840
ccagcattgc tcgtaacaaa acaaaaatgt taactagaaa aactccttat gatgaagaaa 900
tctaaagcca gagctgggac tccaaacccc ttccagggtg gaagacaggt cgctgagtcc 960
aggcaagggg cccccgtaac tgcttcccgc cagaaagccc agccgcgtga gtkcagcagc 1020
agcaccccag ccctggcgtg gccgcaccac ggcctctaga tactcttcta gctcaggctg 1080
aacacgcctg gattgtgtcg gccgggacag ccccgtcagt gtggggcagc tgacccacgt 1140
ctgtgtgaac atgctcctcc aaactaggac ggtgaagggc ccagggcgct gggaactgcc 1200
aggcgctgac tctccttctg ggttctcacc agcaccggaa cccaccccag ccaatagtca 1260
ggaagtgccg cggccgagcc ttcatcaacc ctagtgagtt tcccacagaa ctgaatccct 1320
tttcccaaat tcagctgtgc atgagccctt tttgtttggt gccctggagc actagtgtag 1380
ttcaatattc tcttcagaag gaaaactcca gcagccaccg gcctgcagga tgtgtgctga 1440

gcccacatga cctgaatgga cgggtcatgt gggaggggcc ctggtgggag ctgtgggcca 1500
cacggctgag ttcttccaat acggaagccc cgagctggag gctcacacgc tgtggggcag 1560
cccagagttg ctggaagctt tacaggggtg cgtagctaat ggcgtcggtg tcgctcggtc 1620
gctgtggagg ggtaccccgc tattggggcg gctcctcccg gcatgctcag gtctcaaagt 1680
acttgtagat cgcgtcacat acagtatcac gttctgccag tcgggtcgtt cagtccgtac 1740
catttcatta atgtccagtg tggatttgat g 1771

<210> 679
<211> 1367
<212> DNA
<213> Homo Sapiens

<400> 679
ctagtggatc caaagaattc ggcacgagga aacaagagcc ctgaaagatg aaatagatgt 60
tcttagggct acctctgata aagcaaataa actggagtca acagttgaga tatatcgtca 120
gaagctacaa gatctgaatg accttcgcaa gcaggtgaaa actttacagg aaaccaacat 180
gatgtatatg cataatacag tcagcttaga agaagaatta aaaaaagcaa atgcagcacg 240
tacacaatta gaaacataca aaaggcaggt tcaagatctt catgttaaac tttcctccga 300
atccaagagg gcagacacac tagcgtttga aatgaagcgg cttgaagaaa aacatgaagc 360
tttacttaag gaaaaagaga gactaattga gcagcgtgat actttgaaag aaacaaatga 420
agagcttcga tgttcacaag tacaacagga ccacctaaac caaacagatg catctgctac 480
aaaaagttat gagaatcttg ctgctgagat tatgccagtg gaatataggg aggtgtttat 540
tcgactgcaa catgaaaata agatgcttcg cttacagcaa gaaggctctg agaatgaacg 600
tattgaggaa cttcaggagc agctagaaca gaaacaccgt aaaatgaatg aactggaaac 660
tgagcagagg ctgagcaaag agcgtattag agaattgcag cagcagattg aggacctcca 720
gaaatcttta caggaacaag gttccaagtc tgaaggcgaa agttccagca aattaaagca 780
gaagttggaa gctcatatgg aaaaactcac agaggtccat gaagaattac agaagaaaca 840
agaactcatt gaagatcttc agccagatat aaatcaaaat gtacaaaaga tcaatgaact 900
tgaagctgct cttcagaaga aagatgaaga tatgaaagca atggaggaaa gatataaaat 960
gtacttggag aaagccagaa atgtaataaa aactttggat cccaagttaa atccagcatc 1020
agctgaaata atgctactaa gaaagcagtt ggcagagaaa gagagaagaa ttgagattct 1080
ggagagtgaa tgcaaagtag caaaattccg tgattatgaa gaaaactcat tgtttctgcg 1140
tggtataata agagtctagc attccagaaa ctggggatgg aatctagact tgtgagcggc 1200
ggtggtgcct gcagtgacac tggtgcgtgc actcctgcgc ggtctttctt agcgcagcaa 1260
cggcacatca ccaacaccag aagaaatctc tctgttaaag tccctgctac aacatctgat 1320
taaactgcaa aaaaaacaaa acaaaacaaa aaaaaaaaaa aaaaaac 1367

<210> 680
<211> 2545
<212> DNA
<213> Homo Sapiens

<400> 680
ggatccaaag attcggcacg aggcggagtc gcagcctcgg tcccggagcc caccttcgcc 60
tcgcccttgc ccagcctgcg gtgatggagg cggccaccac actgcaccca ggcccgcgcc 120
cggcgctgcc cctcgggggc ccgggcccgc tgggcgagtt cctgcctcca cccgagtgcc 180
cggtcttcga acccagctgg gaagagttcg cggacccctt cgctttcatc cacaagatcc 240
ggcccatagc cgagcagact ggcatctgta aggtgcggcc gccgccggat tggcagccac 300
catttgcatg tgatgttgat aaacttcatt ttacgccacg tatccagaga ctgaatgaat 360
tggaggccca aactcgtgta aaattgaatt tcttggacca gattgcaaag tactgggagt 420
tacagggaag tactctgaaa attccacatg tggagaggaa gatcttggac ttatttcagc 480
ttaataagtt agttgcagaa gaaggtggat ttgcagttgt ttgcaaggat agaaaatgga 540
ccaaaattgc taccaagatg gggtttgctc ctggcaaagc agtgggctca catatcagag 600
ggcattatga acgaattctc aacccctaca acttattcct gtccggagac agcctaaggt 660
gtttgcagaa gccaaacctg accacagaca ctaaggacaa ggagtacaaa ccccatgata 720

ttccccagag gcagtctgtg cagccttcgg aaacgtgccc cccagcccga cgagcaaaac 780
gcatgagagc agaggccatg aatattaaaa tagaacccga ggagacaaca gaagccagaa 840
ctcataatct gagacgtcga atgggttgtc caactccaaa atgtgaaaat gagaaagaaa 900
tgaagagtag catcaagcaa gaacctattg agaggaaaga ttatattgta gaaaatgaga 960
aggaaaagcc caagagtcga tctaaaaaag ccaccaatgc tgtggacctg tatgtctgtc 1020
ttttatgtgg cagtggcaat gatgaagacc ggctactgtt gtgtgatggc tgtgatgaca 1080
gttaccatac cttttgcttg atcccacctc tccatgatgt tcccaaggga gactggaggt 1140
gtcctaagtg tttggctcag gaatgtagta agccacaaga agcatttggc tttgaacaag 1200
cagccaggga ctataccctc cgtacttttg gggaaatggc agatgcgttc aaatctgatt 1260
acttcaacat gccagtccat atggtcccca cagagcttgt tgagaaagaa ttttggagac 1320
tagtaagcac tattgaggag gatgtcacag tggaatatgg agctgacatt gcctcaaagg 1380
aatttggcag tggctttcct gtccgagatg ggaaaatcaa actctcacct gaggaagagg 1440
agtatcttga tagtggctgg aatttgaaca acatgccagt gatggagcag tctgtccttg 1500
cacatattac tgctgatata tgtggcatga aacttccttg gttgtatgtg ggaatgtgct 1560
tttcttcatt ctgttggcac attgaagacc actggagcta ttcaattaac tacttgcact 1620
ggggtgagcc aaaaacctgg tatggagtcc cagggtatgc tgctgagcag ctagaaaatg 1680
taatgaagaa actagctcca gaactctttg tgtcccagcc ggatctcctc catcagcttg 1740
tgaccatcat gaaccccaat accctgatga ctcatgaagt gcctgtttac cgaactaatc 1800
agtgtgctgg ggagtttgtg attacatttc caagagccta ccacagtgtt tttaaccagg 1860
ttttaatttt gctgaggctg ttaacttctg cactgttgat tggctgccat taggccgaca 1920
gtgtgtggag cattatcgct tgcttcatcg atattgtgtg ttttcccatg atgagatgat 1980
ctgcaagatg gcttccaagg ctgatgtatt agatgttgta gtggcttcaa ctgttcagaa 2040
agacatggcc attatgattg aggatgagaa agctttaaga gaaactgtcc gtaaattggg 2100
agtgattgat tcggaaagaa tggattttga gctgttgcca gatgatgaac gtcagtgtgt 2160
aaaatgcaaa actacatgct tcatgtctgc catctcctgt tcttgtaaac ctggccttct 2220
tgtttgcctg catcatgtaa aagaattgtg ttcctgtcct ccttataaat ataaattgcg 2280
gtataggtac acgctggatg atctctaccc tatgatgaat gcattgaagc ttcgagcaga 2340
atcttacaac gaatgggcct tgaatgtgaa tgaagctttg gaggcaaaga tcaacaagaa 2400
gaaaagtatg tgatacagaa agtgacttgg tgattggcaa attggggctt attgtgatgt 2460
agccaaatta aagtcaacaa aacattaaaa aaaaaaaaaa aaaactcgag agtacttcta 2520
gagcgccgcg ggcccatcga ttttc 2545

<210> 681
<211> 1745
<212> DNA
<213> Homo Sapiens

<400> 681
ctagtggatc caaagaattc ggcacgaggg aagatggctt cgtttcggaa gctaacgctt 60
tctgaaaaag tgccgccaaa tcatcccagt cggaaaaagg ttaacttcct agatatgtct 120
ctagacgaca ttataatcta taaagagtta gaagggacaa atgctgaaga agaaaagaat 180
aaaagacaga accatagtaa aaaggaatcg ccttcaagac agcaatcaaa agctcataga 240
catcgccatc ggagaggcta ctcaagatgc agaagcaact ctgaggaagg aaatcatgat 300
aaaaaaccat cccaaaaacc ttctggattc aagtctggac aacacccttt aaatgggcag 360
cctttaattg agcaggagaa gtgcagtgac aattatgagg cccaagcaga gaagaatcaa 420
ggccagtcag aggggaacca gcatcaatca gaaggaaatc cggacaaatc agaagaatcc 480
cagggccaac cagaagaaaa tcatcattct gagcgatccc gaaaccactt agagagatct 540
ctttctcagt cagacagatc tcaagggcag ctaaagagac atcatcccca atatgagaga 600
tctcatggcc aatacaagag atctcatggt caatctgaga gatctcatgg ccactcagag 660
agatctcatg gtcactcaga gagatctcat ggtcactcag agagatctca tggtcactca 720
aagagatctc gtagccaggg agatcttgtg gacactcaga gtgatctcat agccactcag 780
agagatctca tagccactca gaaagatctc atagccactc agagagatct catagccact 840
cagagagatc tcatagtcac tcagagagat ctcgtggcca ctgagagaga tctcataaat 900
cagtcaggga gatctcatgg ccaatcagaa agacatcaga gatactcaac aggtaaaaat 960
acaataacta cttaatcatc agaacaatgt gttgaattct gtggaaatag aaaagcatat 1020

atctatattc taatggctaa atatgtattt gttgaaacat gtatattggg acaaagacat 1080
aaatattaga atggaggtaa tacatacata gtatcaatat tgtttcaact tgatgtcctc 1140
taagctatca tccagttacc caagatgtcc cattaagttg ttcccggtag gtctgctttc 1200
cctggaagag ccgtatgtac tcagcctttc ctattgggcc ttccccacaa ttagaatatt 1260
ttgacttagt gtcctgtccc ccttggacgt tccaacttga cttagtgtcc agtgcccctt 1320
ggacattcca acctggtagg taagctaatc taacaactaa ctgccaaatt gataatatat 1380
aatctatgat aatgaatatc tcttttgtgt ctccttccta agccatcctc agagagtcct 1440
tagcagacaa atggtagatg tatctttggg cagctgaact tttctgcttt cctcaaatca 1500
gaccatatga gaggatatat tctatgcata gatgtaatgc taaccttctg aatatatttt 1560
gaatacattt atatattcac tgttgcctta taaaactgtt agggtaggtc tgtctaccct 1620
agcaaaagaa acacagaaat ttaaatgtac tgggagttat kkkkttaaaa acacaagata 1680
tgttaactgc agtttgtttg gttattcaat aaaagtttta gttttaaaaa aaaaaaaaaa 1740
aaaac 1745

<210> 682
<211> 1745
<212> DNA
<213> Homo Sapiens

<400> 682
ctagtggatc caaagaattc ggcacgaggg aagatggctt cgtttcggaa gctaacgctt 60
tctgaaaaag tgccgccaaa tcatcccagt cggaaaaagg ttaacttcct agatatgtct 120
ctagacgaca ttataatcta taaagagtta gaaggacaaa atgctgaaga agaaaagaat 180
aaaagacaga accatagtaa aaaggaatcg ccttcaagac agcaatcaaa agctcataga 240
catcgccatc ggagaggcta ctcaagatgc agaagcaact ctgaggaagg aaatcatgat 300
aaaaaaccat cccaaaaacc ttctggattc aagtctggac aacacccttt aaatgggcag 360
cctttaattg agcaggagaa gtgcagtgac aattatgagg cccaagcaga gaagaatcaa 420
ggccagtcag aggggaacca gcatcaatca gaaggaaatc cggacaaatc agaagaatcc 480
cagggccaac cagaagaaaa tcatcattct gagcgatccc gaaaccactt agagagatct 540
ctttctcagt cagacagatc tcaagggcag ctaaagagac atcatcccca atatgagaga 600
tctcatggcc aatacaagag atctcatggt caatctgaga gatctcatgg ccactcagag 660
agatctcatg gtcactcaga gagatctcat ggtcactcag agagatctca tggtcactca 720
aagagatctc gtagccaggg agatcttgtg gacactcaga gtgatctcat agccactcag 780
agagatctca tagccactca gaaagatctc atagccactc agagagatct catagccact 840
cagagagatc tcatagtcac tcagagagat ctcgtggcca ctgagagaga tctcataaat 900
cagtcaggga gatctcatgg ccaatcagaa agacatcaga gatactcaac aggtaaaaat 960
acaataacta cttaatcatc agaacaatgt gttgaattct gtggaaatag aaaagcatat 1020
atctatattc taatggctaa atatgtattt gttgaaacat gtatattggg acaaagacat 1080
aaatattaga atggaggtaa tacatacata gtatcaatat tgtttcaact tgatgtcctc 1140
taagctatca tccagttacc caagatgtcc cattaagttg ttcccggtag gtctgctttc 1200
cctggaagag ccgtatgtac tcagcctttc ctattgggcc ttccccacaa ttagaatatt 1260
ttgacttagt gtcctgtccc ccttggacgt tccaacttga cttagtgtcc agtgcccctt 1320
ggacattcca acctggtagg taagctaatc taacaactaa ctgccaaatt gataatatat 1380
aatctatgat aatgaatatc tcttttgtgt ctccttccta agccatcctc agagagtcct 1440
tagcagacaa atggtagatg tatctttggg cagctgaact tttctgcttt cctcaaatca 1500
gaccatatga gaggatatat tctatgcata gatgtaatgc taaccttctg aatatatttt 1560
gaatacattt atatattcac tgttgcctta taaaactgtt agggtaggtc tgtctaccct 1620
agcaaaagaa acacagaaat ttaaatgtac tgggagttat kkkkttaaaa acacaagata 1680
tgttaactgc agtttgtttg gttattcaat aaaagtttta gttttaaaaa aaaaaaaaaa 1740
aaaac 1745

<210> 683
<211> 3127
<212> DNA
<213> Homo Sapiens

<400> 683
gaattcggca cgagggtcag caattgctta gggcggaatg cgatttcggg ggaggaggcg 60
cgggtatgta gacagagggg gttgggacac accaggaggg gaggagccag ccccagagat 120
cgggaatcct ctcagtcctt agttacaagg ctccatcctc actttgttcg ctcctcagtc 180
gtccaggcgg attccttttt cgccaggcac caaggcacag cttagagtag acccgagtcc 240
tgctctgcgg agttcctctt cccagcgaag gtacagaggc ggatgaactg ctgagacttg 300
attgacgtat tttaagattt ttttaacttc tgaagtctag caggcctgta agaacaaaaa 360
tcattctgta ggaattaaaa acagaatcca gtcttgacaa catatccaca atgtctgatg 420
tatctactag tgtacaatca aaatttgata gacttgcaaa gaaaaaggaa aatatcacct 480
atatgagcag agagcagtta acagaaactg ataaggacat agctccggta ttagcattta 540
aaagtcaagg acgtatcagc aattatgaat aagtttaagg tcttaatgga aattcaagac 600
ctgatgtttg aggagatgag ggaaactctt aaaaatgacc taaaagcagt tttaggagga 660
aaagctacaa tacctgaggt aaagaattca gagaactcca gtagtaggca ggtttcagca 720
aataatcaat ttagcattac aaaaaacagg gatggtaggg aaaatagaag gagaaactct 780
aaaataggtg atgataatga aaatttaacc tttaaattag aagtaaatga gctgagtggt 840
aaattagaca acactaacga atacaatagt aatgatggta agaaattacc ccagggtgaa 900
tcacgaagtt acgaagtcat gggaagtatg gaagaaacct tatgcaatat agatgacaga 960
gatggaaatc gcaatgtcca tttagaattt acagaaagag agagtaggaa ggatggagag 1020
gatgaatttg tcaaagaaat gagagaggaa agaaaatttc agaaattgaa gaataaagag 1080
gaggttttaa aagcctccag agaagaaaaa gtgttgatgg atgaaggagc agtacttacc 1140
ctggcagccg acctttcatc agcaacactg gatattagta agcaatggag taatgtcttc 1200
aacattctga gagaaaatga ttttgaacct aaatttctgt gtgaagttaa attagcattt 1260
aaatgtgatg gtgaaataaa gacattttca gatctgcaaa gccttagaaa atttgccagc 1320
caaaaatctt ctatganaga wttactgana gatgtactcc cacaaaagga agaaataaat 1380
caaggaggaa gaaaatatgg nattcaagaa aaaagggata aaaccctaat agactcanag 1440
catagagctg gagaaataac cagtgatggc ttgagcttcc tatttcttaa agaagtaaaa 1500
gttgctaagc cagaggagat gaaaaactta gagactcaag aggaagagtt ttccgagcta 1560
gaggagctgg atgaagaggc ttcagggatg gaggatgatg aagatacctc agggctggag 1620
gaggaagaag aagaagaggc ttcagggttg gaggaggatg antcctcang gctagaggag 1680
gaagaggaac agacttcaga acaggactca acctttcang gtcatacttt ggtagatgca 1740
aagcatgaag ttgagataac cagtnatggc atggaaacta ctttcattga ctctgtagag 1800
gattctgaat cagaggagga agaggaagga aagagctctg aaacaggaaa ggtaaagact 1860
acctccctga ctgagaaaaa agcctcacgt agacaaaaag aaattccctt tagttatttg 1920
gttggggact ctgggaagaa aaagttggtg aaacaccagg tggtgcacaa aacccaggag 1980
gaagaggaaa cagctgtgcc cacaagtcaa ggaactggca caacctgtct gaccttatgt 2040
ttggcctctc cctcaaagtc actagagatg agtcatgatg agcataaaaa gcattcacat 2100
acaaatttga gtatttcaac aggagtcacc aaacttaaga aaacagaaga aaagaaacac 2160
aggactctgc acacagaaga actaacatcc aaagaagcag acttaacaga ggaaacagaa 2220
gaaaacttga gaagtagtgt gattaatagc atcagagaga taaaagagga gattggaaat 2280
ttgaaaagtt cccattcagg tgtcttggaa attgaaaatt cagtagatga tctgagtagc 2340
agaatggaca tccttgaaga aagaatagac agtctagaag atcaaattga agaattctct 2400
aaggatacaa tgcaaatgac caaacagata attagtaaag aagggccnag agatatagag 2460
gagagatcta gaagttgcaa cattcgtttg ataggaattc cagaaaagga gagttatgag 2520
aatagggcag aggacataat taaggaaata attgatgaaa actttgcaga actaaagaaa 2580
ggttcaagtc ttgagattgt cagtgcttgt cgagtaccta gtaaaattga tgaaaagaga 2640
ctgactccta gacacatctt ggtgaaattt tggaattcta gtgataaaga gaaaataata 2700
aggccttcta gagagagaag agaaattacc taccaaggaa caagaatcag gttgacagca 2760
gacttatcac tggacacact ggatgctaga agtaaatgga gcaatgtctt caaagttctg 2820
ctggaaaaag gctttaaccc tagaacccta tacccagcca aaatggcatt tgattttagg 2880
ggtaaaacaa aggtattcct tagtattgaa gaatttagag attatgtttt gcatatgccc 2940
accttgagag aattactggg gaataatata ccttagcacc ccagggtgac tacaaacaat 3000
atgctttcct cccccagcat gcacccaaaa accaacaagt aaaacgaaag tacacttcta 3060
cccagaagga tggacagcta ataccgtact tggggatgag gagcaaggaa tattacagat 3120
attaccc 3127

<210> 684
<211> 803
<212> PRT
<213> Homo Sapiens

<400> 684
   Met Asn Lys Phe Lys Val Leu Met Glu Ile Gln Asp Leu Met Phe Glu
   1               5                   10                  15
   Glu Met Arg Glu Thr Leu Lys Asn Asp Leu Lys Ala Val Leu Gly Gly
               20                  25                  30
   Lys Ala Thr Ile Pro Glu Val Lys Asn Ser Glu Asn Ser Ser Ser Arg
           35                  40                  45
   Gln Val Ser Ala Asn Asn Gln Phe Ser Ile Thr Lys Asn Arg Asp Gly
       50                  55                  60
   Arg Glu Asn Arg Arg Arg Asn Ser Lys Ile Gly Asp Asp Asn Glu Asn
   65                  70                  75                  80
   Leu Thr Phe Lys Leu Glu Val Asn Glu Leu Ser Gly Lys Leu Asp Asn
                   85                  90                  95
   Thr Asn Glu Tyr Asn Ser Asn Asp Gly Lys Lys Leu Pro Gln Gly Glu
               100                 105                 110
   Ser Arg Ser Tyr Glu Val Met Gly Ser Met Glu Glu Thr Leu Cys Asn
           115                 120                 125
   Ile Asp Asp Arg Asp Gly Asn Arg Asn Val His Leu Glu Phe Thr Glu
       130                 135                 140
   Arg Glu Ser Arg Lys Asp Gly Glu Asp Glu Phe Val Lys Glu Met Arg
   145                 150                 155                 160
   Glu Glu Arg Lys Phe Gln Lys Leu Lys Asn Lys Glu Glu Val Leu Lys
                   165                 170                 175
   Ala Ser Arg Glu Glu Lys Val Leu Met Asp Glu Gly Ala Val Leu Thr
               180                 185                 190
   Leu Ala Ala Asp Leu Ser Ser Ala Thr Leu Asp Ile Ser Lys Gln Trp
           195                 200                 205
   Ser Asn Val Phe Asn Ile Leu Arg Glu Asn Asp Phe Glu Pro Lys Phe
       210                 215                 220
   Leu Cys Glu Val Lys Leu Ala Phe Lys Cys Asp Gly Glu Ile Lys Thr
   225                 230                 235                 240
   Phe Ser Asp Leu Gln Ser Leu Arg Lys Phe Ala Ser Gln Lys Ser Ser
                   245                 250                 255
   Met Xaa Xaa Leu Leu Xaa Asp Val Leu Pro Gln Lys Glu Glu Ile Asn
               260                 265                 270
   Gln Gly Gly Arg Lys Tyr Gly Ile Gln Glu Lys Arg Asp Lys Thr Leu
           275                 280                 285
   Ile Asp Ser Xaa His Arg Ala Gly Glu Ile Thr Ser Asp Gly Leu Ser
       290                 295                 300
   Phe Leu Phe Leu Lys Glu Val Lys Val Ala Lys Pro Glu Glu Met Lys
   305                 310                 315                 320
   Asn Leu Glu Thr Gln Glu Glu Glu Phe Ser Glu Leu Glu Glu Leu Asp
                   325                 330                 335
   Glu Glu Ala Ser Gly Met Glu Asp Asp Glu Asp Thr Ser Gly Leu Glu
               340                 345                 350
   Glu Glu Glu Glu Glu Glu Ala Ser Gly Leu Glu Glu Asp Xaa Ser Ser
           355                 360                 365
   Xaa Leu Glu Glu Glu Glu Glu Gln Thr Ser Glu Gln Asp Ser Thr Phe
       370                 375                 380
   Xaa Gly His Thr Leu Val Asp Ala Lys His Glu Val Glu Ile Thr Ser
   385                 390                 395                 400
   Xaa Gly Met Glu Thr Thr Phe Ile Asp Ser Val Glu Asp Ser Glu Ser
                   405                 410                 415
   Glu Glu Glu Glu Glu Gly Lys Ser Ser Glu Thr Gly Lys Val Lys Thr
               420                 425                 430
   Thr Ser Leu Thr Glu Lys Lys Ala Ser Arg Arg Gln Lys Glu Ile Pro
           435                 440                 445
   Phe Ser Tyr Leu Val Gly Asp Ser Gly Lys Lys Lys Leu Val Lys His
       450                 455                 460
   Gln Val Val His Lys Thr Gln Glu Glu Glu Glu Thr Ala Val Pro Thr
   465                 470                 475                 480
   Ser Gln Gly Thr Gly Thr Thr Cys Leu Thr Leu Cys Leu Ala Ser Pro
                   485                 490                 495
   Ser Lys Ser Leu Glu Met Ser His Asp Glu His Lys Lys His Ser His
               500                 505                 510
   Thr Asn Leu Ser Ile Ser Thr Gly Val Thr Lys Leu Lys Lys Thr Glu
           515                 520                 525
   Glu Lys Lys His Arg Thr Leu His Thr Glu Glu Leu Thr Ser Lys Glu
       530                 535                 540
   Ala Asp Leu Thr Glu Glu Thr Glu Glu Asn Leu Arg Ser Ser Val Ile
   545                 550                 555                 560
   Asn Ser Ile Arg Glu Ile Lys Glu Glu Ile Gly Asn Leu Lys Ser Ser
                   565                 570                 575
   His Ser Gly Val Leu Glu Ile Glu Asn Ser Val Asp Asp Leu Ser Ser
               580                 585                 590
   Arg Met Asp Ile Leu Glu Glu Arg Ile Asp Ser Leu Glu Asp Gln Ile
           595                 600                 605
   Glu Glu Phe Ser Lys Asp Thr Met Gln Met Thr Lys Gln Ile Ile Ser
       610                 615                 620
   Lys Glu Gly Pro Arg Asp Ile Glu Glu Arg Ser Arg Ser Cys Asn Ile
   625                 630                 635                 640
   Arg Leu Ile Gly Ile Pro Glu Lys Glu Ser Tyr Glu Asn Arg Ala Glu
                   645                 650                 655
   Asp Ile Ile Lys Glu Ile Ile Asp Glu Asn Phe Ala Glu Leu Lys Lys
               660                 665                 670
   Gly Ser Ser Leu Glu Ile Val Ser Ala Cys Arg Val Pro Ser Lys Ile
           675                 680                 685
   Asp Glu Lys Arg Leu Thr Pro Arg His Ile Leu Val Lys Phe Trp Asn
       690                 695                 700
   Ser Ser Asp Lys Glu Lys Ile Ile Arg Pro Ser Arg Glu Arg Arg Glu
   705                 710                 715                 720
   Ile Thr Tyr Gln Gly Thr Arg Ile Arg Leu Thr Ala Asp Leu Ser Leu
                   725                 730                 735
   Asp Thr Leu Asp Ala Arg Ser Lys Trp Ser Asn Val Phe Lys Val Leu
               740                 745                 750
   Leu Glu Lys Gly Phe Asn Pro Arg Thr Leu Tyr Pro Ala Lys Met Ala
           755                 760                 765
   Phe Asp Phe Arg Gly Lys Thr Lys Val Phe Leu Ser Ile Glu Glu Phe
       770                 775                 780
   Arg Asp Tyr Val Leu His Met Pro Thr Leu Arg Glu Leu Leu Gly Asn
   785                 790                 795                 800
   Asn Ile Pro

<210> 685

<211> 947
<212> PRT
<213> Homo Sapiens

<400> 685
Met Ser Leu Pro Ser Arg Gln Thr Ala Ile Ile Val Asn Pro Pro Pro
1               5                   10                  15
Pro Glu Tyr Ile Asn Thr Lys Lys Asn Gly Arg Leu Thr Asn Gln Leu
            20                  25                  30
Gln Tyr Leu Gln Lys Val Val Leu Lys Asp Leu Trp Lys His Ser Phe
        35                  40                  45
Ser Trp Pro Phe Gln Arg Pro Val Asp Ala Val Lys Leu Lys Leu Pro
    50                  55                  60
Asp Tyr Tyr Thr Ile Ile Lys Asn Pro Met Asp Leu Asn Thr Ile Lys
65                  70                  75                  80
Lys Arg Leu Glu Asn Lys Tyr Tyr Ala Lys Ala Ser Glu Cys Ile Glu
                85                  90                  95
Asp Phe Asn Thr Met Phe Ser Asn Cys Tyr Leu Tyr Asn Lys Pro Gly
            100                 105                 110
Asp Asp Ile Val Leu Met Ala Gln Ala Leu Glu Lys Leu Phe Met Gln
        115                 120                 125
Lys Leu Ser Gln Met Pro Gln Glu Glu Gln Val Val Gly Val Lys Glu
    130                 135                 140
Arg Ile Lys Lys Gly Thr Gln Gln Asn Ile Ala Val Ser Ser Ala Lys
145                 150                 155                 160
Glu Lys Ser Ser Pro Ser Ala Thr Glu Lys Val Phe Lys Gln Gln Glu
                165                 170                 175
Ile Pro Ser Val Phe Pro Lys Thr Ser Ile Ser Pro Leu Asn Val Val
            180                 185                 190
Gln Gly Ala Ser Val Asn Ser Ser Ser Gln Thr Ala Ala Gln Val Thr
        195                 200                 205
Lys Gly Val Lys Arg Lys Ala Asp Thr Thr Thr Pro Ala Thr Ser Ala
    210                 215                 220
Val Lys Ala Ser Ser Glu Phe Ser Pro Thr Phe Thr Glu Lys Ser Val
225                 230                 235                 240
Ala Leu Pro Pro Ile Lys Glu Asn Met Pro Lys Asn Val Leu Pro Asp
                245                 250                 255
Ser Gln Gln Gln Tyr Asn Val Val Glu Thr Val Lys Val Thr Glu Gln
            260                 265                 270
Leu Arg His Cys Ser Glu Ile Leu Lys Glu Met Leu Ala Lys Lys His
        275                 280                 285
Phe Ser Tyr Ala Trp Pro Phe Tyr Asn Pro Val Asp Val Asn Ala Leu
    290                 295                 300
Gly Leu His Asn Tyr Tyr Asp Val Val Lys Asn Pro Met Asp Leu Gly
305                 310                 315                 320
Thr Ile Lys Glu Lys Met Asp Asn Gln Glu Tyr Lys Asp Ala Tyr Ser
                325                 330                 335
Phe Ala Ala Asp Val Arg Leu Met Phe Met Asn Cys Tyr Lys Tyr Asn
            340                 345                 350
Pro Pro Asp His Glu Val Val Thr Met Ala Arg Met Leu Gln Asp Val
        355                 360                 365
Phe Glu Thr His Phe Ser Lys Ile Pro Ile Glu Pro Val Glu Ser Met
    370                 375                 380
Pro Leu Cys Tyr Ile Lys Thr Asp Ile Thr Glu Thr Thr Gly Arg Glu
385                 390                 395                 400

Asn Thr Asn Glu Ala Ser Ser Glu Gly Asn Ser Ser Asp Asp Ser Glu
                405                 410                 415
Asp Glu Arg Val Lys Arg Leu Ala Lys Leu Gln Glu Gln Leu Lys Ala
            420                 425                 430
Val His Gln Gln Leu Gln Val Leu Ser Gln Val Pro Phe Arg Lys Leu
        435                 440                 445
Asn Lys Lys Lys Glu Lys Ser Lys Lys Glu Lys Lys Lys Glu Lys Val
    450                 455                 460
Asn Asn Ser Asn Glu Asn Pro Arg Lys Met Cys Glu Gln Met Arg Leu
465                 470                 475                 480
Lys Glu Lys Ser Lys Arg Asn Gln Pro Lys Lys Arg Lys Gln Gln Phe
                485                 490                 495
Ile Gly Leu Lys Ser Glu Asp Glu Asp Asn Ala Lys Pro Met Asn Tyr
            500                 505                 510
Asp Glu Lys Arg Gln Leu Ser Leu Asn Ile Asn Lys Leu Pro Gly Asp
        515                 520                 525
Lys Leu Gly Arg Val Val His Ile Ile Gln Ser Arg Glu Pro Ser Leu
    530                 535                 540
Ser Asn Ser Asn Pro Asp Glu Ile Glu Ile Asp Phe Glu Thr Leu Lys
545                 550                 555                 560
Ala Ser Thr Leu Arg Glu Leu Glu Lys Tyr Val Ser Ala Cys Leu Arg
                565                 570                 575
Lys Arg Pro Leu Lys Pro Pro Ala Lys Lys Ile Met Met Ser Lys Glu
            580                 585                 590
Glu Leu His Ser Gln Lys Lys Gln Glu Leu Glu Lys Arg Leu Leu Asp
        595                 600                 605
Val Asn Asn Gln Leu Asn Ser Arg Lys Arg Gln Thr Lys Ser Asp Lys
    610                 615                 620
Thr Gln Pro Ser Lys Ala Val Glu Asn Val Ser Arg Leu Ser Glu Ser
625                 630                 635                 640
Ser Ser Ser Ser Ser Ser Ser Ser Glu Ser Glu Ser Ser Ser Ser Asp
                645                 650                 655
Leu Ser Ser Ser Asp Ser Ser Asp Ser Glu Ser Glu Met Phe Pro Lys
            660                 665                 670
Phe Thr Glu Val Lys Pro Asn Asp Ser Pro Ser Lys Glu His Val Lys
        675                 680                 685
Lys Met Lys Asn Glu Cys Ile Leu Pro Glu Gly Arg Thr Gly Val Thr
    690                 695                 700
Gln Ile Gly Tyr Cys Val Gln Asp Thr Thr Ser Ala Asn Thr Thr Leu
705                 710                 715                 720
Val His Gln Thr Thr Pro Ser His Val Met Pro Pro Asn His His Gln
                725                 730                 735
Leu Ala Phe Asn Tyr Gln Glu Leu Glu His Leu Gln Thr Val Lys Asn
            740                 745                 750
Ile Ser Pro Leu Gln Ile Leu Pro Pro Ser Gly Asp Ser Glu Gln Leu
        755                 760                 765
Ser Asn Gly Ile Thr Val Met His Pro Ser Gly Asp Ser Asp Thr Thr
    770                 775                 780
Met Leu Glu Ser Glu Cys Gln Ala Pro Val Gln Lys Asp Ile Lys Ile
785                 790                 795                 800
Lys Asn Ala Asp Ser Trp Lys Ser Leu Gly Lys Pro Val Lys Pro Ser
                805                 810                 815
Gly Val Met Lys Ser Ser Asp Glu Leu Phe Asn Gln Phe Arg Lys Ala
            820                 825                 830
Ala Ile Glu Lys Glu Val Lys Ala Arg Thr Gln Glu Leu Ile Arg Lys

835                 840                 845
His Leu Glu Gln Asn Thr Lys Glu Leu Lys Ala Ser Gln Glu Asn Gln
    850                 855                 860
Arg Asp Leu Gly Asn Gly Leu Thr Val Glu Ser Phe Ser Asn Lys Ile
865                 870                 875                 880
Gln Asn Lys Cys Ser Gly Glu Glu Gln Lys Glu His Pro Gln Ser Ser
                885                 890                 895
Glu Ala Gln Asp Lys Ser Lys Leu Trp Leu Leu Lys Asp Arg Asp Leu
            900                 905                 910
Ala Arg Pro Lys Glu Gln Glu Arg Arg Arg Arg Glu Ala Met Val Gly
        915                 920                 925
Thr Ile Asp Met Thr Leu Gln Ser Asp Ile Met Thr Met Phe Glu Asn
    930                 935                 940
Asn Phe Asp
945

<210> 686
<211> 3106
<212> DNA
<213> Homo Sapiens

<400> 686
gtggcaagat gttcctggga ggtcaagtta agagtcaaaa ataattcatt agatttaaca      60
atttagcatg gacatgtact tgtagacagg attcaaagca gttaagaatg tctctgccaa     120
gtcgacaaac agctattatt gttaaccctc ctccaccaga atatataaat actaagaaaa     180
atgggcgatt gacaaatcaa cttcagtatc tacaaaaagt tgtcctaaag gatttatgga     240
agcatagttt ttcatggccc tttcaacgtc ctgtggatgc tgtgaaacta aagttgcctg     300
attattatac cattataaaa aacccaatgg atttaaatac aattaagaag cgcttggaga     360
ataaatatta tgcgaaggct tcagaatgta tagaagactt caatacaatg ttctcaaatt     420
gttatttata taacaagcct ggagatgaca ttgttcttat ggcacaagct ctagagaagc     480
tgtttatgca gaaattatct cagatgccac aagaagagca agttgtgggt gttaaggaaa     540
gaatcaagaa aggcactcaa cagaatatag ctgtttcttc tgctaaagaa aaatcatcac     600
ccagcgcaac agaaaaagta tttaagcagc aagaaattcc ttctgtattt cctaagacat     660
ctatttctcc cttgaacgtg gtacagggag cttcagtcaa ctccagttca caaactgcgg     720
cccaagttac aaaaggtgtg aagaggaaag cagatacaac aactcctgca acttcagcag     780
ttaaagcaag tagtgaattt tctccaacat tcacagaaaa atcagtggca ctgccaccta     840
taaaagaaaa tatgccaaag aatgttttgc cagattctca gcaacaatat aatgttgtgg     900
agactgttaa agtaactgaa caattaaggc actgtagtga gattcttaaa gaaatgcttg     960
caaagaaaca tttttcatat gcatggccct tttataatcc tgttgacgtt aatgctttgg    1020
gactccataa ctactatgac gttgtcaaaa atccgatgga tcttggaact attaaggaga    1080
aaatggataa ccaagaatat aaggatgcat actcatttgc ggcagatgtt agattaatgt    1140
tcatgaattg ctacaagtac aatcctccag atcacgaagt tgtgacaatg gcaagaatgc    1200
ttcaggatgt tttcgaaacg catttttcaa agatcccgat tgaacctgtt gagagtatgc    1260
ctttatgtta catcaaaaca gatatcacag aaaccactgg tagagagaac actaatgaag    1320
cctcctctga agggaactct tctgatgatt ctgaagatga gcgagttaag cgtcttgcaa    1380
agcttcagga gcagcttaaa gctgtacatc aacagctcca ggttttgtcc caagtacctt    1440
tccgtaagct aaataaaaag aaagagaagt ctaaaaagga aaagaaaaaa gaaaaggtta    1500
ataacagcaa tgaaaatcca agaaaaatgt gtgagcaaat gaggctaaag gaaaagtcca    1560
agagaaatca gccaaagaaa aggaaacaac agttcattgg tctaaaatct gaagatgaag    1620
ataatgctaa acctatgaac tatgatgaga aaaggcagtt aagtctgaat ataaacaaac    1680
tccctggaga taaacttggg cgagtagttc acataataca atcaagagag ccttctctga    1740
gcaattccaa tcctgatgag atagagatag actttgaaac actgaagcca tcaacactaa    1800
gagaattaga aaaatatgtt tcggcatgtc taagaaagag accattaaaa cctcctgcta    1860
agaaaataat gatgtccaaa gaagaacttc actcacagaa aaaacaggaa ttggaaaagc    1920
ggttactgga tgttaataat cagttaaatt ctagaaaacg tcaaacaaaa tctgataaaa    1980

cgcaaccatc caaagctgtt gaaaatgttt cccgactgag tgagagcagc agcagcagca 2040
gcagctcatc agagtctgaa agtagcagca gtgacttaag ctcttcagac agcagtgatt 2100
ctgaatcaga aatgttccct aagtttacag aagtaaaacc aaatgattct ccttctaaag 2160
agcatgtaaa gaaaatgaag aatgaatgca tactgcctga aggaagaaca ggcgtcacac 2220
agataggata ttgtgtgcaa gacacaacct ctgccaatac tacccttgtt catcagacca 2280
caccttcaca tgtaatgcca ccaaatcacc accaattagc atttaattat caagaattag 2340
aacatttaca gactgtgaaa aacatttcac ctttacaaat tctgcctccc tcaggtgatt 2400
ctgaacagct ctcaaatggc ataactgtga tgcatccatc tggtgatagt gacacaacga 2460
tgttagaatc tgaatgtcaa gctcctgtac agaaggatat aaagattaag aatgcagatt 2520
catggaaaag tttaggcaaa ccagtgaaac catcaggtgt aatgaaatcc tcagatgagc 2580
tcttcaacca atttagaaaa gcagccatag aaaaggaagt aaaagctcgg acacaggaac 2640
tcatacggaa gcatttggaa caaaatacaa aggaactaaa agcatctcaa gaaaatcaga 2700
gggatcttgg gaatggattg actgtagaat ctttttcaaa taaaatacaa aacaagtgct 2760
ctggagaaga gcagaaagaa catccgcagt catcagaagc tcaagataaa tccaaactct 2820
ggcttctcaa agaccgtgat ttagccaggc cgaaagaaca agagaggagg aggagagaag 2880
ccatggtggg taccattgat atgacccttc aaagtgacat tatgacaatg tttgaaaaca 2940
actttgatta aaactcagtt tttaaattaa ccatccactt aaaatgaatg gtaaaagatc 3000
aaaatgcata tggtaaaatg attgctttca gataacaaga taccaatctt atattgtatt 3060
ttgactgctc taaaatgatt aaacagtttt cacttacaaa aaaaaa 3106

<210> 687
<211> 1759
<212> DNA
<213> Homo Sapiens

<400> 687
gtcactccgc aattagacag ctaagagatc tgtgttactt ccctcacata tataaataat 60
tttaaataaa aatcatggcg tgaataattt ctttcctcta ccgatttgaa gctatccatt 120
tggaagacca ctctgaagag atgaaataag tcttctgcca aagattactt attaatttac 180
aaggaaaagg ggaagttttg ttcctctccg tgaatttgat tgaaaatcga gggctttctc 240
gaatagtttt ggcatccagg gtcatttttc attaaaaaga gaaaagtcat gtcaaatatg 300
aatttccgca gattattcag cactagaccc tgggagattc tgtaaagagg ggttttgtta 360
tactcaactt ttccgggtaa aacaaacaca aatactcctc ctccaagggg cgggggcggt 420
gcctaggtga tgcaccaatc acagcgcgcc ctaccctata taagccccga ggccgcccgg 480
gtgtttcatg cttttcgctg gttattacat cttgcgtttc tctgttgtta tgtctgaaac 540
cgtgcctgca gcttctgcca gtgctggtct agccgctatg gagaaacttc caaccaagaa 600
gcgagggagg aagccggctg gcttgataag tgcaagtcgc aaagtgccga acctctctgt 660
gtccaagttg atcaccgagg ccctttcagt gtcacaggaa cgagtaggta tgtctttggt 720
tgcgctcaag aaggcattgg ccgctgctgg ctacgacgta gagaagaata acagccgcat 780
caaactgtcc ctcaagagct tagtgaacaa gggaatcctg gtgcaaacca ggggtactgg 840
tgcttccggt tcctttaagc ttagtaagaa ggtgattcct aaatctacac gaagcaaggc 900
taaaaagtca gtttctgcca agaccaagaa gctggtttta tccaggactc ccaagtcacc 960
aaagactgct aaaaccaata agagagccaa gaagccgaga gcgacaactc ctaaaactgt 1020
taggagcggg agaaaggcta aaggagccaa gggtaagcaa cagcagaaga gcccagtgaa 1080
ggcaagggct tcgaagtcaa aattgaccca acatcatgaa gttaatgtta gaaaggccac 1140
atctaagaag taaagagctt tccgggaggc caatttggaa agaacccaaa ggctctttta 1200
agagccaccc acattatttt aagatggcgt aacactggaa acaagtttct gtgacagtta 1260
tctataggtt taagttgtga tgcagctgag ttgaaaaggc ttgagattgg agaattaatt 1320
caggccaggc ttcaagacca tcctgggcaa catagccaga ctaccatcta taccaggggt 1380
cctcattccc ccggccaccg accggtaacc ggtccctgtc catggcacgt tatgaattga 1440
gccgcacagc tgaggggtga gcgaacatta accaactgag ctccaccgcc tgtcaggtta 1500
gctgcagcat tagatagatt ctcataagct caaactgtat tgtgaatggc acatgcaagg 1560
gatctaggtt tcaggctcct tgtgacaatc taatgcctga tgatctgagg ttggagcagt 1620
tttagtccgg aaatcattgc tcccagcccc tgcaccccct ggtccgtggt ataattgtct 1680
tacacaaacg gtctcttgtg tcaaaaaggt tggagactac tggtttttac aaaaaagtaa 1740

attagtcaag catggttgg 1759

<210> 688
<211> 207
<212> PRT
<213> Homo Sapiens

<400> 688

<210> 689
<211> 1464
<212> DNA
<213> Homo Sapiens

<400> 689
agtaccgggt acgcaggggt gcctcaacca cactccgtcc acggactctc cgttatttta 60
ggaggtccct ggccaaagat ttatttctct tgacaaccaa gggcctccgt ctggatttcc 120
aaggaagaat ttcctctgaa gcaccggaac ttgctactac cagcaccatg ccctaccaat 180
atccagcact gaccccggag cagaagaagg agctgtctga catcgctcac cgcatcgtgg 240
cacctggcaa gggcatcctg gctgcagatg agtccactgg gagcattgcc aagcggctgc 300
agtccattgg caccgagaac accgaggaga accggcgctt ctaccgccag ctgctgctga 360
cagctgacga ccgcgtgaac ccctgcattg ggggtgtcat cctcttccat gagacactct 420
accagaaggc ggatgatggg cgtcccttcc cccaagttat caaatccaag ggcggtgttg 480
tgggcatcaa ggtagacaag ggcgtggtcc ccctggcagg gacaaatggc gagactacca 540
cccaagggtt ggatgggctg tctgagcgct gtgcccagta caagaaggac ggagctgact 600
tcgccaagtg gcgttgtgtg ctgaagattg gggaacacac cccctcagcc ctcgccatca 660
tggaaaatgc caatgttctg gcccgttatg ccagtatctg ccagcagaat ggcattgtgc 720
ccatcgtgga gcctgagatc ctccctgatg gggaccatga cttgaagcgc tgccagtatg 780
tgaccgagaa ggtgctggct gctgtctaca aggctctgag tgaccaccac atctacctgg 840

aaggcacctt gctgaagccc aacatggtca ccccaggcca tgcttgcact cagaagtttt 900
ctcatgagga gattgccatg gcgaccgtca cagcgctgcg ccgcacagtg ccccccgctg 960
tcactgggat caccttcctg tctggaggcc agagtgagga ggaggcgtcc atcaacctca 1020
atgccattaa caagtgcccc ctgctgaagc cctgggccct gaccttctcc tacggccgag 1080
ccctgcaggc ctctgccctg aaggcctggg gcgggaagaa ggagaacctg aaggctgcgc 1140
aggaggagta tgtcaagcga gccctggcca acagccttgc ctgtcaagga aagtacactc 1200
cgagcggtca ggctggggct gctgccagcg agtccctctt cgtctctaac cacgcctatt 1260
aagcggaggt gttcccaggc tgcccccaac aactccaggc cctgccccct cccactcttg 1320
aagaggaggc cgcctcctcg gggctccagg ctggcttgcc cgcgctcttt cttccctcgt 1380
gacagtggtg tgtggtgtcg tctgtgaatg ctaagtccat caccctttcc ggcacactgc 1440
caaataaaca gctatttaag gggg 1464

<210> 690
<211> 363
<212> PRT
<213> Homo Sapiens

<400> 690
Pro Tyr Gln Tyr Pro Ala Leu Thr Pro Glu Gln Lys Lys Glu Leu Ser
1               5                   10                  15
Asp Ile Ala His Arg Ile Val Ala Pro Gly Lys Gly Ile Leu Ala Ala
            20                  25                  30
Asp Glu Ser Thr Gly Ser Ile Ala Lys Arg Leu Gln Ser Ile Gly Thr
        35                  40                  45
Glu Asn Thr Glu Glu Asn Arg Arg Phe Tyr Arg Gln Leu Leu Leu Thr
    50                  55                  60
Ala Asp Asp Arg Val Asn Pro Cys Ile Gly Gly Val Ile Leu Phe His
65                  70                  75                  80
Glu Thr Leu Tyr Gln Lys Ala Asp Asp Gly Arg Pro Phe Pro Gln Val
                85                  90                  95
Ile Lys Ser Lys Gly Gly Val Val Gly Ile Lys Val Asp Lys Gly Val
            100                 105                 110
Val Pro Leu Ala Gly Thr Asn Gly Glu Thr Thr Thr Gln Gly Leu Asp
        115                 120                 125
Gly Leu Ser Glu Arg Cys Ala Gln Tyr Lys Lys Asp Gly Ala Asp Phe
    130                 135                 140
Ala Lys Trp Arg Cys Val Leu Lys Ile Gly Glu His Thr Pro Ser Ala
145                 150                 155                 160
Leu Ala Ile Met Glu Asn Ala Asn Val Leu Ala Arg Tyr Ala Ser Ile
                165                 170                 175
Cys Gln Gln Asn Gly Ile Val Pro Ile Val Glu Pro Glu Ile Leu Pro
            180                 185                 190
Asp Gly Asp His Asp Leu Lys Arg Cys Gln Tyr Val Thr Glu Lys Val
        195                 200                 205
Leu Ala Ala Val Tyr Lys Ala Leu Ser Asp His His Ile Tyr Leu Glu
    210                 215                 220
Gly Thr Leu Leu Lys Pro Asn Met Val Thr Pro Gly His Ala Cys Thr
225                 230                 235                 240
Gln Lys Phe Ser His Glu Glu Ile Ala Met Ala Thr Val Thr Ala Leu
                245                 250                 255
Arg Arg Thr Val Pro Pro Ala Val Thr Gly Ile Thr Phe Leu Ser Gly
            260                 265                 270
Gly Gln Ser Glu Glu Glu Ala Ser Ile Asn Leu Asn Ala Ile Asn Lys
        275                 280                 285
Cys Pro Leu Leu Lys Pro Trp Ala Leu Thr Phe Ser Tyr Gly Arg Ala

290                 295                 300
Leu Gln Ala Ser Ala Leu Lys Ala Trp Gly Gly Lys Lys Glu Asn Leu
305                 310                 315                 320
Lys Ala Ala Gln Glu Glu Tyr Val Lys Arg Ala Leu Ala Asn Ser Leu
                325                 330                 335
Ala Cys Gln Gly Lys Tyr Thr Pro Ser Gly Gln Ala Gly Ala Ala Ala
            340                 345                 350
Ser Glu Ser Leu Phe Val Ser Asn His Ala Tyr
        355                 360

<210> 691
<211> 1216
<212> DNA
<213> Homo Sapiens

<400> 691
atgctcctcg atgtggagcc gctggagcct acacttagca acatcatcga gcagcgcagc      60
ctgaagtgga tcttcgtcgg gggcaagggt ggtgtgggca agaccacctg cagctgcagc     120
ctggcagtcc agctctccaa ggggcgtgag agtgttctga tcatctccac agacccagca     180
cacaacatct cagatgcttt tgaccagaag ttctcaaagg tgcctaccaa ggtcaaaggc     240
tatgacaacc tctttgctat ggagattgac cccagcctgg gcgtggcgga cgtgcctgac     300
gagttcttcg aggaggacaa catgctgagc atgggcaaga agatgatgca ggaggccatg     360
agcgcatttc ccggcatcga tgaggccatg agctatgccg aggtcatgag gctggtgaag     420
ggcatgaact tctcggtggt ggtatttgac acggcaccca cgggccacac cctgaggctg     480
ctcaacttcc ccaccatcgt ggagcggggc ctgggccggc ttatgcagat caagaaccag     540
atcagccctt tcatctcaca gatgtgcaac atgctgggcc tgggggacat gaacgcagac     600
cagctggcct ccaagctgga ggagacgctg cccgtcatcc gctcagtcag cgaacagttc     660
aaggaccctg agcagacaac tttcatctgc gtatgcattg ctgagttcct gtccctgtat     720
gagacagaga ggctgatcca ggagctggcc aagtgcaaga ttgacacaca caatataatt     780
gtcaaccagc tcgtcttccc cgaccccgag aagccctgca agatgtgtga ggcccgtcac     840
aagatccagg ccaagtatct ggaccagatg gaggacctgt atgaagactt ccacatcgtg     900
aagctgccgc tgttacccca tgaggtgcgg ggggcagaca aggtcaacac cttctcggcc     960
ctcctcctgg agccctacaa gccccccagt gcccagtagc acagctgcca gccccaaccg    1020
ctgccatttc acactcaccc tccaccctcc ccaccccctc ggggcagagt ttgcacaaag    1080
tcccccccat aatacagggg gagccacttg ggcaggaggc agggaggggt ccattccccc    1140
tggtggggct ggtggggagc tgtagttgcc ccctacctct cccacctctt gctcttcaat    1200
aaatgatctt aaactg                                                    1216

<210> 692
<211> 1958
<212> DNA
<213> Homo Sapiens

<400> 692
gctgctgcgc ccgcggctcc ccagtgcccc gagtgccccg cgggccccgc gagcgggagt      60
gggacccagc cctaggcaga acccaggcgc cgcgcccggg acgcccgcgg agagagccac     120
tcccgcccac gtcccatttc gcccctcgcg tccggagtcc ccgtggccag atctaaccat     180
gagctaccct ggctatcccc cgcccccagg tggctaccca ccagctgcac caggtggtgg     240
tccctgggga ggtgctgcct accctcctcc gcccagcatg ccccccatcg ggctggataa     300
cgtggccacc tatgcggggc agttcaacca ggactatctc tcgggaatgg cggccaacat     360
gtctgggaca tttggaggag ccaacatgcc caacctgtac cctggggccc ctggggctgg     420
ctacccacca gtgccccctg gcggctttgg gcagcccccc tctgcccagc agcctgttcc     480
tccctatggg atgtatccac ccccaggagg aaacccaccc tccaggatgc cctcatatcc     540
gccataccca ggggcccctg tgccgggcca gcccatgcca ccccccggac agcagccccc     600
aggggcctac cctgggcagc caccagtgac ctaccctggt cagcctccag tgccactccc     660

tgggcagcag cagccagtgc cgagctaccc aggatacccg gggtctggga ctgtcacccc 720
cgctgtgccc ccaacccagt ttggaagccg aggcaccatc actgatgctc ccggctttga 780
ccccctgcga gatgccgagg tcctgcggaa ggccatgaaa ggcttcggga cggatgagca 840
ggccatcatt gactgcctgg ggagtcgctc caacaagcag cggcagcaga tcctactttc 900
cttcaagacg gcttacggca aggatttgat caaagatctg aaatctgaac tgtcaggaaa 960
ctttgagaag acaatcttgg ctctgatgaa gaccccagtc ctctttgaca tttatgagat 1020
aaaggaagcc atcaaggggg ttggcactga tgaagcctgc ctgattgaga tcctcgcttc 1080
ccgcagcaat gagcacatcc gagaattaaa cagagcctac aaagcagaat tcaaaaagac 1140
cctggaagag gccattcgaa gcgacacatc agggcacttc cagcggctcc tcatctctct 1200
ctctcaggga aaccgtgatg aaagcacaaa cgtggacatg tcactcgccc agagagatgc 1260
ccaggagctg tatgcggccg gggagaaccg cctgggaaca gacgagtcca agttcaatgc 1320
ggttctgtgc tcccggagcc gggcccacct ggtagcagtt ttcaatgagt accagagaat 1380
gacaggccgg gacattgaga agagcatctg ccgggagatg tccggggacc tggaggaggg 1440
catgctggcc gtggtgaaat gtctcaagaa taccccagcc ttctttgcgg agaggctcaa 1500
caaggccatg aggggggcag gaacaaagga ccggaccctg attcgcatca tggtgtctcg 1560
cagcgagacc gacctcctgg acatcagatc agagtataag cggatgtacg gcaagtcgct 1620
gtaccacgac atctcgggag atacttcagg ggattaccgg aagattctgc tgaagatctg 1680
tggtggcaat gactgaacag tgactggtgg ctcacttctg cccacctgcc ggcaacacca 1740
gtgccaggaa aaggccaaaa gaatgtctgt ttctaacaaa tccacaaata gccccgagat 1800
tcaccgtcct agagcttagg cctgtcttcc acccctcctg acccgtatag tgtgccacag 1860
gacctgggtc ggtctagaac tctctcagga tgccttttct accccatccc tcacagcctc 1920
ttgctgctaa aatagatgtt tcatttttct gaaaaaaa 1958

<210> 693
<211> 505
<212> PRT
<213> Homo Sapiens

<400> 693

<210> 694
<211> 1141
<212> DNA
<213> Homo Sapiens

<400> 694
cgcagcttgc aaatggcgtc tccctcgctg gagcggccag aaaaaggcgc tggaaaaagt      60
gaatttcgta accagaagcc gaagccggag aaccaagatg aatcagaact ccttacggtt     120
cctgatggtt ggaaggaacc agctttttcc aaagaggaca atcccagagg acttttggag     180
gagagcagtt tcgcaacttt gttcccaaaa tacagggaag cttacttgaa agagtgttgg     240
ccattggtgc agaaagcctt aaatgaacat catgttaatg caaccctgga cctgatcgaa     300
ggcagcatga ctgtttgtac tacaaagaag acttttgatc catatatcat cattagggcc     360
agagatctga taaaactgtt agcaaggagt gtttcatttg aacaggcagt acgaattctt     420
caggatgatg ttgcatgtga catcattaaa ataggttctt tagtaaggaa taaagagaga     480
tttgtaaaac gaagacaacg gcttattggt cccaaaggat ctacattgaa ggcattggaa     540
ctcttaacta attgttacat tatggttcag ggaaacacag tttcagccat tggacctttt     600

agtggcttaa aagaggttag aaaagtagtc cttgatacta tgaagaatat tcatccaatt 660
tataacatta aaagcttaat gattaagaga gagttggcaa aagattctga attacgatca 720
caaagttggg agagattttt gccacagttc aaacacaaaa atgtgaataa acgcaaggaa 780
ccaaagaaaa aaactgttaa gaaagatata cgccattccc accaccacaa ccagaaagtc 840
agatcgataa agaattggct agtggtgaat actttttgaa ggcaaatcag aagaagcggc 900
agaaaatgaa gcaataaagg ctaaacaagc agaagccatc agtaagagac aagaggaaag 960
aaacaaagca tttattccac ctaaggaaaa accaattgtg aaacctaagg aagcttctac 1020
tgaaactaaa attgatgtgg ccagcatcaa ggaaaaggtt aagaaagcaa agaataagaa 1080
actgggagct cttacagctg aagaaattgc acttaagatg gaggcagatg aaaaaaaaaa 1140
a 1141

<210> 695
<211> 288
<212> PRT
<213> Homo Sapiens

<400> 695
Met Ala Ser Pro Ser Leu Glu Arg Pro Glu Lys Gly Ala Gly Lys Ser
1               5                   10                  15
Glu Phe Arg Asn Gln Lys Pro Lys Pro Glu Asn Gln Asp Glu Ser Glu
            20                  25                  30
Leu Leu Thr Val Pro Asp Gly Trp Lys Glu Pro Ala Phe Ser Lys Glu
        35                  40                  45
Asp Asn Pro Arg Gly Leu Leu Glu Glu Ser Ser Phe Ala Thr Leu Phe
    50                  55                  60
Pro Lys Tyr Arg Glu Ala Tyr Leu Lys Glu Cys Trp Pro Leu Val Gln
65                  70                  75                  80
Lys Ala Leu Asn Glu His His Val Asn Ala Thr Leu Asp Leu Ile Glu
                85                  90                  95
Gly Ser Met Thr Val Cys Thr Thr Lys Lys Thr Phe Asp Pro Tyr Ile
            100                 105                 110
Ile Ile Arg Ala Arg Asp Leu Ile Lys Leu Leu Ala Arg Ser Val Ser
        115                 120                 125
Phe Glu Gln Ala Val Arg Ile Leu Gln Asp Asp Val Ala Cys Asp Ile
    130                 135                 140
Ile Lys Ile Gly Ser Leu Val Arg Asn Lys Glu Arg Phe Val Lys Arg
145                 150                 155                 160
Arg Gln Arg Leu Ile Gly Pro Lys Gly Ser Thr Leu Lys Ala Leu Glu
                165                 170                 175
Leu Leu Thr Asn Cys Tyr Ile Met Val Gln Gly Asn Thr Val Ser Ala
            180                 185                 190
Ile Gly Pro Phe Ser Gly Leu Lys Glu Val Arg Lys Val Val Leu Asp
        195                 200                 205
Thr Met Lys Asn Ile His Pro Ile Tyr Asn Ile Lys Ser Leu Met Ile
    210                 215                 220
Lys Arg Glu Leu Ala Lys Asp Ser Glu Leu Arg Ser Gln Ser Trp Glu
225                 230                 235                 240
Arg Phe Leu Pro Gln Phe Lys His Lys Asn Val Asn Lys Arg Lys Glu
                245                 250                 255
Pro Lys Lys Lys Thr Val Lys Lys Asp Ile Arg His Ser His His His
            260                 265                 270
Asn Gln Lys Val Arg Ser Ile Lys Asn Trp Leu Val Val Asn Thr Phe
        275                 280                 285

<210> 696

<211> 1008
<212> DNA
<213> Homo Sapiens

<400> 696
agcaggctag gaagactgca tcagttctta gtaaagatga tgtggcacct gaaagtggtg 60
atactacagt gaagaaacct gaatcaaaga aggaacagac cccagagcat gggaagaaaa 120
aacgtggcag aggaaaagcc caagttaaag caacaaatga atccgaagac gaaatcccac 180
agctggtacc aataggaaag aagactccag ctaatgaaaa agtagagatt caaaaacatg 240
ccacagggaa gaagtctcca gcaaagagtc ctaatcccag cacacctcgt gggaagaaaa 300
agaaaggctt tgccagcatc tgagacccca aaagctgcag agtctgagac cccagggaaa 360
agcccagaga agaagcctaa aatcaaagaa gaggcagtga aggaaaaaag tccttcgctg 420
gggaaaaaag atgcgagaca gactcccaaa aaagccagag gccaagtttt tcaccattcc 480
tagtaaatct gtgagaaaag cttcccacac ccccaaaaaa tggcccaaaa aacccaaagt 540
accccagtcg acctaaagtc agtgattcaa ctggaaggaa acctcaatgc tgcctccaga 600
gctttttgga aatactcaga tcctggccgc ctttgtaacc ttctctaaac gtcaggcctg 660
gacttaaaag attttttaaa acctccataa gtagtccagg ggcggtggct cacgcctgta 720
atcccagcac tttgggaggc cgaggcaggc ggatcacaag gtcaacgaga tcgagaccat 780
cctggccaac atggtgaaac cctgtctgta ccaaaaatac aaaaattaat tgggcatggt 840
ggtggacacc tgtaatccca gctactaggg aggctgaggc aggagaattg cttgaacctg 900
ggaggcggag gttgcagtga gccactgcac tccagcctga tgacagagca agactcagtc 960
tcaaaaataa ataaaaataa taaaacctcc ataagtaatc ctgaaaaa 1008

<210> 697
<211> 685
<212> DNA
<213> Homo Sapiens

<400> 697
acgagctgca ctccagcctg ggcgacagag ggaaactcca tctcaaaaaa aaaaanaaaa 60
aaaaaanaaa aagaaaaaag anaatgccca gcgcggtggc taatgcctgt aaccctagtg 120
agacagccaa gtaaaaacgg ctcccaagac aatctacaag cactgggagg atggggtgca 180
gcaccaaaat gttcacacca tttgcagagg ggaacagcct ggcccctgct gttccaggat 240
agtaaccagg aattcagttg gtgagatgga cagcctgtta gcaggactcc atctcacttt 300
gctgtgttgt tctttttccc ttttgcccaa taaattngta acccctcacc tttcaaagtg 360
tctgcgtgcc taatctttcc ctgccatgtg accagaaccc ggttttgttt acaacaccag 420
cactttggga ggcgaagatg ggctgattgc ttgagctcag gggtttaaga acagcctggg 480
caacatagtg aaaccctagt ttttaccaaa aatacgaaaa ttaaccaggc atgcctgtta 540
tcccagctga ggcacaagaa tcccttgaac ccaggaggcn gaannctaat tnnaacccga 600
aaatttgcnc ccactggccc ccccaggcgg aagctagtga gccgagattg cgccactgca 660
cccctgagac gctgtntcaa aaaaa 685

<210> 698
<211> 1205
<212> DNA
<213> Homo Sapiens

<400> 698
   ggcacgaggg tgtaggccgc tgcaggccnc catganccgg cttccggatg actacgaccc 60
   ctacgcggtt gaagagccta gcgacgagga gccggctttg agcagctctg aggatgaagt 120
   ggatgtgctt ttacatggaa ctcctgacca aaaacgaaaa ctcatcagag aatgtcttac 180
   cggagaaagt gaatcatcta gtgaagatga atttgaaaag gagatggaag ctgaattaaa 240
   ttctaccatg aaaacaatgg aggacaagtt atcctctctg ggaactggat cttcctcagg 300
   aaatggaaaa gttgcaacag ctccgacaag gtactacgat gatatatatt ttgattctga 360
   ttccgaggat gaagacagag cagtacaggt gaccaagaaa aaaaagaaga aacaacacaa 420
   gattccaaca aatgacgaat tactgtntga tcctgaaaaa gataacagag atcaggcctg 480
   ggttgatgca cagagaaggg gttaccatgg tttgggacca cagagatcac gtcaacaaca 540
   gcctgttcca aatagtgatg ctgtcttgan ttgtcctgcc tgcatgacca cactttgcct 600
   tgattgccaa aggcatgant catacaaaac tcaatataga gcaatgtttg taatgaattg 660
   ttctattaac aaagaggagg ttctaagata taaagcctca gagaacagga agaaaaggcg 720
   ggtccataag aagatgaggt ctaaccggga agatgctgcc gagaaggcag agacagatgt 780
   ggaagaaatc tatcacccag tcatgtgcac tgaatgttcc actgaagtgg cagtctacga 840
   caaggatgaa gtctttcatt ttttcaatgt tttagcaagc cattcctaaa cagcccaact 900
   ggcatttaat tacccaatac tgtatataag gcaaatatgg acagttactt tcctcttgcc 960
   tgttcatatc cttcagtgac attgaggaag cagtgtttct ctttttaaag gagaatagtt 1020
   gtcaaccttc attcatctct tacatctctc accctctcct tttttttttc tttgattttc 1080
   ccccttattg atgggactga tattcattct gtttttgatg aacatttgga aactgtcggg 1140
   ctttttatta aagctctgta gaattaaaat gttctggaat tataagcaaa aaaaaaaaaa 1200
   aaaaa 1205

<210> 699
<211> 1427
<212> DNA
<213> Homo Sapiens

<400> 699
anannnctgg agccgcgcgc ctcgcaggtc gacactagtg gatccaaaga attcggcacg 60
agcagtggta gcaaatgaag ccaaactgta tcttgaaaaa cctgttgttc ctttaaatat 120
gatgttgcca caagctgcat tggagactca ttgcagtaat atttccaatg tgccacctac 180
aagagagata cttcaagtct ttcttactga tgtacacatg aaggaagtaa ttcagcagtt 240
cattgatgtc ctgagtgtag cagtcaagaa acgtgtcttg tgtttaccta gggatgaaaa 300
cctgacagca aatgaagttt tgaaaacgtg tgataggaaa gcaaatgttg caatcctgtt 360
ttctgggggc attgattcca tggttattgc aacccttgct gaccgtcata ttcctttaga 420
tgaaccaatt gatcttctta atgtagcttt catagctgaa gaaaagacca tgccaactac 480
ctttaacaga gaagggaata aacagaaaaa taaatgtgaa ataccttcag aagaattctc 540
taaagatgtt gctgctgctg ctgctgacag tcctaataaa catgtcagtg taccagatcg 600
aatcacagga agggcgggac taaaggaact acaagctgtt agcccttccc gaatttggaa 660
ttttgttgaa attaatgttt ctatggaaga actgcagaaa ttaagaagaa ctcgaatatg 720
tcacttaatt cggccattgg atacagtttt ggatgatagc attggctgtg cagtctggtt 780
tgcttctaga ggaattggtt ggttagtggc ccaggaagga gtgaaatcct atcagagcaa 840
tgcaaaggta gttctcactg gaattggtgc agatgagcaa cttgcaggtt attctcgtca 900
tcgtgtccgc tttcagtcgc atgggctgga aggattgaat aaggaaataa tgatggaact 960
gggtcgaatt tcttctagaa atcttggtcg tgatgacaga gttattggtg atcatggaaa 1020
agaagcaaga tttcctttcc tggatgaaaa tgttgtctcc tttctaaatt ctctgccgat 1080
ttgggaaaaa gcaaacttga ctttaccccg aggaattggt gaaaaattac ttttacgcct 1140
tgcagctgtg gaacttggtc ttacagcctc tgctcttctc gccaaacggg ccatgcagtt 1200
tggatcaaga attgcaaaaa tggaaaaaat taatgaaaag gcatctgata aatgtggacg 1260
gctccaaatc atgtccttag aaaatctttc tattgaaaag gagactaaat tgtaatgtga 1320
ttcacaatgt aacaatataa aaataagttt ttatataatt atataaaagt aagatactct 1380
gctgctttac tattgtataa tatagtagtt ttaaagttca aaaaaaa 1427

<210> 700
<211> 1967
<212> DNA
<213> Homo Sapiens

<400> 700
ggcacgaggg aaagaggtac gaaatgatga aggaaaagta attcggttcc attgtaaatt 60
atgcgagtgc agctttaatg atcccaatgc taaggagatg cacttaaaag ggcgaagaca 120
cagacttcaa tataaaaaaa aagtaaatcc agatttgcaa gtagaagtaa agcctagtat 180

tcgagcaaga aagattcaag aagagaaaat gaggaagcaa atgcagaagg aggagtactg 240
gcgaagacga gaagaagagg agcgttggag aatggaaatg agacgttatg aagaggacat 300
gtactggagg agaatggagg aagaacaaca tcattgggat gatcgccgcc gaatgccaga 360
tggaggttat cctcatggtc ctccaggccc attaggcctt ctgggagtcc gaccaggcat 420
gcctcctcag cctcaggggc ctgcaccctt acgtcgtcct gactcatctg atgaccgttt 480
ctgggagtcc gaccaggcat gcctcctcag cctcaggggc ctgcaccctt acgtcgtcct 540
gactcatctg atgaccgtta tgtaatgaca aaacatgcca ccatttatcc aactgaagag 600
gagttacagg cagttcagaa aattgtttct attactgaac gtgctttaaa actcgtttca 660
gacagtttgt ctgaacatga gaagaacaag aacaaagagg gagatgataa gaaagaggga 720
ggtaaagaca gagctttgaa aggagttttg cgagtgggag tatttgcaaa agggattact 780
tctcccggag atagaaatgt caaccttgtt ttgctgtgtc agagaaacct tcaaagacat 840
tattaagccg tattgcagaa aacctaccca aacagcttgc tgttataagc cctgagaagt 900
atgacataaa atgtgctgta tctgaagcgg caataatttt gaattcatgt gtggaaccca 960
aaatgcaagt cactatcaca ctgacatctc caattattcg agaagagaac atgagggaag 1020
gagatgtaac ctcgggtatg gtgaaagacc caccggacgt cttggacagg caaaaatgcc 1080
ttgacgctct ggctgctcta cgccacgcta agtggttcca ggctagagct aatggtctgc 1140
agtcctgtgt gattatcata cgcattcttc gagacctctg tcagcgagtt ccaacttggt 1200
ctgattttcc aagctgggct atggagttac tagtagagaa agcaatcagc agtgcttcta 1260
gccctcagag ccctggggat gcactgagaa gagtttttga atgcatttct tcagggatta 1320
ttcttaaagg tagtcctgga cttctggatc cttgtgaaaa ggatcccttt gataccttgg 1380
caacaatgac tgaccagcag cgtgaagaca tcacatccag tgcacagttt gcattgagac 1440
tccttgcatt ccgccagata cacaaagttc taggcatgga tccattaccg caaatgagcc 1500
aacgttttaa catccacaac aacaggaaac gaagaagaga tagtgatgga gttgatggat 1560
ttgaagctga ggggaaaaaa gacaaaaaag attatgataa cttttaaaaa gtgtctgtaa 1620
atcttcagtg ttaaaaaaac agatgcccat ttgttggctg tttttcattc ataataatgt 1680
ctacattgaa aaatttatca agaatttaaa ggatttcatg gaagaaccaa gtttttctat 1740
gatattaaaa aatgtacagt gttaggtatt atttgaatgg aaagacaccc aaaaaaaaaa 1800
atgtgctccg actaggggga aaacagtagt tccgattttt tcccattatt tttattttat 1860
tttctggttg ccctagcttc cccccctatt tttgtgtctt ttattaacta gtgcattgtc 1920
ttattaaatc ttcactgtat taaaaaaaga aaaaaaaaaa acaaaaa 1967

<210> 701
<211> 3423
<212> DNA
<213> Homo Sapiens

<400> 701
ggtgaagaat gaaatcgtgg cgaatgtggg gaaaagagaa atcttgcaca atactgagaa 60
agaacaacac acagaggaca cagtgaagga ttgtgtggac atagaggtat tcactgctgg 120
tgagaatacc gaggaccaga aatcctctga agacactgcc ccattcctag gaaccttagc 180
aggtgctacc tatgaggaac aggttcaaag ccaaattctt gagagcgctt ctctccctga 240
aaacacagca caggttgagt caaatgaggt catgggtgca ccagatgaca ggaccagaac 300
tccccttgag ccatccaact gttggagtga cttagatggt gggagccaca cagagaatgt 360
gggagaggca gcggtgactc tccccttgag ccatccaact gttggagtga cttagatggt 420
gggagccaca cagagaatgt gggagaggca gcagtgactc aggttgaaga gcaggcaggc 480
acagtggcct cgtgtccttt agggcatagt gatgacacag tttatcatga tgacaaatgt 540
atggtagagg tcccccaaga gttagagaca agcacagggc atagtttgga gaaagaattc 600
accaaccagg aagcagctga gcccaaggag gttccagcgc acagtacaga agtaggtagg 660
gatcacaacg aagaagaggg tgaagaaaca ggattaaggg acgagaaacc aatcaagaca 720
gaattcctgg ttctccagca ggaactgagg gcaactgtca ggaagcgaca ggtccaagta 780
cagtagacac tcaaaatgaa cccttagata tgaaagagcc cgatgaagaa aagagtgacc 840
aacaggagag ggcattggac catcgcagaa gaagacaaag aacaagaaaa aaaaaaaaaa 900
aaaaaaagcg ggttctaggg cgccgggcgc tcgggcctcg gccatggctc acaggccgaa 960
aaggactttt cggcagcgcg cggctgattc cagcgacagc gatggcgccg aggagtcgcc 1020
tgctgagcct ggggcgccga gggaacttcc ggtcccgggt tctgcggagg aagagccgcc 1080

ctctggagga ggccgcgcgc aggtggcggg actgccccac cgggttcggg gccctcgtgg 1140
ccgggggccgg gtctgggcga gctcccggcg tgccaccaaa gcggctcccc gcgcggacga 1200
aggctcagaa tccagaaccc ttgatgtgtc cacagatgaa gaggataaaa tacatcactc 1260
ctcagaaagt aaggatgatc agggtttgtc ttctgacagt tctagctctc ttggagaaaa 1320
agaactttca tcaacagtta agatcccaga tgcagctttt attcaggcag cccgcagaaa 1380
acgttgaatt ggccagggcc caagatgact atatttcttt ggatgtacaa catacctcct 1440
ccatctctgt aagcagaaat gaagaaacaa gtgaagaaag tcaggaagat gaaaagcaag 1500
atacttggga acaacagcaa atgaggaaag cagttaaaat catagaggaa agagacatag 1560
atctttcctg tggcagtgga tcttcaaaag tgaagaaatt tgatacttcc atttcatttc 1620
cgccagtaaa tttagaaatt ataaagaagc aattaaatac tagattaaca ttactacagg 1680
aaactcaccg ctcacacctg agggagtatg aaaaatacgt acaagatgtc aaaagctcaa 1740
agagtaccat ccagaaccta gagagttcat caaatcaagc tctaaattgt aaattctata 1800
aaagcatgaa aatttatgtg gaaaatttaa ttgactgcct taatgaaaag attatcaaca 1860
tccaagaaat agaatcatcc atgcatgcac tccttttaaa acaagctatg acctttatga 1920
aacgcaggca agatgaatta aaacatgaat caacgtattt acaacagtta tcacgcaaag 1980
atgagacatc cacaagtgga aacttctcag tagatgaaaa aactcagtgg attttagaag 2040
agattgaatc tcgaaggaca aaaagaagac aagcaagggt gctttctggg aattgtaacc 2100
atcaggaagg aacatctagt gatgatgaac tgccttcagc agagatgatt gacttccaaa 2160
aaagccaagg tgacatttta cagaaacaga agaaagtttt tgaagaagtg caagatgatt 2220
tttgtaacat ccagaatatt ttgttgaaat ttcagcaatg gcgagaaaag tttcctgact 2280
cctattatga agctttcatt agtttatgca taccaaagct tttaaatccc ctaatacgag 2340
ttcagttgat tgattggaat cctcttaagt tggaatccac aggtttaaaa gagatgccat 2400
ggttcaaatc tgtagaagaa tttatggata gcagtgtaga agattcaaag aaggaaagta 2460
gttcagataa aaaagtcttg tctgcaatca tcaacaaaac aattattccc cgacttacag 2520
actttgtaga attcctttgg gatcctttgt caacctcaca gacaacaagt ttaataacac 2580
attgcagagt gattcttgaa gaacattcca cttgtgaaaa tgaagttagt aaaagcagac 2640
aggatttact taaatccatt gtttcaagaa tgaaaaaggc agtagaagat gatgttttta 2700
ttcctctgta tccaaagagt gctgtagaaa acaaaacatc acctcattca aagttccaag 2760
aaagacagtt ctggtcaggc ctaaagctct tccgcaatat tcttctttgg aatggactcc 2820
ttacagatga caccttgcaa gaactaggac tagggaagct gctaaatcgt taccttatta 2880
tagcacttct caatgccaca cctgggccag atgtggttaa aaagtgcaac caggtagcag 2940
catgtctacc agaaaaatgg tttgaaaatt ctgccatgag gacatctatt ccacagctag 3000
aaaacttcat tcagttttta ttgcagtctg cacataaatt atctagaagt gaattcaggg 3060
atgaagtcga agaaataatt cttattttgg tgaaaataaa agctttgaat caagcagaat 3120
ccttcatagg agagcatcac ctagaccatc ttaaatcact aattaaagaa gattgaataa 3180
actttattgg aaaatgctaa aattttaata tagttacact cagttccttt gtttgagaag 3240
aagctggtgc ctctctcttc tttattccct gtaatagaag gtaggatttg aaaaaaagca 3300
ggactccacc tctgtattcc cccgtgcttt accttctggc atcatgaaaa gctgccatga 3360
ttctgtggtg ttctaaggaa ttaaatgcac tggagcttta agagctcaac gtgtttccct 3420
ttg 3423

<210> 702
<211> 1106
<212> DNA
<213> Homo Sapiens

<400> 702
ggcacgagca gagacgctgc aaattgcttg tggacggtgt aggccgctgc aggccaccat 60
gaaaccggct tccggatgac tacgacccct acgcggttga agagcctagc gacgaggagc 120
cggctttgag cagctctgag gatgaagtgg atgtgctttt acatggaact cctgaccaaa 180
aacgaaaact catcagagaa tgtcttaccg gagaaagtga atcatctagt gaagatgaat 240
ttgaaaagga gatggaagct gaattaaatt ctaccatgaa aacaatggag gacaagttat 300
cctctctggg aactggatct tcctcaggaa atggaaaagt tgcaacagct ccgacaaggt 360
actacgatga tatatatttt gattctgatt ccgaggatga agacagagca gtacaggtga 420
ccaagaaaaa aaagaagaaa caacacaaga ttccaacaaa tgacgaatta ctgtatgatc 480

ctgaaaaaga taacagagat caggcctggg ttgatgcaca gagaaggggt taccatggtt 540
tgggaccaca gagatcacgt caacaacagc ctgttccaaa tagtgatgct gtcttgaatt 600
gtcctgcctg catgaccaca ctttgccttg attgccaaag gcatgaatca tacaaaactc 660
natatagagc aatgtttgtt atgaattgtt ctattaacaa agaggaagtt ctaagatatn 720
aagcctcnga naacaggaag aaaaggcggg tccatnaaaa aaataaggtc taaccgggaa 780
gatctgncga naaggcagaa acagatgtgg aaaaaatcta tcncccntcc tgtnccctga 840
atgttccctg aaattggagt ctacaacaag gatnaattct tcnntttttc cntgttttag 900
caagccntcc taaacngccc nctggctttt atttccccat actgttttta agggcaaatt 960
tggacagttc cttncccctt gcccgntcnt ntccctcntt gacttgaagg aaccatnttt 1020
ccnnttttaa gggaaaaaat tgttcacctc cttcccctct taattcctcc cccccncctt 1080
tttttccctt gaattncccc cttntg 1106

<210> 703
<211> 1095
<212> DNA
<213> Homo Sapiens

<400> 703
tgccgctcct cttcctcttc ttcttcttct tcttcctctt cttcctagct tccttcacca 60
aatcgcactg gctcctggac tcttttccta tcttcaccac gaactgctgc ttgctcgctt 120
gctcctcagt cctagcttca tcaaacactg gttcctggaa tcctgtctgc tgctgtcttc 180
ctanattcac tgaatccact tctgtgtagc acctgggtca gctgtcaatt aatgctagtc 240
ctcaggattt aaaaaataat cttaactcaa agtccaatgc aaaaacatta agttggtaat 300
tactcttgat cttgaattac ttccgttacg aaagtccttc acatttttca aactaagcta 360
ctatatttaa ggccttccaa attcttctaa ctcttccaaa agccttctgc cttagttttt 420
tttaaattac accagtcctt ttagtagctt tttgatgtga tttttaacca acttcccctt 480
ctagcttcaa gtattcttct aaattggttc tggtctacgt aaacaccctc atcttctcaa 540
gctttacctt ctaacttctg caccaccaga aattaaattg atgggctttt aaaataaatt 600
ggttacaata atttcctcat tttttcagtg ctattttatc caatttttgg ctttatattt 660
ttctatcttc tatacttctc caatactgtc ttagcttgtt tttcattttc tatctgaaac 720
tcttgaaaat aatttcattt tctatcttgt ttctatcttc caaattttct tctaaatttg 780
tacattttgc ccttaacttt ttggtttcct aacntggtct ttttcctccg cctcctaatt 840
ttcanggttt aaatttatct tttttcctcc naaaaatttg nttttaanct nccaaattnc 900
ccnaacctnc nctaatccnt ttccttcgcc ttcccgtnat ttcttgtntt ccaattttcc 960
acttcaaatt ctatcttccc aaaatttttt ctnccaacnc cccaaataaa acttcccnnt 1020
tncgtcgggn ttaataaaag ntttaanagg gttaaaagaa annaatcccc cngttttgga 1080
attnangggt ttaaa 1095

<210> 704
<211> 1968
<212> DNA
<213> Homo Sapiens

<400> 704
cggcagccct cctacctgcg cacgtggtgc cgctgctgct gcctcccgct cgccctgaac 60
ccagtgcctg cagccatggc tcccggccag ctcgccttat ttagtgtctc tgacaaaacc 120
ggccttgtgg aatttgcaag aaacctgacc gctcttggtt tgaatctggt cgcttccgga 180
gggactgcaa aagctctcag ggatgctggt ctggcagtca gagatgtctc tgagttgacg 240
ggatttcctg aaatgttggg gggacgtgtg aaaactttgc atcctgcagt ccatgctgga 300
atcctagctc gtaatattcc agaagataat gctgacatgg ccagacttga tttcaatctt 360
ataagagttg ttgcctgcaa tctctatccc tttgtaaaga cagtggcttc tccaggtgta 420
actgttgagg aggctgtgga gcaaattgac attggtggag taaccttact gagagctgca 480
gccaaaaacc acgctcgagt gacagtggtg tgtgaaccag aggactatgt ggtggtgtcc 540
acggagatgc agagctccga gagtaaggac acctccttgg agactagacg ccagttagcc 600
ttgaaggcat tcactcatac ggcacaatat gatgaagcaa tttcagatta tttcaggaaa 660

cagtacagca aaggcgtatc tcagatgccc ttgagatatg gaatgaaccc acatcagacc 720
cctgcccagc tgtacacact gcagcccaag cttcccatca cagttctaaa tggagcccct 780
ggatttataa acttgtgcga tgctttgaac gcctggcagc tggtgaagga actcaaggag 840
gctttaggta ttccagccgc tgcctctttc aaacatgtca gcccagcagg tgctgctgtt 900
ggaattccac tcagtgaaga tgaggccaaa gtctgcatgg tttatgatct ctataaaacc 960
ctcacaccca tctcagcggc atatgcaaga gcaagagggg ctgataggat gtcttcattt 1020
ggtgattttg ttgcattgtc cgatgtttgt gatgtaccaa ctgcaaaaat tatttccaga 1080
gaagtatctg atggtataat tgccccagga tatgaagaag aagccttgac aatactttcc 1140
aaaaagaaaa atggaaacta ttgtgtcctt cagatggacc aatcttacaa accagatgaa 1200
aatgaagttc gaactctctt tggtcttcat ttaagccaga agagaaataa tggtgtcgtc 1260
gacaagtcat tatttagcaa tgttgttacc aaaaataaag atttgccaga gtctgccctc 1320
cgagacctca tcgtagccac cattgctgtc aagtacactc agtctaactc tgtgtgctac 1380
gccaagaacg ggcaggttat cggcattgga gcaggacagc agtctcgtat acactgcact 1440
cgccttgcag gagataaggc aaactattgg tggcttagac accatccaca agtgctttcg 1500
atgaagttta aaacaggagt gaagagagca gaaatctcca atgccatcga tcaatatgtg 1560
actggaacca ttggcgagga tgaagatttg ataaagtgga aggcactgtt tgaggaagtc 1620
cctgagttac tcactgaggc agagaagaag gaatgggttg agaaactgac tgaagtttct 1680
atcagctctg atgccttctt ccctttccga gataacgtag acagagctaa aaggagtggt 1740
gtggcgtaca ttgcggctcc ctccggttct gctgctgaca aagttgtgat tgaggcctgc 1800
gacgaactgg gaatcatcct cgctcatacg aaccttcggc tcttccacca ctgattttac 1860
cacacactgt tttttggctt gcttatgtgt aggtgaacag tcacgcctga aactttgagg 1920
ataacttttt aaaaaaataa aacagtatct cttaatcact ggaaaaaa 1968

<210> 705
<211> 800
<212> DNA
<213> Homo Sapiens

<400> 705
cctgcaggtc gacactaagt gggatccaaa gaattcggca cgagaaaaga agaagactaa 60
gaataaaaag aataaagact caaaagaaga ccaagtccca tatgtggtag aaaaggaaga 120
gcagttgagg aaagaacaag caaatccaca ctcagtcagt agacttataa aagatgatgc 180
aagtgatgtt caagaggatt ctgcaatgga agacaagttc tatagcctgg atgaattgca 240
tattctggac atgatagagc agggctcagc tggcaaagta actacagact atggagaaac 300
tgaaaaggaa aggcttgctc gtcaaaggca gctttataaa ttgcactatc agtgtgaaga 360
tttcaaaaga cagttgagaa cagtgacttt tcggtggcaa gaaaaccaaa tgcagattaa 420
aaagaaagac aaaattatcg catctcttaa tcaacaagtt gcttttggaa tcaataaggt 480
ttccaaatta cagcgtcaaa tccatgctaa agataatgaa atcaagaacc ttaaagagca 540
actttctatg aaaagatctc agtgggaaat ggaaaaacat aatctgggaa agcacaatga 600
aaacatacgt aagcaaactg aacgcagaaa ctagcagagc tttaacagcc gaaggtgtan 660
ttcttacagt gtcgtanggg antttgggtt tgcctcatcc tagagcaaga ctgaaaagga 720
atgtcccaat cagcntgcca agggtgaccc acatggganc caagcaacct agaaancact 780
tcaatttaaa gggctgcggg 800

<210> 706
<211> 487
<212> DNA
<213> Homo Sapiens

<400> 706
caaaggaagc tcagtttttc ttttattatg agctgcttgt ntgagtggtg taaaattatg 60
tgctcttcaa tatagtgcta aagaagccag ctaattttat caaagcagca gccaaagaag 120
tcaggacaaa tcttcaggac ttgtgaaatg aactgaaaga gcttgaagca gatggaattt 180
taatagttac actatatatg ctcttagtag gtttttttct tgtagtggaa acataactgt 240
tagcatattt cttaggatgt tttttcttgt ctttttaaat tcttatttca ctcatccttt 300

actctcccct caagtattct acactttaat ttcctgaaat aaatttaagg aaaagggaaa 360
tagtaaagaa gtaggaatgg gtgcagcaca ccagcatggc acatgaatac acatgtaact 420
aacctgcaca ttgtgcacat gtaccctaaa acttaaagta taataataat aaaaaaaaaa 480
aaaaaaa 487

<210> 707
<211> 3599
<212> DNA
<213> Homo Sapiens

<400> 707
aaggaggagc gggccgtgga ggcttcgccg cctaggtact gctataacca gaatttggta 60
taaaaaggat ttacttgttg gggccctctt gataaaaaga gatgtggggg gattctcgac 120
ctgctaacag aactggacct tttcgtggga gccaagaaga aaggtttgct cccgggtgga 180
acagggatta tcctcctcct ccccttaaga gtcatgctca agagagacac tctggcaact 240
ttcctggcag agattcactt ccctttgatt tccaggggca ttcggggcct ccttttgcaa 300
atgtagagga gcattctttc agctatggag ctagagacgg accgcatggt gactatcgag 360
gaggggaggg acctggacat gatttcaggg ggggagattt ttcgtcttct gatttccaga 420
gcagagattc atcacagttg gacttcaggg gtagggacat acattctggg gattttcggg 480
atagagaagg accacctatg gactataggg gtggagatgg tacttctatg gattatagag 540
gtagggaggc acctcatatg aactacagag acagggatgc tcacgctgtt gacttcagag 600
gtagggatgc tcctccatct gacttcaggg gccggggcac ttatgattta gattttagag 660
gccgggatgg atcccatgca gattttaggg gaagggattt atcagatttg gattttaggg 720
ccagagaaca gtcccgttct gattttagga atagagatgt atctgatttg gactttagag 780
acaaagacgg aacacaagta gactttagag gccgaggttc aggtactact gatctagact 840
ttagggacag ggatacgcca cattcagatt tcagaggtag acaccgatct aggactgatc 900
aggattttag gggcagagag atgggatctt gtatggaatt taaagatagg gagatgcccc 960
ctgtggatcc aaatattttg gattacattc agccctctac acaagataga gaacattctg 1020
gtatgaatgt gaacaggaga gaagaatcca cacacgacca tacgatagaa aggcctgctt 1080
ttggcattca gaagggagaa tttgagcatt cagaaacaag agaaggagaa acacaaggtg 1140
tagcctttga acatgagtct ccagcagact ttcagaacag ccaaagtcca gttcaagacc 1200
aagataagtc acagctttct ggacgtgaag agcagagttc agatgctggt ctgtttaaag 1260
aagaaggcgg tctggacttt cttgggcggc aagacaccga ttacagaagc atggagtacc 1320
gtgatgtgga tcataggctg ccaggaagcc agatgtttgg ctatggccag agcaagtctt 1380
ttccagaggg caaaactgcc cgagatgccc aacgggacct tcaggatcaa gattatagga 1440
ccggcccaag tgaggagaaa cccagcaggc ttattcgatt aagtggggta cctgaagatg 1500
ccacaaaaga agagattctt aatgcttttc ggactcctga tggcatgcct gtaaagaact 1560
tgcagttgaa ggagtataac acaggttacg actatggcta tgtctgcgtg gagttttcac 1620
tcttggaaga tgccatcgga tgcatggagg ccaaccaggg aactctaatg atccaggaca 1680
aagaagttac cctggagtat gtatcaagcc tggatttttg gtactgcaaa cgatgtaagg 1740
caaacattgg tgggcaccga tcttcctgtt cattctgcaa gaacccaaga gaagtgacag 1800
aggccaagca agaattaata acctaccctc agcctcagaa aacatccata ccagcaccat 1860
tggaaaaaca gcccaaccag cccctaagac cagctgataa ggaacctgaa cccaggaaga 1920
gggaagaagg ccaagagtca cgcttaggac atcaaaagag agaagcagaa aggtatctgc 1980
ctccttctcg aagggaaggg ccaactttcc gaagagaccg agagagggag tcatggtctg 2040
gagagacacg ccaggatgga gagagcaaaa ctatcatgct aaagcgtatc tatcgttcca 2100
caccacctga ggtgatagtg gaagtgctgg agccctatgt ccgccttact actgccaacg 2160
tccgtatcat caagaacaga acaggcccta tggggcatac ctatggcttt attgacctcg 2220
actcccatgt ggaagctctt cgtgtggtga agatcttaca gaaccttgat ccgccattta 2280
gcattgatgg gaagatggta gctgtaaacc tggccactgg aaaacgaaga aatgattctg 2340
gggaccattc tgaccacatg cattactatc agggtaaaaa atatttccga gataggaggg 2400
gaggtggcag aaattcagac tggtcttcag atacaaatcg acaaggacaa cagtcatcat 2460
ctgactgcta catatatgat tctgctagtg gctactatta tgaccccttg gcaggaactt 2520
attatgaccc caatacccag caagaagtct atgtgcccca ggatcctgga ttacctgagg 2580
aagaagagat caaggaaaaa aaacccacca gtcaaggaaa gtcaagtagc aagaaggaaa 2640

tgtctaaaag agatggcaag gagaaaaaag acagaggagt gacgaggttt caggaaaatg 2700
ccagtgaagg gaaggcccct gcagaagacg tctttaagaa gcccctgcct cctactgtga 2760
agaaggaaga gagtccccct ccacctaaag tggtaaaccc actgatcggc ctcttgggtg 2820
aatatggagg agacagtgac tatgaggagg aagaagagga ggaacagacc cctcccccac 2880
agccccgcac agcacagccc cagaagcgag aggagcaaac caagaaggag aatgaagaag 2940
acaaactcac tgactggaat aaactggctt gtctgctttg cagaaggcag tttcccaata 3000
aagaagttct gatcaaacac cagcagctgt cagacctgca caagcaaaac ctggaaatcc 3060
accggaagat aaaacagtct gagcaggagc tagcctatct ggaaaggaga gaacgagagg 3120
gaaagtttaa aggaagagga aatgatcgca gggaaaagct ccagtctttt gactctccag 3180
aaaggaaacg gattaagtac tccagggaaa ctgacagtga tcgtaaactt gttgataaag 3240
aagatatcga cactagcagc aaaggaggct gtgtccaaca ggctactggc tggaggaaag 3300
ggacaggcct gggatatggc catcctggat tggcttcatc agaggaggct gaaggccgga 3360
tgaggggccc cagtgttgga gcctcaggaa gaaccagcaa aagacagtcc aacgagactt 3420
atcgagatgc tgttcgaaga gtcatgtttg ctcgatataa agaactcgat taagaaagga 3480
gacaagttcc atgggataca acctccctct tgttttgttt gtctctcctt ttcttttgtt 3540
actgttcttg ctgctagaac ttttttaaat aaactttttt tcaatgtgat taaaaaaaa 3599

<210> 708
<211> 1123
<212> PRT
<213> Homo Sapiens

<400> 708

<210> 709
<211> 3807
<212> DNA
<213> Homo Sapiens

<400> 709
aaggaggagc gggccgtgga ggcttcgccg cctaggtact gctataacca gaatttggta 60
taaaaaggat ttacttgttg gggccctctt gataaaaaga gatgtggggg gattctcgac 120
ctgctaacag aactggacct tttcgagatg gcgtttcgcc gtgttggccg ggctggtctc 180
ggactcctga cctcaagtga tccacctacc tcggcctccc aaagtgctgg gactataggt 240
gtgagccacc gcacctgcca tttggattgg caatctgcaa gattttatta cttaaatgca 300
acagatgttc tcattcattg ttctgaagct tggagttcca atgaaaaatt tagtgggagc 360
caagaagaaa ggtttgctcc cgggtggaac agggattatc ctcctcctcc ccttaagagt 420
catgctcaag agagacactc tggcaacttt cctggcagag attcacttcc ctttgatttc 480
caggggcatt cggggcctcc ttttgcaaat gtagaggagc attctttcag ctatggagct 540
agagacggac cgcatggtga ctatcgagga ggggagggac ctggacatga tttcaggggg 600
ggagattttt cgtcttctga tttccagagc agagattcat cacagttgga cttcaggggt 660
agggacatac attctgggga ttttcgggat agagaaggac cacctatgga ctataggggt 720
ggagatggta cttctatgga ttatagaggt agggaggcac ctcatatgaa ctacagagac 780
agggatgctc acgctgttga cttcagaggt agggatgctc ctccatctga cttcaggggc 840
cggggcactt atgatttaga ttttagaggc cgggatggat cccatgcaga ttttagggga 900
agggatttat cagatttgga ttttagggcc agagaacagt cccgttctga ttttaggaat 960
agagatgtat ctgatttgga ctttagagac aaagacggaa cacaagtaga ctttagaggc 1020
cgaggttcag gtactactga tctagacttt agggacaggg atacgccaca ttcagatttc 1080
agaggtagac accgatctag gactgatcag gattttaggg gcagagagat gggatcttgt 1140
atggaattta aagataggga gatgccccct gtggatccaa atattttgga ttacattcag 1200
ccctctacac aagatagaga acattctggt atgaatgtga acaggagaga agaatccaca 1260
cacgaccata cgatagaaag gcctgctttt ggcattcaga agggagaatt tgagcattca 1320
gaaacaagag aaggagaaac acaaggtgta gcctttgaac atgagtctcc agcagacttt 1380
cagaacagcc aaagtccagt tcaagaccaa gataagtcac agctttctgg acgtgaagag 1440
cagagttcag atgctggtct gtttaaagaa gaaggcggtc tggactttct tgggcggcaa 1500
gacaccgatt acagaagcat ggagtaccgt gatgtggatc ataggctgcc aggaagccag 1560
atgtttggct atggccagag caagtctttt ccagagggca aaactgcccg agatgcccaa 1620
cgggaccttc aggatcaaga ttataggacc ggcccaagtg aggagaaacc cagcaggctt 1680
attcgattaa gtggggtacc tgaagatgcc acaaaagaag agattcttaa tgcttttcgg 1740
actcctgatg gcatgcctgt aaagaacttg cagttgaagg agtataacac aggttacgac 1800
tatggctatg tctgcgtgga gttttcactc ttggaagatg ccatcggatg catggaggcc 1860
aaccagggaa ctctaatgat ccaggacaaa gaagttaccc tggagtatgt atcaagcctg 1920
gatttttggt actgcaaacg atgtaaggca aacattggtg ggcaccgatc ttcctgttca 1980
ttctgcaaga acccaagaga agtgacagag gccaagcaag aattaataac ctaccctcag 2040
cctcagaaaa catccatacc agcaccattg gaaaaacagc ccaaccagcc cctaagacca 2100
gctgataagg aacctgaacc caggaagagg gaagaaggcc aagagtcacg cttaggacat 2160
caaaagagag aagcagaaag gtatctgcct ccttctcgaa gggaagggcc aactttccga 2220
agagaccgag agagggagtc atggtctgga gagacacgcc aggatggaga gagcaaaact 2280
atcatgctaa agcgtatcta tcgttccaca ccacctgagg tgatagtgga agtgctggag 2340
ccctatgtcc gccttactac tgccaacgtc cgtatcatca agaacagaac aggccctatg 2400
gggcatacct atggctttat tgacctcgac tcccatgtgg aagctcttcg tgtggtgaag 2460
atcttacaga accttgatcc gccatttagc attgatggga agatggtagc tgtaaacctg 2520
gccactggaa aacgaagaaa tgattctggg gaccattctg accacatgca ttactatcag 2580
ggtaaaaaat atttccgaga taggagggga ggtggcagaa attcagactg gtcttcagat 2640
acaaatcgac aaggacaaca gtcatcatct gactgctaca tatatgattc tgctagtggc 2700
tactattatg accccttggc aggaacttat tatgacccca atacccagca agaagtctat 2760
gtgccccagg atcctggatt acctgaggaa gaagagatca aggaaaaaaa acccaccagt 2820
caaggaaagt caagtagcaa gaaggaaatg tctaaaagag atggcaagga gaaaaaagac 2880
agaggagtga cgaggtttca ggaaaatgcc agtgaaggga aggcccctgc agaagacgtc 2940

tttaagaagc ccctgcctcc tactgtgaag aaggaagaga gtccccctcc acctaaagtg 3000
gtaaacccac tgatcggcct cttgggtgaa tatggaggag acagtgacta tgaggaggaa 3060
gaagaggagg aacagacccc tcccccacag ccccgcacag cacagcccca gaagcgagag 3120
gagcaaacca agaaggagaa tgaagaagac aaactcactg actggaataa actggcttgt 3180
ctgctttgca gaaggcagtt tcccaataaa gaagttctga tcaaacacca gcagctgtca 3240
gacctgcaca agcaaaacct ggaaatccac cggaagataa aacagtctga gcaggagcta 3300
gcctatctgg aaaggagaga acgagaggga aagtttaaag gaagaggaaa tgatcgcagg 3360
gaaaagctcc agtcttttga ctctccagaa aggaaacgga ttaagtactc cagggaaact 3420
gacagtgatc gtaaacttgt tgataaagaa gatatcgaca ctagcagcaa aggaggctgt 3480
gtccaacagg ctactggctg gaggaaaggg acaggcctgg gatatggcca tcctggattg 3540
gcttcatcag aggaggctga aggccggatg aggggcccca gtgttggagc ctcaggaaga 3600
accagcaaaa gacagtccaa cgagacttat cgagatgctg ttcgaagagt catgtttgct 3660
cgatataaag aactcgatta agaaaggaga caagttccat gggatacaac ctccctcttg 3720
ttttgtttgt ctctcctttt cttttgttac tgttcttgct gctagaactt ttttaaataa 3780
actttttttc aatgtgatta aaaaaaa 3807

<210> 710
<211> 1177
<212> PRT
<213> Homo Sapiens

<400> 710
Met Ala Phe Arg Arg Val Gly Arg Ala Gly Leu Gly Leu Leu Thr Ser
1               5                   10                  15
Ser Asp Pro Pro Thr Ser Ala Ser Gln Ser Ala Gly Thr Ile Gly Val
            20                  25                  30
Ser His Arg Thr Cys His Leu Asp Trp Gln Ser Ala Arg Phe Tyr Tyr
        35                  40                  45
Leu Asn Ala Thr Asp Val Leu Ile His Cys Ser Glu Ala Trp Ser Ser
    50                  55                  60
Asn Glu Lys Phe Ser Gly Ser Gln Glu Glu Arg Phe Ala Pro Gly Trp
65                  70                  75                  80
Asn Arg Asp Tyr Pro Pro Pro Pro Leu Lys Ser His Ala Gln Glu Arg
                85                  90                  95
His Ser Gly Asn Phe Pro Gly Arg Asp Ser Leu Pro Phe Asp Phe Gln
            100                 105                 110
Gly His Ser Gly Pro Pro Phe Ala Asn Val Glu Glu His Ser Phe Ser
        115                 120                 125
Tyr Gly Ala Arg Asp Gly Pro His Gly Asp Tyr Arg Gly Gly Glu Gly
    130                 135                 140
Pro Gly His Asp Phe Arg Gly Gly Asp Phe Ser Ser Ser Asp Phe Gln
145                 150                 155                 160
Ser Arg Asp Ser Ser Gln Leu Asp Phe Arg Gly Arg Asp Ile His Ser
                165                 170                 175
Gly Asp Phe Arg Asp Arg Glu Gly Pro Pro Met Asp Tyr Arg Gly Gly
            180                 185                 190
Asp Gly Thr Ser Met Asp Tyr Arg Gly Arg Glu Ala Pro His Met Asn
        195                 200                 205
Tyr Arg Asp Arg Asp Ala His Ala Val Asp Phe Arg Gly Arg Asp Ala
    210                 215                 220
Pro Pro Ser Asp Phe Arg Gly Arg Gly Thr Tyr Asp Leu Asp Phe Arg
225                 230                 235                 240
Gly Arg Asp Gly Ser His Ala Asp Phe Arg Gly Arg Asp Leu Ser Asp
                245                 250                 255
Leu Asp Phe Arg Ala Arg Glu Gln Ser Arg Ser Asp Phe Arg Asn Arg

            260                 265                 270
Asp Val Ser Asp Leu Asp Phe Arg Asp Lys Asp Gly Thr Gln Val Asp
        275                 280                 285
Phe Arg Gly Arg Gly Ser Gly Thr Thr Asp Leu Asp Phe Arg Asp Arg
    290                 295                 300
Asp Thr Pro His Ser Asp Phe Arg Gly Arg His Arg Ser Arg Thr Asp
305                 310                 315                 320
Gln Asp Phe Arg Gly Arg Glu Met Gly Ser Cys Met Glu Phe Lys Asp
                325                 330                 335
Arg Glu Met Pro Pro Val Asp Pro Asn Ile Leu Asp Tyr Ile Gln Pro
            340                 345                 350
Ser Thr Gln Asp Arg Glu His Ser Gly Met Asn Val Asn Arg Arg Glu
        355                 360                 365
Glu Ser Thr His Asp His Thr Ile Glu Arg Pro Ala Phe Gly Ile Gln
    370                 375                 380
Lys Gly Glu Phe Glu His Ser Glu Thr Arg Glu Gly Glu Thr Gln Gly
385                 390                 395                 400
Val Ala Phe Glu His Glu Ser Pro Ala Asp Phe Gln Asn Ser Gln Ser
                405                 410                 415
Pro Val Gln Asp Gln Asp Lys Ser Gln Leu Ser Gly Arg Glu Glu Gln
            420                 425                 430
Ser Ser Asp Ala Gly Leu Phe Lys Glu Glu Gly Gly Leu Asp Phe Leu
        435                 440                 445
Gly Arg Gln Asp Thr Asp Tyr Arg Ser Met Glu Tyr Arg Asp Val Asp
    450                 455                 460
His Arg Leu Pro Gly Ser Gln Met Phe Gly Tyr Gly Gln Ser Lys Ser
465                 470                 475                 480
Phe Pro Glu Gly Lys Thr Ala Arg Asp Ala Gln Arg Asp Leu Gln Asp
                485                 490                 495
Gln Asp Tyr Arg Thr Gly Pro Ser Glu Glu Lys Pro Ser Arg Leu Ile
            500                 505                 510
Arg Leu Ser Gly Val Pro Glu Asp Ala Thr Lys Glu Glu Ile Leu Asn
        515                 520                 525
Ala Phe Arg Thr Pro Asp Gly Met Pro Val Lys Asn Leu Gln Leu Lys
    530                 535                 540
Glu Tyr Asn Thr Gly Tyr Asp Tyr Gly Tyr Val Cys Val Glu Phe Ser
545                 550                 555                 560
Leu Leu Glu Asp Ala Ile Gly Cys Met Glu Ala Asn Gln Gly Thr Leu
                565                 570                 575
Met Ile Gln Asp Lys Glu Val Thr Leu Glu Tyr Val Ser Ser Leu Asp
            580                 585                 590
Phe Trp Tyr Cys Lys Arg Cys Lys Ala Asn Ile Gly Gly His Arg Ser
        595                 600                 605
Ser Cys Ser Phe Cys Lys Asn Pro Arg Glu Val Thr Glu Ala Lys Gln
    610                 615                 620
Glu Leu Ile Thr Tyr Pro Gln Pro Gln Lys Thr Ser Ile Pro Ala Pro
625                 630                 635                 640
Leu Glu Lys Gln Pro Asn Gln Pro Leu Arg Pro Ala Asp Lys Glu Pro
                645                 650                 655
Glu Pro Arg Lys Arg Glu Glu Gly Gln Glu Ser Arg Leu Gly His Gln
            660                 665                 670
Lys Arg Glu Ala Glu Arg Tyr Leu Pro Pro Ser Arg Arg Glu Gly Pro
        675                 680                 685
Thr Phe Arg Arg Asp Arg Glu Arg Glu Ser Trp Ser Gly Glu Thr Arg
    690                 695                 700

Gln Asp Gly Glu Ser Lys Thr Ile Met Leu Lys Arg Ile Tyr Arg Ser
705                 710                 715                 720
Thr Pro Pro Glu Val Ile Val Glu Val Leu Glu Pro Tyr Val Arg Leu
                725                 730                 735
Thr Thr Ala Asn Val Arg Ile Ile Lys Asn Arg Thr Gly Pro Met Gly
            740                 745                 750
His Thr Tyr Gly Phe Ile Asp Leu Asp Ser His Val Glu Ala Leu Arg
        755                 760                 765
Val Val Lys Ile Leu Gln Asn Leu Asp Pro Pro Phe Ser Ile Asp Gly
    770                 775                 780
Lys Met Val Ala Val Asn Leu Ala Thr Gly Lys Arg Arg Asn Asp Ser
785                 790                 795                 800
Gly Asp His Ser Asp His Met His Tyr Tyr Gln Gly Lys Lys Tyr Phe
                805                 810                 815
Arg Asp Arg Arg Gly Gly Gly Arg Asn Ser Asp Trp Ser Ser Asp Thr
            820                 825                 830
Asn Arg Gln Gly Gln Gln Ser Ser Ser Asp Cys Tyr Ile Tyr Asp Ser
        835                 840                 845
Ala Ser Gly Tyr Tyr Tyr Asp Pro Leu Ala Gly Thr Tyr Tyr Asp Pro
    850                 855                 860
Asn Thr Gln Gln Glu Val Tyr Val Pro Gln Asp Pro Gly Leu Pro Glu
865                 870                 875                 880
Glu Glu Glu Ile Lys Glu Lys Lys Pro Thr Ser Gln Gly Lys Ser Ser
                885                 890                 895
Ser Lys Lys Glu Met Ser Lys Arg Asp Gly Lys Glu Lys Lys Asp Arg
            900                 905                 910
Gly Val Thr Arg Phe Gln Glu Asn Ala Ser Glu Gly Lys Ala Pro Ala
        915                 920                 925
Glu Asp Val Phe Lys Lys Pro Leu Pro Pro Thr Val Lys Lys Glu Glu
    930                 935                 940
Ser Pro Pro Pro Pro Lys Val Val Asn Pro Leu Ile Gly Leu Leu Gly
945                 950                 955                 960
Glu Tyr Gly Gly Asp Ser Asp Tyr Glu Glu Glu Glu Glu Glu Glu Gln
                965                 970                 975
Thr Pro Pro Pro Gln Pro Arg Thr Ala Gln Pro Gln Lys Arg Glu Glu
            980                 985                 990
Gln Thr Lys Lys Glu Asn Glu Glu Asp Lys Leu Thr Asp Trp Asn Lys
        995                 1000                1005
Leu Ala Cys Leu Leu Cys Arg Arg Gln Phe Pro Asn Lys Glu Val Leu
    1010                1015                1020
Ile Lys His Gln Gln Leu Ser Asp Leu His Lys Gln Asn Leu Glu Ile
1025                1030                1035                104
His Arg Lys Ile Lys Gln Ser Glu Gln Glu Leu Ala Tyr Leu Glu Arg
                1045                1050                1055
Arg Glu Arg Glu Gly Lys Phe Lys Gly Arg Gly Asn Asp Arg Arg Glu
            1060                1065                1070
Lys Leu Gln Ser Phe Asp Ser Pro Glu Arg Lys Arg Ile Lys Tyr Ser
        1075                1080                1085
Arg Glu Thr Asp Ser Asp Arg Lys Leu Val Asp Lys Glu Asp Ile Asp
    1090                1095                1100
Thr Ser Ser Lys Gly Gly Cys Val Gln Gln Ala Thr Gly Trp Arg Lys
1105                1110                1115                112
Gly Thr Gly Leu Gly Tyr Gly His Pro Gly Leu Ala Ser Ser Glu Glu
                1125                1130                1135
Ala Glu Gly Arg Met Arg Gly Pro Ser Val Gly Ala Ser Gly Arg Thr

1140                 1145                1150
Ser Lys Arg Gln Ser Asn Glu Thr Tyr Arg Asp Ala Val Arg Arg Val
        1155                1160                1165
Met Phe Ala Arg Tyr Lys Glu Leu Asp
    1170                1175

<210> 711
<211> 4060
<212> DNA
<213> Homo Sapiens

<400> 711
ctgaaggcag cggcgcggcg cctttgtggt agcagtggcc ccgcgcggag gaagttccgg 60
tgtccgcggc gctaggtcgg tggcggaggc tgaggagaag gaggagcggg ccgtggaggc 120
ttcgccgcct aggtactgct ataaccagaa tttggtataa aaaggattta cttgttgggg 180
ccctcttgat aaaaagagat gtggggggat tctcgacctg ctaacagaac tggacctttt 240
cggactgggt gaaagctttt tctgcagcag tcatgttaaa aaccttgtgt tgactttcct 300
cgtgttctga aactaacaga actggacctt ttcggactgg gtgaaagctt tttctgcagc 360
agtcatgttg aaaaccttgt gttgactttc ttcgtgttct gaaatgggag cataaaagtt 420
tactccgcca nttcgtctta aaatagcaaa actttgctgt tttctgcaga tctaggacct 480
tgttacagaa ctctgccaaa aaaaaaatgt ttacagaaga atgtgctgtg attagagaag 540
aatatgctgg tgtgtagatt tcaaactctc tggacaatat gaataacact gtctttgttt 600
ctacagtggg agccaagaag aaaggtttgc tcccgggtgg aacagggatt atcctcctcc 660
tccccttaag agtcatgctc aagagagaca ctctggcaac tttcctggca gagattcact 720
tccctttgat ttccaggggc attcggggcc tccttttgca aatgtagagg agcattcttt 780
cagctatgga gctagagacg gaccgcatgg tgactatcga ggaggggagg gacctggaca 840
tgatttcagg gggggagatt tttcgtcttc tgatttccag agcagagatt catcacagtt 900
ggacttcagg ggtagggaca tacattctgg ggattttcgg gatagagaag gaccacctat 960
ggactatagg ggtggagatg gtacttctat ggattataga ggtagggagg cacctcatat 1020
gaactacaga gacagggatg ctcacgctgt tgacttcaga ggtagggatg ctcctccatc 1080
tgacttcagg ggccggggca cttatgattt agattttaga ggccgggatg gatcccatgc 1140
agattttagg ggaagggatt tatcagattt ggattttagg gccagagaac agtcccgttc 1200
tgattttagg aatagagatg tatctgattt ggactttaga gacaaagacg gaacacaagt 1260
agactttaga ggccgaggtt caggtactac tgatctagac tttagggaca gggatacgcc 1320
acattcagat ttcagaggta gacaccgatc taggactgat caggatttta ggggcagaga 1380
gatgggatct tgtatggaat ttaaagatag ggagatgccc cctgtggatc caaatatttt 1440
ggattacatt cagccctcta cacaagatag agaacattct ggtatgaatg tgaacaggag 1500
agaagaatcc acacacgacc atacgataga aaggcctgct tttggcattc agaagggaga 1560
atttgagcat tcagaaacaa gagaaggaga aacacaaggt gtagcctttg aacatgagtc 1620
tccagcagac tttcagaaca gccaaagtcc agttcaagac caagataagt cacagctttc 1680
tggacgtgaa gagcagagtt cagatgctgg tctgtttaaa gaagaaggcg gtctggactt 1740
tcttgggcgg caagacaccg attacagaag catggagtac cgtgatgtgg atcatagget 1800
gccaggaagc cagatgtttg gctatggcca gagcaagtct tttccagagg gcaaaactgc 1860
ccgagatgcc caacgggacc ttcaggatca agattatagg accggcccaa gtgaggagaa 1920
acccagcagg cttattcgat taagtggggt acctgaagat gccacaaaag aagagattct 1980
taatgctttt cggactcctg atggcatgcc tgtaaagaac ttgcagttga aggagtataa 2040
cacaggttac gactatggct atgtctgcgt ggagttttca ctcttggaag atgccatcgg 2100
atgcatggag gccaaccagg gaactctaat gatccaggac aaagaagtta ccctggagta 2160
tgtatcaagc ctggattttt ggtactgcaa acgatgtaag gcaaacattg gtgggcaccg 2220
atcttcctgt tcattctgca agaacccaag agaagtgaca gaggccaagc aagaattaat 2280
aacctaccct cagcctcaga aaacatccat accagcacca ttggaaaaac agcccaacca 2340
gcccctaaga ccagctgata aggaacctga acccaggaag agggaagaag gccaagagtc 2400
acgcttagga catcaaaaga gagaagcaga aaggtatctg cctccttctc gaagggaagg 2460
gccaactttc cgaagagacc gagagaggga gtcatggtct ggagagacac gccaggatgg 2520
agagagcaaa actatcatgc taaagcgtat ctatcgttcc acaccacctg aggtgatagt 2580

ggaagtgctg gagccctatg tccgccttac tactgccaac gtccgtatca tcaagaacag 2640
aacaggccct atggggcata cctatggctt tattgacctc gactcccatg tggaagctct 2700
tcgtgtggtg aagatcttac agaaccttga tccgccattt agcattgatg ggaagatggt 2760
agctgtaaac ctggccactg gaaaacgaag aaatgattct ggggaccatt ctgaccacat 2820
gcattactat cagggtaaaa aatatttccg agataggagg ggaggtggca gaaattcaga 2880
ctggtcttca gatacaaatc gacaaggaca acagtcatca tctgactgct acatatatga 2940
ttctgctagt ggctactatt atgacccctt ggcaggaact tattatgacc ccaataccca 3000
gcaagaagtc tatgtgcccc aggatcctgg attacctgag gaagaagaga tcaaggaaaa 3060
aaaacccacc agtcaaggaa agtcaagtag caagaaggaa atgtctaaaa gagatggcaa 3120
ggagaaaaaa gacagaggag tgacgaggtt tcaggaaaat gccagtgaag ggaaggcccc 3180
tgcagaagac gtctttaaga agcccctgcc tcctactgtg aagaaggaag agagtccccc 3240
tccacctaaa gtggtaaacc cactgatcgg cctcttgggt gaatatggag gagacagtga 3300
ctatgaggag gaagaagagg aggaacagac ccctccccca cagccccgca cagcacagcc 3360
ccagaagcga gaggagcaaa ccaagaagga gaatgaagaa gacaaactca ctgactggaa 3420
taaactggct tgtctgcttt gcagaaggca gtttcccaat aaagaagttc tgatcaaaca 3480
ccagcagctg tcagacctgc acaagcaaaa cctggaaatc caccggaaga taaaacagtc 3540
tgagcaggag ctagcctatc tggaaaggag agaacgagag ggaaagttta aggaagaggg 3600
aaatgatcgc agggaaaagc tccagtcttt tgactctcca gaaaggaaac ggattaagta 3660
ctccagggaa actgacagtg atcgtaaact tgttgataaa gaagatatcg acactagcag 3720
caaaggaggc tgtgtccaac aggctactgg ctggaggaaa gggacaggcc tgggatatgg 3780
ccatcctgga ttggcttcat cagaggaggc tgaaggccgg atgagggccc ccagtgttgg 3840
agcctcagga agaaccagca aaagacagtc caacgagact tatcgagatg ctgttcgaag 3900
agtcatgttt gctcgatata aagaactcga ttaagaaagg agacaagttc catgggatac 3960
aacctccctc ttgttttgtt tgtctctcct tttcttttgt tactgttctt gctgctagaa 4020
cttttttaaa taaacttttt ttcaatgtga ttaaaaaaaa 4060

<210> 712
<211> 3736
<212> DNA
<213> Homo Sapiens

<400> 712
aaggaggagc gggccgtgga ggcttcgccg cctaggtact gctataacca gaatttggta 60
gaaaaaggat ttacttgttg gggccctctt gataaaaaga gatgtggggg gattctcgac 120
ctgctaacag aactggacct tttcgatcta ggaccttgtt acagaactct gccaaaaaaa 180
aaatgtttac agaagaatgt gctgtgatta gagaagaata tgctggtgtg tagatttcaa 240
actctntgga caatatgaat aacactgtct ttgtttctac agtgggagcc aagaagaaag 300
gtttgctccc gggtggaaca gggattatcc tcctcctccc cttaagagtc atgctcaaga 360
gagacactct ggcaactttc ctggcagaga ttcacttccc tttgatttcc aggggcattc 420
ggggcctcct tttgcaaatg tagaggagca ttctttcagc tatggagcta gagacggacc 480
gcatggtgac tatcgaggag gggagggacc tggacatgat ttcagggggg gagatttttc 540
gtcttctgat ttccagagca gagattcatc acagttggac ttcaggggta gggacataca 600
ttctggggat tttcgggata gagaaggacc acctatggac tataggggtg gagatggtac 660
ttctatggat tatagaggta gggaggcacc tcatatgaac tacagagaca gggatgctca 720
cgctgttgac ttcagaggta gggatgctcc tccatctgac ttcaggggcc ggggcactta 780
tgatttagat tttagaggcc gggatggatc ccatgcagat tttaggggaa gggatttatc 840
agatttggat tttagggcca gagaacagtc ccgttctgat tttaggaata gagatgtatc 900
tgatttggac tttagagaca aagacggaac acaagtagac tttagaggcc gaggttcagg 960
tactactgat ctagacttta gggacaggga tacgccacat tcagatttca gaggtagaca 1020
ccgatctagg actgatcagg attttagggg cagagagatg ggatcttgta tggaatttaa 1080
agatagggag atgccccctg tggatccaaa tattttggat tacattcagc cctctacaca 1140
agatagagaa cattctggta tgaatgtgaa caggagagaa gaatccacac acgaccatac 1200
gatagaaagg cctgcttttg gcattcagaa gggagaattt gagcattcag aaacaagaga 1260
aggagaaaca caaggtgtag cctttgaaca tgagtctcca gcagactttc agaacagcca 1320
aagtccagtt caagaccaag ataagtcaca gctttctgga cgtgaagagc agagttcaga 1380

tgctggtctg tttaaagaag aaggcggtct ggactttctt gggcggcaag acaccgatta 1440
cagaagcatg gagtaccgtg atgtggatca taggctgcca ggaagccaga tgtttggcta 1500
tggccagagc aagtcttttc cagagggcaa aactgcccga gatgcccaac gggaccttca 1560
ggatcaagat tataggaccg gcccaagtga ggagaaaccc agcaggctta ttcgattaag 1620
tggggtacct gaagatgcca caaaagaaga gattcttaat gcttttcgga ctcctgatgg 1680
catgcctgta aagaacttgc agttgaagga gtataacaca ggttacgact atggctatgt 1740
ctgcgtggag ttttcactct tggaagatgc catcggatgc atggaggcca accagggaac 1800
tctaatgatc caggacaaag aagttaccct ggagtatgta tcaagcctgg atttttggta 1860
ctgcaaacga tgtaaggcaa acattggtgg gcaccgatct tcctgttcat tctgcaagaa 1920
cccaagagaa gtgacagagg ccaagcaaga attaataacc taccctcagc ctcagaaaac 1980
atccatacca gcaccattgg aaaaacagcc caaccagccc ctaagaccag ctgataagga 2040
acctgaaccc aggaagaggg aagaaggcca agagtcacgc ttaggacatc aaaagagaga 2100
agcagaaagg tatctgcctc cttctcgaag ggaagggcca actttccgaa gagaccgaga 2160
gagggagtca tggtctggag agacacgcca ggatggagag agcaaaacta tcatgctaaa 2220
gcgtatctat cgttccacac cacctgaggt gatagtggaa gtgctggagc cctatgtccg 2280
ccttactact gccaacgtcc gtatcatcaa gaacagaaca ggccctatgg ggcataccta 2340
tggctttatt gacctcgact cccatgtgga agctcttcgt gtggtgaaga tcttacagaa 2400
ccttgatccg ccatttagca ttgatgggaa gatggtagct gtaaacctgg ccactggaaa 2460
acgaagaaat gattctgggg accattctga ccacatgcat tactatcagg gtaaaaaata 2520
tttccgagat aggaggggag gtggcagaaa ttcagactgg tcttcagata caaatcgaca 2580
aggacaacag tcatcatctg actgctacat atatgattct gctagtggct actattatga 2640
ccccttggca ggaacttatt atgaccccaa tacccagcaa gaagtctatg tgccccagga 2700
tcctggatta cctgaggaag aagagatcaa ggaaaaaaaa cccaccagtc aaggaaagtc 2760
aagtagcaag aaggaaatgt ctaaaagaga tggcaaggag aaaaaagaca gaggagtgac 2820
gaggtttcag gaaaatgcca gtgaaggaaa ggcccctgca gaagacgtct ttaagaagcc 2880
cctgcctcct actgtgaaga aggaagagag tccccctcca cctaaagtgg taaacccact 2940
gatcggcctc ttgggtgaat atggaggaga cagtgactat gaggaggaag aagaggagga 3000
acagacccct cccccacagc cccgcacagc acagccccag aagcgagagg agcaaaccaa 3060
gaaggagaat gaagaagaca aactcactga ctggaataaa ctggcttgtc tgctttgcag 3120
aaggcagttt cccaataaag aagttctgat caaacaccag cagctgtcag acctgcacaa 3180
gcaaaacctg gaaatccacc ggaagataaa acagtctgag caggagctag cctatctgga 3240
aaggagagaa cgagagggaa agtttaaagg aagaggaaat gatcgcaggg aaaagctcca 3300
gtcttttgac tctccagaaa ggaaacggat taagtactcc agggaaactg acagtgatcg 3360
taaacttgtt gataaagaag atatcgacac tagcagcaaa ggaggctgtg tccaacaggc 3420
tactggctgg aggaaaggga caggcctggg atatggccat cctggattgg cttcatcaga 3480
ggaggctgaa ggccggatga ggggccccag tgttggagcc tcaggaagaa ccagcaaaag 3540
acagtccaac gagacttatc gagatgctgt tcgaagagtc atgtttgctc gatataaaga 3600
actcgattaa gaaaggagac aagttccatg ggatacaacc tccctcttgt tttgtttgtc 3660
tctccttttc ttttgttact gttcttgctg ctagaacttt tttaaataaa ctttttttca 3720
atgtgattaa aaaaaa 3736

<210> 713
<211> 10
<212> PRT
<213> Homo Sapiens

<400> 713
Asn Val Glu Glu Xaa His Ser Phe Ser Tyr
1               5                   10

<210> 714
<211> 10
<212> PRT
<213> Homo Sapiens

<400> 714
Pro Val Asp Pro Xaa Asn Ile Leu Asp Tyr
1               5                   10

<210> 715
<211> 10
<212> PRT
<213> Homo Sapiens

<400> 715
Asp Thr Asp Tyr Xaa Arg Ser Met Glu Tyr
1               5                   10

<210> 716
<211> 10
<212> PRT
<213> Homo Sapiens

<400> 716
Ser Leu Leu Glu Xaa Asp Ala Ile Gly Cys
1               5                   10

<210> 717
<211> 10
<212> PRT
<213> Homo Sapiens

<400> 717
Thr Leu Met Ile Xaa Gln Asp Lys Glu Val
1               5                   10

<210> 718
<211> 10
<212> PRT
<213> Homo Sapiens

<400> 718
Tyr Val Ser Ser Leu Asp Phe Trp Tyr Cys
1               5                   10

<210> 719
<211> 10
<212> PRT
<213> Homo Sapiens

<400> 719
Val Ile Val Glu Val Leu Glu Pro Tyr Val
1               5                   10

<210> 720
<211> 10
<212> PRT
<213> Homo Sapiens

<400> 720

Lys Leu Thr Asp Xaa Trp Asn Lys Leu Ala
1               5                   10

<210> 721
<211> 10
<212> PRT
<213> Homo Sapiens

<400> 721
Gln Leu Ser Asp Leu His Lys Gln Asn Leu
1               5                   10

<210> 722
<211> 10
<212> PRT
<213> Homo Sapiens

<400> 722
Lys Gln Ser Glu Gln Glu Leu Ala Tyr Leu
1               5                   10

<210> 723
<211> 10
<212> PRT
<213> Homo Sapiens

<400> 723
Lys Leu Val Asp Lys Glu Asp Ile Asp Thr
1               5                   10

<210> 724
<211> 10
<212> PRT
<213> Homo Sapiens

<400> 724
Val Met Phe Ala Xaa Arg Tyr Lys Glu Leu
1               5                   10

<210> 725
<211> 10
<212> PRT
<213> Homo Sapiens

<400> 725
Gln Met Phe Gly Xaa Tyr Gly Gln Ser Lys
1               5                   10

<210> 726
<211> 10
<212> PRT
<213> Homo Sapiens

<400> 726
Gly Met Pro Val Lys Asn Leu Gln Leu Lys

1                   5                   10

<210> 727
<211> 10
<212> PRT
<213> Homo Sapiens

<400> 727
Gly Leu Pro Glu Xaa Glu Glu Glu Ile Lys
1               5                   10

<210> 728
<211> 10
<212> PRT
<213> Homo Sapiens

<400> 728
Leu Leu Cys Arg Arg Gln Phe Pro Asn Lys
1               5                   10

<210> 729
<211> 10
<212> PRT
<213> Homo Sapiens

<400> 729
Glu Tyr Arg Asp Xaa Val Asp His Arg Leu
1               5                   10

<210> 730
<211> 10
<212> PRT
<213> Homo Sapiens

<400> 730
Gly Tyr Val Cys Xaa Val Glu Phe Ser Leu
1               5                   10

<210> 731
<211> 10
<212> PRT
<213> Homo Sapiens

<400> 731
Asp Tyr Gly Tyr Xaa Val Cys Val Glu Phe
1               5                   10

<210> 732
<211> 10
<212> PRT
<213> Homo Sapiens

<400> 732
Trp Tyr Cys Lys Arg Cys Lys Ala Asn Ile
1               5                   10

<210> 733
<211> 10
<212> PRT
<213> Homo Sapiens

<400> 733
Thr Tyr Pro Gln Pro Gln Lys Thr Ser Ile
1               5                   10

<210> 734
<211> 10
<212> PRT
<213> Homo Sapiens

<400> 734
Ile Tyr Arg Ser Thr Pro Pro Glu Val Ile
1               5                   10

<210> 735
<211> 10
<212> PRT
<213> Homo Sapiens

<400> 735
His Tyr Tyr Gln Xaa Gly Lys Lys Tyr Phe
1               5                   10

<210> 736
<211> 10
<212> PRT
<213> Homo Sapiens

<400> 736
Val Tyr Val Pro Xaa Gln Asp Pro Gly Leu
1               5                   10

<210> 737
<211> 10
<212> PRT
<213> Homo Sapiens

<400> 737
Trp Asn Arg Asp Tyr Pro Pro Pro Pro Leu
1               5                   10

<210> 738
<211> 10
<212> PRT
<213> Homo Sapiens

<400> 738
Met Pro Pro Val Xaa Asp Pro Asn Ile Leu
1               5                   10

<210> 739

<211> 10
<212> PRT
<213> Homo Sapiens

<400> 739
Thr Ala Arg Asp Xaa Ala Gln Arg Asp Leu
1               5                   10

<210> 740
<211> 10
<212> PRT
<213> Homo Sapiens

<400> 740
Gly Pro Ser Glu Glu Lys Pro Ser Arg Leu
1               5                   10

<210> 741
<211> 10
<212> PRT
<213> Homo Sapiens

<400> 741
Thr Pro Pro Glu Val Ile Val Glu Val Leu
1               5                   10

<210> 742
<211> 10
<212> PRT
<213> Homo Sapiens

<400> 742
Arg Val Met Phe Ala Arg Tyr Lys Glu Leu
1               5                   10

<210> 743
<211> 10
<212> PRT
<213> Homo Sapiens

<400> 743
Arg Glu Met Gly Xaa Ser Cys Met Glu Phe
1               5                   10

<210> 744
<211> 10
<212> PRT
<213> Homo Sapiens

<400> 744
Glu Glu Gln Ser Ser Asp Ala Gly Leu Phe
1               5                   10

<210> 745
<211> 10

<212> PRT
<213> Homo Sapiens

<400> 745
Lys Glu Tyr Asn Xaa Thr Gly Tyr Asp Tyr
1               5                   10

<210> 746
<211> 10
<212> PRT
<213> Homo Sapiens

<400> 746
Thr Glu Ala Lys Gln Glu Leu Ile Thr Tyr
1               5                   10

<210> 747
<211> 10
<212> PRT
<213> Homo Sapiens

<400> 747
Val Glu Ala Leu Arg Val Val Lys Ile Leu
1               5                   10

<210> 748
<211> 10
<212> PRT
<213> Homo Sapiens

<400> 748
Gly Glu Tyr Gly Xaa Gly Asp Ser Asp Tyr
1               5                   10

<210> 749
<211> 10
<212> PRT
<213> Homo Sapiens

<400> 749
Leu Glu Arg Arg Glu Arg Glu Gly Lys Phe
1               5                   10

<210> 750
<211> 10
<212> PRT
<213> Homo Sapiens

<400> 750
Arg Gln Asp Gly Glu Ser Lys Thr Ile Met
1               5                   10

<210> 751
<211> 10
<212> PRT

<213> Homo Sapiens

<400> 751
Thr Pro Pro Glu Val Ile Val Glu Val Leu
1               5                   10

<210> 752
<211> 10
<212> PRT
<213> Homo Sapiens

<400> 752
Tyr Gly Phe Ile Asp Leu Asp Ser His Val
1               5                   10

<210> 753
<211> 10
<212> PRT
<213> Homo Sapiens

<400> 753
Arg Gln Phe Pro Xaa Asn Lys Glu Val Leu
1               5                   10

<210> 754
<211> 10
<212> PRT
<213> Homo Sapiens

<400> 754
Asn Val Glu Glu Xaa His Ser Phe Ser Tyr
1               5                   10

<210> 755
<211> 10
<212> PRT
<213> Homo Sapiens

<400> 755
Pro Val Asp Pro Xaa Asn Ile Leu Asp Tyr
1               5                   10

<210> 756
<211> 10
<212> PRT
<213> Homo Sapiens

<400> 756
Asp Thr Asp Tyr Xaa Arg Ser Met Glu Tyr
1               5                   10

<210> 757
<211> 10
<212> PRT
<213> Homo Sapiens

<400> 757
Trp Gln Ser Ala Xaa Arg Phe Tyr Tyr Leu
1               5                   10

<210> 758
<211> 10
<212> PRT
<213> Homo Sapiens

<400> 758
Ser Leu Leu Glu Xaa Asp Ala Ile Gly Cys
1               5                   10

<210> 759
<211> 10
<212> PRT
<213> Homo Sapiens

<400> 759
Thr Leu Met Ile Xaa Gln Asp Lys Glu Val
1               5                   10

<210> 760
<211> 10
<212> PRT
<213> Homo Sapiens

<400> 760
Tyr Val Ser Ser Leu Asp Phe Trp Tyr Cys
1               5                   10

<210> 761
<211> 10
<212> PRT
<213> Homo Sapiens

<400> 761
Val Ile Val Glu Val Leu Glu Pro Tyr Val
1               5                   10

<210> 762
<211> 10
<212> PRT
<213> Homo Sapiens

<400> 762
Lys Leu Thr Asp Xaa Trp Asn Lys Leu Ala
1               5                   10

<210> 763
<211> 10
<212> PRT
<213> Homo Sapiens

<400> 763

Gln Leu Ser Asp Leu His Lys Gln Asn Leu
1               5                   10

<210> 764
<211> 10
<212> PRT
<213> Homo Sapiens

<400> 764
Lys Gln Ser Glu Gln Glu Leu Ala Tyr Leu
1               5                   10

<210> 765
<211> 10
<212> PRT
<213> Homo Sapiens

<400> 765
Lys Leu Val Asp Lys Glu Asp Ile Asp Thr
1               5                   10

<210> 766
<211> 10
<212> PRT
<213> Homo Sapiens

<400> 766
Val Met Phe Ala Xaa Arg Tyr Lys Glu Leu
1               5                   10

<210> 767
<211> 10
<212> PRT
<213> Homo Sapiens

<400> 767
Gln Met Phe Gly Xaa Tyr Gly Gln Ser Lys
1               5                   10

<210> 768
<211> 10
<212> PRT
<213> Homo Sapiens

<400> 768
Gly Met Pro Val Lys Asn Leu Gln Leu Lys
1               5                   10

<210> 769
<211> 10
<212> PRT
<213> Homo Sapiens

<400> 769
Gly Leu Pro Glu Xaa Glu Glu Glu Ile Lys

1                   5                   10

<210> 770
<211> 10
<212> PRT
<213> Homo Sapiens

<400> 770
Leu Leu Cys Arg Arg Gln Phe Pro Asn Lys
1               5                   10

<210> 771
<211> 10
<212> PRT
<213> Homo Sapiens

<400> 771
Tyr Tyr Leu Asn Xaa Ala Thr Asp Val Leu
1               5                   10

<210> 772
<211> 10
<212> PRT
<213> Homo Sapiens

<400> 772
Phe Tyr Tyr Leu Asn Ala Thr Asp Val Leu
1               5                   10

<210> 773
<211> 10
<212> PRT
<213> Homo Sapiens

<400> 773
Glu Tyr Arg Asp Xaa Val Asp His Arg Leu
1               5                   10

<210> 774
<211> 10
<212> PRT
<213> Homo Sapiens

<400> 774
Gly Tyr Val Cys Xaa Val Glu Phe Ser Leu
1               5                   10

<210> 775
<211> 10
<212> PRT
<213> Homo Sapiens

<400> 775
Asp Tyr Gly Tyr Xaa Val Cys Val Glu Phe
1               5                   10

<210> 776
<211> 10
<212> PRT
<213> Homo Sapiens

<400> 776
Trp Tyr Cys Lys Arg Cys Lys Ala Asn Ile
1               5                   10

<210> 777
<211> 10
<212> PRT
<213> Homo Sapiens

<400> 777
Thr Tyr Pro Gln Pro Gln Lys Thr Ser Ile
1               5                   10

<210> 778
<211> 10
<212> PRT
<213> Homo Sapiens

<400> 778
Ile Tyr Arg Ser Thr Pro Pro Glu Val Ile
1               5                   10

<210> 779
<211> 10
<212> PRT
<213> Homo Sapiens

<400> 779
His Tyr Tyr Gln Xaa Gly Lys Lys Tyr Phe
1               5                   10

<210> 780
<211> 10
<212> PRT
<213> Homo Sapiens

<400> 780
Val Tyr Val Pro Xaa Gln Asp Pro Gly Leu
1               5                   10

<210> 781
<211> 10
<212> PRT
<213> Homo Sapiens

<400> 781
Trp Asn Arg Asp Tyr Pro Pro Pro Pro Leu
1               5                   10

<210> 782

<211> 10
<212> PRT
<213> Homo Sapiens

<400> 782
Met Pro Pro Val Xaa Asp Pro Asn Ile Leu
1               5                   10

<210> 783
<211> 10
<212> PRT
<213> Homo Sapiens

<400> 783
Thr Ala Arg Asp Xaa Ala Gln Arg Asp Leu
1               5                   10

<210> 784
<211> 10
<212> PRT
<213> Homo Sapiens

<400> 784
Gly Pro Ser Glu Glu Lys Pro Ser Arg Leu
1               5                   10

<210> 785
<211> 10
<212> PRT
<213> Homo Sapiens

<400> 785
Thr Pro Pro Glu Val Ile Val Glu Val Leu
1               5                   10

<210> 786
<211> 10
<212> PRT
<213> Homo Sapiens

<400> 786
Arg Val Met Phe Ala Arg Tyr Lys Glu Leu
1               5                   10

<210> 787
<211> 10
<212> PRT
<213> Homo Sapiens

<400> 787
Ser Glu Ala Trp Ser Ser Asn Glu Lys Phe
1               5                   10

<210> 788
<211> 10

<212> PRT
<213> Homo Sapiens

<400> 788
Arg Glu Met Gly Xaa Ser Cys Met Glu Phe
1               5                   10

<210> 789
<211> 10
<212> PRT
<213> Homo Sapiens

<400> 789
Glu Glu Gln Ser Ser Asp Ala Gly Leu Phe
1               5                   10

<210> 790
<211> 10
<212> PRT
<213> Homo Sapiens

<400> 790
Lys Glu Tyr Asn Xaa Thr Gly Tyr Asp Tyr
1               5                   10

<210> 791
<211> 10
<212> PRT
<213> Homo Sapiens

<400> 791
Thr Glu Ala Lys Gln Glu Leu Ile Thr Tyr
1               5                   10

<210> 792
<211> 10
<212> PRT
<213> Homo Sapiens

<400> 792
Val Glu Ala Leu Arg Val Val Lys Ile Leu
1               5                   10

<210> 793
<211> 10
<212> PRT
<213> Homo Sapiens

<400> 793
Gly Glu Tyr Gly Xaa Gly Asp Ser Asp Tyr
1               5                   10

<210> 794
<211> 10
<212> PRT

<213> Homo Sapiens

<400> 794
Leu Glu Arg Arg Glu Arg Glu Gly Lys Phe
1               5                   10

<210> 795
<211> 10
<212> PRT
<213> Homo Sapiens

<400> 795
Arg Gln Asp Gly Glu Ser Lys Thr Ile Met
1               5                   10

<210> 796
<211> 10
<212> PRT
<213> Homo Sapiens

<400> 796
Thr Pro Pro Glu Val Ile Val Glu Val Leu
1               5                   10

<210> 797
<211> 10
<212> PRT
<213> Homo Sapiens

<400> 797
Tyr Gly Phe Ile Asp Leu Asp Ser His Val
1               5                   10

<210> 798
<211> 10
<212> PRT
<213> Homo Sapiens

<400> 798
Arg Gln Phe Pro Xaa Asn Lys Glu Val Leu
1               5                   10

<210> 799
<211> 1464
<212> DNA
<213> Homo Sapiens

<400> 799
agtaccgggt acgcaggggt gcctcaacca cactccgtcc acggactctc cgttatttta      60
ggaggtccct ggccaaagat ttatttctct tgacaaccaa gggcctccgt ctggatttcc     120
aaggaagaat ttcctctgaa gcaccggaac ttgctactac cagcaccatg ccctaccaat     180
atccagcact gaccccggag cagaagaagg agctgtctga catcgctcac cgcatcgtgg     240
cacctggcaa gggcatcctg gctgcagatg agtccactgg gagcattgcc aagcggctgc     300
agtccattgg caccgagaac accgaggaga accggcgctt ctaccgccag ctgctgctga     360
cagctgacga ccgcgtgaac ccctgcattg ggggtgtcat cctcttccat gagacactct     420

accagaaggc ggatgatggg cgtcccttcc cccaagttat caaatccaag ggcggtgttg 480
tgggcatcaa ggtagacaag ggcgtggtcc ccctggcagg gacaaatggc gagactacca 540
cccaagggtt ggatgggctg tctgagcgct gtgcccagta caagaaggac ggagctgact 600
tcgccaagtg gcgttgtgtg ctgaagattg gggaacacac cccctcagcc ctcgccatca 660
tggaaaatgc caatgttctg gcccgttatg ccagtatctg ccagcagaat ggcattgtgc 720
ccatcgtgga gcctgagatc ctccctgatg gggaccatga cttgaagcgc tgccagtatg 780
tgaccgagaa ggtgctggct gctgtctaca aggctctgag tgaccaccac atctacctgg 840
aaggcacctt gctgaagccc aacatggtca ccccaggcca tgcttgcact cagaagtttt 900
ctcatgagga gattgccatg gcgaccgtca cagcgctgcg ccgcacagtg ccccccgctg 960
tcactgggat caccttcctg tctggaggcc agagtgagga ggaggcgtcc atcaacctca 1020
atgccattaa caagtgcccc ctgctgaagc cctgggccct gaccttctcc tacggccgag 1080
ccctgcaggc ctctgccctg aaggcctggg gcgggaagaa ggagaacctg aaggctgcgc 1140
aggaggagta tgtcaagcga gccctggcca acagccttgc ctgtcaagga aagtacactc 1200
cgagcggtca ggctggggct gctgccagcg agtccctctt cgtctctaac cacgcctatt 1260
aagcggaggt gttcccaggc tgcccccaac aactccaggc cctgccccct cccactcttg 1320
aagaggaggc cgcctcctcg gggctccagg ctggcttgcc cgcgctcttt cttccctcgt 1380
gacagtggtg tgtggtgtcg tctgtgaatg ctaagtccat caccctttcc ggcacactgc 1440
caaataaaca gctatttaag gggg 1464

<210> 800
<211> 364
<212> PRT
<213> Homo Sapiens

<400> 800
Met Pro Tyr Gln Tyr Pro Ala Leu Thr Pro Glu Gln Lys Lys Glu Leu
1               5                   10                  15
Ser Asp Ile Ala His Arg Ile Val Ala Pro Gly Lys Gly Ile Leu Ala
            20                  25                  30
Ala Asp Glu Ser Thr Gly Ser Ile Ala Lys Arg Leu Gln Ser Ile Gly
        35                  40                  45
Thr Glu Asn Thr Glu Glu Asn Arg Arg Phe Tyr Arg Gln Leu Leu Leu
    50                  55                  60
Thr Ala Asp Asp Arg Val Asn Pro Cys Ile Gly Gly Val Ile Leu Phe
65                  70                  75                  80
His Glu Thr Leu Tyr Gln Lys Ala Asp Asp Gly Arg Pro Phe Pro Gln
                85                  90                  95
Val Ile Lys Ser Lys Gly Gly Val Val Gly Ile Lys Val Asp Lys Gly
            100                 105                 110
Val Val Pro Leu Ala Gly Thr Asn Gly Glu Thr Thr Thr Gln Gly Leu
        115                 120                 125
Asp Gly Leu Ser Glu Arg Cys Ala Gln Tyr Lys Lys Asp Gly Ala Asp
    130                 135                 140
Phe Ala Lys Trp Arg Cys Val Leu Lys Ile Gly Glu His Thr Pro Ser
145                 150                 155                 160
Ala Leu Ala Ile Met Glu Asn Ala Asn Val Leu Ala Arg Tyr Ala Ser
                165                 170                 175
Ile Cys Gln Gln Asn Gly Ile Val Pro Ile Val Glu Pro Glu Ile Leu
            180                 185                 190
Pro Asp Gly Asp His Asp Leu Lys Arg Cys Gln Tyr Val Thr Glu Lys
        195                 200                 205
Val Leu Ala Ala Val Tyr Lys Ala Leu Ser Asp His His Ile Tyr Leu
    210                 215                 220
Glu Gly Thr Leu Leu Lys Pro Asn Met Val Thr Pro Gly His Ala Cys
225                 230                 235                 240

Thr Gln Lys Phe Ser His Glu Glu Ile Ala Met Ala Thr Val Thr Ala
                245                 250                 255
Leu Arg Arg Thr Val Pro Pro Ala Val Thr Gly Ile Thr Phe Leu Ser
            260                 265                 270
Gly Gly Gln Ser Glu Glu Glu Ala Ser Ile Asn Leu Asn Ala Ile Asn
        275                 280                 285
Lys Cys Pro Leu Leu Lys Pro Trp Ala Leu Thr Phe Ser Tyr Gly Arg
    290                 295                 300
Ala Leu Gln Ala Ser Ala Leu Lys Ala Trp Gly Gly Lys Lys Glu Asn
305                 310                 315                 320
Leu Lys Ala Ala Gln Glu Glu Tyr Val Lys Arg Ala Leu Ala Asn Ser
                325                 330                 335
Leu Ala Cys Gln Gly Lys Tyr Thr Pro Ser Gly Gln Ala Gly Ala Ala
            340                 345                 350
Ala Ser Glu Ser Leu Phe Val Ser Asn His Ala Tyr
        355                 360

<210> 801
<211> 3504
<212> DNA
<213> Homo Sapiens

<400> 801
ctctgctttt ctctttcaga gctgttgcgc agccattggt acctgtattg gggaaacata 60
gcatacaagc aagaagctta cagcctcagt ggcgaaaaat ttttcatgtc agagaccgag 120
aactcttgca gtcgtttatg tcatcccttc ttctccagac agaagatacc aaaaagttgc 180
aatcaaagat ctgttcatct tattgataaa gtcactaata agccaaaatg tctgtcaacg 240
tcaaccgcag cgtgtcagac cagttctatc gctacaagat gccccgtttg attgctaagg 300
ttgagggcaa aggaaatgga atcaagacag ttatagtcaa catggttgac gttgcaaagg 360
cgcttaatcg gcctccaacg tatcccacca aatattttgg ttgtgagctg ggagcacaga 420
cccagtttga tgttaagaat gaccgttaca ttgtcaatgg atctcatgag gcgaataagc 480
tgcaagacat gttggatgga ttcattaaaa aatttgttct ctgtcctgag tgtgagaatc 540
ctgaaacaga tctgcatgtc aatccaaaga agcaaacaat aggtaattct tgtaaagcct 600
gtgggtaccg aggcatgctt gacacacatc ataaactctg tacattcatt ctcaaaaacc 660
cacctgagaa tagtgacatt ggtacaggaa agaaagagaa agaaaagaaa aatagaaagg 720
gcaaggacaa ggaaaatggc tctgtatcca ccagtgagac accaccacct ccaccaccaa 780
atgaaattag tcctccacat gctgtggaag aagaggaaga tgatgattgg ggggaggata 840
caactgagga agctcaaagg cgcagaatgg atgaaatcag tgaccatgca aaaggtctga 900
cacttagcga tgatttggaa agaactgtag aagagcgtgt taacatcctg tttgattttg 960
ttaagaaaaa gaaagaagag ggcattattg attcatctga taaagacatt gtggctgagg 1020
cagaaagact ggatgtaaaa gccatgggcc ctctcgtttt gacagaagtt ctctttgatg 1080
agaagataag agagcaaatc aagaaataca ggcgccattt tttaagattt tgtcataaca 1140
acaaaaaggc ccagcggtac cttcttcatg gtttggaatg tgtggtagca atgcatcaag 1200
ctcagttgat ctccaagatt ccacatatct tgaaggagat gtatgatgca gaccttttag 1260
aggaagaggt cattatcagc tggtcagaaa aggcctctaa gaaatatgtc tcaaaagaac 1320
ttgccaaaga gattcgtgtc aaagcagagc catttattaa atggttgaag gaagcggagg 1380
aggaatcttc tggtggtgag gaagaagacg aagatgaaaa tattgaggtg gtatattcga 1440
agactgccag tgtaccaaaa gttgaaactg tgaagtctga caacaaggat gacgacattg 1500
atattgatgc catttaaaag gatggatgca acttagctta acagtgtaat gctgcaaatt 1560
tttctccatt atcagccaga agtgcaacat gtatgtgcaa gagctaaagt ggcttaacat 1620
catgctacac ttgatactaa aaagctatta ctgtgagtgg tctataatta agcccaatga 1680
gacatctagg gagtccatac atatcagtga gcagttgtag tttgcttatt tatagcatgt 1740
ttctttcgga aaaactagtg gtggacacat ttggatcaca tttatacagt tataaaaaat 1800
aaagatttga ttttggtcat tcttcagact ttgggctatg aatggcttat gctgaagtaa 1860
ttggctactt ttaggatgtt acaccattta ataacttaga cttcttaagt ttggtagatt 1920

gttaggtact gaagacttga agaatgcaaa caattataat gaccttactc agccattaag 1980
aaatgaagta ttttgaaagt tgtgtctcca gtccattgag attggcaact gacaattctt 2040
gtcattctaa ggaaatttga tgatttaatg acagtgtgac atcctcatga gaagtaaaaa 2100
tgacctgtgt gtcctatggt ttaagagcaa attttgaaac ttggagttgt ggtttttcag 2160
tttgtgtaca ctcaccccaa attgtagtct attgagtcat gtgcattgca cgttggataa 2220
gccagggaaa tgacaaataa gtattttgtg tgtatttagt ggttgctttg tactgagaga 2280
aaagctttga ggtgtgatta aatcgtaaac tctgattcta tttgggagaa acaggaaaaa 2340
ggtgcactta atctaaaaca gcataagttt tcaactttta cccttaaatt ataatttcaa 2400
gatgtttaga catactgtat cttgtgtttg atgtgttccc cctccctaat attatggttt 2460
attctttaat gccttttaat ttggatataa tagcttgtac tttagatttt ggttgctatc 2520
ttgccaaaat aagtgttact gtttttcaag cttgatcccc ttccctgatt gtcttattta 2580
aagagaaagt taaactcata cttctgagtc agagcctgta ttttggttaa gacttgggat 2640
attttttact tcacattgaa tatagctgga tacctgagaa gtctggtgat ggcactgggt 2700
gggtgcagct agctaaggcc tgaccagccc attcagagcc ttggacttca gacacaaaag 2760
tgagtttctt acccacttgc tggtgtaaac tctatctggg gtcctgacta tatttgaata 2820
cttgtcttca atattaaaaa acatagcaca tttttctttc tacaaaagta cattctggag 2880
ttaagaaccc atgtggttga tttgtgtgtg gcgtgctagc tcatacatta tttggatctt 2940
attctttgtg tcatccatct cacagattat aagactttga ttaatgtaaa aagtatgcgt 3000
taaaatcata ccaaacattt ggtaaaatta aaaccttgat gggaggctgg gcgtggaaca 3060
ggagccatat acctggaatg gtaacagggg aatgtgctat gtcacaccaa agaagtggga 3120
cttggaaagt cacttgtctc ctgggtttca gactctttgt tgcatggcca gcccatccat 3180
atgtcattac tttttgagat tctcaagtag atcagcacat ttcggcctca ggttggcaag 3240
attttgtctt agagctgttg ctttaaaggg aaatggtcag gtcttagaca cttaggaagg 3300
tcttgggctt ctgttcattc tggtgccaaa ccagtgggat tcaaatttca cacaatctgg 3360
gtttttattc atggaggtta acctggtaag agtaatcctt catggctcta ttgaggtgtc 3420
ttaaaaagtt tcctgtttca aacagctaca ttacttgatt aaaacaatgt tataaaatta 3480
aatttccccc tcctttcata ttaa 3504

<210> 802
<211> 429
<212> PRT
<213> Homo Sapiens

<400> 802
Met Ser Val Asn Val Asn Arg Ser Val Ser Asp Gln Phe Tyr Arg Tyr
1               5                   10                  15
Lys Met Pro Arg Leu Ile Ala Lys Val Glu Gly Lys Gly Asn Gly Ile
            20                  25                  30
Lys Thr Val Ile Val Asn Met Val Asp Val Ala Lys Ala Leu Asn Arg
        35                  40                  45
Pro Pro Thr Tyr Pro Thr Lys Tyr Phe Gly Cys Glu Leu Gly Ala Gln
    50                  55                  60
Thr Gln Phe Asp Val Lys Asn Asp Arg Tyr Ile Val Asn Gly Ser His
65                  70                  75                  80
Glu Ala Asn Lys Leu Gln Asp Met Leu Asp Gly Phe Ile Lys Lys Phe
                85                  90                  95
Val Leu Cys Pro Glu Cys Glu Asn Pro Glu Thr Asp Leu His Val Asn
            100                 105                 110
Pro Lys Lys Gln Thr Ile Gly Asn Ser Cys Lys Ala Cys Gly Tyr Arg
        115                 120                 125
Gly Met Leu Asp Thr His His Lys Leu Cys Thr Phe Ile Leu Lys Asn
    130                 135                 140
Pro Pro Glu Asn Ser Asp Ile Gly Thr Gly Lys Lys Glu Lys Glu Lys
145                 150                 155                 160
Lys Asn Arg Lys Gly Lys Asp Lys Glu Asn Gly Ser Val Ser Thr Ser

                165                 170                 175
Glu Thr Pro Pro Pro Pro Pro Pro Asn Glu Ile Ser Pro Pro His Ala
            180                 185                 190
Val Glu Glu Glu Glu Asp Asp Asp Trp Gly Glu Asp Thr Thr Glu Glu
        195                 200                 205
Ala Gln Arg Arg Arg Met Asp Glu Ile Ser Asp His Ala Lys Gly Leu
    210                 215                 220
Thr Leu Ser Asp Asp Leu Glu Arg Thr Val Glu Glu Arg Val Asn Ile
225                 230                 235                 240
Leu Phe Asp Phe Val Lys Lys Lys Lys Glu Glu Gly Ile Ile Asp Ser
                245                 250                 255
Ser Asp Lys Asp Ile Val Ala Glu Ala Glu Arg Leu Asp Val Lys Ala
            260                 265                 270
Met Gly Pro Leu Val Leu Thr Glu Val Leu Phe Asp Glu Lys Ile Arg
        275                 280                 285
Glu Gln Ile Lys Lys Tyr Arg Arg His Phe Leu Arg Phe Cys His Asn
    290                 295                 300
Asn Lys Lys Ala Gln Arg Tyr Leu Leu His Gly Leu Glu Cys Val Val
305                 310                 315                 320
Ala Met His Gln Ala Gln Leu Ile Ser Lys Ile Pro His Ile Leu Lys
                325                 330                 335
Glu Met Tyr Asp Ala Asp Leu Leu Glu Glu Glu Val Ile Ile Ser Trp
            340                 345                 350
Ser Glu Lys Ala Ser Lys Lys Tyr Val Ser Lys Glu Leu Ala Lys Glu
        355                 360                 365
Ile Arg Val Lys Ala Glu Pro Phe Ile Lys Trp Leu Lys Glu Ala Glu
    370                 375                 380
Glu Glu Ser Ser Gly Gly Glu Glu Glu Asp Glu Asp Glu Asn Ile Glu
385                 390                 395                 400
Val Val Tyr Ser Lys Thr Ala Ser Val Pro Lys Val Glu Thr Val Lys
                405                 410                 415
Ser Asp Asn Lys Asp Asp Asp Ile Asp Ile Asp Ala Ile
            420                 425

<210> 803
<211> 2251
<212> DNA
<213> Homo Sapiens

<400> 803
aggatgtctt ctggcaattt catataagta ttttttcaaa aatgtctctt ctgtcaaccc 60
cacgcctttg gcacaatgaa gtgggtaacc tttatttccc ttctttttct ctttagctcg 120
gcttattcca ggggtgtgtt tcgtcgagat gcacacaaga gtgaggttgc tcatcggttt 180
aaagatttgg gagaagaaaa tttcaaagcc ttggtgttga ttgcctttgc tcagtatctt 240
cagcagtgtc catttgaaga tcatgtaaaa ttagtgaatg aagtaactga atttgcaaaa 300
acatgtgtag ctgatgagtc agctgaaaat tgtgacaaat cacttcatac cctttttgga 360
gacaaattat gcacagttgc aactcttcgt gaaacctatg gtgaaatggc tgactgctgt 420
gcaaaacaag aacctgagag aaatgaatgc ttcttgcaac acaaagatga caacccaaac 480
ctcccccgat tggtgagacc agaggttgat gtgatgtgca ctgcttttca tgacaatgaa 540
gagacatttt tgaaaaaata cttatatgaa attgccagaa gacatcctta cttttatgcc 600
ccggaactcc ttttctttgc taaaaggtat aaagctgctt ttacagaatg ttgccaagct 660
gctgataaag ctgcctgcct gttgccaaag ctcgatgaac ttcgggatga agggaaggct 720
tcgtctgcca aacagagact caaatgtgcc agtctccaaa aatttggaga aagagctttc 780
aaagcatggg cagtggctcg cctgagccag agatttccca aagctgagtt tgcagaagtt 840
tccaagttag tgacagatct taccaaagtc cacacggaat gctgccatgg agatctgctt 900

gaatgtgctg atgacagggc ggaccttgcc aagtatatct gtgaaaatca ggattcgatc 960
tccagtaaac tgaaggaatg ctgtgaaaaa cctctgttgg aaaaatccca ctgcattgcc 1020
gaagtggaaa atgatgagat gcctgctgac ttgccttcat tagctgctga ttttgttgaa 1080
agtaaggatg tttgcaaaaa ctatgctgag gcaaaggatg tcttcctggg catgtttttg 1140
tatgaatatg caagaaggca tcctgattac tctgtcgtgc tgctgctgag acttgccaag 1200
acatatgaaa ccactctaga gaagtgctgt gccgctgcag atcctcatga atgctatgcc 1260
aaagtgttcg atgaatttaa acctcttgtg gaagagcctc agaatttaat caaacaaaac 1320
tgtgagcttt ttaagcagct tggagagtac aaattccaga atgcgctatt agttcgttac 1380
accaagaaag taccccaagt gtcaactcca actcttgtag aggtctcaag aaacctagga 1440
aaagtgggca gcaaatgttg taaacatcct gaagcaaaaa gaatgccctg tgcagaagac 1500
tatctatccg tggtcctgaa ccagttatgt gtgttgcatg agaaaacgcc agtaagtgac 1560
agagtcacaa aatgctgcac agagtccttg gtgaacaggc gaccatgctt ttcagctctg 1620
gaagtcgatg aaacatacgt tcccaaagag tttaatgctg aaacattcac cttccatgca 1680
gatatatgca cactttctga gaaggagaga caaatcaaga aacaaactgc acttgttgag 1740
cttgtgaaac acaagcccaa ggcaacaaaa gagcaactga aagctgttat ggatgatttc 1800
gcagcttttg tagagaagtg ctgcaaggct gacgataagg agacctgctt tgccgaggag 1860
ggtaaaaaac ttgttgctgc aagtcaagct gccttaggct tataacatct acatttaaaa 1920
gcatctcagc ctaccatgag aataagagaa agaaaatgaa gatcaaaagc ttattcatct 1980
gttttctttt tcgttggtgt aaagccaaca ccctgtctaa aaaacataaa tttctttaat 2040
cattttgcct cttttctctg tgcttcaatt aataaaaaat ggaaagaatc taatagagtg 2100
gtacagcact gttatttttc aaagatgtgt tgctatcctg aaaattctgt aggttctgtg 2160
gaagttccag tgttctctct tattccactt cggtagagga tttctagttt ctgtgggcta 2220
attaaataaa tcactaatac tcttctaagt t 2251

<210> 804
<211> 609
<212> PRT
<213> Homo Sapiens

<400> 804

<210> 805

<211> 1356
<212> DNA
<213> Homo Sapiens

<400> 805
acaaacacca aggagtggag gtcagagtgt cacttttttg ttttcttttt gaaagatcat 60
tcgagaaaca cgtcactgat ctcccctgcg accatgtctt ccattaagat tgagtgtgtt 120
ttgccagaga actgccggtg tggtgagtct ccagtatggg aggaagtgtc caactctctg 180
ctctttgtag acattcctgc aaaaaaggtt tgccggtggg attcattcac caagcaagta 240
cagcgagtga ccatggatgc cccagtcagc tccgtggctc ttcgccagtc gggaggctat 300
gttgccacca ttggaacaaa gttctgtgct ttgaactgga aagaacaatc agcagttgtc 360
ttggccacgg tggataacga caagaaaaac aatcgcttca atgatgggaa ggtggatccc 420
gccgggaggt actttgctgg caccatggct gaggaaacag ctccagcagt tcttgagcgg 480
caccaggggg ccctgtactc cctctttcct gatcaccacg tgaaaaagta ctttgaccag 540
gtggacattt ccaatggttt ggattggtcg ctagaccaca aaatcttcta ttacattgac 600
agcctgtcct actccgtgga tgcctttgac tatgacctgc agacaggaca gatctccaac 660
cgcagaagtg tttacaagct agaaaaggaa gaacaaatcc cagatggaat gtgtattgat 720
gctgagggga agctctgggt ggcctgttac aatggaggaa gagtgattcg tttagatcct 780
gtgacaggga aaagacttca aactgtgaag ttgcctgttg ataaaacaac ttcatgctgc 840
tttggaggga agaattactc tgaaatgtat gtgacctgcg cccgggatgg gatggacccc 900
gagggtcttt tgaggcaacc tgaagctggt ggaattttca agataactgg tctgggggtc 960
aaaggaattg ctccctactc ctatgcggga tgaggacagg tcttctttcc tgccagaggg 1020
agctctgaag acaactagag aattctgggc ctgaaatttc aatctagtta gaaagaaaaa 1080
tgaggcaatg attttattaa cagcgttaag ttttaattta caacttttaa aaggcagagc 1140
atttttaaca aggggtgaca ggtggttttg ataacacact tataaggctt tctgtaaaag 1200
gtactataga agggcgaaga atcgttcaac tgtcaatcag cctcttgatt ctttgtaaat 1260
tgccagggtg ggtgggtaca tatctcttct tgattctgca tttcatactt aactatatta 1320
aagcttcaag gaacaataaa tagtaacctg gtaatg 1356

<210> 806
<211> 299
<212> PRT
<213> Homo Sapiens

<400> 806
Met Ser Ser Ile Lys Ile Glu Cys Val Leu Pro Glu Asn Cys Arg Cys
1               5                   10                  15
Gly Glu Ser Pro Val Trp Glu Glu Val Ser Asn Ser Leu Leu Phe Val
            20                  25                  30
Asp Ile Pro Ala Lys Lys Val Cys Arg Trp Asp Ser Phe Thr Lys Gln
        35                  40                  45
Val Gln Arg Val Thr Met Asp Ala Pro Val Ser Ser Val Ala Leu Arg
    50                  55                  60
Gln Ser Gly Gly Tyr Val Ala Thr Ile Gly Thr Lys Phe Cys Ala Leu
65                  70                  75                  80
Asn Trp Lys Glu Gln Ser Ala Val Val Leu Ala Thr Val Asp Asn Asp
                85                  90                  95
Lys Lys Asn Asn Arg Phe Asn Asp Gly Lys Val Asp Pro Ala Gly Arg
            100                 105                 110
Tyr Phe Ala Gly Thr Met Ala Glu Glu Thr Ala Pro Ala Val Leu Glu
        115                 120                 125
Arg His Gln Gly Ala Leu Tyr Ser Leu Phe Pro Asp His His Val Lys
    130                 135                 140
Lys Tyr Phe Asp Gln Val Asp Ile Ser Asn Gly Leu Asp Trp Ser Leu
145                 150                 155                 160

Asp His Lys Ile Phe Tyr Tyr Ile Asp Ser Leu Ser Tyr Ser Val Asp
                165                 170                 175
Ala Phe Asp Tyr Asp Leu Gln Thr Gly Gln Ile Ser Asn Arg Arg Ser
            180                 185                 190
Val Tyr Lys Leu Glu Lys Glu Glu Gln Ile Pro Asp Gly Met Cys Ile
        195                 200                 205
Asp Ala Glu Gly Lys Leu Trp Val Ala Cys Tyr Asn Gly Gly Arg Val
    210                 215                 220
Ile Arg Leu Asp Pro Val Thr Gly Lys Arg Leu Gln Thr Val Lys Leu
225                 230                 235                 240
Pro Val Asp Lys Thr Thr Ser Cys Cys Phe Gly Gly Lys Asn Tyr Ser
                245                 250                 255
Glu Met Tyr Val Thr Cys Ala Arg Asp Gly Met Asp Pro Glu Gly Leu
            260                 265                 270
Leu Arg Gln Pro Glu Ala Gly Gly Ile Phe Lys Ile Thr Gly Leu Gly
        275                 280                 285
Val Lys Gly Ile Ala Pro Tyr Ser Tyr Ala Gly
    290                 295

<210> 807
<211> 1980
<212> DNA
<213> Homo Sapiens

<400> 807
atgccaagta gtttgctgct agcaaccaga aaccaaatcc tgtctatgat gaactgttgg 60
ttttcttgtg ctcccaagaa cagacatgca gcagattgga acaaatatga tgaccgattg 120
atgaaagccg cggagagggg agatgtagaa aaagtttcct caatccttgc taaaaagggc 180
atcaatccag gcaaactaga tgtggaaggc agatctgcct tccatgttgt ggcctcaaag 240
gggaatcttg aatgtttgaa tgccatcctt atacatggag ttgatattac aaccagtgac 300
actgcaggaa gaaatgctct tcacttggct gcaaagtatg ggcatgcatt gtgtctacaa 360
aaacttctac agtacaattg tcccactgaa catgcagacc tgcagggaag aaccgcactt 420
catgacgcag caatggcaga ctgtccttcc agcatacagc tgctttgtga ccatggggcc 480
tccgtgaatg ccaaagatgt ggatgggcgg acaccgctgg ttctggctac tcagatgtgt 540
aggccagcaa tctgtcaact gctgatagat cgaggggcag agattaattc cagagacaaa 600
caaaacagaa ctgctctcat gcttggttgc gagtatggtt gtaaggatgc tgtagaagtc 660
ttacttaaaa atggtgctga tgtaagcctg ctggatgcct tgggccatga tagttcttac 720
tatgcaagaa ttggtgacaa tctggacatt ctaactttat tgaagactgc gtcagaaaat 780
accaacaaag ggagagaact ttggaagaaa ggaccatctt tacagcagcg aaatttgccg 840
tacatgctag atgaagtaaa tgtgaagtca agtcagaggg agcatcgaaa cattcaggag 900
ctggagattg aaaatgaaga tttgaaagac aggttgagaa aaattcagca agaacagaga 960
atattactgg ataaagtcaa tggtttacaa ctacagctga atgaggaagt gatggttgct 1020
gatgatctgg aaagtgagaa agaaaagctg aagtctcttt tggtggctaa agaaaagcaa 1080
catgaagaaa gcctaagaac tattgagtct ctgaaaaaca gatttaaata ttttgagtgt 1140
acttccccag gggtgccagc ccacatgcaa agcaggtcta tgttaagacc actggagcta 1200
tcattaccca atcaaacctc atattctgaa aatgacctct taaagaaaga gttagaagca 1260
atgagaactt tctgcgaatc agccaaacaa gaccgcctca agctccagaa cggagtggcg 1320
cacaaggtgg ctgagtgcaa agctttagga ctagaatgtg aacgcatcaa ggaggactct 1380
gatgagcaga taaagcagtt agaagacgca ttgaaagatg tgcagaagag aatgtatgag 1440
tcggaaggta aagtaaaaca aatgcagaca cactttcttg cccttaaaga gcacctgacc 1500
agtgaagcag ctatagggaa tcacagacta atggaggagc tgaaggatca gttgaaggac 1560
atgaaagcga aatatgaggg tgcatcagca gaagtgggaa aactgcgaaa ccaaatcaaa 1620
caaaatgagc tgctagtaga acagtttagg agagatgaag gcaagctggt ggaagagaat 1680
aagcgattgc agaaggaact cagtatgtgt gaaacggagc gagacaagaa aggaaggagg 1740
gttgctgagg tggaaggcca ggtaaaggaa ctcttagcaa agctgacctt gtcagttcca 1800

actgaaaaat ttgagagcat gaagagctta ttatcaagcg aagtaaatga gaaggtgaaa 1860
aaaattggag agacagaaag agagtatgaa aaatcactta ctgaaatcag acagttaagg 1920
agagagcttg agaattgtaa gcgccaaact tcctcagcat gtcaagccag aggagcatga 1980

<210> 808
<211> 659
<212> PRT
<213> Homo Sapiens

<400> 808
Met Pro Ser Ser Leu Leu Leu Ala Thr Arg Asn Gln Ile Leu Ser Met
1               5                   10                  15
Met Asn Cys Trp Phe Ser Cys Ala Pro Lys Asn Arg His Ala Ala Asp
            20                  25                  30
Trp Asn Lys Tyr Asp Asp Arg Leu Met Lys Ala Ala Glu Arg Gly Asp
        35                  40                  45
Val Glu Lys Val Ser Ser Ile Leu Ala Lys Lys Gly Ile Asn Pro Gly
    50                  55                  60
Lys Leu Asp Val Glu Gly Arg Ser Ala Phe His Val Val Ala Ser Lys
65                  70                  75                  80
Gly Asn Leu Glu Cys Leu Asn Ala Ile Leu Ile His Gly Val Asp Ile
                85                  90                  95
Thr Thr Ser Asp Thr Ala Gly Arg Asn Ala Leu His Leu Ala Ala Lys
            100                 105                 110
Tyr Gly His Ala Leu Cys Leu Gln Lys Leu Leu Gln Tyr Asn Cys Pro
        115                 120                 125
Thr Glu His Ala Asp Leu Gln Gly Arg Thr Ala Leu His Asp Ala Ala
    130                 135                 140
Met Ala Asp Cys Pro Ser Ser Ile Gln Leu Leu Cys Asp His Gly Ala
145                 150                 155                 160
Ser Val Asn Ala Lys Asp Val Asp Gly Arg Thr Pro Leu Val Leu Ala
                165                 170                 175
Thr Gln Met Cys Arg Pro Ala Ile Cys Gln Leu Leu Ile Asp Arg Gly
            180                 185                 190
Ala Glu Ile Asn Ser Arg Asp Lys Gln Asn Arg Thr Ala Leu Met Leu
        195                 200                 205
Gly Cys Glu Tyr Gly Cys Lys Asp Ala Val Glu Val Leu Leu Lys Asn
    210                 215                 220
Gly Ala Asp Val Ser Leu Leu Asp Ala Leu Gly His Asp Ser Ser Tyr
225                 230                 235                 240
Tyr Ala Arg Ile Gly Asp Asn Leu Asp Ile Leu Thr Leu Leu Lys Thr
                245                 250                 255
Ala Ser Glu Asn Thr Asn Lys Gly Arg Glu Leu Trp Lys Lys Gly Pro
            260                 265                 270
Ser Leu Gln Gln Arg Asn Leu Pro Tyr Met Leu Asp Glu Val Asn Val
        275                 280                 285
Lys Ser Ser Gln Arg Glu His Arg Asn Ile Gln Glu Leu Glu Ile Glu
    290                 295                 300
Asn Glu Asp Leu Lys Asp Arg Leu Arg Lys Ile Gln Gln Glu Gln Arg
305                 310                 315                 320
Ile Leu Leu Asp Lys Val Asn Gly Leu Gln Leu Gln Leu Asn Glu Glu
                325                 330                 335
Val Met Val Ala Asp Asp Leu Glu Ser Glu Lys Glu Lys Leu Lys Ser
            340                 345                 350
Leu Leu Val Ala Lys Glu Lys Gln His Glu Glu Ser Leu Arg Thr Ile

355                 360                 365
Glu Ser Leu Lys Asn Arg Phe Lys Tyr Phe Glu Cys Thr Ser Pro Gly
    370                 375                 380
Val Pro Ala His Met Gln Ser Arg Ser Met Leu Arg Pro Leu Glu Leu
385                 390                 395                 400
Ser Leu Pro Asn Gln Thr Ser Tyr Ser Glu Asn Asp Leu Leu Lys Lys
                405                 410                 415
Glu Leu Glu Ala Met Arg Thr Phe Cys Glu Ser Ala Lys Gln Asp Arg
            420                 425                 430
Leu Lys Leu Gln Asn Gly Val Ala His Lys Val Ala Glu Cys Lys Ala
        435                 440                 445
Leu Gly Leu Glu Cys Glu Arg Ile Lys Glu Asp Ser Asp Glu Gln Ile
    450                 455                 460
Lys Gln Leu Glu Asp Ala Leu Lys Asp Val Gln Lys Arg Met Tyr Glu
465                 470                 475                 480
Ser Glu Gly Lys Val Lys Gln Met Gln Thr His Phe Leu Ala Leu Lys
                485                 490                 495
Glu His Leu Thr Ser Glu Ala Ala Ile Gly Asn His Arg Leu Met Glu
            500                 505                 510
Glu Leu Lys Asp Gln Leu Lys Asp Met Lys Ala Lys Tyr Glu Gly Ala
        515                 520                 525
Ser Ala Glu Val Gly Lys Leu Arg Asn Gln Ile Lys Gln Asn Glu Leu
    530                 535                 540
Leu Val Glu Gln Phe Arg Arg Asp Glu Gly Lys Leu Val Glu Glu Asn
545                 550                 555                 560
Lys Arg Leu Gln Lys Glu Leu Ser Met Cys Glu Thr Glu Arg Asp Lys
                565                 570                 575
Lys Gly Arg Arg Val Ala Glu Val Glu Gly Gln Val Lys Glu Leu Leu
            580                 585                 590
Ala Lys Leu Thr Leu Ser Val Pro Thr Glu Lys Phe Glu Ser Met Lys
        595                 600                 605
Ser Leu Leu Ser Ser Glu Val Asn Glu Lys Val Lys Lys Ile Gly Glu
    610                 615                 620
Thr Glu Arg Glu Tyr Glu Lys Ser Leu Thr Glu Ile Arg Gln Leu Arg
625                 630                 635                 640
Arg Glu Leu Glu Asn Cys Lys Arg Gln Thr Ser Ser Ala Cys Gln Ala
                645                 650                 655
Arg Gly Ala

<210> 809
<211> 1725
<212> DNA
<213> Homo Sapiens

<400> 809
tttctttgtt aagtcgttcc ctctacaaag gacttcctag tgggtgtgaa aggcagcggt      60
ggccacagag gcggcggaga gatggccttc agcggttccc aggctcccta cctgagtcca     120
gctgtcccct tttctgggac tattcaagga ggtctccagg acggacttca gatcactgtc     180
aatgggaccg ttctcagctc cagtggaacc aggtttgctg tgaactttca gactggcttc     240
agtggaaatg acattgcctt ccacttcaac cctcggtttg aagatggagg gtacgtggtg     300
tgcaacacga ggcagaacgg aagctggggg cccgaggaga ggaagacaca catgcctttc     360
cagaagggga tgccctttga cctctgcttc ctggtgcaga gctcagattt caaggtgatg     420
gtgaacggga tcctcttcgt gcagtacttc caccgcgtgc ccttccaccg tgtggacacc     480
atctccgtca atggctctgt gcagctgtcc tacatcagct tccagaaccc ccgcacagtc     540

cctgttcagc ctgccttctc cacggtgccg ttctcccagc ctgtctgttt cccacccagg 600
cccagggggc gcagacaaaa acctcccggc gtgtggcctg ccaacccggc tcccattacc 660
cagacagtca tccacacagt gcagagcgcc cctggacaga tgttctctac tcccgccatc 720
ccacctatga tgtaccccca ccccgcctat ccgatgcctt tcatcaccac cattctggga 780
gggctgtacc catccaagtc catcctcctg tcaggcactg tcctgcccag tgctcagagg 840
ttccacatca acctgtgctc tgggaaccac atcgccttcc acctgaaccc ccgttttgat 900
gagaatgctg tggtccgcaa cacccagatc gacaactcct gggggtctga ggagcgaagt 960
ctgccccgaa aaatgccctt cgtccgtggc cagagcttct cagtgtggat cttgtgtgaa 1020
gctcactgcc tcaaggtggc cgtggatggt cagcacctgt ttgaatacta ccatcgcctg 1080
aggaacctgc ccaccatcaa cagactggaa gtggggggcg acatccagct gacccatgtg 1140
cagacatagg cggcttcctg gccctggggc cgggggctgg ggtgtggggc agtctgggtc 1200
ctctcatcat ccccacttcc caggcccagc ctttccaacc ctgcctggga tctgggcttt 1260
aatgcagagg ccatgtcctt gtctggtcct gcttctggct acagccaccc tggaacggag 1320
aaggcagctg acggggattg ccttcctcag ccgcagcagc acctggggct ccagctgctg 1380
gaatcctacc atcccaggag gcaggcacag ccagggagag gggaggagtg ggcagtgaag 1440
atgaagcccc atgctcagtc ccctcccatc ccccacgcag ctccacccca gtcccaagcc 1500
accagctgtc tgctcctggt gggaggtggc ctcctcagcc cctcctctct gacctttaac 1560
ctcactctca ccttgcaccg tgcaccaacc cttcacccct cctggaaagc aggcctgatg 1620
gcttcccact ggcctccacc acctgaccag agtgttctct tcagaggact ggctcctttc 1680
ccagtgtcct taaaataaag aaatgaaaat gcttgttggc acatt 1725

<210> 810
<211> 355
<212> PRT
<213> Homo Sapiens

<400> 810
Met Ala Phe Ser Gly Ser Gln Ala Pro Tyr Leu Ser Pro Ala Val Pro
1               5                   10                  15
Phe Ser Gly Thr Ile Gln Gly Gly Leu Gln Asp Gly Leu Gln Ile Thr
            20                  25                  30
Val Asn Gly Thr Val Leu Ser Ser Ser Gly Thr Arg Phe Ala Val Asn
        35                  40                  45
Phe Gln Thr Gly Phe Ser Gly Asn Asp Ile Ala Phe His Phe Asn Pro
    50                  55                  60
Arg Phe Glu Asp Gly Gly Tyr Val Val Cys Asn Thr Arg Gln Asn Gly
65                  70                  75                  80
Ser Trp Gly Pro Glu Glu Arg Lys Thr His Met Pro Phe Gln Lys Gly
                85                  90                  95
Met Pro Phe Asp Leu Cys Phe Leu Val Gln Ser Ser Asp Phe Lys Val
            100                 105                 110
Met Val Asn Gly Ile Leu Phe Val Gln Tyr Phe His Arg Val Pro Phe
        115                 120                 125
His Arg Val Asp Thr Ile Ser Val Asn Gly Ser Val Gln Leu Ser Tyr
    130                 135                 140
Ile Ser Phe Gln Asn Pro Arg Thr Val Pro Val Gln Pro Ala Phe Ser
145                 150                 155                 160
Thr Val Pro Phe Ser Gln Pro Val Cys Phe Pro Pro Arg Pro Arg Gly
                165                 170                 175
Arg Arg Gln Lys Pro Pro Gly Val Trp Pro Ala Asn Pro Ala Pro Ile
            180                 185                 190
Thr Gln Thr Val Ile His Thr Val Gln Ser Ala Pro Gly Gln Met Phe
        195                 200                 205
Ser Thr Pro Ala Ile Pro Pro Met Met Tyr Pro His Pro Ala Tyr Pro
    210                 215                 220

Met Pro Phe Ile Thr Thr Ile Leu Gly Gly Leu Tyr Pro Ser Lys Ser
225                 230                 235                 240
Ile Leu Leu Ser Gly Thr Val Leu Pro Ser Ala Gln Arg Phe His Ile
                245                 250                 255
Asn Leu Cys Ser Gly Asn His Ile Ala Phe His Leu Asn Pro Arg Phe
            260                 265                 270
Asp Glu Asn Ala Val Val Arg Asn Thr Gln Ile Asp Asn Ser Trp Gly
        275                 280                 285
Ser Glu Glu Arg Ser Leu Pro Arg Lys Met Pro Phe Val Arg Gly Gln
    290                 295                 300
Ser Phe Ser Val Trp Ile Leu Cys Glu Ala His Cys Leu Lys Val Ala
305                 310                 315                 320
Val Asp Gly Gln His Leu Phe Glu Tyr Tyr His Arg Leu Arg Asn Leu
                325                 330                 335
Pro Thr Ile Asn Arg Leu Glu Val Gly Gly Asp Ile Gln Leu Thr His
            340                 345                 350
Val Gln Thr
        355

<210> 811
<211> 1022
<212> DNA
<213> Homo Sapiens

<400> 811
gcctgtgggt ctccattgcc cagcttttgc ctgcactctt gcctgctgcc ctgaccagag      60
tcatcatgtc tcttgagcag aagagtcagc actgcaagcc tgaggaaggc gttgaggccc     120
aagaagaggc cctgggcctg gtgggtgcac aggctcctac tactgaggag caggaggctg     180
ctgtctcctc ctcctctcct ctggtcctgg gcaccctgga gaaagtgcct gctgctgagt     240
cagcagatcc tccccagagt cctcagggag cctctgcctt acccactacc atcagcttca     300
cttgctggag gcaacccaat gagggttcca gcagccaaga agaggaggag gccagcacct     360
cgcctgacgc agagtccttg ttccgagaag cactcagtaa caaggtggat gagttggctc     420
attttctgct ccgcaagtat cgagccaagg agctggtcac aaaggcagaa atgctggaga     480
gagtcatcaa aaattacaag cgctgctttc ctgtgatctt cggcaaagcc tccgagtccc     540
tgaagatgat ctttggcatt gacgtgaagg aagtggaccc cgccagcaac acctacaccc     600
ttgtcacctg cctgggcctt tcctatgatg gcctgctggg taataatcag atctttccca     660
agacaggcct cctgataatc gtcctgggca caattgcaat ggagggcgac agcgcctctg     720
aggaggaaat ctgggaggag ctgggtgtga tgggggtgta tgatgggagg gagcacactg     780
tctatgggga gcccaggaaa ctgctcaccc aagattgggt gcaggaaaac tacctggagt     840
accggcaggt acccggcagt aatcctgcgc gctatgagtt cctgtggggt ccaagggctc     900
tggctgaaac cagctatgtg aaagtcctgg agcatgtggt cagggtcaat gcaagagttc     960
gcattgccta cccatccctg cgtgaagcag ctttgttaga ggaggaagag ggagtctgag    1020
ca                                                                  1022

<210> 812
<211> 317
<212> PRT
<213> Homo Sapiens

<400> 812

<210> 813
<211> 5175
<212> DNA
<213> Homo Sapiens

<400> 813
gctgctgctg cagtgggaca ggtggcggcg accggcggcg tccgaggaga tttaatccag      60
agactgactt cactatagaa cccacagttg tatcaatggt tggggaaaga tagtggcaac     120
aggcaaagga gaaacagctc tgacatacaa agaaaatgag tatgctaaag ccaagtgggc     180
ttaaggcccc caccaagatc ctgaagcctg gaagcacagc tctgaagaca cctacggctg     240
ttgtagctcc agtagaaaaa accatatcca gtgaaaaagc atcaagcact ccatcatctg     300
agactcagga ggaatttgtg gatgactttc gagttgggga gcgagtttgg gtgaatggaa     360
ataagcctgg atttatccag tttcttggag aaacccagtt tgcaccaggc cagtgggctg     420
gaattgtttt agatgaaccc ataggcaaga acgatggttc ggtggcagga gttcggtatt     480
tccagtgtga acctttaaag ggcatattta cccgaccttc aaagttaaca aggaaggtgc     540
aagcagaaga tgaagctaat ggcctgcaga caacgcccgc ctcccgagct acttcaccgc     600
tgtgcacttc tacggccagc atggtgtctt cctccccctc caccccttca aacatccctc     660
agaaaccatc acagccagca gcaaaggaac cttcagctac gcctccgatc agcaacctta     720
caaaaactgc cagtgaatct atctccaacc tttcagaggc tggctcaatc aagaaaggag     780

aaagagagct caaaatcgga gacagagtat tggttggtgg cactaaggct ggtgtagtcc 840
ggtttcttgg ggagaccgac tttgccaagg gggagtggtg tggcgtggag ttagatgagc 900
cacttgggaa gaatgatggc gctgttgctg gaacaaggta ttttcagtgt caacccaaat 960
atggcttgtt cgctcctgtc cacaaagtta ccaagattgg cttcccttcc actacaccag 1020
ccaaagccaa ggccaacgca gtgaggcgag tgatggcgac cacgtccgcc agcctgaagc 1080
gcagcccttc tgcctcttcc ctcagctcca tgagctcagt ggcctcctct gtgagcagca 1140
ggcccagtcg gacaggacta ttgactgaaa cctcctcccg ttacgccagg aagatctccg 1200
gtaccactgc cctccaggag gccctgaagg agaagcagca gcacattgag cagctgctgg 1260
cggaacggga tctggagagg gcggaggtgg ccaaggccac gagccacgtg ggggagatag 1320
agcaggagct agctctggcc cgggacggac atgaccagca tgtcctggaa ttggaagcca 1380
aaatggacca gctgcgaaca atggtggaag ctgctgacag ggagaaggtg gagcttctca 1440
accagcttga agaggagaaa aggaaggttg aggaccttca gttccgggtt gaagaagaat 1500
caattaccaa aggtgatctt gaggtggcta cagtttcaga aaagtcacgt ataatggaac 1560
tggagaaaga cctagcattg agagtacagg aagtagctga gctccgaaga aggctagagt 1620
ccaataagcc tgctggggat gtggacatgt cactttccct tttgcaagag ataagctctt 1680
tgcaagaaaa gttagaagtc acccgtactg accaccagag agaaataact tctctgaagg 1740
agcattttgg agcccgggaa gaaactcatc agaaggagat aaaggctctg tataccgcca 1800
cggaaaagct ttccaaagag aacgagtcat tgaaaagcaa gctggagcat gccaacaaag 1860
agaactcaga tgtgatagct ctatggaagt ccaaactgga gactgccatc gcatcccacc 1920
agcaggcgat ggaagaactg aaggtatctt tcagcaaagg gcttggaaca gagacggcag 1980
aatttgctga actaaaaaca caaatagaga aaatgagact agattaccaa cacgaaatag 2040
aaaatttgca gaatcaacaa gactctgaac gggctgccca tgctaaagag atggaagcct 2100
tgagggctaa actgatgaaa gttattaaag aaaaggaaaa cagtctggaa gccatcaggt 2160
cgaaactgga caaagcagaa gaccagcatc tcgtagaaat ggaagacacg ttaaacaaat 2220
tacaggaagc tgaaataaag gtaaaggagc tagaggtact gcaagccaaa tgcaatgaac 2280
aaaccaaggt tattgataat tttacatcac agctcaaggc tactgaagaa aagctcttgg 2340
atcttgatgc acttcggaaa gccagttccg aaggtaaatc ggaaatgaag aaacttagac 2400
agcagcttga ggcagctgag aaacagatta aacatttaga gattgaaaag aatgctgaaa 2460
gtagcaaggc tagtagcatt accagagagc tccaggggag agagctaaag cttactaacc 2520
ttcaggaaaa tttgagtgaa gtcagtcaag tgaaagagac tttggaaaaa gaacttcaga 2580
ttttgaaaga aaagtttgct gaagcttcag aggaggcagt ctctgttcag agaagtatgc 2640
aagaaactgt aaataagtta caccaaaagg aggaacagtt taacatgctg tcttctgact 2700
tggagaagct gagagaaaac ttagcagata tggaggcaaa atttagagag aaagatgaga 2760
gagaagagca gctgataaag gcaaaggaaa aactggaaaa tgacattgca gaaataatga 2820
agatgtcagg agataactct tctcagctga caaaaatgaa cgatgaatta cgtctgaaag 2880
aaagagatgt agaagaatta cagctaaaac ttacaaaggc taatgaaaat gcaagttttc 2940
tgcaaaaaag tattgaggac atgactgtca aagctgaaca gagccagcaa gaagcagcta 3000
aaaagcatga ggaagaaaag aaagaattgg agaggaaatt gtcggacctg gaaaagaaaa 3060
tggaaacaag ccacaaccag tgtcaggagc tgaaagccag gtatgagaga gccacttctg 3120
agacaaaaac caagcatgaa gaaatcctac agaacctcca gaagacgctg ctggacacag 3180
aggacaagct gaagggcgca cgggaggaga acagtggctt gctgcaggag ctggaggagc 3240
tgagaaagca agccgagaaa gccaaagctg ctcaaacagc ggaagatgcc atgcagataa 3300
tggaacagat gaccaaagag aagactgaga ctctggcctc cttggaggac accaagcaaa 3360
caaatgcaaa actacagaat gaattggaca cacttaaaga aaacaacttg aaaaatgtgg 3420
aagagctgaa caaatcaaaa gaactcctga ctgtagagaa tcaaaaaatg gaagaattta 3480
ggaaagaaat agaaacccta aagcaggcag cagctcagaa gtcccagcag ctttcagcgt 3540
tgcaagaaga gaacgttaaa cttgctgagg agctggggag aagcagggac gaagtcacaa 3600
gtcatcaaaa gctggaagaa gaaagatctg tgctcaataa tcagttgtta gaaatgaaaa 3660
aaagagaatc caagttcata aaagacgcag atgaagagaa agcttccttg cagaaatcca 3720
tcagtataac tagtgcctta ctcacagaaa aggatgccga gctggagaaa ctgagaaatg 3780
aggtcacagt gctcagggga gaaaacgcct ctgccaagtc cttgcattca gttgttcaga 3840
ctctagagtc tgataaggtg aagctcgagc tcaaggtaaa gaacttggag cttcaactca 3900
aagaaaacaa gaggcagctc agcagctcct caggtaatac agacactcag gcagacgagg 3960
atgaaagagc ccaggagagt cagattgatt tcctaaattc agtaatagtg gaccttcaaa 4020
ggaagaatca agacctcaag atgaaggtgg agatgatgtc agaagcagcc ctgaatggga 4080

acggggatga cctaaacaat tatgacagtg atgatcagga gaaacagtcc aagaagaaac 4140
ctcgcctctt ctgtgacatt tgtgactgct ttgatctcca cgacacagag gattgtccta 4200
cccaggcaca gatgtcagag gaccctcccc attccacaca ccatggcagt cggggtgagg 4260
aacgcccata ctgtgaaatc tgtgagatgt ttggacactg ggccaccaac tgcaatgacg 4320
acgaaacctt ctgatgaagc ctccagtgga gaactgggct tgctcagacg cactcgcatt 4380
gacacaacgt aacaccagca ttgtgtgtgc agacttcagg agaactcatg ttatttttta 4440
accccgtcaa caaatctagg aaaatatttt gatcttcaac aaattgccct ttagtctccc 4500
cgtatgagtt agaataataa atatttagta ggtgagcttt tcacctcgaa ttttgttttc 4560
ttgattttta cgtttgaaga cattgcacca gatgcsatta catttattgg ccccccgacc 4620
ttgtagaaaa acccctaccc tcacaatacc ttatttaagt aactttaaat tatgccgtta 4680
cttttcatat ttgcactaag atatttccag gctgcatttg tatatttaga ttttttggtt 4740
aagctttgac actggaatga gttgaaaaaa tgtgccattt tgcattttca tctactcatt 4800
taaagtattt tattcttatt caaagaaata tctgagctct ttgcactacc tgttatcagt 4860
agtgccttta cttcaggctt gataatactt aggtgtgatt ataaaatcat gaagcaggta 4920
aagggagggg caagccccca aactgctgtg ggacattttt ataatctata tgctgcaccc 4980
acttaatcta ctgtggtgtt ttgtttatta gttttgcata atttcagctt ctatatattg 5040
tatgtatata ttttttaaaa atctatattt tgggaaaaaa acatacacaa tgtgtctttc 5100
tttttggaca tttacctttt tgaaaaagaa aacacttaaa atgatcatta ggacataaca 5160
gactagggaa ttccg 5175

<210> 814
<211> 1392
<212> PRT
<213> Homo Sapiens

<400> 814

<210> 815
<211> 647
<212> DNA
<213> Homo Sapiens

<400> 815
ccacgcgtcc gcctcccgtt ccctcttccg cttgcgctgc cgcaggacca tggccaacct      60
ggagcgcacc ttcatcgcca tcaagccgga cggcgtgcag cgcggcctgg tgggcgagat     120
catcaagcgc ttcgagcaga agggattccg cctcgtggcc atgaagttcc tccgggcctc     180
tgaagaacac ctgaagcagc actacattga cctgaaagac cgaccattct tccctgggct     240
ggtgaagtac atgaactcag ggccggttgt ggccatggtc tgggaggggc tgaacgtggt     300
gaagacaggc cgagtgatgc ttggggagac caatccagca gattcaaagc caggcaccat     360
tcgtggggac ttctgcattc aggttggcag gaacatcatt catggcagtg attcagtaaa     420
aagtgctgaa aaagaaatca gcctatggtt taagcctgaa gaactggttg actacaagtc     480
ttgtgctcat gactgggtct atgaataaga ggtggacaca acagcagtct ccttcagcac     540
ggcgtggtgt gtccctggac acagctcttc attccattga cttagaggca acaggattga     600
tcattctttt atagagcata tttgccaata aagcttttgg aagccgg                   647

<210> 816
<211> 152
<212> PRT
<213> Homo Sapiens

<400> 816
Met Ala Asn Leu Glu Arg Thr Phe Ile Ala Ile Lys Pro Asp Gly Val
1               5                   10                  15
Gln Arg Gly Leu Val Gly Glu Ile Ile Lys Arg Phe Glu Gln Lys Gly
            20                  25                  30
Phe Arg Leu Val Ala Met Lys Phe Leu Arg Ala Ser Glu Glu His Leu
        35                  40                  45
Lys Gln His Tyr Ile Asp Leu Lys Asp Arg Pro Phe Phe Pro Gly Leu
    50                  55                  60
Val Lys Tyr Met Asn Ser Gly Pro Val Val Ala Met Val Trp Glu Gly
65                  70                  75                  80
Leu Asn Val Val Lys Thr Gly Arg Val Met Leu Gly Glu Thr Asn Pro
                85                  90                  95
Ala Asp Ser Lys Pro Gly Thr Ile Arg Gly Asp Phe Cys Ile Gln Val
            100                 105                 110
Gly Arg Asn Ile Ile His Gly Ser Asp Ser Val Lys Ser Ala Glu Lys
        115                 120                 125
Glu Ile Ser Leu Trp Phe Lys Pro Glu Glu Leu Val Asp Tyr Lys Ser
    130                 135                 140
Cys Ala His Asp Trp Val Tyr Glu
145                 150